(19) Europäisches Patentamt
European Patent Office
Office européen des brevets

(11) EP 4 549 438 A1

(12) EUROPEAN PATENT APPLICATION
published in accordance with Art. 153(4) EPC

(43) Date of publication:
**07.05.2025 Bulletin 2025/19**

(21) Application number: **23830448.9**

(22) Date of filing: **29.06.2023**

(51) International Patent Classification (IPC):
***C07D 471/04*** $^{(2006.01)}$ ***A61K 31/437*** $^{(2006.01)}$
***A61P 35/00*** $^{(2006.01)}$

(52) Cooperative Patent Classification (CPC):
**A61K 31/437; A61K 31/438; A61K 31/4545; A61K 31/496; A61K 31/4985; A61K 31/4995; A61K 31/5377; A61K 31/675; A61P 15/14; A61P 35/00; C07D 471/04; C07D 519/00; C07F 9/6561; C07F 9/6584**

(86) International application number:
**PCT/CN2023/104130**

(87) International publication number:
**WO 2024/002284 (04.01.2024 Gazette 2024/01)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **29.06.2022 CN 202210784933**
**06.12.2022 CN 202211559610**
**19.06.2023 CN 202310730604**

(71) Applicant: **Hangzhou Synrx Therapeutics Biomedical Technology Co., Ltd.**
**Hangzhou, Zhejiang 311121 (CN)**

(72) Inventors:
- **HE, Hu**
  **Hangzhou, Zhejiang 311121 (CN)**
- **LIU, Song**
  **Hangzhou, Zhejiang 311121 (CN)**
- **GE, Chongxun**
  **Hangzhou, Zhejiang 311121 (CN)**
- **SHI, Song**
  **Hangzhou, Zhejiang 311121 (CN)**
- **CUI, Yaqi**
  **Hangzhou, Zhejiang 311121 (CN)**
- **JIN, Huihui**
  **Hangzhou, Zhejiang 311121 (CN)**

(74) Representative: **dompatent von Kreisler Selting Werner - Partnerschaft von Patent- und Rechtsanwälten mbB**
**Deichmannhaus am Dom**
**Bahnhofsvorplatz 1**
**50667 Köln (DE)**

# (54) FIVE-MEMBERED AND SIX-MEMBERED NITROGEN-CONTAINING COMPOUND, AND INTERMEDIATE, PREPARATION METHOD AND USE THEREOF

(57) Disclosed are a five-membered and six-membered nitrogen-containing compound represented by formula I, a pharmaceutically acceptable salt or isotopic compound thereof, as well as an intermediate, preparation method, and use thereof. The permeability and/or solubility of the compound are greatly improved compared with the prior art.

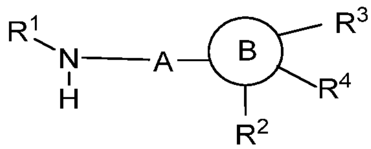

I

EP 4 549 438 A1

## Description

The present application claims the priority to Chinese Patent Application No. 2022107849339 filed on June 29, 2022. The present application claims the priority to Chinese Patent Application No. 2022115596106 filed on December 06, 2022. The present application claims the priority to Chinese Patent Application No. 2023107306040 filed on June 19, 2023. The present application makes reference to the full texts of the Chinese Patent Applications above.

FIELD OF THE INTVENTION

The present invention relates to a five-membered and six-membered nitrogen-containing compound, and intermediate, preparation method and use thereof.

BACKGROUND OF THE INVENTION

PARG, the first identified poly (ADP-ribose) hydrolase that is encoded by a single gene (Mirella et al. Human poly (ADP-ribose) glycohydrolase is expressed in alternative splice variants yielding isoforms that localize to different cell compartments. Experimental Cell Research, 2004, 297(2):521-532.), can hydrolyze the O-glycosidic bonds between ADP-ribose subunits. PARG consists of four domains (Meyer B et al. Clustered DNA damage induces pan-nuclear H2AX phosphorylation mediated by ATM and DNA-PK. Nucleic Acids Res, 2003, 41:6109-6118.; O'Sullivan J. et al. Emerging roles of eraser enzymes in the dynamic control of protein ADP-ribosylation. Nature Communication, 2019, 10,1182.), including an N-terminus disordered domain, a linker domain, a C-terminus catalytic domain, and an ADP-ribose binding domain. The non-conserved N-terminus disordered domain is not essential for in vitro activity, and the C-terminus ADP-ribose binding domain is highly conserved with other macro-domains and forms, together with the catalytic domain, the smallest structure with intact enzymatic activity in vitro (Slade D et al. The structure and catalytic mechanism of a poly (ADP-ribose) glycohydrolase. Nature, 2011, 477:616-620.).

Poly ADP-ribosylation (PARylation) is a unique post-translational modification, which is crucial for maintaining the genome stability in different signaling pathways, in particular in DNA damage repair. Poly ADP-ribose, PAR for short, consists of adenosine diphosphate-ribose units. It is formed with $NAD^+$ as a donor under the catalysis of poly ADP-ribose polymerases (PARPs), and can be rapidly degraded by polyribosyl hydrolases. After DNA damage, many DNA damage response factors recognize PARylation and are recruited by PARylation to places near the site of DNA damage. However, the PARylation needs to be digested timely, such that the DNA damage response factors can directly recognize DNA damage and fulfill their repair functions. Or else, DNA repair factors are trapped near the site of DNA damage by PARylation and cannot perform repair properly. Therefore, inhibiting the dePARylation, which is the direct downstream step of PARylation in DNA repair, would affect the PARylationdependent DNA damage repair and selectively kill tumor cells with DNA repair deficiency.

PARG, as the most important poly (ADP-ribose) hydrolase, exerts its function in about 90% of intracellular PAR (Laetitia D et al. Poly (ADP-ribose) glycohydrolase (PARG) and its therapeutic potential. Front Biosci, 2009, 14(5): 1619-1626.), as well as in many cellular processes such as DNA damage repair, DNA replication, chromatin regulation, transcription and apoptosis. Its dysfunction leads to the imbalance of intracellular PAR level and thus affect the transformation and invasion of cancer cells (Rack J D et al. Macrodomain: structure, function, evolution, and catalytic activities. Annu Rev Biochem. 2016, 85:431-454; Marques M et al. Oncogenic activity of poly (ADP-ribose) glycohydrolase. Oncogene, 2019, 38:2177-2191).

PARG has been shown to be associated with a variety of diseases, and there are data suggesting that (Maud M et al, Oncogenic activity of poly (ADP-ribose) glycohydrolase. Oncogene, 2019, 38:2177-2191.) the elevated PARG is associated with poor prognosis of HER2+ patients, and PARG and HER2 synergistically promote the growth of breast cancer cells, and inhibiting the PARG significantly affects the growth and migration of breast cancer. In addition, studies have shown that (Mincheng Yet al. PARG inhibition limits HCC progression and potentiates the efficacy of immune checkpoint therapy. Hepatic and Biliary Cancer, 2022, S0168-8278(22)00072-1.) high expression of PARG is closely related to poor prognosis of liver cancer, and hepatocytespecific PARG function loss affects oncogenesis. Hence, PARG inhibitors have attracted more and more attention as potential therapeutic means. At present, there are a variety of PARG inhibitors under development, which are all in the preclinical research stage. Therefore, it is of great research significance to develop small molecule medicaments capable of targeting and inhibiting PARG activity to provide patients with safer and more effective PARG inhibitors.

SUMMARY OF THE INVENTION

The technical problem to be solved by the present invention is to overcome the defect of relatively unitary structure of PARG inhibitor compounds in the prior art, and to this end, the present invention provides a five-membered and

six-membered nitrogen-containing compound, and intermediate, preparation method and use thereof. The compound of the present invention has a better PARG inhibitory activity. In addition, the compound of the present invention is greatly improved in permeability and/or solubility compared with the existing inventions.

The present invention provides a compound of formula I, a pharmaceutically acceptable salt thereof or an isotopic compound thereof;

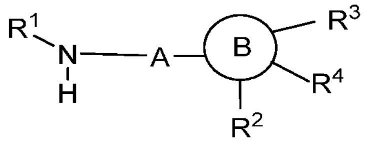

I

A is

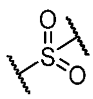, 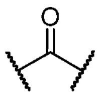 or 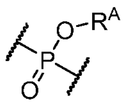;

$R^A$ is hydrogen or $C_1$-$C_6$ alkyl;

ring B is a 5-membered-fused 6-membered heteroaromatic ring unsubstituted or substituted by one or two $R^B$ or a 5-membered-fused 5-membered heteroaromatic ring unsubstituted or substituted by one or two $R^B$; the heteroatom in the 5-membered-fused 6-membered heteroaromatic ring is one or more selected from N, O and S, and the number of heteroatom(s) is 1, 2 or 3; the heteroatom in the 5-membered-fused 5-membered heteroaromatic ring is one or more selected from N, O and S, and the number of heteroatom(s) is 1, 2 or 3; $R^B$ is each independently selected from oxo, hydroxyl, halogen or $C_1$-$C_6$ alkyl, or two $R^B$, together with the atom to which they are attached, form 3-6 membered cycloalkyl;

$R^1$ is hydroxyl, $C_1$-$C_6$ alkyl unsubstituted or substituted by one or more $R^{1-1}$, $C_3$-$C_6$ cycloalkyl unsubstituted or substituted by one or more $R^{1-2}$, 3-6 membered heterocycloalkyl unsubstituted or substituted by one or more $R^{1-3}$,

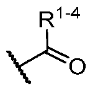

or amino unsubstituted or substituted by one or two $R^{1-5}$; the heteroatom in the 3-6 membered heterocycloalkyl is one or more selected from N, O and S, and the number of heteroatom(s) is 1, 2 or 3;

$R^{1-1}$ is each independently selected from deuterium, hydroxyl, cyano, halogen, 3-6 membered heterocycloalkyl, $C_3$-$C_6$ cycloalkyl unsubstituted or substituted by one or more $R^{1-1-1}$, $C_1$-$C_6$ alkyl unsubstituted or substituted by one or more $R^{1-1-2}$ or $C_1$-$C_6$ alkoxy unsubstituted or substituted by one or more $R^{1-1-3}$; the heteroatom in the 3-6 membered heterocycloalkyl is one or more selected from N, O and S, and the number of heteroatom(s) is 1, 2 or 3; $R^{1-1-1}$ is each independently selected from deuterium, hydroxyl, cyano or halogen; $R^{1-1-2}$ is each independently selected from deuterium, hydroxyl, cyano or halogen; $R^{1-1-3}$ is each independently selected from deuterium, hydroxyl, cyano or halogen;

$R^{1-2}$ is each independently selected from deuterium, hydroxyl, cyano, halogen, $C_1$-$C_6$ alkyl unsubstituted or substituted by one or more $R^{1-2-1}$, $C_2$-$C_6$ alkynyl unsubstituted or substituted by one or more $R^{1-2-2}$, $C_1$-$C_6$ alkoxy unsubstituted or substituted by one or more $R^{1-2-3}$, $C_2$-$C_6$ alkenyl unsubstituted or substituted by one or more $R^{1-2-4}$, amino unsubstituted or substituted by one or two $R^{1-2-5}$ or

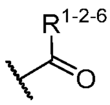;

$R^{1-2-1}$ is each independently selected from deuterium, halogen, hydroxyl or $C_1$-$C_6$ alkoxy unsubstituted or substituted by one or more $R^{1-2-1-1}$; $R^{1-2-1-1}$ is each independently halogen; $R^{1-2-2}$ is each independently selected from halogen, hydroxyl or $C_1$-$C_6$ alkoxy unsubstituted or substituted by one or more $R^{1-2-2-1}$; $R^{1-2-2-1}$ is each independently halogen; $R^{1-2-3}$ is each independently selected from halogen, hydroxyl or $C_1$-$C_6$ alkoxy unsubstituted or substituted by one or

more $R^{1-2-3-1}$; $R^{1-2-3-1}$ is each independently halogen; $R^{1-2-4}$ is each independently selected from halogen, hydroxyl or $C_1$-$C_6$ alkoxy unsubstituted or substituted by one or more $R^{1-2-4-1}$; $R^{1-2-4-1}$ is each independently halogen; $R^{1-2-6}$ is each independently amino; $R^{1-2-5}$ is each independently

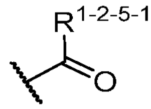
;

$R^{1-2-5-1}$ is each independently $C_1$-$C_6$ alkyl; $R^{1-3}$ is each independently selected from cyano, deuterium, hydroxyl, amino, halogen, $C_1$-$C_6$ alkyl unsubstituted or substituted by one or more $R^{1-3-1}$, $C_1$-$C_6$ alkoxy unsubstituted or substituted by one or more $R^{1-3-2}$ or $C_2$-$C_6$ alkynyl; $R^{1-3-1}$ is each independently halogen; $R^{1-3-2}$ is each independently halogen;

$R^{1-4}$ is $C_1$-$C_6$ alkyl;

$R^{1-5}$ is each independently selected from $C_1$-$C_6$ alkyl,

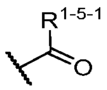

or $C_3$-$C_6$ cycloalkyl;

$R^{1-5-1}$ is $C_1$-$C_6$ alkyl or amino;

or, the atom in $R^1$ is attached to the atom in A to form 5-10 membered heterocycloalkyl unsubstituted or substituted by one or more $R^0$; the heteroatom in the 5-10 membered heterocycloalkyl is one or more selected from N, O, P and S, and the number of heteroatom(s) is 3, 4 or 5; $R^0$ is each independently selected from $C_1$-$C_6$ alkyl or oxo;

$R^2$ is hydrogen, 6-10 membered aryl unsubstituted or substituted by one or more $R^{2-1}$, 5-10 membered heteroaryl unsubstituted or substituted by one or more $R^{2-2}$, 4-12 membered heterocycloalkyl unsubstituted or substituted by one or more $R^{2-}$

3, 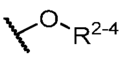, 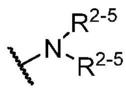,

$C_1$-$C_6$ alkyl unsubstituted or substituted by one or more $R^{2-6}$,

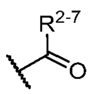, 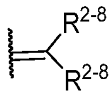,

$C_3$-$C_6$ cycloalkyl unsubstituted or substituted by one or more $R^{2-9}$, $C_3$-$C_6$ cycloalkenyl unsubstituted or substituted by one or more $R^{2-10}$, or

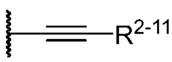

; the heteroatom in the 5-10 membered heteroaryl is one or more selected from N, O and S, and the number of heteroatom(s) is 1, 2 or 3; the heteroatom in the 4-12 membered heterocycloalkyl is one or more selected from N, O and S, and the number of heteroatom(s) is 1, 2 or 3;

$R^{2-1}$ is each independently selected from cyano, halogen, $C_1$-$C_6$ alkyl unsubstituted or substituted by one or more $R^{2-1-}$

1, 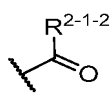, 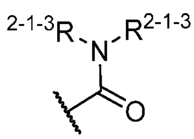

or $C_1$-$C_6$ alkoxy unsubstituted or substituted by one or more $R^{2-1-4}$;

$R^{2-1-1}$ is each independently selected from cyano or halogen; $R^{2-1-2}$ is hydrogen or $C_1$-$C_6$ alkyl; $R^{2-1-3}$ is each independently selected from hydrogen or $C_1$-$C_6$ alkyl; $R^{2-1-4}$ is each independently halogen;
$R^{2-2}$ is each independently selected from cyano, halogen, $C_1$-$C_6$ alkyl unsubstituted or substituted by one or more $R^{2-2-}$

1, 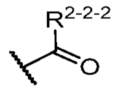 , 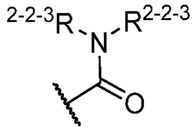 ,

$C_1$-$C_6$ alkoxy or

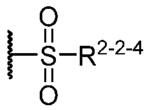 ;

$R^{2-2-1}$ is each independently selected from cyano or halogen; $R^{2-2-2}$ is hydrogen or $C_1$-$C_6$ alkyl; $R^{2-2-3}$ is each independently selected from hydrogen or $C_1$-$C_6$ alkyl; $R^{2-2-4}$ is each independently selected from $C_1$-$C_6$ alkyl or $C_3$-$C_6$ cycloalkyl;
$R^{2-3}$ is each independently selected from cyano, oxo, hydroxyl, halogen, $C_1$-$C_6$ alkyl unsubstituted or substituted by one or more $R^{2-3-1}$,

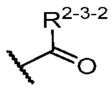 , 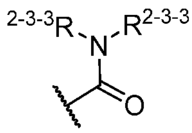 .

3-6 membered heterocycloalkyl unsubstituted or substituted by one or more $R^{2-3-4}$, $C_1$-$C_6$ alkoxy unsubstituted or substituted by one or more $R^{2-3-5}$, $C_3$-$C_6$ cycloalkyl unsubstituted or substituted by one or more $R^{2-3-6}$, amino unsubstituted or substituted by one or more $R^{2-3-7}$,

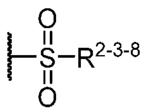 ,

or 5-10 membered heteroaryl unsubstituted or substituted by one or more $R^{2-3-9}$; the heteroatom in the 3-6 membered heterocycloalkyl is one or more selected from N, O and S, and the number of heteroatom(s) is 1, 2 or 3; the heteroatom in the 5-10 membered heteroaryl is one or more selected from N, O and S, and the number of heteroatom(s) is 1, 2 or 3;
$R^{2-3-1}$ is each independently selected from hydroxyl, cyano, halogen, $C_1$-$C_6$ alkoxy unsubstituted or substituted by one or more $R^{2-3-1-1}$, $C_3$-$C_6$ cycloalkyl unsubstituted or substituted by one or more $R^{2-3-1-2}$, or amino unsubstituted or substituted by one or more $R^{2-3-1-3}$;
$R^{2-3-2}$ is hydrogen, $C_1$-$C_6$ alkyl unsubstituted or substituted by one or more $R^{2-3-2-1}$, $C_3$-$C_6$ cycloalkyl unsubstituted or substituted by one or more $R^{2-3-2-2}$,

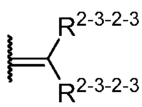 ,

$C_1$-$C_6$ alkoxy unsubstituted or substituted by one or more $R^{2-3-2-4}$, 3-6 membered heterocycloalkyl unsubstituted or substituted by one or more $R^{2-3-2-5}$, or 5-10 membered heteroaryl unsubstituted or substituted by one or more $R^{2-3-2-6}$; the heteroatom in the 3-6 membered heterocycloalkyl is one or more selected from N, O and S, and the number of heteroatom(s) is 1, 2 or 3; the heteroatom in the 5-10 membered heteroaryl is one or more selected from N, O and S, and the number of heteroatom(s) is 1, 2 or 3;
$R^{2-3-3}$ is each independently selected from hydrogen, or $C_1$-$C_6$ alkyl unsubstituted or substituted by one or more $R^{2-3-3-1}$; ,

$R^{2-3-4}$ is each independently selected from $C_1$-$C_6$ alkyl or oxo;
$R^{2-3-5}$ is each independently selected from halogen or deuterium;
$R^{2-3-6}$ is each independently $C_1$-$C_6$ alkyl;
$R^{2-3-7}$ is each independently $C_1$-$C_6$ alkyl;
$R^{2-3-8}$ is each independently selected from $C_1$-$C_6$ alkyl or $C_3$-$C_6$ cycloalkyl;
$R^{2-3-9}$ is each independently $C_1$-$C_6$ alkyl;
$R^{2-3-1-1}$ is each independently halogen;
$R^{2-3-1-2}$ is each independently $C_1$-$C_6$ alkoxy;
$R^{2-3-1-3}$ is each independently $C_1$-$C_6$ alkyl;
$R^{2-3-2-1}$ is each independently selected from halogen, carboxyl, hydroxyl, oxo, $C_1$-$C_6$ alkoxy,

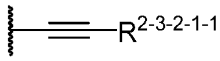
,

amino unsubstituted or substituted by one or more $R^{2-3-2-1-2}$, or 5-10 membered heteroaryl unsubstituted or substituted by one or more $R^{2-3-2-1-3}$; the heteroatom in the 5-10 membered heteroaryl is one or more selected from N, O and S, and the number of heteroatom(s) is 1, 2 or 3;
$R^{2-3-2-1-1}$ is each independently selected from $C_1$-$C_6$ alkyl or 6-10 membered aryl;
$R^{2-3-2-1-2}$ is each independently $C_1$-$C_6$ alkyl;
$R^{2-3-2-1-3}$ is each independently $C_1$-$C_6$ alkyl;
$R^{2-3-2-2}$ is each independently selected from halogen, hydroxyl, $C_1$-$C_6$ alkyl unsubstituted or substituted by one or more $R^{2-3-2-2-1}$, $C_1$-$C_6$ alkoxy unsubstituted or substituted by one or more $R^{2-3-2-2-2}$, or amino unsubstituted or substituted by one or more $R^{2-3-2-2-3}$;
$R^{2-3-2-2-1}$ is each independently selected from halogen or hydroxyl;
$R^{2-3-2-2-2}$ is each independently selected from carboxyl, halogen, deuterium, oxo or hydroxyl;
$R^{2-3-2-2-3}$ is each independently $C_1$-$C_6$ alkyl;
$R^{2-3-2-3}$ is each independently selected from hydrogen or

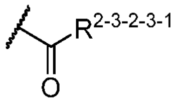
;

$R^{2-3-2-3-1}$ is each independently 6-10 membered aryl unsubstituted or substituted by one or more $R^{2-3-2-3-1-1}$;
$R^{2-3-2-3-1-1}$ is each independently $C_1$-$C_6$ alkoxy;
$R^{2-3-2-4}$ is each independently halogen;
$R^{2-3-2-5}$ is each independently selected from halogen, hydroxyl, oxo, $C_1$-$C_6$ alkoxy, or $C_1$-$C_6$ alkyl unsubstituted or substituted by one or more $R^{2-3-2-5-1}$; $R^{2-3-2-5-1}$ is each independently halogen;
$R^{2-3-2-6}$ is each independently $C_1$-$C_6$ alkyl;
$R^{2-3-3-1}$ is each independently amino unsubstituted or substituted by one or more $R^{2-3-3-1-1}$; $R^{2-3-3-1-1}$ is each independently $C_1$-$C_6$ alkyl;
each $R^{2-4}$ is 4-10 membered heterocycloalkyl unsubstituted or substituted by one or more $R^{2-4-1}$, 5-10 membered heteroaryl unsubstituted or substituted by one or more $R^{2-4-2}$, 6-10 membered aryl unsubstituted or substituted by one or more $R^{2-4-3}$ or $C_3$-$C_6$ cycloalkyl unsubstituted or substituted by one or more $R^{2-4-4}$; the heteroatom in the 4-10 membered heterocycloalkyl is one or more selected from N, O and S, and the number of heteroatom(s) is 1, 2 or 3; the heteroatom in the 5-10 membered heteroaryl is one or more selected from N, O and S, and the number of heteroatom(s) is 1, 2 or 3;
$R^{2-4-1}$ is each independently selected from hydroxyl, halogen, $C_1$-$C_6$ alkyl,

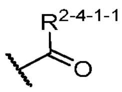
or
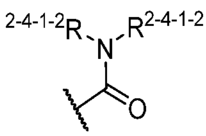
;

$R^{2-4-2}$ is each independently selected from hydroxyl, halogen or $C_1$-$C_6$ alkyl; $R^{2-4-3}$ is each independently selected from hydroxyl, halogen or $C_1$-$C_6$ alkyl; $R^{2-4-4}$ is each independently selected from hydroxyl, halogen or $C_1$-$C_6$ alkyl; $R^{2-4-1-1}$ is $C_1$-$C_6$ alkyl; $R^{2-4-1-2}$ is $C_1$-$C_6$ alkyl;

$R^{2-5}$ is each independently selected from hydrogen, 4-10 membered heterocycloalkyl unsubstituted or substituted by one or more $R^{2-5-1}$,

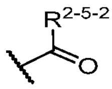

,

6-10 membered aryl unsubstituted or substituted by one or more $R^{2-5-3}$, or $C_1$-$C_6$ alkyl; the heteroatom in the 4-10 membered heterocycloalkyl is one or more selected from N, O and S, and the number of heteroatom(s) is 1, 2 or 3,
$R^{2-5-1}$ is each independently selected from

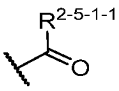 or 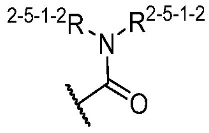 ;

$R^{2-5-1-1}$ is hydrogen or $C_1$-$C_6$ alkyl;
$R^{2-5-1-2}$ is each independently selected from hydrogen or $C_1$-$C_6$ alkyl;
$R^{2-5-2}$ is independently $C_1$-$C_6$ alkyl;
$R^{2-5-3}$ is each independently $C_1$-$C_6$ alkoxy;
$R^{2-6}$ is each independently selected from halogen, $C_1$-$C_6$ alkyl, 4-10 membered heterocycloalkyl unsubstituted or substituted by one or more $R^{2-6-1}$ or 5-10 membered heteroaryl unsubstituted or substituted by one or more $R^{2-6-2}$; the heteroatom in the 4-10 membered heterocycloalkyl is one or more selected from N, O and S, and the number of heteroatom(s) is 1, 2 or 3; the heteroatom in the 5-10 membered heteroaryl is one or more selected from N, O and S, and the number of heteroatom(s) is 1, 2 or 3;
$R^{2-6-1}$ is each independently selected from halogen, $C_1$-$C_6$ alkyl or

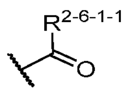

;

$R^{2-6-1-1}$ is $C_1$-$C_6$ alkyl;
$R^{2-6-2}$ is each independently selected from halogen or $C_1$-$C_6$ alkyl;
$R^{2-7}$ is $C_1$-$C_6$ alkyl, 4-10 membered heterocycloalkyl unsubstituted or substituted by one or more $R^{2-7-1}$ or 5-10 membered heteroaryl unsubstituted or substituted by one or more $R^{2-7-2}$; the heteroatom in the 4-10 membered heterocycloalkyl is one or more selected from N, O and S, and the number of heteroatom(s) is 1, 2 or 3; the heteroatom in the 5-10 membered heteroaryl is one or more selected from N, O and S, and the number of heteroatom(s) is 1, 2 or 3;
$R^{2-7-1}$ is each independently selected from halogen, $C_1$-$C_6$ alkyl,

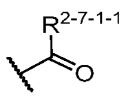 or 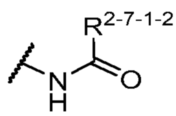 ;

$R^{2-7-1-1}$ is $C_1$-$C_6$ alkyl; $R^{1-2-5-1}$ is $C_1$-$C_6$ alkyl;
$R^{2-7-2}$ is each independently selected from halogen or $C_1$-$C_6$ alkyl;
$R^{2-8}$ is each independently $C_1$-$C_6$ alkyl, or two $R^{2-8}$, together with the atom to which they are attached, form 4-10 membered heterocycloalkyl unsubstituted or substituted by one or more $R^{2-8-1}$; the heteroatom in the 4-10 membered heterocycloalkyl is one or more selected from N, O and S, and the number of heteroatom(s) is 1, 2 or 3;
$R^{2-8-1}$ is each independently

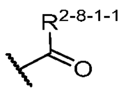

;

$R^{2-8-1-1}$ is hydrogen or $C_1$-$C_6$ alkyl;
$R^{2-9}$ is each independently 5-10 membered heteroaryl unsubstituted or substituted by one or more $R^{2-9-1}$; the

heteroatom in the 5-10 membered heteroaryl is one or more selected from N, O and S, and the number of heteroatom(s) is 1, 2 or 3; and

$R^{2-9-1}$ is each independently $C_1$-$C_6$ alkyl;

$R^{2-10}$ is each independently 5-10 membered heteroaryl unsubstituted or substituted by one or more $R^{2-10-1}$; the heteroatom in the 5-10 membered heteroaryl is one or more selected from N, O and S, and the number of heteroatom(s) is 1, 2 or 3; and

$R^{2-10-1}$ is each independently $C_1$-$C_6$ alkyl;

$R^{2-11}$ is each independently selected from 5-10 membered heteroaryl unsubstituted or substituted by one or more $R^{2-11-1}$, or 3-6 membered heterocycloalkyl unsubstituted or substituted by one or more $R^{2-11-2}$; the heteroatom in the 5-10 membered heteroaryl is one or more selected from N, O and S, and the number of heteroatom(s) is 1, 2 or 3; the heteroatom in the 3-6 membered heterocycloalkyl is one or more selected from N, O and S, and the number of heteroatom(s) is 1, 2 or 3;

$R^{2-11-1}$ is each independently $C_1$-$C_6$ alkyl; $R^{2-11-2}$ is each independently $C_1$-$C_6$ alkyl;

$R^3$ is 5-10 membered heteroaryl unsubstituted or substituted by one or more $R^{3-1}$ or 3-10 membered heterocycloalkyl unsubstituted or substituted by one or more $R^{3-2}$; the heteroatom in the 5-10 membered heteroaryl is one or more selected from N, O and S, and the number of heteroatom(s) is 1, 2 or 3; the heteroatom in the 3-10 membered heterocycloalkyl is one or more selected from N, O and S, and the number of heteroatom(s) is 1, 2 or 3;

$R^{3-1}$ is each independently selected from hydroxyl, halogen, $C_1$-$C_6$ alkyl unsubstituted or substituted by one or more $R^{3-1-1}$ or

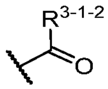
;

$R^{3-1-1}$ is each independently selected from halogen or hydroxyl; $R^{3-1-2}$ is $C_1$-$C_6$ alkyl or $C_2$-$C_6$ alkenyl;

$R^{3-2}$ is each independently selected from hydroxyl, halogen, $C_1$-$C_6$ alkyl unsubstituted or substituted by one or more $R^{3-2-1}$ or

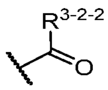
;

$R^{3-2-1}$ is each independently halogen; $R^{3-2-2}$ is $C_1$-$C_6$ alkyl or $C_2$-$C_4$ alkenyl;

$R^4$ is hydrogen, deuterium, halogen, cyano, 5-10 membered heterocycloalkyl unsubstituted or substituted by one or more $R^{4-1}$ or $C_1$-$C_6$ alkyl unsubstituted or substituted by one or more $R^{4-2}$; the heteroatom in the 5-10 membered heterocycloalkyl is one or more selected from N, O and S, and the number of heteroatom(s) is 1, 2 or 3;

$R^{4-1}$ is each independently

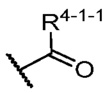
or
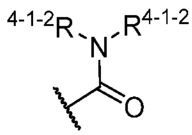
;

$R^{4-2}$ is each independently 5-10 membered heteroaryl unsubstituted or substituted by one or more $R^{4-2-1}$; the heteroatom in the 5-10 membered heteroaryl is one or more selected from N, O and S, and the number of heteroatom(s) is 1, 2 or 3; and

$R^{4-1-1}$ is $C_1$-$C_6$ alkyl; $R^{4-1-2}$ is each independently $C_1$-$C_6$ alkyl; $R^{4-2-1}$ is each independently $C_1$-$C_6$ alkyl; and when ring B is

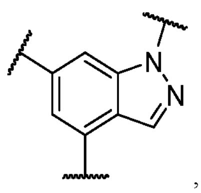
,

$R^2$ is

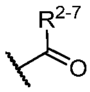 or 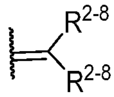

The present invention provides a compound of formula I, a pharmaceutically acceptable salt thereof or an isotopic compound thereof;

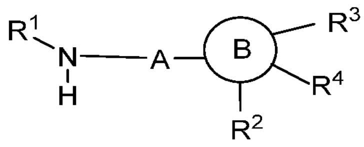

**I**

A is

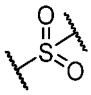, 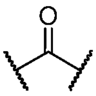 or 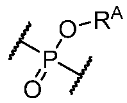 ;

$R^A$ is hydrogen or $C_1$-$C_6$ alkyl;

ring B is a 5-membered-fused 6-membered heteroaromatic ring unsubstituted or substituted by one or two $R^B$ or a 5-membered-fused 5-membered heteroaromatic ring unsubstituted or substituted by one or two $R^B$; the heteroatom in the 5-membered-fused 6-membered heteroaromatic ring is one or more selected from N, O and S, and the number of heteroatom(s) is 1, 2 or 3; the heteroatom in the 5-membered-fused 5-membered heteroaromatic ring is one or more selected from N, O and S, and the number of heteroatom(s) is 1, 2 or 3; $R^B$ is each independently selected from oxo, hydroxyl, halogen or $C_1$-$C_6$ alkyl, or two $R^B$, together with the atom to which they are attached, form 3-6 membered cycloalkyl;

$R^1$ is hydroxyl, $C_1$-$C_6$ alkyl unsubstituted or substituted by one or more $R^{1-1}$, $C_3$-$C_6$ cycloalkyl unsubstituted or substituted by one or more $R^{1-2}$, 3-6 membered heterocycloalkyl unsubstituted or substituted by one or more $R^{1-3}$,

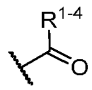

or amino unsubstituted or substituted by one or two $R^{1-5}$; the heteroatom in the 3-6 membered heterocycloalkyl is one or more selected from N, O and S, and the number of heteroatom(s) is 1, 2 or 3;

$R^{1-1}$ is each independently selected from deuterium, hydroxyl, cyano, halogen, 3-6 membered heterocycloalkyl, $C_3$-$C_6$ cycloalkyl unsubstituted or substituted by one or more $R^{1-1-1}$, $C_1$-$C_6$ alkyl unsubstituted or substituted by one or more $R^{1-1-2}$ or $C_1$-$C_6$ alkoxy unsubstituted or substituted by one or more $R^{1-1-3}$; the heteroatom in the 3-6 membered heterocycloalkyl is one or more selected from N, O and S, and the number of heteroatom(s) is 1, 2 or 3; $R^{1-1-1}$ is each independently selected from deuterium, hydroxyl, cyano or halogen; $R^{1-1-2}$ is each independently selected from deuterium, hydroxyl, cyano or halogen; $R^{1-1-3}$ is each independently selected from deuterium, hydroxyl, cyano or halogen;

$R^{1-2}$ is each independently selected from deuterium, hydroxyl, cyano, halogen, $C_1$-$C_6$ alkyl unsubstituted or substituted by one or more $R^{1-2-1}$, $C_2$-$C_6$ alkynyl unsubstituted or substituted by one or more $R^{1-2-2}$, $C_1$-$C_6$ alkoxy unsubstituted or substituted by one or more $R^{1-2-3}$, $C_2$-$C_6$ alkenyl unsubstituted or substituted by one or more $R^{1-2-4}$ or amino unsubstituted or substituted by one or two $R^{1-2-5}$; $R^{1-2-1}$ is each independently selected from halogen, hydroxyl or $C_1$-$C_6$ alkoxy unsubstituted or substituted by one or more $R^{1-2-1-1}$; $R^{1-2-1-1}$ is each independently selected from halogen; $R^{1-2-2}$ is each independently selected from halogen, hydroxyl or $C_1$-$C_6$ alkoxy unsubstituted or substituted by one or more $R^{1-2-2-1}$; $R^{1-2-2-1}$ is each independently selected from halogen; $R^{1-2-3}$ is each independently selected from halogen, hydroxyl or $C_1$-$C_6$ alkoxy unsubstituted or substituted by one or more $R^{1-2-3-1}$; $R^{1-2-3-1}$ is each independently selected from halogen; $R^{1-2-4}$ is each independently selected from halogen, hydroxyl or $C_1$-$C_6$ alkoxy unsubstituted or substituted by one or more $R^{1-2-4-1}$; $R^{1-2-4-1}$ is each independently halogen;

$R^{1-3}$ is each independently selected from cyano, deuterium, hydroxyl, amino, halogen, $C_1$-$C_6$ alkyl unsubstituted or substituted by one or more $R^{1-3-1}$ or $C_1$-$C_6$ alkoxy unsubstituted or substituted by one or more $R^{1-3-2}$; $R^{1-3-1}$ is each

independently halogen; $R^{1-3-2}$ is each independently halogen;
$R^{1-4}$ is $C_1$-$C_6$ alkyl;
$R^{1-5}$ is each independently selected from $C_1$-$C_6$ alkyl,

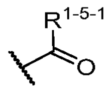

or $C_3$-$C_6$ cycloalkyl;
$R^{1-5-1}$ is $C_1$-$C_6$ alkyl or amino;
or, the atom in $R^1$ is attached to the atom in A to form 5-10 membered heterocycloalkyl unsubstituted or substituted by one or more $R^0$; the heteroatom in the 5-10 membered heterocycloalkyl is one or more selected from N, O, P and S, and the number of heteroatom(s) is 3, 4 or 5; $R^0$ is each independently selected from $C_1$-$C_6$ alkyl or oxo;
$R^2$ is hydrogen, 6-10 membered aryl unsubstituted or substituted by one or more $R^{2-1}$, 5-10 membered heteroaryl unsubstituted or substituted by one or more $R^{2-2}$, 4-10 membered heterocycloalkyl unsubstituted or substituted by one or more $R^{2-}$

3, 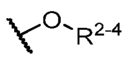 , 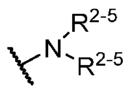 ,

$C_1$-$C_6$ alkyl unsubstituted or substituted by one or more $R^{2-6}$,

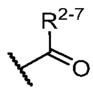 or 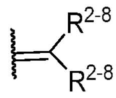 ;

the heteroatom in the 5-10 membered heteroaryl is one or more selected from N, O and S, and the number of heteroatom(s) is 1, 2 or 3; the heteroatom in the 4-10 membered heterocycloalkyl is one or more selected from N, O and S, and the number of heteroatom(s) is 1, 2 or 3,
$R^{2-1}$ is each independently selected from cyano, halogen, $C_1$-$C_6$ alkyl unsubstituted or substituted by one or more $R^{2-1-}$

1, 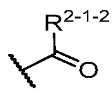 , 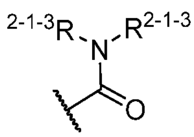

or $C_1$-$C_6$ alkoxy unsubstituted or substituted by one or more $R^{2-1-4}$;
$R^{2-1-1}$ is each independently selected from cyano or halogen; $R^{2-1-2}$ is hydrogen or $C_1$-$C_6$ alkyl; $R^{2-1-3}$ is each independently selected from hydrogen or $C_1$-$C_6$ alkyl; $R^{2-1-4}$ is each independently halogen;
$R^{2-2}$ is each independently selected from cyano, halogen, $C_1$-$C_6$ alkyl unsubstituted or substituted by one or more $R^{2-2-}$

1, 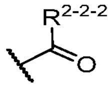 or 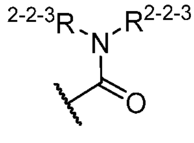 ;

$R^{2-2-1}$ is each independently selected from cyano or halogen; $R^{2-2-2}$ is hydrogen or $C_1$-$C_6$ alkyl; $R^{2-2-3}$ is each independently selected from hydrogen or $C_1$-$C_6$ alkyl;
$R^{2-3}$ is each independently selected from cyano, oxo, hydroxyl, halogen, $C_1$-$C_6$ alkyl unsubstituted or substituted by one or more $R^{2-3-1}$,

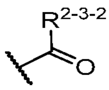, 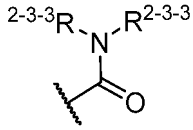,

3-6 membered heterocycloalkyl unsubstituted or substituted by one or more $R^{2-3-4}$, $C_1$-$C_6$ alkoxy unsubstituted or substituted by one or more $R^{2-3-5}$; the heteroatom in the 3-6 membered heterocycloalkyl is one or more selected from N, O and S, and the number of heteroatom(s) is 1, 2 or 3;

$R^{2-3-1}$ is each independently selected from cyano, halogen, or $C_1$-$C_6$ alkoxy unsubstituted or substituted by one or more $R^{2-3-1-1}$;

$R^{2-3-2}$ is hydrogen or $C_1$-$C_6$ alkyl unsubstituted or substituted by one or more $R^{2-3-2-1}$;

$R^{2-3-3}$ is each independently hydrogen or $C_1$-$C_6$ alkyl;

$R^{2-3-4}$ is each independently $C_1$-$C_6$ alkyl;

$R^{2-3-5}$ is each independently halogen;

$R^{2-3-1-1}$ is each independently halogen;

$R^{2-3-2-1}$ is each independently halogen;

each $R^{2-4}$ is 4-10 membered heterocycloalkyl unsubstituted or substituted by one or more $R^{2-4-1}$, 5-10 membered heteroaryl unsubstituted or substituted by one or more $R^{2-4-2}$, 6-10 membered aryl unsubstituted or substituted by one or more $R^{2-4-3}$ or $C_3$-$C_6$ cycloalkyl unsubstituted or substituted by one or more $R^{2-4-4}$; the heteroatom in the 4-10 membered heterocycloalkyl is one or more selected from N, O and S, and the number of heteroatom(s) is 1, 2 or 3; the heteroatom in the 5-10 membered heteroaryl is one or more selected from N, O and S, and the number of heteroatom(s) is 1, 2 or 3;

$R^{2-4-1}$ is each independently selected from hydroxyl, halogen, $C_1$-$C_6$ alkyl,

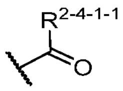 or 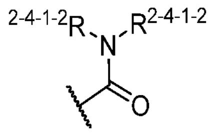;

$R^{2-4-2}$ is each independently selected from hydroxyl, halogen or $C_1$-$C_6$ alkyl; $R^{2-4-3}$ is each independently selected from hydroxyl, halogen or $C_1$-$C_6$ alkyl; $R^{2-4-4}$ is each independently selected from hydroxyl, halogen or $C_1$-$C_6$ alkyl; $R^{2-4-1-1}$ is $C_1$-$C_6$ alkyl; $R^{2-4-1-2}$ is $C_1$-$C_6$ alkyl;

$R^{2-5}$ is each independently hydrogen, 4-10 membered heterocycloalkyl unsubstituted or substituted by one or more $R^{2\ 5-1}$, or

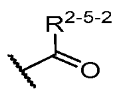;

the heteroatom in the 4-10 membered heteroaryl is one or more selected from N, O and S, and the number of heteroatom(s) is 1, 2 or 3;

$R^{2-5-1}$ is each independently selected from

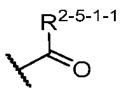 or 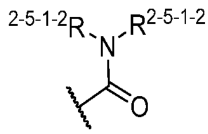;

$R^{1-2-5-1}$ is hydrogen or $C_1$-$C_6$ alkyl;

$R^{2-5-1-2}$ is each independently selected from hydrogen or $C_1$-$C_6$ alkyl;

$R^{2-5-2}$ is independently $C_1$-$C_6$ alkyl;

$R^{2-6}$ is each independently selected from halogen, $C_1$-$C_6$ alkyl, 4-10 membered heterocycloalkyl unsubstituted or substituted by one or more $R^{2-6-1}$ or 5-10 membered heteroaryl unsubstituted or substituted by one or more $R^{2-6-2}$; the heteroatom in the 4-10 membered heterocycloalkyl is one or more selected from N, O and S, and the number of heteroatom(s) is 1, 2 or 3; the heteroatom in the 5-10 membered heteroaryl is one or more selected from N, O and S, and the number of heteroatom(s) is 1, 2 or 3;

$R^{2-6-1}$ is each independently selected from halogen, $C_1$-$C_6$ alkyl or

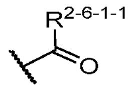;

$R^{2-6-1-1}$ is $C_1$-$C_6$ alkyl;
$R^{2-6-2}$ is each independently selected from halogen or $C_1$-$C_6$ alkyl;
$R^{2-7}$ is $C_1$-$C_6$ alkyl, 4-10 membered heterocycloalkyl unsubstituted or substituted by one or more $R^{2-7-1}$ or 5-10 membered heteroaryl unsubstituted or substituted by one or more $R^{2-7-2}$; the heteroatom in the 4-10 membered heterocycloalkyl is one or more selected from N, O and S, and the number of heteroatom(s) is 1, 2 or 3; the heteroatom in the 5-10 membered heteroaryl is one or more selected from N, O and S, and the number of heteroatom(s) is 1, 2 or 3;
$R^{2-7-1}$ is each independently selected from halogen, $C_1$-$C_6$ alkyl,

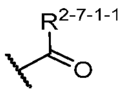 or 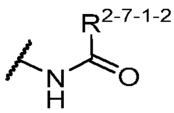;

$R^{2-7-1-1}$ is $C_1$-$C_6$ alkyl; $R^{2-7-1-2}$ is $C_1$-$C_6$ alkyl;
$R^{2-7-2}$ is each independently selected from halogen or $C_1$-$C_6$ alkyl;
$R^{2-8}$ is each independently $C_1$-$C_6$ alkyl, or two $R^{2-8}$, together with the atom to which they are attached, form 4-10 membered heterocycloalkyl unsubstituted or substituted by one or more $R^{2-8-1}$; the heteroatom in the 4-10 membered heterocycloalkyl is one or more selected from N, O and S, and the number of heteroatom(s) is 1, 2 or 3;
$R^{2-8-1}$ is each independently

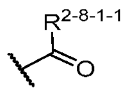;

$R^{2-8-1-1}$ is hydrogen or $C_1$-$C_6$ alkyl,
$R^3$ is 5-10 membered heteroaryl unsubstituted or substituted by one or more $R^{3-1}$ or 3-10 membered heterocycloalkyl unsubstituted or substituted by one or more $R^{3-2}$, the heteroatom in the 5-10 membered heteroaryl is one or more selected from N, O and S, and the number of heteroatom(s) is 1, 2 or 3; the heteroatom in the 3-10 membered heterocycloalkyl is one or more selected from N, O and S, and the number of heteroatom(s) is 1, 2 or 3;
$R^{3-1}$ is each independently selected from hydroxyl, halogen, $C_1$-$C_6$ alkyl unsubstituted or substituted by one or more $R^{3-1-1}$ or

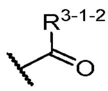;

$R^{3-1-1}$ is each independently halogen; $R^{3-1-2}$ is $C_1$-$C_6$ alkyl or $C_2$-$C_6$ alkenyl;
$R^{3-2}$ is each independently selected from hydroxyl, halogen, $C_1$-$C_6$ alkyl unsubstituted or substituted by one or more $R^{3-2-1}$ or

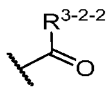;

$R^{3-2-1}$ is each independently halogen; $R^{3-2-2}$ is $C_1$-$C_6$ alkyl or $C_2$-$C_4$ alkenyl;
$R^4$ is hydrogen, halogen, 5-10 membered heterocycloalkyl unsubstituted or substituted by one or more $R^{4-1}$ or $C_1$-$C_6$ alkyl unsubstituted or substituted by one or more $R^{4-2}$; the heteroatom in the 5-10 membered heterocycloalkyl is one or more selected from N, O and S, and the number of heteroatom(s) is 1, 2 or 3;
$R^{4-1}$ is each independently

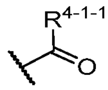 or 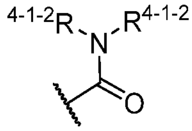 ;

$R^{4-2}$ is each independently 5-10 membered heteroaryl unsubstituted or substituted by one or more $R^{4-2-1}$, the heteroatom in the 5-10 membered heteroaryl is one or more selected from N, O and S, and the number of heteroatom(s) is 1, 2 or 3; and

$R^{4-1-1}$ is $C_1$-$C_6$ alkyl; $R^{4-1-2}$ is each independently $C_1$-$C_6$ alkyl; $R^{4-2-1}$ is each independently $C_1$-$C_6$ alkyl; and when ring B is

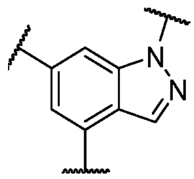 ,

$R^2$ is

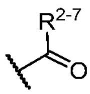 or 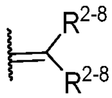

In a preferred embodiment, in the compound of formula I, the pharmaceutically acceptable salt thereof or the isotopic compound thereof, some groups can be defined as follows, and other groups can be defined as in any one of the above embodiments (hereinafter referred to as "in a preferred embodiment").

In a preferred embodiment, $R^1$ is hydroxyl, $C_1$-$C_6$ alkyl unsubstituted or substituted by one or more $R^{1-1}$, $C_3$-$C_6$ cycloalkyl unsubstituted or substituted by one or more $R^{1-2}$, 3-6 membered heterocycloalkyl unsubstituted or substituted by one or more $R^{1-3}$,

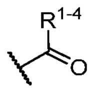

or amino unsubstituted or substituted by one or two $R^{1-5}$; the heteroatom in the 3-6 membered heterocycloalkyl is one or more selected from N, O and S, and the number of heteroatom(s) is 1, 2 or 3;

$R^{1-1}$ is each independently selected from deuterium, hydroxyl, cyano, halogen, 3-6 membered heterocycloalkyl, $C_3$-$C_6$ cycloalkyl unsubstituted or substituted by one or more $R^{1-1-1}$, $C_1$-$C_6$ alkyl unsubstituted or substituted by one or more $R^{1-1-2}$ or $C_1$-$C_6$ alkoxy unsubstituted or substituted by one or more $R^{1-1-3}$; the heteroatom in the 3-6 membered heterocycloalkyl is one or more selected from N, O and S, and the number of heteroatom(s) is 1, 2 or 3; $R^{1-1-1}$ is each independently selected from deuterium, hydroxyl, cyano or halogen; $R^{1-1-2}$ is each independently selected from deuterium, hydroxyl, cyano or halogen; $R^{1-1-3}$ is each independently selected from deuterium, hydroxyl, cyano or halogen;

$R^{1-2}$ is each independently selected from deuterium, hydroxyl, halogen, $C_2$-$C_6$ alkynyl unsubstituted or substituted by one or more $R^{1-2-2}$, $C_1$-$C_6$ alkoxy unsubstituted or substituted by one or more $R^{1-2-3}$, $C_2$-$C_6$ alkenyl unsubstituted or substituted by one or more $R^{1-2-4}$, amino unsubstituted or substituted by one or two $R^{1-2-5}$ or

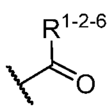 ;

$R^{1-2-2-1}$ is each independently halogen; $R^{1-2-3}$ is each independently selected from halogen, hydroxyl or $C_1$-$C_6$ alkoxy unsubstituted or substituted by one or more $R^{1-2-3-1}$; $R^{1-2-3-1}$ is each independently halogen; $R^{1-2-4}$ is each independently selected from halogen, hydroxyl or $C_1$-$C_6$ alkoxy unsubstituted or substituted by one or more

$R^{1-2-4-1}$; $R^{1-2-4-1}$ is each independently halogen; $R^{1-2-6}$ is each independently amino; $R^{1-2-5}$ is each independently

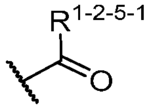

;

, $R^{1-2-5-1}$ is each independently $C_1$-$C_6$ alkyl; $R^{1-3}$ is each independently selected from cyano, deuterium, hydroxyl, amino, halogen, $C_1$-$C_6$ alkyl unsubstituted or substituted by one or more $R^{1-3-1}$, $C_1$-$C_6$ alkoxy unsubstituted or substituted by one or more $R^{1-3-2}$ or $C_2$-$C_6$ alkynyl; $R^{1-3-1}$ is each independently halogen; $R^{1-3-2}$ is each independently halogen;

$R^{1-4}$ is $C_1$-$C_6$ alkyl;

$R^{1-5}$ is each independently selected from $C_1$-$C_6$ alkyl,

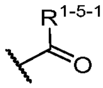

or $C_3$-$C_6$ cycloalkyl;

$R^{1-5-1}$ is $C_1$-$C_6$ alkyl or amino;

or, the atom in $R^1$ is attached to the atom in A to form 5-10 membered heterocycloalkyl unsubstituted or substituted by one or more $R^0$; the heteroatom in the 5-10 membered heterocycloalkyl is one or more selected from N, O, P and S, and the number of heteroatom(s) is 3, 4 or 5; $R^0$ is each independently selected from $C_1$-$C_6$ alkyl or oxo.

In a preferred embodiment, $R^2$ is hydrogen, 6-10 membered aryl unsubstituted or substituted by one or more $R^{2-1}$, 5-10 membered heteroaryl unsubstituted or substituted by one or more $R^{2-2}$, 4-12 membered heterocycloalkyl substituted by one or more $R^{2-3}$,

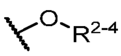
, 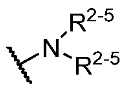
,

$C_1$-$C_6$ alkyl unsubstituted or substituted by one or more $R^{2-6}$,

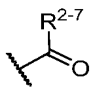
, 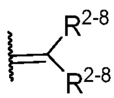
,

$C_3$-$C_6$ cycloalkyl unsubstituted or substituted by one or more $R^{2-9}$, $C_3$-$C_6$ cycloalkenyl unsubstituted or substituted by one or more $R^{2-10}$, or

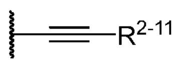

;

the heteroatom in the 5-10 membered heteroaryl is one or more selected from N, O and S, and the number of heteroatom(s) is 1, 2 or 3; the heteroatom in the 4-12 membered heterocycloalkyl is one or more selected from N, O and S, and the number of heteroatom(s) is 1, 2 or 3;

$R^{2-1}$ is each independently selected from cyano, halogen, $C_1$-$C_6$ alkyl unsubstituted or substituted by one or more $R^{2-1-}$

1, 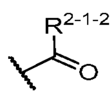
, 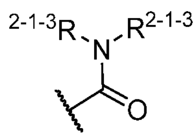

or $C_1$-$C_6$ alkoxy unsubstituted or substituted by one or more $R^{2-1-4}$;

$R^{2-1-1}$ is each independently selected from cyano or halogen; $R^{2-1-2}$ is hydrogen or $C_1$-$C_6$ alkyl; $R^{2-1-3}$ is each independently selected from hydrogen or $C_1$-$C_6$ alkyl; $R^{2-1-4}$ is each independently halogen;

$R^{2-2}$ is each independently selected from cyano, halogen, $C_1$-$C_6$ alkyl unsubstituted or substituted by one or more $R^{2-2-}$ 1,

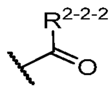

,

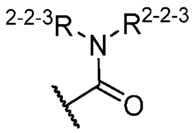

,

$C_1$-$C_6$ alkoxy or

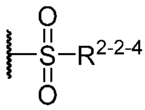

;

$R^{2-2-1}$ is each independently selected from cyano or halogen; $R^{2-2-2}$ is hydrogen or $C_1$-$C_6$ alkyl; $R^{2-2-3}$ is each independently selected from hydrogen or $C_1$-$C_6$ alkyl; $R^{2-2-4}$ is each independently selected from $C_1$-$C_6$ alkyl or $C_3$-$C_6$ cycloalkyl;

$R^{2-3}$ is each independently selected from $C_1$-$C_6$ alkyl substituted by one or more $R^{2-3-1}$,

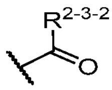

,

3-6 membered heterocycloalkyl unsubstituted or substituted by one or more $R^{2-3-4}$, amino unsubstituted or substituted by one or more $R^{2-3-7}$, 5-10 membered heteroaryl unsubstituted or substituted by one or more $R^{2-3-9}$; the heteroatom in the 3-6 membered heterocycloalkyl is one or more selected from N, O and S, and the number of heteroatom(s) is 1, 2 or 3; the heteroatom in the 5-10 membered heteroaryl is one or more selected from N, O and S, and the number of heteroatom(s) is 1, 2 or 3;

$R^{2-3-1}$ is each independently selected from cyano, or $C_3$-$C_6$ cyano unsubstituted or substituted by one or more $R^{2-3-1-2}$;

$R^{2-3-2}$ is hydrogen, or $C_1$-$C_6$ alkylsubstituted by one or more $R^{2-3-2-1}$, $C_3$-$C_6$ cycloalkyl substituted by one or more $R^{2-}$ 3-2-2,

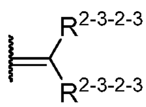

,

3-6 membered heterocycloalkyl unsubstituted or substituted by one or more $R^{2-3-2-5}$, 5-10 membered heteroaryl unsubstituted or substituted by one or more $R^{2-3-2-6}$; the heteroatom in the 3-6 membered heterocycloalkyl is one or more selected from N, O and S, and the number of heteroatom(s) is 1, 2 or 3; the heteroatom in the 5-10 membered heteroaryl is one or more selected from N, O and S, and the number of heteroatom(s) is 1, 2 or 3;

$R^{2-3-4}$ is each independently $C_1$-$C_6$ alkyl;

$R^{2-3-7}$ is each independently $C_1$-$C_6$ alkyl;

$R^{2-3}$ is each independently selected from oxo or $C_1$-$C_6$ alkyl;

$R^{2-3-1-2}$ is each independently $C_1$-$C_6$ alkoxy; $R^{2-3-2-1}$ is each independently selected from hydroxyl, oxo,

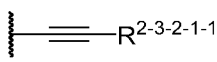

,

or 5-10 membered heteroaryl unsubstituted or substituted by one or more $R^{2-3-2-1-3}$; the heteroatom in the 5-10 membered heteroaryl is one or more selected from N, O and S, and the number of heteroatom(s) is 1, 2 or 3;

$R^{2-3-2-1-1}$ is each independently selected from $C_1$-$C_6$ alkyl or 6-10 membered aryl; $R^{2-3-2-1-2}$ is each independently $C_1$-$C_6$ alkyl, $R^{2-3-2-1-3}$ is each independently $C_1$-$C_6$ alkyl;

$R^{2-3-2-2}$ is each independently selected from halogen, hydroxyl, $C_1$-$C_6$ alkyl unsubstituted or substituted by one or more $R^{2-3-2-2-1}$, $C_1$-$C_6$ alkoxy unsubstituted or substituted by one or more $R^{2-3-2-2-2}$, or amino unsubstituted or

substituted by one or more $R^{2\text{-}3\text{-}2\text{-}2\text{-}3}$;
$R^{2\text{-}3\text{-}2\text{-}2\text{-}1}$ is each independently selected from halogen or hydroxyl;
$R^{2\text{-}3\text{-}2\text{-}2\text{-}2}$ is each independently selected from halogen, deuterium, oxo or hydroxyl;
$R^{2\text{-}3\text{-}2\text{-}2\text{-}3}$ is each independently $C_1$-$C_6$ alkyl;
$R^{2\text{-}3\text{-}2\text{-}3}$ is each independently selected from hydrogen or

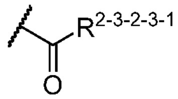
;

$R^{2\text{-}3\text{-}2\text{-}3\text{-}1}$ is each independently 6-10 membered aryl unsubstituted or substituted by one or more $R^{2\text{-}3\text{-}2\text{-}3\text{-}1\text{-}1}$; $R^{2\text{-}3\text{-}2\text{-}3\text{-}1\text{-}1}$ is each independently $C_1$-$C_6$ alkoxy;
$R^{2\text{-}3\text{-}2\text{-}4}$ is each independently halogen;
$R^{2\text{-}3\text{-}2\text{-}5}$ is each independently selected from halogen, hydroxyl, oxo, $C_1$-$C_6$ alkoxy, or $C_1$-$C_6$ alkyl substituted by one or more $R^{2\text{-}3\text{-}2\text{-}5\text{-}1}$; $R^{2\text{-}3\text{-}2\text{-}5\text{-}1}$ is each independently halogen;
$R^{2\text{-}3\text{-}2\text{-}6}$ is each independently $C_1$-$C_6$ alkyl; $R^{2\text{-}4}$ is 4-10 membered heterocycloalkyl substituted by one or more $R^{2\text{-}4\text{-}1}$, 5-10 membered heteroaryl unsubstituted or substituted by one or more $R^{2\text{-}4\text{-}2}$, or 6-10 membered aryl unsubstituted or substituted by one or more $R^{2\text{-}4\text{-}3}$; the heteroatom in the 4-10 membered heterocycloalkyl is one or more selected from N, O and S, and the number of heteroatom(s) is 1, 2 or 3; the heteroatom in the 5-10 membered heteroaryl is one or more selected from N, O and S, and the number of heteroatom(s) is 1, 2 or 3;
$R^{2\text{-}4\text{-}1}$ is each independently selected from hydroxyl, halogen, $C_1$-$C_6$ alkyl,

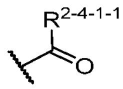
or
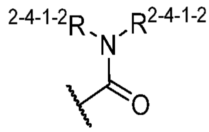
;

$R^{2\text{-}4\text{-}2}$ is each independently selected from hydroxyl, halogen or $C_1$-$C_6$ alkyl; $R^{2\text{-}4\text{-}3}$ is each independently selected from hydroxyl, halogen or $C_1$-$C_6$ alkyl;
$R^{2\text{-}5}$ is each independently selected from hydrogen, 4-10 membered heterocycloalkyl substituted by one or more $R^{2\text{-}5\text{-}1}$,

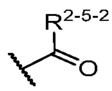
,

6-10 membered aryl unsubstituted or substituted by one or more $R^{2\text{-}5\text{-}3}$, or $C_1$-$C_6$ alkyl; the heteroatom in the 4-10 membered heterocycloalkyl is one or more selected from N, O and S, and the number of heteroatom(s) is 1, 2 or 3;
$R^{2\text{-}5\text{-}1}$ is each independently selected from

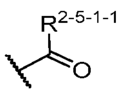
or
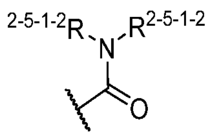
;

$R^{2\text{-}5\text{-}1\text{-}1}$ is hydrogen or $C_1$-$C_6$ alkyl;
$R^{2\text{-}5\text{-}1\text{-}2}$ is each independently selected from hydrogen or $C_1$-$C_6$ alkyl;
$R^{2\text{-}5\text{-}2}$ is independently $C_1$-$C_6$ alkyl;
$R^{2\text{-}5\text{-}3}$ is each independently $C_1$-$C_6$ alkoxy;
$R^{2\text{-}6}$ is each independently selected from halogen, $C_1$-$C_6$ alkyl, 4-10 membered heterocycloalkyl unsubstituted or substituted by one or more $R^{2\text{-}6\text{-}1}$ or 5-10 membered heteroaryl unsubstituted or substituted by one or more $R^{2\text{-}6\text{-}2}$; the heteroatom in the 4-10 membered heterocycloalkyl is one or more selected from N, O and S, and the number of heteroatom(s) is 1, 2 or 3; the heteroatom in the 5-10 membered heteroaryl is one or more selected from N, O and S, and the number of heteroatom(s) is 1, 2 or 3;
$R^{2\text{-}6\text{-}1}$ is each independently selected from halogen, $C_1$-$C_6$ alkyl or

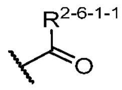
;

$R^{2-6-1-1}$ is $C_1$-$C_6$ alkyl;

$R^{2-6-2}$ is each independently selected from halogen or $C_1$-$C_6$ alkyl;

$R^{2-7}$ is $C_1$-$C_6$ alkyl, 4-10 membered heterocycloalkyl unsubstituted or substituted by one or more $R^{2-7-1}$ or 5-10 membered heteroaryl unsubstituted or substituted by one or more $R^{2-7-2}$; the heteroatom in the 4-10 membered heterocycloalkyl is one or more selected from N, O and S, and the number of heteroatom(s) is 1, 2 or 3; the heteroatom in the 5-10 membered heteroaryl is one or more selected from N, O and S, and the number of heteroatom(s) is 1, 2 or 3;

$R^{2-7-1}$ is each independently selected from halogen, $C_1$-$C_6$ alkyl,

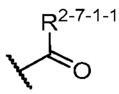
or
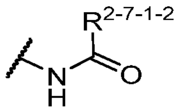
;

$R^{2-7-1-1}$ is $C_1$-$C_6$ alkyl; $R^{2-7-1-2}$ is $C_1$-$C_6$ alkyl;

$R^{2-7-2}$ is each independently selected from halogen or $C_1$-$C_6$ alkyl;

$R^{2-8}$ is each independently $C_1$-$C_6$ alkyl, or two $R^{2-8}$, together with the atom to which they are attached, form 4-10 membered heterocycloalkyl unsubstituted or substituted by one or more $R^{2-8-1}$; the heteroatom in the 4-10 membered heterocycloalkyl is one or more selected from N, O and S, and the number of heteroatom(s) is 1, 2 or 3;

$R^{2-8-1}$ is each independently

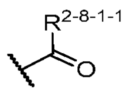
;

$R^{2-8-1-1}$ is hydrogen or $C_1$-$C_6$ alkyl;

$R^{2-9}$ is each independently 5-10 membered heteroaryl unsubstituted or substituted by one or more $R^{2-9-1}$; the heteroatom in the 5-10 membered heteroaryl is one or more selected from N, O and S, and the number of heteroatom(s) is 1, 2 or 3; and

$R^{2-9-1}$ is each independently $C_1$-$C_6$ alkyl;

$R^{2-10}$ is each independently 5-10 membered heteroaryl unsubstituted or substituted by one or more $R^{2-10-1}$; the heteroatom in the 5-10 membered heteroaryl is one or more selected from N, O and S, and the number of heteroatom(s) is 1, 2 or 3; and

$R^{2-10-1}$ is each independently $C_1$-$C_6$ alkyl;

$R^{2-11}$ is each independently selected from 5-10 membered heteroaryl unsubstituted or substituted by one or more $R^{2-11-1}$, or 3-6 membered heterocycloalkyl unsubstituted or substituted by one or more $R^{2-11-2}$; the heteroatom in the 5-10 membered heteroaryl is one or more selected from N, O and S, and the number of heteroatom(s) is 1, 2 or 3; the heteroatom in the 3-6 membered heterocycloalkyl is one or more selected from N, O and S, and the number of heteroatom(s) is 1, 2 or 3;

$R^{2-11-1}$ is each independently $C_1$-$C_6$ alkyl; $R^{2-11-2}$ is each independently $C_1$-$C_6$ alkyl.

In a preferred embodiment, $R^3$ is 5-10 membered heteroaryl unsubstituted or substituted by one or more $R^{3-1}$ or 3-10 membered heterocycloalkyl unsubstituted or substituted by one or more $R^{3-2}$; the heteroatom in the 5-10 membered heteroaryl is one or more selected from N, O and S, and the number of heteroatom(s) is 1, 2 or 3; the heteroatom in the 3-10 membered heterocycloalkyl is one or more selected from N, O and S, and the number of heteroatom(s) is 1, 2 or 3;

$R^{3-1}$ is each independently selected from hydroxyl, halogen, $C_1$-$C_6$ alkyl substituted by one or more $R^{3-1-1}$ or

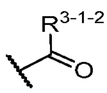
;

$R^{3-1-1}$ is each independently selected from or hydroxyl; $R^{3-1-2}$ is $C_1$-$C_6$ alkyl or $C_2$-$C_6$ alkenyl;

$R^{3-2}$ is each independently selected from hydroxyl, halogen, $C_1$-$C_6$ alkyl unsubstituted or substituted by one or more $R^{3-2-1}$ or

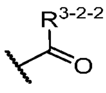
;

$R^{3-2-1}$ is each independently halogen; and $R^{3-2-2}$ is $C_1$-$C_6$ alkyl or $C_2$-$C_4$ alkenyl.

In a preferred embodiment, $R^4$ is deuterium, cyano, 5-10 membered heterocycloalkyl unsubstituted or substituted by one or more $R^{4-1}$ or $C_1$-$C_6$ alkyl substituted by one or more $R^{4-2}$; the heteroatom in the 5-10 membered heterocycloalkyl is one or more selected from N, O and S, and the number of heteroatom(s) is 1, 2 or 3;

$R^{4-1}$ is each independently

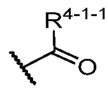
or
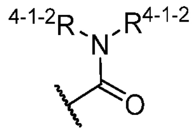
;

$R^{4-2}$ is each independently 5-10 membered heteroaryl unsubstituted or substituted by one or more $R^{4-2-1}$; the heteroatom in the 5-10 membered heteroaryl is one or more selected from N, O and S, and the number of heteroatom(s) is 1, 2 or 3; and
$R^{4-1-1}$ is $C_1$-$C_6$ alkyl; $R^{4-1-2}$ is each independently $C_1$-$C_6$ alkyl; $R^{4-2-1}$ is each independently $C_1$-$C_6$ alkyl.

In a preferred embodiment, $R^1$ is hydroxyl, $C_1$-$C_6$ alkyl unsubstituted or substituted by one or more $R^{1-1}$, $C_3$-$C_6$ cycloalkyl unsubstituted or substituted by one or more $R^{1-2}$, 3-6 membered heterocycloalkyl unsubstituted or substituted by one or more $R^{1-3}$,

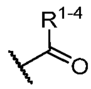

or amino unsubstituted or substituted by one or two $R^{1-5}$; the heteroatom in the 3-6 membered heterocycloalkyl is one or more selected from N, O and S, and the number of heteroatom(s) is 1, 2 or 3;

$R^{1-1}$ is each independently selected from deuterium, hydroxyl, cyano, halogen, 3-6 membered heterocycloalkyl, $C_3$-$C_6$ cycloalkyl unsubstituted or substituted by one or more $R^{1-1-1}$, $C_1$-$C_6$ alkyl unsubstituted or substituted by one or more $R^{1-1-2}$ or $C_1$-$C_6$ alkoxy unsubstituted or substituted by one or more $R^{1-1-3}$; the heteroatom in the 3-6 membered heterocycloalkyl is one or more selected from N, O and S, and the number of heteroatom(s) is 1, 2 or 3; $R^{1-1-1}$ is each independently selected from deuterium, hydroxyl, cyano or halogen; $R^{1-1-2}$ is each independently selected from deuterium, hydroxyl, cyano or halogen; $R^{1-1-3}$ is each independently selected from deuterium, hydroxyl, cyano or halogen;
$R^{1-2}$ is each independently selected from deuterium, hydroxyl, halogen, $C_2$-$C_6$ alkynyl unsubstituted or substituted by one or more $R^{1-2-2}$, $C_1$-$C_6$ alkoxy unsubstituted or substituted by one or more $R^{1-2-3}$, $C_2$-$C_6$ alkenyl unsubstituted or substituted by one or more $R^{1-2-4}$ or amino unsubstituted or substituted by one or two $R^{1-2-5}$; $R^{1-2-2}$ is each independently selected from halogen, hydroxyl or $C_1$-$C_6$ alkoxy unsubstituted or substituted by one or more $R^{1-2-2-1}$; $R^{1-2-2-1}$ is each independently halogen; $R^{1-2-3}$ is each independently selected from halogen, hydroxyl or $C_1$-$C_6$ alkoxy unsubstituted or substituted by one or more $R^{1-2-3-1}$; $R^{1-2-3-1}$ is each independently halogen; $R^{1-2-4}$ is each independently selected from halogen, hydroxyl or $C_1$-$C_6$ alkoxy unsubstituted or substituted by one or more $R^{1-2-4-1}$; $R^{1-2-4-1}$ is each independently halogen;
$R^{1-3}$ is each independently selected from cyano, deuterium, hydroxyl, amino, halogen, $C_1$-$C_6$ alkyl unsubstituted or substituted by one or more $R^{1-3-1}$ or $C_1$-$C_6$ alkoxy unsubstituted or substituted by one or more $R^{1-3-2}$; $R^{1-3-1}$ is each independently halogen; $R^{1-3-2}$ is each independently halogen;
$R^{1-4}$ is $C_1$-$C_6$ alkyl;
$R^{1-5}$ is each independently selected from $C_1$-$C_6$ alkyl,

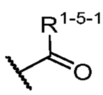

or $C_3$-$C_6$ cycloalkyl;

$R^{1-5-1}$ is $C_1$-$C_6$ alkyl or amino;

or, the atom in $R^1$ is attached to the atom in A to form 5-10 membered heterocycloalkyl unsubstituted or substituted by one or more $R^0$; the heteroatom in the 5-10 membered heterocycloalkyl is one or more selected from N, O, P and S, and the number of heteroatom(s) is 3, 4 or 5; $R^0$ is each independently selected from $C_1$-$C_6$ alkyl or oxo.

In a preferred embodiment, $R^2$ is hydrogen, 6-10 membered aryl unsubstituted or substituted by one or more $R^{2-1}$, 5-10 membered heteroaryl unsubstituted or substituted by one or more $R^{2-2}$, 4-10 membered heterocycloalkyl substituted by one or more $R^{2-3}$,

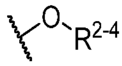 , 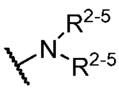 ,

$C_1$-$C_6$ alkyl unsubstituted or substituted by one or more $R^{2-6}$,

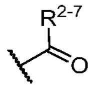 or 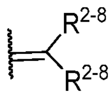 ;

the heteroatom in the 5-10 membered heteroaryl is one or more selected from N, O and S, and the number of heteroatom(s) is 1, 2 or 3; the heteroatom in the 4-10 membered heterocycloalkyl is one or more selected from N, O and S, and the number of heteroatom(s) is 1, 2 or 3,

$R^{2-1}$ is each independently selected from cyano, halogen, $C_1$-$C_6$ alkyl unsubstituted or substituted by one or more $R^{2-1-}$

1, 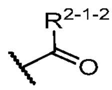 , 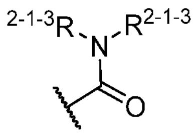

or $C_1$-$C_6$ alkoxy unsubstituted or substituted by one or more $R^{2-1-4}$;

$R^{2-1-1}$ is each independently selected from cyano or halogen; $R^{2-1-2}$ is hydrogen or $C_1$-$C_6$ alkyl; $R^{2-1-3}$ is each independently selected from hydrogen or $C_1$-$C_6$ alkyl; $R^{2-1-4}$ is each independently halogen;

$R^{2-2}$ is each independently selected from cyano, halogen, $C_1$-$C_6$ alkyl unsubstituted or substituted by one or more $R^{2-2-}$

1, 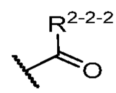 or 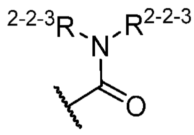 ;

$R^{2-2-1}$ is each independently selected from cyano or halogen; $R^{2-2-2}$ is hydrogen or $C_1$-$C_6$ alkyl; $R^{2-2-3}$ is each independently selected from hydrogen or $C_1$-$C_6$ alkyl;

$R^{2-3}$ is each independently selected from $C_1$-$C_6$ alkyl substituted by one or more $R^{2-3-1}$,

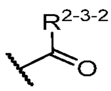 ,

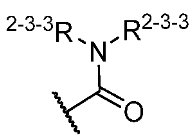 ,

or 3-6 membered heterocycloalkyl unsubstituted or substituted by one or more $R^{2-3-4}$; the heteroatom in the 3-6 membered heterocycloalkyl is one or more selected from N, O and S, and the number of heteroatom(s) is 1, 2 or 3;
$R^{2-3-1}$ is each independently cyano;
$R^{2-3-2}$ is hydrogen; $R^{2-3-4}$ is each independently $C_1$-$C_6$ alkyl;
$R^{2-4}$ is 4-10 membered heterocycloalkyl substituted by one or more $R^{2-4-1}$, 5-10 membered heteroaryl unsubstituted or substituted by one or more $R^{2-4-2}$, 6-10 membered aryl unsubstituted or substituted by one or more $R^{2-4-3}$, the heteroatom in the 4-10 membered heterocycloalkyl is one or more selected from N, O and S, and the number of heteroatom(s) is 1, 2 or 3; the heteroatom in the 5-10 membered heteroaryl is one or more selected from N, O and S, and the number of heteroatom(s) is 1, 2 or 3;
$R^{2-4-1}$ is each independently selected from hydroxyl, halogen, $C_1$-$C_6$ alkyl,

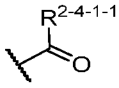 or 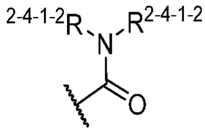 ;

$R^{2-4-2}$ is each independently selected from hydroxyl, halogen or $C_1$-$C_6$ alkyl; $R^{2-4-3}$ is each independently selected from hydroxyl, halogen or $C_1$-$C_6$ alkyl;
$R^{2-5}$ is each independently hydrogen, 4-10 membered heterocycloalkyl substituted by one or more $R^{2-5-1}$, or

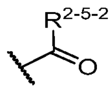 ;

the heteroatom in the 4-10 membered heteroaryl is one or more selected from N, O and S, and the number of heteroatom(s) is 1, 2 or 3;
$R^{2-5-1}$ is each independently selected from

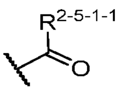 or 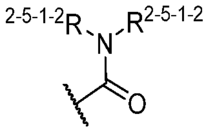 ;

$R^{2-5-1-1}$ is hydrogen or $C_1$-$C_6$ alkyl;
$R^{2-5-1-2}$ is each independently selected from hydrogen or $C_1$-$C_6$ alkyl;
$R^{2-5-2}$ is independently $C_1$-$C_6$ alkyl;
$R^{2-6}$ is each independently selected from halogen, $C_1$-$C_6$ alkyl, 4-10 membered heterocycloalkyl unsubstituted or substituted by one or more $R^{2-6-1}$ or 5-10 membered heteroaryl unsubstituted or substituted by one or more $R^{2-6-2}$; the heteroatom in the 4-10 membered heterocycloalkyl is one or more selected from N, O and S, and the number of heteroatom(s) is 1, 2 or 3; the heteroatom in the 5-10 membered heteroaryl is one or more selected from N, O and S, and the number of heteroatom(s) is 1, 2 or 3;
$R^{2-6-1}$ is each independently selected from halogen, $C_1$-$C_6$ alkyl or

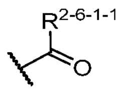 ;

$R^{2-6-1-1}$ is $C_1$-$C_6$ alkyl;
$R^{2-6-2}$ is each independently selected from halogen or $C_1$-$C_6$ alkyl;
$R^{2-7}$ is $C_1$-$C_6$ alkyl, 4-10 membered heterocycloalkyl unsubstituted or substituted by one or more $R^{2-7-1}$ or 5-10 membered heteroaryl unsubstituted or substituted by one or more $R^{2-7-2}$; the heteroatom in the 4-10 membered heterocycloalkyl is one or more selected from N, O and S, and the number of heteroatom(s) is 1, 2 or 3; the heteroatom in the 5-10 membered heteroaryl is one or more selected from N, O and S, and the number of heteroatom(s) is 1, 2 or 3;
$R^{2-7-1}$ is each independently selected from halogen, $C_1$-$C_6$ alkyl,

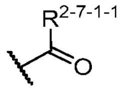 or 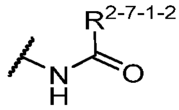 ;

$R^{2-7-1-1}$ is $C_1$-$C_6$ alkyl; $R^{2-7-1-2}$ is $C_1$-$C_6$ alkyl;
$R^{2-7-2}$ is each independently selected from halogen or $C_1$-$C_6$ alkyl;
$R^{2-8}$ is each independently $C_1$-$C_6$ alkyl, or two $R^{2-8}$, together with the atom to which they are attached, form 4-10 membered heterocycloalkyl unsubstituted or substituted by one or more $R^{2-8-1}$; the heteroatom in the 4-10 membered heterocycloalkyl is one or more selected from N, O and S, and the number of heteroatom(s) is 1, 2 or 3;
$R^{2-8-1}$ is each independently

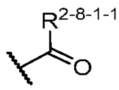 ;

and $R^{2-8-1-1}$ is hydrogen or $C_1$-$C_6$ alkyl.

In a preferred embodiment, $R^3$ is 5-10 membered heteroaryl unsubstituted or substituted by one or more $R^{3-1}$ or 3-10 membered heterocycloalkyl unsubstituted or substituted by one or more $R^{3-2}$; the heteroatom in the 5-10 membered heteroaryl is one or more selected from N, O and S, and the number of heteroatom(s) is 1, 2 or 3; the heteroatom in the 3-10 membered heterocycloalkyl is one or more selected from N, O and S, and the number of heteroatom(s) is 1, 2 or 3;

$R^{3-1}$ is each independently selected from hydroxyl, halogen or

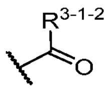 ;

$R^{3-1-2}$ is $C_1$-$C_6$ alkyl or $C_2$-$C_6$ alkenyl;
$R^{3-2}$ is each independently selected from hydroxyl, halogen, $C_1$-$C_6$ alkyl unsubstituted or substituted by one or more $R^{3-2-1}$ or

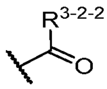 ;

$R^{3-2-1}$ is each independently halogen; and $R^{3-2-2}$ is $C_1$-$C_6$ alkyl or $C_2$-$C_4$ alkenyl.

In a preferred embodiment, $R^4$ is 5-10 membered heterocycloalkyl unsubstituted or substituted by one or more $R^{4-1}$ or $C_1$-$C_6$ alkyl unsubstituted or substituted by one or more $R^{4-2}$; the heteroatom in the 5-10 membered heterocycloalkyl is one or more selected from N, O and S, and the number of heteroatom(s) is 1, 2 or 3;

$R^{4-1}$ is each independently

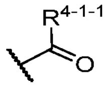 or 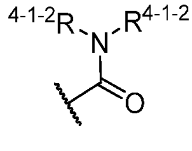 ;

$R^{4-2}$ is each independently 5-10 membered heteroaryl unsubstituted or substituted by one or more $R^{4-2-1}$; the heteroatom in the 5-10 membered heteroaryl is one or more selected from N, O and S, and the number of heteroatom(s) is 1, 2 or 3; and
$R^{4-1-1}$ is $C_1$-$C_6$ alkyl; $R^{4-1-2}$ is each independently $C_1$-$C_6$ alkyl; $R^{4-2-1}$ is each independently $C_1$-$C_6$ alkyl.

In a preferred embodiment, in the compound of formula I, the pharmaceutically acceptable salt thereof or the isotopic compound thereof, some groups can be defined as follows, and other groups can be defined as in any of the above

embodiments (hereinafter referred to as "in a preferred embodiment"):

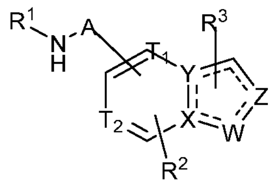

I-a ,

wherein the compound of formula I is a compound of formula I-a,

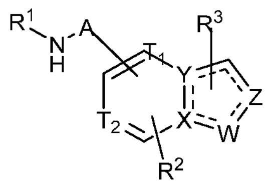

I-a ;

Y is C or N, X is C or N,
W is N, CH, O, $NR^4$ or $CR^4$;
Z is CH, O or N;
$T_1$ is C or N; and
$T_2$ is C or N; and
A, $R^1$, $R^2$, $R^3$ and $R^4$ are as previously defined.

In a preferred embodiment, at least one of $T_1$, $T_2$, X and Y is N, or Y, or Y, W and Z comprise only one heteroatom.

In a preferred embodiment, the compound of formula I-a can be a compound of formula I-A, formula I-B or formula I-C,

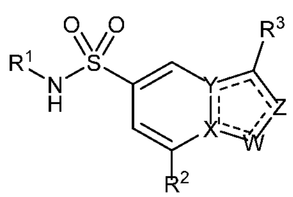

I-A ,

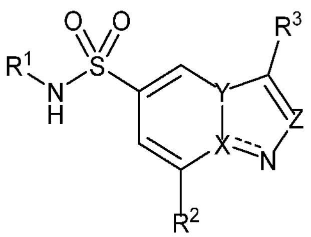

I-B or

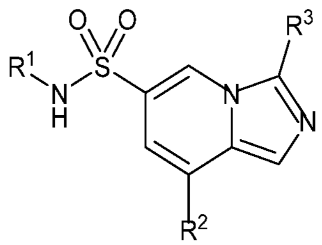

I-C

In a preferred embodiment, the compound of formula I-a can be a compound of formula IV,

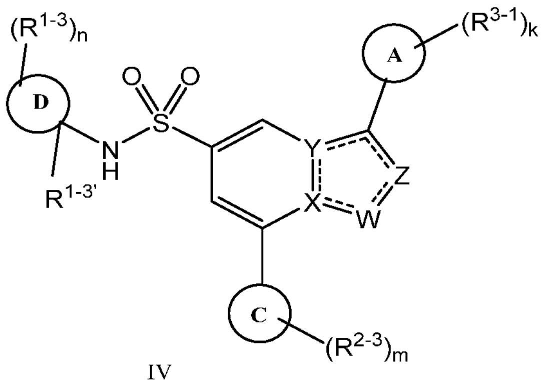

IV

ring D is 3-6 membered heterocycloalkyl; the heteroatom in the 3-6 membered heterocycloalkyl is one or more

selected from N, O and S, and the number of heteroatom(s) is 1, 2 or 3 (the 3-6 membered heterocycloalkyl in ring D is defined as in $R^1$);

ring C is 4-12 membered heterocycloalkyl; the heteroatom in the 4-12 membered heterocycloalkyl is one or more selected from N, O and S, and the number of heteroatom(s) is 1, 2 or 3 (the 4-12 membered heterocycloalkyl in ring C is defined as in $R^2$);

ring A is 5-10 membered heteroaryl; the heteroatom in the 5-10 membered heteroaryl is one or more selected from N, O and S, and the number of heteroatom(s) is 1, 2 or 3 (the 5-10 membered heteroaryl in ring A is defined as in $R^3$);

n is 0, 1 or 2; m is 0, 1 or 2; k is 0, 1 or 2;

$R^{1-3'}$ is hydrogen or $R^{1-3}$; and

$R^{1-3}$, $R^{2-3}$ and $R^{3-1}$ are as previously defined.

In a preferred embodiment, the compound of formula I-a can be a compound of formula V,

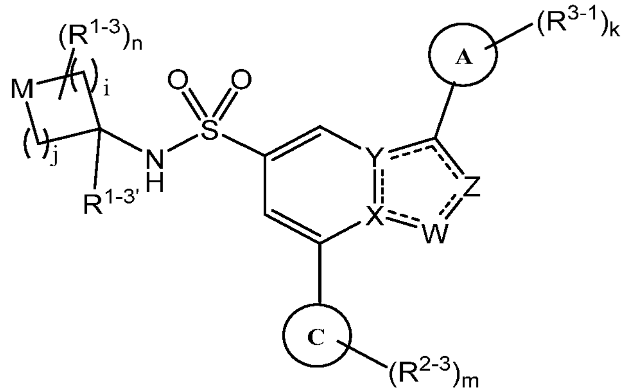

V

M is N, O or S;

i is 0, 1 or 2; j is 0, 1 or 2; i and j are not 0 at the same time; and

ring C, ring A, $R^{1-3'}$, $R^{1-3}$, $R^{2-3}$, $R^{3-1}$, n, m and k are as previously defined.

In a preferred embodiment, the compound of formula I-a can be a compound of formula VI,

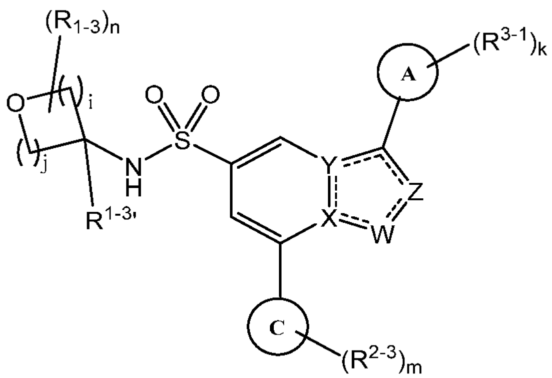

VI

ring C, ring A, $R^{1-3'}$, $R^{1-3}$, $R^{2-3}$, $R^{3-1}$, n, m, k, i and j are as previously defined.

In a preferred embodiment, the compound of formula I-a can be a compound of formula VII,

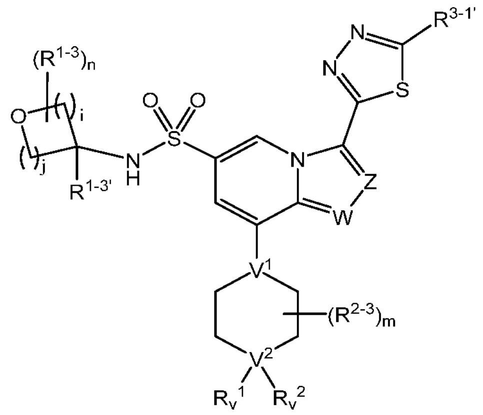

VII

$V^1$ is CH, N or O; $V^2$ is CH, N or O; $V^1$ and $V^2$ are not CH at the same time;
$R^{3-1'}$ is hydrogen or $R^{3-1}$; and
$R_V{}^1$ is hydrogen or $R^{2-3}$; $R_V{}^2$ is hydrogen $R^{2-3}$; and
$R^{1-3'}$, $R^{1-3}$, $R^{2-3}$, $R^{3-1}$, n, m, i and j are as previously defined.

In a preferred embodiment, the compound of formula I-a can be a compound of formula VIII,

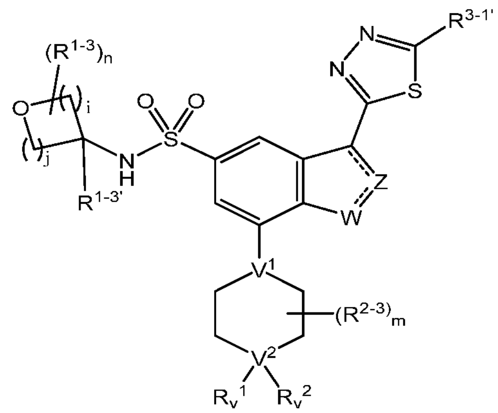

VIII

$R_V1$, $R_V{}^2$, $R^{1-3'}$, $R^{1-3}$, $R^{2-3}$, $R^{3-1'}$, n, m, i and j are as previously defined.

In a preferred embodiment, the compound of formula I-a can be a compound of formula IX,

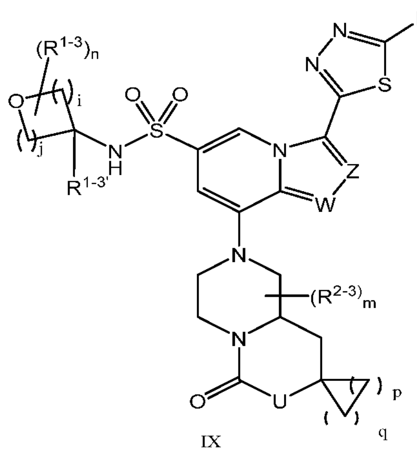

IX

U is CH, N or O;

p is 0, 1 or 2; q is 0, 1 or 2; and
$R^{1-3'}$, $R^{1-3}$, $R^{2-3}$, $R^{3-1}$, n, m, i and j are ss previously defined.

In a preferred embodiment, the compound of formula I-a can be a compound of formula X,

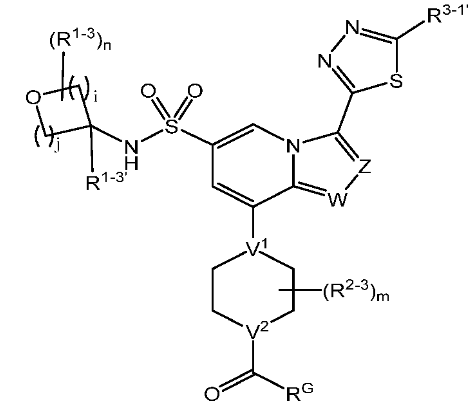

X

$R^G$ is $R^{2-3-2}$ or

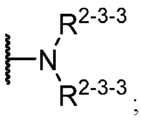

;

and
$R^{1-3'}$, $R^{1-3}$, $R^{2-3}$, $R^{3-1'}$, $R^{2-3-2}$, $R^{2-3-3}$, $V^1$, $V^2$, n, m, i and j are as previously defined

In a preferred embodiment, the compound of formula I is a compound of formula I-b, I-c, I-d, I-e, I-f, I-g, I-h, I-i, I-j, I-k, I-L or I-m,

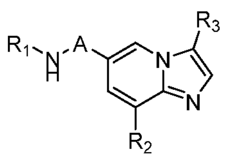

I-b ,

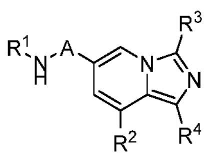

I-c ,

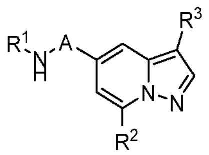

I-d ,

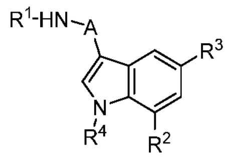

I-e ,

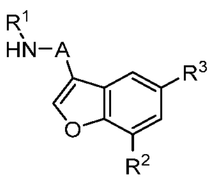

I-f ,

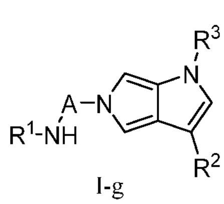

I-g ,

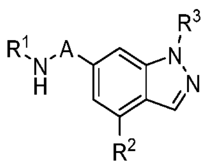

I-h ,

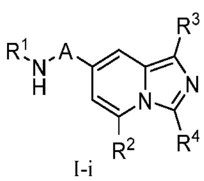

I-i ,

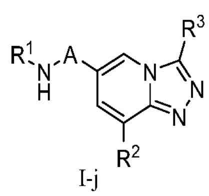

I-j ,

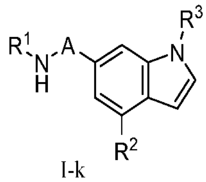

I-k ,

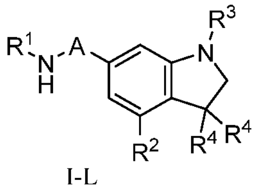

I-L or

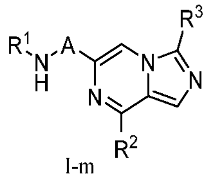

I-m

In a preferred embodiment, the compound of formula I is a compound of formula I-b, formula I-c, formula I-d,

formula I-e, formula I-f or formula I-g,

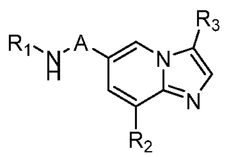
I-b ,
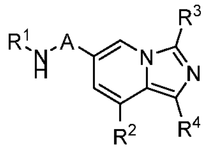
I-c ,
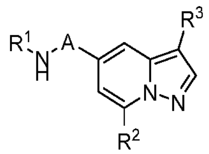
I-d ,
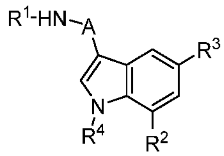
I-e ,

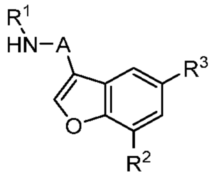
I-f or
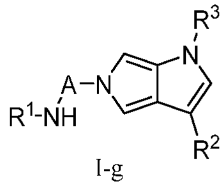
I-g

In a preferred embodiment, in $R^A$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl.

In a preferred embodiment, in ring B, the heteroatom in the 5-membered-fused 5-membered heteroaromatic ring can be N, and the number of heteroatom(s) can be 1, 2 or 3, for example,

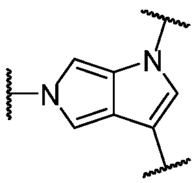

In a preferred embodiment, in ring B, the 3-6 membered cycloalkyl can be cyclopropyl, cyclobutyl, cyclopentyl or cyclohexyl, for example, cyclopropyl.

In a preferred embodiment, in ring B, the heteroatom in the 5-membered-fused 6-membered heteroaromatic ring is N and/or O, and the number of heteroatom(s) can be 1, 2 or 3, for example,

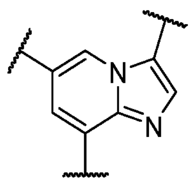 , 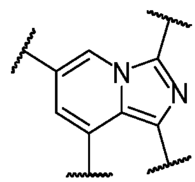 ,

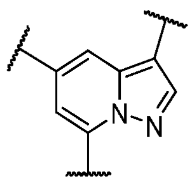 , 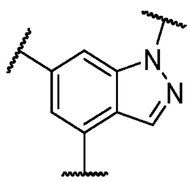 , 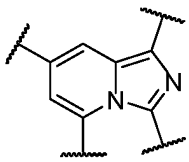 , 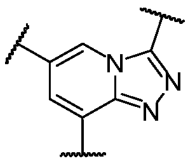 , 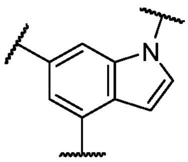 , 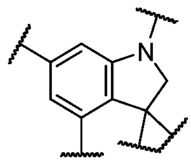 ,

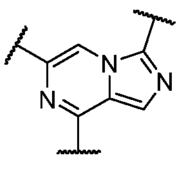 , 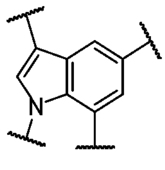 or 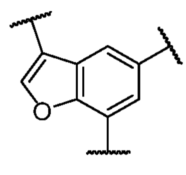

In a preferred embodiment, in ring B, the heteroatom in the 5-membered-fused 6-membered heteroaromatic ring can be N and/or O, and the number of heteroatom(s) can be 1, 2 or 3, for example,

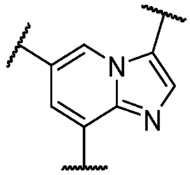, 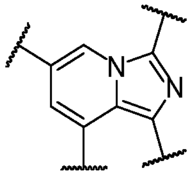,

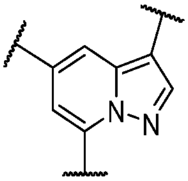, 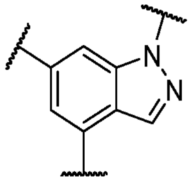, 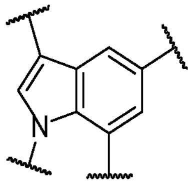 or 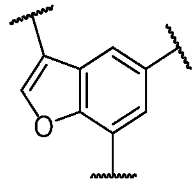

In a preferred embodiment, in $R^1$, the $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl, ethyl, isopropyl or tert-butyl.

In a preferred embodiment, in $R^1$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl, ethyl or isopropyl.

In a preferred embodiment, in $R^1$, the $C_3$-$C_6$ cycloalkyl is cyclopropyl, cyclobutyl, cyclopentyl, cyclohexyl, bicyclopentane or

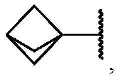,

for example, cyclopropyl, cyclobutane, bicyclopentane or

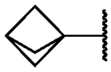

In a preferred embodiment, in $R^1$, the $C_3$-$C_6$ cycloalkyl can be cyclopropyl, cyclobutyl, cyclopentyl, cyclohexyl or bicyclopentane, for example, cyclopropyl, cyclobutane or bicyclopentane.

In a preferred embodiment, in $R^1$, the heteroatom in the 3-6 membered heterocycloalkyl is N, O or S, and the number of heteroatom(s) is 1 or 2; the heteroatom in the 3-6 membered heterocycloalkyl can be O or N, and the number of heteroatom can also be 1; azacyclobutane group, oxacyclobutane group, oxacyclo -azacyclobutanyl, oxacyclobutanyl, oxacyclopentanyl or oxacyclohexanyl is preferred, for example,

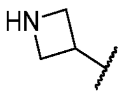, 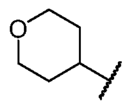, 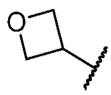 or 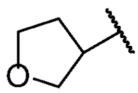

In a preferred embodiment, in $R^1$, the heteroatom in the 3-6 membered heterocycloalkyl can be N, O or S, and the number of heteroatom(s) can be 1 or 2; the heteroatom in the 3-6 membered heterocycloalkyl can also be O or N, and the number of heteroatom can also be 1, for example,

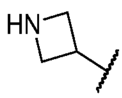, 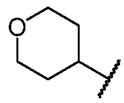 or 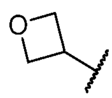

In a preferred embodiment, in $R^{1-1}$, the heteroatom in the 3-6 membered heterocycloalkyl can be N, O or S, and the number of heteroatom(s) can be 1 or 2; the heteroatom in the 3-6 membered heterocycloalkyl can also be O or N, and the number of heteroatom can also be 1, for example,

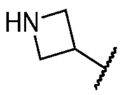, 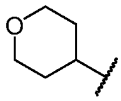 or 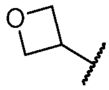

In a preferred embodiment, in each $R^{1-1}$, the halogen can be fluorine, chlorine, bromine or iodine, for example, fluorine.

In a preferred embodiment, in each $R^{1-1}$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl, ethyl or isopropyl.

In a preferred embodiment, in each $R^{1-1}$, the $C_1$-$C_6$ alkoxy is methoxy, ethoxy, n-propoxy, isopropoxy, n-butoxy, isobutoxy, pr-butoxy, sec-butoxy or tert-butoxy, or can be methoxy or ethoxy.

In a preferred embodiment, in each $R^{1-1}$, the $C_3$-$C_6$ cycloalkyl can be cyclopropyl, cyclobutyl, cyclopentyl or cyclohexyl.

In a preferred embodiment, in each $R^{1-1-1}$, the halogen can be fluorine, chlorine, bromine or iodine, for example, fluorine.

In a preferred embodiment, in each $R^{1-1-2}$, the halogen can be fluorine, chlorine, bromine or iodine, for example, fluorine.

In a preferred embodiment, in each $R^{1-1-3}$, the halogen can be fluorine, chlorine, bromine or iodine, for example, fluorine.

In a preferred embodiment, in each $R^{1-2}$, the halogen can be fluorine, chlorine, bromine or iodine, for example, fluorine.

In a preferred embodiment, in each $R^{1-2}$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl, ethyl or isopropyl.

In a preferred embodiment, in each $R^{1-2}$, the $C_2$-$C_6$ alkynyl can be

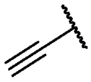, 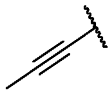, 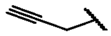,

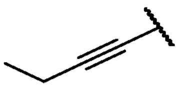, 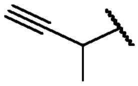 or 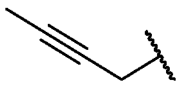,

for example,

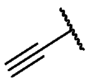

In a preferred embodiment, in each $R^{1-2}$, the $C_2$-$C_6$ alkenyl can be

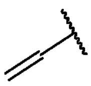, 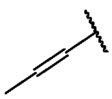, 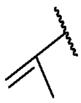 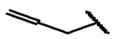,

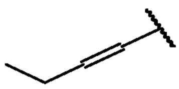, 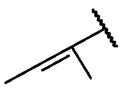, 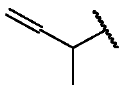 or 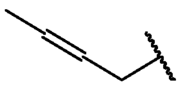,

for example,

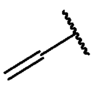

In a preferred embodiment, in $R^{1-2}$, the $C_1$-$C_6$ alkoxy can be methoxy, ethoxy, n-propoxy, isopropoxy, n-butoxy, isobutoxy, pr-butoxy, sec-butoxy or tert-butoxy, or can be methoxy or ethoxy.

In a preferred embodiment, in each $R^{1-2-1}$, the halogen can be fluorine, chlorine, bromine or iodine, for example, fluorine.

In a preferred embodiment, in each $R^{1-2-1}$, the $C_1$-$C_6$ alkoxy can be methoxy, ethoxy, n-propoxy, isopropoxy, n-butoxy, isobutoxy, pr-butoxy, sec-butoxy or tert-butoxy, for example, methoxy or ethoxy.

In a preferred embodiment, in each $R^{1-2-1-1}$, the halogen can be fluorine, chlorine, bromine or iodine, for example, fluorine.

In a preferred embodiment, in each $R^{1-2-2}$, the halogen can be fluorine, chlorine, bromine or iodine, for example, fluorine.

In a preferred embodiment, in each $R^{1-2-2}$, the $C_1$-$C_6$ alkoxy can be methoxy, ethoxy, n-propoxy, isopropoxy, n-butoxy, isobutoxy, pr-butoxy, sec-butoxy or tert-butoxy, for example, methoxy or ethoxy.

In a preferred embodiment, in each $R^{1-2-2-1}$, the halogen can be fluorine, chlorine, bromine or iodine, for example, fluorine.

In a preferred embodiment, in each $R^{1-2-3}$, the halogen can be fluorine, chlorine, bromine or iodine, for example, fluorine.

In a preferred embodiment, in each $R^{1-2-3}$, the $C_1$-$C_6$ alkoxy can be methoxy, ethoxy, n-propoxy, isopropoxy, n-butoxy, isobutoxy, pr-butoxy, sec-butoxy or tert-butoxy, or can be methoxy or ethoxy.

In a preferred embodiment, in each $R^{1-2-3-1}$, the halogen can be fluorine, chlorine, bromine or iodine, for example, fluorine.

In a preferred embodiment, in each $R^{1-2-4}$, the halogen can be fluorine, chlorine, bromine or iodine, for example, fluorine.

In a preferred embodiment, in each $R^{1-2-4}$, the $C_1$-$C_6$ alkoxy can be methoxy, ethoxy, n-propoxy, isopropoxy, n-butoxy, isobutoxy, pr-butoxy, sec-butoxy or tert-butoxy, for example, methoxy or ethoxy.

In a preferred embodiment, in each $R^{1-2-4-1}$, the halogen can be fluorine, chlorine, bromine or iodine, for example, fluorine.

In a preferred embodiment, in each $R^{1-2-5}$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl, ethyl or isopropyl.

In a preferred embodiment, in each $R^{1-2-5}$, the $C_3$-$C_6$ cycloalkyl can be cyclopropyl, cyclobutyl, cyclopentyl, cyclohexyl or bicyclopentane, for example, cyclopropyl, cyclobutane or bicyclopentane.

In a preferred embodiment, in $R^{1-2-5-1}$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl, ethyl or isopropyl.

In a preferred embodiment, in each $R^{1-3}$, the halogen can be fluorine, chlorine, bromine or iodine, for example, fluorine.

In a preferred embodiment, in each $R^{1-3}$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl.

In a preferred embodiment, in each $R^{1-3}$, the $C_1$-$C_6$ alkoxy is methoxy, ethoxy, n-propoxy, isopropoxy, n-butoxy, isobutoxy, pr-butoxy, sec-butoxy or tert-butoxy, or can be methoxy or ethoxy.

In a preferred embodiment, in each $R^{1-3}$, the $C_2$-$C_6$ alkynyl is

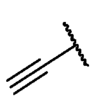 , 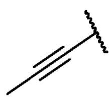 , 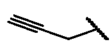 , 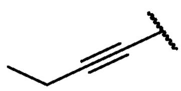 ,

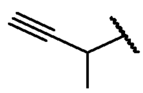 or 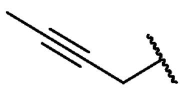 ,

for example,

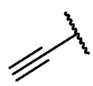

In a preferred embodiment, in each $R^{1-3-1}$, the halogen can be fluorine, chlorine, bromine or iodine, for example, fluorine.

In a preferred embodiment, in each $R^{1-3-2}$, the halogen can be fluorine, chlorine, bromine or iodine, for example, fluorine.

In a preferred embodiment, in $R^{1-4}$, the $C_1$-$C_6$ alkyl can be $C_1$-$C_4$ alkyl, and can also be methyl, ethyl, n-propyl,

isopropyl, n-butyl, isobutyl, pr-butyl, sec-butyl or tert-butyl, or can be methyl.

In a preferred embodiment, in each $R^{1-5}$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl.

In a preferred embodiment, in each $R^{1-5}$, the $C_3$-$C_6$ cycloalkyl can be cyclopropyl, cyclobutyl, cyclopentyl, cyclohexyl or bicyclopentane, for example, cyclopropyl, cyclobutane or bicyclopentane.

In a preferred embodiment, in $R^{1-5-1}$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl.

In a preferred embodiment, the atom in $R^1$, when attached to the atom in A, forms 5-10 membered heterocycloalkyl unsubstituted or substituted by one or more $R^0$; the 5-10 membered heterocycloalkyl can be 5-6 membered heterocycloalkyl, for example,

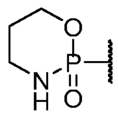 or 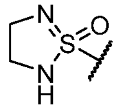

In a preferred embodiment, in each $R^0$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl.

In a preferred embodiment, in $R^2$, the 6-10 membered aryl can be phenyl or naphthyl, for example, phenyl.

In a preferred embodiment, in $R^2$, the 5-10 membered heteroaryl is 5 or 6 membered heteroaryl, or 5-membered-fused 6-membered heteroaryl. The heteroatom in the 5-10 membered heteroaryl is one or more selected from N, O and S, and the number of heteroatom(s) is 1, 2 or 3.

In a preferred embodiment, in $R^2$, the 5-10 membered heteroaryl can be 5 or 6 membered heteroaryl, 5-membered-fused 5-membered heteroaryl, or 5-membered-fused 6-membered heteroaryl. The heteroatom in the 5-10 membered heteroaryl is one or more selected from N, O and S, and the number of heteroatom(s) is 1, 2 or 3.

The heteroatom in the 5 membered heteroaryl can be N, the number of heteroatoms is 2, pyrazolyl is preferred, for example,

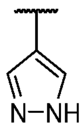.

The heteroatom in the 6 membered heteroaryl can be N, the number of heteroatom(s) is 1, and pyridyl is preferred, for example,

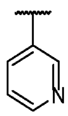

The heteroatom in the 5-membered-fused 5-membered heteroaryl can be N, the number of heteroatoms is 3, and dihydropyrrolopyrazolyl is preferred, for example,

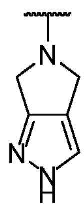

The heteroatom in the 5-membered-fused 6-membered heteroaryl can be N and/or O, the number of heteroatom(s) is 1, 2 or 3, and benzofuryl, dihydroimidazopyrazinyl or benzoimidazolyl is preferred, for example,

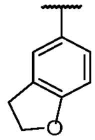,
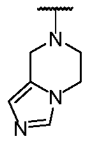

or

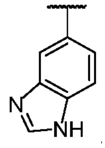

In a preferred embodiment, in $R^2$, the 5-10 membered heteroaryl can be 5 or 6 membered heteroaryl, or 5-membered-fused 6-membered heteroaryl. The heteroatom in the 5-10 membered heteroaryl is one or more selected from N and/or S, and the number of heteroatom(s) is 1 or 2. The heteroatom in the 5 membered heteroaryl can be selected from N and/or S, and the number of heteroatoms is 2. The heteroatom in the 6 membered heteroaryl can be N, and the number of heteroatom(s) is 1 or 2. The heteroatom in the 5-membered-fused 6-membered heteroaryl can be selected from N and/or O, and the number of heteroatoms is 2, or the number of heteroatom(s) is 1, 2 or 3.

In a preferred embodiment, in $R^2$, the 4-12 membered heterocycloalkyl is one or more selected from a monocyclic ring, a bridged ring, a fused ring and a spiro ring. The monocyclic ring can be 4-6 membered heterocycloalkyl. The fused ring can be 5-membered-fused 6-membered heterocycloalkyl, 4-membered-fused 6-membered heterocycloalkyl or 5-membered-fused 5-membered heterocycloalkyl. The spiro ring can be 4 membered-spiro 6-membered ring, 4 membered-spiro 5-membered ring, 4 membered-spiro 4-membered ring, 5 membered-spiro 6-membered ring or 3 membered-spiro 6-membered ring. The bridged ring can be bicycloheterooctane or bicycloheteroheptane. The heteroatom in the 4-12 membered heterocycloalkyl is selected from N and/or O, and the number of heteroatom(s) is 1, 2 or 3.

The 4-12 membered heterocycloalkyl can be 3 rings-spiro-6 membered-fused-6 membered heterocycloalkyl, preferably, hexahydro-spiro-cyclopropan-pyrazinopyrazinyl, for example,

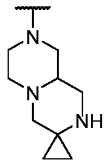

The bicycloheterooctane can be bicyclo[2.2.2]heterooctane, for example,

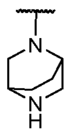

The bicycloheteroheptane can be bicyclo[31.1]heteroheptane, for example,

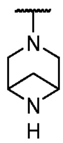

The 4-6 membered heterocycloalkyl can be azacyclohexanyl, tetrahydropyridyl, piperazinyl, azacyclopentanyl or morpholinyl, for example,

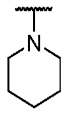, 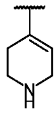, 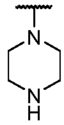, 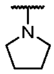, 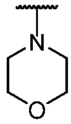 or 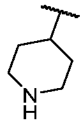

The 5-membered-fused 6-membered heterocycloalkyl can be tetrahydrofuranopyridyl, hexahydropyrrolopyrazinyl, hexahydropyrazinooxazinyl or hexahydroimidazopyrazinyl, for example,

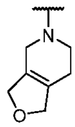, 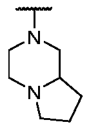, 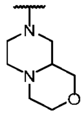 or 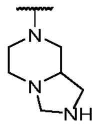

The 5-membered-fused 5-membered heterocycloalkyl can be tetrahydrofuranopyrrolyl, for example,

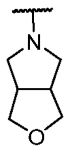.

The 4 membered-spiro 6-membered ring can be oxaaza-spiro-nonanyl, dioxaaza-spiro-nonanyl or aza-spiro-nonanyl, for example,

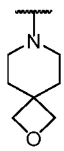, 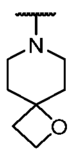, 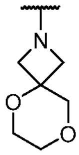 or 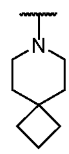.

The 3 membered-spiro 6-membered ring can be diaza-spiro-octanyl, for example,

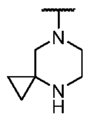.

The 5 membered-spiro 6-membered ring can be diaza-spiro-decanyl, for example,

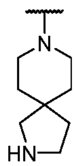

In a preferred embodiment, in $R^2$, the 4-12 membered heterocycloalkyl is saturated heterocycloalkyl, or heterocycloalkyl containing 1-2 double bond(s).

In a preferred embodiment, in $R^2$, the 4-10 membered heterocycloalkyl can be a monocyclic ring, a bridged ring, a fused ring or a spiro ring. The monocyclic ring can be 4-6 membered heterocycloalkyl. The fused ring can be 5-membered-fused 6-membered heterocycloalkyl or 4-membered-fused 6-membered heterocycloalkyl. The spiro ring can be 4 membered-spiro 6-membered ring, 4 membered-spiro 5-membered ring or 4 membered-spiro 4-membered ring. The heteroatom in the 4-10 membered heterocycloalkyl is selected from N and/or O, and the number of heteroatom(s) is 1 or 2.

In a preferred embodiment, in $R^2$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl.

In a preferred embodiment, in $R^2$, the $C_3$-$C_6$ cycloalkyl is cyclopropyl, cyclobutyl, cyclopentyl or cyclohexyl, for example, cyclopentyl.

In a preferred embodiment, in $R^2$, the $C_3$-$C_6$ cycloalkenyl can be cycloalkenyl containing 1 double bond, or can be

5 membered cycloalkenyl, for example,

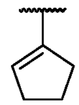

In a preferred embodiment, in each $R^{2-1}$, the halogen can be fluorine, chlorine, bromine or iodine, for example, fluorine.

In a preferred embodiment, in each $R^{2-1}$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl.

In a preferred embodiment, in each $R^{2-1}$, the $C_1$-$C_6$ alkoxy can be methoxy, ethoxy, n-propoxy, isopropoxy, n-butoxy, isobutoxy, pr-butoxy, sec-butoxy or tert-butoxy, or can be methoxy or ethoxy.

In a preferred embodiment, in each $R^{2-1-1}$, the halogen can be fluorine, chlorine, bromine or iodine, for example, fluorine.

In a preferred embodiment, in each $R^{2-1-2}$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl.

In a preferred embodiment, in each $R^{2-1-3}$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl.

In a preferred embodiment, in each $R^{2-1-4}$, the halogen can be fluorine, chlorine, bromine or iodine, for example, fluorine.

In a preferred embodiment, in each $R^{2-2}$, the halogen can be fluorine, chlorine, bromine or iodine, for example, fluorine.

In a preferred embodiment, in each $R^{2-2}$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl or isopropyl.

In a preferred embodiment, in each $R^{2-2}$, the $C_1$-$C_6$ alkoxy is methoxy, ethoxy, n-propoxy, isopropoxy, n-butoxy, isobutoxy, pr-butoxy, sec-butoxy or tert-butoxy, for example, methoxy.

In a preferred embodiment, in each $R^{2-2-1}$, the halogen can be fluorine, chlorine, bromine or iodine, for example, fluorine.

In a preferred embodiment, in each $R^{2-2-2}$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl.

In a preferred embodiment, in each $R^{2-2-3}$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl.

In a preferred embodiment, in each $R^{2-2-4}$, the $C_1$-$C_6$ alkyl can be $C_1$-$C_4$ alkyl, and can be methyl, ethyl, n-propyl, isopropyl, n-butyl, isobutyl, pr-butyl, sec-butyl or tert-butyl, for example, methyl.

In a preferred embodiment, in each $R^{2-2-4}$, the $C_3$-$C_6$ cycloalkyl is cyclopropyl, cyclobutyl, cyclopentyl or cyclohexyl, for example, cyclopropyl.

In a preferred embodiment, in each $R^{2-3}$, the halogen can be fluorine, chlorine, bromine or iodine, for example, fluorine.

In a preferred embodiment, in each $R^{2-3}$, the $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl, ethyl, isopropyl or tert-butyl.

In a preferred embodiment, in each $R^{2-3}$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl.

In a preferred embodiment, in each $R^{2-3}$, the heteroatom in the 3-6 membered heterocycloalkyl can be N, O or S, and the number of heteroatom(s) can be 1 or 2; the heteroatom in the 3-6 membered heterocycloalkyl can also be O or N, and the number of heteroatom(s) can also be 1; and oxacyclobutanyl s preferred, for example,

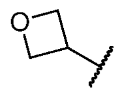

In a preferred embodiment, in each $R^{2-3}$, the $C_1$-$C_6$ alkoxy is methoxy, ethoxy, n-propoxy, isopropoxy, n-butoxy, isobutoxy, pr-butoxy, sec-butoxy or tert-butoxy, or can be methoxy, ethoxy or isopropoxy.

In a preferred embodiment, in each $R^{2-3}$, the $C_1$-$C_6$ alkoxy can be methoxy, ethoxy, n-propoxy, isopropoxy, n-butoxy, isobutoxy, pr-butoxy, sec-butoxy or tert-butoxy, for example, methoxy or ethoxy.

In a preferred embodiment, in each $R^{2-3}$, the $C_3$-$C_6$ cycloalkyl is cyclopropyl, cyclobutyl, cyclopentyl or cyclohexyl, for example, cyclopropyl.

In a preferred embodiment, in each $R^{2-3}$, the 5-10 membered heteroaryl is 5 membered heteroaryl or 6 membered heteroaryl. The heteroatom in the 5-10 membered heteroaryl is N and/or O, and the number of heteroatom(s)

is 1, 2, or 3. The heteroatom in the 5 membered heteroaryl can be N and/or O, the number of heteroatom(s) is 2 or 3, and triazolyl, oxadiazolyl, or imidazolyl is preferred, for example,

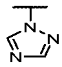 , 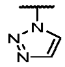 , 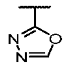 or 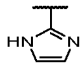

The heteroatom in the 6 membered heteroaryl can beN, the number of heteroatom(s) is 1 or 2, and pyridyl is preferred, for example,

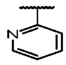 .

In a preferred embodiment, in each $R^{2-3-1}$, the halogen can be fluorine, chlorine, bromine or iodine, for example, fluorine.

In a preferred embodiment, in each $R^{2-3-1}$, the $C_1$-$C_6$ alkoxy can be methoxy, ethoxy, n-propoxy, isopropoxy, n-butoxy, isobutoxy, pr-butoxy, sec-butoxy or tert-butoxy, for example, methoxy or ethoxy.

In a preferred embodiment, in each $R^{2-3-1}$, the $C_3$-$C_6$ cycloalkyl is cyclopropyl, cyclobutyl, cyclopentyl or cyclohexyl, for example, cyclopropyl.

In a preferred embodiment, in each $R^{2-3-2}$, the $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl, ethyl, isopropyl or tert-butyl.

In a preferred embodiment, in $R^{2-3-2}$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl or isopropyl.

In a preferred embodiment, in $R^{2-3-2}$, the $C_3$-$C_6$ cycloalkyl is cyclopropyl, cyclobutyl, cyclopentyl or cyclohexyl, for example, cyclopropyl or cyclobutyl.

In a preferred embodiment, in $R^{2-3-2}$, the $C_1$-$C_6$ alkoxy is methoxy, ethoxy, n-propoxy, isopropoxy, n-butoxy, isobutoxy, pr-butoxy, sec-butoxy or tert-butoxy, for example, isopropoxy.

In a preferred embodiment, in $R^{2-3-2}$, the heteroatom in the 3-6 membered heterocycloalkyl is one or more selected from N, O and S, and the number of heteroatom(s) can be 1 or 2; oxacyclobutanyl, azacyclobutanyl, oxacyclopentanyl, azacyclopentanyl, oxacyclohexanyl, azacyclohexanyl, pyrazinyl, thiazolidinyl or oxabicyclohexanyl is preferred, for example,

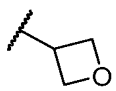 ,

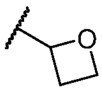 , 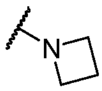 , 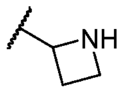 , 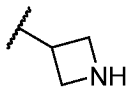 , 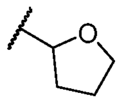 , 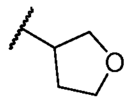 , 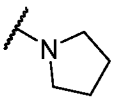 , 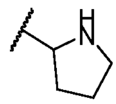 , 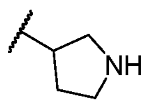 ,

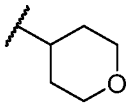 , 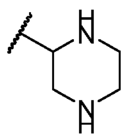 , 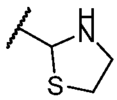 or 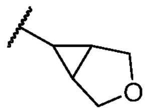

In a preferred embodiment, in $R^{2-3-2}$, the 5-10 membered heteroaryl is 5 or 6 membered heteroaryl, or 5-membered-fused 6-membered heteroaryl. The heteroatom in the 5-10 membered heteroaryl is N and/or O, and the number of heteroatom(s) is 1 or 2. The heteroatom in the 5 membered heteroaryl can be N and/or O, and the number of heteroatoms is 2. The heteroatom in the 6 membered heteroaryl can be N, and the number of heteroatom(s) is 1. The heteroatom in the 5-membered-fused 6-membered heteroaryl can be O, and the number of heteroatoms is 2.

In a preferred embodiment, in each $R^{2-3-3}$, the $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, for example, methyl or ethyl.

In a preferred embodiment, in each $R^{2-3-3}$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl.

In a preferred embodiment, in each $R^{2-3-4}$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be isopropyl.

In a preferred embodiment, in each $R^{2-3-5}$, the halogen can be fluorine, chlorine, bromine or iodine, for example, fluorine.

In a preferred embodiment, in each $R^{2-3-6}$, the $C_1$-$C_6$ alkyl can be $C_1$-$C_4$ alkyl, and can be methyl, ethyl, n-propyl, isopropyl, n-butyl, isobutyl, pr-butyl, sec-butyl or tert-butyl, for example, methyl.

In a preferred embodiment, in each $R^{2-3-7}$, the $C_1$-$C_6$ alkyl can be $C_1$-$C_4$ alkyl, and can be methyl, ethyl, n-propyl, isopropyl, n-butyl, isobutyl, pr-butyl, sec-butyl or tert-butyl, for example, methyl.

In a preferred embodiment, in each $R^{2-3-8}$, the $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, for example, methyl or isopropyl.

In a preferred embodiment, in each $R^{2-3-8}$, the $C_3$-$C_6$ cycloalkyl is cyclopropyl, cyclobutyl, cyclopentyl or cyclohexyl, for example, cyclopropyl.

In a preferred embodiment, in each $R^{2-3-9}$, the $C_1$-$C_6$ alkyl can be $C_1$-$C_4$ alkyl, and can be methyl, ethyl, n-propyl, isopropyl, n-butyl, isobutyl, pr-butyl, sec-butyl or tert-butyl, for example, methyl.

In a preferred embodiment, in each $R^{2-3-1-1}$, the halogen can be fluorine, chlorine, bromine or iodine, for example, fluorine.

In a preferred embodiment, in each $R^{2-3-1-2}$, the $C_1$-$C_6$ alkoxy is methoxy, ethoxy, n-propoxy, isopropoxy, n-butoxy, isobutoxy, pr-butoxy, sec-butoxy or tert-butoxy, for example, methoxy.

In a preferred embodiment, in each $R^{2-3-1-3}$, the $C_1$-$C_6$ alkyl can be $C_1$-$C_4$ alkyl, and can be methyl, ethyl, n-propyl, isopropyl, n-butyl, isobutyl, pr-butyl, sec-butyl or tert-butyl, for example, methyl.

In a preferred embodiment, in each $R^{2-3-2-1}$, the halogen can be fluorine, chlorine, bromine or iodine, for example, fluorine.

In a preferred embodiment, in each $R^{2-3-2-1}$, the $C_1$-$C_6$ alkoxy is methoxy, ethoxy, n-propoxy, isopropoxy, n-butoxy, isobutoxy, pr-butoxy, sec-butoxy or tert-butoxy, for example, methoxy.

In a preferred embodiment, in each $R^{2-3-2-1}$, the 5-10 membered heteroaryl is 5 membered heteroaryl. The heteroatom in the 5 membered heteroaryl can be N, and the number of heteroatoms is 3.

In a preferred embodiment, in each $R^{2-3-2-1-1}$, the $C_1$-$C_6$ alkyl can be $C_1$-$C_4$ alkyl, and can be methyl, ethyl, n-propyl, isopropyl, n-butyl, isobutyl, pr-butyl, sec-butyl or tert-butyl, for example, methyl.

In a preferred embodiment, in each $R^{2-3-2-1-1}$, the 6-10 membered aryl can be phenyl or naphthyl, for example, phenyl.

In a preferred embodiment, in each $R^{2-3-2-1-2}$, the $C_1$-$C_6$ alkyl can be $C_1$-$C_4$ alkyl, and can be methyl, ethyl, n-propyl, isopropyl, n-butyl, isobutyl, pr-butyl, sec-butyl or tert-butyl, for example, methyl.

In a preferred embodiment, in each $R^{2-3-2-1-3}$, the $C_1$-$C_6$ alkyl can be $C_1$-$C_4$ alkyl, and can be methyl, ethyl, n-propyl, isopropyl, n-butyl, isobutyl, pr-butyl, sec-butyl or tert-butyl, for example, methyl.

In a preferred embodiment, in each $R^{2-3-2-2}$, the halogen is fluorine, chlorine, bromine or iodine, for example, fluorine.

In a preferred embodiment, in each $R^{2-3-2-2}$, the $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl.

In a preferred embodiment, in each $R^{2-3-2-2}$, the $C_1$-$C_6$ alkoxy is methoxy, ethoxy, n-propoxy, isopropoxy, n-butoxy, isobutoxy, pr-butoxy, sec-butoxy or tert-butoxy, or can be methoxy, ethoxy or n-propoxy. In a preferred embodiment, in each $R^{2-3-2-2-1}$, the halogen is fluorine, chlorine, bromine or iodine, for example, fluorine.

In a preferred embodiment, in each $R^{2-3-2-2-2}$, the halogen is fluorine, chlorine, bromine or iodine, for example, fluorine.

In a preferred embodiment, in each $R^{2-3-2-2-3}$, the $C_1$-$C_6$ alkyl can be $C_1$-$C_4$ alkyl, and can be methyl, ethyl, n-propyl, isopropyl, n-butyl, isobutyl, pr-butyl, sec-butyl or tert-butyl, for example, methyl.

In a preferred embodiment, in each $R^{2-3-2-3-1}$, the 6-10 membered aryl is phenyl or naphthyl, for example, phenyl.

In a preferred embodiment, in each $R^{2-3-2-3-1-1}$, the $C_1$-$C_6$ alkoxy is methoxy, ethoxy, n-propoxy, isopropoxy, n-butoxy, isobutoxy, pr-butoxy, sec-butoxy or tert-butoxy, for example, methoxy.

In a preferred embodiment, in each $R^{2-3-2-4}$, the halogen is fluorine, chlorine, bromine or iodine, for example, fluorine.

In a preferred embodiment, in each $R^{2-3-2-5}$, the halogen is fluorine, chlorine, bromine or iodine, for example, fluorine.

In a preferred embodiment, in each $R^{2-3-2-5}$, the $C_1$-$C_6$ alkoxy is methoxy, ethoxy, n-propoxy, isopropoxy, n-butoxy, isobutoxy, pr-butoxy, sec-butoxy or tert-butoxy, for example, methoxy.

In a preferred embodiment, in each $R^{2-3-2-5}$, the $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, for example, methyl, ethyl, n-propyl or isopropyl.

In a preferred embodiment, in each $R^{2-3-2-5-1}$, the halogen is fluorine, chlorine, bromine or iodine, for example, fluorine.

In a preferred embodiment, in each $R^{2-3-2-6}$, the $C_1$-$C_6$ alkyl can be $C_1$-$C_4$ alkyl, and can be methyl, ethyl, n-propyl, isopropyl, n-butyl, isobutyl, pr-butyl, sec-butyl or tert-butyl, for example, methyl.

In a preferred embodiment, in each $R^{2-3-3-1-1}$, the $C_1$-$C_6$ alkyl can be $C_1$-$C_4$ alkyl, and can be methyl, ethyl, n-propyl, isopropyl, n-butyl, isobutyl, pr-butyl, sec-butyl or tert-butyl, for example, methyl.

In a preferred embodiment, in each $R^{2-4}$, the 4-10 membered heterocycloalkyl can be a monocyclic ring. The 4-10 membered heterocycloalkyl can be 4-6 membered heterocycloalkyl. The heteroatom in the 4-10 membered heterocycloalkyl is selected from N and/or O, and the number of heteroatom(s) is 1 or 2.

In a preferred embodiment, in each $R^{2-4}$, the 5-10 membered heteroaryl can be 5-membered-fused 6-membered heteroaryl. The heteroatom in the 5-membered-fused 6-membered heteroaryl can be selected from N and/or O, and the number of heteroatoms is 2, or the number of heteroatom(s) is 1, 2 or 3.

In a preferred embodiment, in each $R^{2-4}$, the 6-10 membered aryl can be phenyl or naphthyl, for example, phenyl.

In a preferred embodiment, in each $R^{2-4}$, the $C_3$-$C_6$ cycloalkyl can be cyclopropyl, cyclobutyl, cyclopentyl or cyclohexyl, for example, cyclopentyl.

In a preferred embodiment, in each $R^{2-4-1}$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl.

In a preferred embodiment, in each $R^{2-4-1}$, the halogen can be fluorine, chlorine, bromine or iodine, for example, fluorine.

In a preferred embodiment, in each $R^{2-4-2}$, the halogen can be fluorine, chlorine, bromine or iodine, for example, fluorine.

In a preferred embodiment, in each $R^{2-4-2}$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl.

In a preferred embodiment, in each $R^{2-4-3}$, the halogen can be fluorine, chlorine, bromine or iodine, for example, fluorine.

In a preferred embodiment, in each $R^{2-4-3}$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl.

In a preferred embodiment, in each $R^{2-4-4}$, the halogen can be fluorine, chlorine, bromine or iodine, for example, fluorine.

In a preferred embodiment, in each $R^{2-4-4}$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl.

In a preferred embodiment, in $R^{2-4-1-1}$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl or isopropyl.

In a preferred embodiment, in each $R^{2-4-1-2}$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl or isopropyl.

In a preferred embodiment, in each $R^{2-5}$, the 4-10 membered heterocycloalkyl can be a monocyclic ring. The 4-10 membered heterocycloalkyl can be 4-6 membered heterocycloalkyl. The heteroatom in the 4-10 membered heterocycloalkyl is selected from N and/or O, and the number of heteroatom(s) is 1 or 2.

In a preferred embodiment, in each $R^{2-5}$, the 6-10 membered aryl is phenyl or naphthyl, for example, phenyl.

In a preferred embodiment, in each $R^{2-5}$, the $C_1$-$C_6$ alkyl is methyl, ethyl, n-propyl, isopropyl, n-butyl, isobutyl, pr-butyl, sec-butyl or tert-butyl, for example, methyl.

In a preferred embodiment, in $R^{2-5-1-1}$, the $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl or isopropyl.

In a preferred embodiment, in $R^{2-5-1-1}$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl.

In a preferred embodiment, in each $R^{2-5-1-2}$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl or isopropyl.

In a preferred embodiment, in $R^{2-5-2}$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl or isopropyl.

In a preferred embodiment, in each $R^{2-5-3}$, the $C_1$-$C_6$ alkoxy is methoxy, ethoxy, n-propoxy, isopropoxy, n-butoxy, isobutoxy, pr-butoxy, sec-butoxy or tert-butoxy, for example, methoxy.

In a preferred embodiment, in each $R^{2-6}$, the halogen can be fluorine, chlorine, bromine or iodine, for example, fluorine.

In a preferred embodiment, in each $R^{2-6}$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl.

In a preferred embodiment, in each $R^{2-6}$, the 4-10 membered heterocycloalkyl can be a monocyclic ring. The 4-10 membered heterocycloalkyl can be 4-6 membered heterocycloalkyl. The heteroatom in the 4-10 membered heterocycloalkyl is selected from N and/or O, and the number of heteroatom(s) is 1 or 2.

In a preferred embodiment, in each $R^{2-6}$, the 5-10 membered heteroaryl can be 5-6 membered heteroaryl. The heteroatom in the 5-6 membered heteroaryl can be selected from N and/or S, and the number of heteroatoms is 2, or the number of heteroatom(s) is 1 or 2.

In a preferred embodiment, in each $R^{2-6-1}$, the halogen can be fluorine, chlorine, bromine or iodine, for example,

fluorine.

In a preferred embodiment, in each $R^{2-6-1}$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl.

In a preferred embodiment, in $R^{2-6-1-1}$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl.

In a preferred embodiment, in each $R^{2-6-2}$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl or isopropyl.

In a preferred embodiment, in each $R^{2-6-2}$, the halogen can be fluorine, chlorine, bromine or iodine, for example, fluorine.

In a preferred embodiment, in each $R^{2-7}$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl.

In a preferred embodiment, in each $R^{2-7}$, the 4-10 membered heterocycloalkyl can be a monocyclic or spiro ring. The monocyclic ring can be 4-6 membered heterocycloalkyl. The spiro ring can be 4 membered-spiro 6-membered ring, 4 membered-spiro 5-membered ring or 4 membered-spiro 4-membered ring. The heteroatom in the 4-10 membered heterocycloalkyl is selected from N and/or O, and the number of heteroatom(s) is 1 or 2.

In a preferred embodiment, in each $R^{2-7}$, the 5-10 membered heteroaryl can be 5 or 6 membered heteroaryl, or 5-membered-fused 6-membered heteroaryl. The heteroatom in the 5-10 membered heteroaryl is one or more selected from N and/or S, and the number of heteroatom(s) is 1 or 2. The heteroatom in the 5 membered heteroaryl can be selected from N and/or S, and the number of heteroatoms is 2. The heteroatom in the 6 membered heteroaryl can be N, and the number of heteroatom(s) is 1 or 2. The heteroatom in the 5-membered-fused 6-membered heteroaryl can be selected from N and/or O, and the number of heteroatoms is 2, or the number of heteroatom(s) is 1, 2 or 3.

In a preferred embodiment, in each $R^{2-7-1}$, the halogen can be fluorine, chlorine, bromine or iodine, for example, fluorine.

In a preferred embodiment, in each $R^{2-7-1}$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl.

In a preferred embodiment, in $R^{2-7-1-1}$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl.

In a preferred embodiment, in $R^{2-7-1-2}$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl.

In a preferred embodiment, in each $R^{2-7-2}$, the halogen can be fluorine, chlorine, bromine or iodine, for example, fluorine.

In a preferred embodiment, in each $R^{2-7-2}$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl.

In a preferred embodiment, in each $R^{2-8}$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl.

In a preferred embodiment, in each $R^{2-8}$, the 4-10 membered heterocycloalkyl can be a monocyclic ring. The monocyclic ring can be 4-6 membered heterocycloalkyl. The heteroatom in the 4-10 membered heterocycloalkyl is selected from N and/or O, and the number of heteroatom(s) is 1 or 2.

In a preferred embodiment, in each $R^{2-8-1-1}$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl.

In a preferred embodiment, in each $R^{2-9}$, the 5-10 membered heteroaryl is 5 membered heteroaryl; the heteroatom in the 5-10 membered heteroaryl is N and/or S, and the number of heteroatom(s) is 1 or 2; the heteroatom in the 5 membered heteroaryl can be N, and the number of heteroatoms is 2; pyrazolyl is preferred, for example,

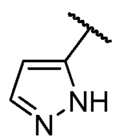

In a preferred embodiment, in each $R^{2-10}$, the 5-10 membered heteroaryl is 5 membered heteroaryl; the heteroatom in the 5-10 membered heteroaryl is N and/or S, and the number of heteroatom(s) is 1 or 2; the heteroatom in the 5 membered heteroaryl can be N, and the number of heteroatoms is 2; pyrazolyl is preferred, for example,

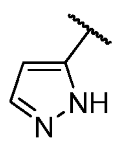

In a preferred embodiment, in each $R^{2-11}$, the 5-10 membered heteroaryl is 5 membered heteroaryl. The heteroatom in the 5 membered heteroaryl can be N and/or S, and the number of heteroatoms is 2; and thiazolyl or imidazolyl is preferred, for example,

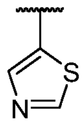 or 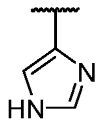

In a preferred embodiment, in each $R^{2-11}$, the heteroatom in the 3-6 membered heterocycloalkyl is O, and the number of heteroatom(s) can be 1; and oxacyclopentanyl is preferred, for example,

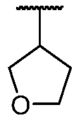

In a preferred embodiment, in each $R^{2-11-1}$, the $C_1$-$C_6$ alkyl can be $C_1$-$C_4$ alkyl, and can be methyl, ethyl, n-propyl, isopropyl, n-butyl, isobutyl, pr-butyl, sec-butyl or tert-butyl, for example, methyl.

In a preferred embodiment, in each $R^{2-11-2}$, the $C_1$-$C_6$ alkyl can be $C_1$-$C_4$ alkyl, and can be methyl, ethyl, n-propyl, isopropyl, n-butyl, isobutyl, pr-butyl, sec-butyl or tert-butyl, for example, methyl.

In a preferred embodiment, in $R^3$, the 5-10 membered heteroaryl is 5 membered heteroaryl, 5-membered-fused 5-membered heteroaryl, or 5-membered-fused 6-membered heteroaryl. The heteroatom in the 5-10 membered heteroaryl is N and/or S, and the number of heteroatom(s) is 1, 2, or 3. The heteroatom in the 5 membered heteroaryl can be N and/or S, and the number of heteroatom(s) is 2 or 3. The heteroatom in the 5-membered-fused 6-membered heteroaryl can be N and/or O, and the number of heteroatoms is 2, or the number of heteroatom(s) is 1, 2 or 3. The heteroatom in the 5-membered-fused 5-membered heteroaryl can be N and/or O, and the number of heteroatoms is 2, or the number of heteroatom(s) is 1, 2 or 3.

In a preferred embodiment, in $R^3$, the 5-10 membered heteroaryl can be 5 membered heteroaryl, 5-membered-fused 5-membered heteroaryl, or 5-membered-fused 6-membered heteroaryl. The heteroatom in the 5-10 membered heteroaryl is one or more selected from N and/or S, and the number of heteroatom(s) is 1 or 2. The heteroatom in the 5 membered heteroaryl can be selected from N and/or S, and the number of heteroatoms is 2. The heteroatom in the 5-membered-fused 6-membered heteroaryl can be selected from N and/or O, and the number of heteroatoms is 2, or the number of heteroatom(s) is 1, 2 or 3. The heteroatom in the 5-membered-fused 5-membered heteroaryl can be selected from N and/or O, and the number of heteroatoms is 2, or the number of heteroatom(s) is 1, 2 or 3.

In a preferred embodiment, in $R^3$, the 3-10 membered heterocycloalkyl can be a monocyclic ring. The monocyclic ring can be 4-6 membered heterocycloalkyl. The heteroatom in the 3-10 membered heterocycloalkyl is selected from N, and the number of heteroatom(s) is 1 or 2.

In a preferred embodiment, in each $R^{3-1}$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl.

In a preferred embodiment, in each $R^{3-1}$, the halogen can be fluorine, chlorine, bromine or iodine, for example, fluorine.

In a preferred embodiment, in each $R^{3-1-1}$, the halogen can be fluorine, chlorine, bromine or iodine, for example, fluorine.

In a preferred embodiment, in $R^{3-1-2}$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl or ethyl.

In a preferred embodiments, in $R^{3-1-2}$, the $C_2$-$C_6$ alkenyl can be

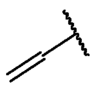, 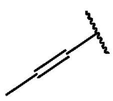, 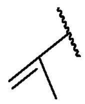 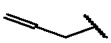,

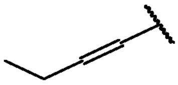, 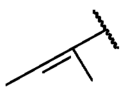, 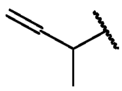 or 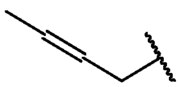,

for example,

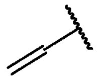

In a preferred embodiment, in $R^{3-2}$, the halogen can be fluorine, chlorine, bromine or iodine, for example, fluorine.

In a preferred embodiment, in $R^{3-2}$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl.

In a preferred embodiment, in $R^{3-2-1}$, the halogen can be fluorine, chlorine, bromine or iodine, for example, fluorine.

In a preferred embodiment, in $R^{3-2-2}$, the $C_1$-$C_6$ alkyl can be $C_1$-$C_4$ alkyl, and can be methyl, ethyl, n-propyl, isopropyl, n-butyl, isobutyl, pr-butyl, sec-butyl or tert-butyl, or can be methyl or ethyl.

In a preferred embodiment, in $R^{3-2-2}$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl.

In a preferred embodiment, in $R^{3-2-2}$, the $C_2$-$C_6$ alkenyl can be

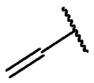, 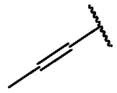, 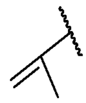 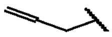,

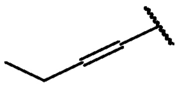, 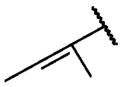, 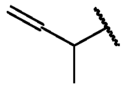 or 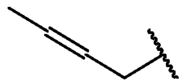

for example,

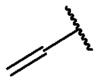

In a preferred embodiment, in $R^{4-2}$, the halogen is fluorine, chlorine, bromine or iodine, for example, fluorine or chlorine.

In a preferred embodiment, in $R^{4-2}$, the halogen can be fluorine, chlorine, bromine or iodine, for example, fluorine.

In a preferred embodiment, in $R^{4-7}$, the 4-10 membered heterocycloalkyl is a monocyclic or spiro ring. The monocyclic ring can be 6 membered heterocycloalkyl. The spiro ring can be 4 membered-spiro 6-membered ring, 4 membered-spiro 5-membered ring or 4 membered-spiro 4-membered ring. The heteroatom in the 4-10 membered heterocycloalkyl can be N, and the number of heteroatom(s) is 1 or 2.

In a preferred embodiment, in $R^4$, the 5-10 membered heterocycloalkyl can be a spiro ring. The spiro ring can be 4 membered-spiro 6-membered ring, 4 membered-spiro 5-membered ring or 4 membered-spiro 4-membered ring. The heteroatom in the 4-10 membered heterocycloalkyl can be N, and the number of heteroatom(s) is 1 or 2.

In a preferred embodiment, in $R^4$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl.

In a preferred embodiment, in each $R^{4-2}$, the 5-10 membered heteroaryl is 5 membered heteroaryl. The heteroatom in the 5 membered heteroaryl can be selected from N and/or S, and the number of heteroatoms is 2.

In a preferred embodiment, in $R^{4-1-1}$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl or isopropyl.

In a preferred embodiment, in each $R^{4-1-2}$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl.

In a preferred embodiment, in each $R^{4-2-1}$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl.

In a preferred embodiment, A is

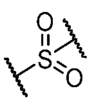, 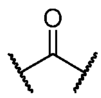 or 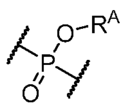;

$R^A$ is hydrogen or $C_1$-$C_6$ alkyl;

ring B is a 5-membered-fused 6-membered heteroaromatic ring unsubstituted or substituted by $R^B$; the heteroatom in the 5-membered-fused 6-membered heteroaromatic ring is one or more selected from N, O and S, and the number of heteroatom(s) is 1, 2 or 3; when the heteroatom in the 5-membered-fused 6-membered heteroaromatic ring is only nitrogen, in the 5-membered-fused 6-membered heteroaromatic ring, at least one of 6 members is N or 5 members comprise only one N; $R^B$ is each independently selected from oxo, hydroxyl, halogen or $C_1$-$C_6$ alkyl;

$R^1$ is hydroxyl, $C_1$-$C_6$ alkyl unsubstituted or substituted by one or more $R^{1-1}$, $C_3$-$C_6$ cycloalkyl unsubstituted or substituted by one or more $R^{1-2}$, or 3-6 membered heterocycloalkyl unsubstituted or substituted by one or more $R^{1-3}$; the heteroatom in the 3-6 membered heterocycloalkyl is one or more selected from N, O and S, and the number of heteroatom(s) is 1, 2 or 3;

$R^{1-1}$ is each independently selected from deuterium, hydroxyl, cyano, halogen or $C_3$-$C_6$ cycloalkyl;

$R^{1-2}$ is each independently selected from deuterium, hydroxyl, cyano, halogen, $C_1$-$C_6$ alkyl unsubstituted or substituted by one or more $R^{1-2-1}$, $C_2$-$C_6$ alkynyl, $C_1$-$C_6$ alkoxy unsubstituted or substituted by one or more $R^{1-2-3}$ or amino unsubstituted or substituted by one or two $R^{1-2-5}$; $R^{1-2-1}$ is each independently selected from halogen, hydroxyl or $C_1$-$C_6$ alkoxy unsubstituted or substituted by one or more $R^{1-2-1-1}$; $R^{1-2-1-1}$ is each independently halogen; $R^{1-2-3}$ is each independently halogen; $R^{1-2-5}$ is each independently selected from $C_1$-$C_6$ alkyl,

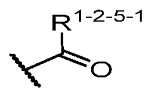

or $C_3$-$C_6$ cycloalkyl; $R^{1-2-5-1}$ is $C_1$-$C_6$ alkyl or amino;

$R^{1-3}$ is each independently $C_1$-$C_6$ alkyl,

or, the atom in $R^1$ is attached to the atom in A to form 5-6 membered heterocycloalkyl unsubstituted or substituted by one or more $R^0$; the heteroatom in the 5-6 membered heterocycloalkyl is one or more selected from N, O, P and S, and the number of heteroatom(s) is 3, 4 or 5; $R^0$ is each independently selected from $C_1$-$C_6$ alkyl or oxo;

$R^2$ is hydrogen, 6-10 membered aryl unsubstituted or substituted by one or more $R^{2-1}$, 5-10 membered heteroaryl unsubstituted or substituted by one or more $R^{2-2}$, 4-10 membered heterocycloalkyl unsubstituted or substituted by one or more $R^{2-}$

3, 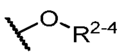 , 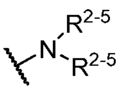 ,

$C_1$-$C_6$ alkyl unsubstituted or substituted by one or more $R^{2-6}$,

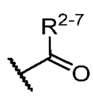 or 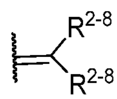 ;

the heteroatom in the 5-10 membered heteroaryl is one or more selected from N, O and S, and the number of heteroatom(s) is 1, 2 or 3; the heteroatom in the 4-10 membered heterocycloalkyl is one or more selected from N, O and S, and the number of heteroatom(s) is 1, 2 or 3,

$R^{2-1}$ is each independently selected from cyano, halogen, $C_1$-$C_6$ alkyl unsubstituted or substituted by one or more $R^{2-1-}$

1, 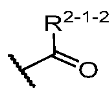 , 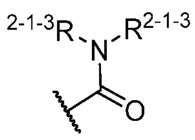

or $C_1$-$C_6$ alkoxy unsubstituted or substituted by one or more $R^{2-1-4}$;

$R^{2-1-1}$ is each independently selected from cyano or halogen; $R^{2-1-2}$ is hydrogen or $C_1$-$C_6$ alkyl; $R^{2-1-3}$ is each independently selected from hydrogen or $C_1$-$C_6$ alkyl; $R^{2-1-4}$ is each independently halogen;

$R^{2-2}$ is each independently selected from cyano, halogen, $C_1$-$C_6$ alkyl unsubstituted or substituted by one or more $R^{2-2-}$

1, 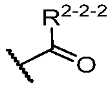 or 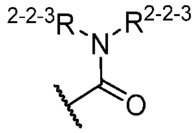 ;

$R^{2-2-1}$ is each independently selected from cyano or halogen; $R^{2-2-2}$ is hydrogen or $C_1$-$C_6$ alkyl; $R^{2-2-3}$ is each independently selected from hydrogen or $C_1$-$C_6$ alkyl;
$R^{2-3}$ is each independently selected from cyano, oxo, hydroxyl, halogen, $C_1$-$C_6$ alkyl unsubstituted or substituted by one or more $R^{2-3-1}$,

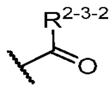 , 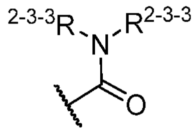 ,

3-6 membered heterocycloalkyl unsubstituted or substituted by one or more $R^{2-3-4}$, $C_1$-$C_6$ alkoxy unsubstituted or substituted by one or more $R^{2-3-5}$; the heteroatom in the 3-6 membered heterocycloalkyl is one or more selected from N, O and S, and the number of heteroatom(s) is 1, 2 or 3;
$R^{2-3-1}$ is each independently selected from cyano, halogen, or $C_1$-$C_6$ alkoxy unsubstituted or substituted by one or more $R^{2-3-1-1}$;
$R^{2-3-2}$ is hydrogen or $C_1$-$C_6$ alkyl unsubstituted or substituted by one or more $R^{2-3-2-1}$;
$R^{2-3-3}$ is each independently hydrogen or $C_1$-$C_6$ alkyl;
$R^{2-3-4}$ is each independently $C_1$-$C_6$ alkyl;
$R^{2-3-1}$ is each independently halogen;
$R^{2-3-1-1}$ is each independently halogen;
$R^{2-3-2-1}$ is each independently halogen;
各 $R^{2-4}$ is 4-10 membered heterocycloalkyl unsubstituted or substituted by one or more $R^{2-4-1}$, 5-10 membered heteroaryl unsubstituted or substituted by one or more $R^{2-4-2}$, 6-10 membered aryl unsubstituted or substituted by one or more $R^{2-4-3}$ or $C_3$-$C_6$ cycloalkyl unsubstituted or substituted by one or more $R^{2-4-4}$; the heteroatom in the 4-10 membered heterocycloalkyl is one or more selected from N, O and S, and the number of heteroatom(s) is 1, 2 or 3;
$R^{2-4-1}$ is each independently selected from hydroxyl, halogen, $C_1$-$C_6$ alkyl,

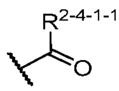 or 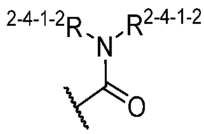 ;

$R^{2-4-2}$ is each independently selected from hydroxyl, halogen or $C_1$-$C_6$ alkyl; $R^{2-4-3}$ is each independently selected from hydroxyl, halogen or $C_1$-$C_6$ alkyl; $R^{2-4-4}$ is each independently selected from hydroxyl, halogen or $C_1$-$C_6$ alkyl; $R^{2-4-1-1}$ is $C_1$-$C_6$ alkyl; each $R^{2-4-1-2}$ is $C_1$-$C_6$ alkyl;
$R^{2-5}$ is each independently hydrogen, 4-10 membered heterocycloalkyl unsubstituted or substituted by one or more $R^{2-5-1}$, or

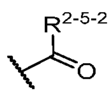 ;

the heteroatom in the 4-10 membered heteroaryl is one or more selected from N, O and S, and the number of heteroatom(s) is 1, 2 or 3;
$R^{2-5-1}$ is each independently selected from

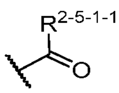 or 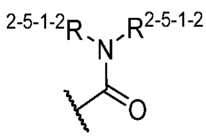 ;

$R^{2-5-1-1}$ is hydrogen or $C_1$-$C_6$ alkyl;
$R^{2-5-1-2}$ is each independently selected from hydrogen or $C_1$-$C_6$ alkyl;
$R^{2-5-2}$ is $C_1$-$C_6$ alkyl;
$R^{2-6}$ is each independently selected from halogen, $C_1$-$C_6$ alkyl, 4-10 membered heterocycloalkyl unsubstituted or substituted by one or more $R^{2-6-1}$ or 5-10 membered heteroaryl unsubstituted or substituted by one or more $R^{2-6-2}$; the heteroatom in the 4-10 membered heterocycloalkyl is one or more selected from N, O and S, and the number of heteroatom(s) is 1, 2 or 3; the heteroatom in the 5-10 membered heteroaryl is one or more selected from N, O and S, and the number of heteroatom(s) is 1, 2 or 3;
$R^{2-6-1}$ is each independently selected from halogen, $C_1$-$C_6$ alkyl or

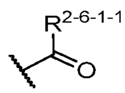
;

$R^{2-6-1-1}$ is $C_1$-$C_6$ alkyl;
$R^{2-6-2}$ is each independently selected from halogen or $C_1$-$C_6$ alkyl;
$R^{2-7}$ is $C_1$-$C_6$ alkyl, 4-10 membered heterocycloalkyl unsubstituted or substituted by one or more $R^{2-7-1}$ or 5-10 membered heteroaryl unsubstituted or substituted by one or more $R^{2-7-2}$; the heteroatom in the 4-10 membered heterocycloalkyl is one or more selected from N, O and S, and the number of heteroatom(s) is 1, 2 or 3; the heteroatom in the 5-10 membered heteroaryl is one or more selected from N, O and S, and the number of heteroatom(s) is 1, 2 or 3;
$R^{2-7-1}$ is each independently selected from halogen, $C_1$-$C_6$ alkyl,

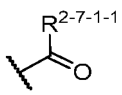
or
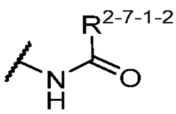
;

$R^{2-7-1-1}$ is $C_1$-$C_6$ alkyl; $R^{2-7-1-2}$ is $C_1$-$C_6$ alkyl;
$R^{2-7-2}$ is each independently selected from halogen or $C_1$-$C_6$ alkyl;
$R^{2-8}$ is each independently $C_1$-$C_6$ alkyl, or two $R^{2-8}$, together with the atom to which they are attached, form 4-10 membered heterocycloalkyl unsubstituted or substituted by one or more $R^{2-8-1}$; the heteroatom in the 4-10 membered heterocycloalkyl is one or more selected from N, O and S, and the number of heteroatom(s) is 1, 2 or 3;
$R^{2-8-1}$ is each independently

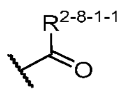
;

and $R^{2-8-1-1}$ is hydrogen or $C_1$-$C_6$ alkyl.
$R^3$ is 5-10 membered heteroaryl unsubstituted or substituted by one or more $R^{3-1}$ or 3-10 membered heterocycloalkyl unsubstituted or substituted by one or more $R^{3-2}$, the heteroatom in the 5-10 membered heteroaryl is one or more selected from N, O and S, and the number of heteroatom(s) is 1, 2 or 3; the heteroatom in the 3-10 membered heterocycloalkyl is one or more selected from N, O and S, and the number of heteroatom(s) is 1, 2 or 3;
$R^{3-1}$ is each independently selected from hydroxyl, halogen, $C_1$-$C_6$ alkyl unsubstituted or substituted by one or more $R^{3-1-1}$;
$R^{3-1-1}$ is each independently halogen;
$R^{3-2}$ is each independently selected from hydroxyl, halogen, $C_1$-$C_6$ alkyl unsubstituted or substituted by one or more $R^{3-2-1}$ or

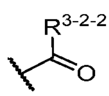
;

$R^{3-2-1}$ is each independently halogen; $R^{3-2-2}$ is $C_1$-$C_6$ alkyl or $C_2$-$C_4$ alkenyl;
$R^4$ is hydrogen, halogen, 5-10 membered heterocycloalkyl unsubstituted or substituted by one or more $R^{4-1}$ or $C_1$-$C_6$ alkyl unsubstituted or substituted by one or more $R^{4-2}$; the heteroatom in the 5-10 membered heterocycloalkyl is one or more selected from N, O and S, and the number of heteroatom(s) is 1, 2 or 3;
$R^{4-1}$ is each independently

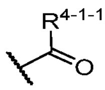 or 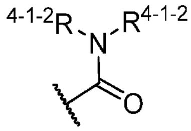 ;

$R^{4-2}$ is each independently 5-10 membered heteroaryl unsubstituted or substituted by one or more $R^{4-2-1}$; the heteroatom in the 5-10 membered heteroaryl is one or more selected from N, O and S, and the number of heteroatom(s) is 1, 2 or 3; and

$R^{4-1-1}$ is $C_1$-$C_6$ alkyl; $R^{4-1-2}$ is each independently $C_1$-$C_6$ alkyl; $R^{4-2-1}$ is each independently $C_1$-$C_6$ alkyl.

In a preferred embodiment, Y is C or N, X is C or N, and at least one of X and Y is N;

W is N, CH or $CR^4$;

Z is CH or N;

A is

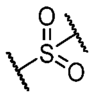 , 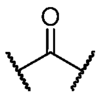 or 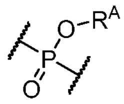 ;

$R^A$ is hydrogen or $C_1$-$C_6$ alkyl;

$R^1$ is hydroxyl, $C_1$-$C_6$ alkyl unsubstituted or substituted by one or more $R^{1-1}$, $C_3$-$C_6$ cycloalkyl unsubstituted or substituted by one or more $R^{1-2}$, or 3-6 membered heterocycloalkyl unsubstituted or substituted by one or more $R^{1-3}$; the heteroatom in the 3-6 membered heterocycloalkyl is one or more selected from N, O and S, and the number of heteroatom(s) is 1, 2 or 3;

$R^{1-1}$ is each independently selected from deuterium, hydroxyl, cyano, halogen or $C_3$-$C_6$ cycloalkyl;

$R^{1-2}$ is each independently selected from deuterium, hydroxyl, cyano, halogen, $C_1$-$C_6$ alkyl unsubstituted or substituted by one or more $R^{1-2-1}$, $C_2$-$C_6$ alkynyl, $C_1$-$C_6$ alkoxy unsubstituted or substituted by one or more $R^{1-2-3}$ or amino unsubstituted or substituted by one or two $R^{1-2-5}$; $R^{1-2-1}$ is each independently selected from halogen, hydroxyl or $C_1$-$C_6$ alkoxy unsubstituted or substituted by one or more $R^{1-2-1-1}$; $R^{1-2-1-1}$ is each independently halogen; $R^{1-2-3}$ is each independently halogen; $R^{1-2-5}$ is each independently selected from $C_1$-$C_6$ alkyl,

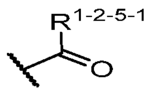

or $C_3$-$C_6$ cycloalkyl; $R^{1-2-5-1}$ is $C_1$-$C_6$ alkyl or amino;

$R^{1-3}$ is each independently $C_1$-$C_6$ alkyl;

or, the atom in $R^1$ is attached to the atom in A to form 5-6 membered heterocycloalkyl unsubstituted or substituted by one or more $R^0$; the heteroatom in the 5-6 membered heterocycloalkyl is one or more selected from N, O, P and S, and the number of heteroatom(s) is 3, 4 or 5; $R^0$ is each independently selected from $C_1$-$C_6$ alkyl or oxo;

$R^2$ is hydrogen, 6-10 membered aryl unsubstituted or substituted by one or more $R^{2-1}$, 5-10 membered heteroaryl unsubstituted or substituted by one or more $R^{2-2}$, 4-10 membered heterocycloalkyl unsubstituted or substituted by one or more $R^{2-3}$, 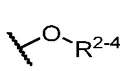 , 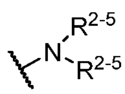 ,

$C_1$-$C_6$ alkyl unsubstituted or substituted by one or more $R^{2-6}$,

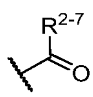 or 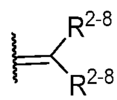 ;

the heteroatom in the 5-10 membered heteroaryl is one or more selected from N, O and S, and the number of heteroatom(s) is 1, 2 or 3; the heteroatom in the 4-10 membered heterocycloalkyl is one or more selected from N, O and S, and the number of heteroatom(s) is 1, 2 or 3,

$R^{2-1}$ is each independently selected from cyano, halogen, $C_1$-$C_6$ alkyl unsubstituted or substituted by one or more $R^{2-1-}$

1, 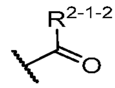 , 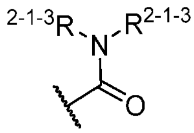

or $C_1$-$C_6$ alkoxy unsubstituted or substituted by one or more $R^{2-1-4}$;

$R^{2-1-1}$ is each independently selected from cyano or halogen; $R^{2-1-2}$ is hydrogen or $C_1$-$C_6$ alkyl; $R^{2-1-3}$ is each independently selected from hydrogen or $C_1$-$C_6$ alkyl; $R^{2-1-4}$ is each independently halogen;

$R^{2-2}$ is each independently selected from cyano, halogen, $C_1$-$C_6$ alkyl unsubstituted or substituted by one or more $R^{2-2-}$

1, 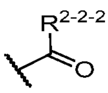 or 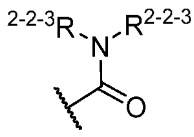 ;

$R^{2-2-1}$ is each independently selected from cyano or halogen; $R^{2-2-2}$ is hydrogen or $C_1$-$C_6$ alkyl; $R^{2-2-3}$ is each independently selected from hydrogen or $C_1$-$C_6$ alkyl;

$R^{2-3}$ is each independently selected from cyano, oxo, hydroxyl, halogen, $C_1$-$C_6$ alkyl unsubstituted or substituted by one or more $R^{2-3-1}$,

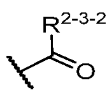 , 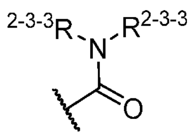 ,

3-6 membered heterocycloalkyl unsubstituted or substituted by one or more $R^{2-3-4}$, $C_1$-$C_6$ alkoxy unsubstituted or substituted by one or more $R^{2-3-5}$; the heteroatom in the 3-6 membered heterocycloalkyl is one or more selected from N, O and S, and the number of heteroatom(s) is 1, 2 or 3;

$R^{2-3-1}$ is each independently selected from cyano, halogen, or $C_1$-$C_6$ alkoxy unsubstituted or substituted by one or more $R^{2-3-1-1}$;

$R^{2-3-2}$ is hydrogen or $C_1$-$C_6$ alkyl unsubstituted or substituted by one or more $R^{2-3-2-1}$;

$R^{2-3-3}$ is each independently hydrogen or $C_1$-$C_6$ alkyl;

$R^{2-3-4}$ is each independently $C_1$-$C_6$ alkyl;

$R^{2-3-5}$ is each independently halogen;

$R^{2-3-1-1}$ is each independently halogen;

$R^{2-3-2-1}$ is each independently halogen;

each $R^{2-4}$ is 4-10 membered heterocycloalkyl unsubstituted or substituted by one or more $R^{2-4-1}$, 5-10 membered heteroaryl unsubstituted or substituted by one or more $R^{2-4-2}$, 6-10 membered aryl unsubstituted or substituted by one or more $R^{2-4-3}$ or $C_3$-$C_6$ cycloalkyl unsubstituted or substituted by one or more $R^{2-4-4}$; the heteroatom in the 4-10 membered heterocycloalkyl is one or more selected from N, O and S, and the number of heteroatom(s) is 1, 2 or 3; the heteroatom in the 5-10 membered heteroaryl is one or more selected from N, O and S, and the number of heteroatom(s) is 1, 2 or 3;

$R^{2-4-1}$ is each independently selected from hydroxyl, halogen, $C_1$-$C_6$ alkyl,

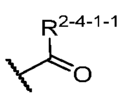 or 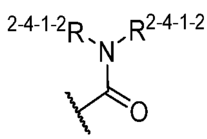 ;

$R^{2-4-2}$ is each independently selected from hydroxyl, halogen or $C_1$-$C_6$ alkyl; $R^{2-4-3}$ is each independently selected from hydroxyl, halogen or $C_1$-$C_6$ alkyl; $R^{2-4-4}$ is each independently selected from hydroxyl, halogen or $C_1$-$C_6$ alkyl; $R^{2-4-1-1}$ is $C_1$-$C_6$ alkyl; $R^{2-4-1-2}$ is $C_1$-$C_6$ alkyl;

$R^{2-5}$ is each independently hydrogen, 4-10 membered heterocycloalkyl unsubstituted or substituted by one or more $R^{2-5-1}$, or

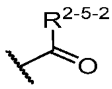;

the heteroatom in the 4-10 membered heteroaryl is one or more selected from N, O and S, and the number of heteroatom(s) is 1, 2 or 3;

$R^{2-5-1}$ is each independently selected from

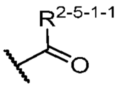 or 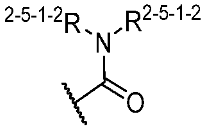;

$R^{2-5-1-1}$ is hydrogen or $C_1$-$C_6$ alkyl;

$R^{2-5-1-2}$ is each independently selected from hydrogen or $C_1$-$C_6$ alkyl;

$R^{2-5-2}$ is $C_1$-$C_6$ alkyl;

$R^{2-6}$ is each independently selected from halogen, $C_1$-$C_6$ alkyl, 4-10 membered heterocycloalkyl unsubstituted or substituted by one or more $R^{2-6-1}$ or 5-10 membered heteroaryl unsubstituted or substituted by one or more $R^{2-6-2}$; the heteroatom in the 4-10 membered heterocycloalkyl is one or more selected from N, O and S, and the number of heteroatom(s) is 1, 2 or 3; the heteroatom in the 5-10 membered heteroaryl is one or more selected from N, O and S, and the number of heteroatom(s) is 1, 2 or 3;

$R^{2-6-1}$ is each independently selected from halogen, $C_1$-$C_6$ alkyl or

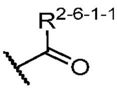;

$R^{2-6-1-1}$ is $C_1$-$C_6$ alkyl;

$R^{2-6-2}$ is each independently selected from halogen or $C_1$-$C_6$ alkyl;

$R^{2-7}$ is $C_1$-$C_6$ alkyl, 4-10 membered heterocycloalkyl unsubstituted or substituted by one or more $R^{2-7-1}$ or 5-10 membered heteroaryl unsubstituted or substituted by one or more $R^{2-7-2}$; the heteroatom in the 4-10 membered heterocycloalkyl is one or more selected from N, O and S, and the number of heteroatom(s) is 1, 2 or 3; the heteroatom in the 5-10 membered heteroaryl is one or more selected from N, O and S, and the number of heteroatom(s) is 1, 2 or 3;

$R^{2-7-1}$ is each independently selected from halogen, $C_1$-$C_6$ alkyl,

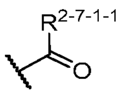 or 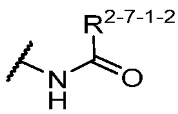;

$R^{2-7-1-1}$ is $C_1$-$C_6$ alkyl; $R^{2-7-1-2}$ is $C_1$-$C_6$ alkyl;

$R^{2-7-2}$ is each independently selected from halogen or $C_1$-$C_6$ alkyl;

$R^{2-8}$ is each independently $C_1$-$C_6$ alkyl, or two $R^{2-8}$, together with the atom to which they are attached, form 4-10 membered heterocycloalkyl unsubstituted or substituted by one or more $R^{2-8-1}$; the heteroatom in the 4-10 membered heterocycloalkyl is one or more selected from N, O and S, and the number of heteroatom(s) is 1, 2 or 3;

$R^{2-8-1}$ is each independently

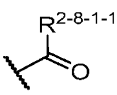;

and $R^{2-8-1-1}$ is hydrogen or $C_1$-$C_6$ alkyl.

$R^3$ is 5-10 membered heteroaryl unsubstituted or substituted by one or more $R^{3-1}$ or 3-10 membered heterocycloalkyl unsubstituted or substituted by one or more $R^{3-2}$; the heteroatom in the 5-10 membered heteroaryl is one or more

selected from N, O and S, and the number of heteroatom(s) is 1, 2 or 3; the heteroatom in the 3-10 membered heterocycloalkyl is one or more selected from N, O and S, and the number of heteroatom(s) is 1, 2 or 3;
$R^{3-1}$ is each independently selected from hydroxyl, halogen, $C_1$-$C_6$ alkyl unsubstituted or substituted by one or more $R^{3-1-1}$;
$R^{3-1-1}$ is each independently halogen;
$R^{3-2}$ is each independently selected from hydroxyl, halogen, $C_1$-$C_6$ alkyl unsubstituted or substituted by one or more $R^{3-2-1}$ or

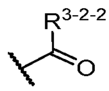
;

$R^{3-2-1}$ is each independently halogen; $R^{3-2-2}$ is $C_1$-$C_6$ alkyl or $C_2$-$C_4$ alkenyl;
$R^4$ is hydrogen, halogen, 5-10 membered heterocycloalkyl unsubstituted or substituted by one or more $R^{4-1}$ or $C_1$-$C_6$ alkyl unsubstituted or substituted by one or more $R^{4-2}$; the heteroatom in the 5-10 membered heterocycloalkyl is one or more selected from N, O and S, and the number of heteroatom(s) is 1, 2 or 3;
$R^{4-1}$ is each independently

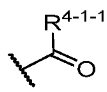
or
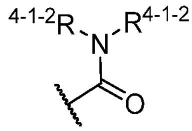
;

$R^{4-2}$ is each independently 5-10 membered heteroaryl unsubstituted or substituted by one or more $R^{4-1-1}$, the heteroatom in the 5-10 membered heteroaryl is one or more selected from N, O and S, and the number of heteroatom(s) is 1, 2 or 3; and
$R^{4-1-1}$ is $C_1$-$C_6$ alkyl; $R^{4-1-2}$ is each independently $C_1$-$C_6$ alkyl; $R^{4-2-1}$ is each independently $C_1$-$C_6$ alkyl.
In an embodiment, $R^1$ is $C_3$-$C_6$ cycloalkyl unsubstituted or substituted by one or more $R^{1-2}$ or 3-6 membered heterocycloalkylunsubstituted or substituted by one or more $R^{1-3}$; the heteroatom in the 3-6 membered heterocycloalkyl is N, O or S, and the number of heteroatom(s) is 1;
$R^{1-2}$ is each independently selected from cyano or $C_1$-$C_6$ alkyl unsubstituted or substituted by one or more $R^{1-2-1}$;
$R^{1-2-1}$ is each independently halogen;
$R^{1-3}$ is each independently $C_1$-$C_6$ alkyl unsubstituted or substituted by one or more $R^{1-3-1}$; $R^{1-3-1}$ is each independently halogen;
$R^2$ is hydrogen or 4-10 membered heterocycloalkyl unsubstituted or substituted by one or more $R^{2-3}$; the heteroatom in the 4-10 membered heterocycloalkyl is N, O or S, and the number of heteroatom(s) is 1 or 2;
$R^{2-3}$ is each independently

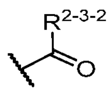
;

$R^{2-3-2}$ is $C_1$-$C_6$ alkyl;
$R^3$ is 5-10 membered heteroaryl unsubstituted or substituted by one or more $R^{3-1}$ or 3-10 membered heterocycloalkyl unsubstituted or substituted by one or more $R^{5-2}$; the heteroatom in the 5-10 membered heteroaryl is one or more selected from N, O and S, and the number of heteroatom(s) is 1, 2 or 3; the heteroatom in the 5-10 membered heterocycloalkyl is N, O and S, and the number of heteroatom(s) is 1 or 2;
$R^{3-1}$ is each independently selected from hydroxyl, halogen, $C_1$-$C_6$ alkyl unsubstituted or substituted by one or more $R^{3-1-1}$;
$R^{3-1-1}$ is each independently halogen;
$R^{3-2}$ is each independently


;

$R^{3-2-2}$ is $C_1$-$C_6$ alkyl or $C_2$-$C_4$ alkenyl;

$R^4$ is 5-10 membered heterocycloalkyl unsubstituted or substituted by one or more $R^{4-1}$; the heteroatom in the 5-10 membered heterocycloalkyl is N, O or S, and the number of heteroatom(s) is 1, 2 or 3;
$R^{4-1}$ is each independently

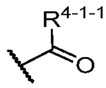 or 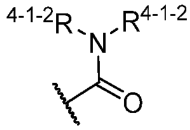 ;

$R^{4-1-1}$ is $C_1$-$C_6$ alkyl;
$R^{4-1-2}$ is each independently $C_1$-$C_6$ alkyl.

In an embodiment, $R^1$ is $C_3$-$C_6$ cycloalkyl or 4 membered heterocycloalkyl, unsubstituted or substituted by one or more $R^{1-2}$; the heteroatom in the 4 membered heterocycloalkyl is O, and the number of heteroatom(s) is 1;

$R^{1-2}$ is each independently selected from cyano or $C_1$-$C_6$ alkyl unsubstituted or substituted by one or more $R^{1-2-1}$;
$R^{1-2-1}$ is each independently halogen;
$R^2$ is hydrogen or 6-10 membered heterocycloalkyl unsubstituted or substituted by one or more $R^{2-3}$; the heteroatom in the 6-10 membered heterocycloalkyl is N, and the number of heteroatom(s) is 1 or 2;
$R^{2-3}$ is each independently

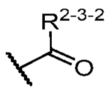 ;

$R^{2-3-2}$ is $C_1$-$C_6$ alkyl;
$R^3$ is 5-6 membered heteroaryl unsubstituted or substituted by one or more $R^{3-1}$ or 5-6 membered heterocycloalkyl unsubstituted or substituted by one or more $R^{3-2}$; the heteroatom in the 5-6 membered heteroaryl is N and/or S, and the number of heteroatom(s) is 1, 2 or 3; the heteroatom in the 5-10 membered heterocycloalkyl is N, and the number of heteroatom(s) is 1;
$R^{3-1}$ is each independently selected from halogen, or $C_1$-$C_6$ alkoxy unsubstituted or substituted by one or more $R^{3-1-1}$;
$R^{3-1-1}$ is each independently halogen;
$R^{3-2}$ is each independently

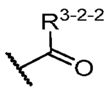 ;

$R^{3-2-2}$ is $C_2$-$C_4$ alkenyl;
$R^4$ is 5-10 membered heterocycloalkyl unsubstituted or substituted by one or more $R^{4-1}$; the heteroatom in the 5-10 membered heterocycloalkyl is N, and the number of heteroatom(s) is 1 or 2;
$R^{4-1}$ is each independently

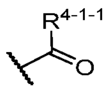 or 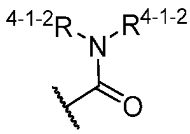 ;

$R^{4-1-1}$ is $C_1$-$C_6$ alkyl;
$R^{4-1-2}$ is each independently $C_1$-$C_6$ alkyl.
In an embodiment, $R^1$ is $C_3$-$C_6$ cycloalkyl unsubstituted or substituted by one or more $R^{1-2}$;
$R^{1-2}$ is each independently selected from cyano or $C_1$-$C_6$ alkyl unsubstituted or substituted by one or more $R^{1-2-1}$;
$R^{1-2-1}$ is each independently halogen;
$R^2$ is hydrogen or 6-10 membered heterocycloalkyl unsubstituted or substituted by one or more $R^{2-3}$; the heteroatom in the 6-10 membered heterocycloalkyl is N, and the number of heteroatom(s) is 1 or 2;
$R^{2-3}$ is each independently

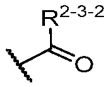

;

$R^{2-3-2}$ is $C_1$-$C_6$ alkyl;
$R^3$ is 5-6 membered heteroaryl unsubstituted or substituted by one or more $R^{3-1}$; the heteroatom in the 5-6 membered heteroaryl is N and/or S, and the number of heteroatoms is 3;
$R^{3-1}$ is each independently selected from halogen, or $C_1$-$C_6$ alkoxy unsubstituted or substituted by one or more $R^{3-1-1}$;
$R^{3-1-1}$ is each independently halogen;
$R^4$ is 7-9 membered heterocycloalkyl unsubstituted or substituted by one or more $R^{4-1}$; the heteroatom in the 7-9 membered heterocycloalkyl is N, and the number of heteroatom(s) is 1;
$R^{4-1}$ is each independently

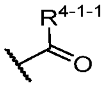

or

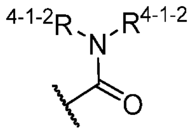

;

$R^{4-1-1}$ is $C_1$-$C_6$ alkyl;
$R^{4-1-2}$ is each independently $C_1$-$C_6$ alkyl.

In an embodiment, $R^1$ is $C_3$-$C_6$ cycloalkyl unsubstituted or substituted by one or more $R^{1-2}$ or 3-6 membered heterocycloalkylunsubstituted or substituted by one or more $R^{1-3}$; and the heteroatom in the 3-6 membered heterocycloalkyl is N, O or S, and the number of heteroatom(s) is 1.
In an embodiment, $R^{1-2}$ is each independently selected from cyano, or $C_1$-$C_6$ alkyl unsubstituted or substituted by one or more $R^{1-2-1}$
In an embodiment, $R^{1-2-1}$ is each independently halogen.
In an embodiment, $R^{13}$ is each independently $C_1$-$C_6$ alkyl unsubstituted or substituted by one or more $R^{1-3-1}$
In an embodiment, $R^{1-3-1}$ is each independently halogen.
In an embodiment, $R^2$ is hydrogen or 4-10 membered heterocycloalkyl unsubstituted or substituted by one or more $R^{2-3}$; the heteroatom in the 4-10 membered heterocycloalkyl is N, O or S, and the number of heteroatom(s) is 1 or 2.
In an embodiment, $R^{2-3}$ is each independently

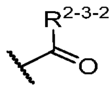

In an embodiment, $R^{2-3-2}$is $C_1$-$C_6$ alkyl.
In an embodiment, $R^3$ is 5-10 membered heteroaryl unsubstituted or substituted by one or more $R^{3-1}$ or 3-10 membered heterocycloalkyl unsubstituted or substituted by one or more $R^{5-2}$; the heteroatom in the 5-10 membered heteroaryl is one or more selected from N, O and S, and the number of heteroatom(s) is 1, 2 or 3; and the heteroatom in the 5-10 membered heterocycloalkyl is N, O and S, and the number of heteroatom(s) is 1 or 2.
In an embodiment, $R^{3-1}$ is each independently selected from hydroxyl, halogen or $C_1$-$C_6$ alkyl unsubstituted or substituted by one or more $R^{3-1-1}$
In an embodiment, $R^{3-1-1}$ is each independently halogen.
In an embodiment, $R^{3-2}$ is each independently

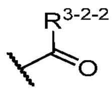

In an embodiment, $R^{3-2-2}$ is $C_1$-$C_6$ alkyl or $C_2$-$C_4$ alkenyl.
In an embodiment, $R^4$ is 5-10 membered heterocycloalkyl unsubstituted or substituted by one or more $R^{4-1}$, and the heteroatom in the 5-10 membered heterocycloalkyl is N, O or S, and the number of heteroatom(s) is 1, 2 or 3.
In an embodiment, $R^{4-1}$ is each independently

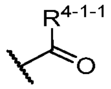 or 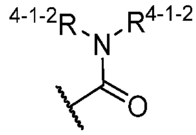

In an embodiment, $R^{4-1-1}$is $C_1$-$C_6$ alkyl.

In an embodiment, $R^{4-1-2}$ is each independently $C_1$-$C_6$ alkyl.

In an embodiment, $R^1$ is $C_3$-$C_6$ cycloalkyl or 4 membered heterocycloalkyl, unsubstituted or substituted by one or more $R^{1-2}$; and the heteroatom in the 4 membered heterocycloalkyl is O, and the number of heteroatom(s) is 1.

In an embodiment, $R^2$ is hydrogen or 6-10 membered heterocycloalkyl unsubstituted or substituted by one or more $R^{2-3}$; and the heteroatom in the 6-10 membered heterocycloalkyl is N, and the number of heteroatom(s) is 1 or 2.

In an embodiment, $R^3$ is 5-6 membered heteroaryl unsubstituted or substituted by one or more $R^{3-1}$ or 5-6 membered heterocycloalkyl unsubstituted or substituted by one or more $R^{3-2}$; the heteroatom in the 5-6 membered heteroaryl is N and/or S, and the number of heteroatom(s) is 1, 2 or 3; the heteroatom in the 5-10 membered heterocycloalkyl is N, and the number of heteroatom(s) is 1.

In an embodiment, $R^{3-2-2}$ is $C_2$-$C_4$ alkenyl.

In an embodiment, $R^4$ is 5-10 membered heterocycloalkyl unsubstituted or substituted by one or more $R^{4-1}$; and the heteroatom in the 5-10 membered heterocycloalkyl is N, and the number of heteroatom(s) is 1 or 2.

In an embodiment, $R^1$ is $C_3$-$C_6$ cycloalkyl unsubstituted or substituted by one or more $R^{1-2}$.

In an embodiment, $R^2$ is hydrogen or 6-10 membered heterocycloalkyl unsubstituted or substituted by one or more $R^{2-3}$; and the heteroatom in the 6-10 membered heterocycloalkyl is N, and the number of heteroatom(s) is 1 or 2.

In an embodiment, $R^3$ is 5-6 membered heteroaryl unsubstituted or substituted by one or more $R^{3-1}$; and the heteroatom in the 5-6 membered heteroaryl is N and/or S, and the number of heteroatoms is 3.

In an embodiment, $R^4$ is 7-9 membered heterocycloalkyl unsubstituted or substituted by one or more $R^{4-1}$; and the heteroatom in the 7-9 membered heterocycloalkyl is N, and the number of heteroatom(s) is 1.

In an embodiment, $R^1$ is $C_3$-$C_6$ cycloalkyl substituted by one or more $R^{1-2}$, or 3-6 membered heterocycloalkyl unsubstituted or substituted by one or more $R^{1-3}$, and the heteroatom in the 3-6 membered heterocycloalkyl is one or more selected from N, O and S, and the number of heteroatom(s) is 1, 2 or 3.

In an embodiment, $R^{1-2}$ is each independently $C_2$-$C_6$ alkynyl.

In an embodiment, $R^{1-3}$ is each independently selected from cyano, $C_1$-$C_6$ alkyl unsubstituted or substituted by one or more $R^{1-3-1}$, or $C_2$-$C_6$ alkynyl.

In an embodiment, $R^{1-3-1}$ is each independently halogen.

In an embodiment, $R^2$ is 4-12 membered heterocycloalkyl substituted by one or more $R^{2-3}$; and the heteroatom in the 4-12 membered heterocycloalkyl is one or more selected from N, O, and S, and the number of heteroatom(s) is 1, 2 or 3.

In an embodiment, $R^{2-3}$ is each independently selected from

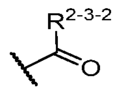,

or 5-10 membered heteroaryl; and the heteroatom in the 5-10 membered heteroaryl is N and/or O, and the number of heteroatoms is 2 or 3.

In an embodiment, $R^{2-3-2}$ is $C_1$-$C_6$ alkyl substituted by one or more $R^{2-3-2-1}$, $C_3$-$C_6$ cycloalkyl unsubstituted or substituted by one or more $R^{2-3-2-2}$,

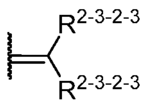,

3-6 membered heterocycloalkyl unsubstituted or substituted by one or more $R^{2-3-2-5}$, or 5-10 membered heteroaryl; the heteroatom in the 3-6 membered heterocycloalkyl is one or more selected from N, O and S, and the number of heteroatom(s) is 1, 2 or 3; and the heteroatom in the 5-10 membered heteroaryl is N and/or O, and the number of heteroatom(s) is 1 or 2.

In an embodiment, $R^{2-3-2-1}$ is each independently selected from hydroxyl or

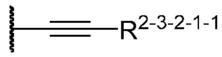

In an embodiment, $R^{2-3-2-1-1}$ is each independently $C_1$-$C_6$ alkyl.

In an embodiment, $R^{2-3-2-2}$ is each independently selected from halogen, hydroxyl, $C_1$-$C_6$ alkyl, $C_1$-$C_6$ alkoxy unsubstituted or substituted by one or more $R^{2-3-2-2-2}$, or amino.

In an embodiment, $R^{2-3-2-2-2}$ is each independently deuterium.

In an embodiment, $R^{2-3-2-3}$ is each independently selected from halogen or

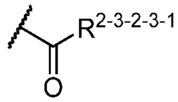

In an embodiment, $R^{2-3-2-3-1}$ is each independently 6-10 membered aryl unsubstituted or substituted by one or more $R^{2-3-2-3-1-1}$, and $R^{2-3-2-3-1-1}$ is each independently $C_1$-$C_6$ alkoxy.

In an embodiment, $R^{2-3-2-5}$ is each independently selected from halogen, $C_1$-$C_6$ alkoxy, or $C_1$-$C_6$ alkyl unsubstituted or substituted by one or more $R^{2-3-2-5-1}$

In an embodiment, $R^{2-2-1}$ is each independently halogen.

In an embodiment, $R^3$ is unsubstituted 5-6 membered heteroaryl; and the heteroatom in the 5-6 membered heteroaryl is N and/or S, and the number of heteroatoms is 3.

In an embodiment, $R^3$ is

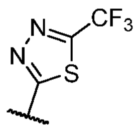

In an embodiment, $R^4$ is deuterium, cyano, 5-10 membered heterocycloalkyl unsubstituted or substituted by one or more $R^{4-1}$s, and the heteroatom in the 5-10 membered heterocycloalkyl is one or more selected from N, O, and S, and the number of heteroatom(s) is 1 or 2.

In an embodiment, $R^{4-1}$ is each independently

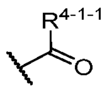
;

and $R^{4-1-1}$ is $C_1$-$C_6$ alkyl.

In an embodiment, $R^1$ is $C_3$-$C_6$ cycloalkyl unsubstituted or substituted by one or more $R^{1-2}$,

$R^{1-2}$ is each independently selected from cyano, $C_1$-$C_6$ alkyl unsubstituted or substituted by one or more $R^{1-2-1}$, or $C_2$-$C_6$ alkynyl;

$R^{1-2-1}$ is each independently selected from deuterium, halogen, or hydroxyl;

$R^2$ is 4-10 membered heterocycloalkyl substituted by one or more $R^{2-3}$; the heteroatom in the 4-10 membered heterocycloalkyl is N and/or O, and the number of heteroatom(s) is 1, 2 or 3; the 4-10 membered heterocycloalkyl is a 4-6 membered saturated monocyclic ring, a 7-10 membered saturated spiro ring or a 7-10 membered saturated bridged ring;

$R^{2-3}$ is each independently selected from

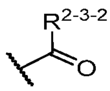
,

or 5-10 membered heteroaryl; the heteroatom in the 5-10 membered heteroaryl is N and/or O, and the number of heteroatoms is 2 or 3;

$R^{2-3-2}$ is $C_1$-$C_6$ alkyl substituted by one or more $R^{2-3-2-1}$, $C_3$-$C_6$ cycloalkyl unsubstituted or substituted by one or more $R^{2-3-2-2}$,

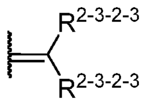,

3-6 membered heterocycloalkyl unsubstituted or substituted by one or more $R^{2-3-2-5}$, or 5-10 membered heteroaryl; the heteroatom in the 3-6 membered heterocycloalkyl is one or more selected from N, O and S, and the number of heteroatom(s) is 1, 2 or 3; the 3-6 membered heterocycloalkyl is a 3-6 membered saturated monocyclic ring; the heteroatom in the 5-10 membered heteroaryl is N and/or O, and the number of heteroatom(s) is 1 or 2;

$R^{2-3-2-1}$ is each independently selected from hydroxyl, or

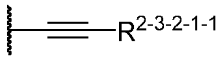;

$R^{2-3-2-1-1}$ is each independently $C_1$-$C_6$ alkyl;

$R^{2-3-2-2}$ is each independently selected from halogen, hydroxyl, $C_1$-$C_6$ alkyl, $C_1$-$C_6$ alkoxy unsubstituted or substituted by one or more $R^{2-3-2-2-2}$, or amino;

$R^{2-3-2-2-2}$ is each independently deuterium;

$R^{2-3-2-3}$ is each independently selected from hydrogen or

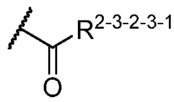;

$R^{2-3-2-3-1}$ is each independently 6-10 membered aryl unsubstituted or substituted by one or more $R^{2-3-2-3-1-1}$;

$R^{2-3-2-3-1-1}$ is each independently $C_1$-$C_6$ alkoxy;

$R^{2-3-2-5}$ is each independently selected from halogen, $C_1$-$C_6$ alkoxy, or $C_1$-$C_6$ alkyl unsubstituted or substituted by one or more $R^{2-3-2-5-1}$;

$R^{2-3-2-5-1}$ is each independently halogen;

$R^3$ is 5-6 membered heteroaryl unsubstituted or substituted by one or more $R^{3-1}$; the heteroatom in the 5-6 membered heteroaryl is N and/or S, and the number of heteroatoms is 3;

$R^{3-1}$ is each independently $C_1$-$C_6$ alkyl substituted by one or more $R^{3-1-1}$;

$R^{3-1-1}$ is each independently halogen;

$R^4$ is hydrogen, cyano, halogen, $C_1$-$C_6$ alkyl, or 5-10 membered heterocycloalkyl unsubstituted or substituted by one or more $R^{4-1}$; the heteroatom in the 5-10 membered heterocycloalkyl is one or more selected from N, O and S, and the number of heteroatom(s) is 1 or 2; the 5-10 membered heterocycloalkyl is a 7-10 membered unsaturated spiro ring;

$R^{4-1}$ is each independently

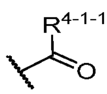;

and $R^{4-1-1}$ is $C_1$-$C_6$ alkyl.

In an embodiment, $R^1$ is $C_3$-$C_6$ cycloalkyl unsubstituted or substituted by one or more $R^{1-2}$;

$R^{1-2}$ is each independently selected from cyano, $C_1$-$C_6$ alkyl unsubstituted or substituted by one or more $R^{1-2-1}$, or $C_2$-$C_6$ alkynyl;

$R^{1-2-1}$ is each independently selected from deuterium, halogen, or hydroxyl;

$R^2$ is 4-10 membered heterocycloalkyl unsubstituted or substituted by one or more $R^{2-3}$; the heteroatom in the 4-10 membered heterocycloalkyl is N and/or O, and the number of heteroatom(s) is 1, 2 or 3;

$R^{2-3}$ is each independently selected from

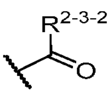,

or 5-10 membered heteroaryl; the heteroatom in the 5-10 membered heteroaryl is N and/or O, and the number of heteroatoms is 2 or 3;

$R^{2-3-2}$ is $C_1$-$C_6$ alkyl substituted by one or more $R^{2-3-2-1}$, $C_3$-$C_6$ cycloalkyl unsubstituted or substituted by one or more $R^{2-3-2-2}$,

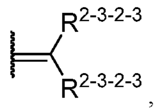

3-6 membered heterocycloalkyl unsubstituted or substituted by one or more $R^{2-3-2-5}$, or 5-10 membered heteroaryl; the heteroatom in the 3-6 membered heterocycloalkyl is one or more selected from N, O and S, and the number of heteroatom(s) is 1, 2 or 3; the heteroatom in the 5-10 membered heteroaryl is N and/or O, and the number of heteroatom(s) is 1 or 2;

$R^{2-3-2-1}$ is each independently selected from hydroxyl, or

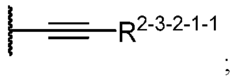

$R^{2-3-2-1-1}$ is each independently $C_1$-$C_6$ alkyl;

$R^{2-3-2-2}$ is each independently selected from halogen, hydroxyl, $C_1$-$C_6$ alkyl, $C_1$-$C_6$ alkoxy unsubstituted or substituted by one or more $R^{2-3-2-2-2}$, or amino;

$R^{2-3-2-2-2}$ is each independently deuterium;

$R^{2-3-2-3}$ is each independently selected from hydrogen or

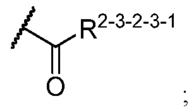

$R^{2-3-2-3-1}$ is each independently 6-10 membered aryl unsubstituted or substituted by one or more $R^{2-3-2-3-1-1}$;

$R^{2-3-2-3-1-1}$ is each independently $C_1$-$C_6$ alkoxy;

$R^{2-3-2-5}$ is each independently selected from halogen, $C_1$-$C_6$ alkoxy, or $C_1$-$C_6$ alkyl unsubstituted or substituted by one or more $R^{2-3-2-5-1}$;

$R^{2-3-2-5-1}$ is each independently halogen;

$R^3$ is 5-6 membered heteroaryl unsubstituted or substituted by one or more $R^{3-1}$; the heteroatom in the 5-6 membered heteroaryl is N and/or S, and the number of heteroatoms is 3;

$R^{3-1}$ is each independently $C_1$-$C_6$ alkyl substituted by one or more $R^{3-1-1}$;

$R^{3-1-1}$ is each independently halogen;

$R^4$ is hydrogen, cyano, halogen, $C_1$-$C_6$ alkyl, or 5-10 membered heterocycloalkyl unsubstituted or substituted by one or more $R^{4-1}$; the heteroatom in the 5-10 membered heterocycloalkyl is one or more selected from N, O and S, and the number of heteroatom(s) is 1 or 2;

$R^{4-1}$ is each independently

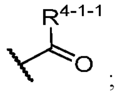

and $R^{4-1-1}$ is $C_1$-$C_6$ alkyl.

In an embodiment, $R^1$ is $C_3$-$C_6$ cycloalkyl unsubstituted or substituted by one or more $R^{1-2}$;

$R^{1-2}$ is each independently selected from cyano, or $C_1$-$C_6$ alkyl unsubstituted or substituted by one or more $R^{1-2-1}$;

$R^{1-2-1}$ is each independently selected from deuterium or halogen;

$R^2$ is 4-10 membered heterocycloalkyl substituted by one or more $R^{2-3}$; the heteroatom in the 4-10 membered heterocycloalkyl is selected from N and/or O, and the number of heteroatom(s) is 1, 2 or 3; the 4-10 membered heterocycloalkyl is a 4-6 membered saturated monocyclic ring or a 7-10 membered saturated spiro ring;

$R^{2-3}$ is each independently

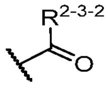

;

$R^{2-3-2}$ is $C_3$-$C_6$ cycloalkyl substituted by one or more $R^{2-3-2-2}$, 3-6 membered heterocycloalkyl substituted by one or more $R^{2-3-2-5}$; the heteroatom in the 3-6 membered heterocycloalkyl is one or more selected from N, O and S, and the number of heteroatom(s) is 1, 2 or 3; the 3-6 membered heterocycloalkyl is a 3-6 membered saturated monocyclic ring; the heteroatom in the 5-10 membered heteroaryl is N and/or O, and the number of heteroatom(s) is 1 or 2;
$R^{2-3-2-2}$ is each independently selected from $C_1$-$C_6$ alkyl, or $C_1$-$C_6$ alkoxy unsubstituted or substituted by one or more $R^{2-3-2-2-2}$;
$R^{2-3-2-2-2}$ is each independently deuterium;
$R^{2-3-2-5}$ is each independently $C_1$-$C_6$ alkyl;
$R^3$ is 5-6 membered heteroaryl unsubstituted or substituted by one or more $R^{3-1}$; the heteroatom in the 5-6 membered heteroaryl is N and/or S, and the number of heteroatoms is 3;
$R^{3-1}$ is each independently $C_1$-$C_6$ alkyl substituted by one or more $R^{3-1-1}$;
$R^{3-1-1}$ is each independently halogen;
$R^4$ is hydrogen, cyano, or $C_1$-$C_6$ alkyl.

In an embodiment, $R^1$ is $C_3$-$C_6$ cycloalkyl unsubstituted or substituted by one or more $R^{1-2}$;

$R^{1-2}$ is each independently selected from cyano, or $C_1$-$C_6$ alkyl unsubstituted or substituted by one or more $R^{1-2-1}$;
$R^{1-2-1}$ is each independently selected from deuterium or halogen;
$R^2$ is 4-10 membered heterocycloalkyl unsubstituted or substituted by one or more $R^{2-3}$; the heteroatom in the 4-10 membered heterocycloalkyl is selected from N and/or O, and the number of heteroatom(s) is 1, 2 or 3;
$R^{2-3}$ is each independently

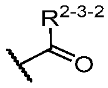

;

$R^{2-3-2}$ is $C_3$-$C_6$ cycloalkyl substituted by one or more $R^{2-3-2-2}$, or 3-6 membered heterocycloalkyl substituted by one or more $R^{2-3-2-5}$; the heteroatom in the 3-6 membered heterocycloalkyl is one or more selected from N, O and S, and the number of heteroatom(s) is 1, 2 or 3; the heteroatom in the 5-10 membered heteroaryl is N and/or O, and the number of heteroatom(s) is 1 or 2;
$R^{2-3-2-2}$ is each independently selected from $C_1$-$C_6$ alkyl, or $C_1$-$C_6$ alkoxy unsubstituted or substituted by one or more $R^{2-3-2-2-2}$;
$R^{2-3-2-2-2}$ is each independently deuterium;
$R^{2-3-2-5}$ is each independently $C_1$-$C_6$ alkyl;
$R^3$ is 5-6 membered heteroaryl unsubstituted or substituted by one or more $R^{3-1}$; the heteroatom in the 5-6 membered heteroaryl is N and/or S, and the number of heteroatoms is 3;
$R^{3-1}$ is each independently $C_1$-$C_6$ alkyl substituted by one or more $R^{3-1-1}$;
$R^{3-1-1}$ is each independently halogen;
$R^4$ is hydrogen, cyano, or $C_1$-$C_6$ alkyl.

In an embodiment, $R^1$ is 3-6 membered heterocycloalkyl unsubstituted or substituted by one or more $R^{1-3}$; the heteroatom in the 3-6 membered heterocycloalkyl is O, and the number of heteroatom(s) is 1; the 3-6 membered heterocycloalkyl is a 3-6 membered saturated monocyclic ring;

$R^{1-3}$ is each independently selected from cyano, $C_1$-$C_6$ alkyl unsubstituted or substituted by one or more $R^{1-3-1}$, or $C_2$-$C_6$ alkynyl;
$R^{1-3-1}$ is each independently selected from halogen or deuterium;
$R^2$ is 4-12 membered heterocycloalkyl unsubstituted or substituted by one or more $R^{2-3}$; the heteroatom in the 4-12 membered heterocycloalkyl is one or more selected from N, O and S, and the number of heteroatom(s) is 1, 2 or 3; the 4-12 membered heterocycloalkyl is a 4-6 membered saturated monocyclic ring, a 7-10 membered saturated fused ring or a 7-10 membered saturated spiro ring;
$R^{2-3}$ is each independently $C_1$-$C_6$ alkyl unsubstituted or substituted by one or more $R^{2-3-1}$,

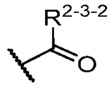

,

or

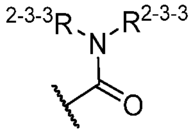

;

$R^{2-3-1}$ is each independently selected from hydroxyl, halogen, $C_1$-$C_6$ alkoxy unsubstituted or substituted by one or more $R^{2-3-1-1}$, or amino unsubstituted or substituted by one or more $R^{2-3-1-3}$;
$R^{2-3-1-1}$ is each independently deuterium;
$R^{2-3-1-3}$ is each independently $C_1$-$C_6$ alkyl;
$R^{2-3-2}$ is $C_1$-$C_6$ alkyl, or $C_3$-$C_6$ cycloalkyl unsubstituted or substituted by one or more $R^{2-3-2-2}$;
$R^{2-3-2-2}$ is each independently selected from $C_1$-$C_6$ alkyl, or $C_1$-$C_6$ alkoxy unsubstituted or substituted by one or more $R^{2-3-2-2-2}$;
$R^{2-3-2-2-2}$ is each independently deuterium;
$R^{2-3-3}$ is each independently selected from hydrogen, or $C_1$-$C_6$ alkyl unsubstituted or substituted by one or more $R^{2-3-3-1}$;
$R^{2-3-3-1}$ is each independently amino unsubstituted or substituted by one or more $R^{2-3-3-1-1}$;
$R^{2-3-3-1-1}$ is each independently $C_1$-$C_6$ alkyl;
$R^3$ is 5-6 membered heteroaryl unsubstituted or substituted by one or more $R^{3-1}$; the heteroatom in the 5-6 membered heteroaryl is N and/or S, and the number of heteroatoms is 3;
$R^{3-1}$ is each independently $C_1$-$C_6$ alkyl substituted by one or more $R^{3-1-1}$;
$R^{3-1-1}$ is each independently halogen;
$R^4$ is hydrogen, deuterium, halogen, or $C_1$-$C_6$ alkyl.

In an embodiment, $R^1$ is 3-6 membered heterocycloalkyl unsubstituted or substituted by one or more $R^{1-3}$; the heteroatom in the 3-6 membered heterocycloalkyl is O, and the number of heteroatom(s) is 1;
$R^{1-3}$ is each independently selected from cyano, $C_1$-$C_6$ alkyl unsubstituted or substituted by one or more $R^{1-3-1}$, or $C_2$-$C_6$ alkynyl;
$R^{1-3-1}$ is each independently selected from halogen or deuterium;
$R^2$ is 4-12 membered heterocycloalkyl unsubstituted or substituted by one or more $R^{2-3}$; the heteroatom in the 4-12 membered heterocycloalkyl is one or more selected from N, O, and S, and the number of heteroatom(s) is 1, 2 or 3;
$R^{2-3}$ is each independently $C_1$-$C_6$ alkyl unsubstituted or substituted by one or more $R^{2-3-1}$,

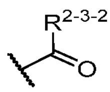

,

or

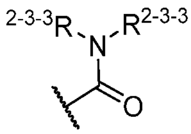

;

$R^{2-3-1}$ is each independently selected from hydroxyl, halogen, $C_1$-$C_6$ alkoxy unsubstituted or substituted by one or more $R^{2-3-1-1}$, or amino unsubstituted or substituted by one or more $R^{2-3-1-3}$;
$R^{2-3-1-1}$ is each independently deuterium;
$R^{2-3-1-3}$ is each independently $C_1$-$C_6$ alkyl;
$R^{2-3-2}$ is $C_1$-$C_6$ alkyl, or $C_3$-$C_6$ cycloalkyl unsubstituted or substituted by one or more $R^{2-3-2-2}$;
$R^{2-3-2-2}$ is each independently selected from $C_1$-$C_6$ alkyl, or $C_1$-$C_6$ alkoxy unsubstituted or substituted by one or more $R^{2-3-2-2-2}$;
$R^{2-3-2-2-2}$ is each independently deuterium;

$R^{2-3-3}$ is each independently selected from hydrogen, or $C_1$-$C_6$ alkyl unsubstituted or substituted by one or more $R^{2-3-3-1}$;

$R^{2-3-3-1}$ is each independently amino unsubstituted or substituted by one or more $R^{2-3-3-1-1}$;

$R^{2-3-3-1-1}$ is each independently $C_1$-$C_6$ alkyl;

$R^3$ is 5-6 membered heteroaryl unsubstituted or substituted by one or more $R^{3-1}$; the heteroatom in the 5-6 membered heteroaryl is N and/or S, and the number of heteroatoms is 3;

$R^{3-1}$ is each independently $C_1$-$C_6$ alkyl substituted by one or more $R^{3-1-1}$;

$R^{3-1-1}$ is each independently halogen;

$R^4$ is hydrogen, deuterium, halogen, or $C_1$-$C_6$ alkyl.

In a preferred embodiment, $R^1$ is 3-6 membered heterocycloalkyl unsubstituted or substituted by one or more $R^{1-3}$; the heteroatom in the 3-6 membered heterocycloalkyl is O, and the number of heteroatom(s) is 1; the 3-6 membered heterocycloalkyl is a 3-6 membered saturated monocyclic ring;

$R^{1-3}$ is each independently selected from cyano, $C_1$-$C_6$ alkyl unsubstituted or substituted by one or more $R^{1-3-1}$, or $C_2$-$C_6$ alkynyl;

$R^{1-3-1}$ is each independently halogen;

$R^2$ is 4-12 membered heterocycloalkyl unsubstituted or substituted by one or more $R^{2-3}$; the heteroatom in the 4-12 membered heterocycloalkyl is one or more selected from N, O and S, and the number of heteroatom(s) is 1, 2 or 3; the 4-12 membered heterocycloalkyl is a 4-6 membered saturated monocyclic ring, a 7-10 membered saturated fused ring or a 7-10 membered saturated spiro ring;

$R^{2-3}$ is each independently $C_1$-$C_6$ alkyl unsubstituted or substituted by one or more $R^{2-3-1}$,

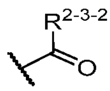
,

or

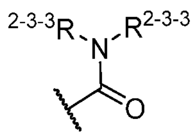
;

$R^{2-3-1}$ is each independently selected from hydroxyl, or $C_1$-$C_6$ alkoxy unsubstituted or substituted by one or more $R^{2-3-1-1}$;

$R^{2-3-1-1}$ is each independently deuterium;

$R^{2-3-2}$ is $C_1$-$C_6$ alkyl, or $C_3$-$C_6$ cycloalkyl unsubstituted or substituted by one or more $R^{2-3-2-2}$;

$R^{2-3-2-2}$ is each independently selected from $C_1$-$C_6$ alkyl, or $C_1$-$C_6$ alkoxy unsubstituted or substituted by one or more $R^{2-3-2-2-2}$;

$R^{2-3-2-2-2}$ is each independently deuterium;

$R^{2-3-3}$ is each independently selected from hydrogen, or $C_1$-$C_6$ alkyl unsubstituted or substituted by one or more $R^{2-3-3-1}$;

$R^{2-3-3-1}$ is each independently amino unsubstituted or substituted by one or more $R^{2-3-3-1-1}$;

$R^{2-3-3-1-1}$ is each independently $C_1$-$C_6$ alkyl;

$R^3$ is 5-6 membered heteroaryl unsubstituted or substituted by one or more $R^{3-1}$; the heteroatom in the 5-6 membered heteroaryl is N and/or S, and the number of heteroatoms is 3;

$R^{3-1}$ is each independently $C_1$-$C_6$ alkyl substituted by one or more $R^{3-1-1}$;

$R^{3-1-1}$ is each independently halogen;

$R^4$ is hydrogen, deuterium, halogen, or $C_1$-$C_6$ alkyl.

In a preferred embodiment, $R^1$ is 3-6 membered heterocycloalkyl unsubstituted or substituted by one or more $R^{1-3}$; the heteroatom in the 3-6 membered heterocycloalkyl is O, and the number of heteroatom(s) is 1;

$R^{1-3}$ is each independently selected from cyano, $C_1$-$C_6$ alkyl unsubstituted or substituted by one or more $R^{1-3-1}$, or $C_2$-$C_6$ alkynyl;

$R^{1-3-1}$ is each independently halogen;

$R^2$ is 4-12 membered heterocycloalkyl unsubstituted or substituted by one or more $R^{2-3}$; the heteroatom in the 4-12 membered heterocycloalkyl is one or more selected from N, O, and S, and the number of heteroatom(s) is 1, 2 or 3; $R^{2-3}$ is each independently $C_1$-$C_6$ alkyl unsubstituted or substituted by one or more $R^{2-3-1}$,

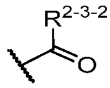
,

or

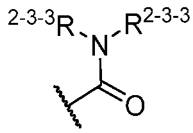
;

$R^{2-3-1}$ is each independently selected from hydroxyl, or $C_1$-$C_6$ alkoxy unsubstituted or substituted by one or more $R^{2-3-1-1}$.
$R^{2-3-1-1}$ is each independently deuterium;
$R^{2-3-2}$ is $C_1$-$C_6$ alkyl, or $C_3$-$C_6$ cycloalkyl unsubstituted or substituted by one or more $R^{2-3-2-2}$;
$R^{2-3-2-2}$ is each independently selected from $C_1$-$C_6$ alkyl, or $C_1$-$C_6$ alkoxy unsubstituted or substituted by one or more $R^{2-3-2-2-2}$;
$R^{2-3-2-2-2}$ is each independently deuterium;
$R^{2-3-3}$ is each independently selected from hydrogen, or $C_1$-$C_6$ alkyl unsubstituted or substituted by one or more $R^{2-3-3-1}$;
$R^{2-3-3-1}$ is each independently amino unsubstituted or substituted by one or more $R^{2-3-3-1-1}$;
$R^{2-3-3-1-1}$ is each independently $C_1$-$C_6$ alkyl;
$R^3$ is 5-6 membered heteroaryl unsubstituted or substituted by one or more $R^{3-1}$; the heteroatom in the 5-6 membered heteroaryl is N and/or S, and the number of heteroatoms is 3;
$R^{3-1}$ is each independently $C_1$-$C_6$ alkyl substituted by one or more $R^{3-1-1}$;
$R^{3-1-1}$ is each independently halogen;
$R^4$ is hydrogen, deuterium, halogen, or $C_1$-$C_6$ alkyl.

In an embodiment, ring B is

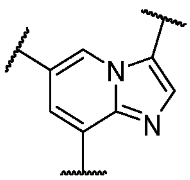
, 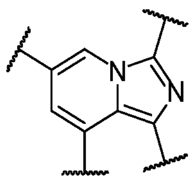
, 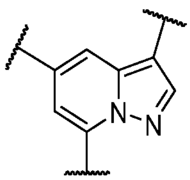
, 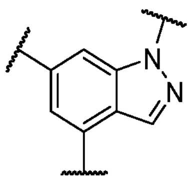
,

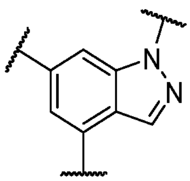
, 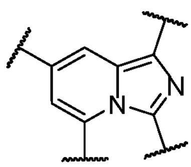
, 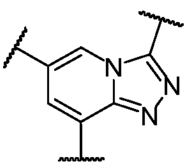
, 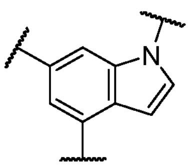
, 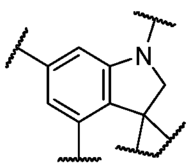
, 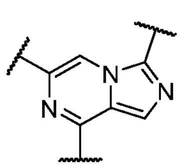
,

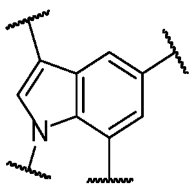
, 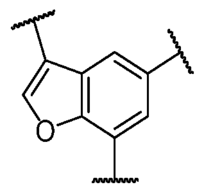
or 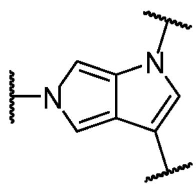

In an embodiment, $R^1$ is

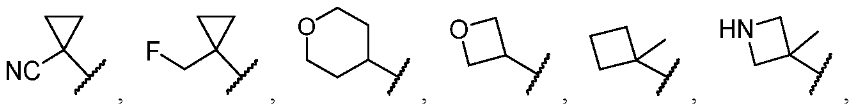

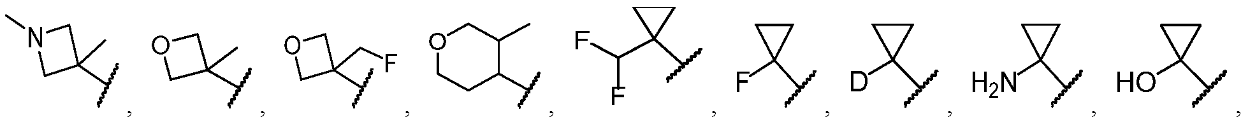

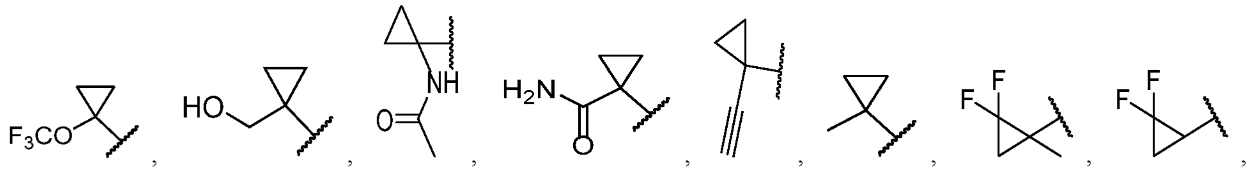

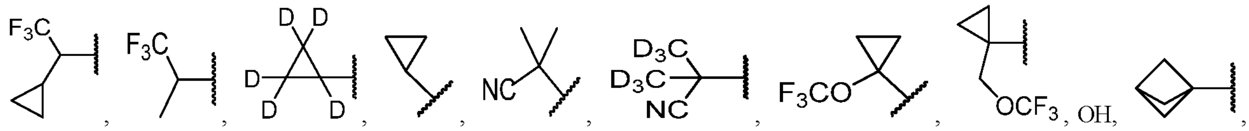
OH,

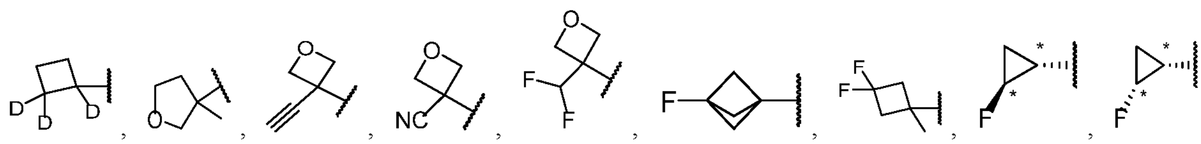
, or

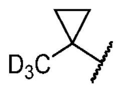
,

or the atom in $R^1$ is attached to the atom in A to form

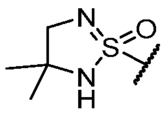 or 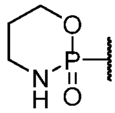

In an embodiment, $R^2$ is hydrogen, methyl,

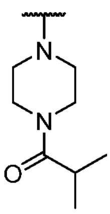, 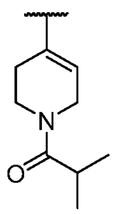, 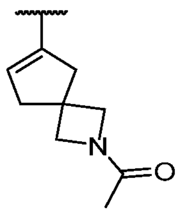, 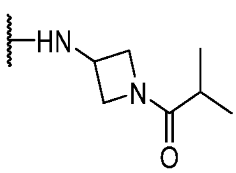,

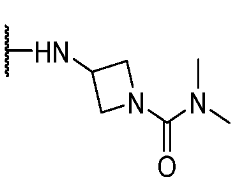, 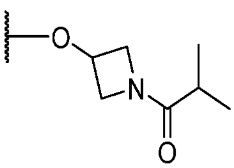, 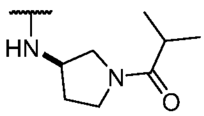, 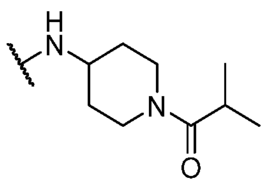, 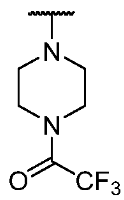,

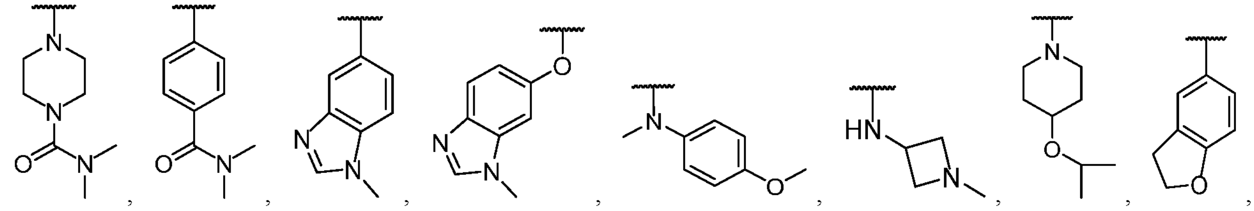
, , , , , , , ,

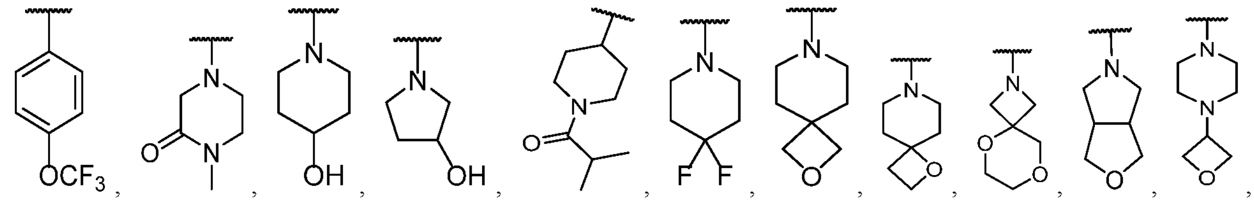
, , , , , , , , , , ,

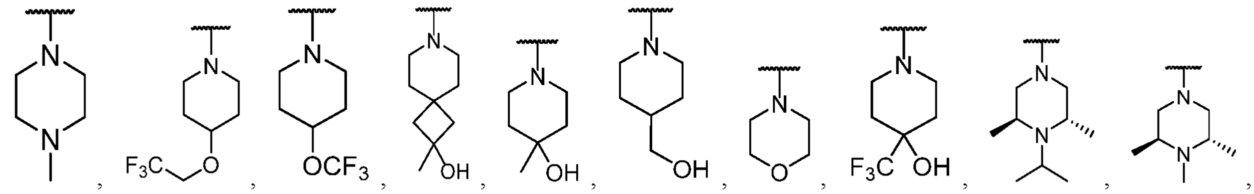
, , , , , , , , , ,

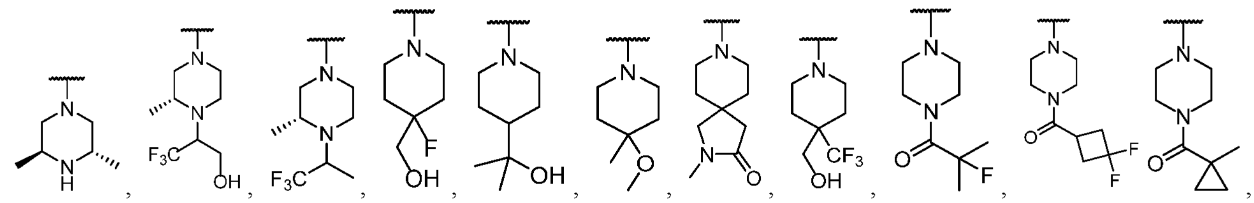
, , , , , , , , , , , ,

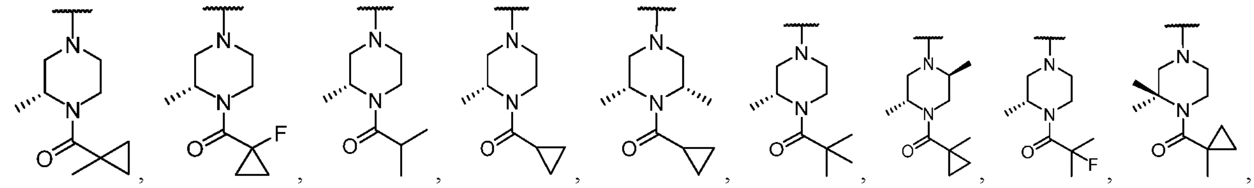
, , , , , , , , ,

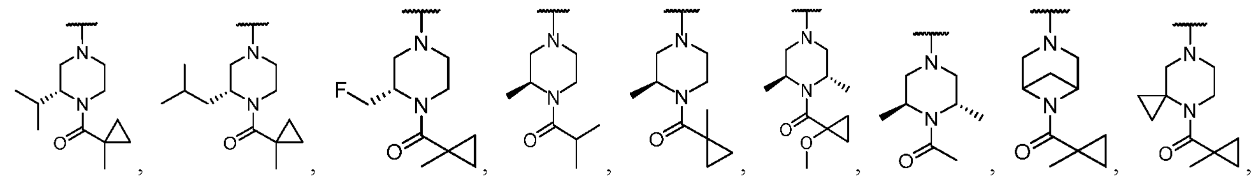
, , , , , , , , ,

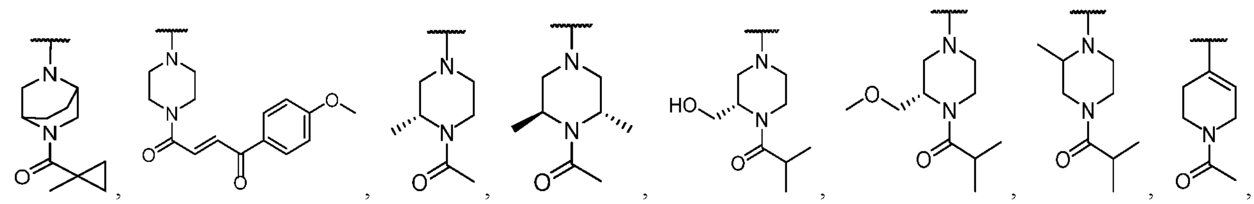
, , , , , , , ,

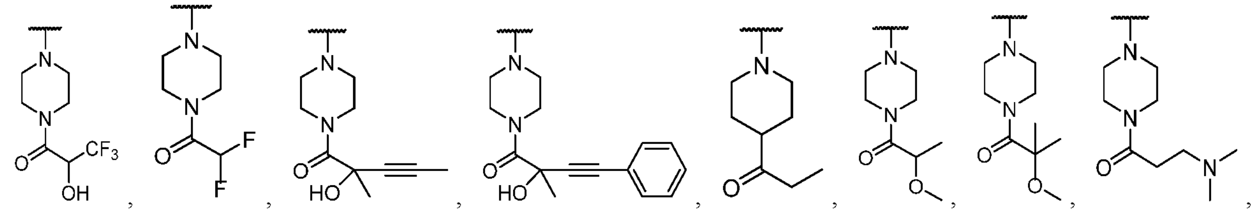

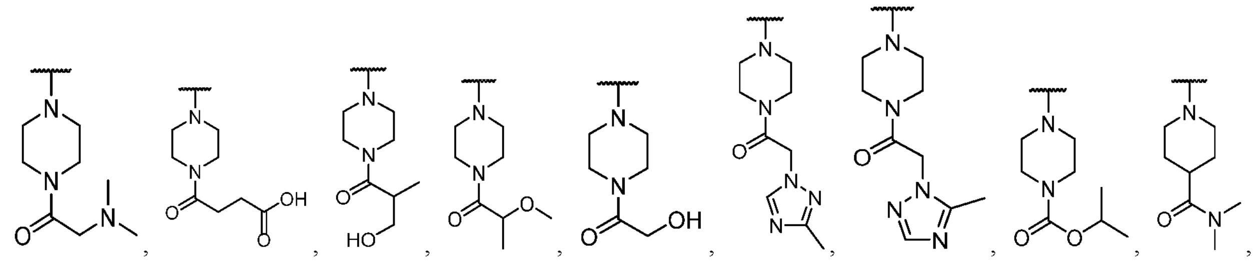

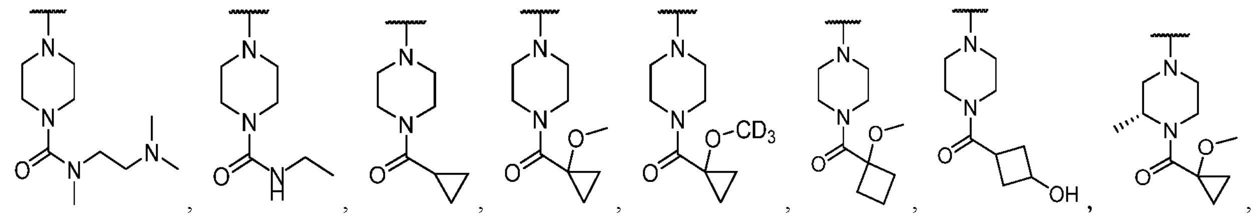

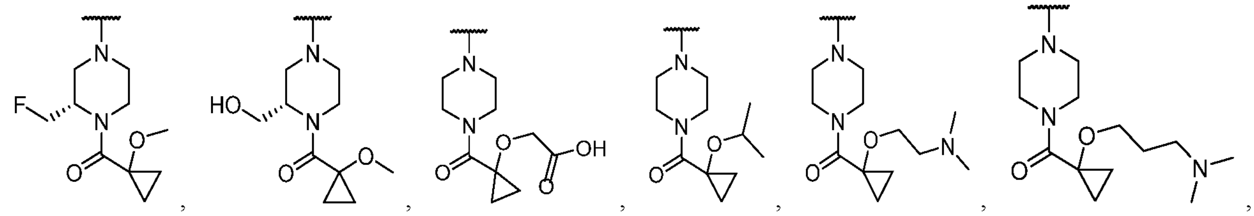

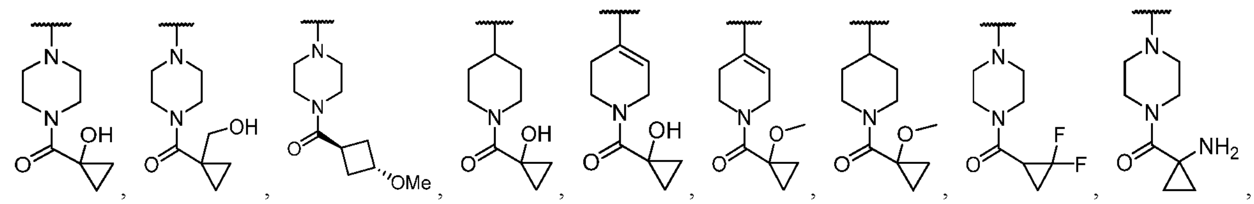

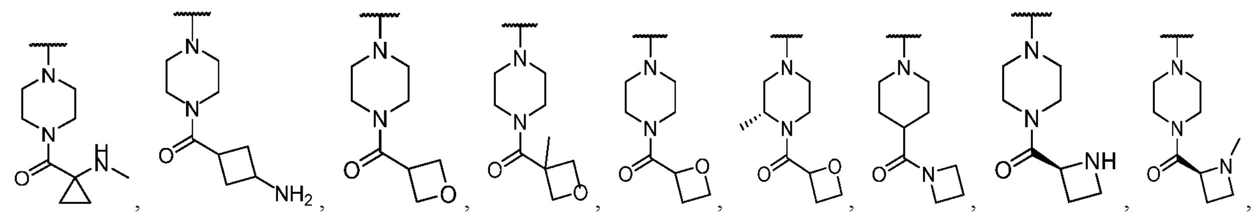

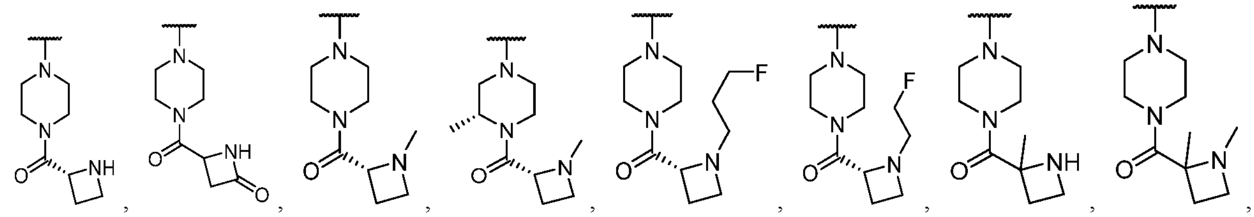

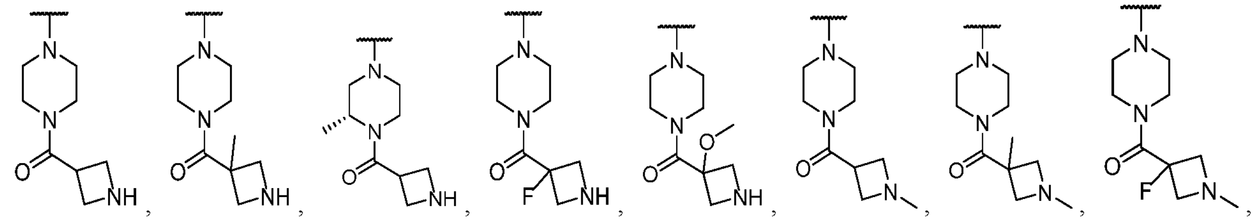
, , , , , , , ,

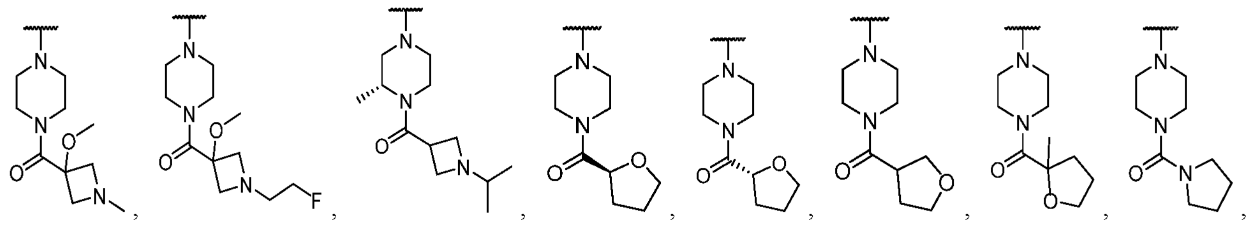
, , , , , , , ,

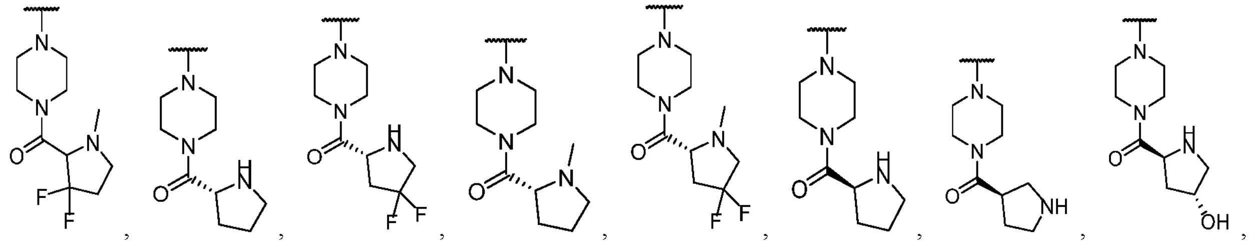
, , , , , , , ,

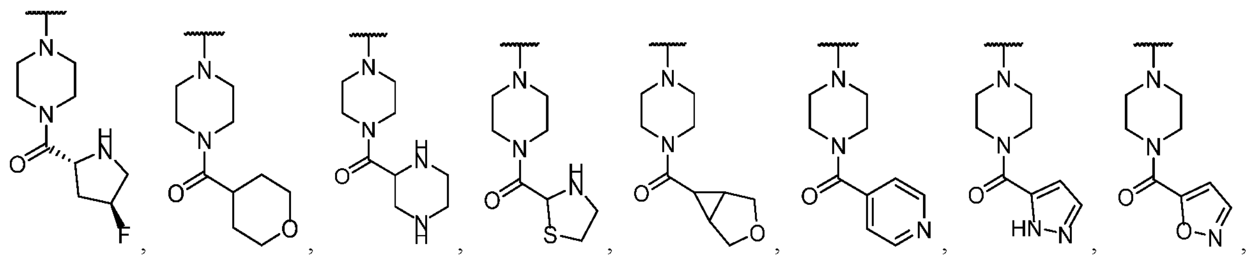
, , , , , , , ,

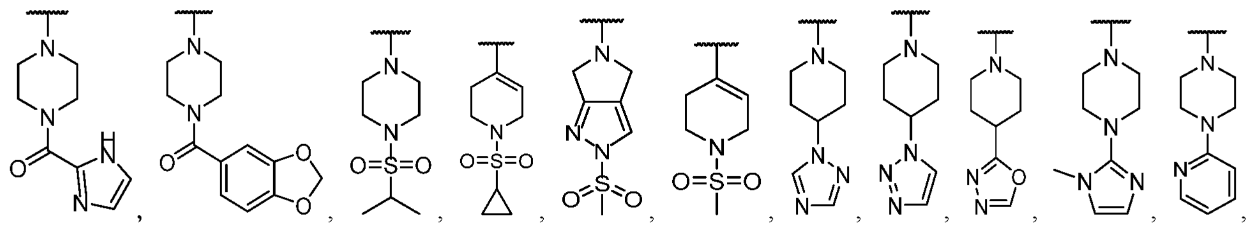
, , , , , , , , , , ,

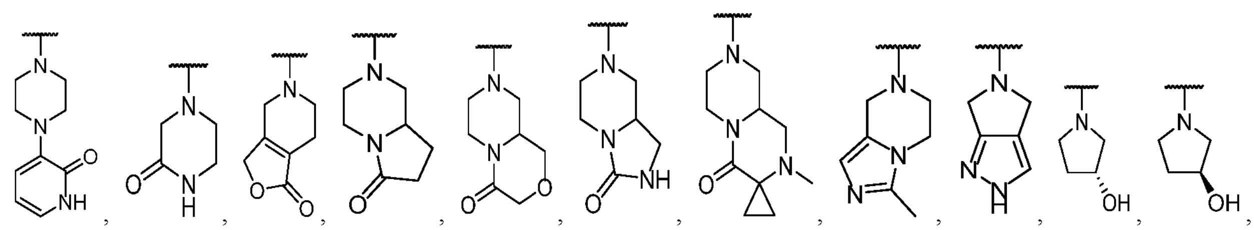
, , , , , , , , , , ,

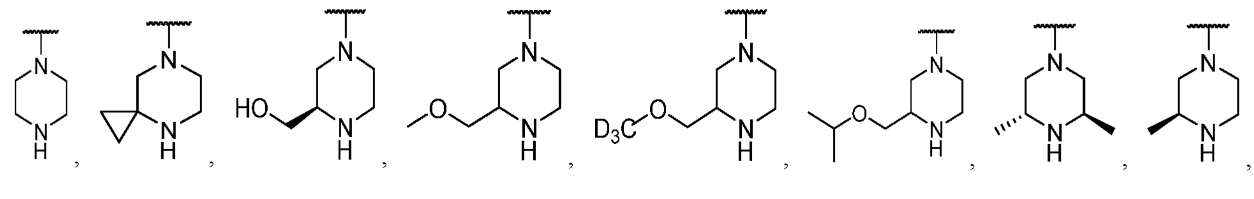
, , , , , , , ,

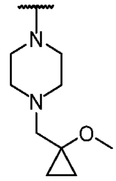, 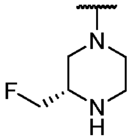, 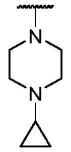, 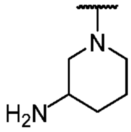, 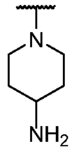, 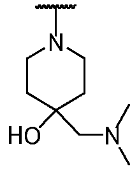, 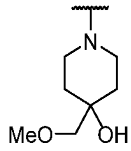, 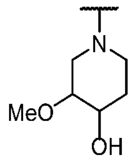 cis,

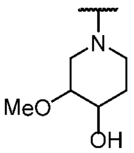 trans, 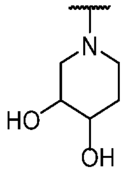, 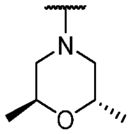, 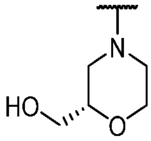, 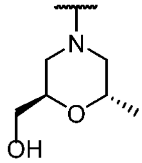, 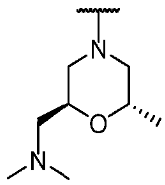, 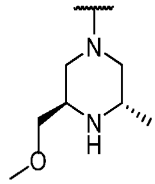,

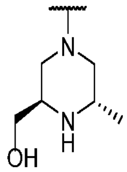, 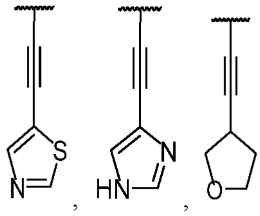, , , 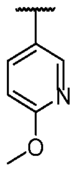, 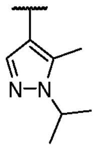, 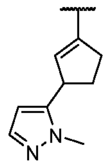 or 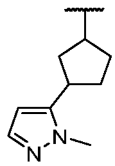

In an embodiment, $R^3$ is

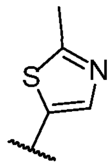, 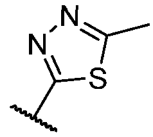, 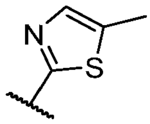, 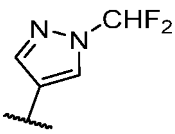, 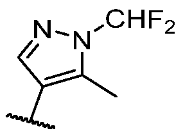,

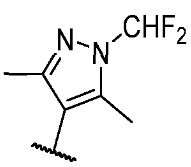, 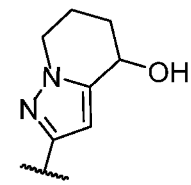, 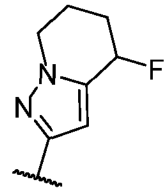, 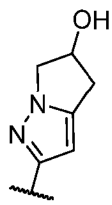, 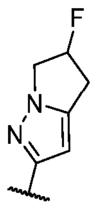, 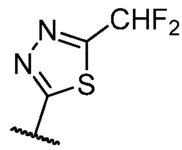, 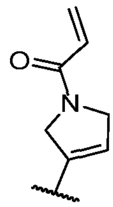,

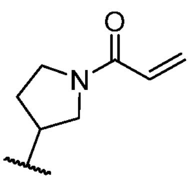, 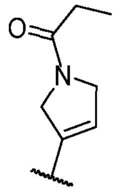, 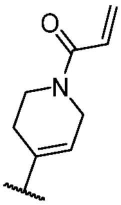, 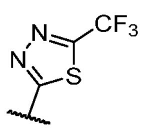 or 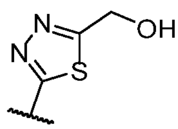

In an embodiment, $R^4$ is hydrogen, deuterium, Cl, F, CN, methyl,

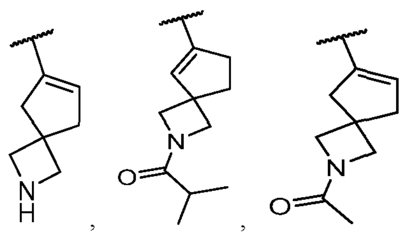, , ,

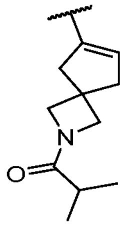 , 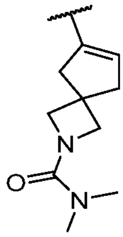 , 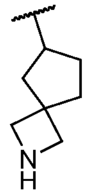 , 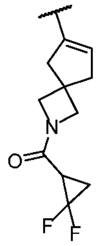 or 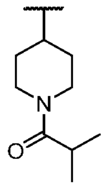

In an embodiment, the compound of formula I is any one of the following compounds:

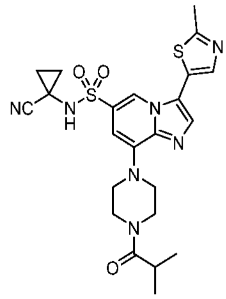 ,

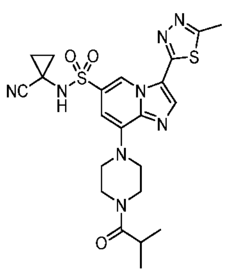 , 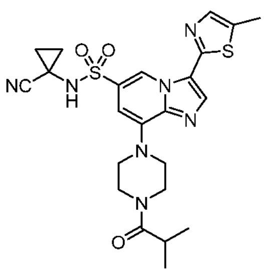 , 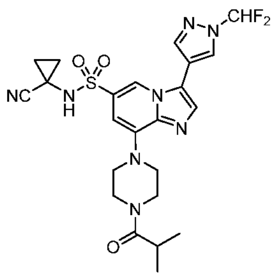 , 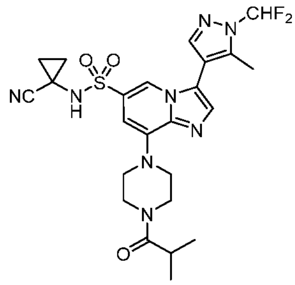 ,

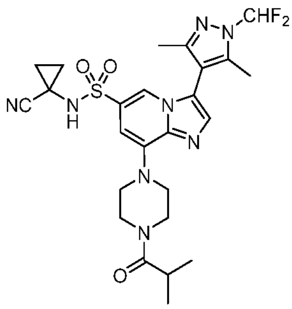 , 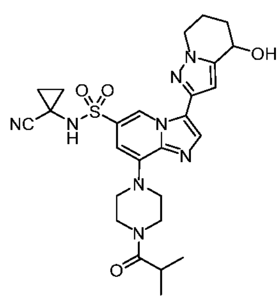 , 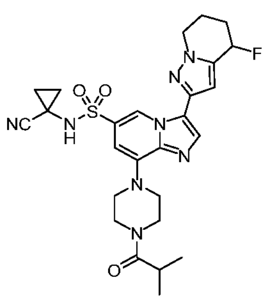 , 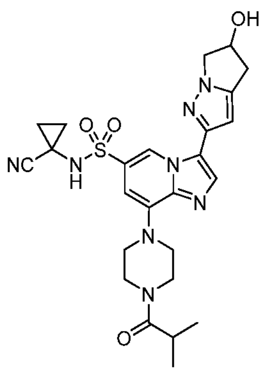 ,

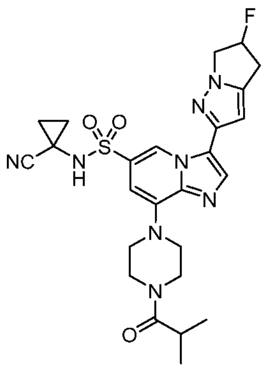 , 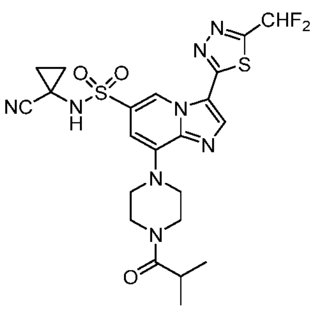 , 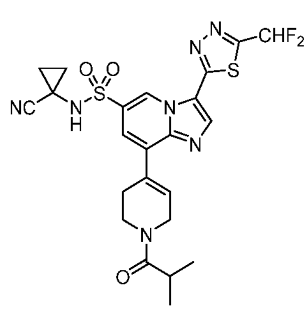 , 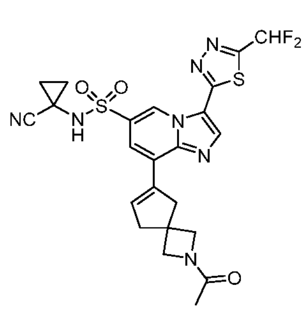 ,

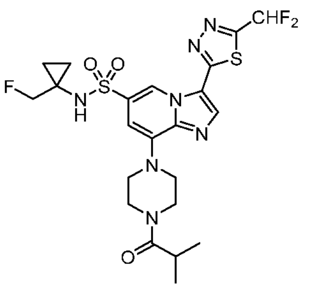 , 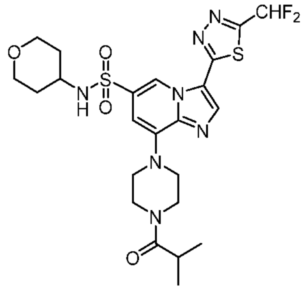 , 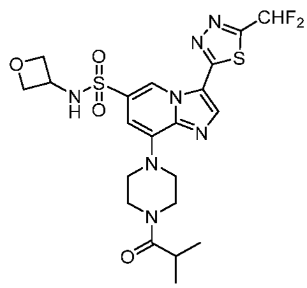 ,

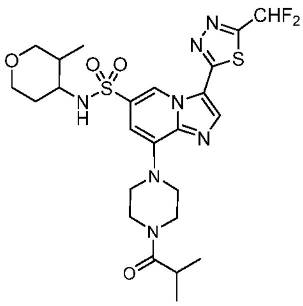 , 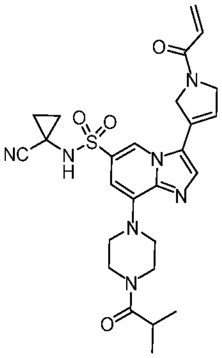 , 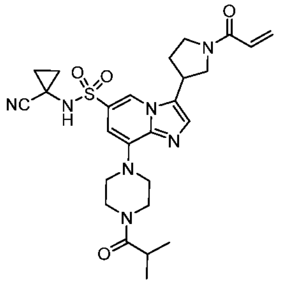 , 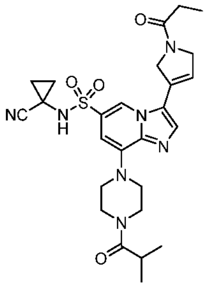 ,

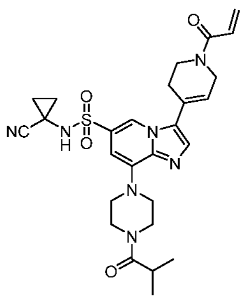 , 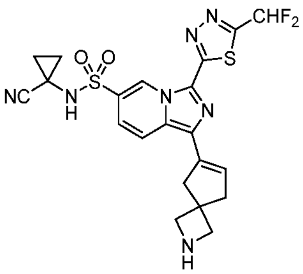 , 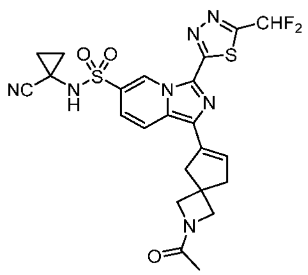 , 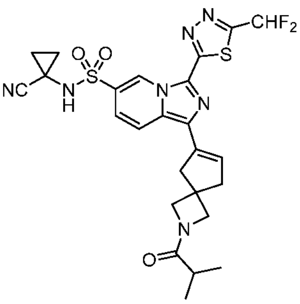 ,

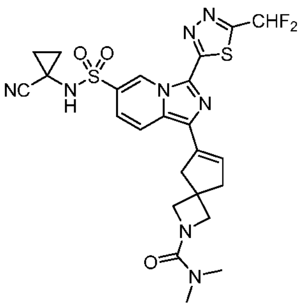 , 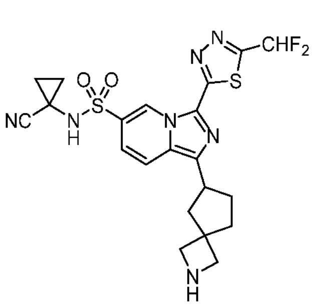 , 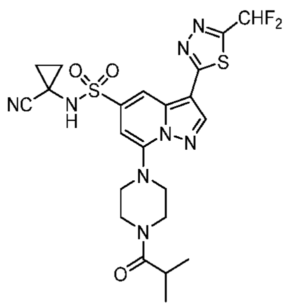 , 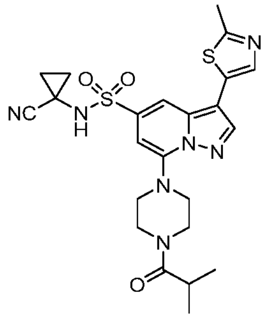 ,

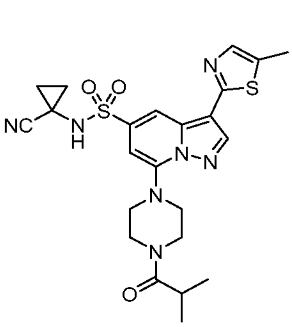 , 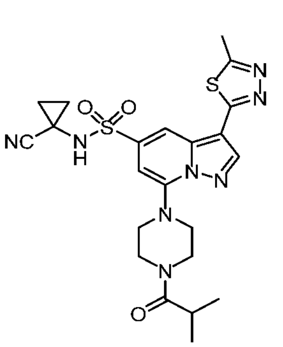 , 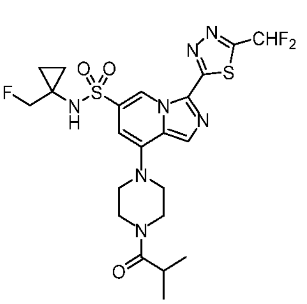 ,

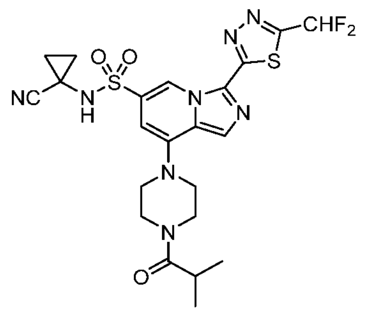
,
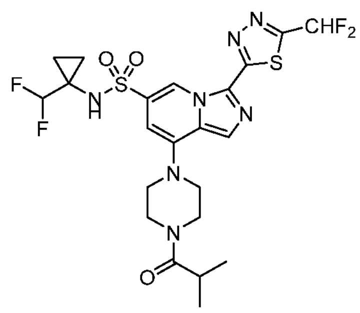
,
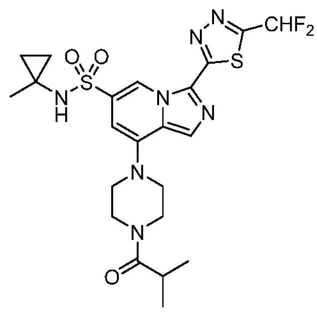
,

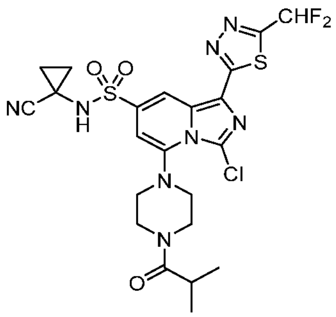
,
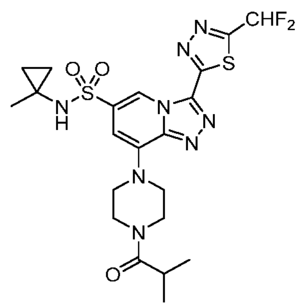
,
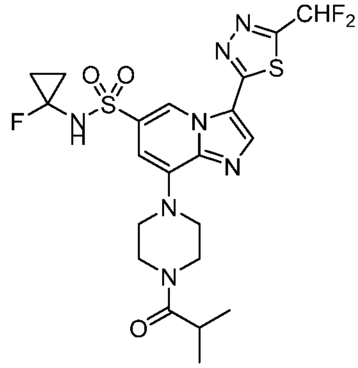
,

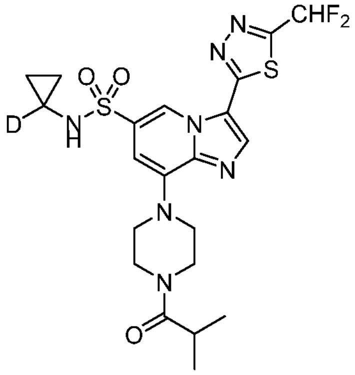
,
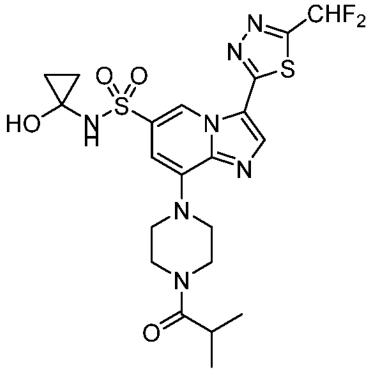
,
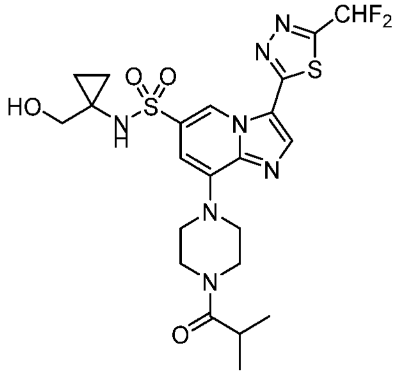
,

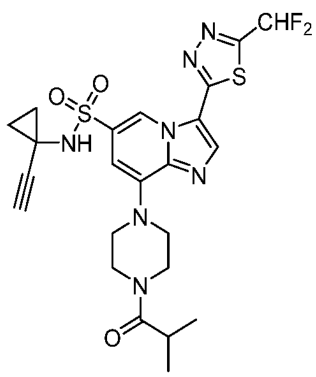
,
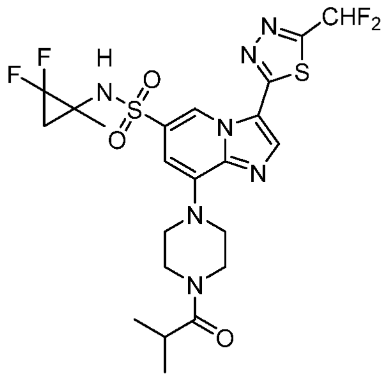
,
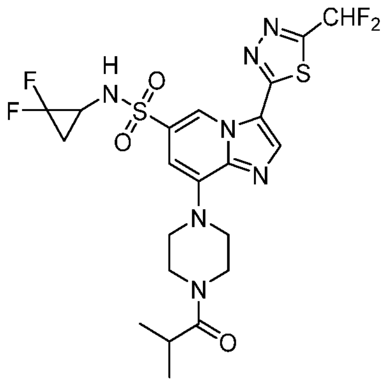
,

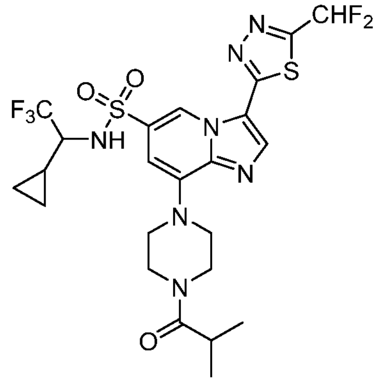
,
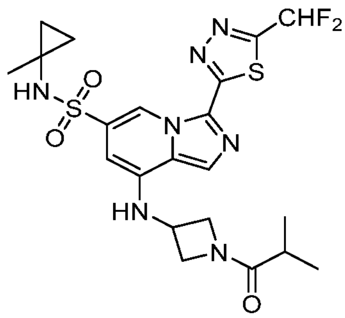
,
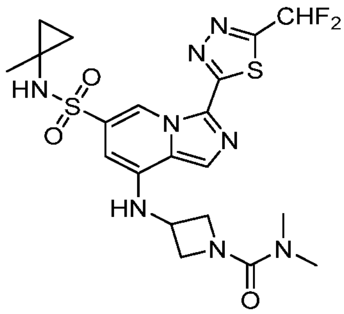
,

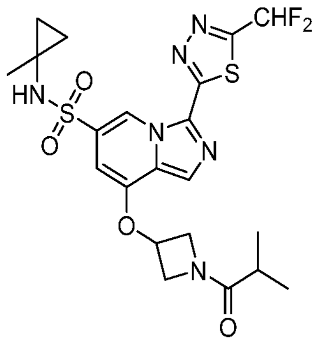
,
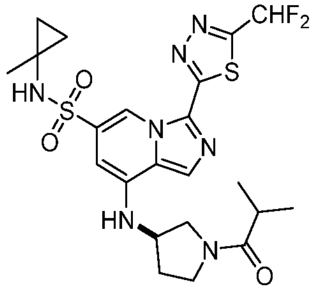
,
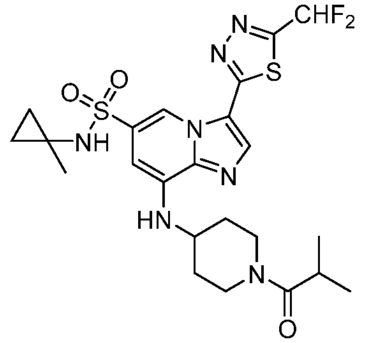
,

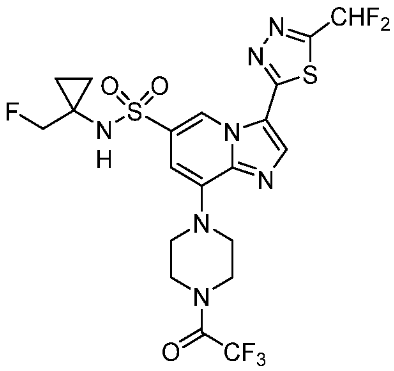
,
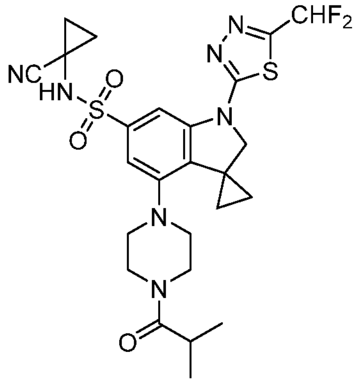
,
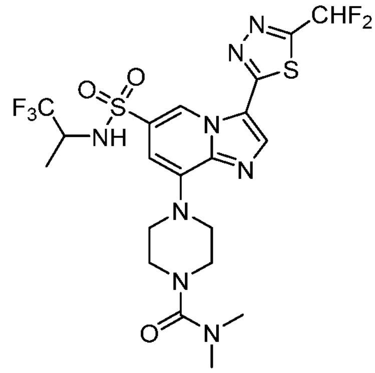
,

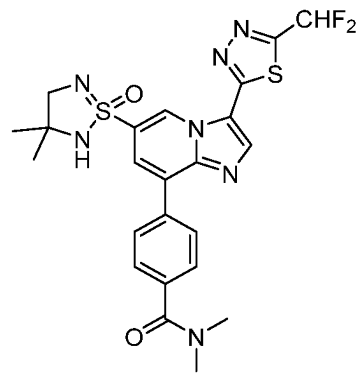
,
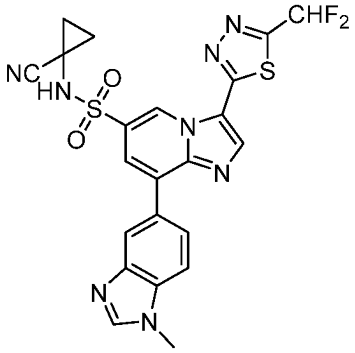
,
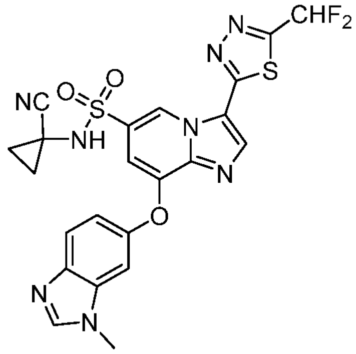
,

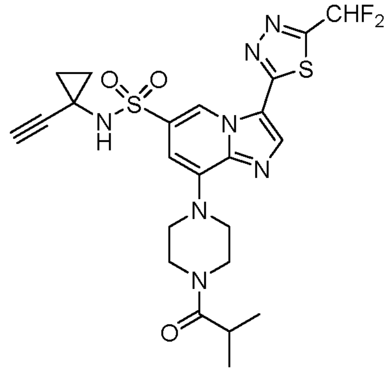 , 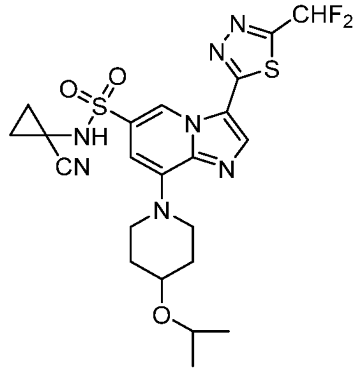 , 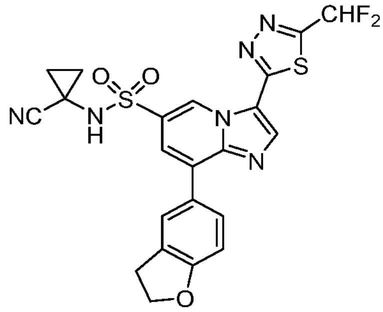 ,

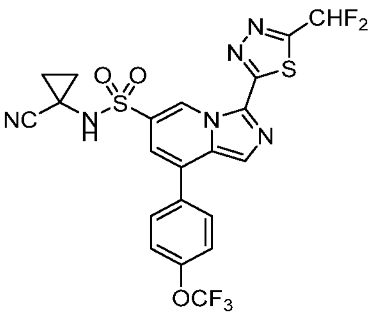 , 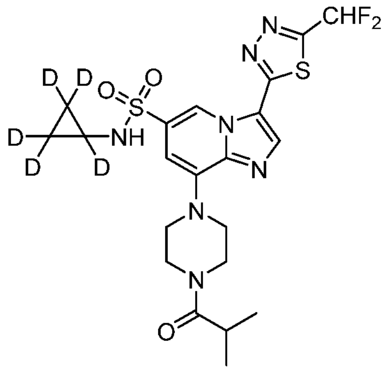 , 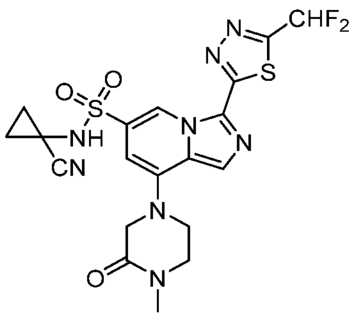 ,

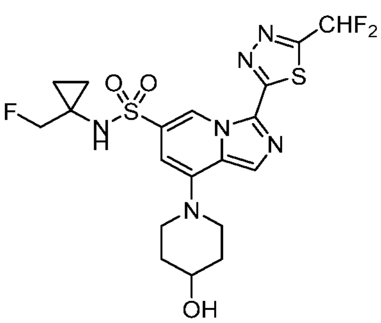 , 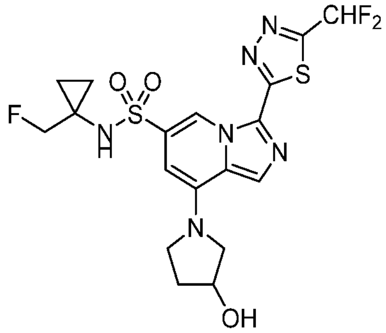 , 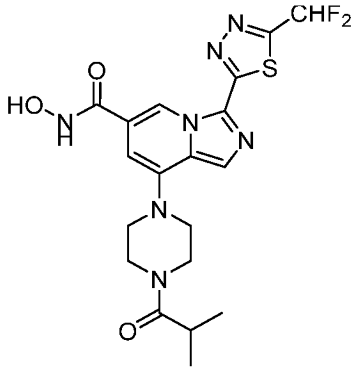 ,

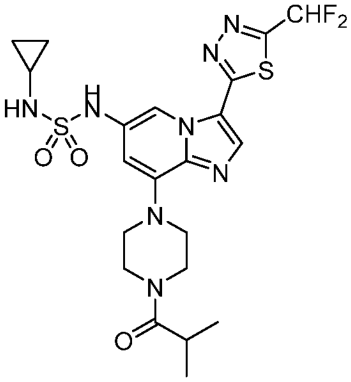 , 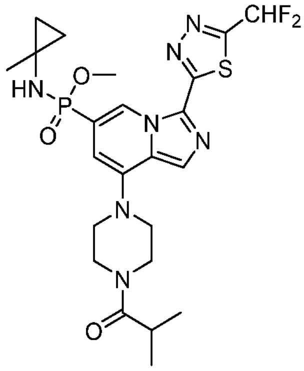 , 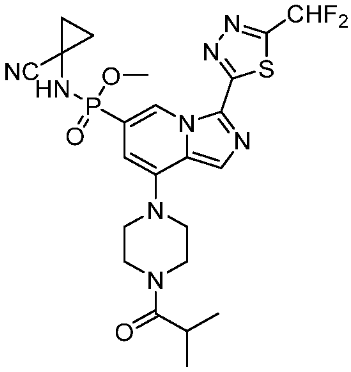 ,

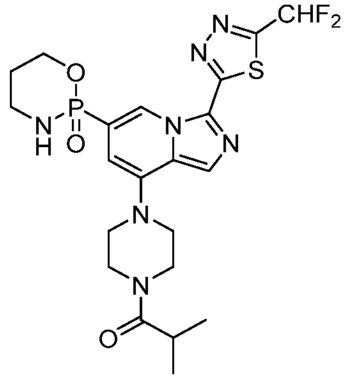
,
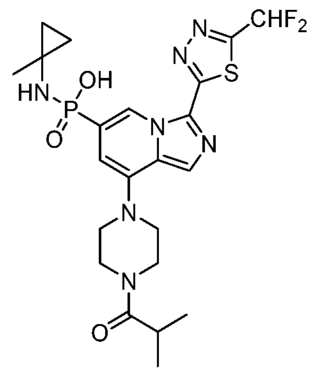
,
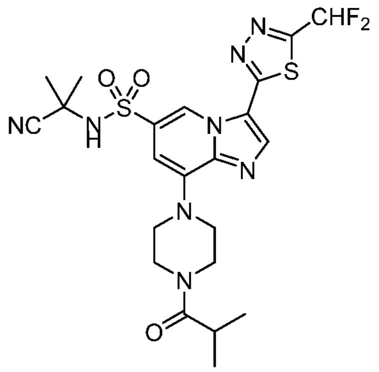
,
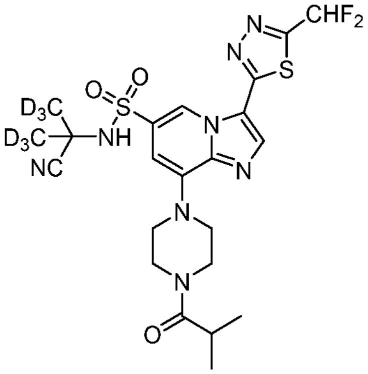
,
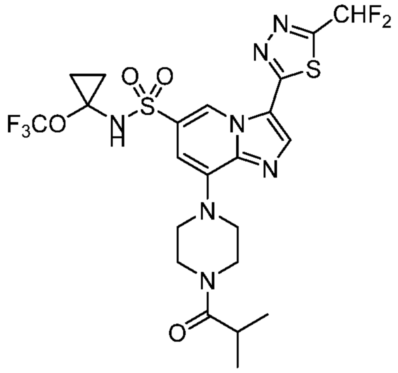
,
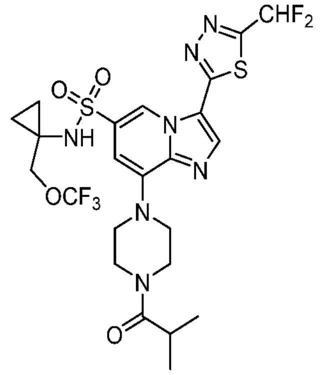
,
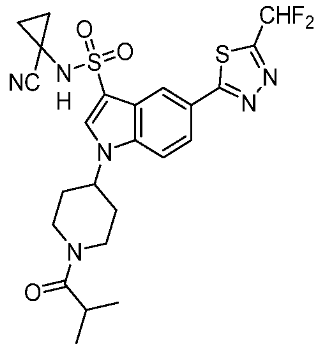
,
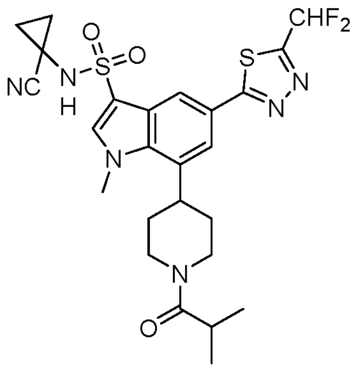
,
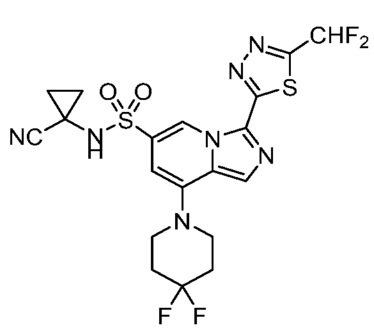
,
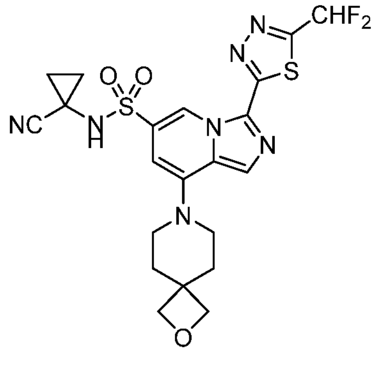
,
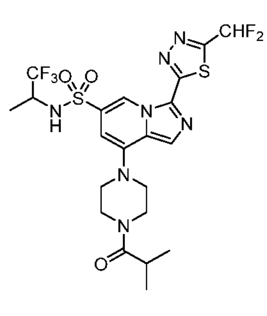
,
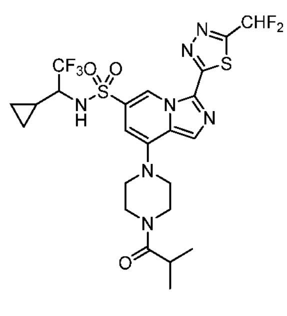
,

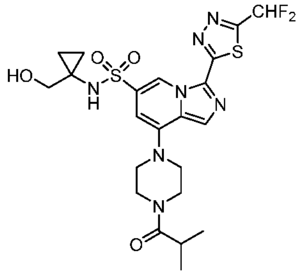,
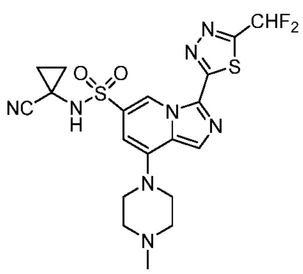,
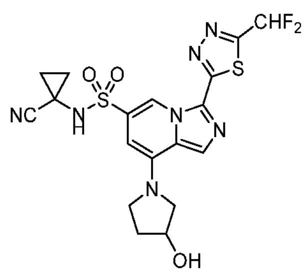,
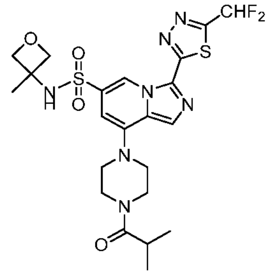,

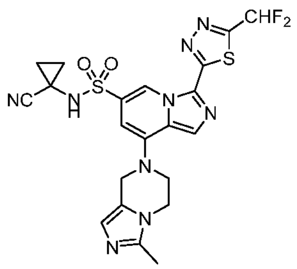,
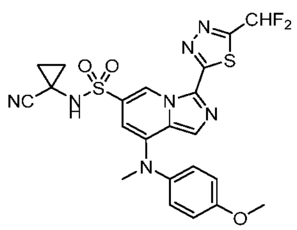,
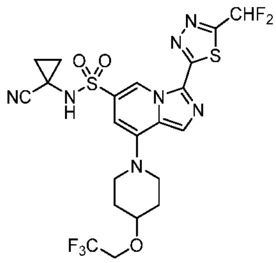,
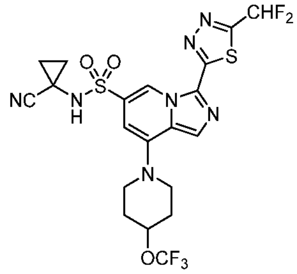,

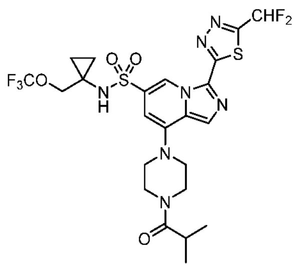,
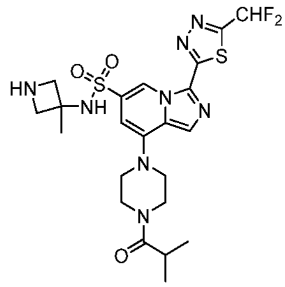,
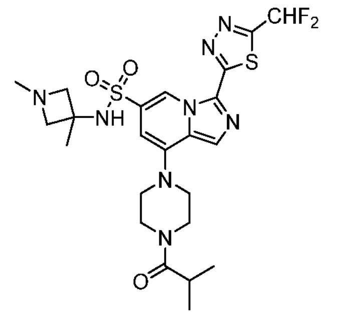,

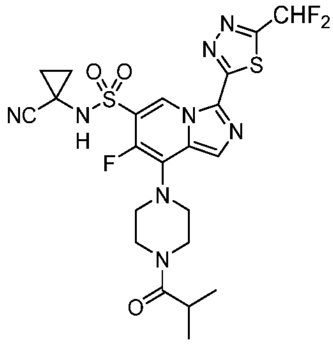,
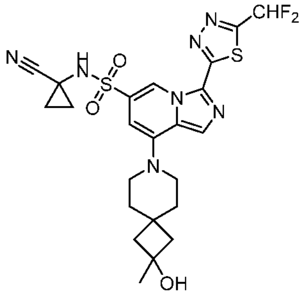,
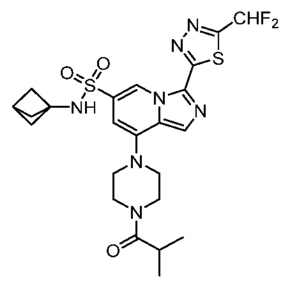,

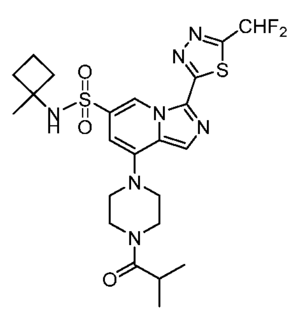,
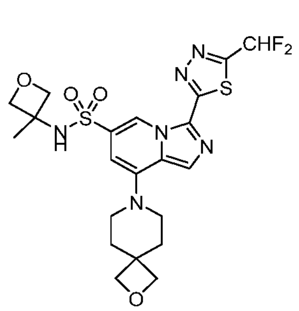,
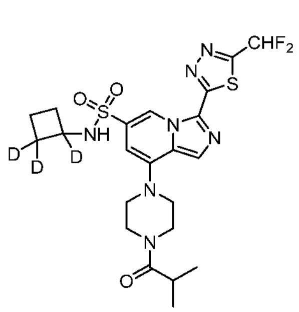,
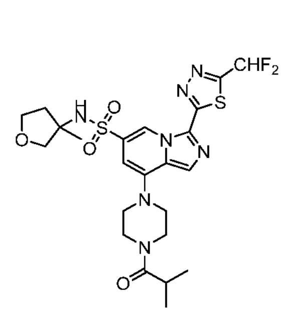,

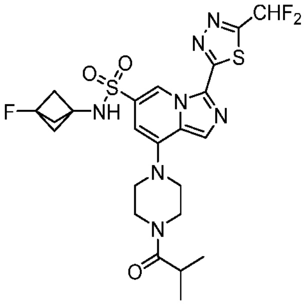 , 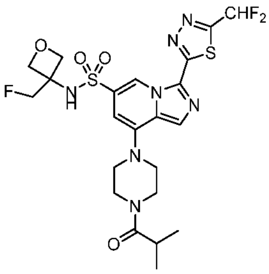 , 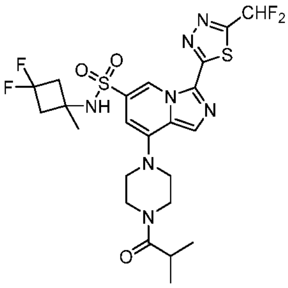 , 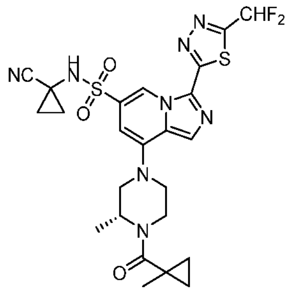 ,

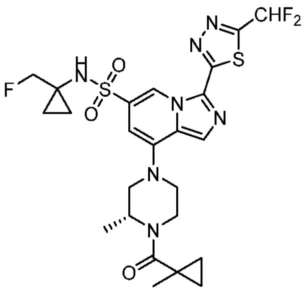 , 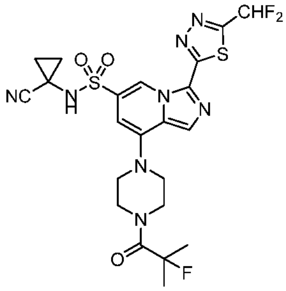 , 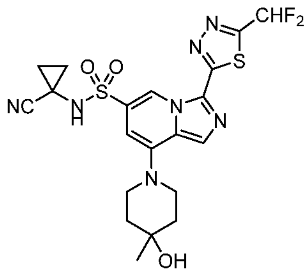 , 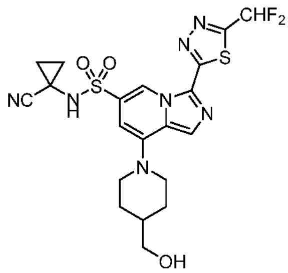 ,

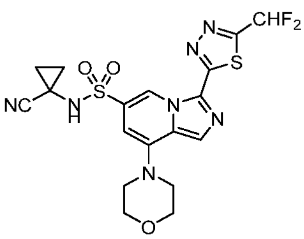 , 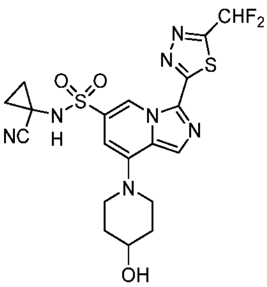 , 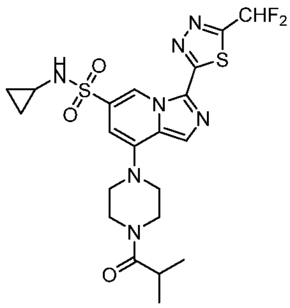 , 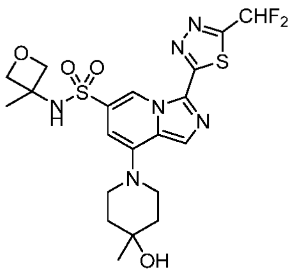 ,

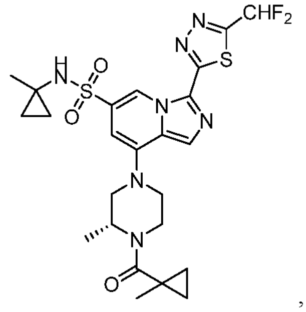 , 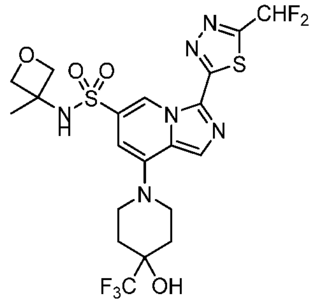 , 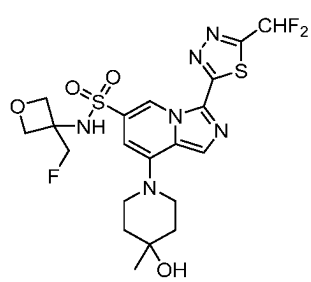 , 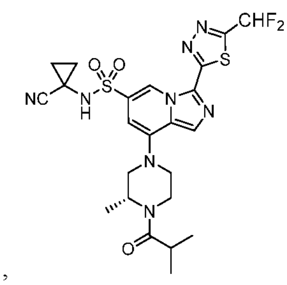 ,

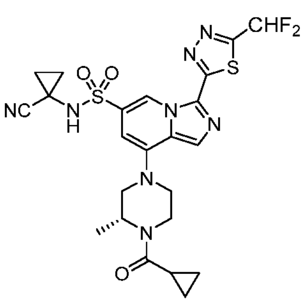 , 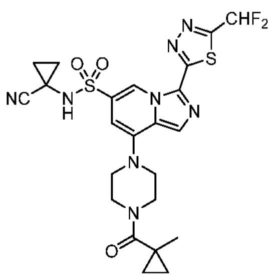 , 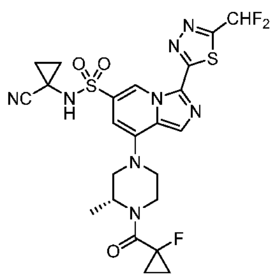 , 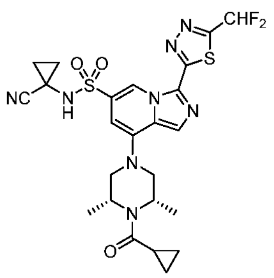 ,

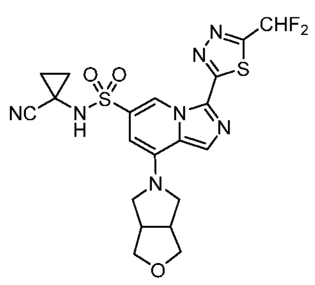,

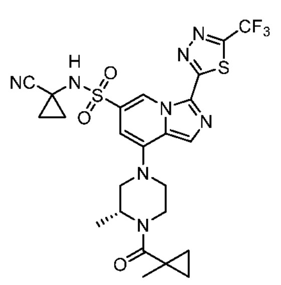,

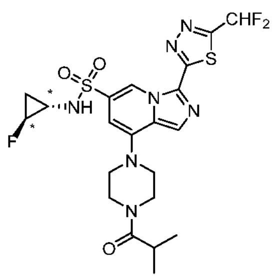,

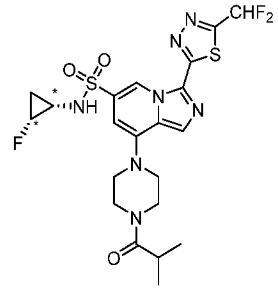,

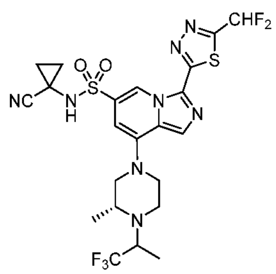,

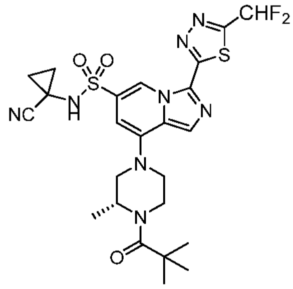,

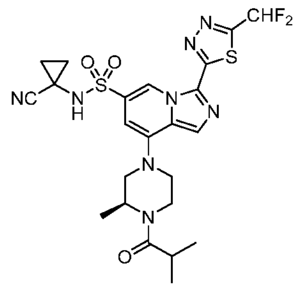,

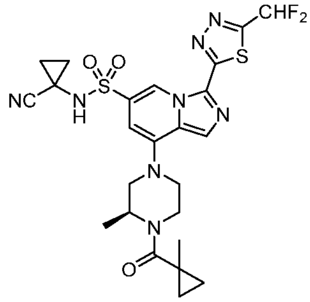,

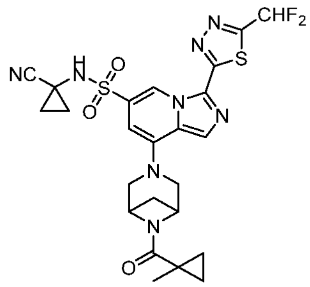,

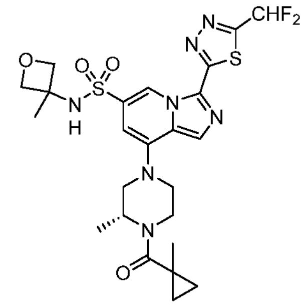,

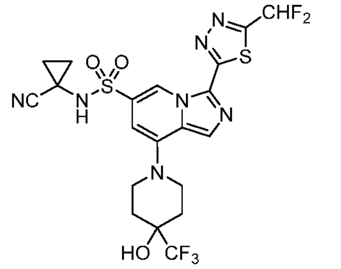,

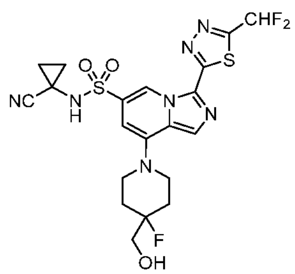,

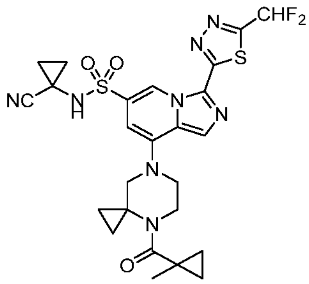,

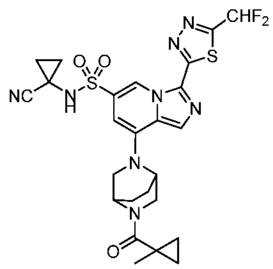,

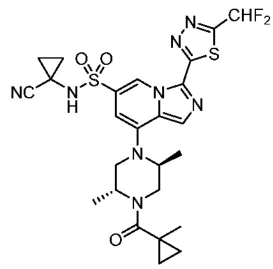,

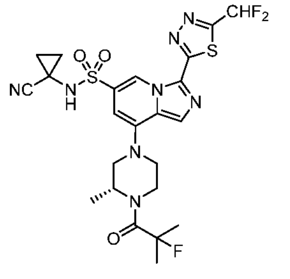,

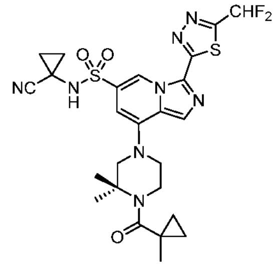,

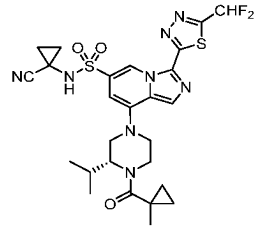,

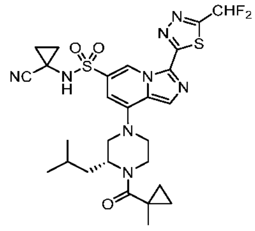,

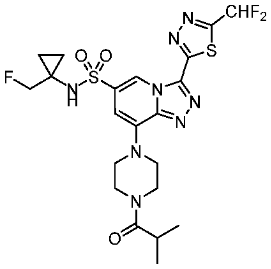 , 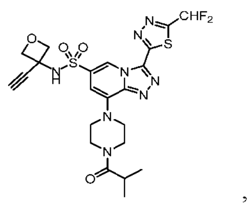 , 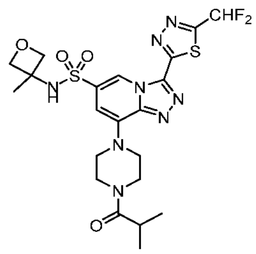 , 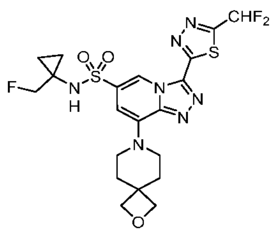 ,

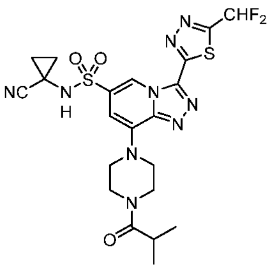 , 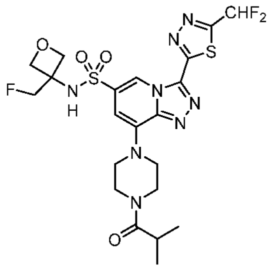 , 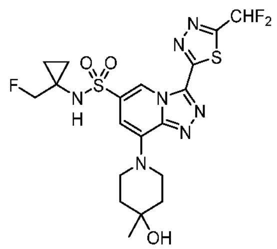 , 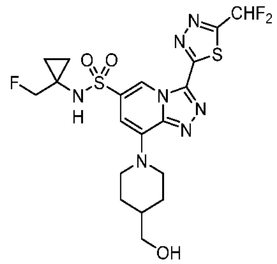 ,

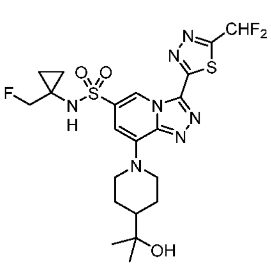 , 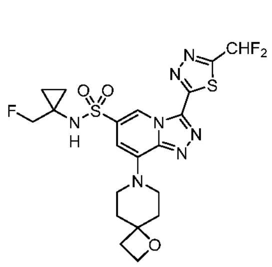 , 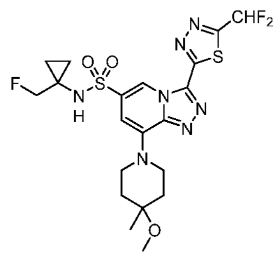 , 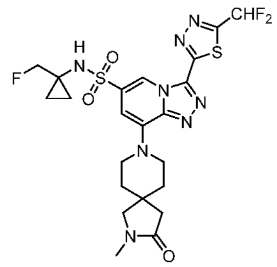 ,

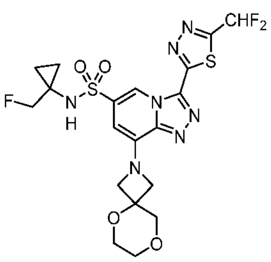 , 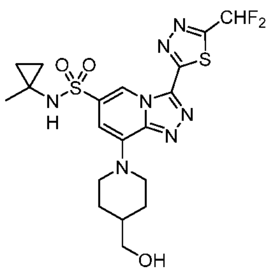 , 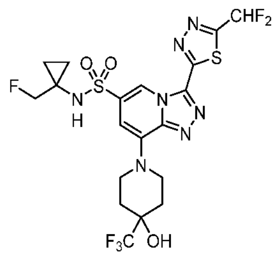 , 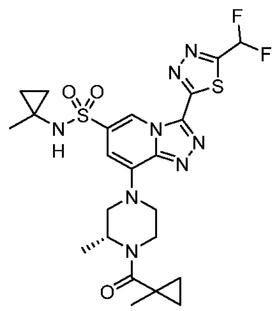 ,

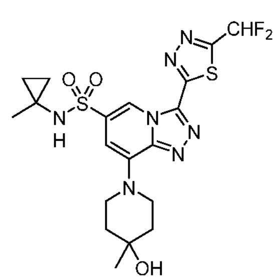 , 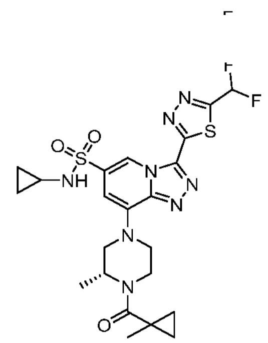 , 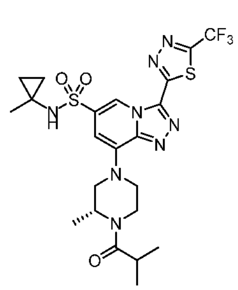 , 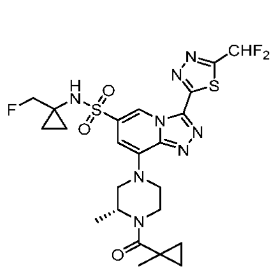 ,

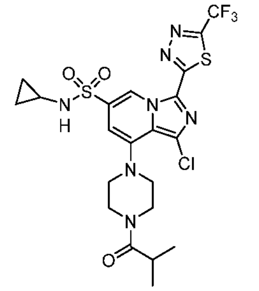 , 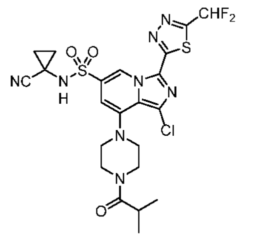 , 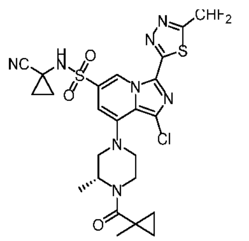 , 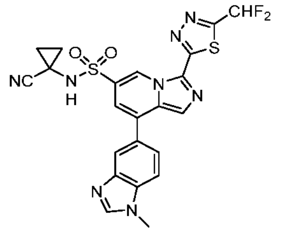 ,

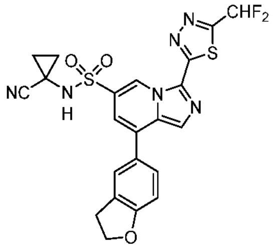 , 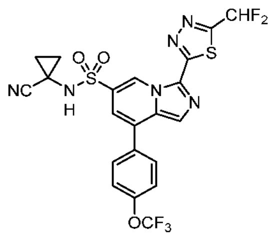 , 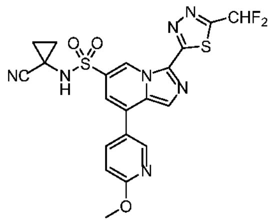 , 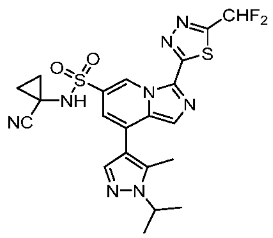 ,

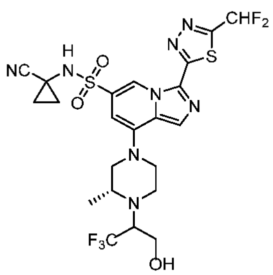 , 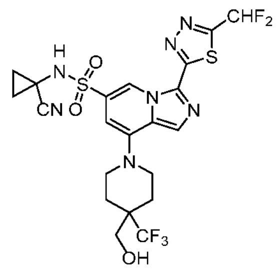 , 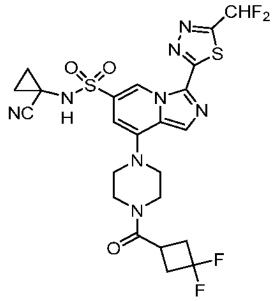 , 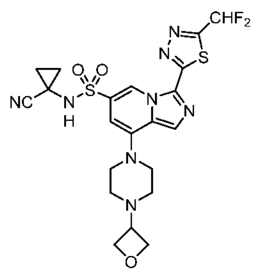 ,

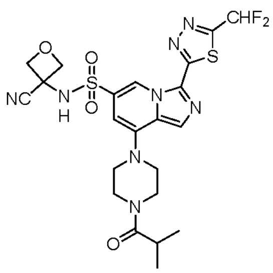 , 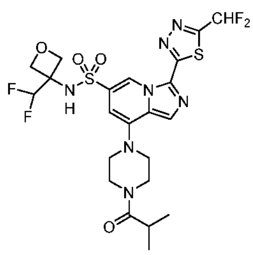 , 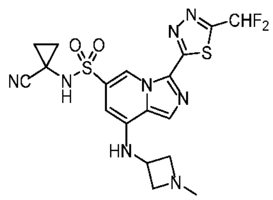 , 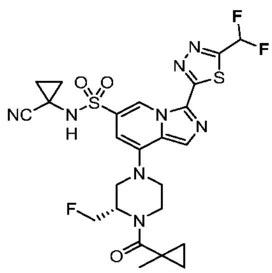 ,

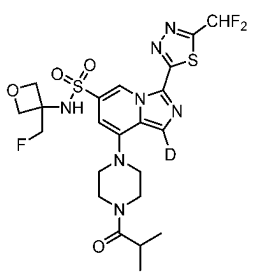 , 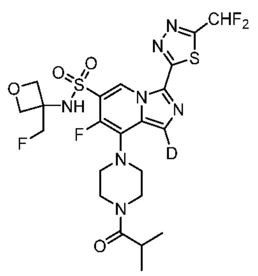 , 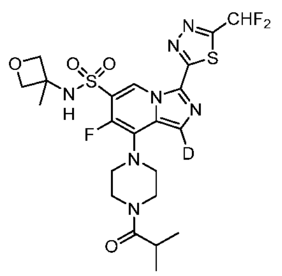 , 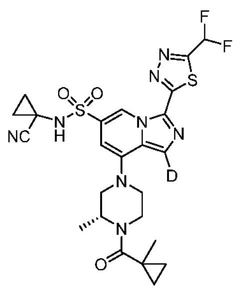 ,

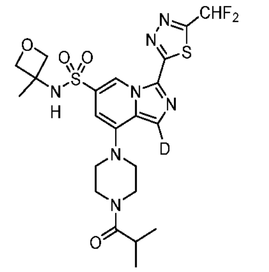 , 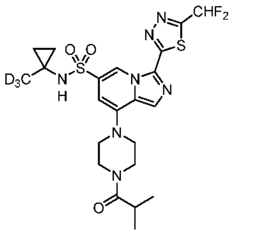 , 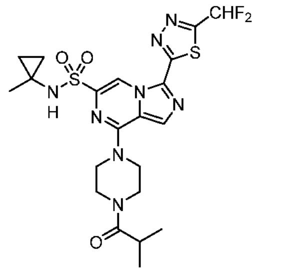 , 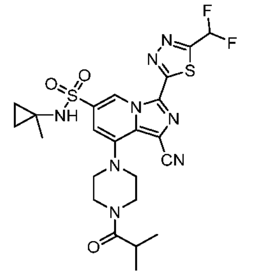 ,

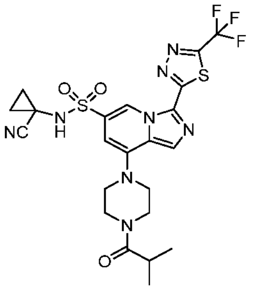 , 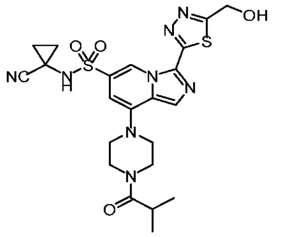 , 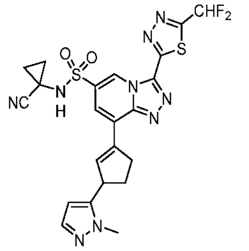 , 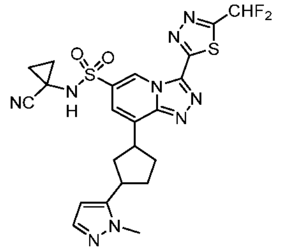 ,

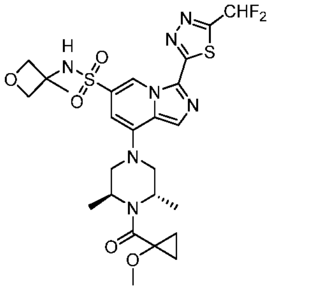 , 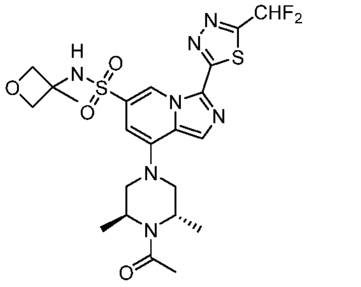 , 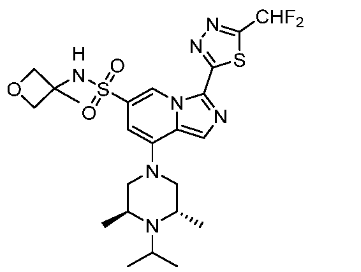 ,

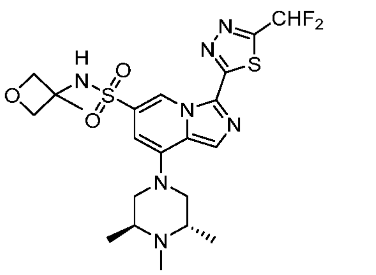 , 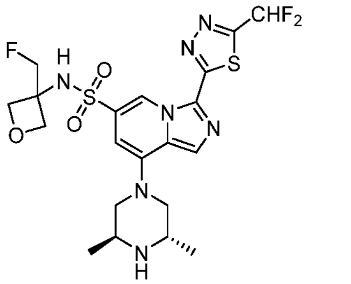 , 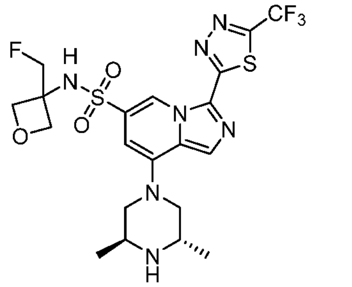 ,

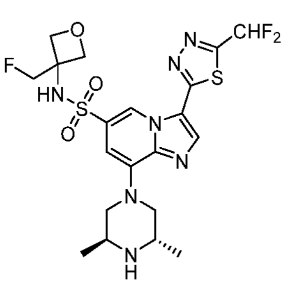 , 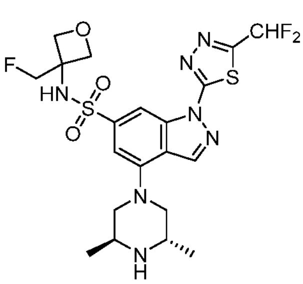 , 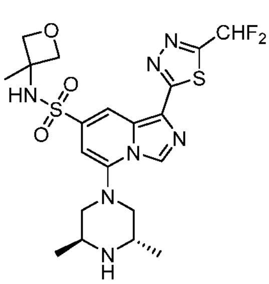 , 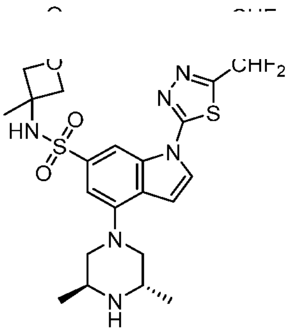 ,

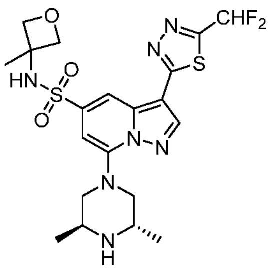
,
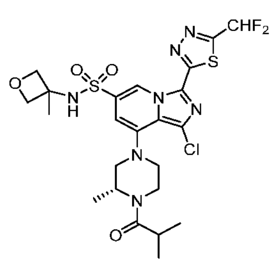
,
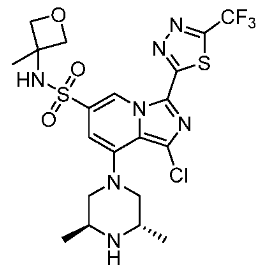
,
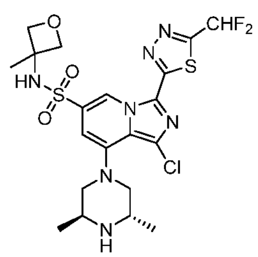
,

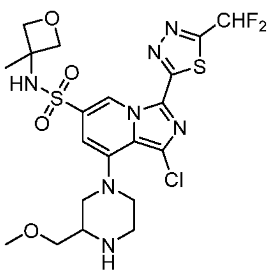
,
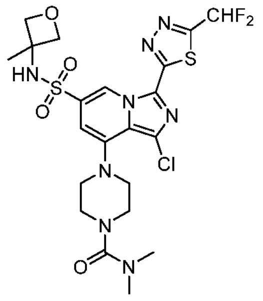
,
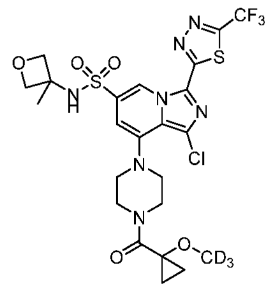
,
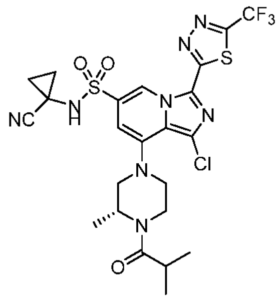
,

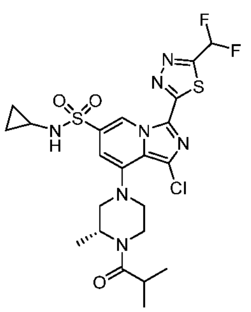
,
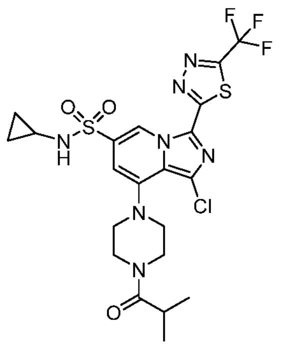
,
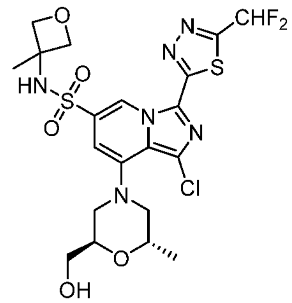
,
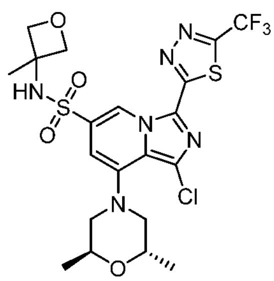
,

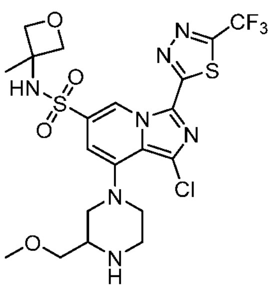
,
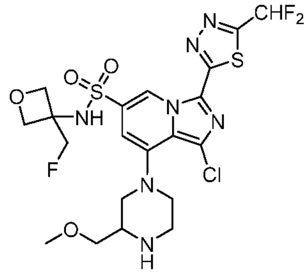
,
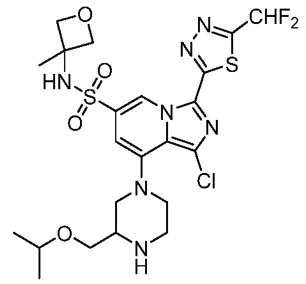
,

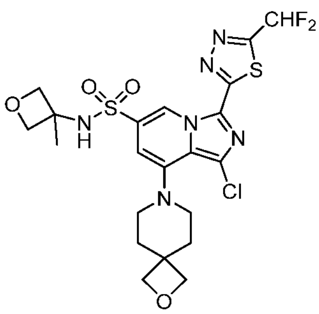
,
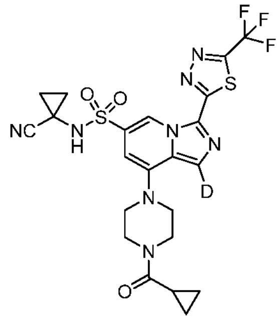
,
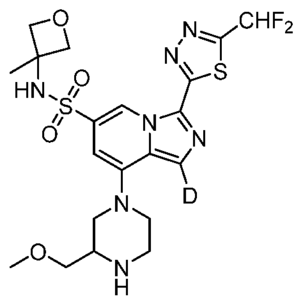
,
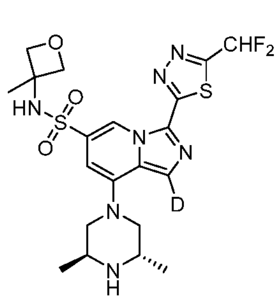
,

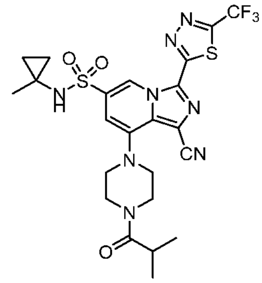,
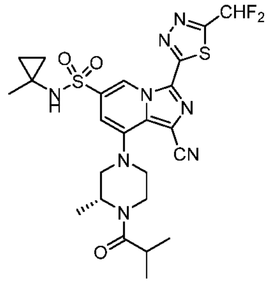,
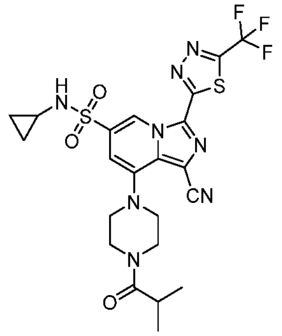,
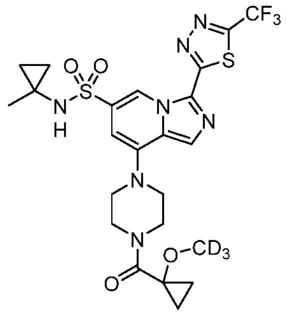,

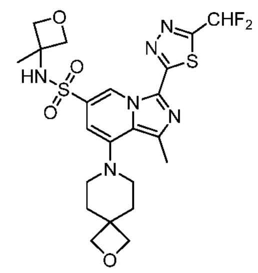,
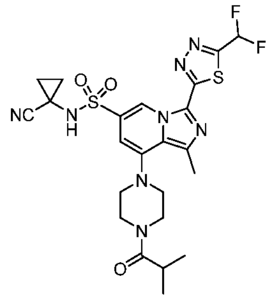,
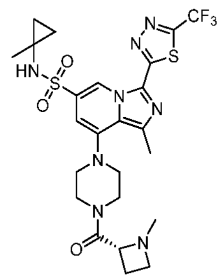,
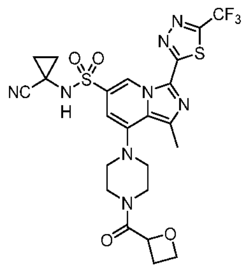,

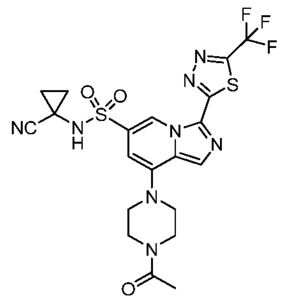,
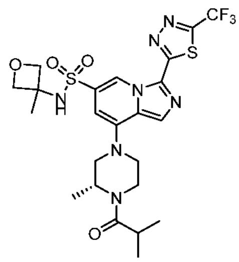,
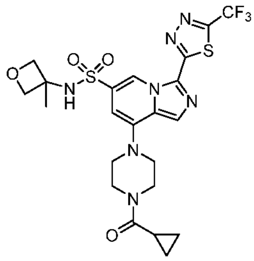,
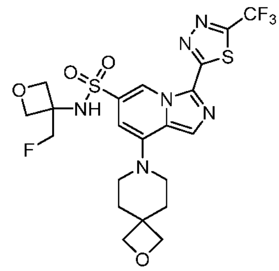,

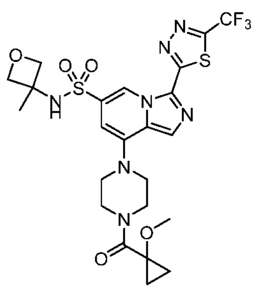,
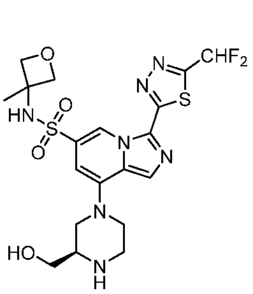,
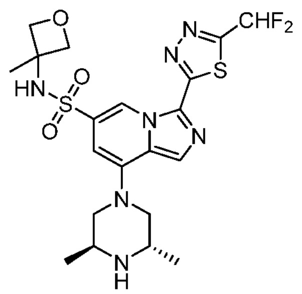,
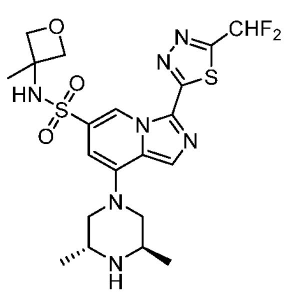,

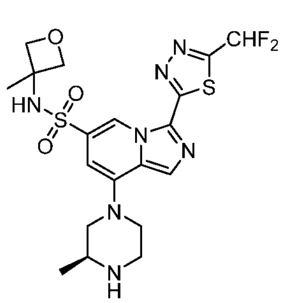,
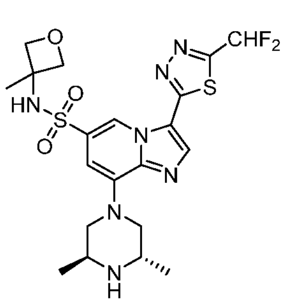,
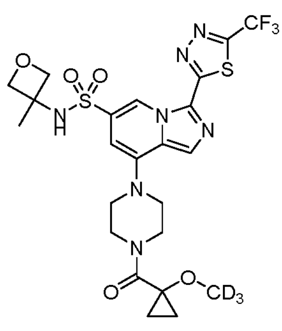,
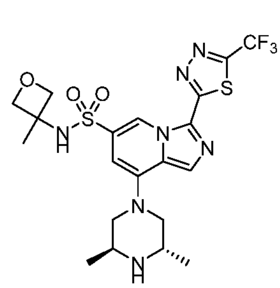,

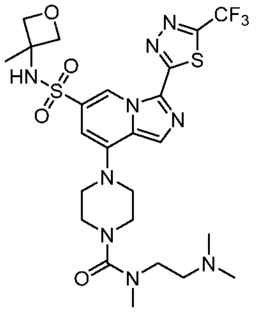 , 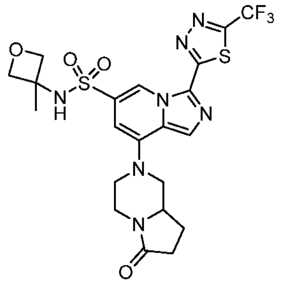 , 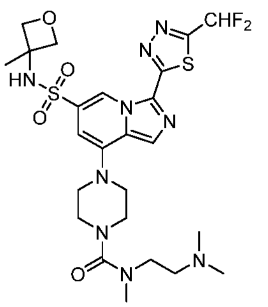 , 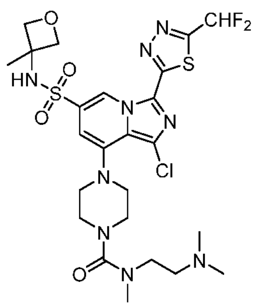 ,

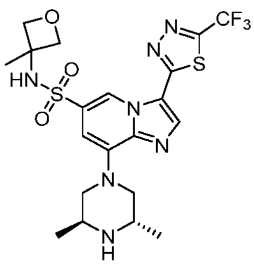 , 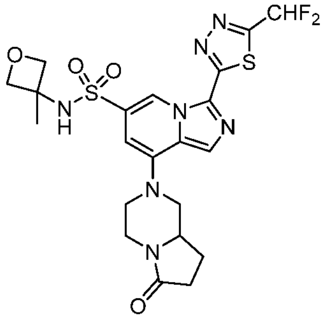 , 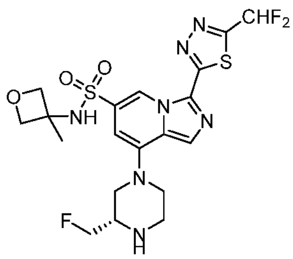 , 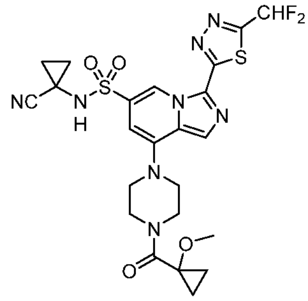 ,

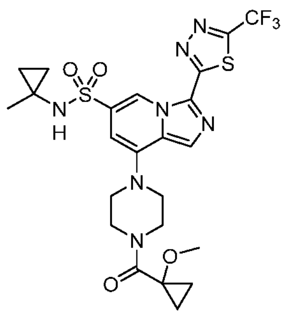 , 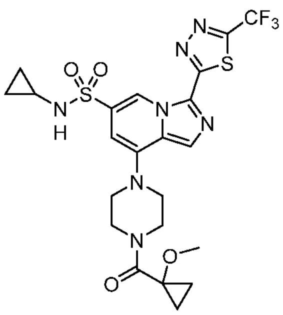 , 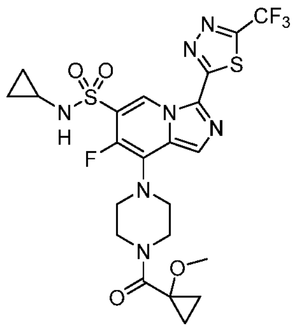 , 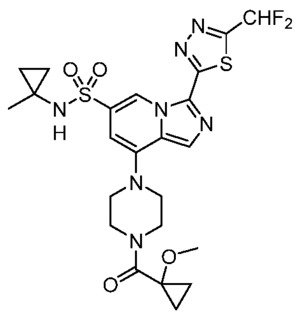 ,

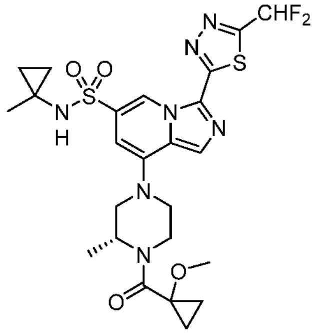 , 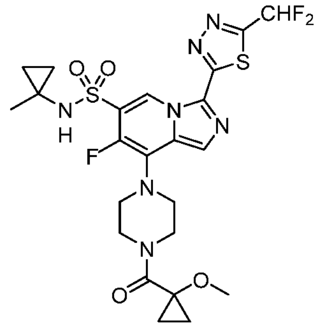 , 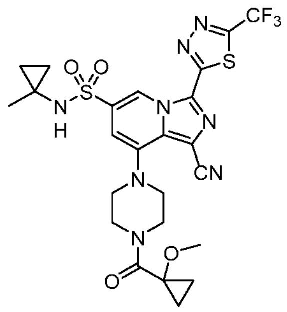 ,

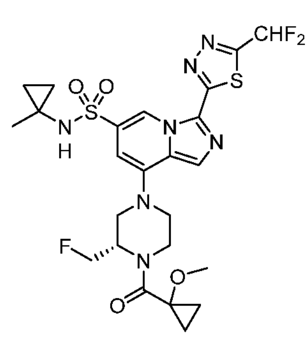,

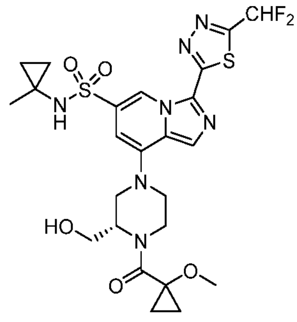,

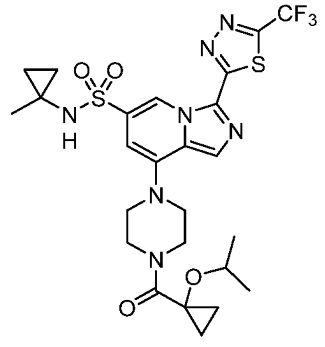,

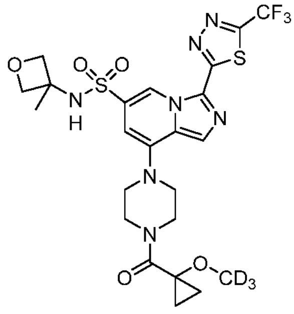,

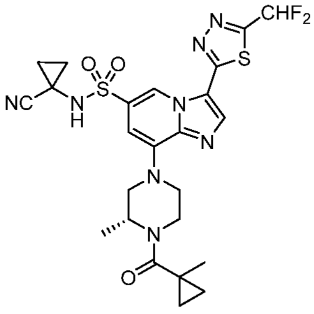,

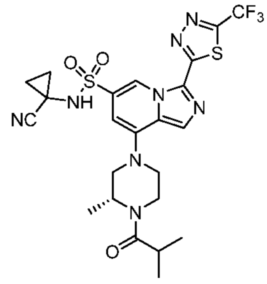,

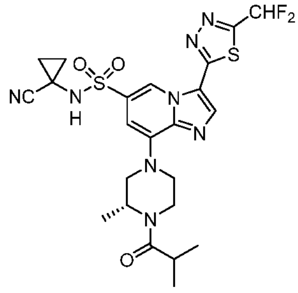,

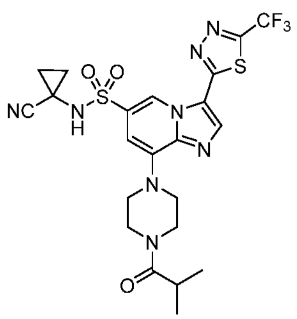,

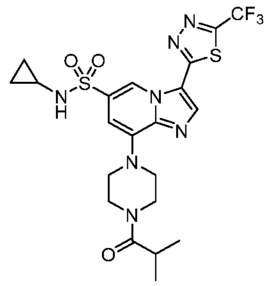,

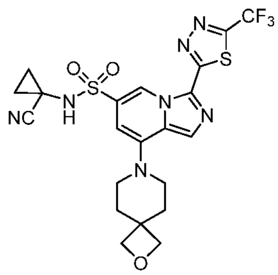,

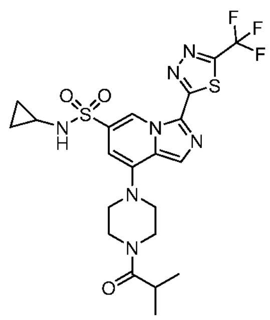,

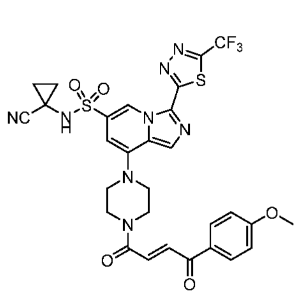,

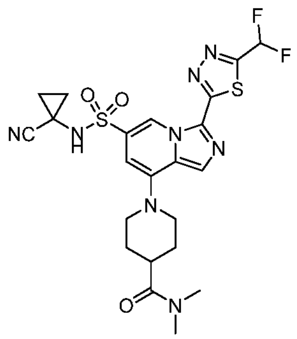,

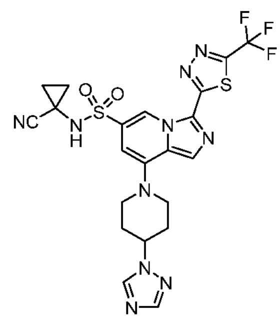,

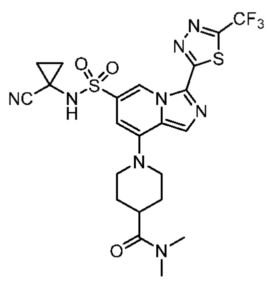,

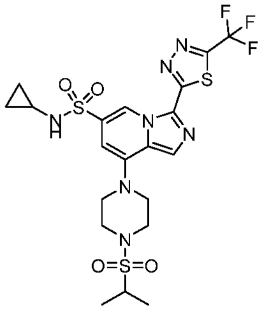,
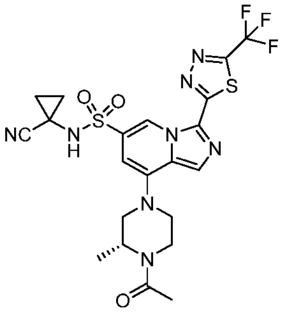,
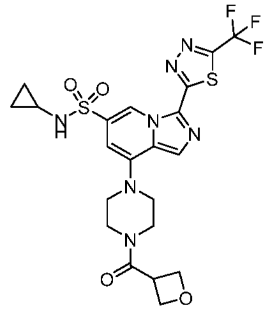,
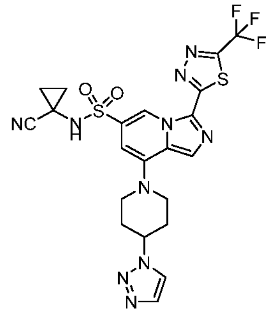,

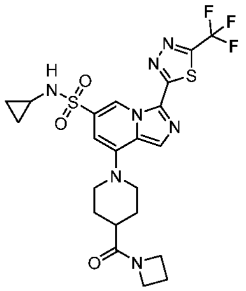,
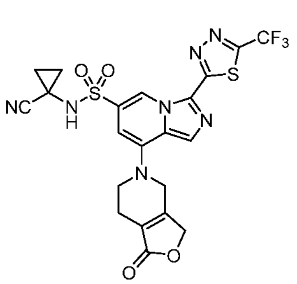,
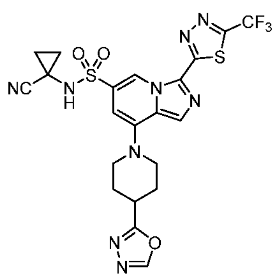,
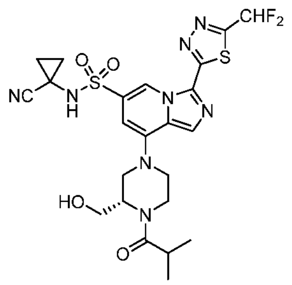,

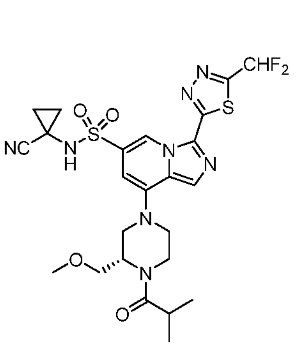,
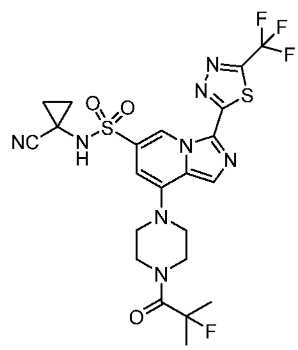,
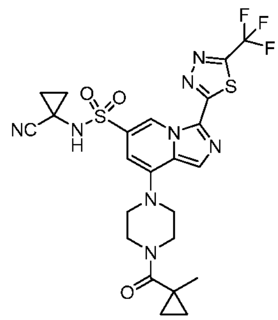,
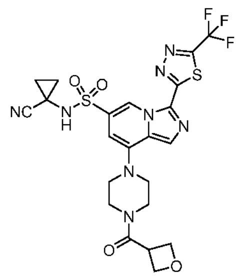,

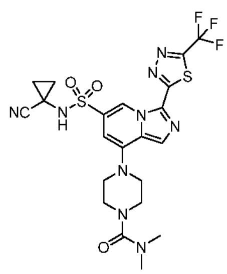,
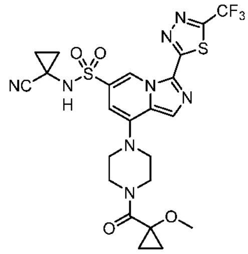,
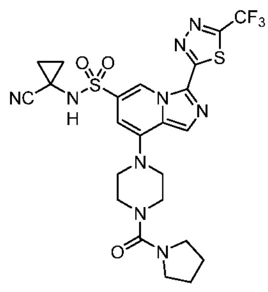,
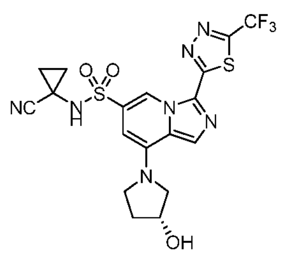,

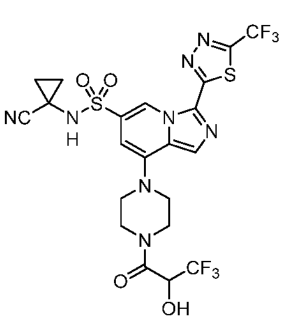,
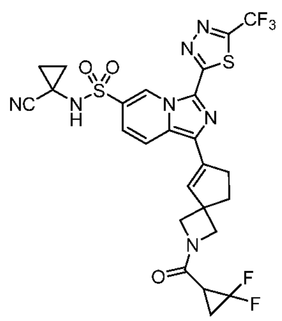,
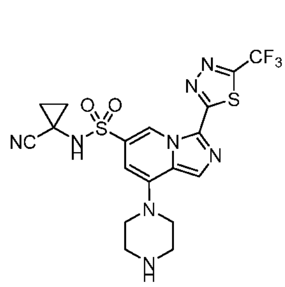,
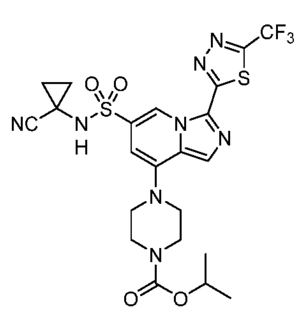,

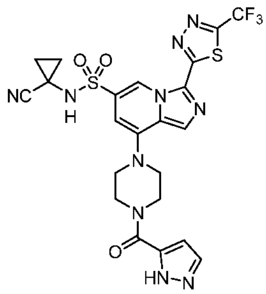,
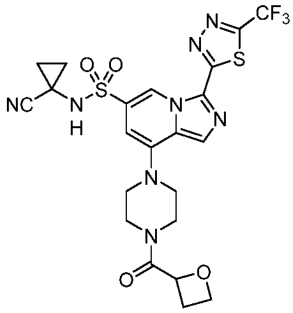,
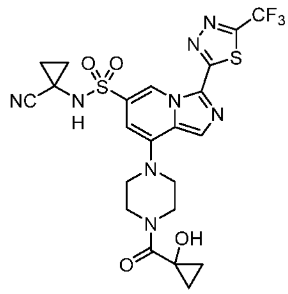,
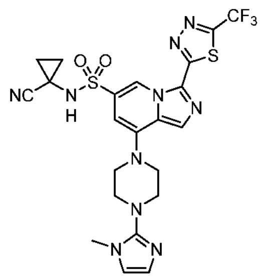,

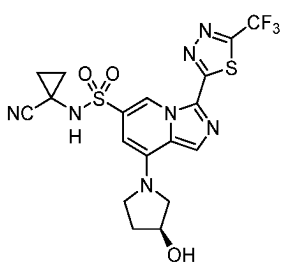,
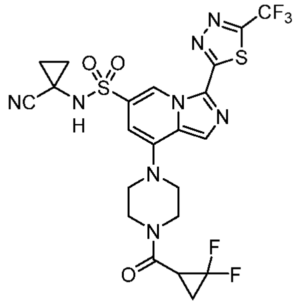,
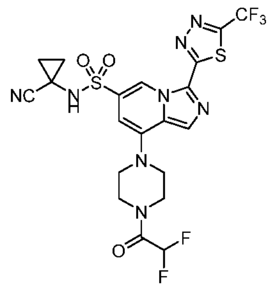,
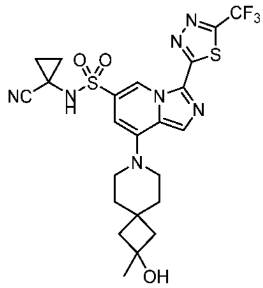,

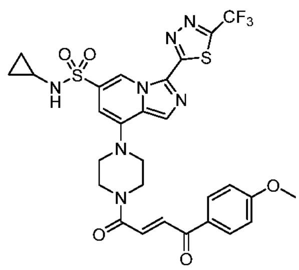,
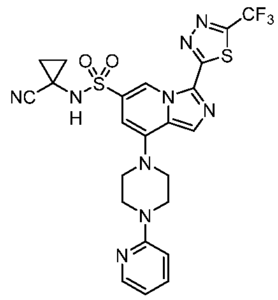,
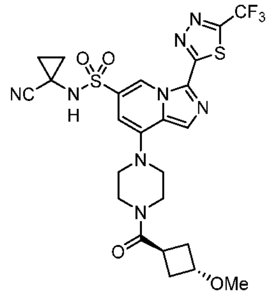,
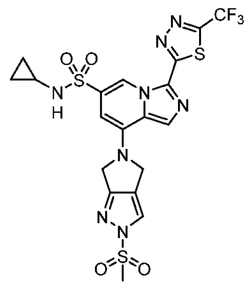,

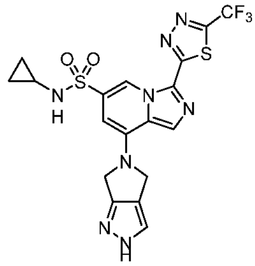,
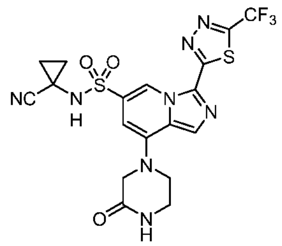,
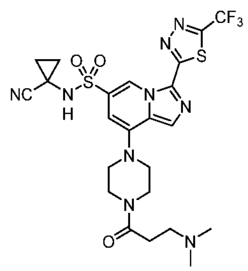,
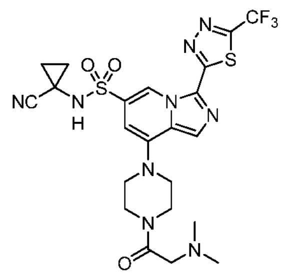,

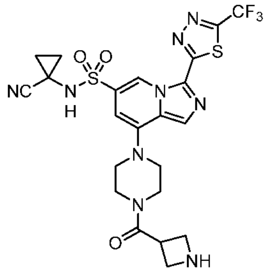,
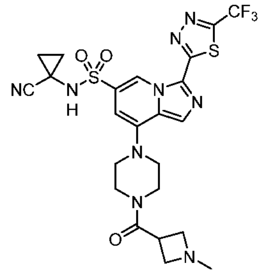,
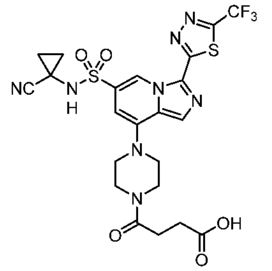,
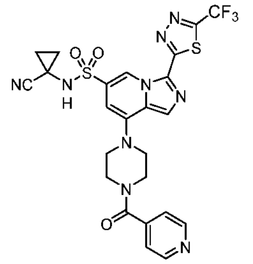,

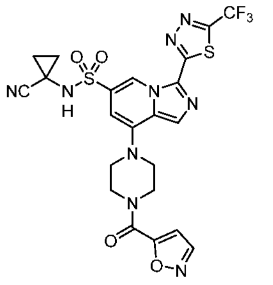 , 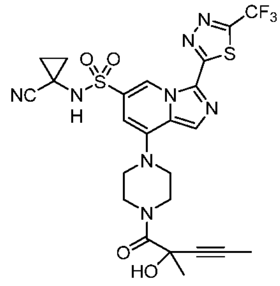 , 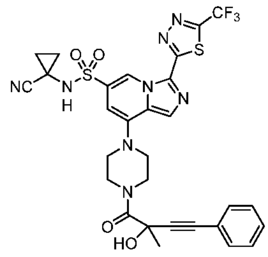 , 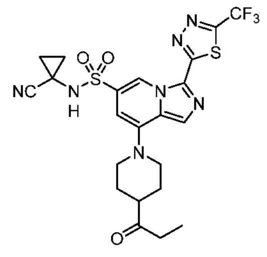 ,

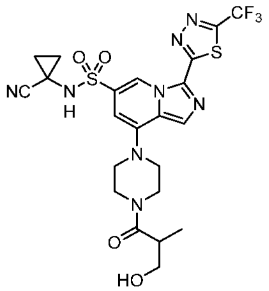 , 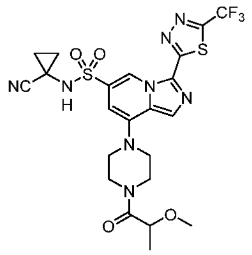 , 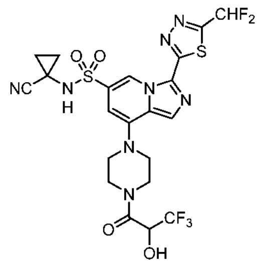 , 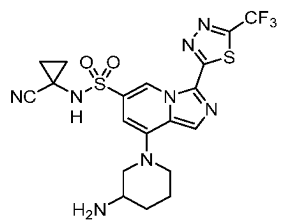 ,

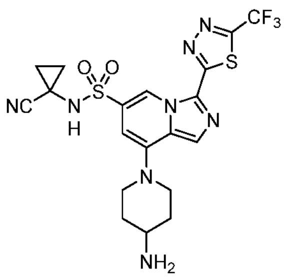 , 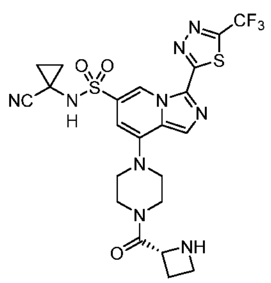 , 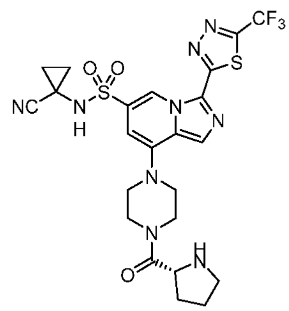 , 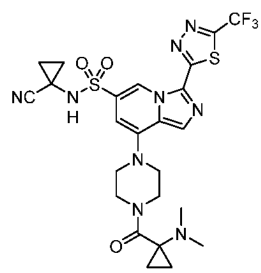 ,

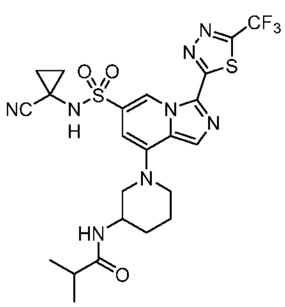 , 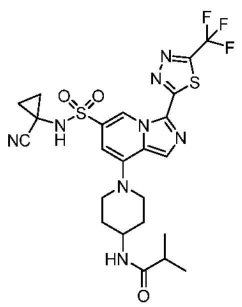 , 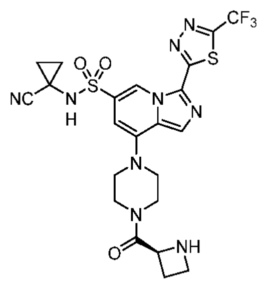 , 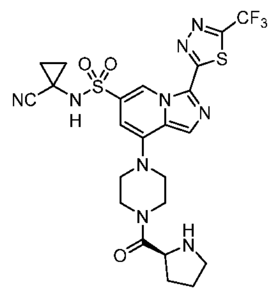 ,

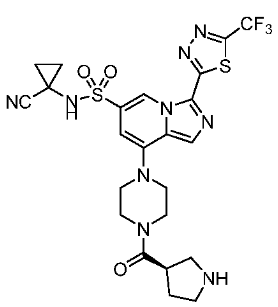 , 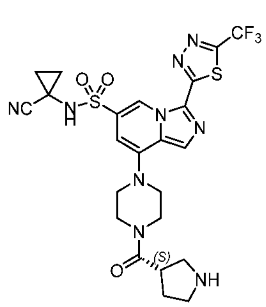 , 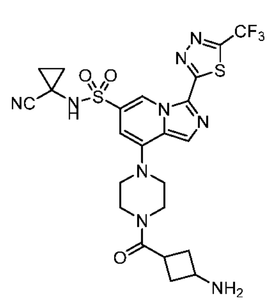 , 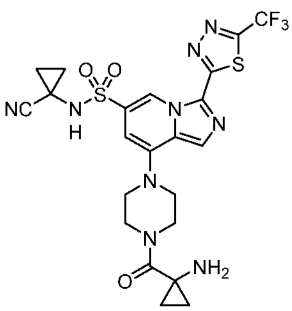 ,

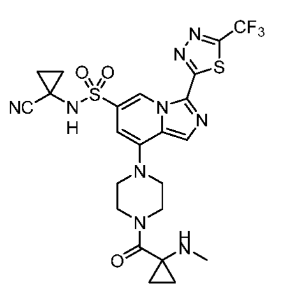,
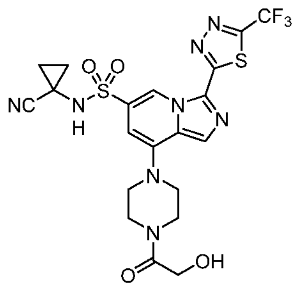,
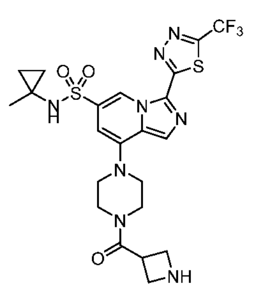,
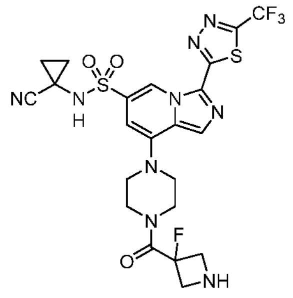,

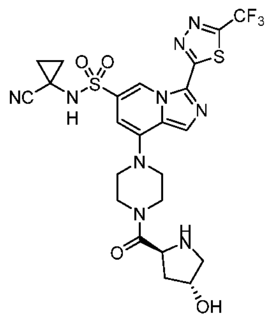,
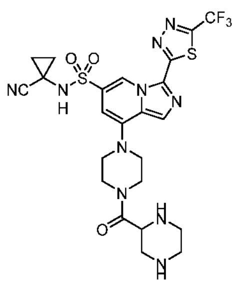,
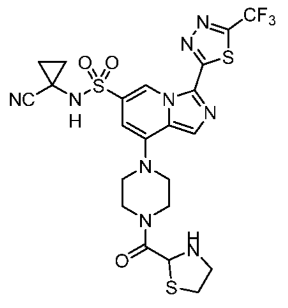,
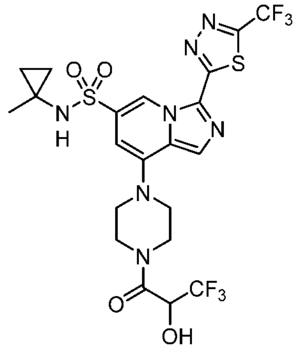,

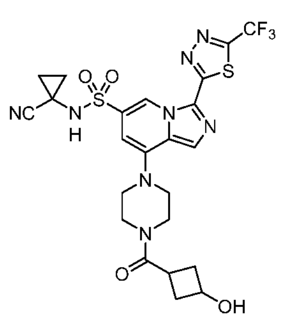,
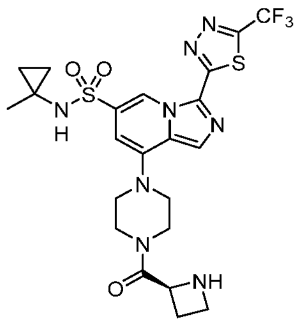,
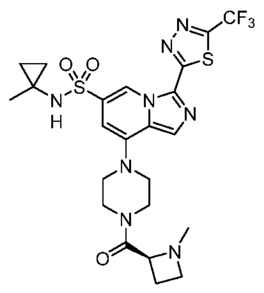,
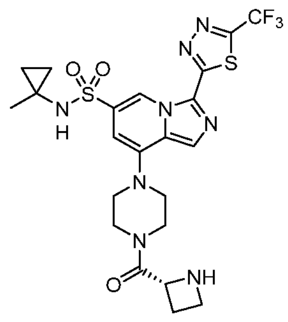,

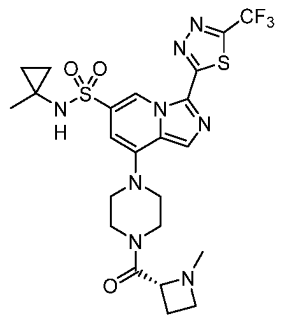,
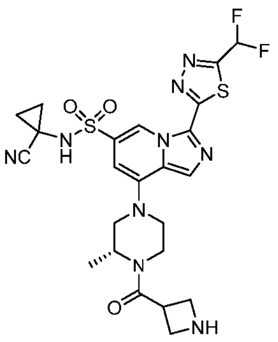,
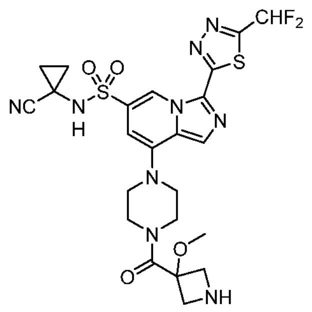,
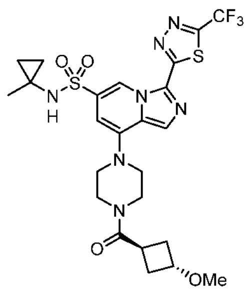,

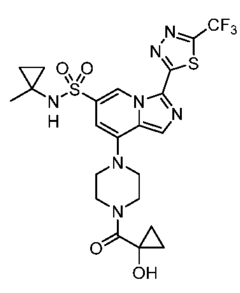,
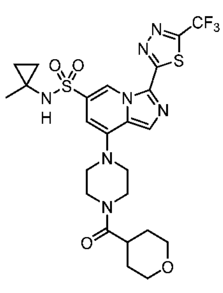,
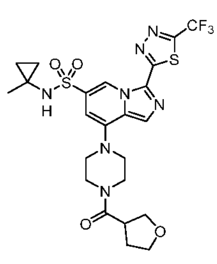,
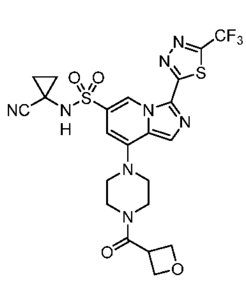,
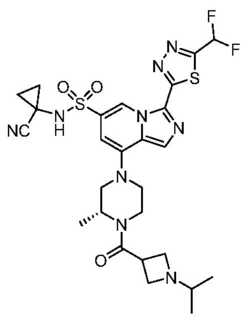,

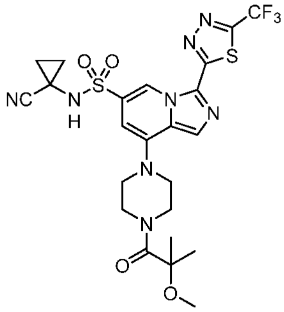 , 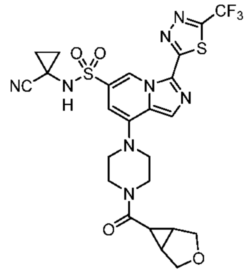 , 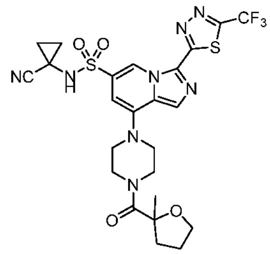 , 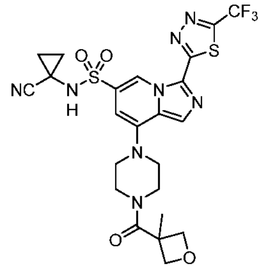 ,

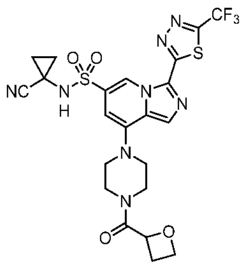 , 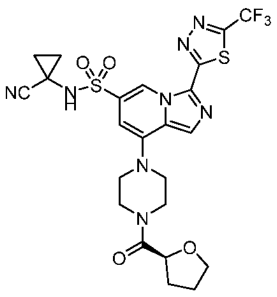 , 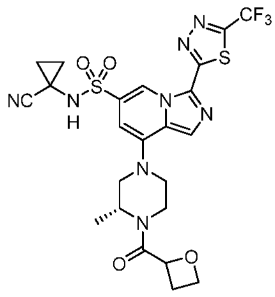 , 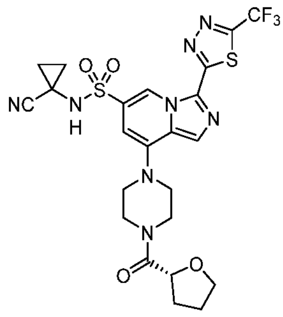 ,

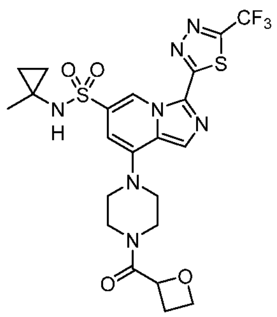 , 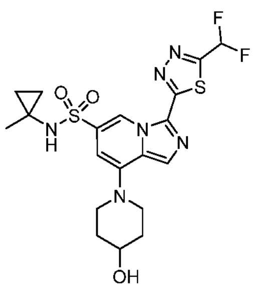 , 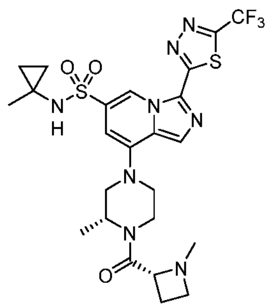 , 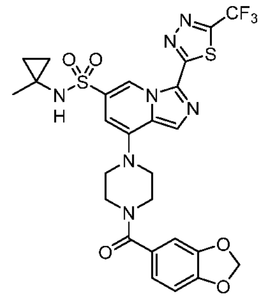 ,

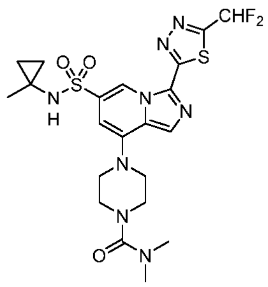 , 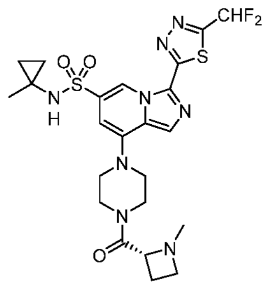 , 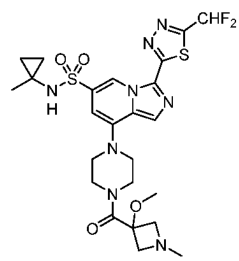 , 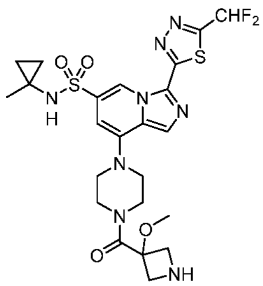 ,

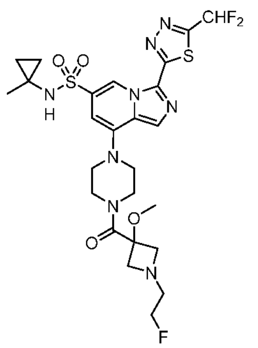 , 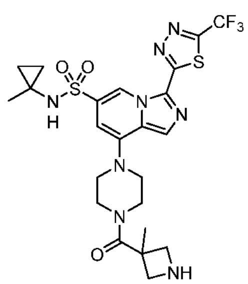 , 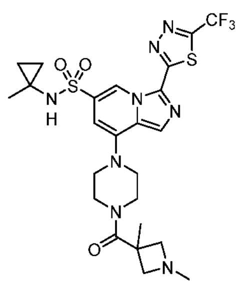 , 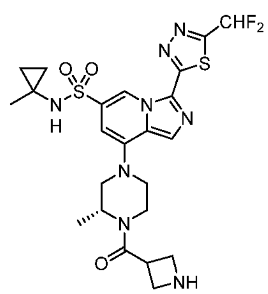 ,

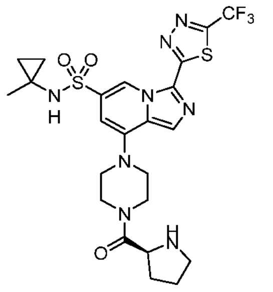 , 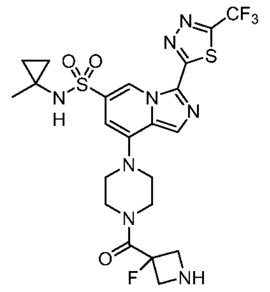 , 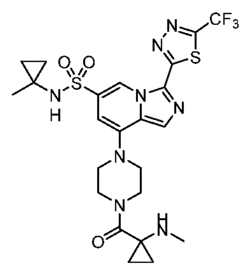 , 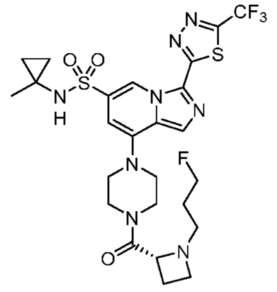 ,

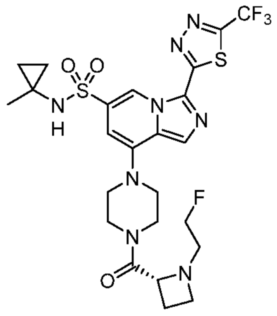 , 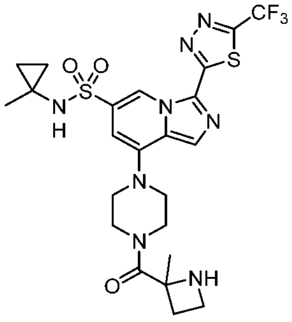 , 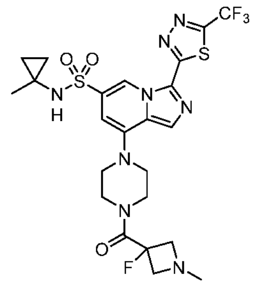 , 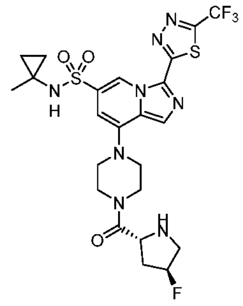 ,

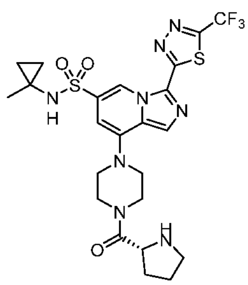 , 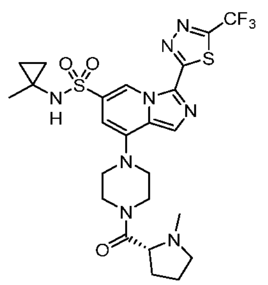 , 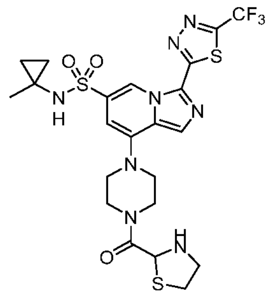 , 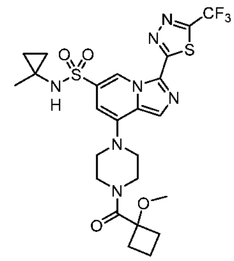 ,

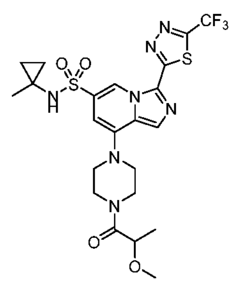 , 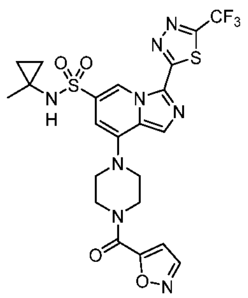 , 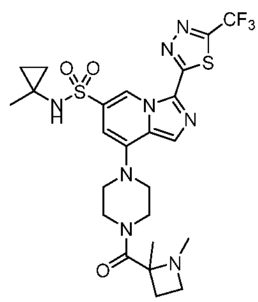 , 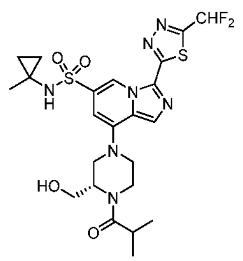 ,

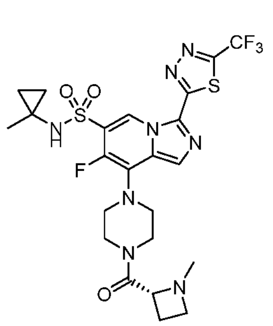 , 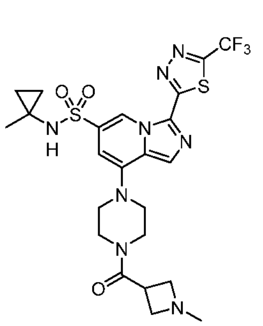 , 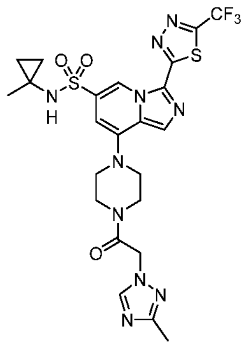 , 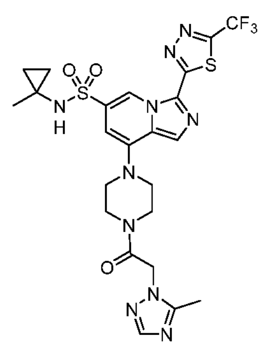 ,

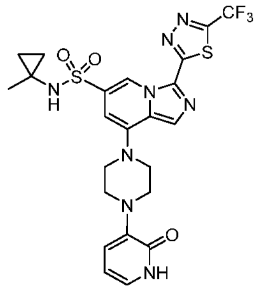
,
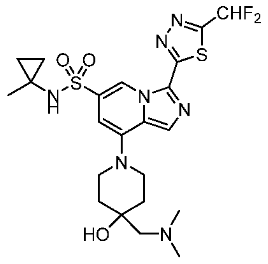
,
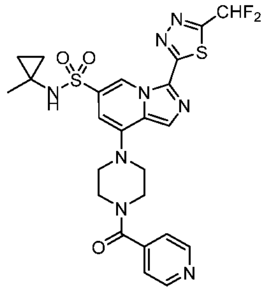
,
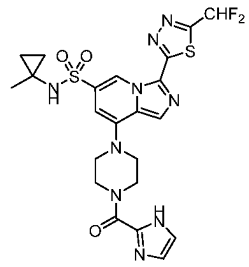
,

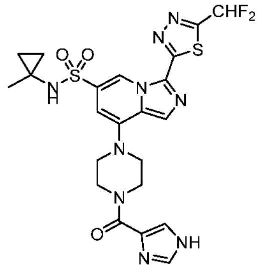
,
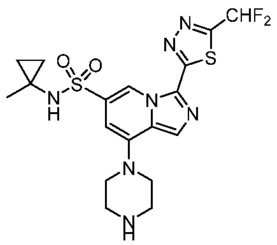
,
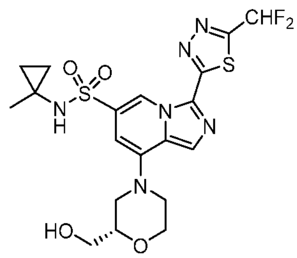
,
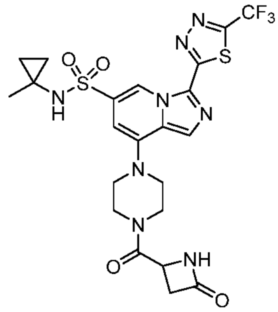
,

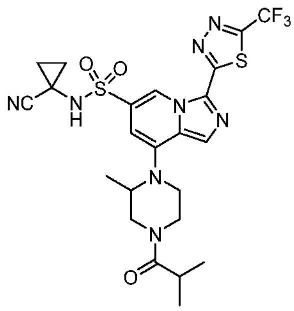
,
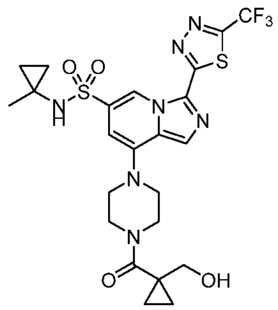
,
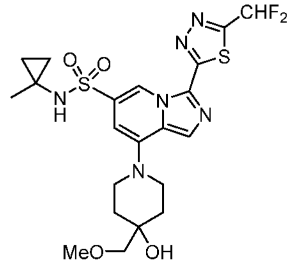
,
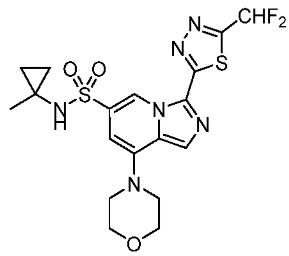
,

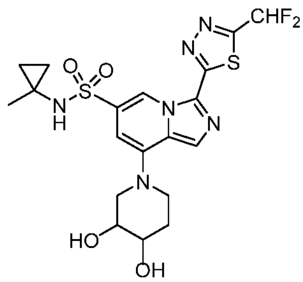
,
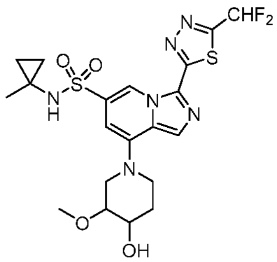
,
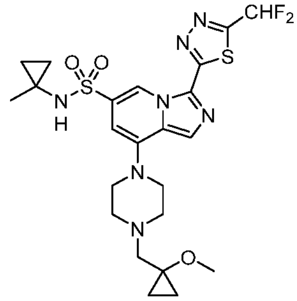
,
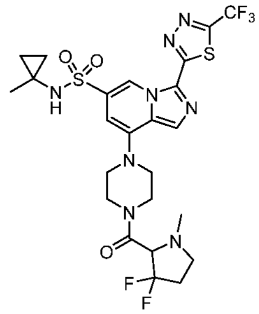
,

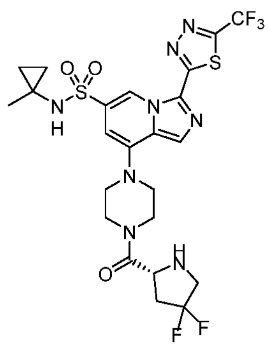
,
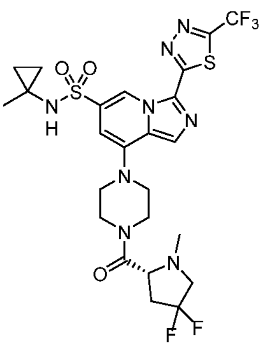
,
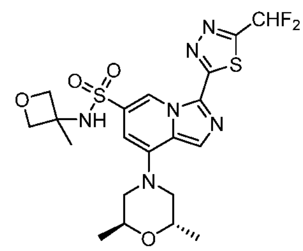
,
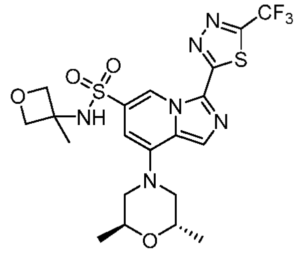
,

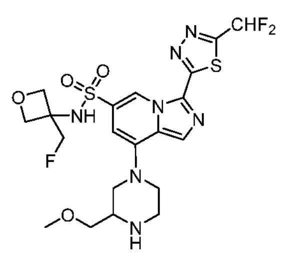,
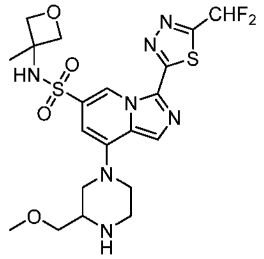,
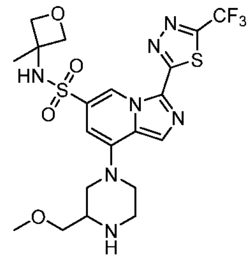,
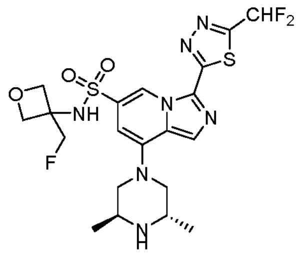,

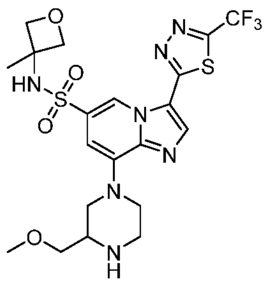,
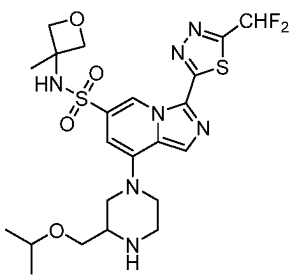,
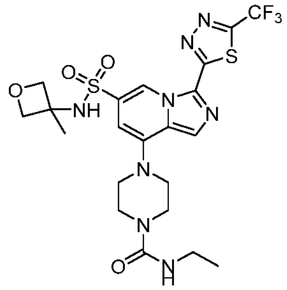,
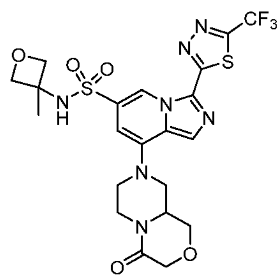,

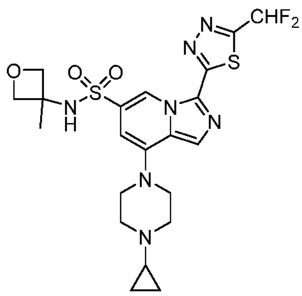,
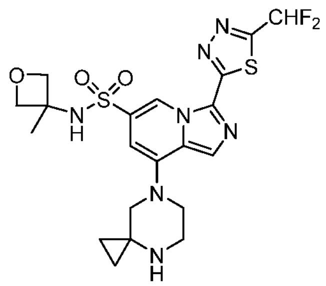,
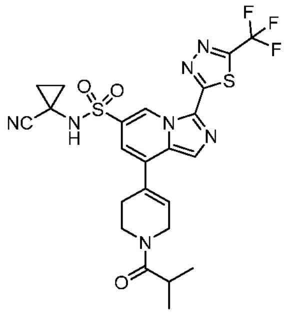,
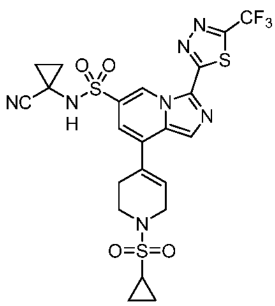,

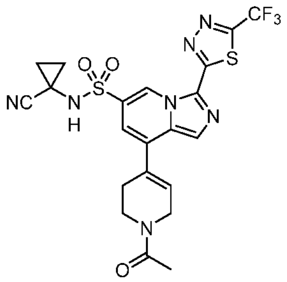,
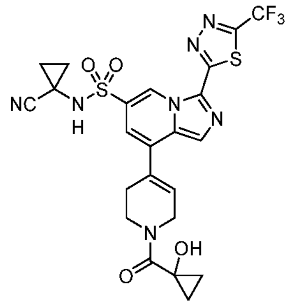,
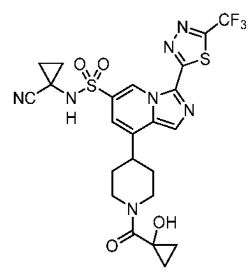,
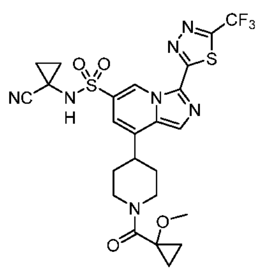,

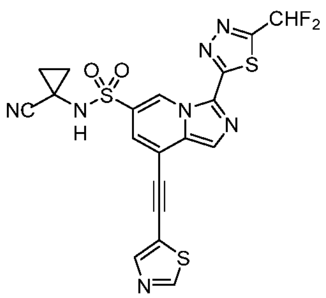,
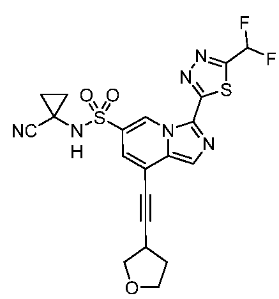,
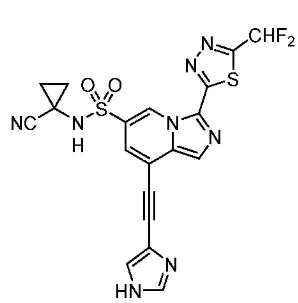,
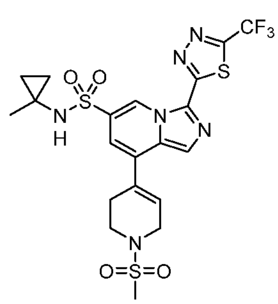,

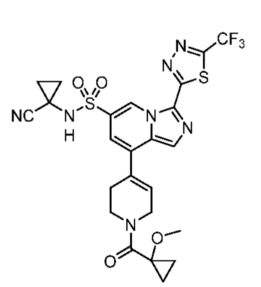,
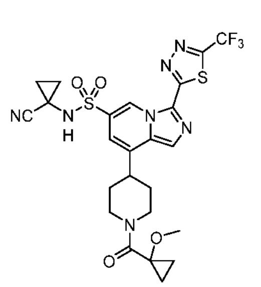,
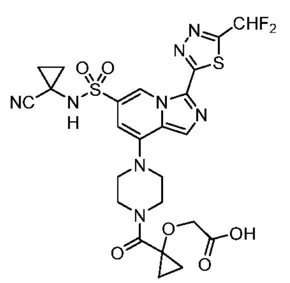,
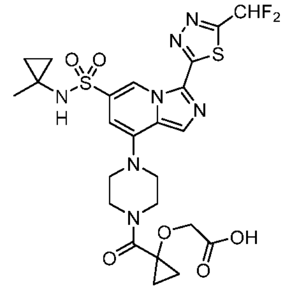,

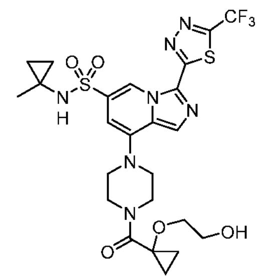,
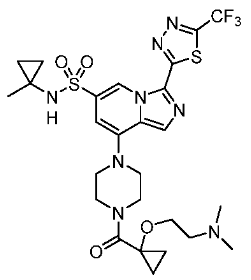,
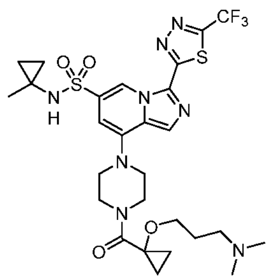,
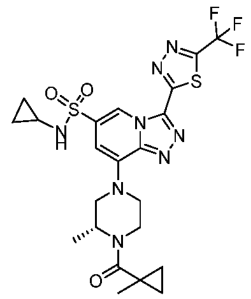,

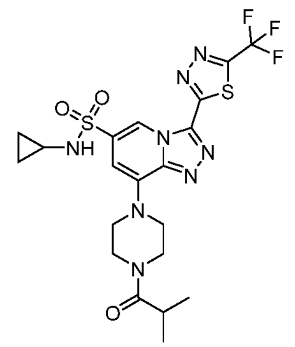,
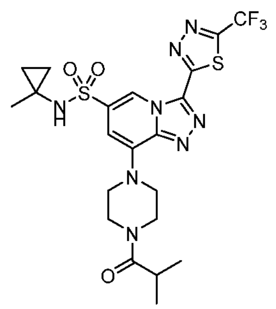,
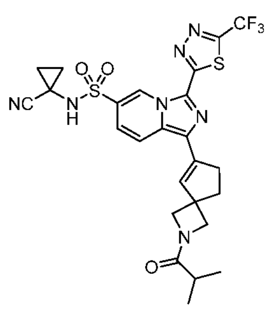,
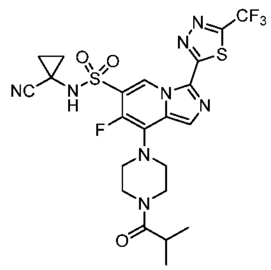,

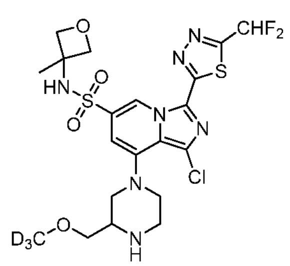,
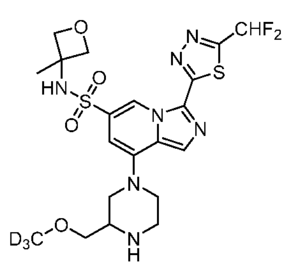,
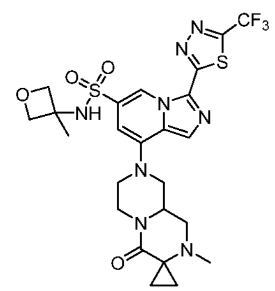,
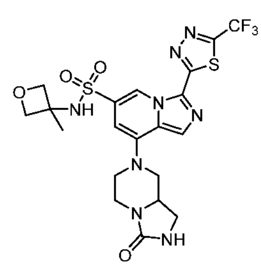,

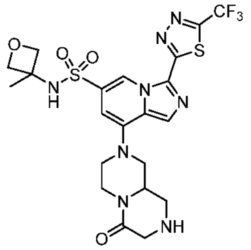,
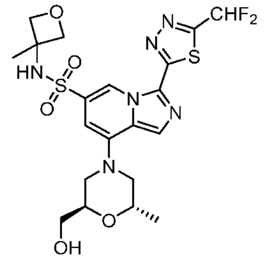,
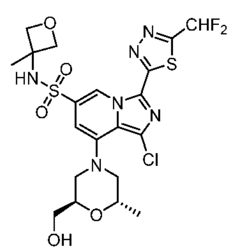,
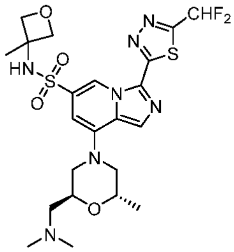,

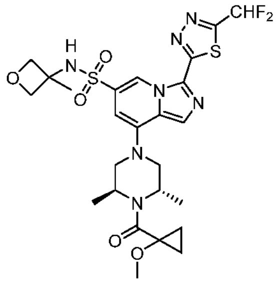, 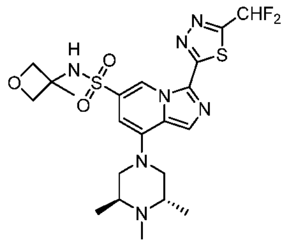, 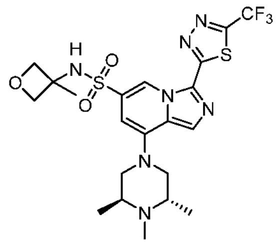, 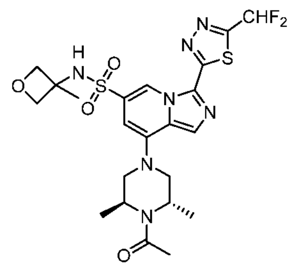,

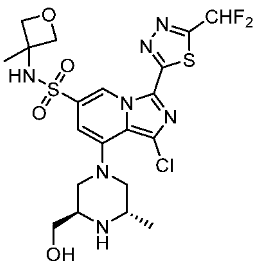, 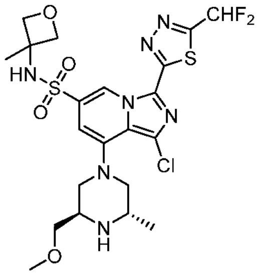 or 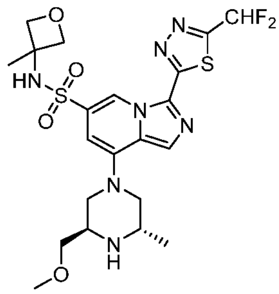

The present invention further provides a pharmaceutical composition, comprising a substance Z and a pharmaceutical excipient, wherein the substance Z is a compound of formula I, a pharmaceutically acceptable salt thereof or an isotopic compound thereof.

The present invention further provides use of a substance Z in preparation of a PARG inhibitor and a medicament for treating and/or preventing PARG-related diseases, wherein the substance Z is a compound of formula I, a pharmaceutically acceptable salt thereof or an isotopic compound thereof.

The present invention further provides a method for treating and/or preventing PARG-related diseases, comprising administering to a patient an effective amount of a substance Z, which is a compound of formula I, a pharmaceutically acceptable salt thereof or an isotopic compound thereof.

In a preferred embodiment, the PARG-related diseases can be breast cancer, for example, triple negative breast cancer, and other diseases associated with PARG.

The present invention further provides use of a substance Z in preparation of a medicament for treating and/or preventing breast cancer, wherein the substance Z is a compound of formula I, a pharmaceutically acceptable salt thereof or an isotopic compound thereof.

In a preferred embodiment, the breast cancer can be triple negative breast cancer.

The present invention further provides a compound of formula II, III, IV or V or a salt thereof,

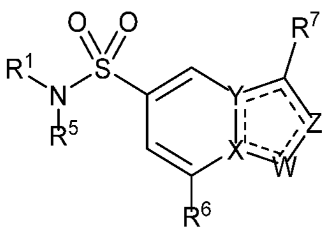
II ,

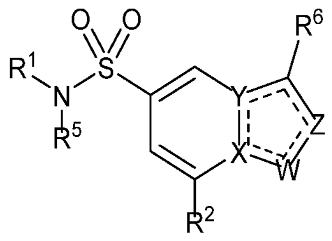
III ,

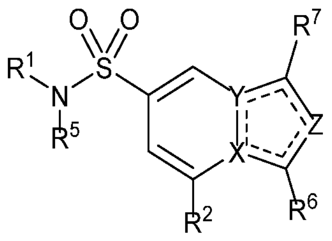
IV , or

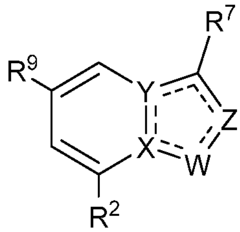
V ,

wherein, $R^5$ is hydrogen or PMB; $R^6$ is halogen or deuterium; $R^7$ is $R^3$, hydrogen or or $C_1$-$C_6$ alkyl; $R^9$

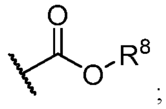

$R^8$ is hydrogen
is

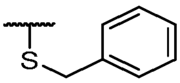, 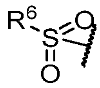

or halogen;
$R^1$, $R^2$, $R^3$, X, Y, Z and W are as previously defined.

The present invention further provides a compound of formula II, III, IV or V or a salt thereof,

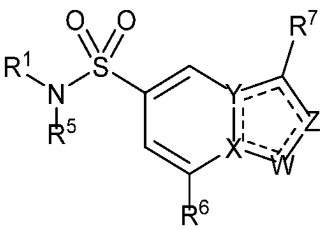
II ,

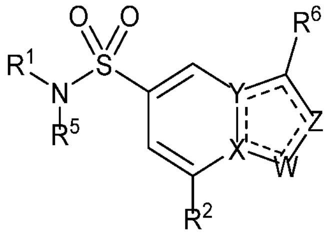
III ,

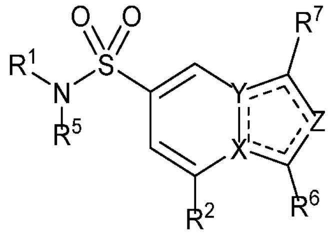
IV , or

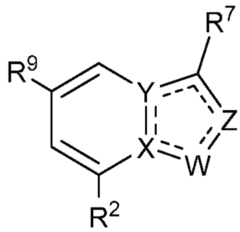
V ,

wherein, $R^5$ is hydrogen or PMB; $R^6$ is halogen; $R^7$ is $R^3$, hydrogen or

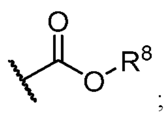

$R^8$ is hydrogen or $C_1$-$C_6$ alkyl;

$R^9$ is 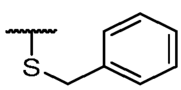, 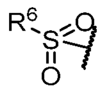

or halogen; and
$R^1$, $R^2$, $R^3$, X, Y, Z and W are as previously defined.

In a preferred embodiment, in $R^6$, the halogen can be fluorine, chlorine, bromine or iodine, for example, chlorine or bromine.

In a preferred embodiment, in $R^8$, the $C_1$-$C_6$ alkyl can be $C_1$-$C_4$ alkyl, and can also be methyl, ethyl, n-propyl, isopropyl, n-butyl, isobutyl, pr-butyl, sec-butyl or tert-butyl, or can be methyl or ethyl.

In a preferred embodiment, in $R^9$, the halogen can be fluorine, chlorine, bromine or iodine, for example, chlorine or bromine.

In a preferred embodiment, the compound of formula II is of any one of the following structures:

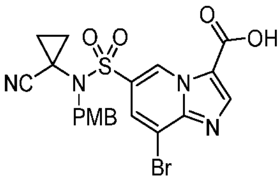, 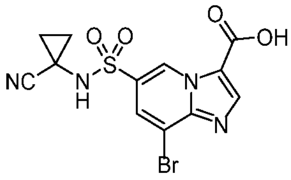, 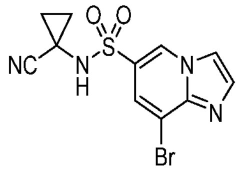, 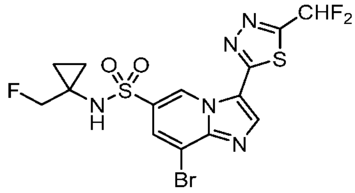,

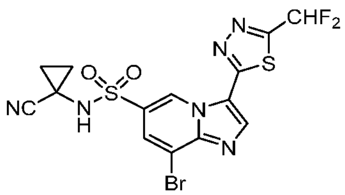, 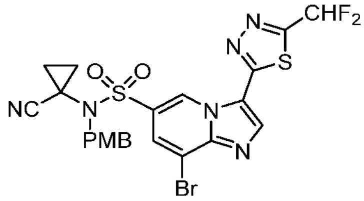, 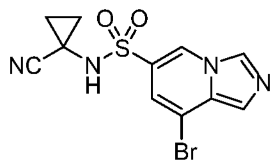,

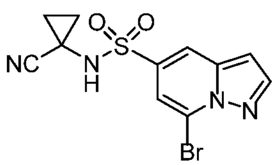, 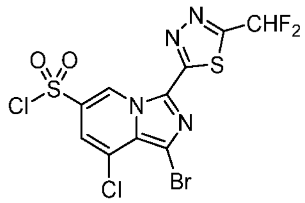, 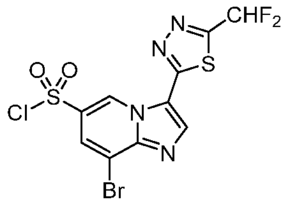,

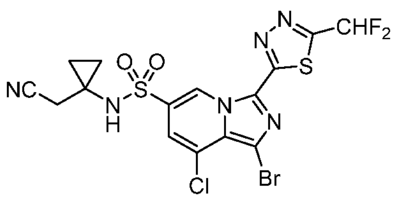, 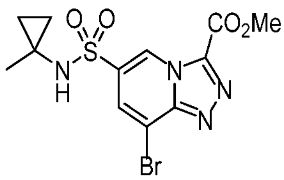, 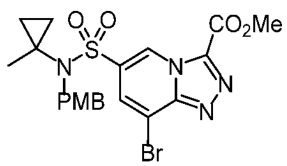,

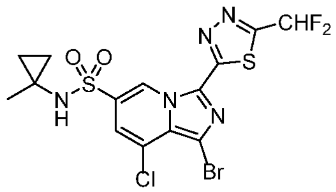, 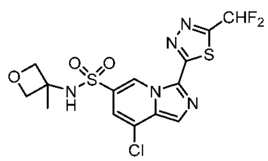, 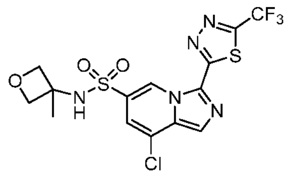, 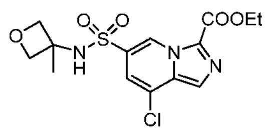,

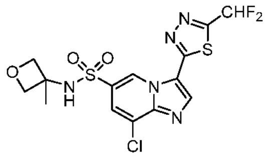, 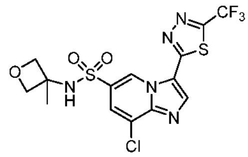, 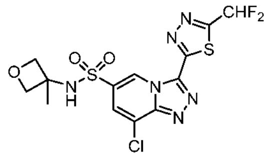, 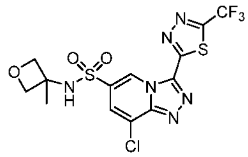,

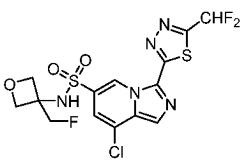 or 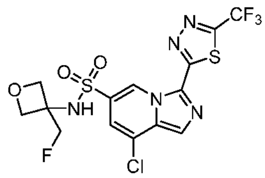

In a preferred embodiment, the compound of formula III is of any one of the following structures:

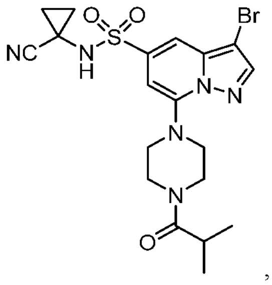, 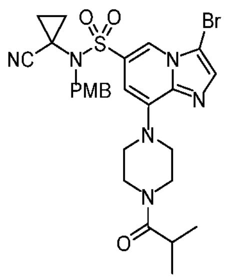 or 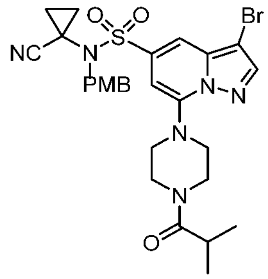

In a preferred embodiment, the compound of formula IV is of any one of the following structures:

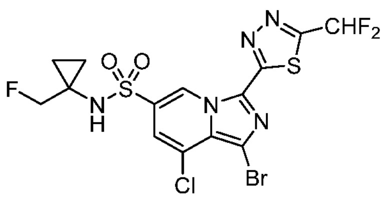, 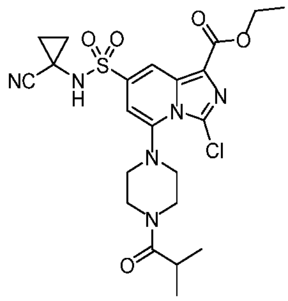, 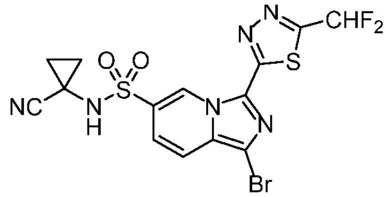,

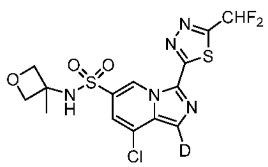, 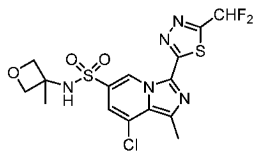, 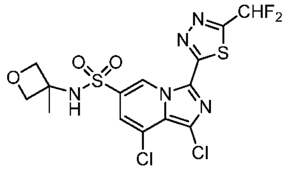, 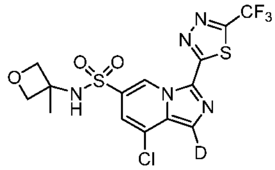,

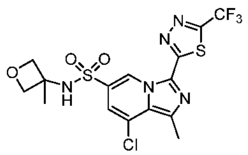, 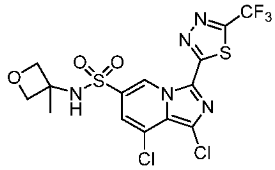, 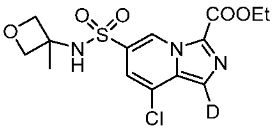, 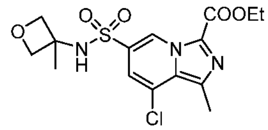,

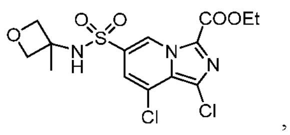, 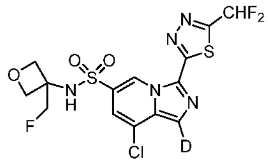, 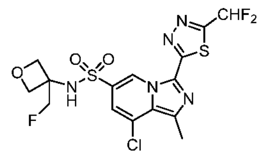, 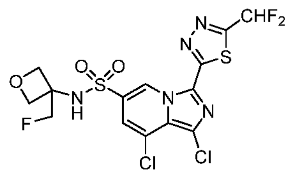,

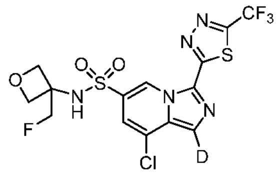, 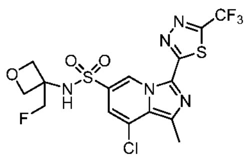 or 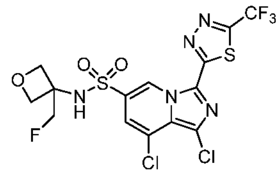

In a preferred embodiment, the compound of formula V is of any one of the following structures:

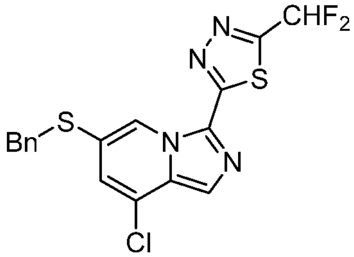

,

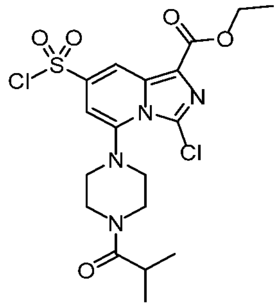

,

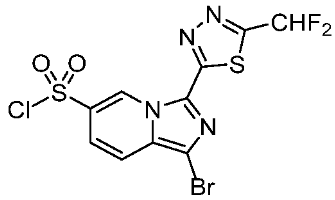

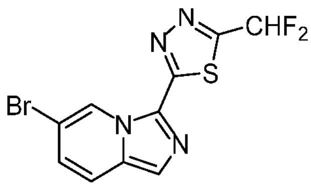

or

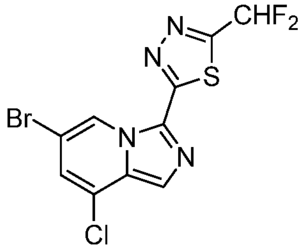

Interpretation of Terms

The term "a group B unsubstituted or substituted by more than one group A" means that one or more hydrogen atoms in the group B are independently substituted by the group A, or B is not substituted. When a plurality of groups A appear at the same time, their definitions are independent of each other without mutual influence, unless otherwise specified. For example, "$C_6$-$C_{1o}$ aryl substituted by 3 halogens" means that $C_6$-$C_{10}$ aryl is to be substituted by 3 halogens, the definitions of the 3 halogens are independent of each other without mutual influence, and the halogens include but are not limited to:

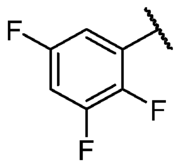

,

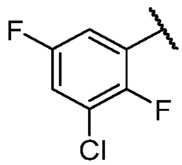

or the like.



has a double bond or a single bond.

The term "a plurality of" means 2 or more, for example 2, 3, 4, or 5.

The term "pharmaceutically acceptable" means a relatively non-toxic and safe condition suitable for use in patients.

The term "pharmaceutically acceptable salt" means the salt obtained by reacting a compound with a pharmaceutically acceptable acid or alkali. When the compound contains relatively acidic functional groups, an alkali addition salt may be obtained by contacting the compound with a sufficient amount of a pharmaceutically acceptable alkali in a suitable inert solvent. The pharmaceutically acceptable alkali addition salt includes, but is not limited to: a sodium salt, a potassium salt, a calcium salt, an aluminum salt, a magnesium salt, a bismuth salt, an ammonium salt or the like. When the compound

contains relatively alkaline functional groups, an acid addition salt may be obtained by contacting the compound with a sufficient amount of a pharmaceutically acceptable acid in a suitable inert solvent. The pharmacologically acceptable acid addition salt includes, but is not limited to: hydrochloride, sulfate, formate, mesylate or the like. For details, see Handbook of Pharmaceutical Salts: Properties, Selection, and Use (P. Heinrich Stahl, Camille G. Wermuth, 2011, 2nd Revised Edition).

"-" in the group means the fact that the group is attached to the rest of the molecule via this site. For example, $CH_3$-C(=O)- means acetyl.

The term "halogen" means fluorine, chlorine, bromine or iodine. Halogen substitution in the present invention includes, but is not limited to, substitution by one, two, or three halogens, and in general, multiple substitutions occur on one carbon atom.

The term "oxo" means =O, indicating the substitution of two hydrogen on the same carbon atom by an oxygen atom. That is, a methylene group is substituted by a carbonyl group.

The term "alkyl" means a linear or branched saturated monovalent hydrocarbon group with a specified number of carbon atoms (for example, $C_1$-$C_6$). Alkyl groups include, but are not limited to: methyl, ethyl, n-propyl, isopropyl, n-butyl, isobutyl, sec-butyl, tert-butyl, n-amyl, n-hexyl or the like.

The term "alkoxy" means the group $R^X$-O-, and $R^X$ is defined in the same way as the term "alkyl". Alkoxy groups include, but are not limited to: methoxy, ethoxy, n-propoxy, isopropoxy or the like.

The term "alkenyl" means a linear or branched unsaturated monovalent hydrocarbon group with a specified number of carbon atoms (for example, $C_2$-$C_6$), with one or more (for example, 1, 2, or 3) carbon-carbon $sp^2$ double bonds. Alkenyl groups include, but are not limited to: vinyl,

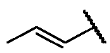, 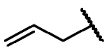

or the like.

The term "alkynyl" means a linear or branched unsaturated monovalent hydrocarbon group with a specified number of carbon atoms (for example, $C_2$-$C_6$), with one or more (for example, 1, 2, or 3) carbon-carbon $sp^3$ triple bonds. Alkynyl groups include, but are not limited to: acetylenyl,

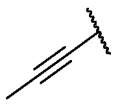, 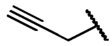

or the like.

Unless otherwise specially stated in the present invention, the term "cycloalkyl" means a cyclic saturated monovalent hydrocarbon group with a specified number of carbon atoms (for example, $C_3$-$C_{10}$), which is monocyclic or polycyclic (for example, 2 or 3 rings, or a bridged, spiro or fused ring), and in the latter case, more than two carton atoms are shared between the monocyclic rings. The monocyclic rings include, but are not limited to:

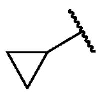, 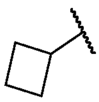, 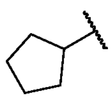, 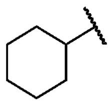

or the like. The bridged-ring cycloalkenyl groups include, but are not limited to:

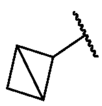, 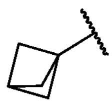, 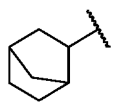

or the like. The spiro-ring cycloalkenyl groups include, but are not limited to,

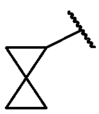, 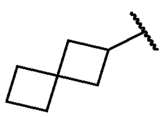, 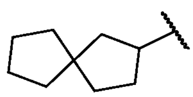

or the like. The fused-ring cycloalkyl groups include, but are not limited to:

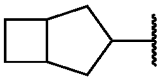, 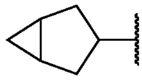, 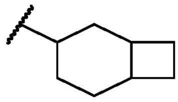, 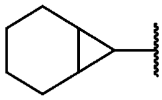,

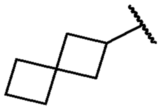, 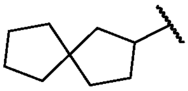, 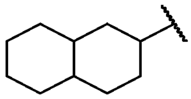 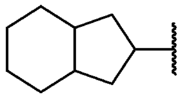

or the like.

Unless otherwise specially stated in the present invention, the term "cycloalkenyl" refers to "cycloalkyl" having one or more carbon-carbon double bonds, and the "cycloalkyl" is defined as above. For example,

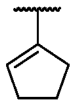

Unless otherwise specially stated in the present invention, the term "aryl" means a cyclic unsaturated monovalent hydrocarbon group with a specified number of carbon atoms (for example, $C_6$-$C_{10}$), which is monocyclic or polycyclic (for example, 2 or 3 rings). In case of a polycyclic ring, monocyclic rings share two atoms and a bond, and is (one or more/each ring is) aromatic. The aryl group is attached to the rest of a molecule by a ring that is aromatic or not. Aryl groups include, but are not limited to: phenyl, naphthyl,

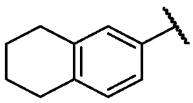, 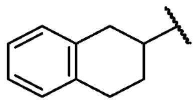

or the like.

Unless otherwise specially stated in the present invention, the term "heterocycloalkyl" means a monocyclo, bridged, spiro, or fused ring with a specified number of cyclic atoms (for example, 3-10 membered), a specified number of heteroatoms (for example, 1, 2 or 3), and a specified heteroatomic species (one or more selected from N, O and S). The in the latter case group can be saturated or unsaturated A (monocyclic) heterocycloalkyl group is attached to the rest of a molecule by a carbon atom or heteroatom. The monocyclic-ring hetercycloalkyl groups include, but are not limited to:

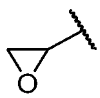, 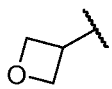, 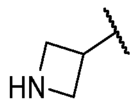,

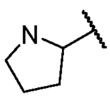, 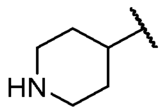

or the like. The spiro-ring hetercycloalkyl groups include, but are not limited to:

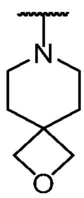,

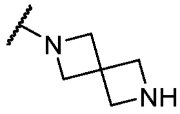, 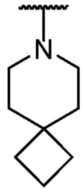, 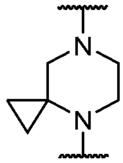

or the like. The bridged-ring hetercycloalkyl groups include, but are not limited to:

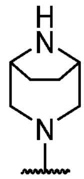

or the like. The fused-ring heterocycloalkyl groups include

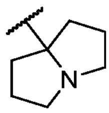, 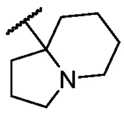, 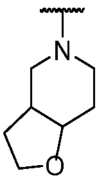 or 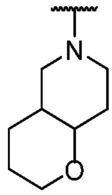

Heterocycloalkyl unsaturation means the presence of a carbon-carbon double bond or triple bond in the ring of the heterocycloalkyl, for example,

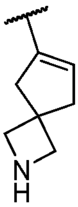, 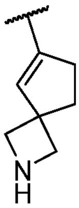, 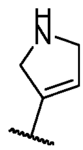 or 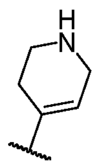

Unless otherwise specially stated in the present invention, the term "heteroaryl" means a cyclic unsaturated monovalent group with a specified number of cyclic atoms (for example, 5-10 membered), a specified number of heteroatoms (for example, 1, 2 or 3), and a specified heteroatom species (one or more selected from N, O and S). It is monocyclic or polycyclic; and in the latter case, the monocyclic rings share two atoms and a bond, and at least one ring is aromatic. The heteroaryl group is attached to the rest of a molecule by a carbon atom or heteroatom, by a ring with or without heteroatoms, or by a ring that is aromatic or not. The heteroaryl groups include, but are not limited to:

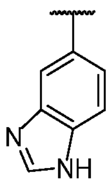, 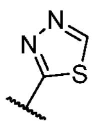, 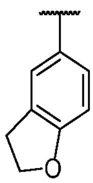, 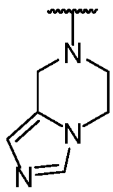, 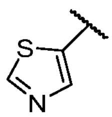, 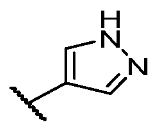,

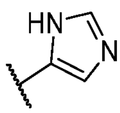, 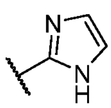, 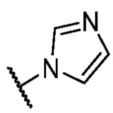 or 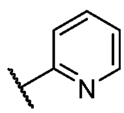

or the like.

The term "isotopic compound" means a compound in which one or more atom(s) have the isotopic abundance that differ from their natural abundance. For example, one or more atom(s) in a compound are substituted by an atom with a lower mass in nature, for instance, one hydrogen atom in a compound is substituted by deuterium, or C is substituted by $^{13}C$.

Without departing from the common knowledge in the art, all the preferred conditions above can be randomly combined to implement the preferred embodiments of the present invention.

The reagents and raw materials used in the present invention are commercially available.

The positive effect of the present invention lies in that the compound of the present invention has a better PARG inhibitory activity. In addition, the compound of the present invention is greatly improved in permeability and/or solubility compared with the existing inventions.

DETAILED DESCRIPTION OF THE INVENTION

The present invention will be further illustrated below by the way of embodiments, which are not intended to limit the present invention. For the test methods without specific conditions indicated in the following embodiments, the specific conditions were selected according to conventional methods and conditions, or according to the product instructions.

All the compounds of the present invention may be synthesized with different methods by a person skilled in the field of organic chemistry. The following describes general synthesis schemes for preparing the compounds of the present invention. These schemes are generic, which, however, does not imply the limitation to the possible techniques used by a person skilled in the art to prepare the compounds disclosed herein. The different methods for preparing the compounds of the present invention are obvious to a person skilled in the art. In addition, the various steps in synthesis may be performed alternately in sequence to obtain one or more of the desired compounds. Examples of the preparation of the compounds of the present invention by the method described in the general schemes are given in the Preparation and Examples sections described below. The preparation of compounds containing chiral center embodiments may be carried out by means of the techniques mastered by a person skilled in the art. For example, chiral compounds may be prepared by separating racemic products by chiral resolution by means of HPLC; or, example compounds may be prepared by known methods to obtain chiral compounds.

The chemical reactions and synthesis techniques described herein are performed in the reagents and solvents described herein, and the corresponding reaction yields are also affected by the reagents and solvents used. In addition, it should be understood that in the synthesis method described below, all the reaction conditions mentioned, including the choice of solvent, the reaction atmosphere, the reaction temperature, the experimental duration, and the feeding sequence for reaction, should be regarded as the standard operating conditions for the the reaction, which should be easily identifiable by a person skilled in the art. Meanwhile, it is also understandable by a technician in the field of organic synthesis. The functional groups present on each part of a molecule must be compatible with the reagents used and the reaction itself. In case of the limitation of the incompatibility of some functional groups on each part of the molecule with the reaction conditions, an alternative method must be used, which is obvious to a person skilled in the art. To derive the desired compounds of the present invention, obviously, it is necessary to make judgement on the adjustment of the sequence of synthesis steps, or on the selection of a specific synthesis process scheme. This is understandable and easily recognizable to a person skilled in the field of organic synthesis. It should also be appreciated that another major consideration for any synthetic route designed in the art is the rational selection of a protective group to protect the tolerance of reactive functional groups present in the compounds described in the present invention. For details, a reference can be made to the work of Greene et al., authorities in the field of chemistry (Protective Groups in Organic Synthesis, Third Edition, Wiley and Sons (1999)).

## Examples

The compounds and the intermediates used in the preparation of the compounds may be prepared using the procedures shown in the following examples and related procedures. The methods and conditions used in these examples and the actual compounds prepared in these examples are not meant to be limited, but imply to elaborate how to prepare the relevant compounds. The starting materials and reagents used in these examples, when not prepared by the procedures described herein, are often commercially available, or have been reported in the relevant chemical literature, or may be prepared by using the procedures described in the chemical literature.

In the examples given herein, the term "drying and concentrating" generally means the addition of an anhydrous sodium sulfate or magnesium sulfate drying solution to an organic solvent, followed by filtration and removal of the solvent from the filtrate (usually under reduced pressure and at a temperature suitable for the stability of the compound being prepared). In case of chromatographic column methods, conventional column chromatography or rapid column chromatography is typically used for column separation and purification, or a medium-pressure chromatograph (Biotage Isola One) preloaded with silica-gel column is used for elution in a specified solvent or solvent mixture. In some cases, the final

product is rapidly purified by preparative thin-layer chromatography using silica gel plates of 20 cm x 20 cm x 0.5 mm or 20 cm x 20 cm x 1 mm in an appropriate solvent system. Preparative high-performance liquid chromatography (HPLC) is performed using a reversed-phase column (Waters Sunfire C18, Waters Xbridge C18, or a similar reversed-phase column) with a size appropriate for the amount of compound being separated, elution is typically performed by adding methanol or acetonitrile of gradient concentrations to an aqueous phase, with an eluent containing 0.05% or 0.1% formic acid, trifluoroacetic acid, or 10 mM ammonium acetate, at an elution rate matching the size of the reversed-phase column used and the resolution of the product to be prepared. The chemical names used herein are generated using ChemDraw Professional version 19.1 and then translated into Chinese.

List of Abbreviations

| Abbreviation | Full name | Abbreviation | Full name |
|---|---|---|---|
| MS | Mass spectrometry | NMR | Nuclear magnetic resonance |
| TLC | Thin-layer chromatography | HPLC | High performance liquid chromatography |
| LCMS | Liquid chromatography-mass spectrometry | DMF | N,N-dimethylformamide |
| DCE | 1,2-dichloroethane | PE | Petroleum ether |
| DCM | Dichloromethane | DMSO | Dimethyl sulfoxide |
| MTBE | Methyl tert-butyl ether | THF | Tetrahydrofuran |
| EA | Ethyl acetate | T3P | 1-propylphosphoric anhydride |
| $Pd(dppf)Cl_2$ | [1,1'-di(diphenylphosphino)-ferrocen] palladium dichlorid(II) | HATU | 2-(7-azobenzotriazol)-N,N,N',N'-tetramethyluronium hexafluorophosphate |
| TFA | Trifluoroacetic acid | TEA | Triethylamine |
| NCS | N-chlorosuccinimide | NBS | N-bromosuccinimide |
| NBS | N-bromosuccinimide | DAST | Diethylaminosulphur trifluoride |
| XantPhos | 4,5-bisdiphenylphosphino-9,9-dimethylxanthene | $Pd_2(dba)_3$ | Tris(dibenzylideneacetone)dipal ladium |
| MsCl | Methylsufonyl chloride | RuPhos | 2-dicyclohexylphosphino-2',6'-diisopropoxy-1,1'-biphenyl |
| RuPhos Pd G3 | Methanesulfonato(2-dicyclohexylphosphino-2',6'-diisopropoxy-1,1'-biphenyl)(2-amino-1,1'-biphenyl-2-yl)palladium (II) | XPhos Pd G2 | Chloro(2-dicyclohexylphosphino-2',6'2',4',6'-triisopropyl-1,1'-biphenyl)[2-(2'-amino-1,1'-biphenyl)]palladium (II) |
| NMP | N-methyl pyrrolidone | $Pd(PPh_3)_4$ | Tetrakis(triphenylphosphine)pal ladium |
| XPhos | 2-dicyclohexylphosphino-2',4',6'-triisopropylbiphenyl | | |

**Example 1: N-(1-cyanocyclopropyl)-8-(4-isobutyrylpiperazin-1-yl)-3-(2-methylthiazol-5-yl)imidazo[1,2-a]pyridine-6-sulfonamide**

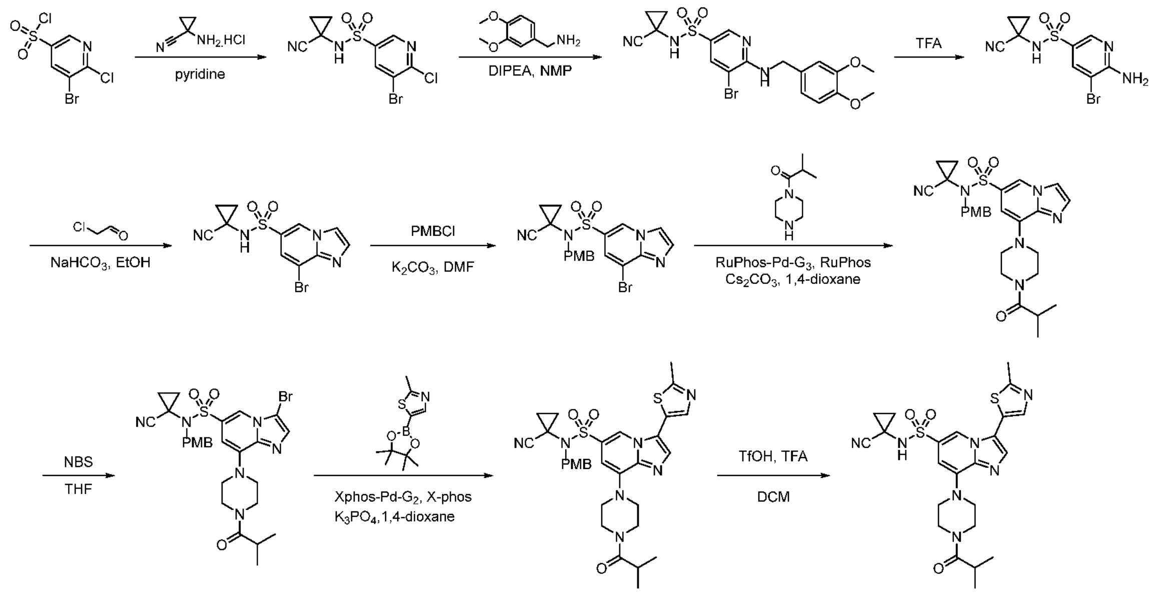


**Step 1: 5-bromo-6-chloro-N-(1-cyanocyclopropyl)pyridine-3-sulfonamide**

At 0°C, 5-bromo-6-chloropyridin-3-sulfonyl chloride (3.3 g, 27.5 mmol) was added portionwise into the solution of 1-aminocyclopropan-1-cyano hydrochloride (4 g, 13.7 mmol) in pyridine (20 mL); and then, the resulting mixture was stirred for 1 hour at room temperature. The mixture was acidified to pH~5 using 1N hydrochloric acid, and extracted with ethyl acetate twice. The organic phase was washed with saturated brine, dried over anhydrous sodium sulfate, and concentrated under reduced pressure to obtain the title compound (yellow solid, 4 g, yield: 86.4%). LC/MS (ESI) m/z: 336 $[M+H]^+$.

**Step 2: 5-bromo-N-(1-cyanocyclopropyl)-6-((3,4-dimethoxybenzyl)amino)pyridin-3-sulfonamide**

N,N-diisopropylethylamine (4.6 g, 35.6 mmol) and (3.4-dimethoxyphenyl)methylamine (3.0 g, 17.8 mmol) were dropwise added slowly to the solution of 5-bromo-6-chloro-N-(1-cyanocyclopropyl)pyridin-3-sulfonamide(4 g, 11.9 mmol) in N-methyl pyrrolidone (40 mL). The mixture was stirred at 100°C for 2 hours under an atmosphere of nitrogen. After completion of the reaction, the reaction mixture was cooled to room temperature, water was added; and the resulting mixture was extracted with ethyl acetate, washed with saturated brine, dried over anhydrous sodium sulfate, and concentrated under reduced pressure. The residue was purified by column chromatography (eluent: petroleum ether/ethyl acetate, gradient: 0-50% ethyl acetate) to obtain the title compound (yellow solid, 5.1 g, yield: 91.7%). LC/MS (ESI) m/z: 469 $[M+H]^+$.

**Step 3: 6-amino-5-bromo-N-(1-cyanocyclopropyl)pyridine-3-sulfonamide**

At 0°C, 5-bromo-N-(1-cyanocyclopropyl)-6-((3,4-dimethoxybenzyl)amino)pyridin-3-sulfonamide (5.0 g, 10.7 mmol) was dissolved in trifluoroacetic acid (20 mL). The reaction mixture was stirred at room temperature for 16 hours. The mixture was concentrated under reduced pressure to obtain the title compound (yellow solid, 3.39 g, yield: 100.0%), which was directly used in the next step without purification. LC/MS (ESI) m/z: 319 $[M+H]^+$.

**Step 4: 8-bromo-N-(1-cyanocyclopropyl)imidazo[1,2-a]pyridine-6-sulfonamide**

Sodium bicarbonate (2.7 g, 32.1 mmol) and 2-chloroacetaldehyde (8.5 mL, 53.5 mmol, 40% aqueous solution) were slowly added into the solution of 6-amino-5-bromo-N-(1-cyanocyclopropyl)pyridin-3-sulfonamide (3.39 g, 10.7 mmol) in ethanol (100 mL), and the reaction mixture was stirred at 90°C overnight. The reaction mixture was filtered, and the filtrate was concentrated under reduced pressure to dryness. The residue was purified by column chromatography (eluent: petroleum ether, ethyl acetate, gradient: 0-60% ethyl acetate) to obtain the title compound (yellow solid, 1.5 g, yield: 41.1%). $^1H$ NMR (400 MHz, DMSO-$d_6$) δ 9.37 (d, $J$ = 1.6 Hz, 1H), 8.31 (d, $J$ = 1.2 Hz, 1H), 7.80 (d, $J$ = 1.2 Hz, 1H), 7.74 (d, $J$ = 1.6 Hz, 1H), 1.49 - 1.45 (m, 2H), 1.38 - 1.33 (m, 2H). LC/MS (ESI) m/z: 343 $[M+H]^+$.

**Step 5: 8-bromo-N-(1-cyanocyclopropyl)-N-(4-methoxybenzyl)imidazo[1,2-a]pyridine-6-sulfonamide**

Potassium carbonate (790 mg, 5.7 mmol) and 1-(chloromethyl)-4-metoxybenzene (454 mg, 2.9 mmol) were added into the solution of 8-bromo-N-(1-cyanocyclopropyl)imidazo[1,2-a]pyridin-6-sulfonamide (650 mg, 1.9 mmol) in N,N-dimethylformamide (5 mL). The reaction mixture was stirred at 45°C for 2 hours under an atmosphere of nitrogen. After completion of the reaction, the reaction mixture was cooled to room temperature; water was added; and the resulting mixture was extracted with ethyl acetate, washed with saturated brine, dried over anhydrous sodium sulfate, and concentrated under reduced pressure. The residue was separated by column chromatography (eluent: petroleum ether/ethyl acetate, gradient: 0-35% ethyl acetate) to obtain the title compound (yellow solid, 720 mg, yield: 81.9%). LC/MS (ESI) m/z: 461 $[M+H]^+$.

**Step 6: N-(1-cyanocyclopropyl)-8-(4-isobutyrylpiperazin-1-yl)-N-(4-methoxybenzyl)imidazo[1,2-a]pyridine-6-sulfonamide**

Cesium carbonate (805 mg, 2.47 mmol), RuPhos (115 mg, 0.25 mmol) and RuPhos Pd G3 (103 mg, 0.12 mmol) were added into the solution of 8-bromo-N-(1-cyanocyclopropyl)-N-(4-methoxybenzyl)imidazo[1,2-a]pyridin-6-sulfonamide (570 mg, 1.24 mmol) and 2-methyl-1-(piperazin-1-yl)propan-1-one (386 mg, 2.47 mmol) in 1,4-dioxane (12 mL). The reaction mixture was stirred at 110°C for 3 hours under an atmosphere of nitrogen. After completion of the reaction, the reaction mixture was cooled to room temperature; water was added; and the resulting mixture was extracted with ethyl acetate, washed with saturated brine, dried over anhydrous sodium sulfate, and concentrated under reduced pressure. The residue was separated by column chromatography (eluent: dichloromethane/methanol, gradient: 0-4% methanol) to obtain the title compound (yellow solid, 530 mg, yield: 79.9%). LC/MS (ESI) m/z: 537 $[M+H]^+$.

**Step 7: 3-bromo-N-(1-cyanocyclopropyl)-8-(4-isobutyrylpiperazin-1-yl)-N-(4-methoxybenzyl)imidazo[1,2-a]pyridine-6-sulfonamide**

At -10°C, the solution of N-bromosuccinimide (176 mg, 0.99 mmol) in tetrahydrofuran (7.5 mL) was slowly added into the solution of N-(1-cyanocyclopropyl)-8-(4-isobutyrylpiperazin-1-yl)-N-(4-methoxybenzyl)imidazo[1,2-a]pyridin-6-sulfonamide (530 mg, 0.99 mmol) in tetrahydrofuran (15 mL). The mixture was stirred at room temperature for 30 minutes. The reaction mixture was filtered, and the filtrate was concentrated. The residue was purified by column chromatography (eluent: dichloromethane/methanol, gradient: 0-4% methanol) to obtain the title compound (yellow solid, 430 mg, yield: 70.7%). LC/MS (ESI) m/z: 617 $[M+H]^+$.

**Step 8: N-(1-cyanocyclopropyl)-8-(4-isobutyrylpiperazin-1-yl)-N-(4-methoxybenzyl)-3-(2-methylthiazol-5-yl)imidazo[1,2-a]pyridine-6-sulfonamide**

Potassium phosphate (52 mg, 0.24 mmol), XPhos (4 mg, 0.008 mmol) and XPhos Pd G2 (6 mg, 0.008 mmol) were sequentially added into the mixed solution of 3-bromo-N-(1-cyanocyclopropyl)-8-(4-isobutyrylpiperazin-1-yl)-N-(4-methoxybenzyl)imidazo[1,2-a]pyridin-6-sulfonamide (50 mg, 0.08 mmol) and 2-methyl-5-(4,4,5,5-tetramethyl-1,3,2-dioxaborolan-2-yl)thiazole (37 mg, 0.16 mmol) in 1,4-dioxane (3 mL) and water (0.6 mL). The reaction mixture was stirred at 100°C for 16 hours under an atmosphere of nitrogen. After completion of the reaction, the reaction mixture was cooled to room temperature; water was added; and the resulting mixture was extracted with ethyl acetate, washed with saturated brine, dried over anhydrous sodium sulfate, and concentrated under reduced pressure. The residue was purified by column chromatography (eluent: dichloromethane/methanol, gradient: 0-5% methanol) to obtain the title compound (yellow solid, 17 mg, yield: 34.0%). LC/MS (ESI) m/z: 634 $[M+H]^+$.

**Step 9: N-(1-cyanocyclopropyl)-8-(4-isobutyrylpiperazin-1-yl)-3-(2-methylthiazol-5-yl)imidazo[1,2-a]pyridine-6-sulfonamide**

At 0°C, trifluoroacetic acid (0.1 mL) and trifluoromethanesulfonic acid (0.1 mL) were dropwise added slowly into the solution of N-(1-cyanocyclopropyl)-8-(4-isobutyrylpiperazin-1-y1)-N-(4-methoxybenzyl)-3-(2-methylthiazol-5-yl)imidazo[1,2-a]pyridin-6-sulfonamide (17 mg, 0.03 mmol) in dichloromethane (0.5 mL). The mixture was stirred at room temperature for 30 minutes. The mixture was concentrated, and the residue was purified by pre-HPLC to obtain the title compound (white solid, 5 mg, yield: 36.3%). $^1$H NMR (400 MHz, DMSO-$d_6$) $\delta$ 8.38 (d, $J$ = 1.2 Hz, 1H), 8.01 (s, 1H), 7.92 (s, 1H), 6.77 (d, $J$ = 1.1 Hz, 1H), 3.77 - 3.57 (m, 8H), 2.95 (dt, $J$ = 13.5, 6.7 Hz, 1H), 2.78 (s, 3H), 1.46 (dd, $J$ = 8.3, 5.4 Hz, 2H), 1.33 (dd, $J$ = 8.4, 5.5 Hz, 2H), 1.04 (d, $J$ = 6.7 Hz, 6H). LC/MS (ESI) (m/z): 514 $[M+H]^+$.

**Example 2: N-(1-cyanocyclopropyl)-8-(4-isobutyrylpiperazin-1-yl)-3-(5-methyl-1,3,4-thiadiazol-2-yl)imidazo [1,2-a]pyridine-6-sulfonamide**

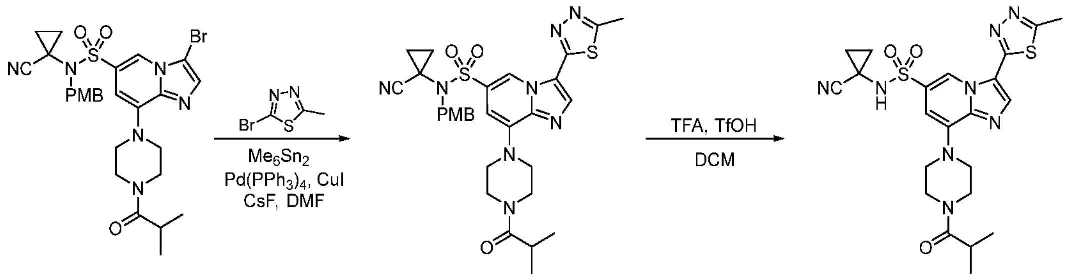


**Step 1: N-(1-cyanocyclopropyl)-8-(4-isobutyrylpiperazin-1-yl)-N-(4-methoxybenzyl)-3-(5-methyl-1,3,4-thiadiazol-2-yl)imidazo[1,2-a]pyridine-6-sulfonamide**

At room temperature, hexamethylditin(0.067 mL, 0.324 mmol), CsF (49 mg, 0.32 mmol) and CuI (6.2 mg, 0.032 mmol) were sequentially added into the solution of 3-bromo-N-(1-cyanocyclopropyl)-N-[(4-methoxyphenyl) methyl]-8-[4-(2-methylpropionyl)piperazin-1-yl]imidazo[1,2-a]pyridin-6-sulfonamide (100 mg, 0.16 mmol) and 2-bromo-5-methyl-1,3,4-thiadiazole (58 mg, 0.32 mmol) in DMF (3 mL). The flask was replaced with nitrogen gas three times and then the mixture was stirred at 100°C for 16 hours under an atmosphere of nitrogen. After completion of the reaction, the reaction mixture was cooled to room temperature, quenched by adding water, extracted with ethyl acetate, washed with saturated brine, dried over anhydrous sodium sulfate, and concentrated under reduced pressure. The residue was purified by silica gel chromatography (eluent: dichloromethane/ methanol, gradient: 0-3% methanol) to obtain the title compound (yellow solid, 30 mg, yield: 29.1%). LC/MS (ESI) m/z: 635 $[M+H]^+$.

**Step 2: N-(1-cyanocyclopropyl)-8-(4-isobutyrylpiperazin-1-yl)-3-(5-methyl-1,3,4-thiadiazol-2-yl)imidazo[1,2-a] pyridine-6-sulfonamide**

The title compound was obtained by referring to the preparation method in Step 9 of Example 1. $^1$H NMR (400 MHz, DMSO-$d_6$) δ 9.78 (s, 1H), 9.42 (s, 1H), 8.44 (s, 1H), 7.02 - 6.91 (m, 1H), 3.81 - 3.71 (m, 4H), 3.69 - 3.59 (m, 4H), 3.01 - 2.91 (m, 1H), 2.83 (s, 3H), 1.53 - 1.33 (m, 4H), 1.09 - 0.99 (m, 6H). LC/MS (ESI) (m/z): 515 $[M+H]^+$.

**Example 3: N-(1-cyanocyclopropyl)-8-(4-isobutyrylpiperazin-1-yl)-3-(5-methylthiazol-2-yl)imidazo[1,2-a]pyridine-6-sulfonamide**

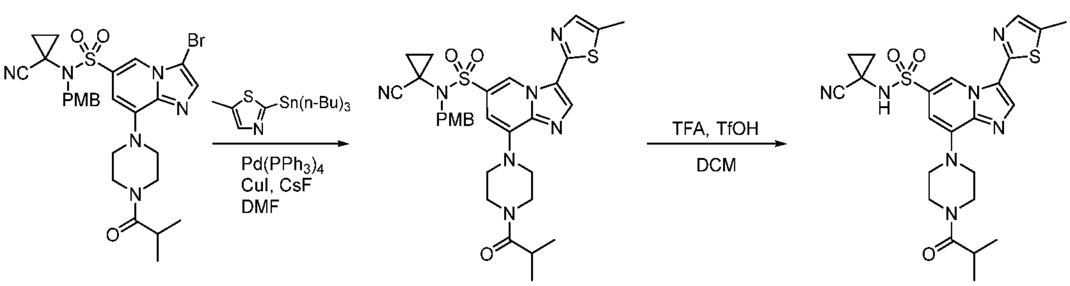


**Step 1: N-(1-cyanocyclopropyl)-8-(4-isobutyrylpiperazin-1-yl)-N-(4-methoxybenzyl)-3-(5-methylthiazol-2-yl)imidazo[1,2-a]pyridine-6-sulfonamide**

At room temperature, CuI (2 mg, 0.008 mmol), CsF (37 mg, 0.24 mmol) and $Pd(PPh_3)_4$ (9 mg, 0.008 mmol) were sequentially added into the solution of 3-bromo-N-(1-cyanocyclopropyl)-8-(4-isobutyrylpiperazin-1-yl)-N-(4-methoxybenzyl)imidazo[1,2-a]pyridin-6-sulfonamide (50 mg, 0.08 mmol) and 5-methyl-2-(tri-n-butylstannyl)thiazole (63 mg, 0.16 mmol) in DMF (2 mL). The flask was replaced with nitrogen gas three times and then the mixture was stirred at 100°C for 2 hours under an atmosphere of nitrogen. After completion of the reaction, the reaction mixture was cooled to room

temperature, quenched by adding water, extracted with ethyl acetate, washed with saturated brine, dried over anhydrous sodium sulfate, and concentrated under reduced pressure. The residue was purified by silica gel chromatography (eluent: dichloromethane/ methanol, gradient: 0-4% methanol) to obtain the title compound (yellow solid, 30 mg, yield: 58.2%). LC/MS (ESI) m/z: 634 $[M+H]^+$.

**Step 2: N-(1-cyanocyclopropyl)-8-(4-isobutyrylpiperazin-1-yl)-3-(5-methylthiazol-2-yl)imidazo[1,2-a]pyridine-6-sulfonamide**

The title compound was obtained by referring to the synthesis method in Step 9 of **Example 1.** $^1$H NMR (400 MHz, DMSO-$d_6$) δ 9.80 (s, 1H), 8.29 (s, 1H), 7.76 (s, 1H), 6.88 (s, 1H), 3.80 - 3.56 (m, 8H), 3.01 - 2.91 (m, 1H), 2.54 (s, 3H), 1.49 - 1.42 (m, 2H), 1.40 - 1.33 (m, 2H), 1.04 (d, *J* = 6.3 Hz, 6H). LC/MS (ESI) (m/z): 514 $[M+H]^+$.

**By referring to the preparation methods in Examples 1, 2 and 3, the following examples were implemented by synthesizing crude products according to a similar route by starting from appropriate starting materials, preparing the crude products via reversed-phase HPLC, and then freeze-drying the crude product to obtain the title compound.**

| Example | Structure and name | Analysis data |
|---|---|---|
| **Example 4** | 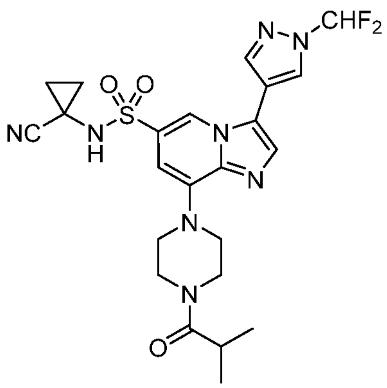 **N-(1-cyanocyclopropyl)-3-(1-(difluoromethyl)-1H-pyrazol-4-yl)-8-(4-isobutyrylpiperazin-1-yl)imidazo[1,2-a]pyridine-6-sulfonamide** | $^1$H NMR (400 MHz, DMSO-$d_6$) δ 9.28 (s, 1H), 8.81 (s, 1H), 8.32 (d, *J* = 1.4 Hz, 1H), 8.27 (s, 1H), 8.12 - 7.83 (m, 2H), 6.76 (d, *J* = 1.3 Hz, 1H), 3.76 - 3.59 (m, 8H), 2.99 - 2.92 (m, 1H), 1.50 - 1.43 (m, 2H), 1.37 - 1.31 (m, 2H), 1.05 (d, *J* = 6.7 Hz, 6H). LC/MS (ESI) (m/z): 533 $[M+H]^+$. |
| **Example 5** | 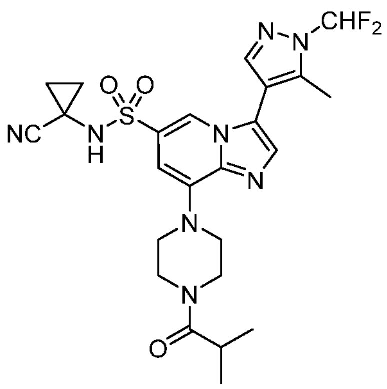 **N-(1-cyanocyclopropyl)-3-(1-(difluoromethyl)-5-methyl-1H-pyrazol-4-yl)-8-(4-isobutyrylpiperazin-1-yl)imidazo[1,2-a]pyridine-6-sulfonamide** | $^1$H NMR (400 MHz, DMSO-$d_6$) δ 9.28 (s, 1H), 8.81 (s, 1H), 8.32 (d, *J* = 1.4 Hz, 1H), 8.12 - 7.83 (m, 2H), 6.76 (d, *J* = 1.3 Hz, 1H), 3.76 - 3.59 (m, 8H), 2.99 - 2.92 (m, 1H), 2.27 (s, 3H), 1.50 - 1.43 (m, 2H), 1.37 - 1.31 (m, 2H), 1.05 (d, *J* = 6.7 Hz, 6H). LC/MS (ESI) (m/z): 547 $[M+H]^+$. |

(continued)

| Example | Structure and name | Analysis data |
|---|---|---|
| Example 6 | 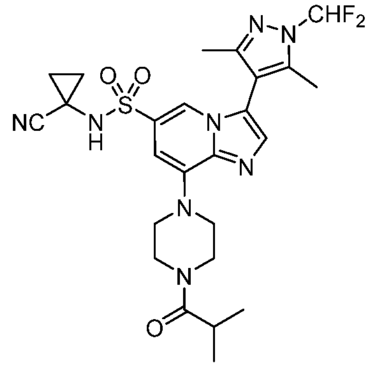 **N-(1-cyanocyclopropyl)-3-(1-(difluoromethyl)-3,5-dimethyl-1H-pyrazol-4-yl)-8-(4-isobutyrylpiperazin-1-yl)imidazo[1,2-a]pyridine-6-sulfonamide** | $^1$H NMR (400 MHz, DMSO-$d_6$) δ 9.29 (s, 1H), 8.03 - 7.72 (m, 3H), 6.76 - 6.70 (m, 1H), 3.79 - 3.61 (m, 8H), 3.01 - 2.91 (m, 1H), 2.28 (s, 3H), 2.07 (s, 3H), 1.46 - 1.39 (m, 2H), 1.39 - 1.32 (m, 2H), 1.05 (d, *J* = 6.7 Hz, 6H). LC/MS (ESI) (m/z): 561 [M+H]$^+$. |

**Example 7: N-(1-cyanocyclopropyl)-3-(4-hydroxy-4,5,6,7-tetrahydropyrazolo[1,5-a]pyridin-2-yl)-8-(4-isobutyrylpiperazin-1-yl)imidazo[1,2-a]pyridine-6-sulfonamide**

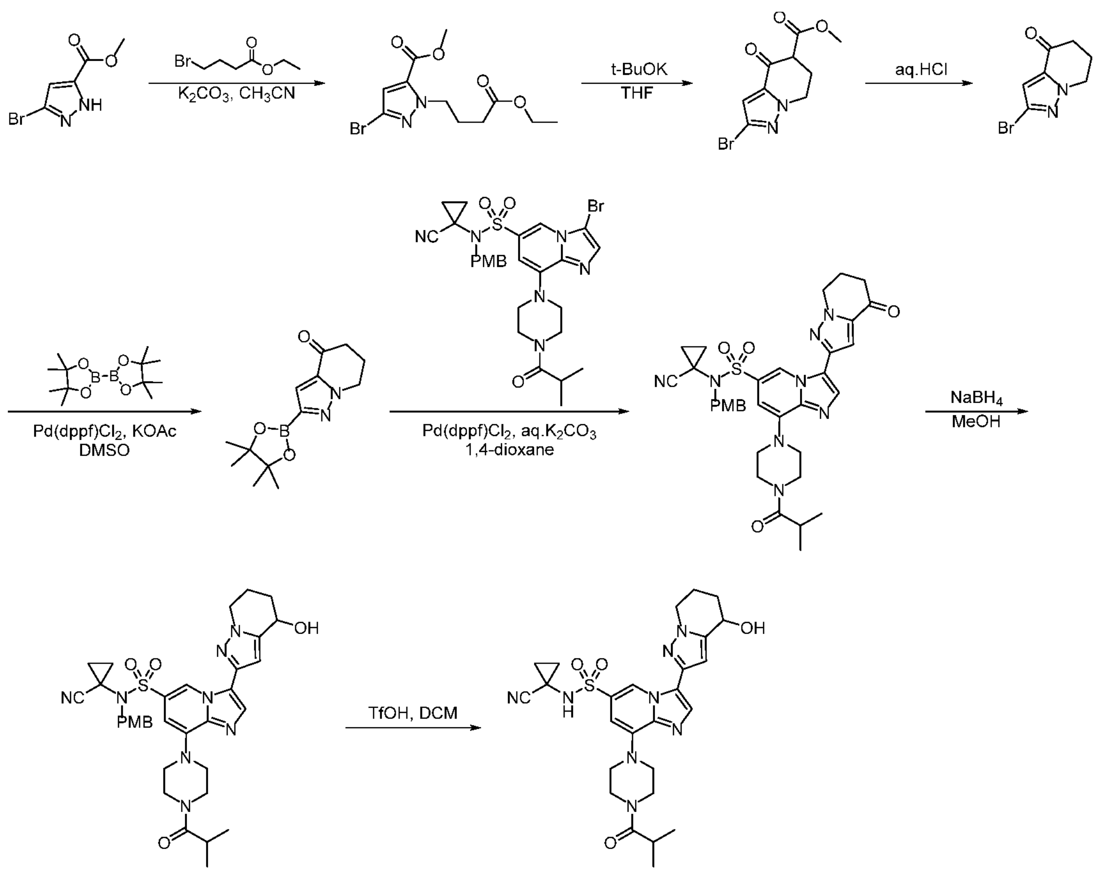


**Step** 1: **Methyl 3-bromo-1-(4-ethoxy-4-oxobutyl)-1H-pyrazole-5-carboxylate**

At 0°C, potassium carbonate (2 g, 14.6 mmol) and 4-ethyl bromobutyrate (2.3 g, 12 mmol) were added into the solution of 3-bromo-1H-pyrazol-5-methyl carboxylate (2.0 g, 9.7 mmol) in acetonitrile (20 mL), and the resulting mixture was stirred at 80°C for 8 hours. After completion of the reaction, the reaction mixture was cooled to room temperature,

quenched by adding ice water, extracted with ethyl acetate, washed with saturated brine, dried over anhydrous sodium sulfate, and concentrated under reduced pressure. The residue was purified by column chromatography (eluent: petroleum ether/ethyl acetate, gradient: 0-50% ethyl acetate) to obtain the title compound (white solid, 3.0 g, yield: 96.4%).

**Step 2: Methyl 2-bromo-4-oxo-4,5,6,7-tetrahydropyrazolo[1,5-a]pyridine-5-carboxylate**

At 0°C, potassium tert-butoxide (1.6 g, 14 mmol) was added into the solution of 3-bromo-1-(4-ethoxy-4-oxybutyl)-1H-pyrazol-5-methyl carboxylate (3 g, 9.4 mmol) in tetrahydrofuran (30 mL). The flask was replaced with nitrogen gas three times and then the mixture was stirred at 50°C for 8 hours. After completion of the reaction, the reaction mixture was cooled to room temperature, poured into ice water, and extracted with ethyl acetate. The organic phase was washed with saturated brine, dried over anhydrous sodium sulfate, and concentrated under reduced pressure. The residue was purified by column chromatography (eluent: petroleum ether/ethyl acetate, gradient: 0-20% ethyl acetate) to obtain the title compound (white solid, 1.4 g, yield: 54.5%).

**Step 3: 2-bromo-6,7-dihydropyrazolo[1,5-a]pyridin-4(5H)-one**

At room temperature, 2-bromo-4-oxo-4,5,6,7-tetrahydropyrazolo[1,5-a]pyridin-5-methyl formate (1.4 g, 5.1 mmol) was added into concentrated hydrochloric acid (20 mL), and the reaction mixture was stirred at 90°C for 16 hours. After completion of the reaction, at 0°C, the reaction mixture was neutralized with 1N sodium hydroxide in water, and extracted with ethyl acetate twice. The organic phase was dried over anhydrous sodium sulfate, filtered and concentrated under reduced pressure. The residue was purified by column chromatography (eluent: petroleum ether/ethyl acetate, gradient: 0-20% ethyl acetate) to obtain the title compound (colorless oil, 0.53 g, yield: 48.1%). $^1H$ NMR (400 MHz, $CDCl_3$) $\delta$ 6.77 (s, 1H), 4.35 - 4.22 (m, 2H), 2.69 - 2.57 (m, 2H), 2.37 - 2.24 (m, 2H). LC/MS (ESI) m /z: 216 $[M+H]^+$.

**Step 4: 2-(4,4,5,5-tetramethyl-1,3,2-dioxaborolan-2-yl)-6,7-dihydropyrazolo[1,5-a]pyridin-4(5H)-one**

Under the nitrogen atmosphere at room temperature, potassium acetate (760 mg, 7.5 mmol), XPhos (250 mg, 0.5 mmol) and $Pd_2(dba)_3$ (225 mg, 0.2 mmol) were added into the solution of 2-bromo-6,7-dihydropyrazolo[1,5-a]pyridin-4(5H)-one (530 mg, 2.5 mmol) and bis(pinacolato)diboron (1.25 g, 4.9 mmol) in1,4-dioxane (10 mL). The flask was replaced with nitrogen gas three times and then the mixture was stirred at 80°C for 3 hours under an atmosphere of nitrogen. After completion of the reaction, the reaction mixture was cooled to room temperature; ice water was added into the reaction mixture; and the reaction mixture was extracted with ethyl acetate. The organic phase was washed with saturated brine, dried over anhydrous sodium sulfate, and concentrated under reduced pressure. The residue was purified by column chromatography (eluent: petroleum ether/ethyl acetate, gradient: 0-20% ethyl acetate) to obtain the title compound (colorless oil, 380 mg, yield: 48.0%). LC/MS (ESI) m/z: 263 $[M+H]^+$.

**Step 5: N-(1-cyanocyclopropyl)-8-(4-isobutyrylpiperazin-1-yl)-N-(4-methoxybenzyl)-3-(4-oxo-4,5,6,7-tetrahydropyrazolo[1,5-a]pyridin-2-yl)imidazo[1,2-a]pyridine-6-sulfonamide**

Under an atmosphere of nitrogen at room temperature, potassium carbonate (120 mg, 0.8 mmol) and Pd(dppf)$Cl_2$ (25 mg, 0.03 mmol) were added into the solution of 3-bromo-N-(1-cyanocyclopropyl)-8-(4-isobutyrylpiperazin-1-yl)-N-(4-methoxybenzyl)imidazo[1,2-a]pyridin-6-sulfonamide (150 mg, 0.26 mmol) and 2-(4,4,5,5-tetramethyl-1,3,2-dioxaborolan-2-yl)-6,7-dihydropyrazolo[1,5-a]pyridin-4(5H)-one (120 mg, 0.5 mmol) in 1,4-dioxane (5 mL) and water (0.5 mL). The flask was replaced with nitrogen gas three times and then the mixture was allowed to react for 3 hours at 90°C under an atmosphere of nitrogen. After completion of the reaction, the reaction mixture was diluted with ethyl acetate, washed with water and saturated brine in sequence, dried over anhydrous sodium sulfate, and concentrated under reduced pressure. The residue was purified by column chromatography (eluent: petroleum ether/ethyl acetate, gradient: 0-20% ethyl acetate) to obtain the title compound (yellow solid, 110 mg, yield: 28.7%). LC/MS(ESI) m/z: 671 $[M+H]^+$.

**Step 6: N-(1-cyanocyclopropyl)-3-(4-hydroxy-4,5,6,7-tetrahydropyrazolo[1,5-a]pyridin-2-yl)-8-(4-isobutyrylpiperazin-1-yl)-N-(4-methoxybenzyl)imidazo [1,2-a]pyridine-6-sulfonamide**

At 0°C, sodium borohydride (10 mg, 0.3 mmol) was added into the solution of N-(1-cyanocyclopropyl)-8-(4-isobutyrylpiperazin-1-y1)-N-(4-methoxybenzy1)-3-(4-carbony1-4,5,6,7-tetrahydropyrazolo[1,5-a]pyridin-2-yl)imidazo [1,2-a]pyridin-6-sulfonamide (100 mg, 0.1 mmol) in methanol (2 mL). The reaction mixture was stirred at room temperature for 30 minutes. After completion of the reaction, the reaction was subjected acid quenching using 1N dilute hydrochloric acid, and the reaction mixture was extracted with ethyl acetate. The organic phase was washed with saturated brine, dried over anhydrous sodium sulfate, and concentrated under reduced pressure. The residue was purified by column

chromatography (eluent: petroleum ether/ethyl acetate, gradient: 0-20% ethyl acetate) to obtain the title compound (yellow solid, 70 mg, yield: 80%). LC/MS (ESI) m /z: 673 [M+H]$^+$.

**Step 7: N-(1-cyanocyclopropyl)-3-(4-hydroxy-4,5,6,7-tetrahydropyrazolo[1,5-a]pyridin-2-yl)-8-(4-isobutyrylpiperazin-1-yl)imidazo[1,2-a]pyridine-6-sulfonamide**

At 0°C, TfOH (0.1 mL) was added into the solution of N-(1-cyanocyclopropyl)-3-(4-hydroxyl-4,5,6,7-tetrahydropyrazolo[1,5-a]pyridin-2-y1)-8-(4-isobutyrylpiperazin-1-yl)-N-(4-methoxybenzyl)imidazo[1,2-a]pyridin-6-sulfonamide (30 mg, 0.04 mmol) in dichloromethane (0.5 mL), and the reaction mixture was stirred at room temperature for 30 minutes. After completion of the reaction, the mixture was concentrated to dryness, and residue was purified by pre-HPLC to obtain the title compound (white solid, 6.3 mg, yield: 25.6%). $^1$H NMR (400 MHz, DMSO-$d_6$) δ 9.58 (s, 1H), 8.08 (s, 1H), 6.76 (s, 1H), 6.75 (s, 1H), 5.58 (d, *J* = 5.5 Hz, 1H), 4.84 - 4.75 (m, 1H), 4.20 - 4.14 (m, 2H), 3.77 - 3.69 (m, 4H), 3.68 - 3.56 (m, 4H), 3.00 - 2.91 (m, 1H), 2.27 - 2.15 (m, 1H), 2.10 - 1.93 (m, 2H), 1.82 - 1.69 (m, 1H), 1.47 - 1.30 (m, 4H), 1.05 (d, *J* = 6.5 Hz, 6H). LC/MS (ESI) (m/z): 553 [M+H]$^+$.

**Example 8: N-(1-cyanocyclopropyl)-3-(4-fluoro-4,5,6,7-tetrahydropyrazolo[1,5-a]pyridin-2-yl)-8-4-isobutyrylpiperazin-1-yl)imidazo[1,2-a]pyridine-6-sulfonamide**

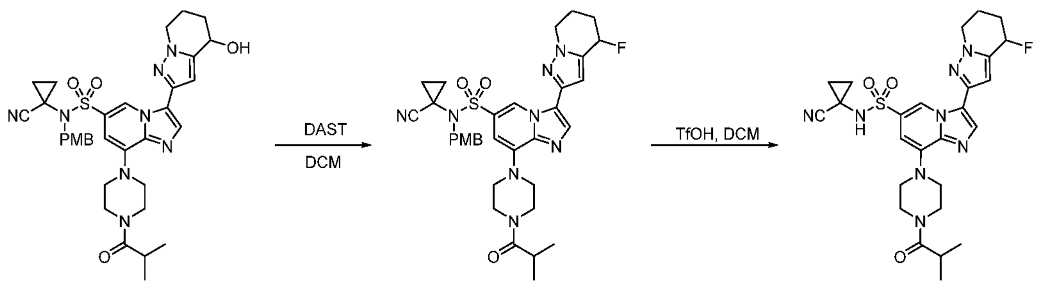


**Step 1: N-(1-cyanocyclopropyl)-3-(4-fluoro-4,5,6,7-tetrahydropyrazolo[1,5-a]pyridin-2-yl)-8-(4-isobutyrylpiperazin-1-yl)-N-(4-methoxybenzyl)imidazo [1,2-a]pyridine-6-sulfonamide**

At 0°C, diethylaminosulphur trifluoride (18 mg, 0.11 mmol) was dropwise dropped to the solution of N-(1-cyanocyclopropy1)-3-(4-hydroxyl-4,5,6,7-tetrahydropyrazolo[1,5-a]pyridin-2-y1)-8-(4-isobutyrylpiperazin-1-yl)-N-(4-methoxybenzyl)imidazo[1,2-a]pyridin-6-sulfonamide (40 mg, 0.06 mmol) in dichloromethane (0.5 mL); and the reaction mixture was stirred at room temperature for 30 minutes. After completion of the reaction, the reaction mixture was quenched using saturated sodium bicarbonate in water at 0°C, and extracted with dichloromethane. The organic phase was washed with saturated brine, dried over anhydrous sodium sulfate, and concentrated under reduced pressure to obtain the title compound (yellow oil, 25 mg, yield: 74.9%), with the crude product directly used in the next reaction. LC/MS (ESI) (m/z): 675 [M+H]$^+$.

**Step 2: N-(1-cyanocyclopropyl)-3-(4-fluoro-4,5,6,7-tetrahydropyrazolo[1,5-a]pyridin-2-yl)-8-4-isobutyrylpiperazin-1-yl)imidazo[1,2-a]pyridine-6-sulfonamide**

At 0°C, TfOH (0.1 mL) was slowly added into the solution of N-(1-cyanocyclopropyl)-3-(4-fluoro-4,5,6,7-tetrahydropyrazolo[1,5-a]pyridin-2-y1)-8-(4-isobutyrylpiperazin-1-y1)-N-(4-methoxybenzyl)imidazo[ 1,2-a]pyridin-6-sulfonamide (20 mg, 0.03 mmol) in dichloromethane (0.5 mL), and the reaction mixture was stirred at room temperature for 10 minutes. After completion of the reaction, the reaction mixture was concentrated under reduced pressure, and the crude product was purified by pre-HPLC to obtain the title compound (white solid, 0.5 mg, yield: 2.4%). LC/MS (ESI) m/z: 555 [M+H]$^+$.

**Example 9: N-(1-cyanocyclopropyl)-3-(5-hydroxy-5,6-dihydro-4H-pyrrolo[1,2-b]pyrazol-2-yl)-8-(4-isobutyrylpiperazin-1-yl)imidazo[1,2-a]pyridine-6-sulfonamide**

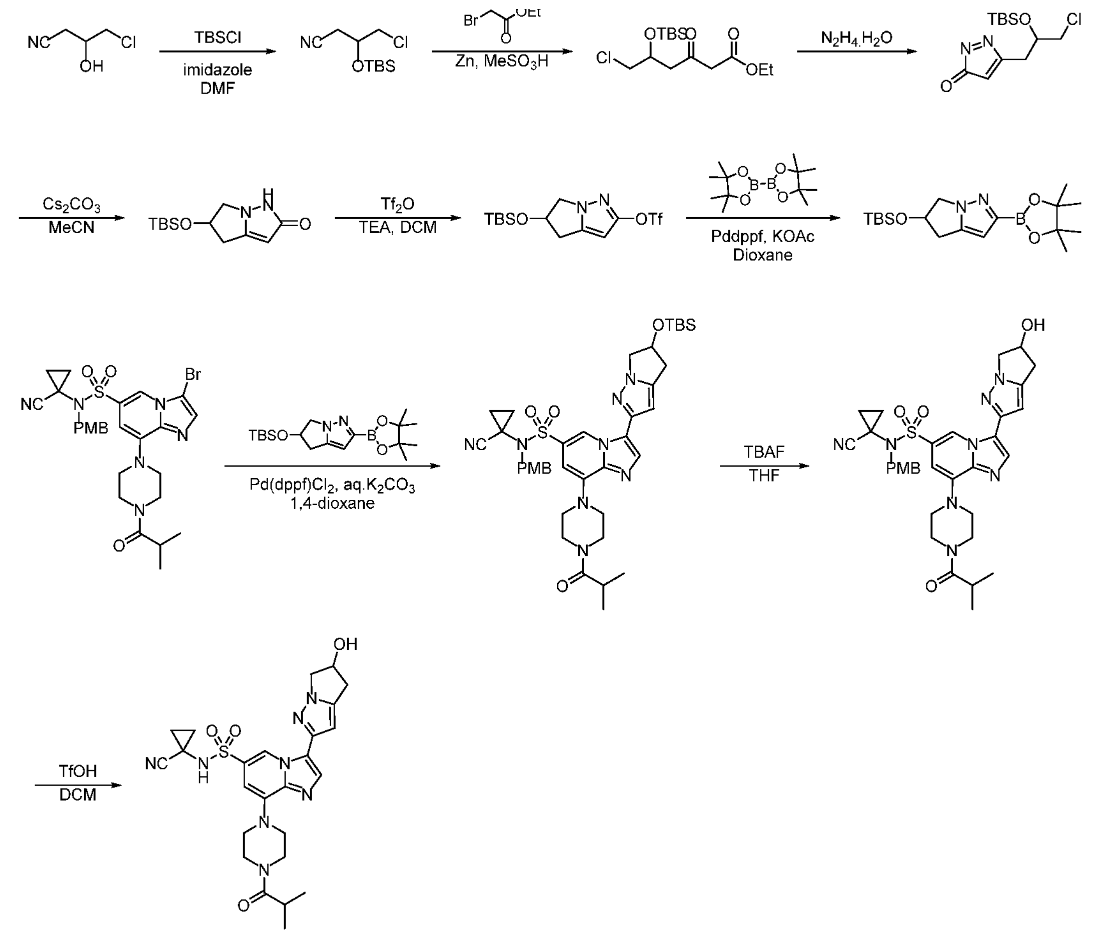


**Step 1: 3-((tert-butyldimethylsilyl)oxy)-4-chlorobutanenitrile**

At 0°C, imidazole (19 g, 281.0 mmol) and tert-butyldimethylchlorosilane (32 g, 210.7 mmol) were slowly added to the solution of 4-chloro-3-hydroxybutyronitrile (24 g, 200.7 mmol) in DMF (200 mL). The flask was replaced with nitrogen gas three times and then the mixture was stirred overnight at room temperature under an atmosphere of nitrogen. After completion of the reaction, the reaction mixture was quenched with saturated aqueous ammonium chloride solution, and extracted with ethyl acetate. The organic phase was washed with saturated brine, dried over anhydrous sodium sulfate, and concentrated under reduced pressure. The residue was purified by column chromatography (eluent: petroleum ether/ethyl acetate, gradient: 0-10% ethyl acetate) to obtain the title compound (colorless oil, 45 g, yield: 95.8%). $^1H$ NMR (400 MHz, $CDCl_3$) δ 4.11 - 4.09 (m, 1H), 3.55 - 3.43 (m, 2H), 2.72 - 2.60 (m, 2H), 0.89 (d, $J$ = 3.0 Hz, 9H), 0.14 (d, $J$ = 3.0 Hz, 3H), 0.11 (d, $J$ = 2.9 Hz, 3H).

**Step 2: Ethyl 5-((tert-butyldimethylsilyl)oxy)-6-chloro-3-oxohexanoate**

3-[(tert-butyldimethylsilyl)oxy]-4-chlorobutyronitrile (20 g, 85.5 mmol) was dissolved in tetrahydrofuran (200 mL); zinc powder (7.8 g, 119 mmol) and methanesulfonic acid (820 mg, 0.86 mmol) were added to the resulting mixture; the reaction mixture was stirred at 75°C for 10 minutes under an atmosphere of nitrogen; and then, 2-bromo ethyl acetate (11.4 mL, 102.6 mmol) was added. The reaction mixture was continued to be stirred at 75°C for 3 hours. After completion of the reaction, the reaction mixture was cooled to room temperature, poured into ice water, and extracted with ethyl acetate. The organic phase was washed with saturated brine, dried over anhydrous sodium sulfate, and concentrated under reduced pressure. The residue was purified by column chromatography (eluent: petroleum ether/ethyl acetate, gradient: 0-10% ethyl acetate) to obtain the title compound (colorless oil, 25 g, yield: 90.5%).

**Step 3: 5-(2-((tert-butyldimethylsilyl)oxy)-3-chloropropyl)-3H-pyrazol-3-one**

Hydrazine hydrate (3 mL) was added to the solution of 5-[(tert-butyldimethylsilyl)oxy]-6-chloro-3-oxoethyl caproate (12 g, 37.1 mmol); the reaction mixture was replaced three times with a nitrogen-filled balloon, and then stirred

at 65°C for 3 hours under an atmosphere of nitrogen. After completion of the reaction, the reaction mixture was cooled to room temperature, diluted with water, and extracted with ethyl acetate. The organic phase was washed with saturated brine, dried over anhydrous sodium sulfate, and concentrated under reduced pressure. The residue was purified by column chromatography (eluent: petroleum ether/ethyl acetate, gradient: 0-50% ethyl acetate) to obtain the title compound (white solid, 8 g, yield: 78.3%). $^1$H NMR (400 MHz, DMSO-$d_6$) δ 5.30 (s, 1H), 4.08 (dd, *J* = 11.0, 5.5 Hz, 1H), 3.59 - 3.54 (m, 1H), 3.52 - 3.48 (m, 1H), 2.72 (dd, *J* = 14.6, 5.7 Hz, 1H), 2.63 (dd, *J* = 14.6, 6.8 Hz, 1H), 0.83 (s, 9H), 0.04 (d, *J* = 2.9 Hz, 3H), -0.07 (s, 3H). LC/MS (ESI) (m/z): 289 $[M+H]^+$.

**Step 4: 5-((tert-butyldimethylsilyl)oxy)-5,6-dihydro-1H-pyrrolo[1,2-b]pyrazol-2(4H)-one**

At room temperature, cesium carbonate (5.0 g, 15.5 mmol) was added to the solution of 5-(2-((tertbutyldimethylsilyl)oxy)-3-chloropropyl)-1,2-dihydro-3H-pyrazol-3-one (3.8 g, 12.8 mmol) in acetonitrile (70 mL). The reaction mixture was replaced three times with a nitrogen-filled balloon and then stirred at 60°C for 8 hours under an atmosphere of nitrogen. After completion of the reaction, the reaction mixture was cooled to room temperature, diluted with water, and extracted with ethyl acetate. The organic phase was washed with saturated brine, dried over anhydrous sodium sulfate, and concentrated under reduced pressure. The residue was purified by column chromatography (eluent: petroleum ether/ethyl acetate, gradient: 0-50% ethyl acetate) to obtain the title compound (white solid, 2.5 g, yield: 76.5%). $^1$H NMR (400 MHz, $CDCl_3$) δ 5.26 (s, 1H), 4.86 - 4.81 (m, 1H), 4.06 (dd, *J* = 10.3, 6.4 Hz, 1H), 3.71 (dd, *J* = 10.3, 3.8 Hz, 1H), 3.02 (dd, *J* = 16.2, 7.0 Hz, 1H), 2.64 (dd, *J* = 16.2, 3.8 Hz, 1H), 0.79 (s, 9H), 0.00 (s, 6H). LC/MS (ESI) (m/z): 255 $[M+H]^+$.

**Step 5: 5-((tert-butyldimethylsilyl)oxy)-5,6-dihydro-4H-pyrrolo[1,2-b]pyrazol-2-yl trifluoromethanesulfonate**

At 0°C, triethylamine (1.8 mL, 12.9 mmol) was added to the solution of 5-((tert-butyldimethylsilyl)oxy]-5,6-dihydro-1H-pyrrolo[1,2-b]pyrazol-2(4H)-one (1.1 g, 4.3 mmol) in dichloromethane (10 mL); and then, trifluoromethanesulfonic anhydride (1.1 mL, 6.5 mmol) was dropwise added slowly. The reaction mixture was stirred at room temperature for 1.5 hours. After completion of the reaction, the mixture was concentrated under reduced pressure, and the residue was purified by column chromatography (eluent: petroleum ether/ethyl acetate, gradient: 0-10% ethyl acetate) to obtain the title compound (colorless oil, 1.3 g, yield: 77.8%). LC/MS (ESI) (m/z): 387 $[M+H]^+$.

**Step 6: 5-((tert-butyldimethylsilyl)oxy)-2-(4,4,5,5-tetramethyl-1,3,2-dioxaborolan-2-yl)-5,6-dihydro-4H-pyrrolo [1,2-b]pyrazole**

At room temperature, bis(pinacolato)diboron (591 mg, 2.3 mmol), potassium acetate (457 mg, 4.6 mmol), XPhos (148 mg, 0.3 mmol) and $Pd_2(dba)_3$ (142 mg, 0.16 mmol) were sequentially added to the solution of 5-((tert-butyldimethylsilyl)oxy)-5,6-dihydro-4H-pyrrolo[1,2-b]pyrazol-2-yl-trifluoromethanesulfonate (600 mg, 1.5 mmol) in 1,4-dioxane (10 mL). The flask was replaced with nitrogen gas three times and then the mixture was stirred at 80°C for 8 hours under an atmosphere of nitrogen. After completion of the reaction, the reaction mixture was cooled to room temperature, diluted with water, and extracted with ethyl acetate. The organic phase was washed with saturated brine, dried over anhydrous sodium sulfate, and concentrated under reduced pressure. The residue was purified by column chromatography (eluent: petroleum ether/ethyl acetate, gradient: 0-30% ethyl acetate) to obtain the title compound (white solid, 400 mg, yield: 70.7%). LC/MS (ESI) (m/z): 365 $[M+H]^+$.

**Step 7: 3-(5-((tert-butyldimethylsilyl)oxy)-5,6-dihydro-4H-pyrrolo[1,2-b]pyrazol-2-yl)-N-(1-cyanocyclopropyl)-8-(4-isobutyrylpiperazin-1-yl)-N-(4-methoxybenzyl)imidazo [1,2-a]pyridine-6-sulfonamide**

The title compound was prepared by referring to the preparation method in Step 5 of Example 7 (white solid, 160 mg, yield: 48.9%). LC/MS (ESI) (m/z): 774 $[M+H]^+$.

**Step 8: N-(1-cyanocyclopropyl)-3-(5-hydroxy-5,6-dihydro-4H-pyrrolo[1,2-b]pyrazol-2-yl)-8-(4-isobutyrylpiperazin-1-yl)-N-(4-methoxybenzyl)imidazo [1,2-a]pyridine-6-sulfonamide**

At 0°C, tetrabutylammonium fluoride (108 mg, 0.4 mmol) was added to the solution of 3-(5-((tertbutyldimethylsilyl)oxy)-5,6-dihydro-4H-pyrrolo[1,2-b]pyrazol-2-y1)-N-(1-cyanocyclopropyl)-8-4-(isobutyrylpiperazin-1-yl)-N-(4-methoxybenzyl)imidazo[1,2-a]pyridin-6-sulfonamide (160 mg, 0.2 mmol) in tetrahydrofuran (2 mL); and after the addition, the reaction mixture was stirred at room temperature for 30 minutes. The reaction mixture was diluted with ethyl acetate, sequentially washed with saturated aqueous ammonium chloride solution and saturated brine, dried over anhydrous sodium sulfate, filtered, and concentrated under reduced pressure. The residue was purified by column chromatography (eluent: petroleum ether/ethyl acetate, gradient: 50-100% ethyl acetate) to obtain the title compound (white solid, 120 mg,

yield: 88%). $^1$H NMR (400 MHz, $CD_3OD$) δ 9.68 (s, 1H), 7.95 (s, 1H), 7.24 (d, $J$ = 7.8 Hz, 2H), 6.82 - 6.78 (m, 3H), 6.52 (s, 1H), 5.08 - 5.04 (m, 1H), 4.53 - 4.51 (m, 2H), 4.47 - 4.43 (m, 1H), 4.12 - 4.08 (m, 2H), 3.87 - 3.84 (m, 4H), 3.75 - 3.73 (m, 3H), 3.56 - 3.52 (m, 2H), 3.47 - 3.44 (m, 2H), 3.04 - 2.99 (m, 1H), 2.91 - 2.86 (m, 1H), 1.49 - 1.43 (m, 4H), 1.14 (d, $J$ = 6.6 Hz, 6H). LC/MS (ESI) (m/z): 660 [M+H]$^+$.

**Step 9: N-(1-cyanocyclopropyl)-3-(5-hydroxy-5,6-dihydro-4H-pyrrolo[1,2-b]pyrazol-2-yl)-8-(4-isobutyrylpiperazin-1-yl)imidazo[1,2-a]pyridine-6-sulfonamide**

The title compound was prepared by referring to the preparation method in Step 7 of **Example 7** (white solid, 4 mg, yield: 25.4%). $^1$H NMR (400 MHz, DMSO-$d_6$) δ 9.55 (s, 1H), 8.04 (s, 1H), 6.76 (s, 1H), 6.59 (s, 1H), 5.67 - 5.62 (m, 1H), 4.98 - 4.93 (m, 1H), 4.45 - 4.40 (m, 1H), 4.02 - 3.97 (m, 1H), 3.76 - 3.69 (m, 4H), 3.66 - 3.53 (m, 5H), 2.98 - 2.93 (m, 1H), 2.79 - 2.74 (m, 1H), 1.44 - 1.39 (m, 2H), 1.33 - 1.28 (m, 2H), 1.04 (d, $J$ = 6.3 Hz, 6H). LC/MS (ESI) (m/z): 539 [M+H]$^+$.

**Example 10: N-(1-cyanocyclopropyl)-3-(5-fluoro-5,6-dihydro-4H-pyrrolo[1,2-b]pyrazol-2-yl)-8-(4-isobutyrylpiperazin-1-yl)imidazo[1,2-a]pyridine-6-sulfonamide**

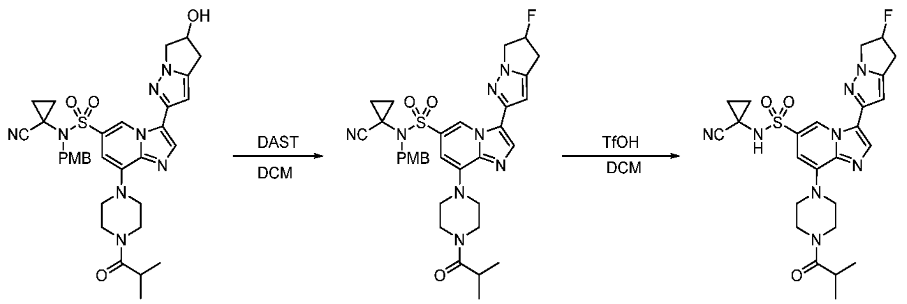


The title compound was prepared by referring to the preparation method in **Example 8** (white solid, 6 mg, yield: 24.4%). $^1$H NMR (400 MHz, DMSO-$d_6$) δ 9.57 (s, 1H), 9.28 (s, 1H), 8.09 (s, 1H), 6.77 (s, 1H), 6.69 (s, 1H), 5.92 (d, $J$ = 52.3 Hz, 1H), 4.62 - 4.41 (m, 2H), 3.76 - 3.65 (m, 6H), 3.60 - 3.54 (m, 2H), 3.47 - 3.39 (m, 1H), 3.18 - 3.11 (m, 1H), 2.98 - 2.91 (m, 1H), 1.48 - 1.43 (m, 2H), 1.37 - 1.33 (m, 2H), 1.04 (d, $J$ = 6.4 Hz, 6H). LC/MS (ESI) (m/z): 541 [M+H]$^+$.

**Example 11: N-(1-cyanocyclopropyl)-3-(5-(difluoromethyl)-1,3,4-thiadiazol-2-yl)-8-(4-isobutyrylpiperazin-1-yl)imidazo[1,2-a]pyridine-6-sulfonamide**

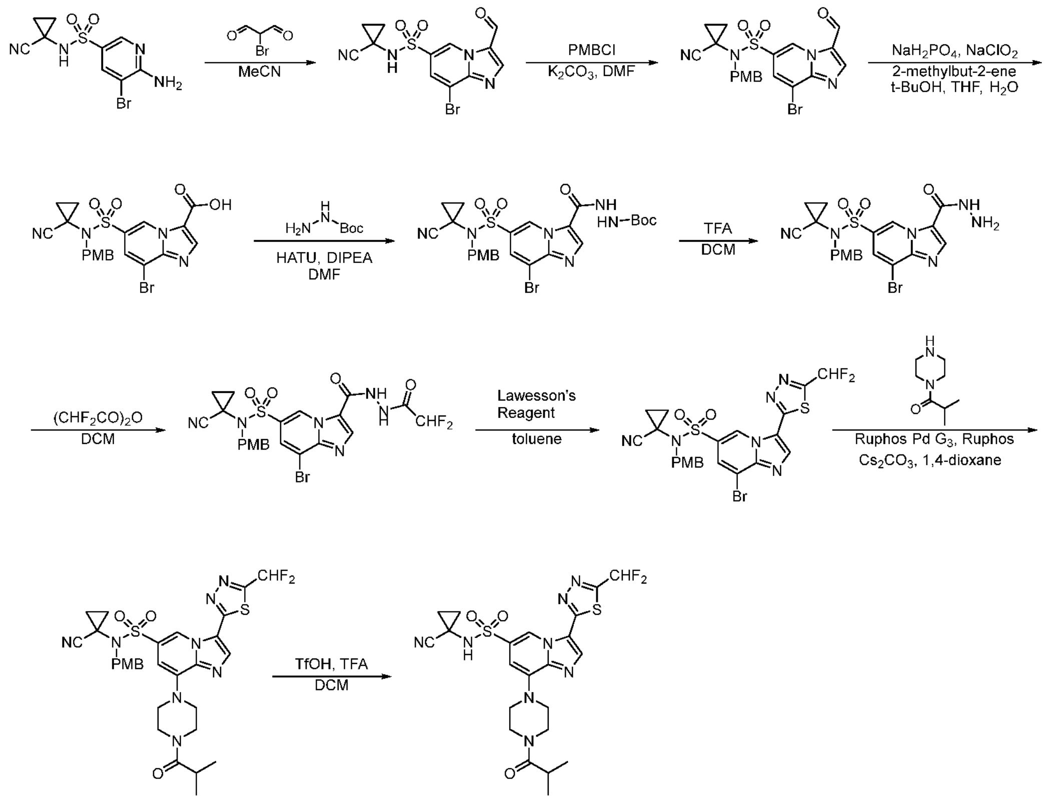


**Step 1: 8-bromo-N-(1-cyanocyclopropyl)-3-formylimidazo[1,2-a]pyridine-6-sulfonamide**

2-bromomalondialdehyde (710 mg, 4.7 mmol) was slowly added to the solution of 6-amino-5-bromo-N-(1-cyanochloropropyl)pyridin-3-sulfonamide (1.5 g, 4.7 mmol) in acetonitrile (15 mL). The reaction mixture was stirred at room temperature for 16 hours. After completion of the reaction, the reaction mixture was concentrated under reduced pressure. The residue was purified by column chromatography (eluent: petroleum ether, ethyl acetate, gradient: 0-60% ethyl acetate) to obtain the title compound (yellow solid, 960 mg, yield: 59.5%). $^1$HNMR (400 MHz, DMSO-$d_6$) δ 10.07 (s, 1H), 9.84 (d, $J$ = 1.6 Hz, 1H), 9.60 (s, 1H), 8.76 (s, 1H), 8.17 (d, $J$ = 1.6 Hz, 1H), 1.54 - 1.47 (m, 2H), 1.40 - 1.35 (m, 2H). LC/MS (ESI) m/z: 369 $[M+H]^+$.

**Step 2: 8-bromo-N-(1-cyanocyclopropyl)-3-formyl-N-(4-methoxybenzyl)imidazo[1,2-a]pyridine-6-sulfonamide**

$K_2CO_3$ (1.1 g, 7.8 mmol) and 4-methoxybenzyl chloride (611 mg, 3.9 mmol) was slowly added to the solution of 8-bromo-N-(1-cyanochloropropyl)-3-formylimidazo[1,2-a]pyridin-6-sulfonamide (960 mg, 2.6 mmol) in N,N-dimethylformamide (8 mL). The reaction mixture was stirred at 45°C for 16 hours under an atmosphere of nitrogen. After completion of the reaction, the reaction mixture was cooled to room temperature; water was added; and the resulting mixture was extracted with ethyl acetate, washed with saturated brine, dried over anhydrous sodium sulfate, and concentrated to dryness under reduced pressure. The residue was purified by column chromatography (eluent: petroleum ether/ethyl acetate, gradient: 0-45% ethyl acetate) to obtain the title compound (yellow solid, 800 mg, yield: 62.9%). LC/MS (ESI) m/z: 489 $[M+H]^+$.

**Step 3: 8-bromo-6-(N-(1-cyanocyclopropyl)-N-(4-methoxybenzyl)sulfamoyl)imidazo[1,2-a]pyridine-3-carboxylic acid**

At 0°C, 8-bromo-N-(1-cyanocyclopropyl)-3-formyl-N-(4-methoxybenzyl)imidazo[1,2-a]pyridin-6-sulfonamide (800 mg, 1.6 mmol) was dissolved in the mixed solution of tertiary butanol (6 mL), tetrahydrofuran (3 mL) and water (2 mL), and sodium dihydrogen phosphate (981 mg, 8.2 mmol), 2-methylbut-2-ene (917 mg, 13.1 mmol) and sodium chlorite (370 mg, 4.1 mmol) were sequentially added. The reaction mixture was stirred at room temperature for 30 minutes. After completion of the reaction, water was added; and the resulting mixture was extracted with ethyl acetate, washed with saturated brine, dried over anhydrous sodium sulfate, and concentrated under reduced pressure. The residue was purified by column chromatography (eluent: dichloromethane/methanol, gradient: 0-4% methanol) to obtain the title compound (yellow solid, 400 mg, yield: 48.2%). LC/MS (ESI) m/z: 505 $[M+H]^+$.

**Step 4: Tert-butyl 2-(8-bromo-6-(N-(1-cyanocyclopropyl)-N-(4-methoxybenzyl)sulfamoyl)imidazo[1,2-a]pyridine-3-carbonyl)hydrazine-1-carboxylate**

At room temperature, N,N-diisopropylethylamine (307 mg, 2.38 mmol) and 2-(7-azobenzotriazol)-N,N,N',N'-tetramethylurea hexafluorophosphate (331 mg, 0.87 mmol) were sequentially added to the solution of 8-bromo-6-(N-(1-cyanocyclopropyl)-N-(4-methoxybenzyl)sulfamoyl)imidazo[1,2-a]pyridin-3-carboxylic acid (400 mg, 0.79 mmol) and N'-[(tert-butoxy)carbonyl](tert-butoxy)formylhydrazine (115 mg, 0.87 mmol) in N,N-dimethylformamide (4 mL). The reaction mixture was stirred at room temperature for 30 minutes. After completion of the reaction, water was added; and the resulting mixture was extracted with ethyl acetate, washed with saturated brine, dried over anhydrous sodium sulfate, and concentrated under reduced pressure. The residue was purified by column chromatography (eluent: petroleum ether, ethyl acetate, gradient: 0-45% ethyl acetate) to obtain the title compound (yellow solid, 260 mg, yield: 53.1%). LC/MS (ESI) (m/z): 619 $[M+H]^+$.

**Step 5: 8-bromo-N-(1-cyanocyclopropyl)-3-(hydrazinecarbonyl)-N-(4-methoxybenzyl)imidazo[1,2-a]pyridine-6-sulfonamide**

At 0°C, trifluoroacetic acid (1 mL) was slowly added to the solution of 2-(8-bromo-6-(N-(1-cyanocyclopropyl)-N-(4-methoxybenzyl)sulfamoyl)imidazo[1,2-a]pyridin-3-carbonyl)hydrazin-1-tert-butyl carboxylate (260 mg, 0.42 mmol) in dichloromethane (2 mL). The reaction mixture was stirred at room temperature for 30 minutes. After completion of the reaction, the reaction mixture was concentrated under reduced pressure to obtain the title compound (yellow oil, 220 mg, yield: 100%). LC/MS (ESI) (m/z): 519 $[M+H]^+$.

**Step 6: 8-bromo-N-(1-cyanocyclopropyl)-3-(2-(2,2-difluoroacetyl)hydrazine-1-carbonyl)-N-(4-methoxybenzyl) imidazo[1,2-a]pyridine-6-sulfonamide**

At 0°C, 2,2-difluoroacetyl-2,2-difluoroacetate (62 mg, 0.47 mmol) was dropwise added slowly into the solution of 8-bromo-N-(1-cyanocyclopropyl)-3-(hydrazinocarbonyl)-N-(4-methoxybenzyl)imidazo[1,2-a]pyridin-6-sulfonamide (220 mg, 0.42 mmol) in dichloromethane (3 mL). The reaction mixture was stirred at room temperature for 30 minutes. After completion of the reaction, the reaction mixture was concentrated under reduced pressure. The residue was purified by column chromatography (eluent: petroleum ether/ethyl acetate, gradient: 0-50% ethyl acetate) to obtain the title compound (yellow solid, 195 mg, yield: 77.1%). LC/MS (ESI) (m/z): 597 $[M+H]^+$.

**Step 7: 8-bromo-N-(1-cyanocyclopropyl)-3-(5-(difluoromethyl)-1,3,4-thiadiazol-2-yl)-N-(4-methoxybenzyl)imidazo[1,2-a]pyridine-6-sulfonamide**

Under an atmosphere of nitrogen at room temperature, the Lawesson's reagent (52 mg, 0.13 mmol) was added to the solution of 8-bromo-N-(1-cyanocyclopropyl)-3-(2-(2,2-difluoroacetyl)hydrazin-1-carbonyl)-N-(4-methoxybenzyl)imidazo[1,2-a]pyridin-6-sulfonamide (70 mg, 0.12 mmol) in toluene (1 mL). The reaction mixture was stirred at 110°C for 4 hours under an atmosphere of nitrogen. After completion of the reaction, the reaction mixture was cooled to room temperature, diluted with ethyl acetate, washed with saturated aqueous sodium bicarbonate solution and saturated brine in sequence, dried over anhydrous sodium sulfate, and concentrated under reduced pressure. The residue was purified by column chromatography (eluent: petroleum ether, ethyl acetate, gradient: 0-55% ethyl acetate) to obtain the title compound (yellow solid, 35 mg, yield: 50%). LC/MS (ESI) (m/z): 595 $[M+H]^+$.

**Step 8: N-(1-cyanocyclopropyl)-3-(5-(difluoromethyl)-1,3,4-thiadiazol-2-yl)-8-(4-isobutyrylpiperazin-1-yl)-N-(4-methoxybenzyl)imidazo[1,2-a]pyridine-6-sulfonamide**

Under an atmosphere of nitrogen at room temperature, cesium carbonate (38 mg, 0.12 mmol), RuPhos (5 mg, 0.012 mmol)and RuPhos Pd G3 (5 mg, 0.006 mmol) were added to the solution of 8-bromo-N-(1-cyanocyclopropyl)-3-(5-(difluoromethyl)-1,3,4-thiadiazol-2-yl)-N-(4-methoxybenzyl)imidazo[1,2-a]pyridin-6-sulfonamide (35 mg, 0.06 mmol) and 2-methyl-1-(piperazin-1-yl)propan-1-one (18 mg, 0.12 mmol) in 1,4-dioxane (1 mL). The flask was replaced with nitrogen gas three times and then the mixture was stirred at 110°C for 3 hours under an atmosphere of nitrogen. After completion of the reaction, the reaction mixture was cooled to room temperature; water was added; and the resulting mixture was extracted with ethyl acetate, washed with saturated brine, dried over anhydrous sodium sulfate, and concentrated under reduced pressure. The residue was separated by column chromatography (eluent: dichloromethane/methanol, gradient: 0-4% methanol) to obtain the title compound (yellow solid, 24 mg, yield: 60%). LC/MS (ESI) m/z: 671 $[M+H]^+$.

**Step 9: N-(1-cyanocyclopropyl)-3-(5-(difluoromethyl)-1,3,4-thiadiazol-2-yl)-8-(4-isobutyrylpiperazin-1-yl)imidazo[1,2-a]pyridine-6-sulfonamide**

At 0°C, trifluoroacetic acid (0.5 mL) and trifluoromethanesulfonic acid (0.1 mL) was slowly added to the solution of N-(1-cyanocyclopropyl)-3-(5-(difluoromethyl)-1,3,4-thiadiazol-2-yl)-8-(4-isobutyrylpiperazin-1-yl)-N-(4-methoxybenzyl) imidazo[1,2-a]pyridin-6-sulfonamide (24 mg, 0.04 mmol) in dichloromethane (0.5 mL). The reaction mixture was stirred at room temperature for 30 minutes. After completion of the reaction, the reaction mixture was concentrated under reduced pressure. The residue was purified by pre-HPLC ($C_{18}$, 10-80% acetonitrile in $H_2O$ and 0.1% HCOOH) to obtain the title compound (white solid, 3 mg, yield: 15.2%). $^1H$ NMR (400 MHz, DMSO-$d_6$) δ 9.75 (d, *J* = 1.2 Hz, 1H), 8.66 (s, 1H), 7.70 (t, *J* = 53.2 Hz, 1H), 7.00 (s, 1H), 3.81 - 3.59 (m, 8H), 3.00 - 2.91 (m, 1H), 1.50 - 1.41 (m, 2H), 1.41 - 1.34 (m, 2H), 1.05 (s, 3H), 1.03 (s, 3H). LC/MS (ESI) (m/z): 551 $[M+H]^+$.

**Example 12: N-(1-cyanocyclopropyl)-3-(5-(difluoromethyl)-1,3,4-thiadiazol-2-yl)-8-(1-isobutyryl-1,2,3,6-tetrahydropyridin-4-yl)imidazo[1,2-a]pyridine-6-sulfonamide**

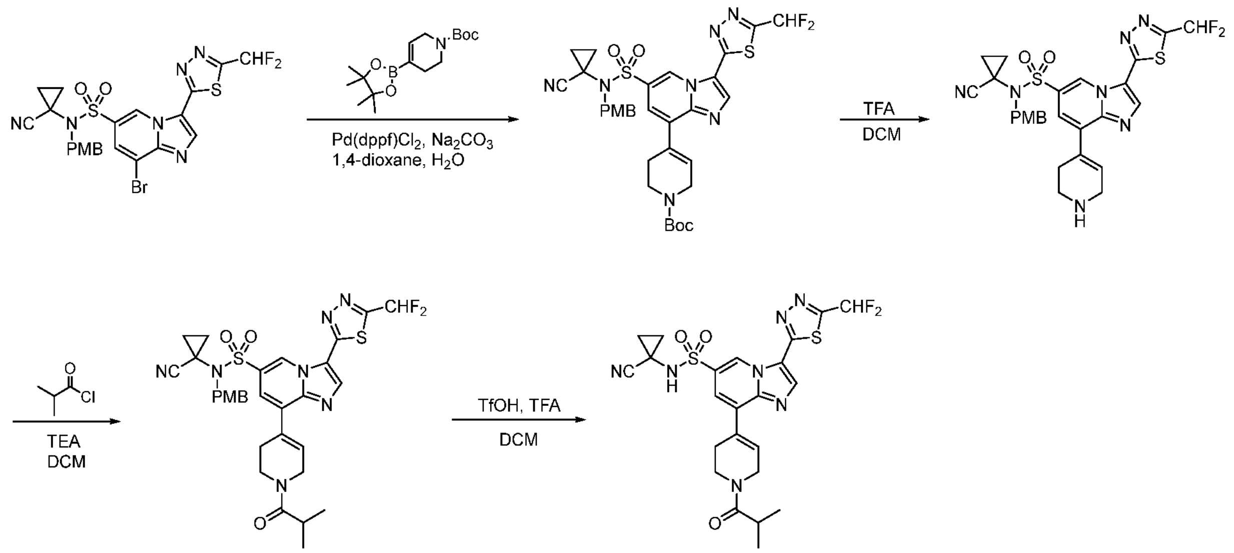


**Step 1: Tert-butyl 4-(6-(N-(1-cyanocyclopropyl)-N-(4-methoxybenzyl)sulfamoyl)-3-(5-(difluoromethyl)-1,3,4-thiadiazol-2-yl)imidazo[1,2-a]pyridin-8-yl)-3,6-dihydropyridine-1(2H)-carboxylate**

At room temperature, sodium carbonate (32 mg, 0.30 mmol), N-tert-butoxycarbonyl-1,2,5,6-tetrahydropyridin-4-pinacolboronate (62 mg, 0.20 mmol) and Pd(dppf)$Cl_2$ (7 mg, 0.01 mmol) were sequentially added to the solution of 8-bromo-N-(1-cyanocyclopropyl)-3-(5-(difluoromethyl)-1,3,4-thiadiazol-2-yl)-N-(4-methoxybenzyl)imidazol[1,2]pyridin-6-sulfonamide (60 mg, 0.10 mmol) in 1,4-dioxane (2.4 mL) and water (0.4 mL). The flask was replaced with nitrogen gas three times and then the mixture was stirred at 90°C for 4 hours under an atmosphere of nitrogen. After completion of the reaction, the reaction mixture was cooled to room temperature, the reaction mixture was diluted with ethyl acetate, washed with saturated brine, dried over anhydrous sodium sulfate, filtered, and concentrated under reduced pressure. The residue was purified by silica gel chromatography (eluent: ethyl acetate/petroleum ether, gradient: 0-50% ethyl acetate) to obtain the title compound (yellow oil, 65 mg, yield: 92.4%). LC/MS (ESI) (m/z): 698 $[M+H]^+$.

**Step 2: N-(1-cyanocyclopropyl)-3-(5-(difluoromethyl)-1,3,4-thiadiazol-2-yl)-N-(4-methoxybenzyl)-8-(1,2,3,6-tetrahydropyridin-4-yl)imidazo[1,2-a]pyridine-6-sulfonamide**

In an ice bath, trifluoroacetic acid (0.4 mL) was slowly added to the solution of 4-(6-(N-(1-cyanocyclopropyl)-N-(4-methoxybenzyl)sulfamoyl)-3-(5-(difluoromethyl)-1,3,4-thiadiazol-2-yl)imidazo[1,2-a]pyridin-8-yl)-3,6-dihydropyridin-1(2H)-tert-butyl carboxylate(65 mg, 0.09 mmol) in dichloromethane (1.2 mL); and the reaction mixture was stirred at room temperature for 30 minutes. After completion of the reaction, the reaction mixture was directly concentrated to obtain the title compound (yellow oil, 60 mg, yield: 100%), which was directly used in the next step of reaction. LC/MS (ESI) (m/z): 598 $[M+H]^+$.

**Step 3: N-(1-cyanocyclopropyl)-3-(5-(difluoromethyl)-1,3,4-thiadiazol-2-yl)-8-(1-isobutyryl-1,2,3,6-tetrahydropyridin-4-yl)-N-(4-methoxybenzyl)imidazo[1,2-a]pyridine-6-sulfonamide**

At 0°C, triethylamine (0.02 mL, 0.16 mmol) and isobutyryl chloride (0.01 mL, 0.12 mmol) were sequentially added to the solution of N-(1-cyanocyclopropyl)-3-(5-(difluoromethyl)-1,3,4-thiadiazol-2-yl)-N-(4-methoxybenzyl)-8-(1,2,3),6-tetrahydropyridin-4-yl)imidazo[1,2-a]pyridin-6-sulfonamide (60 mg, 0.09 mmol) in dichloromethane (2 mL). The reaction mixture was stirred at room temperature for 1 hours. After completion of the reaction, the reaction mixture was cooled to room temperature, quenched by adding water, extracted with dichloromethane, washed with saturated brine, dried over anhydrous sodium sulfate, filtered, and concentrated under reduced pressure to obtain the title compound (yellow solid, 35 mg, yield: 56.3%). LC/MS (ESI) (m/z): 668 $[M+H]^+$.

**Step 4: N-(1-cyanocyclopropyl)-3-(5-(difluoromethyl)-1,3,4-thiadiazol-2-yl)-8-(1-isobutyryl-1,2,3,6-tetrahydropyridin-4-yl)imidazo[1,2-a]pyridine-6-sulfonamide**

The title compound was obtained by referring to the synthesis method in Step 11 of **Example 11.** $^1$H NMR (400 MHz, DMSO-$d_6$) δ 10.09 (s, 1H), 9.56 (s, 1H), 8.79 (s, 1H), 7.86 - 7.58 (m, 2H), 7.38 (d, $J$ = 29.4 Hz, 1H), 4.33 (d, $J$ = 54.7 Hz, 2H), 3.81 (d, 2H), 3.04 - 2.91 (m, 1H), 2.74 (d, $J$ = 51.4 Hz, 2H), 1.48 (d, $J$ = 4.0 Hz, 2H), 1.40 (d, $J$ = 4.6 Hz, 2H), 1.07 (d, $J$ = 6.6 Hz, 6H). LC/MS (ESI) (m/z): 548 $[M+H]^+$.

**Example 13: 8-(2-acetyl-2-azaspiro[3.4]oct-6-en-6-yl)-N-(1-cyanocyclopropyl)-3-(5-(difluoromethyl)-1,3,4-thiadiazol-2-yl)imidazo[1,2-a]pyridine-6-sulfonamide**

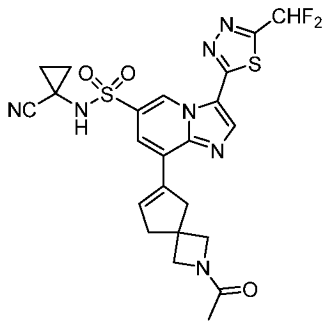

The title compound was prepared by referring to the synthesis method in **Example 12,** using 6-(4,4,5,5-tetramethyl-1,3,2-dioxaborolan-2-yl)-2-azaspirocyclo[3.4]oct-6-ene-2-tert-butyl carboxylate instead of N-tert-butoxycarbonyl-1,2,5,6-tetrahydropyridin-4-pinacolboronate to obtain the target molecules. $^1$H NMR (400 MHz, DMSO-$d_6$) δ 10.08 (d, $J$ = 1.7 Hz, 1H), 8.81 (s, 1H), 7.87 - 7.55 (m, 3H), 4.30 (d, $J$ = 8.4 Hz, 1H), 4.18 (d, $J$ = 8.3 Hz, 1H), 3.97 (d, $J$ = 9.5 Hz, 1H), 3.90 (d, $J$ = 9.5 Hz, 1H), 2.95 (t, $J$ = 6.8 Hz, 2H), 2.36 (t, 2H), 1.80 (s, 3H), 1.52 - 1.46 (m, 2H), 1.43 - 1.35 (m, 2H). LC/MS (ESI) (m/z): 546 $[M+H]^+$.

**Example 14: 3-(5-(difluoromethyl)-1,3,4-thiadiazol-2-yl)-N-(1-(fluoromethyl)cyclopropyl)-8-(4-isobutyrylpiperazin-1-yl)imidazo[1,2-a]pyridine-6-sulfonamide**

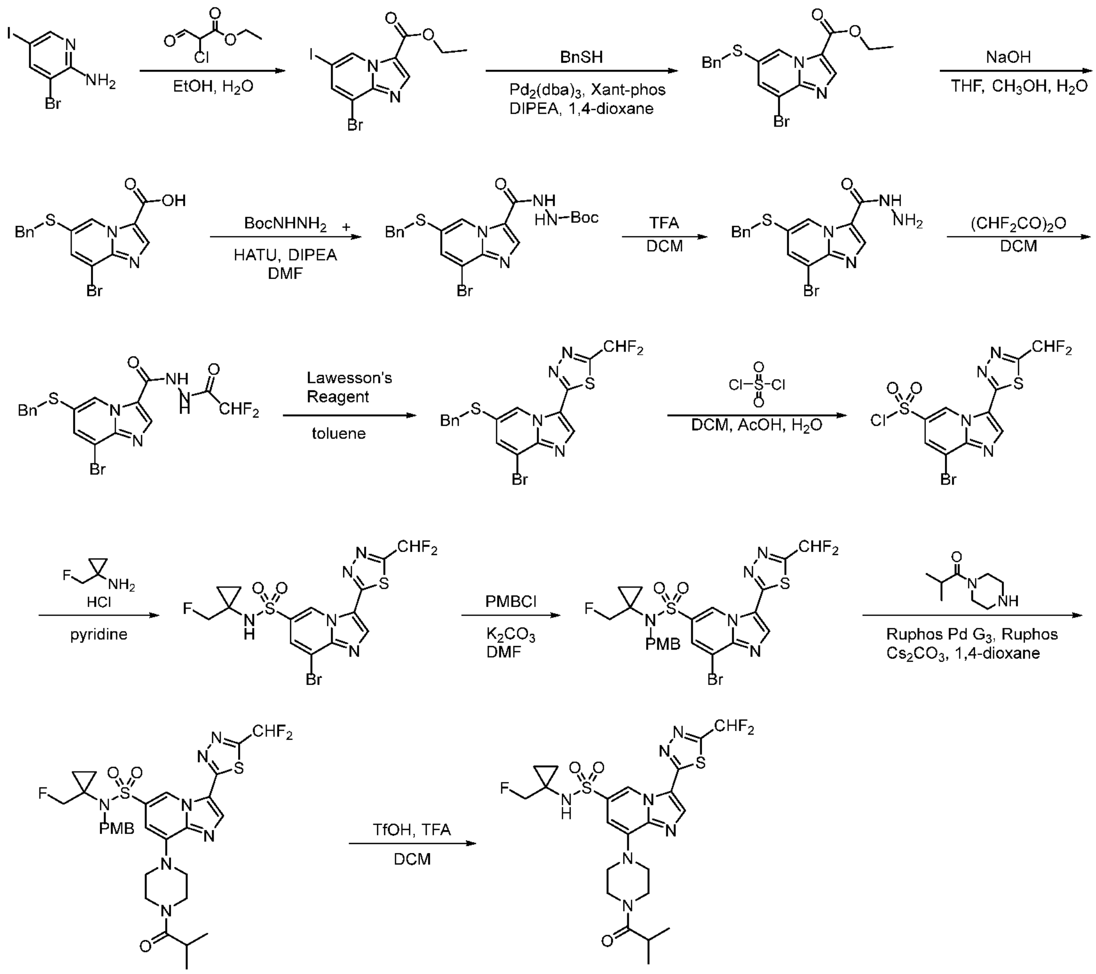


**Step 1: Ethyl 8-bromo-6-iodoimidazo[1,2-a]pyridine-3-carboxylate**

2-chloro-3-oxoethyl propionate (4 g, 13.4 mmol) was dropwise added to the solution of 3-bromo-5-iodopyridin-2-amine (4 g, 13.4 mmol) in ethanol (20 mL) and water (20 mL). The reaction system was stirred overnight at 90°C under an atmosphere of nitrogen. Then, the mixture was cooled to room temperature and filtered, and the filter cake was dried under reduced pressure to obtain target molecules (white solid, 4 g, yield: 75.7%), which were directly used in the next step of reaction. $^1$H NMR (400 MHz, $CDCl_3$) δ 9.54 (d, *J* =1.4 Hz, 1H), 8.21 (s, 1H), 7.84 (d, *J* =1.4 Hz, 1H), 4.45 - 4.32 (m, 2H), 1.39 (t, *J* =7.1 Hz, 3H). LC/MS (ESI) (m/z): 395 $[M+H]^+$.

**Step 2: Ethyl 6-(benzylthio)-8-bromoimidazo[1,2-a]pyridine-3-carboxylate**

N,N-diisopropylethylamine (3.9 g, 30.3 mmol) was dropwise added to the solution of 8-bromo-6-iodoimidazol [1,2-a]pyridin-3-ethyl carboxylate (4 g, 10.1 mmol) in 1,4-dioxane (80 mL); and then, under an atmosphere of nitrogen, benzyl mercaptan (1.3 g, 10.1 mmol), XantPhos (579 mg, 1.0 mmol) and $Pd_2(dba)_3$ (467 mg, 0.51 mmol) were added respectively. The flask was replaced with nitrogen gas three times and then the mixture was stirred at 70°C for 2 hours under an atmosphere of nitrogen. The reaction mixture was diluted with ethyl acetate, sequentially washed with water and saturated brine, dried over anhydrous sodium sulfate, filtered and concentrated under reduced pressure to obtain a crude product. The residue was purified by silica gel chromatography (eluent: petroleum ether/ethyl acetate, gradient: 0-15% ethyl acetate) to obtain the title compound (yellow solid, 3.5 g, yield: 88.3%). LC/MS (ESI) (m/z): 393 $[M+H]^+$.

**Step 3: 6-(benzylthio)-8-bromoimidazo[1,2-a]pyridine-3-carboxylic acid**

At 0°C, sodium hydroxide (82 mg, 2.04 mmol) was added to the solution of 6-(benzylthio)-8-bromoimidazol[1,2-a] pyridin-3-ethyl carboxylate (400 mg, 1.02 mmol) in methanol (1.5 mL), tetrahydrofuran (1.5 mL) and water (1.5 mL); and the reaction mixture was stirred at 45°C for one hour. The reaction mixture was diluted with water, acidified to pH-5 with 1N hydrochloric acid, and extracted with ethyl acetate twice. The combined organic phase was dried over anhydrous sodium

sulfate, and concentrated under reduced pressure to obtain target molecules (yellow solid, 350 mg, yield: 94.3%), which were directly used in the next reaction. LC/MS (ESI) (m/z): 365 $[M+H]^+$.

**Step 4: Tert-butyl 2-(6-(benzylthio)-8-bromoimidazo[1,2-a]pyridine-3-carbonyl)hydrazine-1-carboxylate**

N,N-diisopropylethylamine (373 mg, 2.89 mmol) and 2-(7-azobenzotriazol)-N,N,N',N'-tetramethylurea hexafluorophosphate (403 mg, 1.06 mmol) were sequentially added to the solution of 6-(benzylthio)-8-bromoimidazo[1,2-a] pyridin-3-carboxylic acid (350 mg, 0.96 mmol) and tert-butyl hydrazinoformate (127 mg, 0.96 mmol) in DMF (4 mL); and the reaction mixture was stirred at room temperature for 30 minutes. The reaction mixture was diluted with ethyl acetate, sequentially washed with water and saturated brine, dried over anhydrous sodium sulfate, filtered and concentrated under reduced pressure. The residue was purified by silica gel chromatography (eluent: ethyl acetate/petroleum ether, gradient: 0-50% ethyl acetate) to obtain the title compound (yellow solid, 440 mg, yield: 95.7%). LC/MS (ESI) (m/z): 479 $[M+H]^+$.

**Step 5: 6-(benzylthio)-8-bromoimidazo[1,2-a]pyridine-3-carbohydrazide**

At 0°C, trifluoroacetic acid (1 mL) was dropwise added to the solution of 2-(6-(benzylthio)-8-bromoimidazo[1,2-a] pyridin-3-carbonyl)hydrazin-1-tert-butyl carboxylate (440 mg, 0.92 mmol) in dichloromethane (3 mL); and the reaction mixture was stirred at room temperature for 30 minutes. The reaction mixture was and concentrated under reduced pressure to obtain a crude target product (yellow oil, 348 mg, yield: 100%), which was directly used in the next reaction. LC/MS (ESI) (m/z): 379 $[M+H]^+$.

**Step 6: 6-(benzylthio)-8-bromo-N'-(2,2-difluoroacetyl)imidazo[1,2-a]pyridine-3-carbohydrazide**

At 0°C, 2,2-difluoroacetyl-2,2-difluoroacetate (160 mg, 0.92 mmol) was dropwise added to the solution of 6-(benzylthio)-8-bromoimidazo[1,2-a]pyridin-3-formylhydrazine (348 mg, 0.92 mmol) in dichloromethane (4 mL). The reaction mixture was stirred at room temperature for 30 minutes. After completion of the reaction, the reaction mixture was diluted with dichloromethane, washed with saturated sodium bicarbonate solution and saturated brine respectively, dried over anhydrous sodium sulfate, filtered and concentrated to dryness. The residue was purified by silica gel chromatography (eluent: petroleum ether/ethyl acetate, gradient: 0-50% ethyl acetate) to obtain the target product (yellow solid, 350 mg, yield: 83.3 %). LC/MS (ESI) (m/z): 457 $[M+H]^+$.

**Step 7: 2-(6-(benzylthio)-8-bromoimidazo[1,2-a]pyridin-3-yl)-5-(difluoromethyl)-1,3,4-thiadiazole**

Under an atmosphere of nitrogen, the Lawesson's reagent (326 mg, 0.81 mmol) was added to the solution of 6-(benzylthio)-8-bromo-N'-(2,2-difluoroacetyl)imidazo[1,2-a]pyridin-3-carbohydrazide (350 mg, 0.77 mmol) in toluene (4 mL); the flask was replaced with nitrogen gas three times and then the mixture was stirred at 110°C for 2 hours under an atmosphere of nitrogen. The reaction mixture was cooled to room temperature, diluted with ethyl acetate, sequentially washed with water and saturated brine, dried over anhydrous sodium sulfate, filtered and concentrated under reduced pressure. The residue was purified by silica gel chromatography (eluent: petroleum ether/ethyl acetate, gradient: 0-30% ethyl acetate) to obtain the target product (yellow solid, 310 mg, yield: 89%). LC/MS (ESI) (m/z): 455 $[M+H]^+$.

**Step 8: 8-bromo-3-(5-(difluoromethyl)-1,3,4-thiadiazol-2-yl)imidazo[1,2-a]pyridine-6-sulfonyl chloride**

At 0°C, acetic acid (0.1 mL) and water (0.05 mL) were sequentially added to the solution of 2-(6-(benzylthio)-8-bromoimidazo[1,2-a]pyridin-3-yl)-5-(difluoromethyl)-1,3,4-thiadiazole (310 mg, 0.68 mmol) in dichloromethane (3 mL); then, sulfonyl chloride (369 mg, 2.74 mmol) was dropwise added slowly; and the reaction mixturewas was stirred at room temperature for 30 minutes. The reaction mixture was diluted with dichloromethane, sequentially washed with water and saturated brine, dried over anhydrous sodium sulfate, filtered, and concentrated under reduced pressure to obtain a crude target product (yellow oil, 294 mg, yield: 100%), which was directly used in the next step of reaction. LC/MS (ESI) (m/z): 431 $[M+H]^+$.

**Step 9: 8-bromo-3-(5-(difluoromethyl)-1,3,4-thiadiazol-2-yl)-N-(1-(fluoromethyl)cyclopropyl)imidazo[1,2-a]pyridine-6-sulfonamide**

At 0°C, 8-bromo-3-(5-(difluoromethyl)-1,3,4-thiadiazol-2-yl)imidazo[1,2-a]pyridin-6-sulfonyl chloride (294 mg, 0.68 mmol) was added to the solution of 1-(fluoromethyl)cyclopropyl-1-amine hydrochloride (172 mg, 1.37 mmol) in pyridine (3 mL); and then, the reaction mixture was stirred at room temperature for 30 minutes. The reaction mixture was concentrated under reduced pressure, and the residue was purified by silica gel chromatography (eluent: petroleum ether/

ethyl acetate, gradient: 0 - 45% ethyl acetate) to obtain target molecules (yellow solid, 100 mg, yield: 30.3%). LC/MS (ESI) m/z: 484 [M+H]$^+$.

**Step 10: 8-bromo-3-(5-(difluoromethyl)-1,3,4-thiadiazol-2-yl)-N-(1-(fluoromethyl)cyclopropyl)-N-(4-methoxybenzyl)imidazo[1,2-a]pyridine-6-sulfonamide**

Potassium carbonate (86 mg, 0.63 mmol) and 1-(chloromethyl)-4-metoxybenzene (49 mg, 0.31 mmol) were sequentially added to the solution of 8-bromo-3-(5-(difluoromethyl)-1,3,4-thiadiazol-2-yl)-N-(1-(fluoromethyl)cyclopropyl) imidazo[1,2-a]pyridin-6-sulfonamide (100 mg, 0.21 mmol) in N,N- (2 mL); and the reaction mixture was stirred at 45°C for 2 hours. The mixture was diluted with ethyl acetate, sequentially washed with water and saturated brine, dried over anhydrous sodium sulfate, filtered and concentrated under reduced pressure. The residue was purified by silica gel chromatography (eluent: petroleum ether/ethyl acetate, gradient: 0-35% ethyl acetate) to obtain the target product (yellow solid, 75 mg, yield: 60%). LC/MS (ESI) m/z: 604 [M+H]$^+$.

**Step 11: 3-(5-(difluoromethyl)-1,3,4-thiadiazol-2-yl)-N-(1-(fluoromethyl)cyclopropyl)-8-(4-isobutyrylpiperazin-1-yl)-N-(4-methoxybenzyl)imidazo[1,2-a]pyridine-6-sulfonamide**

Under an atmosphere of nitrogen, 2-methyl-1-(piperazin-1-yl)propan-1-one (39 mg, 0.25 mmol), cesium carbonate (81 mg, 0.25 mmol), RuPhos (12 mg, 0.02 mmol) and RuPhos Pd G3 (10 mg, 0.01 mmol) were sequentially added to the solution of 8-bromo-3-(5-(difluoromethyl)-1,3,4-thiadiazol-2-yl)-N-(1-(fluoromethyl)cyclopropyl)-N-(4-methoxybenzyl)imidazo[1,2-a]pyridin-6-sulfonamide (75 mg, 0.12 mmol) in 1,4-dioxane (2 mL). The flask was replaced with nitrogen gas three times and then the mixture was stirred at 100°C for 3 hours under an atmosphere of nitrogen. The reaction mixture was diluted with ethyl acetate, and filtered; the filtrate was concentrated under reduced pressure; and the residue was purified by silica gel chromatography (eluent: petroleum ether/ethyl acetate, gradient: 0-75% ethyl acetate) to obtain the target product (yellow solid, 60 mg, yield: 71.1%). LC/MS (ESI) m/z: 678 [M+H]$^+$.

**Step 12: 3-(5-(difluoromethyl)-1,3,4-thiadiazol-2-yl)-N-(1-(fluoromethyl)cyclopropyl)-8-(4-isobutyrylpiperazin-1-yl)imidazo[1,2-a]pyridine-6-sulfonamide**

At 0°C, trifluoroacetic acid (0.5 mL) and trifluoromethanesulfonic acid (0.1 mL) were sequentially added to the solution of 3-(5-(difluoromethyl)-1,3,4-thiadiazol-2-yl)-N-(1-(fluoromethyl)cyclopropyl)-8-(4-isobutyrylpiperazin-1-yl)-N-(4-methoxybenzyl)imidazo[1,2-a]pyridin-6-sulfonamide (60 mg, 0.09 mmol) in dichloromethane (0.5 mL); and then, the reaction mixture was stirred at room temperature for 30 minutes. The mixture was concentrated to dryness, and the residue was purified by pre-HPLC to obtain target molecules (white solid, 16 mg, yield: 32.4%). $^1$H NMR (400 MHz, DMSO-$d_6$) δ 9.64 (s, 1H), 8.79 (s, 1H), 8.61 (s, 1H), 7.83 - 7.51 (m, 1H), 7.00 (s, 1H), 4.27 (s, 1H), 4.15 (s, 1H), 3.67 (dd, *J* = 50.5, 26.4 Hz, 8H), 2.95 (dt, *J* = 13.4, 6.6 Hz, 1H), 1.04 (d, *J* = 6.7 Hz, 6H), 0.85 - 0.80 (m, 2H), 0.80 - 0.74 (m, 2H). LC/MS (ESI) (m/z): 558 [M+H]$^+$.

**By referring to the preparation method in Example 14, the following examples were implemented by synthesizing a crude product according to a similar route by starting from appropriate starting materials, treating the crude product via reversed-phase HPLC, and then freeze-drying the crude product to obtain the title compound.**

| Example | Structure and name | Analysis data |
|---|---|---|
| **Example 15** | 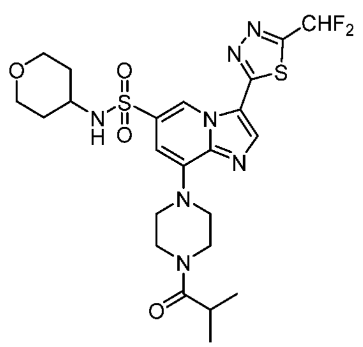 **3-(5-(difluoromethyl)-1,3,4-thiadiazol-2-yl)-8-(4-isobutyrylpiperazin-1-yl)-N-(tetrahydro-2H-pyran-4-yl)imidazo[1,2-a]pyridine-6-sulfonamide** | $^1$H NMR (400 MHz, DMSO-$d_6$) δ 9.65 (d, *J* = 1.3 Hz, 1H), 8.63 (s, 1H), 8.15 (d, *J* = 7.1 Hz, 1H), 7.64 (t, *J* = 53.0 Hz, 1H), 7.07 (d, *J* = 1.3 Hz, 1H), 3.78 - 3.66 (m, 8H), 3.58 (s, 2H), 3.28 - 3.20 (m, 3H), 3.00 - 2.93 (m, 1H), 1.61 (d, *J* = 10.5 Hz, 2H), 1.45 - 1.37 (m, 2H), 1.05 *(d, J* = 6.7 Hz, 6H). LC/MS (ESI) (m/z): 570 [M+H]$^+$. |

(continued)

| Example | Structure and name | Analysis data |
|---|---|---|
| **Example 16** | 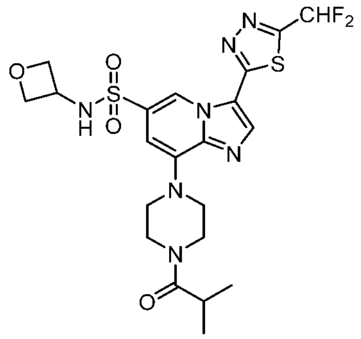<br>**3-(5-(difluoromethyl)-1,3,4-thiadiazol-2-yl)-8-(4-isobutyrylpiperazin-1-yl)-N-(oxetan-3-yl)imidazo[1,2-a]pyridine-6-sulfonamide** | $^1$H NMR (400 MHz, DMSO-$d_6$) δ 9.61 *(d, J* = 1.4 Hz, 1H), 8.92 (d, *J* = 7.7 Hz, 1H), 8.64 (s, 1H), 7.71 (t, *J* = 53.2 Hz, 1H), 6.96 (d, *J* = 1.3 Hz, 1H), 4.58 - 4.52 (m, 2H), 4.51 - 4.43 (m, 1H), 4.38 - 4.32 (m, 2H), 3.79 - 3.58 (m, 8H), 3.00 - 2.93 (m, 1H), 1.05 (d, *J* = 6.7 Hz, 6H). LC/MS (ESI) (m/z): 542 [M+H]$^+$. |
| **Example 17** | 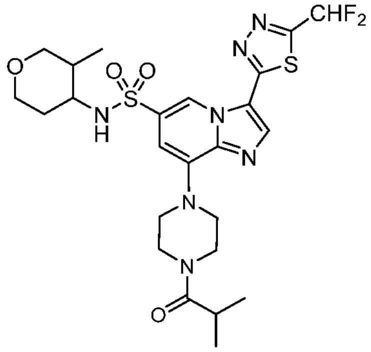<br>**3-(5-(difluoromethyl)-1,3,4-thiadiazol-2-yl)-8-(4-isobutyrylpiperazin-1-yl)-N-(3-methyltetrahydro-2H-pyran-4-yl)imidazo[1,2-a]pyridine-6-sulfonamide** | $^1$H NMR (400 MHz, DMSO-$d_6$) δ 9.65 (d, *J* = 1.4 Hz, 1H), 8.63 (s, 1H), 8.09 (d, *J* = 7.5 Hz, 1H), 7.70 (t, *J* = 53.2 Hz, 1H), 7.10 (d, *J* = 1.4 Hz, 1H), 3.80 - 3.53 (m, 12H), 3.45 - 3.42 (m, 1H), 2.98 - 2.93 (m, 1H), 1.81 - 1.73 (m, 1H), 1.51 - 1.39 (m, 2H), 1.05 *(d, J* = 6.7 Hz, 6H), 0.79 (d, *J* = 7.0 Hz, 3H). LC/MS (ESI) (m/z): 584 [M+H]$^+$. |

**Example 18: 3-(1-acryloyl-2,5-dihydro-1H-pyrrol-3-yl)-N-(1-cyanocyclopropyl)-8-(4-isobutyrylpiperazin-1-yl) imidazo[1,2-a]pyridine-6-sulfonamide**

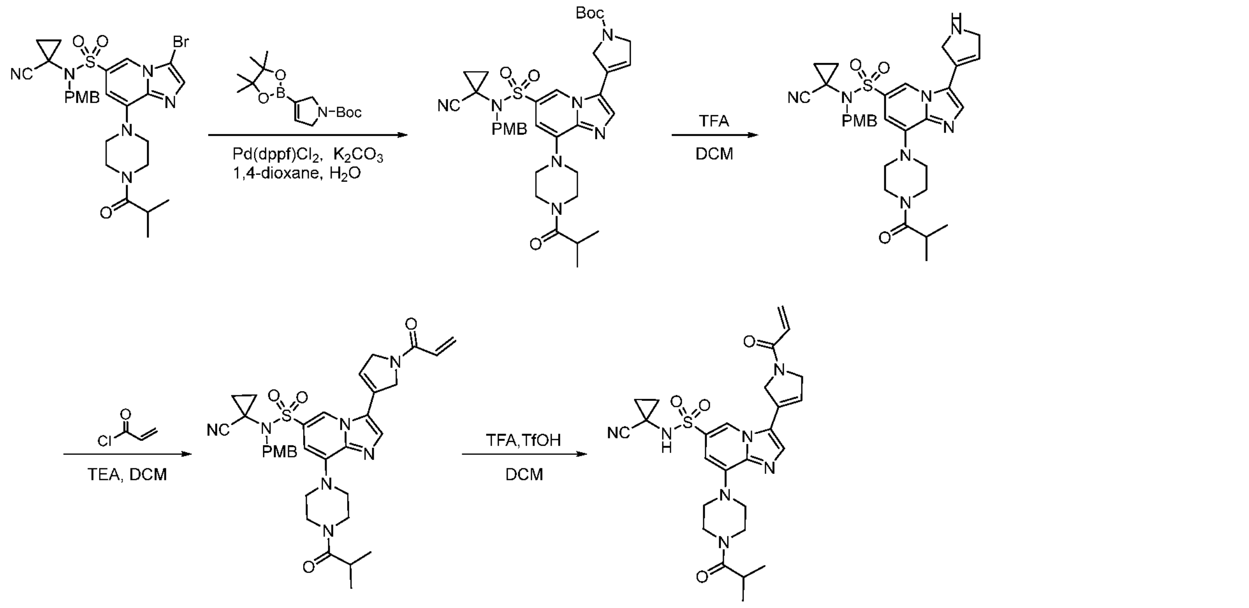


**Step 1: Tert-butyl 3-(6-(N-(1-cyanocyclopropyl)-N-(4-methoxybenzyl)sulfamoyl)-8-(4-isobutyrylpiperazin-1-yl) imidazo[1,2-a]pyridin-3-yl)-2,5-dihydro-1H-pyrrole-1-carboxylate**

At room temperature, potassium carbonate (67 mg, 0.48 mmol) and Pd(dppf)$Cl_2$ (11 mg, 0.02 mmol) were sequentially added to the mixed solution of 3-bromo-N-(1-cyanocyclopropyl)-8-(4-isobutyrylpiperazin-1-yl)-N-(4-methoxybenzyl)imidazo[1,2-a]pyridin-6-sulfonamide (100 mg, 0.16 mmol), 1-tert-butoxycarbonyl-2,5-dihydro-1H-pyrrolo-3-pinacolboronate (65 mg, 0.21 mmol) in 1,4-dioxane (3 mL) and water (1 mL). The flask was replaced with nitrogen gas three times and then the mixture was stirred at 80°C for 4 hours under an atmosphere of nitrogen. After completion of the reaction, the reaction mixture was cooled to room temperature, diluted with water, and extracted with ethyl acetate. The organic phase was washed with saturated brine, dried over anhydrous sodium sulfate, and concentrated under reduced pressure. The residue was purified by silica gel chromatography (eluent: petroleum ether/ethyl acetate, gradient: 0-55% ethyl acetate) to obtain the target product (white solid, 80 mg, yield: 69.9%). LC/MS (ESI) (m/z): 704 $[M+H]^+$.

**Step 2: N-(1-cyanocyclopropyl)-3-(2,5-dihydro-1H-pyrrol-3-yl)-8-(4-isobutyrylpiperazin-1-yl)-N-(4-methoxybenzyl)imidazo[1,2-a]pyridine-6-sulfonamide**

At 0°C, trifluoroacetic acid (1 mL) was dropwise added slowly to the solution of 3-(6-(N-(1-cyanocyclopropyl)-N-(4-methoaybenzyl)sulfamoyl)-8-(4-isobutyrylpiperazin-1-yl)imidazo[1,2-a]pyridin-3-yl)-2,5-dihydro-1H-pyrrolo-1-tert-butyl carboxylate (80 mg, 0.11 mmol) in dichloromethane (2 mL). The reaction mixture was stirred at room temperature for 1.5 hours. After completion of the reaction, the reaction mixture was directly concentrated under reduced pressure to obtain a crude title compound (yellow oil, 80 mg, yield: 100%), which was directly used in the next step of reaction. LC/MS (ESI) (m/z): 604 $[M+H]^+$.

**Step 3: 3-(1-acryloyl-2,5-dihydro-1H-pyrrol-3-yl)-N-(1-cyanocyclopropyl)-8-(4-isobutyrylpiperazin-1-yl)-N-(4-methoxybenzyl)imidazo[1,2-a]pyridine-6-sulfonamide**

At 0°C, TEA (0.1 mL, 0.3 mmol) and acryloyl chloride (0.01 mL, 0.15 mmol) were dropwise added to the solution of N-(1-cyanocyclopropyl)-3-(2,5-dihydro-1H-pyrrolo-3-yl)-8-(4-isobutyrylpiperazin-1-yl)-N-(4-methoxybenzyl)imidazo [1,2-a]pyridin-6-sulfonamide trifluoroacetate (80 mg, 0.11 mmol) in dichloromethane (5 mL). The reaction mixture was stirred at room temperature for 1.5 hours. After completion of the reaction, the reaction mixture was concentrated under reduced pressure. The residue was purified by silica gel chromatography (eluent: ethyl acetate/petroleum ether, gradient: 0-50% ethyl acetate) to obtain the target product (white solid, 40 mg, yield: 61.19%). LC/MS (ESI) (m/z): 658 $[M+H]^+$.

**Step 4: 3-(1-acryloyl-2,5-dihydro-1H-pyrrol-3-yl)-N-(1-cyanocyclopropyl)-8-(4-isobutyrylpiperazin-1-yl)imidazo [1,2-a]pyridine-6-sulfonamide**

At 0°C, trifluoroacetic acid (0.05 mL) and trifluoromethanesulfonic acid (0.05 mL) were dropwise added slowly to

the solution of 3-(1-acryloyl-2,5-dihydro-1H-pyrrolo-3-yl)-N-(1-cyanocyclopropyl)-8-(4-isobutyrylpiperazin-1-yl)-N-(4-methoxybenzyl)imidazo[1,2-a]pyridin-6-sulfonamide (40 mg, 0.06 mmol) in dichloromethane (2 mL). The reaction mixture was stirred at 0°C for 2 hours. After completion of the reaction, the reaction mixture was concentrated under reduced pressure, and the residue was purified by pre-HPLC to obtain the title compound (white solid, 12 mg, yield: 36.7%). $^1$H NMR (400 MHz, DMSO-$d_6$) δ 9.34 (s, 1H), 8.59 (dd, $J$ = 3.7, 1.3 Hz, 1H), 7.88 (d, $J$ = 26.9 Hz, 1H), 6.80 (s, 1H), 6.74 - 6.62 (m, 1H), 6.39 (s, 1H), 6.25 (dd, $J$ = 16.8, 2.2 Hz, 1H), 5.81 - 5.75 (m, 1H), 4.96 - 4.86 (m, 1H), 4.70 (d, $J$ = 9.4 Hz, 2H), 4.51 - 4.46 (m, 1H), 3.77 - 3.66 (m, 6H), 3.60 - 3.55 (m, 2H), 2.98 - 2.93 (m, 1H), 1.51 - 1.46 (m, 2H), 1.39 - 1.35 (m, 2H), 1.04 (d, $J$ = 6.7 Hz, 6H). LC/MS (ESI) (m/z): 538 [M+H]$^+$.

**Example 19: 3-(1-acryloylpyrrolidin-3-yl)-N-(1-cyanocyclopropyl)-8-(4-isobutyrylpiperazin-1-yl)imidazo[1,2-a]pyridine-6-sulfonamide**

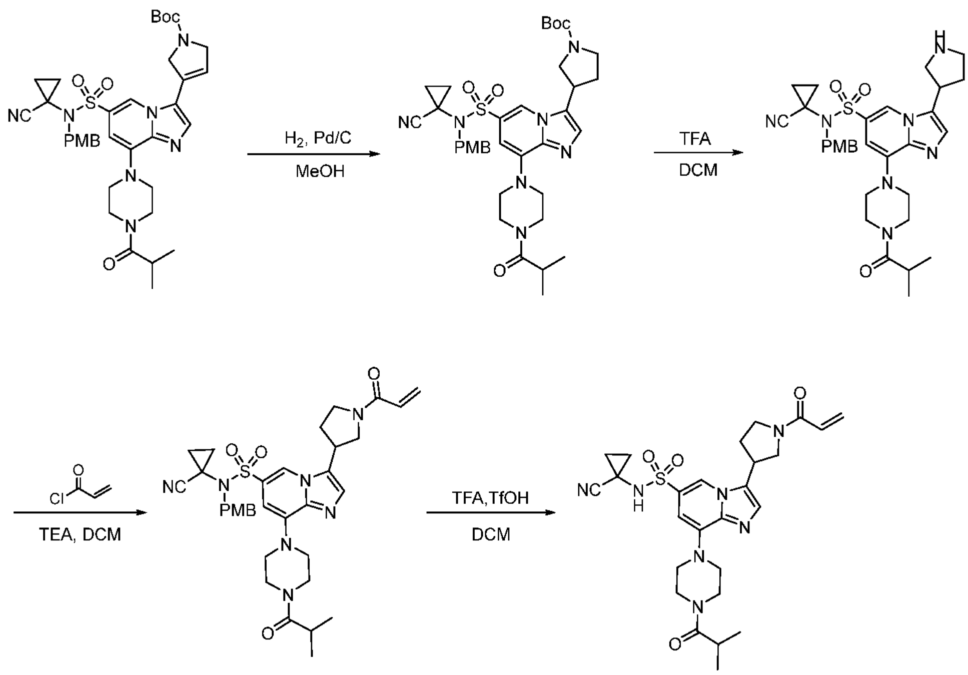


**Step 1: 3-{6-[(1-cyanocyclopropyl)[(4 methoxyphenyl)methyl]sulfamoyl]-8-[4-(2-methylpropionyl)piperazin-1-yl]imidazo[1,2-a]pyridin-3-yl}pyrrolidin-1-tert-butyl carboxylate**

Under an atmosphere of nitrogen, Pd/C (12 mg, mass fraction: 10%) was slowly added to the solution of 3-(6-(N-(1-cyanocyclopropyl)-N-(4-methoxybenzyl)sulfamoyl)-8-(4-isobutyrylpiperazin-1-yl)imidazo[1,2- a]pyridin-3-yl)-2,5-dihydro-1H-pyrrolo-1-tert-butyl carboxylate (80 mg, 0.11 mmol) in methanol (5 mL). The flask was replaced with nitrogen gas three times and then the mixture was stirred for 9 hours at room temperature under an atmosphere provided by a hydrogen-filled balloon. After completion of the reaction, the mixture was filtered, and the filtrate was concentrated under reduced pressure to obtain the title compound (white solid, 78 mg, yield: 95.8%). LC/MS (ESI) m/z: 706 [M+H]$^+$.

**Step 2-4: 3-(1-acryloylpyrrolidin-3-yl)-N-(1-cyanocyclopropyl)-8-(4-isobutyrylpiperazin-1-yl)imidazo[1,2-a]pyridine-6-sulfonamide**

The title compound was prepared by referring to the synthesis methods in Steps 2-4 of **Example 18** (white solid, yield: 36.7%). $^1$H NMR (400 MHz, DMSO-$d_6$) δ 9.24 (d, $J$ = 7.0 Hz, 1H), 8.54 - 8.46 (m, 1H), 7.57 (d, $J$ = 7.9 Hz, 1H), 6.69 - 6.67 (m, 1H), 6.67 - 6.58 (m, 1H), 6.21 - 6.13 (m, 1H), 5.73 - 5.65 (m, 1H), 4.19 - 3.88 (m, 2H), 3.83 - 3.63 (m, 8H), 3.59 - 3.53 (m, 2H), 3.50 - 3.42 (m, 1H), 2.98 - 2.91 (m, 1H), 2.46 - 2.31 (m, 1H), 2.15 - 2.01 (m, 1H), 1.49 - 1.43 (m, 2H), 1.41 - 1.34 (m, 2H), 1.04 (d, $J$ = 6.7 Hz, 6H). LC/MS (ESI) (m/z): 540 [M+H]$^+$.

**Example 20: N-(1-cyanocyclopropyl)-8-(4-isobutyrylpiperazin-1-yl)-3-(1-propionyl-2,5-dihydro-1H-pyrrol-3-yl) imidazo[1,2-a]pyridine-6-sulfonamide**

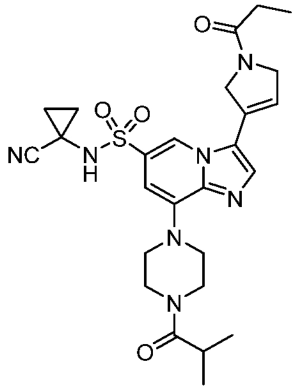

The title compound was prepared by referring to the synthesis methods in Step 3-4 of **Example 18,** using propionyl chloride instead of acryloyl chloride (white solid, yield: 20.4%). $^1$H NMR (400 MHz, DMSO-$d_6$) δ 8.56 (d, $J$ = 5.1 Hz, 1H), 7.84 (d, $J$ = 22.2 Hz, 1H), 6.79 (s, 1H), 6.36 (s, 1H), 4.76 (s, 1H), 4.58 (s, 2H), 4.40 (s, 1H), 3.79 - 3.68 (m, 4H), 3.66 - 3.54 (m, 4H), 2.96 - 2.89 (m, 1H), 2.40 - 2.33 (m, 2H), 1.43 (s, 2H), 1.35 - 1.29 (m, 2H), 1.08 - 1.02 (m, 9H). LC/MS (ESI) (m/z): 540 [M+H]$^+$.

**Example 21: 3-(1-acryloyl-1,2,3,6-tetrahydropyridin-4-yl)-N-(1-cyanocyclopropyl)-8-(4-isobutyrylpiperazin-1-yl) imidazo[1,2-a]pyridine-6-sulfonamide**

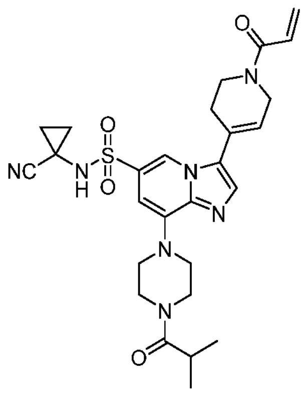

The title compound was prepared by referring to the synthesis method in **Example 18,** using N-tert-butoxycarbonyl-3,6-dihydro-2H-pyridin-4-pinacolboronate instead of 1-tert-butoxycarbonyl-2,5-dihydro-1H-pyrrolo-3-pinacolboronate (white solid, yield: 28.5%). $^1$H NMR (400 MHz, DMSO-$d_6$) δ 9.28 (s, 1H), 8.48 (s, 1H), 7.73 (s, 1H), 6.98 - 6.81 (m, 1H), 6.70 (s, 1H), 6.25 - 6.14 (m, 2H), 5.80 - 5.70 (m, 1H), 4.44 - 4.25 (m, 2H), 3.90 - 3.79 (m, 2H), 3.76 - 3.66 (m, 4H), 3.66 - 3.52 (m, 4H), 2.98 - 2.91 (m, 1H), 2.64 - 2.54 (m, 2H), 1.51 - 1.44 (m, 2H), 1.39 - 1.33 (m, 2H), 1.04 (d, $J$ = 6.7 Hz, 6H). LC/MS (ESI) (m/z): 552 [M+H]$^+$.

**Example 22: N-(1-cyanocyclopropyl)-3-(5-(difluoromethyl)-1,3,4-thiadiazol-2-yl)-1-(2-azaspiro[3.4]oct-6-en-6-yl)imidazo[1,5-a]pyridine-6-sulfonamide**

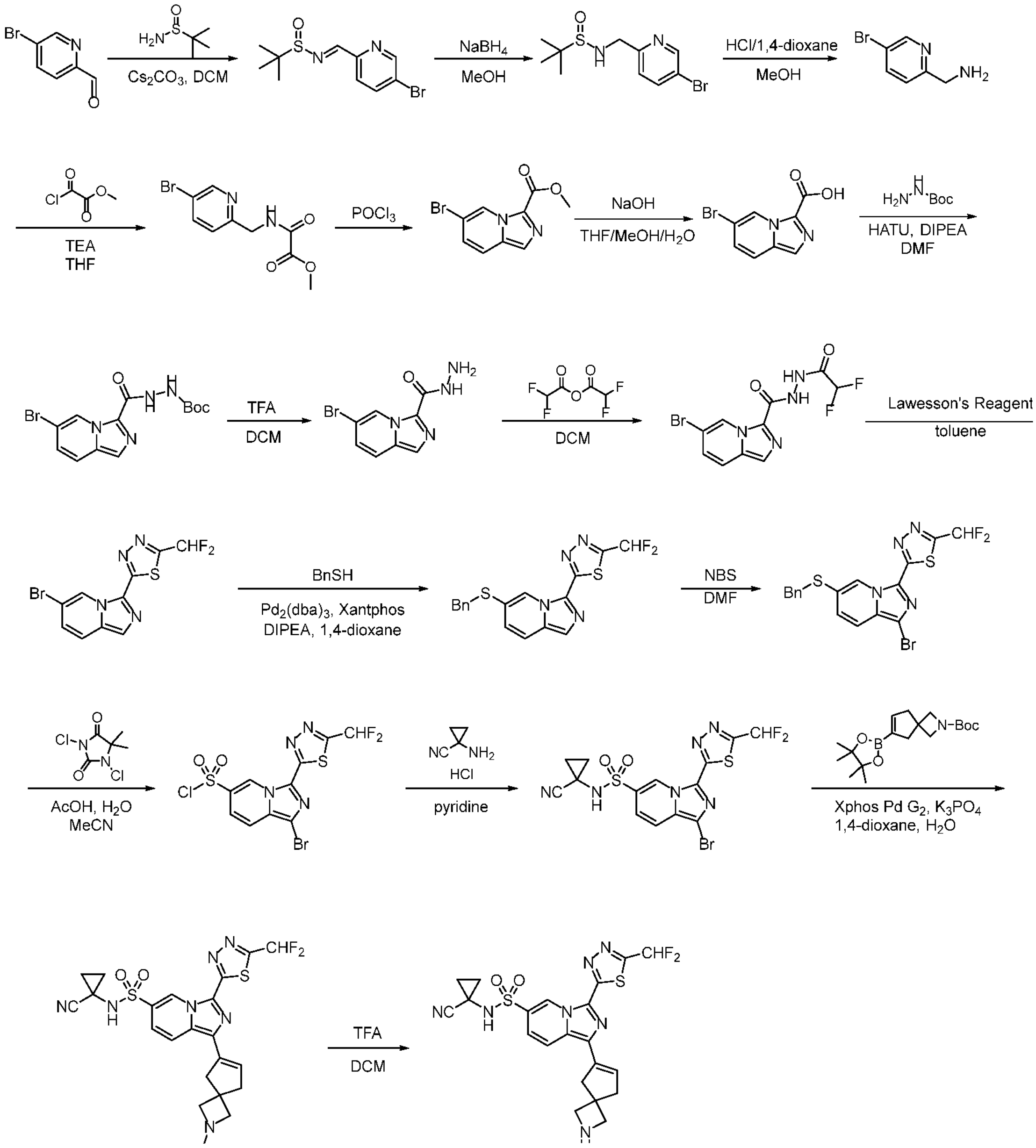


**Step 1: N-((5-bromopyridin-2-yl)methylene)-2-methylpropane-2-sulfinamide**

At room temperature, cesium carbonate (21 g, 64.5 mmol) and tert-butylsulfenamide (6.5 g, 53.8 mmol) were sequentially added to the solution of 5-bromo-2-pyridylaldehyde (10 g, 53.7 mmol) in dichloromethane (110 mL). The reaction mixture was stirred at 40°C for one hour. After completion of the reaction, the reaction mixture was cooled to room temperature, and quenched by adding ice water. The mixture was extracted with dichloromethane twice, and the organic phase were combined, washed with saturated brine, dried over anhydrous sodium sulfate, and concentrated under reduced pressure. The residue was separated by column chromatography (eluent: petroleum ether/ethyl acetate, gradient: 0-15% ethyl acetate) to obtain the title compound (white solid, 12 g, yield: 77.2%). LC/MS (ESI) (m/z): 289 $[M+H]^+$.

**Step 2: N-((5-bromopyridin-2-yl)methyl)-2-methylpropane-2-sulfinamide**

In an ice bath, sodium borohydride (2.1 g, 62.2 mmol) was added portionwise to the solution of N-((5-bromopyridin-2-yl)methylene)-2-methylpropan-2-sulfenamide (12 g, 41.5 mmol) in methanol (120 mL); and then, the reaction mixture was stirred at 30°C for 30 minutes. After completion of the reaction, the reaction mixture was quenched with saturated aqueous ammonium chloride solution in an ice bath, and extracted with ethyl acetate. The organic phase

was washed with saturated brine, dried over anhydrous sodium sulfate, and concentrated under reduced pressure. The residue was purified by column chromatography (eluent: ethyl acetate/petroleum ether, gradient: 0-20% ethyl acetate) to obtain the title compound (yellow liquid, 10.5 g, yield: 86.9%). $^1$H NMR (400 MHz, $CDCl_3$) $\delta$ 8.61 (d, *J* = 2.2 Hz, 1H), 7.79 (dd, *J* = 8.3, 2.3 Hz, 1H), 7.28 - 7.26 (s, 1H), 4.42 - 4.36 (m, 2H), 4.18 (t, *J* = 5.6 Hz, 1H), 1.26 (s, 9H). LC/MS (ESI) (m/z): 291 $[M+H]^+$.

**Step 3: (5-bromopyridin-2-yl)methylamine**

In an ice water bath, the solution of hydrochloric acid/1,4-dioxane (50 mL, 4 M) was slowly added to the solution of N-((5-bromopyridin-2-yl)methyl)-2-methylpropan-2-sulfenamide (12 g, 64.5 mmol) in methanol (120 mL). The reaction mixture was stirred at room temperature for 30 minutes. After completion of the reaction, the saturated aqueous sodium bicarbonate solution was added for neutralization, and the resulting mixture was directly concentrated under reduced pressure. The residue was purified by column chromatography (eluent: dichloromethane/methanol, gradient: 10-20% methanol) to obtain the title compound (white solid, 4.5 g, yield: 70.0%). $^1$H NMR (400 MHz, $CDCl_3$) $\delta$ 8.61 (d, *J* = 2.1 Hz, 1H), 7.77 (dd, *J* = 8.3, 2.3 Hz, 1H), 7.21 (d, *J* = 8.3 Hz, 1H), 3.94 (s, 2H). LC/MS (ESI) (m/z): 187 $[M+H]^+$.

**Step 4: Methyl 2-(((5-bromopyridin-2-yl)methyl)amino)-2-oxoacetate**

At 0°C, triethylamine (5 mL, 36 mmol) was added to the solution of (5-bromopyridin-2-yl)methylamine (4.5 g, 24 mmol) in anhydrous tetrahydrofuran (45 mL); and then, methyl oxalyl chloride (2.4 mL, 26.5 mmol) was dropwise added slowly. The reaction mixture was stirred at room temperature for 1 hours. After completion of the reaction, ice water was added to the reaction mixture, which was then extracted with ethyl acetate. The organic phase was washed with saturated brine, dried over anhydrous sodium sulfate, and concentrated under reduced pressure to obtain the title compound (yellow solid, 4.5 g, yield: 68.4%). LC/MS (ESI) (m/z): 273 $[M+H]^+$.

**Step 5: Methyl 6-bromoimidazo[1,5-a]pyridine-3-carboxylate**

At room temperature, 2-(((5-bromopyridin-2-yl)methyl)amino)-2-methyl oxyacetate (4.5 g, 16.5 mmol) was added portionwise to phosphorus oxychloride (45 mL); and the reaction mixture was stirred at 110°C for 16 hours under an atmosphere of nitrogen. After completion of the reaction, the reaction mixture was concentrated under reduced pressure, and the residue was added to the saturated aqueous sodium bicarbonate solution for neutralization, and then extracted with ethyl acetate. The organic phase was washed with saturated brine, dried over anhydrous sodium sulfate, and concentrated under reduced pressure. The residue was purified by column chromatography (eluent: ethyl acetate/petroleum ether, gradient: 30-50% ethyl acetate) to obtain the title compound (yellow solid, 3.0 g, yield: 71.3%). $^1$H NMR (400 MHz, $CDCl_3$) $\delta$ 9.53 (s, 1H), 7.64 (s, 1H), 7.54 (d, *J* = 9.4 Hz, 1H), 7.15 (dd, *J* = 9.4, 1.3 Hz, 1H), 4.03 (s, 3H). LC/MS (ESI) (m/z): 255 $[M+H]^+$.

**Step 6: 6-bromoimidazo[1,5-a]pyridine-3-carboxylic acid**

At room temperature, 6-bromoimidazo[1,5-a]pyridin-3-methyl carboxylate (3 g, 11.8 mmol) was dissolved in the mixed solution of methanol (15 mL), tetrahydrofuran (15 mL) and water (15 mL); and then, sodium hydroxide (520 mg, 12.9 mmol) was added. The reaction mixture was stirred at room temperature for 2 hours. After completion of the reaction, the reaction mixture was adjusted to the pH value of 6 by using 3N hydrochloric acid, and then concentrated under reduced pressure. The residue was purified by column chromatography (eluent: dichloromethane/methanol, gradient: 0-20% methanol) to obtain the title compound (white solid, 2.8 g, yield: 98.7%). LC/MS (ESI) (m/z): 241 $[M+H]^+$.

**Step 7: Tert-butyl 2-(6-bromoimidazo[1,5-a]pyridine-3-carbonyl)hydrazine-1-carboxylate**

At room temperature, N,N-diisopropylethylamine (5.7 mL, 34.8 mmol) and 2-(7-azobenzotriazol)-N,N,N',N'-tetramethylurea hexafluorophosphate (6.6 g, 17.4 mmol) were sequentially added to the solution of 6-bromoimidazo[1,5-a]pyridin-3-carboxylic acid (2.8 g, 11.6 mmol) and tert-butyl hydrazinoformate (4.8 g, 12.8 mmol) in N,N-dimethylformamide (30 mL). The reaction mixture was stirred at room temperature for 3 hours. After completion of the reaction, the reaction mixture was diluted with ethyl acetate, washed with water and saturated brine in sequence, dried over anhydrous sodium sulfate, and concentrated under reduced pressure. The residue was separated by column chromatography (eluent: petroleum ether/ethyl acetate, gradient: 0-20% ethyl acetate) to obtain the title compound (white solid, 2 g, yield: 48.4%). LC/MS (ESI) (m/z): 355 $[M+H]^+$.

**Step 8: 6-bromoimidazo[1,5-a]pyridine-3-carbohydrazide**

At 0°C, trifluoroacetic acid (5 mL) was slowly added to the solution of 2-(6-bromoimidazo[1,5-a]pyridin-3-carbonyl)hydrazin-1-tert-butyl carboxylate (2 g, 5.63 mmol) in dichloromethane (20 mL). The reaction mixture was stirred at 25°C for one hour. After completion of the reaction, the reaction mixture wa sconcentrated to obtain a crude title compound (yellow liquid, 1.9 g, yield: 100%), which was directly used in the next step of reaction. LC/MS (ESI) (m/z): 255 $[M+H]^+$.

**Step 9: 6-bromo-N'-(2,2-difluoroacetyl)imidazo[1,5-a]pyridine-3-carbohydrazide**

At 0°C, 2,2-difluoroacety12,2-difluoroacetate (0.7 mL, 5.64 mmol) was dropwise added slowly to the solution of 6-bromoimidazo[1,5-a]pyridin-3-carbohydrazide trifluoroacetate (1.9 g, 5.63 mmol) in dichloromethane (20 mL). The reaction mixture was stirred at room temperature for 30 minutes. After completion of the reaction, the reaction mixture was washed with saturated aqueous sodium bicarbonate solution and saturated brine, dried over anhydrous sodium sulfate, and concentrated under reduced pressure. The residue was purified by column chromatography (eluent: ethyl acetate/petroleum ether, gradient: 0-50% ethyl acetate) to obtain the title compound (yellow solid, 1.0 g, yield: 65.1%). LC/MS (ESI) (m/z): 333 $[M+H]^+$.

**Step 10: 2-(6-bromoimidazo[1,5-a]pyridin-3-yl)-5-(difluoromethyl)-1,3,4-thiadiazole**

At room temperature, the Lawesson's reagent (1.4 g, 3.37 mmol) was added to the solution of 6-bromo-N'-(2,2-difluoroacetyl)imidazo[1,5-a]pyridin-3-carbohydrazide (1 g, 3.06 mmol) in toluene (10 mL). The reaction mixture was stirred at 110°C for 16 hours under an atmosphere of nitrogen. After completion of the reaction, the reaction mixture was cooled to room temperature, diluted with ethyl acetate, washed with saturated aqueous sodium bicarbonate solution and saturated brine, dried over anhydrous sodium sulfate, and concentrated under reduced pressure. The residue was purified by column chromatography (eluent: ethyl acetate/petroleum ether, gradient: 0-55% ethyl acetate) to obtain the title compound (yellow liquid, 676 mg, yield: 66.6%). LC/MS (ESI) (m/z): 331 $[M+H]^+$.

**Step 11: 2-(6-(benzylthio)imidazo[1,5-a]pyridin-3-yl)-5-(difluoromethyl)-1,3,4-thiadiazole**

At room temperature, N,N-diisopropylethylamine (1 mL, 6.12 mmol), XantPhos (236 mg, 0.41 mmol), $Pd_2(dba)_3$ (187 mg, 0.20 mmol), benzyl mercaptane (0.4 mL, 3.06 mmol) were sequentially added to the solution of 2-(6-bromoimidazo[1,5-a]pyridin-3-yl)-5-(difluoromethyl)-1,3,4-thiadiazole (676 mg, 2.04 mmol) in anhydrous dioxane (7 mL). The flask was replaced with nitrogen gas three times and then the mixture was stirred at 100 °C for 2 hours under an atmosphere of nitrogen. After completion of the reaction, the reaction mixture was cooled to room temperature, diluted with ethyl acetate, washed with saturated brine, dried over anhydrous sodium sulfate, and concentrated under reduced pressure. The residue was purified by column chromatography (eluent: ethyl acetate/petroleum ether, gradient: 0-30% ethyl acetate) to obtain the title compound (yellow liquid, 700 mg, yield: 91.5%). LC/MS (ESI) (m/z): 375 $[M+H]^+$.

**Step 12: 2-(6-(benzylthio)-1-bromoimidazo[1,5-a]pyridin-3-yl)-5-(difluoromethyl)-1,3,4-thiadiazole**

At 0°C, N-bromosuccinimide (366 mg, 2.06 mmol) was slowly added to the solution of 2-(6-(benzylthio)imidazo [1,5-a]pyridin-3-yl)-5-(difluoromethyl)-1,3,4-thiadiazole (700 mg, 1.87 mmol) in N,N-dimethylformamide (7 mL). The reaction mixture was stirred at 0°C for 30 minutes. After completion of the reaction, ice water was added for quenching, and the reaction mixture was extracted with ethyl acetate, washed with saturated brine, dried over anhydrous sodium sulfate, and concentrated under reduced pressure. The residue was purified by column chromatography (eluent: ethyl acetate/petroleum ether, gradient: 0-55% ethyl acetate) to obtain the title compound (yellow solid, 800 mg, yield: 94.4%). LC/MS (ESI) (m/z): 453 $[M+H]^+$.

**Step 13: 1-bromo-3-(5-(difluoromethyl)-1,3,4-thiadiazol-2-yl)imidazo[1,5-a]pyridine-6-sulfonyl chloride**

At 0°C, acetic acid (0.12 mL, 1.98 mmol) was added to the solution of 2-(6-(benzylthio)-1-bromoimidazo[1,5-a] pyridin-3-yl)-5-(difluoromethyl)-1,3,4-thiadiazole (450 mg, 0.99 mmol) in acetonitrile (10 mL); and then, dichlorohydantoin (489 mg, 2.48 mmol) was added portionwise. The reaction mixture was stirred at 0°C for 30 minutes. After completion of the reaction, the reaction mixture was diluted with dichloromethane, washed with saturated brine, dried over anhydrous sodium sulfate, and concentrated under reduced pressure to obtain the title compound (yellow solid, 350 mg, yield: 82.1%), which was directly used in the next step of reaction. LC/MS (ESI) (m/z): 429 $[M+H]^+$.

**Step 14: 1-bromo-N-(1-cyanocyclopropyl)-3-(5-(difluoromethyl)-1,3,4-thiadiazol-2-yl)imidazo[1,5-a]pyridine-6-sulfonamide**

At 0°C, 1-bromo-3-(5-(difluoromethyl)-1,3,4-thiadiazol-2-yl)imidazo[1,5-a]pyridin-6-sulfonyl chloride (350 mg, 0.81 mmol) was added portionwise to the solution of 1-amido-1-cyclopropyl cyanic hydrochloride (193 mg, 1.63 mmol) in pyridine (7 mL). The reaction mixture was stirred at room temperature for 30 minutes. After completion of the reaction, ice water was added for quenching, the reaction mixture was extracted with ethyl acetate, and the organic phase was washed with saturated brine, dried over anhydrous sodium sulfate, and concentrated under reduced pressure. The residue was purified by column chromatography (eluent: ethyl acetate/petroleum ether, gradient: 0-60% ethyl acetate) to obtain the title compound (yellow solid, 230 mg, yield: 59.4%). LC/MS (ESI) (m/z): 475 $[M+H]^+$.

**Step 15: Tert-butyl 6-(6-(N-(1-cyanocyclopropyl)sulfamoyl)-3-(5-(difluoromethyl)-1,3,4-thiadiazol-2-yl)imidazo [1,5-a]pyridin-1-yl)-2-azaspiro[3.4]oct-6-ene-2-carboxylate**

At room temperature, potassium phosphate (205 mg, 0.96 mmol) and Xphos Pd G2 (76 mg, 0.10 mmol) were added to the mixed solution of 1-bromo-N-(1-cyanocyclopropyl)-3-(5-(difluoromethyl)-1,3,4-thiadiazol-2-yl)imidazo[1,5-a]pyridin-6-sulfonamide (230 mg, 0.48 mmol) and 6-(4,4,5,5-tetramethyl-1,3,2-dioxoboracyclopentan-2-yl)-2-azaspirocyclo[3.4]octan-6-ene-2-tert-butyl carboxylate (162 mg, 0.48 mmol) in 1,4-dioxane (18 mL) and water (4.5 mL). The flask was replaced with nitrogen gas three times and then the mixture was stirred at 80°C for 2 hours under an atmosphere of nitrogen. After completion of the reaction, the reaction mixture was cooled to room temperature, added with water, and extracted with ethyl acetate. The organic phase was washed with saturated brine, dried over anhydrous sodium sulfate, and concentrated under reduced pressure. The residue was purified by column chromatography (eluent: ethyl acetate/petroleum ether, gradient: 0-55% ethyl acetate) to obtain the title compound (yellow solid, 210 mg, yield: 71.9%). LC/MS (ESI) (m/z): 604 $[M+H]^+$.

**Step 16: N-(1-cyanocyclopropyl)-3-(5-(difluoromethyl)-1,3,4-thiadiazol-2-yl)-1-(2-azaspiro[3.4]oct-6-en-6-yl)imidazo[1,5-a]pyridine-6-sulfonamide**

At 0°C, trifluoroacetic acid (0.2 mL) was slowly added to the solution of 6-(6-(N-(1-cyanocyclopropyl)sulfamyl)-3-(5-(difluoromethyl)-1,3,4-thiadiazol-2-yl)imidazo[1,5-a]pyridin-1-yl)-2-azaspiro[3.4]octan-6-ene-2-tert-butyl carboxylate (30 mg, 0.05 mmol) in dichloromethane (1 mL). The reaction mixture was stirred at 25°C for one hour under an atmosphere of nitrogen. After completion of the reaction, the reaction mixture was concentrated under reduced pressure, and the residue was treated by pre-HPLC ($C_{18}$, 10-50% acetonitrile in water (containing 0.1% formic acid)) to obtain the title compound (white solid, 8 mg, yield: 32%). $^1H$ NMR (400 MHz, DMSO-$d_6$) $\delta$ 9.90 (s, 1H), 8.18 (d, $J$ = 8.3 Hz, 1H), 8.17-8.15 (m, 1H), 7.63 (t, $J$ = 53.1 Hz, 1H), 7.43 (d, $J$ = 8.7 Hz, 1H), 6.67 (s, 1H), 3.90-4.15 (m, 5H), 2.97 - 2.90 (m, 2H), 2.39 - 2.33 (m, 2H), 1.32 - 1.25 (m, 2H), 1.21 - 1.14 (m, 2H). LC/MS (ESI) (m/z): 504 $[M+H]^+$.

**Example 23: 1-(2-acetyl-2-azaspiro[3.4]oct-6-en-6-yl)-N-(1-cyanocyclopropyl)-3-(5-(difluoromethyl)-1,3,4-thiadiazol-2-yl)imidazo[1,5-a]pyridine-6-sulfonamide**

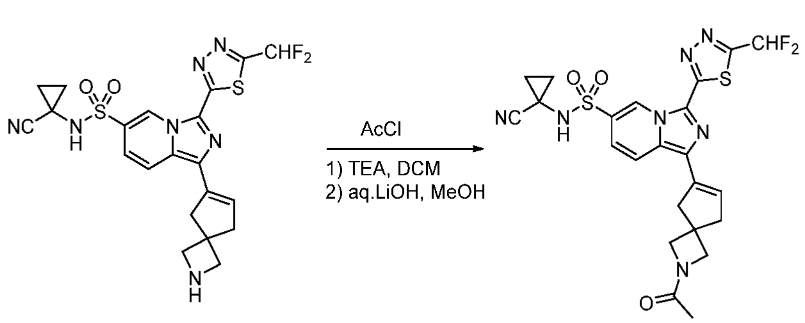


At 0°C, triethylamine (0.04 mL, 0.26 mmol) and acetyl chloride (0.006 mL, 0.09 mmol) were added to the solution of N-(1-cyanocyclopropyl)-3-(5-(difluoromethyl)-1,3,4-thiadiazol-2-yl)-1-(2-azaspiro[3.4]octan-6-ene-6-yl)imidazo[1,5-a]pyridin-6-sulfonamide (43 mg, 0.09 mmol) in anhydrous dichloromethane (1 mL) respectively. The reaction mixture was stirred at room temperature for 10 minutes. After completion of the reaction, ice water was added, and the reaction mixture was extracted with dichloromethane. The organic phase was washed with saturated brine, dried over anhydrous

sodium sulfate, and concentrated under reduced pressure. The concentrated product was dissolved in methanol (1 mL); and then, aqueous lithium hydroxide solution (0.3 mL, 0.3 mmol, 1M) was added. The reaction mixture was stirred at room temperature for 10 minutes, concentrated under reduced pressure, and then purified by pre-HPLC ($C_{18}$, 10-50% acetonitrile in water (containing 0.1% formic acid)) to obtain the title compound (white solid, 16.9 mg, yield: 36.2%). $^1$H NMR (400 MHz, DMSO-$d_6$) δ 9.99 (s, 1H), 9.65 (s, 1H), 8.37 (d, *J* = 9.6 Hz, 1H), 7.76 (t, *J* = 53.2 Hz, 1H), 7.41 (dd, *J* = 9.6, 1.3 Hz, 1H), 6.68 (s, 1H), 4.25 (d, *J* = 8.3 Hz, 1H), 4.15 (d, *J* = 8.3 Hz, 1H), 3.98 (d, *J* = 9.5 Hz, 1H), 3.87 (d, *J* = 9.5 Hz, 1H), 2.97 (t, *J* = 6.8 Hz, 2H), 2.34-2.29 (m, 2H), 1.79 (s, 3H), 1.52-1.47 (m, 2H), 1.38-1.34 (m, 2H). LC/MS (ESI) (m/z): 546 $[M+H]^+$.

**Example 24: N-(1-cyanocyclopropyl)-3-(5-(difluoromethyl)-1,3,4-thiadiazol-2-yl)-1-(2-isobutyryl-2-azaspiro[3.4]oct-6-en-6-yl)imidazo[1,5-a]pyridine-6-sulfonamide**

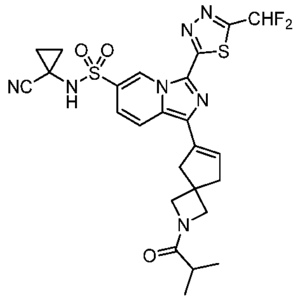

The title compound was prepared by referring to the synthesis method in **Example 23,** using isobutyryl chloride instead of acetyl chloride. $^1$H NMR (400 MHz, DMSO-$d_6$) δ 10.00 (s, 1H), 9.67 (s, 1H), 8.38 (d, *J* = 9.6 Hz, 1H), 7.69 (t, *J* = 54.4, 52.0 Hz, 1H), 7.41 (d, *J* = 9.6 Hz, 1H), 6.70 (s, 1H), 4.29 (d, *J* = 8.1 Hz, 1H), 4.20 *(d, J* = 8.2 Hz, 1H), 3.99 (d, *J* = 9.3 Hz, 1H), 3.88 (d, *J* = 9.4 Hz, 1H), 2.99 - 2.94 (m, 2H), 2.49 - 2.44 (m, 1H), 2.37 - 2.30 (m, 2H), 1.52 - 1.48 (m, 2H), 1.39 - 1.35 (m, 2H), 1.03 - 0.99 (m, 6H). LC/MS (ESI) (m/z): 574 $[M+H]^+$.

**Example 25: 6-(6-(N-(1-cyanocyclopropyl)sulfamoyl)-3-(5-(difluoromethyl)-1,3,4-thiadiazol-2-yl)imidazo[1,5-a]pyridin-1-yl)-N,N-dimethyl-2-azaspiro[3.4]oct-6-ene-2-carboxamide**

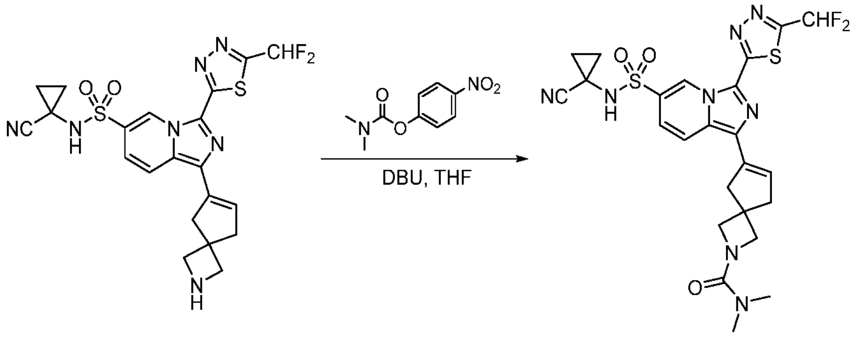


At 0°C, 4-nitrophenyldimethyl carbamate (33 mg, 0.16 mmol) and 1,8-diazabicyclo[5.4.0]undec-7-ene (36 mg, 0.24 mmol) were slowly added to the solution of N-(1-cyanocyclopropyl)-3-(5-(difluoromethyl)-1,3,4-thiadiazol-2-yl)-1-(2-azaspiro[3.4]octan-6-ene-6-yl)imidazo[1,5-a]pyridin-6-sulfonamide (40 mg, 0.079 mmol) in tetrahydrofuran (2 mL). The reaction mixture was stirred at 80°C for one hour. After completion of the reaction, the reaction mixture was diluted with ethyl acetate, washed with saturated aqueous potassium carbonate solution and saturated brine respectively, dried over anhydrous sodium sulfate, and concentrated under reduced pressure. The residue was purified by pre-HPLC to obtain the title compound (white solid, 5.5 mg, yield: 12.1%). $^1$H NMR (400 MHz, DMSO-$d_6$) δ 9.98 (s, 1H), 8.37 *(d, J* = 9.1 Hz, 1H), 7.69 (t, *J* = 53.1 Hz, 1H), 7.40 (dd, *J* = 9.6, 1.5 Hz, 1H), 6.65 (s, 1H), 4.03 (d, *J* = 8.1 Hz, 2H), 3.90 (d, *J* = 8.1 Hz, 2H), 2.95 (t, *J* = 6.3 Hz, 2H), 2.78 (s, 6H), 2.30 (t, *J* = 7.1 Hz, 2H), 1.48 (M, 2H), 1.36 - 1.29 (m, 2H). LC/MS (ESI) (m/z): 575 $[M+H]^+$.

**Example 26: N-(1-cyanocyclopropyl)-3-(5-(difluoromethyl)-1,3,4-thiadiazol-2-yl)-1-(2-azaspiro[3.4]octan-6-yl) imidazo[1,5-a]pyridine-6-sulfonamide**

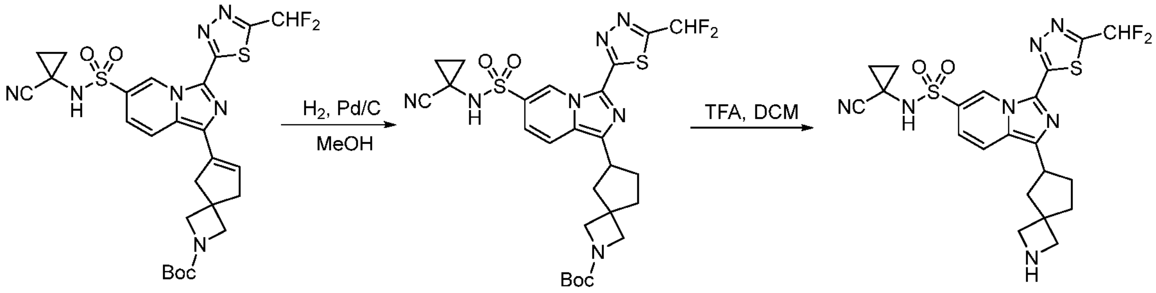


**Step 1: Tert-butyl 6-(6-(N-(1-cyanocyclopropyl)sulfamoyl)-3-(5-(difluoromethyl)-1,3,4-thiadiazol-2-yl)imidazo [1,5-a]pyridin-1-yl)-2-azaspiro[3.4]octane-2-carboxylate**

At room temperature, Pd/C (5 mg, mass fraction: 10%) was added to the solution of 6-(6-(N-(1-cyanocyclopropyl) sulfamyl)-3-(5-(difluoromethyl)-1,3,4-thiadiazol-2-yl)imidazo[1,5-a]pyridin-1-yl)-2-azaspiro[3.4]octan-6-ene-2-tert-butyl carboxylate (30 mg, 0.05 mmol) in methanol (1 mL). The flask was replaced with nitrogen gas three times and then the mixture was stirred for 5 hours at room temperature under an atmosphere provided by a hydrogen-filled balloon. After completion of the reaction, the mixture was filtered, and the filtrate was concentrated under reduced pressure to obtain the title compound (yellow solid, 25 mg, yield: 83.2%). LC/MS (ESI) m/z: 606 $[M+H]^+$.

**Step 2: N-(1-cyanocyclopropyl)-3-(5-(difluoromethyl)-1,3,4-thiadiazol-2-yl)-1-(2-azaspiro[3.4]octan-6-yl)imidazo[1,5-a]pyridine-6-sulfonamide**

The title compound was prepared by referring to the preparation method in Step 16 of **Example 22** (white solid, 4 mg, yield: 19.2%). $^1$H NMR (400 MHz, DMSO-$d_6$) δ 9.74 (s, 1H), 8.32 (s, 1H), 7.99 (d, *J* = 9.6 Hz, 1H), 7.79 - 7.53 (m, 1H), 7.28 (d, J = 8.7 Hz, 1H), 3.95 - 3.83 (m, 4H), 3.67 - 3.64 (m, 1H), 2.38 - 2.30 (m, 2H), 2.18 - 2.12 (m, 2H), 2.03 - 1.98 (m, 1H), 1.86 - 1.82 (m, 1H), 1.18 - 1.12 (m, 2H), 1.07 - 1.02 (m, 2H). LC/MS (ESI) (m/z): 506 $[M+H]^+$.

**Example 27: N-(1-cyanocyclopropyl)-3-(5-(difluoromethyl)-1,3,4-thiadiazol-2-yl)-7-(4-isobutyrylpiperazin-1-yl) pyrazolo[1,5-a]pyridine-5-sulfonamide**

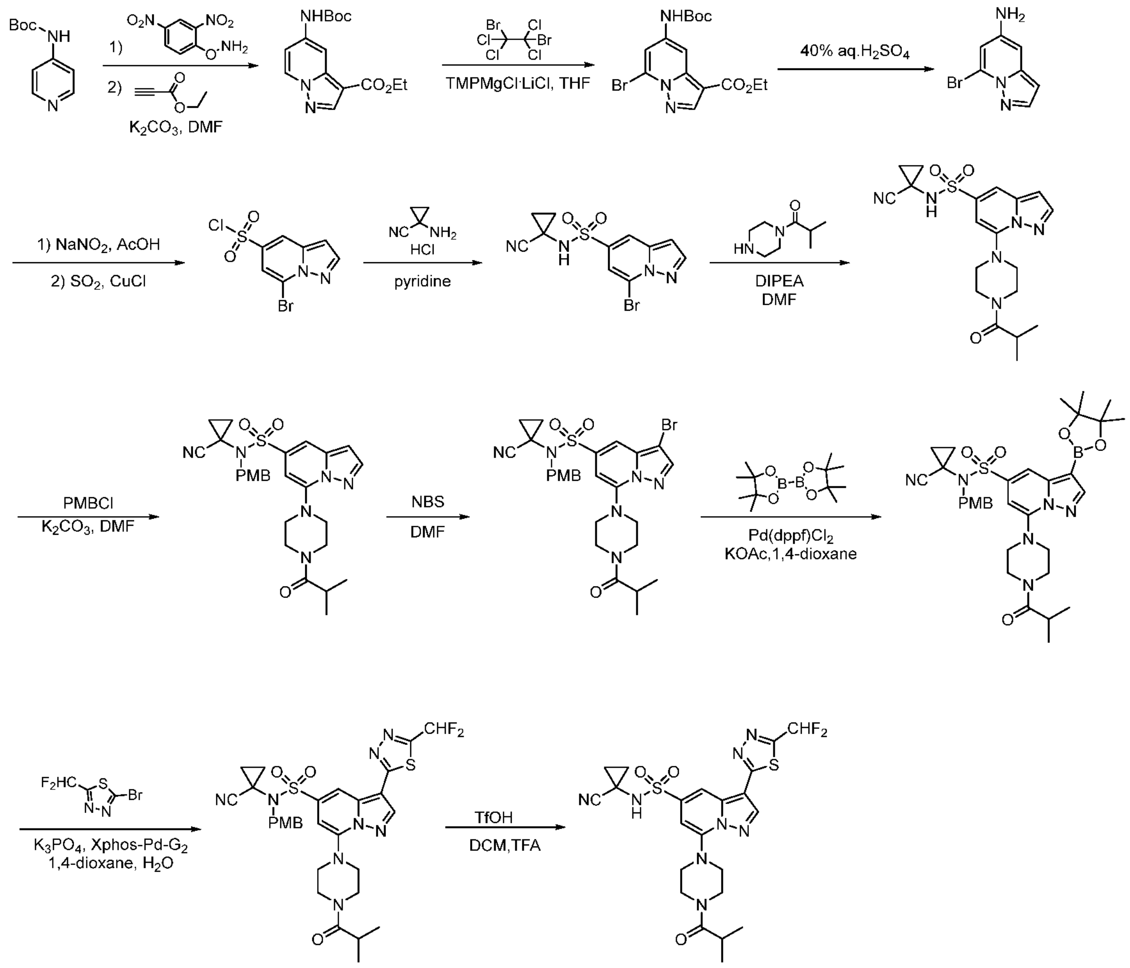


**Step 1: Ethyl 5-((tert-butoxycarbonyl)amino)pyrazolo[1,5-a]pyridine-3-carboxylate**

At room temperature, 2,4-dinitrophenylhydroxylamino (16.4 g, 82.5 mmol) was added portionwise to the solution of 4-(tert-butoxycarbonylamino)pyridine (16 g, 82.5 mmol) in acetonitrile (50 mL); and the reaction mixture was stirred at 40°C for 4 hours. The reaction mixture was concentrated under reduced pressure; the residue was dissolved in N,N-dimethylformamide (160 mL); then, potassium carbonate (22.8 g, 165 mmol) and ethyl propiolate (8.3 mL, 82.5 mmol) were added; and the mixture was stirred at room temperature for 16 hours. After completion of the reaction, the reaction mixture was diluted with ethyl acetate, washed with saturated aqueous potassium carbonate solution and saturated brine respectively, dried over anhydrous sodium sulfate, filtered, and concentrated under reduced pressure. The residue was purified by column chromatography (eluent: petroleum ether/ethyl acetate, gradient: 20-50% ethyl acetate) to obtain the title compound (yellow solid, 8.5 g, yield: 33.9%). LC/MS (ESI) m/z: 306 $[M+H]^+$.

**Step 2: Ethyl 7-bromo-5-((tert-butoxycarbonyl)amino)pyrazolo[1,5-a]pyridine-3-carboxylate**

At -78°C, the 2,2,6,6-tetramethylpiperidyl magnesium chloride-lithium chloride complex (57 mL, 57 mmol, 1M in THF) was dropwise added slowly to the solution of 5-(tert-butoxycarbonyl)amido)pyrazolo[1,5-a]pyridin-3-ethyl carboxylate (7 g, 23.0 mmol) in tetrahydrofuran (90 mL). The reaction mixture was stirred at -78°C for 20 minutes; then, 1,1,2,2-tetrachloro-1,2-dibromoethane (11.2 g, 34.5 mmol) was dropwise added; and thereafter, the reaction mixture was warmed up to room temperature and stirred for 2 hours. The reaction mixture was was dropwise added slowly to ice water for quenching, and extracted with ethyl acetate for liquid separation. The organic phase was washed with saturated sodium chloride solution, dried over anhydrous sodium sulfate, and concentrated under reduced pressure. The residue was purified by column chromatography (eluent: petroleum ether/ethyl acetate, gradient: 20-50% ethyl acetate) to obtain the title compound (yellow solid, 6.5 g, yield: 73.9%). $^1H$ NMR (400 MHz, $CDCl_3$) δ 8.40 (s, 1H), 7.93 (d, *J*= 2.1 Hz, 1H), 7.80 (s, 1H), 6.80 (s, 1H), 4.37 (q, *J*= 7.1 Hz, 2H), 1.55 (s, 9H), 1.41 (t, *J*= 7.1 Hz, 3H). LC/MS (ESI) m/z: 384/386 $[M+H]^+$.

**Step 3: 7-bromopyrazolo[1,5-a]pyridin-5-amine**

7-bromo-5-(tert-butoxycarbonyl)amino)pyrazolo[1,5-a]pyridin-3-ethyl carboxylate (3 g, 7.8 mmol) was dissolved in dilute sulphuric acid (40 mL, mass fraction: 40%), and then was stirred at 100°C for 4 hours. The reaction mixture was cooled to room temperature, then adjusted to pH of 7-8 using 4 mol/L aqueous sodium hydroxide solution, and extracted with ethyl acetate three times. The combined organic layers were washed with water and saturated brine, then dried over anhydrous sodium sulfate, and concentrated under reduced pressure. The residue was purified by column chromatography (eluent: petroleum ether/ethyl acetate, gradient: 0-50% ethyl acetate) to obtain the title compound (yellow solid, 1.4 g, yield: 84.7%). LC/MS (ESI) (m/z): 212 $[M+H]^+$.

**Step 4: 7-bromopyrazolo[1,5-a]pyridin-5-sulfonyl chloride**

At -10°C, aqueous sodium nitrite solution (322 mg, 4.67 mmol dissolved in 1 mL of water) was dropwise added slowly to the mixed solution of 7-bromopyrazolo[1,5-a]pyridine)-5-amine (900 mg, 4.2 mmol) in acetic acid (5 mL) and concentrated hydrochloric acid (5 mL); and the reaction mixture was stirred at -10°C for 45 minutes. In another three-necked flask, cuprous chloride (105 mg, 1.1 mmol) was dissolved in acetic acid (5 mL), and sulfur dioxide was introduced to the resulting mixture for bubbling for 30 minutes at -10°C. The above prepared mixed solution of diazonium salt was dropwise added to the solution of copper chloride and sulfur dioxide in acetic acid; and the reaction mixture was stirred at 10°C for 30 minutes. The reaction mixture was poured into ice water, and extracted with ethyl acetate twice. The combined organic phase was dried over anhydrous sodium sulfate, and concentrated under reduced pressure to obtain a crude title compound (yellow solid, 1.1 g, yield: 100%), which was directly used in the next step of reaction. LC/MS (ESI) m/z: 295 $[M+H]^+$.

**Step 5: 7-bromo-N-(1-cyanocyclopropyl)pyrazolo[1,5-a]pyridine-5-sulfonamide**

At 0°C, 7-bromopyrazolo[1,5-a]pyridin-5-sulfonyl chloride (1.1 g, 4.2 mmol) was added portionwise to the solution of 1-amino-1-cyclopropyl cyanic hydrochloride (500 mg, 4.2 mmol) in pyridine (10 mL); and the reaction mixturewas was stirred at room temperature for 30 minutes. The reaction mixture was diluted with ethyl acetate, washed with water and saturated brine in sequence, dried over anhydrous sodium sulfate, and concentrated under reduced pressure. The residue was separated by column chromatography (eluent: petroleum ether/ethyl acetate, gradient: 0-50% ethyl acetate) to obtain the title compound (yellow solid, 620 mg, yield: 43.2%). LC/MS (ESI) (m/z): 343 $[M+H]^+$.

**Step 6: N-(1-cyanocyclopropyl)-7-(4-isobutyrylpiperazin-1-yl)pyrazolo[1,5-a]pyridine-5-sulfonamide**

At room temperature, 1-(2-methylacetonyl)-piperazine (110 mg, 0.7 mmol) and N,N-diisopropylethylamine (0.3 mL, 1.75 mmol) were sequentially added to the solution of 7-bromo-N-(1-cyanocyclopropyl)pyrazolo[1,5-a]pyridin-5-sulfonamide (200 mg, 0.58 mmol) in N-methyl pyrrolidone (3 mL); and the reaction mixture was stirred at 120°C for 16 hours. The reaction mixture was diluted with ethyl acetate, washed with water and saturated brine, then dried over anhydrous sodium sulfate, and concentrated under reduced pressure. The residue was separated by column chromatography (eluent: petroleum ether/ethyl acetate, gradient: 0-50% ethyl acetate) to obtain the title compound (white solid, 220 mg, yield: 90.1%). LC/MS (ESI) (m/z): 417 $[M+H]^+$.

**Step 7: N-(1-cyanocyclopropyl)-7-(4-isobutyrylpiperazin-1-yl)-N-(4-methoxybenzyl)pyrazolo[1,5-a]pyridine-5-sulfonamide**

At room temperature, potassium carbonate (200 mg, 1.44 mmol) and 4-methoxybenzyl chloride (113 mg, 0.72 mmol) were added to the solution of N-(1-cyanocyclopropyl)-7-[4-isobutyrylpiperazin-1-yl]pyrazolo[1,5-a]pyridin-5-sulfonamide (220 mg, 0.48 mmol) in N,N- (3 mL); and then, the reaction mixture was stirred at 40°C for 2 hours. The reaction mixture was diluted with ethyl acetate, washed with water and saturated brine in sequence, dried over anhydrous sodium sulfate, and concentrated under reduced pressure. The residue was purified by column chromatography (eluent: petroleum ether/ethyl acetate, gradient: 0-30% ethyl acetate) to obtain the title compound (white solid, 210 mg, yield: 81.5%). LC/MS (ESI) (m/z): 537 $[M+H]^+$.

**Step 8: 3-bromo-N-(1-cyanocyclopropyl)-7-(4-isobutyrylpiperazin-1-yl)-N-(4-methoxybenzyl)pyrazolo[1,5-a]pyridine-5-sulfonamide**

At 0°C, NBS (66 mg, 0.37 mmol) was added portionwise to the solution of N-(1-cyanocyclopropyl)-N-[(4-methoxyphenyl)methyl]-7-[4-(2-methylpropionyl)piperazin-1-yl]pyrazolo[1,5-a]pyridin-5-sulfonamide (200 mg, 0.37

mmol) in N,N- (3 mL); and the reaction mixture was stirred at 0°C for 30 minutes. The reaction mixture was diluted with ethyl acetate, washed with water and saturated brine in sequence, dried over anhydrous sodium sulfate, and concentrated under reduced pressure. The residue was separated by column chromatography (eluent: petroleum ether/ethyl acetate, gradient: 0-30% ethyl acetate) to obtain the title compound (white solid, 200 mg, yield: 87.2%). LC/MS (ESI) (m/z): 617 $[M+H]^+$.

**Step 9: N-(1-cyanocyclopropyl)-7-(4-isobutyrylpiperazin-1-yl)-N-(4-methoxybenzyl)-3-(4,4,5,5-tetramethyl-1,3,2-dioxaborolan-2-yl)pyrazolo[1,5-a]pyridine-5-sulfonamide**

At room temperature, potassium acetate (86 mg, 0.88 mmol) and $Pd(dppf)Cl_2$ (21 mg, 0.03 mmol) were sequentially added to the solution of 3-bromo-N-(1-cyanocyclopropyl)-7-(4-isobutyrylpiperazin-1-yl)-N-(4-methoxybenzyl)pyrazolo[1,5-a]pyridin-5-sulfonamide (180 mg, 0.29 mmol) and bis(pinacolato)diboron (111 mg, 0.44 mmol) in 1,4-dioxane (3 mL); the flask was replaced with nitrogen gas three times and then the mixture was stirred at 100°C for 5 hours under an atmosphere of nitrogenn. The reaction mixture was cooled to room temperature, diluted with ethyl acetate, sequentially washed with water and saturated brine, dried over anhydrous sodium sulfate, and concentrated under reduced pressure. The residue was separated by column chromatography (eluent: petroleum ether/ethyl acetate, gradient: 0-30% ethyl acetate) to obtain the title compound (white solid, 160 mg, yield: 82.6%). LC/MS (ESI) (m/z): 663 $[M+H]^+$.

**Step 10: N-(1-cyanocyclopropyl)-3-(5-(difluoromethyl)-1,3,4-thiadiazol-2-yl)-7-(4-isobutyrylpiperazin-1-yl)-N-(4-methoxybenzyl)pyrazolo[1,5-a]pyridine-5-sulfonamide**

At room temperature, potassium phosphate (77 mg, 0.36 mmol), XPhos (9.6 mg, 0.02 mmol) and XPhos Pd G2 (10 mg, 0.01 mmol) were sequentially added to the solution of N-(1-cyanocyclopropyl)-7-(4-isobutyrylpiperazin-1-yl)-N-(4-methoxybenzyl)-3-(4,4,5,5-tetramethyl-1,3,2-dioxoboracyclopentan-2-yl)pyrazolo[1,5-a]pyridin-5-sulfonamide (80 mg, 0.12 mmol) and 2-bromo-5-(difluoromethyl)-1,3,4-thiadiazole (39 mg, 0.18 mmol) in 1,4-dioxane (3 mL) and water (1 mL). The flask was replaced with nitrogen gas three times and then the mixture was stirred at 100°C for 5 hours under an atmosphere of nitrogen. The reaction mixture was cooled to room temperature, diluted with ethyl acetate, washed with saturated brine twice, dried over anhydrous sodium sulfate, filtered and concentrated under reduced pressure. The residue was separated by column chromatography (eluent: petroleum ether/ethyl acetate, gradient: 0-30% ethyl acetate) to obtain the title compound (white solid, 42 mg, yield: 51.8%). LC/MS (ESI) (m/z): 671 $[M+H]^+$.

**Step 11: N-(1-cyanocyclopropyl)-3-(5-(difluoromethyl)-1,3,4-thiadiazol-2-yl)-7-(4-isobutyrylpiperazin-1-yl)pyrazolo[1,5-a]pyridine-5-sulfonamide**

The title compound was prepared by referring to the preparation method in step 9 of **Example 1** (white solid, 9 mg, yield: 49.8%). $^1H$ NMR (400 MHz, DMSO-$d_6$) δ 9.00 (s, 1H), 8.49 (d, $J$ = 1.3 Hz, 1H), 7.67 (t, $J$ = 53.3 Hz, 1H), 6.86 (s, 1H), 3.78 (d, $J$ = 20.0 Hz, 4H), 3.54 (d, $J$ = 24.9 Hz, 4H), 3.00 - 2.93 (m, 1H), 1.47 (t, $J$ = 6.7 Hz, 2H), 1.36 (t, $J$ = 6.8 Hz, 2H), 1.05 (d, $J$ = 6.7 Hz, 6H). LC/MS (ESI) (m/z): 551 $[M+H]^+$.

**By referring to the preparation method in Example 27, the following examples were implemented by synthesizing a crude product according to a similar route by starting from appropriate starting materials, treating the crude product via reversed-phase HPLC, and then freeze-drying the crude product to obtain the title compound.**

| Example | Structure and name | Analysis data |
|---|---|---|
| **Example 28** | 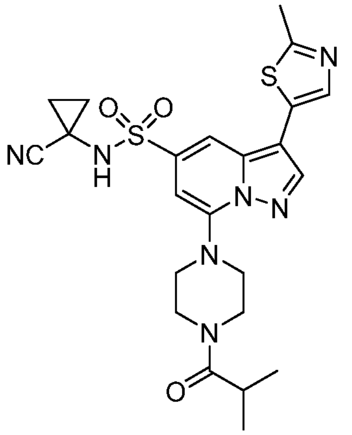 **N-(1-cyanocyclopropyl)-7-(4-isobutyrylpiperazin-1-yl)-3-(2-methylthiazol-5-yl)pyrazolo [1,5-a] pyridine-5-sulfonamide** | $^1$H NMR (400 MHz, DMSO-$d_6$) δ 8.61 (s, 1H), 7.99 (s, 1H), 7.91 (d, *J*= 1.4 Hz, 1H), 6.66 (s, 1H), 3.80 - 3.72 (m, 4H), 3.53 -3.44 (m, 4H), 2.98 - 2.92 (m, 1H), 2.73 (s, 3H), 1.52 - 1.43 (m, 2H), 1.38 - 1.30 (m, 2H), 1.06 (s, 3H), 1.04 (s, 3H). LC/MS (ESI) (m/z): 514 [M+H]+. |
| **Example 29** | 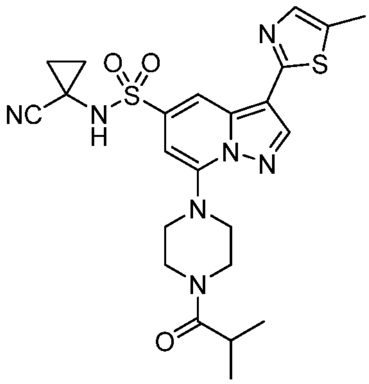 **N-(1-cyanocyclopropyl)-7-(4-isobutyrylpiperazin-1-yl)-3-(5-methylthiazol-2-yl)pyrazolo [1,5-a] pyridine-5-sulfonamide** | $^1$H NMR (400 MHz, DMSO-$d_6$) δ 8.67 (s, 1H), 8.39 (s, 1H), 7.62 (d, *J*= 1.2 Hz, 1H), 6.71 (d, *J*= 1.7 Hz, 1H), 3.80 - 3.72 (m, 4H), 3.52 - 3.46 (m, 4H), 3.43 (s, 3H), 2.99 - 2.95 (m, 1H), 1.34 - 1.29 (m, 2H), 1.25 - 1.23 (m, 2H), 1.06 (s, 3H), 1.04 (s, 3H). LC/MS (ESI) (m/z): 514 [M+H]+. |
| **Example 30** | 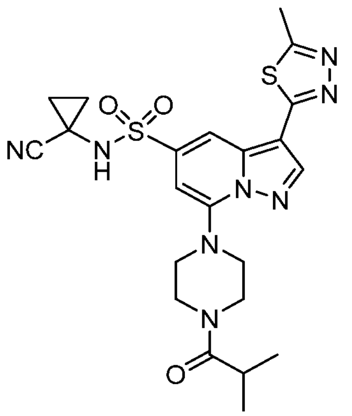 **N-(1-cyanocyclopropyl)-7-(4-isobutyrylpiperazin-1-yl)-3-(5-methyl-1,3,4-thiadiazol-2-yl)pyrazolo[1,5-a]pyridine-5-sulfonamide** | $^1$H NMR (400 MHz, DMSO-$d_6$) δ 8.82 (s, 1H), 8.45 (d, *J* = 1.7 Hz, 1H), 6.80 (d, *J* = 1.8 Hz, 1H), 3.77 (d, *J* = 18.8 Hz, 4H), 3.52 (d, *J* = 23.5 Hz, 4H), 2.98 - 2.94 (m, 1H), 2.80 (s, 3H), 1.47 - 1.44 (m, 2H), 1.38 - 1.34 (m, 2H), 1.06 (s, 3H), 1.04 (s, 3H). LC/MS (ESI) (m/z): 515 [M+H]+. |

**Example 31: 3-(5-(difluoromethyl)-1,3,4-thiadiazol-2-yl)-N-(1-(fluoromethyl)cyclopropyl)-8-(4-isobutyrylpiperazin-1-yl)imidazo[1,5-a]pyridine-6-sulfonamide**

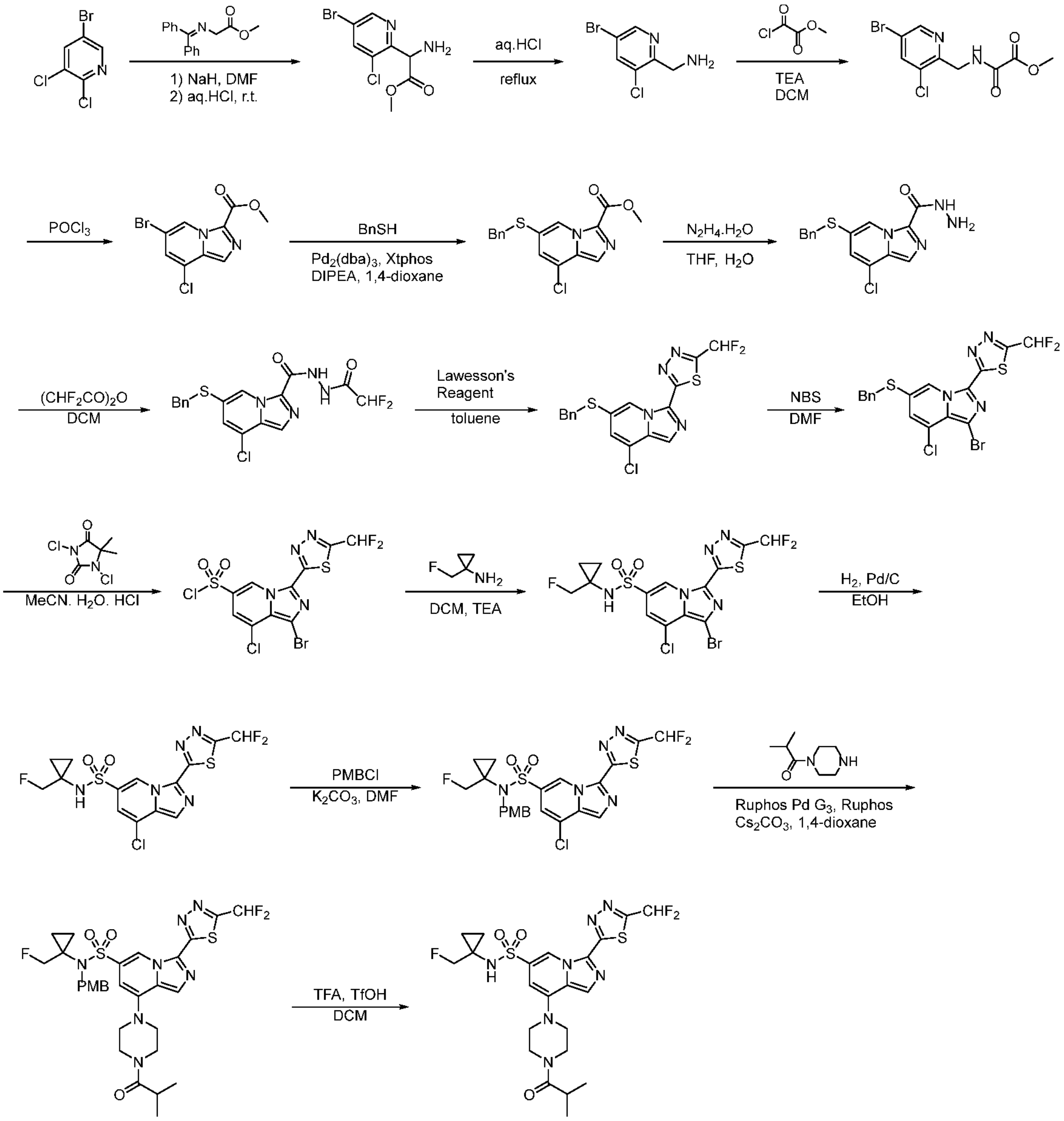


**Step 1: Methyl 2-amino-2-(5-bromo-3-chloropyridin-2-yl)acetate**

At 0°C, sodium hydride (23.7 g, 592.2 mmol, mass fraction: 60%, dispersed in mineral oil) was added portionwise to 2-[(diphenylmethylene)amino]methyl acetate (50 g, 197.4 mmol) in N,N- (250 mL), during which the temperature was controlled to 0°C, and thereafter, the mixture was continued to be stirred at 0°C for 30 minutes. At 0°C, the solution of 6-bromo-2,3-dichloropyridine (67.2 g, 296.1 mmol) in N,N- (150 mL) was slowly dropwise added to the above mixture, and the mixture was stirred at room temperature for 3 hours. The reaction mixture was quenched with 2 mol/L dilute hydrochloric acid at 0°C, and stirred at room temperature for 4 hours. The mixture was neutralized to pH = 8 using 1 mol/L aqueous sodium hydroxide solution, and extracted with ethyl acetate three times. The organic phase was washed with saturated brine, dried over anhydrous sodium sulfate, filtered and concentrated under reduced pressure to obtain a crude title compound (green solid, 50 g, yield: 90.6%), which was directly used in the next step of reaction. LC/MS (ESI) (m/z): 279/281 $[M+H]^+$.

**Step 2: (5-bromo-3-chloropyridin-2-yl)methanamine**

2-amino-2-(6-bromo-3-chloropyridin-2-yl)methyl acetate (50 g, 178.9 mmol) was dissolved in 3N dilute hydrochloric acid (700 mL); and then, the resulting mixture was heated to reflux for 3 hours. The reaction mixture was diluted with ethyl acetate, and adjusted to a pH value around 9 by slowly dropwise adding 1 mol/L aqueous sodium hydroxide solution. The organic layer was washed with saturated brine, dried over anhydrous sodium sulfate, filtered and concentrated under reduced pressure to obtain the title compound (yellow solid, 13.5g, yield: 34.1%). LC/MS (ESI) (m/z): 221/223 $[M+H]^+$.

**Step 3: Methyl 2-(((5-bromo-3-chloropyridin-2-yl)methyl)amino)-2-oxoacetate**

At 0°C, triethylamine (12 mL, 84.6 mmol) and 2-chloro-2-oxomethyl acetate (5.4 mL, 59.3 mmol) were dropwise added to the solution of (5-bromo-3-chloropyridin-2-yl)methylamine (12.5 g, 56.4 mmol) in tetrahydrofuran (250 mL), after which the mixture was stirred at room temperature for 2 hours. After completion of the reaction, the reaction mixture was added with ice water for quenching, and extracted with ethyl acetate. The organic phase was washed with saturated brine, dried over anhydrous sodium sulfate, and concentrated under reduced pressure. The residue was purified by column chromatography (eluent: petroleum ether/ethyl acetate, gradient: 0-50% ethyl acetate) to obtain the title compound (brown solid, 12g, yield: 69.1%). LC/MS (ESI) (m/z): 307/309 $[M+H]^+$.

**Step 4: Methyl 6-bromo-8-chloroimidazo[1,5-a]pyridine-3-carboxylate**

At 0°C, 2-(((5-bromo-3-chloropyridin-2-yl)methyl)amino)-2-oxomethyl acetate (12 g, 39.02 mmol) was dissolved in phosphorus oxychloride (80.0 mL); and the reaction mixture was stirred at 110°C for 16 hours. After completion of the reaction, the reaction mixture was cooled to room temperature, and concentrated under reduced pressure to remove phosphorus oxychloride; then, ethyl acetate was added; and the resulting mixture was concentrated to take out the residual phosphorus oxychloride. Ice water was added to the residues for quenching; the resulting mixture was extracted with ethyl acetate; and the organic phase was washed with saturated brine, dried over anhydrous sodium sulfate, and concentrated under reduced pressure. The residue was purified by column chromatography (eluent: petroleum ether/ethyl acetate, gradient: 5-30% ethyl acetate) to obtain the title compound (brown solid, 5g, yield: 44.3%). LC/MS (ESI) (m/z): 289/291 $[M+H]^+$.

**Step 5: Methyl 6-(benzylthio)-8-chloroimidazo[1,5-a]pyridine-3-carboxylate**

At room temperature and under the nitrogen atmosphere, XantPhos (1.0 g, 1.73 mmol), N,N-diisopropylethylamine (8.6 mL, 51.8 mmol) and $Pd_2(dba)_3$ (0.79 g, 0.86 mmol) were added to the solution of 6-bromo-8-chloroimidazo[1,5-a]pyridin-3-methyl carboxylate (5 g, 17.3 mmol) and benzyl mercaptane (3 mL, 25.9 mmol) in 1,4-dioxane (50 mL). The flask was replaced with nitrogen gas three times and then the mixture was stirred at 95°C for 16 hours under an atmosphere of nitrogen. After completion of the reaction, the reaction mixture was diluted with ethyl acetate, washed with water and saturated brine in sequence, dried over anhydrous sodium sulfate, and concentrated under reduced pressure. The residue was separated by column chromatography (eluent: petroleum ether/ethyl acetate, gradient: 10-50% ethyl acetate) to obtain the title compound (white solid, 5 g, yield: 86.9%). LC/MS (ESI) (m/z): 333/335 $[M+H]^+$.

**Step 6: 6-(benzylthio)-8-chloroimidazo[1,5-a]pyridine-3-carbohydrazide**

At room temperature, hydrazine hydrate (1.35 g, 27.0 mmol) was added to the solution of 6-(benzylthioalkyl)-8-chloroimidazo[1,5-a]pyridin-3-methyl formate (1.5 g, 4.5 mmol) in methanol (15 mL); and the mixture was stirred at room temperature for 30 minutes. After completion of the reaction, water was added; the reaction mixture was extracted with dichloromethane; and the organic phase was washed with saturated brine, dried over anhydrous sodium sulfate, and concentrated under reduced pressure to obtain a crude title compound (yellow solid, 1.4 g, yield: 93.33%), which was directly used in the next step of reaction. LC/MS (ESI) (m/z): 333/335 $[M+H]^+$.

**Step 7: 6-(benzylthio)-8-chloro-N'-(2,2-difluoroacetyl)imidazo[1,5-a]pyridine-3-carbohydrazide**

At 0°C, difluoroacetic anhydride (1.12 mL, 9.0 mmol) was added to the solution of 6-(benzylthioalkyl)-8-chloroimidazo[1,5-a]pyridin-3-formylhydrazine (1.5 g, 4.5 mmol) in dichloromethane (15 mL); and the mixture was stirred at room temperature for 20 minutes. After completion of the reaction, the reaction mixture was diluted with dichloromethane, washed with saturated brine, dried over anhydrous sodium sulfate, and concentrated under reduced pressure. The residue was purified by column chromatography (eluent: petroleum ether/ethyl acetate, gradient: 0-50% ethyl acetate) to obtain the title compound (yellow solid, 1.3 g, yield: 70%). LC/MS (ESI) (m/z): 411/413 $[M+H]^+$.

**Step 8: 2-(6-(benzylthio)-8-chloroimidazo[1,5-a]pyridin-3-yl)-5-(difluoromethyl)-1,3,4-thiadiazole**

At room temperature, the Lawesson's reagent (1.41 g, 3.48 mmol) was added to the solution of 6-(benzylthio)-8-chloro-N'-(2,2-difluoroacetyl)imidazo[1,5-a]pyridin-3-formylhydrazine (1.3 g, 3.16 mmol) in toluene (15 mL); and the reaction mixture was stirred at 110°C for 4 hours under an atmosphere of nitrogen. After completion of the reaction, the reaction mixture was diluted with ethyl acetate, washed with saturated sodium bicarbonate and saturated brine in sequence, dried over anhydrous sodium sulfate, and concentrated under reduced pressure. The residue was purified by column chromatography (eluent: petroleum ether/ethyl acetate, gradient: 0-30% ethyl acetate) to obtain the title compound (yellow solid, 550 mg, yield: 42.5%). LC/MS (ESI) (m/z): 409/411 $[M+H]^+$.

**Step 9: 2-(6-(benzylthio)-1-bromo-8-chloroimidazo[1,5-a]pyridin-3-yl)-5-(difluoromethyl)-1,3,4-thiadiazole**

At room temperature, N-bromosuccinimide (105 mg, 0.56 mmol) was added portionwise to the solution of 2-(6-(benzylthio)-8-chloroimidazo[1,5-a]pyridin-3-yl)-5-(difluoromethyl)-1,3,4-thiadiazole (200 mg, 0.49 mmol) in N,N-dimethylformamide (5 mL). The reaction mixture was stirred at room temperature for 30 minutes. After completion of the reaction, ice water was added for quenching; the reaction mixture was extracted with ethyl acetate; and the organic phase was washed with saturated brine, dried over anhydrous sodium sulfate, and concentrated under reduced pressure. The residue was separated by column chromatography (eluent: petroleum ether/ethyl acetate, gradient: 0-35% ethyl acetate) to obtain the title compound (yellow solid, 205 mg, yield: 86%).

**Step 10: 1-bromo-8-chloro-3-(5-(difluoromethyl)-1,3,4-thiadiazol-2-yl)imidazo[1,5-a]pyridine-6-sulfonyl chloride**

At 0°C, 1,3-dichloro-5,5-dimethylhydantoin (243 mg, 1.23 mmol) was portionwise added to the solution of 2-(6-(benzylthio)-1-bromo-8-chloroimidazo[1,5-a]pyridin-3-yl)-5-(difluoromethyl)-1,3,4-thiadiazole (200 mg, 0.41 mmol) in acetonitrile (5 mL) and 0.5 N hydrochloric acid (1 mL); and the reaction mixture was stirred at 0°C for 30 minutes. After completion of the reaction, the reaction mixture was added with ice water for quenching, and extracted with dichloromethane; and the organic phase was washed with saturated brine, dried over anhydrous sodium sulfate, and concentrated under reduced pressure to obtain a crude title compound (yellow solid, 150 mg, yield: 78.8%), which was directly used in the next reaction..

**Step 11: 1-bromo-8-chloro-3-(5-(difluoromethyl)-1,3,4-thiadiazol-2-yl)-N-(1-(fluoromethyl)cyclopropyl)imidazo[1,5-a]pyridine-6-sulfonamide**

At 0°C, 1-bromo-8-chloro-3-(5-difluoromethyl-1,3,4-thiadiazol-2-yl]imidazo[1,5-a]pyridin-6-sulfonyl chloride(200 mg, 0.43 mmol) was added to the solution of 1-(fluoromethyl)cyclopropylamine hydrochloride (108 mg, 0.86 mmol) in pyridine (3 mL); and the reaction mixture was stirred at room temperature for 30 minutes. After completion of the reaction, the reaction mixture was added with ice water for quenching, and extracted with ethyl acetate. The organic phase was washed with saturated brine, dried over anhydrous sodium sulfate, and concentrated under reduced pressure to obtain the title compound (yellow solid, 110 mg, yield: 49.5%). $^1$H NMR (400 MHz, DMSO-$d_6$) $\delta$ 9.84 (s, 1H), 9.03 (s, 1H), 7.77 (d, $J$ = 52.7 Hz, 1H), 7.49 (s, 1H), 4.26 (d, $J$ = 48.4 Hz, 2H), 0.93 - 0.89 (m, 2H), 0.83 - 0.79 (m, 2H). LC/MS (ESI) (m/z): 517 $[M+H]^+$.

**Step 12: 8-chloro-3-(5-(difluoromethyl)-1,3,4-thiadiazol-2-yl)-N-(1-(fluoromethyl)cyclopropyl)imidazo[1,5-a]pyridine-6-sulfonamide**

At 0°C, Pd/C (15 mg, mass fraction: 10%) was added to the solution of 1-bromo-8-chloro-3-(5-(difluoromethyl)-1,3,4-thiadiazol-2-yl)-N-(1-(fluoromethyl)cyclopropyl)imidazo[1,5-a]pyridin-6-sulfonamide (100 mg, 0.19 mmol) in ethanol (5 mL); the flask was replaced with nitrogen gas three times and then the mixture was stirred at room temperature for 4 hours under an atmosphere provided by a hydrogen-filled balloon. After completion of the reaction, the mixture was filtered. The filtrate was concentrated under reduced pressure to obtain the title compound (yellow solid, 70 mg, yield: 82.8%). LC/MS (ESI) (m/z): 438 $[M+H]^+$.

**Step 13: 8-chloro-3-(5-(difluoromethyl)-1,3,4-thiadiazol-2-yl)-N-(1-(fluoromethyl)cyclopropyl)-N-(4-methoxybenzyl)imidazo[1,5-a]pyridine-6-sulfonamide**

At room temperature, potassium carbonate (67 mg, 0.48 mmol) and 1-(chloromethyl)-4-metoxybenzene (38 mg, 0.24 mmol) were added to the solution of 8-chloro-3-[5-(difluoromethyl)-1,3,4-thiadiazol-2-yl]-N-[1-(fluoromethyl)cyclopropyl]imidazo[1,5-a]pyridin-6-sulfonamide (80 mg, 0.18 mmol) in N,N- (1 mL); and the reaction mixture was stirred at 40°C for 6 hours. After completion of the reaction, the reaction mixture was diluted with ethyl acetate, washed with water and saturated brine in sequence, dried over anhydrous sodium sulfate, and concentrated under reduced pressure. The residue was separated by column chromatography (eluent: petroleum ether/ethyl acetate, gradient: 0-15% ethyl acetate) to obtain the title compound (white solid, 80 mg, yield: 89.6%). LC/MS (ESI) (m/z): 558 $[M+H]^+$.

**Step 14: 3-(5-(difluoromethyl)-1,3,4-thiadiazol-2-yl)-N-(1-(fluoromethyl)cyclopropyl)-8-(4-isobutyrylpiperazin-1-yl)-N-(4-methoxybenzyl)imidazo[1,5-a]pyridine-6-sulfonamide**

Under an atmosphere of nitrogen at room temperature, cesium carbonate (137 mg, 0.42 mmol), RuPhos (13 mg, 0.03 mmol)and RuPhos Pd G3 (12 mg, 0.01 mmol) were sequentially added to the solution of 8-chloro-3-(5-(difluoromethyl)-1,3,4-thiadiazol-2-yl)-N-(1-(fluoromethyl)cyclopropyl)-N-(4-methoxybenzyl)imidazol[1,5-a]pyridin-6-sulfonamide (80 mg, 0.14 mmol) and 1-(2-methylacetonyl)-piperazine (34 mg, 0.21 mmol) in 1,4-dioxane (2 mL). The flask was replaced with nitrogen gas three times and then the mixture was stirred at 70°C for 4 hours under an atmosphere of nitrogen. After completion of the reaction, the reaction mixture was cooled to room temperature, quenched, and extracted with ethyl acetate; and the organic phase was washed with saturated brine, dried over anhydrous sodium sulfate, and concentrated under reduced pressure. The residue was purified by column chromatography (eluent: petroleum ether/ethyl acetate, gradient: 0-60% ethyl acetate) to obtain the title compound (yellow solid, 60 mg, yield: 61.7%). LC/MS (ESI) (m/z): 678 $[M+H]^+$.

**Step 15: 3-(5-(difluoromethyl)-1,3,4-thiadiazol-2-yl)-N-(1-(fluoromethyl)cyclopropyl)-8-(4-isobutyrylpiperazin-1-yl)imidazo[1,5-a]pyridine-6-sulfonamide**

At 0°C, trifluoroacetic acid (0.1 mL) and trifluoromethanesulfonic acid (0.1 mL) were added to the solution of 3-(5-(difluoromethyl)-1,3,4-thiadiazol-2-yl)-N-(1-(fluoromethyl)cyclopropyl)-8-(4-isobutyrylpiperazin-1-yl)-N-(4-methoxybenzyl)imidazo[1,5-a]pyridin-6-sulfonamide (60 mg, 0.09 mmol) in dichloromethane (2 mL); and the reaction mixture was stirred at 0°C for 2 hours. The reaction mixture was concentrated under reduced pressure; and the residue was purified by pre-HPLC to obtain the title compound (white solid, 13 mg, yield: 30.4%). $^1H$ NMR (400 MHz, DMSO-$d_6$) δ 9.58 (s, 1H), 8.82 (s, 1H), 8.07 (s, 1H), 7.68 (t, $J$ = 52.8 Hz, 1H), 6.69 (s, 1H), 4.23 (d, $J$ = 48.2 Hz, 2H), 3.80 - 3.73 (m, 4H), 3.45 - 3.41 (m, 4H), 2.97 - 2.93 (m, 1H), 1.05 (d, $J$ = 5.2 Hz, 6H), 0.89 - 0.84 (m, 2H), 0.81 - 0.76 (m, 2H). LC/MS (ESI) (m/z): 558 $[M+H]^+$.

**By referring to the preparation method in Example 31, the following examples were implemented according to a similar route by starting from appropriate starting materials.**

| Example | Structure and name | Analysis data |
|---|---|---|
| Example 32 | 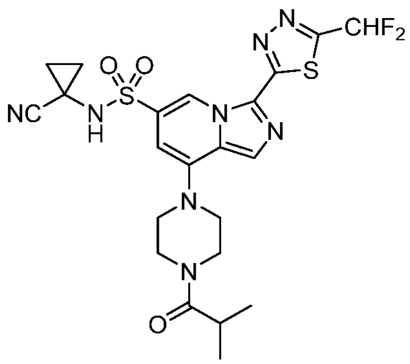 **N-(1-cyanocyclopropyl)-3-(5-(difluoromethyl)-1,3,4-thiadiazol-2-yl)-8-(4-isobutyrylpiperazin-1-yl)imidazo [1,5-a] pyridine-6-sulfonamide** | $^1H$ NMR (400 MHz, DMSO-$d_6$) δ 9.69 (s, 1H), 9.49 (s, 1H), 8.11 (s, 1H), 7.69 (t, $J$ = 53.2 Hz, 1H), 6.68 (s, 1H), 3.88 - 3.68 (m, 4H), 3.32 - 3.27 (m, 4H), 3.03 - 2.87 (m, 1H), 1.56 - 1.46 (m, 2H), 1.42 - 1.35 (m, 2H), 1.06 (s, 3H), 1.04 (s, 3H). LC/MS (ESI) (m/z): 551 $[M+H]^+$. |

(continued)

| Example | Structure and name | Analysis data |
|---|---|---|
| **Example 33** | 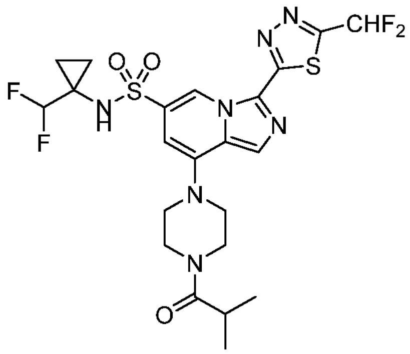 **3-(5-(difluoromethyl)-1,3,4-thiadiazol-2-yl)-N-(1-(difluoromethyl) cyclopropyl)-8-(4-isobutyrylpiperazin-1-yl)imidazo[1,5-a]pyridine-6-sulfonamide** | $^1$H NMR (400 MHz, $CD_3OD$) δ 9.82 (s, 1H), 7.93 (s, 1H), 7.33 (t, 1H), 6.75 (s, 1H), 5.77 (t, *J* = 56.9 Hz, 1H), 3.91 - 3.86 (m, 4H), 3.49 - 3.39 (m, 4H), 3.06 - 2.99 (m, 1H), 1.29 - 1.27 (m, 2H), 1.15 (d, *J* = 6.4 Hz, 6H), 1.04 - 1.02 (m, 2H). LC/MS (ESI) (m/z): 576 $[M+H]^+$. |
| **Example 34** | 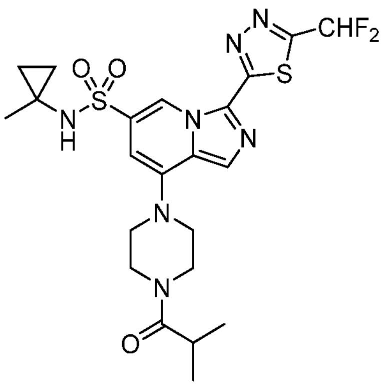 **3-(5-(difluoromethyl)-1,3,4-thiadiazol-2-yl)-8-(4-isobutyrylpiperazin-1-yl)-N-(1-methylcyclopropyl)imidazo[1,5-a]pyridine-6-sulfonamide** | $^1$H NMR (400 MHz, DMSO-$d_6$) δ 9.60 (s, 1H), 8.42 (s, 1H), 8.07 (s, 1H), 7.68 (t, *J* = 53.4 Hz, 1H), 6.69 (s, 1H), 3.80 - 3.71 (m, 4H), 3.29 - 3.23 (m, 4H), 3.00 - 2.90 (m, 1H), 1.16 (s, 3H), 1.05 (d, *J* = 6.6 Hz, 6H), 0.75 - 0.70 (m, 2H), 0.48 - 0.43 (m, 2H). LC/MS (ESI) (m/z): 540 $[M+H]^+$. |
| **Example 35** | 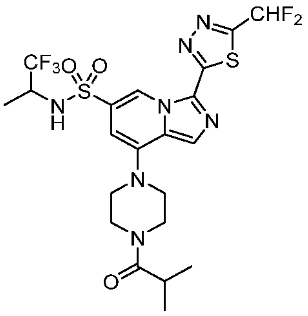 **3-(5-(difluoromethyl)-1,3,4-thiadiazol-2-yl)-8-(4-isobutyrylpiperazin-1-yl)-N-(1,1,1-trifluoropropan-2-yl)imidazo[1,5-a]pyridine-6-sulfonamide** | $^1$H NMR (400 MHz, DMSO-$d_6$) δ 9.62 (s, 1H), 8.88 (d, *J* = 8.6 Hz, 1H), 8.08 (s, 1H), 7.62 (d, *J* = 53.2 Hz, 1H), 6.74 (s, 1H), 4.22 - 4.14 (m, 1H), 3.80 - 3.72 (m, 4H), 3.50 - 3.42 (m, 4H), 2.96 - 2.95 (m, 1H), 1.07 - 1.03 (m, 9H). LC/MS (ESI) (m/z): 582 $[M+H]^+$. |

(continued)

| Example | Structure and name | Analysis data |
|---|---|---|
| **Example 36** | 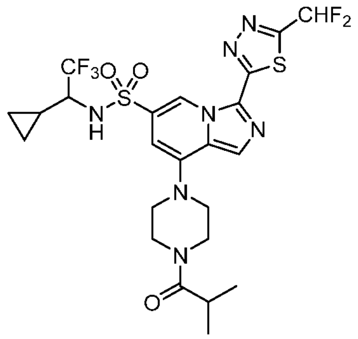 **N-(1-cyclopropyl-2,2,2-trifluoroethyl)-3-(5-(difluoromethyl)-1,3,4-thiadiazol-2-yl)-8-(4-isobutyrylpiperazin-1-yl)imidazo[1,5-a]pyridine-6-sulfonamide** | $^1$H NMR (400 MHz, DMSO-$d_6$) δ 9.83 (s, 1H), 9.33 (s, 1H), 8.31 (s, 1H), 7.91 (t, $J$ = 53.2 Hz, 1H), 7.04 (s, 1H), 4.04 - 3.96 (m, 4H), 3.80 - 3.68 (m, 4H), 3.50 - 3.47 (m, 1H), 3.20 - 3.15 (m, 1H), 1.28 (d, $J$ = 6.7 Hz, 6H), 1.23 - 1.18 (m, 1H), 0.73 - 0.63 (m, 2H), 0.47 - 0.44 (m, 1H), 0.00 - -0.01 (m 1H). LC/MS (ESI) (m/z): 608 [M+H]$^+$. |
| **Example 37** | 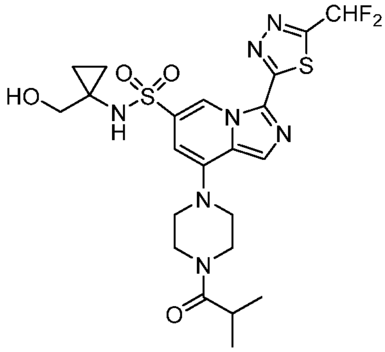 **3-(5-(difluoromethyl)-1,3,4-thiadiazol-2-yl)-N-(1-(hydroxymethyl)cyclopropyl)-8-(4-isobutyrylpiperazin-1-yl)imidazo[1,5-a]pyridine-6-sulfonamide** | $^1$H NMR (400 MHz, DMSO-$d_6$) δ 9.57 (s, 1H), 8.52 (s, 1H), 8.05 (s, 1H), 7.80 - 7.53 (m, 1H), 6.72 (s, 1H), 4.64 (t, $J$ = 5.7 Hz, 1H), 3.75 (d, $J$ = 14.9 Hz, 4H), 3.31 - 3.27 (m, 6H), 2.97 - 2.92 (m, 1H), 1.04 (d, $J$ = 6.7 Hz, 6H), 0.66 - 0.63 (m, 2H), 0.63 - 0.60 (m, 2H). LC/MS (ESI) (m/z): 556 [M+H]$^+$. |
| **Example 38** | 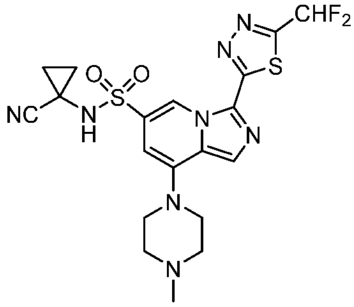 **N-(1-cyanocyclopropyl)-3-(5-(difluoromethyl)-1,3,4-thiadiazol-2-yl)-8-(4-methylpiperazin-1-yl)imidazo[1,5-a]pyridine-6-sulfonamide** | $^1$H NMR (400 MHz, DMSO-$d_6$) δ 9.67 (s, 1H), 8.15 (s, 1H), 8.03 (s, 1H), 7.68 (t, $J$ = 53.1 Hz, 1H), 6.66 (s, 1H), 3.32 - 3.30 (m, 4H), 2.62 - 2.58 (m, 4H), 2.30 (s, 3H), 1.50 - 1.46 (m, 2H), 1.40 - 1.36 (m, 2H). LC/MS (ESI) (m/z): 495 [M+H]$^+$. |

(continued)

| Example | Structure and name | Analysis data |
|---|---|---|
| **Example 39** | 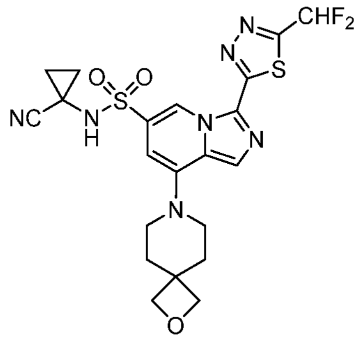 **N-(1-cyanocyclopropyl)-3-(5-(difluoromethyl)-1,3,4-thiadiazol-2-yl)-8-(2-oxa-7-azaspiro[3.5]nonan-7-yl)imidazo[1,5-a]pyridine-6-sulfonamide** | $^1H$ NMR (400 MHz, DMSO-$d_6$) δ 9.71 (s, 1H), 9.51 (s, 1H), 8.06 (s, 1H), 7.74 (t, *J* = 53.2 Hz, 1H), 6.73 (d, *J* = 0.9 Hz, 1H), 4.47 (s, 4H), 3.35 - 3.31 (m, 4H), 2.13 - 2.08 (m, 4H), 1.56 - 1.51 (m, 2H), 1.47 - 1.42 (m, 2H). LC/MS (ESI) (m/z): 522 $[M+H]^+$. |
| **Example 40** | 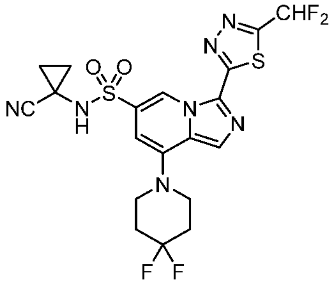 **N-(1-cyanocyclopropyl)-3-(5-(difluoromethyl)-1,3,4-thiadiazol-2-yl)-8-(4,4-difluoropiperidin-1-yl)imidazo[1,5-a]pyridine-6-sulfonamide** | $^1H$ NMR (400 MHz, DMSO-$d_6$) δ 9.69 (s, 1H), 9.48 (s, 1H), 8.10 (s, 1H), 7.82 - 7.55 (m, 1H), 6.74 (s, 1H), 3.50 - 3.45 (m, 4H), 2.31 - 2.21 (m, 4H), 1.51 - 1.45 (m, 2H), 1.43 - 1.38 (m, 2H). LC/MS (ESI) m/z: 516 $[M+H]^+$. |
| **Example 41** | 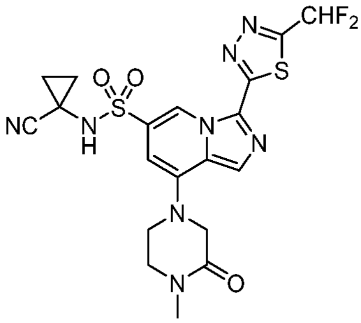 **N-(1-cyanocyclopropyl)-3-(5-(difluoromethyl)-1,3,4-thiadiazol-2-yl)-8-(4-methyl-3-oxopiperazin-1-yl)imidazo[1,5-a]pyridine-6-sulfonamide** | $^1H$ NMR (400 MHz, DMSO-$d_6$) δ 9.67 (s, 1H), 9.46 (s, 1H), 8.12 (s, 1H), 7.82 - 7.54 (m, 1H), 6.63 (d, *J* = 1.0 Hz, 1H), 3.97 (s, 2H), 3.76 (t, *J* = 5.2 Hz, 2H), 3.57 (t, *J* = 5.3 Hz, 2H), 2.95 (s, 3H), 1.51 - 1.44 (m, 2H), 1.40 - 1.35 (m, 2H). LC/MS (ESI) (m/z): 509 $[M+H]^+$. |
| **Example 42** | 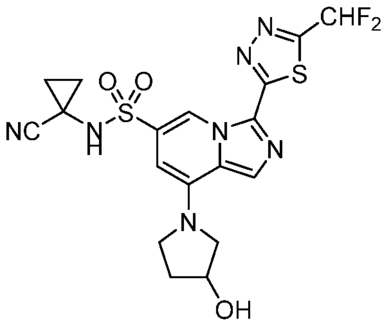 **N-(1-cyanocyclopropyl)-3-(5-(difluoromethyl)-1,3,4-thiadiazol-2-yl)-8-(3-hydroxypyrrolidin-1-yl)imidazo [1,5-a] pyridine-6-sulfonamide** | $^1H$ NMR (400 MHz, DMSO-$d_6$) δ 9.38 (s, 1H), 8.38 (s, 1H), 8.17 (s, 1H), 7.81 - 7.54 (m, 1H), 6.11 (s, 1H), 4.52 - 4.44 (m, 1H), 3.90 - 3.75 (m, 3H), 3.71 - 3.67 (m, 1H), 2.12 - 2.05 (m, 1H), 2.04 - 1.97 (m, 1H), 1.35 - 1.29 (m, 2H), 1.26 - 1.21 (m, 2H). LC/MS (ESI) (m/z): 482 $[M+H]^+$. |

(continued)

| Example | Structure and name | Analysis data |
|---|---|---|
| **Example 43** | 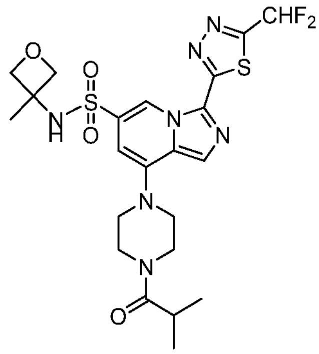 **3-(5-(difluoromethyl)-1,3,4-thiadiazol-2-yl)-8-(4-isobutyrylpiperazin-1-yl)-N-(3-methyloxetan-3-yl)imidazo[1,5-a]pyridine-6-sulfonamide** | $^1H$ NMR (400 MHz, DMSO-$d_6$) δ 9.59 (s, 1H), 8.72 (s, 1H), 8.08 (s, 1H), 7.68 (t, *J* = 53.2 Hz, 1H), 6.70 (s, 1H), 4.63 (d, *J* = 6.2 Hz, 2H), 4.18 (d, *J* = 6.3 Hz, 2H), 3.84 - 3.68 (m, 4H), 3.41 - 3.36 (m, 4H), 3.00 - 2.89 (m, 1H), 1.50 (s, 3H), 1.05 (d, *J* = 6.7 Hz, 6H). LC/MS (ESI) (m/z): 556 $[M+H]^+$. |
| **Example 44** | 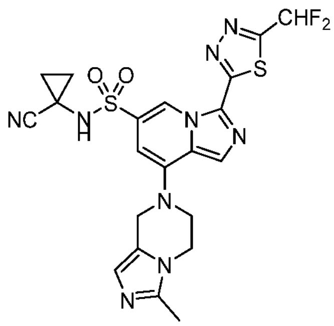 **N-(1-cyanocyclopropyl)-3-(5-(difluoromethyl)-1,3,4-thiadiazol-2-yl)-8-(3-methyl-5,6-dihydroimidazo[1,5-a]pyrazin-7(8H)-yl)imidazo[1,5-a]pyridine-6-sulfonamide** | $^1H$ NMR (400 MHz, DMSO-$d_6$) δ 9.70 (s, 1H), 8.13 (d, *J* = 3.3 Hz, 1H), 7.82 - 7.55 (m, 1H), 6.82 (s, 1H), 6.75 (s, 1H), 4.59 (s, 2H), 4.19 - 4.12 (m, 2H), 3.93 - 3.87 (m, 2H), 2.32 (s, 3H), 1.48 - 1.43 (m, 2H), 1.40 - 1.35 (m, 2H). LC/MS (ESI) (m/z): 532 $[M+H]^+$. |
| **Example 45** | 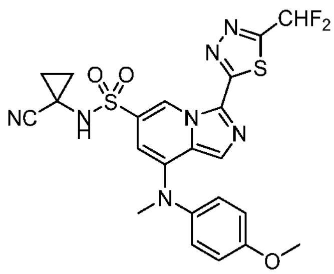 **N-(1-cyanocyclopropyl)-3-(5-(difluoromethyl)-1,3,4-thiadiazol-2-yl)-8-((4-methoxyphenyl)(methyl)amino)imidazo[1,5-a]pyridine-6-sulfonamide** | $^1H$ NMR (400 MHz, $CDCl_3$) δ 9.76 (s, 1H), 7.12 - 7.08 (m, 2H), 6.99 - 6.82 (m, 3H), 6.54 (s, 1H), 6.27 (s, 1H), 6.11 (s, 1H), 3.76 (s, 3H), 3.37 (s, 3H), 1.48 - 1.42 (m, 2H), 1.21 - 1.16 (m, 2H). LC/MS (ESI) (m/z): 532 $[M+H]^+$. |
| **Example 46** | 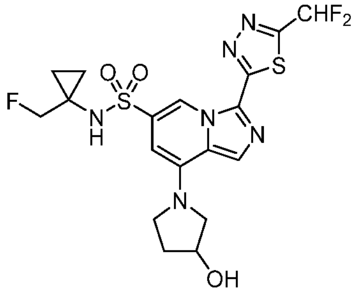 **3-(5-(difluoromethyl)-1,3,4-thiadiazol-2-yl)-N-(1-(fluoromethyl)cyclopropyl)-8-(3-hydroxypyrrolidin-1-yl)imidazo[1,5-a]pyridine-6-sulfonamide** | $^1H$ NMR (400 MHz, DMSO-$d_6$) δ 9.34 (s, 1H), 8.72 (s, 1H), 8.19 (s, 1H), 7.67 (s, 1H), 6.11 (s, 1H), 5.16 - 5.13 (m, 1H), 4.50 - 4.46 (m, 1H), 4.27 (d, *J* = 48.6 Hz, 2H), 3.86 - 3.75 (m, 2H), 3.72 - 3.66 (m, 1H), 0.87 - 0.81 (m, 4H), 0.78 - 0.75 (m, 2H). LC/MS (ESI) (m/z): 489 $[M+H]^+$. |

(continued)

| Example | Structure and name | Analysis data |
|---|---|---|
| **Example 47** | 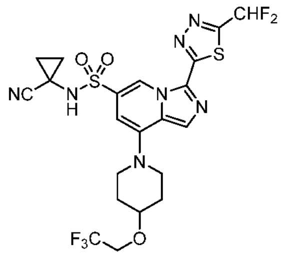<br>**N-(1-cyanocyclopropyl)-3-(5-(difluoromethyl)-1,3,4-thiadiazol-2-yl)-8-(4-(2,2,2-trifluoroethoxy)piperidin-1-yl)imidazo[1,5-a]pyridine-6-sulfonamide** | $^1$H NMR (400 MHz, DMSO-$d_6$) δ 9.66 (s, 1H), 9.47 (s, 1H), 8.03 (s, 1H), 7.68 (t, 1H), 6.68 (s, 1H), 4.19 - 4.13 (m, 2H), 3.84 - 3.79 (m, 1H), 3.61 - 3.57 (m, 2H), 3.19 - 3.14 (m, 2H), 2.12 - 2.04 (m, 2H), 1.82 - 1.75 (m, 2H), 1.50 - 1.47 (m, 2H), 1.41 - 1.37 (m, 2H). LC/MS (ESI) (m/z): 578 [M+H]$^+$. |
| **Example 48** | 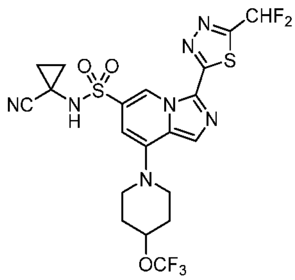<br>**N-(1-cyanocyclopropyl)-3-(5-(difluoromethyl)-1,3,4-thiadiazol-2-yl)-8-(4-(trifluoromethoxy)piperidin-1-yl)imidazo[1,5-a]pyridine-6-sulfonamide** | $^1$H NMR (400 MHz, DMSO-$d_6$) δ 9.66 (s, 1H), 9.48 (s, 1H), 8.05 (s, 1H), 7.82 - 7.54 (m, 1H), 6.69 (s, 1H), 4.80 - 4.71 (m, 1H), 3.63 - 3.56 (m, 2H), 3.26 (d, J = 9.4 Hz, 2H), 2.21 - 2.13 (m, 2H), 2.01 - 1.93 (m, 2H), 1.51 - 1.45 (m, 2H), 1.41 - 1.35 (m, 2H). LC/MS (ESI) (m/z): 564 [M+H]$^+$. |
| **Example 49** | 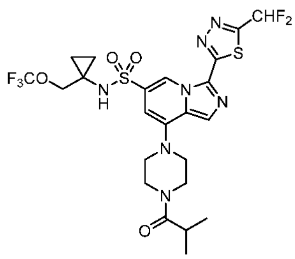<br>**3-(5-(difluoromethyl)-1,3,4-thiadiazol-2-yl)-8-(4-isobutyrylpiperazin-1-yl)-N-(1-((trifluoromethoxy)methyl)cyclopropyl)imidaz o [1,5-a]pyridine-6-sulfonamide** | $^1$H NMR (400 MHz, DMSO-$d_6$) δ 9.59 (s, 1H), 8.88 (s, 1H), 8.07 (s, 1H), 7.82 - 7.54 (m, 1H), 6.68 (s, 1H), 3.95 (s, 2H), 3.80 - 3.71 (m, 4H), 3.29 - 3.24 (m, 4H), 2.99 - 2.91 (m, 1H), 1.05 (d, $J$ = 6.7 Hz, 6H), 0.91 - 0.87 (m, 2H), 0.85 - 0.80 (m, 2H). LC/MS (ESI) (m/z): 624 [M+H]$^+$. |
| **Example 50** | 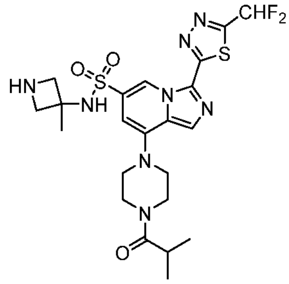<br>**3-(5-(difluoromethyl)-1,3,4-thiadiazol-2-yl)-8-(4-isobutyrylpiperazin-1-yl)-N-(3-methylazetidin-3-yl)imidazo[1,5-a]pyridine-6-sulfonamide** | $^1$H NMR (400 MHz, DMSO-$d_6$) δ 9.59 (s, 1H), 8.27 (s, 1H), 8.07 (s, 1H), 7.81 - 7.54 (m, 1H), 6.72 (s, 1H), 3.80 - 3.73 (m, 6H), 3.33 - 3.23 (m, 6H), 2.98 - 2.92 (m, 1H), 1.46 (s, 3H), 1.05 (d, $J$ = 6.7 Hz, 6H). LC/MS (ESI) (m/z): 555 [M+H]$^+$. |

(continued)

| Example | Structure and name | Analysis data |
|---|---|---|
| **Example 51** | 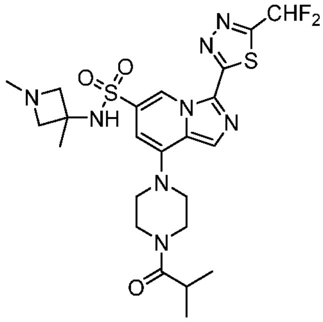 **3-(5-(difluoromethyl)-1,3,4-thiadiazol-2-yl)-N-(1,3-dimethylazetidin-3-yl)-8-(4-isobutyrylpiperazin-1-yl)imidazo[1,5-a]pyridine-6-sulfonamide** | $^1$H NMR (400 MHz, DMSO-*d$_6$*) δ 9.59 (s, 1H), 8.15 (s, 1H), 8.07 (s, 1H), 7.81 - 7.54 (m, 1H), 6.73 (s, 1H), 3.84 - 3.68 (m, 5H), 3.15 - 3.08 (m, 5H), 2.97 - 2.92 (m, 1H), 2.19 (d, *J* = 3.7 Hz, 3H), 1.41 (s, 3H), 1.05 (d, *J* = 6.6 Hz, 6H). LC/MS (ESI) (m/z): 569 [M+H]$^+$. |
| **Example 52** | 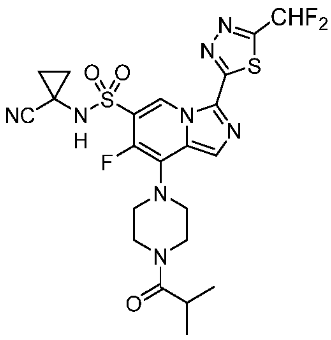 **N-(1-cyanocyclopropyl)-3-(5-(difluoromethyl)-1,3,4-thiadiazol-2-yl)-7-fluoro-8-(4-isobutyrylpiperazin-1-yl)imidazo[1,5-a]pyridine-6-sulfonamide** | $^1$H NMR (400 MHz, DMSO-*d$_6$*) δ 9.72 (d, *J* = 5.1 Hz, 1H), 8.01 (s, 1H), 7.67 (t, 1H), 3.74 - 3.66 (m, 4H), 3.47 - 3.43 (m, 4H), 2.97 - 2.91 (m, 1H), 1.55 - 1.47 (m, 2H), 1.44 - 1.37 (m, 2H), 1.04 (d, *J* = 6.7 Hz, 6H). LC/MS (ESI) (m/z): 569 [M+H]$^+$ |
| **Example 53** | 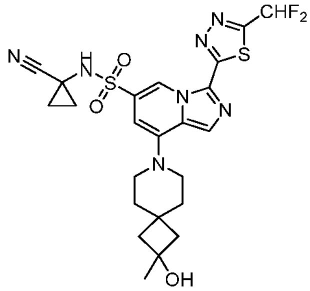 **N-(1-cyanocyclopropyl)-3-(5-(difluoromethyl)-1,3,4-thiadiazol-2-yl)-8-(2-hydroxy-2-methyl-7-azaspiro[3.5]nonan-7-yl)imidazo [1,5-a]pyridine-6-sulfonamide** | $^1$H NMR (400 MHz, DMSO-*d$_6$*) δ 9.64 (s, 1H), 9.44 (s, 1H), 7.97 (s, 1H), 7.61 (d, *J* = 53.0 Hz, 1H), 6.65 (s, 1H), 4.83 (s, 1H), 3.25 (s, 4H), 1.92 (d, *J* = 2.9 Hz, 4H), 1.85 (s, 2H), 1.75 (s, 2H), 1.47 (d, *J* = 4.1 Hz, 2H), 1.39 (d, *J* = 4.3 Hz, 2H), 1.29 (s, 3H). LC/MS (ESI) (m/z): 550 [M+H]$^+$. |

(continued)

| Example | Structure and name | Analysis data |
|---|---|---|
| Example 54 | 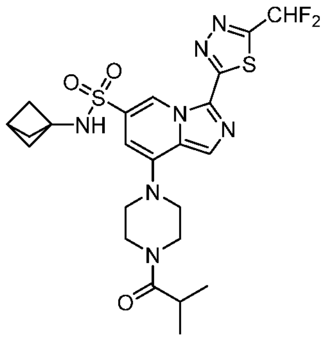<br>**N-(bicyclo[1.1.1]pentan-1-yl)-3-(5-(difluoromethyl)-1,3,4-thiadiazol-2-yl)-8-(4-isobutyrylpiperazin-1-yl)imidazo[1,5-a]pyridine-6-sulfonamide** | $^1$H NMR (400 MHz, $CD_3OD$) δ 9.84 (s, 1H), 7.94 (s, 1H), 7.49 - 7.17 (m, 1H), 6.75 (d, *J* = 1.0 Hz, 1H), 3.89 (d, *J* = 3.7 Hz, 4H), 3.41 (s, 2H), 3.35 (s, 2H), 3.08 - 2.99 (m, 1H), 2.31 (s, 1H), 1.92 (s, 6 H), 1.16 (s, 3H), 1.14 (s, 3H). LC/MS (ESI) (m/z): 552 $[M+H]^+$. |
| Example 55 | 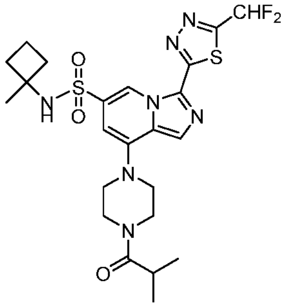<br>**3-(5-(difluoromethyl)-1,3,4-thiadiazol-2-yl)-8-(4-isobutyrylpiperazin-1-yl)-N-(1-methylcyclobutyl)imidazo[1,5-a]pyridine-6-sulfonamide** | $^1$H NMR (400 MHz, DMSO-*$d_6$*) δ 9.60 (s, 1H), 8.21 (s, 1H), 8.07 (s, 1H), 7.68 (t, *J* = 53.2 Hz, 1H), 6.76 (s, 1H), 3.85 - 3.69 (m, 4H), 3.33 - 3.26 (m, 4H), 3.01 - 2.91 (m, 1H), 2.27 - 2.15 (m, 2H), 1.77 - 1.69 (m, 2H), 1.68 - 1.58 (m, 2H), 1.35 (s, 3H), 1.06 (d, *J* = 6.7 Hz, 6H). LC/MS (ESI) (m/z): 553 $[M+H]^+$. |
| Example 56 | 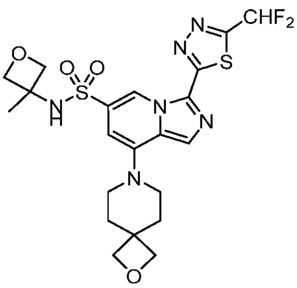<br>**3-(5-(difluoromethyl)-1,3,4-thiadiazol-2-yl)-N-(3-methyloxetan-3-yl)-8-(2-oxa-7-azaspiro[3.5]nonan-7-yl)imidazo[1,5-a]pyridine-6-sulfonamide** | $^1$H NMR (400 MHz, DMSO-*$d_6$*) δ 9.56 (s, 1H), 8.70 (s, 1H), 7.97 (s, 1H), 7.67 (t, *J* = 53.2 Hz, 1H), 6.69 (s, 1H), 4.62 (d, *J* = 6.1 Hz, 2H), 4.41 (s, 4H), 4.18 (d, *J* = 6.3 Hz, 2H), 3.27 - 3.23 (m, 4H), 2.07 - 2.00 (m, 4H), 1.49 (s, 3H). LC/MS (ESI) (m/z): 527 $[M+H]^+$. |
| Example 57 | 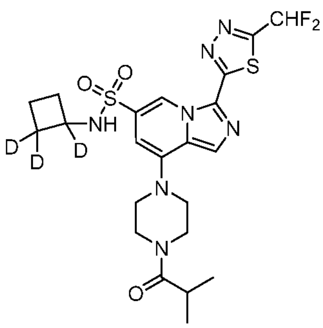<br>**N-(cyclobutyl-1,2,2-d3)-3-(5-(difluoromethyl)-1,3,4-thiadiazol-2-yl)-8-(4-isobutyrylpiperazin-1-yl)imidazo[1,5-a]pyridine-6-sulfonamide** | $^1$H NMR (400 MHz, DMSO-*$d_6$*) δ 9.57 (s, 1H), 8.34 (s, 1H), 8.06 (s, 1H), 7.68 (t, J = 53.2 Hz, 1H), 6.70 (s, 1H), 3.80 - 3.73 (m, 4H), 3.32 - 3.24 (m, 4H), 2.99 - 2.93 (m, 1H), 1.99 - 1.94 (m, 1H), 1.84 - 1.77 (m, 1H), 1.50 - 1.43 (m, 2H), 1.05 (d, *J* = 6.7 Hz, 6H). LC/MS (ESI) (m/z): 543 $[M+H]^+$. |

(continued)

| Example | Structure and name | Analysis data |
|---|---|---|
| **Example 58** | 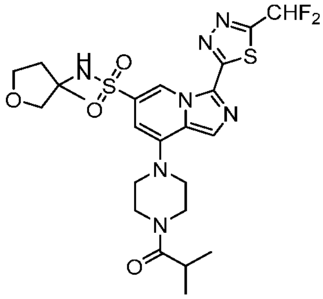 **3-(5-(difluoromethyl)-1,3,4-thiadiazol-2-yl)-8-(4-isobutyrylpiperazin-1-yl)-N-(3-methyltetrahydrofuran-3-yl)imidazo[1,5-a]pyridine-6-sulfonamide** | $^1$H NMR (400 MHz, DMSO-$d_6$) δ 9.61 (s, 1H), 8.25 (s, 1H), 8.06 (s, 1H), 7.67 (t, *J* = 53.2 Hz, 1H), 6.78 (s, 1H), 3.82 -3.68 (m, 8H), 3.32 - 3.26 (m, 4H), 2.98 - 2.92 (m, 1H), 2.22 - 2.15 (m, 1H), 1.81 - 1.73 (m, 1H), 1.27 (s, 3H), 1.05 (d, *J* = 6.7 Hz, 6H). LC/MS (ESI) (m/z): 570 $[M+H]^+$. |
| **Example 59** | 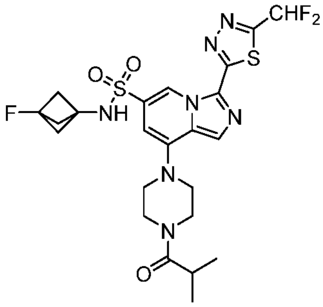 **3-(5-(difluoromethyl)-1,3,4-thiadiazol-2-yl)-N-(3-fluorobicyclo [1.1.1]pentan-1-yl)-8-(4-isobutyrylpiperazin-1-yl)imidazo[1,5-a] pyridine-6-sulfonamide** | $^1$H NMR (400 MHz, $CDCl_3$) δ 9.89 (s, 1H), 7.74 (s, 1H), 7.21 - 6.96 (m, 1H), 6.65 (s, 1H), 5.74 (s, 1H), 3.96 - 3.78 (m, 4H), 3.42 - 3.30 (m, 4H), 2.91 - 2.82 (m, 1H), 2.39 - 2.32 (m, 6H), 1.22 - 1.17 (m, 6H). LC/MS (ESI) (m/z): 570 $[M+H]^+$. |
| **Example 60** | 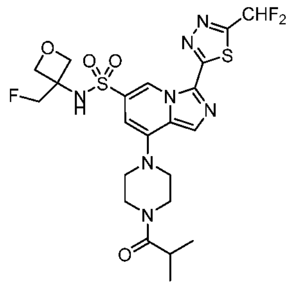 **3-(5-(difluoromethyl)-1,3,4-thiadiazol-2-yl)-N-(3-(fluoromethyl) oxetan-3-yl)-8-(4-isobutyrylpiperazin-1-yl)imidazo[1,5-a]pyridine-6-sulfonamide** | $^1$H NMR (400 MHz, $CD_3OD$) δ 9.85 (s, 1H), 7.94 (s, 1H), 7.46 - 7.20 (m, 2H), 6.79 (s, 1H), 4.81 - 4.78 (m, 4H), 4.48 (d, *J* = 7.0 Hz, 2H), 3.49 - 3.40 (m, 4H), 3.38 - 3.34 (m, 4H), 3.06 - 2.99 (m, 1H), 1.15 *(d, J* = 6.7 Hz, 6H). LC/MS (ESI) (m/z): 574 $[M+H]^+$. |
| **Example 61** | 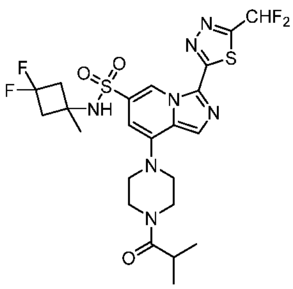 **N-(3,3-difluoro-1-methylcyclobutyl)-3-(5-(difluoromethyl)-1,3,4-thiadiazol-2-yl)-8-(4-isobutyrylpiperazin-1-yl)imidazo[1,5-a]pyridine-6-sulfonamide** | $^1$H NMR (400 MHz, DMSO-$d_6$) δ 9.60 (s, 1H), 8.64 (s, 1H), 8.07 (s, 1H), 7.61 (d, *J* = 53.0 Hz, 1H), 6.71 (s, 1H), 3.76 (d, *J* = 14.6 Hz, 4H), 3.37 - 3.32 (m, 4H), 2.95 - 2.92 (m, 1H), 2.88 - 2.84 (m, 2H), 2.60 - 2.54 (m, 2H), 1.36 (s, 3H), 1.05 *(d, J* = 6.7 Hz, 6H).LC/MS (ESI) (m/z): 590 $[M+H]^+$. |

(continued)

| Example | Structure and name | Analysis data |
|---|---|---|
| Example 62 | 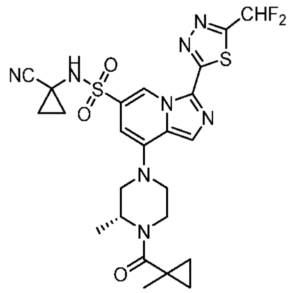 **(R)-N-(1-cyanocyclopropyl)-3-(5-(difluoromethyl)-1,3,4-thiadiazol-2-yl)-8-(3-methyl-4-(1-methylcyclopropane-1-carbonyl)piperazin-1-yl)imidazo[1,5-a]pyridine-6-sulfonamide** | $^1$H NMR (400 MHz, DMSO-*d$_6$*) δ 9.68 (s, 1H), 9.47 (s, 1H), 7.99 (s, 1H), 7.69 (t, *J* = 53.2 Hz, 1H), 6.68 (s, 1H), 4.68 (d, *J* = 6.1 Hz, 1H), 4.27 (d, *J* = 13.4 Hz, 1H), 3.76 - 3.65 (m, 2H), 3.64 - 3.35 (m, 1H), 3.08 - 3.01 (m, 1H), 3.00 - 2.86 (m, 1H), 1.51 - 1.45 (m, 2H), 1.43 - 1.34 (m, 5H), 1.28 (s, 3H), 0.90 - 0.80 (m, 2H), 0.62 - 0.53 (m, 2H). LC/MS (ESI) (m/z): 577 [M+H]$^+$. |
| Example 63 | 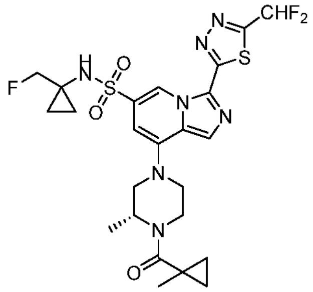 **(R)-3-(5-(difluoromethyl)-1,3,4-thiadiazol-2-yl)-N-(1-(fluoromethyl)cyclopropyl)-8-(3-methyl-4-(1-methylcyclopropane-1-carbonyl)piperazin-1-yl)imidazo[1,5-a]pyridine-6-sulfonamide** | $^1$H NMR (400 MHz, DMSO-*d$_6$*) δ 9.58 (s, 1H), 8.82 (s, 1H), 7.96 (s, 1H), 7.69 (t, *J* = 53.2 Hz, 1H), 6.71 (s, 1H), 4.74 - 4.60 (m, 1H), 4.35 - 4.24 (m, 2H), 4.22 - 4.13 (m, 1H), 3.73 - 3.62 (m, 2H), 3.05 - 2.97 (m, 1H), 2.95 - 2.82 (m, 1H), 1.40 - 1.38 (m, 2H), 1.28 (s, 3H), 1.26 - 1.24 (m, 2H), 0.91 - 0.81 (m, 4H), 0.80 - 0.76 (m, 2H), 0.63 - 0.55 (m, 2H). LC/MS (ESI) (m/z): 584 [M+H]$^+$. |
| Example 64 | 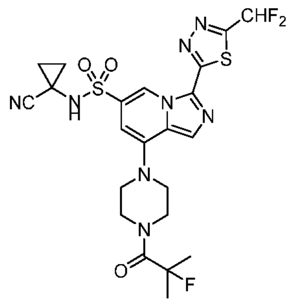 **N-(1-cyanocyclopropyl)-3-(5-(difluoromethyl)-1,3,4-thiadiazol-2-yl)-8-(4-(2-fluoro-2-methylpropanoyl)piperazin-1-yl)imidazo[1,5-a]pyridine-6-sulfonamide** | $^1$H NMR (400 MHz, DMSO-*d$_6$*) δ 9.69 (s, 1H), 9.47 (s, 1H), 8.11 (s, 1H), 7.69 (t, *J* = 53.1 Hz, 1H), 6.69 (s, 1H), 4.01 - 3.75 (m, 4H), 3.41 - 3.37 (m, 4H), 1.63 (s, 3H), 1.57 (s, 3H), 1.50 - 1.46 (m, 2H), 1.41- 1.36 (m, 2H). LC/MS (ESI) (m/z): 568 [M+H]$^+$. |
| Example 65 | 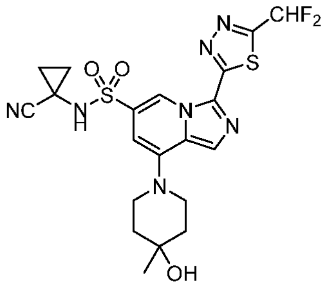 **N-(1-cyanocyclopropyl)-3-(5-(difluoromethyl)-1,3,4-thiadiazol-2-yl)-8-(4-hydroxy-4-methylpiperidin-1-yl)imidazo[1,5-a]pyridine-6-sulfonamide** | $^1$H NMR (400 MHz, DMSO-*d$_6$*) δ 9.63 (s, 1H), 9.45 (s, 1H), 7.99 (s, 1H), 7.68 (t, *J* = 53.2 Hz, 1H), 6.67 (d, *J* = 1.0 Hz, 1H), 4.44 (s, 1H), 3.50 - 3.44 (m, 2H), 3.30 - 3.26 (m, 2H), 1.78 - 1.72 (m, 2H), 1.69 - 1.64 (m, 2H), 1.49 - 1.45 (m, 2H), 1.40 - 1.37 (m, 2H), 1.22 (s, 3H). LC/MS (ESI) (m/z): 510 [M+H]$^+$. |

(continued)

| Example | Structure and name | Analysis data |
|---|---|---|
| Example 66 | 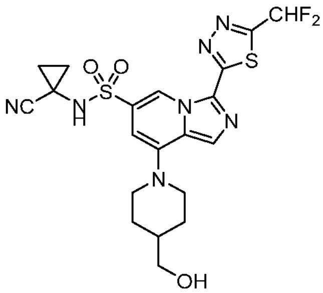 **N-(1-cyanocyclopropyl)-3-(5-(difluoromethyl)-1,3,4-thiadiazol-2-yl)-8-(4-(hydroxymethyl)piperidin-1-yl)imidazo[1,5-a]pyridine-6-sulfonamide** | $^1H$ NMR (400 MHz, DMSO-$d_6$) δ 9.64 (s, 1H), 9.47 (s, 1H), 7.98 (s, 1H), 7.68 (t, *J* = 53.2 Hz, 1H), 6.66 (d, *J* = 0.9 Hz, 1H), 4.57 (t, *J* = 5.2 Hz, 1H), 3.81 (d, *J* = 12.5 Hz, 2H), 3.40 (s, 2H), 2.92 (t, *J* = 10.6 Hz, 2H), 1.86 (d, *J* = 11.2 Hz, 2H), 1.66 (s, 1H), 1.50 - 1.38 (m, 6H). LC/MS (ESI) (m/z): 510 $[M+H]^+$. |
| Example 67 | 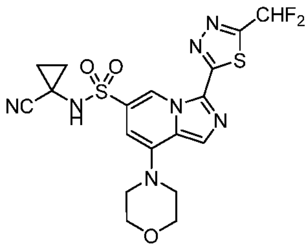 **N-(1-cyanocyclopropyl)-3-(5-(difluoromethyl)-1,3,4-thiadiazol-2-yl)-8-morpholinoimidazo[1,5-a]pyridine-6-sulfonamide** | $^1H$ NMR (400 MHz, DMSO-$d_6$) δ: 9.69 (s, 1H), 9.49 (s, 1H), 8.09 (s, 1H), 7.68 (t, *J* = 53.1 Hz, 1H), 6.68 (s, 1H), 3.89 - 3.84 (m, 4H), 3.31 - 3.29 (m, 4H), 1.48 (t, *J* = 6.8 Hz, 2H), 1.38 (t, *J* = 6.9 Hz, 2H). LC/MS (ESI) (m/z): 482 $[M+H]^+$. |
| Example 68 | 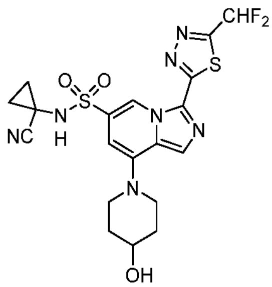 **N-(1-cyanocyclopropyl)-3-(5-(difluoromethyl)-1,3,4-thiadiazol-2-yl)-8-(4-hydroxypiperidin-1-yl)imidazo[1,5-a]pyridine-6-sulfonamide** | $^1H$ NMR (400 MHz, DMSO-$d_6$) δ 9.64 (s, 1H), 9.46 (s, 1H), 7.99 (s, 1H), 7.68 (t, *J* = 53.2 Hz, 1H), 6.66 (d, *J* = 1.2 Hz, 1H), 4.81 (d, *J* = 4.0 Hz, 1H), 3.81 - 3.72 (m, 1H), 3.65 - 3.58 (m, 2H), 3.14 - 3.07 (m, 2H), 1.97 - 1.90 (m, 2H), 1.68 - 1.59 (m, 2H), 1.49 - 1.44 (m, 2H), 1.40 - 1.34 (m, 2H). LC/MS (ESI) (m/z): 496 $[M+H]^+$. |
| Example 69 | 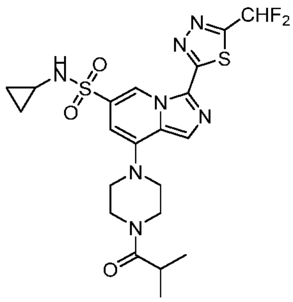 **N-cyclopropyl-3-(5-(difluoromethyl)-1,3,4-thiadiazol-2-yl)-8-(4-isobutyrylpiperazin-1-yl)imidazo[1,5-a]pyridine-6-sulfonamide** | $^1H$ NMR (400 MHz, DMSO-$d_6$) δ 9.62 (s, 1H), 8.26 (s, 1H), 8.08 (s, 1H), 7.68 (t, *J* = 53.2 Hz, 1H), 6.70 (s, 1H), 3.82 - 3.72 (m, 4H), 3.37 - 3.35 (m, 4H), 2.97 - 2.93 (m, 1H), 2.28 - 2.23 (m, 1H), 1.05 *(d, J* = 6.7 Hz, 6H), 0.54 - 0.50 (m, 2H), 0.49 - 0.46 (m, 2H). LC/MS (ESI) (m/z): 526 $[M+H]^+$. |

(continued)

| Example | Structure and name | Analysis data |
|---|---|---|
| Example 70 | 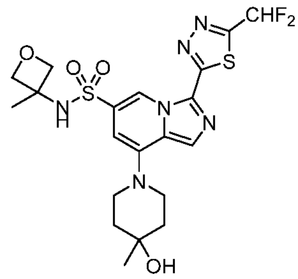 **3-(5-(difluoromethyl)-1,3,4-thiadiazol-2-yl)-8-(4-hydroxy-4-methylpiperidin-1-yl)-N-(3-methyloxetan-3-yl)imidazo[1,5-a]pyridine-6-sulfonamide** | $^1$H NMR (400 MHz, DMSO-$d_6$) δ 9.54 (s, 1H), 8.70 (s, 1H), 7.96 (s, 1H), 7.67 (t, $J$ = 53.2 Hz, 1H), 6.71 (s, 1H), 4.62 (d, $J$ = 6.1 Hz, 2H), 4.46 (s, 1H), 4.18 (d, $J$ = 6.3 Hz, 2H), 3.48 - 3.43 (m, 2H), 3.28 - 3.24 (m, 2H), 1.79 - 1.65 (m, 4H), 1.49 (s, 3H), 1.23 (s, 3H). LC/MS (ESI) (m/z): 515 [M+H]$^+$. |
| Example 71 | 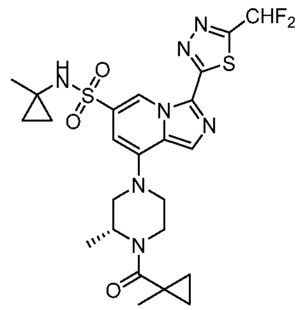 **(R)-3-(5-(difluoromethyl)-1,3,4-thiadiazol-2-yl)-8-(3-methyl-4-(1-methylcyclopropane-1-carbonyl)piperazin-1-yl)-N-(1-methylcyclopropyl)imidazo[1,5-a]pyridine-6-sulfonamide** | $^1$H NMR (400 MHz, DMSO-$d_6$) δ 9.61 (s, 1H), 8.44 (s, 1H), 7.97 (s, 1H), 7.69 (t, $J$ = 53.2 Hz, 1H), 6.71 (s, 1H), 4.69 (dd, $J$ = 6.9, 5.0 Hz, 1H), 4.33 - 4.22 (m, 1H), 3.74 - 3.61 (m, 2H), 3.47 - 3.40 (m, 1H), 3.08 - 2.99 (m, 1H), 2.97 - 2.86 (m, 1H), 1.44 - 1.36 (m, 3H), 1.28 (s, 3H), 1.17 (s, 3H), 0.90 - 0.82 (m, 2H), 0.77 - 0.68 (m, 2H), 0.62 - 0.57 (m, 2H), 0.49 - 0.44 (m, 2H). LC/MS (ESI) (m/z): 566 [M+H]$^+$. |
| Example 72 | 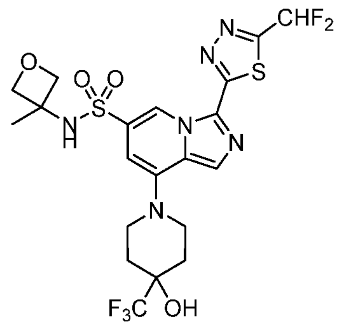 **3-(5-(difluoromethyl)-1,3,4-thiadiazol-2-yl)-8-(4-hydroxy-4-(trifluoromethyl)piperidin-1-yl)-N-(3-methyloxetan-3-yl)imidazo [1,5-a]pyridine-6-sulfonamide** | $^1$H NMR (400 MHz, DMSO-$d_6$) δ 9.58 (s, 1H), 8.73 (s, 1H), 8.06 (s, 1H), 7.68 (t, $J$ = 53.1 Hz, 1H), 6.76 (s, 1H), 6.15 (s, 1H), 4.63 (d, $J$ = 6.2 Hz, 2H), 4.19 (d, $J$ = 6.3 Hz, 2H), 3.72 -3.66 (m, 2H), 3.16 - 3.11 (m, 2H), 2.07 - 1.99 (m, 2H), 1.89 - 1.83 (m, 2H), 1.49 (s, 3H). LC/MS (ESI) m/z: 569 [M+H]$^+$. |
| Example 73 | 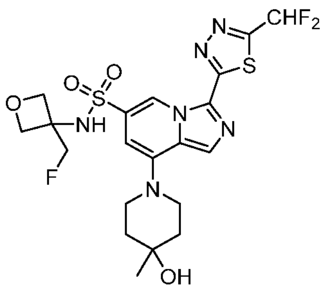 **3-(5-(difluoromethyl)-1,3,4-thiadiazol-2-yl)-N-(3-(fluoromethyl) oxetan-3-yl)-8-(4-hydroxy-4-methylpiperidin-1-yl)imidazo[1,5-a] pyridine-6-sulfonamide** | $^1$H NMR (400 MHz, DMSO-$d_6$) δ 9.55 (s, 1H), 9.04 (s, 1H), 7.96 (s, 1H), 7.67 (t, $J$ = 53.2 Hz, 1H), 6.73 (s, 1H), 4.72 (s, 1H), 4.65 - 4.62 (m, 2H), 4.60 (s, 1H), 4.45 (s, 1H), 4.38 (d, $J$ = 6.9 Hz, 2H), 3.48 - 3.43 (m, 2H), 3.30 - 3.22 (m, 2H), 1.80 - 1.72 (m, 2H), 1.70 - 1.64 (m, 2H), 1.22 (s, 3H). LC/MS (ESI) (m/z): 533 [M+H]$^+$. |

(continued)

| Example | Structure and name | Analysis data |
|---|---|---|
| **Example 74** | 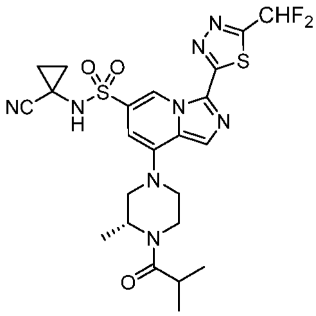 **(R)-N-(1-cyanocyclopropyl)-3-(5-(difluoromethyl)-1,3,4-thiadiazol-2-yl)-8-(4-isobutyryl-3-methylpiperazin-1-yl)imidazo[1,5-a]pyridine-6-sulfonamide** | $^1$H NMR (400 MHz, DMSO-$d_6$) δ 9.67 (s, 1H), 9.48 (s, 1H), 8.00 (s, 1H), 7.69 (t, *J* = 53.2 Hz, 1H), 6.66 (s, 1H), 4.77 - 4.38 (m, 1H), 3.74 - 3.66 (m, 2H), 3.37 - 3.34 (m, 2H), 3.16 - 2.99 (m, 2H), 2.94 - 2.89 (m, 1H), 1.50 - 1.47 (m, 2H), 1.40 - 1.37 (m, 2H), 1.33 - 1.22 (m, 3H), 1.05 (d, *J* = 6.6 Hz, 6H). LC/MS (ESI) (m/z): 565 $[M+H]^+$. |
| **Example 75** | 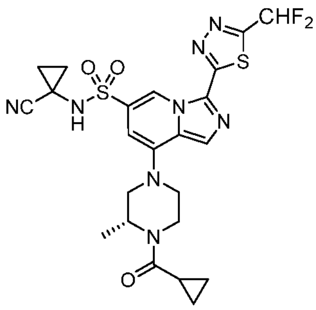 **(R)-N-(1-cyanocyclopropyl)-8-(4-(cyclopropanecarbonyl)-3-methylpiperazin-1-yl)-3-(5-(difluoromethyl)-1,3,4-thiadiazol-2-yl)imidazo[1,5-a]pyridine-6-sulfonamide** | $^1$H NMR (400 MHz, DMSO-$d_6$) δ 9.68 (s, 1H), 9.49 (s, 1H), 8.02 (s, 1H), 7.69 (t, *J* = 53.1 Hz, 1H), 6.67 (s, 1H), 4.76 - 4.66 (m, 1H), 4.30 - 4.27 (m, 1H), 3.77 - 3.74 (m, 3H), 3.07 - 2.87 (m, 2H), 1.49 - 1.39 (m, 8H), 0.86 - 0.78 (m, 4H). LC/MS (ESI) (m/z): 563 $[M+H]^+$. |
| **Example 76** | 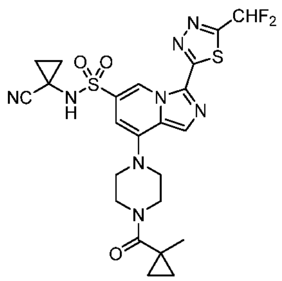 **N-(1-cyanocyclopropyl)-3-(5-(difluoromethyl)-1,3,4-thiadiazol-2-yl)-8-(4-(1-methylcyclopropane-1-carbonyl)piperazin-1-yl)imidazo[1,5-a]pyridine-6-sulfonamide** | $^1$H NMR (400 MHz, DMSO-$d_6$) δ 9.69 (s, 1H), 9.47 (s, 1H), 8.11 (s, 1H), 7.82 - 7.53 (m, 1H), 6.70 (s, 1H), 3.82 (s, 4H), 3.42 - 3.39 (m, 2H), 3.32 - 3.28 (m, 2H), 1.53 - 1.47 (m, 2H), 1.42 - 1.37 (m, 2H), 1.29 (s, 3H), 0.88 - 0.85 (m, 2H), 0.61 - 0.57 (m, 2H). LC/MS (ESI) m/z: 563 $[M+H]^+$. |
| **Example 77** | 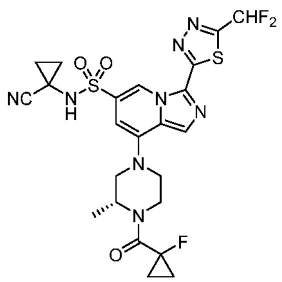 **(R)-N-(1-cyanocyclopropyl)-3-(5-(difluoromethyl)-1,3,4-thiadiazol-2-yl)-8-(4-(1-fluorocyclopropane-1-carbonyl)-3-methylpiperazin-1-yl)imidazo[1,5-a]pyridine-6-sulfonamide** | $^1$H NMR (400 MHz, DMSO-$d_6$) δ 9.69 (s, 1H), 9.47 (s, 1H), 8.02 (s, 1H), 7.69 (t, *J* = 53.2 Hz, 1H), 6.69 (s, 1H), 4.72 - 4.61 (m, 1H), 4.33 - 4.18 (m, 1H), 3.84 - 3.71 (m, 2H), 3.50 - 3.41 (m, 1H), 3.18 - 3.12 (m, 1H), 3.04 - 2.93 (m, 1H), 1.53 - 1.42 (m, 5H), 1.41 - 1.37 (m, 2H), 1.35 - 1.32 (m, 1H), 1.30 - 1.20 (m, 3H). LC/MS (ESI) m/z: 581 $[M+H]^+$. |

(continued)

| Example | Structure and name | Analysis data |
|---|---|---|
| **Example 78** | 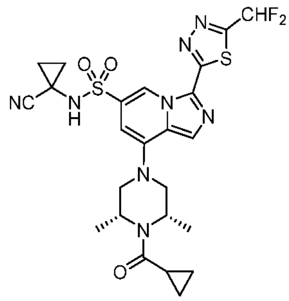 **N-(1-cyanocyclopropyl)-8-((3R,5S)-4-(cyclopropanecarbonyl)-3,5-dimethylpiperazin-1-yl)-3-(5-(difluoromethyl)-1,3,4-thiadiazol-2-yl)imidazo[1,5-a]pyridine-6-sulfonamide** | $^1$H NMR (400 MHz, DMSO-*d$_6$*) δ 9.71 (s, 1H), 9.52 (s, 1H), 7.91 (s, 1H), 7.70 (t, *J* = 53.1 Hz, 1H), 6.75 (s, 1H), 4.66 (s, 2H), 3.68 (d, *J* = 11.9 Hz, 2H), 3.16 - 2.96 (m, 2H), 2.06 - 1.94 (m, 1H), 1.56 - 1.32 (m, 10H), 0.85 - 0.73 (m, 4H). LC/MS (ESI) (m/z): 577 [M+H]$^+$. |
| **Example 79** | 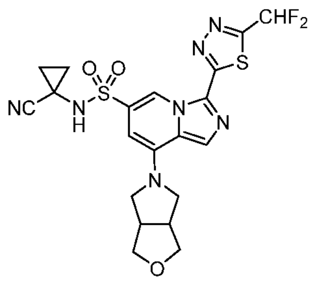 **N-(1-cyanocyclopropyl)-3-(5-(difluoromethyl)-1,3,4-thiadiazol-2-yl)-8-(tetrahydro-1H-furo[3,4-c]pyrrol-5(3H)-yl)imidazo[1,5-a] pyridine-6-sulfonamide** | $^1$H NMR (400 MHz, DMSO-*d$_6$*) δ 9.49 (s, 1H), 9.40 (s, 1H), 8.24 (s, 1H), 7.68 (t, *J* = 53.1 Hz, 1H), 6.18 (s, 1H), 3.96 - 3.82 (m, 4H), 3.72 (d, *J* = 8.9 Hz, 2H), 3.60 (d, *J* = 9.4 Hz, 2H), 3.15 (s, 2H), 1.51 - 1.44 (m, 2H), 1.40 - 1.32 (m, 2H). LC/MS (ESI) (m/z): 508 [M+H]$^+$. |
| **Example 80** | 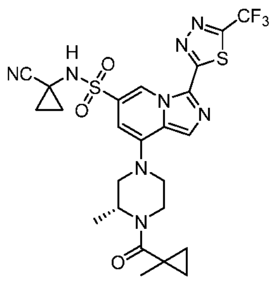 **(R)-N-(1-cyanocyclopropyl)-8-(3-methyl-4-(1-methylcyclopropane-1-carbonyl)piperazin-1-yl)-3-(5-(trifluoromethyl)-1,3,4-thiadiazol-2-yl)imidazo[1,5-a]pyridine-6-sulfonamide** | $^1$H NMR (400 MHz, DMSO-*d$_6$*) δ 9.66 (s, 1H), 9.51 (s, 1H), 8.04 (s, 1H), 6.72 (s, 1H), 4.69 (s, 1H), 4.27 (d, *J* = 13.1 Hz, 1H), 3.71 (dd, *J* = 26.3, 11.2 Hz, 2H), 3.57 - 3.45 (m, 1H), 3.05 (d, *J* = 10.4 Hz, 1H), 2.99 - 2.89 (m, 1H), 1.49 (s, 2H), 1.39 (s, 5H), 1.28 (s, 3H), 0.90 - 0.81 (m, 2H), 0.61 - 0.55 (m, 2H).LC/MS (ESI) (m/z): 595 [M+H]$^+$. |
| **Example 81** | 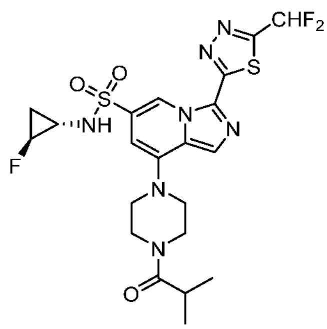 **3-(5-(difluoromethyl)-1,3,4-thiadiazol-2-yl)-N-((1S,2S)-2-fluorocyclopropyl)-8-(4-isobutyrylpiperazin-1-yl)imidazo[1,5-a]pyridine-6-sulfonamide** | $^1$H NMR (400 MHz, DMSO-*d$_6$*) δ 9.63 (s, 1H), 8.45 (s, 1H), 8.09 (s, 1H), 7.68 (t, *J* = 53.2 Hz 1H), 6.67 (s, 1H), 4.70 (d, *J* = 60.3 Hz, 1H), 3.80 - 3.73 (m, 4H), 3.42 - 3.36 (m, 4H), 2.98 - 2.92 (m, 1H), 2.74 - 2.67 (m, 1H), 1.30 - 1.19 (m, 1H), 1.05 (d, *J* = 6.5 Hz, 6H), 1.03 - 0.90 (m, 1H). LC/MS (ESI) (m/z): 544 [M+H]$^+$. |

(continued)

| Example | Structure and name | Analysis data |
|---|---|---|
| **Example 82** | 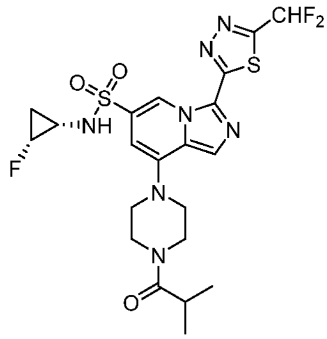<br>**3-(5-(difluoromethyl)-1,3,4-thiadiazol-2-yl)-N-((1S,2R)-2-fluorocyclopropyl)-8-(4-isobutyrylpiperazin-1-yl)imidazo[1,5-a]pyridine-6-sulfonamide** | $^1$H NMR (400 MHz, DMSO-$d_6$) δ 9.63 (s, 1H), 8.51 (s, 1H), 8.08 (s, 1H), 7.69 (t, *J* = 53.1 Hz, 1H), 6.74 (s, 1H), 4.70 - 4.49 (m, 1H), 3.76 (d, *J* = 16.0 Hz, 4H), 3.32 - 3.27 (m, 4H), 2.99 - 2.91 (m, 1H), 2.36 - 2.30 (m, 1H), 1.26 - 1.22 (m, 1H), 1.05 (d, *J* = 6.5 Hz, 6H), 0.99 - 0.92 (m, 1H). LC/MS (ESI) (m/z): 544 $[M+H]^+$. |
| **Example 83** | 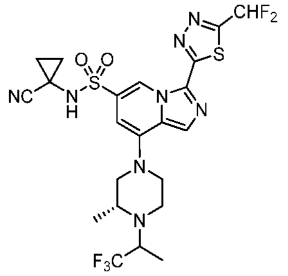<br>**N-(1-cyanocyclopropyl)-3-(5-(difluoromethyl)-1,3,4-thiadiazol-2-yl)-8-((3R)-3-methyl-4-(1,1,1-trifluoropropan-2-yl)piperazin-1-yl) imidazo[1,5-a]pyridine-6-sulfonamide** | $^1$H NMR (400 MHz, DMSO-$d_6$) δ 9.66 (s, 1H), 9.46 (s, 1H), 8.04 (s, 1H), 7.68 (t, *J* = 53.1 Hz, 1H), 6.65 (s, 1H), 3.89 - 3.80 (m, 1H), 3.78 - 3.72 (m, 1H), 3.65 - 3.59 (m, 1H), 2.98 - 2.90 (m, 3H), 2.85 - 2.78 (m, 1H), 2.75 - 2.67 (m, 1H), 1.51 - 1.45 (m, 2H), 1.42 - 1.36 (m, 2H), 1.22 - 1.18 (m, 3H), 1.16 - 1.12 (m, 3H). LC/MS (ESI) (m/z): 591 $[M+H]^+$. |
| **Example 84** | 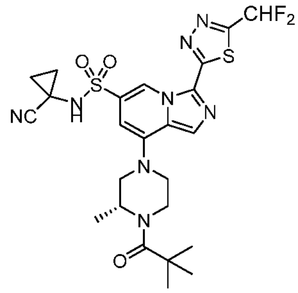<br>**(R)-N-(1-cyanocyclopropyl)-3-(5-(difluoromethyl)-1,3,4-thiadiazol-2-yl)-8-(3-methyl-4-pivaloylpiperazin-1-yl)imidazo[1,5-a]pyridine-6-sulfonamide** | $^1$H NMR (400 MHz, DMSO-$d_6$) δ 9.68 (s, 1H), 9.48 (s, 1H), 7.98 (s, 1H), 7.69 (t, *J* = 53.2 Hz, 1H), 6.67 (s, 1H), 4.72 (s, 1H), 4.27 (d, *J* = 12.7 Hz, 1H), 3.74 - 3.64 (m, 2H), 3.48 (s, 1H), 3.04 (d, *J* = 11.2 Hz, 1H), 2.95 - 2.85 (m, 1H), 1.53 - 1.49 (m, 2H), 1.46 - 1.39 (m, 5H), 1.25 (s, 9H). LC/MS (ESI) (m/z): 579 $[M+H]^+$. |
| **Example 85** | 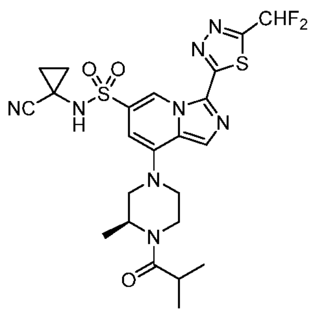<br>**(S)-N-(1-cyanocyclopropyl)-3-(5-(difluoromethyl)-1,3,4-thiadiazol-2-yl)-8-(4-isobutyryl-3-methylpiperazin-1-yl)imidazo[1,5-a]pyridine-6-sulfonamide** | $^1$H NMR (400 MHz, DMSO-$d_6$) δ 9.67 (s, 1H), 9.53 (s, 1H), 7.99 (s, 1H), 7.68 (t, *J* = 53.3 Hz, 1H), 6.66 (s, 1H), 4.77 - 4.37 (m, 1H), 4.00 - 3.61 (m, 3H), 3.25 - 2.70 (m, 4H), 1.49 - 1.47 (m, 3H), 1.41 - 1.25 (m, 4H), 1.05 (d, *J* = 6.4 Hz, 6H). LC/MS (ESI) (m/z): 565 $[M+H]^+$. |

(continued)

| Example | Structure and name | Analysis data |
|---|---|---|
| Example 86 | 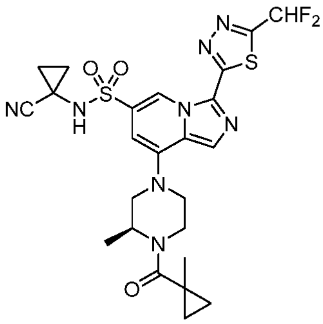 **(S)-N-(1-cyanocyclopropyl)-3-(5-(difluoromethyl)-1,3,4-thiadiazol-2-yl)-8-(3-methyl-4-(1-methylcyclopropane-1-carbonyl)piperazin-1-yl)imidazo[1,5-a]pyridine-6-sulfonamide** | $^1$H NMR (400 MHz, DMSO-$d_6$) δ 9.68 (s, 1H), 9.47 (s, 1H), 7.99 (s, 1H), 7.68 (t, *J* = 53.0 Hz, 1H), 6.68 (s, 1H), 4.77 - 4.62 (m, 1H), 4.34 - 4.19 (m, 1H), 3.79 - 3.63 (m, 2H), 3.61 - 3.36 (m, 1H), 3.07 - 3.00 (m, 1H), 2.98 - 2.87 (m, 1H), 1.53 - 1.46 (m, 2H), 1.43 - 1.34 (m, 5H), 1.28 (s, 3H), 0.90 - 0.79 (m, 2H), 0.62 - 0.55 (m, 2H). LC/MS (ESI) (m/z): 577 [M+H]$^+$. |
| Example 87 | 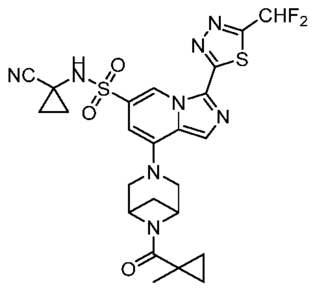 **N-(1-cyanocyclopropyl)-3-(5-(difluoromethyl)-1,3,4-thiadiazol-2-yl)-8-(6-(1-methylcyclopropane-1-carbonyl)-3,6-diazabicyclo[3.1.1]heptan-3-yl)imidazo[1,5-a]pyridine-6-sulfonamide** | $^1$H NMR (400 MHz, DMSO-$d_6$) δ 9.55 (s, 1H), 9.40 (s, 1H), 8.38 (s, 1H), 7.68 (t, *J* = 53.3 Hz, 1H), 6.28 (s, 1H), 5.12 - 4.40 (m, 2H), 4.23 (s, 2H), 3.90 (s, 2H), 2.74 (d, *J* = 6.9 Hz, 1H), 1.69 (d, *J* = 8.6 Hz, 1H), 1.47 (s, 2H), 1.37 (s, 2H), 1.34 (s, 4H), 1.25 (d, *J* = 12.4 Hz, 1H), 0.57-0.50 (m, 2H). LC/MS (ESI) m/z: 575 [M+H]$^+$. |
| Example 88 | 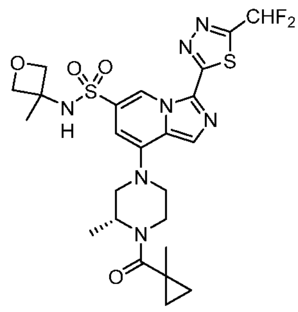 **(R)-3-(5-(difluoromethyl)-1,3,4-thiadiazol-2-yl)-8-(3-methyl-4-(1-methylcyclopropane-1-carbonyl)piperazin-1-yl)-N-(3-methyloxetan-3-yl)imidazo[1,5-a]pyridine-6-sulfonamide** | $^1$H NMR (400 MHz, DMSO-$d_6$) δ 9.59 (s, 1H), 8.71 (s, 1H), 7.96 (s, 1H), 7.67 (t, *J* = 52.9 Hz, 1H), 6.71 (s, 1H), 4.78 - 4.50 (m, 3H), 4.32 - 4.07 (m, 3H), 3.78 - 3.37 (m, 3H), 3.07 - 2.87 (m, 2H), 1.50 - 1.22 (m, 9H), 0.91 - 0.78 (m, 2H), 0.66 - 0.49 (m, 2H). LC/MS (ESI) (m/z): 582 [M+H]$^+$. |
| Example 89 | 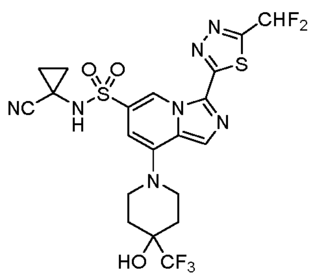 **N-(1-cyanocyclopropyl)-3-(5-(difluoromethyl)-1,3,4-thiadiazol-2-yl)-8-(4-hydroxy-4-(trifluoromethyl)piperidin-1-yl)imidazo[1,5-a]pyridine-6-sulfonamide** | $^1$H NMR (400 MHz, DMSO-$d_6$) δ 9.67 (s, 1H), 9.47 (s, 1H), 8.08 (s, 1H), 7.68 (t, *J* = 53.1 Hz, 1H), 6.72 (s, 1H), 6.13 (s, 1H), 3.70 (d, *J* = 11.2 Hz, 2H), 3.20 - 3.12 (m, 2H), 2.08 - 2.00 (m, 2H), 1.85 (d, *J* = 13.1 Hz, 2H), 1.51 - 1.45 (m, 2H), 1.42 - 1.35 (m, 2H). LC/MS (ESI) (m/z): 564 [M+H]$^+$. |

(continued)

| Example | Structure and name | Analysis data |
|---|---|---|
| **Example 90** | 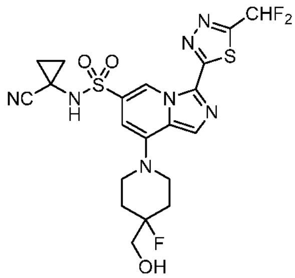 **N-(1-cyanocyclopropyl)-3-(5-(difluoromethyl)-1,3,4-thiadiazol-2-yl)-8-(4-fluoro-4-(hydroxymethyl)piperidin-1-yl)imidazo[1,5-a]pyridine-6-sulfonamide** | $^1$H NMR (400 MHz, DMSO-$d_6$) δ 9.66 (s, 1H), 9.49 (s, 1H), 8.05 (s, 1H), 7.69 (t, $J$ = 53.1 Hz, 1H), 6.72 (s, 1H), 5.16 - 5.06 (m, 1H), 3.66 (d, $J$ = 11.4 Hz, 2H), 3.56 - 3.48 (m, 2H), 3.21 - 3.12 (m, 2H), 2.02 - 1.88 (m, 4H), 1.54 - 1.46 (m, 2H), 1.42 - 1.35 (m, 2H). LC/MS (ESI) (m/z): 528 [M+H]$^+$. |
| **Example 91** | 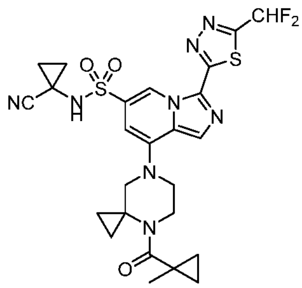 **N-(1-cyanocyclopropyl)-3-(5-(difluoromethyl)-1,3,4-thiadiazol-2-yl)-8-(4-(1-methylcyclopropane-1-carbonyl)-4,7-diazaspiro[2.5]octan-7-yl)imidazo[1,5-a]pyridine-6-sulfonamide** | $^1$H NMR (400 MHz, DMSO-$d_6$) δ 9.66 (s, 1H), 9.42 (s, 1H), 7.99 (s, 1H), 7.68 (t, $J$ = 53.1 Hz, 1H), 6.67 (s, 1H), 4.01 - 3.95 (m, 2H), 3.46 - 3.43 (m, 2H), 3.22 - 3.20 (m, 2H), 1.49 - 1.44 (m, 2H), 1.41 - 1.35 (m, 2H), 1.27 - 1.24 (m, 5H), 1.02 - 0.99 (m, 2H), 0.91 - 0.89 (m, 2H), 0.58 - 0.54 (m, 2H). LC/MS (ESI) m/z: 589 [M+H]$^+$. |
| **Example 92** | 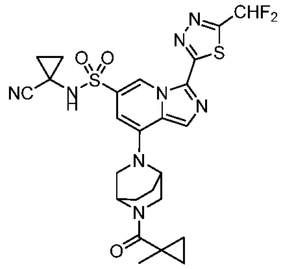 **N-(1-cyanocyclopropyl)-3-(5-(difluoromethyl)-1,3,4-thiadiazol-2-yl)-8-(5-(1-methylcyclopropane-1-carbonyl)-2,5-diazabicyclo[2.2.2]octan-2-yl)imidazo[1,5-a]pyridine-6-sulfonamide** | $^1$H NMR (400 MHz, DMSO-$d_6$) δ 9.52 (s, 1H), 9.40 (s, 1H), 8.14 (s, 1H), 7.74 (t, $J$ = 54.0 Hz, 1H), 6.40 (s, 1H), 4.59 (s, 1H), 4.36 (s, 1H), 4.06 (d, $J$ = 7.2 Hz, 1H), 3.88 (d, $J$ = 9.3 Hz, 1H), 3.63 (d, $J$ = 9.1 Hz, 1H), 3.51 (d, $J$ = 14.8 Hz, 1H), 2.27 - 2.20 (m, 1H), 2.05 (s, 1H), 1.95 - 1.87 (m, 2H), 1.47 (s, 2H), 1.39 (s, 2H), 1.27 (s, 3H), 0.88 - 0.85 (m, 2H), 0.58 - 0.56 (m, 2H)。LC/MS (ESI) (m/z): 589 [M+H]$^+$. |
| **Example 93** | 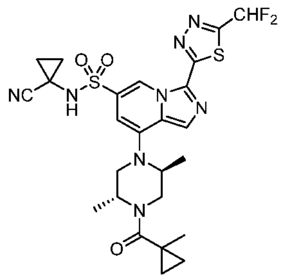 **N-(1-cyanocyclopropyl)-3-(5-(difluoromethyl)-1,3,4-thiadiazol-2-yl)-8-((2S,5R)-2,5-dimethyl-4-(1-methylcyclopropane-1-carbonyl)piperazin-1-yl)imidazo[1,5-a]pyridine-6-sulfonamide** | $^1$H NMR (400 MHz, DMSO-$d_6$) δ 9.68 (s, 1H), 9.47 (s, 1H), 8.14 (s, 1H), 7.68 (t, $J$ = 53.3 Hz, 1H), 6.62 (s, 1H), 4.80 - 4.66 (m, 1H), 4.42 - 4.30 (m, 1H), 4.14 - 3.99 (m, 1H), 3.58 - 3.51 (m, 1H), 3.40 - 3.34 (m, 1H), 3.19 - 3.12 (m, 1H), 1.53 - 1.22 (m, 10H), 0.97 (s, 3H), 0.88 - 0.81 (m, 2H), 0.64 - 0.55 (m, 2H). LC/MS (ESI) (m/z): 591 [M+H]$^+$. |

(continued)

| Example | Structure and name | Analysis data |
|---|---|---|
| **Example 94** | 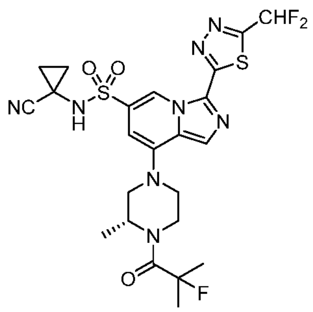 **(R)-N-(1-cyanocyclopropyl)-3-(5-(difluoromethyl)-1,3,4-thiadiazol-2-yl)-8-(4-(2-fluoro-2-methylpropanoyl)-3-methylpiperazin-1-yl)imidazo[1,5-a]pyridine-6-sulfonamide** | $^1$H NMR (400 MHz, DMSO-*d*$_6$) δ 9.68 (s, 1H), 9.49 (s, 1H), 8.01 (s, 1H), 7.76 (d, *J* = 53.1 Hz, 1H), 6.67 (s, 1H), 4.77 - 4.71 (m, 1H), 4.39 - 4.27 (m, 1H), 3.81 - 3.67 (m, 3H), 3.14 - 3.10 (m, 1H), 3.04 - 2.89 (m, 1H), 1.64 - 1.61 (m, 3H), 1.58 - 1.55 (m, 3H), 1.52 - 1.47 (m, 3H), 1.40 - 1.36 (m, 4H). LC/MS (ESI) (m/z): 583 [M+H]$^+$. |
| **Example 95** | 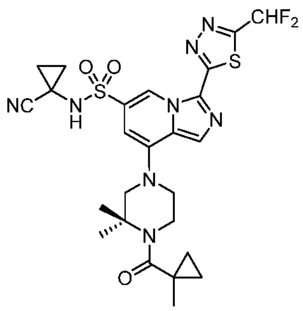 **N-(1-cyanocyclopropyl)-3-(5-(difluoromethyl)-1,3,4-thiadiazol-2-yl)-8-(3,3-dimethyl-4-(1-methylcyclopropane-1-carbonyl)piperazin-1-yl)imidazo[1,5-a]pyridine-6-sulfonamide** | $^1$H NMR (400 MHz, DMSO-*d*$_6$) δ 9.56 (s, 1H), 9.39 (s, 1H), 8.18 (s, 1H), 7.68 (t, *J* = 52.7 Hz, 1H), 6.43 (s, 1H), 4.10 - 4.06 (m, 2H), 3.85 - 3.81 (m, 2H), 3.67 - 3.64 (m, 2H), 1.50 - 1.47 (m, 2H), 1.45 (s, 6H), 1.39 - 1.36 (m, 2H), 1.27 (s, 3H), 0.88 - 0.85 (m, 2H), 0.58 - 0.55 (m, 2H). LC/MS (ESI) (m/z): 591 [M+H]$^+$. |
| **Example 96** | 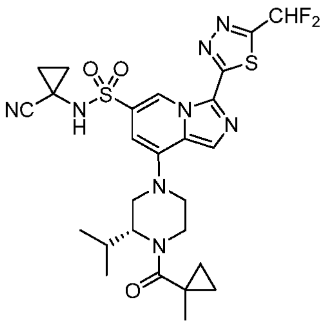 **(R)-N-(1-cyanocyclopropyl)-3-(5-(difluoromethyl)-1,3,4-thiadiazol-2-yl)-8-(3-isopropyl-4-(1-methylcyclopropane-1-carbonyl)piperazin-1-yl)imidazo[1,5-a]pyridine-6-sulfonamide** | $^1$H NMR (400 MHz, DMSO-*d*$_6$) δ: 9.69 (s, 1H), 9.47 (s, 1H), 8.02 (s, 1H), 7.69 (t, *J* = 53.3 Hz, 1H), 6.75 (s, 1H), 4.34 - 4.21 (m, 2H), 3.90 - 3.83 (m, 1H), 3.79 - 3.71 (m, 1H), 3.69 - 3.58 (m, 1H), 2.98 - 2.88 (m, 2H), 2.41 - 2.32 (m, 1H), 1.51 - 1.47 (m, 2H), 1.43 - 1.38 (m, 2H), 1.30 (s, 3H), 1.24 - 1.22 (m, 1H), 0.99 - 0.94 (m, 3H), 0.87 - 0.83 (m, 2H), 0.80 - 0.77 (m, 2H), 0.67 - 0.62 (m, 1H), 0.60 - 0.55 (m, 1H). LC/MS (ESI) (m/z): 605 [M+H]$^+$. |

(continued)

| Example | Structure and name | Analysis data |
|---|---|---|
| **Example 97** | 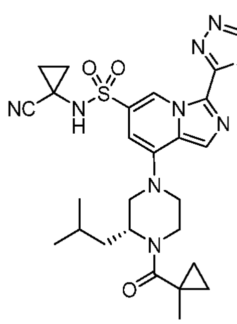 **(R)-N-(1-cyanocyclopropyl)-3-(5-(difluoromethyl)-1,3,4-thiadiazol-2-yl)-8-(3-isobutyl-4-(1-methylcyclopropane-1-carbonyl)piperazin-1-yl)imidazo[1,5-a]pyridine-6-sulfonamide** | $^1$H NMR (400 MHz, DMSO-$d_6$) δ 9.68 (s, 1H), 9.48 (s, 1H), 7.97 (s, 1H), 7.69 (t, $J$ = 53.2 Hz, 1H), 6.70 (s, 1H), 4.70 (s, 1H), 4.33 - 4.23 (m, 1H), 3.76 - 3.65 (m, 3H), 3.03 - 2.92 (m, 2H), 2.02 - 1.89 (m, 1H), 1.56 - 1.35 (m, 7H), 1.30 - 1.26 (m, 2H), 0.96 - 0.89 (m, 6H), 0.87 - 0.83 (m, 2H), 0.67 - 0.61 (m, 1H), 0.59 - 0.52 (m, 1H). LC/MS (ESI) (m/z): 619 [M+H]$^+$. |

**Example 98: 3-chloro-N-(1-cyanocyclopropyl)-1-(5-(difluoromethyl)-1,3,4-thiadiazol-2-yl)-5-(4-isobutyrylpiperazin-1-yl)imidazo[1,5-a]pyridine-7-sulfonamide**

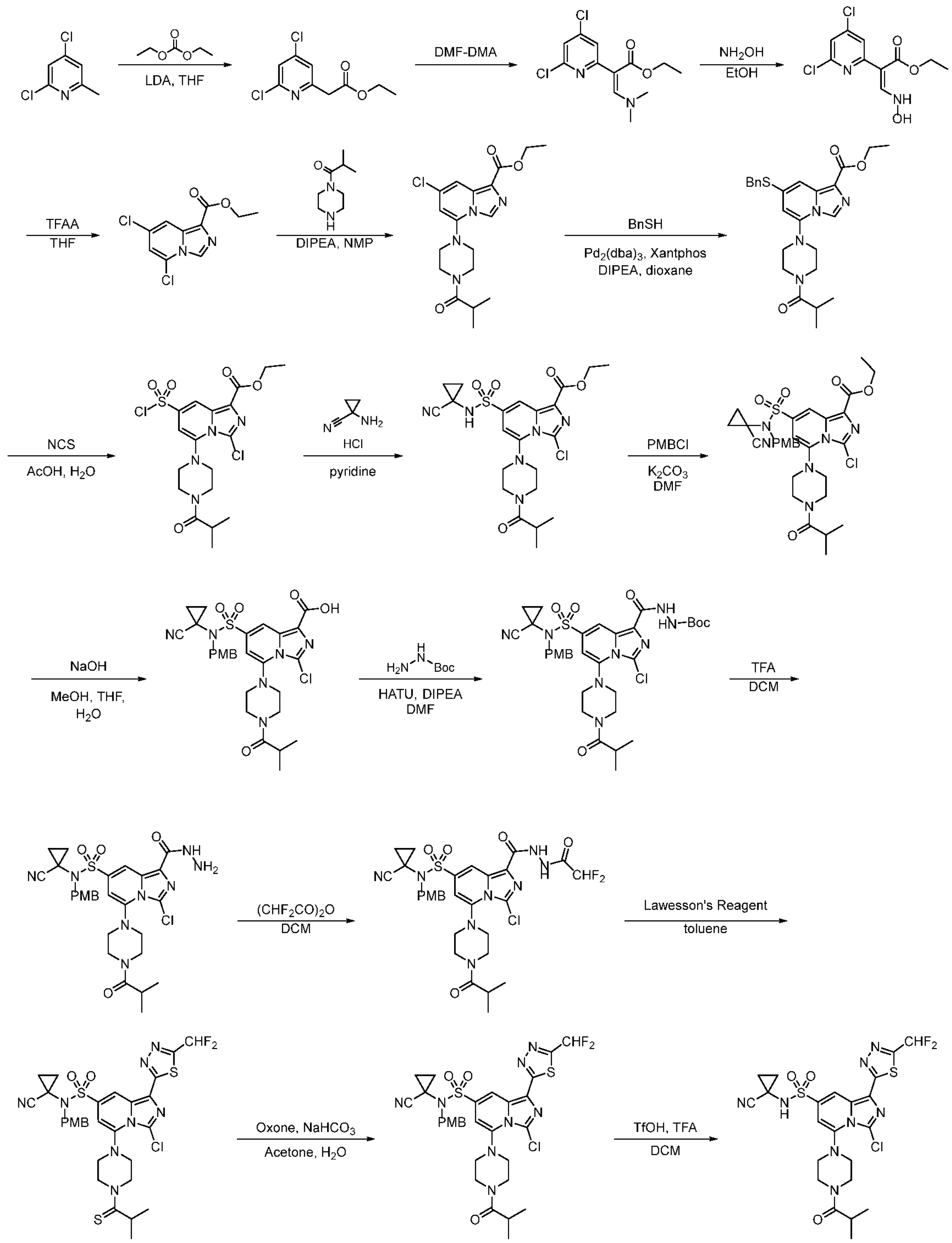


**Step 1: Preparation of ethyl 2-(4,6-dichloropyridin-2-yl)acetate**

At -78°C, the lithium bis(trimethylsilyl)amide solution (210 mL, 210 mmol, 1M in THF) was dropwise added slowly to the solution of 2,4-dichloro-6-methylpyridine (17 g, 104.9 mmol) in tetrahydrofuran (170 mL). The reaction mixture was stirred at -78°C for 30 minutes; and then, dimethyl carbonate (25.4 mL, 209.8 mmol) was dropwise added. After dropwise addition, the reaction mixture was stirred at -78°C for one hour. The saturated ammonium chloride solution was dropwise added slowly to quench the reaction mixture, which was then extracted with ethyl acetate for liquid separation. The organic phase was washed saturated sodium chloride solution, dried over anhydrous sodium sulfate, filtered and concentrated under reduced pressure. The residue was purified by column chromatography (eluent: petroleum ether/ethyl acetate, gradient: 0-30% ethyl acetate) to obtain the title compound (yellow oil, 24 g, yield: 92.8%). $^1H$ NMR (400 MHz, $CDCl_3$) $\delta$ 7.28 (d, $J$ = 6.1 Hz, 2H), 4.24 - 4.17 (m, 2H), 3.80 (s, 2H), 1.28 (t, $J$ = 7.1 Hz, 3H). LC/MS (ESI) (m/z): 234 $[M+H]^+$.

**Step 2: Preparation of ethyl (Z)-2-(4,6-dichloropyridin-2-yl)-3-(dimethylamino)acrylate**

2-(4,6-dichloropyridin-2-yl) ethyl acetate (25 g, 0.427 mmol) was dissolved in N,N-dimethylformamide dimethylacetal (100 mL); and the reaction mixture was heated and stirred at 80°C for 12 hours. After completion of the reaction, the reaction mixture was concentrated under reduced pressure to obtain a crude title compound (brown-yellow solid, 30 g, yield: 97.2%), which was directly used in the next step of reaction. LC/MS (ESI) (m/z): 289 $[M+H]^+$.

**Step 3: Preparation of ethyl (Z)-2-(4,6-dichloropyridin-2-yl)-3-(hydroxyamino)acrylate**

Hydroxylamine hydrochloride (14.4 g, 207.5 mmol) was added to the solution of (Z)-2(4,6-dichloropyridin-2-yl)-3-(dimethylamino)ethyl acrylate (30 g, 103.8 mmol) in tetrahydrofuran (300 mL) and methanol (60 mL). The reaction mixture was stirred at room temperature for 1 hours. After completion of the reaction, the reaction mixture was diluted with ethyl acetate, sequentially washed with water and saturated brine, dried over anhydrous sodium sulfate, filtered and concentrated under reduced pressure to obtain the title compound (yellow oil, 25 g, yield: 86.9%). LC/MS (ESI) m/z: 277 $[M+H]^+$.

**Step 4: Preparation of ethyl 5,7-dichloroimidazo[1,5-a]pyridine-1-carboxylate**

At 0°C, trifluoroacetic anhydride (9.47 g, 45.1 mmol) was dropwise added to the solution of (Z)-2-(4,6-dichloropyridin-2-yl)-3-(hydroxylamino)ethyl acrylate (12.5 g, 45.1 mmol) in tetrahydrofuran (125 mL). The reaction mixture was stirred overnight at room temperature. After completion of the reaction, the reaction mixture was diluted with ethyl acetate, sequentially washed with saturated sodium bicarbonate solution and saturated brine, dried over anhydrous sodium sulfate, filtered and concentrated under reduced pressure. The residue was purified by column chromatography (eluent: petroleum ether/ethyl acetate, gradient: 0-50% ethyl acetate) to obtain the title compound (yellow solid, 5 g, yield: 42.8%). $^1H$ NMR (400 MHz, $CDCl_3$) δ 8.48 (s, 1H), 8.17 (d, *J* = 2.1 Hz, 1H), 7.10 (d, *J* = 2.1 Hz, 1H), 4.40 (q, *J* = 7.1 Hz, 2H), 1.42 (t, *J* = 7.1 Hz, 3H). LC/MS (ESI) m/z: 259 $[M+H]^+$.

**Step 5: Preparation of ethyl 7-chloro-5-(4-isobutyrylpiperazin-1-yl)imidazo[1,5-a]pyridine-1-carboxylate**

1-(2-methylacetonyl)piperazine (3.01 g, 19.3 mmol) and N,N-diisopropylethylamine (9.6 mL, 57.9 mmol) were sequentially added to the solution of 5,7-dichloroimidazol[1,5a]pyridin-1-ethyl formate (5 g, 19.3 mmol) in N-methyl pyrrolidone (50 mL). The reaction mixture was stirred at 120°C for 2 hours. After completion of the reaction, the reaction mixture was cooled to room temperature, diluted with ethyl acetate, sequentially washed with water and saturated brine, dried over anhydrous sodium sulfate, filtered and concentrated under reduced pressure. The residue was purified by column chromatography (eluent: petroleum ether/ethyl acetate, gradient: 0-50% ethyl acetate) to obtain the title compound (4 g, white solid, yield: 54.72%). $^1H$ NMR (400 MHz, $CDCl_3$) δ 8.39 (s, 1H), 7.88 (d, *J* = 2.1 Hz, 1H), 6.30 (d, *J* = 2.1 Hz, 1H), 4.39 (q, *J* = 7.1 Hz, 2H), 3.92 (br, 2H), 3.81 (br, 2H), 3.50 (br, 2H), 3.40 (br, 2H), 2.90 - 2.80 (m, 1H), 1.42 (t, *J* = 7.1 Hz, 3H), 1.18 (d, *J* = 6.8 Hz, 6H). LC/MS (ESI) m/z: 379 $[M+H]^+$.

**Step 6: Preparation of ethyl 7-(benzylthio)-5-(4-isobutyrylpiperazin-1-yl)imidazo[1,5-a]pyridine-1-carboxylate**

N,N-diisopropylethylamine (2.3 mL, 13.8 mmol), 4,5-biphenylphosphino-9, 9-dimethylxanthene (0.53 g, 0.92 mmol), tris(dibenzylideneacetone)dipalladium (0.85 g, 0.92 mmol) were sequentially added to the solution of 7-chloro-5-(4-isobutyrylpiperazin-1-yl)imidazo[1,5a]pyridin-1-ethyl formate (3.5 g, 9.2 mmol) and benzyl mercaptane (1.6 mL, 13.8 mmol) in 1,4-dioxane (35 mL). The flask was replaced with nitrogen gas three times and then the mixture was stirred at 115°C for 16 hours under an atmosphere of nitrogen. After completion of the reaction, the reaction mixture was cooled to room temperature, diluted with ethyl acetate, sequentially washed with water and saturated brine, dried over anhydrous sodium sulfate, filtered and concentrated under reduced pressure. The residue was purified by column chromatography (eluent: petroleum ether/ethyl acetate, gradient: 0-50% ethyl acetate) to obtain the title compound (yellow oil, 3.5 g, yield: 81.4%).LC/MS (ESI) m/z: 467 $[M+H]^+$.

**Step 7: Preparation of ethyl 3-chloro-7-(chlorosulfonyl)-5-(4-isobutyrylpiperazin-1-yl)imidazo[1,5-a]pyridine-1-carboxylate**

At 0°C, water (2 mL) was added to the solution of 7-(benzylthio)-5-(4-isobutyrylpiperazin-1-yl)imidazo[1,5a]pyridin-1-ethyl formate (1 g, 2.1 mmol) in glacial acetic acid (6 mL); and then, N-chlorosuccinimide (1.7 g, 12.6 mmol) was added portionwise. The reaction mixture was stirred at 0°C for 30 minutes. After completion of the reaction, the reaction mixture was diluted with dichloromethane, washed with saturated brine, dried over anhydrous sodium sulfate, and

concentrated under reduced pressure to obtain the title compound (yellow oil, 1 g, yield: 97.8%). LC/MS (ESI) m/z: 477 $[M+H]^+$.

**Step 8: Preparation of ethyl 3-chloro-7-(N-(1-cyanocyclopropyl)sulfamoyl)-5-(4-isobutyrylpiperazin-1-yl)imidazo[1,5-a]pyridine-1-carboxylate**

At 0°C, 3-chloro-7-(sulfonyl chloride)-5-(4-isobutyrylpiperazin-1-yl)imidazo[1,5a]pyridin-1-ethyl formate (500 mg, 4.2 mmol) was added portionwise to the solution of 1-amino-1-cyclopropyl cyanic hydrochloride (1 g, 2.1 mmol) in pyridine (5 mL). The reaction mixture was stirred at room temperature for 30 minutes. After completion of the reaction, ice water was added for quenching; the reaction mixture was extracted with ethyl acetate; and the organic phase was washed with saturated brine, dried over anhydrous sodium sulfate, and concentrated under reduced pressure. The residue was purified by column chromatography (eluent: ethyl acetate/petroleum ether, gradient: 0-50% ethyl acetate) to obtain the title compound (yellow solid, 680 mg, yield: 62.1%). LC/MS (ESI) m/z: 523 $[M+H]^+$.

**Step 9: Preparation of ethyl 3-chloro-7-(N-(1-cyanocyclopropyl)-N-(4-methoxybenzyl)sulfamoyl)-5-(4-isobutyrylpiperazin-1-yl)imidazo[1,5-a]pyridine-1-carboxylate**

Potassium carbonate (539 mg, 3.9 mmol) and p-methoxybenzyl chloride (305 mg, 2.0 mmol) were sequentially added to the solution of 3-chloro-7-(N-(1-cyanocyclopropyl)sulfamoyl)-5-(4-isobutyrylpiperazin-1-yl)imidazo[1,5a]pyridin-1-ethyl formate (680 mg, 1.3 mmol) in N,N-dimethylformamide (7 mL). The reaction mixture was stirred at 45°C for 2 hours. After completion of the reaction, the reaction mixture was cooled to room temperature, diluted with ethyl acetate, sequentially washed with water and saturated brine, dried over anhydrous sodium sulfate, filtered and concentrated under reduced pressure. The residue was purified by silica gel chromatography (eluent: petroleum ether/ethyl acetate, gradient: 0-50% ethyl acetate) to obtain the title compound (yellow solid, 700 mg, yield: 83.7%).LC/MS (ESI) m/z: 643 $[M+H]^+$.

**Step 10: Preparation of 3-chloro-7-(N-(1-cyanocyclopropyl)-N-(4-methoxybenzyl)sulfamoyl)-5-(4-isobutyrylpiperazin-1-yl)imidazo[1,5-a]pyridine-1-carboxylic acid**

At 0°C, sodium hydroxide (43.5 mg, 1.1 mmol) was added to the mixed solution of 3-chloro-7-(N-(1-cyanocyclopropyl)-N-(4-methoxybenzyl)sulfamoyl)-5-(4-isobutyrylpiperazin-1-yl)imidazo[1,5a]pyridin-1-ethyl formate (350 mg, 0.54 mmol) in methanol (2 mL), tetrahydrofuran (1 mL) and water (1 mL); and the reaction mixture was stirred at 45°C for 2 hours. After completion of the reaction, the reaction mixture was cooled to room temperature, diluted with water, acidified to pH-5 with 1N hydrochloric acid, and extracted with ethyl acetate twice. The organic phase was combined, dried over anhydrous sodium sulfate, and concentrated under reduced pressure to obtain the title compound (yellow solid, 320 mg, yield: 97.0%).LC/MS (ESI) m/z: 615 $[M+H]^+$.

**Step 11: Preparation of tert-butyl 2-(3-chloro-7-(N-(1-cyanocyclopropyl)-N-(4-methoxybenzyl)sulfamoyl)-5-(4-isobutyrylpiperazin-1-yl)imidazo[1,5-a]pyridine-1-carbonyl)hydrazine-1-carboxylate**

Diisopropylethylamine (202 mg, 1.56 mmol) and 2-(7-azobenzotriazol)-N,N,N',N'-tetramethylurea hexafluorophosphate (218 mg, 0.57 mmol) were sequentially added to the solution of 3-chloro-7-(N-(1-cyanocyclopropyl)-N-(4-methoxybenzyl)sulfamoyl)-5-(4-isobutyrylpiperazin-1-yl)imidazo[1,5a]pyridin-1-formic acid (320 mg, 0.52 mmol) and tert-butyl hydrazinoformate (76 mg, 0.57 mmol) in N,N-dimethylformamide (4 mL). The reaction mixture was stirred at room temperature for 30 minutes. After completion of the reaction, the reaction mixture was diluted with ethyl acetate, sequentially washed with water and saturated brine, dried over anhydrous sodium sulfate, filtered and concentrated under reduced pressure. The residue was purified by column chromatography (eluent: petroleum ether/ethyl acetate, gradient: 0-50% ethyl acetate) to obtain the title compound (yellow solid, 330 mg, yield: 86.8%).LC/MS (ESI) m/z: 729 $[M+H]^+$.

**Step 12: 3-chloro-N-(1-cyanocyclopropyl)-1-(hydrazinecarbonyl)-5-(4-isobutyrylpiperazin-1-yl)-N-(4-methoxybenzyl)imidazo[1,5-a]pyridine-7-sulfonamide**

At 0°C, trifluoroacetic acid (1.5 mL) was dropwise added to the solution of 2-(3-chloro-7-(N-(1-cyanocyclopropyl)-N-(4-methoxybenzyl)sulfamoyl)-5-(4-isobutyrylpiperazin-1-yl)imidazo[1,5a]pyridin-1-carbonyl)hydrazin-1-tert-butyl formate (330 mg, 0.45 mmol) in dichloromethane (4 mL). The reaction mixture was stirred at room temperature for 30 minutes. After completion of the reaction, the reaction mixture was concentrated under reduced pressure to obtain a crude title compound (yellow oil, 285 mg, yield: 100%), which was directly used in the next step of reaction. LC/MS (ESI) m/z: 629 $[M+H]^+$.

**Step 13: Preparation of 3-chloro-N-(1-cyanocyclopropyl)-1-(2-(2,2-difluoroacetyl)hydrazine-1-carbonyl)-5-(4-isobutyrylpiperazin-1-yl)-N-(4-methoxybenzyl)imidazo[1,5-a]pyridine-7-sulfonamide**

At 0°C, difluoroacetic anhydride (79 mg, 0.45 mmol) was dropwise added to the solution of 3-chloro-N-(1-cyanocyclopropyl)-1-(hydrazinocarbonyl)-5-(4-isobutyrylpiperazin-1-yl)-N-(4-methoxybenzyl)imidazo[1,5-a]pyridin-7-sulfonamide (285 mg, 0.45 mmol) in dichloromethane (3 mL). The reaction mixture was stirred at room temperature for 30 minutes. After completion of the reaction, the reaction mixture was diluted with dichloromethane, sequentially washed with saturated sodium bicarbonate solution and saturated brine, dried over anhydrous sodium sulfate, filtered and concentrated under reduced pressure. The residue was purified by column chromatography (eluent: petroleum ether/ethyl acetate, gradient: 0-50% ethyl acetate) to obtain the title compound (yellow solid, 240 mg, yield: 75%). LC/MS (ESI) m/z: 707 $[M+H]^+$.

**Step 14: Preparation of 3-chloro-N-(1-cyanocyclopropyl)-1-(5-(difluoromethyl)-1,3,4-thiadiazol-2-yl)-N-(4-methoxybenzyl)-5-(4-(2-methylpropanethioyl)piperazin-1-yl)imidazo[1,5-a]pyridine-7-sulfonamide**

The Lawesson's reagent (151 mg, 0.37 mmol) was added to the solution of 3-chloro-N-(1-cyanocyclopropyl)-1-(2-(2,2-difluoroacetyl)hydrazin-1-carbonyl)-5-(4-isobutyrylpiperazin-1-yl)-N-(4-methoxybenzyl)imidazo[1,5-a] pyridin-7-sulfonamide (240 mg, 0.34 mmol) in toluene (3 mL). The flask was replaced with nitrogen gas three times and then the mixture was stirred at 110°C for 4 hours under an atmosphere of nitrogen. After completion of the reaction, the reaction mixture was cooled to room temperature, diluted with ethyl acetate, sequentially washed with water and saturated brine, dried over anhydrous sodium sulfate, filtered and concentrated under reduced pressure. The residue was purified by column chromatography (eluent: petroleum ether/ethyl acetate, gradient: 0-45% ethyl acetate) to obtain the title compound (yellow solid, 125 mg, yield: 71.0%). LC/MS (ESI) m/z: 721 $[M+H]^+$.

**Step 15: 3-chloro-N-(1-cyanocyclopropyl)-1-(5-(difluoromethyl)-1,3,4-thiadiazol-2-yl)-5-(4-isobutyrylpiperazin-1-yl)-N-(4-methoxybenzyl)imidazo[1,5-a]pyridine-7-sulfonamide**

At 0°C, water (0.2 mL), sodium bicarbonate (80 mg, 0.95 mmol) and Oxone (70 mg, 0.12 mmol) were sequentially added to the solution of 3-chloro-N-(1-cyanocyclopropyl)-1-(5-(difluoromethyl)-1,3,4-thiadiazol-2-yl)-N-(4-methoxybenzyl)-5-(4-(2-methylpropylthio)piperazin-1-yl)imidazo[1,5-a]pyridin-7-sulfonamide (180 mg, 0.25 mmol) in acetone (2 mL). The reaction mixture was stirred at room temperature for 2 hours. After completion of the reaction, the reaction mixture was diluted with ethyl acetate, washed with water and saturated brine respectively, dried over anhydrous sodium sulfate, filtered and concentrated under reduced pressure to obtain the title compound (yellow solid, 125 mg, yield: 71.0%).LC/MS (ESI) m/z: 705 $[M+H]^+$.

**Step 16: Preparation of 3-chloro-N-(1-cyanocyclopropyl)-1-(5-(difluoromethyl)-1,3,4-thiadiazol-2-yl)-5-(4-isobutyrylpiperazin-1-yl)imidazo[1,5-a]pyridine-7-sulfonamide**

At 0°C, trifluoroacetic acid (0.5 mL) and trifluoromethanesulfonic acid (0.1 mL) were sequentially added to the solution of 3-chloro-N-(1-cyanocyclopropyl)-1-(5-(difluoromethyl)-1,3,4-thiadiazol-2-yl)-5-(4-isobutyrylpiperazin-1-yl)-N-(4-methoxybenzyl)imidazo[1,5-a]pyridin-7-sulfonamide (40 mg, 0.057 mmol) in dichloromethane (0.5 mL); and then, the reaction mixture was stirred at room temperature for 30 minutes. After completion of the reaction, the mixture was concentrated to dryness, and the residue was purified by pre-HPLC to obtain the title compound (white solid, 5.5 mg, yield: 16.6%). $^1H$ NMR (400 MHz, DMSO-$d_6$) δ 9.74 (s, 1H), 9.01 (s, 1H), 8.81 (s, 1H), 7.67 (t, $J$ = 53.3 Hz, 1H), 3.83 - 3.38 (m, 8H), 3.00 - 2.91 (m, 1H), 1.50 - 1.45 (m, 2H), 1.37 - 1.31 (m, 2H), 1.05 (d, $J$ = 6.7 Hz, 6H). LC/MS (ESI) (m/z): 585 $[M+H]^+$.

**Example 99: 3-(5-(difluoromethyl)-1,3,4-thiadiazol-2-yl)-8-(4-isobutyrylpiperazin-1-yl)-N-(1-methylcyclopropyl)-[1,2,4]triazolo[4,3-a]pyridine-6-sulfonamide**

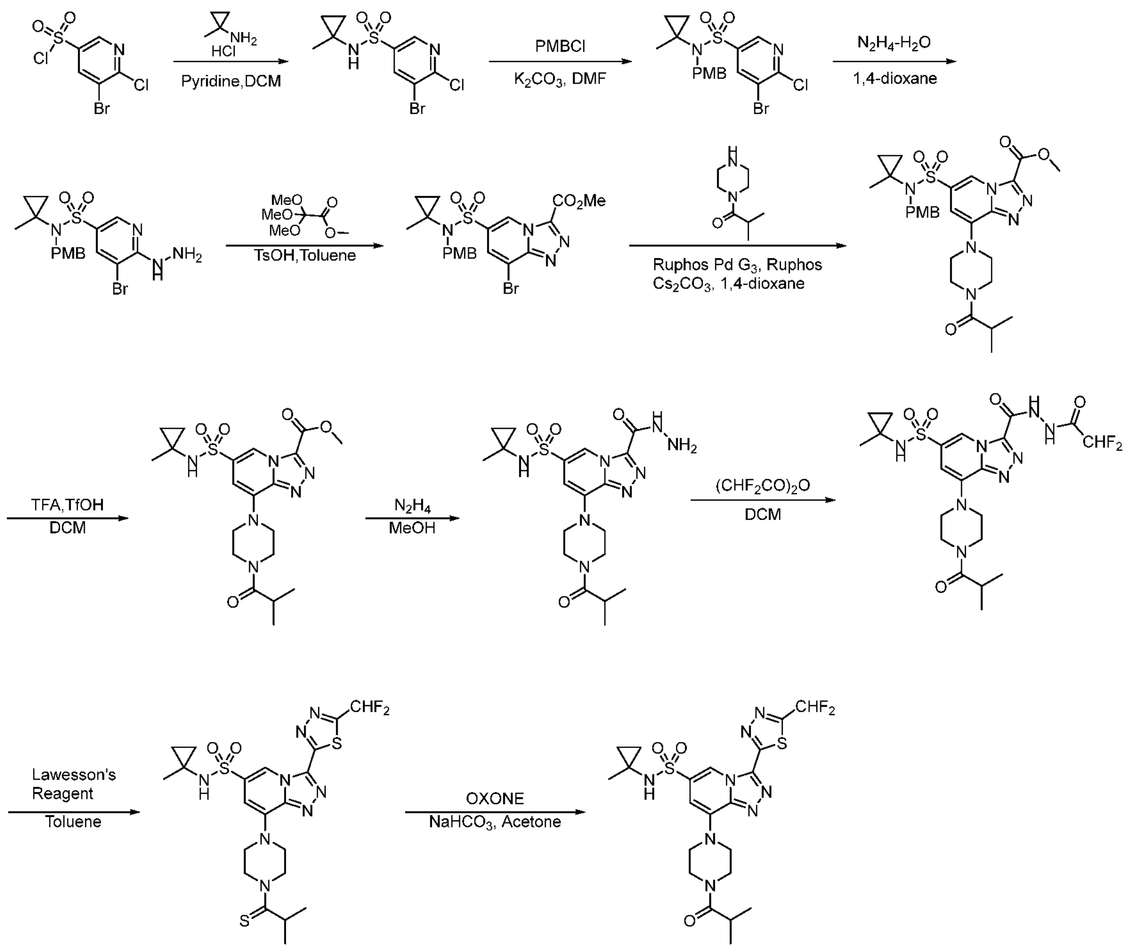


**Step 1: 5-bromo-6-chloro-N-(1-methylcyclopropyl)pyridine-3-sulfonamide**

At 0°C, the solution of 5-bromo-6-chloropyridin-3-sulfonyl chloride (1.5 g, 5.2 mmol) in dichloromethane (5 mL) was dropwise added slowly to the solution of 1-methylcyclopropan-1-amine (1 g, 10 mmol) in pyridine (10 mL). The reaction mixture was stirred at room temperature for 1 hours. After completion of the reaction, the reaction mixture was diluted with dichloromethane, washed with saturated brine, dried over anhydrous sodium sulfate, and concentrated under reduced pressure to obtain the title compound (white solid, 1.2 g, yield: 71.5%). $^1$H NMR (400 MHz, $CDCl_3$) δ 8.71 (s, 1H), 8.29 (s, 1H), 4.99 (s, 1H), 1.25 (s, 3H), 0.75 - 0.68 (m, 2H), 0.56 - 0.47 (m, 2H). LC/MS (ESI) m/z: 326 $[M+H]^+$.

**Step 2: 5-bromo-6-chloro-N-(4-methoxybenzyl)-N-(1-methylcyclopropyl)pyridine-3-sulfonamide**

Potassium carbonate (1.0 g, 7.4 mmol) and 1-(chloromethyl)-4-metoxybenzene (0.9 g, 5.7 mmol) were sequentially added to the solution of 5-bromo-6-chloro-N-(1-methylcyclopropyl)pyridin-3-sulfonamide (1.2 g, 3.7 mmol) in N,N-dimethylformamide (10 mL). The reaction mixture was stirred at 50°C for 2 hours. The mixture was diluted with ethyl acetate, sequentially washed with water and saturated brine, dried over anhydrous sodium sulfate, filtered and concentrated under reduced pressure. The residue was purified by silica gel chromatography (eluent: petroleum ether/ethyl acetate, gradient: 0-20% ethyl acetate) to obtain the title compound (white solid, 1.4 g, yield: 85.2%). $^1$H NMR (400 MHz, $CDCl_3$) δ 8.46 (s, 1H), 7.69 (s, 1H), 7.06 (d, *J* = 7.7 Hz, 2H), 6.71 (d, *J* = 7.8 Hz, 2H), 4.39 (s, 2H), 3.73 (s, 3H), 1.24 (s, 3H), 0.89 - 0.82 (m, 2H), 0.68 - 0.61 (m, 2H). LC/MS (ESI) m/z: 446 $[M+H]^+$.

**Step 3: 5-bromo-6-hydrazineyl-N-(4-methoxybenzyl)-N-(1-methylcyclopropyl)pyridine-3-sulfonamide**

Hydrazine hydrate (0.6 g, 12.0 mmol) was dropwise added slowly to the solution of 5-bromo-6-chloro-N-(4-methoxybenzyl)-N-(1-methylcyclopropyl)pyridin-3-sulfonamide (1.3 g, 2.9 mmol) in 1,4-dioxane (13 mL). The reaction mixture was stirred at 50°C for 2 hours. The mixture was diluted with dichloromethane, sequentially washed with water and saturated brine, dried over anhydrous sodium sulfate, filtered and concentrated under reduced pressure to obtain the title compound (colorless oil, 0.9 g, yield: 69.9%). LC/MS (ESI) m/z: 441 $[M+H]^+$.

**Step 4: Methyl 8-bromo-6-(N-(4-methoxybenzyl)-N-(1-methylcyclopropyl)sulfamoyl)-[1,2,4]triazolo[4,3-a]pyridine-3-carboxylate**

2,2-trimethoxymethyl acetate (1.7 g, 10 mmol) and p-toluenesulfonic acid (0.4 g, 0.2 mmol) were sequentially added to the solution of 5-bromo-6-hydrazino-N-(4-methoxybenzyl)-N-(1-methylcyclopropyl)pyridin-3-sulfonamide (0.9 g, 2.0 mmol) in toluene (15 mL). The reaction mixture was stirred at 85°C for 2 hours. The mixture was diluted with ethyl acetate, sequentially washed with water and saturated brine, dried over anhydrous sodium sulfate, filtered and concentrated under reduced pressure. The residue was purified by silica gel chromatography (eluent: petroleum ether/ethyl acetate, gradient: 0-50% ethyl acetate) to obtain the title compound (yellow solid, 870 mg, yield: 83.8%). $^1$H NMR (400 MHz, $CDCl_3$) δ 9.10 (s, 1H), 7.39 (s, 1H), 7.00 (d, $J$ = 8.1 Hz, 2H), 6.55 (d, $J$ = 8.1 Hz, 2H), 4.44 (s, 2H), 4.07 (s, 3H), 3.56 (s, 3H), 1.35 (s, 3H), 0.90 - 0.76 (m, 2H), 0.61 - 0.47 (m, 2H). LC/MS (ESI) m/z: 509 $[M+H]^+$.

**Step 5: Methyl 8-(4-isobutyrylpiperazin-1-yl)-6-(N-(4-methoxybenzyl)-N-(1-methylcyclopropyl)sulfamoyl)-[1,2,4]triazolo[4,3-a]pyridine-3-carboxylate**

Cesium carbonate (260 mg, 0.8 mmol), RuPhos (25 mg, 0.05 mmol) and RuPhos Pd G3 (25 mg, 0.03 mmol) were sequentially added to the solution of 8-bromo-6-(N-(4-methoxybenzyl)-N-(1-methylcyclopropyl)sulfamoyl)-[1,2,4]triazolo [4,3-a]pyridin-3-methyl carboxylate (135 mg, 0.26 mmol) and 2-methyl-1-(piperazin-1-yl)propan-1-one (60 mg, 0.4 mmol) in 1,4-dioxane (5 mL). The reaction mixture was stirred at 110°C for 3 hours under an atmosphere of nitrogen. The reaction mixture was diluted with ethyl acetate, sequentially washed with water and saturated brine, dried over anhydrous sodium sulfate, filtered and concentrated under reduced pressure. The residue was purified by column chromatography (eluent: petroleum ether/ethyl acetate, gradient: 0-100% ethyl acetate) to obtain the title compound (yellow solid, 79 mg, yield: 52.9%). LC/MS (ESI) m/z: 585 $[M+H]^+$.

**Step 6: Methyl 8-(4-isobutyrylpiperazin-1-yl)-6-(N-(1-methylcyclopropyl)sulfamoyl)-[1,2,4]triazolo[4,3-a]pyridine-3-carboxylate**

At 0°C, trifluoroacetic acid (70 mg, 0.6 mmol) and trifluoromethanesulfonic acid (30 mg, 0.2 mmol) were sequentially added to the solution of 8-(4-isobutylpiperazin-1-yl)-6-(N-(4-methoxybenzyl)-N-(1-methylcyclopropyl)sulfamoyl)-[1,2,4]triazolo[4,3-a]pyridin-3-methyl carboxylate (70 mg, 0.12mmol) in dichloromethane (1 mL); and then, the reaction mixture was stirred at room temperature for 30 minutes. The reaction mixture was alkalinized to pH-7 with saturated sodium bicarbonate solution, and extracted with ethyl acetate twice. The organic phase was combined, dried over anhydrous sodium sulfate, and concentrated under reduced pressure to obtain the title compound (yellow solid, 45 mg, yield: 80%). LC/MS (ESI) (m/z): 465 $[M+H]^+$.

**Step 7: 3-(hydrazinecarbonyl)-8-(4-isobutyrylpiperazin-1-yl)-N-(1-methylcyclopropyl)-[1,2,4]triazolo[4,3-a]pyridine-6-sulfonamide**

Hydrazine hydrate (56 mg, 1.0 mmol) was dropwise added slowly to the solution of 8-(4-isobutylpiperazin-1-yl)-6-(N-(1-methylcyclopropyl)sulfamoyl)-[1,2,4]triazolo[4,3-a]pyridin-3-methyl carboxylate (45 mg, 0.1mmol) in methanol (1 mL); and then, the reaction mixture was stirred at room temperature for 30 minutes. After completion of the reaction, the reaction mixture was concentrated under reduced pressure to obtain the title compound (yellow solid, 40 mg, yield: 88.9%). LC/MS (ESI) (m/z): 465 $[M+H]^+$.

**Step 8: 3-(2-(2,2-difluoroacetyl)hydrazine-1-carbonyl)-8-(4-isobutyrylpiperazin-1-yl)-N-(1-methylcyclopropyl)-[1,2,4]triazolo[4,3-a]pyridine-6-sulfonamide**

At 0°C, difluoroacetic anhydride (18 mg, 0.11 mmol) was slowly added to the solution of 3-(hydrazinocarbonyl)-8-(4-isobutylpiperazin-1-yl)-N-(1-methylcyclopropyl)-[1,2,4]triazolo[4,3-a]pyridin-6-sulfonamide (40 mg, 0.9 mmol) in dichloromethane (1 mL). The reaction mixture was stirred at room temperature for 30 minutes. After completion of the reaction, the reaction mixture was diluted with dichloromethane, washed with saturated sodium bicarbonate solution and saturated brine respectively, dried over anhydrous sodium sulfate, filtered and concentrated under reduced pressure. The residue was purified by column chromatography (eluent: petroleum ether/ethyl acetate, gradient: 0-50% ethyl acetate) to obtain the title compound (yellow solid, 35 mg, yield: 74.9%). LC/MS (ESI) (m/z): 543 $[M+H]^+$.

**Step 9: 3-(5-(difluoromethyl)-1,3,4-thiadiazol-2-yl)-N-(1-methylcyclopropyl)-8-(4-(2-methylpropanethioyl)piperazin-1-yl)-[1,2,4]triazolo[4,3-a]pyridine-6-sulfonamide**

The Lawesson's reagent (52 mg, 0.13 mmol) was added to the solution of 3-(2-(2,2-difluoroacetyl)hydrazin-1-carbonyl)-8-(4-isobutylpiperazin-1-yl)-N-(1-methylcyclopropyl)-[1,2,4]triazolo[4,3-a]pyridin-6-sulfonamide (30 mg, 0.08 mmol) in toluene (1 mL). The flask was replaced with nitrogen gas three times and then the mixture was stirred at 110°C for 4 hours under an atmosphere of nitrogen. The reaction mixture was cooled to room temperature, diluted with ethyl acetate, sequentially washed with water and saturated brine, dried over anhydrous sodium sulfate, filtered and concentrated under reduced pressure. The residue was purified by column chromatography (eluent: petroleum ether, ethyl acetate, gradient: 0-100% ethyl acetate) to obtain the title compound (yellow solid, 15 mg, yield: 30%). LC/MS (ESI) (m/z): 559 $[M+H]^+$.

**Step 10: 3-(5-(difluoromethyl)-1,3,4-thiadiazol-2-yl)-8-(4-isobutyrylpiperazin-1-yl)-N-(1-methylcyclopropyl)-[1,2,4]triazolo[4,3-a]pyridine-6-sulfonamide**

At 0°C, water (0.1 mL), sodium bicarbonate (15 mg, 0.1 mmol) and potassium hydrogen persulfate (18 mg, 0.03 mmol) were sequentially added to the solution of 3-(5-(difluoromethyl)-1,3,4-thiadiazol-2-yl)-N-(1-methylcyclopropyl)-8-(4-(2-methylpropionyl)piperazin-1-yl)-[1,2,4]triazolo[4,3-a]pyridin-6-sulfonamide (15 mg, 0.03 mmol) in acetone (1 mL). The reaction mixture was stirred at 0°C for 30 minutes. After completion of the reaction, the reaction mixture was diluted with ethyl acetate, washed with water and saturated brine , dried over anhydrous sodium sulfate, filtered and concentrated to dryness, and the residue was purified by pre-HPLC to obtain the title compound (yellow solid, 0.7 mg, yield: 4.8%). $^1H$ NMR (400 MHz, DMSO-$d_6$) δ 9.34 (s, 1H), 8.52 (s, 1H), 7.75 (t, $J$ = 53.2 Hz, 1H), 6.92 (s, 1H), 3.83 - 3.77 (m, 4H), 3.76 - 3.68 (m, 4H), 3.02 - 2.91 (m, 1H), 1.16 (s, 3H), 1.06 - 1.03 (m, 6H), 0.75 - 0.70 (m, 2H), 0.50 - 0.42 (m, 2H).

**Example 100: 3-(5-(difluoromethyl)-1,3,4-thiadiazol-2-yl)-N-(1-(fluoromethyl)cyclopropyl)-8-(4-isobutyrylpiperazin-1-yl)-[1,2,4]triazolo[4,3-a]pyridine-6-sulfonamide**

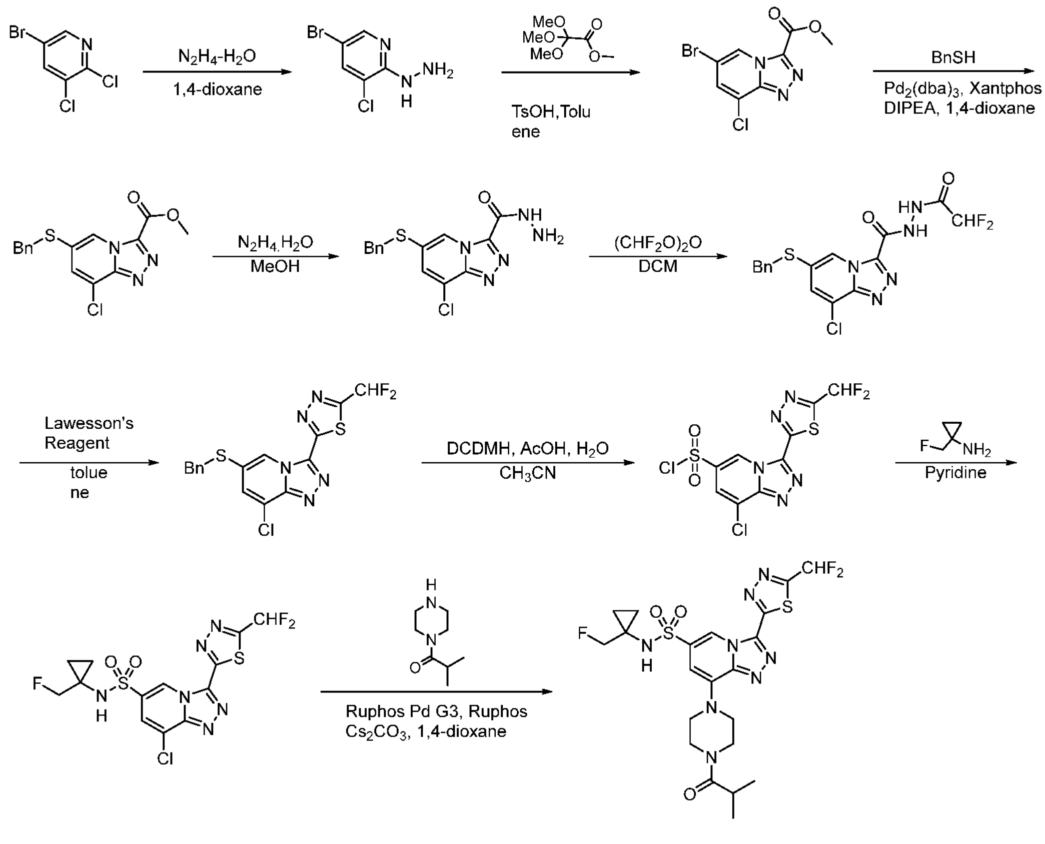

**Step 1: 5-bromo-3-chloro-2-hydrazinopyridine**

At room temperature, hydrazine hydrate (80 mL, 80% wt.) was added to the solution of 5-bromo-2,3-dichloropyridine (20 g, 88.1 mmol) in ethanol (200 mL); and the reaction mixture was stirred at 100°C for 34 hours. After completion of the reaction, water was added; the reaction mixture was extracted with dichloromethane; and the organic phase was washed with saturated brine, dried over anhydrous sodium sulfate, and concentrated under reduced pressure to obtain a crude title compound (yellow solid, 16 g, yield: 82.0%), which was directly used in the next step of reaction. LC/MS (ESI) (m/z) 223 $[M+H]^+$.

**Step 2: Methyl 6-bromo-8-chloro-[1,2,4]triazolo[4,3-a]pyridine-3-carboxylate**

At room temperature, p-toluenesulfonic acid (2.7 g, 19.9 mmol) and 2,2,2-trimethoxymethyl acetate (25 g, 149 mmol) were added to the solution of 5-bromo-3-chloro-2-hydrazinopyridine (11 g, 49.7 mmol) in toluene (150 mL). The reaction mixture was stirred at 80°C for 12 hours. After completion of the reaction, the reaction mixture was diluted with ethyl acetate, extracted with ethyl acetate, washed with saturated brine, dried over anhydrous sodium sulfate, and concentrated under reduced pressure. The residue was separated by column chromatography (ethyl acetate/petroleum ether = 1/2) to obtain the title compound (white solid, 108 g, yield: 78%). $^1H$ NMR (400 MHz, $CDCl_3$) $\delta$ 9.22 (d, *J* = 1.4 Hz, 1H), 7.57 (d, *J* = 1.4 Hz, 1H), 4.06 (s, 3H). LC/MS (ESI) (m/z): 291 $[M+H]^+$.

**Step 3: Methyl 6-(benzylthio)-8-chloro-[1,2,4]triazolo[4,3-a]pyridine-3-carboxylate**

Under an atmosphere of nitrogen at room temperature, N,N-diisopropylethylamine (16.6 mL, 92.8mmol), Xantphos (4.5 g, 7.42 mmol), $Pd_2(dba)_3$ (3.3 g, 3.71 mmol) and benzyl mercaptane (4.7 g, 37.1 mmol) were sequentially added to the solution of 6-bromo-8-chloro-[1,2,4]triazolo[4,3-a]pyridin-3-methyl carboxylate (10.8 g, 37.1 mmol) in 1,4-dioxane (100 mL). The flask was replaced with nitrogen gas three times and then the mixture was stirred at 80 °C for 16 hours under an atmosphere of nitrogen. After completion of the reaction, the reaction mixture was diluted with ethyl acetate, washed with saturated brine, dried over anhydrous sodium sulfate, and concentrated under reduced pressure. The residue was separated by column chromatography (ethyl acetate/petroleum ether = 1/2) to obtain the title compound (yellow solid, 12 g, yield: 97%). LC/MS (ESI) (m/z): 334 $[M+H]^+$.

**Step 4: 6-(benzylthio)-8-chloro-[1,2,4]triazolo[4,3-a]pyridine-3-carbohydrazide**

At room temperature, hydrazine hydrate (12.8 mL, 80% wt.) was added to the solution of 6-(benzylthio)-8-chloro-[1,2,4]triazolo[4,3-a]pyridin-3-methyl carboxylate (12 g, 35.9 mmol) in methanol (200 mL). The reaction mixture was stirred at 25°C for 12 hour. After completion of the reaction, the reaction mixture was concentrated under reduced pressure; and the residue was pulped with the mixed solution of petroleum ether/ethyl acetate, and dried in vacuum to obtain a crude title compound (yellow solid, 20 g, yield: 100%), which was directly used in the next step of reaction. LC/MS (ESI) (m/z): 334 $[M+H]^+$.

**Step 5: 6-(benzylthio)-8-chloro-N'-(2,2-difluoroacetyl)-[1,2,4]triazolo[4,3-a]pyridine-3-carbohydrazide**

At 0°C, difluoroacetic anhydride (15.6 g, 53.9 mmol) was added to the solution of 6-(benzylthio)-8-chloro-[1,2,4] triazolo[4,3-a]pyridin-3-carbohydrazide (20 g, 35.9 mmol) in dichloromethane (200 mL). The reaction mixture was stirred at 25°C for 0.5 hour. After completion of the reaction, the reaction mixture was diluted with ethyl acetate, washed with saturated brine, dried over anhydrous sodium sulfate, and concentrated under reduced pressure. The residue was separated by column chromatography (ethyl acetate/petroleum ether = 1/2) to obtain the title compound (yellow solid, 14 g, yield: 99%). LC/MS (ESI) (m/z): 412 $[M+H]^+$.

**Step 6: 2-(6-(benzylthio)-8-chloro-[1,2,4]triazolo[4,3-a]pyridin-3-yl)-5-(difluoromethyl)-1,3,4-thiadiazole**

Under an atmosphere of nitrogen at 0°C, the Lawesson's reagent (15.6 g, 39.5 mmol) was added to the solution of 6-(benzylthio)-8-chloro-N'-(2,2-difluoroacetyl)-[1,2,4]triazolo[4,3-a]pyridin-3-formylhydrazine (14 g, 35.9 mmol) in toluene (150 mL); and the reaction mixture was stirred at 110°C for 16 hours under an atmosphere of nitrogen. After completion of the reaction, the reaction mixture was diluted with ethyl acetate, sequentially washed with saturated aqueous sodium bicarbonate solution and saturated brine, dried over anhydrous sodium sulfate, and concentrated under reduced pressure. The residue was separated by column chromatography (ethyl acetate/petroleum ether = 1/2) to obtain the title compound (white solid, 4.5g, yield: 31%). $^1H$ NMR (400 MHz, DMSO-$d_6$) $\delta$ 9.09 (d, *J* = 1.4 Hz, 1H), 8.00 (d, *J* = 1.4 Hz, 1H), 7.89-7.58 (m, 1H), 7.42-7.37 (m, 2H), 7.33-7.28 (m, 2H), 7.25-7.20 (m, 1H), 4.38 (s, 2H). LC/MS (ESI) (m/z): 410

[M+H]+.

**Step 7: 8-chloro-3-(5-(difluoromethyl)-1,3,4-thiadiazol-2-yl)-[1,2,4]triazolo[4,3-a]pyridine-6-sulfonyl chloride**

At 0°C, dichlorohydantoin (1.65 g, 8.4 mmol) was added portionwise to 2-(6-(benzylthio)-8-chloro-[1,2,4]triazolo [4,3-a]pyridin-3-yl)-5-(difluoromethyl)-1,3,4-thiadiazole (1.1 g, 2.8 mmol) in the mixed solution of acetonitrile (8 mL), water (2 mL) and 1N dilute hydrochloric acid (2 mL). The reaction mixture was stirred at 0°C for one hour. After completion of the reaction, the reaction mixture was diluted with dichloromethane, sequentially washed with water and saturated brine, dried over anhydrous sodium sulfate, and concentrated under reduced pressure to obtain a crude title compound (white solid, 2.8 g, yield: 100%), which was directly used in the next step of reaction without purification. LC/MS (ESI) (m/z): 386 [M+H]+.

**Step 8: 8-chloro-3-(5-(difluoromethyl)-1,3,4-thiadiazol-2-yl)-N-(1-(fluoromethyl)cyclopropyl)-[1,2,4]triazolo[4,3-a]pyridine-6-sulfonamide**

At 0°C, triethylamine (273 mg, 2.73 mmol) and 8-chloro-3-(5-(difluoromethyl)-1,3,4-thiadiazol-2-yl)-[1,2,4] triazolo[4,3-a]pyridin-6-sulfonyl chloride (350 mg, 0.91 mmol) were added to the solution of 1-(fluoromethyl)cyclopropan-1-amine (117 mg, 1.35 mmol) in dichloromethane (5 mL). The reaction mixture was stirred at 0°C for one hour. After completion of the reaction, the reaction mixture was diluted with dichloromethane, washed with saturated brine, dried over anhydrous sodium sulfate, and concentrated under reduced pressure. The residue was separated by column chromatography (ethyl acetate/petroleum ether= 1/3) to obtain the title compound (white solid, 220 mg, yield: 55%). LC/MS (ESI) (m/z): 410 [M+H]+.

**Step 9: 3-(5-(difluoromethyl)-1,3,4-thiadiazol-2-yl)-N-(1-(fluoromethyl)cyclopropyl)-8-(4-isobutyrylpiperazin-1-yl)-[1,2,4]triazolo[4,3-a]pyridine-6-sulfonamide**

Under an atmosphere of nitrogen, cesium carbonate (67 mg, 0.20 mmol), Ruphos (7 mg, 0.028 mmol), Ruphos Pd G3 (7 mg, 0.014 mmol)were sequentially added to the solution of 8-chloro-3-(5-(difluoromethyl)-1,3,4-thiadiazol-2-yl)-N-(1-(fluoromethyl)cyclopropyl)-[1,2,4]triazolo[4,3-a]pyridin-6-sulfonamide (60 mg, 0.137 mmol) and 1-(2-methylacetonyl)-piperazine (25.6 mg, 0.164 mmol) in 1,4-dioxane (2 mL). The flask was replaced with nitrogen gas three times and then the mixture was stirred at 80 °C for 16 hours under an atmosphere of nitrogen. After completion of the reaction, the reaction mixture was filtered; the filtrate was concentrated under reduced pressure; and the residue was purified by pre-HPLC to obtain the title compound (yellow solid, 1.7 mg, yield: 2%). $^1$H NMR (400 MHz, DMSO-$d_6$) δ 9.32 (s, 1H), 8.91 (s, 1H), 7.68 (d, *J* = 53.0 Hz, 1H), 6.93 (s, 1H), 4.29 (s, 1H), 4.17 (s, 1H), 3.80-3.69 (m, 8H), 2.99-2.95 (m, 1H), 1.05 (d, *J* = 6.7 Hz, 6H), 0.87-0.84 (m, 2H), 0.80-0.76 (m, 2H). LC/MS (ESI) (m/z): 559 [M+H]+.

**Example 101: 3-(5-(difluoromethyl)-1,3,4-thiadiazol-2-yl)-N-(3-ethynyloxetan-3-yl)-8-(4-isobutyrylpiperazin-1-yl)-[1,2,4]triazolo[4,3-a]pyridine-6-sulfonamide**

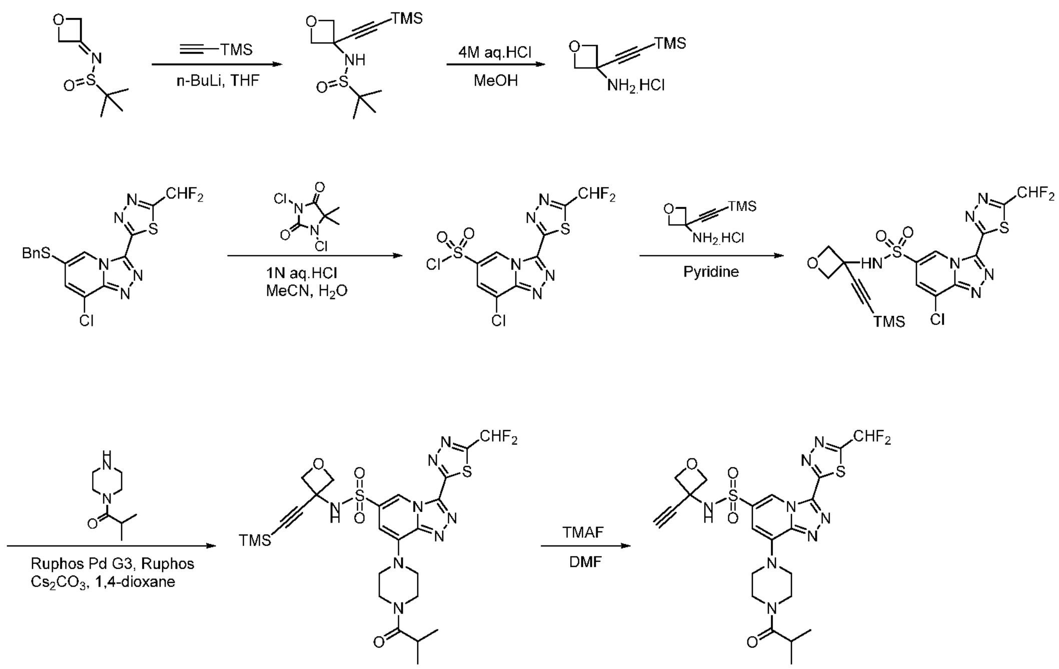


**Step 1: 3-[(tert-butylsulfinyl)amino]-3-[(trimethylsilicyl)acetenyl]oxacyclobutane**

Under an atmosphere of nitrogen at -78°C, the solution of 2.5M n-butyl lithium (3.2 mL, 8.0 mmol) in n-hexane was dropwise added slowly to the solution of acetenyltrimethylsilane (840 mg, 8.5 mmol) in tetrahydrofuran (10 mL). After dropwise adding, the reaction mixture was stirred at -78°C for 30 minutes. The solution of 3-[(tert-butylsulfinyl)imino] oxacyclobutane (1 g, 5.7 mmol) in tetrahydrofuran (5 mL) was slowly added to the above reaction mixture. After dropwise adding, with the temperature kept at -78°C, the mixture was stirred for 10 minutes. After completion of the reaction, with the temperature slowly rising to 0°C, the reaction mixture was quenched with saturated aqueous ammonium chloride solution, and extracted with ethyl acetate. The organic phase was washed with saturated brine, dried over anhydrous sodium sulfate, filtered and concentrated under reduced pressure. The residue was separated by column chromatography (eluent: petroleum ether/ethyl acetate, gradient: 0-40% ethyl acetate) to obtain the title compound (yellow liquid, 1.2 g, yield: 76.9%). LC/MS (ESI) (m/z): 274 $[M+H]^+$.

**Step 2: 3-((trimethylsilyl)ethynyl)oxetan-3-amine hydrochloride**

At 0°C, the solution of 4M dioxane hydrochloride (0.4 mL, 1.6 mmol) was dropwise added slowly to the solution of 3-[(tert-butylsulfinyl)amino]-3-[(trimethylsilicyl)acetenyl]oxacyclobutane (300 mg, 1.1 mmol) in methanol (5 mL). The reaction mixture was stirred at 0°C for 30 minutes under an atmosphere of nitrogen. After completion of the reaction, the reaction mixture was concentrated under reduced pressure to obtain a crude title compound (white solid, 226 mg, yield: 100%), which was directly used in the next step of reaction without purification. LC/MS (ESI) (m/z): 170 $[M+H]^+$.

**By referring to the preparation method in Example 100, Steps 3-5 were implemented according to a similar route by starting from appropriate starting materials to obtain the target compound.**

**Step 6: 3-(5-(difluoromethyl)-1,3,4-thiadiazol-2-yl)-N-(3-ethynyloxetan-3-yl)-8-(4-isobutyrylpiperazin-1-yl)-[1,2,4]triazolo[4,3-a]pyridine-6-sulfonamide**

At 0°C, tetramethylammonium fluoride tetrahydrate (43 mg, 0.26 mmol) was added to the solution of 3-(5-(difluoromethyl)-1,3,4-thiadiazol-2-yl)-8-(4-isobutyrylpiperazin-1-yl)-N-(3-((trimethylsilicyl)acetenyl)oxacyclobutan-3-yl)-[1,2,4]triazolo[4,3-a]pyridin-6-sulfonamide (55 mg, 0.09 mmol) in N,N-dimethylformamide (1 mL). The reaction mixture was stirred at room temperature for one hour. After completion of the reaction, the reaction mixture was diluted with ethyl acetate, sequentially washed with saturated aqueous ammonium chloride solution and saturated brine, dried over anhydrous sodium sulfate, and concentrated under reduced pressure. The residue was purified by pre-HPLC to obtain the title compound (yellow solid, 14.0 mg, yield: 28.7%). $^1$H NMR (400 MHz, DMSO-$d_6$) δ 9.35 (d, $J$ = 1.0 Hz, 1H), 7.91 - 7.58 (m, 1H), 6.93 (s, 1H), 4.76 (d, $J$ = 6.5 Hz, 2H), 4.63 (d, $J$ = 6.4 Hz, 2H), 3.83 - 3.67 (m, 8H), 3.45 (s, 1H), 3.01 - 2.93 (m, 1H), 1.05 (d, $J$ = 6.7 Hz, 6H). LC/MS (ESI) m/z: 567 $[M+H]^+$.

**By referring to the preparation method in Example 100, the following examples were implemented by synthesizing a crude product according to a similar route by starting from appropriate starting materials, treating the crude product via reversed-phase HPLC, and then freeze-drying the crude product to obtain the title compound.**

| Example | Structure and name | Analysis data |
|---|---|---|
| **Example 102** | 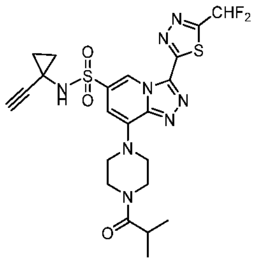 **3-(5-(difluoromethyl)-1,3,4-thiadiazol-2-yl)-N-(1-ethynylcyclopropyl)-8-(4-isobutyrylpiperazin-1-yl)-[1,2,4]triazolo[4,3-a]pyridine-6-sulfonamide** | $^1H$ NMR (400 MHz, DMSO-$d_6$) δ 9.41 (s, 1H), 9.06 (s, 1H), 7.89 - 7.60 (m, 1H), 6.98 (s, 1H), 3.79 - 3.73 (m, 8H), 3.00 - 2.94 (m, 1H), 2.79 (s, 1H), 1.39 - 1.34 (m, 2H), 1.29 - 1.24 (m, 2H), 1.05 (d, *J* = 6.7 Hz, 6H). LC/MS (ESI) m/z: 551 $[M+H]^+$. |
| **Example 103** | 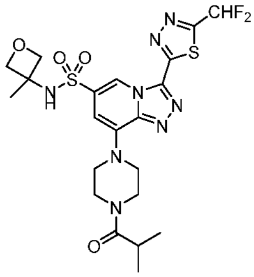 **3-(5-(difluoromethyl)-1,3,4-thiadiazol-2-yl)-8-(4-isobutyrylpiperazin-1-yl)-N-(3-methyloxetan-3-yl)-[1,2,4]triazolo[4,3-a]pyridine-6-sulfonamide** | $^1H$ NMR (400 MHz, DMSO-$d_6$) δ 9.33 (d, *J* = 1.1 Hz, 1H), 8.81 (s, 1H), 7.75 (t, *J* = 53.0 Hz, 1H), 6.95 (d, *J* = 1.0 Hz, 1H), 4.63 (d, *J* = 6.2 Hz, 2H), 4.18 (d, *J* = 6.4 Hz, 2H), 3.88 - 3.78 (m, 4H), 3.76 - 3.69 (m, 4H), 3.01 - 2.93 (m, 1H), 1.49 (s, 3H), 1.05 (d, *J* = 6.7 Hz, 6H). LC/MS (ESI) (m/z): 557 $[M+H]^+$. |
| **Example 104** | 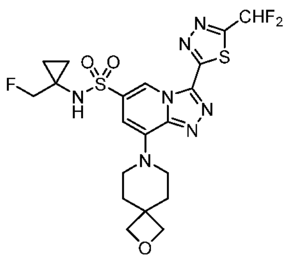 **3-(5-(difluoromethyl)-1,3,4-thiadiazol-2-yl)-N-(1-(fluoromethyl)cyclopropyl)-8-(2-oxa-7-azaspiro[3.5]nonan-7-yl)-[1,2,4]triazolo[4,3-a]pyridine-6-sulfonamide** | $^1H$ NMR (400 MHz, $CD_3OD$) δ 9.56 (s, 1H), 7.34 (t, *J* = 53.4 Hz, 1H), 7.00 (s, 1H), 4.56 - 4.53 (m, 4H), 4.27 (s, 1H), 4.14 (s, 1H), 3.72 - 3.68 (m, 4H), 2.15 - 2.12 (m, 4H), 1.02 - 0.98 (m, 2H), 0.83 - 0.81 (m, 2H). LC/MS (ESI) (m/z): 530 $[M+H]^+$. |
| **Example 105** | 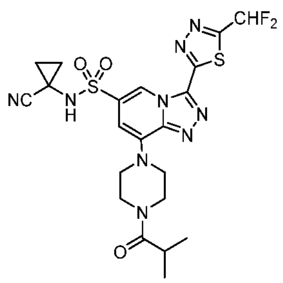 **N-(1-cyanocyclopropyl)-3-(5-(difluoromethyl)-1,3,4-thiadiazol-2-yl)-8-(4-isobutyrylpiperazin-1-yl)-[1,2,4]triazolo[4,3-a]pyridine-6-sulfonamide** | $^1H$ NMR (400 MHz, $CD_3OD$) δ 9.02 (s, 1H), 7.60 - 7.23 (m, 1H), 7.19 (s, 1H), 3.89 (s, 6H), 3.72 (s, 2H), 3.07 - 3.01 (m, 1H), 1.54 - 1.51 (m, 2H), 1.49 - 1.45 (m, 2H), 1.15 (d, *J* = 6.7 Hz, 6H). LC/MS (ESI) (m/z): 552 $[M+H]^+$. |

(continued)

| Example | Structure and name | Analysis data |
|---|---|---|
| **Example 106** | 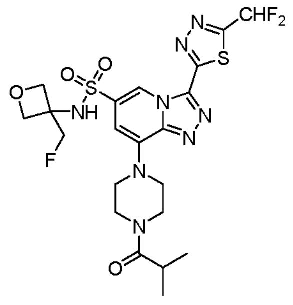 **3-(5-(difluoromethyl)-1,3,4-thiadiazol-2-yl)-N-(3-(fluoromethyl)oxetan-3-yl)-8-(4-isobutyrylpiperazin-1-yl)-[1,2,4]triazolo[4,3-a]pyridine-6-sulfonamide** | $^1$H NMR (400 MHz, DMSO-$d_6$) δ 9.09 (s, 1H), 9.02 (d, *J* = 1.3 Hz, 1H), 7.84 - 7.58 (m, 1H), 7.17 (d, *J* = 1.2 Hz, 1H), 4.75 (s, 1H), 4.64 - 4.60 (m, 3H), 4.37 (d, *J* = 6.9 Hz, 2H), 3.79 - 3.72 (m, 6H), 3.66 - 3.61 (m, 2H), 3.00 - 2.94 (m, 1H), 1.05 (d, *J* = 6.7 Hz, 6H). LC/MS (ESI) (m/z): 575 [M+H]$^+$. |
| **Example 107** | 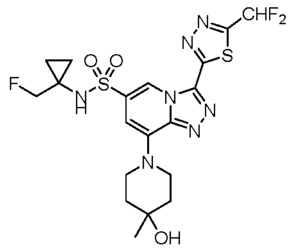 **3-(5-(difluoromethyl)-1,3,4-thiadiazol-2-yl)-N-(1-(fluoromethyl)cyclopropyl)-8-(4-hydroxy-4-methylpiperidin-1-yl)-[1,2,4]triazolo [4,3-a]pyridine-6-sulfonamide** | $^1$H NMR (400 MHz, DMSO-$d_6$) δ 9.26 (d, *J* = 1.3 Hz, 1H), 8.88 (s, 1H), 7.74 (t, *J* = 53.1 Hz, 1H), 6.91 (s, 1H), 4.53 (s, 1H), 4.28 (s, 1H), 4.17 - 4.09 (m, 3H), 3.55 - 3.51 (m, 2H), 1.71 - 1.65 (m, 4H), 1.21 (s, 3H), 0.87 - 0.85 (m, 2H), 0.79 - 0.76 (m, 2H). LC/MS (ESI) (m/z): 518 [M+H]$^+$. |
| **Example 108** | 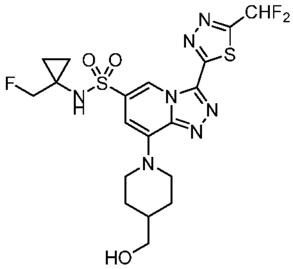 **3-(5-(difluoromethyl)-1,3,4-thiadiazol-2-yl)-N-(1-(fluoromethyl)cyclopropyl)-8-(4-(hydroxymethyl)piperidin-1-yl)-[1,2,4]triazolo[4,3-a]pyridine-6-sulfonamide** | $^1$H NMR (400 MHz, DMSO-$d_6$) δ 9.26 (s, 1H), 8.88 (s, 1H), 7.74 (t, 1H), 6.89 (s, 1H), 4.58 - 4.54 (m, 2H), 4.51 (s, 1H), 4.28 (s, 1H), 4.16 (s, 1H), 3.09 - 3.02 (m, 3H), 1.86-1.80 (m, 3H), 1.42 - 1.30 (m, 3H), 0.87 - 0.84 (m, 2H), 0.79 - 0.76 (m, 2H). LC/MS (ESI) (m/z): 518 [M+H]$^+$. |
| **Example 109** | 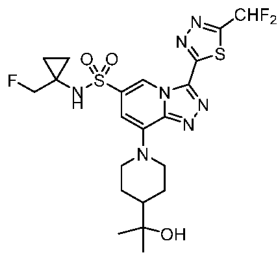 **3-(5-(difluoromethyl)-1,3,4-thiadiazol-2-yl)-N-(1-(fluoromethyl)cyclopropyl)-8-(4-(2-hydroxypropan-2-yl)piperidin-1-yl)-[1,2,4]triazolo[4,3-a]pyridine-6-sulfonamide** | $^1$H NMR (400 MHz, DMSO-$d_6$) δ 8.85 (s, 1H), 7.70 (t, *J* = 53.2 Hz, 1H), 7.08 (s, 1H), 4.50 (d, *J* = 12.2 Hz, 2H), 4.29 (s, 1H), 4.20 (s, 1H), 4.16 (s, 1H), 2.94 - 2.87 (m, 2H), 1.91 - 1.85 (m, 2H), 1.53 - 1.43 (m, 3H), 1.09 (s, 6H), 0.85 - 0.81 (m, 2H), 0.79 - 0.75 (m, 2H). LC/MS (ESI) (m/z): 546 [M+H]$^+$. |

(continued)

| Example | Structure and name | Analysis data |
|---|---|---|
| **Example 110** | 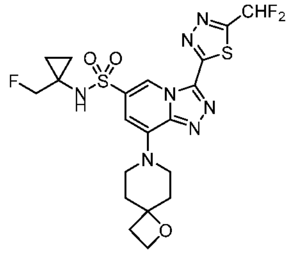 **3-(5-(difluoromethyl)-1,3,4-thiadiazol-2-yl)-N-(1-(fluoromethyl)cyclopropyl)-8-(1-oxa-7-azaspiro[3.5]nonan-7-yl)-[1,2,4]triazolo[4,3-a]pyridine-6-sulfonamide** | $^1H$ NMR (400 MHz, DMSO-$d_6$) δ 9.14 (s, 1H), 7.74 (t, *J* = 53.1 Hz, 1H), 6.90 (s, 1H), 4.48 - 4.43 (m, 2H), 4.22 (d, *J* = 49.3 Hz, 2H), 3.71 - 3.67 (m, 2H), 3.62 - 3.58 (m, 2H), 2.46 - 2.43 (m, 2H), 2.01 - 1.95 (m, 4H), 0.51 - 0.44 (m, 2H), 0.40 - 0.32 (m, 2H). LC/MS (ESI) (m/z): 530 $[M+H]^+$. |
| **Example 111** | 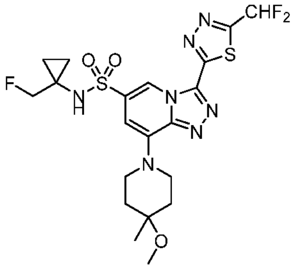 **3-(5-(difluoromethyl)-1,3,4-thiadiazol-2-yl)-N-(1-(fluoromethyl)cyclopropyl)-8-(4-methoxy-4-methylpiperidin-1-yl)-[1,2,4]triazolo [4,3-a]pyridine-6-sulfonamide** | $^1H$ NMR (400 MHz, DMSO-$d_6$) δ 9.27 (d, *J* = 1.1 Hz, 1H), 8.88 (s, 1H), 7.75 (t, *J* = 53.1 Hz, 1H), 6.91 (s, 1H), 4.28 (s, 1H), 4.15 (s, 1H), 4.15 - 4.08 (m, 2H), 3.44 - 3.40 (m, 2H), 3.18 (s, 3H), 1.92 - 1.85 (m, 2H), 1.75 - 1.66 (m, 2H), 1.19 (s, 3H), 0.89 - 0.83 (m, 2H), 0.81 - 0.75 (m, 2H). LC/MS (ESI) (m/z): 532 $[M+H]^+$. |
| **Example 112** | 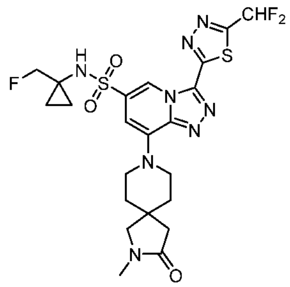 **3-(5-(difluoromethyl)-1,3,4-thiadiazol-2-yl)-N-(1-(fluoromethyl)cyclopropyl)-8-(2-methyl-3-oxo-2,8-diaz aspiro [4.5] decan-8-yl)-[1,2,4]triazolo [4,3-a]pyridine-6-sulfonamide** | $^1H$ NMR (400 MHz, $CD_3OD$) δ 9.56 (s, 1H), 7.53 - 7.27 (m, 1H), 7.01 (s, 1H), 4.27 (s, 1H), 4.15 (s, 1H), 3.89 - 3.84 (m, 2H), 3.74 - 3.68 (m, 2H), 3.41 (s, 2H), 2.87 (s, 3H), 2.43 (s, 2H), 1.94 - 1.88 (m, 4H), 1.03 - 0.99 (m, 2H), 0.93 - 0.85 (m, 2H). LC/MS (ESI) (m/z): 571 $[M+H]^+$. |
| **Example 113** | 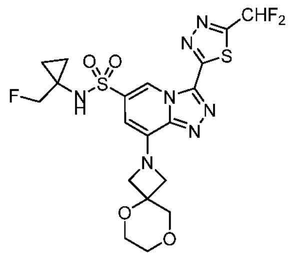 **3-(5-(difluoromethyl)-1,3,4-thiadiazol-2-yl)-N-(1-(fluoromethyl)cyclopropyl)-8-(5,8-dioxa-2-azaspiro[3.5]nonan-2-yl)-[1,2,4]triazolo [4,3-a]pyridine-6-sulfonamide** | $^1H$ NMR (400 MHz, $CD_3OD$) δ 9.36 (s, 1H), 7.40 (t, *J* = 53.4 Hz, 1H), 6.50 (s, 1H), 4.43 - 4.40 (m, 2H), 4.30 (s, 1H), 4.26 - 4.23 (m, 2H), 4.18 (s, 1H), 3.89 (s, 2H), 3.80 - 3.77 (m, 2H), 3.73 - 3.70 (m, 2H), 1.00 - 0.97 (m, 2H), 0.83 - 0.80 (m, 2H). LC/MS (ESI) (m/z): 532 $[M+H]^+$. |

(continued)

| Example | **Structure and name** | Analysis data |
|---|---|---|
| **Example 114** | 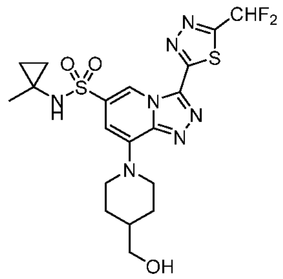 **3-(5-(difluoromethyl)-1,3,4-thiadiazol-2-yl)-8-(4-(hydroxymethyl)piperidin-1-yl)-N-(1-methylcyclopropyl)-[1,2,4]triazolo [4,3-a]pyridine-6-sulfonamide** | $^1$H NMR (400 MHz, DMSO-$d_6$) δ 9.29 (d, *J* = 1.1 Hz, 1H), 8.51 (s, 1H), 7.75 (t, *J* = 53.1 Hz, 1H), 6.89 (s, 1H), 4.57 - 4.54 (m, 2H), 4.54 - 4.51 (m, 1H), 3.36 - 3.34 (m, 2H), 3.10 - 3.02 (m, 2H), 1.88 - 1.82 (m, 2H), 1.77 - 1.68 (m, 1H), 1.41 - 1.31 (m, 2H), 1.16 (s, 3H), 0.76 - 0.72 (m, 2H), 0.48 - 0.43 (m, 2H). LC/MS (ESI) (m/z): 500 [M+H]$^+$. |
| **Example 115** | 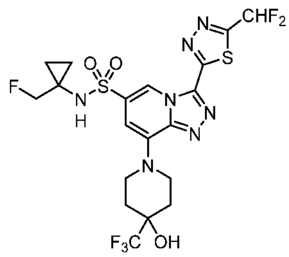 **3-(5-(difluoromethyl)-1,3,4-thiadiazol-2-yl)-N-(1-(fluoromethyl)cyclopropyl)-8-(4-hydroxy-4-(trifluoromethyl)piperidin-1-yl)-[1,2,4] triazolo[4,3-a]pyridine-6-sulfonamide** | $^1$H NMR (400 MHz, DMSO-$d_6$) δ 9.29 (d, *J* = 1.1 Hz, 1H), 8.88 (s, 1H), 7.74 (t, *J* = 53.0 Hz, 1H), 6.97 (d, *J* = 1.0 Hz, 1H), 6.20 (s, 1H), 4.57 - 4.42 (m, 2H), 4.27 (s, 1H), 4.15 (s, 1H), 3.31 - 3.26 (m, 2H), 1.97 - 1.82 (m, 4H), 0.90 - 0.82 (m, 2H), 0.80 - 0.74 (m, 2H). LC/MS (ESI) (m/z): 572 [M+H]$^+$. |
| **Example 116** | 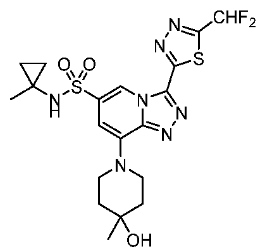 **3-(5-(difluoromethyl)-1,3,4-thiadiazol-2-yl)-8-(4-hydroxy-4-methylpiperidin-1-yl)-N-(1-methylcyclopropyl)-[1,2,4]triazolo [4,3-a]pyridine-6-sulfonamide** | $^1$H NMR (400 MHz, $CDCl_3$) δ 9.57 (s, 1H), 7.12 (t, *J* = 53.2 Hz, 1H), 6.82 (s, 1H), 5.13 (s, 1H), 4.33 - 4.20 (m, 2H), 3.64 - 3.50 (m, 2H), 1.96 - 1.88 (m, 2H), 1.83 - 1.78 (m, 2H), 1.31 (s, 3H), 1.26 (d, *J* = 3.7 Hz, 3H), 0.90 - 0.88 (m, 2H), 0.62 - 0.57 (m, 2H).LC/MS (ESI) (m/z): 500 [M+H]$^+$. |
| **Example 117** | 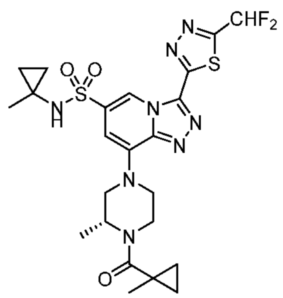 **(R)-3-(5-(difluoromethyl)-1,3,4-thiadiazol-2-yl)-8-(3-methyl-4-(1-methylcyclopropane-1-carbonyl)piperazin-1-yl)-N-(1-methylcyclopropyl)-[1,2,4]triazolo[4,3-a]pyridine-6-sulfonamide** | $^1$H NMR (400 MHz, DMSO-$d_6$) δ 9.34 - 9.31 (m, 1H), 8.51 (s, 1H), 7.76 (t, *J* = 53.1 Hz, 1H), 6.93 (s, 1H), 4.72 - 4.70 (m, 1H), 4.55 - 4.52 (m, 1H), 4.30 (t, *J* = 13.9 Hz, 2H), 3.27 - 3.24 (m, 2H), 3.09 - 3.07 (m, 1H), 1.27 - 1.25 (m, 6H), 1.18 (s, 3H), 0.90 - 0.84 (m, 2H), 0.77 - 0.71 (m, 2H), 0.63 - 0.58 (m, 2H), 0.47 -0.43 (m, 2H). LC/MS (ESI) (m/z): 567 [M+H]$^+$. |

(continued)

| Example | **Structure and name** | Analysis data |
|---|---|---|
| **Example 118** | 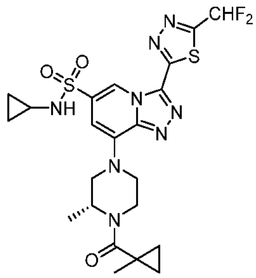<br>**(R)-N-cyclopropyl-3-(5-(difluoromethyl)-1,3,4-thiadiazol-2-yl)-8-(3-methyl-4-(1-methylcyclopropane-1-carbonyl)piperazin-1-yl)-[1,2,4]triazolo[4,3-a]pyridine-6-sulfonamide** | $^1$H NMR (400 MHz, DMSO-$d_6$) δ 9.35 (s, 1H), 8.34 (s, 1H), 7.76 (t, *J* = 53.2 Hz,1H), 6.94 (s, 1H), 4.72 (s, 1H), 4.54 (d, *J* = 12.0 Hz, 1H), 4.31 (t, *J* = 13.9 Hz, 2H), 3.27 - 3.24 (m, 2H), 3.11 - 3.07 (m, 1H), 2.30 - 2.28 (m, 1H), 1.34 - 1.29 (m, 6H), 0.91 - 0.83 (m, 2H), 0.62 - 0.58 (m, 2H), 0.55 - 0.50 (m, 4H). LC/MS (ESI) (m/z): 553 $[M+H]^+$. |
| **Example 119** | 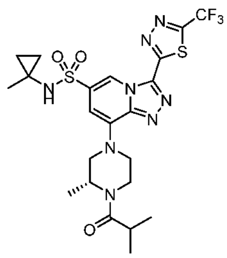<br>**(R)-8-(4-isobutyryl-3-methylpiperazin-1-yl)-N-(1-methylcyclopropyl)-3-(5-(trifluoromethyl)-1,3,4-thiadiazol-2-yl)-[1,2,4]triazolo [4,3-a]pyridine-6-sulfonamide** | $^1$H NMR (400 MHz, DMSO-$d_6$) δ 9.29 (s, 1H), 8.54 (s, 1H), 6.93 (s, 1H), 4.71 (d, *J* = 55.8 Hz, 1H), 4.48 - 4.05 (m, 3H), 3.62 (s, 1H), 3.20 - 3.06 (m, 2H), 2.97 - 2.92 (m, 1H), 1.37 - 1.30 (m, 3H), 1.18 (s, 3H), 1.06 (d, *J* = 3.8 Hz, 6H), 0.79 - 0.71 (m, 2H), 0.50 - 0.43 (m, 2H). LC/MS (ESI) (m/z): 573 $[M+H]^+$. |
| **Example 120** | 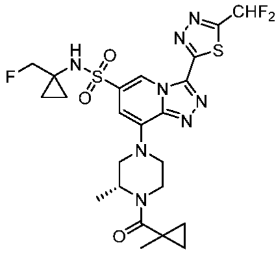<br>**(R)-3-(5-(difluoromethyl)-1,3,4-thiadiazol-2-yl)-N-(1-(fluoromethyl) cyclopropyl)-8-(3-methyl-4-(1-methylcyclopropane-1-carbonyl)piperazin-1-yl)-[1,2,4]triazolo[4,3-a]pyridine-6-sulfonamide** | $^1$H NMR (400 MHz, DMSO-$d_6$) δ 9.30 (s, 1H), 8.89 (s, 1H), 7.75 (t, *J* = 53.0 Hz, 1H), 6.93 (s, 1H), 4.78 - 4.64 (m, 1H), 4.52 (d, *J* = 11.7 Hz, 1H), 4.34 - 4.22 (m, 3H), 4.21 - 4.14 (m, 1H), 3.27 - 3.22 (m, 2H), 3.13 - 3.00 (m, 1H), 1.35 - 1.20 (m, 6H), 0.93 - 0.82 (m, 4H), 0.81 - 0.75 (m, 2H), 0.64 - 0.55 (m, 2H). LC/MS (ESI) (m/z): 585 $[M+H]^+$. |

**Example 121: 1-chloro-N-cyclopropyl-8-(4-isobutyrylpiperazin-1-yl)-3-(5-(trifluoromethyl)-1,3,4-thiadiazol-2-yl) imidazo [1,5-a]pyridine-6-sulfonamide**

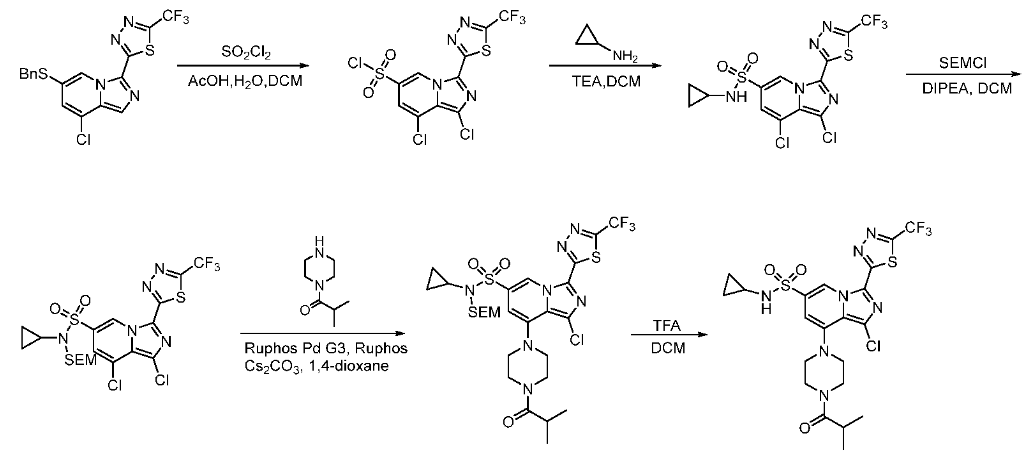


**Step 1: 1,8-dichloro-3-(5-(trifluoromethyl)-1,3,4-thiadiazol-2-yl)imidazo[1,5-a]pyridin-6-sulfonyl chloride**

At 0°C, glacial acetic acid (0.2 mL) and water (0.2 mL) were added to the solution of 2-(6-(benzylthio)-8-chloroimidazo[1,5-a]pyridin-3-yl)-5-(trifluoromethyl)-1,3,4-thiadiazole (300 mg, 0.73 mmol) in dichloromethane (5 mL); then, sulfonyl chloride (379 mg, 2.81 mmol) was added dropwise slowly; and the reaction mixture was stirred at room temperature for 30 minutes. The reaction mixture was diluted with dichloromethane, sequentially washed with water and saturated brine, dried over anhydrous sodium sulfate, filtered and concentrated under reduced pressure to obtain a crude title compound (yellow solid, 330 mg, yield: 100%), which was directly used in the next step of reaction. LC/MS (ESI) (m/z): 439 $[M+H]^+$.

**Step 2: 1,8-dichloro-N-cyclopropyl-3-(5-(trifluoromethyl)-1,3,4-thiadiazol-2-yl)imidazo[1,5-a]pyridine-6-sulfonamide**

At 0°C, triethylamine (207 mg, 2.27 mmol) and 1,8-dichloro-3-(5-(trifluoromethyl)-1,3,4-thiadiazol-2-yl)imidazo [1,5-a]pyridin-6-sulfonyl chloride (330 mg, 0.73 mmol) were sequentially added to the solution of cyclopropylamine (80 mg, 1.20 mmol) in dichloromethane (8 mL); and the reaction mixture was stirred at room temperature for 30 minutes. The reaction mixture was extracted with ethyl acetate. The organic phase was washed with saturated brine, dried over anhydrous sodium sulfate, filtered and concentrated under reduced pressure. The residue was separated by column chromatography (eluent: petroleum ether/ethyl acetate, gradient: 0-30% ethyl acetate) to obtain the title compound (yellow solid, 180 mg, yield: 50.4%). LC/MS (ESI) (m/z): 458 $[M+H]^+$.

**Step 3: 1,8-dichloro-N-cyclopropyl-3-(5-(trifluoromethyl)-1,3,4-thiadiazol-2-yl)-N-((2-(trimethylsilyl)ethoxy) methyl)imidazo[1,5-a]pyridine-6-sulfonamide**

At room temperature, DIPEA (496 mg, 1.20 mmol) and 2-(trimethylsilanyl)ethoxymethyl chloride (100 mg, 0.56 mmol) were added to the solution of 1,8-dichloro-N-cyclopropyl-3-(5-(trifluoromethyl)-1,3,4-thiadiazol-2-yl)imidazo[1,5-a] pyridin-6-sulfonamide (180 mg, 0.40 mmol) in dichloromethane (5 mL). The reaction mixture was stirred at 45°C for 2 hours under an atmosphere of nitrogen. After completion of the reaction, the reaction mixture was cooled to room temperature, diluted with ethyl acetate, washed with saturated brine, dried over anhydrous sodium sulfate, and concentrated under reduced pressure. The residue was purified by column chromatography (eluent: petroleum ether/ethyl acetate, gradient: 0-25% ethyl acetate) to obtain the title compound (yellow solid, 180 mg, yield: 77.8%). LC/MS (ESI) m/z: 588 $[M+H]^+$.

**Step 4: 1-chloro-N-cyclopropyl-8-(4-isobutyrylpiperazin-1-yl)-3-(5-(trifluoromethyl)-1,3,4-thiadiazol-2-yl)-N-((2-(trimethylsilyl)ethoxy)methyl)imidazo[1,5-a]pyridine-6-sulfonamide**

Under an atmosphere of nitrogen, cesium carbonate (308 mg, 0.90 mmol), Ruphos (28 mg, 0.06 mmol) and Ruphos Pd G3 (28 mg, 0.03 mmol) were sequentially added to the solution of 1,8-dichloro-N-cyclopropyl-3-(5-(trifluoromethyl)-1,3,4-thiadiazol-2-yl)-N-(2-(trimethylsilyl)ethoxy)methyl)imidazo[1,5-a]pyridin-6-sulfonamide (180 mg, 0.30 mmol) and 2-methyl-1-(piperazin-1-yl)propan-1-one (72 mg, 0.45 mmol) in 1,4-dioxane (4 mL). The flask was replaced with nitrogen gas three times and then the mixture was stirred at 80°C for 4 hours under an atmosphere of nitrogen. After completion of the reaction, the reaction mixture was cooled to room temperature, diluted with ethyl acetate, washed with saturated brine, dried over anhydrous sodium sulfate, and concentrated under reduced pressure. The residue was purified

by column chromatography (eluent: petroleum ether/ethyl acetate, gradient: 0-45% ethyl acetate) to obtain the title compound (yellow solid, 100 mg, yield: 45.4%). LC/MS (ESI) m/z: 708 [M+H]$^+$.

**Step 5: 1-chloro-N-cyclopropyl-8-(4-isobutyrylpiperazin-1-yl)-3-(5-(trifluoromethyl)-1,3,4-thiadiazol-2-yl)imidazo[1,5-a]pyridine-6-sulfonamide**

At 0°C, trifluoroacetic acid (1 mL) was dropwise added slowly to the solution of 1-chloro-N-cyclopropyl-8-(4-isobutyrylpiperazin-1-yl)-3-(5-(trifluoromethyl)-1,3.4-thiadiazol-2-yl)-N-(2-(trimethylsilyl)ethoxymethyl)imidazo[1,5-a]pyridin-6-sulfonamide (100 mg, 0.14 mmol) in dichloromethane (5 mL). The mixture was stirred at room temperature for 30 minutes. The mixture was concentrated under reduced pressure; and the residue was purified by pre-HPLC to obtain the title compound (yellow solid, 4 mg, yield: 23.2%). $^1$H NMR (400 MHz, DMSO-$d_6$) δ 9.59 (s, 1H), 8.28 (s, 1H), 8.13 (s, 1H), 6.73 (s, 1H), 3.84 - 3.71 (m, 4H), 3.44 - 3.34 (m, 4H), 2.99 - 2.91 (m, 1H), 2.30 - 2.22 (m, 1H), 1.05 (d, $J$ = 6.3 Hz, 6H), 0.55 - 0.44 (m, 4H). LC/MS (ESI) (m/z): 578 [M+H]$^+$.

**By referring to the preparation method in Example 121, the following examples were implemented by synthesizing a crude product according to a similar route by starting from appropriate starting materials, treating the crude product via reversed-phase HPLC, and then freeze-drying the crude product to obtain the title compound.**

| Example | Structure and name | Analysis data |
|---|---|---|
| **Example 122** | 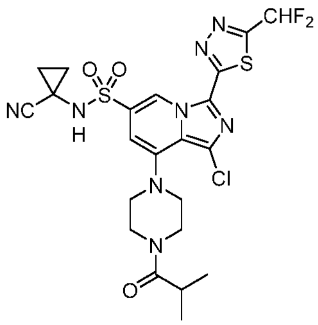 **1-chloro-N-(1-cyanocyclopropyl)-3-(5-(difluoromethyl)-1,3,4-thiadiazol-2-yl)-8-(4-isobutyrylpiperazin-1-yl)imidazo[1,5-a]pyridine-6-sulfonamide** | $^1$H NMR (400 MHz, DMSO-$d_6$) δ 9.72 (s, 1H), 9.55 (s, 1H), 7.70 (t, $J$ = 53.1 Hz, 1H), 6.84 (s, 1H), 3.85 - 3.80 (m, 4H), 3.18 - 3.05 (m, 4H), 2.98 - 2.92 (m, 1H), 1.52 - 1.47 (m, 2H), 1.41 - 1.37 (m, 2H), 1.05 (d, $J$ = 6.7 Hz, 6H). LC/MS (ESI) (m/z): 585 [M+H]$^+$. |
| **Example 123** | 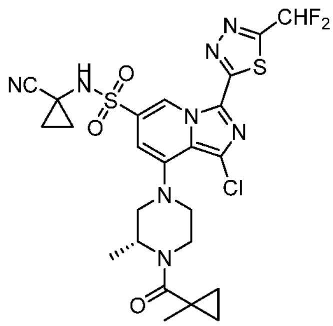 **(R)-1-chloro-N-(1-cyanocyclopropyl)-3-(5-(difluoromethyl)-1,3,4-thiadiazol-2-yl)-8-(3-methyl-4-(1-methylcyclopropane-1-carbonyl)piperazin-1-yl)imidazo [1,5-a]pyridine-6-sulfonamide** | $^1$H NMR (400 MHz, DMSO-$d_6$) δ 9.72 (s, 1H), 9.56 (s, 1H), 7.70 (t, $J$ = 52.6 Hz, 1H), 6.83 (s, 1H), 4.77 - 4.75 (m, 1H), 4.26 - 4.21 (m, 1H), 3.65 - 3.58 (m, 2H), 3.22 - 3.19 (m, 1H), 3.14 - 3.07 (m, 1H), 2.62 - 2.57 (m, 1H), 1.50 - 1.49 (m, 2H), 1.39 - 1.34 (m, 5H), 1.28 (s, 3H), 0.87 - 0.83 (m, 2H), 0.60 - 0.57 (m, 2H). LC/MS (ESI) (m/z): 611 [M+H]$^+$. |

(continued)

| Example | Structure and name | Analysis data |
|---|---|---|
| **Example 124** | 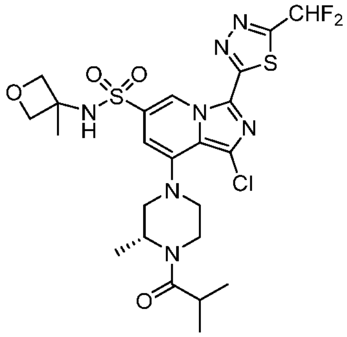 **(R)-1-chloro-3-(5-(difluoromethyl)-1,3,4-thiadiazol-2-yl)-8-(4-isobutyryl-3-methylpiperazin-1-yl)-N-(3-methyloxetan-3-yl)imidazo [1,5-a] pyridine-6-sulfonamide** | $^1$H NMR (400 MHz, DMSO-$d_6$) δ 9.62 (s, 1H), 8.77 (s, 1H), 7.87 - 7.50 (m, 1H), 6.85 (s, 1H), 4.67 - 4.60 (m, 2H), 4.19 (d, *J* = 6.5 Hz, 2H), 3.60 (d, *J* = 12.8 Hz, 1H), 3.20 - 3.11 (m, 4H), 3.07 - 3.02 (m, 1H), 2.97 - 2.85 (m, 2H), 1.50 (s, 3H), 1.40 - 1.23 (m, 3H), 1.04 (d, *J* = 6.8 Hz, 6H). LC/MS (ESI) m/z: 604 $[M+H]^+$. |
| **Example 125** | 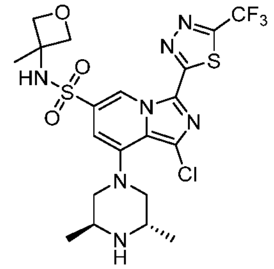 **1-chloro-8-((3S,5S)-3,5-dimethylpiperazin-1-yl)-N-(3-methyloxetan-3-yl)-3-(5-(trifluoromethyl)-1,3,4-thiadiazol-2-yl)imidazo [1,5-a] pyridine-6-sulfonamide** | $^1$H NMR (400 MHz, DMSO-$d_6$) δ 9.57 (s, 1H), 8.21 (s, 1H), 6.86 (s, 1H), 4.63 (t, *J*= 5.5 Hz, 2H), 4.19 (d, *J* = 6.3 Hz, 2H), 3.58 - 3.44 (m, 4H), 2.77 - 2.53 (m, 2H), 1.50 (s, 3H), 1.18 (s, 6H). LC/MS (ESI) m/z: 566 $[M+H]^+$. |
| **Example 126** | 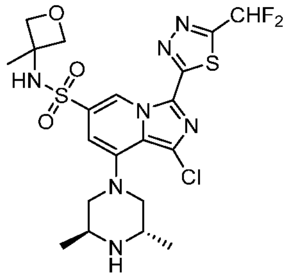 **1-chloro-3-(5-(difluoromethyl)-1,3,4-thiadiazol-2-yl)-8-((3S,5S)-3,5-dimethylpiperazin-1-yl)-N-(3-methyloxetan-3-yl)imidazo[1,5-a]pyridine-6-sulfonamide** | $^1$H NMR (400 MHz, $CD_3OD$) δ 9.86 (s, 1H), 7.34 (t, *J* = 53.5 Hz, 1H), 6.88 (s, 1H), 4.81 - 4.77 (m, 2H), 4.32 (dd, *J* = 6.3, 2.0 Hz, 2H), 3.92 - 3.84 (m, 2H), 3.63 - 3.51 (m, 2H), 3.08 - 2.80 (m, 2H), 1.64 (s, 3H), 1.43 (d, *J* = 3.0 Hz, 6H). LC/MS (ESI) (m/z): 548 $[M+H]^+$. |
| **Example 127** | 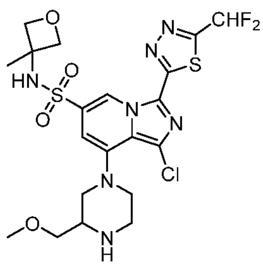 **1-chloro-3-(5-(difluoromethyl)-1,3,4-thiadiazol-2-yl)-8-(3-(methoxymethyl)piperazin-1-yl)-N-(3-methyloxetan-3-yl)imidazo [1,5-a]pyridine-6-sulfonamide** | $^1$H NMR (400 MHz, $CD_3OD$) δ 9.92 (s, 1H), 7.34 (t, *J* = 53.5 Hz, 1H), 6.93 (s, 1H), 4.80 - 4.78 (m, 2H), 4.37 - 4.32 (m, 2H), 3.91 - 3.85 (m, 1H), 3.76 - 3.65 (m, 4H), 3.59 - 3.52 (m, 2H), 3.47 (s, 3H), 3.16 - 3.07 (m, 2H), 1.66 (s, 3H). LC/MS (ESI) (m/z): 564 $[M+H]^+$. |

(continued)

| Example | Structure and name | Analysis data |
|---|---|---|
| **Example 128** | 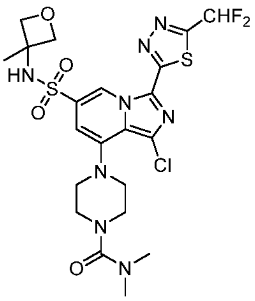 **4-(1-chloro-3-(5-(difluoromethyl)-1,3,4-thiadiazol-2-yl)-6-(N-(3-methyloxetan-3-yl)sulfamoyl)imidazo [1,5-a]pyridin-8-yl)-N,N-dimethylpiperazine-1-carboxamide** | $^1$H NMR (400 MHz, $CD_3OD$) δ 9.83 (s, 1H), 7.33 (t, *J* = 53.5 Hz, 1H), 6.85 (s, 1H), 4.79 (d, *J* = 6.3 Hz, 2H), 4.31 (d, *J* = 6.6 Hz, 2H), 3.57 - 3.52 (m, 4H), 3.24 - 3.18 (m, 4H), 2.91 (s, 6H), 1.63 (s, 3H). LC/MS (ESI) (m/z): 591 $[M+H]^+$. |
| **Example 129** | 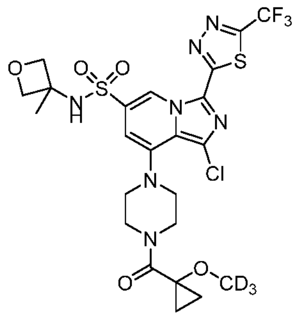 **1-chloro-8-(4-(1-(methoxy-d3)cyclopropane-1-carbonyl)piperazin-1-yl)-N-(3-methyloxetan-3-yl)-3-(5-(trifluoromethyl)-1,3,4-thiadiazol-2-yl)imidazo [1,5-a]pyridine-6-sulfonamide** | $^1$H NMR (400 MHz, DMSO-$d_6$) δ 9.59 (s, 1H), 8.79 (s, 1H), 6.92 (s, 1H), 4.64 (d, *J* = 6.2 Hz, 2H), 4.19 (d, *J* = 6.4 Hz, 2H), 4.10 - 3.69 (m, 4H), 3.23 - 3.11 (m, 4H), 1.50 (s, 3H), 1.05 - 1.00 (m, 2H), 0.95 - 0.91 (m, 2H). LC/MS (ESI) (m/z): 639 $[M+H]^+$. |
| **Example 130** | 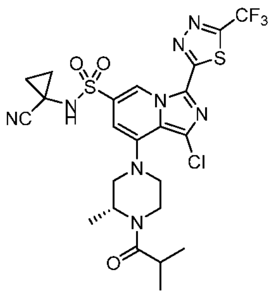 **(R)-1-chloro-N-(1-cyanocyclopropyl)-8-(4-isobutyryl-3-methylpiperazin-1-yl)-3-(5-(trifluoromethyl)-1,3,4-thiadiazol-2-yl)imidazo[1,5-a] pyridine-6-sulfonamide** | $^1$H NMR (400 MHz, DMSO-$d_6$) δ 9.69 (s, 1H), 9.58 (s, 1H), 6.85 (s, 1H), 4.86 - 4.44 (m, 1H), 4.06 - 3.54 (m, 3H), 3.22 - 3.20 (m, 1H), 3.08 - 2.86 (m, 2H), 2.69 - 2.55 (m, 1H), 1.52 - 1.48 (m, 2H), 1.41 - 1.38 (m, 2H), 1.34 - 1.19 (m, 3H), 1.02 (d, *J* = 6.6 Hz, 6H). LC/MS (ESI) (m/z): 617 $[M+H]^+$. |
| **Example 131** | 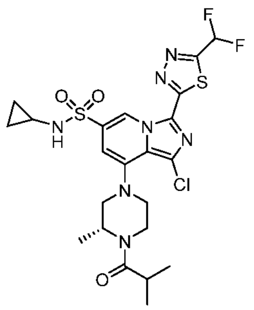 **(R)-1-chloro-N-cyclopropyl-3-(5-(difluoromethyl)-1,3,4-thiadiazol-2-yl)-8-(4-isobutyryl-3-methylpiperazin-1-yl)imidazo[1,5-a]pyridine-6-sulfonamide** | $^1$HNMR(400 MHz, DMSO-$d_6$) δ 9.65 (s, 1H), 8.32 (s, 1H), 7.76 (t, *J* = 53.7 Hz, 1H), 6.83 (s, 1H), 4.63 - 4.13 (m, 1H), 3.95 - 3.69 (m, 1H), 3.64 - 3.58 (m, 1H), 3.23 - 3.15 (m, 2H), 3.10 - 3.01 (m, 1H), 2.99 - 2.80 (m, 2H), 2.31 - 2.27 (m, 1H), 1.28 - 1.22 (m, 3H), 1.04 (d, *J* = 4.6 Hz, 6H), 0.56 - 0.48 (m, 4H). LC/MS (ESI) (m/z): 574 $[M+H]^+$. |

(continued)

| Example | Structure and name | Analysis data |
|---|---|---|
| **Example 132** | 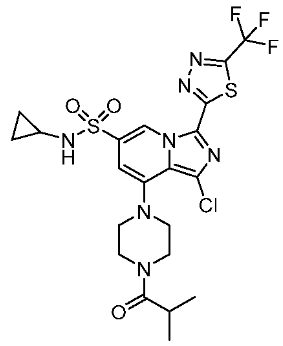 **1-chloro-N-cyclopropyl-8-(4-isobutyrylpiperazin-1-yl)-3-(5-(trifluoromethyl)-1,3,4-thiadiazol-2-yl)imidazo [1,5-a]pyridine-6-sulfonamide** | $^1H$ NMR (400 MHz, DMSO-$d_6$) δ 9.62 (s, 1H), 8.35 (s, 1H), 6.88 (s, 1H), 3.89 - 3.56 (m, 4H), 3.18 - 3.05 (m, 4H), 2.99 - 2.93 (m, 1H), 2.31 - 2.27 (m, 1H), 1.05 (d, $J$ = 6.7 Hz, 6H), 0.57 - 0.52 (m, 2H), 0.51 - 0.46 (m, 2H). LC/MS (ESI) (m/z): 578 $[M+H]^+$. |
| **Example 133** | 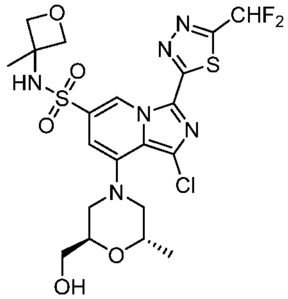 **1-chloro-3-(5-(difluoromethyl)-1,3,4-thiadiazol-2-yl)-8-((2R,6S)-2-(hydroxymethyl)-6-methylmorpholino)-N-(3-methyloxetan-3-yl)imidazo [1,5-a]pyridine-6-sulfonamide** | $^1H$ NMR (400 MHz, DMSO-$d_6$) δ 9.61 (d, 1H), 8.81 (s, 1H), 7.69 (t, $J$ = 53.1 Hz, 1H), 6.86 (s, 1H), 4.76 - 4.71 (m, 1H), 4.65 - 4.61 (m, 2H), 4.27 - 4.22 (m, 1H), 4.21 - 4.18 (m, 2H), 4.05 - 4.00 (m, 1H), 3.67 - 3.61 (m, 2H), 3.30 - 3.26 (m, 2H), 2.93 - 2.85 (m, 1H), 2.66 - 2.60 (m, 1H), 1.50 (s, 3H), 1.23 (d, $J$ = 6.3 Hz, 3H). LC/MS (ESI) (m/z): 565 $[M+H]^+$. |
| **Example 134** | 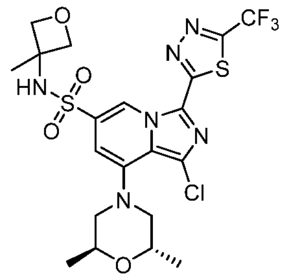 **1-chloro-8-((2S,6S)-2,6-dimethylmorpholino)-N-(3-methyloxetan-3-yl)-3-(5-(trifluoromethyl)-1,3,4-thiadiazol-2-yl)imidazo [1,5-a]pyridine-6-sulfonamide** | $^1H$ NMR (400 MHz, DMSO-$d_6$) δ 9.59 (s, 1H), 8.82 (s, 1H), 6.87 (s, 1H), 4.67 - 4.61 (m, 2H), 4.33 - 4.25 (m, 2H), 4.22 - 4.16 (m, 2H), 3.30 - 3.23 (m, 2H), 2.73 - 2.66 (m, 2H), 1.50 (s, 3H), 1.26 (d, $J$ = 4.1 Hz, 6H). LC/MS (ESI) (m/z): 567 $[M+H]^+$. |
| **Example 135** | 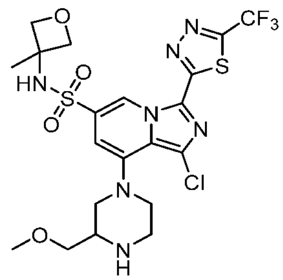 **1-chloro-8-(3-(methoxymethyl)piperazin-1-yl)-N-(3-methyloxetan-3-yl)-3-(5-(trifluoromethyl)-1,3,4-thiadiazol-2-yl)imidazo [1,5-a] pyridine-6-sulfonamide** | $^1H$ NMR (400 MHz, $CD_3OD$) δ 9.89 (s, 1H), 6.97 (s, 1H), 4.87 - 4.79 (m, 2H), 4.34 (d, $J$ = 6.7 Hz, 2H), 3.92 - 3.83 (m, 1H), 3.75 - 3.64 (m, 4H), 3.60 - 3.53 (m, 2H), 3.47 (s, 3H), 3.20 - 3.09 (m, 2H), 1.66 (s, 3H). LC/MS (ESI) (m/z): 582 $[M+H]^+$. |

(continued)

| Example | Structure and name | Analysis data |
|---|---|---|
| **Example 136** | 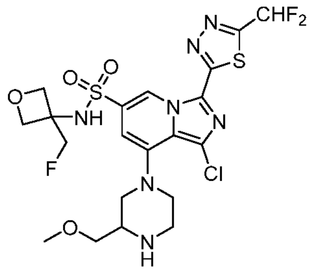 **1-chloro-3-(5-(difluoromethyl)-1,3,4-thiadiazol-2-yl)-N-(3-(fluoromethyl)oxetan-3-yl)-8-(3-(methoxymethyl)piperazin-1-yl)imidazo[1,5-a]pyridine-6-sulfonamide** | $^1$H NMR (400 MHz, DMSO-$d_6$) δ 9.59 (s, 1H), 7.68 (t, *J* = 53.1 Hz, 1H), 6.85 (s, 1H), 4.71 (s, 1H), 4.65 - 4.62 (m, 2H), 4.59 (s, 1H), 4.38 (d, *J* = 6.9 Hz, 2H), 3.64 - 3.41 (m, 3H), 3.28 (s, 3H), 3.19 - 3.15 (m, 3H), 3.07 - 3.02 (m, 2H), 2.76 - 2.71 (m, 1H). LC/MS (ESI) (m/z): 582 $[M+H]^+$. |
| **Example 137** | 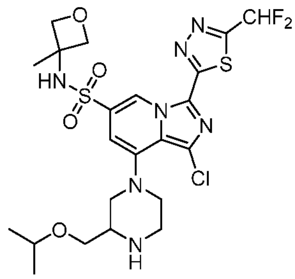 **1-chloro-3-(5-(difluoromethyl)-1,3,4-thiadiazol-2-yl)-8-(3-(isopropoxymethyl)piperazin-1-yl)-N-(3-methyloxetan-3-yl)imidazo [1,5-a]pyridine-6-sulfonamide** | $^1$H NMR (400 MHz, $CD_3OD$) δ 9.90 (s, 1H), 7.34 (t, *J* = 53.4 Hz, 1H), 6.93 (s, 1H), 4.80 - 4.78 (m, 2H), 4.36 - 4.32 (m, 2H), 3.91 - 3.80 (m, 2H), 3.75 - 3.66 (m, 4H), 3.60 - 3.52 (m, 2H), 3.23 - 3.10 (m, 2H), 1.65 (s, 3H), 1.24 (d, *J* = 5.9 Hz, 6H). LC/MS (ESI) (m/z): 592 $[M+H]^+$. |
| **Example 138** | 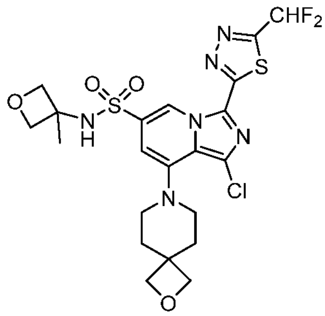 **1-chloro-3-(5-(difluoromethyl)-1,3,4-thiadiazol-2-yl)-N-(3-methyloxetan-3-yl)-8-(2-oxa-7-azaspiro[3.5]nonan-7-yl)imidazo [1,5-a]pyridine-6-sulfonamide** | $^1$H NMR (400 MHz, DMSO-$d_6$) δ 9.59 (s, 1H), 7.74 (d, *J* = 53.2 Hz, 1H), 6.82 (s, 1H), 4.63 (d, *J* = 6.2 Hz, 2H), 4.42 - 4.40 (m, 4H), 4.18 (d, *J* = 6.4 Hz, 2H), 3.37 - 3.36 (m, 4H), 2.08 - 2.06 (m, 2H), 1.49 (s, 3H), 1.25 - 1.23 (m, 2H). LC/MS (ESI) (m/z): 561 $[M+H]^+$. |

**Example 139: N-(1-cyanocyclopropyl)-3-(5-(difluoromethyl)-1,3,4-thiadiazol-2-yl)-8-(1-methyl-1H-benzo [d]imidazol-4-yl)imidazo [1,5-a]pyridine-6-sulfonamide**

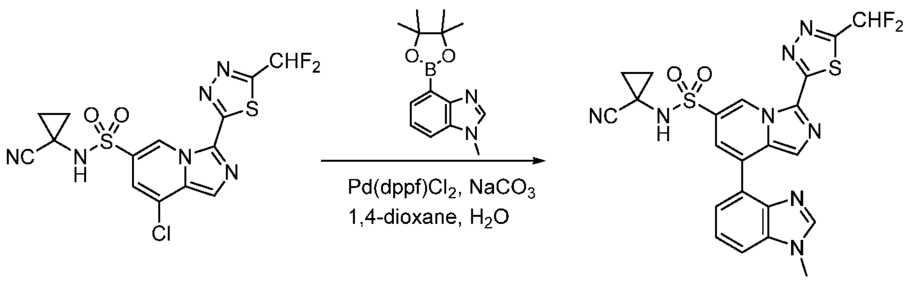

**Step 1: N-(1-cyanocyclopropyl)-3-(5-(difluoromethyl)-1,3,4-thiadiazol-2-yl)-8-(1-methyl-1H-benzo[d]imidazol-4-yl)imidazo[1,5-a]pyridine-6-sulfonamide**

Under an atmosphere of nitrogen at room temperature, sodium carbonate (19 mg, 0.18 mmol) and Pd(dppf)$Cl_2$ (5 mg, 0.01 mmol) were sequentially added to the solution of 8-chloro-N-(1-cyanocyclopropyl)-3-(5-(difluoromethyl)-1,3,4-thiadiazol-2-yl)imidazo[1,5-a]pyridin-6-sulfonamide (30 mg, 0.07 mmol) and 1-methyl-4-(4,4,5,5-tetramethyl-1,3,2-dioxoboracyclopentan-2-yl)-1H-benzo[d]imidazole (36 mg, 0.14 mmol) in 1,4-dioxane (2 mL) and water (0.4 mL). The flask was replaced with nitrogen gas three times and then the mixture was stirred at 85°C for 16 hours under an atmosphere of nitrogen. After completion of the reaction, the reaction mixture was diluted with ethyl acetate, sequentially washed with water and saturated brine, dried over anhydrous sodium sulfate, filtered, and concentrated under reduced pressure. The residue was purified by pre-HPLC to obtain the title compound (yellow solid, 3.0 mg, yield: 8.1%). $^1$H NMR (400 MHz, DMSO-$d_6$) δ 10.03 (s, 1H), 8.36 (s, 1H), 8.09 (s, 1H), 8.04 (s, 1H), 7.84 (d, *J* = 8.2 Hz, 1H), 7.76 - 7.57 (m, 2H), 7.48 (d, *J* = 1.3 Hz, 1H), 3.94 (s, 3H), 1.55 - 1.49 (m, 2H), 1.48 - 1.42 (m, 2H). LC/MS (ESI) m/z: 527 [M+H]$^+$.

**By referring to the preparation method in Example 139, the following examples were implemented by synthesizing a crude product according to a similar route by starting from appropriate starting materials, treating the crude product via reversed-phase HPLC, and then freeze-drying the crude product to obtain the title compound.**

| Example | Structure and name | Analysis data |
|---|---|---|
| **Example 140** | 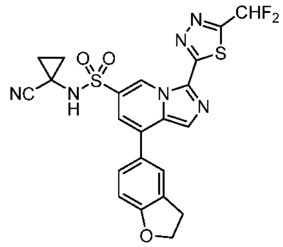 **N-(1-cyanocyclopropyl)-3-(5-(difluoromethyl)-1,3,4-thiadiazol-2-yl)-8-(2,3-dihydrobenzofuran-5-yl)imidazo [1,5-a]pyridine-6-sulfonamide** | $^1$H NMR (400 MHz, DMSO-d6) δ 9.98 (s, 1H), 9.60 (s, 1H), 8.05 (s, 1H), 7.74 (t, *J* = 28.3 Hz, 2H), 7.58 - 7.55 (m, 1H), 7.37 (d, *J* = 1.3 Hz, 1H), 7.01 (d, *J* = 8.3 Hz, 1H), 4.66 (t, *J* = 8.7 Hz, 2H), 3.32 - 3.29 (m, 2H), 1.53 - 1.49 (m, 2H), 1.43 - 1.39 (m, 2H). LC/MS (ESI) (m/z): 515 [M+H]$^+$. |
| **Example 141** | 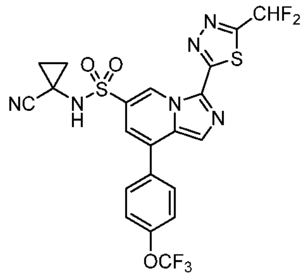 **N-(1-cyanocyclopropyl)-3-(5-(difluoromethyl)-1,3,4-thiadiazol-2-yl)-8-(4-(trifluoromethoxy)phenyl)imidazo [1,5-a]pyridine-6-sulfonamide** | $^1$H NMR (400 MHz, DMSO-$d_6$) δ 10.03 (s, 1H), 8.06 (s, 1H), 7.95 (d, *J* = 8.6 Hz, 2H), 7.85 - 7.57 (m, 3H), 7.46 (s, 1H), 1.54 - 1.46 (m, 2H), 1.45 - 1.37 (m, 2H). LC/MS (ESI) m/z: 557 [M+H]$^+$. |
| **Example 142** | 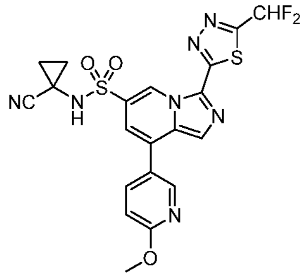 **N-(1-cyanocyclopropyl)-3-(5-(difluoromethyl)-1,3,4-thiadiazol-2-yl)-8-(6-methoxypyridin-3-yl)imidazo [1,5-a]pyridine-6-sulfonamide** | $^1$H NMR (400 MHz, DMSO-$d_6$) δ 9.99 (s, 1H), 8.63 (d, *J* = 2.5 Hz, 1H), 8.14 (dd, *J* = 8.6, 2.5 Hz, 1H), 8.06 (s, 1H), 7.70 (t, 1H), 7.43 (d, *J* = 1.0 Hz, 1H), 7.09 (d, *J* = 8.6 Hz, 1H), 3.97 (s, 3H), 1.47 - 1.43 (m, 2H), 1.37 - 1.34 (m, 2H). LC/MS (ESI) (m/z): 504 [M+H]$^+$. |

(continued)

| Example | Structure and name | Analysis data |
|---|---|---|
| **Example 143** | 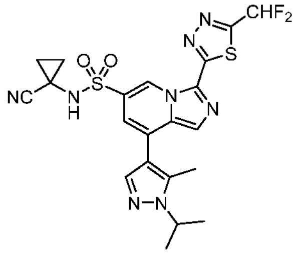<br>**N-(1-cyanocyclopropyl)-3-(5-(difluoromethyl)-1,3,4-thiadiazol-2-yl)-8-(1-isopropyl-5-methyl-1H-pyrazol-4-yl)imidazo [1,5-a] pyridine-6-sulfonamide** | $^1$H NMR (400 MHz, DMSO-$d_6$) δ 9.94 (s, 1H), 9.68 (s, 1H), 7.99 (s, 1H), 7.92 (s, 1H), 7.70 (t, *J* = 53.2 Hz, 1H), 7.20 (s, 1H), 4.71 - 4.64 (m, 1H), 2.45 (s, 3H), 1.60 - 1.30 (m, 10H). LC/MS (ESI) (m/z): 519 [M+H]$^+$. |

**Example 144: N-(1-cyanocyclopropyl)-3-(5-(difluoromethyl)-1,3,4-thiadiazol-2-yl)-8-((3R)-3-methyl-4-(1,1,1-trifluoro-3-hydroxypropan-2-yl)piperazin-1-yl)imidazo[1,5-a]pyridine-6-sulfonamide**

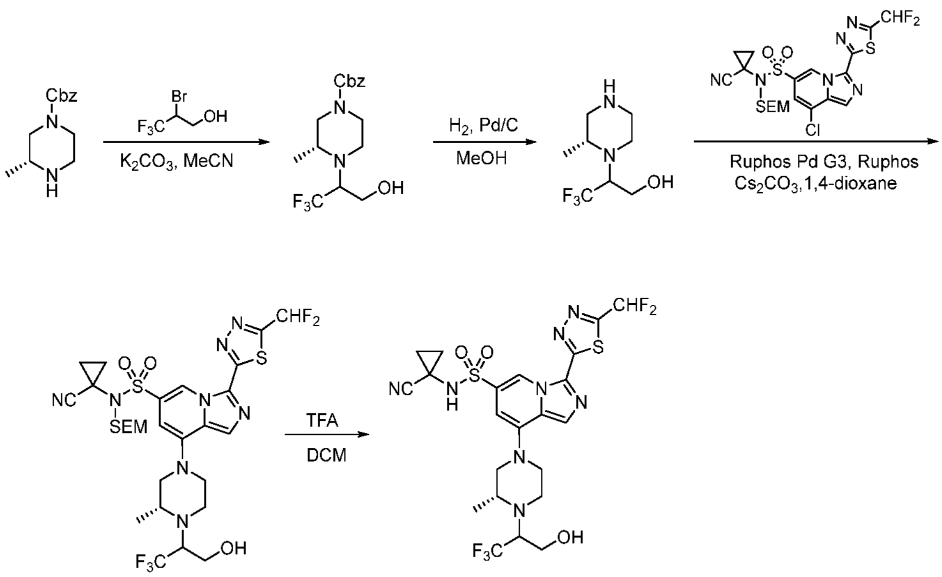


**Step 1: Benzyl (3R)-3-methyl-4-(1,1,1-trifluoro-3-hydroxypropan-2-yl)piperazine-1-carboxylate**

At 0°C, 2-bromo-3,3,3-trifluoropropan-1-ol (200 mg, 1.0 mmol) and potassium carbonate (420 mg, 3.0 mmol) were added to the solution of (R)-3-methylpiperazin-1-benzyl carboxylate (240 mg, 1.0 mmol) in MeCN (5 mL); and the reaction mixture was stirred at 50°C for 16 hours. The reaction mixture was diluted with ethyl acetate, washed with saturated brine, dried over anhydrous sodium sulfate, and concentrated under reduced pressure. The residue was separated by column chromatography (eluent: petroleum ether/ethyl acetate, gradient: 0-50% ethyl acetate) to obtain the title compound (white solid, 120 mg, yield: 34.6%). LC/MS (ESI) (m/z): 347 [M+H]$^+$.

**Step 2: 3,3,3-trifluoro-2-((R)-2-methylpiperazin-1-yl)propan-1-ol**

At room temperature, palladium on carbon (20 mg) with the mass fraction of 10% was added to the solution of (3R)-3-methyl-4-(1,1,1-trifluoro-3-hydroxypropan-2-yl)piperazin-1-benzyl carboxylate (120 mg, 0.35 mmol) in methanol (5 mL). The flask was replaced with nitrogen gas three times and then the mixture was stirred at room temperature for 3 hours under an atmosphere of hydrogen at one atmospheric pressure. After completion of the reaction, the reaction mixture was filtered; and the filtrate was concentrated under reduced pressure to obtain the title compound (colorless oil, 60 mg, yield: 80.1%). LC/MS (ESI) (m/z): 213 [M+H]$^+$.

**Step 3: N-(1-cyanocyclopropyl)-3-(5-(difluoromethyl)-1,3,4-thiadiazol-2-yl)-8-((3R)-3-methyl-4-(1,1,1-trifluoro-3-hydroxypropan-2-yl)piperazin-1-yl)-N-((2-(trimethylsilyl)ethoxy)methyl)imidazo[1,5-a]pyridine-6-sulfonamide**

Under an atmosphere of nitrogen, cesium carbonate (117 mg, 0.36 mmol), Ruphos (6 mg, 0.015 mmol) and Ruphos Pd G3 (7 mg, 0.015 mmol) were sequentially added to the solution of 8-chloro-N-(1-cyanocyclopropyl)-3-(5-(difluoromethyl)-1,3,4-thiadiazol-2-yl)-N-(2-(trimethylsilyl)ethoxy)methyl)imidazo[1,5-a]pyridin-6-sulfonamide (50 mg, 0.12 mmol) and (3,3,3-trifluoro-2-((R)-2-methylpiperazin-1-yl)propan-1-ol (23 mg, 0.18 mmol) in 1,4-dioxane (5 mL). The flask was replaced with nitrogen gas three times and then the mixture was stirred at 80°C for 4 hours under an atmosphere of nitrogen. After completion of the reaction, the reaction mixture was cooled to room temperature, diluted with ethyl acetate, washed with saturated brine, dried over anhydrous sodium sulfate, and concentrated under reduced pressure. The residue was separated by column chromatography (eluent: petroleum ether/ethyl acetate, gradient: 0-50% ethyl acetate) to obtain the title compound (yellow solid, 50 mg, yield: 56.8%). LC/MS (ESI) (m/z): 737 $[M+H]^+$.

**Step 4: N-(1-cyanocyclopropyl)-3-(5-(difluoromethyl)-1,3,4-thiadiazol-2-yl)-8-((3R)-3-methyl-4-(1,1,1-trifluoro-3-hydroxypropan-2-yl)piperazin-1-yl)imidazo[1,5-a]pyridine-6-sulfonamide**

At 0°C, TFA (1 mL) was added to the solution of N-(1-cyanocyclopropyl)-3-(5-(difluoromethyl)-1,3,4-thiadiazol-2-yl)-8-((3R)-3-methyl-4-(1,1,1-trifluoro-3-hydroxypropan-2-yl)piperazin-1-yl)-N-(2-(trimethylsilyl)ethoxy)methyl)imidazo [1,5-a]pyridin-6-sulfonamide (50 mg, 0.067 mmol) in DCM (2 mL); and the reaction mixture was stirred at room temperature for 30 minutes. The reaction mixture was diluted with dichloromethane, washed with saturated sodium bicarbonate solution, dried over anhydrous sodium sulfate, and concentrated under reduced pressure. The residue was purified by pre-HPLC to obtain the title compound (yellow solid, 9 mg, yield: 21.9%). $^1H$ NMR (400 MHz, DMSO-$d_6$) δ 9.66 (s, 1H), 8.01 (s, 1H), 7.75 (d, *J* = 53.2 Hz, 1H), 6.66 (s, 1H), 6.32 - 6.12 (m, 1H), 4.23 - 4.14 (m, 1H), 3.59 - 3.57 (m, 1H), 3.10 - 3.04 (m, 3H), 2.88 - 2.81 (m, 3H), 2.74 - 2.64 (m, 2H), 1.50 - 1.45 (m, 2H), 1.41 - 1.36 (m, 2H), 1.16 - 1.12 (m, 3H). LC/MS (ESI) (m/z): 607 $[M+H]^+$.

**Example 145: N-(1-cyanocyclopropyl)-3-(5-(difluoromethyl)-1,3,4-thiadiazol-2-yl)-8-(4-(hydroxymethyl)-4-(trifluoromethyl)piperidin-1-yl)imidazo [1,5-a] pyridine-6-sulfonamide**

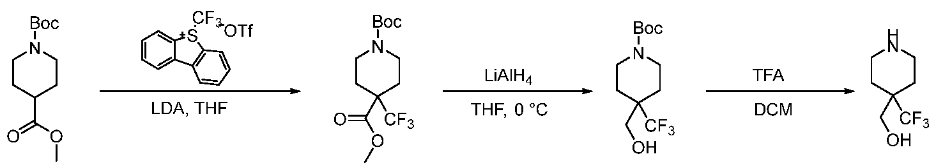


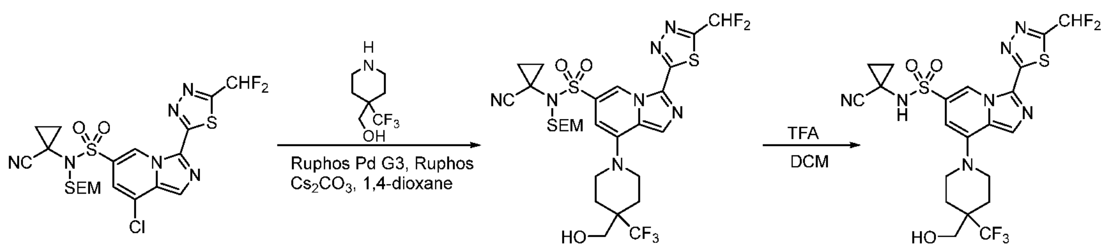


**Step 1: N-tert-butoxycarbonyl-4-trifluoromethylpiperidin-4-methyl formate1-(tert-butyl) 4-methyl 4-(trifluoromethyl)piperidine-1,4-dicarboxylate**

Under an atmosphere of nitrogen at -78°C, lithium diisopropylamide (6.17 mL, 6.17 mmol, 1 M in THF) was dropwise added slowly to the solution of 1-tert-butoxycarbonyl-4-methyl nipecotate (1 g, 4.11 mmol) in tetrahydrofuran (10 mL). The reaction mixture was stirred at -78°C for 45 minutes; then, S-(trifluoromethyl)dibenzothiophentrifluoromethyl sulfonate (2.48 g, 6.17 mmol) was added to the reaction system; the reaction mixture was allowed to gradually warm up to room temperature under an atmosphere of nitrogen, and then stirred for 16 hours. After completion of the reaction, aqueous ammonium chloride solution was added for quenching; the resulting mixture was extracted with ethyl acetate; the organic phase was washed with saturated brine, dried over anhydrous sodium sulfate, and concentrated under reduced

pressure. The residue was separated by column chromatography (eluent: petroleum ether/ethyl acetate, gradient: 0-20% ethyl acetate) to obtain the title compound (yellow oil, 150 mg, yield: 11.7 %). $^1$H NMR (400 MHz, $CDCl_3$) δ 4.24 - 4.01 (m, 2H), 3.83 (s, 3H), 2.83 - 2.62 (m, 2H), 2.31 - 2.27 (m, 2H), 1.77 - 1.70 (m, 2H), 1.45 (s, 9H).

**Step 2: Tert-butyl 4-(hydroxymethyl)-4-(trifluoromethyl)piperidine-1-carboxylate**

Under an atmosphere of nitrogen at 0°C, lithium aluminum hydride (37 mg, 0.96 mmol) was added to the solution of N-tert-butoxycarbonyl-4-trifluoromethylpiperidin-4-methyl formate (150 mg, 0.48 mmol) in tetrahydrofuran (3 mL). The reaction mixture was stirred at room temperature for 1 hours. After completion of the reaction, sodium sulfate decahydrate was added at 0°C; the mixture was stirred for 10 minutes, and filtered; and the filtrate was concentrated under reduced pressure. The residue was separated by column chromatography (eluent: petroleum ether/ethyl acetate, gradient: 0-30% ethyl acetate) to obtain the title compound (yellow oil, 130 mg, yield: 95.2 %). LC/MS (ESI) m/z: 284 $[M+H]^+$.

**Step 3: (4-(trifluoromethyl)piperidin-4-yl) methanol2,2,2- trifluoroacetate**

At 0°C, trifluoroacetic acid (0.2 mL) was added to the solution of 4-(hydroxymethyl)-4-(trifluoromethyl)piperidin-1-tert-butyl formate (130 mg, 0.46 mmol) in dichloromethane (1.5 mL). The reaction mixture was stirred at room temperature for 30 minutes. After completion of the reaction, the reaction mixture was concentrated under reduced pressure to obtain the title compound (yellow oil, 100 mg, yield: 73.5 %). LC/MS (ESI) m/z: 184 $[M+H]^+$.

**Step 4: N-(1-cyanocyclopropyl)-3-(5-(difluoromethyl)-1,3,4-thiadiazol-2-yl)-8-(4-(hydroxymethyl)-4-(trifluoromethyl)piperidin-1-yl)-N-((2-(trimethylsilyl)ethoxy)methyl)imidazo [1,5-a]pyridine-6-sulfonamide**

Under an atmosphere of nitrogen, cesium carbonate (191.6 mg, 0.6 mmol), Ruphos (18.3 mg, 0.04 mmol) and Ruphos Pd G3 (32.8 mg, 0.04 mmol) were sequentially added to the solution of 8-chloro-N-(1-cyanocyclopropyl)-3-(5-(difluoromethyl)-1,3,4-thiadiazol-2-yl)-N-((2-(trimethylsilyl)ethoxy)methyl)imidazo[1,5-a]pyridine-6-sulfonamide (110 mg, 0.2 mmol) and (4-(trifluoromethyl)piperidin-4-yl) methanol (71.8 mg, 0.4 mmol) in 1,4-dioxane (4 mL). The flask was replaced with nitrogen gas three times and then the mixture was stirred at 85°C for 3 hours under an atmosphere of nitrogen. After completion of the reaction, the reaction mixture was diluted with ethyl acetate, sequentially washed with water and saturated brine, dried over anhydrous sodium sulfate, and concentrated under reduced pressure. The residue was separated by column chromatography (eluent: petroleum ether, ethyl acetate, gradient: 0-30% ethyl acetate) to obtain the title compound (yellow solid, 90 mg, yield: 64.9%). LC/MS (ESI) (m/z): 708 $[M+H]^+$.

**Step 5: N-(1-cyanocyclopropyl)-3-(5-(difluoromethyl)-1,3,4-thiadiazol-2-yl)-8-(4-(hydroxymethyl)-4-(trifluoromethyl)piperidin-1-yl)imidazo[1,5-a]pyridine-6-sulfonamide**

At 0°C, trifluoroacetic acid (0.5 mL) 向 was dropwise added to the solution of (R) -N-(1-cyanocyclopropyl)-3-(5-(difluoromethyl)-1,3,4-thiadiazol-2-yl)-8-(3-methyl-4-pivaloyl piperazin-1-yl)-N-((2-(trimethylsilicyl)ethoxy) methyl)imidazo[1,5-a]pyridin-6-sulfonamide (90 mg, 0.13 mmol) in dichloromethane (1 mL). The reaction mixture was stirred at room temperature for 30 minutes. After completion of the reaction, the reaction mixture was neutralized with saturated aqueous sodium bicarbonate solution, and extracted with dichloromethane. The organic phase was washed with saturated brine, dried over anhydrous sodium sulfate, and concentrated under reduced pressure; and the residue was separated by pre-HPLC (C18, 10-50% acetonitrile in water containing 0.1% formic acid) to obtain the title compound (yellow solid, 28.3 mg, yield: 38.5%). $^1$H NMR (400 MHz, DMSO-$d_6$) δ 9.65 (s, 1H), 9.45 (s, 1H), 8.07 (s, 1H), 7.68 (t, $J$ = 52.9 Hz, 1H), 6.66 (s, 1H), 5.13 (s, 1H), 3.74 (d, $J$ = 4.3 Hz, 2H), 3.61 (d, $J$ = 12.6 Hz, 2H), 3.30 - 3.17 (m, 2H), 2.10 - 1.97 (m, 2H), 1.99 - 1.82 (m, 2H), 1.60 - 1.43 (m, 2H), 1.46 - 1.32 (m, 2H). LC/MS (ESI) (m/z): 578 $[M+H]^+$.

**Example 146: N-(1-cyanocyclopropyl)-8-(4-(3,3-difluorocyclobutane-1-carbonyl)piperazin-1-yl)-3-(5-(difluoromethyl)-1,3,4-thiadiazol-2-yl)imidazo[1,5-a]pyridine-6-sulfonamide**

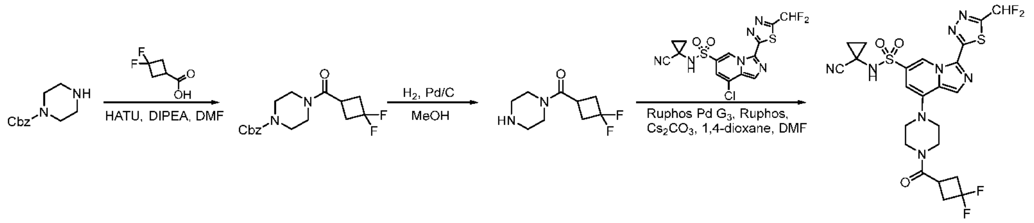


**Step 1: Benzyl 4-(3,3-difluorocyclobutane-1-carbonyl)piperazine-1-carboxylate**

At room temperature, 3,3-difluorocyclobutan-1-carboxylic acid (204 mg, 1.50 mmol), N,N-diisopropylethylamine (439 mg, 3.41 mmol) and 2-(7-azobenzotriazol)-N,N,N',N'-tetramethylurea hexafluorophosphate (569 mg, 1.50 mmol) was slowly added to the solution of piperazin-1-benzyl carboxylate (300 mg, 1.36 mmol) in N,N-dimethylformamide (4.5 mL). The mixture was stirred at room temperature for 2 hours under an atmosphere of nitrogen. After completion of the reaction, the reaction mixture was poured into water and extracted with ethyl acetate twice. The organic phase was washed with saturated brine, dried over anhydrous sodium sulfate, and concentrated under reduced pressure. The residue was separated by column chromatography (eluent: petroleum ether, ethyl acetate, gradient: 0-20% ethyl acetate) to obtain the title compound (colorless oil, 450 mg, yield: 97.7%). LC/MS (ESI) (m/z): 338 $[M+H]^+$.

**Step 2: (3,3-difluorocyclobutyl)(piperazin-1-yl)methanone**

Under an atmosphere of nitrogen, palladium on carbon (100 mg) with the mass fraction of 10% was added to the solution of 4-(3,3-difluorocyclobutan-1-carbonyl)piperazin-1-benzyl formate (450 mg, 1.33 mmol) in methanol (4 mL). The flask was replaced with nitrogen gas three times and then the mixture was stirred at room temperature for 1 hours under an atmosphere of hydrogen at one atmospheric pressure. After completion of the reaction, the reaction mixture was filtered; and the filtrate was concentrated under reduced pressure to obtain the title compound (colorless oil, 250 mg, yield: 92.0%). LC/MS (ESI) (m/z): 204 $[M+H]^+$.

**Step 3: N-(1-cyanocyclopropyl)-8-(4-(3,3-difluorocyclobutane-1-carbonyl)piperazin-1-yl)-3-(5-(difluoromethyl)-1,3,4-thiadiazol-2-yl)imidazo[1,5-a]pyridine-6-sulfonamide**

Uder a protective atmosphere of nitrogen, cesium carbonate (181.5 mg, 0.56 mmol), Ruphos (17 mg, 0.037 mmol) and Ruphos Pd G3 (30 mg, 0.037 mmol) were sequentially added to the solution of 8-chloro-N-(1-cyanocyclopropyl-3-(5-difluoromethyl)-1,3,4-thiadiazol-2-ylimidazo[1,5-a]pyridin-6-sulfonamide (80 mg, 0.19 mmol) and (3,3-difluorocyclobutyl)piperazin-1-ketone (76 mg, 0.37 mmol) in 1,4-dioxane (5 mL) and N,N-dimethylformamide (1.5 mL). The flask was replaced with nitrogen gas three times and then the mixture was stirred at 80°C for 10 minutes under an atmosphere of nitrogen. After completion of the reaction, the reaction mixture was cooled to room temperature, added with water, and extracted with ethyl acetate. The organic phase was washed with saturated brine, dried over anhydrous sodium sulfate, and concentrated under reduced pressure; and the residue was separated by pre-HPLC (C18, 10-50% acetonitrile in $H_2O$ and 0.1% formic acid) to obtain the title compound (yellow solid, 13.7 mg, yield: 12.3%). $^1H$ NMR (400 MHz, DMSO-$d_6$) δ 9.69 (s, 1H), 9.49 (s, 1H), 8.10 (s, 1H), 7.69 (t, $J$ = 53.2 Hz, 1H), 6.68 (s, 1H), 3.78 - 3.74 (m, 2H), 3.69 - 3.64 (m, 2H), 3.36 - 3.33 (m, 5H), 2.88 - 2.79 (m, 4H), 1.51 - 1.46 (m, 2H), 1.41 - 1.36 (m, 2H).LC/MS (ESI) (m/z): 599 $[M+H]^+$.

**Example 147: N-(1-cyanocyclopropyl)-3-(5-(difluoromethyl)-1,3,4-thiadiazol-2-yl)-8-(4-(oxetan-3-yl)piperazin-1-yl)imidazo[1,5-a]pyridine-6-sulfonamide**

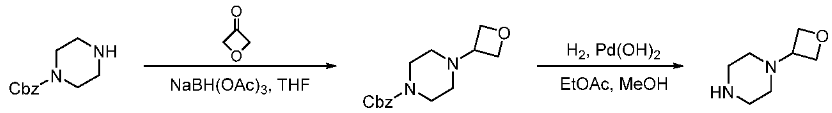


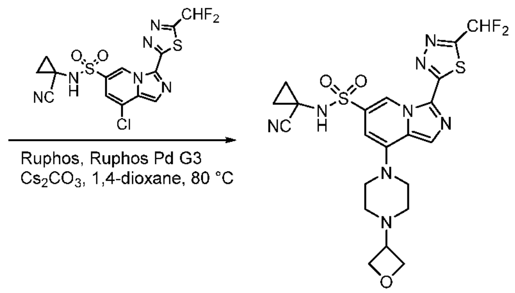


**Step 1: Benzyl 4-(oxetan-3-yl)piperazine-1-carboxylate**

Under an atmosphere of nitrogen at room temperature, 3-oxacyclobutanone (380 mg, 5.45 mmol) and acetic acid sodium borohydride (1.92 g, 9.08 mmol) were added to the solution of benzyl-1-piperazine carbonate (1 g, 4.54 mmol) in tetrahydrofuran (10 mL). The reaction mixture was stirred at room temperature for 16 hours under an atmosphere of nitrogen. After completion of the reaction, the reaction mixture was poured into ice water, and extracted with ethyl acetate; and the organic phase was washed with saturated sodium bicarbonate and brine, dried over anhydrous sodium sulfate, and concentrated under reduced pressure. The residue was separated by column chromatography (eluent: petroleum ether/ethyl acetate, gradient: 0-80% ethyl acetate) to obtain the title compound (yellow oil, 510 mg, yield: 40.6%). LC/MS (ESI) m/z: 277 $[M+H]^+$.

**Step 2: 1-(oxetan-3-yl)piperazine**

At 0°C, palladium hydroxide on carbon (50 mg, 10% wt) was added to benzyl 4-(oxacyclobutan-3-yl)piperazin-1-benzyl carboxylate (500 mg, 1.81 mmol) in the mixed solution of ethyl acetate (5 mL) and methanol (2.5 mL). The flask was replaced with nitrogen gas three times and then the mixture was stirred at room temperature for 3 hours under an atmosphere of hydrogen at one atmospheric pressure. After completion of the reaction, the reaction mixture was filtered; and the filtrate was concentrated under reduced pressure to obtain the title compound (yellow solid, 250 mg, yield: 97.1 %). LC/MS (ESI) m/z: 143 $[M+H]^+$.

**Step 3: N-(1-cyanocyclopropyl)-3-(5-(difluoromethyl)-1,3,4-thiadiazol-2-yl)-8-(4-(oxetan-3-yl)piperazin-1-yl)imidazo[1,5-a]pyridine-6-sulfonamide**

Under an atmosphere of nitrogen at room temperature, cesium carbonate (159 mg, 0.49 mmol), Ruphos (15 mg, 0.03 mmol) and Ruphos Pd G3 (14 mg, 0.02 mmol) were sequentially added to the solution of 8-chloro-N-(1-cyanocyclopropyl)-3-(5-(difluoromethyl)-1,3,4-thiadiazol-2-yl)imidazo[1,5-a]pyridin-6-sulfonamide (70 mg, 0.16 mmol) and 1-(oxacyclobutan-3-yl)piperazine (23 mg, 0.16 mmol) in 1,4-dioxane (2 mL). The reaction mixture was stirred at 80°C for 3 hours under an atmosphere of nitrogen. After completion of the reaction, the reaction mixture was cooled to room temperature, added with water, and extracted with ethyl acetate. The organic phase was washed with saturated brine, dried over anhydrous sodium sulfate, and concentrated under reduced pressure. The residue was separated by column chromatography (eluent: petroleum ether/ethyl acetate, gradient: 0-70% ethyl acetate) and pre-HPLC to obtain the title compound (yellow solid, 5 mg, yield: 5.7%). $^1$H NMR (400 MHz, DMSO-$d_6$) $\delta$ 9.67 (s, 1H), 8.02 (s, 1H), 7.68 (t, $J$ = 53.1 Hz, 1H), 6.67 (s, 1H), 4.60 (t, $J$ = 6.4 Hz, 2H), 4.52 (t, $J$ = 6.0 Hz, 2H), 3.57 - 3.56 (m, 1H), 3.36 - 3.30 (m, 4H), 2.58 - 2.52 (m, 4H), 1.49 - 1.43 (m, 2H), 1.42 - 1.36 (m, 2H). LC/MS (ESI) (m/z): 537 $[M+H]^+$.

**Example 148: N-(3-cyanooxetan-3-yl)-3-(5-(difluoromethyl)-1,3,4-thiadiazol-2-yl)-8-(4-isobutyrylpiperazin-1-yl) imidazo[1,5-a]pyridine-6-sulfonamide**

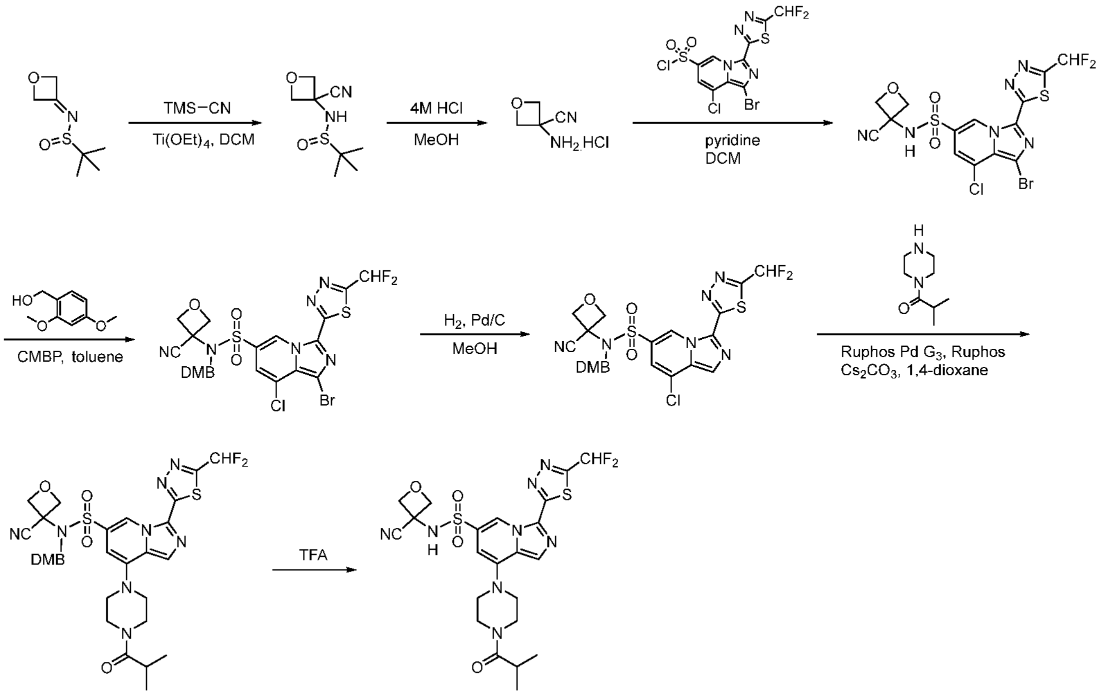


**Step 1: N-(3-cyanooxetan-3-yl)-2-methylpropane-2-sulfinamide**

Tetraethyl titanate (1.3 g, 5.7 mmol) was added to the solution of 2-methyl-N-(oxacyclobutan-3-ylidene)propan-2-sulfenamide (1 g, 5.7 mmol) in dichloromethane (20 mL). After adding, the reaction mixture was stirred for 10 minutes. Trimethylsilyl cyanide (1.5 mL, 11.4 mmol) was continued to be added to the above reaction mixture. The reaction mixture was stirred at room temperature overnight. After completion of the reaction, the reaction mixture was poured into saturated brine and filtered; the filtrate was extracted with dichloromethane three times; and the organic phase was washed with saturated brine, dried over anhydrous sodium sulfate, filtered and concentrated under reduced pressure. The residue was separated by column chromatography (eluent: dichloromethane/methanol, gradient: 0-4% methanol) to obtain the title compound (yellow liquid, 900 mg, yield: 78.3%). LC/MS (ESI) (m/z): 203 $[M+H]^+$.

**Step 2: 3-aminooxetane-3-carbonitrile hydrochloride**

At 0°C, the solution of 4M dioxane hydrochloride (1.5 mL, 6 mmol) was dropwise added slowly to the solution of N-(3-cyanooxacyclobutan-3-yl)-2-methylpropan-2-sulfenamide (400 mg, 2.0 mmol) in methanol (3 mL). The reaction mixture was stirred at 0°C for 30 minutes under an atmosphere of nitrogen. After completion of the reaction, the reaction mixture was concentrated under reduced pressure to obtain the title compound (white solid, 266 mg, yield: 100%), which was directly used in the next step without purification.

$^1$H NMR (400 MHz, DMSO-$d_6$) δ 4.90 (d, $J$ = 8.0 Hz, 2H), 4.85 (d, $J$ = 7.8 Hz, 2H).

**Step 3: 1-bromo-8-chloro-N-(3-cyanooxetan-3-yl)-3-(5-(difluoromethyl)-1,3,4-thiadiazol-2-yl)imidazo[1,5-a]pyridine-6-sulfonamide**

At 0°C, 1-bromo-8-chloro-3-(5-(difluoromethyl)-1,3,4-thiadiazol-2-yl)imidazo[1,5-a]pyridin-6-sulfonyl chloride (400 mg, 0.86 mmol) was added portionwise to the solution of 3-aminooxacyclobutan-3-formonitrile hydrochloride (232 mg, 1.72 mmol) in pyridine(2 mL); and the reaction mixture was stirred at room temperature for 30 minutes. The reaction mixture was acidified to pH=5 with 1N hydrochloric acid, and diluted with ethyl acetate; and the organic phase was washed with saturated brine, dried over anhydrous sodium sulfate, filtered and concentrated under reduced pressure. The residue was separated by column chromatography (eluent: petroleum ether/ethyl acetate, gradient: 0-50% ethyl acetate) to obtain the title compound (yellow solid, 280 mg, yield: 61.8%). LC/MS (ESI) (m/z): 525 $[M+H]^+$.

**Step 4: 1-bromo-8-chloro-N-(3-cyanooxetan-3-yl)-3-(5-(difluoromethyl)-1,3,4-thiadiazol-2-yl)-N-(3,4-dimethylbenzyl)imidazo[1,5-a]pyridine-6-sulfonamide**

2,4-dimethoxybenzyl alcohol (64 mg, 0.38 mmol) and cyanomethylenetri-n-butyl phosphine (138 mg, 0.57

mmol) were added to the solution of 1-bromo-8-chloro-N-(3-cyanooxacyclobutan-3-yl)-3-(5-(difluoromethyl)-1,3,4-thiadiazol-2-yl)imidazo[1,5-a]pyridine -6-sulfonamide (100 mg, 0.19 mmol) in toluene (2 mL). The reaction mixture was stirred at 100°C for 6 hours under an atmosphere of nitrogen. After completion of the reaction, the reaction mixture was concentrated under reduced pressure. The residue was separated by column chromatography (eluent: petroleum ether/ethyl acetate, gradient: 0-45% ethyl acetate) to obtain the title compound (yellow solid, 70 mg, yield: 54.5%). LC/MS (ESI) (m/z): 675 $[M+H]^+$.

**Step 5: 8-chloro-N-(3-cyanooxetan-3-yl)-3-(5-(difluoromethyl)-1,3,4-thiadiazol-2-yl)-N-(3,4-dimethylbenzyl)imidazo[1,5-a]pyridine-6-sulfonamide**

Under an atmosphere of nitrogen, 10% palladium on carbon (35 mg) was added to the solution of 1-bromo-8-chloro-N-(3-cyanooxacyclobutan-3-yl)-3-(5-(difluoromethyl)-1,3,4-thiadiazol-2-yl)-N-(2,4-dimethoxybenzyl)imidazo [1,5-a]pyridin-6-sulfonamide (70 mg, 0.10 mmol) in ethanol (2 mL); the flask was replaced with nitrogen gas three times and then the mixture was stirred at room temperature for 6 hours under an atmosphere of hydrogen. After completion of the reaction, the reaction mixture was filtered, and the filtrate was concentrated under reduced pressure. The residue was separated by column chromatography (eluent: petroleum ether/ethyl acetate, gradient: 0-45% ethyl acetate) to obtain the title compound (yellow solid, 30 mg, yield: 61.8%). LC/MS (ESI) (m/z): 597 $[M+H]^+$.

**Step 6: N-(3-cyanooxetan-3-yl)-3-(5-(difluoromethyl)-1,3,4-thiadiazol-2-yl)-N-(3,4-dimethylbenzyl)-8-(4-isobutyrylpiperazin-1-yl)imidazo[1,5-a]pyridine-6-sulfonamide**

Under an atmosphere of nitrogen at room temperature, cesium carbonate (33 mg, 0.10 mmol), Ruphos (5 mg, 0.01 mmol) and Ruphos Pd G3 (4 mg, 0.005 mmol) were added to the solution of 8-chloro-N-(3-cyanooxacyclobutan-3-yl)-3-(5-(difluoromethyl)-1,3,4-thiadiazol-2-yl)-N-(2,4-dimethoxybenzyl)imidazo[1,5-a]pyridin-6-sulfonamide (30 mg, 0.05 mmol) and 2-methyl-1-(piperazin-1-yl)propan-1-one (16 mg, 0.10 mmol) in 1,4-dioxane (2 mL). The reaction mixture was stirred at 90°C for 3 hours under an atmosphere of nitrogen. After completion of the reaction, the reaction mixture was cooled to room temperature; water was added; and the resulting mixture was extracted with ethyl acetate, washed with saturated brine, dried over anhydrous sodium sulfate, and concentrated under reduced pressure. The residue was separated by column chromatography (eluent: petroleum ether/ethyl acetate, gradient: 0-70% ethyl acetate) to obtain the title compound (yellow solid, 22 mg, yield: 61.4%). LC/MS (ESI) m/z: 717 $[M+H]^+$.

**Step 7: N-(3-cyanooxetan-3-yl)-3-(5-(difluoromethyl)-1,3,4-thiadiazol-2-yl)-8-(4-isobutyrylpiperazin-1-yl)imidazo[1,5-a]pyridine-6-sulfonamide**

At 0°C, trifluoroacetic acid (1 mL) was dropwise added slowly to the reaction flask containing N-(3-cyanooxacyclobutan-3-yl)-3-(5-(difluoromethyl)-1,3,4-thiadiazol-2-yl)-N-(2,4-dimethoxybenzyl)-8-(4-isobutylpiperazin-1-yl)imidazo[1,5-a ]pyridin-6-sulfonamide (22 mg, 0.03 mmol). The reaction mixture was stirred at room temperature for 48 hours. The reaction mixture was concentrated under reduced pressure; and the residue was purified by pre-HPLC to obtain the title compound (yellow solid, 2 mg, yield: 11.5%). $^1H$ NMR (400 MHz, DMSO-$d_6$) δ 9.64 (s, 1H), 8.10 (s, 1H), 7.86 - 7.47 (m, 1H), 6.66 (s, 1H), 4.87 (d, $J$ = 7.4 Hz, 2H), 4.77 (d, $J$ = 7.3 Hz, 2H), 3.80 - 3.69 (m, 4H), 3.40 - 3.35 (m, 4H), 2.98 - 2.90 (m, 1H), 1.04 (d, $J$ = 6.7 Hz, 6H). LC/MS (ESI) (m/z): 567 $[M+H]^+$.

**Example 149: 3-(5-(difluoromethyl)-1,3,4-thiadiazol-2-yl)-N-(3-(difluoromethyl)oxetan-3-yl)-8-(4-isobutyrylpiperazin-1-yl)imidazo[1,5-a]pyridine-6-sulfonamide**

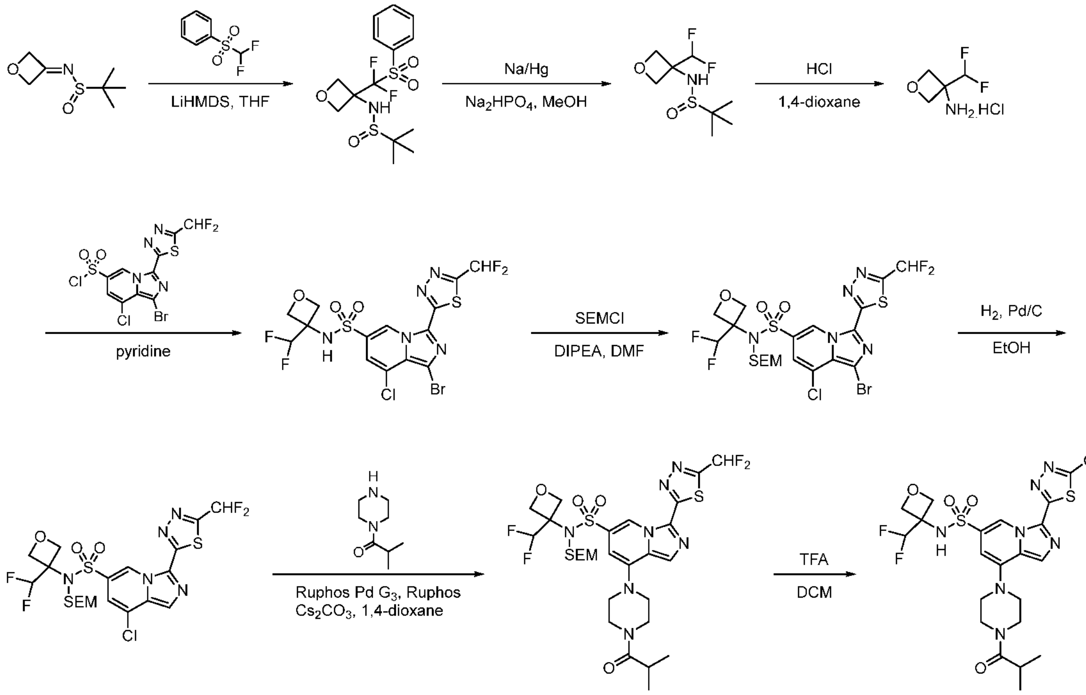


**Step 1: N-(3-(difluoro(phenylsulfonyl)methyl)oxetan-3-yl)-2-methylpropane-2-sulfinamide**

Under an atmosphere of nitrogen at -78°C, lithium bis(trimethylsilyl)amide (11.4 mL, 11.4 mmol, 1M) was dropwise added slowly to the solution of 2-methyl-N-(oxacyclobutan-3-propylidene)propan-2-sulfenamide (1 g, 5.7 mmol) and ((difluoromethyl)sulfonyl)benzene (1.1 g, 5.7 mmol) in tetrahydrofuran (20 mL). After addition, the reaction mixture was warmed up from -78°C to room temperature, and stirred for 3 hours. After completion of the reaction, the reaction mixture was quenched with ice water, and extracted with ethyl acetate three times. The organic phase was washed with saturated brine, dried over anhydrous sodium sulfate, filtered and concentrated under reduced pressure. The residue was separated by column chromatography (eluent: petroleum ether/ethyl acetate, gradient: 0-35% ethyl acetate) to obtain the title compound (yellow liquid, 1.4 g, yield: 66.8%). LC/MS (ESI) (m/z): 368 $[M+H]^+$.

**Step 2: N-(3-(difluoromethyl)oxetan-3-yl)-2-methylpropane-2-sulfinamide**

At room temperature, disodium hydrogen phosphate (5.4 g, 38 mmol) and 20% sodium amalgam (1.1 g, 9.5 mmol) were added to the solution of N-(3-(difluoro(benzenesulfonyl)methyl)oxacyclobutan-3-yl)-2-methylpropan-2-sulfenamide (1.4 g, 3.81 mmol) in methanol (20 mL). The reaction mixture was stirred for 2 hours under an atmosphere of nitrogen. After completion of the reaction, the reaction mixture was filtered and concentrated under reduced pressure. The residue was separated by column chromatography (eluent: petroleum ether/ethyl acetate, gradient: 0-40% ethyl acetate) to obtain the title compound (yellow liquid, 690 mg, yield: 79.7%). LC/MS (ESI) (m/z): 228 $[M+H]^+$.

**Step 3: 3-(difluoromethyl)oxetan-3-amine hydrochloride**

At 0°C, the solution of 4M dioxane hydrochloride (0.8 mL, 3.2 mmol) was dropwise added slowly to the solution of N-(3-(difluoromethyl)oxacyclobutan-3-yl)-2-methylpropan-2-sulfenamide (400 mg, 1.76 mmol) in methanol (3.5 mL). The reaction mixture was stirred at 0°C for 30 minutes under an atmosphere of nitrogen. After completion of the reaction, the reaction mixture was concentrated under reduced pressure to obtain the title compound (white solid, 281 mg, yield: 100%), which was directly used in the next step of reaction without purification. $^1H$ NMR (400 MHz, DMSO-$d_6$) δ 6.60 (t, $J$ = 54.2 Hz, 1H), 4.73 (d, $J$ = 8.1 Hz, 2H), 4.67 (d, $J$ = 8.0 Hz, 2H).

**Step 4: 1-bromo-8-chloro-3-(5-(difluoromethyl)-1,3,4-thiadiazol-2-yl)-N-(3-(difluoromethyl)oxetan-3-yl)imidazo [1,5-a]pyridine-6-sulfonamide**

At 0°C, 1-bromo-8-chloro-3-(5-(difluoromethyl)-1,3,4-thiadiazol-2-yl)imidazo[1,5-a]pyridin-6-sulfonyl chloride (300 mg, 0.65 mmol) was added portionwise to the solution of 3-(difluoromethyl)oxacyclobutan-3-amine hydrochloride (206 mg, 1.29 mmol) in pyridine (5 mL); and the reaction mixture was stirred at room temperature for 30 minutes. The

reaction mixture was acidified to pH-5 with 1N hydrochloric acid, and extracted with ethyl acetate. The organic phase was washed with saturated brine, dried over anhydrous sodium sulfate, filtered and concentrated under reduced pressure. The residue was separated by column chromatography (eluent: petroleum ether/ethyl acetate, gradient: 0-35% ethyl acetate) to obtain the title compound (yellow solid, 120 mg, yield: 33.7%). LC/MS (ESI) (m/z): 550 $[M+H]^+$.

**Step 5: 1-bromo-8-chloro-3-(5-(difluoromethyl)-1,3,4-thiadiazol-2-yl)-N-(3-(difluoromethyl)oxetan-3-yl)-N-((2-(trimethylsilyl)ethoxy)methyl)imidazo[1,5-a]pyridine-6-sulfonamide**

At 0°C, N, N-diisopropylethylamine (84 mg, 0.64 mmol) and 2-(trimethylsilanyl)ethoxymethyl chloride (73 mg, 0.44 mmol) were added to the solution of 1-bromo-8-chloro-3-(5-(difluoromethyl)-1,3,4-thiadiazol-2-yl)-N-(3-(difluoromethyl)oxacyclobutan-3-yl)imidazo[1,5-a]pyridin-6-sulfonamide (120 mg, 0.22 mmol) in DMF (2 mL). The reaction mixture was stirred at 0°C for 0.5 hour under an atmosphere of nitrogen. After completion of the reaction, water was added, and the reaction mixture was extracted with ethyl acetate, washed with saturated brine, dried over anhydrous sodium sulfate, and concentrated under reduced pressure. The residue was purified by column chromatography (eluent: petroleum ether/ethyl acetate, gradient: 0-25% ethyl acetate) to obtain the title compound (yellow solid, 100 mg, yield: 67.4%). LC/MS (ESI) m/z: 680 $[M+H]^+$.

**Step 6: 8-chloro-3-(5-(difluoromethyl)-1,3,4-thiadiazol-2-yl)-N-(3-(difluoromethyl)oxetan-3-yl)-N-((2-(trimethylsilyl)ethoxy)methyl)imidazo[1,5-a]pyridine-6-sulfonamide**

Under an atmosphere of nitrogen, 10% palladium on carbon (10 mg) was added to the solution of 1-bromo-8-chloro-3-(5-(difluoromethyl)-1,3,4-thiadiazol-2-yl)-N-(3-(difluoromethyl)oxacyclobutan-3-yl)-N-((2-(trimethylsilicyl) ethoxy)methyl)imidazo[1,5-a]pyridin-6-sulfonamide (100 mg, 0.15mmol) in ethanol (10 mL); the flask was replaced with nitrogen gas three times and then the mixture was stirred at room temperature for 6 hours under an atmosphere of hydrogen. After completion of the reaction, the reaction mixture was filtered, and the filtrate was concentrated under reduced pressure. The residue was separated by column chromatography (eluent: petroleum ether/ethyl acetate, gradient: 0-25% ethyl acetate) to obtain the title compound (yellow solid, 70 mg, yield: 79.2%). LC/MS (ESI) (m/z): 602 $[M+H]^+$.

**Step 7: 3-(5-(difluoromethyl)-1,3,4-thiadiazol-2-yl)-N-(3-(difluoromethyl)oxetan-3-yl)-8-(4-isobutyrylpiperazin-1-yl)-N-((2-(trimethylsilyl)ethoxy)methyl)imidazo[1,5-a]pyridine-6-sulfonamide**

Under an atmosphere of nitrogen at room temperature, cesium carbonate (76 mg, 0.23 mmol), Ruphos (11 mg, 0.02 mmol) and Ruphos Pd G3 (10 mg, 0.01 mmol) were added to the solution of 8-chloro-3-(5-(difluoromethyl)-1,3,4-thiadiazol-2-yl)-N-(3-(difluoromethyl)oxacyclobutan-3-yl)-N-((2-(trimethylsilicyl)) ethoxy)methyl)imidazo[1,5-a]pyridin-6-sulfonamide (70 mg, 0.12 mmol) and 2-methyl-1-(piperazin-1-yl)propan-1-one (36 mg, 0.23 mmol) in 1,4-dioxane (2 mL). The reaction mixture was stirred at 90°C for 3 hours under an atmosphere of nitrogen. After completion of the reaction, the reaction mixture was cooled to room temperature, added with water, and extracted with ethyl acetate, the organic phase was washed with saturated brine, dried over anhydrous sodium sulfate, and concentrated under reduced pressure. The residue was purified by column chromatography (eluent: petroleum ether/ethyl acetate, gradient: 0-50% ethyl acetate) to obtain the title compound (yellow solid, 60 mg, yield: 71.5%). LC/MS (ESI) m/z: 722 $[M+H]^+$.

**Step 8: 3-(5-(difluoromethyl)-1,3,4-thiadiazol-2-yl)-N-(3-(difluoromethyl)oxetan-3-yl)-8-(4-isobutyrylpiperazin-1-yl)imidazo[1,5-a]pyridine-6-sulfonamide**

At 0°C, trifluoroacetic acid (1 mL) was dropwise added slowly to the solution of 3-(5-(difluoromethyl)-1,3,4-thiadiazol-2-yl)-N-(3-(difluoromethyl)oxacyclobutan-3-yl)-8-(4-isobutyrylpiperazin-1-yl)-N-((2-(trimethylsilicyl)ethoxy) methyl)imidazo[1,5-a]pyridin-6-sulfonamide (60 mg, 0.08 mmol) in dichloromethane (1 mL). The reaction mixture was stirred at room temperature for 3 hours. After completion of the reaction, the reaction mixture was diluted with dichloromethane, washed with saturated sodium bicarbonate, dried over anhydrous sodium sulfate, and concentrated under reduced pressure. The residue was purified by pre-HPLC to obtain the title compound (yellow solid, 21 mg, yield: 42.7%). $^1H$ NMR (400 MHz, $CD_3OD$) δ 9.86 (s, 1H), 7.95 (s, 1H), 7.33 (t, $J$ = 53.5 Hz, 1H), 6.78 (d, $J$ = 1.2 Hz, 1H), 6.23 (t, $J$ = 55.6 Hz, 1H), 4.83 - 4.75 (m, 2H), 4.73 - 4.64 (m, 2H), 3.94 - 3.84 (m, 4H), 3.50 - 3.34 (m, 4H), 3.09 - 2.95 (m, 1H), 1.15 (d, $J$ = 6.7 Hz, 6H). LC/MS (ESI) (m/z): 592 $[M+H]^+$.

**Example 150: N-(1-cyanocyclopropyl)-3-(5-(difluoromethyl)-1,3,4-thiadiazol-2-yl)-8-((1-methylazetidin-3-yl)amino)imidazo[1,5-a]pyridine-6-sulfonamide**

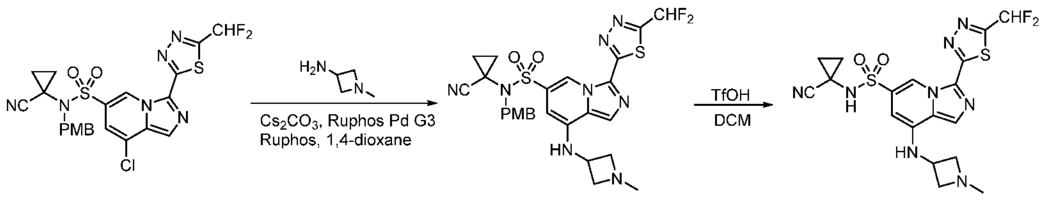


**Step 1: N-(1-cyanocyclopropyl)-3-(5-(difluoromethyl)-1,3,4-thiadiazol-2-yl)-N-(4-methoxybenzyl)-8-((1-methylazetidin-3-yl)amino)imidazo[1,5-a]pyridine-6-sulfonamide**

Under an atmosphere of nitrogen at room temperature, cesium carbonate (45 mg, 0.14 mmol), Ruphos (4 mg, 0.09 mmol) and Ruphos Pd G3 (4 mg, 0.05 mmol) were sequentially added to the mixed solution of 8-chloro-3-(5-(difluoromethyl)-1,3,4-thiadiazol-2-yl)-N-(1-(cyano)cyclopropyl)-N-(4-methoxybenzyl)imidazo[1,5-a]pyridin-6-sulfonamide (25 mg, 0.046 mmol) and 1-methylazacyclobutan-3-amine (6 mg, 0.07 mmol) in 1,4-dioxane (1 mL). The flask was replaced with nitrogen gas three times and then the mixture was stirred at 80°C for 4 hours under an atmosphere of nitrogen. After completion of the reaction, the reaction mixture was cooled to room temperature, quenched, and extracted with ethyl acetate; and the organic phase was washed with saturated brine, dried over anhydrous sodium sulfate, and concentrated under reduced pressure. The residue was separated by column chromatography (eluent: petroleum ether/ethyl acetate, gradient: 0-60% ethyl acetate) to obtain the title compound (yellow solid, 20 mg, yield: 74.1%). LC/MS (ESI) (m/z): 601 $[M+H]^+$.

**Step 2: N-(1-cyanocyclopropyl)-3-(5-(difluoromethyl)-1,3,4-thiadiazol-2-yl)-8-((1-methylazetidin-3-yl)amino)imidazo [1,5-a]pyridine-6-sulfonamide**

At 0°C, trifluoromethanesulfonic acid (0.1 mL) was added to the solution of N-(1-(cyano)cyclopropyl)-3-(5-(difluoromethyl)-1,3,4-thiadiazol-2-yl)-N-(4-methoxybenzyl)-8-((1-methylazacyclobutan-3-yl)amido)imidazo[1,5-a]pyridin-6-sulfonamide (20 mg, 0.03 mmol) in dichloromethane (2 mL); and the reaction mixture was stirred at 0°C for 2 hours. The reaction mixture was concentrated under reduced pressure; and the residue was purified by pre-HPLC to obtain the title compound (white solid, 8 mg, yield: 50.6%). $^1H$ NMR (400 MHz, DMSO-$d_6$) δ 9.43 (s, 1H), 8.12 (s, 1H), 7.81 - 7.55 (m, 2H), 6.07 (s, 1H), 4.49 - 4.42 (m, 1H), 4.33 - 4.25 (m, 2H), 3.85 - 3.74 (m, 2H), 2.76 (s, 3H), 1.49 - 1.45 (m, 2H), 1.41 - 1.37 (m, 2H). LC/MS (ESI) (m/z): 481 $[M+H]^+$.

**Example 151: N-(1-cyanocyclopropyl)-3-(5-(difluoromethyl)-1,3,4-thiadiazol-2-yl)-8-((3-(trifluoromethoxy)cyclopentyl)amino)imidazo [1,5-a] pyridine-6-sulfonamide**

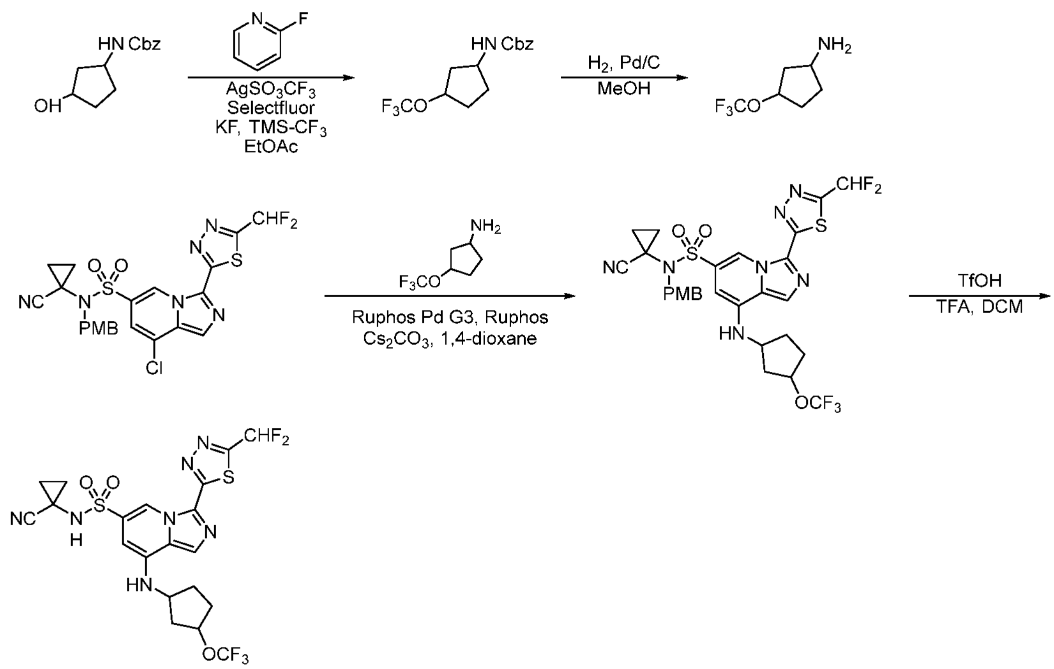


**Step 1: Benzyl (3-(trifluoromethoxy)cyclopentyl)carbamate**

Under an atmosphere of nitrogen at room temperature, silver trifluoromethanesulfonate (3.3 g, 12.8 mmol), 1-chloromethyl-4-fluoro-1,4-diazabicyclo[2.2.2]octane bis(tetrafluoroborate) (2.3 g, 6.4 mmol) and potassium fluoride were sequentially added to a sealed tube; and then, ethyl acetate (10 mL), 2-fluoropyridine (1.2 g, 12.8 mmol) and (trifluoromethyl)trimethylsilane (1.8 g, 12.8 mmol) were sequentially added. The reaction mixture was stirred in the sealed tube at room temperature for 16 hours. After completion of the reaction, the reaction mixture was filtered and concentrated under reduced pressure. The residue was separated by silica gel chromatography (eluent: petroleum ether/ethyl acetate, gradient: 0-10% ethyl acetate) to obtain the title compound (colorless liquid, 290 mg, yield: 22.5%).

**Step 2: 3-(trifluoromethoxy)cyclopentan-1-amine**

Under an atmosphere of nitrogen at room temperature, 10% palladium on carbon (50 mg) was added to the solution of (3-(trifluoromethoxy)cyclopentyl)aminobenzyl formate (290 mg, 0.96 mmol) in methanol (4 mL). The flask was replaced with nitrogen gas three times and then the mixture was stirred at room temperature for one hour under an atmosphere of hydrogen. After completion of the reaction, the reaction mixture was filtered; and the filtrate was concentrated under reduced pressure to obtain a crude title compound (colorless liquid, 90 mg, yield: 55.6%), which was directly used in the next step of reaction without purification.

**By referring to the preparation method in Example 135, Steps 3-4 were implemented according to a similar route by starting from appropriate starting materials to obtain the target compound.** N-(1-cyanocyclopropyl)-3-(5-(difluoromethyl)-1,3,4-thiadiazol-2-yl)-8-((3-(trifluoromethoxy)cyclopentyl)amido)imidazo[1,5-a]pyridin-6-sulfonamide (yellow solid). $^1$H NMR (400 MHz, DMSO-$d_6$) δ 9.41 (s, 1H), 9.37 (s, 1H), 8.21 (s, 1H), 7.82 - 7.54 (m, 1H), 7.21 (d, $J$ = 6.6 Hz, 1H), 6.20 (s, 1H), 4.98 - 4.93 (m, 1H), 4.01 - 3.95 (m, 1H), 2.69 (d, $J$= 7.5 Hz, 1H), 2.24 - 2.16 (m, 1H), 2.03 (d, $J$= 6.5 Hz, 2H), 1.93 - 1.81 (m, 2H), 1.48 - 1.44 (m, 2H), 1.39 - 1.34 (m, 2H). LC/MS (ESI) (m/z): 564 [M+H]$^+$.

**Example 152: (S)-N-(1-cyanocyclopropyl)-3-(5-(difluoromethyl)-1,3,4-thiadiazol-2-yl)-8-(3-(fluoromethyl)-4-(1-methylcyclopropane-1-carbonyl)piperazin-1-yl)imidazo[1,5-a]pyridine-6-sulfonamide**

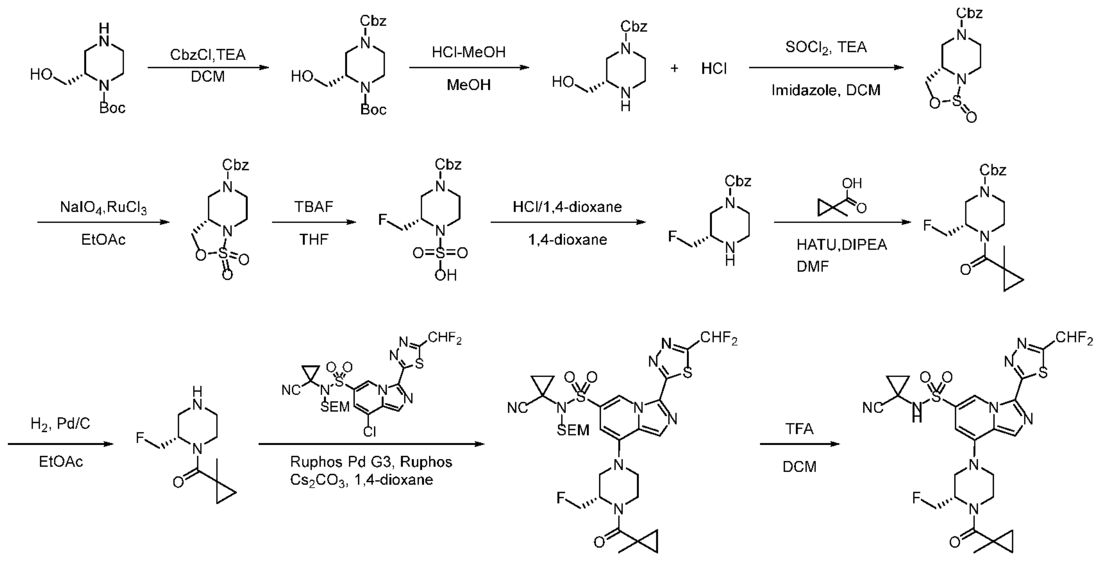


**Step 1: 4-benzyl 1-(tert-butyl) (S)-2-(hydroxymethyl)piperazine-1,4-dicarboxylate**

At 0°C, benzyl chloroformate (2.37 g, 13.8 mmol) and triethylamine (2.81 g, 27.7 mmol) were sequentially dropwise added to the solution of (S)-2-(hydroxymethyl)piperazin-1-tert-butyl carboxylate (2 g, 9.3 mmol) in dichloromethane (20 mL); and the reaction mixture was stirred at room temperature for 30 minutes. The reaction mixture was diluted with ethyl acetate, washed with saturated brine, dried over anhydrous sodium sulfate, and concentrated under reduced pressure. The residue was separated by column chromatography (eluent: petroleum ether/ethyl acetate, gradient: 0-60% ethyl acetate) to obtain the title compound (white solid, 3.1 g, yield: 95.67%). LC/MS (ESI) (m/z): 351 $[M+H]^+$.

**Step 2: Benzyl (S)-3-(hydroxymethyl)piperazine-1-carboxylate hydrochloride**

At 0°C, hydrochloric acid/methanol (5 mL, 4 mol/L) was added to the solution of 1-(tert-butyl)(S)-2-(hydroxymethyl)piperazine4-benzyl-1,4-dicarboxylate (1.0 g, 2.8 mmol) in methanol (5 mL). The reaction mixture was stirred at room temperature for one hour. After completion of the reaction, the reaction mixture was concentrated under reduced pressure to obtain a crude title compound (white solid, 800 mg, yield: 100%), which was directly used in the next step of reaction. LC/MS (ESI) (m/z): 251 $[M+H]^+$.

**Step 3: Benzyl (3aS)-tetrahydro-[1,2,3]oxathiazolo[3,4-a]pyrazine-5(3H)-carboxylate 1-oxide**

At 0°C, imidazole (8 mg, 0.6 mmol) was added to the solution of (S)-3-(hydroxymethyl)piperazin-1-benzyl carboxylate hydrochloride (800 mg, 2.8 mmol) in DCM (10 mL); and then, $SOCl_2$ (0.24 mL, 3.36 mmol) was dropwise added slowly. The reaction mixture was stirred at room temperature for 2 hours. After completion of the reaction, 10 mL of ice water was added to the reaction mixture, and stirred at for 10 minutes. The organic phase and the aqueous phase were separated; and the organic phase was dried over anhydrous sodium sulfate, filtered, and concentrated under reduced pressure. The residue was separated by column chromatography (eluent: petroleum ether/ethyl acetate, gradient: 0-35% ethyl acetate) to obtain the title compound (yellow oil, 500 mg, yield: 60.3%). LC/MS (ESI) (m/z): 297 $[M+H]^+$.

**Step 4: Benzyl (S)-tetrahydro-[1,2,3]oxathiazolo[3,4-a]pyrazine-5(3H)-carboxylate 1,1-dioxide**

At 0°C, (3aS)-tetrahydro-[1,2,3]oxathiazolo[3,4-a]pyrazin-5(3H)-benzyl carboxylate-1-oxide (300 mg, 1.0 mmol) was dissolved in the solution of ethyl acetate (3 mL); acetonitrile (8 mL), water (8 mL) and ruthenium trichloride (5 mg, 0.02 mmol) were added; and then, sodium periodate (540 mg, 2.5 mmol) was added portionwise. The reaction mixture was stirred at 0°C for 2 hours. After completion of the reaction, the reaction mixture was diluted with ethyl acetate, sequentially washed with water and saturated brine, dried over anhydrous sodium sulfate, and concentrated under reduced pressure. The residue was separated by column chromatography (eluent: petroleum ether/ethyl acetate, gradient: 0-35% ethyl acetate) to obtain the title compound (yellow oil, 270 mg, yield: 85.4%). LC/MS (ESI) (m/z): 313 $[M+H]^+$.

**Step 5: (S)-4-((benzyloxy)carbonyl)-2-(fluoromethyl)piperazine-1-sulfonic acid**

At room temperature, tetrabutylammonium fluoride (360 mg, 2.2 mmol) was added to the solution of (S)-tetrahydro-[1,2,3]oxathiazolo[3,4-a]pyrazin-5(3H)-benzyl carboxylate1,1-dioxide (270 mg, 0.87 mmol) in tetrahydrofuran (8 mL); and the reaction mixture was stirred at 65-70°C for 3 hours. The reaction mixture was concentrated under reduced pressure to obtain a crude title compound (yellow oil, 400 mg, yield: 100%), which was directly used in the next step of reaction.

**Step 6: Benzyl (S)-3-(fluoromethyl)piperazine-1-carboxylate**

At 0°C, hydrogen chloride /1,4-dioxane (4 mL, 4 mol/L) was added to the solution of (S)-4-((benzyloxy) carbonyl)-2-(fluoromethyl)piperazin-1-sulfonic acid (400 mg, 0.87 mmol) in 1,4-dioxane (5 mL); and the reaction mixture was stirred at room temperature for one hour. The reaction mixture was poured into ice water, and washed twice with methyltert-butyl ether. The aqueous phase was alkalized to pH 8-9 with 1N sodium hydroxide aqueous solution, and then extracted with dichloromethane twice. The organic phase was dried over anhydrous sodium sulfate, and concentrated under reduced pressure to obtain the title compound (yellow oil, 210 mg, yield: 95.8%). LC/MS (ESI) (m/z): 253 $[M+H]^+$.

**Step 7: Benzyl (S)-3-(fluoromethyl)-4-(1-methylcyclopropane-1-carbonyl)piperazine-1-carboxylate**

At 0°C, 1-methylcyclopropan-1-carboxylic acid (107 mg, 1.07mmol), N,N-diisopropylethylamine (416 mg, 2.6 mmol) and HATU (380 mg, 1.5 mmol) were added to the solution of (S)-3-(fluoromethyl)piperazin-1-benzyl carboxylate (210 mg, 0.83 mmol) in N,N- (5 mL); and the reaction mixture was stirred at room temperature for 30 minutes. The reaction mixture was diluted with ethyl acetate, washed with water and saturated brine in sequence, dried over anhydrous sodium sulfate, and concentrated under reduced pressure. The residue was separated by column chromatography (eluent: petroleum ether/ethyl acetate, gradient: 0-25% ethyl acetate) to obtain the title compound (white solid, 50 mg, yield: 18.0%). LC/MS (ESI) (m/z): 335 $[M+H]^+$.

**Step 8: (S)-(2-(fluoromethyl)piperazin-1-yl)(1-methylcyclopropyl)methanone**

Under an atmosphere of nitrogen, palladium on carbon (10 mg) with the mass fraction of 10% was added to the solution of (S)-3-(fluoromethyl)-4-(1-methylcyclopropan-1-carbonyl)piperazin-1-benzyl carboxylate (50 mg, 0.15 mmol) in ethyl acetate (3 mL). The flask was replaced with nitrogen gas three times and then the mixture wasstirred at room temperature for 2 hours under an atmosphere of hydrogen at one atmospheric pressure. After completion of the reaction, the reaction mixture was filtered; and the filtrate was concentrated under reduced pressure to obtain the title compound (colorless oil, 25 mg, yield: 83.5%). LC/MS (ESI) (m/z): 201 $[M+H]^+$.

**Step 9: (S)-N-(1-cyanocyclopropyl)-3-(5-(difluoromethyl)-1,3,4-thiadiazol-2-yl)-8-(3-(fluoromethyl)-4-(1-methylcyclopropane-1-carbonyl)piperazin-1-yl)-N-((2-(trimethylsilyl)ethoxy)methyl)imidazo[1,5-a]pyridine-6-sulfonamide**

Under an atmosphere of nitrogen, cesium carbonate (87 mg, 0.27mmol), Ruphos (8 mg, 0.02 mmol) and Ruphos Pd G3 (8 mg, 0.01 mmol) were sequentially added to the solution of 8-chloro-N-(1-cyanocyclopropyl)-3-(5-(difluoromethyl)-1,3,4-thiadiazol-2-yl) -N-(2-(trimethylsilyl)ethoxy)methyl)imidazo[1,5-a]pyridin-6-sulfonamide (50 mg, 0.09 mmol) and (S)-(2-(fluoromethyl)piperazin-1-yl)(1-methylcyclopropyl)ketone (25 mg, 0.13 mmol) in 1,4-dioxane (2 mL). The flask was replaced with nitrogen gas three times and then the mixture was stirred at 80°C for 4 hours under an atmosphere of nitrogen. After completion of the reaction, the reaction mixture was cooled to room temperature, diluted with ethyl acetate, washed with saturated brine, dried over anhydrous sodium sulfate, and concentrated under reduced pressure. The residue was separated by column chromatography (eluent: petroleum ether/ethyl acetate, gradient: 0-50% ethyl acetate) to obtain the title compound (yellow solid, 40 mg, yield: 61.9%). LC/MS (ESI) (m/z): 725 $[M+H]^+$.

**Step 10: (S)-N-(1-cyanocyclopropyl)-3-(5-(difluoromethyl)-1,3,4-thiadiazol-2-yl)-8-(3-(fluoromethyl)-4-(1-methylcyclopropane-1-carbonyl)piperazin-1-yl)imidazo[1,5-a]pyridine-6-sulfonamide**

At 0°C, trifluoroacetic acid (0.5 mL) was added to the solution of (S)-N-(1-cyanocyclopropyl)-3-(5-(difluoromethyl)-1,3,4-thiadiazol-2-yl)-8-(3-(fluoromethyl)-4-(1-methylcyclopropan-1-carbonyl)piperazin-1-yl)-N-(2-(trimethylsilyl)ethoxy)methyl)imidazo[1,5-a]pyridin-6-sulfonamide (40 mg, 0.06 mmol) in dichloromethane (1 mL); and the reaction mixture was stirred at room temperature for 30 minutes. The reaction mixture was diluted with dichloromethane, washed with saturated sodium bicarbonate solution, dried over anhydrous sodium sulfate, and concentrated under reduced

pressure. The residue was purified by pre-HPLC to obtain the title compound (yellow solid, 6.5 mg, yield: 19.8%). $^1$H NMR (400 MHz, DMSO-$d_6$) δ 971 (s, 1H), 9.49 (s, 1H), 7.98 (s, 1H), 7.82 - 7.55 (m, 1H), 6.74 (s, 1H), 4.91 - 4.78 (m, 2H), 4.36 - 4.27 (m, 1H), 3.87 - 3.81 (m, 1H), 3.75 - 3.70 (m, 1H), 3.35 - 3.32 (m, 1H), 3.14 - 3.00 (m, 3H), 1.52 - 1.48 (m, 2H), 1.41 - 1.37 (m, 2H), 1.30 (s, 3H), 0.93 - 0.87 (m, 2H), 0.67 - 0.58 (m, 2H). LC/MS (ESI) (m/z): 595 [M+H]$^+$.

**Example 153: 3-(5-(difluoromethyl)-1,3,4-thiadiazol-2-yl)-N-(3-(fluoromethyl)oxetan-3-yl)-8-(4-isobutyrylpiperazin-1-yl)imidazo[1,5-a]pyridine-6-sulfonamide-1-d**

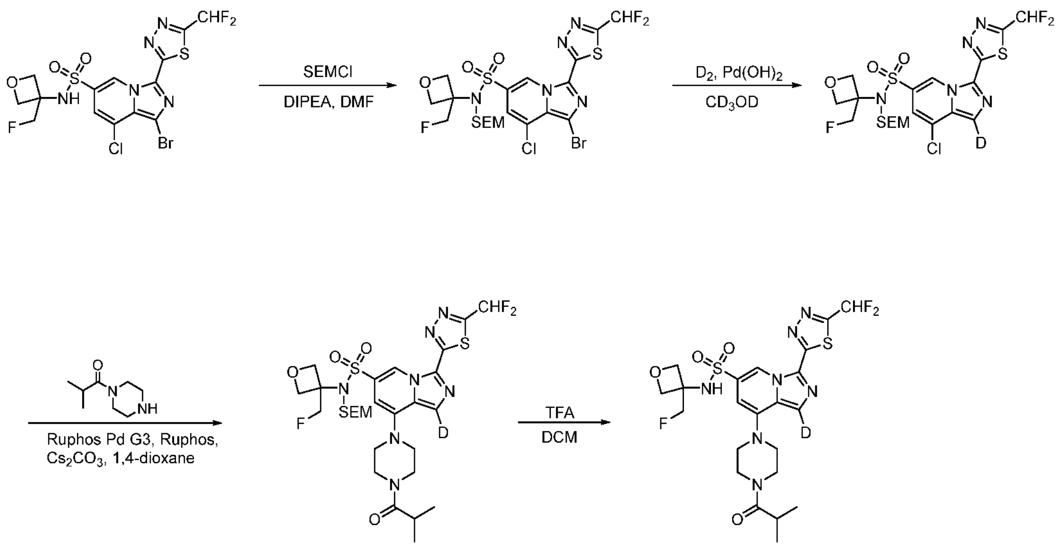


**Step 1: 1-bromo-8-chloro-3-(5-(difluoromethyl)-1,3,4-thiadiazol-2-yl)-N-(3-(fluoromethyl)oxetan-3-yl)-N-((2-(trimethylsilyl)ethoxy)methyl)imidazo[1,5-a]pyridine-6-sulfonamide**

At 0°C, N,N-diisopropylethylamine (105 mg, 0.81 mmol) and 2-(trimethylsilanyl)ethoxymethyl chloride (67 mg, 0.40 mmol) were sequentially added to the solution of 1-bromo-8-chloro-3-(5-(difluoromethyl)-1,3,4-thiadiazol-2-yl)-N-(3-(fluoromethyl)oxacyclobutan-3-yl)imidazo[1,5-a]pyridin-6-sulfonamide (145 mg, 0.27 mmol) in N,N-dimethylformamide (2 mL). The reaction mixture was stirred at room temperature for one hour. After completion of the reaction, water was added, and the reaction mixture was extracted with ethyl acetate. The organic phase was washed with saturated brine, dried over anhydrous sodium sulfate, and concentrated under reduced pressure. The residue was pulped with petroleum ether to obtain the title compound (yellow solid, 143 mg, yield: 79.4%). LC/MS (ESI) (m/z): 662 [M+H]+.

**Step 2: 8-chloro-3-(5-(difluoromethyl)-1,3,4-thiadiazol-2-yl)-N-(3-(fluoromethyl)oxetan-3-yl)-N-((2-(trimethylsilyl)ethoxy)methyl)imidazo[1,5-a]pyridine-6-sulfonamide-1-d**

Under an atmosphere of nitrogen, palladium hydroxide on carbon (10 mg) with the mass fraction of 20% was added to the solution of 1-bromo-8-chloro-3-(5-(difluoromethyl)-1,3,4-thiadiazol-2-yl)-N-(3-(fluoromethyl)oxacyclobutan-3-yl)-N-((2-(trimethylsilicyl)ethoxy)methyl)imidazo[1,5-a]pyridin-6-sulfonamide (65 mg, 0.62 mmol) in deutero methanol (5 mL). The flask was replaced with nitrogen gas three times and then the mixture was stirred at 20 °C for one hour under an atmosphere of deuterium at one atmospheric pressure. After completion of the reaction, the reaction mixture was filtered; and the filtrate was concentrated under reduced pressure to obtain the title compound (yellow solid, 40 mg, yield: 69.6%). LC/MS (ESI) (m/z): 585 [M+H]$^+$.

**Step 3: 3-(5-(difluoromethyl)-1,3,4-thiadiazol-2-yl)-N-(3-(fluoromethyl)oxetan-3-yl)-8-(4-isobutyrylpiperazin-1-yl)-N-((2-(trimethylsilyl)ethoxy)methyl)imidazo[1,5-a]pyridine-6-sulfonamide-1-d**

Under an atmosphere of nitrogen, cesium carbonate (44 mg, 0.13mmol), Ruphos (6 mg, 0.014 mmol), and Ruphos Pd G3 (11 mg, 0.014 mmol) were sequentially added to the solution of 8-chloro-3-(5-(difluoromethyl)-1,3,4-thiadiazol-2-yl)-N-(3-(fluoromethyl)oxacyclobutan-3-yl)-N-((2-(trimethylsilicyl))ethoxy)methyl)imidazo[1,5-a]pyridin-6-sulfonamide-1-deuterium (40 mg, 0.068 mmol) and 2-methyl-1-(piperazin-1-yl)propan-1-one (21 mg, 0.13 mmol) in 1,4-dioxane (2 mL). The flask was replaced with nitrogen gas three times and then the mixture was stirred at 80 °C for 30 minutes under an atmosphere of nitrogen. After completion of the reaction, water was added, and the reaction mixture was extracted with ethyl acetate. The organic phase was washed with saturated brine, dried over anhydrous sodium sulfate, and concentrated under reduced pressure. The residue was purified by column chromatography (eluent: petroleum ether, ethyl acetate, gradient: 0-70% ethyl acetate) to obtain the title compound (yellow solid, 44 mg, yield: 91.6%). LC/MS (ESI) (m/z): 705 $[M+H]^+$.

**Step 4: 3-(5-(difluoromethyl)-1,3,4-thiadiazol-2-yl)-N-(3-(fluoromethyl)oxetan-3-yl)-8-(4-isobutyrylpiperazin-1-yl)imidazo[1,5-a]pyridine-6-sulfonamide-1-d**

At 0°C, trifluoroacetic acid (0.2 mL) was added to the solution of 3-(5-(difluoromethyl)-1,3,4-thiadiazol-2-yl)-N-(3-(fluoromethyl)oxacyclobutan-3-yl)-8-(4-isobutyrylpiperazin-1-yl)-N-((2-(trimethylsilicyl)ethoxy)methyl)imidazo [1,5-a]pyridin-6-sulfonamide-1-deuterium (15 mg, 0.02 mmol) in dichloromethane (0.6 mL). The reaction mixture was stirred at 0°C for one hour. After completion of the reaction, the reaction mixture was dropped to 5% aqueous sodium bicarbonate solution that was cooled with ice water, and extracted with dichloromethane. The organic phase was washed with saturated brine, dried over anhydrous sodium sulfate, and concentrated under reduced pressure. The residue was separated by pre-HPLC (C18, 10-50% acetonitrile in $H_2O$ and 0.1% formic acid) to obtain the title compound (yellow solid, 3 mg, yield: 24.5%). $^1H$ NMR (400 MHz, $CD_3OD$) δ 9.85 (s, 1H), 7.33 (t, *J* = 53.5 Hz, 1H), 6.78 (s, 1H), 4.82 - 4.79 (m, 2H), 4.78 (s, 1H), 4.66 (s, 1H), 4.47 (d, *J* = 7.1 Hz, 2H), 3.92 - 3.86 (m, 4H), 3.44 - 3.41 (m, 2H), 3.37 - 3.34 (m, 2H), 3.07 - 2.99 (m, 1H), 1.15 (d, *J* = 6.7 Hz, 6H). LC/MS (ESI) (m/z): 575 $[M+H]^+$.

**Example 154: 3-(5-(difluoromethyl)-1,3,4-thiadiazol-2-yl)-7-fluoro-N-(3-(fluoromethyl)oxetan-3-yl)-8-(4-isobutyrylpiperazin-1-yl)imidazo[1,5-a]pyridine-6-sulfonamide-1-d**

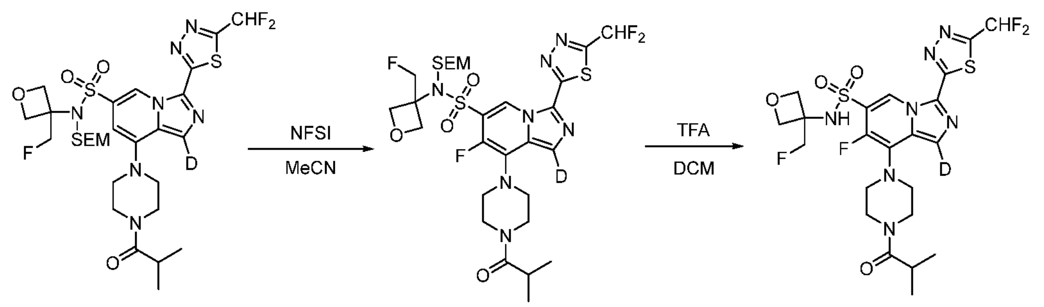


**Step 1: 3-(5-(difluoromethyl)-1,3,4-thiadiazol-2-yl)-7-fluoro-N-(3-(fluoromethyl)oxetan-3-yl)-8-(4-isobutyrylpiperazin-1-yl)-N-((2-(trimethylsilyl)ethoxy)methyl)imidazo[1,5-a]pyridine-6-sulfonamide-1-d**

Under an atmosphere of nitrogen, N-fluorodibenzene sulfonamide (16 mg, 0.05 mmol) was added to the solution of 3-(5-(difluoromethyl)-1,3,4-thiadiazol-2-yl)-N-(3-(fluoromethyl)oxacyclobutan-3-yl)-8-(4-isobutyrylpiperazin-1-yl)-N-((2-(trimethylsilicyl)ethoxy)methyl)imidazo[1,5-a]pyridin-6-sulfonamide-1-deuterium (30 mg, 0.04 mmol) in acetonitrile (0.7 mL). The reaction mixture was stirred at room temperature for 1 hours. After completion of the reaction, the reaction mixture was diluted with ethyl acetate; and the organic phase was washed with saturated brine, dried over anhydrous sodium sulfate, filtered and concentrated under reduced pressure. The residue was separated by column chromatography (eluent: petroleum ether/ethyl acetate, gradient: 0-35% ethyl acetate) to obtain the title compound (yellow solid, 12 mg, yield: 39.0%). LC/MS (ESI) (m/z): 605 $[M+H]^+$.

**Step 2: 3-(5-(difluoromethyl)-1,3,4-thiadiazol-2-yl)-7-fluoro-N-(3-(fluoromethyl)oxetan-3-yl)-8-(4-isobutyrylpiperazin-1-yl)imidazo[1,5-a]pyridine-6-sulfonamide-1-d**

At 0°C, trifluoroacetic acid (0.2 mL) was dropwise added slowly to the solution of 3-(5-(difluoromethyl)-1,3,4-thiadiazol-2-yl)-7-fluoro-N-(3-(fluoromethyl)oxacyclobutan-3-yl)-8-(4-isobutyrylpiperazin-1-yl)-N-((2-(trimethylsilicyl) ethoxy)methyl)imidazo[1,5-a]pyridin-6-sulfonamide-1-deuterium (12 mg, 0.02 mmol) in dichloromethane (0.6 mL). The reaction mixture was stirred at room temperature for 0.5 hour. The reaction mixture was concentrated under reduced pressure; and the residue was purified by pre-HPLC to obtain the title compound (yellow solid, 1.1 mg, yield: 11.2%). $^1H$ NMR (400 MHz, $CD_3OD$) δ 9.89 (d, *J* = 5.4 Hz, 1H), 7.33 (t, *J* = 53.5 Hz, 1H), 4.85 - 4.82 (m, 2H), 4.79 (s, 1H), 4.67 (s, 1H),

4.51 - 4.46 (m, 2H), 3.89 - 3.77 (m, 4H), 3.50 - 3.41 (m, 4H), 3.07 - 2.98 (m, 1H), 1.16 (d, *J* = 6.7 Hz, 6H). LC/MS (ESI) (m/z): 593 $[M+H]^+$.

**Example 155: 3-(5-(difluoromethyl)-1,3,4-thiadiazol-2-yl)-7-fluoro-8-(4-isobutyrylpiperazin-1-yl)-N-(3-methyloxetan-3-yl)imidazo[1,5-a]pyridine-6-sulfonamide-1-d**

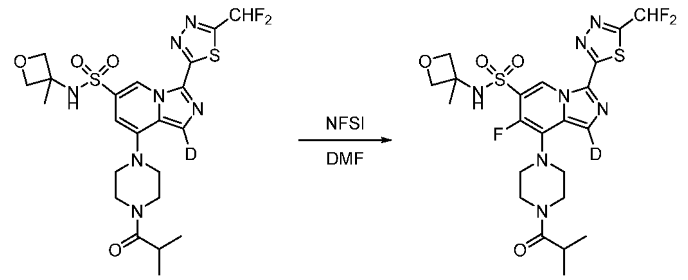


**Step 1: 3-(5-(difluoromethyl)-1,3,4-thiadiazol-2-yl)-7-fluoro-8-(4-isobutyrylpiperazin-1-yl)-N-(3-methyloxetan-3-yl)imidazo[1,5-a]pyridine-6-sulfonamide-1-d**

Under an atmosphere of nitrogen at room temperature, N-fluorodibenzene sulfonamide (5.7 mg, 0.018 mmol) was added to the solution of 3-(5-(difluoromethyl)-1,3,4-thiadiazol-2-yl)-8-(4-isobutyrylpiperazin-1-yl)-N-(3-methyloxacyclobutan-3-yl)imidazo[1,5-a]pyridin-6-sulfonamide-1-deuterium (5 mg, 0.009 mmol) in N,N-dimethylformamide (0.5 mL). The reaction mixture was stirred at room temperature for 16 hours. After completion of the reaction, the reaction mixture was filtered; and the filtrate was purified by pre-HPLC to obtain the title compound (yellow solid, 2 mg, yield: 38.7%). $^1H$ NMR (400 MHz, DMSO-$d_6$) δ 9.65 (d, *J* = 5.3 Hz, 1H), 8.97 (s, 1H), 7.81 - 7.53 (m, 1H), 4.71 (d, *J* = 6.3 Hz, 2H), 4.23 (d, *J* = 6.4 Hz, 2H), 3.74 - 3.66 (m, 4H), 3.43 - 3.39 (m, 4H), 2.97 - 2.91 (m, 1H), 1.55 (s, 3H), 1.04 (d, *J* = 6.7 Hz, 6H). LC/MS (ESI) (m/z): 575 $[M+H]^+$.

**Example 156: (R)-N-(1-cyanocyclopropyl)-3-(5-(difluoromethyl)-1,3,4-thiadiazol-2-yl)-8-(3-methyl-4-(1-methylcyclopropane-1-carbonyl)piperazin-1-yl)imidazo[1,5-a]pyridine-6-sulfonamide-1-d**

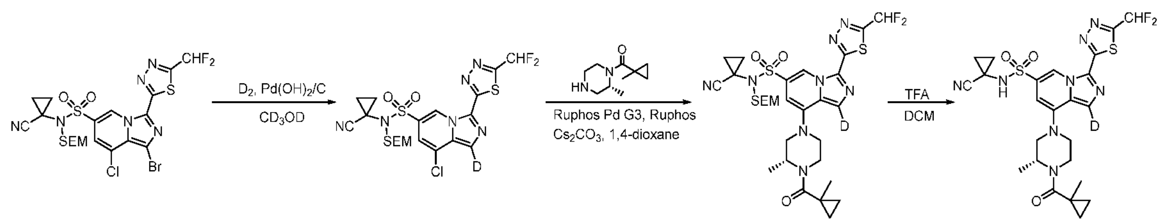


By referring to the preparation method in Example **153,** this example was implemented by synthesizing a crude product according to a similar route by starting from appropriate starting materials, treating the crude product via reversed-phase HPLC, and then freeze-drying the crude product to obtain the title compound. $^1H$ NMR (400 MHz, DMSO-$d_6$) δ 9.68 (s, 1H), 9.48 (s, 1H), 7.69 (t, *J* = 53.2 Hz, 1H), 6.68 (s, 1H), 4.76 - 4.63 (m, 1H), 4.27 (d, *J* = 13.4 Hz, 1H), 3.78 - 3.43 (m, 3H), 3.07 - 2.87 (m, 2H), 1.50 - 1.26 (m, 10H), 0.90 - 0.79 (m, 2H), 0.64 - 0.53 (m, 2H). LC/MS (ESI) (m/z): 578 $[M+H]^+$.

**By referring to the preparation method in Example 156, the following examples were implemented by synthesizing a crude product according to a similar route by starting from appropriate starting materials, treating the crude product via reversed-phase HPLC, and then freeze-drying the crude product to obtain the title compound.**

| Example | Structure and name | Analysis data |
|---|---|---|
| **Example 157** | 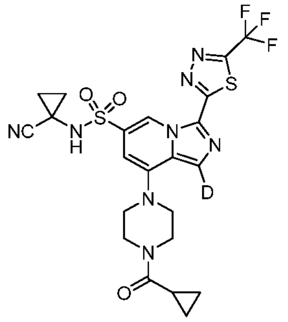 **N-(1-cyanocyclopropyl)-8-(4-(cyclopropanecarbonyl)piperazin-1-yl)-3-(5-(trifluoromethyl)-1,3,4-thiadiazol-2-yl)imidazo [1,5-a]pyridine-6-sulfonamide-1-d** | $^1$H NMR (400 MHz, DMSO-$d_6$) δ 9.64 (s, 1H), 9.49 (s, 1H), 6.70 (s, 1H), 4.07 - 3.88 (m, 2H), 3.83 - 3.66 (m, 2H), 3.48 - 3.36 (m, 2H), 3.36 - 3.34 (m, 2H), 2.06 - 1.95 (m, 1H), 1.52 - 1.35 (m, 4H), 0.82 - 0.70 (m, 4H). LC/MS (ESI) (m/z): 568 $[M+H]^+$. |
| **Example 158** | 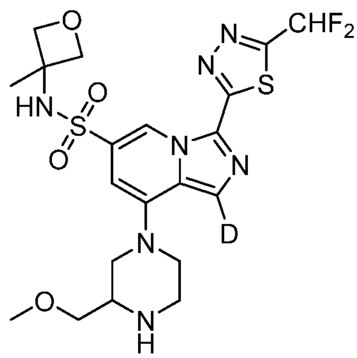 **3-(5-(difluoromethyl)-1,3,4-thiadiazol-2-yl)-8-(3-(methoxymethyl)piperazin-1-yl)-N-(3-methyloxetan-3-yl)imidazo[1,5-a]pyridine-6-sulfonamide-1-d** | $^1$H NMR (400 MHz, $CD_3OD$) δ 9.88 (s, 1H), 7.33 (t, *J* = 53.5 Hz, 1H), 6.82 (s, 1H), 4.78 - 4.76 (m, 2H), 4.33 (d, *J* = 6.7 Hz, 2H), 3.89 - 3.80 (m, 3H), 3.72 - 3.68 (m, 2H), 3.65 - 3.60 (m, 2H), 3.47 (s, 3H), 3.14 - 3.11 (m, 2H), 1.65 (s, 3H). LC/MS (ESI) (m/z): 531 $[M+H]^+$. |

**Example 159: 3-(5-(difluoromethyl)-1,3,4-thiadiazol-2-yl)-8-(4-isobutyrylpiperazin-1-yl)-N-(3-methyloxetan-3-yl) imidazo[1,5-a]pyridine-6-sulfonamide-1-d**

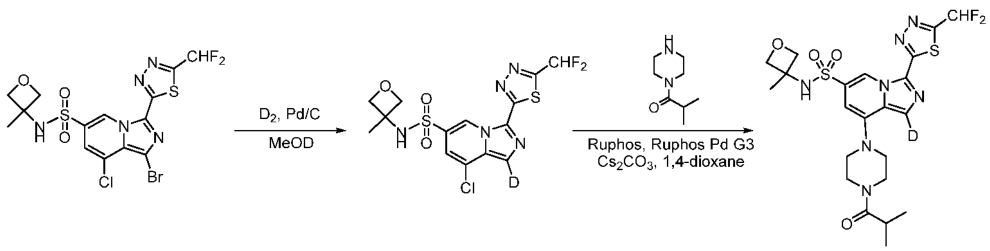


**Step 1: Preparation of 8-chloro-3-(5-(difluoromethyl)-1,3,4-thiadiazol-2-yl)-N-(3-methyloxetan-3-yl)imidazo[1,5-a]pyridine-6-sulfonamide-1-d**

Under an atmosphere of nitrogen, palladium on carbon (50 mg) with the mass fraction of 10% was added to the solution of 1-bromo-8-chloro-3-(5-(difluoromethyl)-1,3,4-thiadiazol-2-yl)-N-(3-methyloxacyclobutan-3-yl)imidazo[1,5-a] pyridin-6-sulfonamide (180 mg, 0.35 mmol) in deutero methanol (5 mL). The flask was replaced with nitrogen gas three times and then the mixture was stirred at room temperature for 6 hours under an atmosphere of deuterium at one atmospheric pressure. After completion of the reaction, the reaction mixture was filtered; and the filtrate was concentrated under reduced pressure to obtain the title compound (yellow solid, 120 mg, yield: 78.55%). LC/MS (ESI) m/z: 437/439 $[M+H]^+$.

**Step 2: Preparation of 3-(5-(difluoromethyl)-1,3,4-thiadiazol-2-yl)-8-(4-isobutyrylpiperazin-1-yl)-N-(3-methyloxetan-3-yl)imidazo[1,5-a]pyridine-6-sulfonamide-1-d**

Under an atmosphere of nitrogen, cesium carbonate (112 mg, 0.344 mmol), Ruphos (11 mg, 0.024 mmol) and Ruphos Pd G3 (10 mg, 0.012 mmol) were sequentially added to the solution of 8-chloro-3-(5-(difluoromethyl)-1,3,4-thiadiazol-2-yl)-N-(3-methyloxacyclobutan-3-yl)imidazo[1,5-a]pyridin-6-sulfonamide-1-deuterium (50 mg, 0.114 mmol) and 2-methyl-1-(piperazin-1-yl)propan-1-one (36 mg, 0.23 mmol) in 1,4-dioxane (1 mL). The flask was replaced with nitrogen gas three times and then the mixture was stirred at 80 °C for 3 hours under an atmosphere of nitrogen. After completion of the reaction, the reaction mixture was cooled to room temperature, diluted with ethyl acetate, sequentially washed with water and saturated brine, dried over anhydrous sodium sulfate, filtered and concentrated under reduced pressure. The residue was purified by pre-HPLC to obtain the title compound(yellow solid, 18.2 mg, yield: 28.7%). $^1H$ NMR (400 MHz, DMSO-$d_6$) δ 9.61 - 9.57 (m, 1H), 8.72 (s, 1H), 7.83 - 7.53 (m, 1H), 6.70 (s, 1H), 4.63 (d, $J$ = 6.2 Hz, 2H), 4.18 (d, $J$ = 6.5 Hz, 2H), 3.84 - 3.70 (m, 4H), 3.39 - 3.34 (m, 4H), 2.99 - 2.91 (m, 1H), 1.50 (s, 3H), 1.05 (d, $J$ = 6.7 Hz, 6H). LC/MS (ESI) m/z: 557 $[M+H]^+$.

**Example 160: 3-(5-(difluoromethyl)-1,3,4-thiadiazol-2-yl)-8-((3S,5S)-3,5-dimethylpiperazin-1-yl)-N-(3-methyloxetan-3-yl)imidazo[1,5-a]pyridine-6-sulfonamide-1-d**

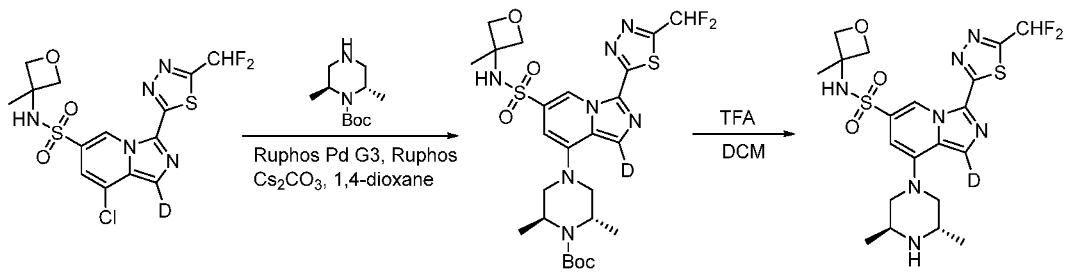


The title compound was a light-yellow solid obtained by referring to the synthesis method in Example 159. $^1H$ NMR (400 MHz, DMSO-$d_6$) δ 9.57 (s, 1H), 8.29 (d, $J$ = 1.8 Hz, 1H), 7.83 - 7.49 (m, 1H), 6.71 (s, 1H), 4.69 - 4.58 (m, 2H), 4.22 - 4.16 (m, 2H), 3.53 - 3.43 (m, 2H), 3.36 - 3.29 (m, 2H), 3.15 - 3.06 (m, 2H), 1.50 (s, 3H), 1.37 - 1.16 (m, 6H). LC/MS (ESI) (m/z): 515 $[M+H]^+$.

**Example 161: 3-(5-(difluoromethyl)-1,3,4-thiadiazol-2-yl)-8-(4-isobutyrylpiperazin-1-yl)-N-(1-(methyl-$d_3$)cyclopropyl)imidazo[1,5-a]pyridine-6-sulfonamide**

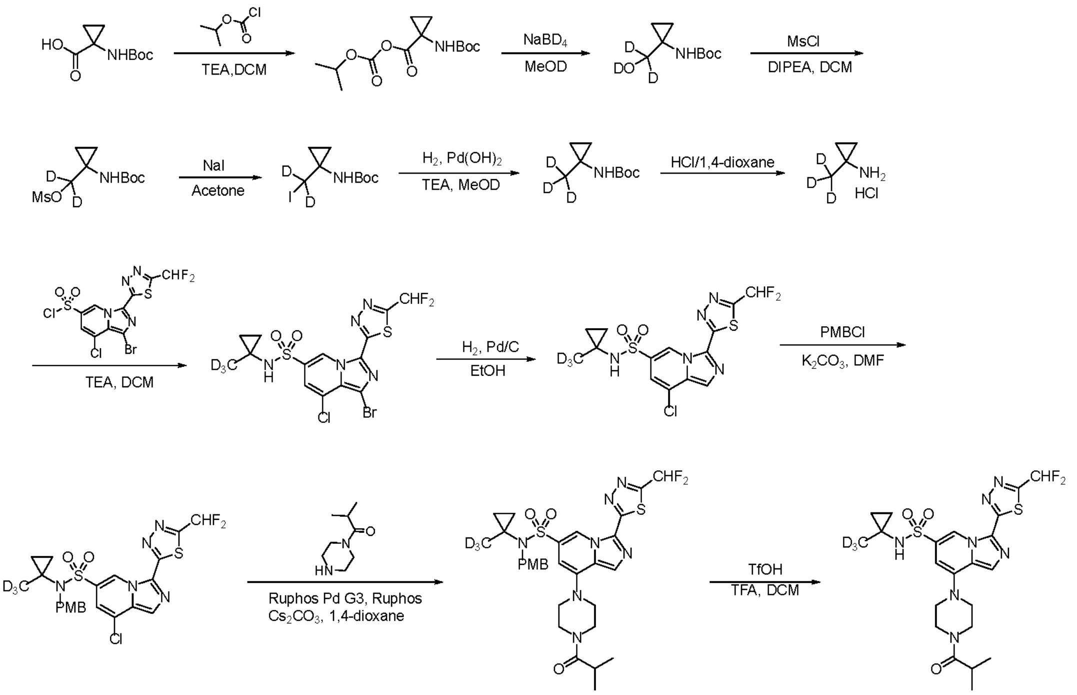


**Step 1: 1-((tert-butoxycarbonyl)amino)cyclopropane-1-carboxylic (isopropyl carbonic) anhydride**

of nitrogen at 0°C, triethylamine (14 mL, 99.4 mmol) was added to the solution of 1-((tert-butoxycarbonyl)amino) cyclopropan-1-formic acid (10 g, 49.7 mmol) in dichloromethane (100 mL); then, isopropyl chlorocarbonate (7.3 g, 59.6 mmol)was dropwise added slowly; and after dropwise adding, the reaction mixture was stirred at 0°C Under an atmospherefor 30 minutes. After completion of the reaction, the reaction mixture was quenched with ice water, and extracted with dichloromethane. The organic phase was washed with saturated brine, dried over anhydrous sodium sulfate, and concentrated under reduced pressure. The residue was separated by column chromatography (eluent: petroleum ether/ethyl acetate, gradient: 0-10% ethyl acetate) to obtain the title compound (white solid, 7 g, yield: 47.9%).

**Step 2: Tert-butyl (1-((hydroxy-d)methyl-$d_2$)cyclopropyl)carbamate**

At 0°C, the solution of 1-((tert-butoxycarbonyl)amino)cyclopropan-1-(isopropyl carbonate)formic anhydride (7 g, 24.3 mmol) in deutero methanol (50 mL) was slowly added to deuterated sodium borohydride (1.2 g, 29.3 mmol), the reaction mixture was stirred at 0°C for 30 minutes. After completion of the reaction, the reaction mixture was quenched with aqueous ammonium chloride solution, and extracted with dichloromethane. The organic phase was washed with saturated brine, dried over anhydrous sodium sulfate, and concentrated under reduced pressure. The residue was separated by column chromatography (eluent: petroleum ether/ethyl acetate, gradient: 0-50% ethyl acetate) to obtain the title compound (white solid, 4 g, yield: 86.9%). $^1$H NMR (400 MHz, $CDCl_3$) δ 5.04 (s, 1H), 1.37 (s, 9H), 0.78 - 0.73 (m, 4H).

**Step 3: (1-((tert-butoxycarbonyl)amino)cyclopropyl)methyl-$d_2$ methanesulfonate**

At 0°C, triethylamine (6.6 mL, 47.3 mmol) was added to the solution of (1-((hydroxy-d)methyl-$d_2$)cyclopropyl) aminotert-butyl formate (3 g, 15.8 mmol) in dichloromethane (30 mL); then, methylsulfonyl chloride (2.7 g, 23.7 mmol)was dropwise added slowly; and the mixture was contnued to be stirred at 0°C for 30 minutes. After completion of the reaction, the reaction mixture was quenched with ice water, and extracted with dichloromethane. The organic phase was washed with saturated brine, dried over anhydrous sodium sulfate, and concentrated under reduced pressure. The residue was separated by column chromatography (eluent: petroleum ether/ethyl acetate, gradient: 0-40% ethyl acetate) to obtain the title compound (white solid, 4 g, yield: 95.2%). $^1$H NMR (400 MHz, $CDCl_3$) δ 3.06 - 3.03 (m, 3H), 1.47 (s, 9H), 0.98 - 0.93 (m, 4H).

**Step 4: Tert-butyl (1-(iodomethyl-$d_2$)cyclopropyl)carbamate**

At 0°C, sodium iodide (1.6 g, 10.8 mmol) was added to the solution of (1-((tert-butoxycarbonyl)amino)cyclo-propyl)methyl-$d_2$methanesulfonate (1g, 3.6 mmol) in acetone (10 mL); and the mixture was stirred at 50°C for 4 hours.

After completion of the reaction, the reaction mixture was diluted with ethyl acetate, washed with water and saturated brine in sequence, dried over anhydrous sodium sulfate, and concentrated under reduced pressure. The residue was separated by column chromatography (eluent: petroleum ether/ethyl acetate, gradient: 0-10% ethyl acetate) to obtain the title compound (white solid, 600 mg, yield: 54.5%), LC/MS (ESI) (m/z): 244 $(M-56+H)^+$.

**Step 5: Tert-butyl (1-(methyl-$d_3$)cyclopropyl)carbamate**

Under an atmosphere of nitrogen, palladium hydroxide (100 mg) with the mass fraction of 10% and triethylamine (0.5 mL, 3.6 mmol) were added to the solution of (1-iodomethyl-d2)cyclopropyl)aminotert-butyl formate (600 mg, 2 mmol) in deutero methanol (6 mL). The reaction mixture was replaced three times with deuterium and then stirred at 50°C for 6 hours under an atmosphere of deuterium at one atmospheric pressure. After completion of the reaction, the reaction mixture was filtered; and the filtrate was concentrated under reduced pressure to obtain the title compound (white solid, 360 mg, yield: 99%). LC/MS (ESI) (m/z): 119 $[M+H]^+$.

**Step 6: 1-(methyl-$d_3$)-cyclopropan-1-amine hydrochloride**

1-(methyl-$d_3$)cyclopropylaminotert-butyl formate (200 mg, 1.1 mmol) was dissolved in 1,4-dioxane (1 mL); hydrochloric acid-dioxane (1 mL, 4M) was dropwise added slowly at room temperature; and the mixture was stirred at 40°C for 2 hours. After completion of the reaction, the reaction mixture was concentrated under reduced pressure to obtain a crude title compound (light yellow solid, 200 mg, yield: 100%), which was directly used in the next step of reaction.

**Step 7: 1-bromo-8-chloro-3-(5-(difluoromethyl)-1,3,4-thiadiazol-2-yl)-N-(1-(methyl-ds)cyclopropyl)imidazo[1,5-a]pyridine-6-sulfonamide**

At 0°C, triethylamine (0.5 mL) and 1-bromo-8-chloro-3-(5-difluoromethyl-1,3,4-thiadiazol-2-yl]imidazo[1,5-a] pyridin-6-sulfonyl chloride (200 mg, 0.43 mmol) were sequentially added to the solution of 1-(methyl-$d_3$)-cyclopropyl-1-amine hydrochloride (200 mg, 1.1 mmol) in dichloromethane (3 mL); and the reaction mixture was stirred at room temperature for 30 minutes. After completion of the reaction, the reaction mixture was added with ice water for quenching, and extracted with ethyl acetate. The organic phase was washed with saturated brine, dried over anhydrous sodium sulfate, and concentrated under reduced pressure; and the residue was purified by column chromatography (eluent: petroleum ether/ethyl acetate, gradient: 0-30% ethyl acetate) to obtain the title compound (yellow solid, 35 mg, yield: 16.2%). $^1$H NMR (400 MHz, DMSO-$d_6$) $\delta$ 9.83 (d, $J$ = 1.1 Hz, 1H), 8.51 (s, 1H), 7.71 (t, $J$ = 53.0 Hz, 2H), 7.54 (d, $J$ = 1.0 Hz, 1H), 1.56 - 1.35 (m, 4H). LC/MS (ESI) (m/z): 501 $[M+H]^+$.

**Step 8: 8-chloro-3-(5-(difluoromethyl)-1,3,4-thiadiazol-2-yl)-N-(1-(methyl-$d_3$)cyclopropyl)imidazo[1,5-a]pyridine-6-sulfonamide**

At 0°C, Pd/C (10 mg, mass fraction: 10%) was added to the solution of 1-bromo-8-chloro-3-(5-difluoromethyl-1,3,4-thiadiazol-2-yl)-N-(1-(methyl-$d_3$)cyclopropyl)imidazo[1,5-a]pyridin-6-sulfonamide (35 mg, 0.07 mmol) in ethanol (5 mL); The flask was replaced with nitrogen gas three times and then the mixture was stirred at room temperature for 2 hours under an atmosphere provided by a hydrogen-filled balloon. After completion of the reaction,the mixture was filtered. The filtrate was concentrated under reduced pressure to obtain the title compound (yellow solid, 25 mg, yield: 86.2%). LC/MS (ESI) (m/z): 423 $[M+H]^+$.

**Step 9: 8-chloro-3-(5-(difluoromethyl)-1,3,4-thiadiazol-2-yl)-N-(4-methoxybenzyl)-N-(1-(methyl-$d_3$)cyclopropyl) imidazo[1,5-a]pyridine-6-sulfonamide**

At room temperature, potassium carbonate (40 mg, 0.3 mmol) and 1-(chloromethyl)-4-metoxybenzene (28 mg, 0.18 mmol) were added to the solution of 8-chloro-3-(5-difluoromethyl-1,3,4-thiadiazol-2-yl)-N-(1-(deuteromethyl)cyclopropyl)imidazo[1,5-a]pyridin-6-sulfonamide (50 mg, 0.12 mmol) in N,N- (1 mL); and the reaction mixture was stirred at 40°C for 6 hours. After completion of the reaction, the reaction mixture was diluted with ethyl acetate, washed with water and saturated brine in sequence, dried over anhydrous sodium sulfate, and concentrated under reduced pressure. The residue was separated by column chromatography (eluent: petroleum ether/ethyl acetate, gradient: 0-15% ethyl acetate) to obtain the title compound (white solid, 30 mg, yield: 46.8%). LC/MS (ESI) (m/z): 543 $[M+H]^+$.

**Step 10: 3-(5-(difluoromethyl)-1,3,4-thiadiazol-2-yl)-8-(4-isobutyrylpiperazin-1-yl)-N-(4-methoxybenzyl)-N-(1-(methyl-$d_3$)cyclopropyl)imidazo[1,5-a]pyridine-6-sulfonamide**

Under an atmosphere of nitrogen at room temperature, cesium carbonate (54 mg, 0.16 mmol), Ruphos (5 mg, 0.011 mmol) and Ruphos Pd G3 (5 mg, 0.005 mmol) were sequentially added to the solution of 8-chloro-3-(5-difluoromethyl-1,3,4-thiadiazol-2-yl)-N-(1-(methyl-$d_3$)cyclopropyl)-N-(4-methoxybenzyl)imidazo[1,5-a]pyridin-6-sulfonamide (30 mg, 0.055 mmol) and 1-(2-methylacetonyl)-piperazine (13 mg, 0.083 mmol) in 1,4-dioxane (1 mL). The flask was replaced with nitrogen gas three times and then the mixture was stirred at 60°C for 2 hours under an atmosphere of nitrogen. After completion of the reaction, the reaction mixture was cooled to room temperature, quenched by adding water, and extracted with ethyl acetate. The organic phase was washed with saturated brine, dried over anhydrous sodium sulfate, and concentrated under reduced pressure. The residue was separated by column chromatography (eluent: petroleum ether/ethyl acetate, gradient: 0-50% ethyl acetate) to obtain the title compound (yellow solid, 30 mg, yield: 82.1%). LC/MS (ESI) (m/z): 663 $[M+H]^+$.

**Step 11: 3-(5-(difluoromethyl)-1,3,4-thiadiazol-2-yl)-8-(4-isobutyrylpiperazin-1-yl)-N-(1-(methyl-$d_3$)cyclopropyl) imidazo[1,5-a]pyridine-6-sulfonamide**

At 0°C, trifluoroacetic acid (0.1 mL) and trifluoromethanesulfonic acid (0.1 mL) was added to the solution of 3-(5-(difluoromethyl)-1,3,4-thiadiazol-2-yl)-8-(4-isobutyrylpiperazin-1yl)-N-(4-methoxybenzyl)-N-(1-(methyl-$d_3$)cyclopropyl)imidazo[1,5-a]pyridin-6-sulfonamide (30 mg, 0.045 mmol) in dichloromethane (2 mL); and the reaction mixture was stirred at 0°C for 2 hours. The reaction mixture was concentrated under reduced pressure; and the residue was purified by pre-HPLC to obtain the title compound (white solid, 7 mg, yield: 30.4%). $^1H$ NMR (400 MHz, $CD_3OD$) δ 9.82 (s, 1H), 7.92 (s, 1H), 7.50 - 7.15 (m, 1H), 6.72 (s, 1H), 3.91 - 3.79 (m, 4H), 3.44 - 3.33 (m, 4H), 3.08 - 2.98 (m, 1H), 1.15 (d, *J* = 6.7 Hz, 6H), 0.85 - 0.76 (m, 2H), 0.53 - 0.46 (m, 2H). LC/MS (ESI) (m/z): 543 $[M+H]^+$.

**Example 162: 3-(5-(difluoromethyl)-1,3,4-thiadiazol-2-yl)-8-(4-isobutyrylpiperazin-1-yl)-N-(1-**methylcyclopropyl) imidazo[1,5-a]pyrazine-6-sulfonamide

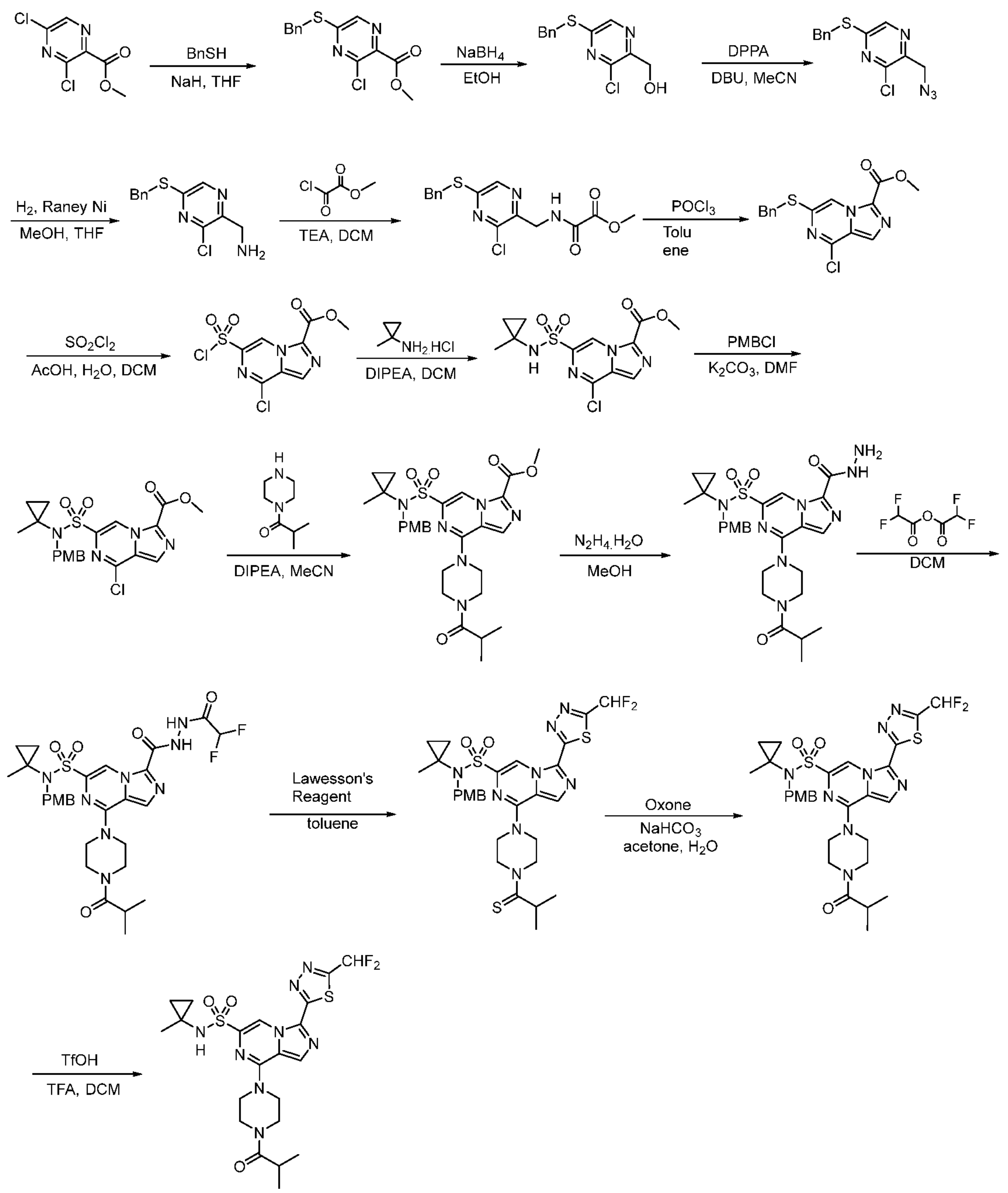


**Step** 1: **Methyl 5-(benzylthio)-3-chloropyrazine-2-carboxylate**

At 0°C, sodium hydride (1.2 g, 29.0 mmol, 60% dispersed in paraffins) was added portionwise to the solution of 3,5-dichloropyrazin-2-methyl carboxylate (5 g, 24.2 mmol) in tetrahydrofuran (50 mL). The reaction mixture was stirred at 0°C for 30 minutes under an atmosphere of nitrogen. Then, benzyl mercaptane was dropwise added slowly(3 g, 24.2 mmol). After dropwise adding, the reaction mixture was stirred at room temperature for 2 hours. After completion of the reaction, saturated ammonium chloride solution was dropwise added slowly to quench the reaction mixture, which was then extracted with ethyl acetate. The organic phase was washed with saturated sodium chloride solution, dried over anhydrous sodium sulfate, filtered and concentrated under reduced pressure. The residue was separated by column chromatography (eluent: petroleum ether/ethyl acetate, gradient: 0-10% ethyl acetate) to obtain the title compound (colorless liquid, 5.5 g, yield: 77.3%). $^1H$ NMR (400 MHz, $CDCl_3$) δ 8.32 (s, 1H), 7.45 - 7.35 (m, 2H), 7.32 - 7.23 (m, 3H), 4.41 (s, 2H), 3.96 (s, 3H). LC/MS (ESI) (m/z): 295 $[M+H]^+$.

**Step 2: (5-(benzylthio)-3-chloropyrazin-2-yl) methanol**

At 0°C, sodium borohydride (770 mg, 22.9 mmol) was added portionwise to the solution of 5-(benzylthio)-3-chloropyrazin-2-methyl carboxylate (4.5 g, 15.3 mmol) in ethanol (40 mL). The reaction mixture was stirred at room temperature for 2 hours. After completion of the reaction, ice water was dropwise added slowly to quench the reaction mixture, which was then concentrated under reduced pressure to remove ethanol. The reaction mixture was extracted with ethyl acetate; and the organic phase was washed with saturated brine, dried over anhydrous sodium sulfate, filtered and concentrated under reduced pressure. The residue was separated by column chromatography (eluent: petroleum ether/ethyl acetate, gradient: 0-20% ethyl acetate) to obtain the title compound (colorless oil, 3.5 g, yield: 86.0%). LC/MS (ESI) (m/z): 267 $[M+H]^+$.

**Step 3: 2-(azidomethyl)-5-(benzylthio)-3-chloropyrazine**

At 0°C, 1,8-diazabicyclo[5.4.0]undec-7-ene (3.0 g, 19.7 mmol) and azidodiphenyl phosphate (3.6 g, 13.1 mmol) were slowly added to the solution of (5-(benzylthio)-3-chloropyrazin-2-yl) methanol (3.5 g, 13.1 mmol) in acetonitrile (35 mL). The reaction mixture was stirred at room temperature for 4 hours. After completion of the reaction, the reaction mixture was extracted with ethyl acetate, and the organic phase was washed with saturated brine, dried over anhydrous sodium sulfate, filtered and concentrated under reduced pressure. The residue was separated by column chromatography (eluent: petroleum ether/ethyl acetate, gradient: 0-3% ethyl acetate) to obtain the title compound (yellow oil, 1.4 g, yield: 36.6%). LC/MS (ESI) (m/z): 292 $[M+H]^+$.

**Step 4: (5-(benzylthio)-3-chloropyrazin-2-yl)methanamine**

Under an atmosphere of nitrogen at room temperature, raney nickel (200 mg) was added to the solution of 2-(azidomethyl)-5-(benzylthio)-3-chloropyrazine (1.3 g, 4.47 mmol) in methanol (8 mL) and tetrahydrofuran (4 mL). The flask was replaced with nitrogen gas three times and then the mixture was stirred at room temperature for 18 hours under an atmosphere of hydrogen at one atmospheric pressure. After completion of the reaction, the reaction mixture was filtered, and the filtrate was concentrated under reduced pressure. The residue was separated by column chromatography (eluent: dichloromethane/methanol, gradient: 0-10% methanol) to obtain the title compound (green solid, 780 mg, yield: 65.9%). LC/MS (ESI) (m/z): 266 $[M+H]^+$.

**Step 5: Methyl 2-(((5-(benzylthio)-3-chloropyrazin-2-yl)methyl)amino)-2-oxoacetate**

At 0°C, triethylamine (445 mg, 4.40 mmol) and methyl oxalyl chloride (360 mg, 2.93 mmol) were slowly added to the solution of (5-(benzylthio)-3-chloropyrazin-2-yl) formamide (780 mg, 2.93 mmol) in dichloromethane (8 mL). The reaction mixture was stirred at room temperature for 0.5 hour. After completion of the reaction, the reaction mixture was diluted with dichloromethane; and the organic phase was washed with saturated brine, dried over anhydrous sodium sulfate, filtered and concentrated under reduced pressure. The residue was separated by column chromatography (eluent: petroleum ether/ethyl acetate, gradient: 0-30% ethyl acetate) to obtain the title compound (yellow solid, 720 mg, yield: 69.7%). LC/MS (ESI) (m/z): 352 $[M+H]^+$.

**Step 6: Methyl 6-(benzylthio)-8-chloroimidazo[1,5-a]pyrazine-3-carboxylate**

Under an atmosphere of nitrogen at 0°C, phosphorus oxychloride (5 mL) was slowly added to the solution of 2-((5-(benzylthio)-3-chloropyrazin-2-yl)methyl)amino)-2-methyl oxyacetate (720 mg, 2.05 mmol) in toluene (5 mL). The reaction mixture was stirred at 110°C for 16 hours under an atmosphere of nitrogen. After completion of the reaction, the reaction mixture was concentrated under reduced pressure. The residue was diluted with dichloromethane; the organic phase was washed with saturated aqueous sodium bicarbonate solution and saturated brine respectively, dried over anhydrous sodium sulfate, filtered and concentrated under reduced pressure. The residue was separated by column chromatography (eluent: petroleum ether/ethyl acetate, gradient: 0-25% ethyl acetate) to obtain the title compound (yellow solid, 330 mg, yield: 48.3%). LC/MS (ESI) (m/z): 334 $[M+H]^+$.

**Step 7: Methyl 8-chloro-6-(chlorosulfonyl)imidazo[1,5-a]pyrazine-3-carboxylate**

At 0°C, sulfonyl chloride (534 mg, 3.96 mmol) was dropwise added slowly to the solution of 6-(benzylthio)-8-chloroimidazo[1,5-a]pyrazin-3-methyl carboxylate (330 mg, 0.99 mmol) in dichloromethane (4 mL), water (0.4 mL) and acetic acid (0.2 mL). The reaction mixture was stirred at room temperature for 0.5 hours. After completion of the reaction, the reaction mixture was diluted with dichloromethane, sequentially washed with saturated aqueous sodium bicarbonate

solution and saturated brine, dried over anhydrous sodium sulfate, filtered and concentrated under reduced pressure to obtain a crude title compound (yellow solid, 307 mg, yield: 100%), which was directly used in the next step of reaction without purification.

**Step 8: Methyl 8-chloro-6-(N-(1-methylcyclopropyl)sulfamoyl)imidazo[1,5-a]pyrazine-3-carboxylate**

At 0°C, N,N-diisopropylethylamine (0.43 mL, 2.47 mmol) and 8-chloro-6-(chlorosulfonyl)imidazo[1,5-a]pyrazin-3-methyl carboxylate (307 mg, 0.989 mmol) were slowly added to the solution of 1-methylcyclopropan-1-amine hydrochloride (160 mg, 1.48 mmol) in dichloromethane (3 mL). The reaction mixture was stirred at room temperature for 0.5 hour. After completion of the reaction, the reaction mixture was diluted with dichloromethane, washed with water and saturated brine, dried over anhydrous sodium sulfate, filtered and concentrated under reduced pressure. The residue was separated by column chromatography (eluent: petroleum ether/ethyl acetate, gradient: 0-28% ethyl acetate) to obtain the title compound (yellow solid, 160 mg, yield: 46.9%). LC/MS (ESI) (m/z): 345 $[M+H]^+$.

Step 9: Methyl 8-chloro-6-(N-(4-methoxybenzyl)-N-(1-methylcyclopropyl)sulfamoyl)imidazo[1,5-a]pyrazine-3-carboxylate

At room temperature, potassium carbonate (192 mg, 1.39 mmol) and 1-(chloromethyl)-4-metoxybenzene (109 mg, 0.696 mmol) were added to the solution of 8-chloro-6-(N-(1-methylcyclopropyl)sulfonamideformyl)imidazo[1,5-a]pyrazin-3-methyl carboxylate (160 mg, 0.464 mmol) in N,N-dimethylformamide (2 mL). The reaction mixture was stirred at 45°C for 2 hours under an atmosphere of nitrogen. After completion of the reaction, the reaction mixture was cooled to room temperature, added with water, and extracted with ethyl acetate; the organic phase was washed with saturated brine, dried over anhydrous sodium sulfate, and concentrated under reduced pressure. The residue was purified by column chromatography (eluent: petroleum ether/ethyl acetate, gradient: 0-25% ethyl acetate) to obtain the title compound (yellow solid, 155 mg, yield: 71.8%). LC/MS (ESI) m/z: 465 $[M+H]^+$.

**Step 10: Methyl 8-(4-isobutyrylpiperazin-1-yl)-6-(N-(4-methoxybenzyl)-N-(1-methylcyclopropyl)sulfamoyl)imidazo[1,5-a]pyrazine-3-carboxylate**

At room temperature, N,N-diisopropylethylamine (0.16 mL, 0.94 mmol) and 2-methyl-1-(piperazin-1-yl)propan-1-one (73 mg, 0.468 mmol) were added to the solution of 8-chloro-6-(N-(4-methoxybenzyl)-N-(1-methylcyclopropyl)sulfanilamido)imidazo[1,5-a]pyrazin-3-methyl carboxylate (145 mg, 0.312 mmol) in acetonitrile (2 mL). The reaction mixture was stirred at 45°C for 4 hours. After completion of the reaction, the reaction mixture was cooled to room temperature, added with water, and extracted with ethyl acetate. The organic phase was washed with saturated brine, dried over anhydrous sodium sulfate, and concentrated under reduced pressure. The residue was separated by column chromatography (eluent: dichloromethane/methanol, gradient: 0-10% methanol) to obtain the title compound (yellow solid, 170 mg, yield: 93.2%). LC/MS (ESI) m/z: 585 $[M+H]^+$.

**Step 11: 3-(hydrazinecarbonyl)-8-(4-isobutyrylpiperazin-1-yl)-N-(4-methoxybenzyl)-N-(1-methylcyclopropyl) imidazo [1,5-a]pyrazine-6-sulfonamide**

At room temperature, hydrazine hydrate (0.18 mL, 2.91 mmol) with the mass fraction of 80% was added to the solution of 8-(4-isobutylpiperazin-1-y1)-6-(N-(4-methoxybenzyl)-N-(1-methylcyclopropyl)sulfanilamido)imidazo[1,5-a]pyrazin-3-methyl carboxylate (170 mg, 0.291 mmol) in methanol (2 mL). The reaction mixture was stirred at 75 °C for 1 hour. After completion of the reaction, the reaction mixture was cooled to room temperature, and concentrated under reduced pressure to obtain a crude title compound (yellow solid, 170 mg, yield: 78.5%), which was directly used in the next step of reaction without purification. LC/MS (ESI) m/z: 585 $[M+H]^+$.

**Step 12: 3-(2-(2,2-difluoroacetyl)hydrazine-1-carbonyl)-8-(4-isobutyrylpiperazin-1-yl)-N-(4-methoxybenzyl)-N-(1-methylcyclopropyl)imidazo[1,5-a]pyrazine-6-sulfonamide**

At 0°C, difluoroacetic anhydride (0.036 mL, 0.291 mmol) was slowly added to the solution of 3-(hydrazinocarbonyl)-8-(4-isobutylpiperazin-1-yl1)-N-(4-methoxybenzyl)-N-(1-methylcyclopropyl)imidazo[1,5-a]pyrazin-6-sulfonamide (170 mg, 0.291 mmol) in dichloromethane (2 mL). The reaction mixture was stirred at 0 °C for 0.5 hour. After completion of the reaction, the reaction mixture was concentrated under reduced pressure; and the residue was purified by column chromatography (eluent: dichloromethane/methanol, gradient: 0-10% methanol) to obtain the title compound (yellow solid, 160 mg, yield: 83.0%). LC/MS (ESI) m/z: 663 $[M+H]^+$.

**Step 13: 3-(5-(difluoromethyl)-1,3,4-thiadiazol-2-yl)-N-(4-methoxybenzyl)-N-(1-methylcyclopropyl)-8-(4-(2-methylpropanethioyl)piperazin-1-yl)imidazo [1,5-a]pyrazine-6-sulfonamide**

Under an atmosphere of nitrogen at room temperature, the Lawesson's reagent (34 mg, 0.083 mmol) was added to the solution of 3-(2-(2,2-difluoroacetyl)hydrazin-1-carbonyl)-8-(4-isobutylpiperazin-1-yl)-N-(4-methoxybenzyl)-N-(1-methylcyclopropyl)imidazo[1,5-a]pyrazin-6-sulfonamide (50 mg, 0.075 mmol) in toluene (2 mL). The reaction mixture was stirred at 110°C for 18 hours under an atmosphere of nitrogen. After completion of the reaction, the reaction mixture was cooled to room temperature. The reaction mixture was diluted with ethyl acetate, washed with saturated aqueous sodium bicarbonate solution, dried over anhydrous sodium sulfate, and concentrated under reduced pressure. The residue was purified by column chromatography (eluent: petroleum ether/ethyl acetate, gradient: 0-35% ethyl acetate) to obtain the title compound (yellow solid, 20 mg, yield: 39.4%). LC/MS (ESI) m/z: 677 $[M+H]^+$.

**Step 14: 3-(5-(difluoromethyl)-1,3,4-thiadiazol-2-yl)-8-(4-isobutyrylpiperazin-1-yl)-N-(4-methoxybenzyl)-N-(1-methylcyclopropyl)imidazo[1,5-a]pyrazine-6-sulfonamide**

At 0°C, sodium bicarbonate (7 mg, 0.089 mmol) and potassium hydrogen persulfate (51 mg, 0.059 mmol) were slowly added to the solution of 3-(5-(difluoromethyl)-1,3,4-thiadiazol-2-yl)-N-(4-methoxybenzyl)-N-(1-methylcyclopropyl)-8-(4-(2-methylpropylthio)piperazin-1-yl)imidazo[1,5-a]pyrazin-6-sulfonamide (20 mg, 0.030 mmol) in acetone (1.5 mL) and water (0.5 mL). The reaction mixture was stirred at room temperature for 0.5 hours. After completion of the reaction, the reaction mixture was diluted with dichloromethane; and the organic phase was washed with saturated aqueous sodium bicarbonate solution, dried over anhydrous sodium sulfate, and concentrated under reduced pressure. The residue was purified by column chromatography (eluent: petroleum ether/ethyl acetate, gradient: 0-35% ethyl acetate) to obtain the title compound (yellow solid, 12 mg, yield: 61.5%). LC/MS (ESI) m/z: 661 $[M+H]^+$.

**Step 15: 3-(5-(difluoromethyl)-1,3,4-thiadiazol-2-yl)-8-(4-isobutyrylpiperazin-1-yl)-N-(1-methylcyclopropyl)imidazo[1,5-a]pyrazine-6-sulfonamide**

At 0°C, trifluoroacetic acid (0.5 mL) and trifluoromethanesulfonic acid (0.1 mL) were dropwise added slowly to the solution of 3-(5-(difluoromethyl)-1,3,4-thiadiazol-2-yl)-8-(4-isobutylpiperazin-1-yl)-N-(4-methoxybenzyl)-N-(1-methylcyclopropyl)imidazo[1,5-a]pyrazin-6-sulfonamide (12 mg, 0.018 mmol) in dichloromethane (1 mL). The reaction mixture was stirred at room temperature for 30 minutes. The reaction mixture was concentrated under reduced pressure; and the residue was purified by pre-HPLC to obtain the title compound (yellow solid, 3.5 mg, yield: 35.6%). $^1H$ NMR (400 MHz, DMSO-$d_6$) δ 9.27 (s, 1H), 8.33 (d, *J* = 10.5 Hz, 2H), 7.70 (t, *J* = 53.1 Hz, 1H), 4.10 - 3.98 (m, 4H), 3.78 - 3.67 (m, 4H), 2.96 - 2.87 (m, 1H), 1.20 (s, 3H), 1.04 (d, *J* = 6.6 Hz, 6H), 0.81 - 0.75 (m, 2H), 0.47 - 0.41 (m, 2H). LC/MS (ESI) (m/z): 541 $[M+H]^+$.

**Example 163: 1-cyano-3-(5-(difluoromethyl)-1,3,4-thiadiazol-2-yl)-8-(4-isobutyrylpiperazin-1-yl)-N-(1-methylcyclopropyl)imidazo [1,5-a] pyridine-6-sulfonamide**

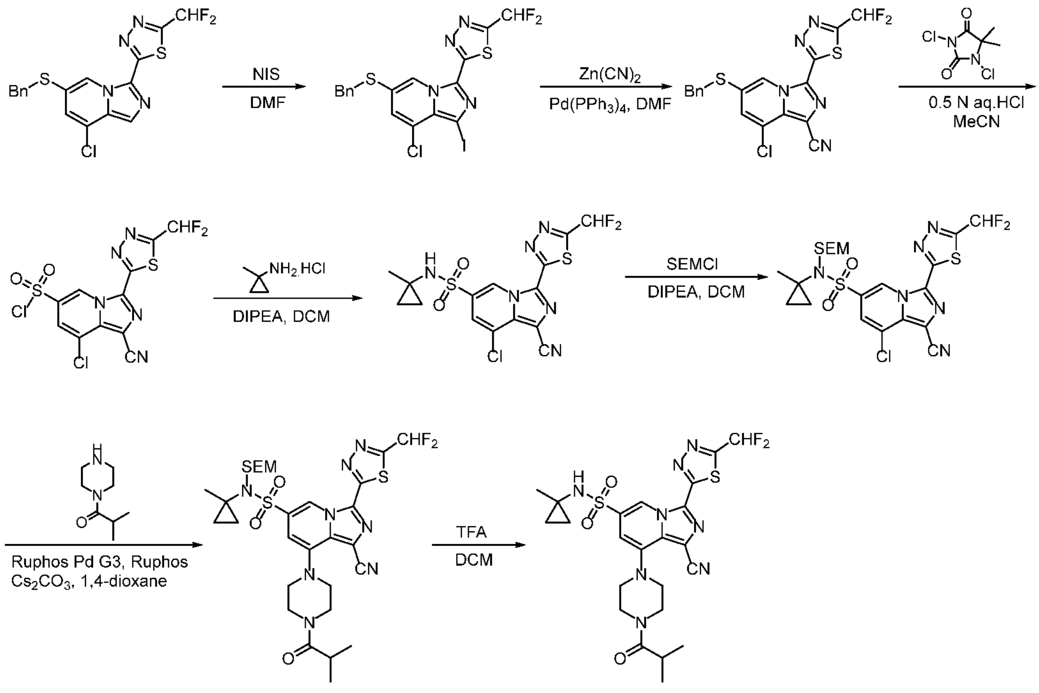


**Step 1: 2-(6-(benzylthio)-8-chloro-1-iodoimidazo[1,5-a]pyridin-3-yl)-5-(difluoromethyl)-1,3,4-thiadiazole**

At room temperature, N-iodosuccinimide (275 mg, 1.22 mmol) was added to the solution of 2-(6-(benzylthio)-8-chloroimidazo[1,5-a]pyridin-3-yl)-5-(difluoromethyl)-1,3,4-thiadiazole (500 mg, 1.22 mmol) in N,N-dimethylformamide (5 mL). The reaction mixture was stirred at 70°C for 18 hours under an atmosphere of nitrogen. After completion of the reaction, the reaction mixture was cooled to room temperature, added with water, and extracted with ethyl acetate; and the organic phase was washed with saturated brine, dried over anhydrous sodium sulfate, and concentrated under reduced pressure. The residue was separated by column chromatography (eluent: petroleum ether/ethyl acetate, gradient: 0-25% ethyl acetate) to obtain the title compound (yellow solid, 380 mg, yield: 58.1%). LC/MS (ESI) m/z: 535 $[M+H]^+$.

**Step 2: 6-(benzylthio)-8-chloro-3-(5-(difluoromethyl)-1,3,4-thiadiazol-2-yl)imidazo[1,5-a]pyridine-1-carbonitrile**

At room temperature, tetrakis(triphenylphosphine)palladium (164 mg, 0.142 mmol) was added to the solution of 2-(6-(benzylthio)-8-chloro-1-iodoimidazo[1,5-a]pyridin-3-yl)-5-(difluoromethyl)-1,3,4-thiadiazole (380 mg, 0.711 mmol) and zinc cyanide (167 mg, 1.42 mmol) in N,N-dimethylformamide (4 mL). The flask was replaced with nitrogen gas three times and then the mixture was stirred at 100°C for 5 hours under an atmosphere of nitrogen. After completion of the reaction, the reaction mixture was cooled to room temperature, added with water, and extracted with ethyl acetate. The organic phase was washed with saturated aqueous ammonium chloride solution, dried over anhydrous sodium sulfate, and concentrated under reduced pressure. The residue was purified by column chromatography (eluent: petroleum ether/ethyl acetate, gradient: 0-25 % ethyl acetate) to obtain the title compound (yellow solid, 160 mg, yield: 51.9%). LC/MS (ESI) m/z: 434 $[M+H]^+$.

**Step 3: 8-chloro-1-cyano-3-(5-(difluoromethyl)-1,3,4-thiadiazol-2-yl)imidazo[1,5-a]pyridine-6-sulfonyl chloride**

At 0°C, dichlorohydantoin (291 mg, 1.48 mmol) was added to the solution of 6-(benzylthio)-8-chloro-3-(5-(difluoromethyl)-1,3,4-thiadiazol-2-yl)imidazo[1,5-a]pyridin-1-nitrile (160 mg, 0.369 mmol) in acetonitrile (2 mL), 1N hydrochloric acid (0.5 mL) and water (0.5 mL). The reaction mixture was stirred at room temperature for 10 minutes. After completion of the reaction, the reaction mixture was diluted with dichloromethane, washed with saturated sodium bicarbonate, dried over anhydrous sodium sulfate, filtered and concentrated under reduced pressure to obtain a crude title compound (yellow solid, 151 mg, yield: 100%), which was directly used in the next step of reaction without purification.

**Step 4: 8-chloro-1-cyano-3-(5-(difluoromethyl)-1,3,4-thiadiazol-2-yl)-N-(1-methylcyclopropyl)imidazo[1,5-a]pyridine-6-sulfonamide**

At 0°C, N,N-diisopropylethylamine (0.16 mL, 0.92 mmol) and 8-chloro-1-cyano-3-(5-(difluoromethyl)-1,3,4-thiadiazol-2-yl)imidazo[1,5-a]pyridin-6-sulfonyl chloride (151 mg, 0.37 mmol) were added to the solution of 1-methylcyclopropan-1-amine hydrochloride (60 mg, 0.55 mmol) in dichloromethane (2 mL); and the reaction mixture was stirred at room temperature for 30 minutes. After completion of the reaction, the reaction mixture was diluted with dichloromethane, washed with saturated brine, dried over anhydrous sodium sulfate, filtered and concentrated under reduced pressure. The residue was separated by column chromatography (eluent: petroleum ether/ethyl acetate, gradient: 0-30% ethyl acetate) to obtain the title compound (yellow solid, 70 mg, yield: 42.7%). LC/MS (ESI) (m/z): 445 $[M+H]^+$.

**Step 5: 8-chloro-1-cyano-3-(5-(difluoromethyl)-1,3,4-thiadiazol-2-yl)-N-(1-methylcyclopropyl)-N-((2-(trimethylsilyl)ethoxy)methyl)imidazo[1,5-a]pyridine-6-sulfonamide**

At room temperature, N,N-diisopropylethylamine (61 mg, 0.472 mmol) and 2-(trimethylsilanyl)ethoxymethyl chloride (39 mg, 0.236 mmol) were added to the solution of 8-chloro-1-cyano-3-(5-(difluoromethyl)-1,3,4-thiadiazol-2-yl)-N-(1-methylcyclopropyl)imidazo[1,5-a]pyridin-6-sulfonamide (70 mg, 0.157 mmol) in N,N-dimethylformamide (2 mL). The reaction mixture was stirred at 0°C for 10 minutes. After completion of the reaction, the reaction mixture was diluted with dichloromethane, sequentially washed with saturated brine, dried over anhydrous sodium sulfate, and concentrated under reduced pressure. The residue was separated by column chromatography (eluent: petroleum ether/ethyl acetate, gradient: 0-25 % ethyl acetate) to obtain the title compound (yellow solid, 70 mg, yield: 77.4%). LC/MS (ESI) m/z: 575 $[M+H]^+$.

**Step 6: 1-cyano-3-(5-(difluoromethyl)-1,3,4-thiadiazol-2-yl)-8-(4-isobutyrylpiperazin-1-yl)-N-(1-methylcyclopropyl)-N-((2-(trimethylsilyl)ethoxy)methyl)imidazo[1,5-a]pyridine-6-sulfonamide**

Under an atmosphere of nitrogen at room temperature, cesium carbonate (79 mg, 0.244 mmol), Ruphos (11 mg, 0.024 mmol) and Ruphos Pd G3 (10 mg, 0.012 mmol) were added to the solution of 8-chloro-1-cyano-3-(5-(difluoromethyl)-1,3,4-thiadiazol-2-yl)-N-(1-methylcyclopropyl)-N-((2-(trimethylsilanyl)ethoxy)methyl)imidazo[1,5-a]pyridin-6-sulfonamide (70 mg, 0.122 mmol) and 2-methyl-1-(piperazin-1-yl)propan-1-one (38 mg, 0.244 mmol) in 1,4-dioxane (3 mL). The flask was replaced with nitrogen gas three times and then the mixture was stirred at 85°C for 3 hours under an atmosphere of nitrogen. After completion of the reaction, the reaction mixture was cooled to room temperature, added with water, and extracted with ethyl acetate. The organic phase was washed with saturated brine, dried over anhydrous sodium sulfate, and concentrated under reduced pressure. The residue was separated by column chromatography (eluent: petroleum ether/ethyl acetate, gradient: 0-45% ethyl acetate) to obtain the title compound (yellow solid, 50 mg, yield: 59.1%). LC/MS (ESI) m/z: 695 $[M+H]^+$.

**Step 7: 1-cyano-3-(5-(difluoromethyl)-1,3,4-thiadiazol-2-yl)-8-(4-isobutyrylpiperazin-1-yl)-N-(1-methylcyclopropyl)imidazo[1,5-a]pyridine-6-sulfonamide**

At 0°C, trifluoroacetic acid (1 mL) was dropwise added slowly to the solution of 1-cyano-3-(5-(difluoromethyl)-1,3,4-thiadiazol-2-yl)-8-(4-isobutyrylpiperazin-1-yl)-N-(1-methylcyclopropyl)-N-((2-(trimethylsilicyl)ethoay)methyl)imidazo[1,5-a]pyridin-6-sulfonamide (50 mg, 0.072 mmol) in dichloromethane (2 mL). The mixture was stirred at room temperature for 30 minutes. The reaction mixture was concentrated under reduced pressure; and the residue was purified by pre-HPLC to obtain the title compound (yellow solid, 18.5 mg, yield: 45.5%). $^1H$ NMR (400 MHz, DMSO-$d_6$) $\delta$ 9.74 (s, 1H), 8.59 (s, 1H), 7.72 (t, $J$ = 53.1 Hz, 1H), 7.19 (s, 1H), 3.86 - 3.72 (m, 4H), 3.22 - 3.10 (m, 4H), 3.02 - 2.93 (m, 1H), 1.16 (s, 3H), 1.05 (d, $J$ = 6.4 Hz, 6H), 0.77 - 0.70 (m, 2H), 0.50 - 0.43 (m, 2H). LC/MS (ESI) (m/z): 565 $[M+H]^+$.

**By referring to the preparation method in Example 163, the following examples were implemented by synthesizing a crude product according to a similar route by starting from appropriate starting materials, treating the crude product via reversed-phase HPLC, and then freeze-drying the crude product to obtain the title compound.**

| Example | Structure and name | Analysis data |
|---|---|---|
| **Example 164** | 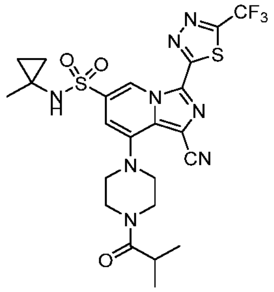 **1-cyano-8-(4-isobutyrylpiperazin-1-yl)-N-(1-methylcyclopropyl)-3-(5-(trifluoromethyl)-1,3,4-thiadiazol-2-yl)imidazo[1,5-a]pyridine-6-sulfonamide** | $^1$H NMR (400 MHz, DMSO-$d_6$) δ 9.71 (s, 1H), 8.61 (s, 1H), 7.22 (s, 1H), 3.86 - 3.75 (m, 4H), 3.22 - 3.12 (m, 4H), 3.00 - 2.94 (m, 1H), 1.17 (s, 3H), 1.05 (d, *J* = 6.7 Hz, 6H), 0.77 - 0.71 (m, 2H), 0.50 - 0.44 (m, 2H). LC/MS (ESI) (m/z): 583 [M+H]$^+$. |
| **Example 165** | 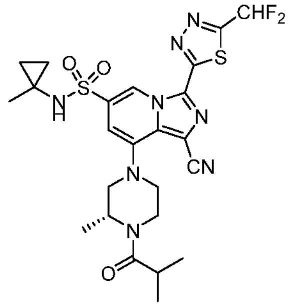 **(R)-1-cyano-3-(5-(difluoromethyl)-1,3,4-thiadiazol-2-yl)-8-(4-isobutyryl-3-methylpiperazin-1-yl)-N-(1-methylcyclopropyl)imidazo[1,5-a]pyridine-6-sulfonamide** | $^1$H NMR (400 MHz, DMSO-$d_6$) δ 9.74 (s, 1H), 8.60 (s, 1H), 7.73 (t, *J* = 53.1 Hz, 1H), 7.19 (s, 1H), 4.93 - 4.28 (m, 1H), 4.01 - 3.81 (m, 1H), 3.70 (d, *J* = 12.4 Hz, 1H), 3.21 - 3.07 (m, 3H), 3.00 - 2.77 (m, 2H), 1.51 - 1.32 (m, 3H), 1.18 (s, 3H), 1.05 (d, *J* = 6.6 Hz, 6H), 0.77 - 0.72 (m, 2H), 0.50 - 0.45 (m, 2H). LC/MS (ESI) (m/z): 579 [M+H]$^+$. |
| **Example 166** | 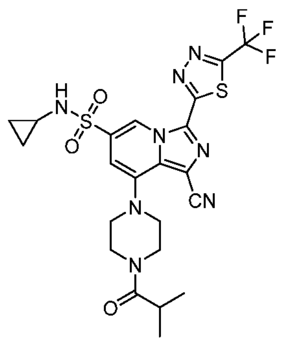 **1-cyano-N-cyclopropyl-8-(4-isobutyrylpiperazin-1-yl)-3-(5-(trifluoromethyl)-1,3,4-thiadiazol-2-yl)imidazo [1,5-a]pyridine-6-sulfonamide** | $^1$H NMR (400 MHz, DMSO-$d_6$) δ 9.74 (s, 1H), 8.40 (s, 1H), 7.22 (s, 1H), 3.88 - 3.77 (m, 4H), 3.24 - 3.16 (m, 4H), 3.01 - 2.95 (m, 1H), 2.34 - 2.29 (m, 1H), 1.08 - 1.04 (m, 6H), 0.57 - 0.49 (m, 4H). LC/MS (ESI) (m/z): 569 [M+H]$^+$. |

**Example 167: N-(1-cyanocyclopropyl)-8-(4-isobutyrylpiperazin-1-yl)-3-(5-(trifluoromethyl)-1,3,4-thiadiazol-2-yl) imidazo[1,5-a]pyridine-6-sulfonamide**

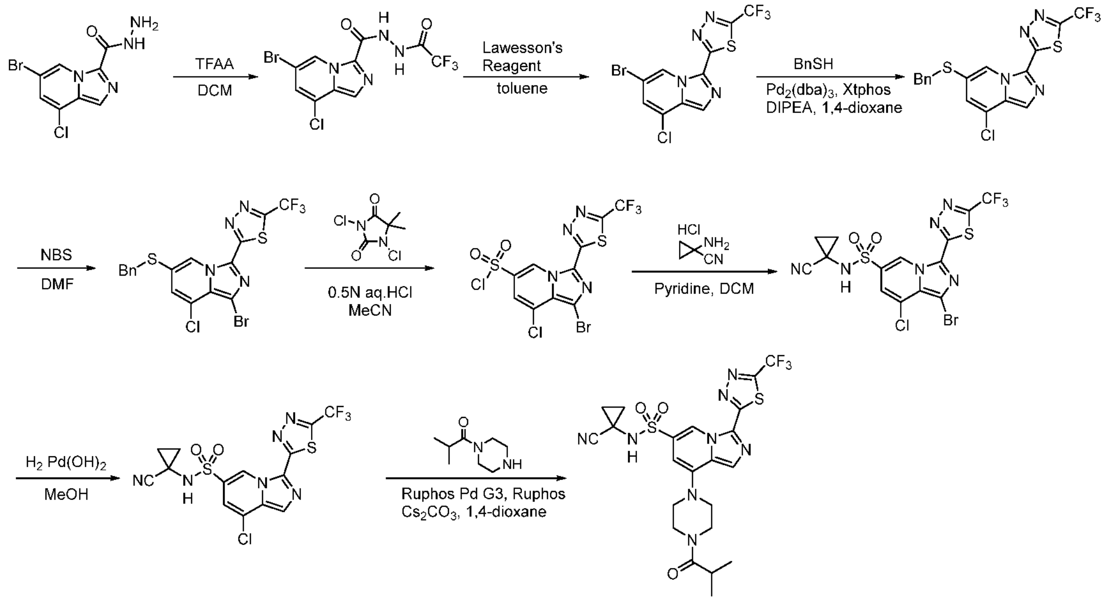


**Step 1: 6-bromo-8-chloro-N'-(2,2,2-trifluoroacetyl)imidazo[1,5-a]pyridine-3-carbohydrazide**

Under an atmosphere of nitrogen, trifluoroacetic anhydride (435 mg, 2.07 mmol) was added to the solution of 6-bromo-8-chloroimidazo[1,5-a]pyridin-3-carbohydrazide (600 mg, 2.07 mmol) in dichloromethane (6 mL). The reaction mixture was stirred at room temperature for 30 minutes. After completion of the reaction, water was added, and the reaction mixture was extracted with dichloromethane. The organic phase was washed with saturated brine, dried over anhydrous sodium sulfate, and concentrated under reduced pressure. The residue was separated by column chromatography (eluent: petroleum ether, ethyl acetate, gradient: 0-15% ethyl acetate) to obtain the title compound (yellow solid, 600 mg, yield: 75.1%). LC/MS (ESI) (m/z): 385 $[M+H]^+$.

**Step 2: 2-(6-bromo-8-chloroimidazo[1,5-a]pyridin-3-yl)-5-(trifluoromethyl)-1,3,4-thiadiazole**

Under an atmosphere of nitrogen, the Lawesson's reagent (692 mg, 1.71 mmol) was added to the solution of 6-bromo-8-chloro-N'-(2,2,2-trifluoroacetyl)imidazo[1,5-a]pyridin-3-carbohydrazide (600 mg, 1.56 mmol) in anhydrous toluene (6 mL). The reaction mixture was stirred at 110°C for 4 hours. After completion of the reaction, sodium bicarbonate was added, and the reaction mixture was extracted with ethyl acetate. The organic phase was washed with saturated brine, dried over anhydrous sodium sulfate, and concentrated under reduced pressure. The residue was separated by column chromatography (eluent: petroleum ether, ethyl acetate, gradient: 0-20% ethyl acetate) to obtain the title compound (yellow solid, 500 mg, yield: 83.7%). LC/MS (ESI) (m/z): 383 $[M+H]^+$.

**Step 3: 2-(6-(benzylthio)-8-chloroimidazo[1,5-a]pyridin-3-yl)-5-(trifluoromethyl)-1,3,4-thiadiazole**

Under an atmosphere of nitrogen, N,N-diisopropylethylamine (505 mg, 3.91 mmol), Xantphos (151 mg, 0.26 mmol), $Pd_2(dba)_3$ (119 mg, 0.13 mmol) were sequentially added to the solution of 2-(6-bromo-8-chloroimidazo[1,5-a]pyridin-3-yl)-5-(trifluoromethyl)-1,3,4-thiadiazole (500 mg, 1.30 mmol) and benzyl mercaptane (0.15 mL, 1.30 mmol) in 1,4-dioxane (5 mL). The flask was replaced with nitrogen gas three times and then the mixture was stirred at 80 °C for 3 hours under an atmosphere of nitrogen. After completion of the reaction, water was added, and the reaction mixture was extracted with ethyl acetate. The organic phase was washed with saturated brine, dried over anhydrous sodium sulfate, and concentrated under reduced pressure. The residue was purified by column chromatography (eluent: petroleum ether, ethyl acetate, gradient: 0-25% ethyl acetate) to obtain the title compound (yellow solid, 510 mg, yield: 91.6%). LC/MS (ESI) (m/z): 427 $[M+H]^+$.

**Step 4: 2-(6-(benzylthio)-1-bromo-8-chloroimidazo[1,5-a]pyridin-3-yl)-5-(trifluoromethyl)-1,3,4-thiadiazole**

At 0°C, NBS (213 mg, 1.20 mmol) was added to the solution of 2-(6-(benzylthio)-8-chloroimidazo[1,5-a]pyridin-3-yl)-5-(trifluoromethyl)-1,3,4-thiadiazole (510 mg, 1.20 mmol) in N,N-dimethylformamide (5 mL). The reaction mixture was stirred at room temperature for 30 minutes. After completion of the reaction, water was added, and the reaction mixture was

extracted with ethyl acetate. The organic phase was washed with saturated brine, dried over anhydrous sodium sulfate, and concentrated under reduced pressure. The residue was purified by column chromatography (eluent: petroleum ether, ethyl acetate, gradient: 0-25% ethyl acetate) to obtain the title compound (yellow solid, 550 mg, yield: 91.2%). LC/MS (ESI) (m/z): 505 $[M+H]^+$.

**Step 5: 1-bromo-8-chloro-3-(5-(trifluoromethyl)-1,3,4-thiadiazol-2-yl)imidazo[1,5-a]pyridine-6-sulfonyl chloride**

At 0°C,1N hydrochloric acid (1.25 mL), water (1.25 mL) and dichlorohydantoin (643 mg, 3.27 mmol) were sequentially added to the solution of 2-(6-(benzylthio)-1-bromo-8-chloroimidazo[1,5-a]pyridin-3-yl)-5-(trifluoromethyl)-1,3,4-thiadiazole (550 mg, 1.10 mmol) in acetonitrile (5 mL). The reaction mixture was stirred at 0°C for 10 minutes. After completion of the reaction, water was added, and the reaction mixture was extracted with dichloromethane. The organic phase was washed with saturated brine, dried over anhydrous sodium sulfate, and concentrated under reduced pressure. The residue was pulped with petroleum ether, and then dried in vacuum to obtain the title compound (yellow solid, 520 mg, yield: 99.2%). LC/MS (ESI) (m/z): 481 $[M+H]^+$.

**Step 6: 1-bromo-8-chloro-N-(1-cyanocyclopropyl)-3-(5-(trifluoromethyl)-1,3,4-thiadiazol-2-yl)imidazo[1,5-a]pyridine-6-sulfonamide**

At 0°C, anhydrous pyridine (1 mL) was added to the solution of 1-amino-1-cyclopropyl cyanic hydrochloride (255 mg, 2.16 mmol) in anhydrous dichloromethane (4 mL); and the reaction mixture was stirred at 0°C at for 5 minutes. Then, 1-bromo-8-chloro-3-(5-(trifluoromethyl)-1,3,4-thiadiazol-2-yl)imidazo[1,5-a]pyridin-6-sulfonyl chloride (520 mg, 1.01 mmol) was added to the reaction mixture. The reaction mixture was stirred at room temperature for 30 minutes. After completion of the reaction, water was added, and the reaction mixture was extracted with ethyl acetate. The organic phase was washed with saturated brine, dried over anhydrous sodium sulfate, and concentrated under reduced pressure. The residue was purified by column chromatography (eluent: petroleum ether, ethyl acetate, gradient: 0-30% ethyl acetate) to obtain the title compound (yellow solid, 450 mg, yield: 79.0%). LC/MS (ESI) (m/z): 527 $[M+H]^+$.

**Step 7: 8-chloro-N-(1-cyanocyclopropyl)-3-(5-(trifluoromethyl)-1,3,4-thiadiazol-2-yl)imidazo[1,5-a]pyridine-6-sulfonamide**

Under an atmosphere of nitrogen, palladium hydroxide on carbon (40 mg) with the mass fraction of 20% was added to the solution of 1-bromo-8-chloro-N-(1-cyanocyclopropyl)-3-(5-(trifluoromethyl)-1,3,4-thiadiazol-2-yl)imidazo [1,5-a]pyridin-6-sulfonamide (450 mg, 0.85 mmol) in anhydrous methanol (20 mL). The flask was replaced with nitrogen gas three times and then the mixture was stirred at 20°C for 24 hours under an atmosphere of hydrogen at one atmospheric pressure. After completion of the reaction, the reaction mixture was filtered; and the filtrate as concentrated under reduced pressure to obtain the title compound (yellow solid, 150 mg, yield: 39.2%). LC/MS (ESI) (m/z): 449 $[M+H]^+$.

**Step 8: N-(1-cyanocyclopropyl)-8-(4-isobutyrylpiperazin-1-yl)-3-(5-(trifluoromethyl)-1,3,4-thiadiazol-2-yl)imidazo[1,5-a]pyridine-6-sulfonamide**

Under an atmosphere of nitrogen, cesium carbonate (101 mg, 0.31 mmol), Ruphos (14 mg, 0.031 mmol), Ruphos Pd G3 (26 mg, 0.031 mmol) were sequentially added to the solution of 8-chloro-N-(1-cyanocyclopropyl)-3-(5-(trifluoromethyl)-1,3,4-thiadiazol-2-yl)imidazo[1,5-a]pyridin-6-sulfonamide (70 mg, 0.16 mmol) and 2-methyl-1-(piperazin-1-yl) propan-1-one (48 mg, 0.31 mmol) in 1,4-dioxane (2.1 mL). The flask was replaced with nitrogen gas three times and then the mixture was stirred at 80 °C for 30 minutes under an atmosphere of nitrogen. After completion of the reaction, water was added, and the reaction mixture was extracted with ethyl acetate. The organic phase was washed with saturated brine, dried over anhydrous sodium sulfate, and concentrated under reduced pressure. The residue was separated by column chromatography (eluent: petroleum ether, ethyl acetate, gradient: 0-50% ethyl acetate), and then purified by pre-HPLC (C18, 10-50% acetonitrile in $H_2O$, containing 0.1% formic acid) to further obtain the title compound (yellow solid, 32.2 mg, yield: 36.3%). $^1H$ NMR (400 MHz, DMSO-$d_6$) δ 9.66 (s, 1H), 9.50 (s, 1H), 8.15 (s, 1H), 6.71 (s, 1H), 3.82 - 3.72 (m, 4H), 3.40 - 3.35 (m, 4H), 2.98 - 2.92 (m, 1H), 1.51 - 1.46 (m, 2H), 1.42 - 1.35 (m, 2H), 1.05 (d, *J* = 6.7 Hz, 6H). LC/MS (ESI) (m/z): 569 $[M+H]^+$.

**Example 168: 8-(4-(1-(methoxy-d3)cyclopropane-1-carbonyl)piperazin-1-yl)-N-(1-methylcyclopropyl)-3-(5-(trifluoromethyl)-1,3,4-thiadiazol-2-yl)imidazo [1,5-a]pyridine-6-sulfonamide**

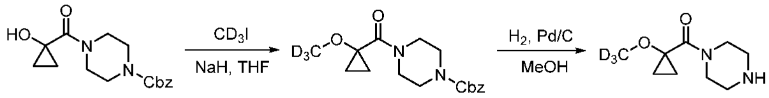


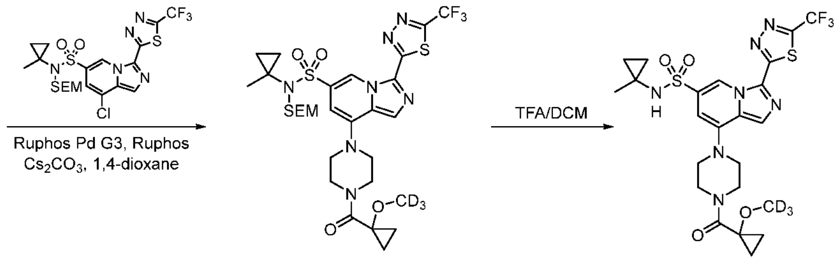


**Step 1: Benzyl 4-(1-(methoxy-d3)cyclopropane-1-carbonyl)piperazine-1-carboxylate**

At 0°C, sodium hydride (160 mg, 3.94 mmol, mass fraction: 60%) was added portionwise to the solution of 4-(1-hydroxycyclopropan-1-acyl)piperazin-1-benzyl formate (400 mg, 1.31 mmol) in tetrahydrofuran (10 mL); and the reaction mixture was stirred at room temperature for 30 minutes. The reaction mixture was cooled to room temperature, deuteroiodomethane (380 mg, 2.62 mmol) was dropwise added at 0°C, and the reaction mixture was stirred at room temperature for 3 hours. After completion of the reaction, the reaction mixture was quenched with saturated ammonium chloride that was cooled with ice water, and extracted with ethyl acetate twice. The organic phase was washed with saturated brine, dried over anhydrous sodium sulfate, and concentrated under reduced pressure. The residue was separated by column chromatography (eluent: petroleum ether/ethyl acetate, gradient: 0-60% ethyl acetate) to obtain the title compound (white solid, 150 mg, yield: 35.7%). LC/MS (ESI) (m/z): 322 $[M+H]^+$.

**Step 2: (1-(methoxy-d3)cyclopropyl)(piperazin-1-yl)methanone**

Under an atmosphere of nitrogen, palladium on carbon (20 mg) with the mass fraction of 10% was added to the solution of 4-(1-(deuteromethoxy)cyclopropan-1-acyl)piperazin-1-benzyl carboxylate (150 mg, 0.47 mmol) in ethyl acetate (8 mL). The flask was replaced with nitrogen gas three times and then the mixture wasstirred at room temperature for 3 hours under an atmosphere of hydrogen at one atmospheric pressure. After completion of the reaction, the reaction mixture was filtered; and the filtrate was concentrated under reduced pressure to obtain the title compound (colorless oil, 80 mg, yield: 91.1%). LC/MS (ESI) (m/z): 188 $[M+H]^+$.

**Step 3: 8-(4-(1-(methoxy-d3)cyclopropane-1-carbonyl)piperazin-1-yl)-N-(1-methylcyclopropyl)-3-(5-(trifluoromethyl)-1,3,4-thiadiazol-2-yl)-N-((2-(trimethylsilyl)ethoxy)methyl)imidazo[1,5-a]pyridine-6-sulfonamide**

Under an atmosphere of nitrogen, cesium carbonate (87 mg, 0.27mmol), Ruphos (8 mg, 0.02 mmol) and Ruphos Pd G3 (8 mg, 0.01 mmol) were sequentially added to the solution of 8-chloro-N-(1-methylcyclopropyl)-3-(5-(trifluoromethyl)-1,3,4-thiadiazol-2-yl)-N-(2-(trimethylsilyl)ethoxy)methyl)imidazo[1,5-a]pyridin-6-sulfonamide (50 mg, 0.09 mmol) and (1-(deuteromethoxy)cyclopropan-1-acyl)piperazine (25 mg, 0.13 mmol) in 1,4-dioxane (5 mL). The flask was replaced with nitrogen gas three times and then the mixture was stirred at 80°C for 3 hours under an atmosphere of nitrogen. After completion of the reaction, the reaction mixture was cooled to room temperature, diluted with ethyl acetate, washed with saturated brine, dried over anhydrous sodium sulfate, and concentrated under reduced pressure. The residue was separated by column chromatography (eluent: petroleum ether/ethyl acetate, gradient: 0-50% ethyl acetate) to obtain the title compound (yellow solid, 40 mg, yield: 63.2%). LC/MS (ESI) (m/z): 719 $[M+H]^+$.

**Step 4: 8-(4-(1-(methoxy-d3)cyclopropane-1-carbonyl)piperazin-1-yl)-N-(1-methylcyclopropyl)-3-(5-(trifluoromethyl)-1,3,4-thiadiazol-2-yl)imidazo[1,5-a]pyridine-6-sulfonamide**

At 0°C, trifluoroacetic acid (2 mL) was added to the solution of (8-(4-(1-(deuteromethoxy)cyclopropan-1-acyl) piperazin-1-yl)-N-(1-methylcyclopropyl)-3-(5-(trifluoromethyl)-1,3,4-thiadiazol-2-yl)-N-(2-(trimethylsilyl)ethoxy)methyl) imidazo[1,5-a]pyridin-6-sulfonamide (40 mg, 0.06 mmol) in dichloromethane (5 mL); and the reaction mixture was stirred at room temperature for 30 minutes. The reaction mixture was diluted with dichloromethane, washed with saturated brine, dried over anhydrous sodium sulfate, and concentrated under reduced pressure. The residue was purified by pre-HPLC to obtain the title compound (yellow solid, 12 mg, yield: 36.6%). $^1H$ NMR (400 MHz, DMSO-$d_6$) $\delta$ 9.59 (s, 1H), 8.44 (s, 1H),

8.14 (s, 1H), 6.76 (s, 1H), 4.12 - 3.70 (m, 4H), 3.42 - 3.36 (m, 4H), 1.16 (s, 3H), 1.06 - 1.01 (m, 2H), 0.96 - 0.91 (m, 2H), 0.76 - 0.71 (m, 2H), 0.48 - 0.44 (m, 2H). LC/MS (ESI) (m/z): 589 [M+H]$^+$.

**Example 169: 3-(5-(difluoromethyl)-1,3,4-thiadiazol-2-yl)-1-methyl-N-(3-methyloxetan-3-yl)-8-(2-oxa-7-azaspiro [3.5]nonan-7-yl)imidazo[1,5-a]pyridine-6-sulfonamide**

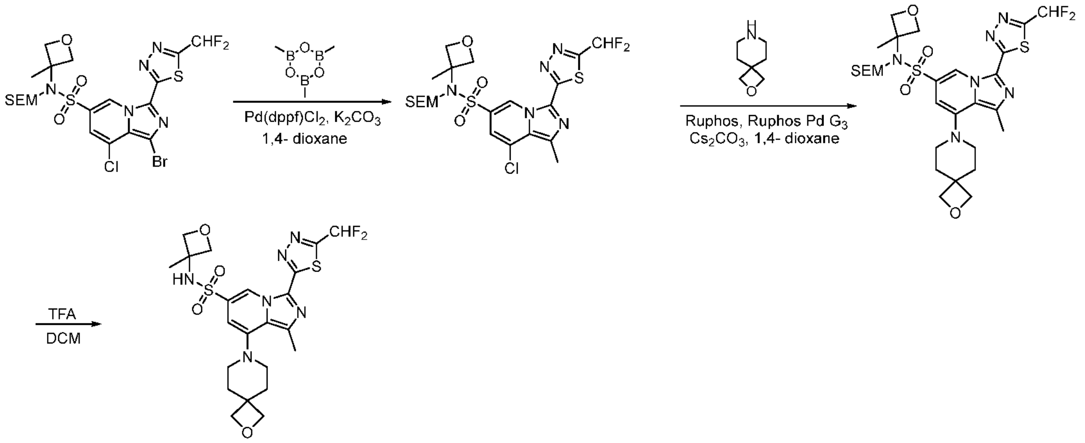


**Step 1: 8-chloro-3-(5-(difluoromethyl)-1,3,4-thiadiazol-2-yl)-1-methyl-N-(3-methyloxetan-3-yl)-N-((2-(trimethylsilyl)ethoxy)methyl)imidazo[1,5-a]pyridine-6-sulfonamide**

Under an atmosphere of nitrogen, Pd(dppf)$Cl_2$ (9 mg, 0.02 mmol) and potassium carbonate (120 mg, 0.69 mmol) were added to the solution of 1-bromo-8-chloro-3-(5-(difluoromethyl)-1,3,4-thiadiazol-2-yl)-N-(3-methyloxacyclobutan-3-yl)-N-((2-(trimethylsilicyl)ethoxy)methyl)imidazo[1,5-a]pyridin-6-sulfonamide (150 mg, 0.23 mmol) and 2,4,6-trimethyl-1,3,5,2,4,6-trimethoxytriborane (0.1 ml, 0.34 mmol) in 1, 4- dioxane (2 mL) and water (0.5 mL). The flask was replaced with nitrogen gas three times and then the mixture was stirred at 80°C for 16 hours under an atmosphere of nitrogen. After completion of the reaction, the reaction mixture was diluted with ethyl acetate, and extracted with ethyl acetate three times. The organic phase was dried over anhydrous sodium sulfate, filtered and concentrated under reduced pressure. The residue was separated by column chromatography (eluent: petroleum ether/ethyl acetate, gradient: 0-100% ethyl acetate) to obtain the title compound (yellow solid, 50 mg, yield: 37.3%). LC/MS (ESI) (m/z): 580 [M+H]$^+$.

**Step 2: 3-(5-(difluoromethyl)-1,3,4-thiadiazol-2-yl)-1-methyl-N-(3-methyloxetan-3-yl)-8-(2-oxa-7-azaspiro[3.5] nonan-7-yl)-N-((2-(trimethylsilyl)ethoxy)methyl)imidazo[1,5-a]pyridine-6-sulfonamide**

Under an atmosphere of nitrogen, Ruphos (4 mg, 0.01 mmol), Ruphos Pd $G_3$(4 mg, 0.01 mmol), and cesium carbonate (75 mg, 0.27 mmol) were added to the solution of 8-chloro-3-(5-(difluoromethyl)-1,3,4-thiadiazol-2-yl)-1-methyl-N-(3-methyloxacyclobutan-3-yl)-N-((2-(trimethylsilicyl)ethoxy)methyl)imidazo[1,5-a]pyridin-6-sulfonamide (50 mg, 0.09 mmol) and 2-oxa-7-azaspiro[3.5]nonane (15 mg, 0.09 mmol) in 1,4-dioxane (2 mL). of nitrogen, the reaction mixture was stirred at 80°C Under an atmospherefor 3 hours. After completion of the reaction, the reaction mixture was diluted with ethyl acetate, washed with water and saturated brine, dried over anhydrous sodium sulfate, filtered and concentrated under reduced pressure. The residue was separated by column chromatography (eluent: petroleum ether/ethyl acetate, gradient: 0-100% ethyl acetate) to obtain the title compound (yellow solid, 20 mg, yield: 33%). LC/MS (ESI) (m/z): 671 [M+H]$^+$.

**Step 3: 3-(5-(difluoromethyl)-1,3,4-thiadiazol-2-yl)-1-methyl-N-(3-methyloxetan-3-yl)-8-(2-oxa-7-azaspiro[3.5] nonan-7-yl)imidazo[1,5-a]pyridine-6-sulfonamide**

At 0°C, trifluoroacetic acid (1 mL) was added to the solution of 3-(5-(difluoromethyl)-1,3,4-thiadiazol-2-yl)-1-methyl-N-(3-methyloxacyclobutan-3-yl)-8-(2-oxa-7-azaspiro[3.5]non-7-yl)-N-(2-(trimethylsilicyl)ethoxy)methyl)imidazo [1,5-a]pyridin-6-sulfonamide (20 mg, 0.03 mmol) in dichloromethane (1 mL). The reaction mixture was stirred at 25°C for 1 hours. After completion of the reaction, the reaction mixture was concentrated under reduced pressure. The residue was purified by pre-HPLC to obtain the title compound (yellow solid, 1.5 mg, yield: 9.2%). $^1$H NMR (400 MHz, DMSO-$d_6$) δ 9.56

(s, 1H), 8.66 (s, 1H), 7.66 (t, *J* = 53.6 Hz, 1H), 6.73 (s, 1H), 4.62 (d, *J* = 6.2 Hz, 2H), 4.40 (s, 4H), 4.17 (d, *J* = 6.3 Hz, 2H), 3.01 - 2.97 (m, 4H), 2.70 (s, 3H), 2.10 - 1.98 (m, 4H), 1.48 (s, 3H). LC/MS (ESI) (m/z): 541 [M+H]$^+$.

**By referring to the preparation method in Example 169, the following examples were implemented by synthesizing a crude product according to a similar route by starting from appropriate starting materials, treating the crude product via reversed-phase HPLC, and then freeze-drying the crude product to obtain the title compound.**

| Example | Structure and name | Analysis data |
|---|---|---|
| **Example 170** | 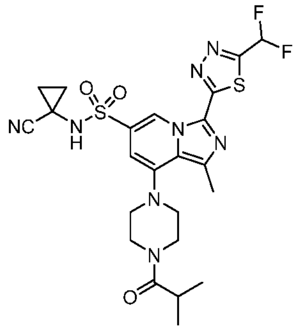 **N-(1-cyanocyclopropyl)-3-(5-(difluoromethyl)-1,3,4-thiadiazol-2-yl)-8-(4-isobutyrylpiperazin-1-yl)-1-methylimidazo[1,5-a]pyridine-6-sulfonamide** | $^1$H NMR (400 MHz, DMSO-$d_6$) δ 9.66 (s, 1H), 7.61 (t, *J* = 52.9 Hz, 1H), 6.75 (s, 1H), 3.86 - 3.67 (m, 4H), 3.12 - 3.01 (m, 4H), 2.96 - 2.92 (m, 1H), 2.74 (s, 3H), 1.46 - 1.41 (m, 2H), 1.37 - 1.32 (m, 2H), 1.05 (d, *J* = 6.7 Hz, 6H). LC/MS (ESI) (m/z): 565 [M+H]$^+$. |
| **Example 171** | 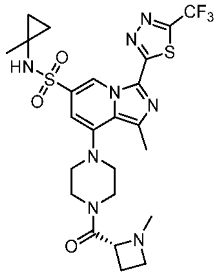 **(R)-1-methyl-8-(4-(1-methylazetidine-2-carbonyl)piperazin-1-yl)-N-(1-methylcyclopropyl)-3-(5-(trifluoromethyl)-1,3,4-thiadiazol-2-yl)imidazo [1,5-a]pyridine-6-sulfonamide** | $^1$H NMR (400 MHz, MeOD) δ 9.80 (s, 1H), 6.81 (s, 1H), 4.97 - 4.90 (m, 1H), 3.98 - 3.83 (m, 2H), 3.83 - 3.65 (m, 2H), 3.64 - 3.55 (m, 2H), 3.25 - 3.06 (m, 4H), 2.80 (s, 3H), 2.76 (s, 3H), 2.73 - 2.64 (m, 1H), 2.56 - 2.47 (m, 1H), 1.26 (s, 3H), 0.86 - 0.77 (m, 2H), 0.56 - 0.47 (m, 2H). LC/MS (ESI) (m/z): 600 [M+H]$^+$. |
| **Example 172** | 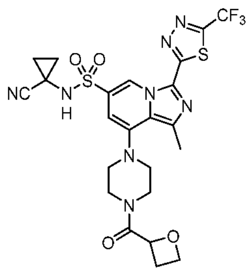 **N-(1-cyanocyclopropyl)-1-methyl-8-(4-(oxetane-2-carbonyl)piperazin-1-yl)-3-(5-(trifluoromethyl)-1,3,4-thiadiazol-2-yl)imidazo[1,5-a]pyridine-6-sulfonamide** | $^1$H NMR (400 MHz, MeOD) δ 9.89 (s, 1H), 6.85 (s, 1H), 5.61 - 5.49 (m, 1H), 4.75 - 4.51 (m, 2H), 4.00 - 3.69 (m, 4H), 3.66 - 3.55 (m, 2H), 3.23 - 3.17 (m, 2H), 3.02 - 2.84 (m, 2H), 2.80 (s, 3H), 1.55 - 1.52 (m, 2H), 1.47 - 1.44 (m, 2H). LC/MS (ESI) (m/z): 597 [M+H]$^+$. |

**Example 173: 8-(4-acetylpiperazin-1-yl)-N-(1-cyanocyclopropyl)-3-(5-(trifluoromethyl)-1,3,4-thiadiazol-2-yl)imidazo [1,5-a]pyridine-6-sulfonamide**

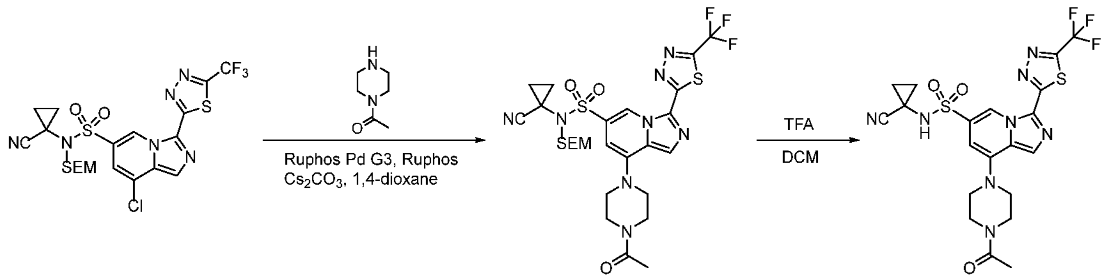


**Step 1: 8-(4-acetylpiperazin-1-yl)-N-(1-cyanocyclopropyl)-3-(5-(trifluoromethyl)-1,3,4-thiadiazol-2-yl)-N-((2-(trimethylsilyl)ethoxy)methyl)imidazo[1,5-a]pyridine-6-sulfonamide**

Under an atmosphere of nitrogen, cesium carbonate (68 mg, 0.21 mmol), Ruphos (7 mg, 0.014 mmol) and Ruphos Pd G3 (7 mg, 0.007 mmol) were sequentially added to the solution of 8-chloro-N-(1-cyanocyclopropyl)-3-(5-(trifluoromethyl)-1,3,4-thiadiazol-2-yl)-N-(2-(trimethylsilyl)ethoxy)methyl)imidazol[1,5-a]pyridin-6-sulfonamide (40 mg, 0.07 mmol) and 1-acetpiperazine (12 mg, 0.09 mmol) in 1,4-dioxane (3 mL). The flask was replaced with nitrogen gas three times and then the mixture was stirred at 80°C for 4 hours under an atmosphere of nitrogen. After completion of the reaction, the reaction mixture was cooled to room temperature, diluted with ethyl acetate, washed with saturated brine, dried over anhydrous sodium sulfate, and concentrated under reduced pressure. The residue was separated by column chromatography (eluent: dichloromethane/methanol, gradient: 0-20% methanol) to obtain the title compound (yellow solid, 40 mg, yield: 86.3%). LC/MS (ESI) m/z: 671 $[M+H]^+$.

**Step 2: 8-(4-acetylpiperazin-1-yl)-N-(1-cyanocyclopropyl)-3-(5-(trifluoromethyl)-1,3,4-thiadiazol-2-yl)imidazo [1,5-a]pyridine-6-sulfonamide**

At 0°C, trifluoroacetic acid (0.5 mL) was dropwise added slowly to the solution of 8-(4-acetpiperazin-1-yl)-N-(1-cyanocyclopropyl)-3-(5-(trifluoromethyl)-1,3,4-thiadiazol-2-yl)-N-(2-(trimethylsilyl)ethoxy)methyl)imidazol[1,5-a]pyridin-6-sulfonamide (40 mg, 0.06mmol) in dichloromethane (2 mL). The mixture was stirred at room temperature for 30 minutes. The mixture was concentrated under reduced pressure; and the residue was purified by pre-HPLC to obtain the title compound (yellow solid, 8 mg, yield: 24.6%). $^1$H NMR (400 MHz, DMSO-$d_6$) δ 9.66 (s, 1H), 8.14 (s, 1H), 6.71 (s, 1H), 3.74 - 3.69 (m, 4H), 3.45 - 3.40 (m, 4H), 2.09 (s, 3H), 1.51 - 1.46 (m, 2H), 1.41 - 1.33 (m, 2H). LC/MS (ESI) (m/z): 541 $[M+H]^+$.

**By referring to the preparation method in Example 173, the following examples were implemented by synthesizing a crude product according to a similar route by starting from appropriate starting materials, treating the crude product via reversed-phase HPLC, and then freeze-drying the crude product to obtain the title compound.**

| Example | Structure and name | Analysis data |
|---|---|---|
| **Example 174** | 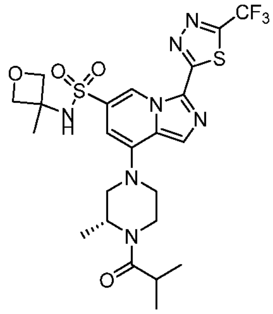 **(R)-8-(4-isobutyryl-3-methylpiperazin-1-yl)-N-(3-methyloxetan-3-yl)-3-(5-(trifluoromethyl)-1,3,4-thiadiazol-2-yl)imidazo[1,5-a]pyridine-6-sulfonamide** | $^1$H NMR (400 MHz, DMSO-$d_6$) δ 9.55 (s, 1H), 8.74 (s, 1H), 7.99 (s, 1H), 6.72 (s, 1H), 4.89 - 4.67 (m, 1H), 4.65 - 4.61 (m, 2H), 4.47 - 4.32 (m, 1H), 4.20 - 4.15 (m, 2H), 4.12 - 3.85 (m, 1H), 3.73 - 3.64 (m, 2H), 3.13 - 2.87 (m, 3H), 1.52 - 1.43 (m, 3H), 1.37 - 1.25 (m, 3H), 1.08 - 1.00 (d, *J* = 6.5 Hz, 6H). LC/MS (ESI) (m/z): 588 $[M+H]^+$. |

(continued)

| Example | Structure and name | Analysis data |
|---|---|---|
| **Example 175** | 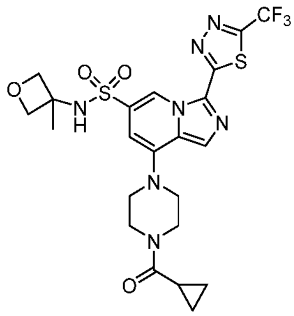 **8-(4-(cyclopropanecarbonyl)piperazin-1-yl)-N-(3-methyloxetan-3-yl)-3-(5-(trifluoromethyl)-1,3,4-thiadiazol-2-yl)imidazo[1,5-a]pyridine-6-sulfonamide** | $^1$H NMR (400 MHz, DMSO-$d_6$) δ 9.56 (s, 1H), 8.74 (s, 1H), 8.11 (s, 1H), 6.73 (s, 1H), 4.63 (d, *J* = 6.1 Hz, 2H), 4.18 (d, J = 6.2 Hz, 2H), 4.03 - 3.88 (m, 2H), 3.80 - 3.66 (m, 2H), 3.45 - 3.37 (m, 2H), 3.33 - 3.30 (m, 2H), 2.10 - 200 (m, 1H), 1.50 (s, 3H), 0.81 - 0.72 (m, 4H). LC/MS (ESI) (m/z): 572 [M+H]$^+$. |
| **Example 176** | 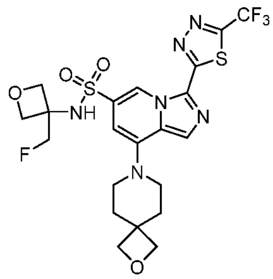 **N-(3-(fluoromethyl)oxetan-3-yl)-8-(2-oxa-7-azaspiro [3.5]nonan-7-yl)-3-(5-(trifluoromethyl)-1,3,4-thiadiazol-2-yl)imidazo[1,5-a]pyridine-6-sulfonamide** | $^1$H NMR (400 MHz, DMSO-$d_6$) δ 9.53 (s, 1H), 9.06 (s, 1H), 8.02 (s, 1H), 6.74 (s, 1H), 4.71 (s, 1H), 4.65 - 4.60 (m, 2H), 4.59 (s, 1H), 4.44 - 4.39 (m, 4H), 4.38 - 4.35 (m, 2H), 3.28 - 3.21 (m, 4H), 2.07 - 2.00 (m, 4H). LC/MS (ESI) (m/z): 563 [M+H]$^+$. |
| **Example 177** | 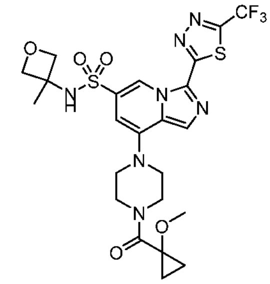 **8-(4-(1-methoxycyclopropane-1-carbonyl)piperazin-1-yl)-N-(3-methyloxetan-3-yl)-3-(5-(trifluoromethyl)-1,3,4-thiadiazol-2-yl) imidazo[1,5-a]pyridine-6-sulfonamide** | $^1$H NMR (400 MHz, DMSO-$d_6$) δ 9.56 (s, 1H), 8.72 (s, 1H), 8.12 (s, 1H), 6.76 (s, 1H), 4.63 (d, *J* = 6.1 Hz, 2H), 4.18 (d, *J* = 6.3 Hz, 2H), 4.09 - 3.70 (m, 4H), 3.41 - 3.37 (m, 4H), 3.27 (s, 3H), 1.49 (s, 3H), 1.08 - 0.99 (m, 2H), 0.97 - 0.89 (m, 2H). LC/MS (ESI) (m/z): 602 [M+H]$^+$. |
| **Example 178** | 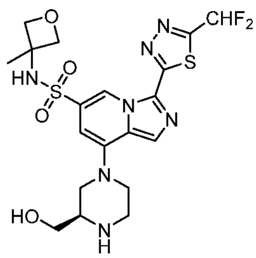 **(R)-3-(5-(difluoromethyl)-1,3,4-thiadiazol-2-yl)-8-(3-(hydroxymethyl)piperazin-1-yl)-N-(3-methyloxetan-3-yl)imidazo[1,5-a] pyridine-6-sulfonamide** | $^1$H NMR (400 MHz, $CD_3OD$) δ 9.84 (s, 1H), 7.92 (s, 1H), 7.33 (t, *J* = 53.5 Hz, 1H), 6.81 (s, 1H), 4.79 (d, *J* = 6.3 Hz, 2H), 4.32 (d, *J* = 6.5 Hz, 2H), 3.87 - 3.81 (m, 3H), 3.79 - 3.74 (m, 1H), 3.55 - 3.50 (m, 1H), 3.45 - 3.35 (m, 2H), 3.21 - 3.15 (m, 1H), 3.12 - 3.06 (m, 1H), 1.64 (s, 3H). LC/MS (ESI) (m/z): 516 [M+H]$^+$. |

(continued)

| Example | Structure and name | Analysis data |
|---|---|---|
| **Example 179** | 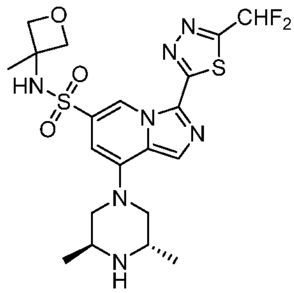 **3-(5-(difluoromethyl)-1,3,4-thiadiazol-2-yl)-8-((3S,5S)-3,5-dimethylpiperazin-1-yl)-N-(3-methyloxetan-3-yl)imidazo[1,5-a]pyridine-6-sulfonamide** | $^1$H NMR (400 MHz, $CD_3OD$) δ 9.85 (s, 1H), 7.86 (s, 1H), 7.33 (t, *J* = 53.5 Hz, 1H), 4.80 - 4.79 (m, 2H), 4.33 - 4.31 (m, 2H), 3.75 - 3.69 (m, 2H), 3.49 - 3.45 (m, 2H), 3.28 - 3.22 (m, 2H), 1.64 (s, 3H), 1.46 (d, *J* = 6.6 Hz, 6H). LC/MS (ESI) (m/z): 514 $[M+H]^+$. |
| **Example 180** | 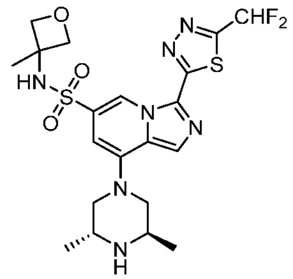 **3-(5-(difluoromethyl)-1,3,4-thiadiazol-2-yl)-8-((3R,5R)-3,5-dimethylpiperazin-1-yl)-N-(3-methyloxetan-3-yl)imidazo[1,5-a]pyridine-6-sulfonamide** | $^1$H NMR (400 MHz, DMSO-$d_6$) δ 9.62 (s, 1H), 8.96 (s, 1H), 8.74 (s, 1H), 8.01 (s, 1H), 7.68 (t, *J* = 53.1 Hz, 1H), 6.79 (s, 1H), 4.67 - 4.61 (m, 2H), 4.22 - 4.17 (m, 2H), 3.94 - 3.85 (m, 2H), 3.53 - 3.48 (m, 2H), 3.29 - 3.24 (m, 2H), 1.51 (s, 3H), 1.47 - 1.44 (m, 3H), 1.43 - 1.41 (m, 3H). LC/MS (ESI) (m/z): 514 $[M+H]^+$. |
| **Example 181** | 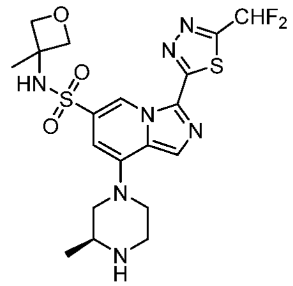 **(S)-3-(5-(difluoromethyl)-1,3,4-thiadiazol-2-yl)-N-(3-methyloxetan-3-yl)-8-(3-methylpiperazin-1-yl)imidazo[1,5-a]pyridine-6-sulfonamide** | $^1$H NMR (400 MHz, $CD_3OD$) δ 9.85 (d, J= 3.1 Hz, 1H), 7.91 (d, *J* = 2.2 Hz, 1H), 7.26 (t, *J* = 53.5 Hz, 1H), 6.80 (s, 1H), 4.79 (d, *J* = 6.1 Hz, 2H), 4.32 (d, *J* = 5.7 Hz, 2H), 3.86 - 3.80 (m, 2H), 3.54 - 3.41 (m, 3H), 3.17 - 3.11 (m, 1H), 2.93 - 2.87 (m, 1H), 1.64 (s, 3H), 1.45 - 1.34 (m, 4H). LC/MS (ESI) (m/z): 500 $[M+H]^+$. |
| **Example 182** | 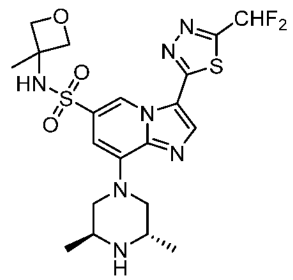 **3-(5-(difluoromethyl)-1,3,4-thiadiazol-2-yl)-8-((3S,5S)-3,5-dimethylpiperazin-1-yl)-N-(3-methyloxetan-3-yl)imidazo[1,2-a]pyridine-6-sulfonamide** | $^1$H NMR (400 MHz, $CD_3OD$) δ 9.93 (d, *J* = 1.5 Hz, 1H), 8.45 (s, 1H), 7.35 (t, J = 53.5 Hz, 1H), 7.11 (d, *J* = 1.5 Hz, 1H), 4.79 (d, *J* = 6.3 Hz, 2H), 4.32 (d, *J* = 6.6 Hz, 2H), 3.84 - 3.79 (m, 4H), 3.58 - 3.53 (m, 2H), 1.64 (s, 3H), 1.46 (d, *J* = 6.5 Hz, 7H). LC/MS (ESI) (m/z): 514 $[M+H]^+$. |

(continued)

| Example | Structure and name | Analysis data |
|---|---|---|
| **Example 183** | 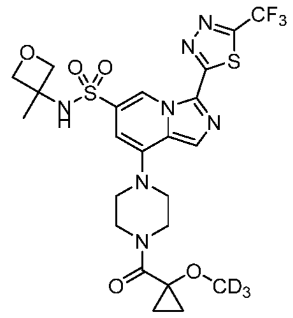 **8-(4-(1-(methoxy-d3)cyclopropane-1-carbonyl)piperazin-1-yl)-N-(3-methyloxetan-3-yl)-3-(5-(trifluoromethyl)-1,3,4-thiadiazol-2-yl)imidazo[1,5-a]pyridine-6-sulfonamide** | $^1H$ NMR (400 MHz, DMSO-$d_6$) δ 9.57 (s, 1H), 8.73 (s, 1H), 8.14 (s, 1H), 6.77 (s, 1H), 4.65 - 4.62 (m, 2H), 4.20 - 4.17 (m, 2H), 4.01 - 3.69 (m, 4H), 3.42 - 3.35 (m, 4H), 1.50 (s, 3H), 1.05 - 1.01 (m, 2H), 0.95 - 0.91 (m, 2H). LC/MS (ESI) (m/z): 605 $[M+H]^+$. |
| **Example 184** | 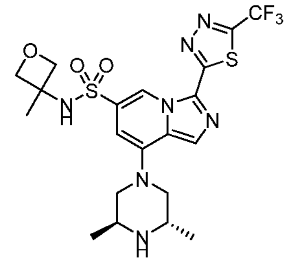 **8-((3S,5S)-3,5-dimethylpiperazin-1-yl)-N-(3-methyloxetan-3-yl)-3-(5-(trifluoromethyl)-1,3,4-thiadiazol-2-yl)imidazo[1,5-a]pyridine-6-sulfonamide** | $^1H$ NMR (400 MHz, $CD_3OD$) δ 9.85 (d, *J* = 7.3 Hz, 1H), 7.91 (d, *J* = 4.7 Hz, 1H), 6.86 (s, 1H), 4.82 - 4.76 (m, 2H), 4.37 - 4.29 (m, 2H), 4.03 - 3.94 (m, 2H), 3.65 - 3.58 (m, 2H), 3.41 - 3.35 (m, 2H), 1.65 (s, 3H), 1.59 - 1.55 (m, 6H). LC/MS (ESI) (m/z): 532 $[M+H]^+$. |
| **Example 185** | 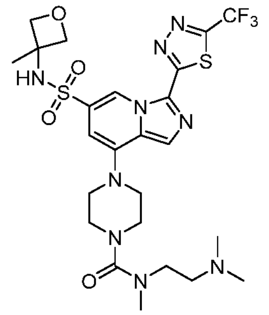 **N-(2-(dimethylamino)ethyl)-N-methyl-4-(6-(N-(3-methyloxetan-3-yl)sulfamoyl)-3-(5-(trifluoromethyl)-1,3,4-thiadiazol-2-yl)imidazo[1,5-a]pyridin-8-yl)piperazine-1-carboxamide** | $^1H$ NMR (400 MHz, $CD_3OD$) δ 9.81 (d, *J* = 4.7 Hz, 1H), 7.93 (d, *J* = 3.5 Hz, 1H), 6.78 (d, *J* = 1.7 Hz, 1H), 4.81 - 4.77 (m, 2H), 4.36 - 4.30 (m, 2H), 3.64 - 3.59 (m, 4H), 3.57 - 3.53 (m, 2H), 3.44 - 3.39 (m, 4H), 3.38 - 3.35 (m, 2H), 3.04 (s, 3H), 2.99 - 2.97 (m, 6H), 1.65 (s, 3H). LC/MS (ESI) (m/z): 632 $[M+H]^+$. |
| **Example 186** | 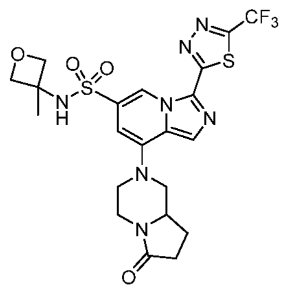 **N-(3-methyloxetan-3-yl)-8-(6-oxohexahydropyrrolo[1,2-a]pyrazin-2(1H)-yl)-3-(5-(trifluoromethyl)-1,3,4-thiadiazol-2-yl)imidazo[1,5-a]pyridine-6-sulfonamide** | $^1H$ NMR (400 MHz, DMSO-$d_6$) δ 9.57 (s, 1H), 8.76 (s, 1H), 8.13 (s, 1H), 6.79 (s, 1H), 4.63 (d, *J* = 5.6 Hz, 2H), 4.19 (d, *J* = 6.2 Hz, 2H), 4.01 - 3.96 (m, 1H), 3.91 - 3.85 (m, 2H), 3.81 - 3.77 (m, 1H), 3.14 - 3.12 (m, 1H), 2.82 - 2.80 (m, 1H), 2.71 - 2.69 (m, 1H), 2.34 - 2.30 (m, 2H), 2.24 - 2.20 (m, 1H), 1.70 - 1.64 (m, 1H), 1.50 (s, 3H). LC/MS (ESI) (m/z): 558 $[M+H]^+$. |

(continued)

| Example | Structure and name | Analysis data |
|---|---|---|
| **Example 187** | 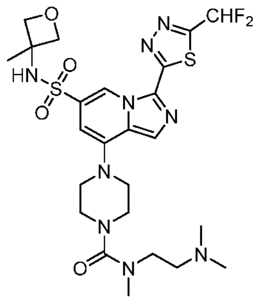<br>**4-(3-(5-(difluoromethyl)-1,3,4-thiadiazol-2-yl)-6-(N-(3-methyloxetan-3-yl)sulfamoyl)imidazo[1,5-a]pyridin-8-yl)-N-(2-(dimethylamino)ethyl)-N-methylpiperazine-1-carboxamide** | $^1$H NMR (400 MHz, $CD_3OD$) δ 9.84 (s, 1H), 7.90 (s, 1H), 7.26 (d, *J* = 53.5 Hz, 1H), 6.75 (s, 1H), 4.79 (d, *J* = 6.3 Hz, 2H), 4.32 (d, *J* = 6.6 Hz, 2H), 3.63 - 3.60 (m, 4H), 3.57 - 3.54 (m, 2H), 3.42 - 3.39 (m, 4H), 3.38 - 3.34 (m, 3H), 3.04 - 3.03 (m, 2H), 2.98 (s, 6H), 1.65 (s, 3H). LC/MS (ESI) (m/z): 614 $[M+H]^+$. |
| **Example 188** | 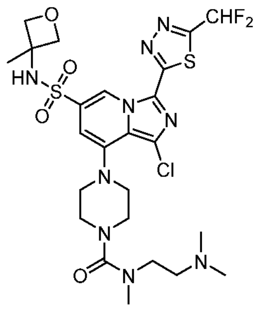<br>**4-(1-chloro-3-(5-(difluoromethyl)-1,3,4-thiadiazol-2-yl)-6-(N-(3-methyloxetan-3-yl)sulfamoyl)imidazo[1,5-a]pyridin-8-yl)-N-(2-(dimethylamino)ethyl)-N-methylpiperazine-1-carboxamide** | $^1$H NMR (400 MHz, DMSO-$d_6$) δ 9.61 (s, 1H), 8.77 (s, 1H), 7.76 (t, *J* = 53.2 Hz, 1H), 6.87 (s, 1H), 4.63 (d, *J* = 6.2 Hz, 2H), 4.19 (d, *J* = 6.5 Hz, 2H), 3.42 - 3.37 (m, 4H), 3.17 - 3.10 (m, 4H), 2.86 (s, 3H), 2.59 - 2.51 (m, 4H), 2.27 (s, 6H), 1.50 (s, 3H). LC/MS (ESI) (m/z): 648 $[M+H]^+$. |
| **Example 189** | 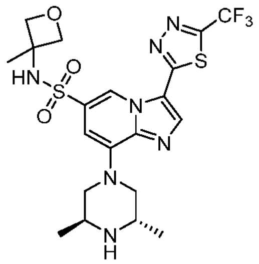<br>**8-((3S,5S)-3,5-dimethylpiperazin-1-yl)-N-(3-methyloxetan-3-yl)-3-(5-(trifluoromethyl)-1,3,4-thiadiazol-2-yl)imidazo[1,2-a]pyridine-6-sulfonamide** | $^1$H NMR (400 MHz, $CD_3OD$) δ 9.91 (d, *J* = 1.5 Hz, 1H), 8.50 (s, 1H), 7.14 (d, *J* = 1.5 Hz, 1H), 4.79 (d, *J* = 6.3 Hz, 2H), 4.32 (d, *J* = 6.7 Hz, 2H), 3.85 - 3.80 (m, 4H), 3.61 - 3.55 (m, 2H), 1.64 (s, 3H), 1.47 (d, *J* = 6.5 Hz, 6H). LC/MS (ESI) (m/z): 532 $[M+H]^+$. |
| **Example 190** | 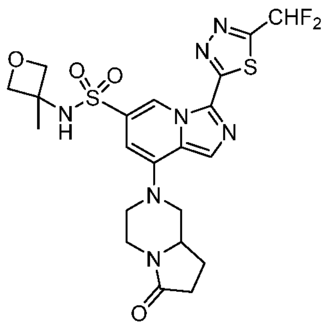<br>**3-(5-(difluoromethyl)-1,3,4-thiadiazol-2-yl)-N-(3-methyloxetan-3-yl)-8-(6-oxohexahydropyrrolo[1,2-a]pyrazin-2(1H)-yl)imidazo [1,5-a]pyridine-6-sulfonamide** | $^1$H NMR (400 MHz, DMSO-$d_6$) δ 9.60 (s, 1H), 8.73 (s, 1H), 8.09 (s, 1H), 7.68 (t, *J* = 53.2 Hz, 1H), 6.75 (s, 1H), 4.63 (d, *J* = 5.0 Hz, 2H), 4.19 (d, *J* = 6.3 Hz, 2H), 4.02 - 3.96 (m, 1H), 3.91 - 3.84 (m, 2H), 3.81 - 3.76 (m, 1 H), 3.17 - 310 (m, 1H), 2.85 - 2.77 (m, 1H), 2.71 - 2.65 (m, 1H), 2.38 - 2.29 (m, 2H), 2.25 - 2.18 (m, 1H), 1.70 - 1.62 (m, 1H), 1.50 (s, 3H). LC/MS (ESI) (m/z): 540 $[M+H]^+$. |

(continued)

| Example | Structure and name | Analysis data |
|---|---|---|
| **Example 191** | 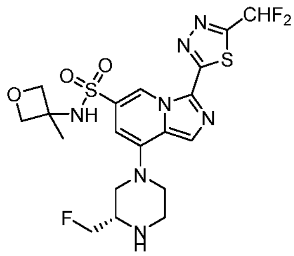 **(S)-3-(5-(difluoromethyl)-1,3,4-thiadiazol-2-yl)-8-(3-(fluoromethyl)piperazin-1-yl)-N-(3-methyloxetan-3-yl)imidazo[1,5-a]pyridine-6-sulfonamide** | $^1H$ NMR (400 MHz, $CD_3OD$) δ 9.83 (s, 1H), 7.90 (s, 1H), 7.47 - 7.17 (t, *J* = 53.6, 1H), 6.81 (s, 1H), 4.79 (d, *J* = 6.3 Hz, 2H), 4.74 - 4.58 (m, 2H), 4.31 (d, *J* = 6.3 Hz, 2H), 3.86 - 3.78 (m, 2H), 3.74 - 3.65 (m, 1H), 3.47 - 3.35 (m, 2H), 3.19 - 3.04 (m, 2H), 1.64 (s, 3H). LC/MS (ESI) (m/z): 518 $[M+H]^+$. |
| **Example 192** | 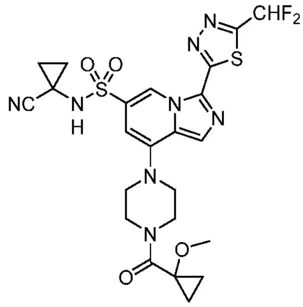 **N-(1-cyanocyclopropyl)-3-(5-(difluoromethyl)-1,3,4-thiadiazol-2-yl)-8-(4-(1-methoxycyclopropane-1-carbonyl)piperazin-1-yl)imidazo[1,5-a]pyridine-6-sulfonamide** | $^1H$ NMR (400 MHz, $CDCl_3$) δ 9.99 (s, 1H), 7.75 (s, 1H), 7.07 (t, *J* = 53.2 Hz, 1H), 6.70 (s, 1H), 5.91 (s, 1H), 4.13 - 3.90 (m, 4H), 3.50 - 3.23 (m, 4H), 3.36 (s, 3H), 1.73 - 1.68 (m, 2H), 1.54 - 1.49 (m, 2H), 1.18 - 1.12 (m, 2H), 1.02 - 0.96 (m, 2H). LC/MS (ESI) (m/z): 579 $[M+H]^+$. |
| **Example 193** | 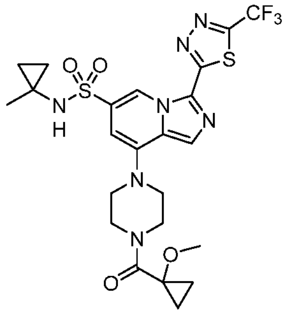 **8-(4-(1-methoxycyclopropane-1-carbonyl)piperazin-1-yl)-N-(1-methylcyclopropyl)-3-(5-(trifluoromethyl)-1,3,4-thiadiazol-2-yl)imidazo[1,5-a]pyridine-6-sulfonamide** | $^1H$ NMR (400 MHz, DMSO-$d_6$) δ 9.59 (s, 1H), 8.44 (s, 1H), 8.14 (s, 1H), 6.76 (s, 1H), 4.05 - 3.76 (m, 4H), 3.39 - 3.35 (m, 4H), 3.28 (s, 3H), 1.16 (s, 3H), 1.06 - 1.02 (m, 2H), 0.96 - 0.92 (m, 2H), 0.75 - 0.71 (m, 2H), 0.48 - 0.44 (m, 2H). LC/MS (ESI) (m/z): 586 $[M+H]^+$. |
| **Example 194** | 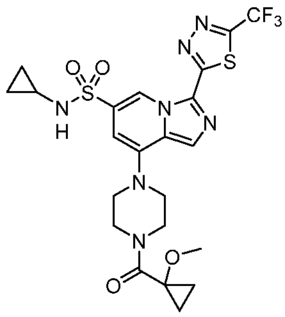 **N-cyclopropyl-8-(4-(1-methoxycyclopropane-1-carbonyl)piperazin-1-yl)-3-(5-(trifluoromethyl)-1,3,4-thiadiazol-2-yl)imidazo[1,5-a]pyridine-6-sulfonamide** | $^1H$ NMR (400 MHz, $CDCl_3$) δ 9.93 (s, 1H), 7.77 (s, 1H), 6.74 (s, 1H), 5.10 (s, 1H), 4.14 - 3.96 (m, 4H), 3.46 - 3.32 (m, 4H), 3.39 (s, 3H), 2.51 - 2.45 (m, 1H), 1.21 - 1.15 (m, 2H), 1.05 - 0.99 (m, 2H), 0.79 - 0.71 (m, 4H). LC/MS (ESI) (m/z): 572 $[M+H]^+$. |

(continued)

| Example | Structure and name | Analysis data |
|---|---|---|
| **Example 195** | 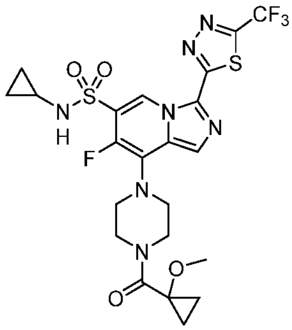 **N-cyclopropyl-7-fluoro-8-(4-(1-methoxycyclopropane-1-carbonyl)piperazin-1-yl)-3-(5-(trifluoromethyl)-1,3,4-thiadiazol-2-yl)imidazo[1,5-a]pyridine-6-sulfonamide** | $^{1}H$ NMR (400 MHz, DMSO-$d_6$) δ 9.65 (d, *J* = 5.1 Hz, 1H), 8.62 (d, *J* = 2.3 Hz, 1H), 8.05 (s, 1H), 3.97 - 3.70 (m, 4H), 3.44 - 3.40 (m, 4H), 2.46 - 2.41 (m, 1H), 1.05 - 1.00 (m, 2H), 0.95 - 0.91 (m, 2H), 0.57 - 0.52 (m, 4H). LC/MS (ESI) (m/z): 590 $[M+H]^+$. |
| **Example 196** | 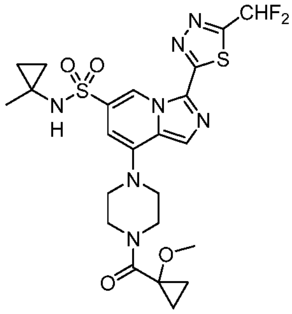 **3-(5-(difluoromethyl)-1,3,4-thiadiazol-2-yl)-8-(4-(1-methoxycyclopropane-1-carbonyl)piperazin-1-yl)-N-(1-methylcyclopropyl)imidazo[1,5-a]pyridine-6-sulfonamide** | $^{1}H$ NMR (400 MHz, DMSO-$d_6$) δ 9.61 (s, 1H), 8.42 (s, 1H), 8.09 (s, 1H), 7.68 (t, *J* = 53.2 Hz, 1H), 6.72 (s, 1H), 4.16 - 3.71 (m, 4H), 3.38 - 3.33 (m, 4H), 3.27 (s, 3H), 1.16 (s, 3H), 1.06 - 1.01 (m, 2H), 0.95 - 0.90 (m, 2H), 0.76 - 0.71 (m, 2H), 0.48 - 0.43 (m, 2H). LC/MS (ESI) (m/z): 568 $[M+H]^+$ |
| **Example 197** | 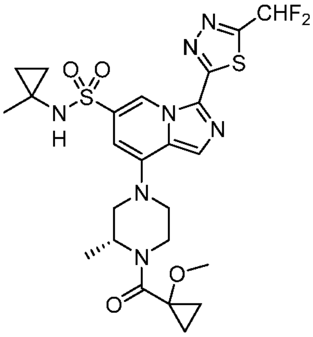 **(R)-3-(5-(difluoromethyl)-1,3,4-thiadiazol-2-yl)-8-(4-(1-methoxycyclopropane-1-carbonyl)-3-methylpiperazin-1-yl)-N-(1-methylcyclopropyl)imidazo[1,5-a]pyridine-6-sulfonamide** | $^{1}H$ NMR (400 MHz, DMSO-$d_6$) δ 9.60 (s, 1H), 8.42 (s, 1H), 7.97 (s, 1H), 7.68 (t, *J* = 53.2 Hz, 1H), 6.71 (s, 1H), 5.00 - 4.62 (m, 1H), 4.50 - 4.26 (m, 1H), 3.74 - 3.67 (m, 2H), 3.31 - 3.28 (m, 1H), 3.26 (s, 3H), 3.09 - 3.03 (m, 1H), 3.01 - 2.80 (m, 1H), 1.58 - 1.32 (m, 3H), 1.16 (s, 3H), 1.10 - 0.97 (m, 2H), 0.93 - 0.85 (m, 2H), 0.79 - 0.67 (m, 2H), 0.51 - 0.40 (m, 2H). LC/MS (ESI) (m/z): 582 $[M+H]^+$. |

(continued)

| Example | Structure and name | Analysis data |
|---|---|---|
| Example 198 | 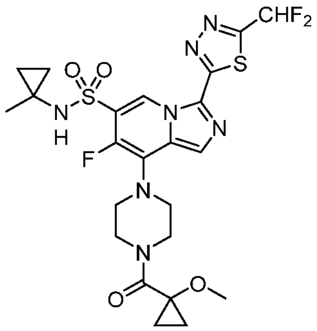 **3-(5-(difluoromethyl)-1,3,4-thiadiazol-2-yl)-7-fluoro-8-(4-(1-methoxycyclopropane-1-carbonyl)piperazin-1-yl)-N-(1-methylcyclopropyl)imidazo[1,5-a]pyridine-6-sulfonamide** | $^1$H NMR (400 MHz, $CD_3OD$) δ 9.89 (d, *J* = 5.1 Hz, 1H), 7.89 (s, 1H), 7.33 (t, *J* = 53.5 Hz, 1H), 4.12 - 3.79 (m, 4H), 3.50 - 3.45 (m, 4H), 3.37 (s, 3H), 1.32 (s, 3H), 1.12 - 1.08 (m, 2H), 1.02 - 0.98 (m, 2H), 0.91 - 0.87 (m, 2H), 0.56 - 0.52 (m, 2H). LC/MS (ESI) (m/z): 586 $[M+H]^+$. |
| Example 199 | 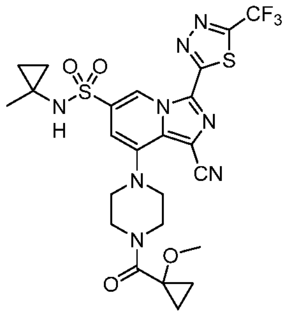 **1-cyano-8-(4-(1-methoxycyclopropane-1-carbonyl)piperazin-1-yl)-N-(1-methylcyclopropyl)-3-(5-(trifluoromethyl)-1,3,4-thiadiazol-2-yl)imidazo[1,5-a]pyridine-6-sulfonamide** | $^1$H NMR (400 MHz, DMSO-$d_6$) δ 9.72 (s, 1H), 8.61 (s, 1H), 7.25 (s, 1H), 4.11 - 3.76 (m, 4H), 3.28 (s, 3H), 3.26 - 3.20 (m, 4H), 1.16 (s, 3H), 1.08 - 1.02 (m, 2H), 0.96 - 0.90 (m, 2H), 0.77 - 0.71 (m, 2H), 0.51 - 0.44 (m, 2H). LC/MS (ESI) (m/z): 611 $[M+H]^+$. |
| Example 200 | 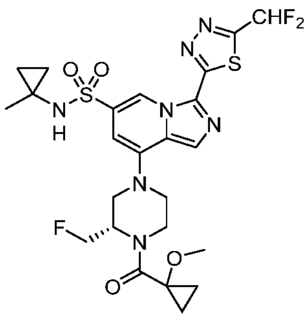 **(S)-3-(5-(difluoromethyl)-1,3,4-thiadiazol-2-yl)-8-(3-(fluoromethyl)-4-(1-methoxycyclopropane-1-carbonyl)piperazin-1-yl)-N-(1-methylcyclopropyl)imidazo[1,5-a]pyridine-6-sulfonamide** | $^1$H NMR (400 MHz, $CD_3OD$) δ 9.86 (s, 1H), 7.91 (s, 1H), 7.33 (t, *J* = 53.6 Hz, 1H), 6.78 (s, 1H), 5.37 - 4.95 (m, 2H), 4.79 - 4.42 (m, 2H), 3.99 (d, *J* = 13.2 Hz, 1H), 3.74 (d, *J* = 10.8 Hz, 1H), 3.37 (s, 3H), 3.35 - 3.32 (m, 1H), 3.15 - 2.97 (m, 2H), 1.26 (s, 3H), 1.16 - 1.09 (m, 2H), 1.07 - 0.96 (m, 2H), 0.86 - 0.79 (m, 2H), 0.57 - 0.46 (m, 2H). LC/MS (ESI) (m/z): 600 $[M+H]^+$ |

(continued)

| Example | Structure and name | Analysis data |
|---|---|---|
| Example 201 | 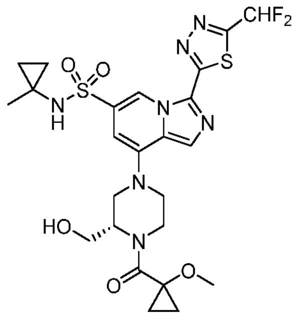 (S)-3-(5-(difluoromethyl)-1,3,4-thiadiazol-2-yl)-8-(3-(hydroxymethyl)-4-(1-methoxycyclopropane-1-carbonyl)piperazin-1-yl)-N-(1-methylcyclopropyl)imidazo [1,5-a]pyridine-6-sulfonamide | $^1$H NMR (400 MHz, $CD_3OD$) δ 9.83 (s, 1H), 8.20 (s, 1H), 7.33 (t, *J* = 53.6 Hz, 1H), 6.75 (s, 1H), 4.72 - 4.58 (m, 2H), 4.25 - 4.12 (m, 2H), 3.90 - 3.74 (m, 1H), 3.71 - 3.60 (m, 2H), 3.37 (s, 3H), 3.04 - 2.92 (m, 2H), 1.26 (s, 3H), 1.13 - 1.09 (m, 2H), 1.04 - 0.98 (m, 2H), 0.85 - 0.81 (m, 2H), 0.52 - 0.48 (m, 2H). LC/MS (ESI) (m/z): 598 $[M+H]^+$. |
| Example 202 | 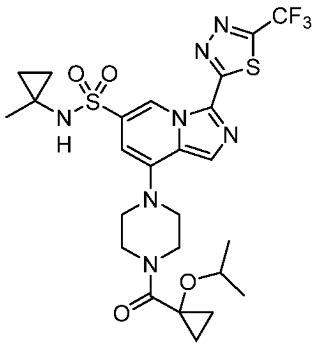 8-(4-(1-isopropoxycyclopropane-1-carbonyl)piperazin-1-yl)-N-(1-methylcyclopropyl)-3-(5-(trifluoromethyl)-1,3,4-thiadiazol-2-yl)imidazo[1,5-a]pyridine-6-sulfonamide | $^1$H NMR (400 MHz, DMSO-$d_6$) δ 9.59 (s, 1H), 8.45 (s, 1H), 8.14 (s, 1H), 6.75 (s, 1H), 4.06 - 4.01 (m, 1H), 3.81 - 3.74 (m, 2H), 3.43 - 3.33 (m, 6H), 1.16 (s, 3H), 1.12 (d, *J* = 6.2 Hz, 6H), 1.05 - 0.99 (m, 2H), 0.97 - 0.89 (m, 2H), 0.75 - 0.72 (m, 2H), 0.48 - 0.44 (m, 2H). LC/MS (ESI) (m/z): 614 $[M+H]^+$. |
| Example 203 | 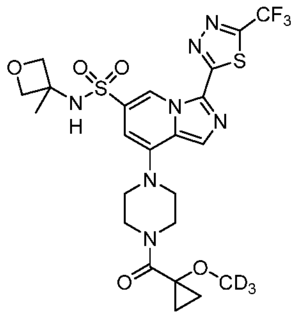 8-(4-(1-(methoxy-d3)cyclopropane-1-carbonyl)piperazin-1-yl)-N-(3-methyloxetan-3-yl)-3-(5-(trifluoromethyl)-1,3,4-thiadiazol-2-yl)imidazo[1,5-a]pyridine-6-sulfonamide | $^1$H NMR (400 MHz, DMSO-$d_6$) δ 9.57 (s, 1H), 8.73 (s, 1H), 8.14 (s, 1H), 6.77 (s, 1H), 4.65 - 4.62 (m, 2H), 4.20 - 4.17 (m, 2H), 4.01 - 3.69 (m, 4H), 3.42 - 3.35 (m, 4H), 1.50 (s, 3H), 1.05 - 1.01 (m, 2H), 0.95 - 0.91 (m, 2H). LC/MS (ESI) (m/z): 605 $[M+H]^+$. |

(continued)

| Example | Structure and name | Analysis data |
|---|---|---|
| **Example 204** | 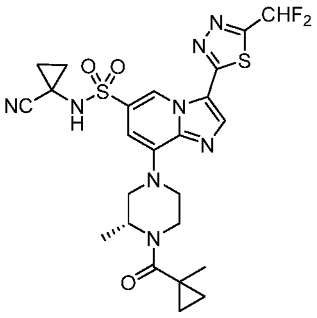 **(R)-N-(1-cyanocyclopropyl)-3-(5-(difluoromethyl)-1,3,4-thiadiazol-2-yl)-8-(3-methyl-4-(1-methylcyclopropane-1-carbonyl)piperazin-1-yl)imidazo[1,2-a]pyridine-6-sulfonamide** | $^1$H NMR (400 MHz, DMSO-$d_6$) δ 9.74 (s, 1H), 9.43 (s, 1H), 8.66 (s, 1H), 7.70 (t, *J* = 53.0 Hz, 1H), 6.99 (s, 1H), 4.75 - 4.61 (m, 1H), 4.46 - 4.35 (m, 1H), 4.34 - 4.17 (m, 2H), 3.74 - 3.57 (m, 1H), 3.13 - 3.03 (m, 1H), 3.00 - 2.87 (m, 1H), 1.52 - 1.43 (m, 2H), 1.42 - 1.36 (m, 2H), 1.32 (s, 3H), 1.27 (s, 3H), 0.90 - 0.78 (m, 2H), 0.63 - 0.53 (m, 2H). LC/MS (ESI) (m/z): 577 [M+H]$^+$. |
| **Example 205** | 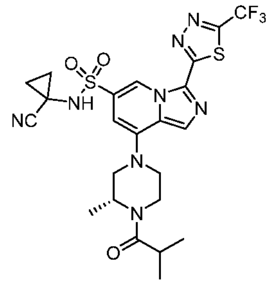 **(R)-N-(1-cyanocyclopropyl)-8-(4-isobutyryl-3-methylpiperazin-1-yl)-3-(5-(trifluoromethyl)-1,3,4-thiadiazol-2-yl)imidazo[1,5-a]pyridine-6-sulfonamide** | $^1$H NMR (400 MHz, DMSO-$d_6$) δ 9.65 (s, 1H), 9.49 (s, 1H), 8.04 (s, 1H), 6.69 (s, 1H), 4.78 - 4.40 (m, 1H), 3.99 - 3.65 (m, 3H), 3.12 - 2.85 (m, 4H), 1.50 - 1.47 (m, 2H), 1.40 - 1.37 (m, 2H), 1.32 - 1.21 (m, 3H), 1.06 - 1.01 (m, 6H). LC/MS (ESI) (m/z): 583 [M+H]$^+$. |
| **Example 206** | 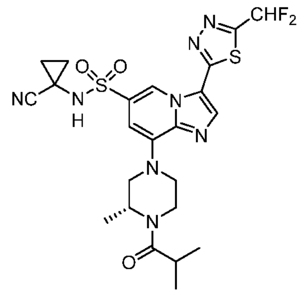 **(R)-N-(1-cyanocyclopropyl)-3-(5-(difluoromethyl)-1,3,4-thiadiazol-2-yl)-8-(4-isobutyryl-3-methylpiperazin-1-yl)imidazo[1,2-a]pyridine-6-sulfonamide** | $^1$H NMR (400 MHz, DMSO-$d_6$) δ 9.74 (d, *J* = 1.5 Hz, 1H), 9.45 (s, 1H), 8.67 (s, 1H), 7.71 (t, J= 53.2 Hz, 1H), 6.98 (s, 1H), 4.92 - 4.06 (m, 4H), 4.05 - 3.53 (m, 1H), 3.15 - 3.10 (m, 1H), 3.02 - 2.85 (m, 2H), 1.53 - 1.46 (m, 2H), 1.42 - 1.35 (m, 3H), 1.30 - 1.18 (m, 2H), 1.06 (d, *J* = 6.6 Hz, 6H). LC/MS (ESI) (m/z): 565 [M+H]$^+$. |
| **Example 207** | 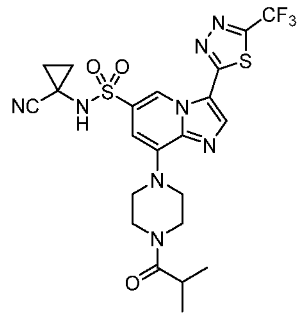 **N-(1-cyanocyclopropyl)-8-(4-isobutyrylpiperazin-1-yl)-3-(5-(trifluoromethyl)-1,3,4-thiadiazol-2-yl)imidazo[1,2-a]pyridine-6-sulfonamide** | $^1$H NMR (400 MHz, DMSO-$d_6$) δ 9.73 (s, 1H), 9.48 (s, 1H), 8.72 (s, 1H), 7.02 (s, 1H), 3.79 - 3.59 (m, 8H), 3.00 - 2.92 (m, 1H), 1.49 - 1.42 (m, 2H), 1.41 - 1.34 (m, 2H), 1.04 (d, *J* = 6.1 Hz, 6H). LC/MS (ESI) (m/z): 569 [M+H]$^+$. |

(continued)

| Example | Structure and name | Analysis data |
|---|---|---|
| **Example 208** | 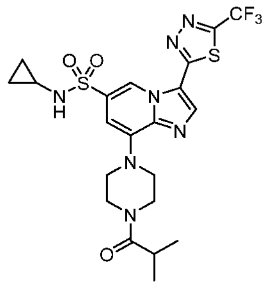 **N-cyclopropyl-8-(4-isobutyrylpiperazin-1-yl)-3-(5-(trifluoromethyl)-1,3,4-thiadiazol-2-yl)imidazo[1,2-a]pyridine-6-sulfonamide** | $^1$H NMR (400 MHz, DMSO-$d_6$) δ 9.66 (d, *J* = 1.4 Hz, 1H), 8.70 (s, 1H), 8.25 (s, 1H), 7.04 (d, *J* = 1.3 Hz, 1H), 3.81 - 3.59 (m, 8H), 3.02 - 2.93 (m, 1H), 2.28 - 2.21 (m, 1H), 1.05 (d, *J* = 6.7 Hz, 6H), 0.55 - 0.50 (m, 2H), 0.50 - 0.45 (m, 2H). LC/MS (ESI) (m/z): 544 $[M+H]^+$. |
| **Example 209** | 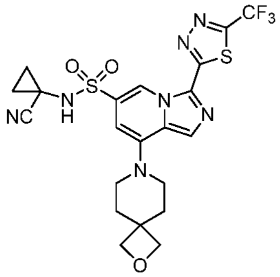 **N-(1-cyanocyclopropyl)-8-(2-oxa-7-azaspiro[3.5]nonan-7-yl)-3-(5-(trifluoromethyl)-1,3,4-thiadiazol-2-yl)imidazo[1,5-a]pyridine-6-sulfonamide** | $^1$H NMR (400 MHz, DMSO-$d_6$) δ 962 (s, 1H), 9.47 (s, 1H), 8.04 (s, 1H), 6.70 (d, *J* = 0.9 Hz, 1H), 4.41 (s, 4H), 3.29 - 3.25 (m, 4H), 2.07 - 1.99 (m, 4H), 1.51 - 1.45 (m, 2H), 1.44 - 1.37 (m, 2H). LC/MS (ESI) (m/z): 540 $[M+H]^+$. |
| **Example 210** | 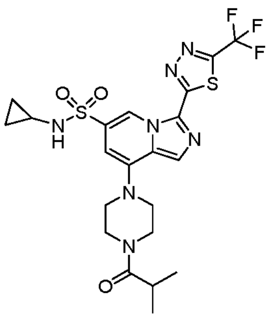 **N-cyclopropyl-8-(4-isobutyrylpiperazin-1-yl)-3-(5-(trifluoromethyl)-1,3,4-thiadiazol-2-yl)imidazo[1,5-a]pyridine-6-sulfonamide** | $^1$H NMR (400 MHz, DMSO-$d_6$) δ 9.59 (s, 1H), 8.28 (s, 1H), 8.13 (s, 1H), 6.73 (s, 1H), 3.82 - 3.71 (m, 4H), 3.42 - 3.34 (m, 4H), 2.97 - 2.90 (m, 1H), 2.29 - 2.22 (m, 1H), 1.05 (d, *J* = 6.3 Hz, 6H), 0.54 - 0.45 (m, 4H). LC/MS (ESI) (m/z): 544 $[M+H]^+$. |
| **Example 211** | 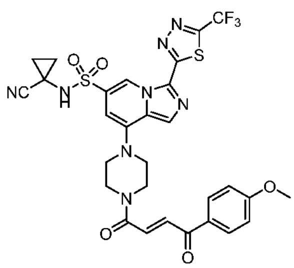 **(E)-N-(1-cyanocyclopropyl)-8-(4-(4-(4-methoxyphenyl)-4-oxobut-2-enoyl)piperazin-1-yl)-3-(5-(trifluoromethyl)-1,3,4-thiadiazol-2-yl)imidazo[1,5-a]pyridine-6-sulfonamide** | $^1$H NMR (400 MHz, DMSO-$d_6$) δ 9.66 (s, 1H), 9.50 (s, 1H), 8.16 (s, 1H), 8.07 (d, *J* = 8.6 Hz, 2H), 7.84 (d, *J* = 15.1 Hz, 1H), 7.49 (d, *J* = 15.2 Hz, 1H), 7.11 (d, *J* = 8.8 Hz, 2H), 6.71 (s, 1H), 3.92 - 3.85 (m, 7H), 3.45 - 3.40 (m, 4H), 1.49 - 1.44 (m, 2H), 1.41 - 1.35 (m, 2H). LC/MS (ESI) (m/z): 687 $[M+H]^+$. |

(continued)

| Example | Structure and name | Analysis data |
|---|---|---|
| **Example 212** | 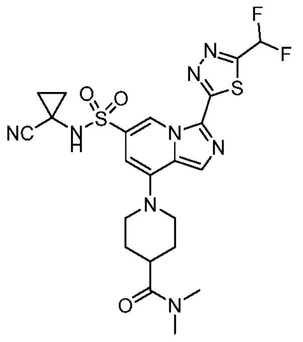 **1-(6-(N-(1-cyanocyclopropyl)sulfamoyl)-3-(5-(difluoromethyl)-1,3,4-thiadiazol-2-yl)imidazo[1,5-a]pyridin-8-yl)-N,N-dimethylpiperidine-4-carboxamide** | $^1$H NMR (400 MHz, DMSO-$d_6$) δ 9.65 (s, 1H), 9.47 (s, 1H), 8.02 (s, 1H), 7.61 (t, *J* = 53.1 Hz, 1H), 6.66 (s, 1H), 3.86 - 3.78 (m, 2H), 3.09 (s, 3H), 3.05 - 2.98 (m, 2H), 2.95 - 2.88 (m, 1H), 2.85 (s, 3H), 1.87 - 1.76 (m, 4H), 1.51 - 1.46 (m, 2H), 1.41 - 1.35 (m, 2H). LC/MS (ESI) (m/z): 551 [M+H]$^+$. |
| **Example 213** | 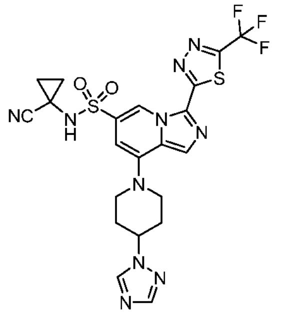 **8-(4-(1H-1,2,4-triazol-1-yl)piperidin-1-yl)-N-(1-cyanocyclopropyl)-3-(5-(trifluoromethyl)-1,3,4-thiadiazol-2-yl)imidazo[1,5-a]pyridine-6-sulfonamide** | $^1$H NMR (400 MHz, DMSO-$d_6$) δ 9.65 (s, 1H), 864 (s, 1H), 8.11 (s, 1H), 8.02 (s, 1H), 6.76 (s, 1H), 4.69 - 4.60 (m, 1H), 3.93 - 3.86 (m, 2H), 3.20 - 3.13 (m, 2H), 2.30 - 2.22 (m, 4H), 1.52 - 1.46 (m, 2H), 1.42 - 1.36 (m, 2H). LC/MS (ESI) (m/z): 565 [M+H]$^+$. |
| **Example 214** | 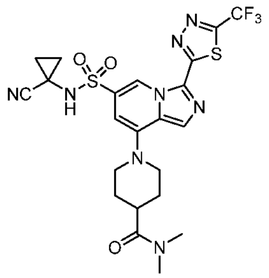 **1-(6-(N-(1-cyanocyclopropyl)sulfamoyl)-3-(5-(trifluoromethyl)-1,3,4-thiadiazol-2-yl)imidazo[1,5-a]pyridin-8-yl)-N,N-dimethylpiperidine-4-carboxamide** | $^1$H NMR (400 MHz, DMSO-$d_6$) δ 9.63 (s, 1H), 9.50 (s, 1H), 8.07 (s, 1H), 6.69 (s, 1H), 3.85 - 3.78 (m, 2H), 3.09 (s, 3H), 3.04 - 3.00 (m, 2H), 2.96 - 2.91 (m, 1H), 2.85 (s, 3H), 1.88 - 1.81 (m, 4H), 1.51 - 1.45 (m, 2H), 1.41 - 1.36 (m, 2H). LC/MS (ESI) (m/z): 569 [M+H]$^+$. |
| **Example 215** | 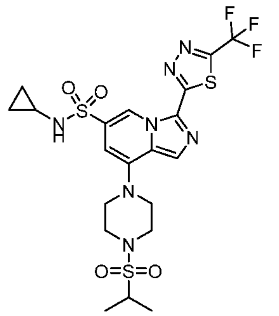 **N-cyclopropyl-8-(4-(isopropylsulfonyl)piperazin-1-yl)-3-(5-(trifluoromethyl)-1,3,4-thiadiazol-2-yl)imidazo[1,5-a]pyridine-6-sulfonamide** | $^1$H NMR (400 MHz, DMSO-$d_6$) δ 9.61 (s, 1H), 8.29 (d, *J* = 2.2 Hz, 1H), 8.11 (s, 1H), 6.77 (s, 1H), 3.57 - 3.52 (m, 4H), 3.47 - 3.42 (m, 1H), 3.40 - 3.36 (m, 4H), 2.30 - 2.24 (m, 1H), 1.29 (d, *J* = 6.8 Hz, 6H), 0.56 - 0.52 (m, 2H), 0.51 - 0.46 (m, 2H). LC/MS (ESI) (m/z): 580 [M+H]$^+$. |

(continued)

| Example | Structure and name | Analysis data |
|---|---|---|
| **Example 216** | 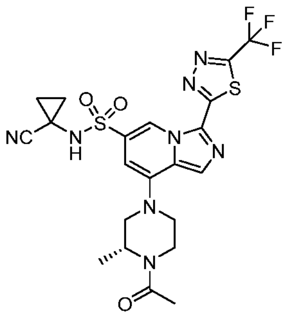 **(R)-8-(4-acetyl-3-methylpiperazin-1-yl)-N-(1-cyanocyclopropyl)-3-(5-(trifluoromethyl)-1,3,4-thiadiazol-2-yl)imidazo[1,5-a] pyridine-6-sulfonamide** | $^1$H NMR (400 MHz, DMSO-$d_6$) δ 9.64 (s, 1H), 8.03 (s, 1H), 6.69 (s, 1H), 4.37 - 4.26 (m, 1H), 3.84 - 3.73 (m, 3H), 3.16 - 3.02 (m, 3H), 2.08 (d, *J* = 11.6 Hz, 3H), 1.48 - 1.42 (m, 3H), 1.38 - 1.33 (m, 2H), 1.32 - 1.26 (m, 2H). LC/MS (ESI) (m/z): 555 $[M+H]^+$. |
| **Example 217** | 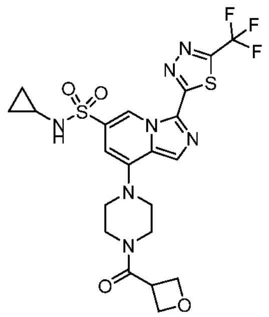 **N-cyclopropyl-8-(4-(oxetane-3-carbonyl)piperazin-1-yl)-3-(5-(trifluoromethyl)-1,3,4-thiadiazol-2-yl)imidazo[1,5-a]pyridine-6-sulfonamide** | $^1$H NMR (400 MHz, DMSO-$d_6$) δ 9.59 (s, 1H), 8.29 (s, 1H), 8.12 (s, 1H), 6.72 (s, 1H), 4.79 - 4.65 (m, 4H), 4.29 - 4.15 (m, 1H), 3.83 - 3.74 (m, 2H), 3.47 - 3.43 (m, 2H), 3.33 - 3.22 (m, 4H), 2.29 - 2.23 (m, 1H), 0.57 - 0.51 (m, 2H), 0.50 - 0.43 (m, 2H). LC/MS (ESI) (m/z): 558 [M+H] |
| **Example 218** | 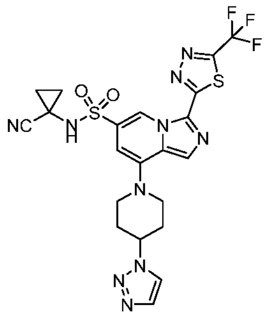 **8-(4-(1H-1,2,3-triazol-1-yl)piperidin-1-yl)-N-(1-cyanocyclopropyl)-3-(5-(trifluoromethyl)-1,3,4-thiadiazol-2-yl)imidazo [1,5-a] pyridine-6-sulfonamide** | $^1$H NMR (400 MHz, DMSO-$d_6$) δ 9.66 (s, 1H), 9.53 (s, 1H), 8.29 (s, 1H), 8.12 (s, 1H), 7.79 (s, 1H), 6.78 (s, 1H), 4.97 - 4.82 (m, 1H), 3.93 (d, *J* = 12.6 Hz, 2H), 3.26 - 3.17 (m, 2H), 2.38 - 2.26 (m, 4H), 1.52 - 1.38 (m, 4H). LC/MS (ESI) (m/z): 565 $[M+H]^+$. |
| **Example 219** | 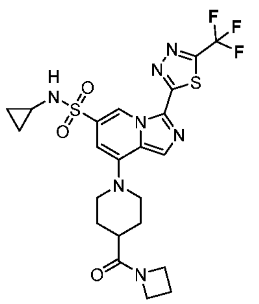 **8-(4-(azetidine-1-carbonyl)piperidin-1-yl)-N-cyclopropyl-3-(5-(trifluoromethyl)-1,3,4-thiadiazol-2-yl)imidazo[1,5-a]pyridine-6-sulfonamide** | $^1$H NMR (400 MHz, DMSO-$d_6$) δ 9.55 (s, 1H), 8.28 (d, *J* = 2.5 Hz, 1H), 8.04 (s, 1H), 6.71 (d, *J* = 0.9 Hz, 1H), 4.22 (t, *J* = 7.6 Hz, 2H), 3.86 (t, *J* = 7.7 Hz, 2H), 3.81 - 3.76 (m, 2H), 3.02 - 2.94 (m, 2H), 2.27 - 2.20 (m, 3H), 1.84 - 1.77 (m, 4H), 0.89 - 0.81 (m, 1H), 0.54 - 0.47 (m, 4H)。LC/MS (ESI) (m/z): 556 $[M+H]^+$ |

(continued)

| Example | Structure and name | Analysis data |
|---|---|---|
| **Example 220** | 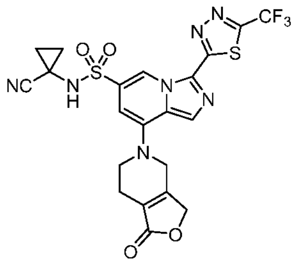 **N-(1-cyanocyclopropyl)-8-(1-oxo-1,4,6,7-tetrahydrofuro[3,4-c] pyridin-5(3H)-yl)-3-(5-(trifluoromethyl)-1,3,4-thiadiazol-2-yl)imidazo[1,5-a]pyridine-6-sulfonamide** | $^1$H NMR (400 MHz, DMSO-$d_6$) δ 9.64 (s, 1H), 8.15 (s, 1H), 6.75 (s, 1H), 5.00 (s, 2H), 4.32 (s, 2H), 3.71 - 3.64 (m, 2H), 2.57 - 2.53 (m, 2H), 1.48 - 1.43 (m, 2H), 1.40 - 1.34 (m, 2H). LC/MS (ESI) (m/z): 552 $[M+H]^+$. |
| **Example 221** | 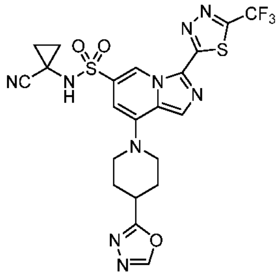 **8-(4-(1,3,4-oxadiazol-2-yl)piperidin-1-yl)-N-(1-cyanocyclopropyl)-3-(5-(trifluoromethyl)-1,3,4-thiadiazol-2-yl)imidazo[1,5-a] pyridine-6-sulfonamide** | $^1$H NMR (400 MHz, DMSO-$d_6$) δ 9.64 (s, 1H), 9.50 (s, 1H), 9.21 (s, 1H), 8.10 (s, 1H), 6.74 (s, 1H), 3.85 - 3.75 (m, 2H), 3.40 - 3.34 (m, 1H), 3.20 - 3.12 (m, 2H), 2.27 - 2.21 (m, 2H), 2.09 - 2.01 (m, 2H), 1.51 - 1.45 (m, 2H), 1.42 - 1.36 (m, 2H). LC/MS (ESI) (m/z): 566 $[M+H]^+$. |
| **Example 222** | 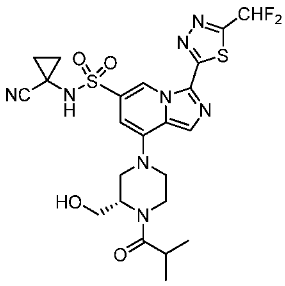 **(S)-N-(1-cyanocyclopropyl)-3-(5-(difluoromethyl)-1,3,4-thiadiazol-2-yl)-8-(3-(hydroxymethyl)-4-isobutyrylpiperazin-1-yl)imidazo[1,5-a]pyridine-6-sulfonamide** | $^1$H NMR (400 MHz, DMSO-$d_6$) δ 9.68 (s, 1H), 9.48 (s, 1H), 8.30 - 8.10 (m, 1H), 7.68 (t, *J* = 53.2 Hz, 1H), 6.69 (s, 1H), 5.36 - 5.18 (m, 1H), 4.58 - 4.36 (m, 1H), 4.19 - 4.10 (m, 1H), 4.04 - 3.87 (m, 2H), 3.61 - 3.55 (m, 2H), 3.05 - 2.82 (m, 4H), 1.53 - 1.44 (m, 2H), 1.40 - 1.32 (m, 2H), 1.10 - 1.00 (m, 6H), LC/MS (ESI) (m/z): 581 $[M+H]^+$ . |
| **Example 223** | 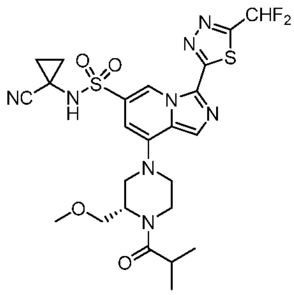 **(S)-N-(1-cyanocyclopropyl)-3-(5-(difluoromethyl)-1,3,4-thiadiazol-2-yl)-8-(4-isobutyryl-3-(methoxymethyl)piperazin-1-yl)imidazo[1,5-a]pyridine-6-sulfonamide** | $^1$H NMR (400 MHz, DMSO-$d_6$) δ 9.72 (s, 1H), 9.52 (s, 1H), 8.05 (s, 1H), 7.72 (t, *J* = 53.2 Hz, 1H), 6.73 (s, 1H), 4.80 - 4.40 (m, 1H), 4.08 - 4.00 (m, 1H), 3.95 - 3.90 (m, 1H), 3.69 - 3.52 (m, 2H), 3.42 (s, 3H), 3.31 - 2.95 (m, 4H), 2.94 - 2.88 (m, 1H), 1.54 - 1.49 (m, 2H), 1.45 - 1.38 (m, 2H), 1.11 - 1.05 (m, 6H), LC/MS (ESI) (m/z): 595 $[M+H]^+$ . |

(continued)

| Example | Structure and name | Analysis data |
|---|---|---|
| **Example 224** | 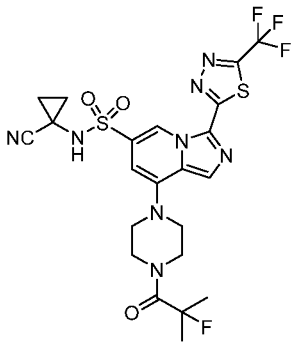 **N-(1-cyanocyclopropyl)-8-(4-(2-fluoro-2-methylpropanoyl)piperazin-1-yl)-3-(5-(trifluoromethyl)-1,3,4-thiadiazol-2-yl)imidazo[1,5-a]pyridine-6-sulfonamide** | $^1$H NMR (400 MHz, DMSO-$d_6$) δ 9.67 (s, 1H), 8.16 (s, 1H), 6.72 (s, 1H), 4.02 - 3.77 (m, 4H), 3.50 - 3.42 (m, 4H), 1.63 (s, 3H), 1.58 (s, 3H), 1.50 - 1.46 (m, 2H), 1.41 - 1.37 (m, 2H). LC/MS (ESI) (m/z): 587 $[M+H]^+$. |
| **Example 225** | 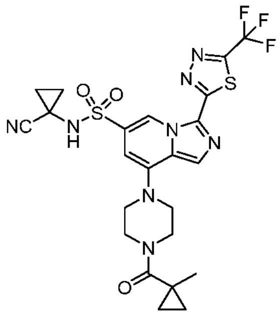 **N-(1-cyanocyclopropyl)-8-(4-(1-methylcyclopropane-1-carbonyl)piperazin-1-yl)-3-(5-(trifluoromethyl)-1,3,4-thiadiazol-2-yl)imidazo[1,5-a]pyridine-6-sulfonamide** | $^1$H NMR (400 MHz, DMSO-$d_6$) δ 9.67 (s, 1H), 9.50 (s, 1H), 8.15 (s, 1H), 6.73 (s, 1H), 3.90 - 3.71 (m, 4H), 3.42 - 3.37 (m, 4H), 1.52 - 1.46 (m, 2H), 1.42 - 1.34 (m, 2H), 1.29 (s, 3H), 0.88 - 0.84 (m, 2H), 0.62 - 0.53 (m, 2H). LC/MS (ESI) (m/z): 581 $[M+H]^+$. |
| **Example 226** | 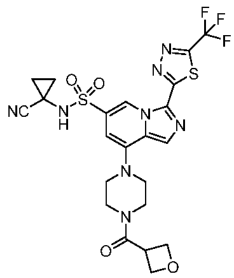 **N-(1-cyanocyclopropyl)-8-(4-(oxetane-3-carbonyl)piperazin-1-yl)-3-(5-(trifluoromethyl)-1,3,4-thiadiazol-2-yl)imidazo[1,5-a]pyridine-6-sulfonamide** | $^1$H NMR (400 MHz, DMSO) δ 9.66 (s, 1H), 9.51 (s, 1H), 8.13 (s, 1H), 6.70 (s, 1H), 4.80 - 4.67 (m, 4H), 4.26 - 4.15 (m, 1H), 3.80 - 3.71 (m, 2H), 3.48 - 3.43 (m, 2H), 3.39 - 3.35 (m, 2H), 3.32 - 3.26 (m, 2H), 1.51 - 1.44 (m, 2H), 1.43 - 1.36 (m, 2H). LC/MS (ESI) (m/z): 583 $[M+H]^+$. |
| **Example 227** | 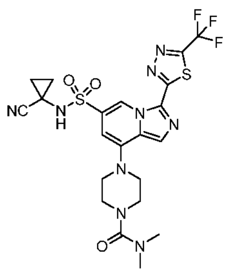 **4-(6-(N-(1-cyanocyclopropyl)sulfamoyl)-3-(5-(trifluoromethyl)-1,3,4-thiadiazol-2-yl)imidazo[1,5-a]pyridin-8-yl)-N,N-dimethylpiperazine-1-carboxamide** | $^1$H NMR (400 MHz, DMSO-$d_6$) δ 9.65 (s, 1H), 9.48 (s, 1H), 8.12 (d, *J*= 1.8 Hz, 1H), 6.72 (s, 1H), 3.39 - 3.37 (m, 4H), 3.33 - 3.18 (m, 4H), 2.80 (s, 6H), 1.52 - 1.45 (m, 2H), 1.42 - 1.36 (m, 2H). LC/MS (ESI) (m/z): 570 $[M+H]^+$. |

(continued)

| Example | Structure and name | Analysis data |
|---|---|---|
| **Example 228** | 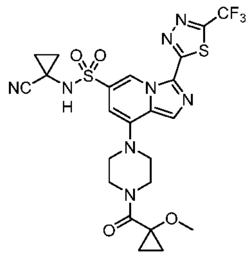 **N-(1-cyanocyclopropyl)-8-(4-(1-methoxycyclopropane-1-carbonyl)piperazin-1-yl)-3-(5-(trifluoromethyl)-1,3,4-thiadiazol-2-yl) imidazo[1,5-a]pyridine-6-sulfonamide** | $^1H$ NMR (400 MHz, DMSO-$d_6$) δ 9.67 (s, 1H), 8.17 (s, 1H), 6.74 (s, 1H), 4.05 - 3.73 (m, 4H), 3.41 3.39 (m, 4H), 3.27 (s, 3H), 1.50 - 1.45 (m, 2H), 1.41 - 1.36 (m, 2H), 1.06 - 1.01 (m, 2H), 0.96 - 0.91 (m, 2H). LC/MS (ESI) (m/z): 597 $[M+H]^+$. |
| **Example 229** | 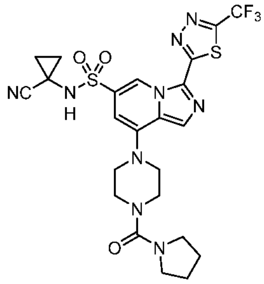 **N-(1-cyanocyclopropyl)-8-(4-(pyrrolidine-1-carbonyl)piperazin-1-yl)-3-(5-(trifluoromethyl)-1,3,4-thiadiazol-2-yl)imidazo[1,5-a]pyridine-6-sulfonamide** | $^1H$ NMR (400 MHz, DMSO-$d_6$) δ 9.65 (s, 1H), 9.54 (s, 1H), 8.14 (s, 1H), 6.71 (s, 1H), 3.47 - 3.42 (m, 4H), 3.37 - 3.33 (m, 8H), 1.81 - 1.75 (m, 4H), 1.50 - 1.45 (m, 2H), 1.41 - 1.36 (m, 2H). LC/MS (ESI) (m/z): 596 $[M+H]^+$. |
| **Example 230** | 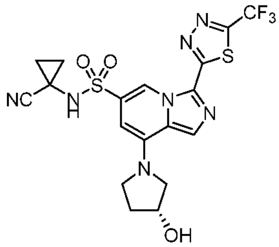 **(R)-N-(1-cyanocyclopropyl)-8-(3-hydroxypyrrolidin-1-yl)-3-(5-(trifluoromethyl)-1,3,4-thiadiazol-2-yl)imidazo[1,5-a]pyridine-6-sulfonamide** | $^1H$ NMR (400 MHz, DMSO-$d_6$) δ 9.41 (s, 1H), 8.26 (s, 1H), 6.14 (s, 1H), 5.24 - 5.06 (m, 1H), 4.50 (s, 1H), 3.92 - 3.67 (m, 3H), 3.55 - 3.50 (m, 1H), 2.15 - 2.08 (m, 1H), 2.05 - 2.00 (m, 1H), 1.49 - 1.43 (m, 2H), 1.39 - 1.34 (m, 2H). LC/MS (ESI) (m/z): 500 $[M+H]^+$ |
| **Example 231** | 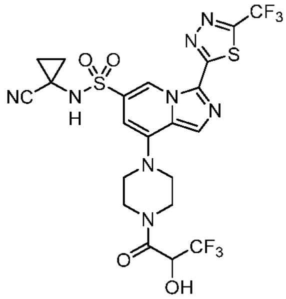 **N-(1-cyanocyclopropyl)-8-(4-(3,3,3-trifluoro-2-hydroxypropanoyl)piperazin-1-yl)-3-(5-(trifluoromethyl)-1,3,4-thiadiazol-2-yl) imidazo[1,5-a]pyridine-6-sulfonamide** | $^1H$ NMR (400 MHz, DMSO-$d_6$) δ 9.64 (s, 1H), 8.15 (s, 1H), 6.86 (d, $J$ = 8.6 Hz, 1H), 6.70 (s, 1H), 5.23 - 5.14 (m, 1H), 3.88 - 3.73 (m, 4H), 3.40 - 3.37 (m, 4H), 1.48 - 1.43 (m, 2H), 1.39 - 1.34 (m, 2H). LC/MS (ESI) (m/z): 625 $[M+H]^+$. |

(continued)

| Example | Structure and name | Analysis data |
|---|---|---|
| **Example 232** | 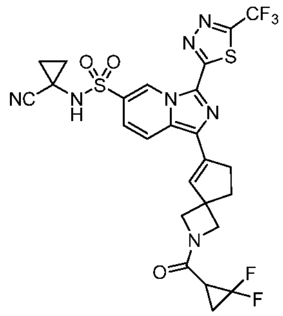 **N-(1-cyanocyclopropyl)-1-(2-(2,2-difluorocyclopropane-1-carbonyl)-2-azaspiro[3.4]oct-5-en-6-yl)-3-(5-(trifluoromethyl)-1,3,4-thiadiazol-2-yl)imidazo[1,5-a]pyridine-6-sulfonamide** | $^1$H NMR (400 MHz, DMSO-$d_6$) δ 9.97 (s, 1H), 8.45 - 8.38 (m, 1H), 7.48 - 7.44 (m, 1H), 6.74 (d, *J* = 3.2 Hz, 1H), 4.44 - 4.26 (m, 2H), 4.09 - 4.05 (m, 1H), 3.99 - 3.95 (m, 1H), 3.01 - 2.97 (m, 2H), 2.36 - 2.32 (m, 2H), 1.92 - 1.89 (m, 1H), 1.47 - 1.43 (m, 2H), 1.35 - 1.32 (m, 2H), 1.29 - 1.21 (m, 2H).LC/MS (ESI) (m/z): 626 [M+H]$^+$. |
| **Example 233** | 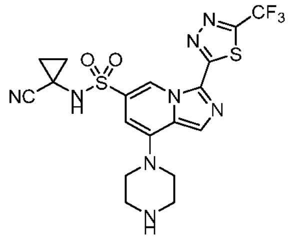 **N-(1-cyanocyclopropyl)-8-(piperazin-1-yl)-3-(5-(trifluoromethyl)-1,3,4-thiadiazol-2-yl)imidazo[1,5-a]pyridine-6-sulfonamide** | $^1$H NMR (400 MHz, DMSO-$d_6$) δ 9.74 (s, 1H), 8.22 (s, 1H), 6.82 (s, 1H), 3.51 (s, 4H), 3.33 (s, 4H), 1.57 - 1.53 (m, 2H), 1.49 - 1.45 (m, 2H). LC/MS (ESI) (m/z): 499 [M+H]$^+$. |
| **Example 234** | 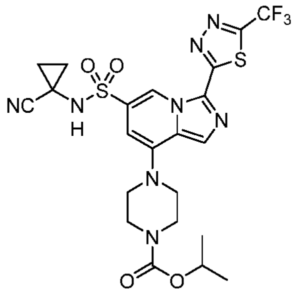 **isopropyl 4-(6-(N-(1-cyanocyclopropyl)sulfamoyl)-3-(5-(trifluoromethyl)-1,3,4-thiadiazol-2-yl)imidazo[1,5-a]pyridin-8-yl)piperazine-1-carboxylate** | $^1$H NMR (400 MHz, DMSO-$d_6$) δ 9.66 (s, 1H), 9.50 (s, 1H), 8.11 (s, 1H), 6.71 (s, 1H), 4.87 - 4.81 (m, 1H), 3.66 - 3.62 (m, 4H), 3.36 - 3.35 (m, 4H), 1.51 - 1.47 (m, 2H), 1.41 - 1.38 (m, 2H), 1.23 (d, *J* = 6.2 Hz, 6H). LC/MS (ESI) (m/z): 585 [M+H]$^+$. |
| **Example 235** | 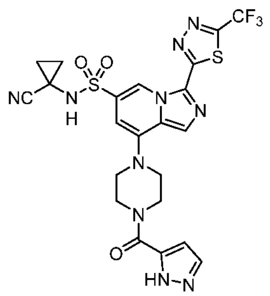 **8-(4-(1H-pyrazole-5-carbonyl)piperazin-1-yl)-N-(1-cyanocyclopropyl)-3-(5-(trifluoromethyl)-1,3,4-thiadiazol-2-yl)imidazo[1,5-a]pyridine-6-sulfonamide** | $^1$H NMR (400 MHz, DMSO-$d_6$) δ 9.67 (s, 1H), 9.49 (s, 1H), 8.17 (s, 1H), 7.83 (s, 1H), 6.74 (s, 1H), 6.65 (d, *J* = 2.3 Hz, 1H), 4.28 - 4.19 (m, 2H), 3.95 - 3.88 (m, 2H), 3.50 - 3.45 (m, 4H), 1.50 - 1.46 (m, 2H), 1.42 - 1.38 (m, 2H). LC/MS (ESI) (m/z): 593 [M+H]$^+$. |

(continued)

| Example | Structure and name | Analysis data |
|---|---|---|
| **Example 236** | 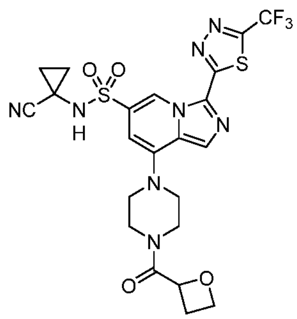<br>**N-(1-cyanocyclopropyl)-8-(4-(oxetane-2-carbonyl)piperazin-1-yl)-3-(5-(trifluoromethyl)-1,3,4-thiadiazol-2-yl)imidazo[1,5-a]pyridine-6-sulfonamide** | $^1$H NMR (400 MHz, DMSO-$d_6$) δ 9.66 (s, 1H), 9.50 (s, 1H), 8.15 (s, 1H), 6.71 (s, 1H), 5.55 - 5.43 (m, 1H), 4.62 - 4.54 (m, 1H), 4.47 - 4.38 (m, 1H), 3.80 - 3.71 (m, 2H), 3.60 - 3.51 (m, 2H), 3.43 - 3.36 (m, 4H), 2.91 - 2.76 (m, 2H), 1.52 - 1.45 (m, 2H), 1.42 - 1.33 (m, 2H). LC/MS (ESI) (m/z): 583 [M+H]$^+$. |
| **Example 237** | 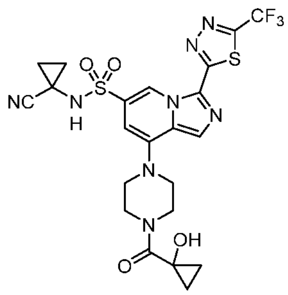<br>**N-(1-cyanocyclopropyl)-8-(4-(1-hydroxycyclopropane-1-carbonyl)piperazin-1-yl)-3-(5-(trifluoromethyl)-1,3,4-thiadiazol-2-yl) imidazo[1,5-a]pyridine-6-sulfonamide** | $^1$H NMR (400 MHz, DMSO-$d_6$) δ 9.66 (s, 1H), 8.16 (s, 1H), 6.73 (s, 1H), 6.44 (s, 1H), 4.13 - 3.72 (m, 4H), 3.38 - 3.35 (m, 4H), 1.52 - 1.44 (m, 2H), 1.41 - 1.33 (m, 2H), 1.02 - 0.95 (m, 2H), 0.84 - 0.78 (m, 2H). LC/MS (ESI) (m/z): 583 [M+H]$^+$. |
| **Example 238** | 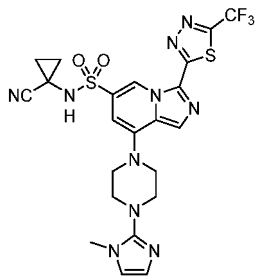<br>**N-(1-cyanocyclopropyl)-8-(4-(1-methyl-1H-imidazol-2-yl)piperazin-1-yl)-3-(5-(trifluoromethyl)-1,3,4-thiadiazol-2-yl)imidazo[1,5-a]pyridine-6-sulfonamide** | $^1$H NMR (400 MHz, DMSO-$d_6$) δ 9.70 (s, 1H), 9.54 (s, 1H), 8.16 (s, 1H), 7.36 (d, $J$ = 11.2 Hz, 2H), 6.79 (s, 1H), 3.68 (s, 3H), 3.57 - 3.52 (m, 8H), 1.53 - 1.47 (m, 2H), 1.44 - 1.39 (m, 2H). LC/MS (ESI) (m/z): 579 [M+H]$^+$. |
| **Example 239** | 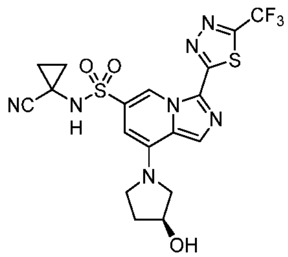<br>**(S)-N-(1-cyanocyclopropyl)-8-(3-hydroxypyrrolidin-1-yl)-3-(5-(trifluoromethyl)-1,3,4-thiadiazol-2-yl)imidazo[1,5-a]pyridine-6-sulfonamide** | $^1$H NMR (400 MHz, DMSO-$d_6$) δ 9.41 (s, 1H), 9.40 (s, 1H), 8.26 (s, 1H), 6.14 (s, 1H), 5.17 (s, 1H), 3.93 - 3.70 (m, 4H), 3.55 - 3.51 (m, 1H), 2.05 - 2.00 (m, 2H), 1.50 - 1.45 (m, 2H), 1.38 - 1.34 (m, 2H), LC/MS (ESI) (m/z): 500 [M+H]$^+$. |

(continued)

| Example | Structure and name | Analysis data |
|---|---|---|
| **Example 240** | 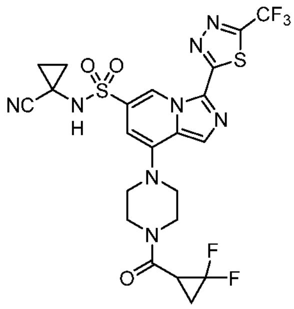 **N-(1-cyanocyclopropyl)-8-(4-(2,2-difluorocyclopropane-1-carbonyl)piperazin-1-yl)-3-(5-(trifluoromethyl)-1,3,4-thiadiazol-2-yl) imidazo[1,5-a]pyridine-6-sulfonamide** | $^1$H NMR (400 MHz, DMSO-$d_6$) δ 9.67 (s, 1H), 9.51 (s, 1H), 8.18 (s, 1H), 6.73 (s, 1H), 3.97 - 3.75 (m, 4H), 3.48 - 3.36 (m, 4H), 3.29 - 3.23 (m, 1H), 2.00 - 1.87 (m, 2H), 1.52 - 1.46 (m, 2H), 1.43 - 1.36 (m, 2H). LC/MS (ESI) (m/z): 603 [M+H]$^+$. |
| **Example 241** | 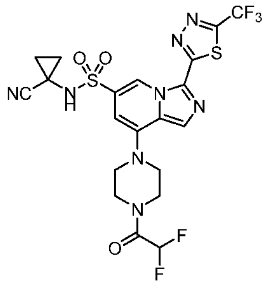 **N-(1-cyanocyclopropyl)-8-(4-(2,2-difluoroacetyl)piperazin-1-yl)-3-(5-(trifluoromethyl)-1,3,4-thiadiazol-2-yl)imidazo[1,5-a]pyridine-6-sulfonamide** | $^1$H NMR (400 MHz, DMSO-$d_6$) δ 9.68 (s, 1H), 9.51 (s, 1H), 8.18 (s, 1H), 6.97 - 6.69 (m, 2H), 3.84 - 3.77 (m, 4H), 3.45 - 3.38 (m, 4H), 1.53 - 1.46 (m, 2H), 1.43 - 1.35 (m, 2H). LC/MS (ESI) (m/z): 577 [M+H]$^+$. |
| **Example 242** | 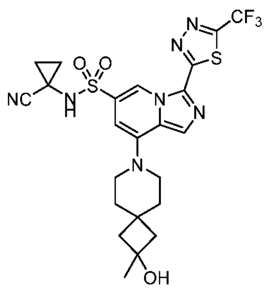 **N-(1-cyanocyclopropyl)-8-(2-hydroxy-2-methyl-7-azaspiro[3.5] nonan-7-yl)-3-(5-(trifluoromethyl)-1,3,4-thiadiazol-2-yl)imidazo [1,5-a]pyridine-6-sulfonamide** | $^1$H NMR (400 MHz, DMSO-$d_6$) δ 9.60 (s, 1H), 8.01 (s, 1H), 6.68 (d, $J$ = 0.9 Hz, 1H), 4.81 (s, 1H), 3.25 (d, $J$ = 1.0 Hz, 4H), 1.97 - 1.87 (m, 4H), 1.86 - 1.80 (m, 2H), 1.78 - 1.71 (m, 2H), 1.49 - 1.42 (m, 2H), 1.39 - 1.33 (m, 2H), 1.28 (s, 3H). LC/MS (ESI) (m/z): 568 [M+H]$^+$. |
| **Example 243** | 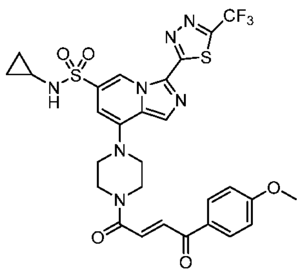 **(E)-N-cyclopropyl-8-(4-(4-(4-methoxyphenyl)-4-oxobut-2-enoyl) piperazin-1-yl)-3-(5-(trifluoromethyl)-1,3,4-thiadiazol-2-yl)imidazo[1,5-a]pyridine-6-sulfonamide** | $^1$H NMR (400 MHz, DMSO-$d_6$) δ 9.60 (s, 1H), 8.30 (d, $J$ = 2.5 Hz, 1H), 8.14 (s, 1H), 8.07 (d, $J$ = 8.9 Hz, 2H), 7.84 (d, $J$ = 15.2 Hz, 1H), 7.49 (d, $J$ = 15.2 Hz, 1H), 7.11 (d, $J$ = 8.9 Hz, 2H), 6.73 (s, 1H), 3.91 - 3.85 (m, 7H), 3.44 - 3.38 (m, 4H), 2.29 - 2.23 (m, 1H), 0.56 - 0.51 (m, 2H), 0.50 - 0.46 (m, 2H). LC/MS (ESI) (m/z): 662 [M+H]$^+$. |

(continued)

| Example | Structure and name | Analysis data |
|---|---|---|
| **Example 244** | 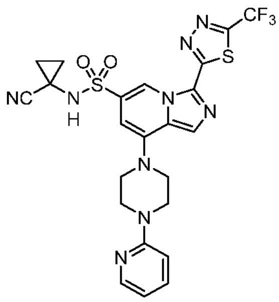 **N-(1-cyanocyclopropyl)-8-(4-(pyridin-2-yl)piperazin-1-yl)-3-(5-(trifluoromethyl)-1,3,4-thiadiazol-2-yl)imidazo[1,5-a]pyridine-6-sulfonamide** | $^1H$ NMR (400 MHz, DMSO-$d_6$) δ 9.66 (s, 1H), 9.51 (s, 1H), 8.18 - 8.13 (m, 2H), 7.63 - 7.57 (m, 1H), 6.93 (d, *J* = 8.6 Hz, 1H), 6.75 (s, 1H), 6.71 (dd, *J* = 6.8, 4.9 Hz, 1H), 3.80 - 3.75 (m, 4H), 3.50 - 3.46 (m, 4H), 1.49 - 1.38 (m, 4H). LC/MS (ESI) (m/z): 576 $[M+H]^+$ |
| **Example 245** | 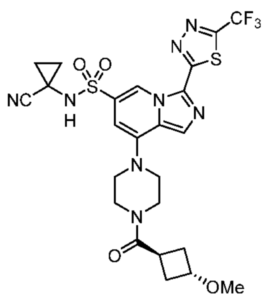 **N-(1-cyanocyclopropyl)-8-(4-((1r,3r)-3-methoxycyclobutane-1-carbonyl)piperazin-1-yl)-3-(5-(trifluoromethyl)-1,3,4-thiadiazol-2-yl)imidazo[1,5-a]pyridine-6-sulfonamide** | $^1H$ NMR (400 MHz, DMSO-$d_6$) δ 9.66 (s, 1H), 8.14 (s, 1H), 6.71 (s, 1H), 3.89 - 3.84 (m, 1H), 3.77 - 3.72 (m, 2H), 3.62 - 3.58 (m, 2H), 3.39 - 3.36 (m, 4H), 3.14 (s, 3H), 2.56 - 2.54 (m, 1H), 2.46 - 2.40 (m, 2H), 2.19 - 2.10 (m, 2H), 1.52 - 1.46 (m, 2H), 1.41 - 1.33 (m, 2H). LC/MS (ESI) (m/z): 611 $[M+H]^+$. |
| **Example 246** | 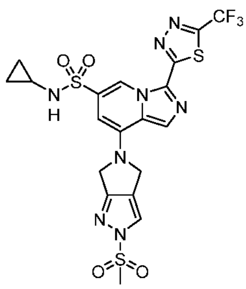 **N-cyclopropyl-8-(2-(methylsulfonyl)-2,6-dihydropyrrolo[3,4-c]pyrazol-5(4H)-yl)-3-(5-(trifluoromethyl)-1,3,4-thiadiazol-2-yl)imidazo[1,5-a]pyridine-6-sulfonamide** | $^1H$ NMR (400 MHz, DMSO-$d_6$) δ 9.47 (s, 1H), 8.43 (s, 1H), 8.26 (s, 1H), 8.23 (s, 1H), 6.37 (s, 1H), 5.02 (s, 2H), 4.89 (s, 2H), 3.60 (s, 3H), 2.31 - 2.28 (m, 1H), 0.56 - 0.51 (m, 4H). LC/MS (ESI) (m/z): 575 $[M+H]^+$. |
| **Example 247** | 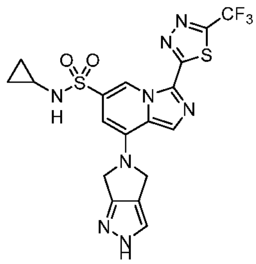 **N-cyclopropyl-8-(2,6-dihydropyrrolo[3,4-c]pyrazol-5(4H)-yl)-3-(5-(trifluoromethyl)-1,3,4-thiadiazol-2-yl)imidazo[1,5-a]pyridine-6-sulfonamide** | $^1H$ NMR (400 MHz, DMSO-$d_6$) δ 12.85 (s, 1H), 9.43 (s, 1H), 8.42 (s, 1H), 8.23 (s, 1H), 7.68 (s, 1H), 6.31 (s, 1H), 4.87 - 4.84 (m, 4H), 2.31 - 2.26 (m, 1H), 0.56 - 0.47 (m, 4H). LC/MS (ESI) (m/z): 497 $[M+H]^+$. |

(continued)

| Example | Structure and name | Analysis data |
|---|---|---|
| **Example 248** | 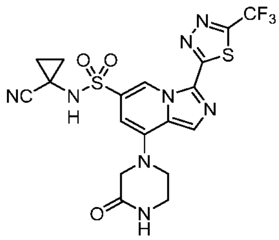 **N-(1-cyanocyclopropyl)-8-(3-oxopiperazin-1-yl)-3-(5-(trifluoromethyl)-1,3,4-thiadiazol-2-yl)imidazo[1,5-a]pyridine-6-sulfonamide** | $^1$H NMR (400 MHz, DMSO-$d_6$) δ 9.64 (s, 1H), 9.48 (s, 1H), 8.20 (s, 1H), 8.17 (s, 1H), 6.65 (s, 1H), 3.94 (s, 2H), 3.72 - 3.65 (m, 2H), 3.49 - 3.42 (m, 2H), 1.49 - 1.45 (m, 2H), 1.41 - 1.36 (m, 2H). LC/MS (ESI) (m/z): 513 [M+H]. |
| **Example 249** | 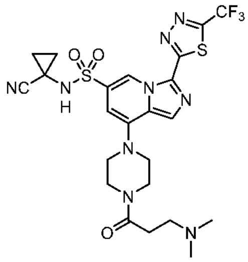 **N-(1-cyanocyclopropyl)-8-(4-(3-(dimethylamino)propanoyl)piperazin-1-yl)-3-(5-(trifluoromethyl)-1,3,4-thiadiazol-2-yl)imidazo [1,5-a]pyridine-6-sulfonamide** | $^1$H NMR (400 MHz, DMSO-$d_6$) δ 9.67 (s, 1H), 9.53 (s, 1H), 9.12 (s, 1H), 8.17 (s, 1H), 6.72 (s, 1H), 3.79 - 3.73 (m, 4H), 3.38 - 3.31 (m, 6H), 2.94 - 2.89 (m, 2H), 2.81 (d, *J* = 4.8 Hz, 6H), 1.52 - 1.47 (m, 2H), 1.43 - 1.38 (m, 2H). LC/MS (ESI) (m/z): 598 $[M+H]^+$. |
| **Example 250** | 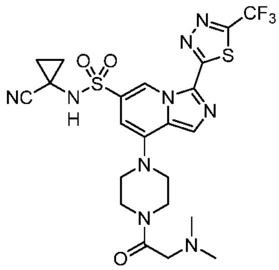 **N-(1-cyanocyclopropyl)-8-(4-(dimethylglycyl)piperazin-1-yl)-3-(5-(trifluoromethyl)-1,3,4-thiadiazol-2-yl)imidazo[1,5-a]pyridine-6-sulfonamide** | $^1$H NMR (400 MHz, DMSO-$d_6$) δ 9.66 (s, 1H), 8.16 (s, 1H), 6.72 (s, 1H), 3.83 - 3.72 (m, 4H), 3.45 - 3.36 (m, 6H), 2.35 (s, 6H), 1.51 - 1.44 (m, 2H), 1.43 - 1.36 (m, 2H). LC/MS (ESI) (m/z): 584 $[M+H]^+$. |
| **Example 251** | 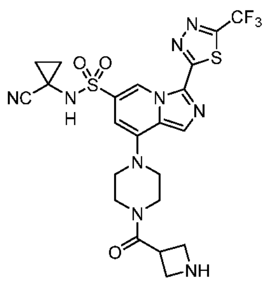 **8-(4-(azetidine-3-carbonyl)piperazin-1-yl)-N-(1-cyanocyclopropyl)-3-(5-(trifluoromethyl)-1,3,4-thiadiazol-2-yl)imidazo[1,5-a] pyridine-6-sulfonamide** | $^1$H NMR (400 MHz, DMSO-$d_6$) δ 9.60 (s, 1H), 8.08 (s, 1H), 6.71 (s, 1H), 3.99 - 3.88 (m, 6H), 3.76 - 3.73 (m, 3H), 3.55 - 3.46 (m, 5H), 1.33 - 1.29 (m, 2H), 1.25 - 1.22 (m, 2H). LC/MS (ESI) (m/z): 582 $[M+H]^+$ |

(continued)

| Example | Structure and name | Analysis data |
|---|---|---|
| **Example 252** | 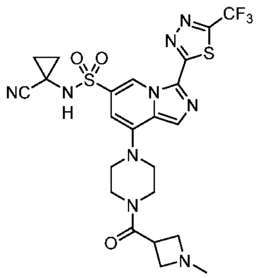 **N-(1-cyanocyclopropyl)-8-(4-(1-methylazetidine-3-carbonyl)piperazin-1-yl)-3-(5-(trifluoromethyl)-1,3,4-thiadiazol-2-yl)imidazo[1,5-a]pyridine-6-sulfonamide** | $^1$H NMR (400 MHz, DMSO-$d_6$) δ 9.65 (s, 1H), 8.19 - 8.15 (m, 1H), 6.70 (s, 1H), 3.75 - 3.71 (m, 2H), 3.58 - 3.50 (m, 6H), 3.34 - 3.30 (m, 4H), 3.29 - 3.27 (m, 1H), 2.27 (s, 3H), 1.49 - 1.43 (m, 2H), 1.39 - 1.33 (m, 2H). LC/MS (ESI) (m/z): 596 $[M+H]^+$. |
| **Example 253** | 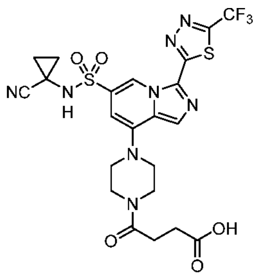 **4-(4-(6-(N-(1-cyanocyclopropyl)sulfamoyl)-3-(5-(trifluoromethyl)-1,3,4-thiadiazol-2-yl)imidazo[1,5-a]pyridin-8-yl)piperazin-1-yl)-4-oxobutanoic acid** | $^1$H NMR (400 MHz, $CDCl_3$) δ 9.97 (s, 1H), 7.74 (s, 1H), 6.77 (s, 1H), 3.96 - 3.77 (m, 4H), 3.43 - 3.28 (m, 4H), 2.78 - 2.72 (m, 4H), 1.71 - 1.65 (m, 2H), 1.51 - 1.46 (m, 2H). LC/MS (ESI) (m/z): 599 $[M+H]^+$. |
| **Example 254** | 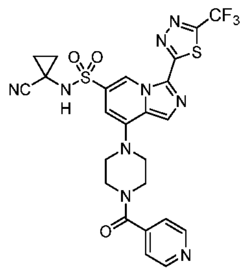 **N-(1-cyanocyclopropyl)-8-(4-isonicotinoylpiperazin-1-yl)-3-(5-(trifluoromethyl)-1,3,4-thiadiazol-2-yl)imidazo[1,5-a]pyridine-6-sulfonamide** | $^1$H NMR (400 MHz, DMSO-$d_6$) δ 9.66 (s, 1H), 9.51 (s, 1H), 8.73 - 8.70 (m, 2H), 8.13 (s, 1H), 7.50 - 7.47 (m, 2H), 6.73 (s, 1H), 3.97 - 3.88 (m, 2H), 3.59 - 3.52 (m, 2H), 3.50 - 3.44 (m, 2H), 3.40 - 3.35 (m, 2H), 1.51 - 1.46 (m, 2H), 1.41 - 1.36 (m, 2H). LC/MS (ESI) (m/z): 604 $[M+H]^+$. |
| **Example 255** | 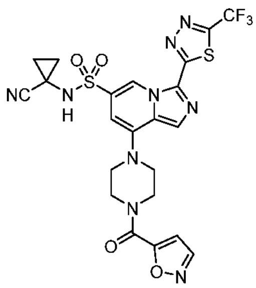 **N-(1-cyanocyclopropyl)-8-(4-(isoxazole-5-carbonyl)piperazin-1-yl)-3-(5-(trifluoromethyl)-1,3,4-thiadiazol-2-yl)imidazo[1,5-a]pyridine-6-sulfonamide** | $^1$H NMR (400 MHz, DMSO-$d_6$) δ 9.67 (s, 1H), 9.51 (s, 1H), 8.80 (d, $J$ = 1.9 Hz, 1H), 8.16 (s, 1H), 7.03 (d, $J$ = 1.9 Hz, 1H), 6.73 (d, $J$ = 0.9 Hz, 1H), 3.95 - 3.80 (m, 4H), 3.50 - 3.43 (m, 4H), 1.51 - 1.45 (m, 2H), 1.42 - 1.36 (m, 2H). LC/MS (ESI) (m/z): 594 $[M+H]^+$. |

(continued)

| Example | Structure and name | Analysis data |
| --- | --- | --- |
| **Example 256** | 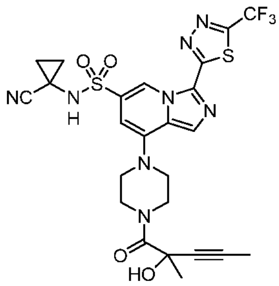 **N-(1-cyanocyclopropyl)-8-(4-(2-hydroxy-2-methylpent-3-ynoyl) piperazin-1-yl)-3-(5-(trifluoromethyl)-1,3,4-thiadiazol-2-yl)imidazo[1,5-a]pyridine-6-sulfonamide** | $^1$H NMR (400 MHz, DMSO-$d_6$) δ 9.65 (s, 1H), 9.49 (s, 1H), 8.17 (s, 1H), 6.72 (s, 1H), 6.10 (s, 1H), 4.33 - 4.05 (m, 2H), 4.03 - 3.65 (m, 2H), 3.42 - 3.36 (m, 4H), 1.84 (s, 3H), 1.55 (s, 3H), 1.49 - 1.44 (m, 2H), 1.41 - 1.34 (m, 2H). LC/MS (ESI) (m/z): 609 [M+H]$^+$. |
| **Example 257** | 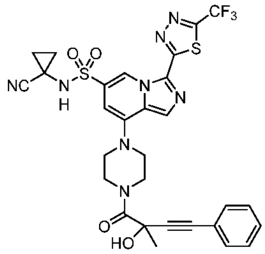 **N-(1-cyanocyclopropyl)-8-(4-(2-hydroxy-2-methyl-4-phenylbut-3-ynoyl)piperazin-1-yl)-3-(5-(trifluoromethyl)-1,3,4-thiadiazol-2-yl)imidazo[1,5-a]pyridine-6-sulfonamide** | $^1$H NMR (400 MHz, DMSO-$d_6$). δ 9.66 (s, 1H), 9.48 (s, 1H), 8.18 (s, 1H), 7.48 - 7.45 (m, 2H), 7.41 - 7.37 (m, 3H), 6.72 (s, 1H), 6.43 (s, 1H), 4.34 - 4.22 (m, 2H), 3.85 - 3.74 (m, 2H), 3.59 - 3.49 (m, 4H), 1.69 (s, 3H), 1.49 - 1.45 (m, 2H), 1.40 - 1.34 (m, 2H). LC/MS (ESI) (m/z): 671 [M+H]$^+$. |
| **Example 258** | 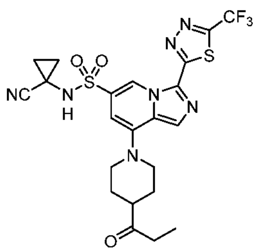 **N-(1-cyanocyclopropyl)-8-(4-propionylpiperidin-1-yl)-3-(5-(trifluoromethyl)-1,3,4-thiadiazol-2-yl)imidazo[1,5-a]pyridine-6-sulfonamide** | $^1$H NMR (400 MHz, $CDCl_3$) δ 9.94 (s, 1H), 7.73 (s, 1H), 6.69 (s, 1H), 5.65 (s, 1H), 3.86 - 3.80 (m, 2H), 3.06 - 2.98 (m, 2H), 2.58 - 2.53 (m, 3H), 2.10 -1.92 (m, 4H), 1.72 - 1.69 (m, 2H), 1.53 - 1.50 (m, 2H), 1.12 - 1.08 (m, 3H). LC/MS (ESI) (m/z): 554 [M+H]$^+$. |
| **Example 259** | 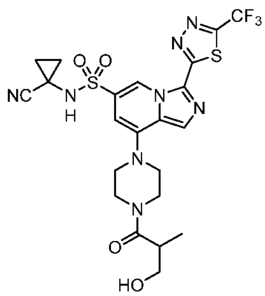 **N-(1-cyanocyclopropyl)-8-(4-(3-hydroxy-2-methylpropanoyl)piperazin-1-yl)-3-(5-(trifluoromethyl)-1,3,4-thiadiazol-2-yl)imidazo[1,5-a]pyridine-6-sulfonamide** | $^1$H NMR (400 MHz, $CDCl_3$) δ 9.99 (s, 1H), 7.75 (s, 1H), 6.74 (s, 1H), 6.20 (s, 1H), 4.04 - 3.94 (m, 1H), 3.86 - 3.76 (m, 4H), 3.43 - 3.32 (m, 4H), 3.06 - 2.81 (m, 2H), 1.73 - 1.69 (m, 2H), 1.53 - 1.50 (m, 2H), 1.19 (d, $J$ = 7.1 Hz, 3H). LC/MS (ESI) (m/z): 585 [M+H]$^+$. |

(continued)

| Example | Structure and name | Analysis data |
|---|---|---|
| **Example 260** | 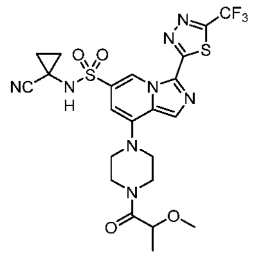 **N-(1-cyanocyclopropyl)-8-(4-(2-methoxypropanoyl)piperazin-1-yl)-3-(5-(trifluoromethyl)-1,3,4-thiadiazol-2-yl)imidazo[1,5-a]pyridine-6-sulfonamide** | $^1$H NMR (400 MHz, $CDCl_3$) δ 10.00 (s, 1H), 7.75 (s, 1H), 6.83 (s, 1H), 6.52 (s, 1H), 4.26 - 4.19 (m, 1H), 3.98 - 3.92 (m, 4H), 3.44 - 3.34 (m, 7H), 1.73 - 1.68 (m, 2H), 1.52 - 1.48 (m, 2H), 1.46 - 1.45 (m, 3H). LC/MS (ESI) (m/z): 585 $[M+H]^+$. |
| **Example 261** | 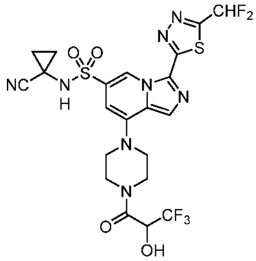 **N-(1-cyanocyclopropyl)-3-(5-(difluoromethyl)-1,3,4-thiadiazol-2-yl)-8-(4-(3,3,3-trifluoro-2-hydroxypropanoyl)piperazin-1-yl)imidazo[1,5-a]pyridine-6-sulfonamide** | $^1$H NMR (400 MHz, DMSO-$d_6$) δ 9.69 (s, 1H), 9.49 (s, 1H), 8.13 (s, 1H), 7.69 (t, *J* = 53.2 Hz, 1H), 6.87 (d, *J* = 8.6 Hz, 1H), 6.69 (d, *J* = 0.8 Hz, 1H), 5.26 - 5.14 (m, 1H), 3.92 - 3.74 (m, 4H), 3.42 - 3.36 (m, 4H), 1.50 - 1.37 (m, 4H). LC/MS (ESI) (m/z): 607 $[M+H]^+$. |
| **Example 262** | 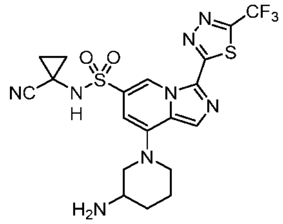 **8-(3-aminopiperidin-1-yl)-N-(1-cyanocyclopropyl)-3-(5-(trifluoromethyl)-1,3,4-thiadiazol-2-yl)imidazo[1,5-a]pyridine-6-sulfonamide** | $^1$H NMR (400 MHz, $CDCl_3$) δ 9.85 (s, 1H), 7.71 (s, 1H), 6.81 (s, 1H), 3.86 - 3.76 (m, 2H), 3.68 - 3.59 (m, 1H), 3.42 - 3.26 (m, 2H), 3.24 - 3.14 (m, 2H), 2.22 - 2.14 (m, 1H), 2.00 - 1.92 (m, 1H), 1.83 - 1.72 (m, 2H), 1.68 - 1.59 (m, 2H), 1.43 - 1.35 (m, 2H). LC/MS (ESI) (m/z): 513 [M+H]. |
| **Example 263** | 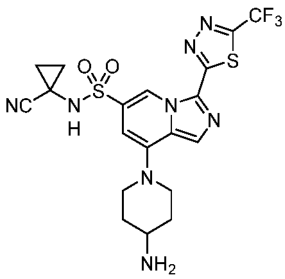 **8-(4-aminopiperidin-1-yl)-N-(1-cyanocyclopropyl)-3-(5-(trifluoromethyl)-1,3,4-thiadiazol-2-yl)imidazo[1,5-a]pyridine-6-sulfonamide** | $^1$H NMR (400 MHz, DMSO-$d_6$) δ 9.55 (s, 1H), 7.99 (s, 1H), 6.71 (s, 1H), 3.80 - 3.74 (m, 2H), 3.62 - 3.58 (m, 1H), 3.18 - 3.15 (m, 2H), 3.03 - 2.96 (m, 2H), 2.05 - 1.98 (m, 2H), 1.78 - 1.70 (m, 2H), 1.30 - 1.24 (m, 2H), 1.21 - 1.14 (m, 2H). LC/MS (ESI) (m/z): 513 [M+H]. |

(continued)

| Example | Structure and name | Analysis data |
|---|---|---|
| **Example 264** | 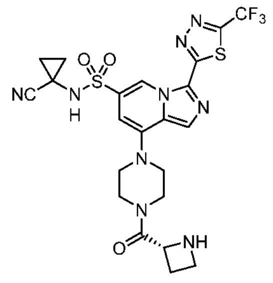<br>**(R)-8-(4-(azetidine-2-carbonyl)piperazin-1-yl)-N-(1-cyanocyclopropyl)-3-(5-(trifluoromethyl)-1,3,4-thiadiazol-2-yl)imidazo[1,5-a]pyridine-6-sulfonamide** | $^1$H NMR (400 MHz, $CDCl_3$) δ 9.97 (s, 1H), 7.69 (s, 1H), 6.80 (s, 1H), 5.37 - 5.23 (m, 1H), 4.17 (d, *J* = 12.8 Hz, 1H), 4.02 - 3.84 (m, 2H), 3.69 - 3.59 (m, 2H), 3.55 - 3.46 (m, 2H), 3.41 - 3.34 (m, 1H), 3.31 - 3.16 (m, 2H), 3.00 - 2.92 (m, 1H), 2.50 - 2.46 (m, 2H), 1.73 - 1.64 (m, 2H), 1.52 - 1.45 (m, 2H). LC/MS (ESI) (m/z): 582 $[M+H]^+$. |
| **Example 265** | 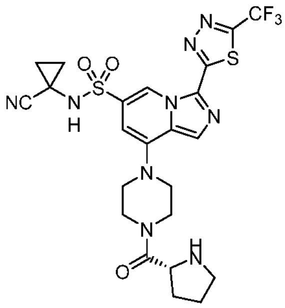<br>**(R)-N-(1-cyanocyclopropyl)-8-(4-prolylpiperazin-1-yl)-3-(5-(trifluoromethyl)-1,3,4-thiadiazol-2-yl)imidazo[1,5-a]pyridine-6-sulfonamide** | $^1$H NMR (400 MHz, $CDCl_3$) δ 9.95 (s, 1H), 7.66 (s, 1H), 6.85 (s, 1H), 5.02 - 4.82 (m, 1H), 4.29 (d, *J* = 12.1 Hz, 1H), 3.96 (d, *J* = 13.1 Hz, 1H), 3.66 - 3.33 (m, 8H), 2.52 - 2.45 (m, 1H), 2.22 - 1.79 (m, 4H), 1.70 - 1.59 (m, 2H), 1.48 - 1.40 (m, 2H). LC/MS (ESI) (m/z): 596 $[M+H]^+$. |
| **Example 266** | 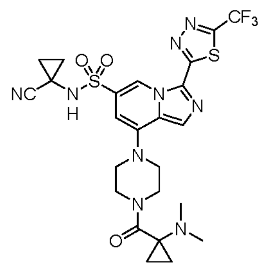<br>**N-(1-cyanocyclopropyl)-8-(4-(1-(dimethylamino)cyclopropane-1-carbonyl)piperazin-1-yl)-3-(5-(trifluoromethyl)-1,3,4-thiadiazol-2-yl)imidazo[1,5-a]pyridine-6-sulfonamide** | $^1$H NMR (400 MHz, $CDCl_3$) δ 9.98 (s, 1H), 7.76 (s, 1H), 6.76 (d, *J* = 0.9 Hz, 1H), 6.43 (s, 1H), 4.01 - 3.93 (m, 4H), 3.41 - 3.35 (m, 4H), 2.33 (s, 6H), 1.72 - 1.68 (m, 2H), 1.52 - 1.47 (m, 2H), 1.09 - 1.00 (m, 2H), 0.97 - 0.91 (m, 2H). LC/MS (ESI) (m/z): 610 $[M+H]^+$. |
| **Example 267** | 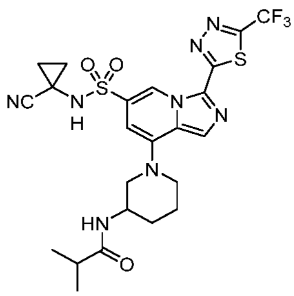<br>**N-(1-(6-(N-(1-cyanocyclopropyl)sulfamoyl)-3-(5-(trifluoromethyl)-1,3,4-thiadiazol-2-yl)imidazo[1,5-a]pyridin-8-yl)piperidin-3-yl)isobutyramide** | $^1$H NMR (400 MHz, $CDCl_3$) δ 9.91 (s, 1H), 7.83 (s, 1H), 7.00 (s, 1H), 6.63 (s, 1H), 5.75 - 5.67 (m, 1H), 4.41 - 4.31 (m, 1H), 3.85 - 3.73 (m, 2H), 3.39 - 3.31 (m, 1H), 3.12 - 3.05 (m, 1H), 2.46 - 2.37 (m, 1H), 2.05 - 1.97 (m, 1H), 1.90 - 1.83 (m, 1H), 1.81 - 1.72 (m, 2H), 1.71 - 1.63 (m, 2H), 1.49 - 1.40 (m, 2H), 1.20 - 1.14 (m, 6H). LC/MS (ESI) (m/z): 583 [M+H]. |

(continued)

| Example | Structure and name | Analysis data |
|---|---|---|
| **Example 268** | 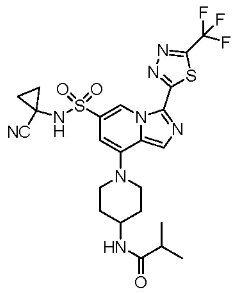 **N-(1-(6-(N-(1-cyanocyclopropyl)sulfamoyl)-3-(5-(trifluoromethyl)-1,3,4-thiadiazol-2-yl)imidazo[1,5-a]pyridin-8-yl)piperidin-4-yl)isobutyramide** | $^1$H NMR (400 MHz, MeOD) δ 9.85 (s, 1H), 7.87 (s, 1H), 6.79 (s, 1H), 3.99 - 3.89 (m, 1H), 3.86 - 3.79 (m, 2H), 3.15 - 3.06 (m, 2H), 2.52 - 2.43 (m, 1H), 2.12 - 2.04 (m, 2H), 1.85 - 1.75 (m, 2H), 1.57 - 1.52 (m, 2H), 1.47 - 1.42 (m, 2H), 1.13 (d, J = 6.9 Hz, 6H). LC/MS (ESI) (m/z): 583 [M+H]. |
| **Example 269** | 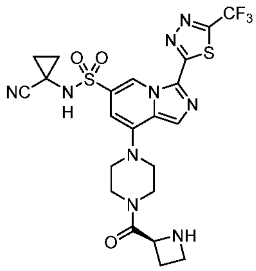 **(S)-8-(4-(azetidine-2-carbonyl)piperazin-1-yl)-N-(1-cyanocyclopropyl)-3-(5-(trifluoromethyl)-1,3,4-thiadiazol-2-yl)imidazo[1,5-a]pyridine-6-sulfonamide** | $^1$H NMR (400 MHz, MeOD) δ 9.91 (s, 1H), 7.96 (s, 1H), 6.83 (s, 1H), 5.51 - 5.44 (m, 1H), 4.21 - 4.13 (m, 1H), 4.05 - 3.82 (m, 4H), 3.67 - 3.58 (m, 2H), 3.52 - 3.43 (m, 4H), 3.01 - 2.94 (m, 1H), 2.72 - 2.63 (m, 1H), 1.59 - 1.53 (m, 2H), 1.48 - 1.42 (m, 2H). LC/MS (ESI) (m/z): 582 $[M+H]^+$. |
| **Example 270** | 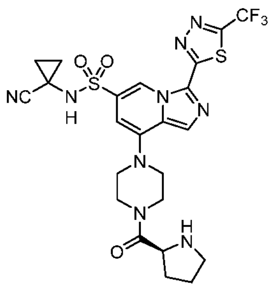 **(S)-N-(1-cyanocyclopropyl)-8-(4-prolylpiperazin-1-yl)-3-(5-(trifluoromethyl)-1,3,4-thiadiazol-2-yl)imidazo[1,5-a]pyridine-6-sulfonamide** | $^1$H NMR (400 MHz, MeOD) δ 9.91 (s, 1H), 7.97 (s, 1H), 6.83 (s, 1H), 4.78 - 4.72 (m, 1H), 4.04 - 3.95 (m, 1H), 3.94 - 3.87 (m, 1H), 3.86 - 3.81 (m, 2H), 3.53 - 3.45 (m, 4H), 3.44 - 3.41 (m, 1H), 3.39 - 3.34 (m, 1H), 2.63 - 2.53 (m, 1H), 2.18 - 2.08 (m, 2H), 2.06 - 1.96 (m, 1H), 1.59 - 1.53 (m, 2H), 1.48 - 1.42 (m, 2H). LC/MS (ESI) (m/z): 596 $[M+H]^+$. |
| **Example 271** | 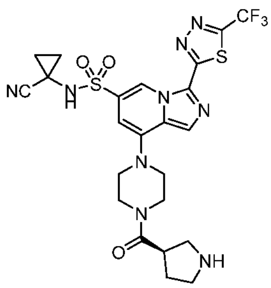 **(R)-N-(1-cyanocyclopropyl)-8-(4-(pyrrolidine-3-carbonyl)piperazin-1-yl)-3-(5-(trifluoromethyl)-1,3,4-thiadiazol-2-yl)imidazo[1,5-a]pyridine-6-sulfonamide** | $^1$H NMR (400 MHz, DMSO-$d_6$) δ 9.62 (s, 1H), 8.32 (s, 1H), 8.11 (s, 1H), 6.73 (s, 1H), 3.85 - 3.73 (m, 4H), 3.57 - 3.42 (m, 2H), 3.40 - 3.35 (m, 2H), 3.30 - 3.22 (m, 2H), 3.17 (s, 1H), 3.14 - 3.07 (m, 2H), 2.21 - 2.10 (m, 1H), 2.00 - 1.90 (m, 1H), 1.40 - 1.31 (m, 2H), 1.30 - 1.23 (m, 2H). LC/MS (ESI) (m/z): 596 $[M+H]^+$. |

(continued)

| Example | Structure and name | Analysis data |
|---|---|---|
| **Example 272** | 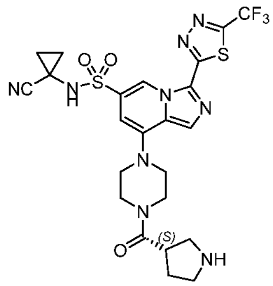 **(S)-N-(1-cyanocyclopropyl)-8-(4-(pyrrolidine-3-carbonyl)piperazin-1-yl)-3-(5-(trifluoromethyl)-1,3,4-thiadiazol-2-yl)imidazo[1,5-a]pyridine-6-sulfonamide** | $^1$H NMR (400 MHz, $CD_3OD$) δ 9.88 (s, 1H), 8.53 (s, 1H), 7.95 (s, 1H), 6.80 (d, *J* = 1.0 Hz, 1H), 3.94 - 3.88 (m, 4H), 3.76 - 3.70 (m, 1H), 3.69 - 3.64 (m, 1H), 3.50 - 3.45 (m, 2H), 3.43 - 3.40 (m, 2H), 3.39 - 3.33 (m, 3H), 2.45 - 2.34 (m, 1H), 2.19 - 2.09 (m, 1H), 1.56 - 1.51 (m, 2H), 1.47 - 1.42 (m, 2H). LC/MS (ESI) (m/z): 596 [M+H]$^+$. |
| **Example 273** | 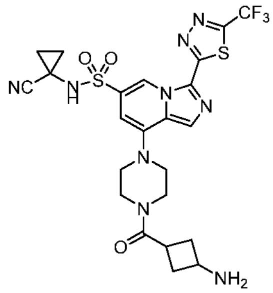 **8-(4-(3-aminocyclobutane-1-carbonyl)piperazin-1-yl)-N-(1-cyanocyclopropyl)-3-(5-(trifluoromethyl)-1,3,4-thiadiazol-2-yl)imidazo[1,5-a]pyridine-6-sulfonamide** | $^1$H NMR (400 MHz, DMSO-$d_6$) δ 9.60 (s, 1H), 8.08 (s, 1H), 6.72 (s, 1H), 3.77 - 3.70 (m, 2H), 3.65 - 3.44 (m, 4H), 3.39 - 3.07 (m, 5H), 2.49 - 2.35 (m, 3H), 2.28 - 2.12 (m, 2H), 1.39 - 1.30 (m, 2H), 1.29 - 1.22 (m, 2H). LC/MS (ESI) (m/z): 596 [M+H]$^+$. |
| **Example 274** | 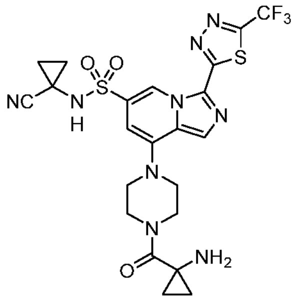 **8-(4-(1-aminocyclopropane-1-carbonyl)piperazin-1-yl)-N-(1-cyanocyclopropyl)-3-(5-(trifluoromethyl)-1,3,4-thiadiazol-2-yl)imidazo[1,5-a]pyridine-6-sulfonamide** | $^1$H NMR (400 MHz, $CDCl_3$) δ 9.96 (s, 1H), 7.71 (s, 1H), 6.79 (s, 1H), 4.10 - 3.86 (m, 4H), 3.50 - 3.35 (m, 4H), 1.72 - 1.65 (m, 2H), 1.51 - 1.45 (m, 2H), 1.25 - 1.07 (m, 4H). LC/MS (ESI) (m/z): 582 [M+H]$^+$. |
| **Example 275** | 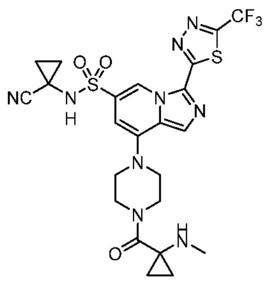 **N-(1-cyanocyclopropyl)-8-(4-(1-(methylamino)cyclopropane-1-carbonyl)piperazin-1-yl)-3-(5-(trifluoromethyl)-1,3,4-thiadiazol-2-yl)imidazo[1,5-a]pyridine-6-sulfonamide** | $^1$H NMR (400 MHz, $CDCl_3$) δ 9.99 (s, 1H), 7.76 (s, 1H), 6.78 (d, *J* = 1.0 Hz, 1H), 4.11 - 3.94 (m, 4H), 3.45 - 3.37 (m, 4H), 2.48 (s, 3H), 1.73 - 1.69 (m, 2H), 1.52 - 1.48 (m, 2H), 1.15 - 1.08 (m, 2H), 0.94 - 0.84 (m, 2H). LC/MS (ESI) (m/z): 596 [M+H]$^+$. |

(continued)

| Example | Structure and name | Analysis data |
|---|---|---|
| **Example 276** | 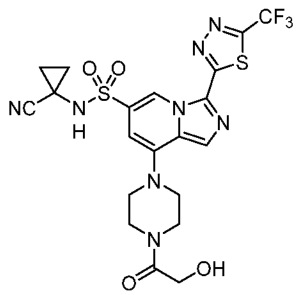<br>**N-(1-cyanocyclopropyl)-8-(4-(2-hydroxyacetyl)piperazin-1-yl)-3-(5-(trifluoromethyl)-1,3,4-thiadiazol-2-yl)imidazo[1,5-a]pyridine-6-sulfonamide** | $^1$H NMR (400 MHz, $CDCl_3$) δ 10.06 - 10.00 (m, 1H), 7.80 - 7.73 (m, 1H), 6.78 - 6.74 (m, 1H), 5.74 - 5.68 (m, 1H), 4.31 - 4.24 (m, 2H), 3.98 (s, 2H), 3.59 (s, 2H), 3.45 - 3.39 (m, 4H), 2.04 - 2.01 (m, 1H), 1.76 - 1.72 (m, 2H), 1.56 - 1.54 (m, 2H). LC/MS (ESI) (m/z): 557 [M+H] +. |
| **Example 277** | 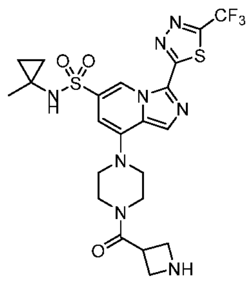<br>**8-(4-(azetidine-3-carbonyl)piperazin-1-yl)-N-(1-methylcyclopropyl)-3-(5-(trifluoromethyl)-1,3,4-thiadiazol-2-yl)imidazo[1,5-a]pyridine-6-sulfonamide** | $^1$H NMR (400 MHz, DMSO-$d_6$) δ 9.59 (s, 1H), 8.33 (s, 1H), 8.11 (s, 1H), 6.72 (s, 1H), 3.97 - 3.91 (m, 2H), 3.88 - 3.82 (m, 2H), 3.77 - 3.74 (m, 2H), 3.33 - 3.29 (m, 7H), 1.16 (s, 3H), 0.78 - 0.69 (m, 2H), 0.49 - 0.42 (m, 2H). LC/MS (ESI) (m/z): 571 $[M+H]^+$. |
| **Example 278** | 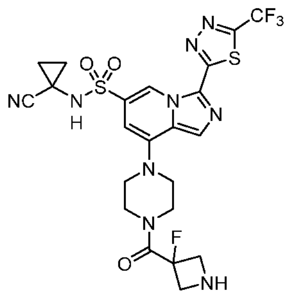<br>**N-(1-cyanocyclopropyl)-8-(4-(3-fluoroazetidine-3-carbonyl)piperazin-1-yl)-3-(5-(trifluoromethyl)-1,3,4-thiadiazol-2-yl)imidazo[1,5-a]pyridine-6-sulfonamide** | $^1$H NMR (400 MHz, $CDCl_3$) δ 10.05 (s, 1H), 7.80 (s, 1H), 6.83 (s, 1H), 4.84 - 4.39 (m, 2H), 4.38 - 4.06 (m, 2H), 4.04 - 3.95 (m, 2H), 3.81 - 3.72 (m, 2H), 3.52 - 3.42 (m, 4H), 1.77 - 1.75 (m, 2H), 1.59 - 1.54 (m, 2H). LC/MS (ESI) (m/z): 600 $[M+H]^+$. |
| **Example 279** | 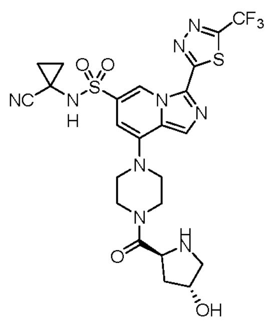<br>**N-(1-cyanocyclopropyl)-8-(4-((2S,4R)-4-hydroxypyrrolidine-2-carbonyl)piperazin-1-yl)-3-(5-(trifluoromethyl)-1,3,4-thiadiazol-2-yl)imidazo[1,5-a]pyridine-6-sulfonamide** | $^1$H NMR(400 MHz, DMSO-$d_6$) δ 9.68 (s, 1H), 8.17 (s, 1H), 6.74 (s, 1H), 5.45 (s, 1H), 4.77 - 4.67 (m, 1H), 4.44 (s, 1H), 3.88 - 3.72 (m, 4H), 3.67 - 3.50 (m, 1H), 3.44 - 3.39 (m, 4H), 3.09 - 3.05 (m, 1H), 2.36 - 2.31 (m, 1H), 2.03 - 1.96 (m, 1H), 1.52 - 1.47 (m, 2H), 1.42 - 1.37 (m, 2H). LC/MS (ESI) (m/z): 612 $[M+H]^+$. |

(continued)

| Example | Structure and name | Analysis data |
|---|---|---|
| **Example 280** | 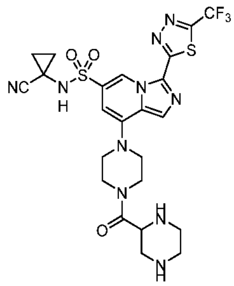 **N-(1-cyanocyclopropyl)-8-(4-(piperazine-2-carbonyl)piperazin-1-yl)-3-(5-(trifluoromethyl)-1,3,4-thiadiazol-2-yl)imidazo[1,5-a]pyridine-6-sulfonamide** | $^{1}H$ NMR(400 MHz, $CD_3OD$) δ 9.89 (s, 1H), 8.47 (s, 1H), 7.96 (s, 1H), 6.81 (s, 1H), 4.19 - 4.15 (m, 1H), 4.03 - 3.96 (m, 2H), 3.86 - 3.79 (m, 2H), 3.51 - 3.44 (m, 3H), 3.38 - 3.34 (m, 1H), 3.27 - 3.24 (m, 1H), 3.18 - 3.11 (m, 3H), 3.07 - 3.01 (m, 2H), 1.57 - 1.52 (m, 2H), 1.47 - 1.43 (m, 2H). LC/MS (ESI) (m/z): 611 $[M+H]^+$. |
| **Example 281** | 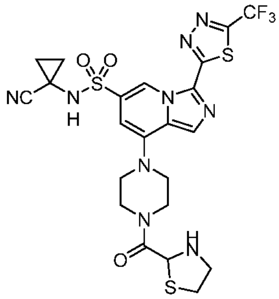 **N-(1-cyanocyclopropyl)-8-(4-(thiazolidine-2-carbonyl)piperazin-1-yl)-3-(5-(trifluoromethyl)-1,3,4-thiadiazol-2-yl)imidazo[1,5-a]pyridine-6-sulfonamide** | $^{1}H$ NMR (400 MHz, DMSO) δ 9.70 - 9.65 (m, 1H), 9.51 (s, 1H), 8.18 (s, 1H), 6.73 (s, 1H), 5.26 (s, 1H), 3.90 - 3.61 (m, 6H), 3.42 - 3.39 (m, 2H), 3.07 - 2.97 (m, 2H), 2.75 - 2.66 (m, 2H), 1.51 - 1.46 (m, 2H), 1.39 (t, *J* = 6.4 Hz, 2H).LC/MS (ESI) (m/z): 614 [M+H]+. |
| **Example 282** | 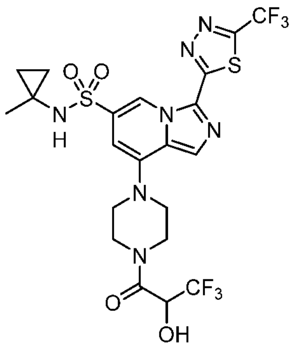 **N-(1-methylcyclopropyl)-8-(4-(3,3,3-trifluoro-2-hydroxypropanoyl)piperazin-1-yl)-3-(5-(trifluoromethyl)-1,3,4-thiadiazol-2-yl) imidazo[1,5-a]pyridine-6-sulfonamide** | $^{1}H$ NMR(400 MHz, DMSO-$d_6$) δ 9.59 (s, 1H), 8.15 (s, 1H), 6.87 (d, *J* = 8.4 Hz, 1H), 6.74 (s, 1H), 5.26 - 5.16 (m, 1H), 3.91 - 3.73 (m, 4H), 3.41 - 3.34 (m, 4H), 1.16 (s, 3H), 0.76 - 0.70 (m, 2H), 0.49 - 0.43 (m, 2H). LC/MS (ESI) (m/z): 614 $[M+H]^+$. |
| **Example 283** | 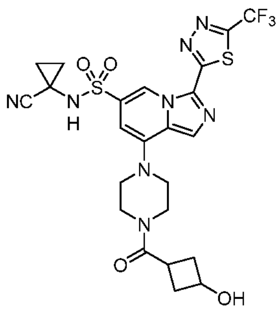 **N-(1-cyanocyclopropyl)-8-(4-(3-hydroxycyclobutane-1-carbonyl)piperazin-1-yl)-3-(5-(trifluoromethyl)-1,3,4-thiadiazol-2-yl) imidazo[1,5-a]pyridine-6-sulfonamide** | $^{1}H$ NMR (400 MHz, $CDCl_3$) δ 9.99 (s, 1H), 7.75 (s, 1H), 6.73 (s, 1H), 6.05 (s, 1H), 4.33 - 4.18 (m, 1H), 3.91 (s, 2H), 3.69 (s, 2H), 3.36 (d, J = 4.2 Hz, 4H), 2.89 - 2.79 (m, 1H), 2.66 - 2.57 (m, 2H), 2.31 - 2.23 (m, 2H), 1.73 - 1.70 (m, 2H), 1.53 - 1.50 (m, 2H). LC/MS (ESI) (m/z): 597$[M+H]^+$. |

(continued)

| Example | Structure and name | Analysis data |
|---|---|---|
| **Example 284** | 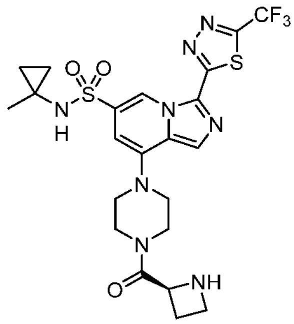<br>**(S)-8-(4-(azetidine-2-carbonyl)piperazin-1-yl)-N-(1-methylcyclopropyl)-3-(5-(trifluoromethyl)-1,3,4-thiadiazol-2-yl)imidazo[1,5-a]pyridine-6-sulfonamide** | $^1$H NMR (400 MHz, DMSO-$d_6$) δ 9.62 (s, 1H), 9.40 (s, 1H), 8.49 (s, 1H), 8.13 (s, 1H), 6.74 (s, 1H), 5.49 - 5.36 (m, 1H), 4.05 - 3.96 (m, 1H), 3.88 - 3.82 (m, 1H), 3.81 - 3.74 (m, 2H), 3.45 - 3.28 (m, 6H), 2.86 - 2.76 (m, 1H), 2.62 - 2.53 (m, 1H), 1.17 (s, 3H), 0.77 - 0.70 (m, 2H), 0.51 - 0.43 (m, 2H). LC/MS (ESI) (m/z): 571 [M+H]$^+$. |
| **Example 285** | 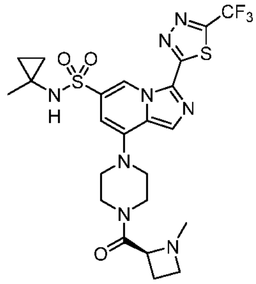<br>**(S)-8-(4-(1-methylazetidine-2-carbonyl)piperazin-1-yl)-N-(1-methylcyclopropyl)-3-(5-(trifluoromethyl)-1,3,4-thiadiazol-2-yl) imidazo[1,5-a]pyridine-6-sulfonamide** | $^1$H NMR (400 MHz, DMSO-$d_6$) δ 9.88 (s, 1H), 9.60 (s, 1H), 8.14 (s, 1H), 6.74 (s, 1H), 5.49 - 5.40 (m, 1H), 4.07 - 3.95 (m, 2H), 3.86 - 3.79 (m, 2H), 3.38 - 3.33 (m, 4H), 2.84 (s, 3H), 2.82 - 2.65 (m, 2H), 2.56 - 2.52 (m, 2H), 1.17 (s, 3H), 0.77 - 0.72 (m, 2H), 0.48 - 0.42 (m, 2H). LC/MS (ESI) (m/z): 585 [M+H]$^+$. |
| **Example 286** | 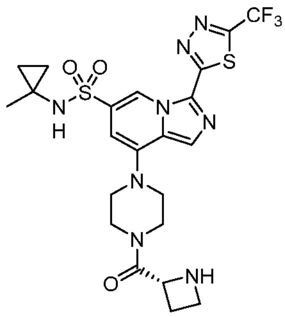<br>**(R)-8-(4-(azetidine-2-carbonyl)piperazin-1-yl)-N-(1-methylcyclopropyl)-3-(5-(trifluoromethyl)-1,3,4-thiadiazol-2-yl)imidazo[1,5-a]pyridine-6-sulfonamide** | $^1$H NMR (400 MHz, MeOD) δ 9.79 (s, 1H), 7.92 (s, 1H), 6.76 (d, *J* = 0.9 Hz, 1H), 5.51 - 5.44 (m, 1H), 4.21 - 4.13 (m, 1H), 4.00 - 3.87 (m, 3H), 3.65 - 3.56 (m, 2H), 3.46 - 3.35 (m, 4H), 3.02 - 2.91 (m, 1H), 2.71 - 2.62 (m, 1H), 1.26 (s, 3H), 0.85 - 0.79 (m, 2H), 0.55 - 0.45 (m, 2H). LC/MS (ESI) (m/z): 571 [M+H]$^+$. |
| **Example 287** | 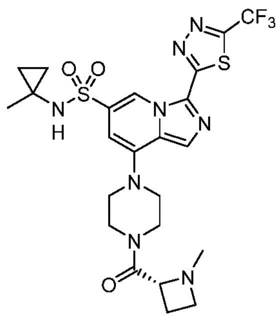<br>**(R)-8-(4-(1-methylazetidine-2-carbonyl)piperazin-1-yl)-N-(1-methylcyclopropyl)-3-(5-(trifluoromethyl)-1,3,4-thiadiazol-2-yl) imidazo[1,5-a]pyridine-6-sulfonamide** | $^1$H NMR (400 MHz, MeOD) δ 9.80 (s, 1H), 793 (s, 1H), 6.76 (d, *J* = 0.9 Hz, 1H), 5.45 - 5.37 (m, 1H), 4.17 - 4.02 (m, 2H), 3.98 - 3.86 (m, 2H), 3.63 - 3.54 (m, 2H), 3.45 - 3.36 (m, 4H), 2.95 (s, 3H), 2.93 - 2.85 (m, 1H), 2.69 - 2.58 (m, 1H), 1.27 (s, 3H), 0.85 - 0.80 (m, 2H), 0.54 - 0.48 (m, 2H). LC/MS (ESI) (m/z): 585 [M+H]$^+$. |

(continued)

| Example | Structure and name | Analysis data |
|---|---|---|
| **Example 288** | 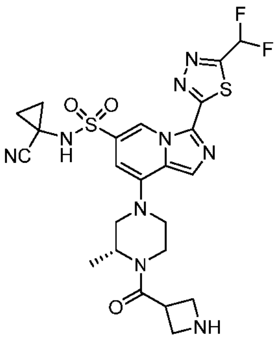 **(R)-8-(4-(azetidine-3-carbonyl)-3-methylpiperazin-1-yl)-N-(1-cyanocyclopropyl)-3-(5-(difluoromethyl)-1,3,4-thiadiazol-2-yl)imidazo[1,5-a]pyridine-6-sulfonamide** | $^1$H NMR (400 MHz, MeOD) δ 9.93 (s, 1H), 7.89 (d, *J* = 4.1 Hz, 1H), 7.34 (t, *J* = 53.5 Hz, 1H), 6.77 (d, *J= 4.2* Hz, 1H), 4.43 - 4.27 (m, 4H), 4.17 - 4.08 (m, 1H), 3.86 - 3.33 (m, 5H), 3.17 - 3.11 (m, 1H), 3.05 - 2.92 (m, 1H), 1.60 - 1.53 (m, 3H), 1.50 - 1.42 (m, 4H). LC/MS (ESI) (m/z): 577 [M+H]. |
| **Example 289** | 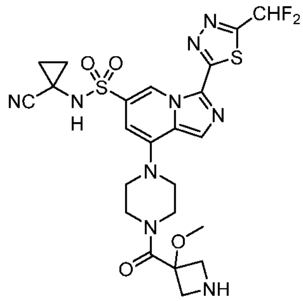 **N-(1-cyanocyclopropyl)-3-(5-(difluoromethyl)-1,3,4-thiadiazol-2-yl)-8-(4-(3-methoxyazetidine-3-carbonyl)piperazin-1-yl)imidazo [1,5-a]pyridine-6-sulfonamide** | $^1$H NMR(400 MHz, $CD_3OD$) δ 9.91 (s, 1H), 8.49 (s, 1H), 7.92 (s, 1H), 7.34 (t, J= 53.5 Hz, 1H), 6.79 (s, 1H), 4.54 (d, *J* = 11.9 Hz, 2H), 4.12 (d, *J* = 12.0 Hz, 2H), 3.98 - 3.88 (m, 2H), 3.79 - 3.70 (m, 2H), 3.48 - 3.41 (m, 4H), 3.34 (s, 3H), 1.57 - 1.50 (m, 2H), 1.48 - 1.42 (m, 2H). LC/MS (ESI) (m/z): 594 $[M+H]^+$. |
| **Example 290** | 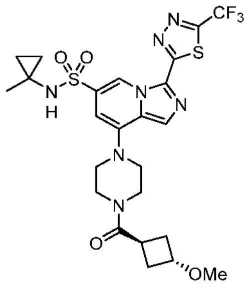 **8-(4-((1r,3r)-3-methoxycyclobutane-1-carbonyl)piperazin-1-yl)-N-(1-methylcyclopropyl)-3-(5-(trifluoromethyl)-1,3,4-thiadiazol-2-yl)imidazo [1,5-a]pyridine-6-sulfonamide** | $^1$H NMR(400 MHz, $CDCl_3$) δ 9.89 (s, 1H), 7.74 (s, 1H), 6.70 (s, 1H), 5.18 (s, 1H), 4.08 - 4.02 (m, 1H), 3.96 - 3.90 (m, 2H), 3.69 - 3.63 (m, 2H), 3.36 - 3.32 (m, 4H), 3.29 (s, 3H), 2.65 - 2.59 (m, 2H), 2.31 - 2.24 (m, 2H), 1.41 (s, 3H), 0.96 - 0.92 (m, 2H), 0.65 - 0.61 (m, 2H). LC/MS (ESI) (m/z): 600 $[M+H]^+$. |
| **Example 291** | 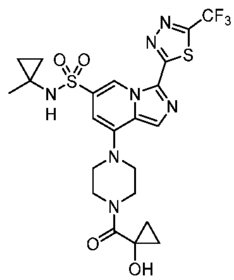 **8-(4-(1-hydroxycyclopropane-1-carbonyl)piperazin-1-yl)-N-(1-methylcyclopropyl)-3-(5-(trifluoromethyl)-1,3,4-thiadiazol-2-yl) imidazo[1,5-a]pyridine-6-sulfonamide** | $^1$H NMR(400 MHz, $CDCl_3$) δ 9.89 (s, 1H), 7.76 (s, 1H), 6.71 (s, 1H), 5.49 (s, 1H), 4.12 - 3.95 (m, 4H), 3.43 - 3.37 (m, 4H), 1.40 (s, 3H), 1.23 - 1.18 (m, 2H), 1.10 - 1.05 (m, 2H), 0.96 - 0.92 (m, 2H), 0.64 - 0.60 (m, 2H). LC/MS (ESI) (m/z): 572 $[M+H]^+$. |

(continued)

| Example | Structure and name | Analysis data |
|---|---|---|
| **Example 292** | 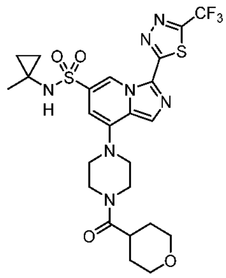 **N-(1-methylcyclopropyl)-8-(4-(tetrahydro-2H-pyran-4-carbonyl) piperazin-1-yl)-3-(5-(trifluoromethyl)-1,3,4-thiadiazol-2-yl)imidazo[1,5-a]pyridine-6-sulfonamide** | $^1$H NMR (400 MHz, $CDCl_3$) δ 9.90 (s, 1H), 7.75 (s, 1H), 6.71 (s, 1H), 5.15 (s, 1H), 4.08 (d, *J* = 10.8 Hz, 2H), 3.95 - 3.81 (m, 4H), 3.54 - 3.48 (m, 2H), 3.40 - 3.33 (m, 4H), 2.87 - 2.79 (m, 1H), 2.04 - 1.95 (m, 2H), 1.71 - 1.67 (m, 2H), 1.41 (s, 3H), 0.97 - 0.92 (m, 2H), 0.66 - 0.61 (m, 2H).LC/MS (ESI) (m/z): 600 $[M+H]^+$. |
| **Example 293** | 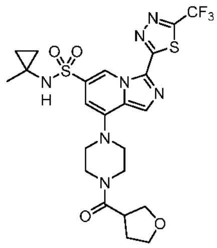 **N-(1-methylcyclopropyl)-8-(4-(tetrahydrofuran-3-carbonyl)piperazin-1-yl)-3-(5-(trifluoromethyl)-1,3,4-thiadiazol-2-yl)imidazo [1,5-a]pyridine-6-sulfonamide** | $^1$H NMR (400 MHz, $CDCl_3$) δ 9.87 (s, 1H), 7.72 (s, 1H), 6.70 (s, 1H), 5.24 (s, 1H), 4.08 - 4.03 (m, 1H), 3.97 - 3.89 (m, 5H), 3.81 (s, 2H), 3.39 - 3.29 (m, 5H), 2.33 - 2.25 (m, 1H), 2.18 - 2.10 (m, 1H), 1.39 (s, 3H), 0.94 - 0.90 (m, 2H), 0.63 - 0.58 (m, 2H). LC/MS (ESI) (m/z): 586 $[M+H]^+$. |
| **Example 294** | 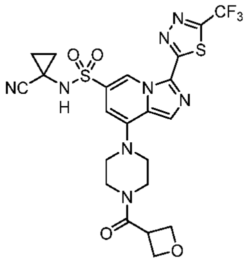 **N-(1-cyanocyclopropyl)-8-(4-(oxetane-3-carbonyl)piperazin-1-yl)-3-(5-(trifluoromethyl)-1,3,4-thiadiazol-2-yl)imidazo[1,5-a]pyridine-6-sulfonamide** | $^1$H NMR (400 MHz, DMSO-$d_6$) δ 9.58 (s, 1H), 8.45 (s, 1H), 8.10 (s, 1H), 6.71 (d, J = 0.9 Hz, 1H), 4.77 - 4.68 (m, 4H), 4.20 (dd, *J* = 15.5, 8.4 Hz, 1H), 3.81 - 3.70 (m, 2H), 3.48 - 3.41 (m, 2H), 3.31 - 3.25 (m, 4H), 1.15 (s, 3H), 0.75 - 0.68 (m, 2H), 0.48 - 0.41 (m, 2H). LC/MS (ESI) (m/z): 572 $[M+H]^+$. |
| **Example 295** | 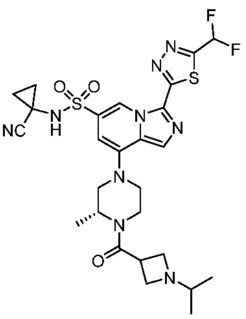 **(R)-N-(1-cyanocyclopropyl)-3-(5-(difluoromethyl)-1,3,4-thiadiazol-2-yl)-8-(4-(1-isopropylazetidine-3-carbonyl)-3-methylpiperazin-1-yl)imidazo[1,5-a]pyridine-6-sulfonamide** | $^1$H NMR (400 MHz, MeOD) δ 9.92 (s, 1H), 8.48 (s, 1H), 7.89 (d, J = 3.6 Hz, 1H), 7.34 (t, J = 53.6 Hz, 1H), 6.77 (d, J = 4.1 Hz, 1H), 4.18 - 3.40 (m, 10H), 3.16 - 3.11 (m, 1H), 3.05 - 2.90 (m, 2H), 1.61 - 1.53 (m, 3H), 1.50 - 1.41 (m, 4H), 1.17 - 1.12 (m, 6H). LC/MS (ESI) (m/z): 620 $[M+H]^+$. |

(continued)

| Example | Structure and name | Analysis data |
|---|---|---|
| **Example 296** | 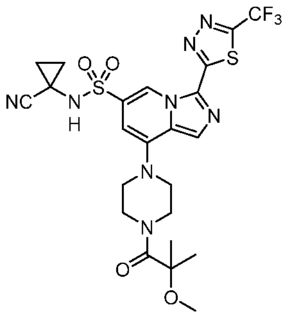 **N-(1-cyanocyclopropyl)-8-(4-(2-methoxy-2-methylpropanoyl)piperazin-1-yl)-3-(5-(trifluoromethyl)-1,3,4-thiadiazol-2-yl)imidazo [1,5-a]pyridine-6-sulfonamide** | $^1$H NMR (400 MHz, $CDCl_3$) δ 10.02 (s, 1H), 7.79 (s, 1H), 6.74 (s, 1H), 5.71 (s, 1H), 4.36 - 4.25 (m, 2H), 4.01 - 3.91 (m, 2H), 3.43 - 3.39 (m, 4H), 3.31 (s, 3H), 1.76 - 1.72 (m, 2H), 1.55 - 1.51 (m, 8H). LC/MS (ESI) (m/z): 599 $[M+H]^+$. |
| **Example 297** | 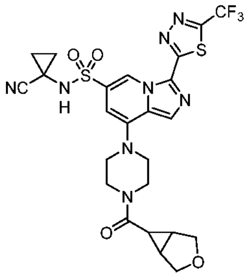 **8-(4-(3-oxabicyclo [3.1.0]hexane-6-carbonyl)piperazin-1-yl)-N-(1-cyanocyclopropyl)-3-(5-(trifluoromethyl)-1,3,4-thiadiazol-2-yl)imidazo[1,5-a]pyridine-6-sulfonamide** | $^1$H NMR (400 MHz, $CDCl_3$) δ 10.01 (s, 1H), 7.79 (s, 1H), 6.74 (s, 1H), 6.02 (s, 1H), 3.99 - 3.96 (m, 2H), 3.95 - 3.90 (m, 4H), 3.84 - 3.80 (m, 2H), 3.48 - 3.36 (m, 4H), 2.26 (s, 2H), 1.82 - 1.79 (m, 1H), 1.76 - 1.72 (m, 2H), 1.56 - 1.53 (m, 2H). LC/MS (ESI) (m/z): 609 $[M+H]^+$ |
| **Example 298** | 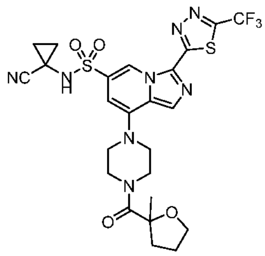 **N-(1-cyanocyclopropyl)-8-(4-(2-methyltetrahydrofuran-2-carbonyl)piperazin-1-yl)-3-(5-(trifluoromethyl)-1,3,4-thiadiazol-2-yl) imidazo[1,5-a]pyridine-6-sulfonamide** | $^1$H NMR (400 MHz, $CDCl_3$) δ 9.98 (s, 1H), 7.76 (s, 1H), 6.71 (s, 1H), 5.93 (s, 1H), 4.07 - 3.94 (m, 3H), 3.89 - 3.71 (m, 2H), 3.47 - 3.26 (m, 4H), 2.88 - 2.81 (m, 1H), 1.96 - 1.86 (m, 2H), 1.76 - 1.62 (m, 4H), 1.59 (s, 3H), 1.54 - 1.52 (m, 2H). LC/MS (ESI) (m/z): 611 $[M+H]^+$. |
| **Example 299** | 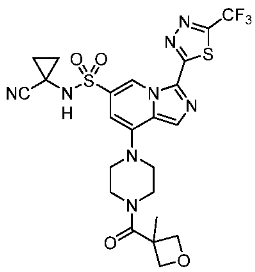 **N-(1-cyanocyclopropyl)-8-(4-(3-methyloxetane-3-carbonyl)piperazin-1-yl)-3-(5-(trifluoromethyl)-1,3,4-thiadiazol-2-yl)imidazo [1,5-a]pyridine-6-sulfonamide** | $^1$H NMR (400 MHz, DMSO-$d_6$) δ 9.67 (s, 1H), 9.50 (s, 1H), 8.13 (s, 1H), 6.73 (s, 1H), 4.91 - 4.85 (m, 2H), 4.36 - 4.30 (m, 2H), 3.81 - 3.72 (m, 2H), 3.40 - 3.35 (m, 4H), 3.31 - 3.24 (m, 2H), 1.63 - 1.60 (m, 3H), 1.52 - 1.47 (m, 2H), 1.42 - 1.36 (m, 2H). LC/MS (ESI) (m/z): 597 $[M+H]^+$. |

(continued)

| Example | Structure and name | Analysis data |
|---|---|---|
| **Example 300** | 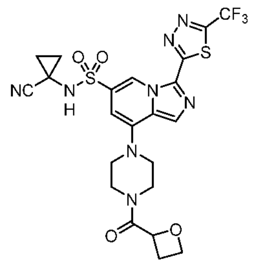 **N-(1-cyanocyclopropyl)-8-(4-(oxetane-2-carbonyl)piperazin-1-yl)-3-(5-(trifluoromethyl)-1,3,4-thiadiazol-2-yl)imidazo[1,5-a]pyridine-6-sulfonamide** | $^1$H NMR (400 MHz, DMSO-$d_6$) δ 9.66 (s, 1H), 9.48 (s, 1H), 8.15 (s, 1H), 6.71 (s, 1H), 5.51 - 5.45 (m, 1H),4.61 - 4.55 (m, 1H), 4.47 - 4.41 (m, 1H), 3.82 - 3.72 (m, 2H), 3.65 - 3.54 (m, 2H), 3.44 - 3.34 (m, 4H), 2.97 - 2.86 (m, 1H), 2.82 - 2.72 (m, 1H), 1.53 - 1.46 (m, 2H), 1.41 - 1.34 (m, 2H). LC/MS (ESI) (m/z): 583 [M+H]$^+$. |
| **Example 301** | 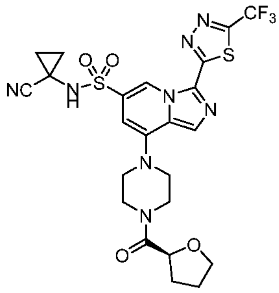 **(S)-N-(1-cyanocyclopropyl)-8-(4-(tetrahydrofuran-2-carbonyl)piperazin-1-yl)-3-(5-(trifluoromethyl)-1,3,4-thiadiazol-2-yl)imidazo [1,5-a]pyridine-6-sulfonamide** | $^1$H NMR (400 MHz, DMSO-$d_6$) δ 9.66 (s, 1H), 9.50 (s, 1H), 8.14 (s, 1H), 6.72 (s, 1H), 4.80 - 4.69 (m, 1H), 3.90 - 3.69 (m, 6H), 3.42 - 3.36 (m, 2H), 3.32 - 3.24 (m, 2H), 2.17 - 1.98 (m, 2H), 1.93 - 1.80 (m, 2H), 1.52 - 1.44 (m, 2H), 1.44 - 1.36 (m, 2H). LC/MS (ESI) (m/z): 597 [M+H]$^+$. |
| **Example 302** | 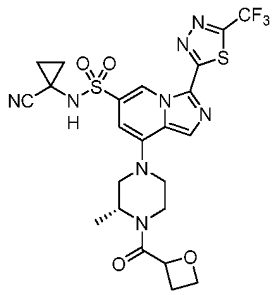 **N-(1-cyanocyclopropyl)-8-((3R)-3-methyl-4-(oxetane-2-carbonyl)piperazin-1-yl)-3-(5-(trifluoromethyl)-1,3,4-thiadiazol-2-yl) imidazo[1,5-a]pyridine-6-sulfonamide** | $^1$H NMR (400 MHz, DMSO-$d_6$) δ 9.65 (s, 1H), 9.50 (s, 1H), 8.03 (s, 1H), 6.77 - 6.66 (m, 1H), 5.54 - 5.42 (m, 1H), 4.86 - 4.44 (m, 2H), 4.43 - 3.97 (m, 2H), 3.81 - 3.59 (m, 3H), 3.14 - 3.04 (m, 1H), 2.96 - 2.70 (m, 3H), 1.52 - 1.47 (m, 2H), 1.44 - 1.30 (m, 5H). LC/MS (ESI) (m/z): 597 [M+H]$^+$. |
| **Example 303** | 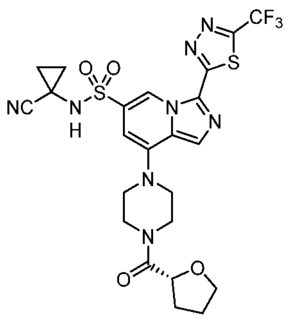 **(R)-N-(1-cyanocyclopropyl)-8-(4-(tetrahydrofuran-2-carbonyl)piperazin-1-yl)-3-(5-(trifluoromethyl)-1,3,4-thiadiazol-2-yl)imidazo [1,5-a]pyridine-6-sulfonamide** | $^1$H NMR (400 MHz, $CDCl_3$) δ 10.01 (s, 1H), 7.77 (s, 1H), 6.79 (s, 1H), 6.24 (s, 1H), 4.73 - 4.66 (m, 1H), 4.08 - 3.80 (m, 6H), 3.48 - 3.29 (m, 4H), 2.44 - 2.34 (m, 1H), 2.15 - 1.96 (m, 3H), 1.75 - 1.71 (m, 2H), 1.54 - 1.51 (m, 2H).LC/MS (ESI) (m/z): 597 [M+H]$^+$. |

(continued)

| Example | Structure and name | Analysis data |
|---|---|---|
| **Example 304** | 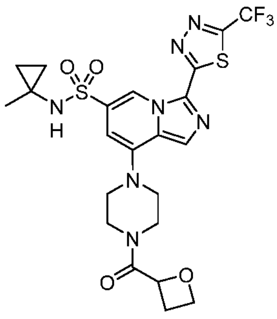 **N-(1-methylcyclopropyl)-8-(4-(oxetane-2-carbonyl)piperazin-1-yl)-3-(5-(trifluoromethyl)-1,3,4-thiadiazol-2-yl)imidazo[1,5-a]pyridine-6-sulfonamide** | $^1$H NMR (400 MHz, DMSO-$d_6$) δ 9.58 (s, 1H), 8.45 (s, 1H), 8.12 (s, 1H), 6.73 (s, 1H), 5.48 (t, *J* = 7.6 Hz, 1H), 4.62 - 4.55 (m, 1H), 4.47 - 4.41 (m, 1H), 3.82 - 3.73 (m, 2H), 3.64 - 3.54 (m, 2H), 3.28 - 3.13 (m, 4H), 2.95 - 2.80 (m, 2H), 1.16 (s, 3H), 0.76 - 0.71 (m, 2H), 0.49 - 0.44 (m, 2H). LC/MS (ESI) (m/z): 572 [M+H]$^+$. |
| **Example 305** | 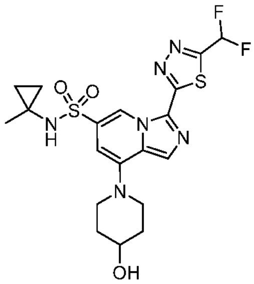 **3-(5-(difluoromethyl)-1,3,4-thiadiazol-2-yl)-8-(4-hydroxypiperidin-1-yl)-N-(1-methylcyclopropyl)imidazo[1,5-a]pyridine-6-sulfonamide** | $^1$H NMR (400 MHz, DMSO-$d_6$) δ 9.56 (s, 1H), 8.42 (s, 1H), 7.96 (s, 1H), 7.68 (t, *J* = 53.2 Hz, 1H), 6.67 (s, 1H), 4.82 (d, *J* = 4.0 Hz, 1H), 3.82 - 3.74 (m, 1H), 3.64 - 3.56 (m, 2H), 3.13 - 3.05 (m, 2H), 2.00 - 1.92 (m, 2H), 1.70 - 1.61 (m, 2H), 1.15 (s, 3H), 0.76 - 0.71 (m, 2H), 0.48 - 0.43 (m, 2H). LC/MS (ESI) (m/z): 485 [M+H] $^+$. |
| **Example 306** | 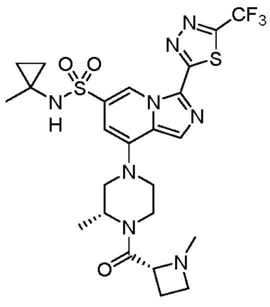 **8-((R)-3-methyl-4-((R)-1-methylazetidine-2-carbonyl)piperazin-1-yl)-N-(1-methylcyclopropyl)-3-(5-(trifluoromethyl)-1,3,4-thiadiazol-2-yl)imidazo[1,5-a]pyridine-6-sulfonamide** | $^1$H NMR (400 MHz, DMSO-$d_6$) δ 9.58 (s, 1H), 8.48 (s, 1H), 8.00 (s, 1H), 6.72 (s, 1H), 4.76 - 4.32 (m, 1H), 3.87 - 3.77 (m, 1H), 3.71 - 3.60 (m, 2H), 3.54 - 3.46 (m, 2H), 3.20 - 3.09 (m, 2H), 3.07 - 3.03 (m, 2H), 2.91 - 2.86 (m, 1H), 2.77 - 2.67 (m, 4H), 1.41 (s, 3H), 1.17 (s, 3H), 0.76 - 0.68 (m, 2H), 0.51 - 0.43 (m, 2H). LC/MS (ESI) (m/z): 599[M+H]$^+$ |
| **Example 307** | 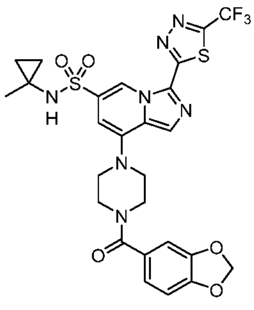 **8-(4-(benzo[d][1,3]dioxole-5-carbonyl)piperazin-1-yl)-N-(1-methylcyclopropyl)-3-(5-(trifluoromethyl)-1,3,4-thiadiazol-2-yl) imidazo[1,5-a]pyridine-6-sulfonamide** | $^1$H NMR (400 MHz, DMSO-$d_6$) δ 9.58 (s, 1H), 8.45 (s, 1H), 8.10 (s, 1H), 7.07 (s, 1H), 7.00 (s, 2H), 6.74 (s, 1H), 6.13 - 6.08 (m, 2H), 3.97 - 3.64 (m, 4H), 3.45 - 3.36 (m, 4H), 1.16 (s, 3H), 0.78 - 0.70 (m, 2H), 0.50 - 0.42 (m, 2H). LC/MS (ESI) (m/z): 636 [M+H]$^+$. |

(continued)

| Example | Structure and name | Analysis data |
|---|---|---|
| **Example 308** | 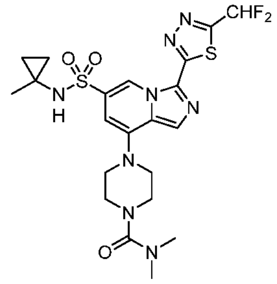<br>**4-(3-(5-(difluoromethyl)-1,3,4-thiadiazol-2-yl)-6-(N-(1-methylcyclopropyl)sulfamoyl)imidazo [1,5-a]pyridin-8-yl)-N,N-dimethylpiperazine-1-carboxamide** | $^1$H NMR (400 MHz, DMSO-$d_6$) δ 9.60 (s, 1H), 8.42 (s, 1H), 8.05 (s, 1H), 7.68 (t, *J* = 53.3 Hz, 1H), 6.71 (s, 1H), 3.60 - 3.37 (m, 8H), 2.89 - 2.77 (m, 6H), 1.16 (s, 3H), 0.81 - 0.69 (m, 2H), 0.55 - 0.40 (m, 2H). LC/MS (ESI) (m/z): 541 $[M+H]^+$. |
| **Example 309** | 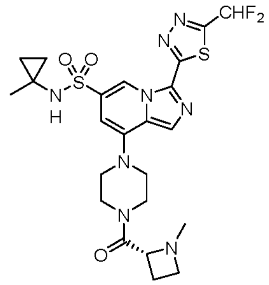<br>**(R)-3-(5-(difluoromethyl)-1,3,4-thiadiazol-2-yl)-8-(4-(1-methylazetidine-2-carbonyl)piperazin-1-yl)-N-(1-methylcyclopropyl)imidazo[1,5-a]pyridine-6-sulfonamide** | $^1$H NMR (400 MHz, DMSO-$d_6$) δ 962 (s, 1H), 8.46 (s, 1H), 8.09 (s, 1H), 7.69 (t, *J* = 53.6 Hz, 1H), 6.70 (s, 1H), 5.47 - 5.37 (m, 1H), 4.01 - 3.92 (m, 2H), 3.85 - 3.75 (m, 2H), 3.51 - 3.47 (m, 2H), 3.35 - 3.28 (m, 5H), 2.83 (s, 3H), 2.80 - 2.73 (m, 1H), 1.17 (s, 3H), 0.78 - 0.68 (m, 2H), 0.49 - 0.42 (m, 2H). LC/MS (ESI) (m/z): 567 $[M+H]^+$. |
| **Example 310** | 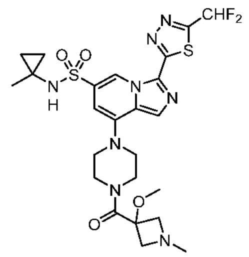<br>**3-(5-(difluoromethyl)-1,3,4-thiadiazol-2-yl)-8-(4-(3-methoxy-1-methylazetidine-3-carbonyl)piperazin-1-yl)-N-(1-methylcyclopropyl)imidazo[1,5-a]pyridine-6-sulfonamide** | $^1$H NMR(400 MHz, $CD_3OD$) δ 9.83 (s, 1H), 7.91 (s, 1H), 7.33 (t, *J* = 53.5 Hz, 1H), 6.74 (s, 1H), 4.22 - 4.16 (m, 2H), 3.95 - 3.90 (m, 2H), 3.86 - 3.81 (m, 2H), 3.78 - 3.74 (m, 2H), 3.41 - 3.37 (m, 4H), 3.32 (s, 3H), 2.67 (s, 3H), 1.26 (s, 3H), 0.85 - 0.81 (m, 2H), 0.53 - 0.48 (m, 2H). LC/MS (ESI) (m/z): 597 $[M+H]^+$. |
| **Example 311** | 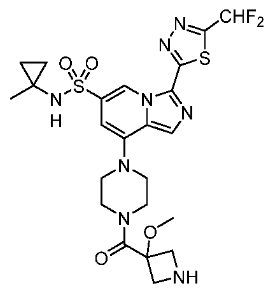<br>**3-(5-(difluoromethyl)-1,3,4-thiadiazol-2-yl)-8-(4-(3-methoxyazetidine-3-carbonyl)piperazin-1-yl)-N-(1-methylcyclopropyl)imidazo [1,5-a]pyridine-6-sulfonamide** | $^1$H NMR(400 MHz, $CD_3OD$) δ 9.83 (s, 1H), 7.91 (s, 1H), 7.34 (t, *J* = 53.5 Hz, 1H), 6.74 (s, 1H), 4.57 (d, *J* = 11.5 Hz, 2H), 4.14 (d, *J* = 11.4 Hz, 2H), 3.97 - 3.91 (m, 2H), 3.79 - 3.73 (m, 2H), 3.43 - 3.38 (m, 4H), 3.35 (s, 3H), 1.27 (s, 3H), 0.86 - 0.80 (m, 2H), 0.54 - 0.48 (m, 2H). LC/MS (ESI) (m/z): 583 $[M+H]^+$. |

(continued)

| Example | Structure and name | Analysis data |
|---|---|---|
| **Example 312** | 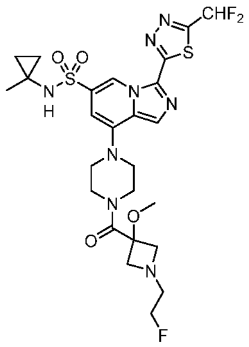 **3-(5-(difluoromethyl)-1,3,4-thiadiazol-2-yl)-8-(4-(1-(2-fluoroethyl)-3-methoxyazetidine-3-carbonyl)piperazin-1-yl)-N-(1-methylcyclopropyl)imidazo[1,5-a]pyridine-6-sulfonamide** | $^1$H NMR(400 MHz, $CD_3OD$) δ 9.82 (s, 1H), 7.91 (s, 1H), 7.33 (t, *J* = 53.6 Hz, 1H), 6.73 (d, *J* = 0.9 Hz, 1H), 4.58 - 4.54 (m, 1H), 4.46 - 4.42 (m, 1H), 3.95 - 3.90 (m, 4H), 3.79 - 3.75 (m, 2H), 3.60 - 3.56 (m, 2H), 3.40 - 3.36 (m, 4H), 3.28 (s, 3H), 2.97 - 2.93 (m, 1H), 2.90 - 2.86 (m, 1H), 1.25 (s, 3H), 0.84 - 0.80 (m, 2H), 0.52 - 0.48 (m, 2H). LC/MS (ESI) (m/z): 629 $[M+H]^+$. |
| **Example 313** | 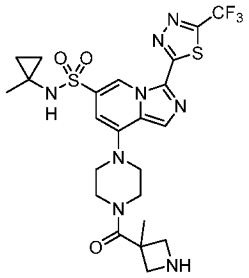 **8-(4-(3-methylazetidine-3-carbonyl)piperazin-1-yl)-N-(1-methylcyclopropyl)-3-(5-(trifluoromethyl)-1,3,4-thiadiazol-2-yl)imidazo [1,5-a]pyridine-6-sulfonamide** | $^1$H NMR (400 MHz, $CD_3OD$) δ 9.83 (s, 1H), 7.95 (s, 1H), 6.79 (s, 1H), 4.65 - 4.34 (m, 2H), 4.04 - 3.82 (m, 4H), 3.55 - 3.38 (m, 6H), 1.73 (s, 3H), 1.29 (s, 3H), 0.87 - 0.77 (m, 2H), 0.57 - 0.48 (m, 2H). LC/MS (ESI) (m/z): 585 $[M+H]^+$. |
| **Example 314** | 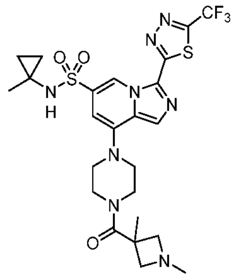 **8-(4-(1,3-dimethylazetidine-3-carbonyl)piperazin-1-yl)-N-(1-methylcyclopropyl)-3-(5-(trifluoromethyl)-1,3,4-thiadiazol-2-yl) imidazo[1,5-a]pyridine-6-sulfonamide** | $^1$H NMR (400 MHz, $CD_3OD$) δ 9.82 (s, 1H), 7.95 (s, 1H), 6.78 (s, 1H), 3.95 - 3.85 (m, 4H), 3.75 - 3.70 (m, 2H), 3.57 - 3.49 (m, 2H), 3.43 - 3.37 (m, 4H), 2.60 (s, 3H), 1.68 (s, 3H), 1.28 (s, 3H), 0.87 - 0.81 (m, 2H), 0.55 - 0.50 (m, 2H). LC/MS (ESI) (m/z): 599 $[M+H]^+$. |
| **Example 315** | 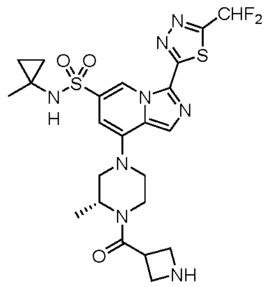 **(R)-8-(4-(azetidine-3-carbonyl)-3-methylpiperazin-1-yl)-3-(5-(difluoromethyl)-1,3,4-thiadiazol-2-yl)-N-(1-methylcyclopropyl)imidazo[1,5-a]pyridine-6-sulfonamide** | $^1$H NMR (400 MHz, DMSO-$d_6$) δ 9.59 (s, 1H), 8.32 (s, 1H), 7.94 (s, 1H), 7.68 (t, *J* = 53.2 Hz, 1H), 6.67 (d, *J* = 6.2 Hz, 1H), 4.74 - 4.34 (m, 1H), 3.97 - 3.83 (m, 4H), 3.70 - 3.61 (m, 2H), 3.28 - 2.78 (m, 6H), 1.44 - 1.31 (m, 3H), 1.16 (s, 3H), 0.78 - 0.67 (m, 2H), 0.50 - 0.40 (m, 2H). LC/MS (ESI) (m/z): 567 $[M+H]^+$. |

(continued)

| Example | Structure and name | Analysis data |
|---|---|---|
| **Example 316** | 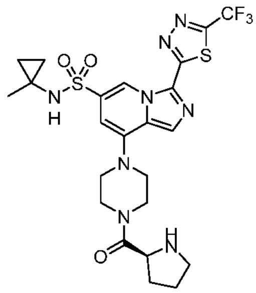 **(S)-N-(1-methylcyclopropyl)-8-(4-prolylpiperazin-1-yl)-3-(5-(trifluoromethyl)-1,3,4-thiadiazol-2-yl)imidazo[1,5-a]pyridine-6-sulfonamide** | $^{1}H$ NMR (400 MHz, $CD_3OD$) δ 9.83 (s, 1H), 7.97 (s, 1H), 6.79 (s, 1H), 4.52 - 4.47 (m, 1H), 4.01 - 3.81 (m, 4H), 3.46 - 3.38 (m, 4H), 3.24 - 3.13 (m, 2H), 2.66 - 2.27 (m, 1H), 2.08 - 1.90 (m, 3H), 1.29 (s, 3H), 0.88 - 0.81 (m, 2H), 0.56 - 0.49 (m, 2H). LC/MS (ESI) (m/z): 585 $[M+H]^+$. |
| **Example 317** | 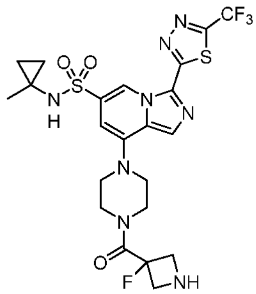 **8-(4-(3-fluoroazetidine-3-carbonyl)piperazin-1-yl)-N-(1-methylcyclopropyl)-3-(5-(trifluoromethyl)-1,3,4-thiadiazol-2-yl)imidazo[1,5-a]pyridine-6-sulfonamide** | $^{1}H$ NMR (400 MHz, MeOD) δ 9.82 (s, 1H), 7.95 (s, 1H), 6.77 (d, J = 1.0 Hz, 1H), 4.82 - 4.77 (m, 2H), 4.58 - 4.45 (m, 2H), 4.02 - 3.89 (m, 2H), 3.81 - 3.68 (m, 2H), 3.45 - 3.37 (m, 4H), 1.27 (s, 3H), 0.88 - 0.76 (m, 2H), 0.60 - 0.42 (m, 2H). LC/MS (ESI) (m/z): 589 $[M+H]^+$. |
| **Example 318** | 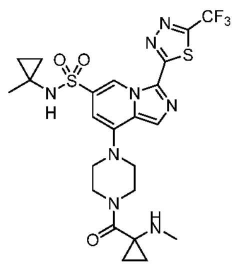 **8-(4-(1-(methylamino)cyclopropane-1-carbonyl)piperazin-1-yl)-N-(1-methylcyclopropyl)-3-(5-(trifluoromethyl)-1,3,4-thiadiazol-2-yl)imidazo[1,5-a]pyridine-6-sulfonamide** | $^{1}H$ NMR (400 MHz, $CD_3OD$) δ 9.82 (s, 1H), 7.97 (s, 1H), 6.78 (s, 1H), 4.09 - 3.90 (m, 4H), 3.45 - 3.36 (m, 4H), 2.42 (s, 3H), 1.28 (s, 3H), 1.07 - 0.99 (m, 2H), 0.90 - 0.82 (m, 4H), 0.57 - 0.50 (m, 2H). LC/MS (ESI) (m/z): 585 $[M+H]^+$. |
| **Example 319** | 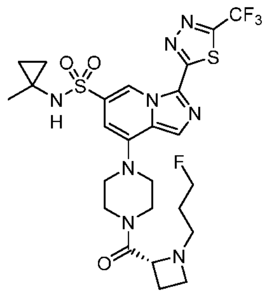 **(R)-8-(4-(1-(3-fluoropropyl)azetidine-2-carbonyl)piperazin-1-yl)-N-(1-methylcyclopropyl)-3-(5-(trifluoromethyl)-1,3,4-thiadiazol-2-yl)imidazo[1,5-a]pyridine-6-sulfonamide** | $^{1}H$ NMR (400 MHz, DMSO-$d_6$) δ 9.60 (s, 1H), 8.48 (s, 1H), 8.14 (s, 1H), 6.74 (s, 1H), 5.68 - 5.29 (m, 1H), 4.60 (t, *J* = 5.7 Hz, 1H), 4.49 (t, *J* = 5.6 Hz, 1H), 4.05 - 3.89 (m, 2H), 3.83 - 3.78 (m, 2H), 3.65 - 3.59 (m, 1H), 3.53 - 3.49 (m, 2H), 3.39 - 3.36 (m, 3H), 3.27 - 3.23 (m, 1H), 3.19 - 3.02 (m, 2H), 2.82 - 2.71 (m, 1H), 2.00 - 1.85 (m, 2H), 1.17 (s, 3H), 0.75 - 0.71 (m, 2H), 0.49 - 0.45 (m, 2H). LC/MS (ESI) (m/z): 631 $[M+H]^+$ |

(continued)

| Example | Structure and name | Analysis data |
|---|---|---|
| **Example 320** | 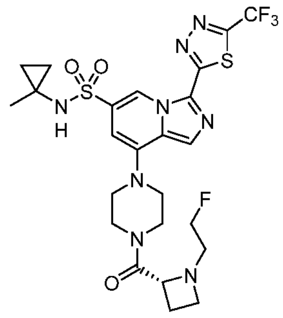 **(R)-8-(4-(1-(2-fluoroethyl)azetidine-2-carbonyl)piperazin-1-yl)-N-(1-methylcyclopropyl)-3-(5-(trifluoromethyl)-1,3,4-thiadiazol-2-yl)imidazo[1,5-a]pyridine-6-sulfonamide** | $^1$H NMR (400 MHz, MeOD) δ 9.82 (s, 1H), 7.94 (s, 1H), 6.77 (s, 1H), 5.58 - 5.43 (m, 1H), 4.80 - 4.77 (m, 1H), 4.69 - 4.65 (m, 1H), 4.17 - 4.07 (m, 2H), 4.00 - 3.94 (m, 1H), 3.91 - 3.85 (m, 1H), 3.68 - 3.53 (m, 4H), 3.44 - 3.36 (m, 4H), 2.91 - 2.83 (m, 1H), 2.73 - 2.63 (m, 1H), 1.27 (s, 3H), 0.85 - 0.81 (m, 2H), 0.53 - 0.49 (m, 2H). LC/MS (ESI) (m/z): 617 $[M+H]^+$. |
| **Example 321** | 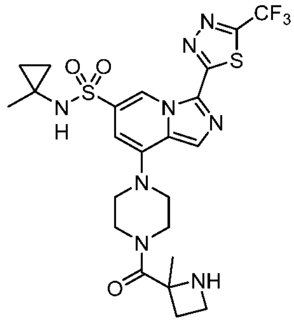 **8-(4-(2-methylazetidine-2-carbonyl)piperazin-1-yl)-N-(1-methylcyclopropyl)-3-(5-(trifluoromethyl)-1,3,4-thiadiazol-2-yl)imidazo[1,5-a]pyridine-6-sulfonamide** | $^1$H NMR (400 MHz, DMSO-$d_6$) δ 9.59 (s, 1H), 8.46 (s, 1H), 8.11 (s, 1H), 6.74 (s, 1H), 3.82 - 3.73 (m, 2H), 3.64 - 3.57 (m, 2H), 3.49 - 3.45 (m, 2H), 3.30 - 3.25 (m, 2H), 3.22 - 3.12 (m, 2H), 2.68 - 2.61 (m, 1H), 2.31 - 2.21 (m, 1H), 1.50 (s, 3H), 1.24 (d, *J* = 5.8 Hz, 1H), 1.20 - 1.11 (m, 3H), 0.76 - 0.69 (m, 2H), 0.51 - 0.42 (m, 2H). LC/MS (ESI) (m/z): 585 $[M+H]^+$. |
| **Example 322** | 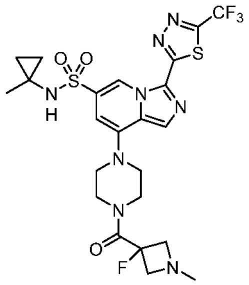 **8-(4-(3-fluoro-1-methylazetidine-3-carbonyl)piperazin-1-yl)-N-(1-methylcyclopropyl)-3-(5-(trifluoromethyl)-1,3,4-thiadiazol-2-yl)imidazo[1,5-a]pyridine-6-sulfonamide** | $^1$H NMR (400 MHz, MeOD) δ 9.82 (s, 1H), 7.95 (s, 1H), 6.77 (s, 1H), 4.81 - 4.77 (m, 2H), 4.70 - 4.60 (m, 2H), 3.97 - 3.89 (m, 2H), 3.78 - 3.72 (m, 2H), 3.44 - 3.39 (m, 4H), 3.05 (s, 3H), 1.27 (s, 3H), 0.86 - 0.79 (m, 2H), 0.54 - 0.49 (m, 2H). LC/MS (ESI) (m/z): 603 $[M+H]^+$. |
| **Example 323** | 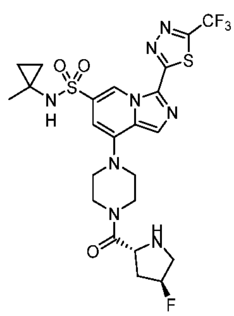 **8-(4-((2R,4S)-4-fluoropyrrolidine-2-carbonyl)piperazin-1-yl)-N-(1-methylcyclopropyl)-3-(5-(trifluoromethyl)-1,3,4-thiadiazol-2-yl)imidazo[1,5-a]pyridine-6-sulfonamide** | $^1$H NMR (400 MHz, MeOD) δ 9.82 (s, 1H), 7.95 (s, 1H), 6.78 (s, 1H), 5.53 (d, *J* = 51.5 Hz, 1H), 5.06 - 4.99 (m, 1H), 4.00 - 3.86 (m, 2H), 3.85 - 3.77 (m, 2H), 3.76 - 3.53 (m, 2H), 3.47 - 3.38 (m, 4H), 3.04 - 2.90 (m, 1H), 2.41 - 2.21 (m, 1H), 1.27 (s, 3H), 0.86 - 0.80 (m, 2H), 0.55 - 0.49 (m, 2H). LC/MS (ESI) (m/z): 603 $[M+H]^+$. |

(continued)

| Example | Structure and name | Analysis data |
|---|---|---|
| **Example 324** | 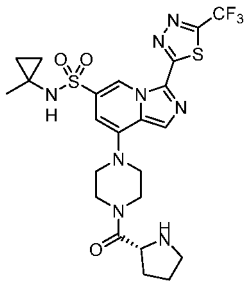 **(R)-N-(1-methylcyclopropyl)-8-(4-prolylpiperazin-1-yl)-3-(5-(trifluoromethyl)-1,3,4-thiadiazol-2-yl)imidazo[1,5-a]pyridine-6-sulfonamide** | $^1$H NMR(400 MHz, $CD_3OD$) δ 9.82 - 9.79 (m, 1H), 7.95 (s, 1H), 6.77 (s, 1H), 4.55 - 4.49 (m, 1H), 4.00 - 3.93 (m, 1H), 3.92 - 3.87 (m, 1H), 3.87 - 3.80 (m, 2H), 3.45 - 3.41 (m, 2H), 3.40 - 3.33 (m, 3H), 3.22 - 3.16 (m, 1H), 2.51 - 2.41 (m, 1H), 2.06 - 1.92 (m, 3H), 1.27 (s, 3H), 0.85 - 0.81 (m, 2H), 0.53 - 0.49 (m, 2H). LC/MS (ESI) (m/z): 585 $[M+H]^+$. |
| **Example 325** | 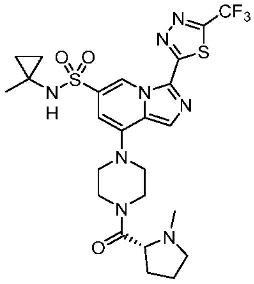 **8-(4-(methyl-D-prolyl)piperazin-1-yl)-N-(1-methylcyclopropyl)-3-(5-(trifluoromethyl)-1,3,4-thiadiazol-2-yl)imidazo[1,5-a]pyridine-6-sulfonamide** | $^1$H NMR(400 MHz, $CD_3OD$) δ 9.81 - 9.78 (m, 1H), 7.94 (s, 1H), 6.76 (s, 1H), 3.98 - 3.90 (m, 2H), 3.88 - 3.80 (m, 3H), 3.47 - 3.36 (m, 5H), 2.78 - 2.69 (m, 1H), 2.64 - 2.60 (m, 3H), 2.52 - 2.42 (m, 1H), 2.09 - 2.01 (m, 1H), 2.00 - 1.90 (m, 2H), 1.26 (s, 3H), 0.85 - 0.80 (m, 2H), 0.53 - 0.48 (m, 2H). LC/MS (ESI) (m/z): 599 $[M+H]^+$. |
| **Example 326** | 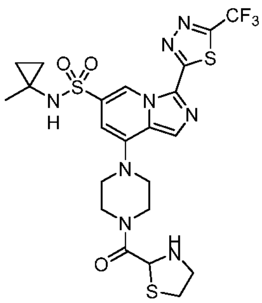 **N-(1-methylcyclopropyl)-8-(4-(thiazolidine-2-carbonyl)piperazin-1-yl)-3-(5-(trifluoromethyl)-1,3,4-thiadiazol-2-yl)imidazo[1,5-a]pyridine-6-sulfonamide** | $^1$H NMR (400 MHz, MeOD) δ 9.84 (s, 1H), 7.99 (s, 1H), 6.80 (s, 1H), 5.32 (s, 1H), 4.07 - 3.96 (m, 1H), 3.91 - 3.76 (m, 4H), 3.52 - 3.37 (m, 4H), 3.15 (t, *J* = 6.5 Hz, 2H), 3.08 - 3.00 (m, 1H), 2.98 - 2.90 (m, 1H), 1.28 (s, 3H), 0.87 - 0.80 (m, 2H), 0.58 - 0.48 (m, 2H). LC/MS (ESI) (m/z): 603 $[M+H]^+$. |
| **Example 327** | 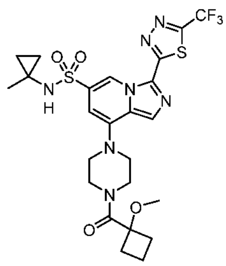 **8-(4-(1-methoxycyclobutane-1-carbonyl)piperazin-1-yl)-N-(1-methylcyclopropyl)-3-(5-(trifluoromethyl)-1,3,4-thiadiazol-2-yl)imidazo[1,5-a]pyridine-6-sulfonamide** | $^1$H NMR (400 MHz, MeOD) δ 9.82 (s, 1H), 7.97 (s, 1H), 6.78 (d, *J* = 1.0 Hz, 1H), 3.96 - 3.90 (m, 4H), 3.44 - 3.37 (m, 4H), 3.20 (s, 3H), 2.70 - 2.60 (m, 2H), 2.30 - 2.19 (m, 2H), 1.96 - 1.82 (m, 1H), 1.75 - 1.58 (m, 1H), 1.27 (s, 3H), 0.87 - 0.81 (m, 2H), 0.57 - 0.49 (m, 2H). LC/MS (ESI) (m/z): 600 $[M+H]^+$. |

(continued)

| Example | Structure and name | Analysis data |
|---|---|---|
| **Example 328** | 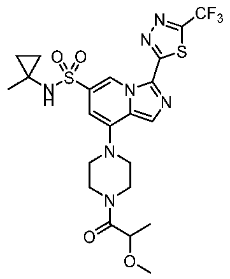<br>**8-(4-(2-methoxypropanoyl)piperazin-1-yl)-N-(1-methylcyclopropyl)-3-(5-(trifluoromethyl)-1,3,4-thiadiazol-2-yl)imidazo[1,5-a] pyridine-6-sulfonamide** | $^1$H NMR (400 MHz, DMSO-$d_6$) δ 9.58 (s, 1H), , 8.45 (s, 1H), 8.12 (s, 1H), 6.73 (s, 1H), 4.33 - 4.26 (m, 1H), 3.87 - 3.75 (m, 4H), 3.39 - 3.30 (m, 4H), 3.25 (s, 3H), 1.30 - 1.26 (m, 3H), 1.16 (s, 3H), 0.76 - 0.69 (m, 2H), 0.48 - 0.43 (m, 2H). LC/MS (ESI) (m/z): 574 [M+H]$^+$. |
| **Example 329** | 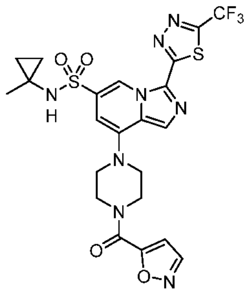<br>**8-(4-(isoxazole-5-carbonyl)piperazin-1-yl)-N-(1-methylcyclopropyl)-3-(5-(trifluoromethyl)-1,3,4-thiadiazol-2-yl)imidazo[1,5-a] pyridine-6-sulfonamide** | $^1$H NMR (400 MHz, DMSO-$d_6$) δ 9.59 (s, 1H), 8.80 (d, *J* = 1.9 Hz, 1H), 8.46 (s, 1H), 8.13 (s, 1H), 7.02 (d, *J* = 1.8 Hz, 1H), 6.74 (s, 1H), 3.99 - 3.82 (m, 4H), 3.52 - 3.40 (m, 4H), 1.16 (s, 3H), 0.77 - 0.71 (m, 2H), 0.48 - 0.41 (m, 2H). LC/MS (ESI) (m/z): 583 [M+H]$^+$. |
| **Example 330** | 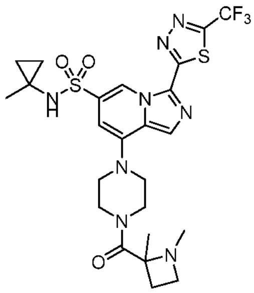<br>**8-(4-(1,2-dimethylazetidine-2-carbonyl)piperazin-1-yl)-N-(1-methylcyclopropyl)-3-(5-(trifluoromethyl)-1,3,4-thiadiazol-2-yl) imidazo[1,5-a]pyridine-6-sulfonamide** | $^1$H NMR (400 MHz, DMSO-$d_6$) δ 9.60 (s, 1H), 8.47 (s, 1H), 8.11 (s, 1H), 6.74 (s, 1H), 3.95 - 3.65 (m, 6H), 3.61 - 3.54 (m, 4H), 2.88 - 2.79 (m, 2H), 2.68 (s, 3H), 1.75 (s, 3H), 1.17 (s, 3H), 0.78 - 0.68 (m, 2H), 0.52 - 0.41 (m, 2H). LC/MS (ESI) (m/z): 599 [M+H]$^+$. |
| **Example 331** | 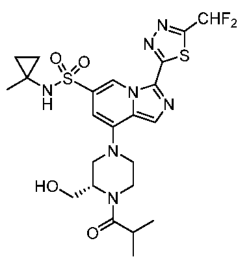<br>**(S)-3-(5-(difluoromethyl)-1,3,4-thiadiazol-2-yl)-8-(3-(hydroxymethyl)-4-isobutyrylpiperazin-1-yl)-N-(1-methylcyclopropyl)imidazo[1,5-a]pyridine-6-sulfonamide** | $^1$H NMR(400 MHz, $CD_3OD$) δ 9.83 (s, 1H), 8.15 (d, *J* = 41.5 Hz, 1H), 7.26 (t, *J* = 53.5 Hz, 1H), 6.74 (s, 1H), 4.78 - 4.72 (m, 1H), 4.60 (d, *J* = 14.0 Hz, 1H), 4.29 - 4.21 (m, 1H), 4.15 - 4.06 (m, 2H), 3.69 - 3.65 (m, 1H), 3.15 - 2.96 (m, 3H), 2.93 - 2.85 (m, 1H), 1.26 (s, 3H), 1.18 - 1.13 (m, 6H), 0.85 - 0.81 (m, 2H), 0.53 - 0.49 (m, 2H). LC/MS (ESI) (m/z): 570 [M+H]$^+$. |

(continued)

| Example | Structure and name | Analysis data |
|---|---|---|
| **Example 332** | 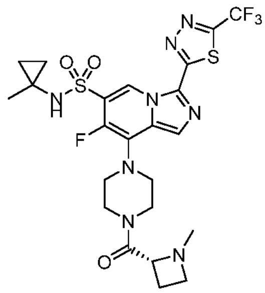<br>**(R)-7-fluoro-8-(4-(1-methylazetidine-2-carbonyl)piperazin-1-yl)-N-(1-methylcyclopropyl)-3-(5-(trifluoromethyl)-1,3,4-thiadiazol-2-yl)imidazo[1,5-a]pyridine-6-sulfonamide** | $^1$H NMR (400 MHz, DMSO-$d_6$) δ 9.64 (d, $J$ = 5.2 Hz, 1H), 8.81 (s, 1H), 8.03 (s, 1H), 5.47 - 5.38 (m, 1H), 4.00 - 3.93 (m, 2H), 3.79 - 3.73 (m, 2H), 3.45 - 3.42 (m, 6H), 2.85 - 2.82 (m, 3H), 2.80 - 2.77 (m, 1H), 2.69 - 2.66 (m, 1H), 1.27 (s, 3H), 0.82 - 0.77 (m, 2H), 0.54 - 0.49 (m, 2H). LC/MS (ESI) m/z: 603 [M+H]$^+$. |
| **Example 333** | 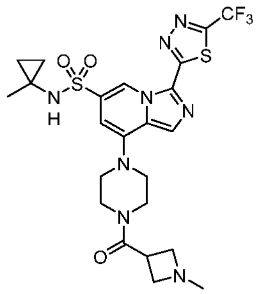<br>**8-(4-(1-methylazetidine-3-carbonyl)piperazin-1-yl)-N-(1-methylcyclopropyl)-3-(5-(trifluoromethyl)-1,3,4-thiadiazol-2-yl)imidazo[1,5-a]pyridine-6-sulfonamide** | $^1$H NMR (400 MHz, DMSO-$d_6$) δ 9.59 (s, 1H), 8.48 (s, 1H), 8.12 (s, 1H), 6.72 (s, 1H), 4.49 - 4.34 (m, 2H), 4.18 - 4.07 (m, 2H), 4.03 - 3.82 (m, 1H), 3.81 - 3.72 (m, 2H), 3.61 - 3.54 (m, 2H), 3.34 - 3.28 (m, 4H), 2.91 - 2.78 (m, 3H), 1.17 (s, 3H), 0.78 - 0.67 (m, 2H), 0.51 - 0.38 (m, 2H). LC/MS (ESI) (m/z): 585 [M+H]$^+$ |
| **Example 334** | 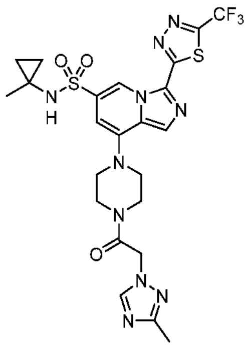<br>**8-(4-(2-(3-methyl-1H-1,2,4-triazol-1-yl)acetyl)piperazin-1-yl)-N-(1-methylcyclopropyl)-3-(5-(trifluoromethyl)-1,3,4-thiadiazol-2-yl)imidazo[1,5-a]pyridine-6-sulfonamide** | $^1$H NMR (400 MHz, DMSO-$d_6$) δ 9.60 (s, 1H), 8.48 (s, 1H), 8.31 (s, 1H), 8.15 (s, 1H), 6.74 (s, 1H), 5.27 (s, 2H), 3.81 - 3.73 (m, 4H), 3.44 - 3.36 (m, 4H), 2.25 (s, 3H), 1.17 (s, 3H), 0.75 - 0.71 (m, 2H), 0.49 - 0.44 (m, 2H). LC/MS (ESI) (m/z): 611 M +H)$^+$. |
| **Example 335** | 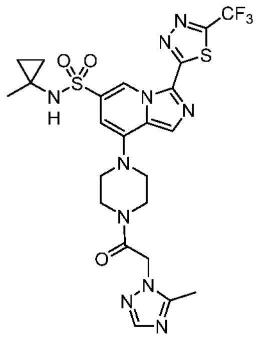<br>**8-(4-(2-(5-methyl-1H-1,2,4-triazol-1-yl)acetyl)piperazin-1-yl)-N-(1-methylcyclopropyl)-3-(5-(trifluoromethyl)-1,3,4-thiadiazol-2-yl)imidazo[1,5-a]pyridine-6-sulfonamide** | $^1$H NMR (400 MHz, DMSO-$d_6$) δ 9.59 (s, 1H), 8.47 (s, 1H), 8.15 (s, 1H), 7.79 (s, 1H), 6.73 (s, 1H), 5.31 (s, 2H), 3.82 - 3.73 (m, 4H), 3.45 - 3.36 (m, 4H), 2.33 (s, 3H), 1.16 (s, 3H), 0.75 - 0.71 (m, 2H), 0.48 - 0.44 (m, 2H). LC/MS (ESI) (m/z): 611 M +H)$^+$. |

(continued)

| Example | Structure and name | Analysis data |
|---|---|---|
| **Example 336** | 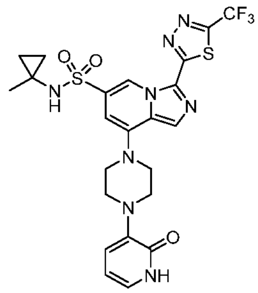 **N-(1-methylcyclopropyl)-8-(4-(2-oxo-1,2-dihydropyridin-3-yl)piperazin-1-yl)-3-(5-(trifluoromethyl)-1,3,4-thiadiazol-2-yl)imidazo [1,5-a]pyridine-6-sulfonamide** | $^1$H NMR (400 MHz, $CD_3OD$) δ 9.80 (s, 1H), 7.93 (s, 1H), 7.11 (dd, *J* = 6.5, 1.6 Hz, 1H), 7.07 (dd, *J* = 7.3, 1.5 Hz, 1H), 6.79 (s, 1H), 6.39 (t, *J* = 6.9 Hz, 1H), 3.54 - 350 (m, 4H), 3.39 - 3.35 (m, 4H), 1.27 (s, 3H), 0.86 - 0.82 (m, 2H), 0.53 - 0.49 (m, 2H). LC/MS (ESI) (m/z): 581 $[M+H]^+$. |
| **Example 337** | 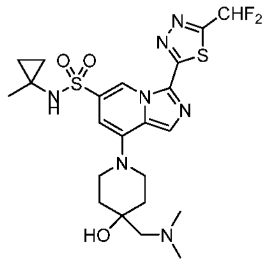 **3-(5-(difluoromethyl)-1,3,4-thiadiazol-2-yl)-8-(4-((dimethylamino)methyl)-4-hydroxypiperidin-1-yl)-N-(1-methylcyclopropyl) imidazo[1,5-a]pyridine-6-sulfonamide** | $^1$H NMR (400 MHz, DMSO-$d_6$) δ 9.56 (s, 1H), 8.44 (s, 1H), 7.96 (s, 1H), 7.68 (t, *J* = 53.2 Hz, 1H), 6.70 (s, 1H), 3.51 - 3.48 (m, 2H), 3.28 - 3.22 (m, 2H), 2.48 (s, 2H), 2.40 (s, 6H), 1.86 - 1.79 (m, 2H), 1.71 (d, *J* = 13.2 Hz, 2H), 1.16 (s, 3H), 0.75 - 0.71 (m, 2H), 0.47 - 0.43 (m, 2H). LC/MS (ESI) (m/z): 542 $(M +H)^+$. |
| **Example 338** | 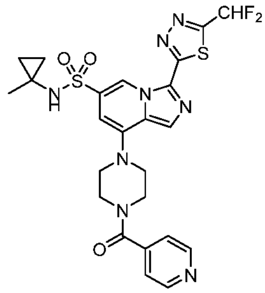 **3-(5-(difluoromethyl)-1,3,4-thiadiazol-2-yl)-8-(4-isonicotinoylpiperazin-1-yl)-N-(1-methylcyclopropyl)imidazo[1,5-a]pyridine-6-sulfonamide** | $^1$H NMR (400 MHz, DMSO-$d_6$) δ 9.61 (s, 1H), 8.71 (d, *J* = 5.8 Hz, 2H), 8.44 (s, 1H), 8.06 (s, 1H), 7.75 (t, *J* = 53.3 Hz, 1H), 7.49 (d, *J* = 5.9 Hz, 2H),6.71 (s, 1H), 3.98 - 3.88 (m, 2H), 3.60 - 3.51 (m, 2H), 3.47 - 3.40 (m, 2H), 3.38 - 3.34 (m, 2H), 1.16 (s, 3H), 0.76 - 0.71 (m, 2H), 0.48 - 0.43 (m, 2H). LC/MS (ESI) (m/z): 575 $[M+H]^+$. |
| **Example 339** | 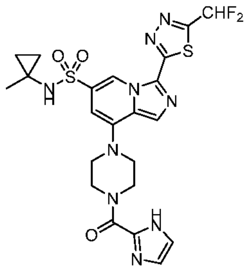 **8-(4-(1H-imidazole-2-carbonyl)piperazin-1-yl)-3-(5-(difluoromethyl)-1,3,4-thiadiazol-2-yl)-N-(1-methylcyclopropyl)imidazo [1,5-a]pyridine-6-sulfonamide** | $^1$H NMR (400 MHz, DMSO-$d_6$) δ 9.61 (s, 1H), 8.42 (s, 1H), 8.11 (s, 1H), 7.69 (t, *J*= 53.1 Hz, 1H), 7.29 (s, 1H), 7.11 (s, 1H), 6.73 (s, 1H), 4.84 - 4.72 (m, 2H), 3.98 - 3.87 (m, 2H), 3.49 - 3.41 (m, 4H), 1.16 (s, 3H), 0.76 - 0.71 (m, 2H), 0.49 - 0.43 (m, 2H). LC/MS (ESI) (m/z): 564 $[M+H]^+$. |

(continued)

| Example | Structure and name | Analysis data |
|---|---|---|
| **Example 340** | 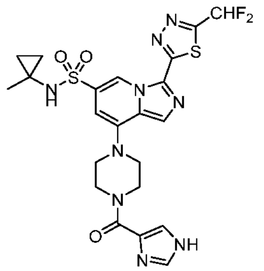<br>**8-(4-(1H-imidazole-4-carbonyl)piperazin-1-yl)-3-(5-(difluoromethyl)-1,3,4-thiadiazol-2-yl)-N-(1-methylcyclopropyl)imidazo [1,5-a]pyridine-6-sulfonamide** | $^1$H NMR (400 MHz, $CD_3OD$) δ 9.83 (s, 1H), 7.94 (s, 1H), 7.78 (s, 1H), 7.64 (s, 1H), 7.26 (t, *J*= 53.5 Hz, 1H), 6.75 (s, 1H), 4.28 - 4.04 (m, 4H), 3.46 - 3.42 (m, 4H), 1.25 (s, 3H), 0.84 - 0.81 (m, 2H), 0.52 - 0.48 (m, 2H). LC/MS (ESI) (m/z): 564 $[M+H]^+$. |
| **Example 341** | 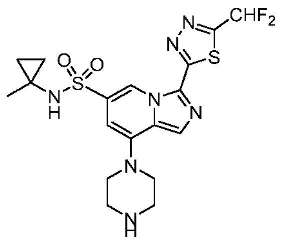<br>**3-(5-(difluoromethyl)-1,3,4-thiadiazol-2-yl)-N-(1-methylcyclopropyl)-8-(piperazin-1-yl)imidazo[1,5-a]pyridine-6-sulfonamide** | $^1$H NMR (400 MHz, DMSO-$d_6$) δ 9.60 (s, 1H), 8.02 (s, 1H), 7.68 (t, *J* = 53.1 Hz, 1H), 6.70 (s, 1H), 3.32 - 3.29 (m, 4H), 3.10 - 3.06 (m, 4H), 1.16 (s, 3H), 0.74 - 0.71 (m, 2H), 0.47 - 0.44 (m, 2H). LC/MS (ESI) (m/z): 470 $[M+H]^+$. |
| **Example 342** | 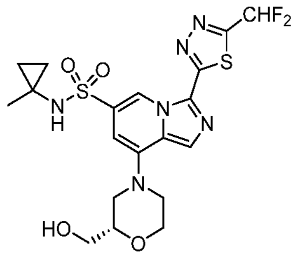<br>**(S)-3-(5-(difluoromethyl)-1,3,4-thiadiazol-2-yl)-8-(2-(hydroxymethyl)morpholino)-N-(1-methylcyclopropyl)imidazo[1,5-a]pyri-dine-6-sulfonamide** | $^1$H NMR (400 MHz, $CD_3OD$) δ 9.82 (s, 1H), 7.89 (s, 1H), 7.26 (t, *J* = 53.5 Hz, 1H), 6.73 (s, 1H), 4.11 - 4.06 (m, 1H), 3.97 - 3.91 (m, 1H), 3.88 - 3.83 (m, 1H), 3.73 - 3.66 (m, 3H), 3.64 - 3.60 (m, 1H), 3.03 (dd, *J* = 11.6, 8.6 Hz, 1H), 2.85 (dd, *J* = 11.8, 10.3 Hz, 1H), 1.25 (s, 3H), 0.85 - 0.81 (m, 2H), 0.52 - 0.48 (m, 2H). LC/MS (ESI) (m/z): 501 $[M+H]^+$. |
| **Example 343** | 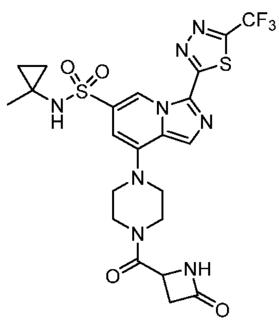<br>**N-(1-methylcyclopropyl)-8-(4-(4-oxoazetidine-2-carbonyl)piperazin-1-yl)-3-(5-(trifluoromethyl)-1,3,4-thiadiazol-2-yl)imidazo [1,5-a]pyridine-6-sulfonamide** | $^1$H NMR (400 MHz, DMSO-$d_6$) δ 9.59 (s, 1H), 8.47 (s, 1H), 8.30 (s, 1H), 8.12 (s, 1H), 6.73 (s, 1H), 4.54 - 4.46 (m, 1H), 3.81 - 3.73 (m, 2H), 3.71 - 3.57 (m, 2H), 3.34 - 3.30 (m, 4H), 3.28 - 3.22 (m, 1H), 2.99 - 2.90 (m, 1H), 1.16 (s, 3H), 0.77 - 0.69 (m, 2H), 0.49 - 0.43 (m, 2H). LC/MS (ESI) (m/z): 585 $[M+H]^+$. |

(continued)

| Example | Structure and name | Analysis data |
|---|---|---|
| **Example 344** | 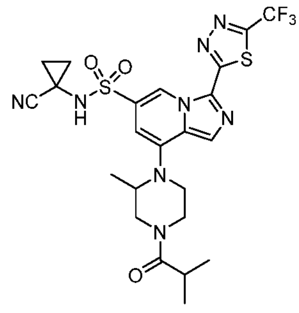 **N-(1-cyanocyclopropyl)-8-(4-isobutyryl-2-methylpiperazin-1-yl)-3-(5-(trifluoromethyl)-1,3,4-thiadiazol-2-yl)imidazo[1,5-a]pyridine-6-sulfonamide** | $^1$H NMR (400 MHz, MeOD) δ 9.89 (s, 1H), 7.97 (s, 1H), 6.79 (s, 1H), 4.65 - 4.44 (m, 1H), 4.30 - 4.13 (m, 2H), 3.99 - 3.79 (m, 1H), 3.62 - 3.55 (m, 1H), 3.51 - 3.44 (m, 1H), 3.29 - 3.22 (m, 1H), 3.11 - 2.98 (m, 1H), 1.60 - 1.54 (m, 1H), 1.50 - 1.43 (m, 2H), 1.24 - 1.18 (m, 2H), 1.18 - 1.11 (m, 6H), 1.11 - 0.99 (m, 2H). LC/MS (ESI) (m/z): 583 $[M+H]^+$. |
| **Example 345** | 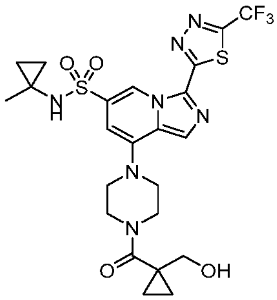 **8-(4-(1-(hydroxymethyl)cyclopropane-1-carbonyl)piperazin-1-yl)-N-(1-methylcyclopropyl)-3-(5-(trifluoromethyl)-1,3,4-thiadiazol-2-yl)imidazo[1,5-a]pyridine-6-sulfonamide** | $^1$H NMR (400 MHz, DMSO-$d_6$) δ 9.58 (s, 1H), 8.46 (s, 1H), 8.12 (s, 1H), 6.73 (s, 1H), 4.97 - 4.89 (m, 1H), 3.99 - 3.71 (m, 4H), 3.54 - 3.47 (m, 2H), 3.31 - 3.25 (m, 4H), 1.16 (s, 3H), 0.83 - 0.77 (m, 2H), 0.75 - 0.68 (m, 4H), 0.50 - 0.41 (m, 2H). LC/MS (ESI) (m/z): 586 $[M+H]^+$. |
| **Example 346** | 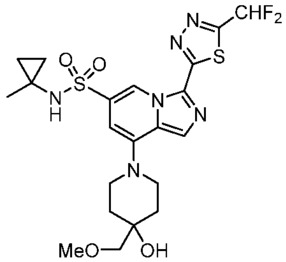 **3-(5-(difluoromethyl)-1,3,4-thiadiazol-2-yl)-8-(4-hydroxy-4-(methoxymethyl)piperidin-1-yl)-N-(1-methylcyclopropyl) imidazo [1,5-a]pyridine-6-sulfonamide** | $^1$H NMR (400 MHz, DMSO-$d_6$) δ 9.56 (s, 1H), 8.42 (s, 1H), 7.97 (s, 1H), 7.68 (t, *J* = 53.2 Hz, 1H), 6.69 (s, 1H), 4.58 (s, 1H), 3.53 (d, *J* = 12.1 Hz, 2H), 3.30 - 3.23 (m, 5H), 3.22 - 3.17 (m, 2H), 1.91 - 1.83 (m, 2H), 1.62 - 1.57 (m, 2H), 1.15 (s, 3H), 0.72 (t, *J* = 5.5 Hz, 2H), 0.45 (q, *J* = 4.9 Hz, 2H). LC/MS (ESI) (m/z): 529 $[M+H]^+$. |
| **Example 347** | 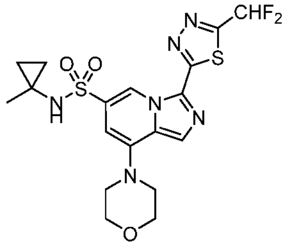 **3-(5-(difluoromethyl)-1,3,4-thiadiazol-2-yl)-N-(1-methylcyclopropyl)-8-morpholinoimidazo[1,5-a]pyridine-6-sulfonamide** | $^1$H NMR (400 MHz, DMSO-$d_6$) δ 9.61 (s, 1H), 8.44 (s, 1H), 8.06 (s, 1H), 7.68 (t, *J* = 53.1 Hz, 1H), 6.69 (s, 1H), 3.90 - 3.84 (m, 4H), 3.30 - 3.25 (m, 4H), 1.15 (s, 3H), 0.76 - 0.69 (m, 2H), 0.48 - 0.42 (m, 2H). LC/MS (ESI) (m/z): 471 [M+H]. |

(continued)

| Example | Structure and name | Analysis data |
|---|---|---|
| **Example 348** | 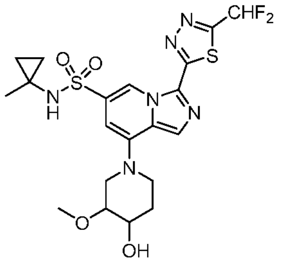 **3-(5-(difluoromethyl)-1,3,4-thiadiazol-2-yl)-8-(4-hydroxy-3-methoxypiperidin-1-yl)-N-(1-methylcyclopropyl)imidazo[1,5-a]pyridine-6-sulfonamide** | $^1$H NMR (400 MHz, DMSO-$d_6$) δ 9.56 (s, 1H), 8.42 (s, 1H), 7.96 (s, 1H), 7.68 (t, *J* = 53.1 Hz, 1H), 6.68 (s, 1H), 5.16 - 5.02 (m, 1H), 3.80 - 3.65 (m, 1H), 3.64 - 3.46 (m, 2H), 3.39 (s, 3H), 3.28 - 3.20 (m, 1H), 3.19 - 2.83 (m, 2H), 2.08 - 1.94 (m, 1H), 1.81 - 1.56 (m, 1H), 1.15 (s, 3H), 0.78 - 0.70 (m, 2H), 0.49 - 0.43 (m, 2H). LC/MS (ESI) (m/z): 515 $[M+H]^+$. |
| **Example 349** | 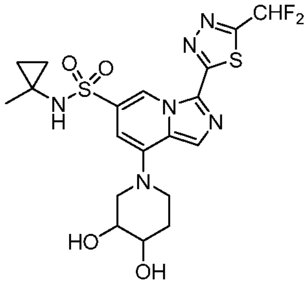 **3-(5-(difluoromethyl)-1,3,4-thiadiazol-2-yl)-8-(3,4-dihydroxypiperidin-1-yl)-N-(1-methylcyclopropyl)imidazo[1,5-a]pyridine-6-sulfonamide** | $^1$H NMR (400 MHz, DMSO-$d_6$) δ 9.54 (s, 1H), 8.43 (s, 1H), 7.96 (s, 1H), 7.68 (s, 1H), 6.67 (s, 1H), 4.79 (d, *J* = 4.8 Hz, 1H), 4.65 (d, *J* = 4.2 Hz, 1H), 3.84 - 3.76 (m, 2H), 3.46 - 3.33 (m, 2H), 3.27 - 3.25 (m, 2H), 1.86 - 1.81 (m, 2H), 1.15 (s, 3H), 0.73 - 0.70 (m, 2H), 0.46 - 0.42 (m, 2H). LC/MS (ESI) (m/z): 501 $[M+H]^+$ |
| **Example 350** | 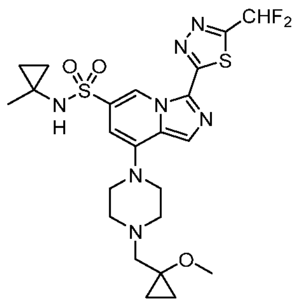 **3-(5-(difluoromethyl)-1,3,4-thiadiazol-2-yl)-8-(4-((1-methoxycyclopropyl)methyl)piperazin-1-yl)-N-(1-methylcyclopropyl)imidazo[1,5-a]pyridine-6-sulfonamide** | $^1$H NMR (400 MHz, DMSO-$d_6$) δ 9.65 (s, 1H), 8.48 (s, 1H), 8.14 (s, 1H), 7.69 (t, *J* = 53.1 Hz, 1H), 6.77 (s, 1H), 3.96 - 3.86 (m, 2H), 3.81 - 3.75 (m, 2H), 3.50 - 3.41 (m, 4H), 3.37 - 3.31 (m, 2H), 3.30 (s, 3H), 1.18 (s, 3H), 1.01 - 0.95 (m, 2H), 0.84 - 0.78 (m, 2H), 0.76 - 0.71 (m, 2H), 0.50 - 0.44 (m, 2H). LC/MS (ESI) (m/z): 554 $[M+H]^+$. |
| **Example 351** | 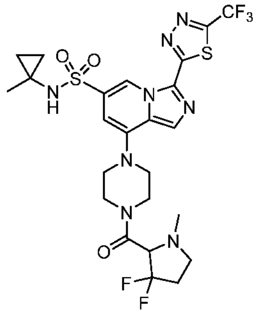 **8-(4-(3,3-difluoro-1-methylpyrrolidine-2-carbonyl)piperazin-1-yl)-N-(1-methylcyclopropyl)-3-(5-(trifluoromethyl)-1,3,4-thiadiazol-2-yl)imidazo[1,5-a]pyridine-6-sulfonamide** | $^1$H NMR (400 MHz, $CD_3OD$) δ 9.81 (s, 1H), 7.96 (s, 1H), 6.79 - 6.73 (m, 1H), 4.10 - 3.95 (m, 3H), 3.89 - 3.77 (m, 2H), 3.50 - 3.43 (m, 2H), 3.38 - 3.32 (m, 2H), 3.24 - 3.18 (m, 1H), 2.62 - 2.54 (m, 1H), 2.42 - 2.34 (m, 5H), 1.26 (s, 3H), 0.85 - 0.81 (m, 2H), 0.52 - 0.49 (m, 2H). LC/MS (ESI) (m/z): 635 $[M+H]^+$. |

(continued)

| Example | Structure and name | Analysis data |
|---|---|---|
| **Example 352** | 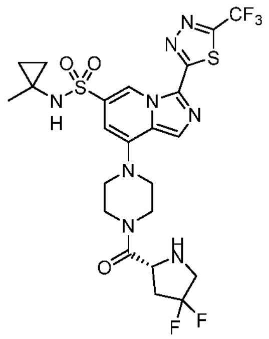 **(R)-8-(4-(4,4-difluoropyrrolidine-2-carbonyl)piperazin-1-yl)-N-(1-methylcyclopropyl)-3-(5-(trifluoromethyl)-1,3,4-thiadiazol-2-yl) imidazo[1,5-a]pyridine-6-sulfonamide** | $^1H$ NMR (400 MHz, DMSO-$d_6$) δ 9.58 (s, 1H), 8.46 (s, 1H), 8.12 (s, 1H), 6.72 (s, 1H), 4.32 - 4.26 (m, 1H), 3.85 - 3.79 (m, 2H), 3.75 - 3.69 (m, 2H), 3.31 - 3.19 (m, 6H), 3.06 - 2.98 (m, 1H), 2.48 - 2.28 (m, 2H), 1.16 (s, 3H), 0.74 - 0.70 (m, 2H), 0.47 - 0.44 (m, 2H). LC/MS (ESI) (m/z): 621 $[M+H]^+$ |
| **Example 353** | 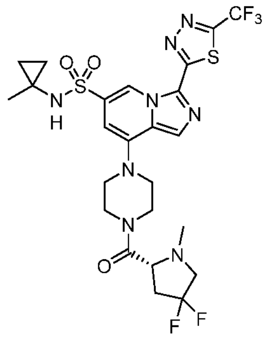 **(R)-8-(4-(4,4-difluoro-1-methylpyrrolidine-2-carbonyl)piperazin-1-yl)-N-(1-methylcyclopropyl)-3-(5-(trifluoromethyl)-1,3,4-thiadiazol-2-yl)imidazo[1,5-a]pyridine-6-sulfonamide** | $^1H$ NMR (400 MHz, $CD_3OD$) δ 9.80 (s, 1H), 7.94 (d, *J* = 1.5 Hz, 1H), 6.75 (s, 1H), 3.97 - 3.88 (m, 4H), 3.76 - 3.72 (m, 1H), 3.45 - 3.36 (m, 4H), 2.80 - 2.68 (m, 2H), 2.44 - 2.16 (m, 5H), 1.27 - 1.24 (m, 3H), 0.84 - 0.80 (m, 2H), 0.52 - 0.48 (m, 2H). LC/MS (ESI) (m/z): 635 $[M+H]^+$ |
| **Example 354** | 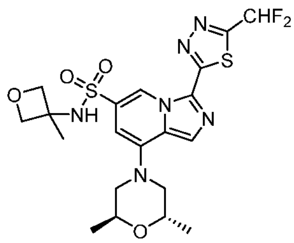 **3-(5-(difluoromethyl)-1,3,4-thiadiazol-2-yl)-8-((2S,6S)-2,6-dimethylmorpholino)-N-(3-methyloxetan-3-yl)imidazo[1,5-a]pyridine-6-sulfonamide** | $^1H$ NMR (400 MHz, DMSO-$d_6$) δ 9.57 (s, 1H), 8.71 (s, 1H), 7.94 (s, 1H), 7.74 (t, *J* = 53.2 Hz, 1H), 6.68 (s, 1H), 4.63 (t, *J* = 6.6 Hz, 2H), 4.25 - 4.13 (m, 6H), 3.14 - 3.10 (m, 2H), 1.50 (s, 3H), 1.31 (d, *J* = 6.3 Hz, 6H).LC/MS (ESI) (m/z): 515 $[M+H]^+$. |
| **Example 355** | 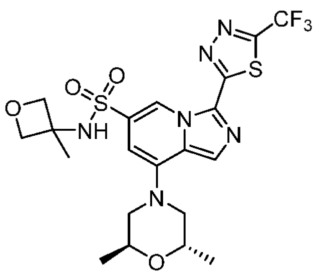 **8-((2S,6S)-2,6-dimethylmorpholino)-N-(3-methyloxetan-3-yl)-3-(5-(trifluoromethyl)-1,3,4-thiadiazol-2-yl)imidazo[1,5-a]pyridine-6-sulfonamide** | $^1H$ NMR (400 MHz, $CDCl_3$) δ 9.80 (s, 1H), 7.65 (s, 1H), 6.57 (s, 1H), 4.74 (d, *J* = 5.4 Hz, 2H), 4.37 (d, *J* = 6.8 Hz, 2H), 4.22 - 4.18 (m, 2H), 3.26 - 3.22 (m, 2H), 3.11 - 3.06 (m, 2H), 1.68 (s, 3H), 1.35 (d, *J* = 6.4 Hz, 6H). LC/MS (ESI) (m/z): 533 $[M+H]^+$. |

(continued)

| Example | Structure and name | Analysis data |
|---|---|---|
| **Example 356** | 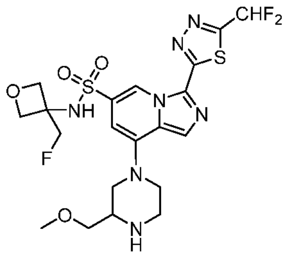 **3-(5-(difluoromethyl)-1,3,4-thiadiazol-2-yl)-N-(3-(fluoromethyl) oxetan-3-yl)-8-(3-(methoxymethyl)piperazin-1-yl)imidazo[1,5-a] pyridine-6-sulfonamide** | $^1$H NMR (400 MHz, DMSO-$d_6$) δ 9.57 (s, 1H), 7.97 (s, 1H), 7.67 (t, *J* = 53.1 Hz, 1H), 6.71 (s, 1H), 4.72 (s, 1H), 4.65 - 4.62 (m, 2H), 4.60 (s, 1H), 4.38 (d, *J* = 6.8 Hz, 2H), 3.64 - 3.54 (m, 1H), 3.45 - 3.41 (m, 2H), 3.31 (s, 3H), 3.13 - 3.05 (m, 3H), 3.00 - 2.95 (m, 1H), 2.90 - 2.85 (m, 1H), 2.76 - 2.70 (m, 1H). LC/MS (ESI) (m/z): 548 [M+H]$^+$ |
| **Example 357** | 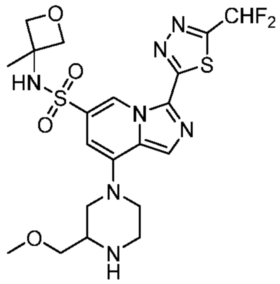 **3-(5-(difluoromethyl)-1,3,4-thiadiazol-2-yl)-8-(3-(methoxymethyl) piperazin-1-yl)-N-(3-methyloxetan-3-yl)imidazo[1,5-a]pyridine-6-sulfonamide** | $^1$H NMR (400 MHz, $CD_3OD$) δ 9.85 (s, 1H), 7.92 (s, 1H), 7.46 - 7.20 (m, 1H), 6.81 (d, *J* = 1.1 Hz, 1H), 4.79 (d, *J* = 6.3 Hz, 2H), 4.32 (d, *J* = 6.7 Hz, 2H), 3.93 - 3.77 (m, 2H), 3.73 - 3.61 (m, 3H), 3.50 - 3.38 (m, 2H), 3.49 (s, 3H), 3.23 - 3.09 (m, 2H), 1.64 (s, 3H). LC/MS (ESI) (m/z): 530 [M+H]$^+$. |
| **Example 358** | 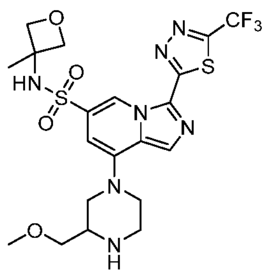 **8-(3-(methoxymethyl)piperazin-1-yl)-N-(3-methyloxetan-3-yl)-3-(5-(trifluoromethyl)-1,3,4-thiadiazol-2-yl)imidazo[1,5-a]pyridine-6-sulfonamide** | $^1$H NMR (400 MHz, $CD_3OD$) δ 9.86 (s, 1H), 7.99 (s, 1H), 6.87 (s, 1H), 4.85 - 4.79 (m, 2H), 4.33 (d, *J* = 6.6 Hz, 2H), 3.90 - 3.86 (m, 2H), 3.87 - 3.84 (m, 1H), 3.79 - 3.75 (m, 1H), 3.71 - 3.67 (m, 1H), 3.59 - 3.52 (m, 2H), 3.49 (s, 3H), 3.27 - 3.21 (m, 2H), 1.65 (s, 3H). LC/MS (ESI) (m/z): 548 [M+H]$^+$. |
| **Example 359** | 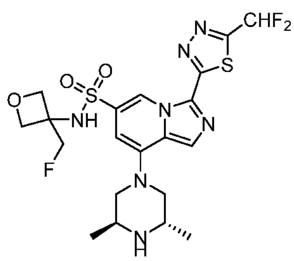 **3-(5-(difluoromethyl)-1,3,4-thiadiazol-2-yl)-8-((3S,5S)-3,5-dimethylpiperazin-1-yl)-N-(3-(fluoromethyl)oxetan-3-yl)imidazo [1,5-a]pyridine-6-sulfonamide** | $^1$H NMR (400 MHz, DMSO-$d_6$) δ 9.57 (s, 1H), 7.90 (s, 1H), 7.67 (t, *J* = 53.4 Hz, 1H), 6.71 (s, 1H), 4.72 (s, 1H), 4.66 - 4.60 (m, 3H), 4.38 (d, *J* = 6.6 Hz, 2H), 3.08 - 3.04 (m, 6H), 1.23 (d, *J* = 6.4 Hz, 6H). LC/MS (ESI) (m/z): 532 [M+H]$^+$. |

(continued)

| Example | Structure and name | Analysis data |
|---|---|---|
| **Example 360** | 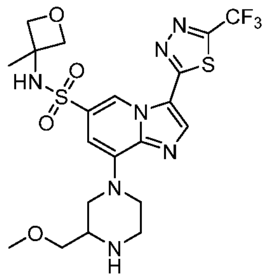 **8-(3-(methoxymethyl)piperazin-1-yl)-N-(3-methyloxetan-3-yl)-3-(5-(trifluoromethyl)-1,3,4-thiadiazol-2-yl)imidazo[1,2-a]pyridine-6-sulfonamide** | $^1H$ NMR (400 MHz, $CD_3OD$) δ 9.90 (s, 1H), 8.50 (s, 1H), 7.15 (s, 1H), 4.78 (d, *J* = 6.2 Hz, 2H), 4.41 - 4.17 (m, 4H), 3.67 - 3.50 (m, 3H), 3.44 (s, 3H), 3.40 - 3.33 (m, 2H), 3.16 - 3.09 (m, 1H), 3.06 - 2.96 (m, 1H), 1.63 (s, 3H). LC/MS (ESI) (m/z): 548 $[M+H]^+$. |
| **Example 361** | 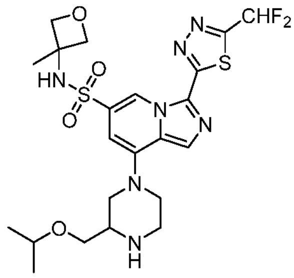 **3-(5-(difluoromethyl)-1,3,4-thiadiazol-2-yl)-8-(3-(isopropoxymethyl)piperazin-1-yl)-N-(3-methyloxetan-3-yl)imidazo[1,5-a] pyridine-6-sulfonamide** | $^1H$ NMR (400 MHz, $CD_3OD$) δ 9.88 (s, 1H), 7.96 (s, 1H), 7.34 (t, *J* = 53.5 Hz, 1H), 6.84 (s, 1H), 4.80 - 4.77 (m, 2H), 4.36 - 4.31 (m, 2H), 3.98 - 3.82 (m, 4H), 3.77 - 3.70 (m, 2H), 3.59 - 3.51 (m, 2H), 3.28 - 3.17 (m, 2H), 1.65 (s, 3H), 1.25 (d, *J* = 6.1 Hz, 6H). LC/MS (ESI) (m/z): 558 $[M+H]^+$. |
| **Example 362** | 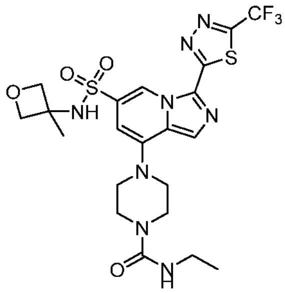 **N-ethyl-4-(6-(N-(3-methyloxetan-3-yl)sulfamoyl)-3-(5-(trifluoromethyl)-1,3,4-thiadiazol-2-yl)imidazo[1,5-a]pyridin-8-yl)piperazine-1-carboxamide** | $^1H$ NMR (400 MHz, $CD_3OD$) δ 9.80 (s, 1H), 7.93 (s, 1H), 6.78 (s, 1H), 4.79 (d, *J* = 6.3 Hz, 2H), 4.31 (d, *J* = 6.4 Hz, 2H), 3.69 - 3.65 (m, 4H), 3.39 - 3.36 (m, 4H), 3.26 - 3.21 (m, 2H), 1.64 (s, 3H), 1.16 - 1.12 (m, 3H). LC/MS (ESI) (m/z): 575 $[M+H]^+$. |
| **Example 363** | 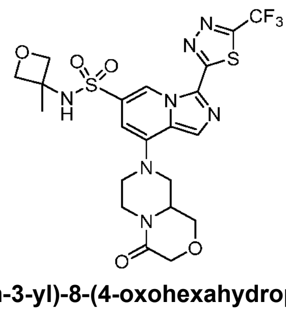 **N-(3-methyloxetan-3-yl)-8-(4-oxohexahydropyrazino[2,1-c][1,4] oxazin-8(1H)-yl)-3-(5-(trifluoromethyl)-1,3,4-thiadiazol-2-yl)imidazo[1,5-a]pyridine-6-sulfonamide** | $^1H$ NMR (400 MHz, $CD_3OD$) δ 9.82 (s, 1H), 7.99 (s, 1H), 6.82 (s, 1H), 4.81 (d, *J* = 6.3 Hz, 2H), 4.59 - 4.58 (m, 2H), 4.32 (d, *J* = 6.3 Hz, 2H), 4.20 - 4.19 (m, 2H), 4.03 - 3.98 (m, 1H), 3.88 - 3.71 (m, 4H), 3.20 - 3.15 (m, 1H), 2.98 - 2.91 (m, 1H), 1.64 (s, 3H)。LC/MS (ESI) (m/z): 574 $[M+H]^+$. |

(continued)

| Example | Structure and name | Analysis data |
|---|---|---|
| **Example 364** | 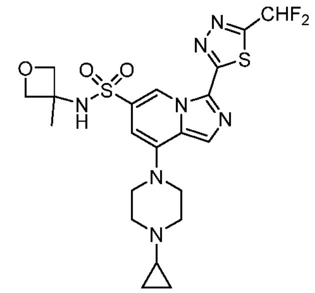 **8-(4-cyclopropylpiperazin-1-yl)-3-(5-(difluoromethyl)-1,3,4-thiadiazol-2-yl)-N-(3-methyloxetan-3-yl)imidazo[1,5-a]pyridine-6-sulfonamide** | $^1$H NMR (400 MHz, $CD_3OD$) δ 9.81 (s, 1H), 7.88 (s, 1H), 7.33 (t, *J* = 53.6 Hz, 1H), 6.73 (s, 1H), 4.79 (d, *J* = 5.8 Hz, 2H), 4.31 (d, *J* = 5.2 Hz, 2H), 3.38 - 3.35 (m, 4H), 2.95 - 2.92 (m, 4H), 1.86 - 1.81 (m, 1H), 1.63 (s, 3H), 0.58 - 0.49 (m, 4H). LC/MS (ESI) (m/z): 526 $[M+H]^+$. |
| **Example 365** | 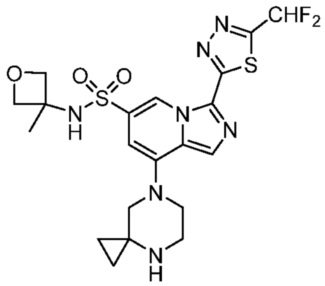 **3-(5-(difluoromethyl)-1,3,4-thiadiazol-2-yl)-N-(3-methyloxetan-3-yl)-8-(4,7-diazaspiro [2.5]octan-7-yl)imidazo[1,5-a]pyridine-6-sulfonamide** | $^1$H NMR (400 MHz, $CD_3OD$) δ 9.83 (s, 1H), 7.82 (s, 1H), 7.33 (t, *J* = 53.5 Hz, 1H), 6.75 (s, 1H), 4.81 - 4.78 (m, 2H), 4.33 - 4.30 (m, 2H), 3.52 - 3.48 (m, 2H), 3.38 - 3.34 (m, 2H), 3.33 - 3.32 (m, 2H), 1.64 (s, 3H), 0.92 - 0.87 (m, 4H). LC/MS (ESI) (m/z): 512 $[M+H]^+$. |

**Example 366: N-(1-cyanocyclopropyl)-3-(5-(hydroxymethyl)-1,3,4-thiadiazol-2-yl)-8-(4-isobutyrylpiperazin-1-yl) imidazo[1,5-a]pyridine-6-sulfonamide**

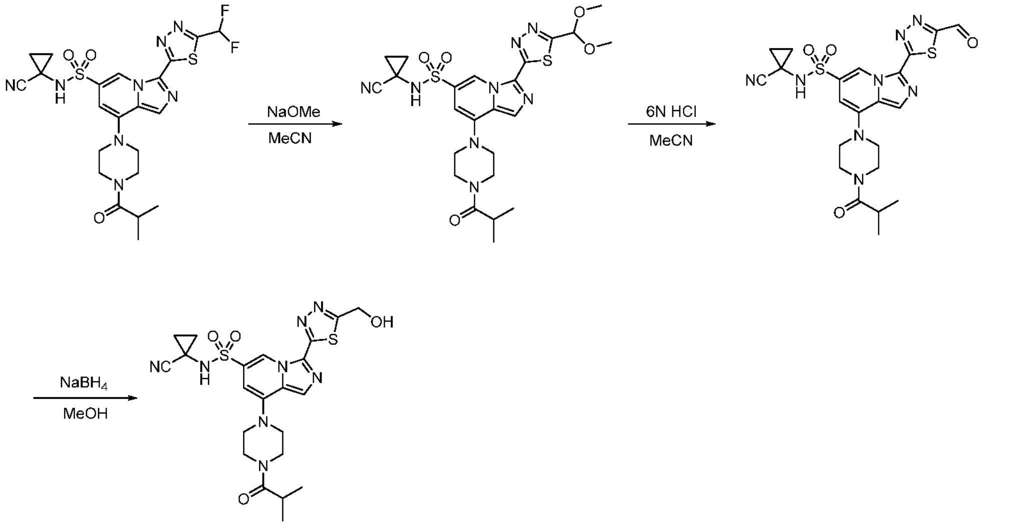


**Step 1: N-(1-cyanocyclopropyl)-3-(5-(dimethoxymethyl)-1,3,4-thiadiazol-2-yl)-8-(4-isobutyrylpiperazin-1-yl)imidazo[1,5-a]pyridine-6-sulfonamide**

At 0°C, 5M sodium methoxide/methanol solution (1 mL) was slowly added to the solution of N-(1-cyanocyclopropylyl)-3-(5-(difluoromethyl)-1,3,4-thiadiazol-2-yl)-8-(4-isobutyrylpiperazin-1-yl)imidazo[1,5-a]pyridin-6-sulfonamide

(230 mg, 0.418 mmol) in acetonitrile (3 mL). The reaction mixture was stirred at room temperature for 3 hours. After completion of the reaction, the reaction mixture was poured into ice water and extracted with ethyl acetate twice. The organic phase was washed with saturated brine, dried over anhydrous sodium sulfate, and concentrated under reduced pressure to obtain a crude title compound (yellow solid, 170 mg, yield: 70.8%), which was directly used in the next step of reaction without purification. LC/MS (ESI) m/z: 575 $[M+H]^+$.

**Step 2: N-(1-cyanocyclopropyl)-3-(5-formyl-1,3,4-thiadiazol-2-yl)-8-(4-isobutyrylpiperazin-1-yl)imidazo[1,5-a]pyridine-6-sulfonamide**

At 0°C, 6N hydrochloric acid solution (0.5 mL) was slowly added to the solution of N-(1-cyanocyclopropyl)-3-(5-(dimethoxymethyl)-1,3,4-thiadiazol-2-yl)-8-(4-isobutyrylpiperazin-1-yl)imidazo[1, 5-a]pyridin-6-sulfonamide (35 mg, 0.061 mmol) in acetonitrile (1.5 mL). The reaction mixture was stirred at 70 °C for 3 hour. After completion of the reaction, the reaction mixture was diluted with water, and extracted with chloroform/isopropanol (3:1). The organic phase was washed with saturated brine, dried over anhydrous sodium sulfate, and concentrated under reduced pressure to obtain a crude title compound (yellow solid, 28 mg, yield: 87.0%), which was directly used in the next step of reaction without purification. LC/MS (ESI) m/z: 529 $[M+H]^+$.

**Step 3: N-(1-cyanocyclopropyl)-3-(5-(hydroxymethyl)-1,3,4-thiadiazol-2-yl)-8-(4-isobutyrylpiperazin-1-yl)imidazo[1,5-a]pyridine-6-sulfonamide**

At 0°C, sodium borohydride (3 mg, 0.08 mmol) was slowly added to the solution of N-(1-cyanocyclopropyl)-3-(5-formyl-1,3,4-thiadiazol-2-yl)-8-(4-isobutyrylpiperazin-1-yl)imidazo[1,5-a]pyridin-6-sulfonamide (28 mg, 0.053 mmol) in methanol (1 mL). The mixture was stirred at room temperature for 30 minutes. The reaction mixture was quenched with saturated aqueous ammonium chloride solution, and concentrated under reduced pressure. The residue was purified by pre-HPLC to obtain the title compound (yellow solid, 7 mg, yield: 24.9%). $^1H$ NMR (400 MHz, DMSO-$d_6$) δ 9.71 (s, 1H), 9.47 (s, 1H), 8.01 (s, 1H), 6.61 (s, 1H), 6.38 (s, 1H), 4.95 (d, $J$ = 4.1 Hz, 2H), 3.81-3.70 (m, 4H), 3.32-3.27 (m, 4H), 2.99-2.90 (m, 1H), 1.52-1.43 (m, 2H), 1.40-1.35 (m, 2H), 1.05 (d, $J$ = 6.4 Hz, 6H). LC/MS (ESI) m/z: 531 $[M+H]^+$.

**Example 367: N-(1-cyanocyclopropyl)-3-(5-(difluoromethyl)-1,3,4-thiadiazol-2-yl)-8-(3-(1-methyl-1H-pyrazol-5-yl)cyclopent-1-en-1-yl)-[1,2,4]triazolo[4,3-a]pyridine-6-sulfonamide**

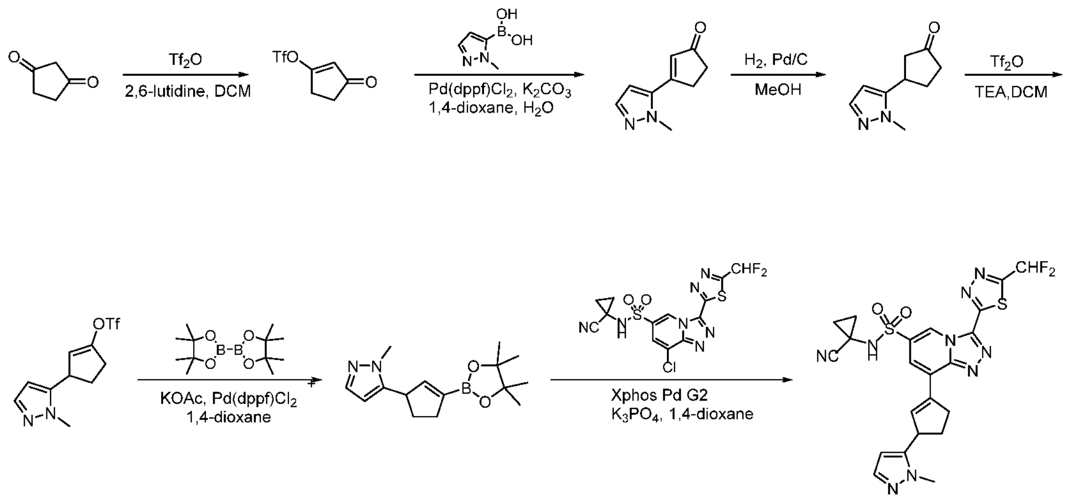


**Step 1: 3-oxocyclopent-1-en-1-yl trifluoromethanesulfonate**

- of nitrogen at 70°C, trifluoromethanesulfonic anhydride (1 g, 3.67 mmol) was dropwise added slowly to the solution of cyclopentan-1,3-diketone (300 mg, 3.06 mmol) and 2,6-dimethylpyridine (200 mg, 4.59 mmol) in dichloromethane (10 mL); and the mixture was stirred at -70°C Under an atmosphere for one hour. The reaction mixture was quenched with ice water and extracted with dichloromethane twice. The organic phase was washed with saturated brine, dried over

anhydrous sodium sulfate, filtered and concentrated under reduced pressure. The residue was separated by column chromatography (eluent: petroleum ether/ethyl acetate, gradient: 0-40% ethyl acetate) to obtain the title compound (yellow oil, 600 mg, yield: 85.2%).LC/MS (ESI) (m/z): 231 $[M+H]^+$.

**Step 2: 3-(1-methyl-1H-pyrazol-5-yl)cyclopent-2-en-1-one**

Under an atmosphere of nitrogen, 1-methyl-1H-pyrazol-5-boric acid (126 mg, 1.0 mmol), potassium carbonate (414 mg, 3.0 mmol), and Pd(dppf)$Cl_2$ (73 mg, 0.1 mmol) were sequentially added to the solution of 3-oxocyclopent-1-ene-1-yltrifluoromethanesulfonate (230 mg, 1.0 mmol) in 1,4-dioxane (4 mL) and water (1 mL). The flask was replaced with nitrogen gas three times and then the mixture was stirred at 80°C for 2 hours under an atmosphere of nitrogen. The reaction mixture was diluted with ethyl acetate, and sequentially washed with water and saturated brine; and the organic phase was dried over anhydrous sodium sulfate, and concentrated under reduced pressure. The residue was purified and separated by column chromatography (eluent: petroleum ether/ethyl acetate, gradient: 0-50% ethyl acetate) to obtain the title compound (white sold, 140 mg, yield: 85.8%). LC/MS (ESI) (m/z): 163 $[M+H]^+$.

**Step 3: 3-(1-methyl-1H-pyrazol-5-yl)cyclopentan-1-one**

Under an atmosphere of nitrogen, Pd/C (20 mg) with the mass fraction of 10% was added to the solution of 3-(1-methyl-1H-pyrazol-5-yl)cyclopent-2-ene-1-one (140 mg, 0.86 mmol) in methanol (5 mL); the flask was replaced with nitrogen gas three times and then the mixture was stirred at room temperature for 6 hours under an atmosphere of hydrogen. After completion of the reaction, the reaction mixture was filtered; and the filtrate was concentrated under reduced pressure to obtain the title compound (white solid, 145 mg, yield: 100%). LC/MS (ESI) (m/z): 165 $[M+H]^+$.

**Step 4: 3-(1-methyl-1H-pyrazol-5-yl)cyclopent-1-en-1-yl trifluoromethanesulfonate**

At -70°C, NaHMDS (4.5 mL, 4.5 mmol, 1M in THF) was dropwise added slowly to the solution of 3-(1-methyl-1H-pyrazol-5-yl)cyclopentan-1-one (500 mg, 3.03 mmol) in tetrahydrofuran (10mL); the mixture was stirred at -70°C for 0.5 hour; then, N-phenylbis(trifluorotrifluoromethanesulfon)imide (1.3 g, 3.6 mmol) in tetrahydrofuran (5 mL) was dropwise added slowly; and the reaction mixture was continued to be stirred at -70°C for 0.5 hour. The reaction mixture was quenched with ice water and extracted with ethyl acetate; and the organic phase was washed with saturated brine, dried over anhydrous sodium sulfate, filtered and concentrated under reduced pressure. The residue was separated by column chromatography (eluent: petroleum ether/ethyl acetate, gradient: 0-50% ethyl acetate) to obtain the title compound (yellow oil, 600 mg, yield: 67.5%). LC/MS (ESI) (m/z): 297 $[M+H]^+$.

**Step 5: 1-methyl-5-(3-(4,4,5,5-tetramethyl-1,3,2-dioxaborolan-2-yl)cyclopent-2-en-1-yl)-1H-pyrazole**

Under an atmosphere of nitrogen, bis(pinacolato)diboron (430 mg, 1.69 mmol), potassium acetate (497 mg, 5.1 mmol) and Pd(dppf)$Cl_2$ (123 mg, 0.17 mmol) were sequentially added to the solution of 3-(1-methyl-1H-pyrazol-5-yl) cyclopent-1-ene-1-yltrifluoromethanesulfonate (500 mg, 1.69 mmol) in 1,4-dioxane (4 mL). The flask was replaced with nitrogen gas three times and then the mixture was stirred at 80°C for 2 hours under an atmosphere of nitrogen. The reaction mixture was diluted with ethyl acetate, washed with water and saturated brine in sequence, dried over anhydrous sodium sulfate, and concentrated under reduced pressure. The residue was purified and separated by column chromatography (eluent: petroleum ether/ethyl acetate, gradient: 0-30% ethyl acetate) to obtain the title compound (white sold, 400 mg, yield: 86.4%). LC/MS (ESI) (m/z): 275 $[M+H]^+$.

**Step 6: N-(1-cyanocyclopropyl)-3-(5-(difluoromethyl)-1,3,4-thiadiazol-2-yl)-8-(3-(1-methyl-1H-pyrazol-5-yl)cyclopent-1-en-1-yl)-[1,2,4]triazolo[4,3-a]pyridine-6-sulfonamide**

Under an atmosphere of nitrogen, 8-chloro-N-(1-cyanocyclopropyl)-3-(5-(difluoromethyl)-1,3,4-thiadiazol-2-yl)-[1,2,4]triazolo[4,3-a]pyridin-6-sulfonamide (80 mg, 0.18 mmol), potassium phosphate (115 mg, 0.54 mmol), Pd(dppf)$Cl_2$ (14 mg, 0.02 mmol) were sequentially added to the solution of 1-methyl-5-(3-(4,4,5,5-tetramethyl-1,3,2-dioxoboracyclopentan-2-yl)cyclopent-2-ene-1-yl)-1H-pyrazole (50 mg, 0.18 mmol) in 1,4-dioxane (4 mL). The flask was replaced with nitrogen gas three times and then the mixture was stirred at 80°C for 2 hours under an atmosphere of nitrogen. The reaction mixture was diluted with ethyl acetate, washed with water and saturated brine in sequence, dried over anhydrous sodium sulfate, and concentrated under reduced pressure. The residue was separated by column chromatography (eluent: petroleum ether/ethyl acetate, gradient: 0-30% ethyl acetate) to obtain the title compound (white solid, 60 mg, yield: 61.2%). $^1H$ NMR (400 MHz, DMSO-$d_6$) $\delta$ 9.76 (d, $J$ = 1.3 Hz, 1H), 7.92-7.61 (m, 4H), 7.31 (d, $J$ = 1.6 Hz, 1H), 6.04 (d, $J$ = 1.8 Hz, 1H), 4.51-4.44 (m, 1H), 3.87 (s, 3H), 3.12-3.04 (m, 1H), 3.02-2.96 (m, 1H), 2.69-2.60 (m, 1H),

2.02-1.94 (m, 1H), 1.50-1.47 (m, 2H), 1.39-1.36 (m, 2H). LC/MS (ESI) (m/z): 544 [M+H]+.

**By referring to the preparation method in Example 367, the following examples were implemented by synthesizing a crude product according to a similar route by starting from appropriate starting materials, treating the crude product via reversed-phase HPLC, and then freeze-drying the crude product to obtain the title compound.**

| Example | Structure and name | Analysis data |
|---|---|---|
| **Example 368** | 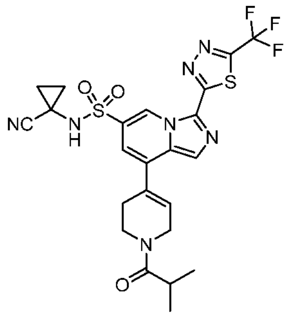<br>**N-(1-cyanocyclopropyl)-8-(1-isobutyryl-1,2,3,6-tetrahydropyridin-4-yl)-3-(5-(trifluoromethyl)-1,3,4-thiadiazol-2-yl)imidazo[1,5-a]pyridine-6-sulfonamide** | $^1$H NMR (400 MHz, DMSO-$d_6$) δ 9.93 (s, 1H), 9.59 (s, 1H), 8.24 (s, 1H), 7.35 (s, 1H), 6.55 (d, $J$ = 11.4 Hz, 1H), 4.39 - 4.22 (m, 2H), 3.82 - 3.75 (m, 2H), 3.04 - 2.94 (m, 1H), 2.71 - 2.67 (m, 1H), 2.62 - 2.56 (m, 1H), 1.53 - 1.48 (m, 2H), 1.43 - 1.38 (m, 2H), 1.07 (d, $J$ = 6.2 Hz, 6H). LC/MS (ESI) m/z: 566 [M+H]+. |
| **Example 369** | 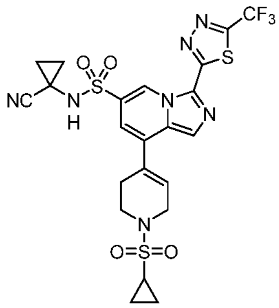<br>**N-(1-cyanocyclopropyl)-8-(1-(cyclopropylsulfonyl)-1,2,3,6-tetrahydropyridin-4-yl)-3-(5-(trifluoromethyl)-1,3,4-thiadiazol-2-yl)imidazo [1,5-a]pyridine-6-sulfonamide** | $^1$H NMR (400 MHz, DMSO-$d_6$) δ 9.94 (s, 1H), 9.60 (s, 1H), 8.25 (s, 1H), 7.36 (s, 1H), 6.56 (s, 1H), 4.13 - 4.03 (m, 2H), 3.60 - 3.52 (m, 2H), 2.76 - 2.73 (m, 2H), 1.53 - 1.47 (m, 2H), 1.43 - 1.36 (m, 2H), 1.27 - 1.20 (m, 1H), 1.06 - 0.99 (m, 4H). LC/MS (ESI) (m/z): 600[M+H]+. |
| **Example 370** | 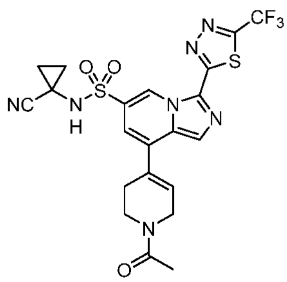<br>**8-(1-acetyl-1,2,3,6-tetrahydropyridin-4-yl)-N-(1-cyanocyclopropyl)-3-(5-(trifluoromethyl)-1,3,4-thiadiazol-2-yl)imidazo [1,5-a]pyridine-6-sulfonamide** | $^1$H NMR (400 MHz, DMSO-$d_6$) δ 9.91 (s, 1H), 8.20 (d, $J$= 6.1 Hz, 1H), 7.32 (s, 1H), 6.51 (s, 1H), 4.33 - 4.18 (m, 2H), 3.77 - 3.69 (m, 2H), 2.70 - 2.65 (m, 1H), 2.61 - 2.54 (m, 1H), 2.12 - 2.05 (m, 3H), 1.51 - 1.43 (m, 2H), 1.40 - 1.32 (m, 2H). LC/MS (ESI) (m/z): 538 [M+H]+. |

(continued)

| Example | Structure and name | Analysis data |
|---|---|---|
| **Example 371** | 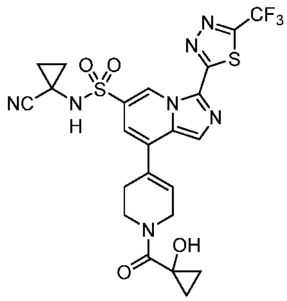 **N-(1-cyanocyclopropyl)-8-(1-(1-hydroxycyclopropane-1-carbonyl)-1,2,3,6-tetrahydropyridin-4-yl)-3-(5-(trifluoromethyl)-1,3,4-thiadiazol-2-yl)imidazo [1,5-a]pyridine-6-sulfonamide** | $^1$H NMR (400 MHz, DMSO-$d_6$) δ 9.92 (s, 1H), 8.21 (s, 1H), 7.34 (s, 1H), 6.58 (s, 1H), 6.42 (s, 1H), 4.73 - 4.15 (m, 2H), 4.11 - 3.66 (m, 2H), 2.73 - 2.55 (m, 2H), 1.56 - 1.45 (m, 2H), 1.41 - 1.31 (m, 2H), 1.04 - 0.92 (m, 2H), 0.87 - 0.76 (m, 2H). LC/MS (ESI) (m/z): 580 [M+H]$^+$. |
| **Example 372** | 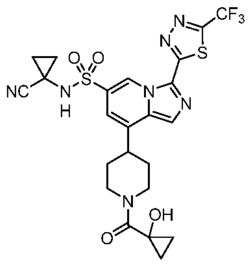 **N-(1-cyanocyclopropyl)-8-(1-(1-hydroxycyclopropane-1-carbonyl) piperidin-4-yl)-3-(5-(trifluoromethyl)-1,3,4-thiadiazol-2-yl)imidazo [1,5-a]pyridine-6-sulfonamide** | $^1$H NMR (400 MHz, DMSO-$d_6$) δ 9.87 (s, 1H), 9.57 (s, 1H), 8.29 (s, 1H), 7.28 (s, 1H), 6.38 (s, 1H), 4.66 - 4.56 (m, 1H), 3.49 - 3.48 (m, 1H), 3.23 - 3.18 (m, 2H), 2.02 (d, $J$ = 12.4 Hz, 2H), 1.88 - 1.58 (m, 3H), 1.50 - 1.46 (m, 2H), 1.41 - 1.37 (m, 2H), 0.99 - 0.94 (m, 2H), 0.82 - 0.78 (m, 2H). LC/MS (ESI) (m/z): 582 [M+H]$^+$ |
| **Example 373** | 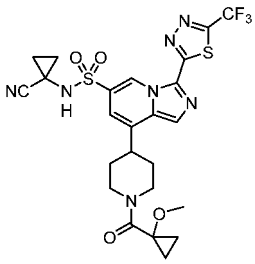 **N-(1-cyanocyclopropyl)-8-(1-(1-methoxycyclopropane-1-carbonyl) piperidin-4-yl)-3-(5-(trifluoromethyl)-1,3,4-thiadiazol-2-yl)imidazo [1,5-a]pyridine-6-sulfonamide** | $^1$H NMR (400 MHz, DMSO-$d_6$) δ 10.20 (s, 1H), 7.86 (s, 1H), 7.28 (s, 1H), 6.31 (s, 1H), 4.81 (d, $J$ = 12.0 Hz, 2H), 3.37 (s, 3H), 3.33 - 3.13 (m, 2H), 3.06 - 2.81 (m, 1H), 2.16 (d, $J$ = 12.7 Hz, 2H), 1.91 - 1.78 (m, 2H), 1.74 - 1.67 (m, 2H), 1.54 - 1.46 (m, 2H), 1.17 - 1.11 (m, 2H), 1.02 - 0.94 (m, 2H). LC/MS (ESI) (m/z): 596 [M+H]$^+$ |
| **Example 374** | 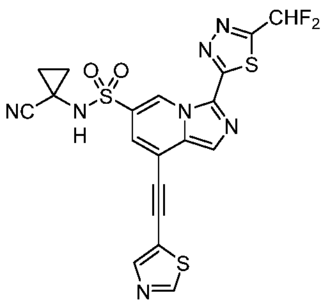 **N-(1-cyanocyclopropyl)-3-(5-(difluoromethyl)-1,3,4-thiadiazol-2-yl)-8-(thiazol-5-ylethynyl)imidazo [1,5-a]pyridine-6-sulfonamide** | $^1$H NMR (400 MHz, DMSO-$d_6$) δ 9.99 (s, 1H), 9.64 (s, 1H), 9.32 (s, 1H), 8.52 (s, 1H), 8.21 (s, 1H), 7.78 (t, $J$ = 53.2 Hz, 1H), 7.65 (s, 1H), 1.53 - 1.50 (m, 2H), 1.43 - 1.39 (m, 2H). LC/MS (ESI) (m/z): 504 [M+H]$^+$. |

(continued)

| Example | Structure and name | Analysis data |
|---|---|---|
| **Example 375** | 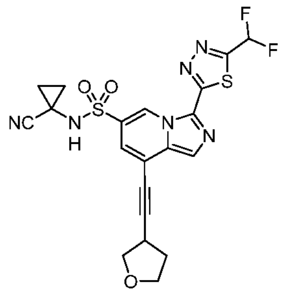 **N-(1-cyanocyclopropyl)-3-(5-(difluoromethyl)-1,3,4-thiadiazol-2-yl)-8-((tetrahydrofuran-3-yl)ethynyl)imidazo [1,5-a]pyridine-6-sulfonamide** | $^1$H NMR (400 MHz, DMSO-$d_6$) δ 9.93 (s, 1H), 9.59 (s, 1H), 8.01 (s, 1H), 7.70 (t, J = 53.1 Hz, 1H), 7.43 (d, J = 1.0 Hz, 1H), 4.07 - 4.01 (m, 1H), 3.95 - 3.88 (m, 1H), 3.85 - 3.76 (m, 2H), 3.52 - 3.45 (m, 1H), 2.41 - 2.31 (m, 1H), 2.16 - 2.08 (m, 1H), 1.55 - 1.48 (m, 2H), 1.42 - 1.35 (m, 2H).LC/MS (ESI) (m/z): 491 [M+H] $^+$. |
| **Example 376** | 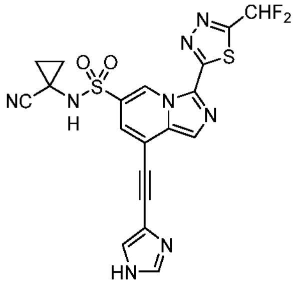 **8-((1H-imidazol-4-yl)ethynyl)-N-(1-cyanocyclopropyl)-3-(5-(difluoromethyl)-1,3,4-thiadiazol-2-yl)imidazo [1,5-a]pyridine-6-sulfonamide** | $^1$H NMR (400 MHz, DMSO-$d_6$) δ 9.94 (s, 1H), 9.63 (s, 1H), 8.08 - 7.55 (m, 4H), 7.50 (d, *J* = 1.3 Hz, 1H), 1.54 - 1.48 (m, 2H), 1.43 - 1.36 (m, 2H). LC/MS (ESI) (m/z): 487 [M+H]$^+$. |
| **Example 377** | 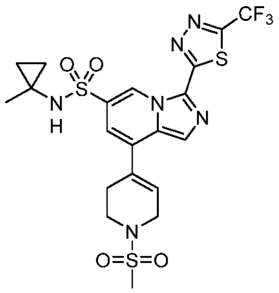 **N-(1-methylcyclopropyl)-8-(1-(methylsulfonyl)-1,2,3,6-tetrahydropyridin-4-yl)-3-(5-(trifluoromethyl)-1,3,4-thiadiazol-2-yl)imidazo[1,5-a]pyridine-6-sulfonamide** | $^1$H NMR (400 MHz, DMSO-$d_6$) δ 9.84 (s, 1H), 8.52 (s, 1H), 8.21 (s, 1H), 7.36 (s, 1H), 6.54 (s, 1H), 4.05 - 3.94 (m, 2H), 3.53 - 3.47 (m, 2H), 3.05 (s, 3H), 2.77 - 2.69 (m, 2H), 1.17 (s, 3H), 0.77 - 0.67 (m, 2H), 0.52 - 0.40 (m, 2H). LC/MS (ESI) (m/z): 563 [M+H]$^+$. |

**Example 378: N-(1-cyanocyclopropyl)-3-(5-(difluoromethyl)-1,3,4-thiadiazol-1-yl)-8-(3-(1-methyl-1H-pyrazol-5-yl)cyclopentyl)-[1,2,4]triazolo[4,3-a]pyridine-6-sulfonamide**

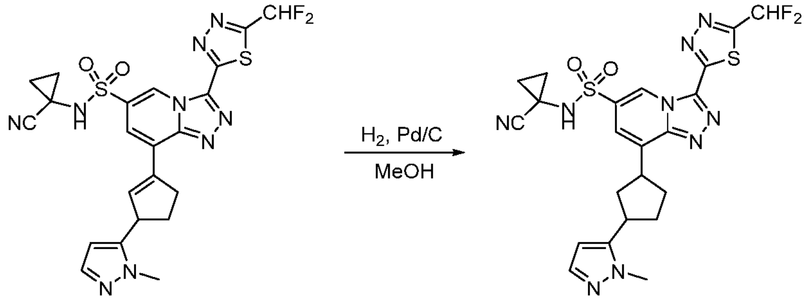

Under an atmosphere of nitrogen, Pd/C (10 mg) with the mass fraction of 10% was added to the solution of N-(1-cyanocyclopropyl)-3-(5-(difluoromethyl)-1,3,4-thiadiazol-2-yl)-8-(3-(1-methyl-1H-pyrazol-5-yl)cyclopent-1-ene-1yl) [1,2,4]triazolo[4,3-a]pyridin-6-sulfonamide (50 mg, 0.09 mmol) in methanol (5 mL). The flask was replaced with nitrogen gas three times and then the mixture was stirred for 2 hours at room temperature under an atmosphere provided by a hydrogen-filled balloon. After completion of the reaction, the reaction mixture was filtered, and the filtrate was concentrated under reduced pressure. The residue was purified by pre-HPLC to obtain the title compound (white solid, 8 mg, yield: 16.1%). $^1$H NMR (400 MHz, DMSO-$d_6$) δ 9.71 (s, 1H), 7.75 (dd, *J* = 60.3, 45.8 Hz, 2H), 7.30 (d, *J* = 1.7 Hz, 1H), 6.16 (d, *J* = 1.8 Hz, 1H), 4.01 - 3.87 (m, 1H), 3.81 (s, 3H), 3.53 - 3.46 (m, 1H), 2.66 - 2.59 (m, 1H), 2.34 - 2.31 (m, 1H), 2.04 - 1.95 (m, 4H), 1.48 - 1.44 (m, 2H), 1.38 - 1.31 (m, 2H). LC/MS (ESI) (m/z): 546 [M+H]$^+$.

**Example 379: N-(1--cyanocyclopropyl)-8-(1-(1-methoxycyclopropane-1-carbonyl)-1,2,3,6-tetrahydropyridin-4-yl)-3-(5-(trifluoromethyl)-1,3,4-thiadiazol-2-yl)imidiazol[1,5-a]pyridine-6-sulfonamide**

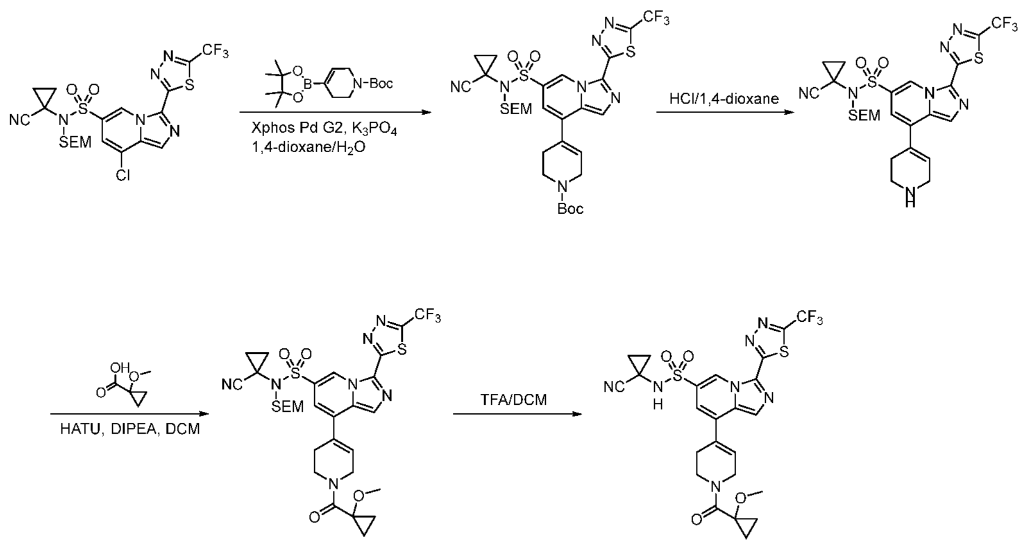


**Step 1: Tert-butyl 4-(6-(N-(1-cyanocyclopropyl)-N-((2-(trimethylsilyl)ethoxy)methyl)sulfamoyl)-3-(5-(trifluoromethyl)-1,3,4-thiadiazol-2-yl)imidazo[1,5-a]pyridin-8-yl)-3,6-dihydropyridine-1(2H)-carboxylate**

Under an atmosphere of nitrogen at room temperature, potassium phosphate (220 mg, 1.04 mmol) and Xphos Pd G2 (4 mg, 0.005 mmol) were sequentially added to the solution of 8-chloro-N-(1-cyanocyclopropyl)-3-(5-(trifluoromethyl)-1,3,4-thiadiazol-2-yl)-N-(2-(trimethylsilicyl)ethoxy)methyl)imidazo[1,5-a]pyridin-6-sulfonamide (300 mg, 0.52 mmol) and 4-(4,4,5,5-tetramethyl-1,3,2-dioxaboran-2-yl)-3,6-dihydropyridin-1(2H)-tert-butyl carboxylate (240 mg, 0.78 mmol) in 1,4-dioxane (6 mL) and water (1 mL). The flask was replaced with nitrogen gas three times and then the mixture was stirred at 85°C for 16 hours under an atmosphere of nitrogen. After completion of the reaction, the reaction mixture was diluted with ethyl acetate, sequentially washed with water and saturated brine, dried over anhydrous sodium sulfate, filtered, and concentrated under reduced pressure. The residue was separated by column chromatography (eluent: petroleum ether/ethyl acetate, gradient: 0-60% ethyl acetate) to obtain the title compound (yellow solid, 330 mg, yield: 51%). LC/MS (ESI) (m/z): 726 [M+H]$^+$.

**Step 2: N-(1-cyanocyclopropyl)-8-(1,2,3,6-tetrahydropyridin-4-yl)-3-(5-(trifluoromethyl)-1,3,4-thiadiazol-2-yl)-N-((2-(trimethylsilyl)ethoxy)methyl)imidazo[1,5-a]pyridine-6-sulfonamide**

At 0°C, 4N hydrochloric acid/1,4-dioxane (5 mL) solution was slowly added to the solution of 4-(6-(N-(1-cyanocyclopropyl)-N-((2-(trimethylsilicyl)ethoxy)methyl)sulfamoyl)-3-(5-(trifluoromethyl)-1,3,4-thiadiazol-2-yl)imidazo [1,5-a]pyridin-8-yl)-3,6-dihydropyridin-1(2H)-tert-butyl carboxylate (100 mg, 0.14 mmol) in 1,4-dioxane (1 mL). The reaction mixture was stirred at room temperature for 2 hours. After completion of the reaction, and concentrated under reduced pressure to obtain a crude title compound (yellow solid, 85 mg, yield: 98.6%), which was directly used in the next step of reaction without purification. LC/MS (ESI) (m/z): 626 [M+H]$^+$.

**Step 3: N-(1-cyanocyclopropyl)-8-(1-(1-methoxycyclopropane-1-carbonyl)-1,2,3,6-tetrahydropyridin-4-yl)-3-(5-(trifluoromethyl)-1,3,4-thiadiazol-2-yl)-N-((2-(trimethylsilyl)ethoxy)methyl)imidazo[1,5-a]pyridine-6-sulfonamide**

At 0°C, HATU (65 mg, 0.17 mmol) and DIEA (40 mg, 0.33 mmol) were added to the solution of N-(1-cyanocyclopropyl)-8-(1,2,3,6-tetrahydropyridin-4-yl)-3-(5(trifluoromethyl)-1,3,4-thiadiazol-2-yl)-N-((2(trimethylsilicyl) ethoxy)methyl)imidazo[1,5-a]pyridin-6-sulfonamide (85 mg, 0.14 mmol) and 1-hydroxycyclopropan-1-formic acid (24 mg, 0.20 mmol) in dichloromethane (2 mL). The reaction mixture was stirred at 0°C for 1 hours. After completion of the reaction, the reaction mixture was diluted with dichloromethane, sequentially washed with water and saturated brine, dried over anhydrous sodium sulfate, filtered and concentrated under reduced pressure. The residue was separated by column chromatography (eluent: petroleum ether/ethyl acetate, gradient: 0-60% ethyl acetate) to obtain the title compound (yellow solid, 70 mg, yield: 71%). LC/MS (ESI) (m/z): 724 $[M+H]^+$.

**Step 4: N-(1-cyanocyclopropyl)-8-(1-(1-methoxycyclopropane-1-carbonyl)-1,2,3,6-tetrahydropyridin-4-yl)-3-(5-(trifluoromethyl)-1,3,4-thiadiazol-2-yl)imidazo[1,5-a]pyridine-6-sulfonamide**

At 0°C, trifluoroacetic acid (0.3 mL) was dropwise added slowly to the solution of N-(1-cyanocyclopropyl)-8-)1-)1-methoxycyclopropan-1-carbonyl)-1,2,3,6-tetrahydropyridin-4-yl)-3-)5-)trifluoromethyl)-1,3,4-thiadiazol-2-yl)-N-))2-)trimethylsilicyl)ethoxy)methyl)imidazo[1,5-a]pyridin-6-sulfonamide (70 mg, 0.10 mmol) in dichloromethane (1 mL). The mixture was stirred at room temperature for 30 minutes. The mixture was concentrated, and the residue was purified by pre-HPLC to obtain the title compound (yellow solid, 18 mg, yield: 31.6%). $^1$H NMR (400 MHz, DMSO-$d_6$) $\delta$ 9.94 (s, 1H), 9.58 (s, 1H), 8.23 (s, 1H), 7.36 (s, 1H), 6.60 (s, 1H), 4.65 - 4.17 (m, 2H), 4.07 - 3.72 (m, 2H), 3.27 (s, 3H), 2.68 (s, 2H), 1.58 - 1.47 (m, 2H), 1.43 - 1.35 (m, 2H), 1.07 - 1.00 (m, 2H), 0.99 - 0.91 (m, 2H). LC/MS (ESI) (m/z): 594 $[M+H]^+$.

**Example 380 : N-(1-cyanocyclopropyl)-8-(1-(1-methoxycyclopropane-1-carbonyl)piperidin-4-yl)-3-(5-(trifluoromethyl)-1,3,4-thiadiazol-2-yl)imidazo[1,5-a]pyridine-6-sulfonamide**

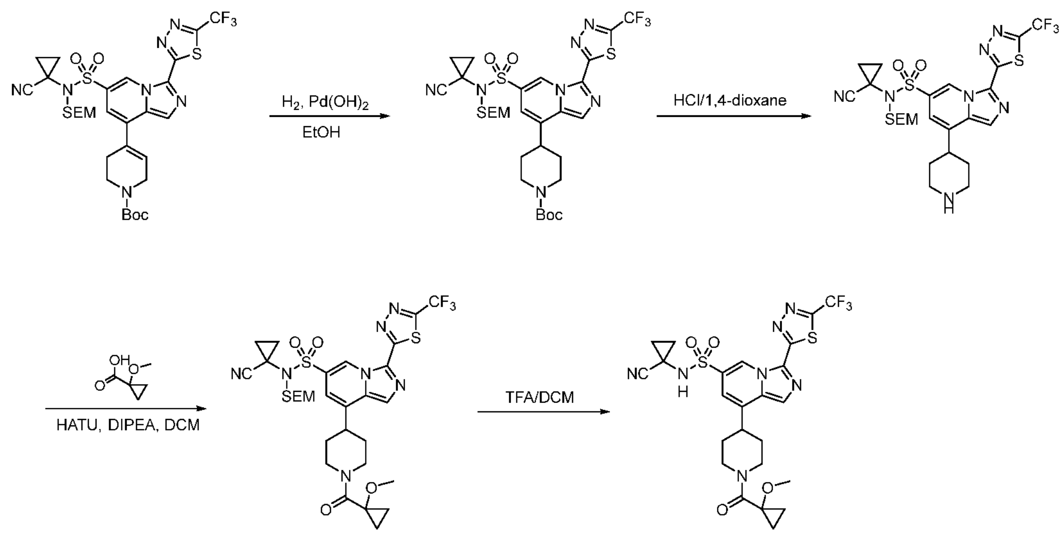


**Step 1: Tert-butyl 4-(6-(N-(1-cyanocyclopropyl)-N-((2-(trimethylsilyl)ethoxy)methyl)sulfamoyl)-3-(5-(trifluoromethyl)-1,3,4-thiadiazol-2-yl)imidazo[1,5-a]pyridin-8-yl)piperidine-1-carboxylate**

Under an atmosphere of nitrogen, Pd/C (12 mg, mass fraction: 10%) was slowly added to the solution of 4-(6-(N-(1-cyanocyclopropyl)-N-((2-(trimethylsilicyl)ethoxy)methyl)sulfamyl)-3-(5-(trifluoromethyl)-1,3,4-thiadiazol-2-yl) imidazo[1,5-a]pyridin-8-yl)-3,6-dihydropyridin-1(2H)-tert-butyl carboxylate (270 mg, 0.37 mmol) in ethanol (5 mL). The flask was replaced with nitrogen gas three times and then the mixture was stirred for 9 hours at room temperature under an atmosphere provided by a hydrogen-filled balloon. After completion of the reaction, the mixture was filtered, and the filtrate was concentrated under reduced pressure to obtain the title compound (white solid, 220 mg, yield: 81.5%). LC/MS (ESI) m/z: 728 $[M+H]^+$.

**Step 2: N-(1-cyanocyclopropyl)-8-(piperidin-4-yl)-3-(5-(trifluoromethyl)-1,3,4-thiadiazol-2-yl)-N-((2-(trimethylsilyl)ethoxy)methyl)imidazo[1,5-a]pyridine-6-sulfonamide**

At 0°C, 4N hydrochloric acid/1,4-dioxane soltion (5 mL) was slowly added to the solution of 4-(6-(N-(1-cyanocyclopropyl)-N-((2-(trimethylsilicyl)ethoxy)methyl)sulfamoyl)-3-(5-(trifluoromethyl)-1,3,4-thiadiazol-2-yl)imidazo [1,5-a]pyridin-8-yl)piperidin-1-tert-butyl formate (220 mg, 0.30 mmol) in 1,4-dioxane (1 mL). The reaction mixture was stirred at room temperature for 2 hours. After completion of the reaction, the reaction mixture was concentrated under reduced pressure to obtain a crude title compound (yellow solid, 180 mg, yield: 95.2%), which was directly used in the next step of reaction without purification LC/MS (ESI) (m/z): 628 $[M+H]^+$.

**Step 3: N-(1-cyanocyclopropyl)-8-(1-(1-methoxycyclopropane-1-carbonyl)piperidin-4-yl)-3-(5-(trifluoromethyl)-1,3,4-thiadiazol-2-yl)-N-((2-(trimethylsilyl)ethoxy)methyl)imidazo[1,5-a]pyridine-6-sulfonamide**

At 0°C, HATU (65 mg, 0.17 mmol) and DIEA (40 mg, 0.33 mmol) were added to the solution of N-(1-cyanocyclopropyl)-8-(piperidin-4-yl)-3-(5-(trifluoromethyl)-1,3,4-thiadiazol-2-yl)-N-((2-(trimethylsilanyl)ethoxy)methyl) imidazo[1,5-a]pyridin-6-sulfonamide (70 mg, 0.11 mmol) and 1-hydroxycyclopropan-1-formic acid (25 mg, 0.22 mmol) in dichloromethane (2 mL). The reaction mixture was stirred at 0°C for 1 hours. After completion of the reaction, the reaction mixture was diluted with dichloromethane, sequentially washed with water and saturated brine, dried over anhydrous sodium sulfate, filtered and concentrated under reduced pressure. The residue was separated by column chromatography (eluent: petroleum ether/ethyl acetate, gradient: 0-50% ethyl acetate) to obtain the title compound (yellow solid, 40 mg, yield: 50%). LC/MS (ESI) (m/z): 726 $[M+H]^+$.

**Step 4: N-(1-cyanocyclopropyl)-8-(1-(1-methoxycyclopropane-1-carbonyl)piperidin-4-yl)-3-(5-(trifluoromethyl)-1,3,4-thiadiazol-2-yl)imidazo[1,5-a]pyridine-6-sulfonamide**

At 0°C, trifluoroacetic acid (0.3 mL) was added to the solution of N-(1-cyanocyclopropyl)-8-(1-(1-methoxycyclopropan-1-carbonyl)piperidin-4-yl)-3-(5-(trifluoromethyl)-1,3,4-thiadiazol-2-yl)-N-((2-(trimethylsilanyl)ethoxy)methyl) imidazo[1,5-a]pyridin-6-sulfonamide (40 mg, 0.06 mmol) in dichloromethane (1 mL). The reaction mixture was stirred at room temperature for 1 hours. After completion of the reaction, the reaction mixture was concentrated under reduced pressure; and the residue was purified by pre-HPLC to obtain the title compound (yellow solid, 5 mg, yield: 16%). $^1H$ NMR (400 MHz, $CDCl_3$) δ 10.20 (s, 1H), 7.86 (s, 1H), 7.28 (s, 1H), 6.31 (s, 1H), 4.81 (d, *J* = 12.0 Hz, 2H), 3.37 (s, 3H), 3.33 - 3.13 (m, 2H), 3.06 - 2.81 (m, 1H), 2.16 (d, *J* = 12.7 Hz, 2H), 1.91 - 1.78 (m, 2H), 1.74 - 1.67 (m, 2H), 1.54 - 1.46 (m, 2H), 1.17 - 1.11 (m, 2H), 1.02 - 0.94 (m, 2H). LC/MS (ESI) (m/z): 596 $[M+H]^+$.

**Example 381: 2-(1-(4-(6-(N-(1-cyanocyclopropyl)sulfamoyl)-3-(5-(difluoromethyl)-1,3,4-thiadiazol-2-yl)imidazo [1,5-a]pyridin-8-yl)piperazine-1-carbonyl)cyclopropoxy)acetic acid**

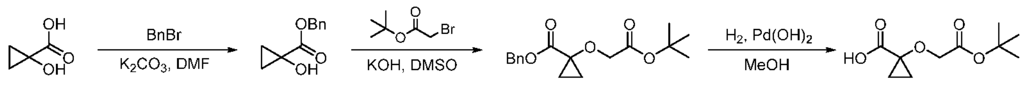


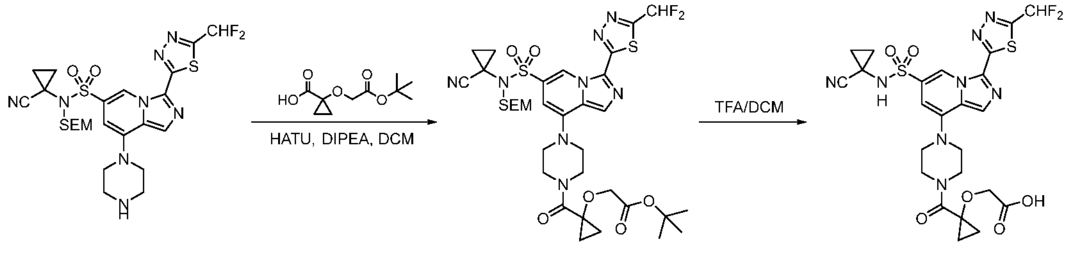


**Step 1: Benzyl 1-hydroxycyclopropane-1-carboxylate**

At 0°C, potassium carbonate (7 g, 50.7 mmol) and benzyl bromide (4 g, 23.5 mmol) were added to the solution of 1-hydroxycyclopropan-1-formic acid (2 g, 19.6 mmol) in N,N-dimethylformamide (20 mL). The reaction mixture was stirred

for 3 hours at room temperature under an atmosphere of nitrogen. After completion of the reaction, the saturated ammonium chloride solution was used for quenching; and the reaction mixture was extracted with ethyl acetate, washed with saturated brine, dried over anhydrous sodium sulfate, and concentrated under reduced pressure. The residue was separated by column chromatography (eluent: petroleum ether/ethyl acetate, gradient: 0-10% ethyl acetate) to obtain the title compound (colorless liquid, 3 g, yield: 79.7%). $^1$H NMR (400 MHz, $CDCl_3$) δ 7.35 - 7.21 (m, 5H), 5.10 (s, 2H), 4.04 (q, *J* = 7.1 Hz, 1H), 1.25 (dd, *J* = 8.0, 4.9 Hz, 2H), 1.09 (dd, *J* = 8.0, 4.9 Hz, 2H).

**Step 2: Benzyl 1-(2-(tert-butoxy)-2-oxoethoxy)cyclopropane-1-carboxylate**

At 0°C, potassium hydroxide (520 mg, 9.25 mmol) and 2-bromotert-butyl acetate (1.2 g, 6.01 mmol) were sequentially added to the solution of 1-hydroxycyclopropan-1-benzyl formate (1 g, 4.62 mmol) in dimethyl sulfoxide (10 mL). The reaction mixture was stirred at 0°C for 3 hours. After completion of the reaction, the reaction mixture was diluted with ethyl acetate, washed with saturated brine, dried over anhydrous sodium sulfate, and concentrated under reduced pressure. The residue was separated by column chromatography (eluent: petroleum ether, ethyl acetate, gradient: 0-5% ethyl acetate) to obtain the title compound (colorless liquid, 1.2 g, yield: 84.7%). $^1$H NMR (400 MHz, $CDCl_3$) δ 7.41 - 7.27 (m, 5H), 5.17 (s, 2H), 4.15 (s, 2H), 1.45 (s, 9H), 1.35 - 1.28 (m, 4H).

**Step 3: 1-(2-(tert-butoxy)-2-oxoethoxy)cyclopropane-1-carboxylic acid**

At room temperature, palladium hydroxide on carbon (50 mg, mass fraction: 10%) were added to the solution of 1-(2-(tert-butoxy)-2-oxoethoxy)cyclopropan-1-benzyl formate (600 mg, 1.96 mmol) in methanol (8 mL). The flask was replaced with nitrogen gas three times and then the mixture was stirred for 2 hours at room temperature under an atmosphere provided by a hydrogen-filled balloon. After completion of the reaction, the reaction mixture was filtered; and the filtrate was concentrated under reduced pressure to obtain the title compound (white solid, 400 mg, yield: 94.4%), which was directly used in the next step without purification. LC/MS (ESI) (m/z): 217 $[M+H]^+$.

**Step 4: Tert-butyl 2-(1-(4-(6-(N-(1-cyanocyclopropyl)-N-((2-(trimethylsilyl)ethoxy)methyl)sulfamoyl)-3-(5-(difluoromethyl)-1,3,4-thiadiazol-2-yl)imidazo[1,5-a]pyridin-8-yl)piperazine-1-carbonyl)cyclopropoxy)acetate**

At room temperature, 1-(2-(tert-butoxy)-2-oxoethoxy)cyclopropan-1-formic acid (39 mg, 0.180 mmol), N,N-diisopropylethylamine (29 mg, 0.225 mmol)and 2-(7-azobenzotriazol)-N,N,N',N'-tetramethylurea hexafluorophosphate (41 mg, 0.108 mmol) were added to the solution of N-(1-cyanocyclopropyl)-3-(5-(difluoromethyl)-1,3,4-thiadiazol-2-yl)-8-(piperazin-1-yl)-N-(2-(trimethylsilanyl)ethoxy)methyl)imidazo[1,5-a]pyridin-6-sulfonamide (55 mg, 0.090 mmol) in N,N-dimethylformamide (2 mL). The reaction mixture was stirred at room temperature for 0.5 hours under an atmosphere of nitrogen. After completion of the reaction, the reaction mixture was diluted with ethyl acetate, sequentially washed with water and saturated brine, dried over anhydrous sodium sulfate, and concentrated under reduced pressure. The residue was separated by column chromatography (eluent: petroleum ether, ethyl acetate, gradient: 0-35% ethyl acetate) to obtain the title compound (yellow solid, 55 mg, yield: 75.5%). LC/MS (ESI) (m/z): 809 $[M+H]^+$.

**Step 5: 2-(1-(4-(6-(N-(1-cyanocyclopropyl)sulfamoyl)-3-(5-(difluoromethyl)-1,3,4-thiadiazol-2-yl)imidazo[1,5-a]pyridin-8-yl)piperazine-1-carbonyl)cyclopropoxy)acetic acid**

At 0°C, trifluoroacetic acid (1 mL) was added to the solution of 2-(1-(4-(6-(N-(1-cyanocyclopropyl)-N-((2-(trimethylmethylsilyl)ethoxy)methyl)aminosulfonyl)-3-(5-(difluoromethyl)-1,3,4-thiadiazol-2-yl)imidazo[1,5-a]pyridin-8-yl) piperazin-1-carbonyl)cyclopropoxy)tert-butyl acetate (55 mg, 0.068 mmol) in dichloromethane (1 mL). The reaction mixture was stirred at room temperature for 2 hours. After completion of the reaction, the reaction mixture was dripped to 5% aqueous sodium bicarbonate solution cooled in the ice water bath, and extracted with dichloromethane. The organic phase was combined, washed with saturated brine, dried over anhydrous sodium sulfate, and concentrated under reduced pressure. The residue was separated by pre-HPLC (C18, 10-50% acetonitrile in water containing 0.1% FA) to obtain the title compound (yellow solid, 15 mg, yield: 35.4%). $^1$H NMR (400 MHz, DMSO-$d_6$) δ 9.68 (s, 1H), 8.12 (s, 1H), 7.69 (t, *J* = 53.2 Hz, 1H), 6.68 (s, 1H), 4.05 (s, 2H), 3.98 - 3.63 (m, 4H), 3.53 - 3.37 (m, 4H), 1.50 - 1.47 (m, 2H), 1.40 - 1.36 (m, 2H), 1.07 - 1.03 (m, 4H). LC/MS (ESI) (m/z): 623 $[M+H]^+$.

**Example 382: 2-(1-(4-(3-(5-(difluoromethyl)-1,3,4-thiadiazol-2-yl)-6-(N-(1-methylcyclopropyl)sulfamoyl)imidazo[1,5-a]pyridin-8-yl)piperazine-1-carbonyl)cyclopropoxy)acetic acid**

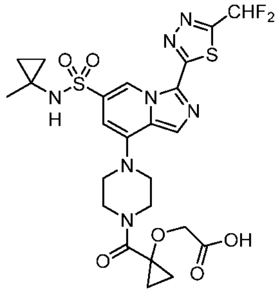

The title compound was obtained by referring to the preparation method in **Example 381.** $^1H$ NMR (400 MHz, DMSO-$d_6$) δ 9.61 (s, 1H), 8.44 (s, 1H), 8.09 (s, 1H), 7.69 (t, $J$ = 53.1 Hz, 1H), 6.70 (s, 1H), 4.05 (s, 2H), 3.98 - 3.59 (m, 4H), 3.61 - 3.10 (m, 4H), 1.16 (s, 3H), 1.09 - 0.99 (m, 4H), 0.80 - 0.68 (m, 2H), 0.52 - 0.40 (m, 2H). LC/MS (ESI) (m/z): 612 [M+H]$^+$.

**Example 383: 8-(4-(1-(2-hydroxyethoxy)cyclopropane-1-carbonyl)piperazin-1-yl)-N-(1-methylcyclopropyl)-3-(5-(trifluoromethyl)-1,3,4-thiadiazol-2-yl)imidazo [1,5-a]pyridine-6-sulfonamide**

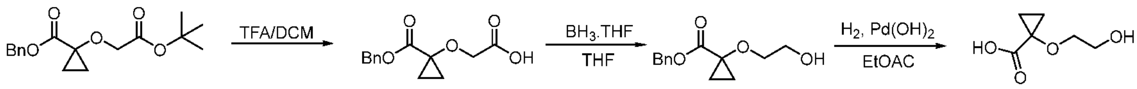


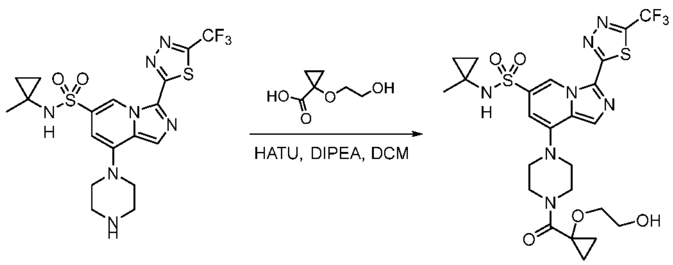


**Step 1: 2-(1-((benzyloxy)carbonyl)cyclopropoxy)acetic acid**

At 0°C, trifluoroacetic acid (4.5 mL) was added to the solution of 1-(2-(tert-butoxy)-2-oxoethoxy)cyclopropan-1-benzyl formate (1.8 g, 5.87 mmol) in dichloromethane (9 mL). The reaction mixture was stirred at room temperature for 2 hours. After completion of the reaction, the reaction mixture was concentrated under reduced pressure to obtain the title compound (yellow liquid, 1.5 g, yield: 100%). LC/MS (ESI) (m/z): 251 [M+H]$^+$.

**Step 2: Benzyl 1-(2-hydroxyethoxy)cyclopropane-1-carboxylate**

At 0°C, borane tetrahydrofuran (11.7 mL, 11.7 mmol, 1M) solution was dropwise added slowly to the solution of 2-(1-(benzyloxy)carbonyl)cyclopropoxy)acetic acid (1.5 g, 5.87 mmol) in tetrahydrofuran (10 mL). The reaction mixture was stirred at 65°C for one hour. After completion of the reaction, the reaction mixture was quenched by dropwise adding methanol in an ice water bath. The reaction mixture was concentrated under reduced pressure. The residue was separated by column chromatography (eluent: petroleum ether/ethyl acetate, gradient: 0-15% ethyl acetate) to obtain the title compound (colorless liquid, 900 mg, yield: 64.8%). LC/MS (ESI) (m/z): 237 [M+H]$^+$.

**Step 3: 1-(2-hydroxyethoxy)cyclopropane-1-carboxylic acid**

At room temperature, palladium hydroxide (10 mg, mass fraction: 10%) was added to the solution of 1-(2-hydroxyethoxy)cyclopropan-1-benzyl formate (50 mg, 2.12 mmol) in ethyl acetate (2 mL). The flask was replaced with nitrogen gas three times and then the mixture was stirred for 2 hours at room temperature under an atmosphere provided by a hydrogen-filled balloon. After completion of the reaction, the reaction mixture was filtered; and the filtrate was concentrated under reduced pressure to obtain the title compound (colorless liquid, 30 mg, yield: 97.0%), which was

directly used in the next step without purification. LC/MS (ESI) (m/z): 147 $[M+H]^+$.

**Step 4: 8-(4-(1-(2-hydroxyethoxy)cyclopropane-1-carbonyl)piperazin-1-yl)-N-(1-methylcyclopropyl)-3-(5-(trifluoromethyl)-1,3,4-thiadiazol-2-yl)imidazo[1,5-a]pyridine-6-sulfonamide**

At room temperature, 1-(2-hydroxyethoxy)cyclopropan-1-formic acid (23 mg, 0.206 mmol), N,N-diisopropylethylamine (33 mg, 0.256 mmol) and 2-(7-azobenzotriazol)-N,N,N',N'-tetramethylurea hexafluorophosphate (47 mg, 0.123 mmol) were added to the solution of N-(1-methylcyclopropyl)-8-(piperazin-1-yl)-3-(5-(trifluoromethyl)-1,3,4-thiadiazol-2-yl)imidazo[1,5-a]pyridin-6-sulfonamide (50 mg, 0.103 mmol) in N,N-dimethylformamide (2 mL). The reaction mixture was stirred at room temperature for 4 hours under an atmosphere of nitrogen. After completion of the reaction, the reaction mixture was diluted with ethyl acetate, sequentially washed with water and saturated brine, dried over anhydrous sodium sulfate, and concentrated under reduced pressure. The residue was separated by pre-HPLC (C18, 10-50% acetonitrile in water containing 0.1% FA) to obtain the title compound (yellow solid, 16 mg, yield: 25.2%). $^1H$ NMR (400 MHz, DMSO-$d_6$) δ 9.58 (s, 1H), 8.45 (s, 1H), 8.13 (s, 1H), 6.73 (s, 1H), 4.62 (t, *J* = 5.1 Hz, 1H), 4.18 - 3.65 (m, 4H), 3.51 - 3.45 (m, 4H), 3.43 - 3.33 (m, 4H), 1.16 (s, 3H), 1.08 - 1.00 (m, 2H), 0.98 - 0.90 (m, 2H), 0.77 - 0.69 (m, 2H), 0.49 - 0.41 (m, 2H). LC/MS (ESI) (m/z): 616 $[M+H]^+$.

**Example 384: 8-(4-(1-(2-(dimethylamino)ethoxy)cyclopropane-1-carbonyl)piperazin-1-yl)-N-(1-methylcyclopropyl)-3-(5-(trifluoromethyl)-1,3,4-thiadiazol-2-yl)imidazo[1,5-a]pyridine-6-sulfonamide**

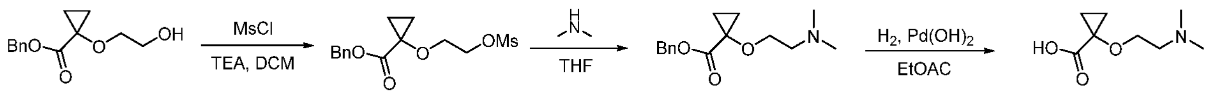


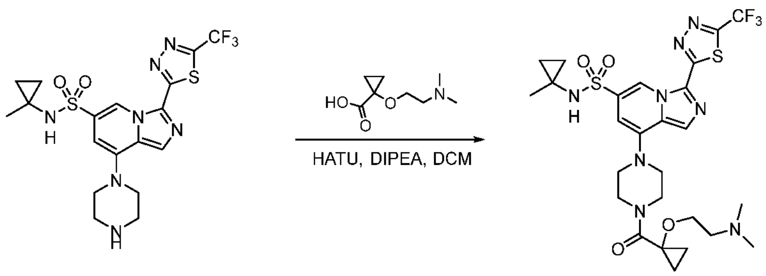


**Step 1: Benzyl 1-(2-((methylsulfonyl)oxy)ethoxy)cyclopropane-1-carboxylate**

At 0°C, triethylamine (684 mg, 6.77 mmol) and methylsulfonyl chloride (503 mg, 4.39 mmol) were added to the solution of 1-(2-hydroxyethoxy)cyclopropan-1-benzyl formate (800 mg, 3.38 mmol) in dichloromethane (8 mL). The reaction mixture was stirred at 0°C for 30 minutes. After completion of the reaction, the reaction mixture was washed with saturated brine, dried over anhydrous sodium sulfate, and concentrated under reduced pressure to obtain the title compound (colorless liquid, 888 mg, yield: 83.5%), which was directly used in the next step without purification. LC/MS (ESI) (m/z): 315 $[M+H]^+$.

**Step 2: Benzyl 1-(2-(dimethylamino)ethoxy)cyclopropane-1-carboxylate**

At 0°C, the solution of 2M dimethylamine in tetrahydrofuran (5.6 mL, 5.64 mmol) was added to the solution of 1-(2-((methylsulfonyl)oxy)ethoxy)cyclopropan-1-benzyl formate (888 mg, 2.82 mmol) in tetrahydrofuran (5 mL). The reaction mixture was allowed to react under a sealed condition at 65°C for 3 hours. After completion of the reaction, the reaction mixture was concentrated under reduced pressure. The residue was separated by column chromatography (eluent: dichloromethane/methanol, gradient: 0-8% methanol) to obtain the title compound (colorless liquid, 500 mg, yield: 67.2%). LC/MS (ESI) (m/z): 264 $[M+H]^+$.

**Step 3: 1-(2-(dimethylamino)ethoxy)cyclopropane-1-carboxylic acid**

At room temperature, palladium hydroxide (20 mg, mass fraction: 10%) was added to the solution of 1-(2-(dimethylamino)ethoxy)cyclopropan-1-benzyl formate (150 mg, 0.570 mmol) in ethyl acetate (3 mL). The flask was replaced with nitrogen gas three times and then the mixture was stirred for 2 hours at room temperature under an atmosphere provided by a hydrogen-filled balloon. After completion of the reaction, the reaction mixture was filtered; and the filtrate was concentrated under reduced pressure to obtain the title compound (colorless liquid, 90 mg, yield: 91.2%), which was directly used in the next step without purification. LC/MS (ESI) (m/z): 174 $[M+H]^+$.

**Step 4: 8-(4-(1-(2-(dimethylamino)ethoxy)cyclopropane-1-carbonyl)piperazin-1-yl)-N-(1-methylcyclopropyl)-3-(5-(trifluoromethyl)-1,3,4-thiadiazol-2-yl)imidazo[1,5-a]pyridine-6-sulfonamide**

At room temperature, 1-(2-(dimethylamino)ethoxy)cyclopropan-1-formic acid (39 mg, 0.266 mmol), N,N-diisopropylethylamine (36 mg, 0.282 mmol)and 2-(7-azobenzotriazol)-N,N,N',N'-tetramethylurea hexafluorophosphate (51 mg, 0.135 mmol) were sequentially added to the solution of N-(1-methylcyclopropyl)-8-(piperazin-1-yl)-3-(5-(trifluoromethyl)-1,3,4-thiadiazol-2-yl)imidazo[1,5-a]pyridin-6-sulfonamide (55 mg, 0.133 mmol) in N,N-dimethylformamide (2 mL). The reaction mixture was stirred at room temperature for 4 hours under an atmosphere of nitrogen. After completion of the reaction, the reaction mixture was diluted with ethyl acetate, sequentially washed with water and saturated brine, dried over anhydrous sodium sulfate, and concentrated under reduced pressure. The residue was separated by pre-HPLC (C18, 10-50% acetonitrile in water containing 0.1% FA) to obtain the title compound (yellow solid, 28 mg, yield: 38.5%). $^1H$ NMR (400 MHz, DMSO-$d_6$) δ 9.58 (s, 1H), 8.45 (s, 1H), 8.13 (s, 1H), 6.74 (s, 1H), 4.13 - 3.68 (m, 4H), 3.55 - 3.50 (m, 2H), 3.42 - 3.36 (m, 4H), 2.48 - 2.43 (m, 2H), 2.18 (s, 6H), 1.16 (s, 3H), 1.07 - 0.99 (m, 2H), 0.99 - 0.92 (m, 2H), 0.75 - 0.68 (m, 2H), 0.50 - 0.41 (m, 2H). LC/MS (ESI) (m/z): 643 $[M+H]^+$.

**Example 385: 8-(4-(1-(3-(dimethylamino)propoxy)cyclopropane-1-carbonyl)piperazin-1-yl)-N-(1-methylcyclopropyl)-3-(5-(trifluoromethyl)-1,3,4-thiadiazol-2-yl)imidazo[1,5-a]pyridine-6-sulfonamide**

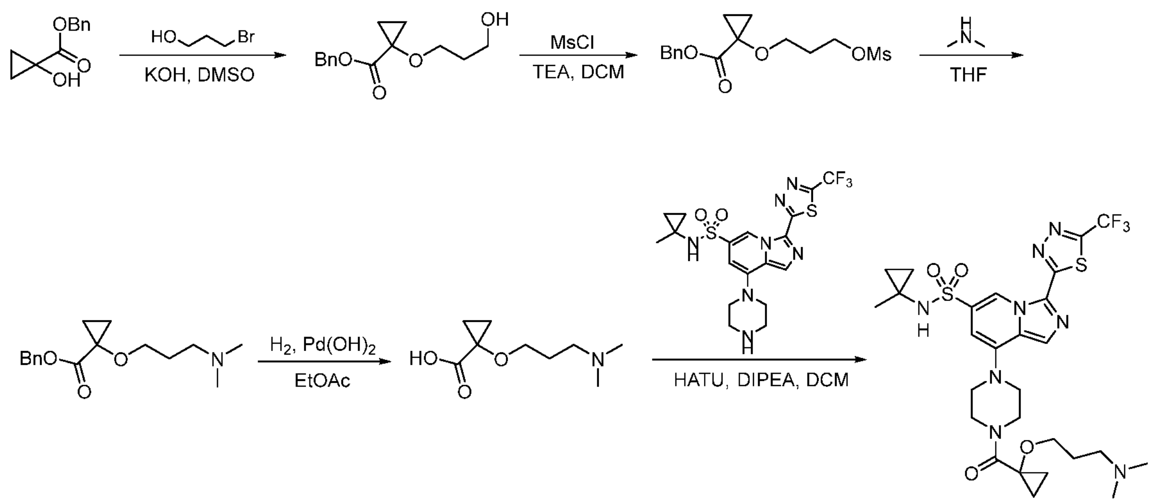


**Step 1: Benzyl 1-(3-hydroxypropoxy)cyclopropane-1-carboxylate**

At 0°C, potassium hydroxide (525 mg, 9.36 mmol) and 3-bromopropan-1-ol (840 mg, 6.08 mmol) were added to the solution of 1-hydroxycyclopropan-1-benzyl carboxylate (900 mg, 4.68 mmol) in dimethyl sulfoxide (9 mL). The reaction mixture was stirred at room temperature for 3 hours. After completion of the reaction, the reaction mixture was diluted with ethyl acetate, sequentially washed with water and saturated brine, dried over anhydrous sodium sulfate, and concentrated under reduced pressure. The residue was separated by column chromatography (eluent: petroleum ether, ethyl acetate, gradient: 0-45% ethyl acetate) to obtain the title compound (colorless liquid, 900 mg, yield: 76.8%). LC/MS (ESI) (m/z): 251 $[M+H]^+$.

**Step 2: Benzyl 1-(3-((methylsulfonyl)oxy)propoxy)cyclopropane-1-carboxylate**

At 0°C, triethylamine (910 mg, 8.99 mmol) and methylsulfonyl chloride (540 mg, 4.67 mmol) were added to the solution of 1-(3-hydroxypropoxy)cyclopropan-1-benzyl carboxylate (900 mg, 3.60 mmol) in dichloromethane (9 mL). The reaction mixture was stirred at 0°C for 30 minutes. After completion of the reaction, the reaction mixture was diluted with dichloromethane, washed with saturated brine, dried over anhydrous sodium sulfate, and concentrated under reduced pressure to obtain the title compound (colorless liquid, 570 mg, yield: 48.2%), which was directly used in the next step without purification. LC/MS (ESI) (m/z): 329 $[M+H]^+$.

**Step 3: Benzyl 1-(3-(dimethylamino)propoxy)cyclopropane-1-carboxylate**

At 0°C, 2 M dimethylaminetetrahydrofuran (5 mL) solution was added to the solution of 1-(3-((methylsulfonyl)oxy) propoxy)cyclopropan-1-benzyl carboxylate (570 mg, 1.74 mmol) in tetrahydrofuran (2 mL). The reaction mixture was allowed to react under a sealed condition at 65°C for 3 hours. After completion of the reaction, the reaction mixture was concentrated under reduced pressure. The residue was separated by column chromatography (eluent: dichloromethane/methanol, gradient: 0-8% methanol) to obtain the title compound (colorless liquid, 217 mg, yield: 45.1%). LC/MS (ESI) (m/z): 278 $[M+H]^+$.

**Step 4: 1-(3-(dimethylamino)propoxy)cyclopropane-1-carboxylic acid**

At room temperature, palladium hydroxide (20 mg, mass fraction: 10%) was added to the solution of 1-(3-(dimethylamido)propoxy)cyclopropan-1-benzyl carboxylate (217 mg, 0.78 mmol) in ethyl acetate (3 mL). The reaction mixture was ventilated three times under an atmosphere provided by a hydrogen-filled balloon and then stirred for 2 hours at room temperature under hydrogen atmosphere. After completion of the reaction, the reaction mixture was filtered; and the filtrate of the reaction mixture was concentrated under reduced pressure to obtain a crude title compound (colorless liquid, 140 mg, yield: 95.5%), which was directly used in the next step without purification. LC/MS (ESI) (m/z): 188 $[M+H]^+$.

**Step 5: 8-(4-(1-(3-(dimethylamino)propoxy)cyclopropane-1-carbonyl)piperazin-1-yl)-N-(1-methylcyclopropyl)-3-(5-(trifluoromethyl)-1,3,4-thiadiazol-2-yl)imidazo [1,5-a]pyridine-6-sulfonamide**

At room temperature, 1-(3-(dimethylamino)propoxy)cyclopropan-1-carboxylic acid (32 mg, 0.17 mmol), N,N-diisopropylethylamine (55 mg, 0.43 mmol) and 2-(7-azobenzotriazol)-N,N,N',N'-tetramethylurea hexafluorophosphate (82 mg, 0.21 mmol) were added to the solution of N-(1-methylcyclopropyl)-8-(piperazin-1-yl)-3-(5-(trifluoromethyl)-1,3,4-thiadiazol-2-yl)imidazo[1,5-a]pyridin-6-sulfonamide (70 mg, 0.14 mmol) in N,N-dimethylformamide (2 mL). The reaction mixture was stirred at room temperature for 4 hours under an atmosphere of nitrogen. After completion of the reaction, the reaction mixture was diluted with ethyl acetate, sequentially washed with water and saturated brine, dried over anhydrous sodium sulfate, and concentrated under reduced pressure. The residue was separated by pre-HPLC (C18, 10-50% acetonitrile in water containing 0.1% FA) to obtain the title compound (yellow solid, 26 mg, yield: 27.5%). $^1H$ NMR (400 MHz, $CD_3OD$) δ 9.81 (s, 1H), 7.96 (s, 1H), 6.77 (s, 1H), 4.23 - 3.85 (m, 4H), 3.61 - 3.57 (m, 2H), 3.44 - 3.39 (m, 4H), 2.76 - 2.69 (m, 2H), 2.51 (s, 6H), 1.89 - 1.82 (m, 2H), 1.27 (s, 3H), 1.15 - 1.10 (m, 2H), 1.06 - 1.02 (m, 2H), 0.85 - 0.81 (m, 2H), 0.51 (m, 2H). LC/MS (ESI) (m/z): 657 $[M+H]^+$.

**Example 386: (R)-N-cyclopropyl-8-(3-methyl-4-(1-methylcyclopropane-1-carbonyl)piperazin-1-yl)-3-(5-(trifluoromethyl)-1,3,4-thiadiazol-2-yl)-[1,2,4]triazolo[4,3-a]pyridine-6-sulfonamide**

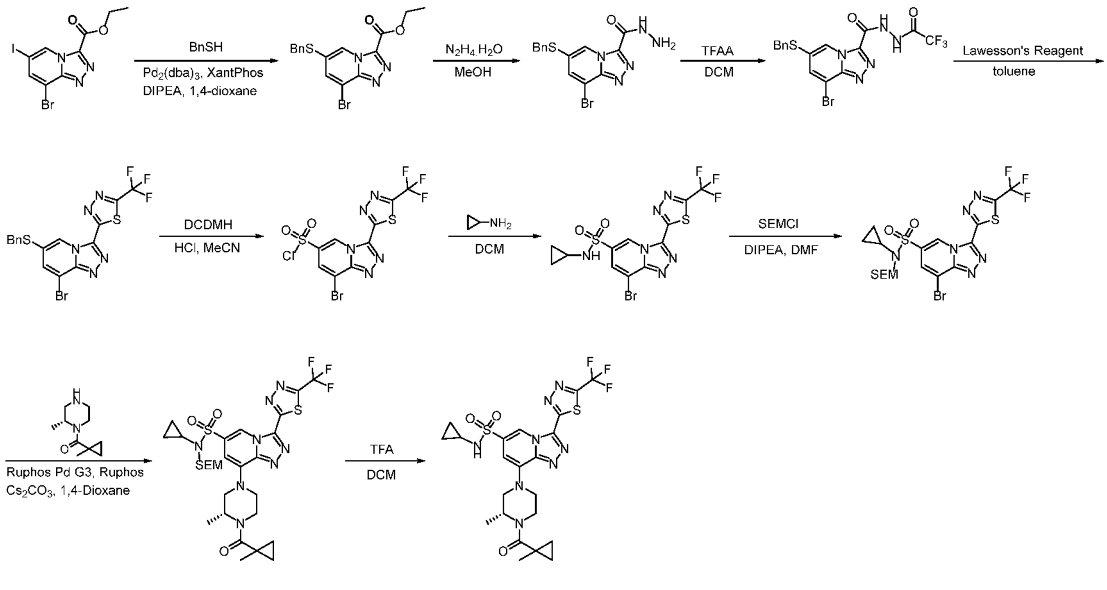


**Step 1: Ethyl 6-(benzylthio)-8-bromo-[1,2,4]triazolo[4,3-a]pyridine-3-carboxylate**

Under an atmosphere of nitrogen, N,N-diisopropylethylamine (2.0 g, 15.7 mmol), XantPhos (510 g, 0.503 mmol), $Pd_2(dba)_3$ (480 mg, 0.503 mmol) were sequentially added to the solution of 8-bromo-6-iodo-[1,2,4]triazolo[4,3-a]pyridin-3-ethyl carboxylate (2 g, 5.25 mmol) and phenylmethyl mercaptan (0.65 g, 5.25 mmol) in 1,4-dioxane (40 mL). The flask was replaced with nitrogen gas three times and then the mixture was stirred at 80 °C for 4 hours under an atmosphere of nitrogen. After completion of the reaction, water was added, and the reaction mixture was extracted with ethyl acetate. The organic phase was washed with saturated brine, dried over anhydrous sodium sulfate, and concentrated under reduced pressure. The residue was purified by column chromatography (eluent: petroleum ether, ethyl acetate, gradient: 0-30% ethyl acetate) to obtain the title compound (yellow solid, 1.5 g, yield: 78%). LC/MS (ESI) (m/z): 392 $[M+H]^+$.

**Step 2: 6-(benzylthio)-8-bromo-[1,2,4]triazolo[4,3-a]pyridine-3-carbohydrazide**

Under an atmosphere of nitrogen, hydrazine hydrate (660 mg, 16.6 mmol) was added to the solution of 6-(benzylthio)-8-bromo-[1,2,4]triazolo[4,3-a]pyridin-3-ethyl carboxylate (1.3 g, 3.3 mmol) in methanol (20 mL). The reaction mixture was stirred at 60°C for 30 minutes. After completion of the reaction, the reaction mixture was concentrated under reduced pressure to obtain the title compound (yellow solid, 1.5 g). LC/MS (ESI) (m/z): 378 $[M+H]^+$.

**Step 3: 6-(benzylthio)-8-bromo-N'-(2,2,2-trifluoroacetyl)-[1,2,4]triazolo[4,3-a]pyridine-3-carbohydrazide**

Under an atmosphere of nitrogen, trifluoroacetic anhydride (722 mg, 3.44mmol) was added to the solution of 6-(benzylthio)-8-bromo-[1,2,4]triazolo[4,3-a]pyridin-3-carbohydrazide (1 g, 2.64 mmol) in dichloromethane (10 mL). The reaction mixture was stirred at room temperature for 30 minutes. After completion of the reaction, water was added, and the reaction mixture was extracted with dichloromethane. The organic phase was washed with saturated brine, dried over anhydrous sodium sulfate, and concentrated under reduced pressure. The residue was purified and separated by column chromatography (eluent: petroleum ether, ethyl acetate, gradient: 0-60% ethyl acetate) to obtain the title compound (yellow solid, 750 mg, yield: 60%). LC/MS (ESI) (m/z): 474 $[M+H]^+$.

**Step 4: 6-(benzylthio)-8-bromo-N'-(2,2,2-trifluoroacetyl)-[1,2,4]triazolo[4,3-a]pyridine-3-carbohydrazide**

Under an atmosphere of nitrogen, the Lawesson's reagent (704 mg, 1.74 mmol) was added to the solution of 6-(benzylthio)-8-bromo-N'-(2,2,2-trifluoroacetyl)-[1,2,4]triazolo[4,3-a]pyridin-3-carbohydrazide (750 mg, 1.58 mmol) in anhydrous toluene (7.5 mL). The reaction mixture was stirred at 110°C for 4 hours. After completion of the reaction, saturated sodium bicarbonate was added, and the mixture was extracted with ethyl acetate. The organic phase was washed with saturated brine, dried over anhydrous sodium sulfate, and concentrated under reduced pressure. The residue was purified by column chromatography (eluent: petroleum ether, ethyl acetate, gradient: 0-25% ethyl acetate) to obtain the title compound (yellow solid, 700 mg, yield: 94%). LC/MS (ESI) (m/z): 472 $[M+H]^+$.

**Step 5: 8-bromo-3-(5-(trifluoromethyl)-1,3,4-thiadiazol-2-yl)-[1,2,4]triazolo[4,3-a]pyridine-6-sulfonyl chloride**

At 0°C,1N hydrochloric acid (2 mL), water (2 mL) and dichlorohydantoin (941 g, 4.77 mmol) were sequentially to the solution of 2-(6-(benzylthio)-8-bromo-[1,2,4]triazol[4,3-a]pyridin-3-yl)-5-(trifluoromethyl)-1,3,4-thiadiazole (750 mg, 1.59 mmol) in acetonitrile (8 mL). The reaction mixture was stirred in an ice bath for 10 minutes. After completion of the reaction, water was added, and the reaction mixture was extracted with dichloromethane. The organic phase was washed with saturated brine, dried over anhydrous sodium sulfate, and concentrated under reduced pressure to obtain the title compound (yellow solid, 1.05 g). It was directly used in the next step without purification.

**Step 6: 8-bromo-N-cyclopropyl-3-(5-(trifluoromethyl)-1,3,4-thiadiazol-2-yl)-[1,2,4]triazolo[4,3-a]pyridine-6-sulfonamide**

At 0°C, 8-bromo-3-(5-(trifluoromethyl)-1,3,4-thiadiazol-2-yl)-[1,2,4]triazolo[4,3-a]pyridin-6-sulfonyl chloride (400 mg, 0.89 mmol) was added to the solution of cyclopropylamine (255 mg, 4.46 mmol) in anhydrous dichloromethane (4 mL). The reaction mixture was stirred at room temperature for 30 minutes. After completion of the reaction, water was added, and the reaction mixture was extracted with ethyl acetate. The organic phase was washed with saturated brine, dried over anhydrous sodium sulfate, and concentrated under reduced pressure. The residue was purified by column chromatography (eluent: petroleum ether, ethyl acetate, gradient: 0-50% ethyl acetate) to obtain the title compound (yellow solid, 300 mg, yield: 72%). LC/MS (ESI) (m/z): 469 $[M+H]^+$.

**Step 7: 8-bromo-N-cyclopropyl-3-(5-(trifluoromethyl)-1,3,4-thiadiazol-2-yl)-N-((2-(trimethylmethylsilyl)ethoxy) methyl)-[1,2,4]triazol[4,3-a]pyridin-6-sulfonamide8-bromo-N-cyclopropyl-3-(5-(trifluoromethyl)-1,3,4-thiadiazol-2-yl)-N-((2-(trimethylsilyl)ethoxy)methyl)-[1,2,4]triazolo[4,3-a]pyridine-6-sulfonamide**

At 0°C, N,N-diisopropylethylamine (247 mg, 1.92 mmol) and 2-(trisilicyl)ethoxymethyl chloride (160 mg, 0.96 mmol) were sequentially added to the solution of 8-bromo-N-cyclopropyl-3-(5-(trifluoromethyl)-1,3,4-thiadiazol-2-yl)-[1,2,4]triazolo[4,3-a]pyridin-6-sulfonamide (300 mg, 0.64 mmol) in N,N-dimethylformamide (3 mL). The reaction mixture was stirred at room temperature for one hour. After completion of the reaction, water was added, and the reaction mixture was extracted with ethyl acetate. The organic phase was washed with saturated brine, dried over anhydrous sodium sulfate, and concentrated under reduced pressure. The residue was purified by column chromatography (eluent: petroleum ether, ethyl acetate, gradient: 0-15% ethyl acetate) to obtain the title compound (yellow solid, 300 mg, yield: 78%). LC/MS (ESI) (m/z): 599 $[M+H]^+$.

**Step 8: (R)-N-cyclopropyl-8-(3-methyl-4-(1-methylcyclopropane-1-carbonyl)piperazin-1-yl)-3-(5-(trifluoromethyl)-1,3,4-thiadiazol-2-yl)-N-((2-(trimethylsilyl)ethoxy)methyl)-[1,2,4]triazolo[4,3-a]pyridine-6-sulfonamide**

Under an atmosphere of nitrogen, cesium carbonate (490 mg, 1.51 mmol), Ruphos (23 mg, 0.05 mmol), Ruphos Pd G3 (42 mg, 0.05 mmol) were sequentially added to the solution of 8-bromo-N-cyclopropyl-3-(5-(trifluoromethyl)-1,3,4-thiadiazol-2-yl)-N-((2-(trimethylmethylsilyl)ethoxy)methyl)-[1,2,4]triazol[4,3-a]pyridin-6-sulfonamide (300 mg, 0.50 mmol) and (R) -(1-methylcyclopropyl)(2-methylpiperazin-1-yl)ketone (183 mg, 1.00 mmol) in 1,4-dioxane (6 mL). The flask was replaced with nitrogen gas three times and then the mixture was stirred at 80 °C for 3 hours under an atmosphere of nitrogen. After completion of the reaction, water was added, and the reaction mixture was extracted with ethyl acetate. The organic phase was washed with saturated brine, dried over anhydrous sodium sulfate, and concentrated under reduced pressure. The residue was purified by column chromatography (eluent: petroleum ether, ethyl acetate, gradient: 40-80% ethyl acetate) to obtain the title compound (yellow solid, 150 mg, yield: 43%). LC/MS (ESI) (m/z): 701 $[M+H]^+$.

**Step 9: (R)-N-cyclopropyl-8-(3-methyl-4-(1-methylcyclopropane-1-carbonyl)piperazin-1-yl)-3-(5-(trifluoromethyl)-1,3,4-thiadiazol-2-yl)-[1,2,4]triazolo[4,3-a]pyridine-6-sulfonamide**

At 0°C, trifluoroacetic acid (0.5 mL) was added to the solution of (R)-N-cyclopropyl-8-(3-methyl-4-(1-methylcyclopropan-1-carbonyl)piperazin-1-yl)-3-(5-(trifluoromethyl)-1,3,4-thiadiazol-2-yl)-N-((2-(trimethylsilicyl)ethoxy) methyl)-[1,2,4]triazol[4,3-a]pyridin-6-sulfonamide (150 mg, 0.21 mmol) in dichloromethane (1 mL). The reaction mixture was stirred at 0°C for one hour. After completion of the reaction, the reaction mixture was dripped to 5% aqueous sodium bicarbonate solution cooled in the ice bath, and extracted with dichloromethane. The organic phase was washed with saturated brine, dried over anhydrous sodium sulfate, and concentrated under reduced pressure. The residue was separated by pre-HPLC (C18, 10-50% acetonitrile in $H_2O$ and 0.1% FA) to obtain the title compound (yellow solid, 42 mg, yield: 34%). $^1H$ NMR (400 MHz, DMSO) $\delta$ 9.32 (s, 1H), 8.35 (s, 1H), 6.95 (s, 1H), 4.78 - 4.66 (m, 1H), 4.53 (d, *J* = 12.1 Hz, 1H), 4.37 - 4.24 (m, 2H), 3.47 (s, 1H), 3.29 - 3.24 (m, 1H), 3.15 - 3.02 (m, 1H), 2.32 - 2.24 (m, 1H), 1.33 (d, *J* = 5.7 Hz, 3H),

1.29 (s, 3H), 0.93 - 0.81 (m, 2H), 0.64 - 0.48 (m, 6H). LC/MS (ESI) (m/z): 571 [M+H]+.

**By referring to the preparation method in Example 386, the following examples were implemented by synthesizing a crude product according to a similar route by starting from appropriate starting materials, treating the crude product via reversed-phase HPLC, and then freeze-drying the crude product to obtain the title compound.**

| Example | Structure and name | Analysis data |
|---|---|---|
| **Example 387** | 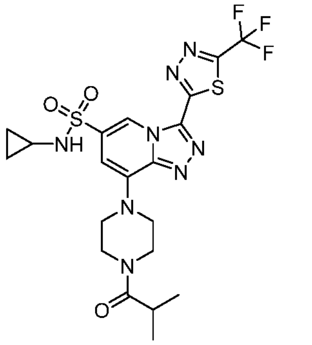 **N-cyclopropyl-8-(4-isobutyrylpiperazin-1-yl)-3-(5-(trifluoromethyl)-1,3,4-thiadiazol-2-yl)-[1,2,4]triazolo [4,3-a]pyridine-6-sulfonamide** | $^1H$ NMR (400 MHz, $CD_3OD$) δ 9.59 (d, *J* = 1.0 Hz, 1H), 7.02 (s, 1H), 3.92 - 3.85 (m, 6H), 3.73 - 3.69 (m, 2H), 3.09 - 2.99 (m, 1H), 2.34 - 2.29 (m, 1H), 1.15 (d, *J* = 6.7 Hz, 6H), 0.64 - 0.58 (m, 4H). LC/MS (ESI) (m/z): 545 [M+H]+. |
| **Example 388** | 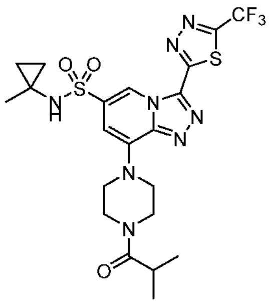 **8-(4-isobutyrylpiperazin-1-yl)-N-(1-methylcyclopropyl)-3-(5-(trifluoromethyl)-1,3,4-thiadiazol-2-yl)-[1,2,4]triazolo[4,3-a]pyridine-6-sulfonamide** | $^1H$ NMR (400 MHz, $CD_3OD$) δ 9.58 (s, 1H), 7.00 (s, 1H), 3.92 - 3.88 (m, 4H), 3.87 - 3.83 (m, 2H), 3.73 - 3.69 (m, 2H), 3.09 - 3.01 (m, 1H), 1.27 (s, 3H), 1.15 (d, *J* = 6.7 Hz, 6H), 0.84 - 0.81 (m, 2H), 0.54 - 0.50 (m, 2H). LC/MS (ESI) (m/z): 559 [M+H]+. |

**Example 389: N-(1-cyanocyclopropyl)-1-(2-isobutyryl-2-azaspiro[3.4]oct-5-en-6-yl)-3-(5-(trifluoromethyl)-1,3,4-thiadiazol-2-yl)imidazol[1,5-a]pyridine-6-sulfonamide**

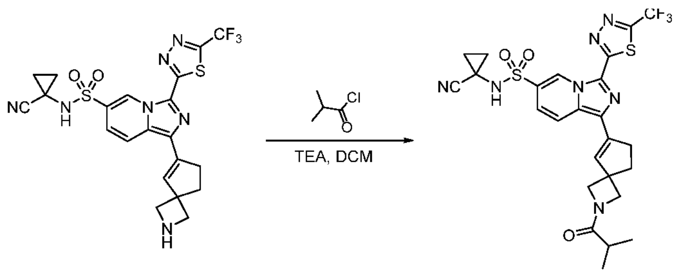


**Step 1: N-(1-cyanocyclopropyl)-1-(2-isobutyryl-2-azaspiro[3.4]oct-5-en-6-yl)-3-(5-(trifluoromethyl)-1,3,4-thiadiazol-2-yl)imidazo[1,5-a]pyridine-6-sulfonamide**

At 0°C, triethylamine (40 mg, 0.38 mmol) and isobutyryl chloride (25 mg, 0.24 mmol) were sequentially added to the solution of N-(1-cyanopropyl)-1-(2-azaspiro[3.4]oct-5-ene-6-yl)-3-(5-(trifluoromethyl)-1,3,4-thiadiazol-2-yl)imidazo [1,5-a]pyridin-6-sulfonamide (60 mg, 0.096 mmol) in dichloromethane (4 mL); and after addition, the reaction mixture was stirred at room temperature for one hour. After completion of the reaction, water was added to the reaction mixture,

which was then extracted with dichloromethane three times; and the organic phase was washed with saturated brine, dried over anhydrous sodium sulfate, filtered and concentrated under reduced pressure. The residue was dissolved in methanol (5 mL); lithium hydroxide (10 mg, 0.19mmol) was added at 0°C, and the reaction mixture was stirred at room temperature for 30 minutes; after completion of the reaction, the reaction mixture was added with water, and extracted with dichloromethane three times; and the organic phase was washed with saturated brine, dried over anhydrous sodium sulfate, filtered and concentrated under reduced pressure. The residue was separated by pre-HPLC to obtain the title compound (yellow solid, 8.1 mg, yield: 14.1%). $^1$H NMR (400 MHz, DMSO-$d_6$) δ 9.98 (s, 1H), 9.69 (s, 1H), 8.42 (d, *J* = 9.5 Hz, 1H), 7.45 (d, *J* = 9.7 Hz, 1H), 6.73 (s, 1H), 4.30 (d, *J* = 8.3 Hz, 1H), 4.20 (d, *J* = 8.3 Hz, 1H), 3.99 (d, *J* = 9.7 Hz, 1H), 3.88 (d, *J* = 9.4 Hz, 1H), 2.99 - 2.95 (m, 2H), 2.47 - 2.44 (m, 1H), 2.36 - 2.31 (m, 2H), 1.52 - 1.49 (m, 2H), 1.39 - 1.36 (m, 2H), 1.02 - 1.00 (m, 6H). LC/MS (ESI) (m/z): 592 [M+H]$^+$.

**Example 390: N-(1-cyanocyclopropyl)-7-fluoro-8-(4-isobutyrylpiperazin-1-yl)-3-(5-(trifluoromethyl)-1,3,4-thiadiazol-2-yl)imidazo[1,5-a]pyridine-6-sulfonamide**

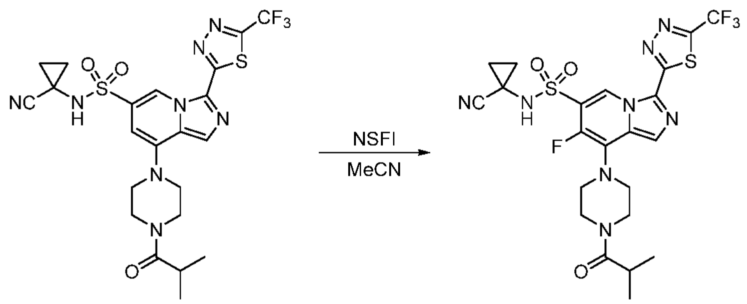


**Step 1: N-(1-cyanocyclopropyl)-7-fluoro-8-(4-isobutyrylpiperazin-1-yl)-3-(5-(trifluoromethyl)-1,3,4-thiadiazol-2-yl)imidazo[1,5-a]pyridine-6-sulfonamide**

- NFSI (30 mg, 0.14 mmol) was added to the solution of N-(1-cyanocyclopropyl)-8-(4-isobutyrylpiperazin-1-yl)-3-(5-(trifluoromethyl)-1,3,4-thiadiazol-2-yl)imidazo[1,5-a]pyridin-6-sulfonamide (80 mg, 0.14 mmol) in acetonitrile (2 ml). The reaction mixture was stirred at 25°C for one hour. After completion of the reaction, the reaction mixture was diluted with ethyl acetate, and extracted with ethyl acetate three times. The organic phase was dried over anhydrous sodium sulfate, filtered and concentrated under reduced pressure. The residue was purified by pre-HPLC to obtain the title compound (yellow solid, 20 mg, yield: 25%). $^1$H NMR (400 MHz, MeOD) δ 9.95 (d, *J* = 5.2 Hz, 1H), 7.93 (s, 1H), 3.85 - 3.80 (m, 4H), 3.50 - 3.44 (m, 4H), 3.05 - 3.00 (m, 1H), 1.59 - 1.55 (m, 2H), 1.49 - 1.45 (m, 2H), 1.15 (d, *J* = 6.7 Hz, 6H). LC/MS (ESI) (m/z): 586 [M+H]$^+$.

**Example 391: 1-chloro-3-(5-(difluoromethyl)-1,3,4-thiadiazol-2-yl)-8-(3-((methoxy-d3)methyl)piperazin-1-yl)-N-(3-methyloxetan-3-yl)imidazo[1,5-a]pyridine-6-sulfonamide**

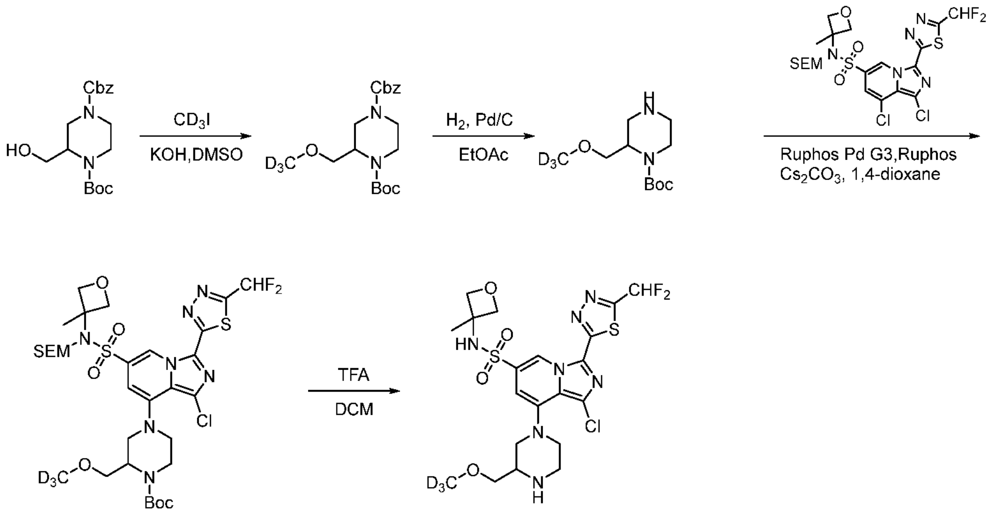


**Step 1: 4-benzyl 1-(tert-butyl) 2-((methoxy-d3)methyl)piperazine-1,4-dicarboxylate**

At 0°C, potassium hydroxide (159 mg, 2.84 mmol) and deuteroiodomethane (613 mg, 4.26 mmol) were sequentially added to the solution of 4-benzyl-1-(tert-butyl)2-(hydroxymethyl)piperazin-1,4-dicarboxylate (500 mg, 1.42 mmol) in DMSO (10 mL). The reaction mixture was stirred in an ice bath for 2 hours. After completion of the reaction, water was added, and the reaction mixture was extracted with dichloromethane. The organic phase was washed with saturated brine, dried over anhydrous sodium sulfate, and concentrated under reduced pressure. The residue was separated by column chromatography (eluent: petroleum ether/ethyl acetate, gradient: 0-30% ethyl acetate) to obtain the title compound (white solid, 320 mg, yield: 30%). LC/MS (ESI) (m/z): 368 $[M+H]^+$.

**Step 2: Tert-butyl 2-((methoxy-d3)methyl)piperazine-1-carboxylate**

Under an atmosphere of nitrogen, palladium on carbon (30 mg) with the mass fraction of 10% was added to the solution of 4-benzyl-1-(tert-butyl)-2-((methoxy-d3)methyl)piperazin-1,4-dicarboxylate (320 mg, 0.87 mmol) in ethyl acetate (20 mL); the flask was replaced with nitrogen gas three times and then the mixture was stirred for 2 hours under an atmosphere of hydrogen at 15psi. The reaction mixture was filtered, and the filtrate was concentrated under reduced pressure to obtain the title compound (colorless oil, 180 mg, yield: 88%). LC/MS (ESI) (m/z): 234 $[M+H]^+$.

**Step 3: Tert-butyl 4-(1-chloro-3-(5-(difluoromethyl)-1,3,4-thiadiazol-2-yl)-6-(N-(3-methyloxetan-3-yl)-N-((2-(trimethylsilyl)ethoxy)methyl)sulfamoyl)imidazo[1,5-a]pyridin-8-yl)-2-((methoxy-d3)methyl)piperazine-1-carboxylate**

Under an atmosphere of nitrogen, cesium carbonate (97 mg, 0.30 mmol), Ruphos (9 mg, 0.02 mmol) and Ruphos Pd $G_3$ (9 mg, 0.01mmol) were sequentially added to the solution of 1,8-dichloro-3-(5-(difluoromethyl)-1,3,4-thiadiazol-2-yl)-N-(3-methyloxacyclobutan-3-yl)-N-((2-(trimethylsilicyl)ethoxy)methyl)imidazo[1,5-a]pyridin-6-sulfonamide (60 mg, 0.10 mmol) and 2-((methoxy-d3)methyl)piperazin-1-tert-butyl carboxylate (30 mg, 0.13 mmol) in 1,4-dioxane (8 mL). The flask was replaced with nitrogen gas three times and then the mixture was stirred at 80°C for 2 hours under an atmosphere of nitrogen. After completion of the reaction, the reaction mixture was cooled to room temperature, diluted with ethyl acetate, washed with saturated brine, dried over anhydrous sodium sulfate, and concentrated under reduced pressure. The residue was purified by column chromatography (eluent: petroleum ether/ethyl acetate, gradient: 0-30% ethyl acetate) to obtain the title compound (yellow solid, 30 mg, yield: 37%). LC/MS (ESI) m/z: 797 $[M+H]^+$.

**Step 4: 1-chloro-3-(5-(difluoromethyl)-1,3,4-thiadiazol-2-yl)-8-(3-((methoxy-d3)methyl)piperazin-1-yl)-N-(3-methyloxetan-3-yl)imidazo [1,5-a] pyridine-6-sulfonamide**

At 0°C, trifluoroacetic acid (3 mL) was dropwise added slowly to the solution of 4-(1-chloro-3-(5-(difluoromethyl)-1,3,4-thiadiazol-2-yl)-6-(N-(3-methyloxacyclobutan-3-yl)-N-((2-(trimethylsilicyl)ethoxy)methyl)aminosulfonyl) imidazo[1,5-a]pyridin-8-yl)-2-((methoxy-$d_3$)methyl)piperazin-1-tert-butyl carboxylate (30 mg, 0.03mmol) in dichloromethane (8 mL). The mixture was stirred at room temperature for 30 minutes. The mixture was concentrated under reduced pressure; and the residue was purified by pre-HPLC to obtain the title compound (yellow solid, 4.8 mg, yield: 22%).

$^1H$ NMR (400 MHz, DMSO-$d_6$) δ 9.64 (d, $J$ = 1.1 Hz, 1H), 9.25 (s, 1H), 7.70 (t, $J$ = 53.1 Hz, 1H), 6.92 (s, 1H), 4.66 - 4.61 (m, 2H), 4.21 - 4.18 (m, 2H), 3.80 - 3.71 (m, 1H), 3.68 - 3.47 (m, 6H), 3.09 - 2.95 (m, 2H), 1.50 (s, 3H). LC/MS (ESI) (m/z): 567 $[M+H]^+$.

**Example 392: 3-(5-(difluoromethyl)-1,3,4-thiadiazol-2-yl)-8-(3-((methoxy-d3)methyl)piperazin-1-yl)-N-(3-methyloxetan-3-yl)imidazo [1,5-a] pyridine-6-sulfonamide**

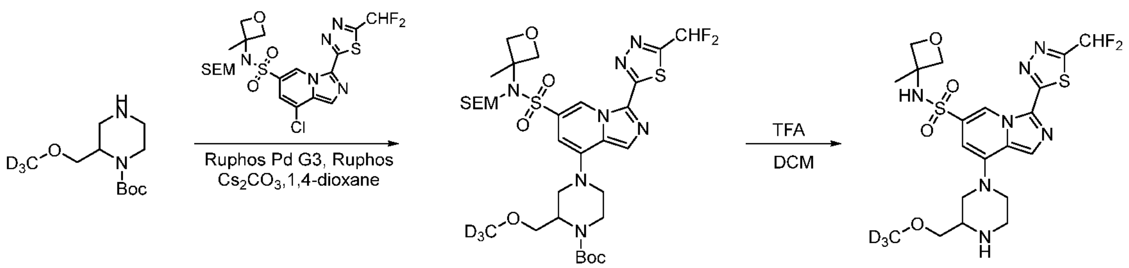


The target molecules of the title compound were obtained by referring to the preparation method in Steps 3-4 of **Example 391.** $^1$HNMR (400 MHz, DMSO-$d_6$) δ 9.63 (s, 1H), 9.22 (s, 1H), 8.87 (s, 1H), 8.14 (s, 1H), 7.69 (t, $J$ = 53.1 Hz, 1H), 6.80 (s, 1H), 4.65 - 4.61 (m, 2H), 4.21 - 4.17 (m, 2H), 3.80 - 3.46 (m, 7H), 3.21 - 3.01 (m, 2H), 1.50 (s, 3H). LC/MS (ESI) (m/z): 533 $[M+H]^+$.

**Example 393: 8-(2'-methyl-4'-oxohexahydro-4'H,8'H-spiro[cyclopropane-1,3'-pyrazino[1,2-a]pyrazin]-8'-yl)-N-(3-methyloxetan-3-yl)-3-(5-(trifluoromethyl)-1,3,4-thiadiazol-2-yl)imidazo[1,5-a]pyridine-6-sulfonamide**

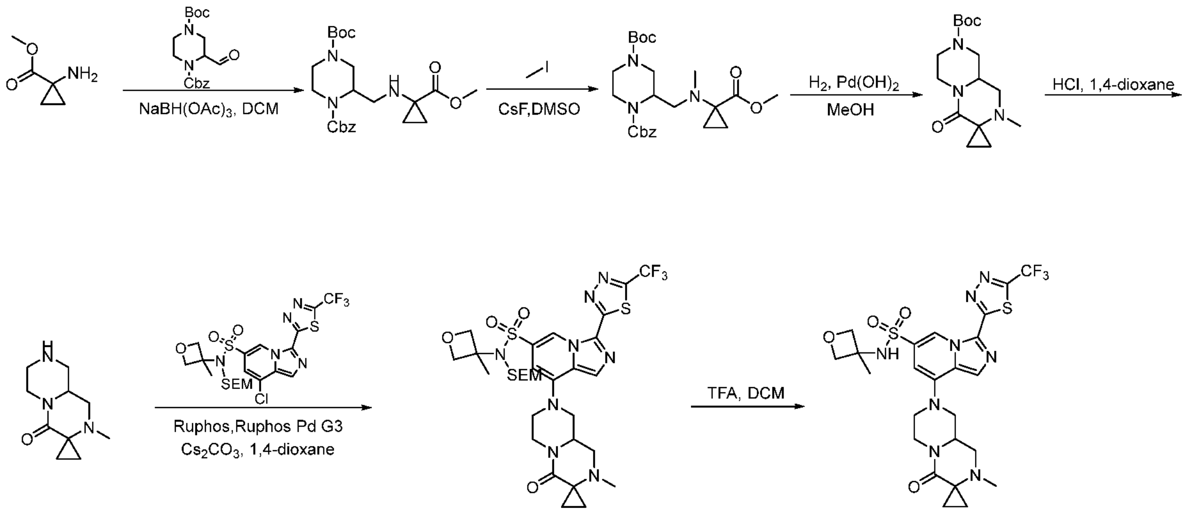


**Step 1: 1-benzyl 4-(tert-butyl) 2-(((1-(methoxycarbonyl)cyclopropyl)amino)methyl)piperazine-1,4-dicarboxylate**

Under an atmosphere of nitrogen, 1-aminocyclopropan-1-methyl formate (154 mg, 1.34 mmol) and sodium triacetoxyborohydride (731 mg, 3.45 mmol) were added to the solution of 4-(tert-butyl)2-formylpiperazin-1,4-1-benzyl dicarboxylate (400 mg, 0.89 mmol) in dichloromethane (6 mL). The reaction mixture was stirred at room temperature for 2 hours under an atmosphere of nitrogen. After completion of the reaction, saturated aqueous ammonium chloride solution was added to the reaction mixture, which was then extracted with ethyl acetate. The organic phase was washed with saturated brine, dried over anhydrous sodium sulfate, and concentrated under reduced pressure. The residue was purified by column chromatography (eluent: petroleum ether/ethyl acetate, gradient: 0-60% ethyl acetate) to obtain the title compound (yellow oil, 340 mg, yield: 66.0%). LC/MS (ESI) m/z: 448 $[M+H]^+$.

**Step 2: 1-benzyl 4-(tert-butyl) 2-(((1-(methoxycarbonyl)cyclopropyl)(methyl)amino)methyl)piperazine-1,4-dicarboxylate**

At 0°C, cesium fluoride (346 mg, 2.28 mmol) and iodomethane (162 mg, 1.14 mmol) were sequentially added to the solution of 4-(tert-butyl)-2-(((1-(methoxycarbonyl)cyclopropyl)amino)methyl)piperazin-1,4-1-benzyl dicarboxylate (340 mg, 0.76 mmol) in N,N-dimethylformamide (5 mL). The reaction mixture was stirred at 80°C for 16 hours. After completion of the reaction, water was added, and the reaction mixture was extracted with ethyl acetate. The organic phase was washed with saturated brine, dried over anhydrous sodium sulfate, and concentrated under reduced pressure. The residue was purified by column chromatography (eluent: petroleum ether/ethyl acetate, gradient: 0-30% ethyl acetate) to obtain the title compound (yellow oil, 140 mg, yield: 39.9%). LC/MS (ESI) (m/z): 462 $[M+H]^+$.

**Step 3: Tert-butyl 2'-methyl-4'-oxohexahydro-4'H,8'H-spiro[cyclopropane-1,3'-pyrazino[1,2-a]pyrazine]-8'-carboxylate**

At 0°C, palladium hydroxide on carbon (28 mg) with the mass fraction of 10% was added to the solution of 4-(tert-butyl)-2-(((1-(methoxycarbonyl)cyclopropyl)(methyl)amino)methyl)piperazin-1,4-1-benzyl dicarboxylate (140 mg, 0.30 mmol) in anhydrous methanol (5 mL). The reaction mixture was stirred at room temperature for 16 hours. After completion of the reaction, the reaction mixture was filtered; and the filtrate was concentrated under reduced pressure. The residue was purified by column chromatography (eluent: petroleum ether/dichloromethane, gradient: 0-35% dichloromethane) to obtain the title compound (yellow solid, 70 mg, yield: 78.2%). LC/MS (ESI) (m/z): 296 $[M+H]^+$.

**Step 4: 2'-methylhexahydro-2'H,4'H-spiro[cyclopropane-1,3'-pyrazino[1,2-a]pyrazin]-4'-one**

At room temperature, the solution of hydrochloric acid in 1,4-dioxane (1 mL) was added to the solution of 2'-methyl-4'-oxohexahydro-4'H,8'H-pyra[cyclopropan-1,3'-pyrazino[1,2-a]pyrazine]-8'-tert-butyl carboxylate (70 mg, 0.23 mmol) in methanol (2 mL). The reaction mixture was stirred at room temperature for 2 hours under an atmosphere of nitrogen. After completion of the reaction, the reaction mixture was concentrated under reduced pressure; and the residue was added to saturated aqueous sodium bicarbonate solution and extracted with ethyl acetate. The organic phase was washed with saturated brine, dried over anhydrous sodium sulfate, and concentrated under reduced pressure to obtain the title compound (yellow oil, 45 mg, yield: 97.2%). LC/MS (ESI) m/z: 196 $[M+H]^+$.

**Step 5-6: e8-(2'-methyl-4'-oxohexahydro-4'H,8'H-spiro[cyclopropane-1,3'-pyrazino[1,2-a]pyrazin]-8'-yl)-N-(3-methyloxetan-3-yl)-3-(5-(trifluoromethyl)-1,3,4-thiadiazol-2-yl)imidazo[1,5-a]pyridine-6-sulfonamide**

The title compound was obtained by referring to the preparation method in Steps 3-4 of **Example 391.** $^1H$ NMR (400 MHz, $CD_3OD$) δ 9.81 (s, 1H), 7.99 (s, 1H), 6.84 - 6.79 (m, 1H), 4.80 - 4.71 (m, 4H), 4.34 - 4.30 (m, 2H), 3.87 - 3.79 (m, 2H), 3.27 - 3.24 (m, 1H), 3.18 - 3.11 (m, 2H), 3.00 - 2.93 (m, 1H), 2.83 - 2.76 (m, 1H), 2.58 (s, 3H), 1.64 (s, 3H), 1.55 - 1.50 (m, 1H), 1.32 - 1.28 (m, 1H), 1.08 - 1.02 (m, 1H), 0.96 - 0.94 (m, 1H). LC/MS (ESI) (m/z): 613 $[M+H]^+$.

**Example 394: N-(3-methyloxetan-3-yl)-8-(3-oxohexahydroimidazo[1,5-a]pyrazin-7(1H)-yl)-3-(5-(trifluoromethyl)-1,3,4-thiadiazol-2-yl)imidazo[1,5-a]pyridine-6-sulfonamide**

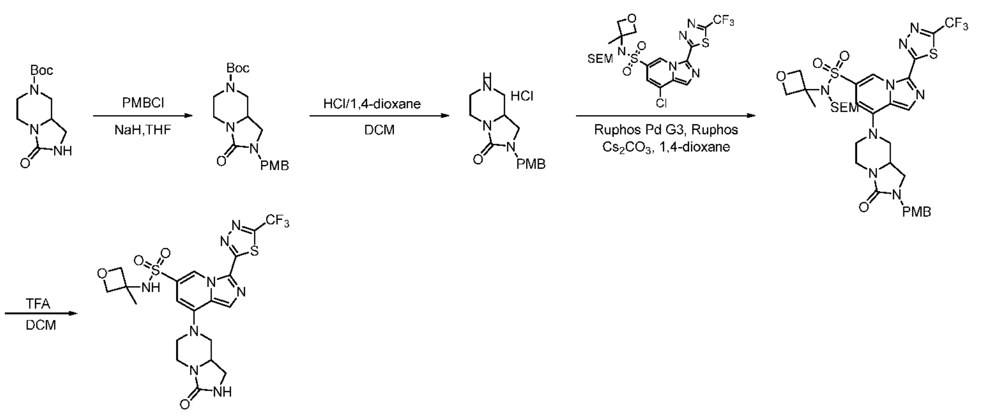

**Step 1: Tert-butyl 2-(4-methoxybenzyl)-3-oxohexahydroimidazo[1,5-a]pyrazine-7(1H)-carboxylate**

At 0°C, sodium hydrogen (10 mg, 0.25 mmol) with the mass fraction of 60% and 4-methoxybenzyl chloride (45 mg, 0.3 mmol) were added to the solution of 7(1H)-(tert-butoxycarbonyl)-3-oxohexahydroimidazo[1,5-a]pyrazine (30 mg, 0.12 mmol) in tetrahydrofuran (3 mL). The reaction mixture was stirred at 65°C for 2 hours. After completion of the reaction, the reaction mixture was quenched with saturated aqueous ammonium chloride solution cooled with ice water, and extracted with ethyl acetate three times. The organic phase was dried over anhydrous sodium sulfate, filtered and concentrated under reduced pressure. The residue was separated by column chromatography (eluent: petroleum ether/ethyl acetate, gradient: 0-50% ethyl acetate) to obtain the title compound (yellow solid, 35 mg, yield: 77.9%). LC/MS (ESI) (m/z): 362 $[M+H]^+$. $^1$H NMR (400 MHz, $CDCl_3$) δ 7.10 (d, *J* = 8.6 Hz, 2H), 6.79 (d, *J* = 8.6 Hz, 2H), 4.25 (s, 2H), 3.94 (s, 2H), 3.79 (d, *J* = 10.8 Hz, 1H), 3.73 (s, 3H), 3.46 (dd, *J* = 11.0, 8.1 Hz, 1H), 3.20 (t, *J* = 8.7 Hz, 1H), 2.82 (t, *J*= 11.2 Hz, 1H), 2.71 (dd, *J*= 9.0, 4.7 Hz, 2H), 2.50 (s, 1H), 1.39 (s, 9H).

**Step 2: 2-(4-methoxybenzyl)hexahydroimidazo[1,5-a]pyrazin-3(2H)-one hydrochloride**

At 0°C, trifluoroacetic acid (0.5 mL) was added to the solution of (2-(4-methoxybenzyl)-3-oxohexahydroimidazo [1,5-a]pyrazin-7(1H)-tert-butyl carboxylate(35 mg, 0.1 mmol) in dichloromethane (1 mL). The reaction mixture was stirred at 25°C for 1 hours. After completion of the reaction, the reaction mixture was concentrated under reduced pressure to obtain the title compound (yellow solid, 31 mg, yield: 99%). LC/MS (ESI) (m/z): 262 $[M+H]^+$.

**Step 3-4: N-(3-methyloxetan-3-yl)-8-(3-oxohexahydroimidazo[1,5-a]pyrazin-7(1H)-yl)-3-(5-(trifluoromethyl)-1,3,4-thiadiazol-2-yl)imidazo[1,5-a]pyridine-6-sulfonamide**

The title compound was obtained by referring to the preparation method in Steps 3-4 of **Example 391.** $^1$H NMR (400 MHz, $CD_3OD$) δ 9.81 (s, 1H), 7.96 (s, 1H), 6.82 (s, 1H), 4.79 (d, *J* = 6.1 Hz, 2H), 4.34 - 4.17 (m, 2H), 4.16 - 4.09 (m, 1H), 3.93 (d, *J* = 13.0 Hz, 1H), 3.83 - 3.77 (m, 1H), 3.71 (d, *J* = 8.2 Hz, 1H), 3.67 - 3.61 (m, 1H), 3.28 - 3.23 (m, 1H), 3.21 - 3.14 (m, 1H), 2.96 - 2.86 (m, 2H), 1.63 (s, 3H).

**Example 395: N-(3-methyloxetan-3-yl)-8-(6-oxooctahydro-2H-pyrazino[1,2-a]pyrazin-2-yl)-3-(5-(trifluoromethyl)-1,3,4-thiadiazol-2-yl)imidazo[1,5-a]pyridine-6-sulfonamide**

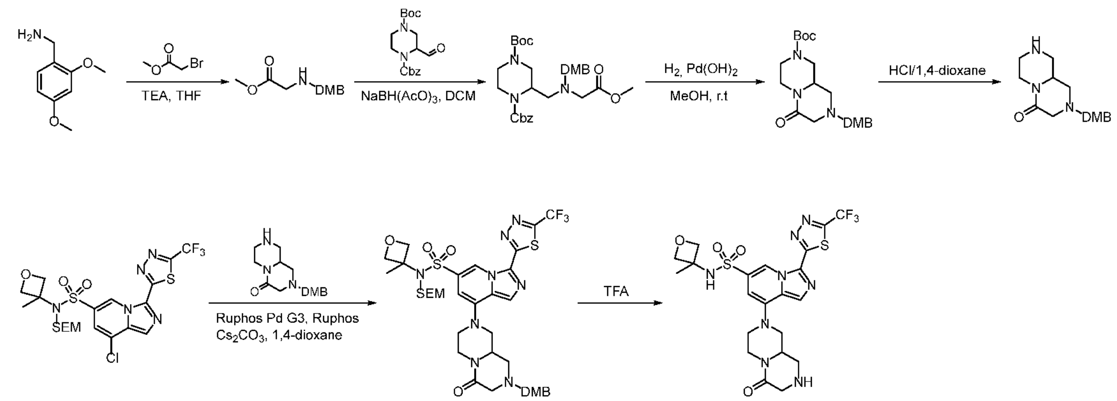


**Step 1: Methyl (3,4-dimethylbenzyl)glycinate**

Under an atmosphere of nitrogen, triethylamine (1.81 g, 18.0 mmol) and methyl bromoacetate (1.83 g, 12.0 mmol) were added to the solution of (2,4-dimethoxyphenyl)methylamine (2 g, 12.0 mmol) in tetrahydrofuran (6 mL). The reaction mixture was stirred overnight at room temperature under an atmosphere of nitrogen. After completion of the reaction, ethyl acetate was added; the mixture was filtered; and the filtrate was washed with saturated brine, dried over anhydrous sodium sulfate, and concentrated under reduced pressure. The residue was purified by column chromatography (eluent: petroleum ether, ethyl acetate, gradient: 0-100% ethyl acetate) to obtain the title compound (yellow oil, 2.3 g, yield: 80.4%). LC/MS (ESI) m/z: 240 $[M+H]^+$.

**Step 2: 1-benzyl 4-(tert-butyl) 2-(((3,4-dimethylbenzyl)(2-methoxy-2-oxoethyl)amino)methyl)piperazine-1,4-dicarboxylate**

Under an atmosphere of nitrogen, (2,4-dimethoxybenzyl)glycine methyl ester (206 mg, 0.86 mmol) and sodium triacetoxyborohydride (366 mg, 1.72 mmol) were added to the solution of 4-(tert-butyl)2-formylpiperazin-1,4-1-benzyl dicarboxylate (200 mg, 0.57 mmol) in dichloromethane (6 mL). The reaction mixture was stirred at room temperature for 2 hours under an atmosphere of nitrogen. After completion of the reaction, saturated aqueous ammonium chloride solution was added; and the resulting mixture was extracted with dichloromethane, washed with saturated brine, dried over anhydrous sodium sulfate, and concentrated under reduced pressure. The residue was purified by column chromatography (eluent: petroleum ether, ethyl acetate, gradient: 0-30% ethyl acetate) to obtain the title compound (yellow oil, 230 mg, yield: 70.1%). LC/MS (ESI) m/z: 572 $[M+H]^+$.

**Step 3: Tert-butyl 8-(3,4-dimethylbenzyl)-6-oxooctahydro-2H-pyrazino[1,2-a]pyrazine-2-carboxylate**

At room temperature, palladium hydroxide on carbon (40 mg) with the mass fraction of 10% was added to the solution of 4-((tert-butyl)-2-(((2,4-dimethoxybenzyl)(2-methoxy-2-oxyethyl)amino)methyl)piperazin-1,4-1-benzyl dicarboxylate (230 mg, 0.40 mmol) in methanol (6 mL). The reaction mixture was stirred at room temperature overnight. After completion of the reaction, the reaction mixture was filtered through diatomite; and the filtrate was concentrated under reduced pressure. The residue was purified by column chromatography eluent: petroleum ether/dichloromethane, gradient: 0-35% dichloromethane to obtain the title compound (yellow solid, 100 mg, yield: 61.3%). LC/MS (ESI) (m/z): 406 $[M+H]^+$.

**Step 4: 2-(3,4-dimethylbenzyl)octahydro-4H-pyrazino[1,2-a]pyrazin-4-one**

The solution of hydrochloric acid and 1, 4-dioxane (1 mL, 4 M) was added to the solution of 8-(2,4-dimethoxybenzyl)-6-oxooctahydro-2H-pyrazino[1,2-a]pyrazin-2-tert-butyl carboxylate (100 mg, 0.23 mmol) in methanol (2 mL). The reaction mixture was stirred at room temperature for 2 hours under an atmosphere of nitrogen. After completion of the reaction, the reaction mixture was concentrated under reduced pressure, adjusted to pH 8 by adding saturated aqueous sodium bicarbonate solution, and extracted with ethyl acetate. The organic phase was washed with saturated brine, dried over anhydrous sodium sulfate, and concentrated under reduced pressure to obtain the title compound (yellow oil, 65 mg, yield: 86.3%). LC/MS (ESI) m/z: 306 $[M+H]^+$.

**Step 5-6: N-(3-methyloxetan-3-yl)-8-(6-oxooctahydro-2H-pyrazino[1,2-a]pyrazin-2-yl)-3-(5-(trifluoromethyl)-1,3,4-thiadiazol-2-yl)imidazo[1,5-a]pyridine-6-sulfonamide**

The title compound was obtained by referring to the preparation method in Steps 3-4 of **Example 391.** $^1$H NMR (400 MHz, $CD_3OD$) δ 9.82 (s, 1H), 8.32 (s, 1H), 7.99 (s, 1H), 6.81 (s, 1H), 5.37 - 5.33 (m, 2H), 5.21 - 5.14 (m, 2H), 4.32 (d, *J* = 6.6 Hz, 2H), 3.64 - 3.55 (m, 3H), 2.21 - 2.17 (m, 4H), 2.04 - 2.02 (m, 2H), 1.64 (s, 3H). LC/MS (ESI) (m/z): 573 $[M+H]^+$.

**Example 396: 3-(5-(difluoromethyl)-1,3,4-thiadiazol-2-yl)-8-((2R,6S)-2-(hydroxymethyl)-6-methylmorpholino)-N-(3-methyloxetan-3-yl)imidazo[1,5-a]pyridine-6-sulfonamide**

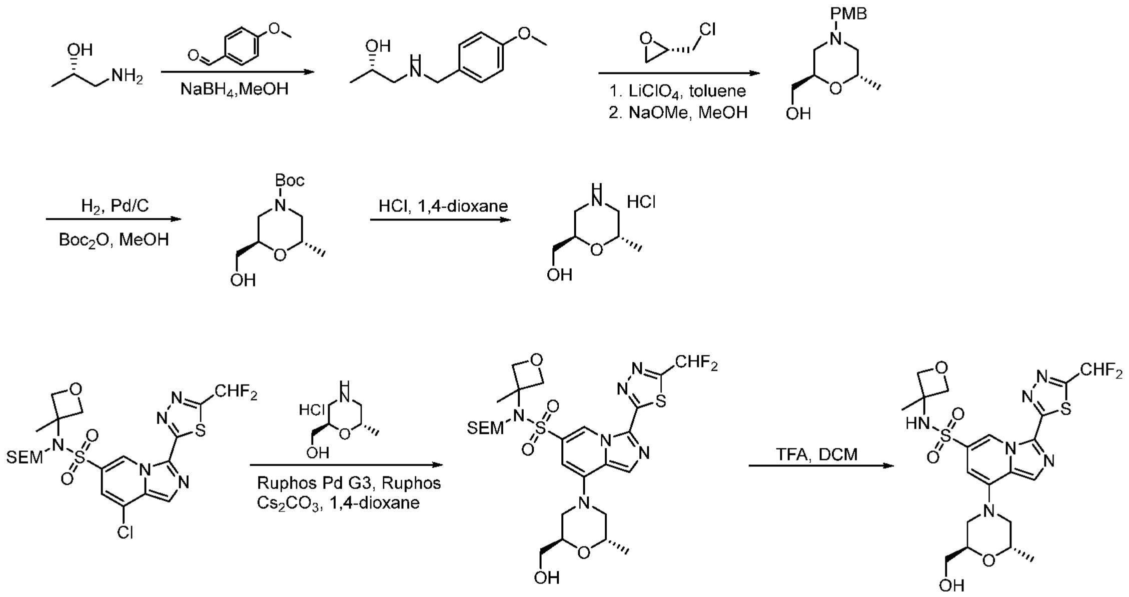


**Step 1: (S)-1-((4-methoxybenzyl)amino)propan-2-ol**

Under an atmosphere of nitrogen, p-methoxybenzaldehyde (10.3 mg, 76 mmol) was added to the solution of (S)-1-aminopropan-2-ol (6 g, 80 mmol) in methanol (60 mL); the reaction mixture was stirred at room temperature for one hour under an atmosphere of nitrogen; and then, 0°C, sodium borohydride (3.0 g, 80 mmol) was added portionwise. The reaction mixture was stirred at room temperature for 2 hours under an atmosphere of nitrogen. After completion of the reaction, saturated aqueous ammonium chloride solution was added to the reaction mixture, which was then extracted with ethyl acetate. The organic phase was washed with saturated brine, dried over anhydrous sodium sulfate, and concentrated under reduced pressure to obtain the title compound (white solid, 10 g, yield: 64.1%). LC/MS (ESI) m/z: 196 $[M+H]^+$.

**Step 2: ((2R,6S)-4-(4-methoxybenzyl)-6-methylmorpholin-2-yl)methanol**

Under an atmosphere of nitrogen, (S)-2-(chloromethyl)ethylene oxide (3 g, 32.61 mmol) and and lithium perchlorate (3.5 g, 32.6 mmol) were added to the solution of (S)-1-((4-methoxybenzyl)amino)propan-2-ol (8 g, 41.03 mmol) in toluene (60 mL). The reaction mixture was stirred at room temperature for 18 hours under an atmosphere of nitrogen. The solution of 30% sodium methoxide in methanol (25 mL) was added; after completion of the reaction, saturated aqueous ammonium chloride solution was added; and the resulting mixture was extracted with ethyl acetate, washed with saturated brine, dried over anhydrous sodium sulfate, and concentrated under reduced pressure. The residue was purified by column chromatography (eluent: petroleum ether/ethyl acetate, gradient: 0-60% ethyl acetate) to obtain the title compound (yellow oil, 1.8 g, yield: 22.0%). LC/MS (ESI) m/z: 252 $[M+H]^+$.

**Step 3: Tert-butyl (2R,6S)-2-(hydroxymethyl)-6-methylmorpholine-4-carboxylate**

At 0°C, palladium on carbon (90 mg) with the mass fraction of 10%, palladium hydroxide on carbon (90 mg) with the mass fraction of 10% and di-tert-butyl dicarbonate (1.17 g, 5.38 mmol) were added to the solution of ((2R,6S)-4-(4-methoxybenzyl)-6-methylmorpholin-2-yl) methanol (900 mg, 3.59 mmol) in anhydrous methanol (10 mL). The flask was replaced with nitrogen gas three times and then the mixture was stirred at 60°C for 16 hours under an atmosphere of hydrogen at15 psi. After completion of the reaction, the reaction mixture was filtered, and concentrated under reduced pressure. The residue was purified by column chromatography (eluent: petroleum ether/dichloromethane, gradient: 0-35% dichloromethane) to obtain the title compound (yellow oil, 690 mg, yield: 83.3%). LC/MS (ESI) (m/z): 232 $[M+H]^+$.

**Step 4: ((2R,6S)-6-methylmorpholin-2-yl)methanol hydrochloride**

The solution of hydrogen chloride in 1,4-dioxane (3 mL) was added to the solution of (2R,6S)-2-(hydroxymethyl)-6-methylmorpholin-4-tert-butyl carboxylate (400 mg, 0.23 mmol) in 1,4-dioxane (5 mL). The reaction mixture was stirred at room temperature for 2 hours under an atmosphere of nitrogen. After completion of the reaction, the reaction mixture was concentrated under reduced pressure to obtain the title compound (yellow oil, 350 mg, yield: 91.3%). LC/MS

(ESI) m/z: 132 [M+H]+.

**Step 5-6: 3-(5-(difluoromethyl)-1,3,4-thiadiazol-2-yl)-8-((2R,6S)-2-(hydroxymethyl)-6-methylmorpholino)-N-(3-methyloxetan-3-yl)imidazo [1,5-a] pyridine-6-sulfonamide**

The title compound was obtained by referring to the preparation method in Steps 3-4 of **Example 391.** $^1$H NMR (400 MHz, DMSO) δ 9.57 (s, 1H), 8.73 (s, 1H), 8.05 (s, 1H), 7.84 - 7.52 (m, 1H), 6.70 (d, *J* = 0.9 Hz, 1H), 5.01 - 4.96 (m, 1H), 4.66 - 4.61 (m, 2H), 4.20 - 4.09 (m, 3H), 3.96 - 3.83 (m, 2H), 3.67 - 3.55 (m, 2H), 3.17 - 3.13 (m, 1H), 3.01 - 2.95 (m, 1H), 1.50 (s, 3H), 1.26 (d, *J* = 6.2 Hz, 3H). LC/MS (ESI) (m/z): 531 [M+H]+.

**Example 397: 1-chloro-3-(5-(difluoromethyl)-1,3,4-thiadiazol-2-yl)-8-((2R,6S)-2-(hydroxymethyl)-6-methylmorpholino)-N-(3-methyloxetan-3-yl)imidazo[1,5-a]pyridine-6-sulfonamide**

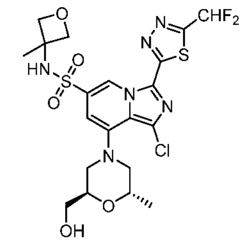

This example was implemented by referring to the synthesis method in **Example 396.** $^1$H NMR (400 MHz, DMSO-$d_6$) δ 9.61 (s, 1H), 8.81 (s, 1H), 7.69 (t, *J* = 53.1 Hz, 1H), 6.86 (s, 1H), 4.76 - 4.71 (m, 1H), 4.65 - 4.61 (m, 2H), 4.27 - 4.22 (m, 1H), 4.19 (d, *J* = 6.3 Hz, 2H), 4.05 - 4.00 (m, 1H), 3.67 - 3.61 (m, 2H), 3.30 - 3.26 (m, 2H), 2.93 - 2.85 (m, 1H), 2.66 - 2.60 (m, 1H), 1.50 (s, 3H), 1.23 (d, *J* = 6.3 Hz, 3H). LC/MS (ESI) (m/z): 565 [M+H]+.

**Example 398: 3-(5-(difluoromethyl)-1,3,4-thiadiazol-2-yl)-8-((2S,6S)-2-((dimethylamino)methyl)-6-methylmorpholino)-N-(3-methyloxetan-3-yl)imidazo[1,5-a]pyridine-6-sulfonamide**

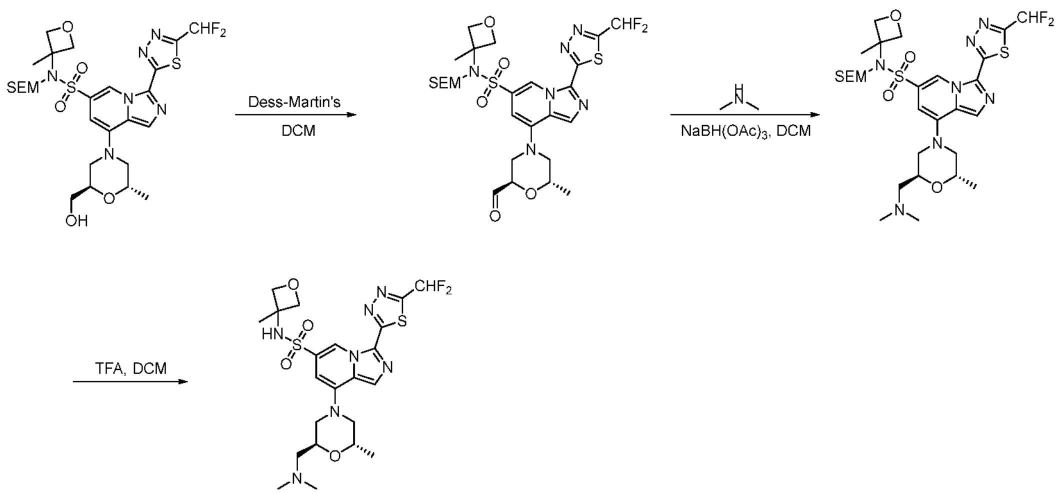


**Step 1: 3-(5-(difluoromethyl)-1,3,4-thiadiazol-2-yl)-8-((2R,6S)-2-formyl-6-methylmorpholino)-N-(3-methyloxetan-3-yl)-N-((2-(trimethylsilyl)ethoxy)methyl)imidazo[1,5-a]pyridine-6-sulfonamide**

Under an atmosphere of nitrogen, Dess-Martin periodinane (26 mg, 0.06 mmol) was added to the solution of 3-(5-(difluoromethyl)-1,3,4-thiadiazol-2-yl)-8-((2R, 6S)-2-(hydroxymethyl)-6-methylmorpholinyl)-N-(3-methyloxacyclobutan-3-yl)-N-[(2-(trimethylsilicyl)ethoxy)methyl)imidazo[1,5-a]pyridin-6-sulfonamide (20 mg, 0.03 mmol) in dichloromethane (2 mL). The reaction mixture was stirred at room temperature for 1 hours under an atmosphere of nitrogen.

After completion of the reaction, the reaction mixture was diluted with dichloromethane, sequentially washed with saturated aqueous sodium sulfite solution and saturated sodium bicarbonate, dried over anhydrous sodium sulfate, and concentrated under reduced pressure to obtain the title compound (yellow oil, 19 mg, yield: 95.3%). LC/MS (ESI) m/z: 659 $[M+H]^+$.

**Step 2: 3-(5-(difluoromethyl)-1,3,4-thiadiazol-2-yl)-8-((2S,6S)-2-((dimethylamino)methyl)-6-methylmorpholino)-N-(3-methyloxetan-3-yl)-N-((2-(trimethylsilyl)ethoxy)methyl)imidazo[1,5-a]pyridine-6-sulfonamide**

At room temperature, the solution of dimethylamine in tetrahydrofuran (1 mL, 2 mol/L) was added to the solution of 3-(5-(difluoromethyl)-1,3,4-thiadiazol-2-yl)-8-((2R,6S)-2-formyl-6-methylmorpholinyl)-N-(3-methyloxacyclobutan-3-yl)-N((2-(trimethylsilicyl)ethoxy)methyl)imidazo[1,5-a]pyridin-6-sulfonamide (19 mg, 0.03 mmol) in dichloromethane (1 mL). The reaction mixture was stirred at room temperature for 30 minutes under an atmosphere of nitrogen; then, sodium triacetoxyborohydride (18 mg, 0.09 mmol) was added; and the reaction mixture was continued to be stirred at room temperature for 30 minutes under an atmosphere of nitrogen. After completion of the reaction, water was added, and the reaction mixture was extracted with dichloromethane. The organic phase was dried over anhydrous sodium sulfate, filtered and concentrated under reduced pressure to obtain the title compound (yellow oil, 19 mg, yield: 95.8%). LC/MS (ESI) (m/z): 688 $[M+H]^+$.

**Step 3: 3-(5-(difluoromethyl)-1,3,4-thiadiazol-2-yl)-8-((2S,6S)-2-((dimethylamino)methyl)-6-methylmorpholino)-N-(3-methyloxetan-3-yl)imidazo[1,5-a]pyridine-6-sulfonamide**

At 0°C, trifluoroacetic acid (1 mL) was added to the solution of 3-(5-(difluoromethyl)-1,3,4-thiadiazol-2-yl)-8-((2S,6S)-2-((dimethylamino)methyl)-6-methylmorpholinyl)-N-(3-methyloxacyclobutan-3-yl)-N(((2-(trimethylsilicyl) ethoxy)methyl)imidazo[1,5-a]pyridin-6-sulfonamide (19 mg, 003 mmol) in dichloromethane (1 mL). The reaction mixture was stirred at 25°C for 1 hours. After completion of the reaction, the reaction mixture was concentrated under reduced pressure. The residue was purified by pre-HPLC to obtain the title compound (yellow solid, 5.0 mg, yield: 31.3%). $^1H$ NMR (400 MHz, $CD_3OD$) δ 9.86 (s, 1H), 7.86 (s, 1H), 7.34 (s, 1H), 6.82 - 6.77 (m, 1H), 4.81 - 4.78 (m, 2H), 4.59 - 4.54 (m, 1H), 4.40 - 4.36 (m, 1H), 4.33 - 4.31 (m, 2H), 3.85 - 3.79 (m, 1H), 3.50 - 3.43 (m, 2H), 3.37 - 3.34 (m, 1H), 3.22 - 3.15 (m, 2H), 3.00 (d, *J* = 14.3 Hz, 6H), 1.65 (s, 3H), 1.48 (d, *J* = 6.4 Hz, 3H). LC/MS (ESI) (m/z): 558 $[M+H]^+$.

**Example 399: 3-(5-(difluoromethyl)-1,3,4-thiadiazol-2-yl)-8-((3S,5S)-4-(1-methoxycyclopropane-1-carbonyl)-3,5-dimethylpiperazin-1-yl)-N-(3-methyloxetan-3-yl)imidazo[1,5-a]pyridine-6-sulfonamide**

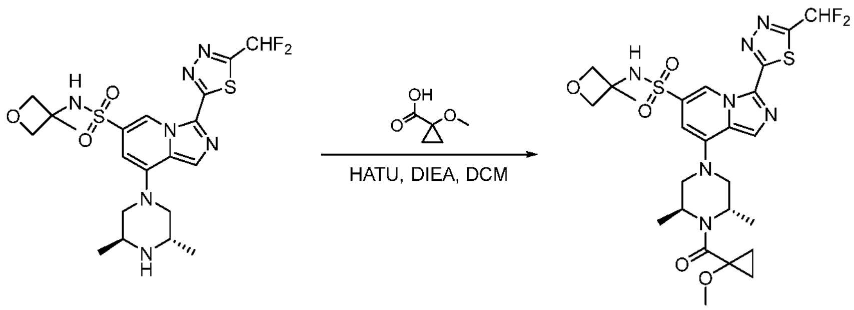


**Step 1: 3-(5-(difluoromethyl)-1,3,4-thiadiazol-2-yl)-8-((3S,5S)-4-(1-methoxycyclopropane-1-carbonyl)-3,5-dimethylpiperazin-1-yl)-N-(3-methyloxetan-3-yl)imidazo[1,5-a]pyridine-6-sulfonamide**

At 0°C, N,N-diisopropylethylamine (50 mg, 0.39 mmol), 1-methoxycyclopropanecarboxylic acid (19 mg, 0.17 mmol) and HATU (74 mg, 0.19 mmol) were sequentially added to the solution of 3-(5-(difluoromethyl)-1,3,4-thiadiazol-2-yl)-8-((3S, 5S)-3,5-dimethylpiperazin-1-yl)-N-(3-methyloxacyclobutan-3-yl)imidazo[1,5-a]pyridin-6-sulfonamide (70 mg, 0.13 mmol) in dichloromethane (2 mL); and after adding, the reaction mixture was stirred at 50°C for one hour. After completion of the reaction, water was added to the reaction mixture, which was then extracted with dichloromethane three times; and the organic phase was washed with saturated brine, dried over anhydrous sodium sulfate, filtered and concentrated under reduced pressure. The residue was separated by column chromatography (eluent: petroleum ether/ethyl acetate, gradient: 0-70% ethyl acetate), and then separated by pre-HPLC to obtain the title compound (yellow solid, 9.6 mg, yield: 12%). $^1H$ NMR (400 MHz, DMSO-$d_6$) δ 9.48 (s, 1H), 8.65 (s, 1H), 8.14 (s, 1H), 7.68 (t, *J* = 53.2 Hz, 1H),

6.44 (s, 1H), 4.67 - 4.64 (m, 2H), 4.20 - 4.17 (m, 2H), 4.17 - 4.11 (m, 2H), 3.69 - 3.59 (m, 2H), 3.42 - 3.38 (m, 1H), 3.32 - 3.30 (m, 1H), 3.28 (s, 3H), 1.52 (s, 3H), 1.37 - 1.27 (m, 6H), 1.08 - 1.04 (m, 2H), 1.03 - 0.97 (m, 1H), 0.94 - 0.87 (m, 1H). LC/MS (ESI) (m/z): 612 [M+H]$^+$.

**Example 400: 3-(5-(difluoromethyl)-1,3,4-thiadiazol-2-yl)-N-(3-methyloxetan-3-yl)-8-((3S,5S)-3,4,5-trimethylpiperazin-1-yl)imidazo[1,5-a]pyridine-6-sulfonamide**

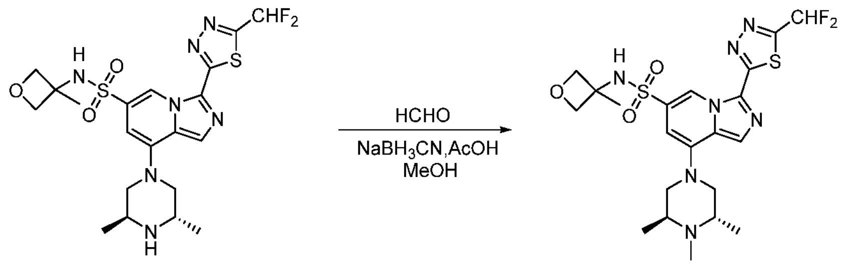


**Step 1: 3-(5-(difluoromethyl)-1,3,4-thiadiazol-2-yl)-N-(3-methyloxetan-3-yl)-8-((3S,5S)-3,4,5-trimethylpiperazin-1-yl)imidazo[1,5-a]pyridine-6-sulfonamide**

At 0°C, formaldehyde (8 mg, 0.26 mmol), acetic acid (10 mg, 0.17 mmol) and cyano sodium borohydride (24 mg, 0.39 mmol) were sequentially added to the solution of 3-(5-(difluoromethyl)-1,3,4-thiadiazol-2-yl)-8-((3S,5S)-3,5-dimethylpiperazin-1-yl)-N-(3-methyloxacyclobutan-3-yl)imidazo[1,5-a]pyridin-6-sulfonamide (70 mg, 0.13 mmol) in methanol (2 mL). After adding, the reaction mixture was stirred at 0°C for 20 minutes. After completion of the reaction, water was added to the reaction mixture, which was then extracted with dichloromethane three times. The organic phase was washed with saturated brine, dried over anhydrous sodium sulfate, filtered and concentrated under reduced pressure. The residue was purified by column chromatography (eluent: petroleum ether/ethyl acetate, gradient: 50-80% ethyl acetate) and then purified by pre-HPLC to further obtain the title compound (yellow solid, 17 mg, yield: 24%). $^1$H NMR (400 MHz, DMSO-$d_6$) δ 9.64 (d, $J$ = 2.5 Hz, 1H), 8.77 (d, $J$ = 7.6 Hz, 1H), 8.03 (d, $J$ = 3.1 Hz, 1H), 7.82 (t, $J$ = 53.6 Hz, 1H), 6.79 (s, 1H), 4.70 - 4.61 (m, 2H), 4.25 - 4.16 (m, 2H), 3.99 - 3.92 (m, 1H), 3.86 - 3.70 (m, 4H), 3.10 - 3.02 (m, 1H), 2.89 (s, 3H), 1.55 - 1.47 (m, 6H), 1.41 (s, 3H). LC/MS (ESI) (m/z): 528 [M+H]$^+$

**Example 401: 8-((3S,5S)-4-acetyl-3,5-dimethylpiperazin-1-yl)-3-(5-(difluoromethyl)-1,3,4-thiadiazol-2-yl)-N-(3-methyloxetan-3-yl)imidazo [1,5-a] pyridine-6-sulfonamide**

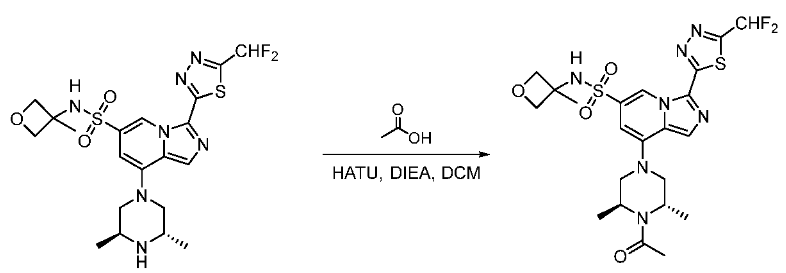


**Step 1: 8-((3S,5S)-4-acetyl-3,5-dimethylpiperazin-1-yl)-3-(5-(difluoromethyl)-1,3,4-thiadiazol-2-yl)-N-(3-methyloxetan-3-yl)imidazo [1,5-a] pyridine-6-sulfonamide**

At 0°C, N,N-diisopropylethylamine (50 mg, 0.39 mmol), acetic acid (10 mg, 0.17 mmol) and HATU (74 mg, 0.19 mmol) were sequentially added to the solution of 3-(5-(difluoromethyl)-1,3,4-thiadiazol-2-yl)-8-((3S,5S)-3,5-dimethylpiperazin-1-yl)-N-(3-methyloxacyclobutan-3-yl)imidazo[1,5-a]pyridin-6-sulfonamide (70 mg, 0.13 mmol) in dichloromethane (2 mL). After the addition, the reaction mixture was stirred at 50°C for one hour. After completion of the reaction, water was added to the reaction mixture, which was then extracted with dichloromethane three times. The organic phase was washed with saturated brine, dried over anhydrous sodium sulfate, filtered and concentrated under reduced pressure.

The residue was separated by pre-HPLC to obtain the title compound (yellow solid, 14 mg, yield: 19%). $^1$H NMR (400 MHz, DMSO-$d_6$) δ 9.47 (s, 1H), 8.68 (s, 1H), 8.12 (s, 1H), 7.68 (t, $J$ = 53.2 Hz, 1H), 6.38 (s, 1H), 4.68 - 4.62 (m, 2H), 4.50 - 4.41 (m, 1H), 4.34 - 4.26 (m, 1H), 4.20 - 4.12 (m, 4H), 3.69 - 3.59 (m, 2H), 2.09 (s, 3H), 1.52 (s, 3H), 1.38 - 1.31 (m, 3H), 1.23 - 1.16 (m, 3H). LC/MS (ESI) (m/z): 556 [M+H]$^+$.

**Example 402: 1-chloro-3-(5-(difluoromethyl)-1,3,4-thiadiazol-2-yl)-8-((3R,5S)-3-(hydroxymethyl)-5-methylpiperazin-1-yl)-N-(3-methyloxetan-3-yl)imidazo[1,5-a]pyridine-6-sulfonamide**

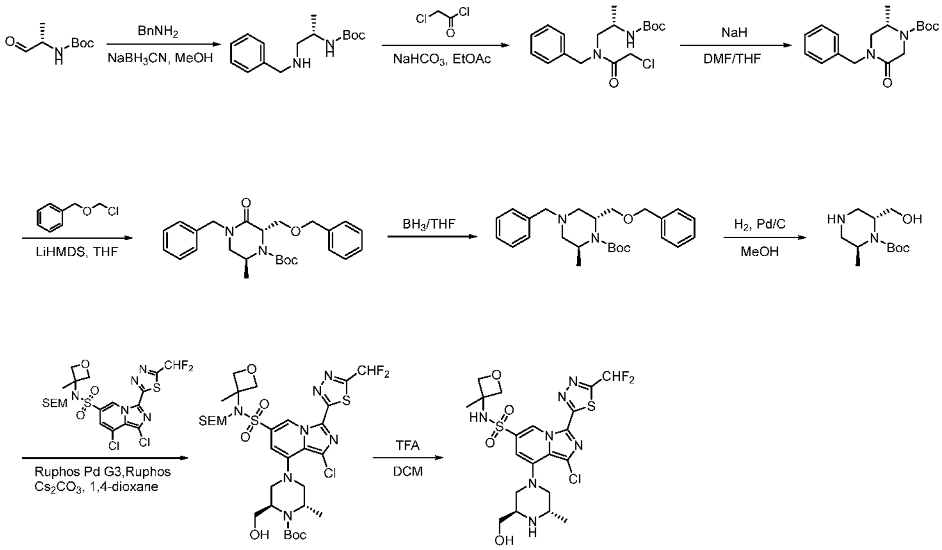


**Step 1: Tert-butyl (S)-(1-(N-benzyl-2-chloroacetamido)propan-2-yl)carbamate**

(S)-(1-oxoprop-2-yl)aminotert-butyl formate (5 g, 28.9 mmol) and benzylamine (3.7 g, 34.7 mmol) was dissolved in methanol (50 mL). In an ice bath, cyano sodium borohydride (5.5 g, 86.7 mmol) and acetic acid (0.327 g, 2.89mmol) were sequentially added to the reaction mixture; and the mixture was stirred at room temperature for 16 hours. After completion of the reaction, in an ice bath for cooling, saturated ammonium chloride was added for quenching, and the mixture was extracted with ethyl acetate, washed with saturated brine, dried over anhydrous sodium sulfate, and concentrated under reduced pressure. The residue was purified by column chromatography (eluent: petroleum ether, ethyl acetate, gradient: 10-30% ethyl acetate) to obtain the title compound (colorless oil, 5.5 g, yield: 72.4%). LC/MS (ESI) m/z: 265 [M+H]$^+$.

**Step 2: Tert-butyl (S)-4-benzyl-2-methyl-5-oxopiperazine-1-carboxylate**

(S)-(1-(benzylamino)propan-2-yl)aminotert-butyl formate (5.5 g, 20.8 mmol) was mixed with saturated aqueous sodium bicarbonate solution (50 mL) and ethyl acetate (50 mL). In an ice water bath, 2-chloroacetyl chloride (4.7 g, 41.6 mmol) was added to the mixture, and stirred for 2 hours. The organic layer was separated; the aqueous phase was extracted with ethyl acetate; and the organic phase was combined, washed with saturated brine, dried over anhydrous sodium sulfate, and concentrated under reduced pressure. The residue was purified by column chromatography (eluent: petroleum ether, ethyl acetate, gradient: 10-30% ethyl acetate) to obtain the title compound (colorless oil, 5.4 g, yield: 77.7%). LC/MS (ESI) m/z: 341 [M+H]$^+$.

**Step 3: Tert-butyl (2S,6S)-4-benzyl-2-((benzyloxy)methyl)-6-methyl-3-oxopiperazine-1-carboxylate**

(S)-(1-(N-benzyl-2-chloroacetamido)propan-2-yl)aminotert-butyl formate (5.4 g, 15.9 mmol) was mixed with tetrahydrofuran (50 mL) and N, N-dimethylformamide (50 mL). Under cooling with ice water, sodium hydride (1.6 g, 41.3 mmol) with the mass fraction of 60% was portionwise slowly added. After adding, with the temperature rising to room temperature, the mixture was stirred at room temperature for 2 hours. The reaction mixture was poured into saturated aqueous ammonium chloride solution cooled with ice water, and then extracted with ethyl acetate. The organic phase was washed with saturated brine, dried over anhydrous sodium sulfate, and concentrated under reduced pressure. The residue was purified by column chromatography (eluent: petroleum ether, ethyl acetate, gradient: 10-30% ethyl acetate) to

obtain the title compound (colorless oil, 2.4 g, yield: 49.7%). LC/MS (ESI) m/z: 305 $[M+H]^+$.

**Step 4: Tert-butyl (2S,6S)-4-benzyl-2-((benzyloxy)methyl)-6-methyl-3-oxopiperazine-1-carboxylate**

(S)-4-benzyl-2-methyl-5-oxopiperazin-1-tert-butyl carboxylate (1.0 g, 3.3 mmol) was dissolved inanhydrous tetrahydrofuran (10 mL). Under the nitrogen atmosphere, 1.0 M lithium bis(trimethylsilicyl)amide/tetrahydrofuran solution (4 mL, 4 mmol) was dropwise added at -78°C to the mixture. The reaction mixture was stirred at -78°C for one hour, and benzylchloromethyl ether (1.3 g, 8.6 mmol) was dropwise added. After the addition, the mixture was continued to be stirred at - 78°C for one hour. At -20°C, 1N hydrochloric acid (4 mL) was added to the reaction mixture, which was then extracted with ethyl acetate. The organic phase was washed with saturated brine, dried over anhydrous sodium sulfate, and concentrated under reduced pressure. The residue was purified by column chromatography (eluent: petroleum ether, ethyl acetate, gradient: 10-30% ethyl acetate) to obtain the title compound (colorless oil, 800 mg, yield: 77.7%). LC/MS (ESI) m/z: 425 $[M+H]^+$.

**Step 5: Tert-butyl (2R,6S)-4-benzyl-2-((benzyloxy)methyl)-6-methylpiperazine-1-carboxylate**

(2S, 6S)-4-benzyl-2-((benzyloxy)methyl)-6-methyl-3-oxopiperazin-1-tert-butyl carboxylate (800 mg, 1.9 mmol) was dissolved in tetrahydrofuran (10 mL). At 0°C, 1M borane /tetrahydrofuran solution (9.5 mL, 9.5 mmol) was dropwise added to the mixture. After the addition, the mixture was heated to 60°C, and stirred for 6 hours. Methanol (-10 mL) was added to the reaction mixture at 0°C for quenching, and the resulting mixture was stirred at 60°C for one hour. The resulting mixture was concentrated under reduced pressure; and the residue was purified by column chromatography (eluent: petroleum ether/ethyl acetate, gradient: 10-30% ethyl acetate) to obtain the title compound (colorless oil, 450 mg, yield: 57.8%). LC/MS (ESI) m/z: 411 $[M+H]^+$.

**Step 6: Tert-butyl (2R,6S)-2-(hydroxymethyl)-6-methylpiperazine-1-carboxylate**

In an autoclave, palladium hydroxide on carbon (50 mg) with the mass fraction of 20% was added to the solution of (2R,6S)-4-benzyl-2-((benzyloxy)methyl)-6-methylpiperazin-1-tert-butyl carboxylate (200 mg, 488 mmol) in methanol (5 mL); the flask was replaced with nitrogen gas three times and then the mixture was stirred for 16 hours at room temperature under an atmosphere of hydrogen at 1 MPa. After completion of the reaction, the reaction mixture was filtered through diatomite, and the filtrate was concentrated under reduced pressure to obtain the title compound (white solid, 90 mg, yield: 80.4%). LC/MS (ESI) (m/z): 231 $[M+H]^+$.

**Step 7-8: 1-chloro-3-(5-(difluoromethyl)-1,3,4-thiadiazol-2-yl)-8-((3R,5S)-3-(hydroxymethyl)-5-methylpiperazin-1-yl)-N-(3-methyloxetan-3-yl)imidazo[1,5-a]pyridine-6-sulfonamide**

The title compound was obtained by referring to the preparation method in Steps 3-4 of **Example 391.** $^1$H NMR (400 MHz, $CD_3OD$) δ 9.91 (d, *J* = 1.1 Hz, 1H), 7.35 (t, *J* = 53.5 Hz, 1H), 6.95 (d, *J* = 1.1 Hz, 1H), 4.40 - 4.32 (m, 2H), 4.07 - 3.86 (m, 4H), 3.74 - 3.58 (m, 2H), 3.39 - 3.32 (m, 2H), 3.26 - 3.21 (m, 1H), 3.12 - 2.96 (m, 1H), 1.66 (s, 3H), 1.53 (d, *J* = 4.8 Hz, 3H). LC/MS (ESI) (m/z): 564 $[M+H]^+$.

**Example 403: 1-chloro-3-(5-(difluoromethyl)-1,3,4-thiadiazol-2-yl)-8-((3R,5S)-3-(methoxymethyl)-5-methylpiperazin-1-yl)-N-(3-methyloxetan-3-yl)imidazo[1,5-a]pyridine-6-sulfonamide**

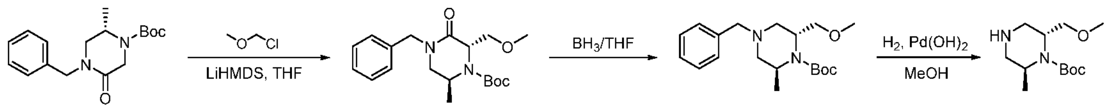


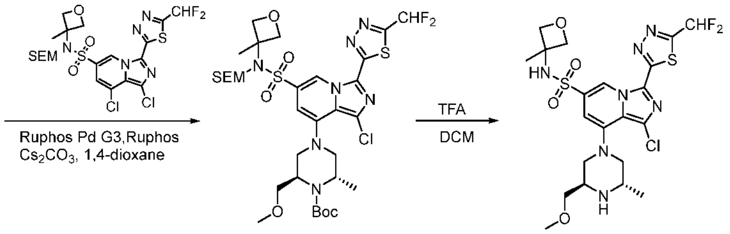


**Step 1: tert-butyl (2S,6S)-4-benzyl-2-(methoxymethyl)-6-methyl-3-oxopiperazine-1-carboxylate**

At -78°C, 1.0 M lithium bis(trimethylsilicyl)amide/tetrahydrofuran solution (2 mL, 1.97 mmol) was dropwise added slowly to the solution of (S)-4-benzyl-2-methyl-5-oxopiperazin-1-tert-butyl carboxylate (500 mg, 1.64 mmol) in anhydrous tetrahydrofuran (5 mL). The reaction mixture was stirred at -78°C for one hour; chloromethyl methyl ether (328 mg, 4.10 mmol) was then dropwise added; and the mixture was continued to be stirred for one hour. After completion of the reaction, 1 N hydrochloric acid (2 mL) was added at -20°C; the reaction mixture was extracted with ethyl acetate; the organic phase was washed with saturated brine, dried over anhydrous sodium sulfate, and concentrated under reduced pressure. The residue was purified by column chromatography (eluent: petroleum ether, ethyl acetate, gradient: 10-30% ethyl acetate) to obtain the title compound (colorless oil, 200 mg, yield: 34.8%). LC/MS (ESI) m/z: 349 $[M+H]^+$.

**Step 2: Tert-butyl (2R,6S)-4-benzyl-2-(methoxymethyl)-6-methylpiperazine-1-carboxylate**

At 0°C, 1.0 M borane/tetrahydrofuran solution (2.9 mL, 2.85 mmol) was added was dropwise added slowly to the solution of (2S,6S)-4-benzyl-2-(methoxymethyl)-6-methyl-3-oxopiperazin-1-tert-butyl carboxylate (200 mg, 0.57 mmol) in anhydrous tetrahydrofuran (10 mL). The reaction mixture was stirred at 60°C for 6 hours. After completion of the reaction, methanol (-5 mL) was added to the reaction mixture at 0°C for quenching, and then the mixture was heated to 60°C, and stirred for one hour. The resulting mixture was concentrated under reduced pressure; and the residue was purified by column chromatography (eluent: petroleum ether/ethyl acetate, gradient: 10-30% ethyl acetate) to obtain the title compound (colorless oil, 120 mg, yield: 62.8%). LC/MS (ESI) m/z: 335 $[M+H]^+$.

**Step 3: Tert-butyl (2R,6S)-2-(methoxymethyl)-6-methylpiperazine-1-carboxylate**

At room temperature, palladium hydroxide on carbon (10 mg) with the mass fraction of 10% was added to the solution of (2R,6S)-4-benzyl-2-(methoxymethyl)-6-methylpiperazin-1-tert-butyl carboxylate (120 mg, 0.36 mmol) in methanol (2 mL). The reaction mixture was ventilated three times under an atmosphere of nitrogen and then stirred for one hour at room temperature under an atmosphere provided by a hydrogen-filled balloon. After completion of the reaction, the reaction mixture was filtered through diatomite, and the filtrate was concentrated under reduced pressure to obtain a crude title compound (colorless oil, 85 mg, yield: 96.9%), which was directly used in the next step without purification. $^1$H NMR (400 MHz, $CDCl_3$) δ 3.94 - 3.89 (m, 1H), 3.85 - 3.79 (m, 1H), 3.63 - 3.58 (m, 1H), 3.52 - 3.47 (m, 1H), 3.37 (s, 3H), 3.35 - 3.32 (m, 1H), 3.13 - 3.10 (m, 2H), 2.75 - 2.69 (m, 1H), 1.47 (s, 9H), 1.34 (d, $J$ = 6.6 Hz, 3H). LC/MS (ESI) (m/z): 245 $[M+H]^+$.

**Step 4-5: 1-chloro-3-(5-(difluoromethyl)-1,3,4-thiadiazol-2-yl)-8-((3R,5S)-3-(methoxymethyl)-5-methylpiperazin-1-yl)-N-(3-methyloxetan-3-yl)imidazo[1,5-a]pyridine-6-sulfonamide**

The title compound was obtained by referring to the preparation method in Steps 3-4 of **Example 391.** $^1$H NMR (400 MHz, $CD_3OD$) δ 9.90 (d, $J$ = 1.7 Hz, 1H), 7.34 (t, $J$ = 53.5 Hz, 1H), 6.92 (s, 1H), 4.83 - 4.79 (m, 2H), 4.34 (d, $J$ = 5.9 Hz, 2H), 4.04 - 3.93 (m, 2H), 3.88 - 3.82 (m, 1H), 3.75 - 3.71 (m, 1H), 3.65 - 3.59 (m, 2H), 3.46 (s, 3H), 3.21 - 3.13 (m, 2H), 1.65 (s, 3H), 1.49 (d, $J$ = 3.7 Hz, 3H). LC/MS (ESI) (m/z): 578 $[M+H]^+$.

**Example 404: 3-(5-(difluoromethyl)-1,3,4-thiadiazol-2-yl)-8-((3R,5S)-3-(methoxymethyl)-5-methylpiperazin-1-yl)-N-(3-methyloxetan-3-yl)imidazo[1,5-a]pyridine-6-sulfonamide**

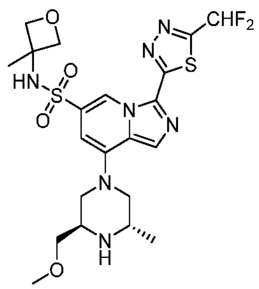

The title compound was obtained by referring to the preparation method in **Example 403.** $^1H$ NMR (400 MHz, $CD_3OD$) δ 9.82 (s, 1H), 7.88 (s, 1H), 7.33 (t, *J* = 53.5 Hz, 1H), 6.79 (s, 1H), 4.79 (d, *J* = 6.0 Hz, 2H), 4.32 (d, *J* = 5.2 Hz, 2H), 3.92 - 3.85 (m, 1H), 3.83 - 3.77 (m, 2H), 3.76 - 3.71 (m, 1H), 3.64 - 3.53 (m, 2H), 3.50 (s, 3H), 3.46 - 3.40 (m, 1H), 3.26 - 3.19 (m, 1H), 1.63 (s, 3H), 1.47 (d, *J* = 5.4 Hz, 3H). LC/MS (ESI) (m/z): 544 $[M+H]^+$.

**Effect Examples**

**Experimental Approaches:**

**1. Biochemical Assay**

1) 20 nL compounds at different concentrations were added to the 384-well plate (final concentrations of compounds: 1000 nM, 300 nM, 100 nM, 30 nM, 10 nM, 3 nM, 1 nM, 0.3 nM, 0.1 nM, 0.03 nM, 0 nM) byEcho 550 (LABCYTE, #550);
2) preparation of reaction buffer I: 50 mM Tris (2-amino-2-hydroxymethyl-1,3-propanediol, Sigma, #T1503) at pH 7.4, 0.015% Triton X-100 (4-(1,1,3,3-tetramethylbutyl)phenyl-polyethylene glycol, Sigma, #T8787), 0.1% BSA (bovine serum albumin, Sigma, #A1933), 5 mM KCl, and 66.2 uM TFMU-ADPr (fluorescein-4-trifluoromethylumbelliferone-ADP ribose);
3) the reaction buffer I was added to the 384-well plate at 10 μL/well;
4) preparation of reaction buffer II: 50 mM Tris at PH 7.4, 0.015% Triton X-100, 0.1% BSA, 5 mM KCl, and 4 nM PARG (poly ADP-ribonal hydrolase);
5) the reaction buffer II was added to the 384-well plate at 10 μL/well to initiate the reaction;
6) the plate was incubated at room temperature for 3min;
7) the 384-well plate was placed in the microplate reader, and shook for 5 seconds for signal detection;
8) Data analysis was carried out using GraphPad software to determine IC50.

**2. Cell Function Assay (In-Cell Western)**

1) Human breast squamous carcinoma cells HCC1806 (ATCC) were seeded to the 384-well plate (Corning, #3764) at 20,000 cells/well, incubated for 24 h in a $CO_2$ (5%) incubator at 37°C to allow for complete adherence of the cells;
2) 120 nL compounds or DMSO were added to the 384-well plate by Echo 550 (LABCYTE, #550) (final concentrations of compounds: 30000 nM, 10000 nM, 3000 nM, 1000 nM, 300 nM, 100 nM, 30 nM, 10 nM, 3 nM, 1 nM, 0 nM), and then the cells were incubated for 1 h in a $CO_2$ (5%) incubator at 37°C;
3) 40 nL MMS (methyl mesylate, Sigma, #129925)(final concentration: 0.5mM) was added to the plate by Echo 550, and the cells were treated for 1 h in a $CO_2$ (5%) incubator at 37°C 4) the culture medium was discarded, and the cells were fixed with 50 μL/well 3.7% paraformaldehyde for 20 min at room temperature;
5) the cells were washed three times with PBST (136 mM NaCl, 2.6 mM KCl, 8 mM $Na_2HPO_4$, 2 mM $KH_2PO_4$, 0.05% Tween-20) buffer, each time 5 min;
6) after fixation, the cells were permeabilized with 50 μL/well 0.3% Triton X-100 for 5 min at room temperature;
7) the cells were washed three times with PBST (136 mM NaCl, 2.6 mM KCl, 8 mM $Na_2HPO_4$, 2 mM $KH_2PO_4$, 0.05%

Tween-20) buffer, each time 5 min;
8) after cell permeabilization, the cells were blocked with 50 μL/well 5% BSA for 90 min at room temperature ;
9) after cell blocking, the PAR/pADPr (R&D, #4335-MC-100) antibody (diluted 1:2000) was added at 25 μL/well to the cellsand then incubated for 16 h at 4°C;
10) the cells were washed four times with PBST (136 mM NaCl, 2.6 mM KCl, 8 mM $Na_2HPO_4$, 2 mM $KH_2PO_4$, 0.05% Tween-20) buffer, each time 5 min;
11) 800CW goat anti-mouse (diluted 1:200) (LiCRO, #926-32210) and DRAQ5 (diluted 1:200) (Cell Signaling Technology, #4084L) were added at 25 μL/well to the cells, and then incubated for 60 minutes at room temperature;
12) the cells were washed four times with PBST (136 mM NaCl, 2.6 mM KCl, 8 mM $Na_2HPO_4$, 2 mM $KH_2PO_4$, 0.05% Tween-20) buffer, each time 5 min;
13) PBS (136 mM NaCl, 2.6 mM KCl, 8 mM $Na_2HPO_4$, 2 mM $KH_2PO_4$) buffer was added to the cells, and Li-Cor Bioscience scanning was used for signal detection;
14) the data were analyzed using GraphPad software to determine the IC50.

**3. Cell Viability Assay**

1) HCC1806 (Nanjing Cobioer, ATCC cell bank, 200 cells/well) was plated in the 96-well plate, and incubated for 24 h in a $CO_2$ (5%) incubator at 37°C to allow for complete adherence of the cells;
2) media (100 μL/well) containing the compounds (final concentrations of compounds: 30000 nM, 10000 nM, 3000 nM, 1000 nM, 300 nM, 100 nM, 30 nM, 10 nM, 3 nM, 1 nM, 0 nM) were added to the cells, and then, incubated for 7 days in the CO2 (5%) incubator at 37°C;
3) the culture plate containing the cells was held at room temperature for 30 min to equilibrate the plate and its contents to room temperature;
4) CellTiter-Glo (Promega: V9101) reagent (100 μL/well) was added to each well of cells, and the contents were mixed on an orbital shaker for 2 minutes;
5) the plate was incubated at room temperature for 10 min to stabilize the signal, and detected by the microplate reader (Thermo: VarioskanLUX);
6) data were analyzed using GraphPad software to determine the $IC_{50}$.

**Data list:**

Biochemical assay and cell function assay:

$$++++ \leq 100nM,\ 100\ nM < +++ \leq 500\ nM,\ 500\ nM < ++ \leq 1\ \mu M,\ + > 1\ \mu M;$$

Cell viability assay:

$$++++ \leq 500\ nM,\ 500\ nM < +++ \leq 5\ \mu M,\ 5\ \mu m < ++ \leq 30\ \mu M,\ + > 30\ \mu M;$$

NT = not detected;

| Example | Compound structure | $IC_{50}$ (nM) in biochemical assay | $IC_{50}$ (nM) in cell function assay | $IC_{50}$ (nM) in cell viability assay |
|---|---|---|---|---|
| 1 | 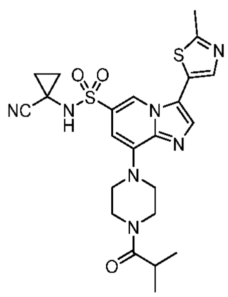 | NT | NT | NT |

(continued)

| Example | Compound structure | $IC_{50}$ (nM) in biochemical assay | $IC_{50}$ (nM) in cell function assay | $IC_{50}$ (nM) in cell viability assay |
|---|---|---|---|---|
| 2 | 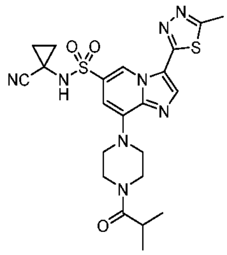 | NT | NT | NT |
| 3 | 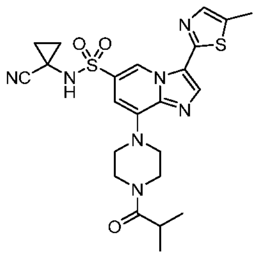 | NT | NT | NT |
| 4 | 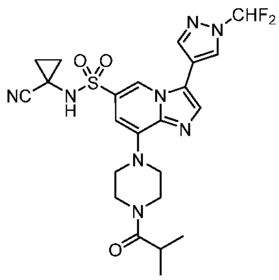 | NT | NT | NT |
| 5 | 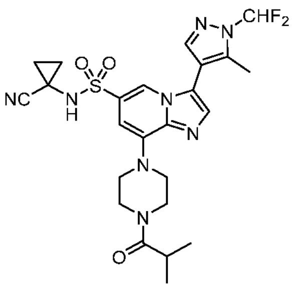 | NT | NT | NT |
| 6 | 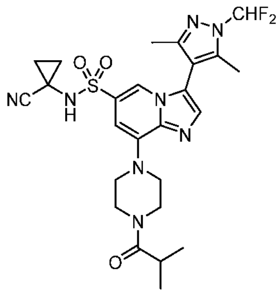 | NT | NT | NT |

(continued)

| Example | Compound structure | IC50 (nM) in biochemical assay | IC50 (nM) in cell function assay | IC50 (nM) in cell viability assay |
|---|---|---|---|---|
| 7 | 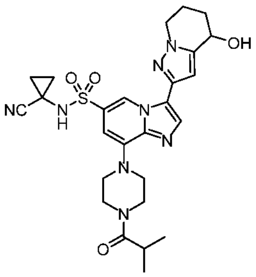 | NT | NT | NT |
| 8 | 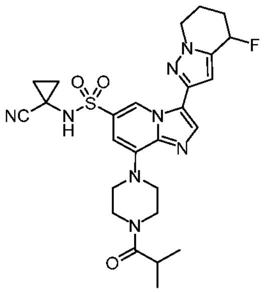 | NT | NT | NT |
| 9 | 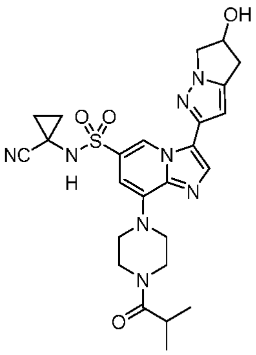 | NT | NT | NT |
| 10 | 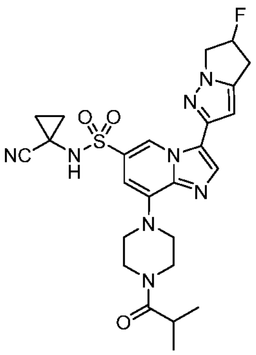 | ++ | NT | NT |
| 11 | 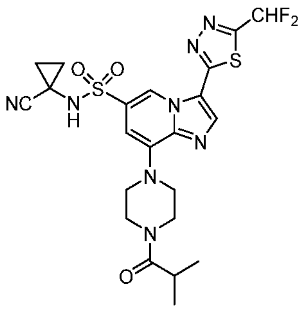 | ++++ | ++++ | +++ |

(continued)

| Example | Compound structure | $IC_{50}$ (nM) in biochemical assay | $IC_{50}$ (nM) in cell function assay | $IC_{50}$ (nM) in cell viability assay |
|---|---|---|---|---|
| 12 | 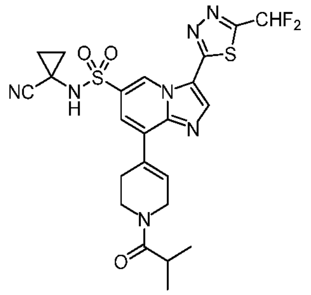 | ++++ | NT | ++++ |
| 13 | 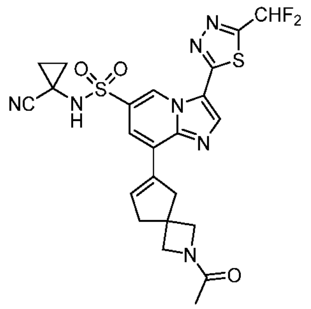 | ++++ | NT | NT |
| 14 | 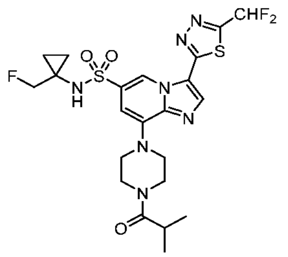 | ++++ | ++++ | ++++ |
| 15 | 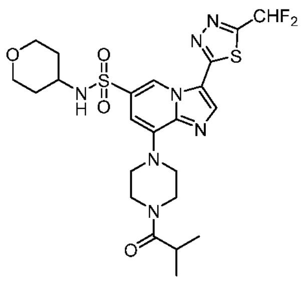 | NT | NT | NT |
| 16 | 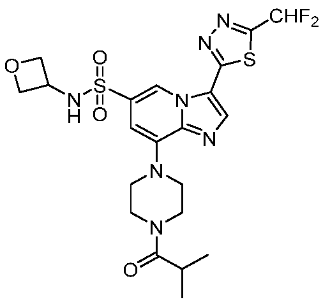 | ++++ | NT | NT |

(continued)

| Example | Compound structure | $IC_{50}$ (nM) in biochemical assay | $IC_{50}$ (nM) in cell function assay | $IC_{50}$ (nM) in cell viability assay |
|---|---|---|---|---|
| 17 | 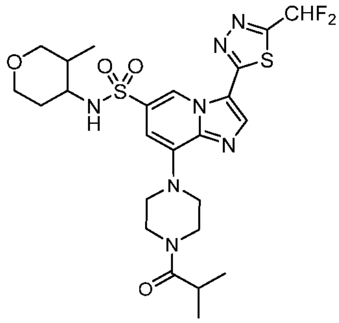 | NT | NT | NT |
| 18 | 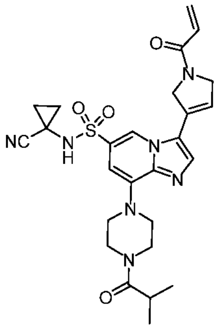 | ++++ | NT | NT |
| 19 | 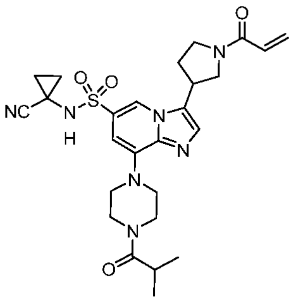 | NT | NT | NT |
| 20 | 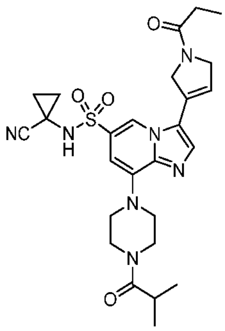 | ++++ | NT | NT |
| 21 | 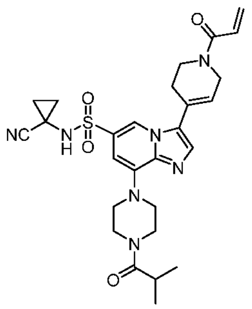 | NT | NT | NT |

(continued)

| Example | Compound structure | $IC_{50}$ (nM) in biochemical assay | $IC_{50}$ (nM) in cell function assay | $IC_{50}$ (nM) in cell viability assay |
|---|---|---|---|---|
| 22 | 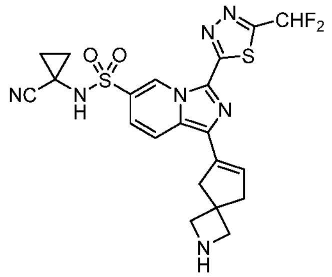 | ++++ | NT | + |
| 23 | 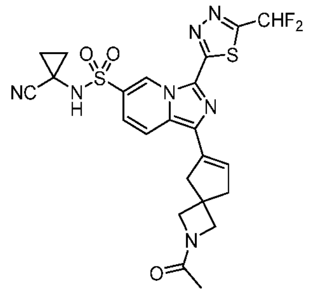 | NT | NT | NT |
| 24 | 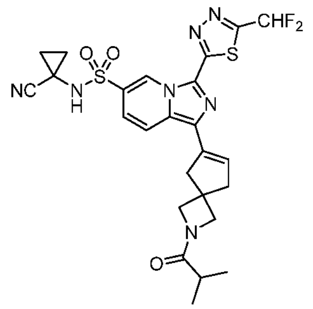 | ++++ | ++++ | ++++ |
| 25 | 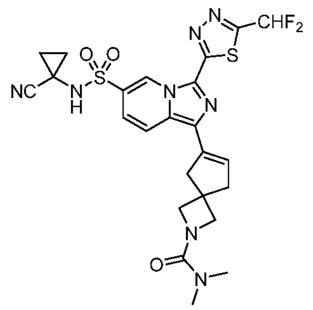 | ++++ | NT | ++++ |
| 26 | 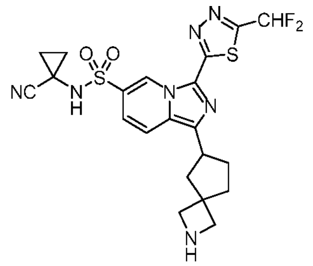 | ++++ | + | NT |

(continued)

| Example | Compound structure | $IC_{50}$ (nM) in biochemical assay | $IC_{50}$ (nM) in cell function assay | $IC_{50}$ (nM) in cell viability assay |
|---|---|---|---|---|
| 27 | 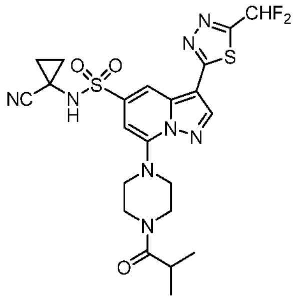 | ++++ | +++ | +++ |
| 28 | 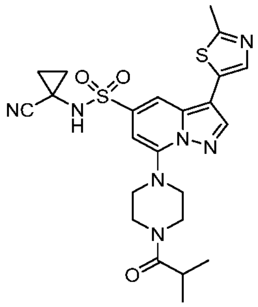 | ++++ | NT | NT |
| 29 | 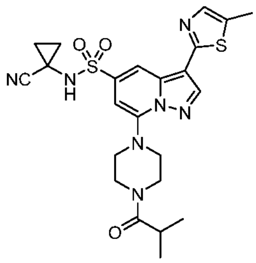 | ++++ | NT | ++ |
| 30 | 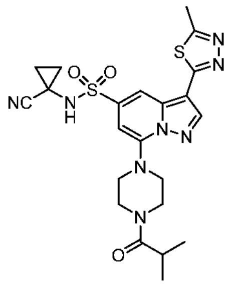 | ++++ | NT | + |
| 31 | 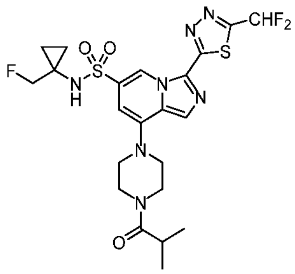 | ++++ | ++++ | ++++ |

(continued)

| Example | Compound structure | $IC_{50}$ (nM) in biochemical assay | $IC_{50}$ (nM) in cell function assay | $IC_{50}$ (nM) in cell viability assay |
|---|---|---|---|---|
| 32 | 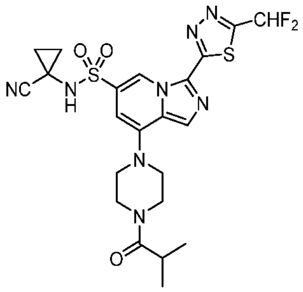 | ++++ | ++++ | +++ |
| 33 | 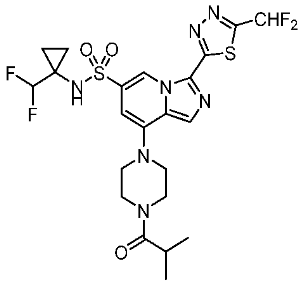 | ++++ | +++ | NT |
| 34 | 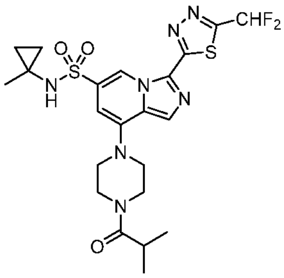 | ++++ | ++++ | ++++ |
| 35 | 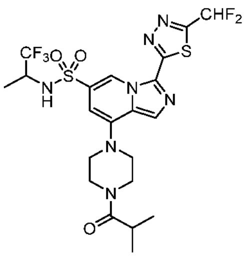 | ++ | NT | NT |
| 36 | 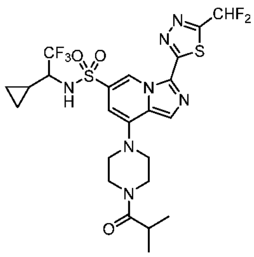 | ++ | NT | NT |

(continued)

| Example | Compound structure | IC50 (nM) in biochemical assay | IC50 (nM) in cell function assay | IC50 (nM) in cell viability assay |
|---|---|---|---|---|
| 37 | 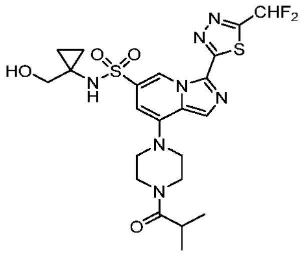 | ++++ | NT | NT |
| 38 | 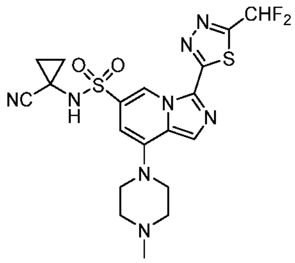 | ++++ | NT | NT |
| 39 | 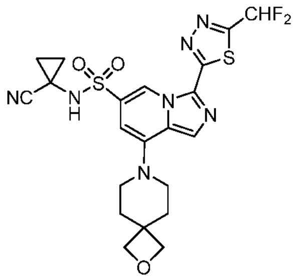 | ++++ | ++++ | ++++ |
| 40 | 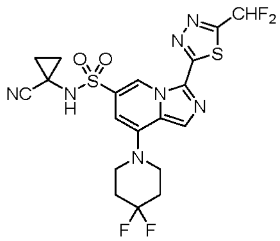 | ++++ | +++ | +++ |
| 41 | 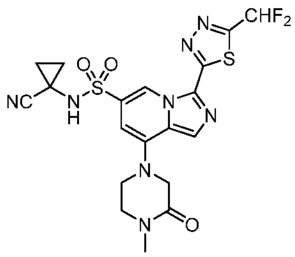 | ++++ | NT | NT |
| 42 | 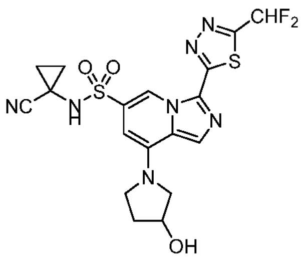 | ++++ | NT | NT |

(continued)

| Example | Compound structure | $IC_{50}$ (nM) in biochemical assay | $IC_{50}$ (nM) in cell function assay | $IC_{50}$ (nM) in cell viability assay |
|---|---|---|---|---|
| 43 | 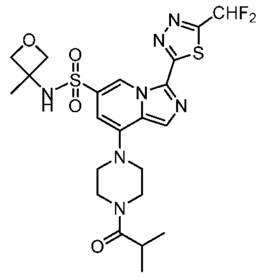 | ++++ | ++++ | ++++ |
| 44 | 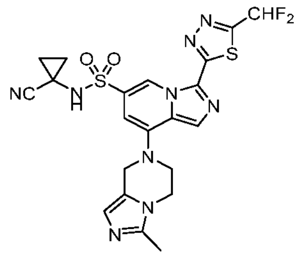 | ++++ | NT | ++ |
| 45 | 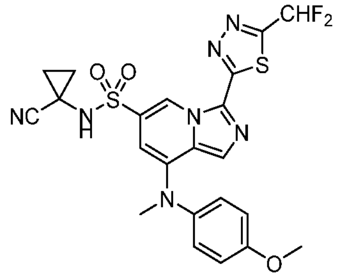 | +++ | NT | NT |
| 46 | 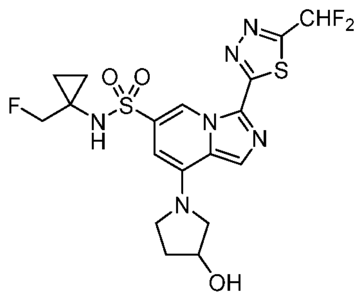 | ++++ | NT | NT |
| 47 | 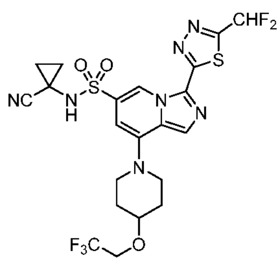 | ++++ | NT | NT |

(continued)

| Example | Compound structure | $IC_{50}$ (nM) in biochemical assay | $IC_{50}$ (nM) in cell function assay | $IC_{50}$ (nM) in cell viability assay |
|---|---|---|---|---|
| 48 | 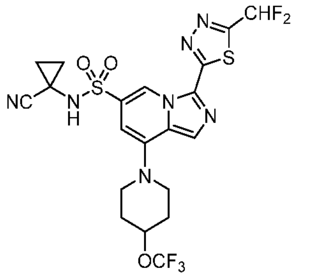 | ++++ | NT | NT |
| 49 | 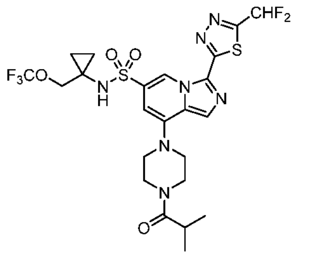 | ++++ | NT | ++ |
| 50 | 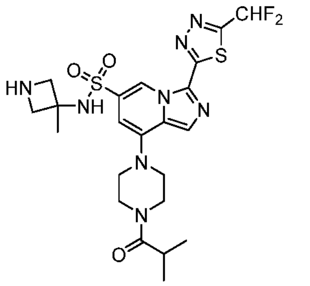 | + | NT | + |
| 51 | 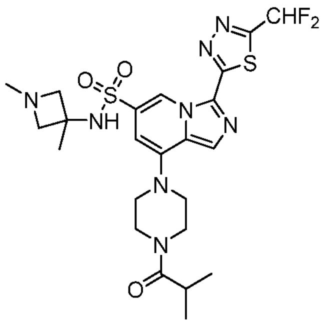 | + | NT | + |
| 52 | 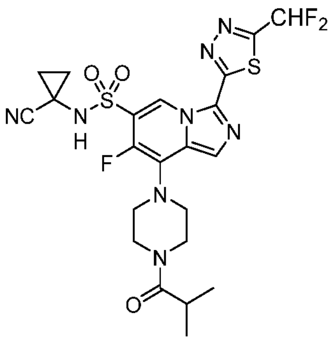 | ++++ | +++ | +++ |

(continued)

| Example | Compound structure | $IC_{50}$ (nM) in biochemical assay | $IC_{50}$ (nM) in cell function assay | $IC_{50}$ (nM) in cell viability assay |
|---|---|---|---|---|
| 53 | 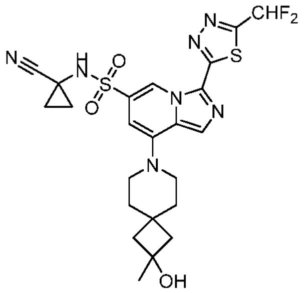 | ++++ | +++ | +++ |
| 54 | 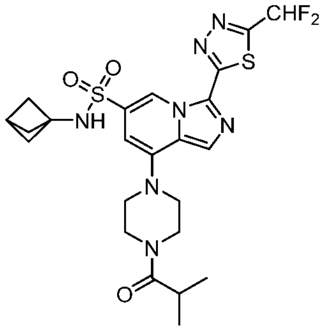 | ++++ | ++++ | ++ |
| 55 | 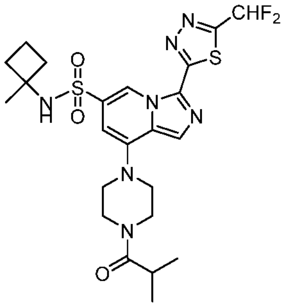 | ++++ | +++ | ++ |
| 56 | 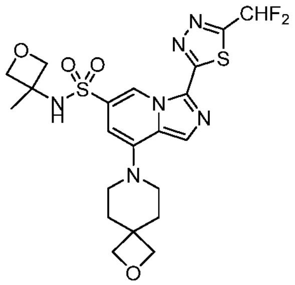 | ++++ | ++++ | +++ |
| 57 | 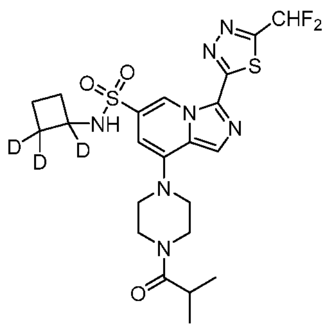 | ++++ | + | + |

(continued)

| Example | Compound structure | $IC_{50}$ (nM) in biochemical assay | $IC_{50}$ (nM) in cell function assay | $IC_{50}$ (nM) in cell viability assay |
|---|---|---|---|---|
| 58 | 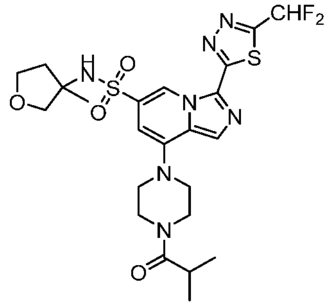 | ++++ | + | + |
| 59 | 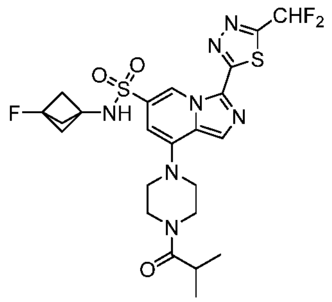 | + | NT | + |
| 60 | 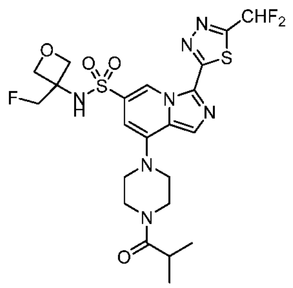 | ++++ | ++++ | ++++ |
| 61 | 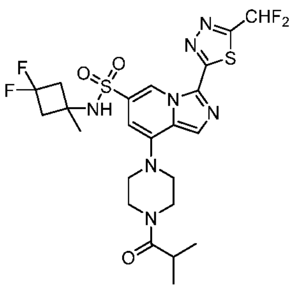 | +++ | NT | + |
| 62 | 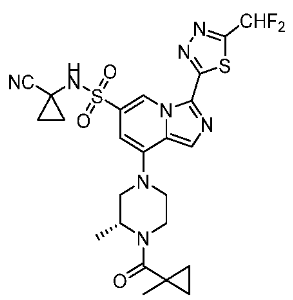 | ++++ | ++++ | ++++ |

(continued)

| Example | Compound structure | $IC_{50}$ (nM) in biochemical assay | $IC_{50}$ (nM) in cell function assay | $IC_{50}$ (nM) in cell viability assay |
|---|---|---|---|---|
| 63 | 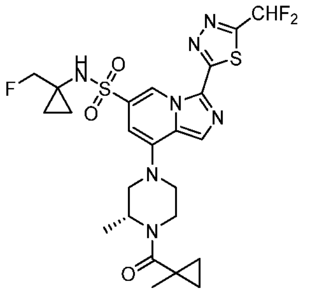 | ++++ | ++++ | ++++ |
| 64 | 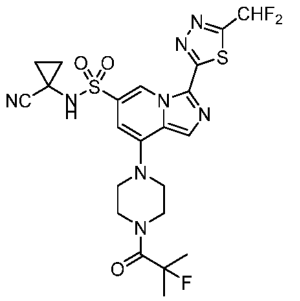 | ++++ | ++++ | ++++ |
| 65 | 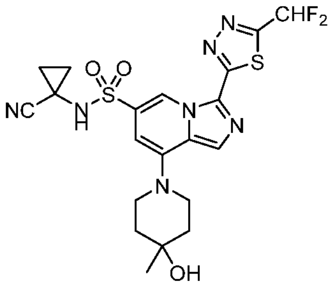 | ++++ | +++ | ++ |
| 66 | 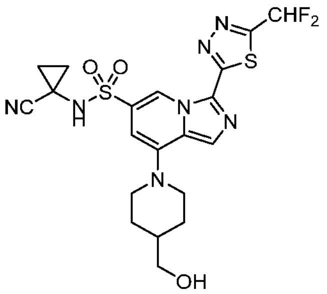 | ++++ | ++++ | +++ |
| 67 | 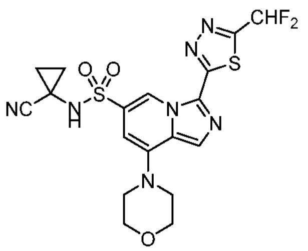 | ++++ | ++++ | +++ |

(continued)

| Example | Compound structure | $IC_{50}$ (nM) in biochemical assay | $IC_{50}$ (nM) in cell function assay | $IC_{50}$ (nM) in cell viability assay |
|---|---|---|---|---|
| 68 | 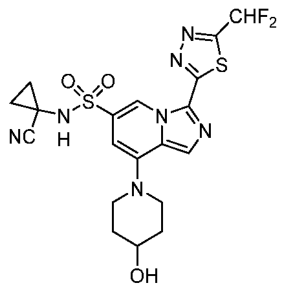 | ++++ | ++++ | +++ |
| 69 | 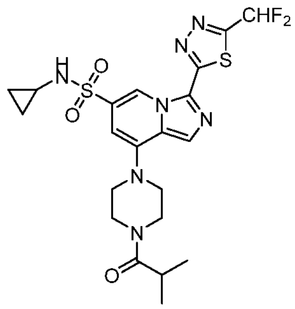 | ++++ | ++++ | ++++ |
| 70 | 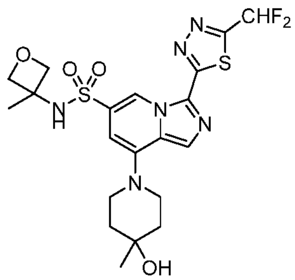 | ++++ | NT | +++ |
| 71 | 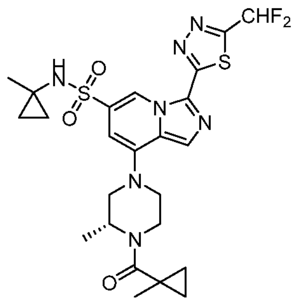 | ++++ | ++++ | ++++ |
| 72 | 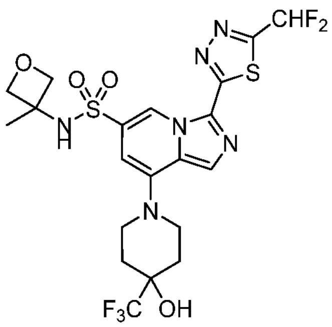 | ++++ | NT | +++ |

(continued)

| Example | Compound structure | $IC_{50}$ (nM) in biochemical assay | $IC_{50}$ (nM) in cell function assay | $IC_{50}$ (nM) in cell viability assay |
|---|---|---|---|---|
| 73 | 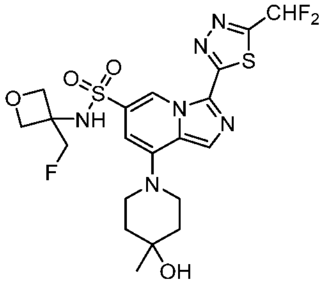 | ++++ | NT | +++ |
| 74 | 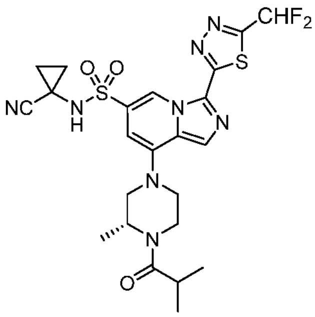 | ++++ | ++++ | ++++ |
| 75 | 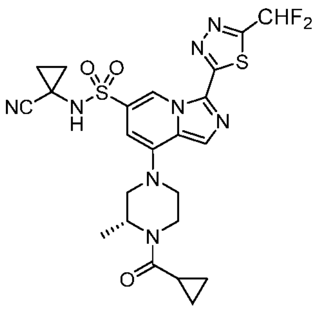 | ++++ | ++++ | ++++ |
| 76 | 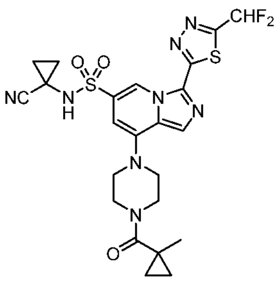 | ++++ | ++++ | ++++ |
| 77 | 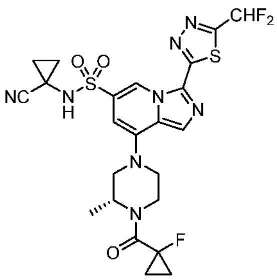 | ++++ | ++++ | +++ |

(continued)

| Example | Compound structure | $IC_{50}$ (nM) in biochemical assay | $IC_{50}$ (nM) in cell function assay | $IC_{50}$ (nM) in cell viability assay |
|---|---|---|---|---|
| 78 | 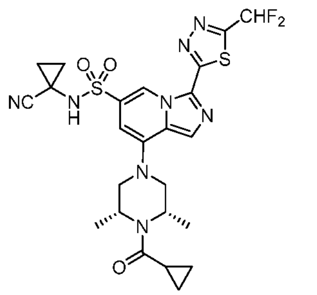 | ++++ | ++++ | +++ |
| 79 | 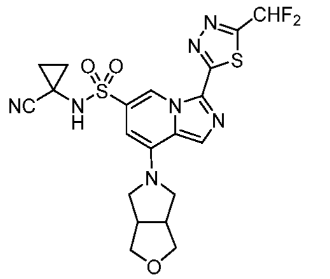 | ++++ | NT | +++ |
| 80 | 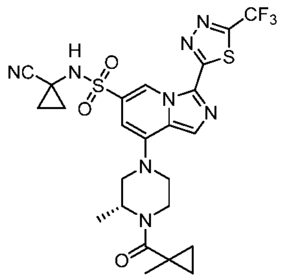 | ++++ | ++++ | +++ |
| 81 | 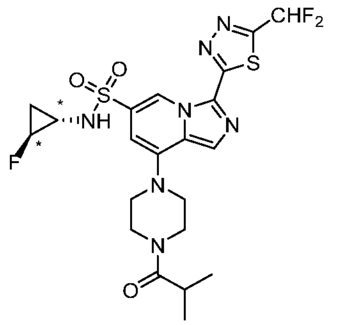 | + | NT | ++ |
| 82 | 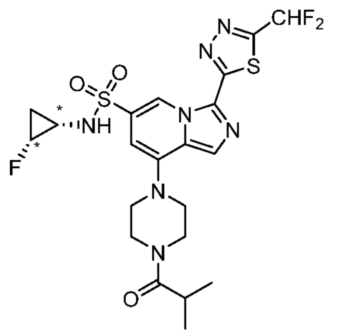 | +++ | NT | ++ |

(continued)

| Example | Compound structure | $IC_{50}$ (nM) in biochemical assay | $IC_{50}$ (nM) in cell function assay | $IC_{50}$ (nM) in cell viability assay |
|---|---|---|---|---|
| 83 | 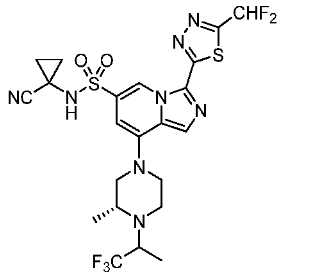 | +++ | NT | +++ |
| 84 | 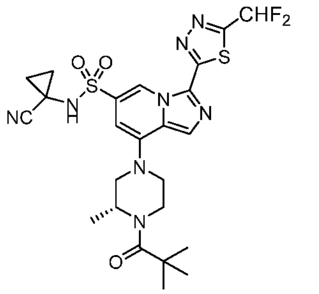 | ++++ | ++++ | ++++ |
| 85 | 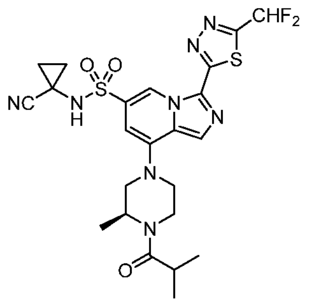 | ++++ | ++++ | +++ |
| 86 | 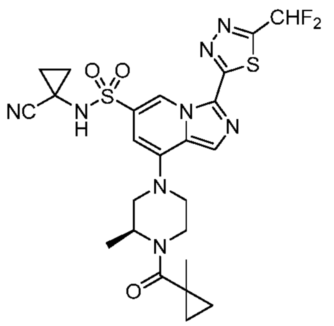 | ++++ | ++++ | +++ |
| 87 | 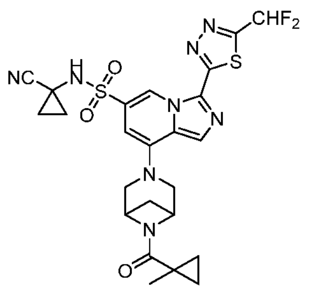 | ++++ | ++++ | +++ |

(continued)

| Example | Compound structure | $IC_{50}$ (nM) in biochemical assay | $IC_{50}$ (nM) in cell function assay | $IC_{50}$ (nM) in cell viability assay |
|---|---|---|---|---|
| 88 | 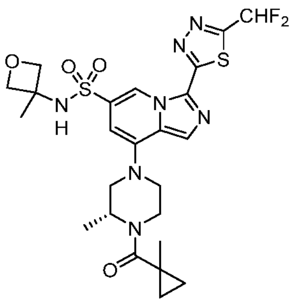 | ++++ | ++++ | +++ |
| 89 | 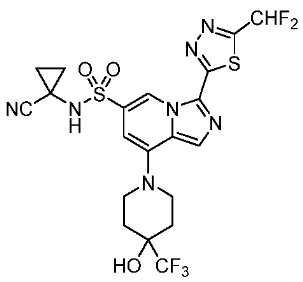 | +++ | NT | +++ |
| 90 | 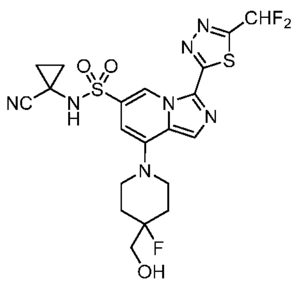 | ++++ | NT | +++ |
| 91 | 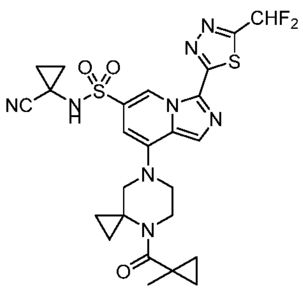 | ++++ | NT | ++++ |
| 92 | 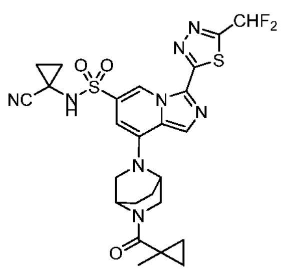 | ++++ | NT | +++ |

(continued)

| Example | Compound structure | $IC_{50}$ (nM) in biochemical assay | $IC_{50}$ (nM) in cell function assay | $IC_{50}$ (nM) in cell viability assay |
|---|---|---|---|---|
| 93 | 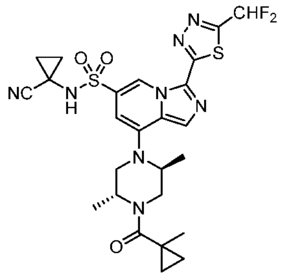 | ++++ | NT | +++ |
| 94 | 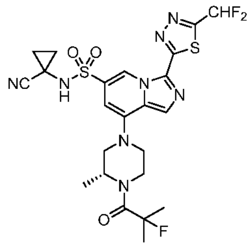 | ++++ | ++++ | ++++ |
| 95 | 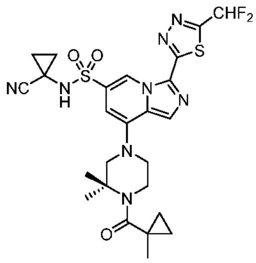 | ++++ | NT | +++ |
| 96 | 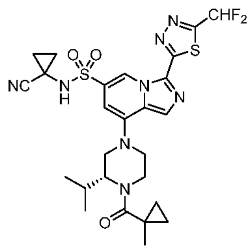 | ++++ | ++++ | ++++ |
| 97 | 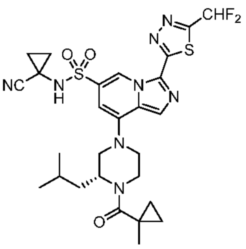 | ++++ | ++++ | ++++ |
| 98 | 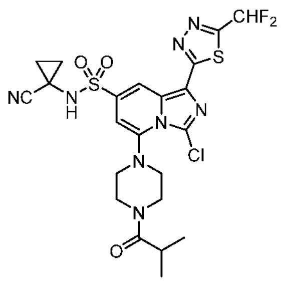 | NT | NT | + |

(continued)

| Example | Compound structure | $IC_{50}$ (nM) in biochemical assay | $IC_{50}$ (nM) in cell function assay | $IC_{50}$ (nM) in cell viability assay |
|---|---|---|---|---|
| 99 | 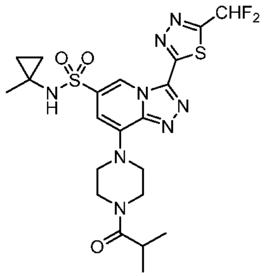 | ++++ | ++++ | ++++ |
| 100 | 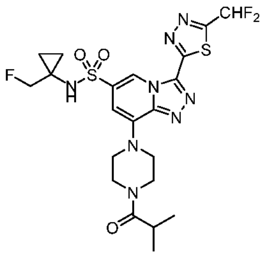 | ++++ | ++++ | ++++ |
| 101 | 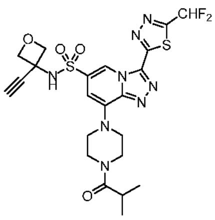 | ++++ | + | ++ |
| 102 | 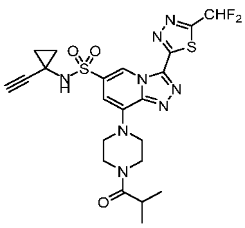 | ++++ | ++++ | ++ |
| 103 | 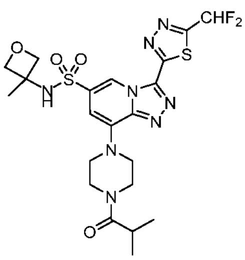 | ++++ | ++++ | +++ |
| 104 | 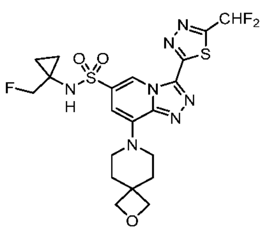 | ++++ | ++++ | ++++ |

(continued)

| Example | Compound structure | $IC_{50}$ (nM) in biochemical assay | $IC_{50}$ (nM) in cell function assay | $IC_{50}$ (nM) in cell viability assay |
|---|---|---|---|---|
| 105 | 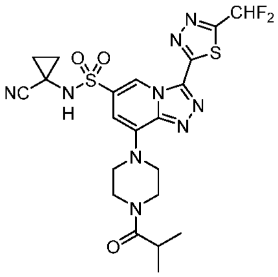 | ++++ | NT | ++ |
| 106 | 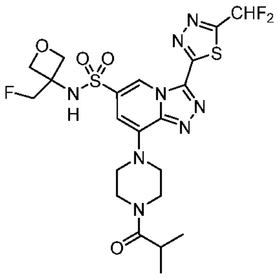 | ++++ | NT | ++ |
| 107 | 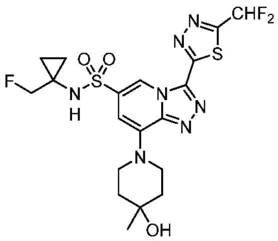 | ++++ | ++++ | +++ |
| 108 | 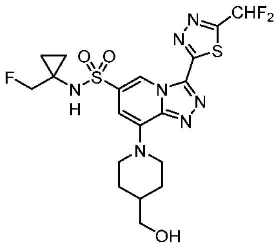 | ++++ | ++++ | +++ |
| 109 | 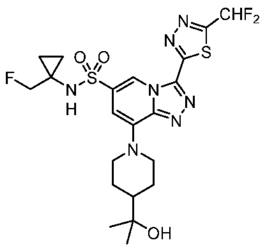 | ++++ | NT | ++ |
| 110 | 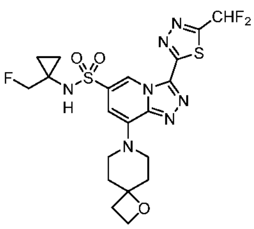 | ++++ | ++++ | +++ |

(continued)

| Example | Compound structure | $IC_{50}$ (nM) in biochemical assay | $IC_{50}$ (nM) in cell function assay | $IC_{50}$ (nM) in cell viability assay |
|---|---|---|---|---|
| 111 | 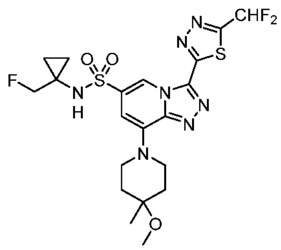 | ++++ | ++++ | +++ |
| 112 | 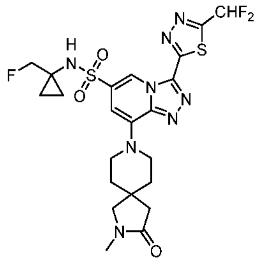 | ++++ | ++++ | +++ |
| 113 | 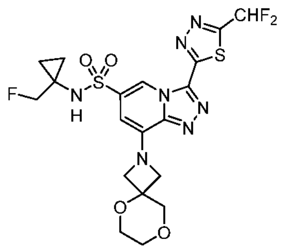 | ++++ | ++++ | +++ |
| 114 | 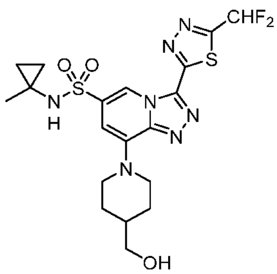 | ++++ | ++++ | +++ |
| 115 | 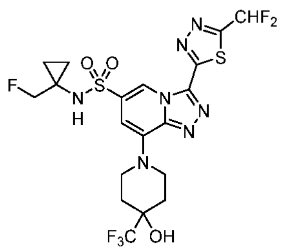 | ++++ | ++++ | ++++ |
| 116 | 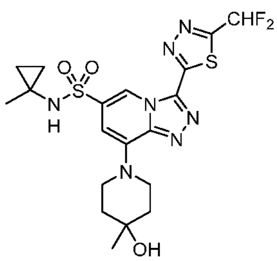 | ++++ | NT | +++ |

(continued)

| Example | Compound structure | $IC_{50}$ (nM) in biochemical assay | $IC_{50}$ (nM) in cell function assay | $IC_{50}$ (nM) in cell viability assay |
|---|---|---|---|---|
| 117 | 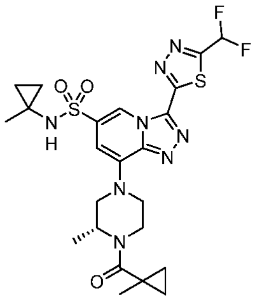 | ++++ | ++++ | ++++ |
| 118 | 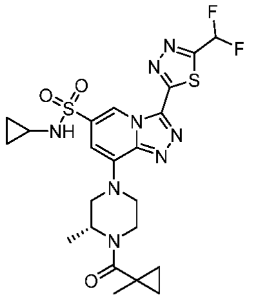 | ++++ | NT | +++ |
| 119 | 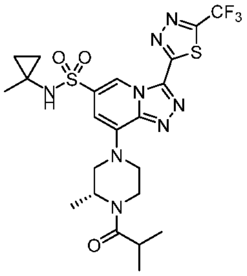 | ++++ | NT | ++++ |
| 120 | 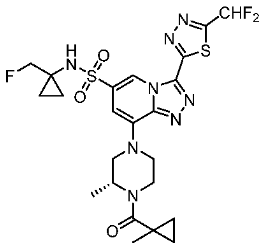 | ++++ | ++++ | ++++ |
| 121 | 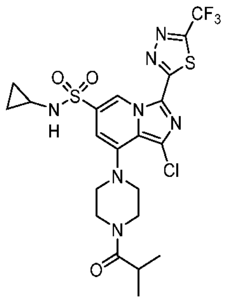 | ++++ | NT | NT |

(continued)

| Example | Compound structure | $IC_{50}$ (nM) in biochemical assay | $IC_{50}$ (nM) in cell function assay | $IC_{50}$ (nM) in cell viability assay |
|---|---|---|---|---|
| 122 | 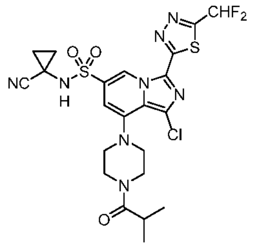 | ++++ | ++++ | ++++ |
| 123 | 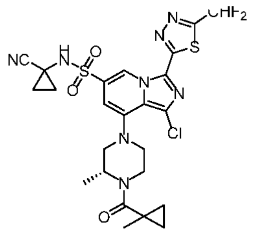 | ++++ | ++++ | +++ |
| 139 | 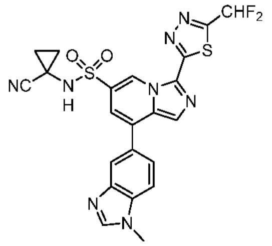 | ++++ | NT | NT |
| 140 | 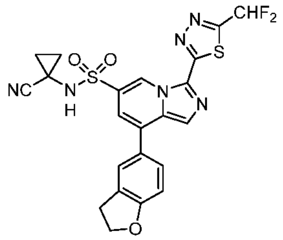 | ++++ | NT | NT |
| 141 | 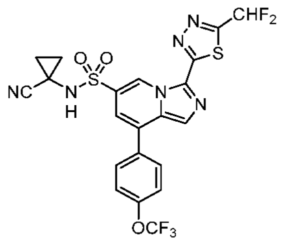 | +++ | NT | NT |
| 142 | 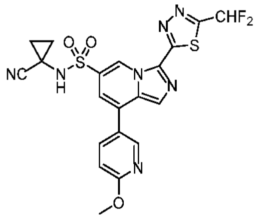 | ++++ | NT | NT |

(continued)

| Example | Compound structure | $IC_{50}$ (nM) in biochemical assay | $IC_{50}$ (nM) in cell function assay | $IC_{50}$ (nM) in cell viability assay |
|---|---|---|---|---|
| 143 | 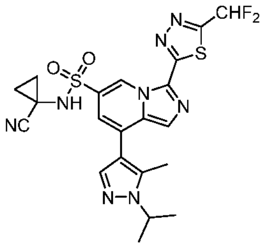 | ++++ | NT | NT |
| 144 | 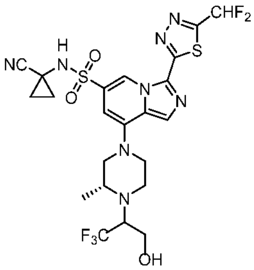 | +++ | NT | ++ |
| 145 | 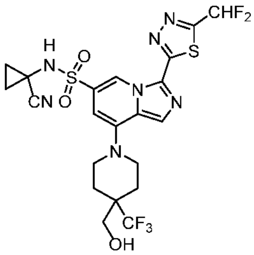 | +++ | NT | +++ |
| 146 | 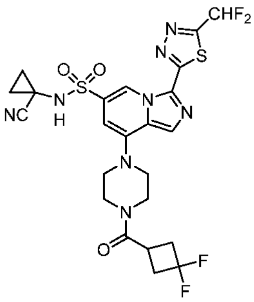 | ++++ | ++++ | +++ |
| 147 | 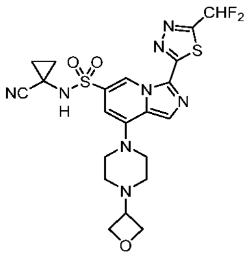 | ++++ | NT | ++ |
| 148 | 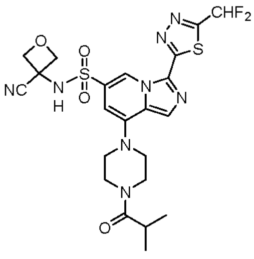 | ++++ | + | ++ |

(continued)

| Example | Compound structure | $IC_{50}$ (nM) in biochemical assay | $IC_{50}$ (nM) in cell function assay | $IC_{50}$ (nM) in cell viability assay |
|---|---|---|---|---|
| 149 | 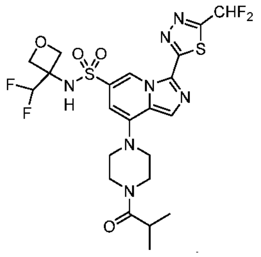 | ++++ | ++++ | +++ |
| 150 | 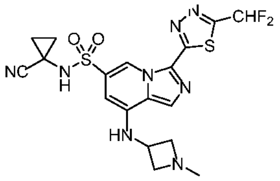 | ++++ | NT | NT |
| 151 | 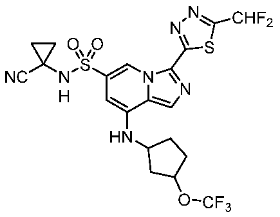 | +++ | NT | NT |
| 152 | 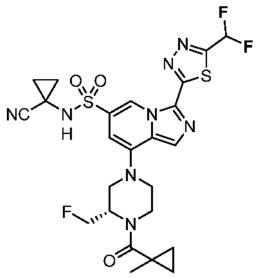 | ++++ | ++++ | +++ |
| 153 | 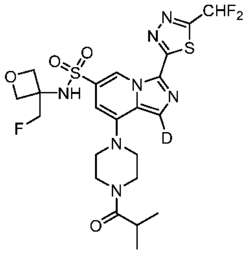 | ++++ | ++++ | +++ |
| 154 | 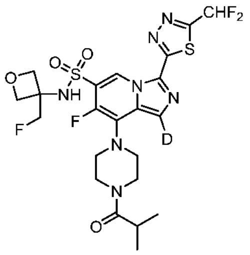 | ++++ | ++++ | +++ |

(continued)

| Example | Compound structure | $IC_{50}$ (nM) in biochemical assay | $IC_{50}$ (nM) in cell function assay | $IC_{50}$ (nM) in cell viability assay |
|---|---|---|---|---|
| 155 | 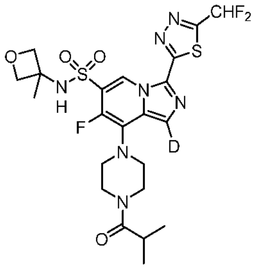 | ++++ | +++ | +++ |
| 156 | 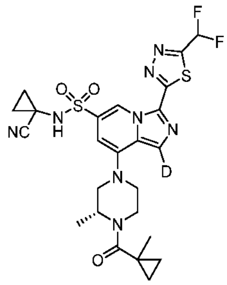 | ++++ | ++++ | ++++ |
| 159 | 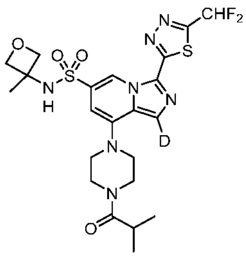 | ++++ | ++++ | ++++ |
| 161 | 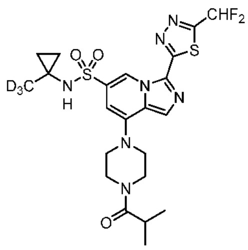 | ++++ | ++++ | ++++ |
| 162 | 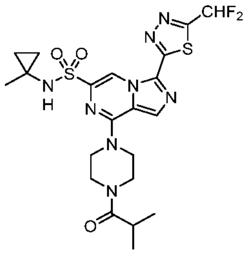 | ++ | NT | ++ |
| 163 | 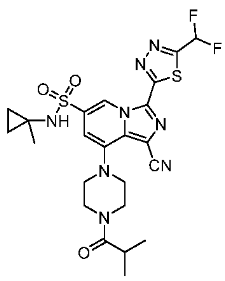 | ++++ | ++++ | ++++ |

(continued)

| Example | Compound structure | $IC_{50}$ (nM) in biochemical assay | $IC_{50}$ (nM) in cell function assay | $IC_{50}$ (nM) in cell viability assay |
|---|---|---|---|---|
| 167 | 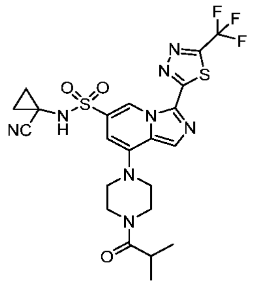 | ++++ | ++++ | ++++ |
| 366 | 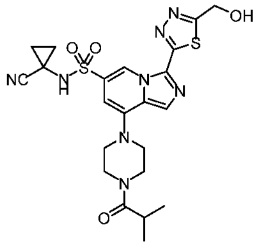 | ++++ | ++++ | ++ |
| 367 | 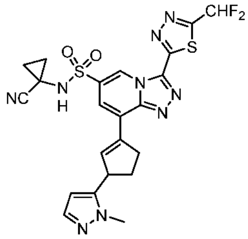 | ++++ | NT | ++ |
| 378 | 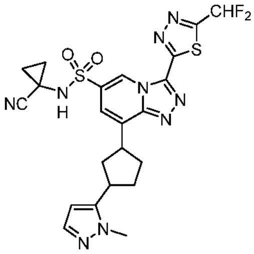 | ++++ | NT | ++ |

| Example | $IC_{50}$ (nM) in biochemical assay | $IC_{50}$ (nM) in cell function assay | $IC_{50}$ (nM) in cell viability assay |
|---|---|---|---|
| 1 | 1535 | NT | NT |
| 2 | 2100 | NT | NT |
| 3 | 33.1 | NT | NT |
| 4 | NT | NT | NT |
| 5 | NT | NT | NT |
| 6 | NT | NT | NT |
| 7 | NT | NT | NT |
| 8 | 9685 | NT | NT |
| 9 | NT | NT | NT |
| 10 | NT | NT | NT |
| 11 | 1.99 | 22.4 | 456 |

(continued)

| Example | $IC_{50}$ (nM) in biochemical assay | $IC_{50}$ (nM) in cell function assay | $IC_{50}$ (nM) in cell viability assay |
|---|---|---|---|
| 12 | 2.4 | 9.15 | 500 |
| 13 | 10.9 | NT | NT |
| 14 | 0.76 | 2.01 | 64 |
| 15 | NT | NT | NT |
| 16 | 29.7 | NT | NT |
| 17 | NT | NT | NT |
| 18 | 40 | NT | NT |
| 19 | 7244 | NT | NT |
| 20 | 178 | NT | NT |
| 21 | 912 | NT | NT |
| 22 | 5.17 | NT | 30000 |
| 23 | NT | NT | NT |
| 24 | 2.46 | 52.6 | 500 |
| 25 | 4.84 | NT | 490 |
| 26 | 5.37 | 4693 | NT |
| 27 | 4.98 | 76.3 | 3526 |
| 28 | 33 | NT | NT |
| 29 | 94.6 | NT | NT |
| 30 | 88.9 | NT | NT |
| 31 | 0.84 | 4.7 | 101 |
| 32 | 0.77 | 10.8 | 222 |
| 33 | 6.52 | 155 | NT |
| 34 | 1.22 | 5.77 | 77 |
| 35 | 128 | NT | NT |
| 36 | 649 | NT | NT |
| 37 | 4.7 | 166 | 2666 |
| 38 | 16.4 | NT | NT |
| 39 | 2.72 | 13.2 | 220 |
| 40 | 8.37 | 309 | 1408 |
| 41 | 29.8 | NT | NT |
| 42 | 18 | NT | NT |
| 43 | 0.96 | 9.32 | 283 |
| 44 | 10.7 | NT | 14000 |
| 45 | 135 | NT | NT |
| 46 | 6.31 | NT | NT |
| 47 | 5.91 | NT | NT |
| 48 | 13.2 | NT | NT |
| 49 | 38.4 | NT | 11932 |
| 50 | NT | NT | NT |

(continued)

| Example | $IC_{50}$ (nM) in biochemical assay | $IC_{50}$ (nM) in cell function assay | $IC_{50}$ (nM) in cell viability assay |
|---|---|---|---|
| 51 | NT | NT | NT |
| 52 | 3.66 | 53.2 | 738 |
| 53 | 2.37 | 145 | 1376 |
| 54 | 11.4 | 26.5 | 9382 |
| 55 | 3.92 | 324 | 9890 |
| 56 | 0.86 | 22.9 | 802 |
| 57 | 21.2 | 1106 | NT |
| 58 | 25.1 | 2872 | NT |
| 59 | NT | NT | NT |
| 60 | 0.19 | 2.85 | 438 |
| 61 | 121 | NT | NT |
| 62 | 3.75 | 3.52 | 242 |
| 63 | 2.12 | 1.356 | 48 |
| 64 | 3.44 | 6.92 | 220 |
| 65 | 7.02 | 122 | 6000 |
| 66 | 2.89 | 18.2 | 1002 |
| 67 | 8.83 | 40.1 | 1931 |
| 68 | 25.1 | 34.7 | 1664 |
| 69 | 20.0 | 9.05 | 432 |
| 70 | 32.9 | NT | 2068 |
| 71 | 1.32 | 0.67 | 55 |
| 72 | 17.8 | NT | 805 |
| 73 | 41.7 | NT | 2046 |
| 74 | 6.61 | 1.50 | 200 |
| 75 | 13.2 | 3.78 | 370 |
| 76 | 13.8 | 3.59 | 450 |
| 77 | 24.0 | 14.8 | 1587 |
| 78 | 7.59 | 7.34 | 866 |
| 79 | 52.5 | NT | 3090 |
| 80 | 28.8 | 7.57 | 764 |
| 81 | NT | NT | 10735 |
| 82 | 209 | NT | 7049 |
| 83 | 347 | NT | 5016 |
| 84 | 11.0 | 6.85 | 472 |
| 85 | 15.2 | 13.7 | 1051 |
| 86 | 16.6 | 11.4 | 1331 |
| 87 | 7.24 | 49.9 | 2458 |
| 88 | 8.69 | 6.77 | 1525 |
| 89 | 115 | NT | 2436 |

(continued)

| Example | $IC_{50}$ (nM) in biochemical assay | $IC_{50}$ (nM) in cell function assay | $IC_{50}$ (nM) in cell viability assay |
|---|---|---|---|
| 90 | 34.7 | NT | 1526 |
| 91 | 31.6 | NT | 264 |
| 92 | 27.5 | NT | 974 |
| 93 | 75.9 | NT | 1759 |
| 94 | 9.55 | 23.4 | 350 |
| 95 | 100 | NT | 4514 |
| 96 | 2.09 | 1.68 | 100 |
| 97 | 5.01 | 7.52 | 135 |
| 98 | NT | NT | NT |
| 99 | 6.51 | 26.8 | 372 |
| 100 | 1.99 | 4.96 | 448 |
| 101 | 8.15 | 1543 | 20000 |
| 102 | 2.54 | 32.1 | 5000 |
| 103 | 1.50 | 33.6 | 1619 |
| 104 | 0.65 | 9.76 | 206 |
| 105 | 78.6 | NT | 10000 |
| 106 | 28.2 | NT | 5000 |
| 107 | 2.04 | 74.0 | 513 |
| 108 | 1.50 | 22.6 | 520 |
| 109 | 30.5 | NT | 7000 |
| 110 | 14.0 | 89.5 | 850 |
| 111 | 0.37 | 72.9 | 916 |
| 112 | 3.67 | 36.3 | 3540 |
| 113 | 5.20 | 9.00 | 1140 |
| 114 | 1.57 | 17.5 | 462 |
| 115 | 1.88 | 8.51 | 215 |
| 116 | 50 | NT | 2700 |
| 117 | 8.71 | 1.49 | 256 |
| 118 | 72.4 | NT | 1113 |
| 119 | 41.7 | NT | 431 |
| 120 | 1.49 | 2.28 | 218 |
| 121 | 60.1 | NT | 2588 |
| 122 | 3.16 | 4.58 | 218 |
| 123 | 9.12 | 7.13 | 584 |
| 124 | 2.54 | NT | 759 |
| 125 | 31.2 | NT | 518 |
| 126 | 5.34 | NT | 602 |
| 127 | 1.01 | NT | 95 |
| 128 | 0.99 | NT | 28 |

(continued)

| Example | $IC_{50}$ (nM) in biochemical assay | $IC_{50}$ (nM) in cell function assay | $IC_{50}$ (nM) in cell viability assay |
|---|---|---|---|
| 129 | 0.16 | NT | 12.7 |
| 130 | 36.3 | NT | 1200 |
| 131 | 22.9 | NT | 1685 |
| 132 | 60.1 | NT | 2588 |
| 133 | 13.18 | NT | NT |
| 134 | 69.18 | NT | NT |
| 135 | 12.58 | NT | 213 |
| 136 | 7.94 | NT | 199 |
| 137 | 15.84 | NT | 332 |
| 138 | 0.93 | NT | NT |
| 139 | 45.6 | NT | NT |
| 140 | 26.2 | NT | NT |
| 141 | 280 | NT | NT |
| 142 | 38.4 | NT | NT |
| 143 | 12 | NT | NT |
| 144 | 263 | NT | 7018 |
| 145 | 316 | NT | 1877 |
| 146 | 11.5 | 28.9 | 692 |
| 147 | 34.1 | NT | 9944 |
| 148 | 2.04 | 8507 | 8809 |
| 149 | 6.23 | 57.7 | 3453 |
| 150 | 8.29 | NT | NT |
| 151 | 178 | NT | NT |
| 152 | 13.2 | 2.89 | 990 |
| 153 | 5.34 | 2.99 | 352 |
| 154 | 8.22 | 9.94 | 2000 |
| 155 | 1.68 | 39.1 | 727 |
| 156 | 6.92 | 12.0 | 280 |
| 157 | 33.1 | NT | 1015 |
| 158 | 15.84 | NT | 1093 |
| 159 | 0.46 | 1.12 | 199 |
| 160 | 0.23 | NT | 23.5 |
| 161 | 0.88 | 1.02 | 77 |
| 162 | 525 | NT | 6069 |
| 163 | 1.58 | 5.14 | 87 |
| 164 | 3.31 | NT | 100 |
| 165 | 20.0 | NT | 1000 |
| 166 | 13.2 | NT | 882 |
| 167 | 16.6 | 6.34 | 290 |

(continued)

| Example | $IC_{50}$ (nM) in biochemical assay | $IC_{50}$ (nM) in cell function assay | $IC_{50}$ (nM) in cell viability assay |
|---|---|---|---|
| 168 | 0.15 | NT | 14 |
| 169 | 1.82 | NT | 27 |
| 170 | 1.66 | NT | 122 |
| 171 | 0.71 | NT | 32 |
| 172 | 10 | NT | 308 |
| 173 | 50.1 | NT | 400 |
| 174 | 5.01 | NT | 856 |
| 175 | 18.2 | NT | 565 |
| 176 | 4.37 | NT | 1340 |
| 177 | 0.54 | NT | 15 |
| 178 | 1.18 | NT | 945 |
| 179 | 0.35 | NT | 24.0 |
| 180 | 5.62 | NT | 517 |
| 181 | 1.78 | NT | 151 |
| 182 | 0.53 | NT | 128 |
| 183 | 0.91 | NT | 26.8 |
| 184 | 1.15 | NT | 18.0 |
| 185 | 4.58 | NT | 797 |
| 186 | 5.62 | NT | 485 |
| 187 | 3.37 | NT | 874 |
| 188 | 0.71 | NT | 85.0 |
| 189 | 3.55 | NT | 112 |
| 190 | 6.23 | NT | 593 |
| 191 | 32.0 | NT | 723 |
| 192 | 0.83 | NT | 30.0 |
| 193 | 0.36 | NT | 5.76 |
| 194 | 3.21 | NT | 84.8 |
| 195 | 5.62 | NT | 666 |
| 196 | <0.10 | NT | 6.34 |
| 197 | 0.19 | NT | 6.58 |
| 198 | 0.66 | NT | 27.8 |
| 199 | 0.34 | NT | 11.5 |
| 200 | 0.63 | NT | 54.6 |
| 201 | 0.14 | NT | 9.06 |
| 202 | 1.16 | NT | 133 |
| 203 | 0.91 | NT | 26.8 |
| 204 | 36.3 | NT | 360 |
| 205 | 22.9 | NT | 699 |
| 206 | 21.9 | NT | 1178 |

(continued)

| Example | $IC_{50}$ (nM) in biochemical assay | $IC_{50}$ (nM) in cell function assay | $IC_{50}$ (nM) in cell viability assay |
|---|---|---|---|
| 207 | 39.8 | NT | 1200 |
| 208 | 110 | NT | 3350 |
| 209 | 60.3 | NT | 765 |
| 210 | 83.2 | NT | 929 |
| 211 | 209 | NT | 222 |
| 212 | 110 | NT | 3000 |
| 213 | 31.6 | NT | 1200 |
| 214 | 174 | NT | 4000 |
| 215 | 69.2 | NT | 1500 |
| 216 | 31.6 | NT | 2338 |
| 217 | 66.1 | NT | 3069 |
| 218 | 39.8 | NT | 1046 |
| 219 | 100 | NT | 4613 |
| 220 | 57.5 | NT | 664 |
| 221 | 43.7 | NT | 150 |
| 222 | 2.09 | NT | 1672 |
| 223 | 1.45 | NT | 250 |
| 224 | 13.8 | NT | 1024 |
| 225 | 7.94 | NT | 600 |
| 226 | 26.3 | NT | 4854 |
| 227 | 5.01 | NT | 300 |
| 228 | 0.38 | NT | 107 |
| 229 | 3.31 | NT | 800 |
| 230 | 120 | NT | 8000 |
| 231 | 8.32 | NT | 500 |
| 232 | 31.6 | NT | 953 |
| 233 | 45.7 | NT | 3000 |
| 234 | 13.2 | NT | 4000 |
| 235 | 4.37 | NT | 4937 |
| 236 | 3.80 | NT | 1100 |
| 237 | 2.88 | NT | 4393 |
| 238 | 38.0 | NT | 15000 |
| 239 | 380 | NT | 9786 |
| 240 | 13.2 | NT | 2437 |
| 241 | 24.0 | NT | 4183 |
| 242 | 52.5 | NT | 2981 |
| 243 | 871 | NT | 200 |
| 244 | 75.9 | NT | 9000 |
| 245 | 28.8 | NT | 2362 |

(continued)

| Example | $IC_{50}$ (nM) in biochemical assay | $IC_{50}$ (nM) in cell function assay | $IC_{50}$ (nM) in cell viability assay |
|---|---|---|---|
| 246 | NT | NT | NT |
| 247 | NT | NT | 27235 |
| 248 | 692 | NT | 6441 |
| 249 | 115 | NT | 7321 |
| 250 | 38.0 | NT | 1283 |
| 251 | 6.03 | NT | 8784 |
| 252 | 16.6 | NT | 2000 |
| 253 | 57.5 | NT | 1000 |
| 254 | 11.5 | NT | 700 |
| 255 | 17.4 | NT | 420 |
| 256 | 8.71 | NT | 80.0 |
| 257 | 191 | NT | 2107 |
| 258 | 550 | NT | 2832 |
| 259 | 45.7 | NT | 1500 |
| 260 | 28.8 | NT | 250 |
| 261 | 100 | NT | 370 |
| 262 | NT | NT | 2000 |
| 263 | NT | NT | 4500 |
| 264 | 75.9 | NT | 6640 |
| 265 | 158 | NT | 8000 |
| 266 | 132 | NT | 370 |
| 267 | NT | NT | 1500 |
| 268 | 100 | NT | 2211 |
| 269 | 79.4 | NT | 19952 |
| 270 | 91.2 | NT | 35925 |
| 271 | 11.5 | NT | 36240 |
| 272 | 7.24 | NT | 5410 |
| 273 | 1.91 | NT | 5939 |
| 274 | 41.7 | NT | 700 |
| 275 | 22.9 | NT | 472 |
| 276 | 45.7 | NT | 1750 |
| 277 | 25.1 | NT | 4474 |
| 278 | 40.7 | NT | 1983 |
| 279 | 95.5 | NT | 29853 |
| 280 | 45.7 | NT | NT |
| 281 | 66.1 | NT | 229 |
| 282 | 20.0 | NT | 84.3 |
| 283 | 3.80 | NT | 539 |
| 284 | 16.6 | NT | 310 |

(continued)

| Example | $IC_{50}$ (nM) in biochemical assay | $IC_{50}$ (nM) in cell function assay | $IC_{50}$ (nM) in cell viability assay |
|---|---|---|---|
| 285 | 20.0 | NT | 246 |
| 286 | 3.98 | NT | 235 |
| 287 | 4.37 | NT | 137 |
| 288 | 6.03 | NT | NT |
| 289 | 1.51 | NT | 6148 |
| 290 | 14.5 | NT | 151 |
| 291 | 9.55 | NT | 65 |
| 292 | 4.57 | NT | 40 |
| 293 | 9.12 | NT | 97 |
| 294 | 12.0 | NT | 110 |
| 295 | 39.8 | NT | 2818 |
| 296 | 3.02 | NT | 38.7 |
| 297 | 55.0 | NT | 362 |
| 298 | 19.1 | NT | 175 |
| 299 | 24.0 | NT | 1032 |
| 300 | 31.6 | NT | 403 |
| 301 | 50.1 | NT | 436 |
| 302 | 15.4 | NT | 329 |
| 303 | 20.9 | NT | 435 |
| 304 | 3.86 | NT | 70.0 |
| 305 | 6.91 | NT | 130 |
| 306 | 6.11 | NT | 89 |
| 307 | 4.13 | NT | 540 |
| 308 | 0.73 | NT | 104 |
| 309 | 1.03 | NT | 570 |
| 310 | <0.10 | NT | 38.0 |
| 311 | 0.21 | NT | 234 |
| 312 | <0.10 | NT | 6.02 |
| 313 | 1.64 | NT | 2050 |
| 314 | 0.68 | NT | 241 |
| 315 | 0.96 | NT | 5700 |
| 316 | 4.46 | NT | 918 |
| 317 | 3.54 | NT | 1140 |
| 318 | 1.84 | NT | 363 |
| 319 | 5.62 | NT | 147 |
| 320 | 2.71 | NT | 93.9 |
| 321 | 2.93 | NT | 175 |
| 322 | 16.5 | NT | 334 |
| 323 | 5.41 | NT | 181 |

(continued)

| Example | $IC_{50}$ (nM) in biochemical assay | $IC_{50}$ (nM) in cell function assay | $IC_{50}$ (nM) in cell viability assay |
|---|---|---|---|
| 324 | 4.29 | NT | 205 |
| 325 | 4.46 | NT | 120 |
| 326 | 4.82 | NT | 257 |
| 327 | 0.15 | NT | 6.13 |
| 328 | 1.21 | NT | 55.6 |
| 329 | 7.64 | NT | 187 |
| 330 | 6.55 | NT | 194 |
| 331 | 0.44 | NT | 78.1 |
| 332 | 10.8 | NT | 745 |
| 333 | 3.83 | NT | 310 |
| 334 | 6.31 | NT | 354 |
| 335 | 6.55 | NT | 480 |
| 336 | 54.1 | NT | 1078 |
| 337 | 4.82 | NT | 245 |
| 338 | 0.48 | NT | 50.0 |
| 339 | 3.16 | NT | 226 |
| 340 | 2.15 | NT | 1585 |
| 341 | 2.42 | NT | 139 |
| 342 | 3.04 | NT | 297 |
| 343 | 5.41 | NT | 852 |
| 344 | 38.3 | NT | 586 |
| 345 | 5.01 | NT | 374 |
| 346 | 1.53 | NT | 109 |
| 347 | 4.64 | NT | 282 |
| 348 | 1.99 | NT | 257 |
| 349 | 3.98 | NT | 455 |
| 350 | 20.0 | NT | 713 |
| 351 | 4.13 | NT | 163 |
| 352 | 15.3 | NT | 561 |
| 353 | 12.6 | NT | 434 |
| 354 | 0.27 | NT | 65.8 |
| 355 | 0.50 | NT | 85.0 |
| 356 | 6.31 | NT | 304.7 |
| 357 | 8.31 | NT | 208 |
| 358 | 15.84 | NT | 261 |
| 359 | 1.44 | NT | 47 |
| 360 | 36.31 | NT | 637 |
| 361 | 18.19 | NT | 642 |
| 362 | 6.31 | NT | 158 |

(continued)

| Example | $IC_{50}$ (nM) in biochemical assay | $IC_{50}$ (nM) in cell function assay | $IC_{50}$ (nM) in cell viability assay |
|---|---|---|---|
| 363 | 1.71 | NT | 63.0 |
| 364 | 41.4 | NT | 791 |
| 365 | 8.58 | NT | 1071 |
| 366 | 7.94 | 70.4 | 7728 |
| 367 | 32.8 | NT | 6777 |
| 368 | 13.8 | NT | 900 |
| 369 | 1.91 | NT | 505 |
| 370 | 41.7 | NT | 240 |
| 371 | 4.57 | NT | 360 |
| 372 | 3.80 | NT | 580 |
| 373 | 3.31 | NT | 21.1 |
| 374 | NT | NT | 2318 |
| 375 | NT | NT | 855 |
| 376 | NT | NT | 30000 |
| 377 | 1.41 | NT | 60.0 |
| 378 | 27.7 | NT | 6000 |
| 379 | 2.09 | NT | 20.0 |
| 380 | 3.31 | NT | 21.1 |
| 381 | 27.1 | NT | 3630 |
| 382 | 6.55 | NT | 828 |
| 383 | 0.25 | NT | 14.8 |
| 384 | <0.15 | NT | 17.6 |
| 385 | 0.48 | NT | 40.0 |
| 386 | 263 | NT | 7881 |
| 387 | 38.0 | NT | 800 |
| 388 | 10.00 | NT | 320 |
| 389 | 20.0 | NT | 2132 |
| 390 | 28.2 | NT | 793 |
| 391 | 5.01 | NT | 177.3 |
| 392 | 10.96 | NT | 132 |
| 393 | 5.75 | NT | 222 |
| 394 | 6.56 | NT | 1700 |
| 395 | NT | NT | NT |
| 396 | 2.08 | NT | 36.8 |
| 397 | 13.18 | NT | 530 |
| 398 | 27.54 | NT | 992 |
| 399 | 12.6 | NT | 755 |
| 400 | 6.31 | NT | 267 |
| 401 | 158 | NT | 2804 |

(continued)

| Example | $IC_{50}$ (nM) in biochemical assay | $IC_{50}$ (nM) in cell function assay | $IC_{50}$ (nM) in cell viability assay |
|---|---|---|---|
| 402 | 20.89 | NT | 1840 |
| 403 | 21.87 | NT | 458 |
| 404 | 1.09 | NT | 27.0 |

**4. Solubility Assay**

1). Preparation of solutions:

The FaSSGF solution was prepared as follows:

a) preparation of FaSSGF buffer: dissolve 2.0 g of NaCl as the raw material in about 0.9 L of purified water;
b) adjustment of the pH of the buffer: adjust the pH of the buffer to 1.6 with 1N HCL, and replenish the buffer with purified water to the volume of 1L; and
c) preparation of FaSSGF solution by addition of FaSSGF powder: add 0.06g of the FaSSGF powder to about 0.5 L of the buffer, stir until the powder was completely dissolved, and make up for the volume (1L) with the buffer.

The FaSSIF solution was prepared as follows:

a) preparation of FaSSIF buffer: dissolve 0.42 g of NaOH, 3.44 g of anhydrous NaH2PO4, and 6.19 g of NaCl as raw materials in about 0.9 L of purified water;
b) adjustment the pH of the buffer: adjust the pH of the buffer to 6.5 with 1 N NaOH or 1 N HCL, and replenish the buffer with purified water to the volume of 1 L; and
c) preparation of FaSSIF solution by addition of FaSSIF powder: add 2.24g of the FaSSIF powder to about 0.5 L of the buffer, stir until the powder was completely dissolved, and make up for the volume (1L) with the buffer.

2). Preparation of the solution of control compound:

The control compound was weighed and dissolved in DMSO to 10 mM to obtain a stock solution.

3) Preparation of the solution of compound to be tested:

The compound to be tested was formulated intoa 10 mM DMSO stock solution.

4) Preparation of internal standard solution:

a) The internal standard was weighed and dissolved in DMSO to 10 mM to obtain an internal standard stock solution (ISTD stock solution).
b) The internal standard stock solution was diluted to 500 nM (Tolbutamide) with an ACN:methano mixture (volume ratio: 1:1) to obtain a quenching solution.

5) Testing

a) The FaSSGF solution at pH 1.6 and the FaSSIF solution at pH 6.5 were pre-warmed; and then, the compound was injected into the buffer.
b) 8 μL of the stock solution (10 mM) of the compound to be tested/control compound was pipetted to 792 μL of the FaSSGF solution at pH 1.6, to achieve the concentration of 100 μM (as test sample for FaSSGF solubility assay).
c) 8 μL of the stock solution (10 mM) of the compound to be tested SY002-410-01/control compound was pipetted to 792 μL of the FaSSIF solution at pH 6.5 to achieve the concentration of 100 μM (as test sample for FaSSIF solubility assay).
d) Then, the samples for the solubility assay were transferred to a shaker and shaken for 2 h at 25°C and 1000 rpm.

Once done, the samples were centrifuged at 12,000 rpm for 10 min, and the supernatant was transferred to a new tube. The supernatant was diluted 10-fold and 100-fold with the FaSSGF solution at pH 1.6 or the FaSSIF solution at pH 6.5. 20 μL of each sample (1x, 10x, 100x and standard curve) was added to 500 μL of the IS solution. The resulting mixture was vortexed vigorously for 1 min and centrifuged at 4,500 rpm for 15 min at 4°C. 150 μL of the supernatant was transferred for LC-MS/MS sample analysis.

Information of control compound:

| Compound name | Manufacturer | Lot number |
|---|---|---|
| Ketoconazole | Sigma | SCCC4160 |
| Propranolol | Sigma | BCCB3791 |

Information of reagent:

| Reagent name | Manufacturer | Lot number | Storage conditions |
|---|---|---|---|
| FaSSIF, FeSSIF & FaSSGF powder | biorelevant | FFF-0722-A | 2-8°C |
| Acetonitrile | Thermo Fisher Scientific | A998-4 | RT |
| Methanol | Thermo Fisher Scientific | FCM#A452-4 | RT |

**Experimental results:**

| **Example** | FaSSGF solubility (μM) | FaSSIF solubility (μM) |
|---|---|---|
| **32** | **0.90** | **3.18** |
| **167** | **57.5** | **96.5** |
| **159** | **5.19** | **17.4** |
| **60** | **3.83** | **10.0** |
| **107** | **15.9** | **24.2** |
| **120** | **81.8** | **140** |
| **124** | **38.5** | **88.2** |
| **175** | **21.5** | **97.2** |
| **227** | **73.8** | **82.4** |
| **236** | **75.7** | **82.6** |
| **251** | **80.6** | **78.1** |
| **298** | **>100** | **97.7** |

**5. PXR Assay**

1) Information of cell line

DPX2 (HepG2 cells stably transfected with PXR and luciferase reporters) cells were purchased from Puracyp Inc (Carlsbad, CA).

2) Experimental procedures

a) Reagents and consumables

| Item | Supplier | Catalog or model number |
|---|---|---|
| 384-well plate | Corning | 354660 |
| Growth medium | Puracyp | C-500-100 |
| Treatment medium | Puracyp | D-500-100 |
| Fetal bovine serum | Acantor | 76294-180 |
| CellTiter-Fluor™ Cell Viability Assay Kit | Promega | G6081 |
| One-Glo Fluorescence Detection Kit | Promega | E6120 |
| Centrifuge | Eppendorf | 5810R |
| Carbon dioxide incubator | Thermo Scientific | 371 |
| Cell counter | Invitrogen | Countess II |
| Microplate reader | Tecan | Infinite M200 |
| TECAN | Tecan | 30020020 |
| Echo | Beckman | Echo, 655T |
| Shake mixer | IKA | MS 3 |

b) Cell seeding

The DPX2 cells were cultured in the growth medium containing 10% fetal bovine serum. The DPX2 cells in a T-75 flask were cultured in the 5% $CO_2$incubator at 37°C with 95% relative humidity, and digested when the cells grew to cover 80-90% of the bottom of the flask. The surface layer of the T-75 cultured cells was washed with 10 mL of PBS; PBS was pipetted; 3-5 mL of trypsin was added; the cells were digested at 37°C for 5 minutes or until the cells were digested to be suspended; and excessive medium containing fetal bovine serum was added to stop trypsinization. The cell suspension was transferred to a conical-bottomed centrifuge tube and centrifuged at 150 g rpm for 5 minutes at room temperature; the supernatant was carefully pipetted; and the cells were resuspended with the treatment medium, with the concentration adjusted to 3.2×105 cells/mL (incubation time: 24 hours, plate seeding density: 4.0×105 cells/mL). 25 μL of the cell suspension was added to each well of the 384-well cell culture plate. The cell culture plate was placed and cultured for 24 h in the 5% $CO_2$ incubator with 95% humidity at 37°C.

c) Formulation of compound

The stock solution of each of 1000× test compound, the positive control (rifampicin), and the negative control (propranolol) was prepared with DMSO. The final concentrations of the positive control (rifampicin) were 1 μM and 10 μM, and the final concentration of the negative control (propranolol) was 10 μM. The final concentrations of the test compound were 10 μM, 1 μM, 0.1 μM, or EC50 (30 μM, 10 μM, 3 μM, 1 μM, 0.3 μM, 0.1 μM). The final concentration of DMSO was 0.1%. The cell culture plate was taken out from the incubator, and 25 nL of the stock solution of the positive control, negative control or test compound was directly added with Echo, each with three parallel concentrations. The cell culture plate was placed in the incubator and continued to be incubated for 24 h. Before starting the assay with the substrate, the cell morphology and the monolayer integrity were checked to ensure that the monolayer was of acceptable research quality.

d) Quantitative detection with PXR activation

After 24 hours of dosing, the culture could be quantitatively detected with PXR activation. The CellTiter-Fluor™ Cell Viability Assay Kit and One-Glo Luciferase reagent were equilibrated to room temperature. The GF-AFC substrate (10 μL) was added to the assay buffer (10 ml) to develop a 2X reagent, which was then diluted into a 1X reagent with 10 ml of PBS. The ONE-Glo Luciferase substrate was added to the ONE-Glo Luciferase assay buffer. The culture plate was taken out from the incubator; the medium was discarded; 1X of CellTiter-Fluor™ reagent was poured into the loading slot; 25 μL

of reagent were added per well to the culture plate with a pipetting workstation; and the culture plate was then incubated in the incubator for 30 minutes. The cell culture plate was taken out from the incubator, and cooled slightly to room temperature; and the fluorescence value was determined with a fully automatic quantitative microplate reader, with the excitation light of 400 nm and the emission light of 505 nm. The ONE-Glo reagent was poured into the loading slot, with 25 μL per well, and the resulting mixture was gently mixed in the plate, and incubated at room temperature for 5 minutes to determine the luminescence value.

**Experimental results:**

| Example | PXR Activation @30uM (Fold) | PXR Activation @30uM (% Ctrl) |
|---|---|---|
| 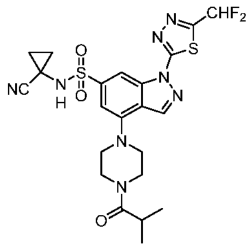 | 6.4 | 44 |
| 32 | 2.7 | 19 |
| 167 | 5.0 | 34 |
| 236 | 1.5 | 7 |
| 251 | 1.5 | 11 |
| 254 | 1.6 | 12 |
| 193 | 2.4 | 15 |
| 284 | 1.1 | 7 |
| 285 | 1.7 | 10 |
| 287 | 1.6 | 15 |
| 311 | 1.1 | 5 |
| 324 | 1.8 | 14 |
| 330 | 1.3 | 9 |
| 336 | 0.3 | 2 |
| 179 | 3.2 | 22 |
| 190 | 2.2 | 12 |

**6. Mouse PK Assay**

The goal of this assay was to study the pharmacokinetics of the test compound on CD1 mice (6-8 weeks old, Zhejiang Vegavit Bio-technology Co.,Ltd.) after oral administration. The specific experimental procedures were as follows:

1) the compound was dissolved in 8% DMSO + 72% PEG 400 + 20% (10%TPGS in purified water) to arrive at the concentration of 0.5 mg/mL, and then administered orally (5 mL/Kg); and
2) blood samples were collected, with 0.03 mL collected at 0.25 h, 0.5 h, 1 h, 2 h, 4 h, 8 h, and 24 h, and EDTA-K2 was added. CD1 mice: Approximately 0.03 mL of blood was collected at each time point. The blood was centrifuged for 5 min at 4000 g in a centrifuge at 4°C to collect plasma. Plasma samples were aliquoted into clean polyethylene microcentrifuge tubes and then stored in a freezer at -75 ± 15°C before analysis; and
3) samples were analyzed, and the concentration of the compound in each plasma sample was analyzed with LC-MS/MS. For plasma measurements, pharmacokinetic parameters were calculated using WinNonlin (PhoenixTM, version 8.3) or other similar software.

**Experimental results:**

| Example | 32 | 167 |
|---|---|---|
| Administration dose (mg/kg) | 30 | 30 |
| Half-life period (hr) | 1.31 | 2.75 |
| Mean residence time (hr) | 1.92 | 4.81 |
| Time to peak (hr) | 0.333 | 1.67 |
| Peak concentration (ng/mL) | 5317 | 4087 |
| Area under the curve (0-last; hr*ng/mL) | 13174 | 29273 |
| Bioavailability (%) | 47 | 85 |

## Claims

1. A compound of formula I, a pharmaceutically acceptable salt thereof or an isotopic compound thereof, wherein

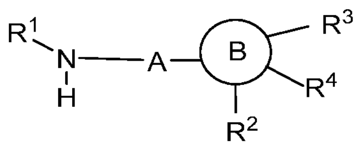

I

A is

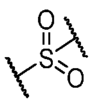, 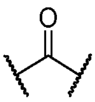 or 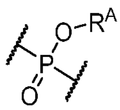;

$R^A$ is hydrogen or $C_1$-$C_6$ alkyl;

ring B is a 5-membered-fused 6-membered heteroaromatic ring unsubstituted or substituted by one or two $R^B$ or a 5-membered-fused 5-membered heteroaromatic ring unsubstituted or substituted by one or two $R^B$; the heteroatom in said 5-membered-fused 6-membered heteroaromatic ring is one or more selected from N, O and S, and the number of heteroatom(s) is 1, 2 or 3; the heteroatom in said 5-membered-fused 5-membered heteroaromatic ring is one or more selected from N, O and S, and the number of heteroatom(s) is 1, 2 or 3; $R^B$ is each independently selected from oxo, hydroxyl, halogen or $C_1$-$C_6$ alkyl, or two $R^B$, together with the atoms to which they are attached, form a 3-6 membered cycloalkyl;

$R^1$ is hydroxyl, $C_1$-$C_6$ alkyl unsubstituted or substituted by one or more $R^{1-1}$, $C_3$-$C_6$ cycloalkyl unsubstituted or substituted by one or more $R^{1-2}$, 3-6 membered heterocycloalkyl unsubstituted or substituted by one or more $R^{1-3}$,

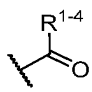

or amino unsubstituted or substituted by one or two $R^{1-5}$; the heteroatom in said 3-6 membered heterocycloalkyl is one or more selected from N, O and S, and the number of heteroatom(s) is 1, 2 or 3;

$R^{1-1}$ is each independently selected from deuterium, hydroxyl, cyano, halogen, 3-6 membered heterocycloalkyl, $C_3$-$C_6$ cycloalkyl unsubstituted or substituted by one or more $R^{1-1-1}$, $C_1$-$C_6$ alkyl unsubstituted or substituted by one or more $R^{1-1-2}$ or $C_1$-$C_6$ alkoxy unsubstituted or substituted by one or more $R^{1-1-3}$; the heteroatom in said 3-6 membered heterocycloalkyl is one or more selected from N, O and S, and the number of heteroatom(s) is 1, 2 or 3;

$R^{1-1-1}$ is each independently selected from deuterium, hydroxyl, cyano or halogen; $R^{1-1-2}$ is each independently selected from deuterium, hydroxyl, cyano or halogen; $R^{1-1-3}$ is each independently selected from deuterium, hydroxyl, cyano or halogen;

$R^{1-2}$ is each independently selected from deuterium, hydroxyl, cyano, halogen, $C_1$-$C_6$ alkyl unsubstituted or substituted by one or more $R^{1-2-1}$, $C_2$-$C_6$ alkynyl unsubstituted or substituted by one or more $R^{1-2-2}$, $C_1$-$C_6$ alkoxy unsubstituted or substituted by one or more $R^{1-2-3}$, $C_2$-$C_6$ alkenyl unsubstituted or substituted by one or more $R^{1-2-4}$, amino unsubstituted or substituted by one or two $R^{1-2-5}$ or

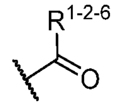;

$R^{1-2-1}$ is each independently selected from deuterium, halogen, hydroxyl or $C_1$-$C_6$ alkoxy unsubstituted or substituted by one or more $R^{1-2-1-1}$; $R^{1-2-1-1}$ is each independently halogen; $R^{1-2-2}$ is each independently selected from halogen, hydroxyl or $C_1$-$C_6$ alkoxy unsubstituted or substituted by one or more $R^{1-2-2-1}$; $R^{1-2-2-1}$ is each independently halogen; $R^{1-2-1}$ is each independently selected from halogen, hydroxyl or $C_1$-$C_6$ alkoxy unsubstituted or substituted by one or more $R^{1-2-3-1}$; $R^{1-2-3-1}$ is each independently halogen; $R^{1-2-4}$ is each independently selected from halogen, hydroxyl or $C_1$-$C_6$ alkoxy unsubstituted or substituted by one or more $R^{1-2-4-1}$; $R^{1-2-4-1}$ is each independently halogen; $R^{1-2-6}$ is each independently amino; $R^{1-2-5}$ is each independently

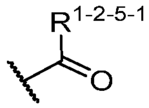;

$R^{1-2-5-1}$ is each independently $C_1$-$C_6$ alkyl;

$R^{1-3}$ is each independently selected from cyano, deuterium, hydroxyl, amino, halogen, $C_1$-$C_6$ alkyl unsubstituted or substituted by one or more $R^{1-3-1}$, $C_1$-$C_6$ alkoxy unsubstituted or substituted by one or more $R^{1-3-2}$ or $C_2$-$C_6$ alkynyl; $R^{1-3-1}$ is each independently halogen; $R^{1-3-2}$ is each independently halogen;

$R^{1-4}$ is $C_1$-$C_6$ alkyl;

$R^{1-5}$ is each independently selected from $C_1$-$C_6$ alkyl,

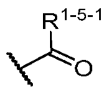

or $C_3$-$C_6$ cycloalkyl;

$R^{1-5-1}$ is $C_1$-$C_6$ alkyl or amino;

or, a 5-10 membered heterocycloalkyl unsubstituted or substituted by one or more $R^0$ is formed when the atom in $R^1$ is attached to the atom in A; the heteroatom in said 5-10 membered heterocycloalkyl is one or more selected from N, O, P and S, and the number of heteroatom(s) is 3, 4 or 5; $R^0$ is each independently selected from $C_1$-$C_6$ alkyl or oxo;

$R^2$ is hydrogen, 6-10 membered aryl unsubstituted or substituted by one or more $R^{2-1}$, 5-10 membered heteroaryl unsubstituted or substituted by one or more $R^{2-2}$, 4-12 membered heterocycloalkyl unsubstituted or substituted by one or more $R^{2-3}$,

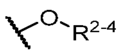, 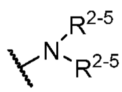,

$C_1$-$C_6$ alkyl unsubstituted or substituted by one or more $R^{2-6}$,

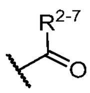, 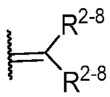,

$C_3$-$C_6$ cycloalkyl unsubstituted or substituted by one or more $R^{2-9}$, $C_3$-$C_6$ cycloalkenyl unsubstituted or sub-

stituted by one or more $R^{2-10}$, or

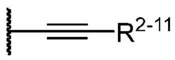;

the heteroatom in said 5-10 membered heteroaryl is one or more selected from N, O and S, and the number of heteroatom(s) is 1, 2 or 3; the heteroatom in said 4-12 membered heterocycloalkyl is one or more selected from N, O and S, and the number of heteroatom(s) is 1, 2 or 3;
$R^{2-1}$ is each independently selected from cyano, halogen, $C_1$-$C_6$ alkyl unsubstituted or substituted by one or more $R^{2-1-1}$,

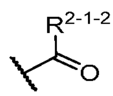, 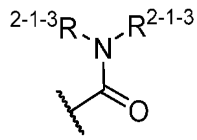

or $C_1$-$C_6$ alkoxy unsubstituted or substituted by one or more $R^{2-1-4}$;
$R^{2-1-1}$ is each independently selected from cyano or halogen; $R^{2-1-2}$ is hydrogen or $C_1$-$C_6$ alkyl; $R^{2-1-3}$ is each independently selected from hydrogen or $C_1$-$C_6$ alkyl; $R^{2-1-4}$ is each independently selected from halogen;
$R^{2-2}$ is each independently selected from cyano, halogen, $C_1$-$C_6$ alkyl unsubstituted or substituted by one or more $R^{2-2-1}$,

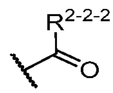, 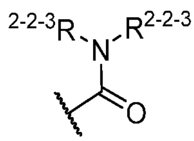,

$C_1$-$C_6$ alkoxy or

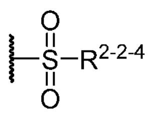;

$R^{2-2-1}$ is each independently selected from cyano or halogen; $R^{2-2-2}$ is hydrogen or $C_1$-$C_6$ alkyl; $R^{2-2-3}$ is each independently selected from hydrogen or $C_1$-$C_6$ alkyl; $R^{2-2-4}$ is each independently selected from $C_1$-$C_6$ alkyl or $C_3$-$C_6$ cycloalkyl;
$R^{2-3}$ is each independently selected from cyano, oxo, hydroxyl, halogen, $C_1$-$C_6$ alkyl unsubstituted or substituted by one or more $R^{2-3-1}$,

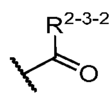, 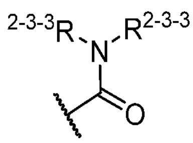,

3-6 membered heterocycloalkyl unsubstituted or substituted by one or more $R^{2-3-4}$, $C_1$-$C_6$ alkoxy unsubstituted or substituted by one or more $R^{2-3-5}$, $C_3$-$C_6$ cycloalkyl unsubstituted or substituted by one or more $R^{2-3-6}$, amino unsubstituted or substituted by one or more $R^{2-3-7}$,

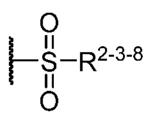,

or 5-10 membered heteroaryl unsubstituted or substituted by one or more $R^{2-3-9}$; the heteroatom in said 3-6 membered heterocycloalkyl is one or more selected from N, O and S, and the number of heteroatom(s) is 1, 2 or 3;

the heteroatom in said 5-10 membered heteroaryl is one or more selected from N, O and S, and the number of heteroatom(s) is 1, 2 or 3;
$R^{2-3-1}$ is each independently selected from hydroxyl, cyano, halogen, $C_1$-$C_6$ alkoxy unsubstituted or substituted by one or more $R^{2-3-1-1}$, $C_3$-$C_6$ cycloalkyl unsubstituted or substituted by one or more $R^{2-3-1-2}$, or amino unsubstituted or substituted by one or more $R^{2-3-1-3}$;
$R^{2-3-2}$ is hydrogen, $C_1$-$C_6$ alkyl unsubstituted or substituted by one or more $R^{2-3-2-1}$, $C_3$-$C_6$ cycloalkyl unsubstituted or substituted by one or more $R^{2-3-2-2}$,

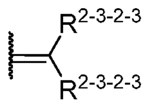
,

$C_1$-$C_6$ alkoxy unsubstituted or substituted by one or more $R^{2-3-2-4}$, 3-6 membered heterocycloalkyl unsubstituted or substituted by one or more $R^{2-3-2-5}$, or 5-10 membered heteroaryl unsubstituted or substituted by one or more $R^{2-3-2-6}$; the heteroatom in said 3-6 membered heterocycloalkyl is one or more selected from N, O and S, and the number of heteroatom(s) is 1, 2 or 3; the heteroatom in said 5-10 membered heteroaryl is one or more selected from N, O and S, and the number of heteroatom(s) is 1, 2 or 3;
$R^{2-3-3}$ is each independently selected from hydrogen, or $C_1$-$C_6$ alkyl unsubstituted or substituted by one or more $R^{2-3-3-1}$;
$R^{2-3-4}$ is each independently selected from $C_1$-$C_6$ alkyl or oxo;
$R^{2-3-5}$ is each independently selected from halogen or deuterium;
$R^{2-3-6}$ is each independently $C_1$-$C_6$ alkyl;
$R^{2-3-7}$ is each independently $C_1$-$C_6$ alkyl;
$R^{2-3-8}$ is each independently selected from $C_1$-$C_6$ alkyl or $C_3$-$C_6$ cycloalkyl;
$R^{2-3-9}$ is each independently $C_1$-$C_6$ alkyl;
$R^{2-3-1-1}$ is each independently halogen;
$R^{2-3-1-2}$ is each independently $C_1$-$C_6$ alkoxy;
$R^{2-3-1-3}$ is each independently $C_1$-$C_6$ alkyl;
$R^{2-3-2-1}$ is each independently selected from halogen, carboxyl, hydroxyl, oxo, $C_1$-$C_6$ alkoxy,

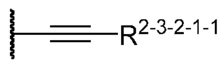
,

amino unsubstituted or substituted by one or more $R^{2-3-2-1-2}$, or 5-10 membered heteroaryl unsubstituted or substituted by one or more $R^{2-3-2-1-3}$; the heteroatom in said 5-10 membered heteroaryl is one or more selected from N, O and S, and the number of heteroatom(s) is 1, 2 or 3;
$R^{2-3-2-1-1}$ is each independently selected from $C_1$-$C_6$ alkyl or 6-10 membered aryl;
$R^{2-3-2-1-2}$ is each independently $C_1$-$C_6$ alkyl;
$R^{2-3-2-1-3}$ is each independently $C_1$-$C_6$ alkyl;
$R^{2-3-2-2}$ is each independently selected from halogen, hydroxyl, $C_1$-$C_6$ alkyl unsubstituted or substituted by one or more $R^{2-3-2-2-1}$, $C_1$-$C_6$ alkoxy unsubstituted or substituted by one or more $R^{2-3-2-2-2}$, or amino unsubstituted or substituted by one or more $R^{2-3-2-2-3}$;
$R^{2-3-2-2-1}$ is each independently selected from halogen or hydroxyl;
$R^{2-3-2-2-2}$ is each independently selected from carboxyl, halogen, deuterium, oxo or hydroxyl;
$R^{2-3-2-2-3}$ is each independently $C_1$-$C_6$ alkyl;
$R^{2-3-2-3}$ is each independently selected from hydrogen or

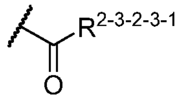
;

$R^{2-3-2-3-1}$ is each independently 6-10 membered aryl unsubstituted or substituted by one or more $R^{2-3-2-3-1-1}$;
$R^{2-3-2-3-1-1}$ is each independently $C_1$-$C_6$ alkoxy;
$R^{2-3-2-4}$ is each independently halogen;
$R^{2-3-2-5}$ is each independently selected from halogen, hydroxyl, oxo, $C_1$-$C_6$ alkoxy, or $C_1$-$C_6$ alkyl unsubstituted or substituted by one or more $R^{2-3-2-5-1}$; $R^{2-3-2-5-1}$ is each independently halogen;

$R^{2-3-2-6}$ is each independently $C_1$-$C_6$ alkyl;

$R^{2-3-3-1}$ is each independently amino unsubstituted or substituted by one or more $R^{2-3-3-1-1}$; $R^{2-3-3-1-1}$ is each independently $C_1$-$C_6$ alkyl;

each $R^{2-4}$ is 4-10 membered heterocycloalkyl unsubstituted or substituted by one or more $R^{2-4-1}$, 5-10 membered heteroaryl unsubstituted or substituted by one or more $R^{2-4-2}$, 6-10 membered aryl unsubstituted or substituted by one or more $R^{2-4-3}$ or $C_3$-$C_6$ cycloalkyl unsubstituted or substituted by one or more $R^{2-4-4}$; the heteroatom in said 4-10 membered heterocycloalkyl is one or more selected from N, O and S, and the number of heteroatom(s) is 1, 2 or 3; the heteroatom in said 5-10 membered heteroaryl is one or more selected from N, O and S, and the number of heteroatom(s) is 1, 2 or 3;

$R^{2-4-1}$ is each independently selected from hydroxyl, halogen, $C_1$-$C_6$ alkyl,

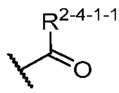 or 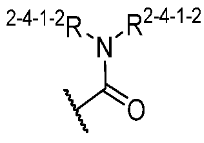 ;

$R^{2-4-2}$ is each independently selected from hydroxyl, halogen or $C_1$-$C_6$ alkyl; $R^{2-4-3}$ is each independently selected from hydroxyl, halogen or $C_1$-$C_6$ alkyl; $R^{2-4-4}$ is each independently selected from hydroxyl, halogen or $C_1$-$C_6$ alkyl; $R^{2-4-1-1}$ is $C_1$-$C_6$ alkyl; $R^{2-4-1-2}$ is $C_1$-$C_6$ alkyl;

$R^{2-5}$ is each independently selected from hydrogen, 4-10 membered heterocycloalkyl unsubstituted or substituted by one or more $R^{2-5-1}$,

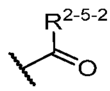 ,

6-10 membered aryl unsubstituted or substituted by one or more $R^{2-5-3}$, or $C_1$-$C_6$ alkyl, the heteroatom in said 4-10 membered heterocycloalkyl is one or more selected from N, O and S, and the number of heteroatom(s) is 1, 2 or 3;

$R^{2-5-1}$ is each independently selected from

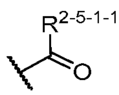 or 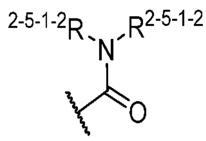 ;

$R^{2-5-1-1}$ is hydrogen or $C_1$-$C_6$ alkyl;

$R^{2-5-1-2}$ is each independently selected from hydrogen or $C_1$-$C_6$ alkyl;

$R^{2-5-2}$ is independently $C_1$-$C_6$ alkyl;

$R^{2-5-3}$ is each independently $C_1$-$C_6$ alkoxy;

$R^{2-6}$ is each independently selected from halogen, $C_1$-$C_6$ alkyl, 4-10 membered heterocycloalkyl unsubstituted or substituted by one or more $R^{2-6-1}$ or 5-10 membered heteroaryl unsubstituted or substituted by one or more $R^{2-6-2}$; the heteroatom in said 4-10 membered heterocycloalkyl is one or more selected from N, O and S, and the number of heteroatom(s) is 1, 2 or 3; the heteroatom in said 5-10 membered heteroaryl is one or more selected from N, O and S, and the number of heteroatom(s) is 1, 2 or 3;

$R^{2-6-1}$ is each independently selected from halogen, $C_1$-$C_6$ alkyl or

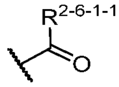 ;

$R^{2-6-1-1}$ is $C_1$-$C_6$ alkyl;

$R^{2-6-2}$ is each independently selected from halogen or $C_1$-$C_6$ alkyl;

$R^{2-7}$ is $C_1$-$C_6$ alkyl, 4-10 membered heterocycloalkyl unsubstituted or substituted by one or more $R^{2-7-1}$ or 5-10 membered heteroaryl unsubstituted or substituted by one or more $R^{2-7-2}$; the heteroatom in said 4-10 membered heterocycloalkyl is one or more selected from N, O and S, and the number of heteroatom(s) is 1, 2 or 3; the

heteroatom in said 5-10 membered heteroaryl is one or more selected from N, O and S, and the number of heteroatom(s) is 1, 2 or 3;
$R^{2-7-1}$ is each independently selected from halogen, $C_1$-$C_6$ alkyl,

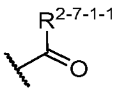
or
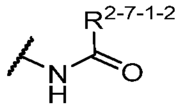
;

$R^{2-7-1-1}$ is $C_1$-$C_6$ alkyl;
$R^{2-7-1-2}$ is $C_1$-$C_6$ alkyl;
$R^{2-7-2}$ is each independently selected from halogen or $C_1$-$C_6$ alkyl;
$R^{2-8}$ is each independently $C_1$-$C_6$ alkyl, or two $R^{2-8}$, together with the atoms to which they are attached, form a 4-10 membered heterocycloalkyl unsubstituted or substituted by one or more $R^{2-8-1}$; the heteroatom in said 4-10 membered heterocycloalkyl is one or more selected from N, O and S, and the number of heteroatom(s) is 1, 2 or 3;
$R^{2-8-1}$ is each independently

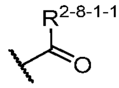
;

$R^{2-8-1-1}$ is hydrogen or $C_1$-$C_6$ alkyl;
$R^{2-9}$ is each independently 5-10 membered heteroaryl unsubstituted or substituted by one or more $R^{2-9-1}$; the heteroatom in said 5-10 membered heteroaryl is one or more selected from N, O and S, and the number of heteroatom(s) is 1, 2 or 3;
$R^{2-9-1}$ is each independently $C_1$-$C_6$ alkyl;
$R^{2-10}$ is each independently 5-10 membered heteroaryl unsubstituted or substituted by one or more $R^{2-10-1}$; the heteroatom in said 5-10 membered heteroaryl is one or more selected from N, O and S, and the number of heteroatom(s) is 1, 2 or 3;
$R^{2-10-1}$ is each independently $C_1$-$C_6$ alkyl;
$R^{2-11}$ is each independently selected from 5-10 membered heteroaryl unsubstituted or substituted by one or more $R^{2-11-1}$, or 3-6 membered heterocycloalkyl unsubstituted or substituted by one or more $R^{2-11-2}$; the heteroatom in said 5-10 membered heteroaryl is one or more selected from N, O and S, and the number of heteroatom(s) is 1, 2 or 3; the heteroatom in said 3-6 membered heterocycloalkyl is one or more selected from N, O and S, and the number of heteroatom(s) is 1, 2 or 3;
$R^{2-11-1}$ is each independently $C_1$-$C_6$ alkyl; $R^{2-11-2}$ is each independently $C_1$-$C_6$ alkyl;
$R^3$ is 5-10 membered heteroaryl unsubstituted or substituted by one or more $R^{3-1}$ or 3-10 membered heterocycloalkyl unsubstituted or substituted by one or more $R^{3-2}$; the heteroatom in said 5-10 membered heteroaryl is one or more selected from N, O and S, and the number of heteroatom(s) is 1, 2 or 3; the heteroatom in said 3-10 membered heterocycloalkyl is one or more selected from N, O and S, and the number of heteroatom(s) is 1, 2 or 3;
$R^{3-1}$ is each independently selected from hydroxyl, halogen, $C_1$-$C_6$ alkyl unsubstituted or substituted by one or more $R^{3-1-1}$ or

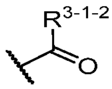
;

$R^{3-1-1}$ is each independently selected from halogen or hydroxyl; $R^{3-1-2}$ is $C_1$-$C_6$ alkyl or $C_2$-$C_6$ alkenyl;
$R^{3-2}$ is each independently selected from hydroxyl, halogen, $C_1$-$C_6$ alkyl unsubstituted or substituted by one or more $R^{3-2-1}$ or

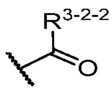
;

$R^{3-2-1}$ is each independently halogen; $R^{3-2-2}$ is $C_1$-$C_6$ alkyl or $C_2$-$C_4$ alkenyl;
$R^4$ is hydrogen, deuterium, halogen, cyano, 5-10 membered heterocycloalkyl unsubstituted or substituted by one or more $R^{4-1}$ or $C_1$-$C_6$ alkyl unsubstituted or substituted by one or more $R^{4-2}$; the heteroatom in said 5-10

membered heterocycloalkyl is one or more selected from N, O and S, and the number of heteroatom(s) is 1, 2 or 3; $R^{4-1}$ is each independently selected from

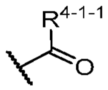 or 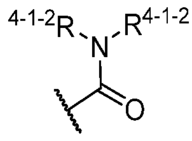 ;

$R^{4-2}$ is each independently 5-10 membered heteroaryl unsubstituted or substituted by one or more $R^{4-2-1}$; the heteroatom in said 5-10 membered heteroaryl is one or more selected from N, O and S, and the number of heteroatom(s) is 1, 2 or 3;

$R^{4-1-1}$ is $C_1$-$C_6$ alkyl; $R^{4-1-2}$ is each independently $C_1$-$C_6$ alkyl; $R^{4-2-1}$ is each independently $C_1$-$C_6$ alkyl; and $R^2$ is

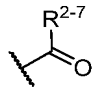 or 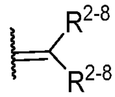

when ring B is

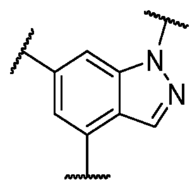

**2.** The compound of formula I, the pharmaceutically acceptable salt thereof or the isotopic compound thereof according to claim 1; wherein,

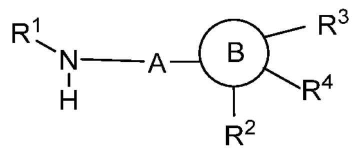

**I**

A is

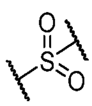 , 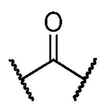 or 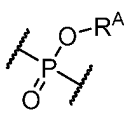 ;

$R^A$ is hydrogen or $C_1$-$C_6$ alkyl;

ring B is a 5-membered-fused 6-membered heteroaromatic ring unsubstituted or substituted by one or two $R^B$ or a 5-membered-fused 5-membered heteroaromatic ring unsubstituted or substituted by one or two $R^B$; the heteroatom in said 5-membered-fused 6-membered heteroaromatic ring is one or more selected from N, O and S, and the number of heteroatom(s) is 1, 2 or 3; the heteroatom in said 5-membered-fused 5-membered heteroaromatic ring is one or more selected from N, O and S, and the number of heteroatom(s) is 1, 2 or 3; $R^B$ is each independently selected from oxo, hydroxyl, halogen or $C_1$-$C_6$ alkyl, or two $R^B$, together with the atoms to which they are attached, form a 3-6 membered cycloalkyl;

$R^1$ is hydroxyl, $C_1$-$C_6$ alkyl unsubstituted or substituted by one or more $R^{1-1}$, $C_3$-$C_6$ cycloalkyl unsubstituted or substituted by one or more $R^{1-2}$, 3-6 membered heterocycloalkyl unsubstituted or substituted by one or more $R^{1-3}$,

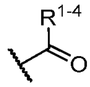

or amino unsubstituted or substituted by one or two $R^{1-5}$; the heteroatom in said 3-6 membered heterocycloalkyl is one or more selected from N, O and S, and the number of heteroatom(s) is 1, 2 or 3;

$R^{1-1}$ is each independently selected from deuterium, hydroxyl, cyano, halogen, 3-6 membered heterocycloalkyl, $C_3$-$C_6$ cycloalkyl unsubstituted or substituted by one or more $R^{1-1-1}$, $C_1$-$C_6$ alkyl unsubstituted or substituted by one or more $R^{1-1-2}$ or $C_1$-$C_6$ alkoxy unsubstituted or substituted by one or more $R^{1-1-3}$; the heteroatom in said 3-6 membered heterocycloalkyl is one or more selected from N, O and S, and the number of heteroatom(s) is 1, 2 or 3; $R^{1-1-1}$ is each independently selected from deuterium, hydroxyl, cyano or halogen; $R^{1-1-2}$ is each independently selected from deuterium, hydroxyl, cyano or halogen; $R^{1-1-3}$ is each independently selected from deuterium, hydroxyl, cyano or halogen;

$R^{1-2}$ is each independently selected from deuterium, hydroxyl, cyano, halogen, $C_1$-$C_6$ alkyl unsubstituted or substituted by one or more $R^{1-2-1}$, $C_2$-$C_6$ alkynyl unsubstituted or substituted by one or more $R^{1-2-2}$, $C_1$-$C_6$ alkoxy unsubstituted or substituted by one or more $R^{1-2-3}$, $C_2$-$C_6$ alkenyl unsubstituted or substituted by one or more $R^{1-2-4}$ or amino unsubstituted or substituted by one or two $R^{1-2-5}$; $R^{1-2-1}$ is each independently selected from halogen, hydroxyl or $C_1$-$C_6$ alkoxy unsubstituted or substituted by one or more $R^{1-2-1-1}$; $R^{1-2-1-1}$ is each independently selected from halogen; $R^{1-2-2}$ is each independently selected from halogen, hydroxyl or $C_1$-$C_6$ alkoxy unsubstituted or substituted by one or more $R^{1-2-2-1}$; $R^{1-2-2-1}$ is each independently selected from halogen; $R^{1-2-3}$ is each independently selected from halogen, hydroxyl or $C_1$-$C_6$ alkoxy unsubstituted or substituted by one or more $R^{1-2-3-1}$; $R^{1-2-3-1}$ is each independently selected from halogen; $R^{1-2-4}$ is each independently selected from halogen, hydroxyl or $C_1$-$C_6$ alkoxy unsubstituted or substituted by one or more $R^{1-2-4-1}$; $R^{1-2-4-1}$ is each independently halogen;

$R^{1-3}$ is each independently selected from cyano, deuterium, hydroxyl, amino, halogen, $C_1$-$C_6$ alkyl unsubstituted or substituted by one or more $R^{1-3-1}$ or $C_1$-$C_6$ alkoxy unsubstituted or substituted by one or more $R^{1-3-2}$; $R^{1-3-1}$ is each independently halogen;

$R^{1-3-2}$ is each independently halogen;

$R^{1-4}$ is $C_1$-$C_6$ alkyl;

$R^{1-5}$ is each independently selected from $C_1$-$C_6$ alkyl,

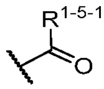

or $C_3$-$C_6$ cycloalkyl;

$R^{1-5-1}$ is $C_1$-$C_6$ alkyl or amino;

or, a 5-10 membered heterocycloalkyl unsubstituted or substituted by one or more $R^0$ is formed when the atom in R1 is attached to the atom in A; the heteroatom in said 5-10 membered heterocycloalkyl is one or more selected from N, O, P and S, and the number of heteroatom(s) is 3, 4 or 5; $R^0$ is each independently selected from $C_1$-$C_6$ alkyl or oxo;

$R^2$ is hydrogen, 6-10 membered aryl unsubstituted or substituted by one or more $R^{2-1}$, 5-10 membered heteroaryl unsubstituted or substituted by one or more $R^{2-2}$, 4-10 membered heterocycloalkyl unsubstituted or substituted by one or more $R^{2-3}$,

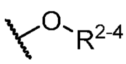
,
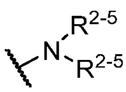
,

$C_1$-$C_6$ alkyl unsubstituted or substituted by one or more $R^{2-6}$,

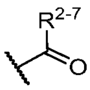
or
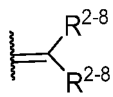
;

the heteroatom in said 5-10 membered heteroaryl is one or more selected from N, O and S, and the number of heteroatom(s) is 1, 2 or 3; the heteroatom in said 4-10 membered heterocycloalkyl is one or more selected from N, O and S, and the number of heteroatom(s) is 1, 2 or 3;

$R^{2-1}$ is each independently selected from cyano, halogen, $C_1$-$C_6$ alkyl unsubstituted or substituted by one or more $R^{2-1-1}$,

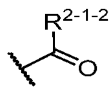 , 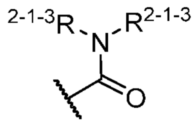

or $C_1$-$C_6$ alkoxy unsubstituted or substituted by one or more $R^{2-1-4}$;

$R^{2-1-1}$ is each independently selected from cyano or halogen; $R^{2-1-2}$ is hydrogen or $C_1$-$C_6$ alkyl; $R^{2-1-3}$ is each independently selected from hydrogen or $C_1$-$C_6$ alkyl; $R^{2-1-4}$ is each independently selected from halogen;

$R^{2-2}$ is each independently selected from cyano, halogen, $C_1$-$C_6$ alkyl unsubstituted or substituted by one or more $R^{2-2-1}$,

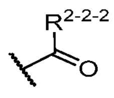 or 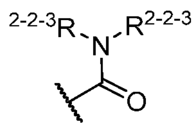 ;

$R^{2-2-1}$ is each independently selected from cyano or halogen; $R^{2-2-2}$ is hydrogen or $C_1$-$C_6$ alkyl; $R^{2-2-3}$ is each independently selected from hydrogen or $C_1$-$C_6$ alkyl;

$R^{2-3}$ is each independently selected from cyano, oxo, hydroxyl, halogen, $C_1$-$C_6$ alkyl unsubstituted or substituted by one or more $R^{2-3-1}$,

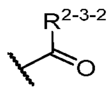 , 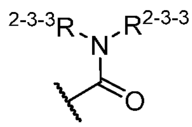 ,

3-6 membered heterocycloalkyl unsubstituted or substituted by one or more

$R^{2-3-4}$, $C_1$-$C_6$ alkoxy unsubstituted or substituted by one or more $R^{2-3-5}$; the heteroatom in said 3-6 membered heterocycloalkyl is one or more selected from N, O and S, and the number of heteroatom(s) is 1, 2 or 3;

$R^{2-3-1}$ is each independently selected from cyano, halogen, or $C_1$-$C_6$ alkoxy unsubstituted or substituted by one or more $R^{2-3-1-1}$;

$R^{2-3-2}$ is hydrogen or $C_1$-$C_6$ alkyl unsubstituted or substituted by one or more $R^{2-3-2-1}$;

$R^{2-3-3}$ is each independently selected from hydrogen or $C_1$-$C_6$ alkyl;

$R^{2-3-4}$ is each independently $C_1$-$C_6$ alkyl;

$R^{2-3-5}$ is each independently halogen;

$R^{2-3-1-1}$ is each independently halogen;

$R^{2-3-2-1}$ is each independently halogen;

each $R^{2-4}$ is 4-10 membered heterocycloalkyl unsubstituted or substituted by one or more $R^{2-4-1}$, 5-10 membered heteroaryl unsubstituted or substituted by one or more $R^{2-4-2}$, 6-10 membered aryl unsubstituted or substituted by one or more $R^{2-4-3}$ or $C_3$-$C_6$ cycloalkyl unsubstituted or substituted by one or more $R^{2-4-4}$; the heteroatom in said 4-10 membered heterocycloalkyl is one or more selected from N, O and S, and the number of heteroatom(s) is 1, 2 or 3; the heteroatom in said 5-10 membered heteroaryl is one or more selected from N, O and S, and the number of heteroatom(s) is 1, 2 or 3;

$R^{2-4-1}$ is each independently selected from hydroxyl, halogen, $C_1$-$C_6$ alkyl,

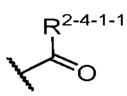 or 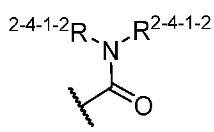 ;

$R^{2-4-2}$ is each independently selected from hydroxyl, halogen or $C_1$-$C_6$ alkyl; $R^{2-4-3}$ is each independently selected from hydroxyl, halogen or $C_1$-$C_6$ alkyl; $R^{2-4-4}$ is each independently selected from hydroxyl, halogen or $C_1$-$C_6$ alkyl; $R^{2-4-1-1}$ is $C_1$-$C_6$ alkyl; $R^{2-4-1-2}$ is $C_1$-$C_6$ alkyl;

$R^{2-5}$ is each independently hydrogen, 4-10 membered heterocycloalkyl unsubstituted or substituted by one or

more $R^{2-5-1}$, or

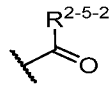
;

the heteroatom in said 4-10 membered heteroaryl is one or more selected from N, O and S, and the number of heteroatom(s) is 1, 2 or 3;
$R^{2-5-1}$ is each independently selected from

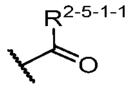
or
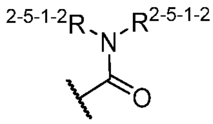
;

$R^{2-5-1-1}$ is hydrogen or $C_1$-$C_6$ alkyl;
$R^{2-5-1-2}$ is each independently selected from hydrogen or $C_1$-$C_6$ alkyl;
$R^{2-5-2}$ is independently $C_1$-$C_6$ alkyl;
$R^{2-6}$ is each independently selected from halogen, $C_1$-$C_6$ alkyl, 4-10 membered heterocycloalkyl unsubstituted or substituted by one or more $R^{2-6-1}$ or 5-10 membered heteroaryl unsubstituted or substituted by one or more $R^{2-6-2}$; the heteroatom in said 4-10 membered heterocycloalkyl is one or more selected from N, O and S, and the number of heteroatom(s) is 1, 2 or 3; the heteroatom in said 5-10 membered heteroaryl is one or more selected from N, O and S, and the number of heteroatom(s) is 1, 2 or 3;
$R^{2-6-1}$ is each independently selected from halogen, $C_1$-$C_6$ alkyl or

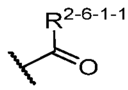
;

$R^{2-6-1-1}$ is $C_1$-$C_6$ alkyl;
$R^{2-6-2}$ is each independently selected from halogen or $C_1$-$C_6$ alkyl;
$R^{2-7}$ is $C_1$-$C_6$ alkyl, 4-10 membered heterocycloalkyl unsubstituted or substituted by one or more $R^{2-7-1}$ or 5-10 membered heteroaryl unsubstituted or substituted by one or more $R^{2-7-2}$; the heteroatom in said 4-10 membered heterocycloalkyl is one or more selected from N, O and S, and the number of heteroatom(s) is 1, 2 or 3; the heteroatom in said 5-10 membered heteroaryl is one or more selected from N, O and S, and the number of heteroatom(s) is 1, 2 or 3;
$R^{2-7-1}$ is each independently selected from halogen, $C_1$-$C_6$ alkyl,

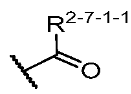
or
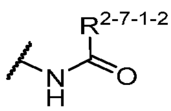

; $R^{2-7-1-1}$ is $C_1$-$C_6$ alkyl;
$R^{2-7-1-2}$ is $C_1$-$C_6$ alkyl;
$R^{2-7-2}$ is each independently selected from halogen or $C_1$-$C_6$ alkyl;
$R^{2-8}$ is each independently $C_1$-$C_6$ alkyl, or two $R^{2-8}$, together with the atoms to which they are attached, form a 4-10 membered heterocycloalkyl unsubstituted or substituted by one or more $R^{2-8-1}$; the heteroatom in said 4-10 membered heterocycloalkyl is one or more selected from N, O and S, and the number of heteroatom(s) is 1, 2 or 3;
$R^{2-8-1}$ is each independently

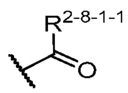
;

$R^{2-8-1-1}$ is hydrogen or $C_1$-$C_6$ alkyl;
$R^3$ is a 5-10 membered heteroaryl unsubstituted or substituted by one or more $R^{3-1}$ or 3-10 membered heterocycloalkyl unsubstituted or substituted by one or more $R^{3-2}$; the heteroatom in said 5-10 membered

heteroaryl is one or more selected from N, O and S, and the number of heteroatom(s) is 1, 2 or 3; the heteroatom in said 3-10 membered heterocycloalkyl is one or more selected from N, O and S, and the number of heteroatom(s) is 1, 2 or 3;

$R^{3-1}$ is each independently selected from hydroxyl, halogen, $C_1$-$C_6$ alkyl unsubstituted or substituted by one or more $R^{3-1-1}$ or

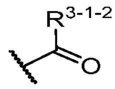

;

$R^{3-1-1}$ is each independently halogen; $R^{3-1-2}$ is $C_1$-$C_6$ alkyl or $C_2$-$C_6$ alkenyl;

$R^{3-2}$ is each independently selected from hydroxyl, halogen, $C_1$-$C_6$ alkyl unsubstituted or substituted by one or more $R^{3-2-1}$ or

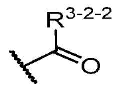

;

$R^{3-2-1}$ is each independently halogen; $R^{3-2-2}$ is $C_1$-$C_6$ alkyl or $C_2$-$C_4$ alkenyl;

$R^4$ is hydrogen, halogen, a 5-10 membered heterocycloalkyl unsubstituted or substituted by one or more $R^{4-1}$ or $C_1$-$C_6$ alkyl unsubstituted or substituted by one or more $R^{4-2}$; the heteroatom in said 5-10 membered heterocycloalkyl is one or more selected from N, O and S, and the number of heteroatom(s) is 1, 2 or 3;

$R^{4-1}$ is each independently selected from

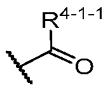

or

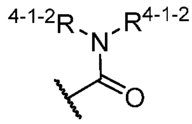

;

$R^{4-2}$ is each independently 5-10 membered heteroaryl unsubstituted or substituted by one or more $R^{4-2-1}$; the heteroatom in said 5-10 membered heteroaryl is one or more selected from N, O and S, and the number of heteroatom(s) is 1, 2 or 3;

$R^{4-1-1}$ is $C_1$-$C_6$ alkyl; $R^{4-1-2}$ is each independently $C_1$-$C_6$ alkyl; $R^{4-2-1}$ is each independently $C_1$-$C_6$ alkyl; and $R^2$ is

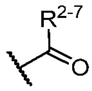

or

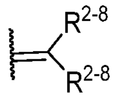

when ring B is

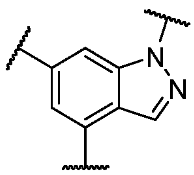

**3.** The compound of formula I, the pharmaceutically acceptable salt thereof, or the isotopic compound thereof according to claim 2, wherein the compound of formula I satisfies one or more of the following conditions:

(1) in $R^A$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl;

(2) in ring B, the heteroatom in said 5-membered-fused 5-membered heteroaromatic ring is N, and the number of heteroatom(s) can be 1, 2 or 3, for example,

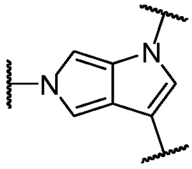;

(3) in ring B, said 3-6 membered cycloalkyl is cyclopropyl, cyclobutyl, cyclopentyl or cyclohexyl, for example, cyclopropyl;

(4) in ring B, the heteroatom in said 5-membered-fused 6-membered heteroaromatic ring is selected from N and/or O, and the number of heteroatom(s) can be 1, 2 or 3, for example,

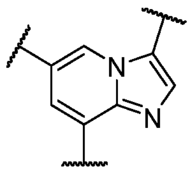, 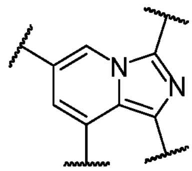, 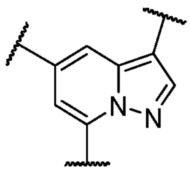,

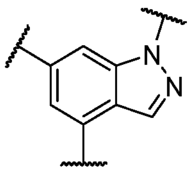, 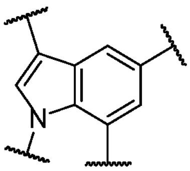 or 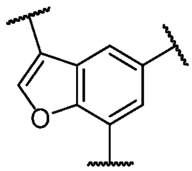;

(5) in $R^1$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl, ethyl or isopropyl;

(6) in $R^1$, said $C_3$-$C_6$ cycloalkyl is cyclopropyl, cyclobutyl, cyclopentyl, cyclohexyl or bicyclopentane, for example, cyclopropyl, cyclobutane or bicyclopentane;

(7) in $R^1$, the heteroatom in said 3-6 membered heterocycloalkyl is N, O or S, and the number of heteroatom(s) is 1 or 2; the heteroatom in said 3-6 membered heterocycloalkyl can be O or N, and the number of heteroatom can also be 1; for example, said 3-6 membered heterocycloalkyl can be

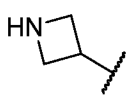, 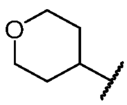 or 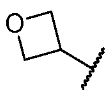;

(8) in $R^{1-1}$, the heteroatom in said 3-6 membered heterocycloalkyl is N, O or S, and the number of heteroatom(s) is 1 or 2; the heteroatom in said 3-6 membered heterocycloalkyl can also be O or N, and the number of heteroatom can also be 1, for example,

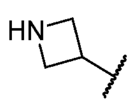, 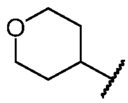 or 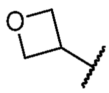;

(9) in each $R^{1-1}$, said halogen is fluorine, chlorine, bromine or iodine, for example, fluorine;

(10) in each $R^{1-1}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl, ethyl or isopropyl;

(11) in each $R^{1-1}$, said $C_1$-$C_6$ alkoxy is methoxy, ethoxy, n-propoxy, isopropoxy, n-butoxy, isobutoxy, pr-butoxy, sec-butoxy or tert-butoxy, or can be methoxy or ethoxy;

(12) in each $R^{1-1}$, said $C_3$-$C_6$ cycloalkyl is cyclopropyl, cyclobutyl, cyclopentyl or cyclohexyl;

(13) in each $R^{1-1-1}$, said halogen is fluorine, chlorine, bromine or iodine, for example, fluorine;

(14) in each $R^{1-1-2}$, said halogen is fluorine, chlorine, bromine or iodine, for example, fluorine;

*(15)* in each $R^{1-1-3}$, said halogen is fluorine, chlorine, bromine or iodine, for example, fluorine;

(16) in each $R^{1-2}$, said halogen is fluorine, chlorine, bromine or iodine, for example, fluorine;

(17) in each $R^{1-2}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl, ethyl or isopropyl;

(18) in each $R^{1-2}$, said $C_2$-$C_6$ alkynyl is

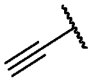, 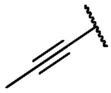, 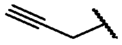, 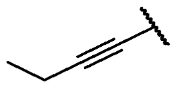, 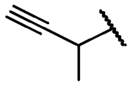 or

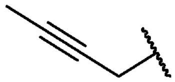;

for example,

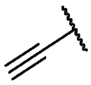;

(19) in each $R^{1-2}$, said $C_2$-$C_6$ alkenyl is

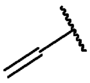, 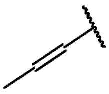, 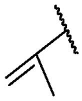 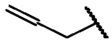, 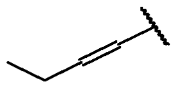, 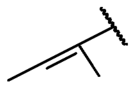,

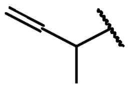 or 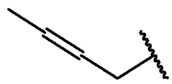;

for example,

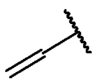;

(20) in $R^{1-2}$, said $C_1$-$C_6$ alkoxy is methoxy, ethoxy, n-propoxy, isopropoxy, n-butoxy, isobutoxy, pr-butoxy, sec-butoxy or tert-butoxy, or can be methoxy or ethoxy;

(21) in each $R^{1-2-1}$, said halogen is fluorine, chlorine, bromine or iodine, for example, fluorine;

(22) in each $R^{1-2-1}$, said $C_1$-$C_6$ alkoxy is methoxy, ethoxy, n-propoxy, isopropoxy, n-butoxy, isobutoxy, pr-butoxy, sec-butoxy or tert-butoxy, or can be methoxy or ethoxy;

(23) in each $R^{1-2-1-1}$, said halogen is fluorine, chlorine, bromine or iodine, for example, fluorine;

(24) in each $R^{1-2-2}$, said halogen is fluorine, chlorine, bromine or iodine, for example, fluorine;

(25) in each $R^{1-2-2}$, said $C_1$-$C_6$ alkoxy is methoxy, ethoxy, n-propoxy, isopropoxy, n-butoxy, isobutoxy, pr-butoxy, sec-butoxy or tert-butoxy, or can be methoxy or ethoxy;

(26) in each $R^{1-2-2-1}$, said halogen is fluorine, chlorine, bromine or iodine, for example, fluorine;

(27) in each $R^{1-2-3}$, said halogen is fluorine, chlorine, bromine or iodine, for example, fluorine;

(28) in each $R^{1-2-3}$, said $C_1$-$C_6$ alkoxy is methoxy, ethoxy, n-propoxy, isopropoxy, n-butoxy, isobutoxy, pr-butoxy, sec-butoxy or tert-butoxy, or can be methoxy or ethoxy;

(29) in each $R^{1-2-3-1}$, said halogen is fluorine, chlorine, bromine or iodine, for example, fluorine;

(30) in each $R^{1-2-4}$, said halogen is fluorine, chlorine, bromine or iodine, for example, fluorine;

(31) in each $R^{1-2-4}$, said $C_1$-$C_6$ alkoxy is methoxy, ethoxy, n-propoxy, isopropoxy, n-butoxy, isobutoxy, pr-butoxy, sec-butoxy or tert-butoxy, or can be methoxy or ethoxy;

(32) in each $R^{1-2-4-1}$, said halogen is fluorine, chlorine, bromine or iodine, for example, fluorine;

(33) in each $R^{1-2-5}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl, ethyl or isopropyl;

(34) in each $R^{1-2-5}$, said $C_3$-$C_6$ cycloalkyl is cyclopropyl, cyclobutyl, cyclopentyl, cyclohexyl or bicyclopentane, for example, cyclopropyl, cyclobutane or bicyclopentane;

(35) in each $R^{1-3}$, said halogen is fluorine, chlorine, bromine or iodine, for example, fluorine;

(36) in each $R^{1-3}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl;
(37) in each $R^{1-3}$, said $C_1$-$C_6$ alkoxy is methoxy, ethoxy, n-propoxy, isopropoxy, n-butoxy, isobutoxy, pr-butoxy, sec-butoxy or tert-butoxy, or can be methoxy or ethoxy;
(38) in each $R^{1-3-1}$, said halogen is fluorine, chlorine, bromine or iodine, for example, fluorine;
(39) in each $R^{1-3-2}$, said halogen is fluorine, chlorine, bromine or iodine, for example, fluorine;
(40) in $R^{1-4}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl;
(41) in each $R^{1-5}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl;
(42) in each $R^{1-5}$, said $C_3$-$C_6$ cycloalkyl is cyclopropyl, cyclobutyl, cyclopentyl, cyclohexyl or bicyclopentane; for example, cyclopropyl, cyclobutane or bicyclopentane;
(43) in $R^{1-5-1}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl;
(44) A 5-10 membered heterocycloalkyl unsubstituted or substituted by one or more $R^0$ is formed when the atom in $R^1$ is attached to the atom in A; said 5-10 membered heterocycloalkyl is 5-6 membered heterocycloalkyl, for example,

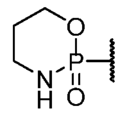

or

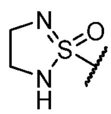

;

(45) in each $R^0$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl;
(46) in $R^2$, said 6-10 membered aryl is phenyl or naphthyl, for example, phenyl;
(47) in $R^2$, said 5-10 membered heteroaryl is 5 or 6 membered heteroaryl or 5-membered-fused 6-membered heteroaryl; the heteroatom in said 5-10 membered heteroaryl can be one or more selected from N and/or S, and the number of heteroatom(s) can be 1 or 2; the heteroatom in said 5 membered heteroaryl can be selected from N and/or S, and the number of heteroatoms is 2; the heteroatom in said 6 membered heteroaryl can be N, and the number of heteroatom(s) is 1 or 2; the heteroatom in said 5-membered-fused 6-membered heteroaryl can be selected from N and/or O, and the number of heteroatoms is 2, or the number of heteroatom(s) is 1, 2 or 3;
(48) in $R^2$, said 4-10 membered heterocycloalkyl is a monocyclic, bridged, fused or spiro ring; said monocyclic ring can be 4-6 membered heterocycloalkyl; said fused ring can be 5-membered-fused 6-membered heterocycloalkyl or 4-membered-fused 6-membered heterocycloalkyl; said spiro ring can be 4 membered-spiro 6-membered ring, 4 membered-spiro 5-membered ring or 4 membered-spiro 4-membered ring; the heteroatom in said 4-10 membered heterocycloalkyl can be selected from N and/or O, and the number of heteroatom(s) can be 1 or 2;
(49) in $R^2$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl;
(50) in each $R^{2-1}$, said halogen is fluorine, chlorine, bromine or iodine, for example, fluorine;
(51) in each $R^{2-1}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl;
(51) in each $R^{2-1}$, said $C_1$-$C_6$ alkoxy is methoxy, ethoxy, n-propoxy, isopropoxy, n-butoxy, isobutoxy, pr-butoxy, sec-butoxy or tert-butoxy, or can be methoxy or ethoxy;
(53) in each $R^{2-1-1}$, said halogen is fluorine, chlorine, bromine or iodine, for example, fluorine;
(54) in each $R^{2-1-2}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl;
(55) in each $R^{2-1-3}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl;
(56) in each $R^{2-1-4}$, said halogen is fluorine, chlorine, bromine or iodine, for example, fluorine;
(57) in each $R^{2-2}$, said halogen is fluorine, chlorine, bromine or iodine, for example, fluorine;

(58) in each $R^{2-2}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl or isopropyl;
(59) in each $R^{2-2-1}$, said halogen is fluorine, chlorine, bromine or iodine, for example, fluorine;
(60) in each $R^{2-2-2}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl;
(61) in each $R^{2-2-3}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl;
(62) in each $R^{2-3}$, said halogen is fluorine, chlorine, bromine or iodine, for example, fluorine;
(63) in each $R^{2-3}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl;
(64) in each $R^{2-3}$, the heteroatom in said 3-6 membered heterocycloalkyl is N, O or S, and the number of heteroatom(s) can be 1 or 2; the heteroatom in said 3-6 membered heterocycloalkyl can also be O or N, and the number of heteroatom(s) can also be 1; for example,

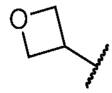;

(65) in each $R^{2-3}$, said $C_1$-$C_6$ alkoxy is methoxy, ethoxy, n-propoxy, isopropoxy, n-butoxy, isobutoxy, pr-butoxy, sec-butoxy or tert-butoxy, or can be methoxy or ethoxy;
(66) in each $R^{2-3-1}$, said halogen is fluorine, chlorine, bromine or iodine, for example, fluorine;
(67) in each $R^{2-3-1}$, said $C_1$-$C_6$ alkoxy is methoxy, ethoxy, n-propoxy, isopropoxy, n-butoxy, isobutoxy, pr-butoxy, sec-butoxy or tert-butoxy, and can be methoxy or ethoxy; in $R^{2-3-2}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl or isopropyl;
(68) in each $R^{2-3-3}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl;
(69) in each $R^{2-3}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be isopropyl;
(70) in each $R^{2-3-5}$, said halogen is fluorine, chlorine, bromine or iodine, for example, fluorine;
(71) in each $R^{2-3-1-1}$, said halogen is fluorine, chlorine, bromine or iodine, for example, fluorine;
(72) in each $R^{2-3-2-1}$, said halogen is fluorine, chlorine, bromine or iodine, for example, fluorine;
(73) in each $R^{2-4}$, said 4-10 membered heterocycloalkyl is a monocyclic ring; said 4-10 membered heterocycloalkyl can be 4-6 membered heterocycloalkyl; the heteroatom in said 4-10 membered heterocycloalkyl can be selected from N and/or O, and the number of heteroatom(s) is 1 or 2;
(75) in each $R^{2-4}$, said 5-10 membered heteroaryl is 5-membered-fused 6-membered heteroaryl; the heteroatom in said 5-membered-fused 6-membered heteroaryl can be selected from N and/or O, and the number of heteroatom(s) is 1, 2 or 3;
(76) in each $R^{2-4}$, said 6-10 membered aryl is phenyl or naphthyl, for example, phenyl;
(77) in each $R^{2-4}$, said $C_3$-$C_6$ cycloalkyl is cyclopropyl, cyclobutyl, cyclopentyl or cyclohexyl, for example, cyclopentyl;
(78) in each $R^{2-4-1}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl;
(79) in each $R^{2-4-1}$, said halogen is fluorine, chlorine, bromine or iodine, for example, fluorine;
(80) in each $R^{2-4-2}$, said halogen is fluorine, chlorine, bromine or iodine, for example, fluorine;
(81) in each $R^{2-4-2}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl;
(82) in each $R^{2-4-3}$, said halogen is fluorine, chlorine, bromine or iodine, for example, fluorine;
(83) in each $R^{2-4-3}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl;
(84) in each $R^{2-4-4}$, said halogen is fluorine, chlorine, bromine or iodine, for example, fluorine;
(85) in each $R^{2-4-4}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl;
(86) in $R^{2-4-1-1}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl or isopropyl;
(86) in each $R^{2-4-1-2}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl or isopropyl;
(88) in each $R^{2-5}$, said 4-10 membered heterocycloalkyl is a monocyclic ring; said 4-10 membered heterocycloalkyl can be 4-6 membered heterocycloalkyl; the heteroatom in said 4-10 membered heterocycloalkyl can

be selected from N and/or O, and the number of heteroatom(s) can be 1 or 2;
(89) in $R^{2-5-1-1}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl;
(90) in each $R^{2-5-1-2}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl or isopropyl;
(91) in $R^{2-5-2}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl or isopropyl;
(92) in each $R^{2-6}$, said halogen is fluorine, chlorine, bromine or iodine, for example, fluorine;
(93) in each $R^{2-6}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl;
(94) in each $R^{2-6}$, said 4-10 membered heterocycloalkyl is a monocyclic ring; said 4-10 membered heterocycloalkyl can be 4-6 membered heterocycloalkyl; the heteroatom in said 4-10 membered heterocycloalkyl is selected from N and/or O, and the number of heteroatom(s) is 1 or 2;
(95) in each $R^{2-6}$, said 5-10 membered heteroaryl is 5-6 membered heteroaryl; the heteroatom in said 5-6 membered heteroaryl can be selected from N and/or S, and the number of heteroatoms is 2, or the number of heteroatom(s) is 1 or 2;
(96) in each $R^{2-6-1}$, said halogen is fluorine, chlorine, bromine or iodine, for example, fluorine;
(97) in each $R^{2-6-1}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl;
(98) in $R^{2-6-1-1}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl;
(99) in each $R^{2-6-2}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl or isopropyl;
(100) in each $R^{2-6-2}$, said halogen is fluorine, chlorine, bromine or iodine, for example, fluorine;
(101) in each $R^{2-7}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl;
(102) in each $R^{2-7}$, said 4-10 membered heterocycloalkyl can be a monocyclic or spiro ring; said monocyclic ring can be 4-6 membered heterocycloalkyl; said spiro ring can be 4 membered-spiro 6-membered ring, 4 membered-spiro 5-membered ring or 4 membered-spiro 4-membered ring; the heteroatom in said 4-10 membered heterocycloalkyl can be selected from N and/or O, and the number of heteroatom(s) can be 1 or 2;
(103) in each $R^{2-7}$, said 5-10 membered heteroaryl is 5 or 6 membered heteroaryl or 5-membered-fused 6-membered heteroaryl; the heteroatom in said 5-10 membered heteroaryl can be one or more selected from N and/or S, and the number of heteroatom(s) can be 1 or 2; the heteroatom in said 5 membered heteroaryl can be selected from N and/or S, and the number of heteroatoms can be 2; the heteroatom in said 6 membered heteroaryl can be N, and the number of heteroatom(s) is 1 or 2; the heteroatom in said 5-membered-fused 6-membered heteroaryl can be selected from N and/or O, and the number of heteroatoms can be 2, or the number of heteroatom(s) is 1, 2 or 3;
(104) in each $R^{2-7-1}$, said halogen is fluorine, chlorine, bromine or iodine, for example, fluorine;
(105) in each $R^{2-7-1}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl;
(106) in $R^{2-7-1-2}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl;
(107) in each $R^{2-7-2}$, said halogen is fluorine, chlorine, bromine or iodine, for example, fluorine;
(108) in each $R^{2-7-2}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl;
(109) in each $R^{2-8}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl;
(110) in each $R^{2-8}$, said 4-10 membered heterocycloalkyl is a monocyclic ring; said monocyclic ring can be 4-6 membered heterocycloalkyl; the heteroatom in said 4-10 membered heterocycloalkyl can be selected from N and/or O, and the number of heteroatom(s) can be 1 or 2;
(111) in each $R^{2-8-1-1}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl;
(112) in $R^3$, said 5-10 membered heteroaryl is 5 membered heteroaryl, 5-membered-fused 5-membered heteroaryl or 5-membered-fused 6-membered heteroaryl; the heteroatom in said 5-10 membered heteroaryl can be one or more selected from N and/or S, and the number of heteroatom(s) can be 1 or 2; the heteroatom in said 5 membered heteroaryl can be selected from N and/or S, and the number of heteroatom(s) can be 2; the heteroatom in said 5-membered-fused 6-membered heteroaryl can be selected from N and/or O, and the number of heteroatoms is 2, or the number of heteroatom(s) can be 1, 2 or 3; the heteroatom in said 5-membered-fused 5-membered heteroaryl can be selected from N and/or O, and the number of heteroatoms can be 2;

(113) in $R^3$, said 3-10 membered heterocycloalkyl is a monocyclic ring; said monocyclic ring can be 4-6 membered heterocycloalkyl; the heteroatom in said 3-10 membered heterocycloalkyl can be selected from N, and the number of heteroatom(s) can be 1 or 2;

(114) in each $R^{3-1}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl;

(115) in each $R^{3-1}$, said halogen is fluorine, chlorine, bromine or iodine, for example, fluorine;

(116) in each $R^{3-1-1}$, said halogen is fluorine, chlorine, bromine or iodine, for example, fluorine;

(117) in $R^{3-1-2}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl or ethyl;

(118) in $R^{3-1-2}$, said $C_2$-$C_6$ alkenyl is

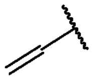 , 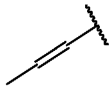 , 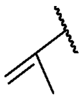 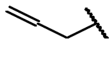 , 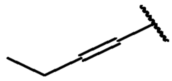 , 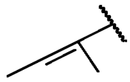 ,

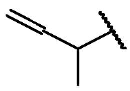 or 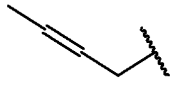 ,

for example,

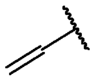 ;

(119) in $R^{3-2}$, said halogen is fluorine, chlorine, bromine or iodine, for example, fluorine;

(120) in $R^{3-2}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl;

(121) in $R^{3-2-1}$, said halogen is fluorine, chlorine, bromine or iodine, for example, fluorine;

(122) in $R^{3-2-2}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl;

(123) in $R^{3-2-2}$, said $C_2$-$C_6$ alkenyl is

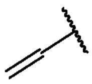 , 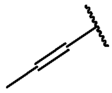 , 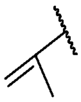 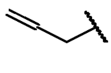 , 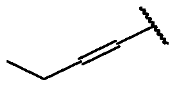 , 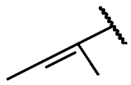 ,

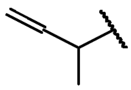 or 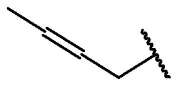 ,

for example,

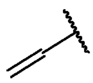 ;

(125) in $R^4$, said halogen can be fluorine, chlorine, bromine or iodine, for example, fluorine;

(126) in $R^4$, said 5-10 membered heterocycloalkyl is a spiro ring; said spiro ring can be 4 membered-spiro 6-membered ring, 4 membered-spiro 5-membered ring, or 4 membered-spiro 4-membered ring; the heteroatom in said 4-10 membered heterocycloalkyl can be N, and the number of heteroatom(s) is 1 or 2;

(127) in $R^4$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl;

(128) in each $R^{4-2}$, said 5-10 membered heteroaryl is 5 membered heteroaryl; the heteroatom in said 5 membered heteroaryl can be selected from N and/or S, and the number of heteroatoms can be 2;

(129) in $R^{4-1-1}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl or isopropyl;
(130) in each $R^{4-1-2}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl;
(131) in each $R^{4-2-1}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl;
(132) in $R^{1-2-5-1}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl, ethyl or isopropyl;
and (133) in $R^{2-7-1-1}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl.

**4.** The compound of formula I, the pharmaceutically acceptable salt thereof, or the isotopic compound thereof according to claim 2, wherein the compound of formula I satisfies one or more of the following conditions:

(1) $R^1$ is $C_3$-$C_6$ cycloalkyl unsubstituted or substituted by one or more $R^{1-2}$ or 3-6 membered heterocycloalkylunsubstituted or substituted by one or more $R^{1-3}$; the heteroatom in said 3-6 membered heterocycloalkyl is N, O or S, and the number of heteroatom(s) is 1;
(2) $R^{1-2}$ is each independently selected from cyano or $C_1$-$C_6$ alkyl unsubstituted or substituted by one or more $R^{1-2-1}$;
(3) $R^{1-2-1}$ is each independently halogen;
(4) $R^{1-3}$ is each independently $C_1$-$C_6$ alkyl unsubstituted or substituted by one or more $R^{1-3-1}$;
(5) $R^{1-3-1}$ is each independently halogen;
(6) $R^2$ is hydrogen or 4-10 membered heterocycloalkyl unsubstituted or substituted by one or more $R^{2-3}$; the heteroatom in said 4-10 membered heterocycloalkyl is N, O or S, and the number of heteroatom(s) is 1 or 2;
(7) $R^{2-3}$ is each independently

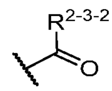 ;

(8) $R^{2-3-2}$ is $C_1$-$C_6$ alkyl;
(9) $R^3$ is 5-10 membered heteroaryl unsubstituted or substituted by one or more $R^{3-1}$ or 3-10 membered heterocycloalkyl unsubstituted or substituted by one or more $R^{5-2}$; the heteroatom in said 5-10 membered heteroaryl is one or more selected from N, O and S, and the number of heteroatom(s) is 1, 2 or 3; the heteroatom in said 5-10 membered heterocycloalkyl is N, O and S, and the number of heteroatom(s) is 1 or 2;
(10) $R^{3-1}$ is each independently selected from hydroxyl, halogen, $C_1$-$C_6$ alkyl unsubstituted or substituted by one or more $R^{3-1-1}$;
(11) $R^{3-1-1}$ is each independently halogen;
(12) $R^{3-2}$ is each independently

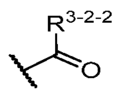 ;

(13) $R^{3-2-2}$ is $C_1$-$C_6$ alkyl or $C_2$-$C_4$ alkenyl;
(14) $R^4$ is 5-10 membered heterocycloalkyl unsubstituted or substituted by one or more $R^{4-1}$; the heteroatom in said 5-10 membered heterocycloalkyl is N, O or S, and the number of heteroatom(s) is 1, 2 or 3;
*(15)* $R^{4-1}$ is each independently selected from

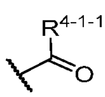 or 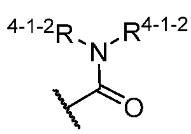 ;

(16) $R^{4-1-1}$ is $C_1$-$C_6$ alkyl;

and (17) $R^{4-1-2}$ is each independently $C_1$-$C_6$ alkyl.

**5.** The compound of formula I, the pharmaceutically acceptable salt thereof, or the isotopic compound thereof according to claim 2, wherein the compound of formula I satisfies one or more of the following conditions:

(1) $R^1$ is $C_3$-$C_6$ cycloalkyl or 4 membered heterocycloalkyl, unsubstituted or substituted by one or more $R^{1-2}$; the heteroatom in said 4 membered heterocycloalkyl is O, and the number of heteroatom(s) is 1;
(2) $R^2$ is hydrogen or 6-10 membered heterocycloalkyl unsubstituted or substituted by one or more $R^{2-3}$; the heteroatom in said 6-10 membered heterocycloalkyl is N, and the number of heteroatom(s) is 1 or 2;
(3) $R^3$ is 5-6 membered heteroaryl unsubstituted or substituted by one or more $R^{3-1}$ or 5-6 membered heterocycloalkyl unsubstituted or substituted by one or more $R^{3-2}$; the heteroatom in said 5-6 membered heteroaryl is N and/or S, and the number of heteroatom(s) is 1, 2 or 3; the heteroatom in said 5-10 membered heterocycloalkyl is N, and the number of heteroatom(s) is 1;
(4) $R^{3-2-2}$ is $C_2$-$C_4$ alkenyl;

and (5) $R^4$ is 5-10 membered heterocycloalkyl unsubstituted or substituted by one or more $R^{4-1}$; the heteroatom in said 5-10 membered heterocycloalkyl is N, and the number of heteroatom(s) is 1 or 2.

**6.** The compound of formula I, the pharmaceutically acceptable salt thereof, or the isotopic compound thereof according to claim 2, wherein the compound of formula I satisfies one or more of the following conditions:

(1) $R^1$ is $C_3$-$C_6$ cycloalkyl unsubstituted or substituted by one or more $R^{1-2}$;
(2) $R^2$ is hydrogen or 6-10 membered heterocycloalkyl unsubstituted or substituted by one or more $R^{2-3}$; the heteroatom in said 6-10 membered heterocycloalkyl is N, and the number of heteroatom(s) is 1 or 2;
(3) $R^3$ is 5-6 membered heteroaryl unsubstituted or substituted by one or more $R^{3-1}$; the heteroatom in said 5-6 membered heteroaryl is N and/or S, and the number of heteroatoms is 3;

and (4) $R^4$ is 7-9 membered heterocycloalkyl unsubstituted or substituted by one or more $R^{4-1}$; the heteroatom in said 7-9 membered heterocycloalkyl is N, and the number of heteroatom(s) is 1.

**7.** The compound of formula I, the pharmaceutically acceptable salt thereof, or the isotopic compound thereof according to claim 1, wherein the compound of formula I is a compound of formula I-a,

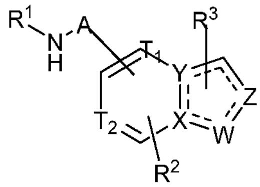

**I-a** ;

Y is C or N,X is C or N,
W is N, CH, O, $NR^4$ or $CR^4$;
Z is CH, O or N;
$T_1$ is C or N;
$T_2$ is C or N; and
A, $R^1$, $R^2$, $R^3$ and $R^4$ are defined as in any one of claims 1-6.

**8.** The compound of formula I, the pharmaceutically acceptable salt thereof or the isotopic compound thereof according to claim 7, wherein at least one of $T_1$, $T_2$, X and Y is N, or Y, W and Z comprise only one heteroatom.

**9.** The compound of formula I, the pharmaceutically acceptable salt thereof or the isotopic compound thereof according to claim 1, wherein the compound of formula I-a can be a compound of formula I-A, I-B or I-C,

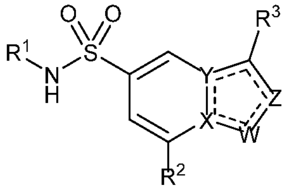

I-A ,

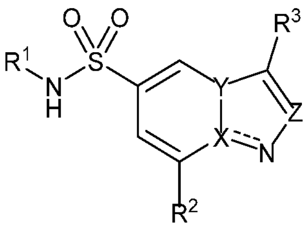

I-B or 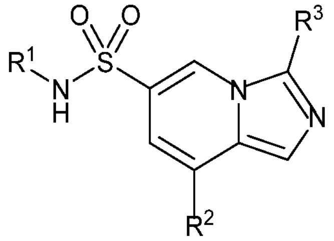

I-C ;

and/or, the compound of formula I is a compound of formula I-b, I-c, I-d, I-e, I-f, I-g, I-h, I-i, I-j, I-k, I-L or I-m,

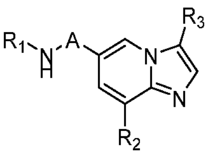

I-b ,

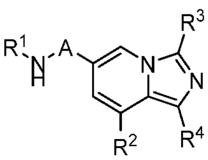

I-c ,

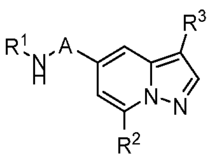

I-d ,

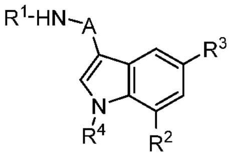

I-e ,

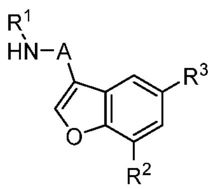

I-f ,

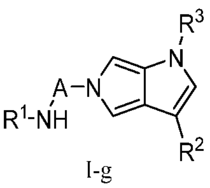

I-g ,

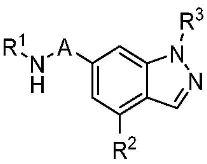

I-h ,

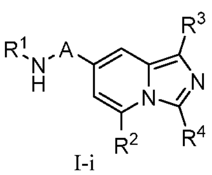

I-i ,

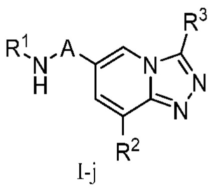

I-j ,

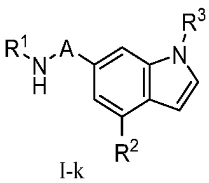

I-k ,

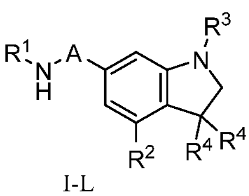

I-L or

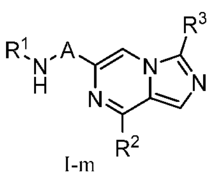

I-m

**10.** The compound of formula I, the pharmaceutically acceptable salt thereof or the isotopic compound thereof according to claim 2, wherein the compound of formula I is defined as in a scheme 1, a scheme 2, or a scheme 3;

in the scheme 1, $R^1$ is $C_3$-$C_6$ cycloalkyl unsubstituted or substituted by one or more $R^{1-2}$ or 3-6 membered heterocycloalkylunsubstituted or substituted by one or more $R^{1-3}$; the heteroatom in said 3-6 membered heterocycloalkyl is N, O or S, and the number of heteroatom(s) is 1;
$R^{1-2}$ is each independently selected from cyano or $C_1$-$C_6$ alkyl unsubstituted or substituted by one or more $R^{1-2-1}$; $R^{1-2-1}$ is each independently halogen;
$R^{1-3}$ is each independently $C_1$-$C_6$ alkyl unsubstituted or substituted by one or more $R^{1-3-1}$; $R^{1-3-1}$ is each independently halogen; $R^2$ is hydrogen or 4-10 membered heterocycloalkyl unsubstituted or substituted by one or more $R^{2-3}$; the heteroatom in said 4-10 membered heterocycloalkyl is N, O or S, and the number of heteroatom(s) is 1 or 2;

$R^{2-3}$ is each independently

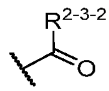

;

$R^{2-3-2}$ is $C_1$-$C_6$ alkyl;
$R^3$ is 5-10 membered heteroaryl unsubstituted or substituted by one or more $R^{3-1}$ or 3-10 membered heterocycloalkyl unsubstituted or substituted by one or more $R^{5-2}$; the heteroatom in said 5-10 membered heteroaryl is one or more selected from N, O and S, and the number of heteroatom(s) is 1, 2 or 3; the heteroatom in said 5-10 membered heterocycloalkyl is N, O and S, and the number of heteroatom(s) is 1 or 2;
$R^{3-1}$ is each independently selected from hydroxyl, halogen, $C_1$-$C_6$ alkyl unsubstituted or substituted by one or more $R^{3-1-1}$;
$R^{3-1-1}$ is each independently halogen;
$R^{3-2}$ is each independently

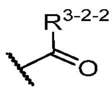

;

$R^{3-2-2}$ is $C_1$-$C_6$ alkyl or $C_2$-$C_4$ alkenyl;
$R^4$ is 5-10 membered heterocycloalkyl unsubstituted or substituted by one or more $R^{4-1}$; the heteroatom in said 5-10 membered heterocycloalkyl is N, O or S, and the number of heteroatom(s) is 1, 2 or 3;
$R^{4-1}$ is each independently selected from

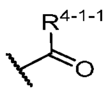 or 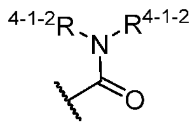

;

$R^{4-1-1}$ is $C_1$-$C_6$ alkyl;
$R^{4-1-2}$ is each independently $C_1$-$C_6$ alkyl;
in the scheme 2, $R^1$ is $C_3$-$C_6$ cycloalkyl or 4 membered heterocycloalkyl, unsubstituted or substituted by one or more $R^{1-2}$; the heteroatom in said 4 membered heterocycloalkyl is O, and the number of heteroatom(s) is 1;
$R^{1-2}$ is each independently selected from cyano or $C_1$-$C_6$ alkyl unsubstituted or substituted by one or more $R^{1-2-1}$;
$R^{1-2-1}$ is each independently halogen;
$R^2$ is hydrogen or 6-10 membered heterocycloalkyl unsubstituted or substituted by one or more $R^{2-3}$; the heteroatom in said 6-10 membered heterocycloalkyl is N, and the number of heteroatom(s) is 1 or 2;
$R^{2-3}$ is each independently

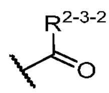

;

$R^{2-3-2}$ is $C_1$-$C_6$ alkyl;
$R^3$ is 5-6 membered heteroaryl unsubstituted or substituted by one or more $R^{3-1}$ or 5-6 membered heterocycloalkyl unsubstituted or substituted by one or more $R^{3-2}$; the heteroatom in said 5-6 membered heteroaryl is N and/or S, and the number of heteroatom(s) is 1, 2 or 3; the heteroatom in said 5-10 membered heterocycloalkyl is N, and the number of heteroatom(s) is 1;
$R^{3-1}$ is each independently selected from halogen, or $C_1$-$C_6$ alkoxy unsubstituted or substituted by one or more $R^{3-1-1}$;
$R^{3-1-1}$ is each independently halogen;
$R^{3-2}$ is each independently

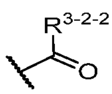

;

$R^{3-2-2}$ is $C_2$-$C_4$ alkenyl;
$R^4$ is 5-10 membered heterocycloalkyl unsubstituted or substituted by one or more $R^{4-1}$; the heteroatom in said 5-10 membered heterocycloalkyl is N, and the number of heteroatom(s) is 1 or 2;
$R^{4-1}$ is each independently selected from

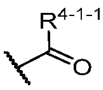
or
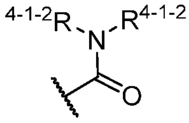
;

$R^{4-1-1}$ is $C_1$-$C_6$ alkyl;
$R^{4-1-2}$ is each independently $C_1$-$C_6$ alkyl;
in the scheme 3, $R^1$ is $C_3$-$C_6$ cycloalkyl unsubstituted or substituted by one or more $R^{1-2}$;
$R^{1-2}$ is each independently selected from cyano or $C_1$-$C_6$ alkyl unsubstituted or substituted by one or more $R^{1-2-1}$;
$R^{1-2-1}$ is each independently halogen;
$R^2$ is hydrogen or 6-10 membered heterocycloalkyl unsubstituted or substituted by one or more $R^{2-3}$; the heteroatom in said 6-10 membered heterocycloalkyl is N, and the number of heteroatom(s) is 1 or 2;
$R^{2-3}$ is each independently

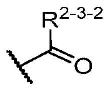
;

$R^{2-3-2}$ is $C_1$-$C_6$ alkyl;
$R^3$ is 5-6 membered heteroaryl unsubstituted or substituted by one or more $R^{3-1}$; the heteroatom in said 5-6 membered heteroaryl is N and/or S, and the number of heteroatoms is 3;
$R^{3-1}$ is each independently selected from halogen, or $C_1$-$C_6$ alkoxy unsubstituted or substituted by one or more $R^{3-1-1}$;
$R^{3-1-1}$ is each independently halogen;
$R^4$ is 7-9 membered heterocycloalkyl unsubstituted or substituted by one or more $R^{4-1}$; the heteroatom in said 7-9 membered heterocycloalkyl is N, and the number of heteroatom(s) is 1;
$R^{4-1}$ is each independently selected from

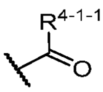
or
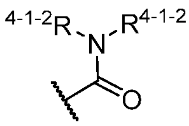
;

$R^{4-1-1}$ is $C_1$-$C_6$ alkyl; and
$R^{4-1-2}$ is each independently $C_1$-$C_6$ alkyl.

**11.** The compound of formula I, the pharmaceutically acceptable salt thereof, or the isotopic compound thereof according to claim 1, wherein the compound of formula I satisfies one or more of the following conditions:

(1) $R^1$ is hydroxyl, $C_1$-$C_6$ alkyl unsubstituted or substituted by one or more $R^{1-1}$, $C_3$-$C_6$ cycloalkyl unsubstituted or substituted by one or more $R^{1-2}$, 3-6 membered heterocycloalkyl unsubstituted or substituted by one or more $R^{1-3}$,

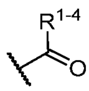

or amino unsubstituted or substituted by one or two $R^{1-5}$; the heteroatom in said 3-6 membered heterocycloalkyl is one or more selected from N, O and S, and the number of heteroatom(s) is 1, 2 or 3;

$R^{1-1}$ is each independently selected from deuterium, hydroxyl, cyano, halogen, 3-6 membered heterocycloalkyl, $C_3$-$C_6$ cycloalkyl unsubstituted or substituted by one or more $R^{1-1-1}$, $C_1$-$C_6$ alkyl unsubstituted or substituted by one or more $R^{1-1-2}$ or $C_1$-$C_6$ alkoxy unsubstituted or substituted by one or more $R^{1-1-3}$; the

heteroatom in said 3-6 membered heterocycloalkyl is one or more selected from N, O and S, and the number of heteroatom(s) is 1, 2 or 3; $R^{1-1-1}$ is each independently selected from deuterium, hydroxyl, cyano or halogen; $R^{1-1-2}$ is each independently selected from deuterium, hydroxyl, cyano or halogen; $R^{1-1-3}$ is each independently selected from deuterium, hydroxyl, cyano or halogen;

$R^{1-2}$ is each independently selected from deuterium, hydroxyl, halogen, $C_2$-$C_6$ alkynyl unsubstituted or substituted by one or more $R^{1-2-2}$, $C_1$-$C_6$ alkoxy unsubstituted or substituted by one or more $R^{1-2-3}$, $C_2$-$C_6$ alkenyl unsubstituted or substituted by one or more $R^{1-2-4}$, amino unsubstituted or substituted by one or two $R^{1-2-5}$ or

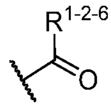
;

$R^{1-2-2-1}$ is each independently halogen; $R^{1-2-3}$ is each independently selected from halogen, hydroxyl or $C_1$-$C_6$ alkoxy unsubstituted or substituted by one or more $R^{1-2-3-1}$; $R^{1-2-3-1}$ is each independently halogen; $R^{1-2-4}$ is each independently selected from halogen, hydroxyl or $C_1$-$C_6$ alkoxy unsubstituted or substituted by one or more $R^{1-2-4-1}$; $R^{1-2-4-1}$ is each independently halogen; $R^{1-2-6}$ is each independently amino;

$R^{1-2-5}$ is each independently

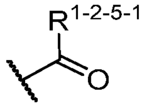
:

, $R^{1-2-5-1}$ is each independently $C_1$-$C_6$ alkyl; $R^{1-3}$ is each independently selected from cyano, deuterium, hydroxyl, amino, halogen, $C_1$-$C_6$ alkyl unsubstituted or substituted by one or more $R^{1-3-1}$, $C_1$-$C_6$ alkoxy unsubstituted or substituted by one or more $R^{1-3-2}$ or $C_2$-$C_6$ alkynyl; $R^{1-3-1}$ is each independently halogen; $R^{1-3-2}$ is each independently halogen;

$R^{1-4}$ is $C_1$-$C_6$ alkyl;

$R^{1-5}$ is each independently selected from $C_1$-$C_6$ alkyl,

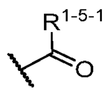

or $C_3$-$C_6$ cycloalkyl;

$R^{1-5-1}$ is $C_1$-$C_6$ alkyl or amino;

or, a 5-10 membered heterocycloalkyl unsubstituted or substituted by one or more $R^0$ is formed when the atom in R1 is attached to the atom in A; the heteroatom in said 5-10 membered heterocycloalkyl is one or more selected from N, O, P and S, and the number of heteroatom(s) is 3, 4 or 5; $R^0$ is each independently selected from $C_1$-$C_6$ alkyl or oxo;

(2) $R^2$ is hydrogen, 6-10 membered aryl unsubstituted or substituted by one or more $R^{2-1}$, 5-10 membered heteroaryl unsubstituted or substituted by one or more $R^{2-2}$, 4-12 membered heterocycloalkyl substituted by one or more $R^{2-3}$,

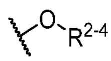
,

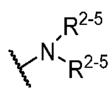
,

$C_1$-$C_6$ alkyl unsubstituted or substituted by one or more $R^{2-6}$,

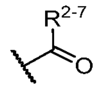, 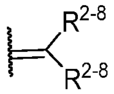,

$C_3$-$C_6$ cycloalkyl unsubstituted or substituted by one or more $R^{2-9}$, $C_3$-$C_6$ cycloalkenyl unsubstituted or substituted by one or more $R^{2-10}$, or

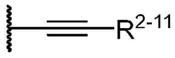;

the heteroatom in said 5-10 membered heteroaryl is one or more selected from N, O and S, and the number of heteroatom(s) is 1, 2 or 3; the heteroatom in said 4-12 membered heterocycloalkyl is one or more selected from N, O and S, and the number of heteroatom(s) is 1, 2 or 3;

$R^{2-1}$ is each independently selected from cyano, halogen, $C_1$-$C_6$ alkyl unsubstituted or substituted by one or more $R^{2-1-1}$,

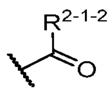, 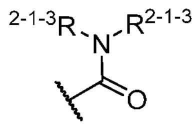

or $C_1$-$C_6$ alkoxy unsubstituted or substituted by one or more $R^{2-1-4}$;
$R^{2-1-1}$ is each independently selected from cyano or halogen; $R^{2-1-2}$ is hydrogen or $C_1$-$C_6$ alkyl; $R^{2-1-3}$ is each independently selected from hydrogen or $C_1$-$C_6$ alkyl; $R^{2-1-4}$ is each independently selected from halogen;
$R^{2-2}$ is each independently selected from cyano, halogen, $C_1$-$C_6$ alkyl unsubstituted or substituted by one or more $R^{2-2-1}$,

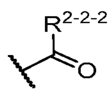, 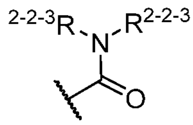,

$C_1$-$C_6$ alkoxy or

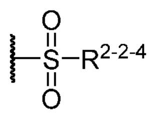;

$R^{2-2-1}$ is each independently selected from cyano or halogen; $R^{2-2-2}$ is hydrogen or $C_1$-$C_6$ alkyl; $R^{2-2-3}$ is each independently selected from hydrogen or $C_1$-$C_6$ alkyl; $R^{2-2-4}$ is each independently selected from $C_1$-$C_6$ alkyl or $C_3$-$C_6$ cycloalkyl;
$R^{2-3}$ is each independently selected from $C_1$-$C_6$ alkyl substituted by one or more $R^{2-3-1}$,

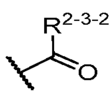,

3-6 membered heterocycloalkyl unsubstituted or substituted by one or more $R^{2-3-4}$, amino unsubstituted or substituted by one or more $R^{2-3-7}$, 5-10 membered heteroaryl unsubstituted or substituted by one or more $R^{2-3-9}$; the heteroatom in said 3-6 membered heterocycloalkyl is one or more selected from N, O and S, and the number of heteroatom(s) is 1, 2 or 3; the heteroatom in said 5-10 membered heteroaryl is one or more selected from N, O and S, and the number of heteroatom(s) is 1, 2 or 3;
$R^{2-3-1}$ is each independently selected from cyano, or $C_3$-$C_6$ cyano unsubstituted or substituted by one or more $R^{2-3-1-2}$;

$R^{2-3-2}$ is hydrogen, or $C_1$-$C_6$ alkylsubstituted by one or more $R^{2-3-2-1}$, $C_3$-$C_6$ cycloalkyl substituted by one or more $R^{2-3-2-2}$,

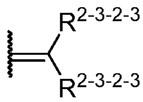
,

3-6 membered heterocycloalkyl unsubstituted or substituted by one or more $R^{2-3-2-5}$, 5-10 membered heteroaryl unsubstituted or substituted by one or more $R^{2-3-2-6}$; the heteroatom in said 3-6 membered heterocycloalkyl is one or more selected from N, O and S, and the number of heteroatom(s) is 1, 2 or 3; the heteroatom in said 5-10 membered heteroaryl is one or more selected from N, O and S, and the number of heteroatom(s) is 1, 2 or 3;
$R^{2-3-4}$ is each independently $C_1$-$C_6$ alkyl;
$R^{2-3-7}$ is each independently $C_1$-$C_6$ alkyl;
$R^{2-3}$ is each independently selected from oxo or $C_1$-$C_6$ alkyl;
$R^{2-3-1-2}$ is each independently $C_1$-$C_6$ alkoxy; $R^{2-3-2-1}$ is each independently selected from hydroxyl, oxo,

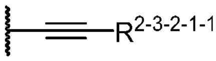
,

or 5-10 membered heteroaryl unsubstituted or substituted by one or more $R^{2-3-2-1-3}$; the heteroatom in said 5-10 membered heteroaryl is one or more selected from N, O and S, and the number of heteroatom(s) is 1, 2 or 3;
$R^{2-3-2-1-1}$ is each independently selected from $C_1$-$C_6$ alkyl or 6-10 membered aryl; $R^{2-3-2-1-2}$ is each independently $C_1$-$C_6$ alkyl;
$R^{2-3-2-1-3}$ is each independently $C_1$-$C_6$ alkyl;
$R^{2-3-2-2}$ is each independently selected from halogen, hydroxyl, $C_1$-$C_6$ alkyl unsubstituted or substituted by one or more $R^{2-3-2-2-1}$, $C_1$-$C_6$ alkoxy unsubstituted or substituted by one or more $R^{2-3-2-2-2}$, or amino unsubstituted or substituted by one or more $R^{2-3-2-2-3}$;
$R^{2-3-2-2-1}$ is each independently selected from halogen or hydroxyl;
$R^{2-3-2-2-2}$ is each independently selected from halogen, deuterium, oxo or hydroxyl;
$R^{2-3-2-2-3}$ is each independently $C_1$-$C_6$ alkyl;
$R^{2-3-2-3}$ is each independently selected from hydrogen or

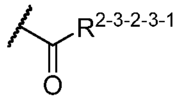
;

$R^{2-3-2-3-1}$ is each independently 6-10 membered aryl unsubstituted or substituted by one or more $R^{2-3-2-3-1-1}$;
$R^{2-3-2-3-1-1}$ is each independently $C_1$-$C_6$ alkoxy;
$R^{2-3-2-4}$ is each independently halogen;
$R^{2-3-2-5}$ is each independently selected from halogen, hydroxyl, oxo, $C_1$-$C_6$ alkoxy, or $C_1$-$C_6$ alkyl substituted by one or more $R^{2-3-2-5-1}$; $R^{2-3-2-5-1}$ is each independently halogen;
$R^{2-3-2-6}$ is each independently $C_1$-$C_6$ alkyl; $R^{2-4}$ is 4-10 membered heterocycloalkyl substituted by one or more $R^{2-4-1}$, 5-10 membered heteroaryl unsubstituted or substituted by one or more $R^{2-4-2}$, or 6-10 membered aryl unsubstituted or substituted by one or more $R^{2-4-3}$; the heteroatom in said 4-10 membered heterocycloalkyl is one or more selected from N, O and S, and the number of heteroatom(s) is 1, 2 or 3; the heteroatom in said 5-10 membered heteroaryl is one or more selected from N, O and S, and the number of heteroatom(s) is 1, 2 or 3;
$R^{2-4-1}$ is each independently selected from hydroxyl, halogen, $C_1$-$C_6$ alkyl,

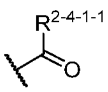 or 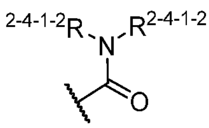
;

$R^{2-4-2}$ is each independently selected from hydroxyl, halogen or $C_1$-$C_6$ alkyl; $R^{2-4-3}$ is each independently selected from hydroxyl, halogen or $C_1$-$C_6$ alkyl;

$R^{2-5}$ is each independently selected from hydrogen, 4-10 membered heterocycloalkyl substituted by one or more $R^{2-5-1}$,

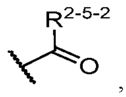,

6-10 membered aryl unsubstituted or substituted by one or more $R^{2-5-3}$, or $C_1$-$C_6$ alkyl; the heteroatom in said 4-10 membered heterocycloalkyl is one or more selected from N, O and S, and the number of heteroatom(s) is 1, 2 or 3;

$R^{2-5-1}$ is each independently selected from

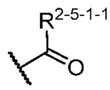 or 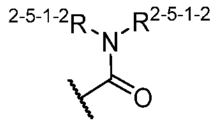;

$R^{2-5-1-1}$ is hydrogen or $C_1$-$C_6$ alkyl;

$R^{2-5-1-2}$ is each independently selected from hydrogen or $C_1$-$C_6$ alkyl;

$R^{2-5-2}$ is independently $C_1$-$C_6$ alkyl;

$R^{2-5-3}$ is each independently $C_1$-$C_6$ alkoxy;

$R^{2-6}$ is each independently selected from halogen, $C_1$-$C_6$ alkyl, 4-10 membered heterocycloalkyl unsubstituted or substituted by one or more $R^{2-6-1}$ or 5-10 membered heteroaryl unsubstituted or substituted by one or more $R^{2-6-2}$; the heteroatom in said 4-10 membered heterocycloalkyl is one or more selected from N, O and S, and the number of heteroatom(s) is 1, 2 or 3; the heteroatom in said 5-10 membered heteroaryl is one or more selected from N, O and S, and the number of heteroatom(s) is 1, 2 or 3;

$R^{2-6-1}$ is each independently selected from halogen, $C_1$-$C_6$ alkyl or

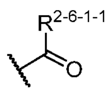;

$R^{2-6-1-1}$ is $C_1$-$C_6$ alkyl;

$R^{2-6-2}$ is each independently selected from halogen or $C_1$-$C_6$ alkyl;

$R^{2-7}$ is $C_1$-$C_6$ alkyl, 4-10 membered heterocycloalkyl unsubstituted or substituted by one or more $R^{2-7-1}$ or 5-10 membered heteroaryl unsubstituted or substituted by one or more $R^{2-7-2}$; the heteroatom in said 4-10 membered heterocycloalkyl is one or more selected from N, O and S, and the number of heteroatom(s) is 1, 2 or 3; the heteroatom in said 5-10 membered heteroaryl is one or more selected from N, O and S, and the number of heteroatom(s) is 1, 2 or 3;

$R^{2-7-1}$ is each independently selected from halogen, $C_1$-$C_6$ alkyl,

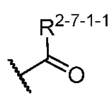 or 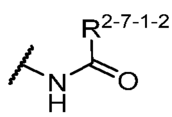;

$R^{2-7-1-1}$ is $C_1$-$C_6$ alkyl; $R^{2-7-1-2}$ is $C_1$-$C_6$ alkyl;

$R^{2-7-2}$ is each independently selected from halogen or $C_1$-$C_6$ alkyl;

$R^{2-8}$ is each independently $C_1$-$C_6$ alkyl, or two $R^{2-1}$, together with the atoms to which they are attached, form a 4-10 membered heterocycloalkyl unsubstituted or substituted by one or more $R^{2-8-1}$; the heteroatom in said 4-10 membered heterocycloalkyl is one or more selected from N, O and S, and the number of heteroatom(s) is 1, 2 or 3;

$R^{2-8-1}$ is each independently

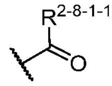
;

$R^{2-8-1-1}$ is hydrogen or $C_1$-$C_6$ alkyl;

$R^{2-9}$ is each independently 5-10 membered heteroaryl unsubstituted or substituted by one or more $R^{2-9-1}$; the heteroatom in said 5-10 membered heteroaryl is one or more selected from N, O and S, and the number of heteroatom(s) is 1, 2 or 3;

$R^{2-9-1}$ is each independently $C_1$-$C_6$ alkyl;

$R^{2-10}$ is each independently 5-10 membered heteroaryl unsubstituted or substituted by one or more $R^{2-10-1}$; the heteroatom in said 5-10 membered heteroaryl is one or more selected from N, O and S, and the number of heteroatom(s) is 1, 2 or 3;

$R^{2-10-1}$ is each independently $C_1$-$C_6$ alkyl;

$R^{2-11}$ is each independently selected from 5-10 membered heteroaryl unsubstituted or substituted by one or more $R^{2-11-1}$, or 3-6 membered heterocycloalkyl unsubstituted or substituted by one or more $R^{2-11-2}$; the heteroatom in said 5-10 membered heteroaryl is one or more selected from N, O and S, and the number of heteroatom(s) is 1, 2 or 3; the heteroatom in said 3-6 membered heterocycloalkyl is one or more selected from N, O and S, and the number of heteroatom(s) is 1, 2 or 3;

$R^{2-11-1}$ is each independently $C_1$-$C_6$ alkyl; $R^{2-11-2}$ is each independently $C_1$-$C_6$ alkyl;

(3) $R^3$ is 5-10 membered heteroaryl unsubstituted or substituted by one or more $R^{3-1}$ or 3-10 membered heterocycloalkyl unsubstituted or substituted by one or more $R^{3-2}$; the heteroatom in said 5-10 membered heteroaryl is one or more selected from N, O and S, and the number of heteroatom(s) is 1, 2 or 3; the heteroatom in said 3-10 membered heterocycloalkyl is one or more selected from N, O and S, and the number of heteroatom(s) is 1, 2 or 3;

$R^{3-1}$ is each independently selected from hydroxyl, halogen, $C_1$-$C_6$ alkyl substituted by one or more $R^{3-1-1}$ or

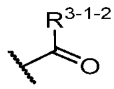
;

$R^{3-1-1}$ is each independently selected from or hydroxyl; $R^{3-1-2}$ is $C_1$-$C_6$ alkyl or $C_2$-$C_6$ alkenyl;

$R^{3-2}$ is each independently selected from hydroxyl, halogen, $C_1$-$C_6$ alkyl unsubstituted or substituted by one or more $R^{3-2-1}$ or

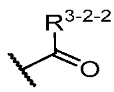
;

$R^{3-2-1}$ is each independently halogen; $R^{3-2-2}$ is $C_1$-$C_6$ alkyl or $C_2$-$C_4$ alkenyl;

(4) $R^4$ is deuterium, cyano, 5-10 membered heterocycloalkyl unsubstituted or substituted by one or more $R^{4-1}$ or $C_1$-$C_6$ alkyl substituted by one or more $R^{4-2}$; the heteroatom in said 5-10 membered heterocycloalkyl is one or more selected from N, O and S, and the number of heteroatom(s) is 1, 2 or 3;

$R^{4-1}$ is each independently selected from

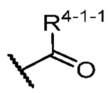
or
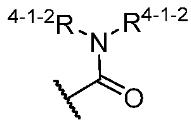
;

$R^{4-2}$ is each independently 5-10 membered heteroaryl unsubstituted or substituted by one or more $R^{4-2-1}$; the heteroatom in said 5-10 membered heteroaryl is one or more selected from N, O and S, and the number of heteroatom(s) is 1, 2 or 3; and

$R^{4-1-1}$ is $C_1$-$C_6$ alkyl; $R^{4-1-2}$ is each independently $C_1$-$C_6$ alkyl; $R^{4-2-1}$ is each independently $C_1$-$C_6$ alkyl.

**12.** The compound of formula I, the pharmaceutically acceptable salt thereof, or the isotopic compound thereof according to claim 2, wherein the compound of formula I satisfies one or more of the following conditions:

(1) $R^1$ is hydroxyl, $C_1$-$C_6$ alkyl unsubstituted or substituted by one or more $R^{1-1}$, $C_3$-$C_6$ cycloalkyl unsubstituted or substituted

by one or more $R^{1-2}$, 3-6 membered heterocycloalkyl unsubstituted or substituted by one or more $R^{1-3}$,

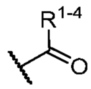

or amino unsubstituted or substituted by one or two $R^{1-5}$; the heteroatom in said 3-6 membered heterocycloalkyl is one or more selected from N, O and S, and the number of heteroatom(s) is 1, 2 or 3;
$R^{1-1}$ is each independently selected from deuterium, hydroxyl, cyano, halogen, 3-6 membered heterocycloalkyl, $C_3$-$C_6$ cycloalkyl unsubstituted or substituted by one or more $R^{1-1-1}$, $C_1$-$C_6$ alkyl unsubstituted or substituted by one or more $R^{1-1-2}$ or $C_1$-$C_6$ alkoxy unsubstituted or substituted by one or more $R^{1-1-3}$; the heteroatom in said 3-6 membered heterocycloalkyl is one or more selected from N, O and S, and the number of heteroatom(s) is 1, 2 or 3; $R^{1-1-1}$ is each independently selected from deuterium, hydroxyl, cyano or halogen; $R^{1-1-2}$ is each independently selected from deuterium, hydroxyl, cyano or halogen; $R^{1-1-3}$ is each independently selected from deuterium, hydroxyl, cyano or halogen;
$R^{1-2}$ is each independently selected from deuterium, hydroxyl, halogen, $C_2$-$C_6$ alkynyl unsubstituted or substituted by one or more $R^{1-2-2}$, $C_1$-$C_6$ alkoxy unsubstituted or substituted by one or more $R^{1-2-3}$, $C_2$-$C_6$ alkenyl unsubstituted or substituted by one or more $R^{1-2-4}$ or amino unsubstituted or substituted by one or two $R^{1-2-5}$; $R^{1-2-2}$ is each independently selected from halogen, hydroxyl or $C_1$-$C_6$ alkoxy unsubstituted or substituted by one or more $R^{1-2-2-1}$; $R^{1-2-2-1}$ is each independently halogen; $R^{1-2-3}$ is each independently selected from halogen, hydroxyl or $C_1$-$C_6$ alkoxy unsubstituted or substituted by one or more $R^{1-2-3-1}$; $R^{1-2-3-1}$ is each independently halogen; $R^{1-2-4}$ is each independently selected from halogen, hydroxyl or $C_1$-$C_6$ alkoxy unsubstituted or substituted by one or more $R^{1-2-4-1}$; $R^{1-2-4-1}$ is each independently halogen;
$R^{1-3}$ is each independently selected from cyano, deuterium, hydroxyl, amino, halogen, $C_1$-$C_6$ alkyl unsubstituted or substituted by one or more $R^{1-3-1}$ or $C_1$-$C_6$ alkoxy unsubstituted or substituted by one or more $R^{1-3-2}$; $R^{1-3-1}$ is each independently halogen; $R^{1-3-2}$ is each independently halogen;
$R^{1-4}$ is $C_1$-$C_6$ alkyl;
$R^{1-5}$ is each independently selected from $C_1$-$C_6$ alkyl,

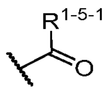

or $C_3$-$C_6$ cycloalkyl;
$R^{1-5-1}$ is $C_1$-$C_6$ alkyl or amino;
or, a 5-10 membered heterocycloalkyl unsubstituted or substituted by one or more $R^0$ is formed when the atom in R1 is attached to the atom in A; the heteroatom in said 5-10 membered heterocycloalkyl is one or more selected from N, O, P and S, and the number of heteroatom(s) is 3, 4 or 5; $R^0$ is each independently selected from $C_1$-$C_6$ alkyl or oxo;

(2) $R^2$ is hydrogen, 6-10 membered aryl unsubstituted or substituted by one or more $R^{2-1}$, 5-10 membered heteroaryl unsubstituted or substituted by one or more $R^{2-2}$, 4-10 membered heterocycloalkyl substituted by one or more $R^{2-3}$,

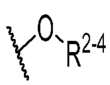

,

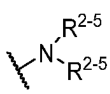

,

$C_1$-$C_6$ alkyl unsubstituted or substituted by one or more $R^{2-6}$,

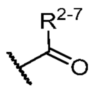

or

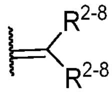

;

the heteroatom in said 5-10 membered heteroaryl is one or more selected from N, O and S, and the number of heteroatom(s) is 1, 2 or 3; the heteroatom in said 4-10 membered heterocycloalkyl is one or more selected from N, O and S, and the number of heteroatom(s) is 1, 2 or 3;

$R^{2-1}$ is each independently selected from cyano, halogen, $C_1$-$C_6$ alkyl unsubstituted or substituted by one or more $R^{2-1-1}$,

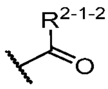

,

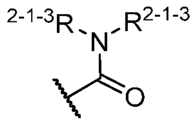

or $C_1$-$C_6$ alkoxy unsubstituted or substituted by one or more $R^{2-1-4}$;

$R^{2-1-1}$ is each independently selected from cyano or halogen; $R^{2-1-2}$ is hydrogen or $C_1$-$C_6$ alkyl; $R^{2-1-3}$ is each independently selected from hydrogen or $C_1$-$C_6$ alkyl; $R^{2-1-4}$ is each independently selected from halogen;

$R^{2-2}$ is each independently selected from cyano, halogen, $C_1$-$C_6$ alkyl unsubstituted or substituted by one or more $R^{2-2-1}$,

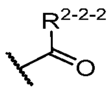

or

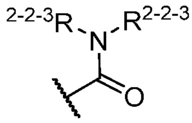

;

$R^{2-2-1}$ is each independently selected from cyano or halogen; $R^{2-2-2}$ is hydrogen or $C_1$-$C_6$ alkyl; $R^{2-2-3}$ is each independently selected from hydrogen or $C_1$-$C_6$ alkyl;

$R^{2-3}$ is each independently selected from $C_1$-$C_6$ alkyl substituted by one or more $R^{2-3-1}$,

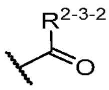

,

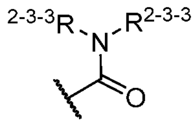

, 3-6 membered heterocycloalkyl unsubstituted or substituted by one or more $R^{2-3-4}$; the heteroatom in said 3-6 membered heterocycloalkyl is one or more selected from N, O and S, and the number of heteroatom(s) is 1, 2 or 3;

$R^{2-3-1}$ is each independently cyano;

$R^{2-3-2}$ is hydrogen; $R^{2-3-4}$ is each independently $C_1$-$C_6$ alkyl;

$R^{2-4}$ is 4-10 membered heterocycloalkyl substituted by one or more $R^{2-4-1}$, 5-10 membered heteroaryl unsubstituted or substituted by one or more $R^{2-4-2}$, 6-10 membered aryl unsubstituted or substituted by one or more $R^{2-4-3}$; the heteroatom in said 4-10 membered heterocycloalkyl is one or more selected from N, O and S, and the number of heteroatom(s) is 1, 2 or 3; the heteroatom in said 5-10 membered heteroaryl is one or more selected from N, O and S, and the number of heteroatom(s) is 1, 2 or 3;

$R^{2-4-1}$ is each independently selected from hydroxyl, halogen, $C_1$-$C_6$ alkyl,

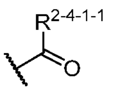

or

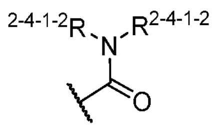

;

$R^{2-4-2}$ is each independently selected from hydroxyl, halogen or $C_1$-$C_6$ alkyl; $R^{2-4-3}$ is each independently

selected from hydroxyl, halogen or $C_1$-$C_6$ alkyl;

$R^{2-5}$ is each independently hydrogen, 4-10 membered heterocycloalkyl substituted by one or more $R^{2-5-1}$, or

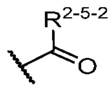
;

the heteroatom in said 4-10 membered heteroaryl is one or more selected from N, O and S, and the number of heteroatom(s) is 1, 2 or 3;

$R^{2-5-1}$ is each independently selected from

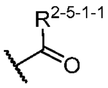
or
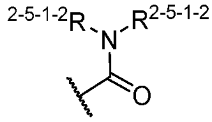
;

$R^{2-5-1-1}$ is hydrogen or $C_1$-$C_6$ alkyl;

$R^{2-5-1-2}$ is each independently selected from hydrogen or $C_1$-$C_6$ alkyl;

$R^{2-5-2}$ is independently $C_1$-$C_6$ alkyl;

$R^{2-6}$ is each independently selected from halogen, $C_1$-$C_6$ alkyl, 4-10 membered heterocycloalkyl unsubstituted or substituted by one or more $R^{2-6-1}$ or 5-10 membered heteroaryl unsubstituted or substituted by one or more $R^{2-6-2}$; the heteroatom in said 4-10 membered heterocycloalkyl is one or more selected from N, O and S, and the number of heteroatom(s) is 1, 2 or 3; the heteroatom in said 5-10 membered heteroaryl is one or more selected from N, O and S, and the number of heteroatom(s) is 1, 2 or 3;

$R^{2-6-1}$ is each independently selected from halogen, $C_1$-$C_6$ alkyl or

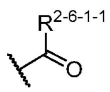
;

$R^{2-6-1-1}$ is $C_1$-$C_6$ alkyl;

$R^{2-6-2}$ is each independently selected from halogen or $C_1$-$C_6$ alkyl;

$R^{2-7}$ is $C_1$-$C_6$ alkyl, 4-10 membered heterocycloalkyl unsubstituted or substituted by one or more $R^{2-7-1}$ or 5-10 membered heteroaryl unsubstituted or substituted by one or more $R^{2-7-2}$; the heteroatom in said 4-10 membered heterocycloalkyl is one or more selected from N, O and S, and the number of heteroatom(s) is 1, 2 or 3; the heteroatom in said 5-10 membered heteroaryl is one or more selected from N, O and S, and the number of heteroatom(s) is 1, 2 or 3;

$R^{2-7-1}$ is each independently selected from halogen, $C_1$-$C_6$ alkyl,

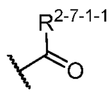
or
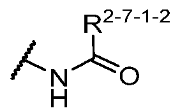
;

$R^{2-7-1-1}$ is $C_1$-$C_6$ alkyl; $R^{2-7-1-2}$ is $C_1$-$C_6$ alkyl;

$R^{2-7-2}$ is each independently selected from halogen or $C_1$-$C_6$ alkyl;

$R^{2-8}$ is each independently $C_1$-$C_6$ alkyl, or two $R^{2-1}$, together with the atoms to which they are attached, form a 4-10 membered heterocycloalkyl unsubstituted or substituted by one or more $R^{2-8-1}$; the heteroatom in said 4-10 membered heterocycloalkyl is one or more selected from N, O and S, and the number of heteroatom(s) is 1, 2 or 3;

$R^{2-8-1}$ is each independently

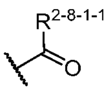
;

$R^{2-8-1-1}$ is hydrogen or $C_1$-$C_6$ alkyl;

(3) $R^3$ is 5-10 membered heteroaryl unsubstituted or substituted by one or more $R^{3-1}$ or 3-10 membered heterocycloalkyl unsubstituted or substituted by one or more $R^{3-2}$; the heteroatom in said 5-10 membered heteroaryl is one or more selected from N, O and S, and the number of heteroatom(s) is 1, 2 or 3; the heteroatom in said 3-10 membered heterocycloalkyl is one or more selected from N, O and S, and the number of heteroatom(s) is 1, 2 or 3;

$R^{3-1}$ is each independently selected from hydroxyl, halogen or

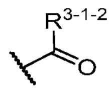
;

$R^{3-1-2}$ is $C_1$-$C_6$ alkyl or $C_2$-$C_6$ alkenyl;
$R^{3-2}$ is each independently selected from hydroxyl, halogen, $C_1$-$C_6$ alkyl unsubstituted or substituted by one or more $R^{3-2-1}$ or

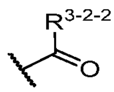
;

$R^{3-2-1}$ is each independently halogen; $R^{3-2-2}$ is $C_1$-$C_6$ alkyl or $C_2$-$C_4$ alkenyl;
$R^4$ is 5-10 membered heterocycloalkyl unsubstituted or substituted by one or more $R^{4-1}$ or $C_1$-$C_6$ alkyl unsubstituted or substituted by one or more $R^{4-2}$; the heteroatom in said 5-10 membered heterocycloalkyl is one or more selected from N, O and S, and the number of heteroatom(s) is 1, 2 or 3;
$R^{4-1}$ is each independently selected from

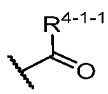
or
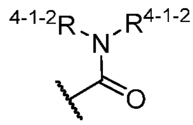
;

$R^{4-2}$ is each independently 5-10 membered heteroaryl unsubstituted or substituted by one or more $R^{4-2-1}$; the heteroatom in said 5-10 membered heteroaryl is one or more selected from N, O and S, and the number of heteroatom(s) is 1, 2 or 3; and
$R^{4-1-1}$ is $C_1$-$C_6$ alkyl; $R^{4-1-2}$ is each independently $C_1$-$C_6$ alkyl; $R^{4-2-1}$ is each independently $C_1$-$C_6$ alkyl.

**13.** The compound of formula I, the pharmaceutically acceptable salt thereof, or the isotopic compound thereof according to claim 1, wherein the compound of formula I satisfies one or more of the following conditions:

(1) in ring B, the heteroatom in said 5-membered-fused 6-membered heteroaromatic ring is N and/or O, and the number of heteroatom(s) can be 1, 2 or 3, for example,

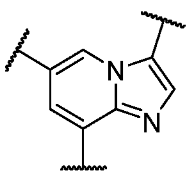
,
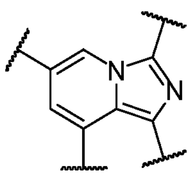
,
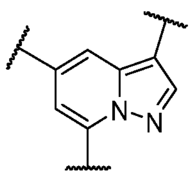
,

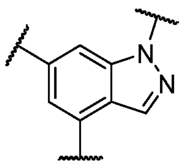
,
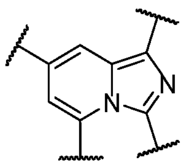
,
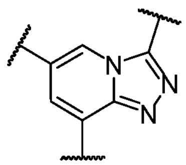
,
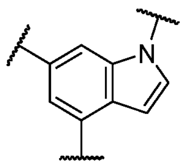
,
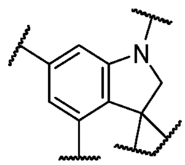
,

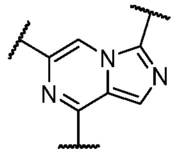, 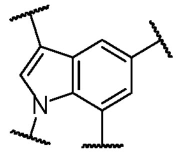 or 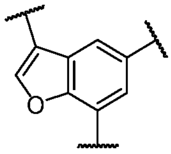;

(2) in $R^1$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, for example, methyl, ethyl, isopropyl or tert-butyl;

(3) in $R^1$, said $C_3$-$C_6$ cycloalkyl is cyclopropyl, cyclobutyl, cyclopentyl, cyclohexyl, bicyclopentane or

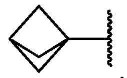,

for example, cyclopropyl, cyclobutane, bicyclopentane or

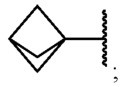;

(4) in $R^1$, the heteroatom in said 3-6 membered heterocycloalkyl is N, O or S, and the number of heteroatom(s) is 1 or 2; the heteroatom in said 3-6 membered heterocycloalkyl can be O or N, and the number of heteroatom can also be 1; azacyclobutane group, oxacyclobutane group, oxacyclo -azacyclobutanyl, oxacyclobutanyl, oxacyclopentanyl or oxacyclohexanyl is preferred, for example,

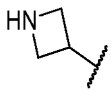, 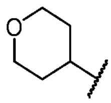, 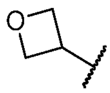 or 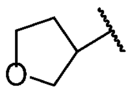;

(5) in each $R^{1-3}$, said $C_2$-$C_6$ alkynyl is

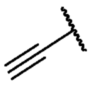, 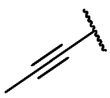, 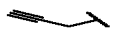, 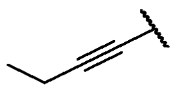, 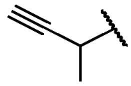 or

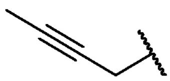,

for example,

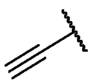;

(6) in $R^2$, said 5-10 membered heteroaryl is 5 or 6 membered heteroaryl, 5-membered-fused 5-membered heteroaryl, or 5-membered-fused 6-membered heteroaryl; the heteroatom in said 5-10 membered heteroaryl is one or more selected from N, O and S, and the number of heteroatom(s) is 1, 2 or 3; the heteroatom in said 5 membered heteroaryl can be N, the number of heteroatoms is 2, and pyrazolyl is preferred, for example,

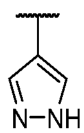,

the heteroatom in said 6 membered heteroaryl can be N, the number of heteroatom(s) is 1, and pyridyl is

preferred, for example,

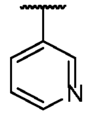;

the heteroatom in said 5-membered-fused 5-membered heteroaryl can be N, the number of heteroatoms is 3, dyhydropyrrolopyrazolyl is preferred, for example,

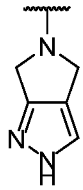;

the heteroatom in said 5-membered-fused 6-membered heteroaryl can be N and/or O, the number of heteroatom(s) is 1, 2 or 3, and benzofuryl, dihydroimidazopyrazinyl or benzoimidazolyl is preferred, for example,

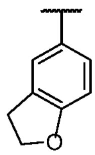, 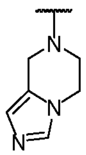 or 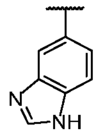;

(7) in $R^2$, said 4-12 membered heterocycloalkyl is one or more selected from a monocyclic ring, a bridged ring, a fused ring and a spiro ring; the heteroatom in said 4-12 membered heterocycloalkyl can be N and/or O, and the number of heteroatom(s) can be 1, 2 or 3; the heteroatom in said monocyclic ring can be 4-6 membered heterocycloalkyl; said fused ring can be 5-membered-fused 6-membered heterocycloalkyl, 4-membered-fused 6-membered heterocycloalkyl or 5-membered-fused 5-membered heterocycloalkyl; said spiro ring can be 4 membered-spiro 6-membered ring, 4 membered-spiro 5-membered ring, 4 membered-spiro 4-membered ring, 5 membered-spiro 6-membered ring or 3 membered-spiro 6-membered ring; said bridged ring can be bicycloheterooctane or bicycloheteroheptane;
(8) in $R^2$, said 4-12 membered heterocycloalkyl is saturated heterocycloalkyl, or heterocycloalkyl containing 1-2 double bond(s);
(9) in $R^2$, said $C_3$-$C_6$ cycloalkyl is cyclopropyl, cyclobutyl, cyclopentyl or cyclohexyl, for example, cyclopentyl;
(10) in $R^2$, said $C_3$-$C_6$ cycloalkenyl is cycloalkenyl containing 1 double bond, and can be 5 membered cycloalkenyl, for example,

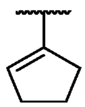;

(11) in each $R^{2-2}$, said $C_1$-$C_6$ alkoxy is methoxy, ethoxy, n-propoxy, isopropoxy, n-butoxy, isobutoxy, pr-butoxy, sec-butoxy or tert-butoxy, for example, methoxy;
(12) in each $R^{2-2-4}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, for example, methyl;
(13) in each $R^{2-2-4}$, said $C_3$-$C_6$ cycloalkyl is cyclopropyl, cyclobutyl, cyclopentyl or cyclohexyl, for example, cyclopropyl;
(14) in each $R^{2-3}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, or can be methyl, ethyl, isopropyl or tert-butyl;
(15) in each $R^{2-3}$, said $C_1$-$C_6$ alkoxy is methoxy, ethoxy, n-propoxy, isopropoxy, n-butoxy, isobutoxy, pr-butoxy, sec-butoxy or tert-butoxy, or can be methoxy, ethoxy or isopropoxy;
(16) in each $R^{2-3}$, said $C_3$-$C_6$ cycloalkyl is cyclopropyl, cyclobutyl, cyclopentyl or cyclohexyl, for example, cyclopropyl;

(17) in each $R^{2-3}$, said 5-10 membered heteroaryl is 5 membered heteroaryl or 6 membered heteroaryl; the heteroatom in said 5-10 membered heteroaryl can be N and/or O, and the number of heteroatom(s) is 1, 2 or 3; the heteroatom in said 5 membered heteroaryl can be N and/or O, the number of heteroatoms can be 2 or 3, and triazolyl, oxadiazolyl, or imidazolyl is preferred, for example,

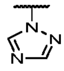, 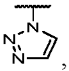, 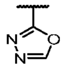 or 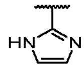;

the heteroatom in said 6 membered heteroaryl can be N, the number of heteroatom(s) is 1 or 2, and pyridyl is preferred, for example,

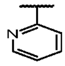;

(18) in each $R^{2-3-1}$, said $C_3$-$C_6$ cycloalkyl is cyclopropyl, cyclobutyl, cyclopentyl or cyclohexyl, for example, cyclopropyl;
(19) in $R^{2-3-2}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, for example, methyl, ethyl, isopropyl or tert-butyl;
(20) in $R^{2-3-2}$, said $C_3$-$C_6$ cycloalkyl is cyclopropyl, cyclobutyl, cyclopentyl or cyclohexyl, for example, cyclopropyl or cyclobutyl;
(21) in $R^{2-3-2}$, said $C_1$-$C_6$ alkoxy is methoxy, ethoxy, n-propoxy, isopropoxy, n-butoxy, isobutoxy, pr-butoxy, sec-butoxy or tert-butoxy, for example, isopropoxy;
(22) in $R^{2-3-2}$, the heteroatom in said 3-6 membered heterocycloalkyl is one or more selected from N, O and S, and the number of heteroatom(s) is 1 or 2; oxacyclobutanyl, azacyclobutanyl, oxacyclopentanyl, azacyclopentanyl, oxacyclohexanyl, azacyclohexanyl, pyrazinyl, thiazolidinyl or oxabicyclohexanyl is preferred, for example,

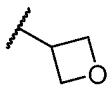, 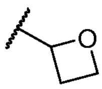,

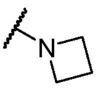, 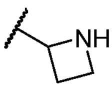, 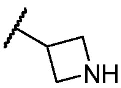, 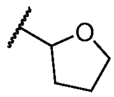, 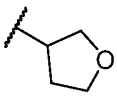, 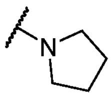, 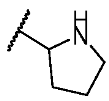, 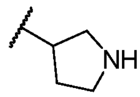,

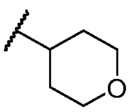, 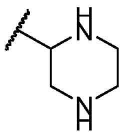, 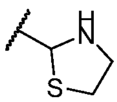 or 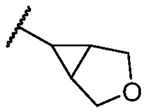;

(23) in $R^{2-3-2}$, said 5-10 membered heteroaryl is 5 or 6 membered heteroaryl or 5-membered-fused 6-membered heteroaryl; the heteroatom in said 5-10 membered heteroaryl can be N and/or O, and the number of heteroatom(s) can be 1 or 2; the heteroatom in said 5 membered heteroaryl can be N and/or O, and the number of heteroatom(s) is 2; the heteroatom in said 6 membered heteroaryl can be N, and the number of heteroatom(s) is 1; the heteroatom in said 5-membered-fused 6-membered heteroaryl can be O, and the number of heteroatoms is 2;
(24) in each $R^{2-3-3}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, for example, methyl or ethyl;
(25) in each $R^{2-3-6}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, for example, methyl;
(26) in each $R^{2-3-7}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, for example, methyl;
(27) in each $R^{2-3-8}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, for example, methyl or isopropyl;
(28) in each $R^{2-3-8}$, said $C_3$-$C_6$ cycloalkyl is cyclopropyl, cyclobutyl, cyclopentyl or cyclohexyl, for example, cyclopropyl;
(29) in each $R^{2-3-9}$, said $C_1$-$C_6$ alkyl is $C_1$-$C_4$ alkyl, and can be methyl, ethyl, n-propyl, isopropyl, n-butyl, isobutyl,

pr-butyl, sec-butyl or tert-butyl, for example, methyl;
(30) in each $R^{2-3-1-2}$, said $C_1$-$C_6$ alkoxy is methoxy, ethoxy, n-propoxy, isopropoxy, n-butoxy, isobutoxy, pr-butoxy, sec-butoxy or tert-butoxy, for example, methoxy;
(31) in each $R^{2-3-1-3}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, for example, methyl;
(32) in each $R^{2-3-2-1}$, said $C_1$-$C_6$ alkoxy is methoxy, ethoxy, n-propoxy, isopropoxy, n-butoxy, isobutoxy, pr-butoxy, sec-butoxy or tert-butoxy, for example, methoxy;
(33) in each $R^{2-3-2-1}$, said 5-10 membered heteroaryl is 5 membered heteroaryl; the heteroatom in said 5 membered heteroaryl can be N, and the number of heteroatoms is 3;
(34) in each $R^{2-3-2-1-1}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, for example, methyl;
(35) in each $R^{2-3-2-1-1}$, said 6-10 membered aryl is phenyl or naphthyl, for example, phenyl;
(36) in each $R^{2-3-2-1-2}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, for example, methyl;
(37) in each $R^{2-3-2-1-3}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, for example, methyl;
(38) in each $R^{2-3-2-2}$, said halogen is fluorine, chlorine, bromine or iodine, for example, fluorine;
(39) in each $R^{2-3-2-2}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, for example, methyl;
(40) in each $R^{2-3-2-2}$, said $C_1$-$C_6$ alkoxy is methoxy, ethoxy, n-propoxy, isopropoxy, n-butoxy, isobutoxy, pr-butoxy, sec-butoxy or tert-butoxy, for example, methoxy, ethoxy or n-propoxy;
(41) in each $R^{2-3-2-2-1}$, said halogen is fluorine, chlorine, bromine or iodine, for example, fluorine;
(42) in each $R^{2-3-2-2-2}$, said halogen is fluorine, chlorine, bromine or iodine, for example, fluorine;
(43) in each $R^{2-3-2-2-3}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, for example, methyl;
(44) in each $R^{2-3-2-3-1}$, said 6-10 membered aryl is phenyl or naphthyl, for example, phenyl;
(45) in each $R^{2-3-2-3-1-1}$, said $C_1$-$C_6$ alkoxy is methoxy, ethoxy, n-propoxy, isopropoxy, n-butoxy, isobutoxy, pr-butoxy, sec-butoxy or tert-butoxy, for example, methoxy;
(46) in each $R^{2-3-2-4}$, said halogen is fluorine, chlorine, bromine or iodine, for example, fluorine;
(47) in each $R^{2-3-2-5}$, said halogen is fluorine, chlorine, bromine or iodine, for example, fluorine;
(48) in each $R^{2-3-2-5}$, said $C_1$-$C_6$ alkoxy is methoxy, ethoxy, n-propoxy, isopropoxy, n-butoxy, isobutoxy, pr-butoxy, sec-butoxy or tert-butoxy, for example, methoxy;
(49) in each $R^{2-3-2-5}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, for example, methyl, ethyl, n-propyl or isopropyl;
(50) in each $R^{2-3-2-5-1}$, said halogen is fluorine, chlorine, bromine or iodine, for example, fluorine;
(51) in each $R^{2-3-2-6}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, for example, methyl;
(52) in each $R^{2-3-3-1-1}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, for example, methyl;
(53) in each $R^{2-5}$, said 6-10 membered aryl is phenyl or naphthyl, for example, phenyl;
(54) in each $R^{2-5}$, said $C_1$-$C_6$ alkyl is methyl, ethyl, n-propyl, isopropyl, n-butyl, isobutyl, pr-butyl, sec-butyl or tert-butyl, for example, methyl;
(55) in each $R^{2-5-1-1}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, for example, methyl or isopropyl;
(56) in each $R^{2-5-3}$, said $C_1$-$C_6$ alkoxy is methoxy, ethoxy, n-propoxy, isopropoxy, n-butoxy, isobutoxy, pr-butoxy, sec-butoxy or tert-butoxy, for example, methoxy;
(57) in each $R^{2-9}$, said 5-10 membered heteroaryl is 5 membered heteroaryl; the heteroatom in said 5-10 membered heteroaryl can be N and/or S, and the number of heteroatom(s) can be 1 or 2; the heteroatom in said 5 membered heteroaryl can be N, and the number of heteroatoms is 2; pyrazolyl is preferred, for example,

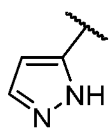;

(58) in each $R^{2-10}$, said 5-10 membered heteroaryl is 5 membered heteroaryl; the heteroatom in said 5-10 membered heteroaryl can be N and/or S, and the number of heteroatom(s) can be 1 or 2; the heteroatom in said 5

membered heteroaryl can be N, and the number of heteroatoms is 2; pyrazolyl is preferred, for example,

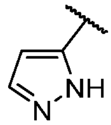

;

(59) in each $R^{2-11}$, said 5-10 membered heteroaryl is 5 membered heteroaryl; the heteroatom in said 5 membered heteroaryl can be N and/or S, and the number of heteroatoms is 2; thiazolyl or imidazolyl is preferred, for example,

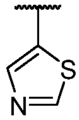

or

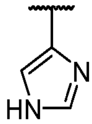

;

(60) in each $R^{2-11}$, the heteroatom in said 3-6 membered heterocycloalkyl is O, and the number of heteroatom(s) can be 1; oxacyclopentanyl is preferred, for example,

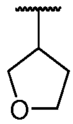

;

(61) in each $R^{2-11-1}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, for example, methyl;

(62) in each $R^{2-11-2}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, for example, methyl;

(63) in $R^3$, said 5-10 membered heteroaryl is 5 membered heteroaryl, 5-membered-fused 5-membered heteroaryl or 5-membered-fused 6-membered heteroaryl; the heteroatom in said 5-10 membered heteroaryl can be N and/or S, and the number of heteroatom(s) is 1, 2 or 3; the heteroatom in said 5 membered heteroaryl can be N and/or S, and the number of heteroatom(s) is 2 or 3; the heteroatom in said 5-membered-fused 6-membered heteroaryl can be N and/or O, and the number of heteroatoms is 2, or the number of heteroatom(s) is 1, 2 or 3; the heteroatom in said 5-membered-fused 5-membered heteroaryl can be N and/or O, and the number of heteroatoms is 2, or the number of heteroatom(s) is 1, 2 or 3;

(64) in $R^{3-2-2}$, said $C_1$-$C_6$ alkyl is $C_1$-$C_4$ alkyl, and can be methyl, ethyl, n-propyl, isopropyl, n-butyl, isobutyl, pr-butyl, sec-butyl or tert-butyl, for example, methyl or ethyl;

(65) in $R^4$, said halogen is fluorine, chlorine, bromine or iodine, for example, fluorine or chlorine; and

(66) in $R^4$, said 4-10 membered heterocycloalkyl is a monocyclic or spiro ring; said monocyclic ring can be 6 membered heterocycloalkyl; said spiro ring can be 4 membered-spiro 6-membered ring, 4 membered-spiro 5-membered ring or 4 membered-spiro 4-membered ring; the heteroatom in said 4-10 membered heterocycloalkyl can be N, and the number of heteroatom(s) is 1 or 2.

**14.** The compound of formula I, the pharmaceutically acceptable salt thereof, or the isotopic compound thereof according to claim 13, wherein the compound of formula I satisfies one or more of the following conditions:

(1) said 4-12 membered heterocycloalkyl is 3 rings-spiro-6 membered-fused-6 membered heterocycloalkyl, preferably, hexahydro-spiro-cyclopropan-pyrazinopyrazinyl, for example,

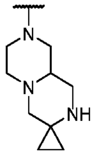 ;

(2) said bicycloheterooctane is bicyclo[2.2.2]heterooctane, for example,

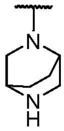 ;

(3) said bicycloheteroheptane is bicyclo[3.1.1]heteroheptane, for example,

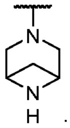 ;

(4) said 4-6 membered heterocycloalkyl is azacyclohexanyl, tetrahydropyridyl, piperazinyl, azacyclopentanyl or morpholinyl, for example,

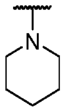 , 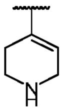 , 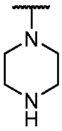 , 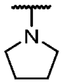 , 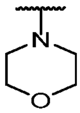 or 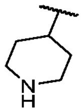 ;

(5) said 5-membered-fused 6-membered heterocycloalkyl is tetrahydrofuranopyridyl, hexahydropyrrolopyrazinyl, hexahydropyrazinooxazinyl or hexahydroimidazopyrazinyl, for example,

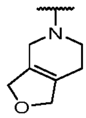 , 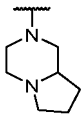 , 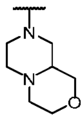 or 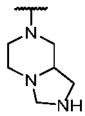 ;

(6) said 5-membered-fused 5-membered heterocycloalkyl is tetrahydrofuranopyrrolyl, for example,

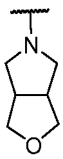 ;

(7) said 4 membered-spiro 6-membered ring is oxaaza-spiro-nonanyl, dioxaaza-spiro-nonanyl or aza-spiro-nonanyl, for example,

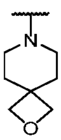 , 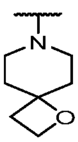 , 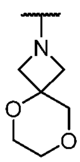 or 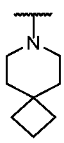 ;

(8) said 3 membered-spiro 6-membered ring is diaza-spiro-octanyl, for example,

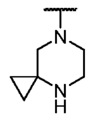;

and

(9) said 5 membered-spiro 6-membered ring is diaza-spiro-decanyl, for example,

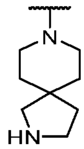

**15.** The compound of formula I, the pharmaceutically acceptable salt thereof, or the isotopic compound thereof according to claim 1, wherein the compound of formula I satisfies one or more of the following conditions:

(1) $R^1$ is $C_3$-$C_6$ cycloalkyl substituted by one or more $R^{1-2}$, or 3-6 membered heterocycloalkyl unsubstituted or substituted by one or more $R^{1-3}$; the heteroatom in said 3-6 membered heterocycloalkyl is one or more selected from N, O and S, and the number of heteroatom(s) is 1, 2 or 3;
(2) $R^{1-2}$ is each independently $C_2$-$C_6$ alkynyl;
(3) $R^{13}$ is each independently selected from cyano, $C_1$-$C_6$ alkyl unsubstituted or substituted by one or more $R^{1-3-1}$, or $C_2$-$C_6$ alkynyl;
(4) $R^{1-3-1}$ is each independently halogen;
(5) $R^2$ is 4-12 membered heterocycloalkyl substituted by one or more $R^{2-3}$; the heteroatom in said 4-12 membered heterocycloalkyl is one or more selected from N, O, and S, and the number of heteroatom(s) is 1, 2 or 3;
(6) $R^{2-3}$ is each independently selected from

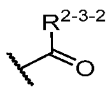,

or 5-10 membered heteroaryl; the heteroatom in said 5-10 membered heteroaryl is N and/or O, and the number of heteroatoms is 2 or 3;
(7) $R^{2-3-2}$ is $C_1$-$C_6$ alkyl substituted by one or more $R^{2-3-2-1}$, $C_3$-$C_6$ cycloalkyl unsubstituted or substituted by one or more $R^{2-3-2-2}$,

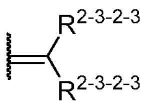,

3-6 membered heterocycloalkyl unsubstituted or substituted by one or more $R^{2-3-2-5}$, or 5-10 membered heteroaryl; the heteroatom in said 3-6 membered heterocycloalkyl is one or more selected from N, O and S, and the number of heteroatom(s) is 1, 2 or 3; the heteroatom in said 5-10 membered heteroaryl is selected from N and/or O, and the number of heteroatom(s) is 1 or 2;
(8) $R^{2-3-2-1}$ is each independently selected from hydroxyl, or

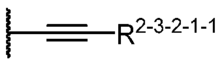;

(9) $R^{2-3-2-1-1}$ is each independently $C_1$-$C_6$ alkyl;
(10) $R^{2-3-2-2}$ is each independently selected from halogen, hydroxyl, $C_1$-$C_6$ alkyl, $C_1$-$C_6$ alkoxy unsubstituted or substituted by one or more $R^{2-3-2-2-2}$, or amino;
(11) $R^{2-3-2-2-2}$ is each independently deuterium;
(12) $R^{2-3-2-3}$ is each independently selected from hydrogen or

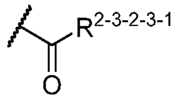

;

(13) $R^{2-3-2-3-1}$ is each independently 6-10 membered aryl unsubstituted or substituted by one or more $R^{2-3-2-3-1-1}$; $R^{2-3-2-3-1-1}$ is each independently $C_1$-$C_6$ alkoxy;
(14) $R^{2-3-2-5}$ is each independently selected from halogen, $C_1$-$C_6$ alkoxy, or $C_1$-$C_6$ alkyl unsubstituted or substituted by one or more $R^{2-3-2-5-1}$;
(15) $R^{2-3-2-5-1}$ is each independently halogen;
(16) $R^3$ is unsubstituted 5-6 membered heteroaryl; the heteroatom in said 5-6 membered heteroaryl is N and/or S, and the number of heteroatoms is 3;
(17) $R^3$ is

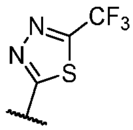

;

(18) $R^4$ is deuterium, cyano, 5-10 membered heterocycloalkyl unsubstituted or substituted by one or more $R^{4-1}$s; a heteroatom in the 5-10 membered heterocycloalkyl is one or more selected from N, O, and S, and a heteroatom number is 1 or 2; and
(19) $R^{4-1}$ is each independently

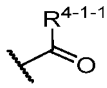

;

and $R^{4-1-1}$ is $C_1$-$C_6$ alkyl.

**16.** The compound of formula I, the pharmaceutically acceptable salt thereof or the isotopic compound thereof according to claim 1, wherein the compound of formula I is defined as in a scheme 4, a scheme 5, a scheme 6, a scheme 7, a scheme 8, a scheme 9, a scheme 10 or a scheme 11:

in a solution 4,
$R^1$ is $C_3$-$C_6$ cycloalkyl unsubstituted or substituted by one or more $R^{1-2}$;
$R^{1-2}$ is each independently selected from cyano, $C_1$-$C_6$ alkyl unsubstituted or substituted by one or more $R^{1-2-1}$, or $C_2$-$C_6$ alkynyl;
$R^{1-2-1}$ is each independently selected from deuterium, halogen, or hydroxyl;
$R^2$ is 4-10 membered heterocycloalkyl unsubstituted or substituted by one or more $R^{2-3}$; the heteroatom in said 4-10 membered heterocycloalkyl is N and/or O, and the number of heteroatom(s) is 1, 2 or 3;
$R^{2-3}$ is each independently selected from

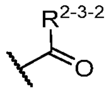

,

or 5-10 membered heteroaryl; the heteroatom in said 5-10 membered heteroaryl is N and/or O, and the number of heteroatoms is 2 or 3;
$R^{2-3-2}$ is $C_1$-$C_6$ alkyl substituted by one or more $R^{2-3-2-1}$, $C_3$-$C_6$ cycloalkyl unsubstituted or substituted by one or more $R^{2-3-2-2}$,

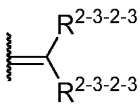

,

3-6 membered heterocycloalkyl unsubstituted or substituted by one or more $R^{2-3-2-5}$, or 5-10 membered

heteroaryl; the heteroatom in said 3-6 membered heterocycloalkyl is one or more selected from N, O and S, and the number of heteroatom(s) is 1, 2 or 3; the heteroatom in said 5-10 membered heteroaryl is N and/or O, and the number of heteroatom(s) is 1 or 2;

$R^{2-3-2-1}$ is each independently selected from hydroxyl, or

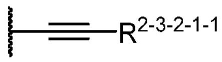
;

$R^{2-3-2-1-1}$ is each independently $C_1$-$C_6$ alkyl;

$R^{2-3-2-2}$ is each independently selected from halogen, hydroxyl, $C_1$-$C_6$ alkyl, $C_1$-$C_6$ alkoxy unsubstituted or substituted by one or more $R^{2-3-2-2-2}$, or amino;

$R^{2-3-2-2-2}$ is each independently deuterium;

$R^{2-3-2-3}$ is each independently selected from hydrogen or

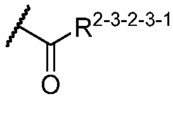
;

$R^{2-3-2-3-1}$ is each independently 6-10 membered aryl unsubstituted or substituted by one or more $R^{2-3-2-3-1-1}$;

$R^{2-3-2-3-1-1}$ is each independently $C_1$-$C_6$ alkoxy;

$R^{2-3-2-5}$ is each independently selected from halogen, $C_1$-$C_6$ alkoxy, or $C_1$-$C_6$ alkyl unsubstituted or substituted by one or more $R^{2-3-2-5-1}$;

$R^{2-3-2-5-1}$ is each independently halogen;

$R^3$ is 5-6 membered heteroaryl unsubstituted or substituted by one or more $R^{3-1}$; the heteroatom in said 5-6 membered heteroaryl is N and/or S, and the number of heteroatoms is 3;

$R^{3-1}$ is each independently $C_1$-$C_6$ alkyl substituted by one or more $R^{3-1-1}$;

$R^{3-1-1}$ is each independently halogen;

$R^4$ is hydrogen, cyano, halogen, $C_1$-$C_6$ alkyl, or 5-10 membered heterocycloalkyl unsubstituted or substituted by one or more $R^{4-1}$; the heteroatom in said 5-10 membered heterocycloalkyl is one or more selected from N, O and S, and the number of heteroatom(s) is 1 or 2;

$R^{4-1}$ is each independently

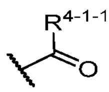
;

$R^{4-1-1}$ is $C_1$-$C_6$ alkyl;

in the scheme 5,

$R^1$ is $C_3$-$C_6$ cycloalkyl unsubstituted or substituted by one or more $R^{1-2}$;

$R^{1-2}$ is each independently selected from cyano, or $C_1$-$C_6$ alkyl unsubstituted or substituted by one or more $R^{1-2-1}$;

$R^{1-2-1}$ is each independently selected from deuterium or halogen;

$R^2$ is 4-10 membered heterocycloalkyl unsubstituted or substituted by one or more $R^{2-3}$; the heteroatom in said 4-10 membered heterocycloalkyl is N and/or O, and the number of heteroatom(s) is 1, 2 or 3;

$R^{2-3}$ is each independently

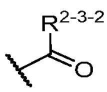
;

$R^{2-3-2}$ is $C_3$-$C_6$ cycloalkyl substituted by one or more $R^{2-3-2-2}$, or 3-6 membered heterocycloalkyl substituted by one or more $R^{2-3-2-5}$; the heteroatom in said 3-6 membered heterocycloalkyl is one or more selected from N, O and S, and the number of heteroatom(s) is 1, 2 or 3; the heteroatom in said 5-10 membered heteroaryl is N and/or O, and the number of heteroatom(s) is 1 or 2;

$R^{2-3-2-2}$ is each independently selected from $C_1$-$C_6$ alkyl, or $C_1$-$C_6$ alkoxy unsubstituted or substituted by one or more $R^{2-3-2-2-2}$;

$R^{2-3-2-2-2}$ is each independently deuterium;

$R^{2-3-2-5}$ is each independently $C_1$-$C_6$ alkyl;
$R^3$ is 5-6 membered heteroaryl unsubstituted or substituted by one or more $R^{3-1}$; the heteroatom in said 5-6 membered heteroaryl is N and/or S, and the number of heteroatoms is 3;
$R^{3-1}$ is each independently $C_1$-$C_6$ alkyl substituted by one or more $R^{3-1-1}$;
$R^{3-1-1}$ is each independently halogen;
$R^4$ is hydrogen, cyano, or $C_1$-$C_6$ alkyl;
in the scheme 6,
$R^1$ is 3-6 membered heterocycloalkyl unsubstituted or substituted by one or more $R^{1-3}$; the heteroatom in said 3-6 membered heterocycloalkyl is O, and the number of heteroatom(s) is 1;
$R^{1-3}$ is each independently selected from cyano, $C_1$-$C_6$ alkyl unsubstituted or substituted by one or more $R^{1-3-1}$, or $C_2$-$C_6$ alkynyl;
$R^{1-3-1}$ is each independently selected from halogen or deuterium;
$R^2$ is 4-12 membered heterocycloalkyl unsubstituted or substituted by one or more $R^{2-3}$; the heteroatom in said 4-12 membered heterocycloalkyl is one or more selected from N, O, and S, and the number of heteroatom(s) is 1, 2 or 3;
$R^{2-3}$ is each independently $C_1$-$C_6$ alkyl unsubstituted or substituted by one or more $R^{2-3-1}$,

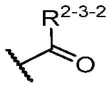 , or 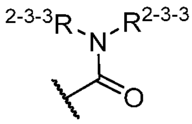 ;

$R^{2-3-1}$ is each independently selected from hydroxyl, halogen, $C_1$-$C_6$ alkoxy unsubstituted or substituted by one or more $R^{2-3-1-1}$, or amino unsubstituted or substituted by one or more $R^{2-3-1-3}$;
$R^{2-3-1-1}$ is each independently deuterium;
$R^{2-3-1-3}$ is each independently $C_1$-$C_6$ alkyl;
$R^{2-3-2}$ is $C_1$-$C_6$ alkyl, or $C_3$-$C_6$ cycloalkyl unsubstituted or substituted by one or more $R^{2-3-2-2}$;
$R^{2-3-2-2}$ is each independently selected from $C_1$-$C_6$ alkyl, or $C_1$-$C_6$ alkoxy unsubstituted or substituted by one or more $R^{2-3-2-2-2}$;
$R^{2-3-2-2-2}$ is each independently deuterium;
$R^{2-3-3}$ is each independently selected from hydrogen, or $C_1$-$C_6$ alkyl unsubstituted or substituted by one or more $R^{2-3-3-1}$;
$R^{2-3-3-1}$ is each independently amino unsubstituted or substituted by one or more $R^{2-3-3-1-1}$;
$R^{2-3-3-1-1}$ is each independently $C_1$-$C_6$ alkyl;
$R^3$ is 5-6 membered heteroaryl unsubstituted or substituted by one or more $R^{3-1}$; the heteroatom in said 5-6 membered heteroaryl is N and/or S, and the number of heteroatoms is 3;
$R^{3-1}$ is each independently $C_1$-$C_6$ alkyl substituted by one or more $R^{3-1-1}$;
$R^{3-1-1}$ is each independently halogen;
$R^4$ is hydrogen, deuterium, halogen, or $C_1$-$C_6$ alkyl;
in the scheme 7,
$R^1$ is 3-6 membered heterocycloalkyl unsubstituted or substituted by one or more $R^{1-3}$; the heteroatom in said 3-6 membered heterocycloalkyl is O, and the number of heteroatom(s) is 1;
$R^{1-3}$ is each independently selected from cyano, $C_1$-$C_6$ alkyl unsubstituted or substituted by one or more $R^{1-3-1}$, or $C_2$-$C_6$ alkynyl;
$R^{1-3-1}$ is each independently halogen;
$R^2$ is 4-12 membered heterocycloalkyl unsubstituted or substituted by one or more $R^{2-3}$; the heteroatom in said 4-12 membered heterocycloalkyl is one or more selected from N, O, and S, and the number of heteroatom(s) is 1, 2 or 3;
$R^{2-3}$ is each independently $C_1$-$C_6$ alkyl unsubstituted or substituted by one or more $R^{2-3-1}$,

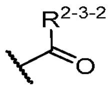 , or 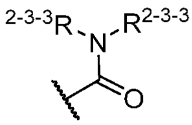 ;

$R^{2-3-1}$ is each independently selected from hydroxyl, or $C_1$-$C_6$ alkoxy unsubstituted or substituted by one or more $R^{2-3-1-1}$;

$R^{2-3-1-1}$ is each independently deuterium;

$R^{2-3-2}$ is $C_1$-$C_6$ alkyl, or $C_3$-$C_6$ cycloalkyl unsubstituted or substituted by one or more $R^{2-3-2-2}$;

$R^{2-3-2-2}$ is each independently selected from $C_1$-$C_6$ alkyl, or $C_1$-$C_6$ alkoxy unsubstituted or substituted by one or more $R^{2-3-2-2-2}$;

$R^{2-3-2-2-2}$ is each independently deuterium;

$R^{2-3-3}$ is each independently selected from hydrogen, or $C_1$-$C_6$ alkyl unsubstituted or substituted by one or more $R^{2-3-3-1}$;

$R^{2-3-3-1}$ is each independently amino unsubstituted or substituted by one or more $R^{2-3-3-1-1}$;

$R^{2-3-3-1-1}$ is each independently $C_1$-$C_6$ alkyl;

$R^3$ is 5-6 membered heteroaryl unsubstituted or substituted by one or more $R^{3-1}$; the heteroatom in said 5-6 membered heteroaryl is N and/or S, and the number of heteroatoms is 3;

$R^{3-1}$ is each independently $C_1$-$C_6$ alkyl substituted by one or more $R^{3-1-1}$;

$R^{3-1-1}$ is each independently halogen;

$R^4$ is hydrogen, deuterium, halogen, or $C_1$-$C_6$ alkyl;

in a solution 8,

$R^1$ is $C_3$-$C_6$ cycloalkyl unsubstituted or substituted by one or more $R^{1-2}$;

$R^{1-2}$ is each independently selected from cyano, $C_1$-$C_6$ alkyl unsubstituted or substituted by one or more $R^{1-2-1}$, or $C_2$-$C_6$ alkynyl;

$R^{1-2-1}$ is each independently selected from deuterium, halogen, or hydroxyl;

$R^2$ is 4-10 membered heterocycloalkyl substituted by one or more $R^{2-3}$; the heteroatom in said 4-10 membered heterocycloalkyl is N and/or O, and the number of heteroatom(s) is 1, 2 or 3; said 4-10 membered heterocycloalkyl is a 4-6 membered saturated monocyclic ring, a 7-10 membered saturated spiro ring or a 7-10 membered saturated bridged ring;

$R^{2-3}$ is each independently selected from

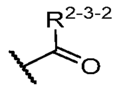
,

or 5-10 membered heteroaryl; the heteroatom in said 5-10 membered heteroaryl is N and/or O, and the number of heteroatoms is 2 or 3;

$R^{2-3-2}$ is $C_1$-$C_6$ alkyl substituted by one or more $R^{2-3-2-1}$, $C_3$-$C_6$ cycloalkyl unsubstituted or substituted by one or more $R^{2-3-2-2}$,

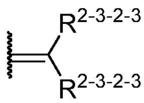
,

3-6 membered heterocycloalkyl unsubstituted or substituted by one or more $R^{2-3-2-5}$, or 5-10 membered heteroaryl; the heteroatom in said 3-6 membered heterocycloalkyl is one or more selected from N, O and S, and the number of heteroatom(s) is 1, 2 or 3; said 3-6 membered heterocycloalkyl is a 3-6 membered saturated monocyclic ring; the heteroatom in said 5-10 membered heteroaryl is N and/or O, and the number of heteroatom(s) is 1 or 2;

$R^{2-3-2-1}$ is each independently selected from hydroxyl, or

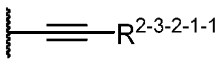
;

$R^{2-3-2-1-1}$ is each independently $C_1$-$C_6$ alkyl;

$R^{2-3-2-2}$ is each independently selected from halogen, hydroxyl, $C_1$-$C_6$ alkyl, $C_1$-$C_6$ alkoxy unsubstituted or substituted by one or more $R^{2-3-2-2-2}$, or amino;

$R^{2-3-2-2-2}$ is each independently deuterium;

$R^{2-3-2-3}$ is each independently selected from hydrogen or

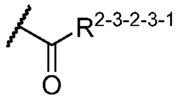

;

$R^{2-3-2-3-1}$ is each independently 6-10 membered aryl unsubstituted or substituted by one or more $R^{2-3-2-3-1-1}$;
$R^{2-3-2-3-1-1}$ is each independently $C_1$-$C_6$ alkoxy;
$R^{2-3-2-5}$ is each independently selected from halogen, $C_1$-$C_6$ alkoxy, or $C_1$-$C_6$ alkyl unsubstituted or substituted by one or more $R^{2-3-2-5-1}$;
$R^{2-3-2-5-1}$ is each independently halogen;
$R^3$ is 5-6 membered heteroaryl unsubstituted or substituted by one or more $R^{3-1}$; the heteroatom in said 5-6 membered heteroaryl is N and/or S, and the number of heteroatoms is 3;
$R^{3-1}$ is each independently $C_1$-$C_6$ alkyl substituted by one or more $R^{3-1-1}$;
$R^{3-1-1}$ is each independently halogen; and
$R^4$ is hydrogen, cyano, halogen, $C_1$-$C_6$ alkyl, or 5-10 membered heterocycloalkyl unsubstituted or substituted by one or more $R^{4-1}$; the heteroatom in said 5-10 membered heterocycloalkyl is one or more selected from N, O and S, and the number of heteroatom(s) is 1 or 2; said 5-10 membered heterocycloalkyl is a 7-10 membered unsaturated spiro ring;
$R^{4-1}$ is each independently

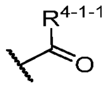

;

$R^{4-1-1}$ is $C_1$-$C_6$ alkyl;
in a solution 9,
$R^1$ is $C_3$-$C_6$ cycloalkyl unsubstituted or substituted by one or more $R^{1-2}$;
$R^{1-2}$ is each independently selected from cyano, or $C_1$-$C_6$ alkyl unsubstituted or substituted by one or more $R^{1-2-1}$;
$R^{1-2-1}$ is each independently selected from deuterium or halogen;
$R^2$ is 4-10 membered heterocycloalkyl substituted by one or more $R^{2-3}$; the heteroatom in said 4-10 membered heterocycloalkyl is selected from N and/or O, and the number of heteroatom(s) is 1, 2 or 3; said 4-10 membered heterocycloalkyl is a 4-6 membered saturated monocyclic ring or a 7-10 membered saturated spiro ring;
$R^{2-3}$ is each independently

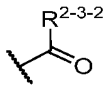

;

$R^{2-3-2}$ is $C_3$-$C_6$ cycloalkyl substituted by one or more $R^{2-3-2-2}$, 3-6 membered heterocycloalkyl substituted by one or more $R^{2-3-2-5}$; the heteroatom in said 3-6 membered heterocycloalkyl is one or more selected from N, O and S, and the number of heteroatom(s) is 1, 2 or 3; said 3-6 membered heterocycloalkyl is a 3-6 membered saturated monocyclic ring; the heteroatom in said 5-10 membered heteroaryl is N and/or O, and the number of heteroatom(s) is 1 or 2;
$R^{2-3-2-2}$ is each independently selected from $C_1$-$C_6$ alkyl, or $C_1$-$C_6$ alkoxy unsubstituted or substituted by one or more $R^{2-3-2-2-2}$;
$R^{2-3-2-2-2}$ is each independently deuterium;
$R^{2-3-2-5}$ is each independently $C_1$-$C_6$ alkyl;
$R^3$ is 5-6 membered heteroaryl unsubstituted or substituted by one or more $R^{3-1}$; the heteroatom in said 5-6 membered heteroaryl is N and/or S, and the number of heteroatoms is 3;
$R^{3-1}$ is each independently $C_1$-$C_6$ alkyl substituted by one or more $R^{3-1-1}$;
$R^{3-1-1}$ is each independently halogen;
$R^4$ is hydrogen, cyano, or $C_1$-$C_6$ alkyl;
in a solution 10,
$R^1$ is 3-6 membered heterocycloalkyl unsubstituted or substituted by one or more $R^{1-3}$; the heteroatom in said 3-6 membered heterocycloalkyl is O, and the number of heteroatom(s) is 1; said 3-6 membered heterocycloalkyl is a 3-6 membered saturated monocyclic ring;
$R^{1-3}$ is each independently selected from cyano, $C_1$-$C_6$ alkyl unsubstituted or substituted by one or more $R^{1-3-1}$, or $C_2$-$C_6$ alkynyl;

$R^{1-3-1}$ is each independently selected from halogen or deuterium;

$R^2$ is 4-12 membered heterocycloalkyl unsubstituted or substituted by one or more $R^{2-3}$; the heteroatom in said 4-12 membered heterocycloalkyl is one or more selected from N, O and S, and the number of heteroatom(s) is 1, 2 or 3; said 4-12 membered heterocycloalkyl is a 4-6 membered saturated monocyclic ring, a 7-10 membered saturated fused ring or a 7-10 membered saturated spiro ring;

$R^{2-3}$ is each independently $C_1$-$C_6$ alkyl unsubstituted or substituted by one or more $R^{2-3-1}$,

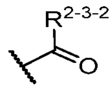, or 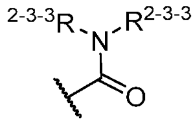;

$R^{2-3-1}$ is each independently selected from hydroxyl, halogen, $C_1$-$C_6$ alkoxy unsubstituted or substituted by one or more $R^{2-3-1-1}$, or amino unsubstituted or substituted by one or more $R^{2-3-1-3}$;

$R^{2-3-1-1}$ is each independently deuterium;

$R^{2-3-1-3}$ is each independently $C_1$-$C_6$ alkyl;

$R^{2-3-2}$ is $C_1$-$C_6$ alkyl, or $C_3$-$C_6$ cycloalkyl unsubstituted or substituted by one or more $R^{2-3-2-2}$;

$R^{2-3-2-2}$ is each independently selected from $C_1$-$C_6$ alkyl, or $C_1$-$C_6$ alkoxy unsubstituted or substituted by one or more $R^{2-3-2-2-2}$;

$R^{2-3-2-2-2}$ is each independently deuterium;

$R^{2-3-3}$ is each independently selected from hydrogen, or $C_1$-$C_6$ alkyl unsubstituted or substituted by one or more $R^{2-3-3-1}$;

$R^{2-3-3-1}$ is each independently amino unsubstituted or substituted by one or more $R^{2-3-3-1-1}$;

$R^{2-3-3-1-1}$ is each independently $C_1$-$C_6$ alkyl;

$R^3$ is 5-6 membered heteroaryl unsubstituted or substituted by one or more $R^{3-1}$; the heteroatom in said 5-6 membered heteroaryl is N and/or S, and the number of heteroatoms is 3;

$R^{3-1}$ is each independently $C_1$-$C_6$ alkyl substituted by one or more $R^{3-1-1}$;

$R^{3-1-1}$ is each independently halogen;

$R^4$ is hydrogen, deuterium, halogen, or $C_1$-$C_6$ alkyl;

in a solution 11,

$R^1$ is 3-6 membered heterocycloalkyl unsubstituted or substituted by one or more $R^{1-3}$; the heteroatom in said 3-6 membered heterocycloalkyl is O, and the number of heteroatom(s) is 1; said 3-6 membered heterocycloalkyl is a 3-6 membered saturated monocyclic ring;

$R^{1-3}$ is each independently selected from cyano, $C_1$-$C_6$ alkyl unsubstituted or substituted by one or more $R^{1-3-1}$, or $C_2$-$C_6$ alkynyl;

$R^{1-3-1}$ is each independently halogen;

$R^2$ is 4-12 membered heterocycloalkyl unsubstituted or substituted by one or more $R^{2-3}$; the heteroatom in said 4-12 membered heterocycloalkyl is one or more selected from N, O and S, and the number of heteroatom(s) is 1, 2 or 3; said 4-12 membered heterocycloalkyl is a 4-6 membered saturated monocyclic ring, a 7-10 membered saturated fused ring or a 7-10 membered saturated spiro ring;

$R^{2-3}$ is each independently $C_1$-$C_6$ alkyl unsubstituted or substituted by one or more $R^{2-3-1}$,

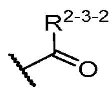, or 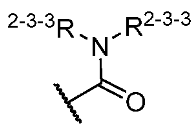;

$R^{2-3-1}$ is each independently selected from hydroxyl, or $C_1$-$C_6$ alkoxy unsubstituted or substituted by one or more $R^{2-3-1-1}$;

$R^{2-3-1-1}$ is each independently deuterium;

$R^{2-3-2}$ is $C_1$-$C_6$ alkyl, or $C_3$-$C_6$ cycloalkyl unsubstituted or substituted by one or more $R^{2-3-2-2}$;

$R^{2-3-2-2}$ is each independently selected from $C_1$-$C_6$ alkyl, or $C_1$-$C_6$ alkoxy unsubstituted or substituted by one or more $R^{2-3-2-2-2}$;

$R^{2-3-2-2-2}$ is each independently deuterium;

$R^{2-3-3}$ is each independently selected from hydrogen, or $C_1$-$C_6$ alkyl unsubstituted or substituted by one or more $R^{2-3-3-1}$;

$R^{2-3-3-1}$ is each independently amino unsubstituted or substituted by one or more $R^{2-3-3-1-1}$;

$R^{2-3-3-1-1}$ is each independently $C_1$-$C_6$ alkyl;
$R^3$ is 5-6 membered heteroaryl unsubstituted or substituted by one or more $R^{3-1}$; the heteroatom in said 5-6 membered heteroaryl is N and/or S, and the number of heteroatoms is 3;
$R^{3-1}$ is each independently $C_1$-$C_6$ alkyl substituted by one or more $R^{3-1-1}$;
$R^{3-1-1}$ is each independently halogen; and
$R^4$ is hydrogen, deuterium, halogen, or $C_1$-$C_6$ alkyl.

**17.** The compound of formula I, the pharmaceutically acceptable salt thereof, or the isotopic compound thereof according to claim 7, wherein the compound of formula I-a can be a compound of formula IV,

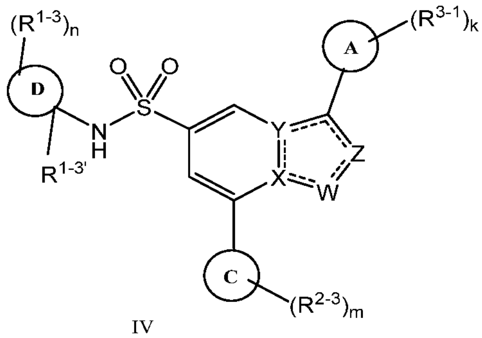

IV

ring D is 3-6 membered heterocycloalkyl; the heteroatom in said 3-6 membered heterocycloalkyl is one or more selected from N, O and S, and the number of heteroatom(s) is 1, 2 or 3;
ring C is 4-12 membered heterocycloalkyl; the heteroatom in said 4-12 membered heterocycloalkyl is one or more selected from N, O and S, and the number of heteroatom(s) is 1, 2 or 3;
ring A is 5-10 membered heteroaryl; the heteroatom in said 5-10 membered heteroaryl is one or more selected from N, O and S, and the number of heteroatom(s) is 1, 2 or 3;
n is 0, 1 or 2; m is 0, 1 or 2; k is 0, 1 or 2;
$R^{1-3'}$ is hydrogen or $R^{1-3}$; and
$R^{1-3}$, $R^{2-3}$ and $R^{3-1}$ are defined as in any one of claims 1-6 and 11-16.

**18.** The compound of formula I, the pharmaceutically acceptable salt thereof, or the isotopic compound thereof according to claim 17, wherein the compound of formula I-a can be a compound of formula V,

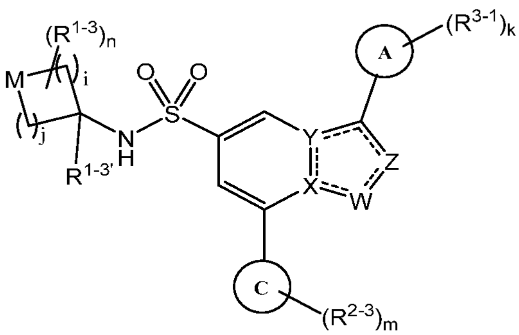

V

M is N, O or S;
i is 0, 1 or 2; j is 0, 1 or 2; i and j are not 0 at the same time;
ring C, ring A, $R^{1-3'}$, $R^{1-3}$, $R^{2-3}$, $R^{3-1}$, n, m and k are defined as in claim 17;
more preferably, the compound of formula I-a can be a compound represented by formula VI,

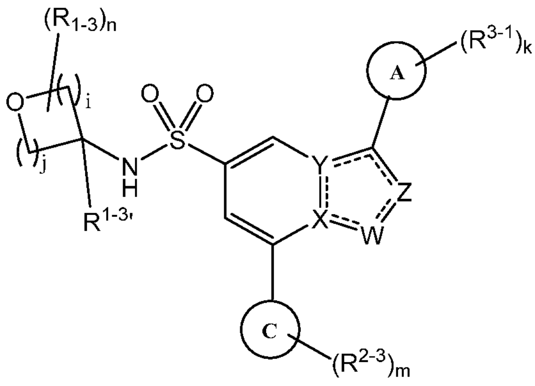

VI

ring C, ring A, $R^{1-3'}$, $R^{1-3}$, $R^{2-3}$, $R^{3-1}$, n, m, k, i and j are defined as in claim 17;
most preferably, the compound of formula I-a can be a compound represented by formula VII,

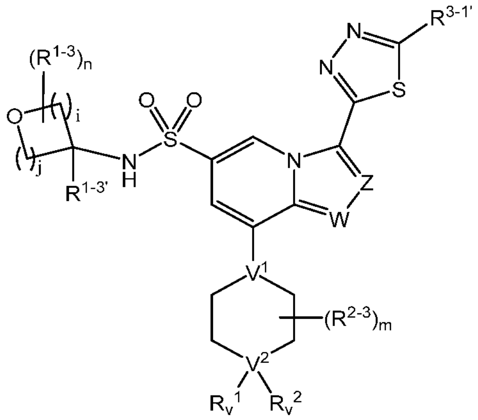

VII

$V^1$ is CH, N or O; $V^2$ is CH, N or O; $V^1$ and $V^2$ are not CH at the same time;
$R^{3-1'}$ is hydrogen or $R^{3-1}$; and
$R_V^1$ is hydrogen or $R^{2-3}$; $R_V^2$ is hydrogen or $R^{2-3}$;
$R^{1-3'}$, $R^{1-3}$, $R^{2-3}$, $R^{3-1}$, n, m, i and j are defined as in claim 17;
or, the compound of formula I-a can be a compound represented by formula VIII,

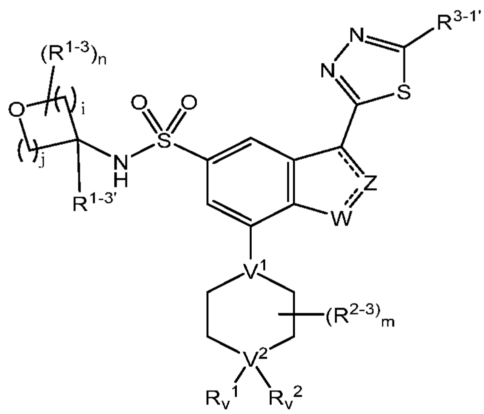

VIII

$R_V^1$, $R_V^2$, $R^{1-3'}$, $R^{1-3}$, $R^{2-3}$, $R^{3-1'}$, n, m, i and j are defined as in claim 17,
most preferably, the compound of formula I-a can be a compound represented by formula IX,

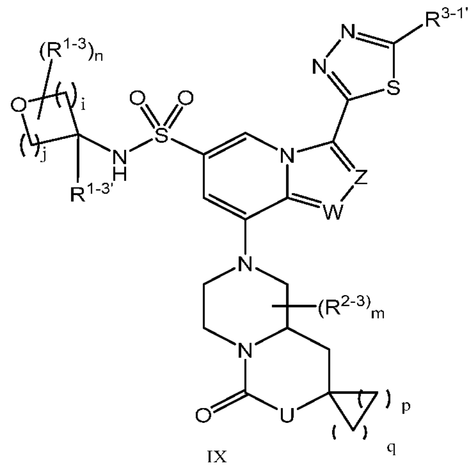

IX

U is CH, N or O;
p is 0, 1 or 2; q is 0, 1 or 2;
$R^{1-3'}$, $R^{1-3}$ $R^{2-3}$, $R^{3-1'}$, n, m, i and j are defined as in claim 17;
or, the compound of formula I-a can be a compound represented by formula X,

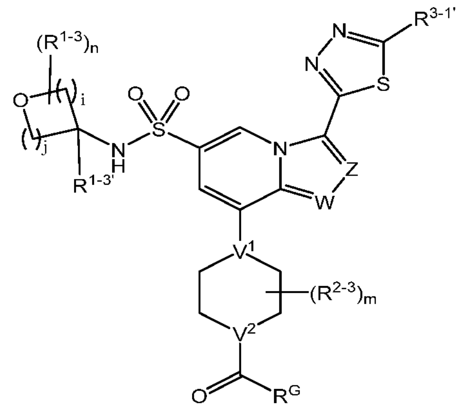

X

$R^G$ is $R^{2-3-2}$ or

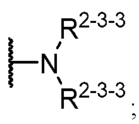;

and
$R^{1-3'}$, $R^{1-3}$, $R^{2-3}$, $R^{3-1'}$, $R^{2-3-2}$, $R^{2-3-3}$, $V^1$, $V^2$, n, m, i and j are defined as in claim 17.

**19.** The compound of formula I, the pharmaceutically acceptable salt thereof or the isotopic compound thereof according to claim 1, wherein the compound of formula I satisfies one or more of the following conditions:

(1) ring B is

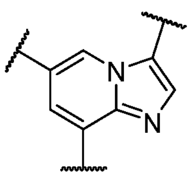, 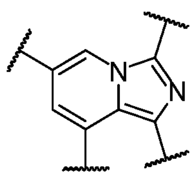, 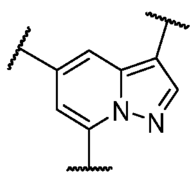, 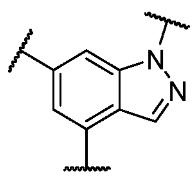, 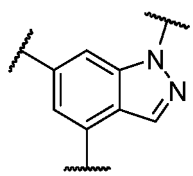,

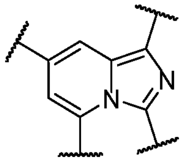, 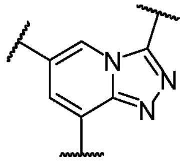, 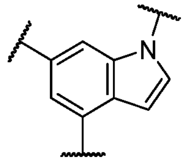, 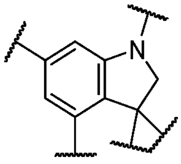, 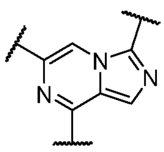,

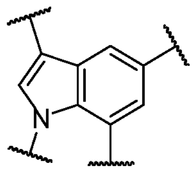, 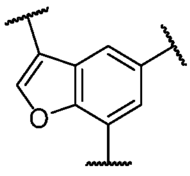 or 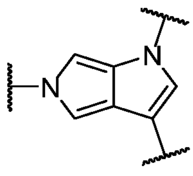;

(2) $R^1$ is

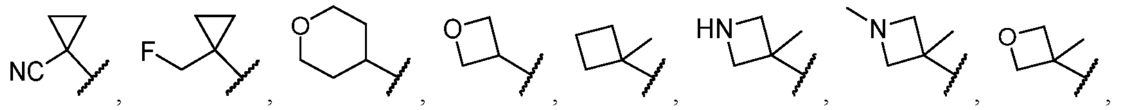
, , , , , , , ,

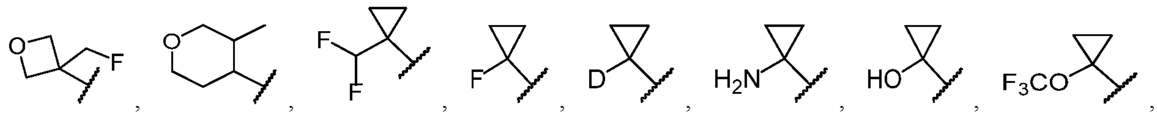
, , , , , , , ,

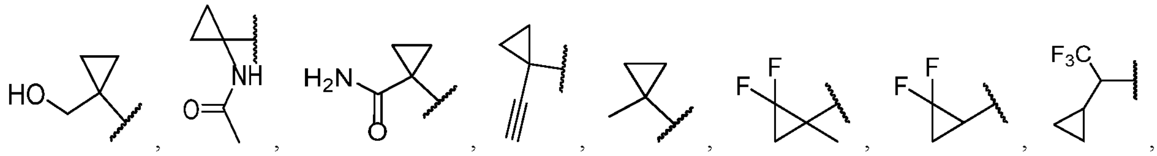
, , , , , , , ,

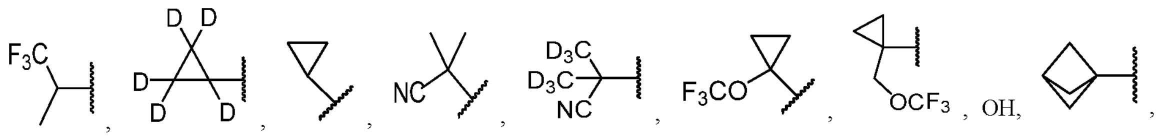
, , , , , , , OH, ,

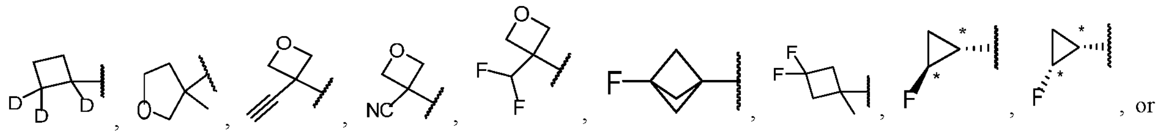
, , , , , , , , , or

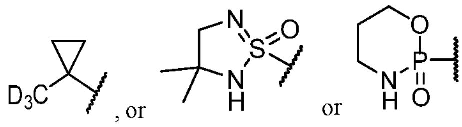
, or or

is formed when the atom in $R^1$ is attached to the atom in A;

(3) $R^2$ is hydrogen, methyl,

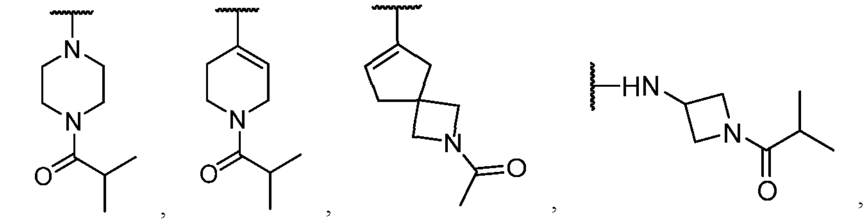

, , , ,

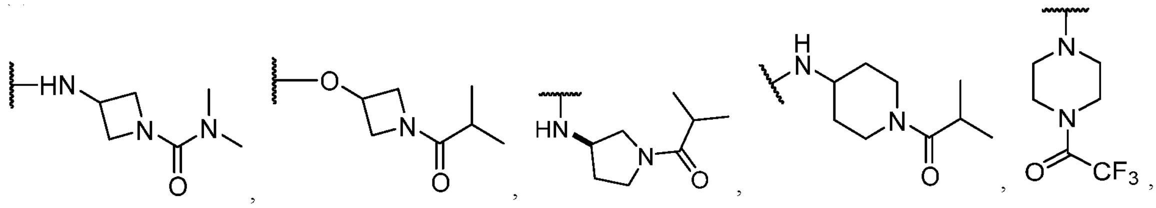

, , , , ,

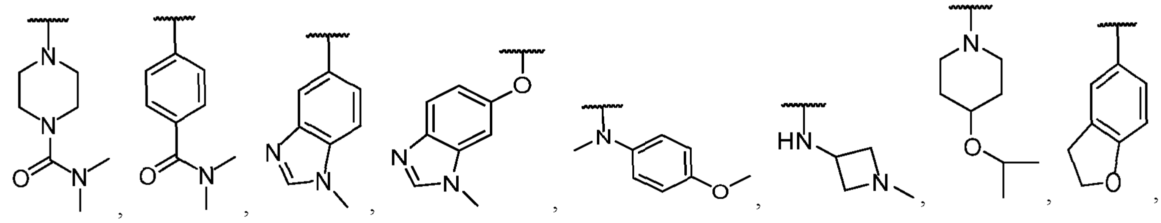

, , , , , , , ,

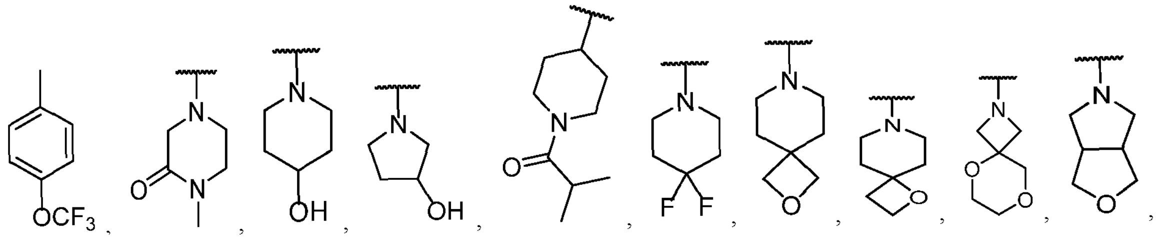

, , , , , , , , , ,

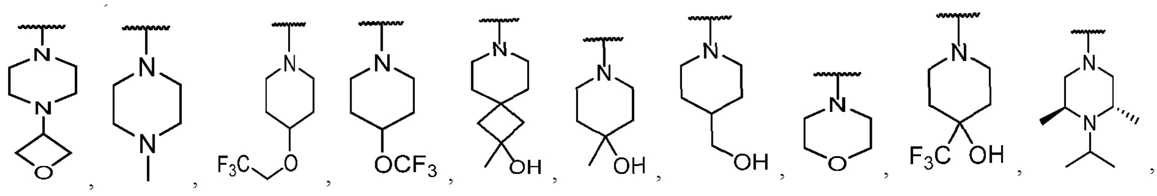

, , , , , , , , , ,

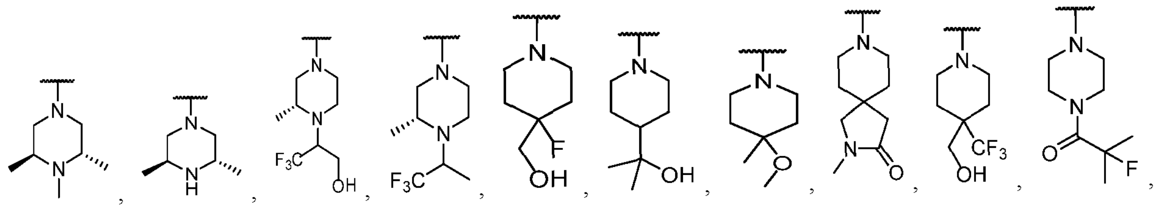

, , , , , , , , , ,

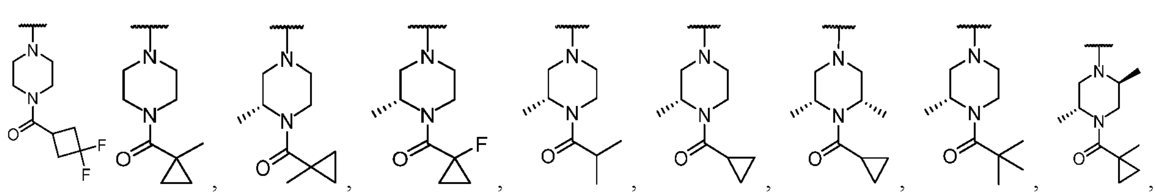

, , , , , , , ,

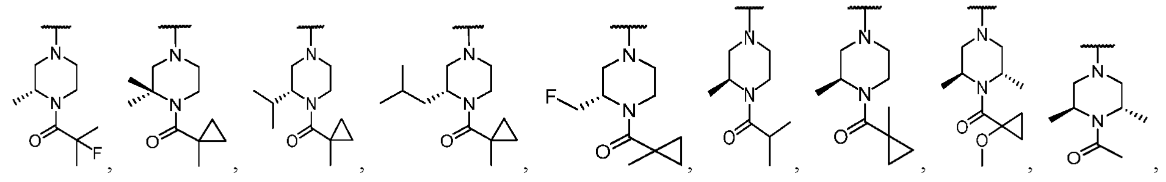
, , , , , , , , ,

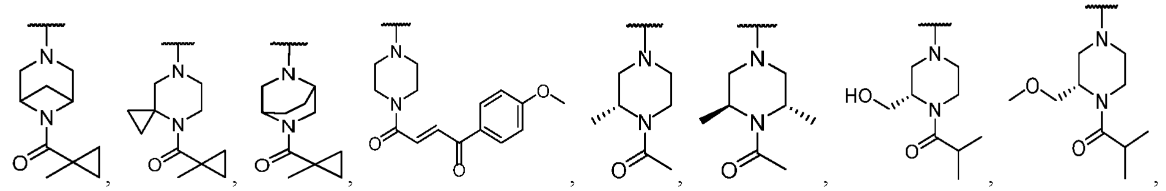
, , , , , , , ,

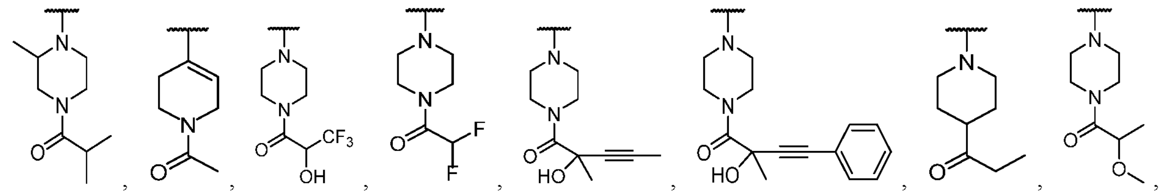
, , , , , , , ,

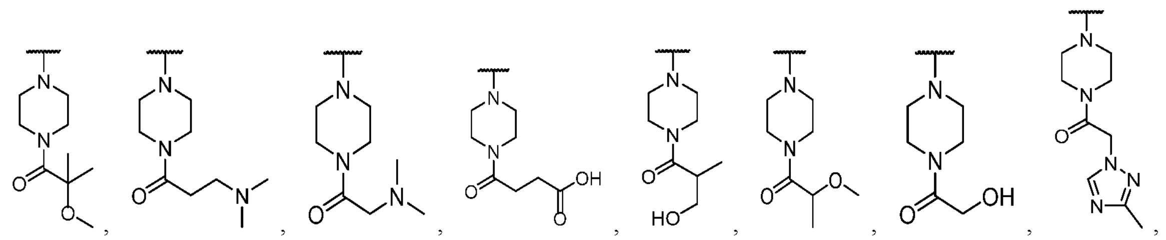
, , , , , , , ,

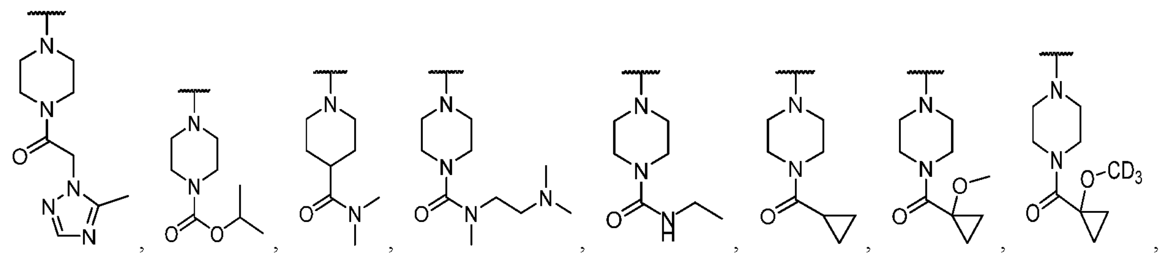
, , , , , , , ,

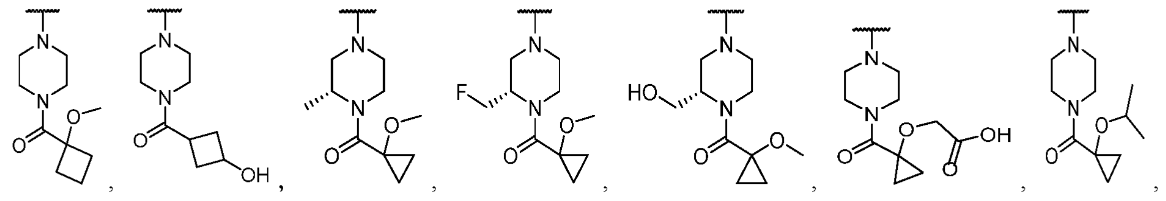
, , , , , , ,

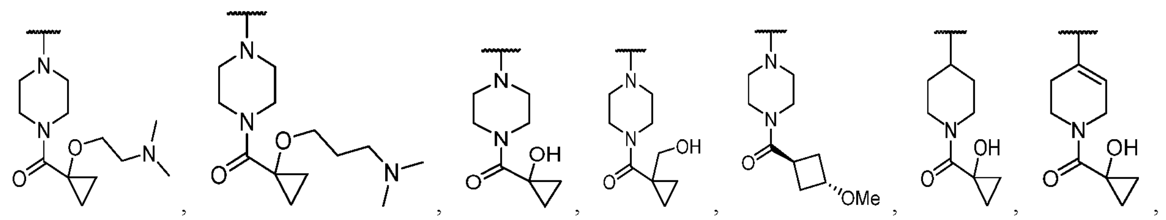
, , , , , , ,

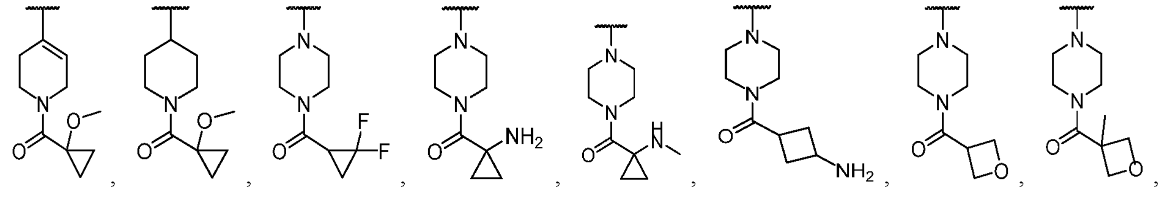
, , , , , , , ,

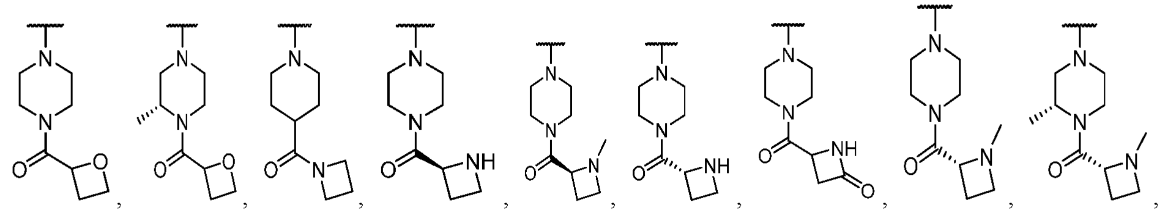
, , , , , , , , ,

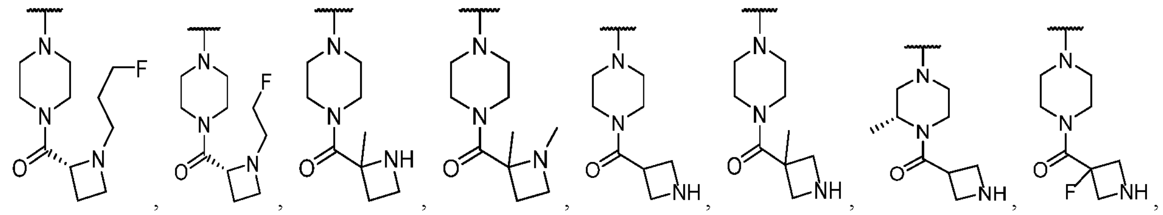
, , , , , , , ,

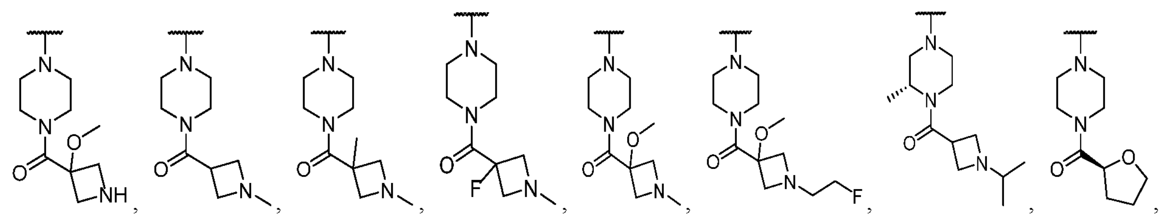
, , , , , , , ,

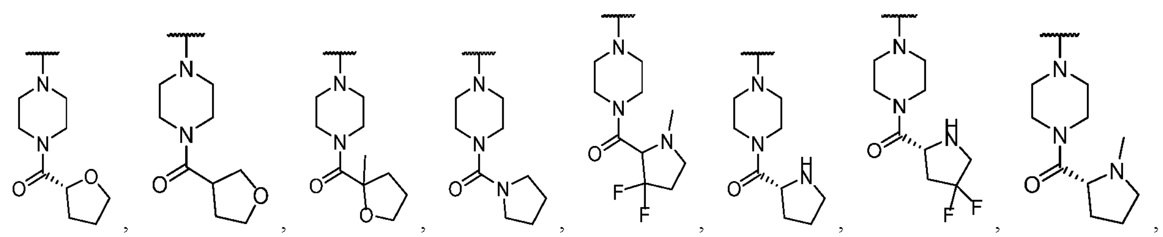
, , , , , , , ,

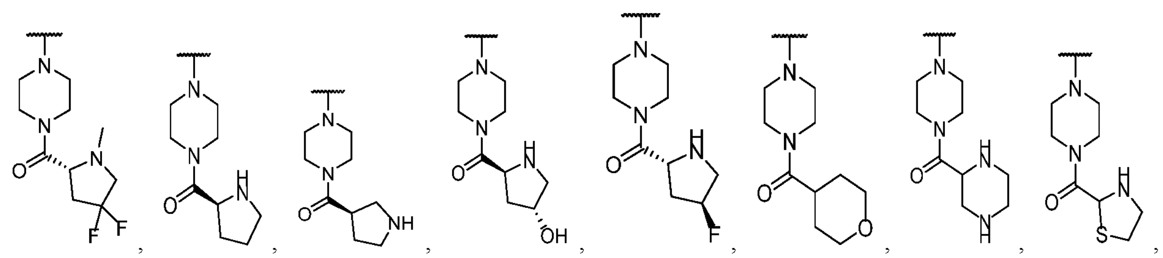
, , , , , , , ,

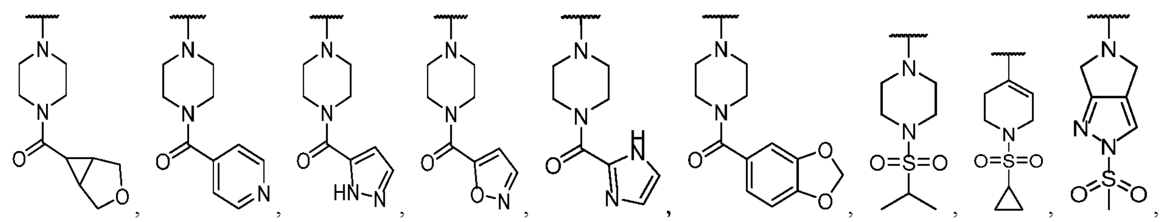
, , , , , , , , ,

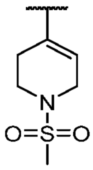, 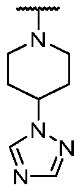, 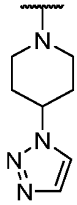, 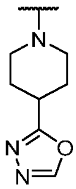, 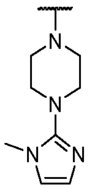, 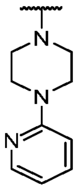, 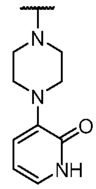, 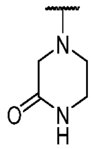, 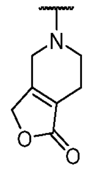, 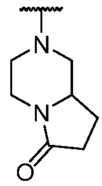, 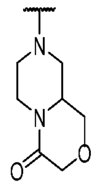,

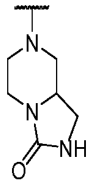, 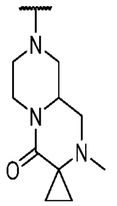, 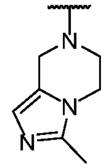, 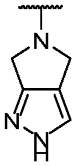, 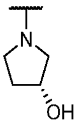, 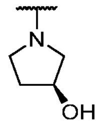, 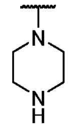, 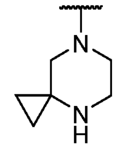, 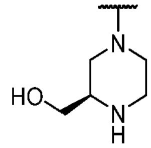,

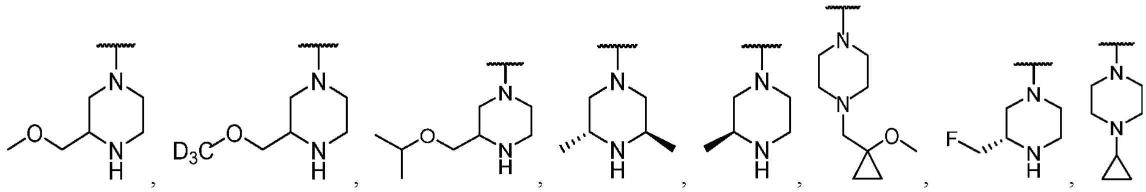
, , , , , , , ,

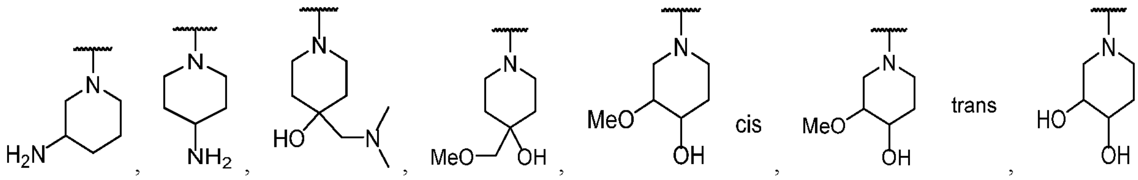
, , cis , trans ,

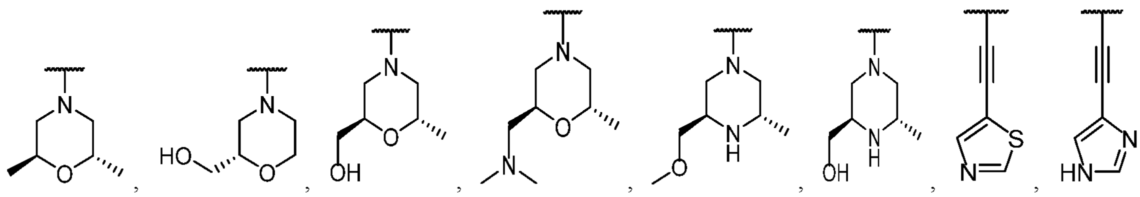
, , , , , , , ,

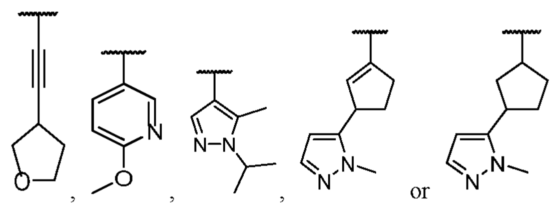
, , , or ;

(4) $R^3$ is

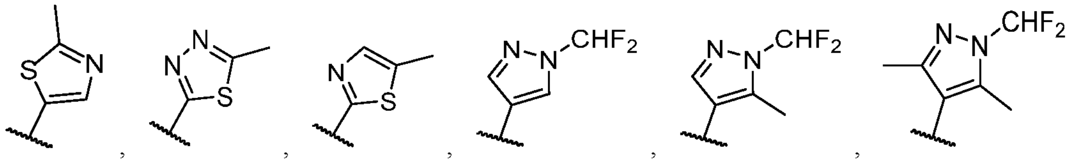
, , , , , ,

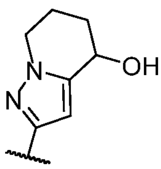, 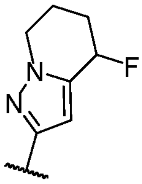, 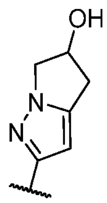, 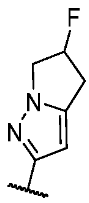, 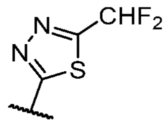, 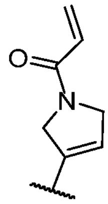, 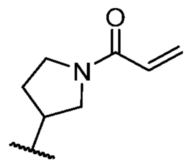,

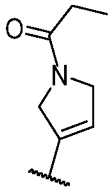, 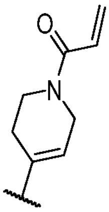, 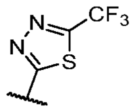 or 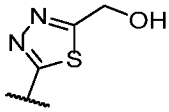;

and (5) $R^4$ is hydrogen, deuterium, Cl, F, CN, methyl,

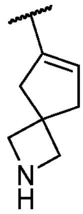, 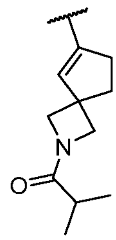, 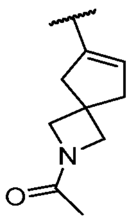, 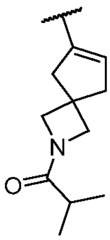, 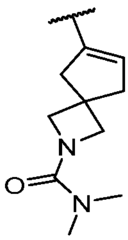,

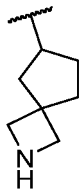, 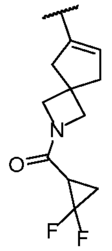 or 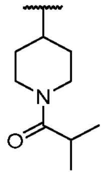

**20.** The compound of formula I, the pharmaceutically acceptable salt thereof or the isotopic compound thereof according to claim 1, wherein the compound of formula I is any one of the following compounds:

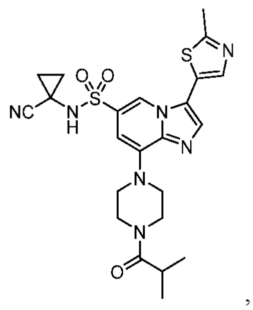,

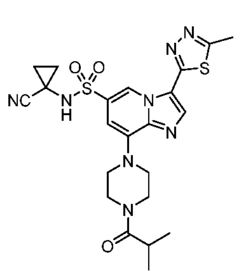, 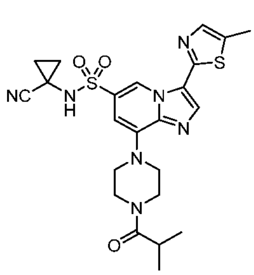, 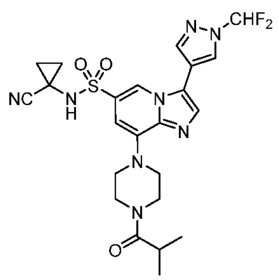, 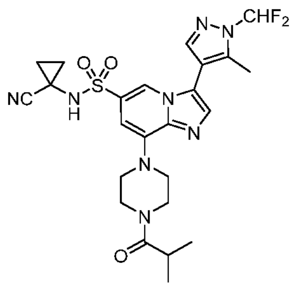,

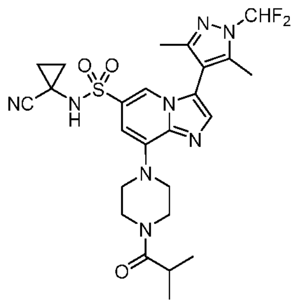, 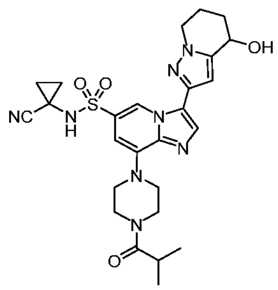, 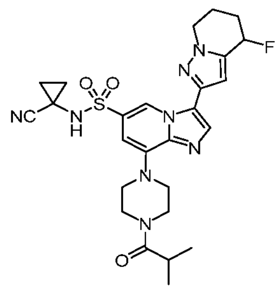, 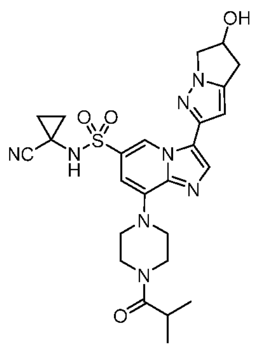,

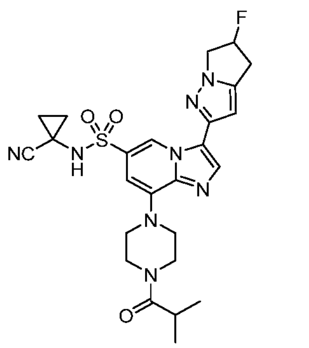, 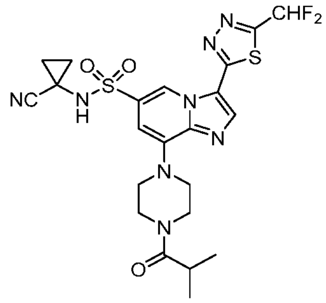, 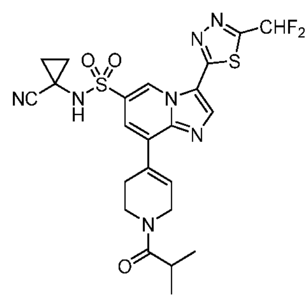,

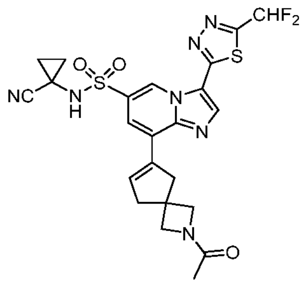, 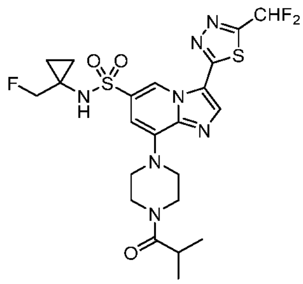, 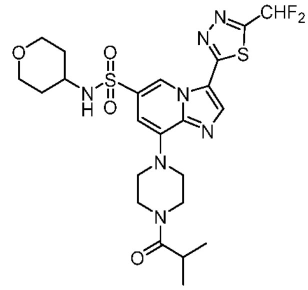,

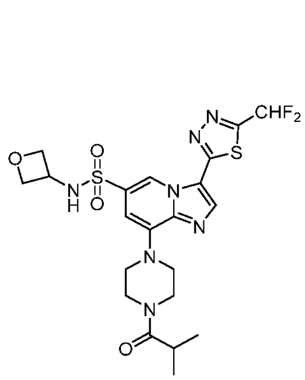, 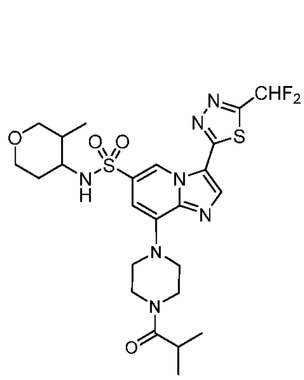, 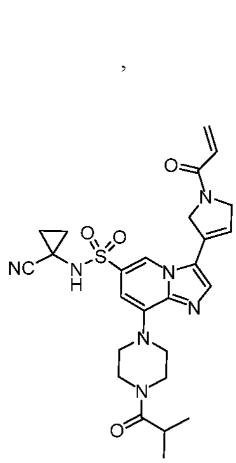, 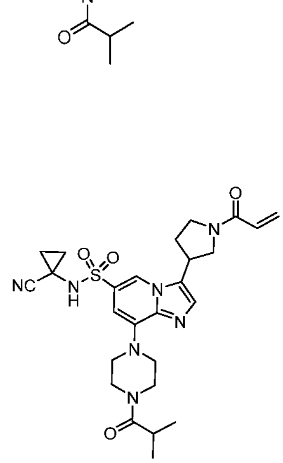,

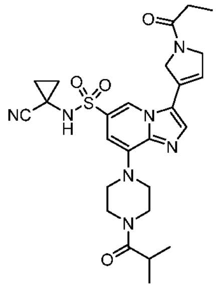
,
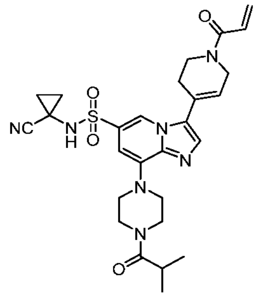
,
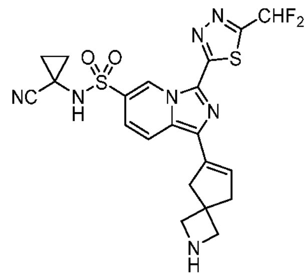
,
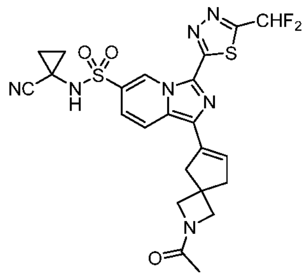
,
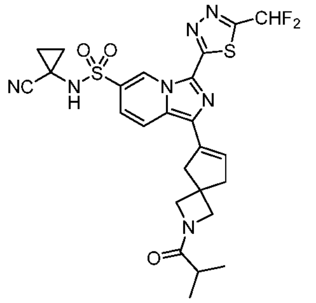
,
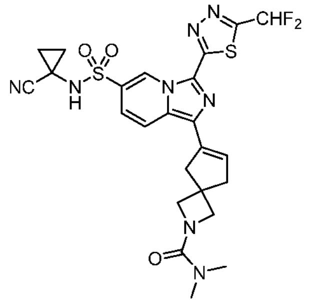
,
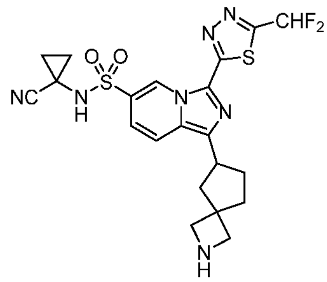
,
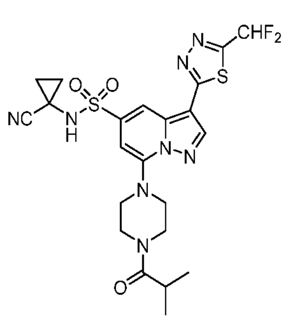
,
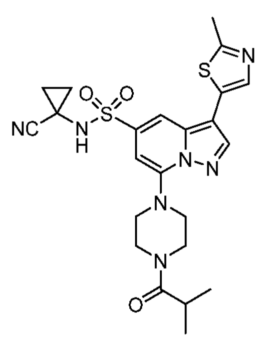
,
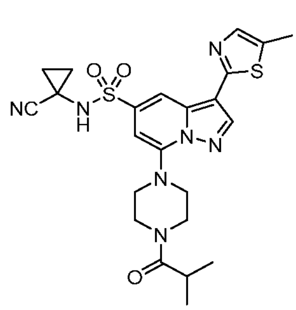
,
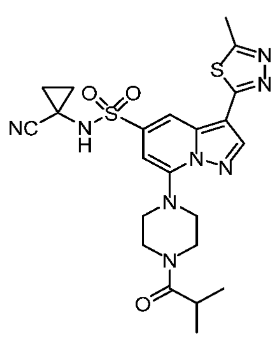
,
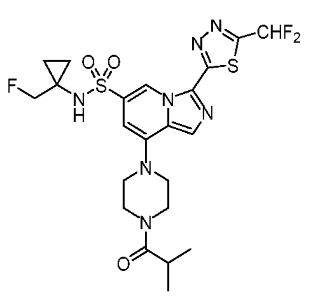
,
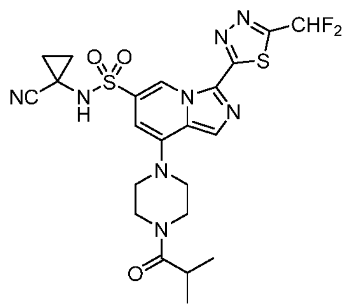
,
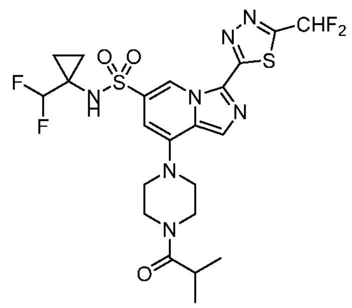
,
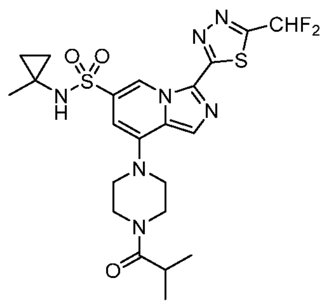
,
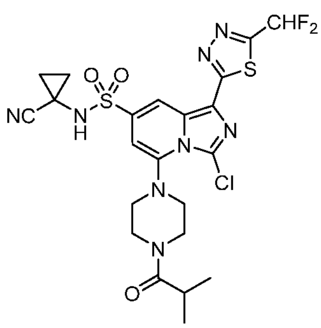
,
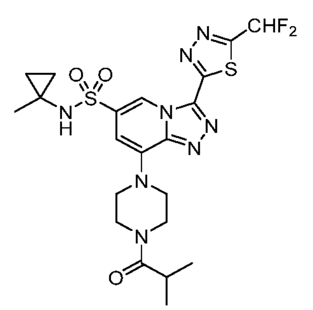
,

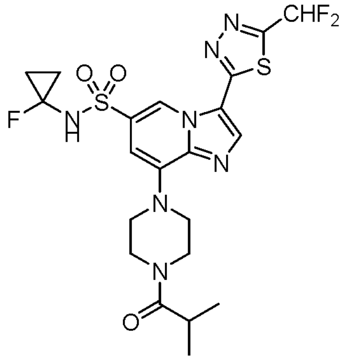
,
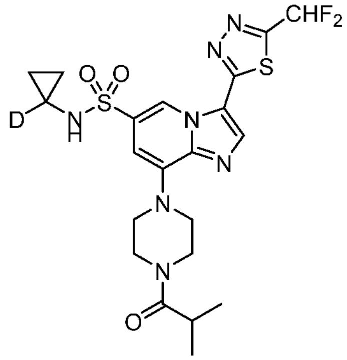
,
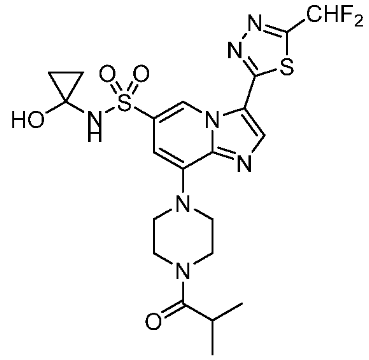
,
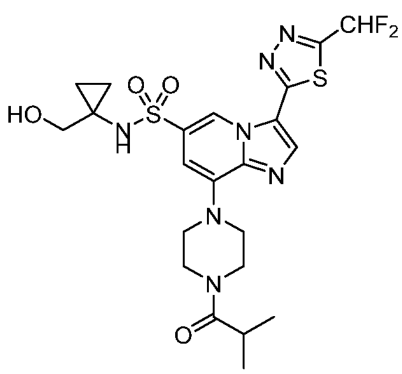
,
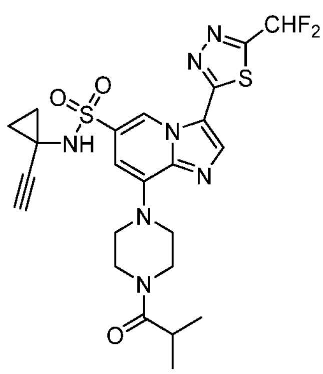
,
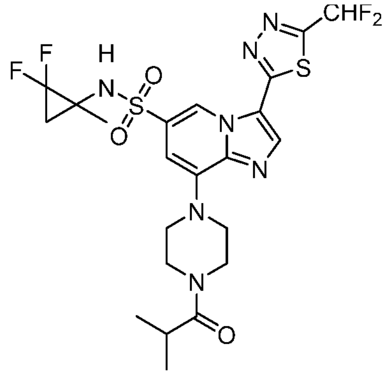
,
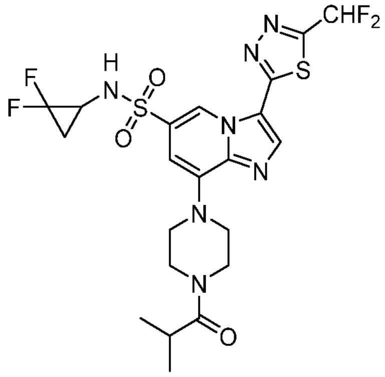
,
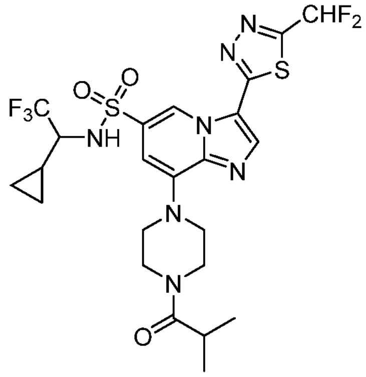
,
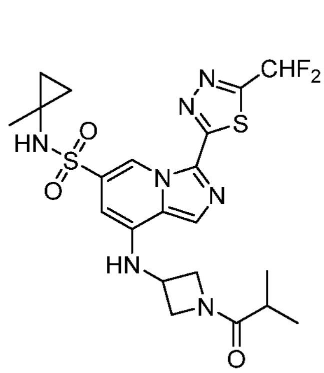
,
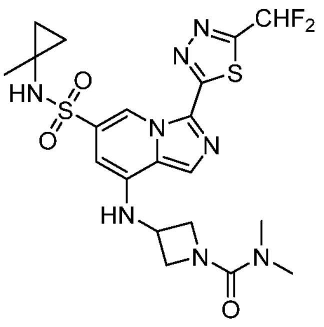
,
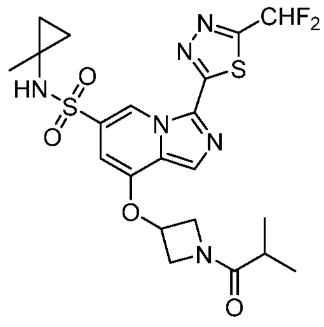
,
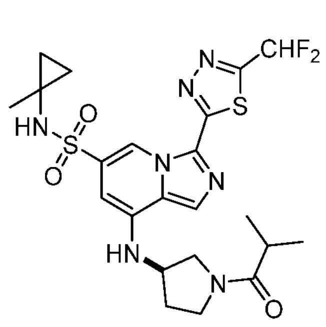
,

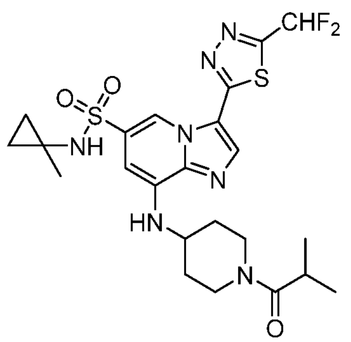,
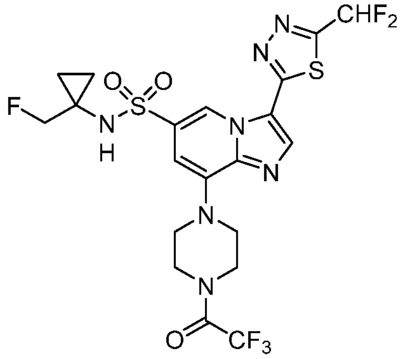,
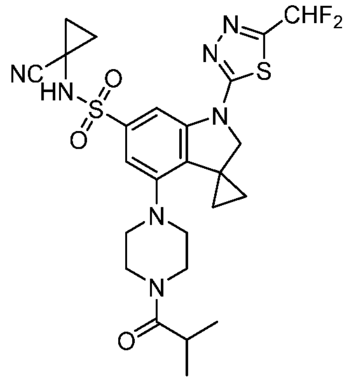,

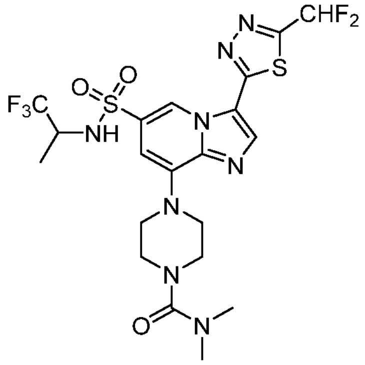,
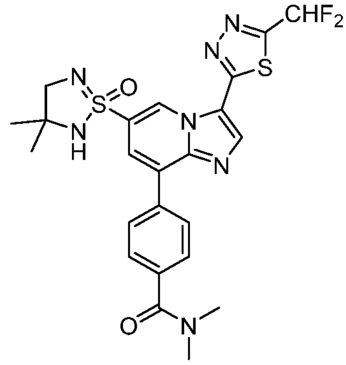,
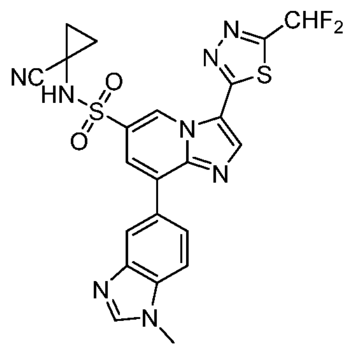,

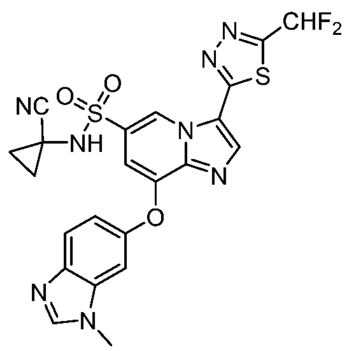,
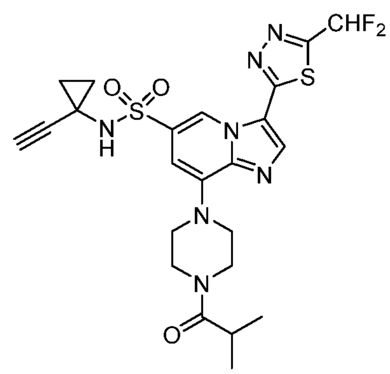,
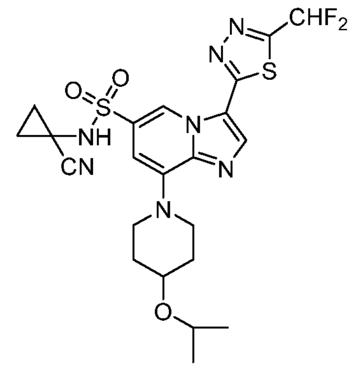,

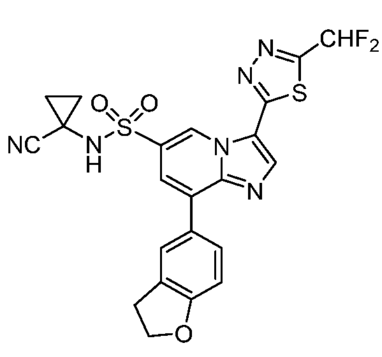,
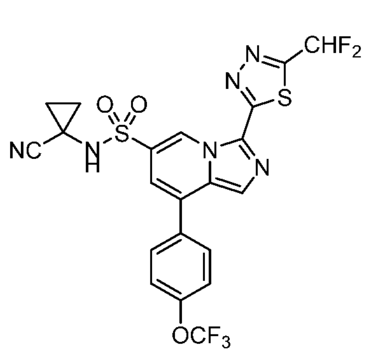,
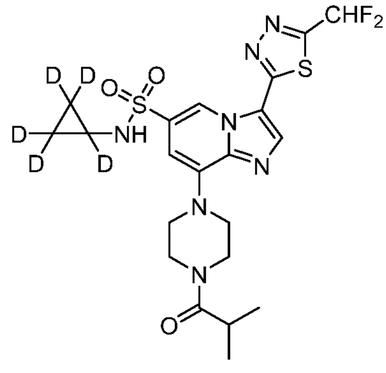,

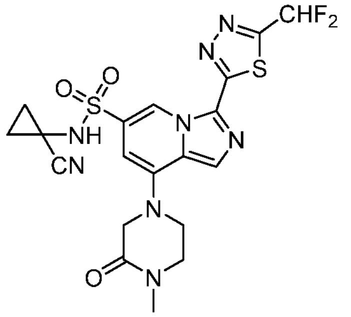
,
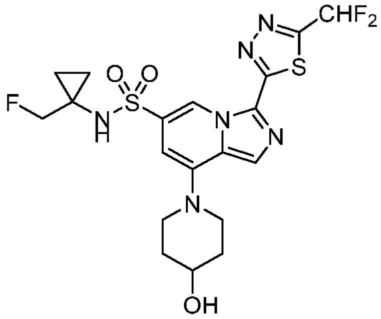
,
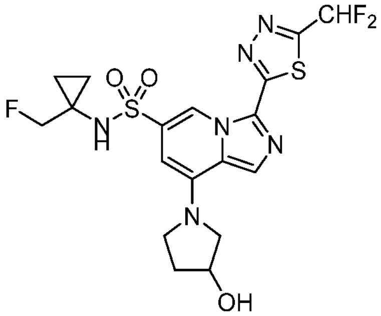
,

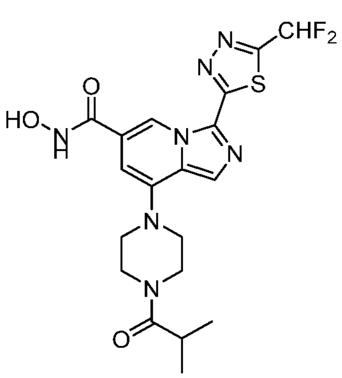
,
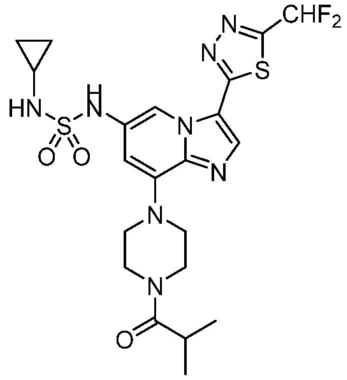
,
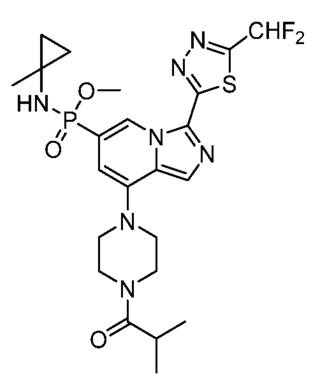
,

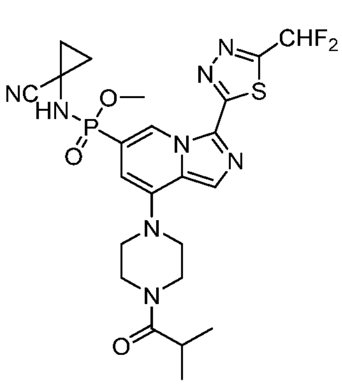
,
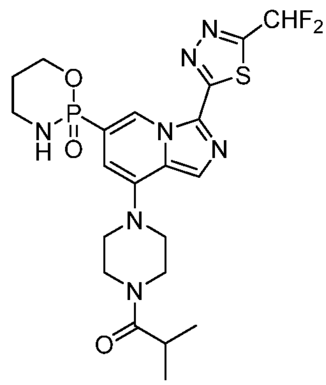
,
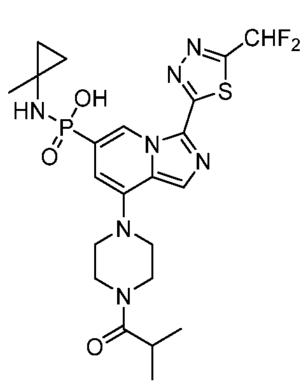
,

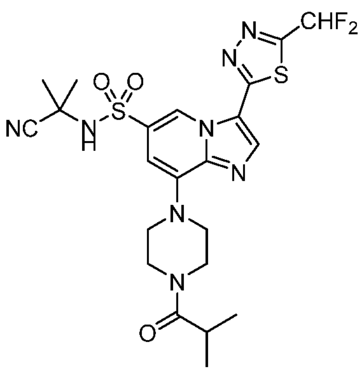
,
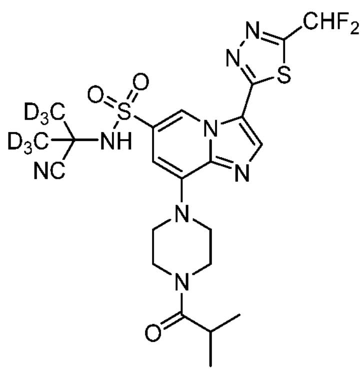
,
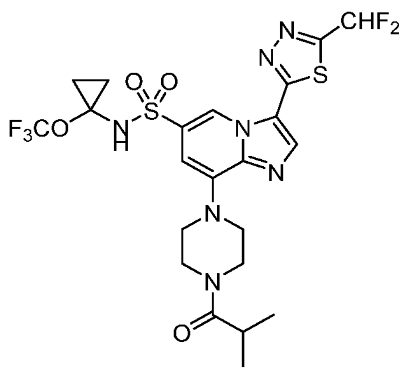
,

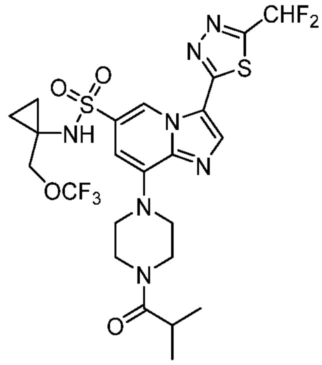 , 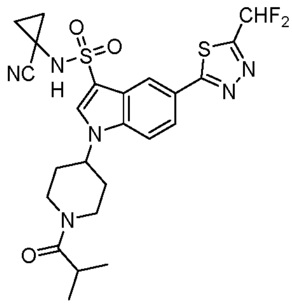 , 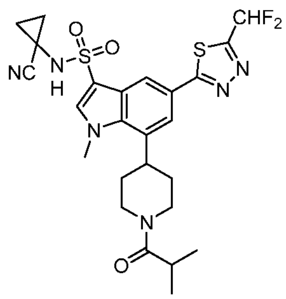 ,

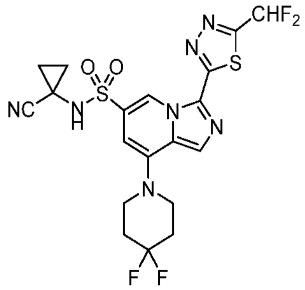 , 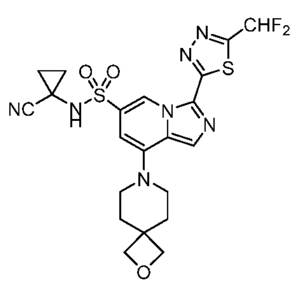 , 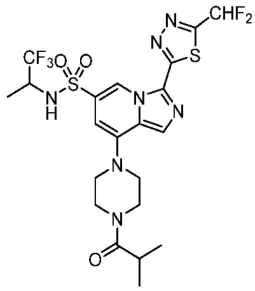 , 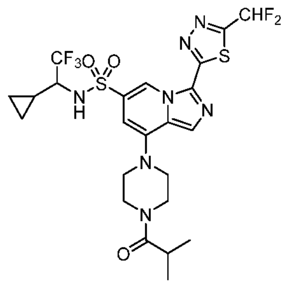 ,

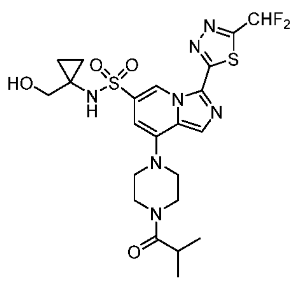 , 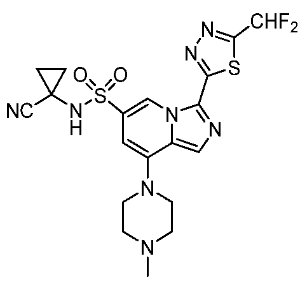 , 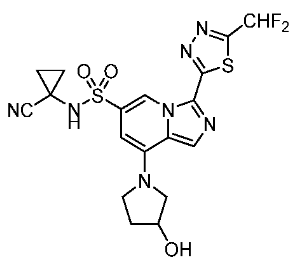 , 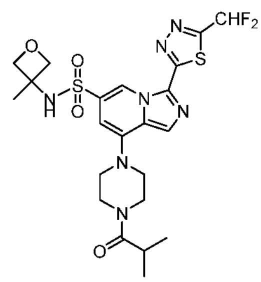 ,

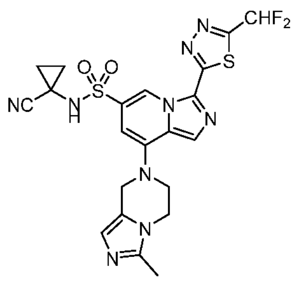 , 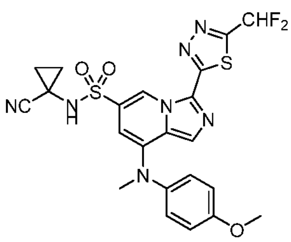 , 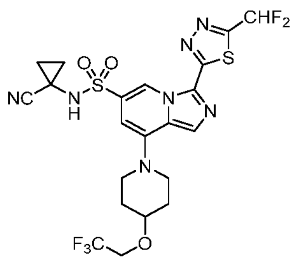 ,

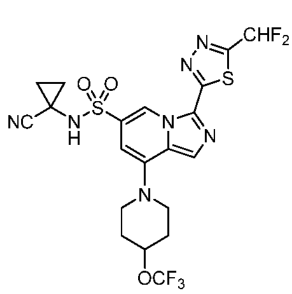 , 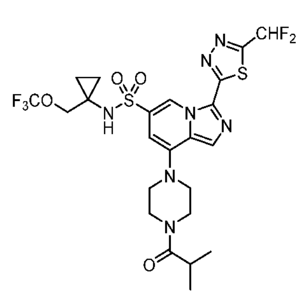 , 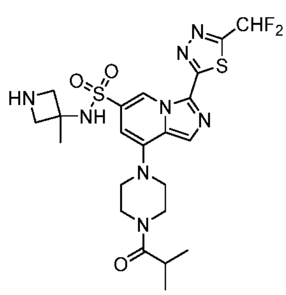 ,

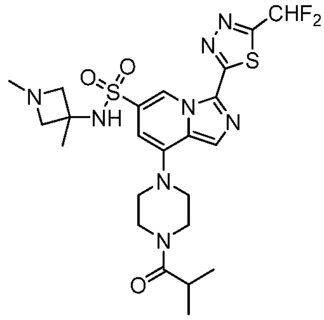,
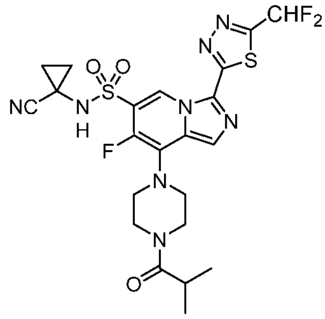,
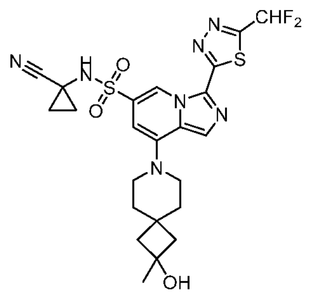,

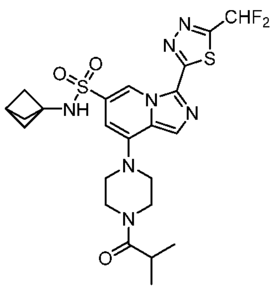,
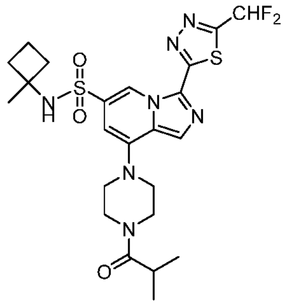,
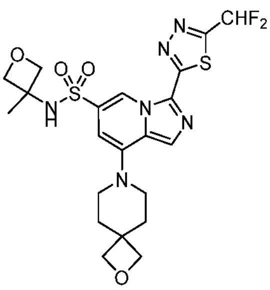,
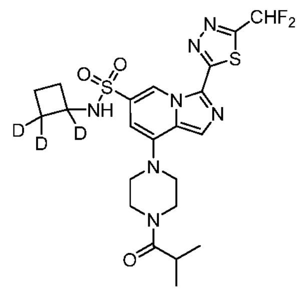,

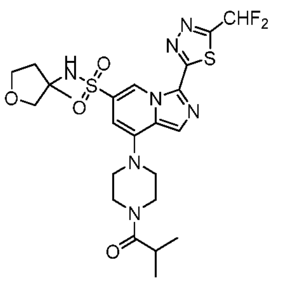,
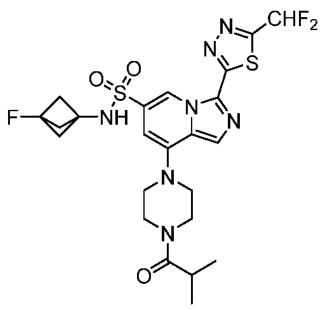,
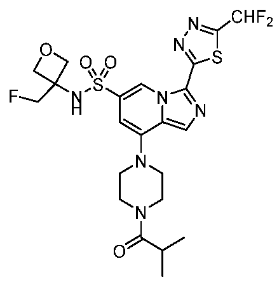,

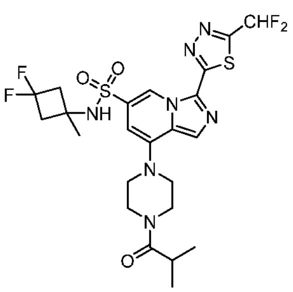,
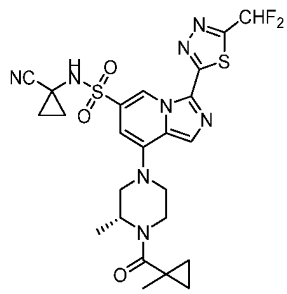,
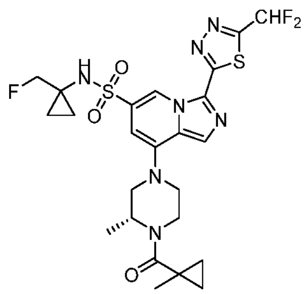,

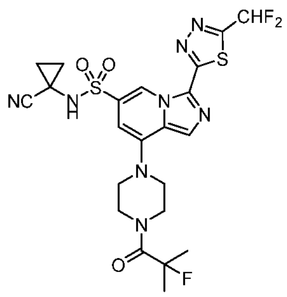,
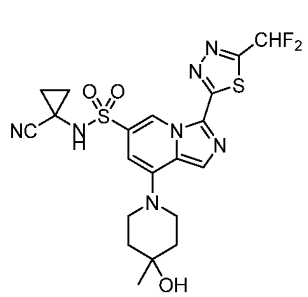,
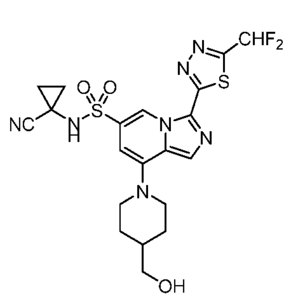,

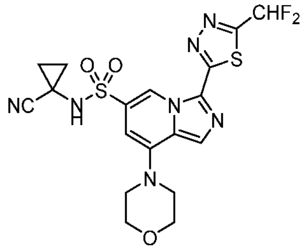
,
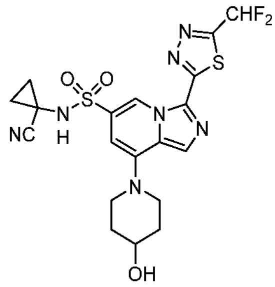
,
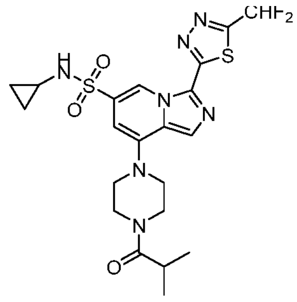
,

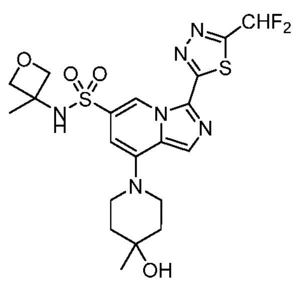
,
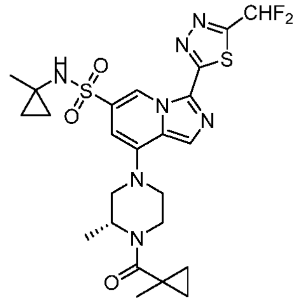
,
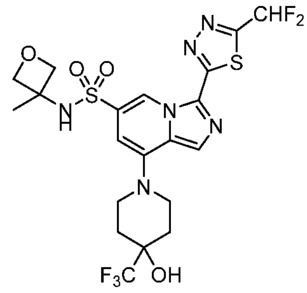
,

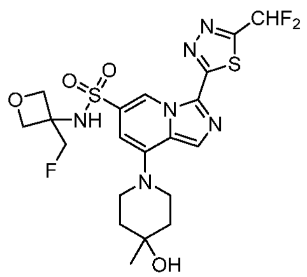
,
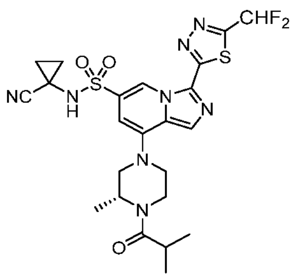
,
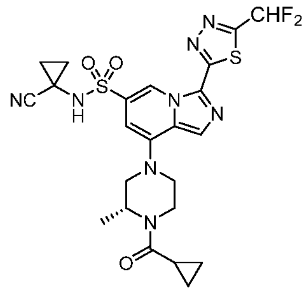
,

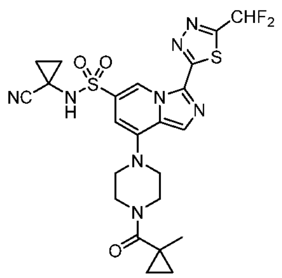
,
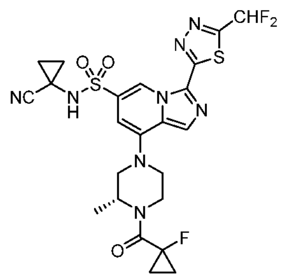
,
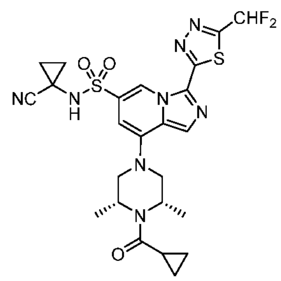
,
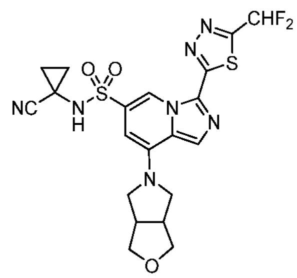
,

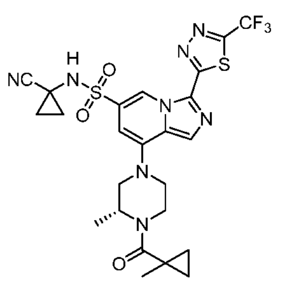
,
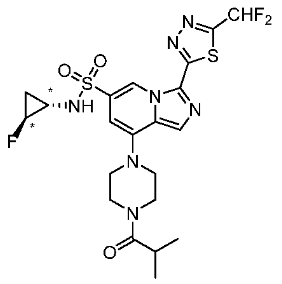
,
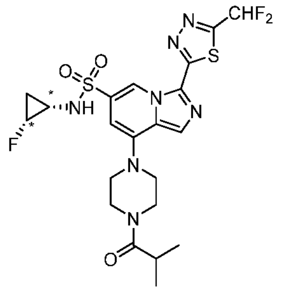
,
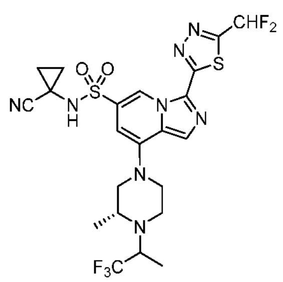
,

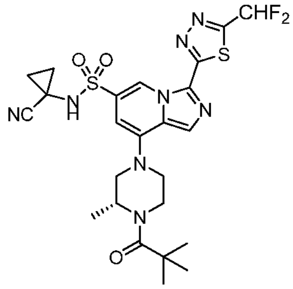 , 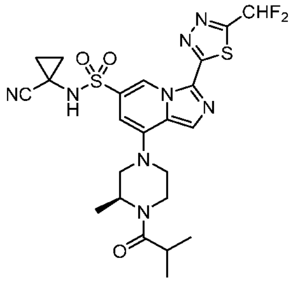 , 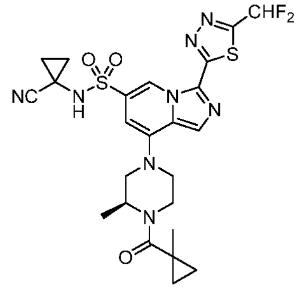 ,

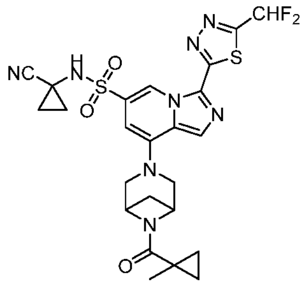 , 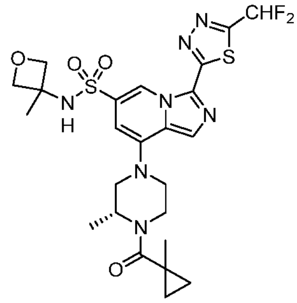 , 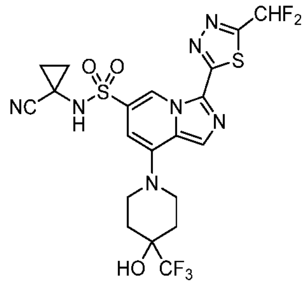 ,

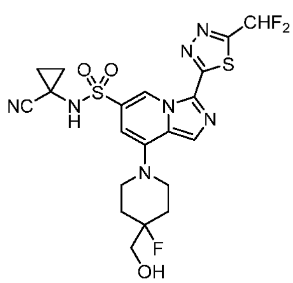 , 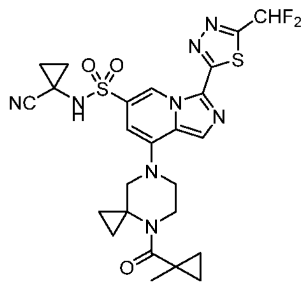 , 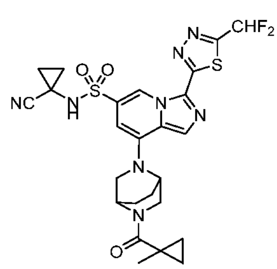 , 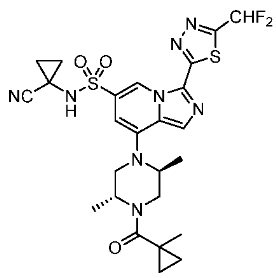 ,

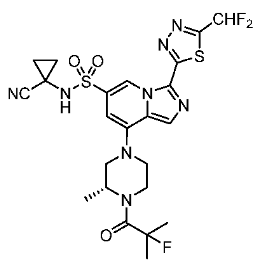 , 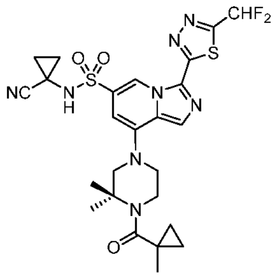 , 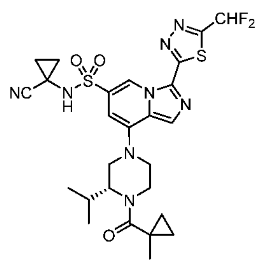 , 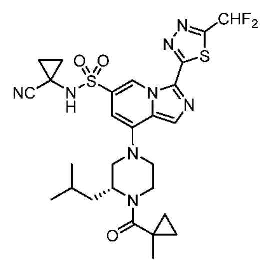 ,

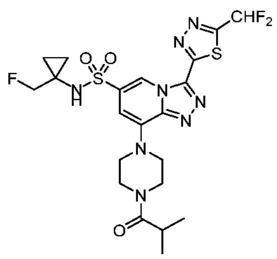 , 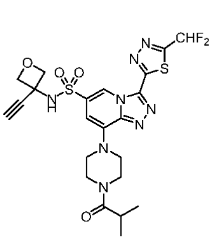 , 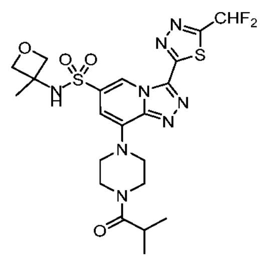 , 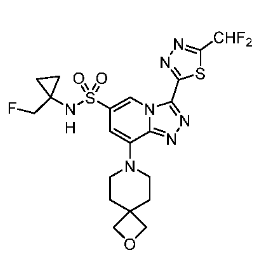 ,

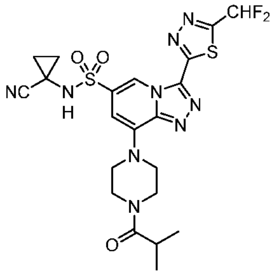,
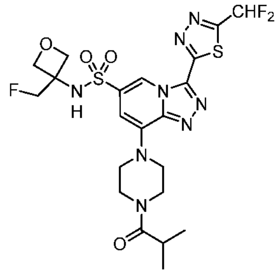,
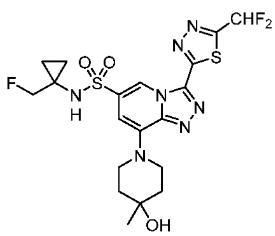,
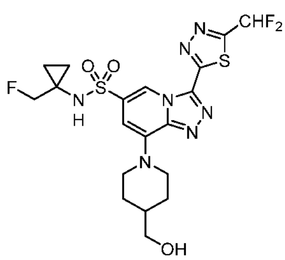,

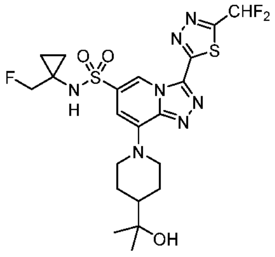,
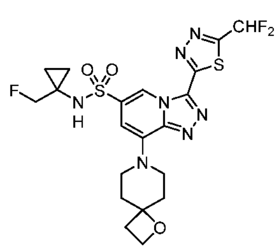,
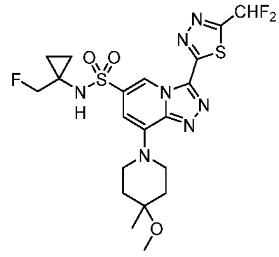,
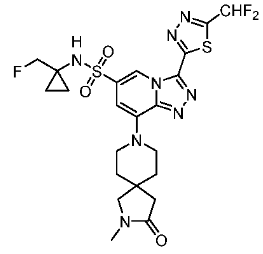,

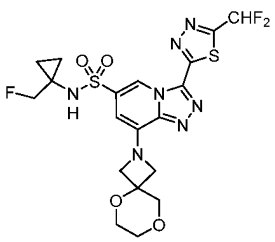,
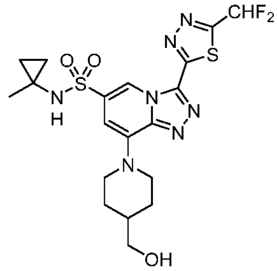,
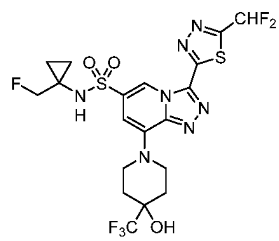,
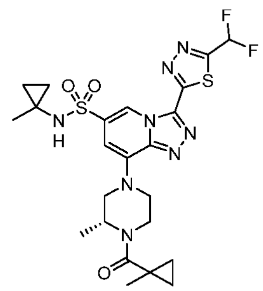,

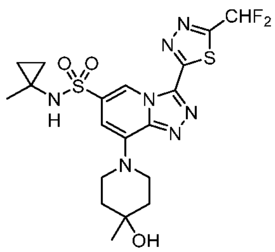,
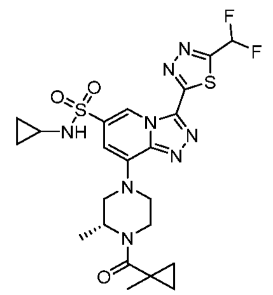,
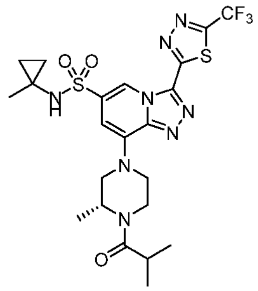,
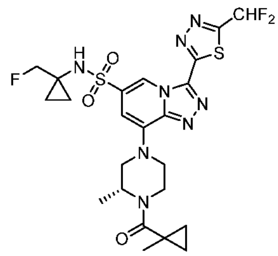,

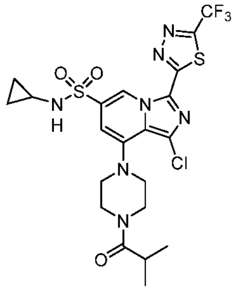,
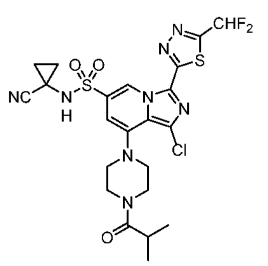,
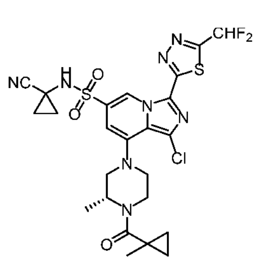,
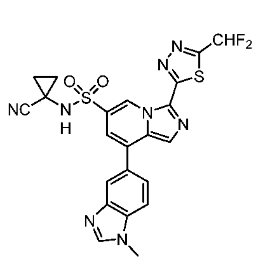,

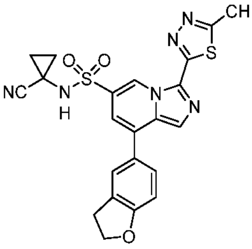 , 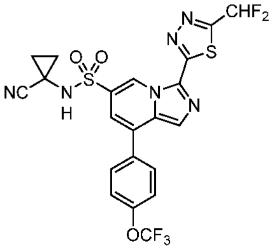 , 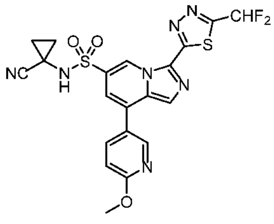 , 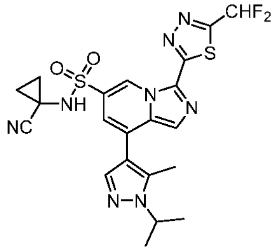 ,

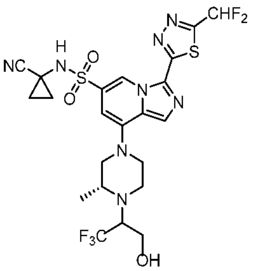 , 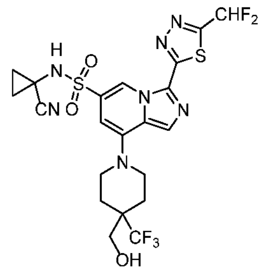 , 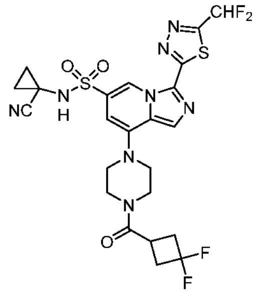 , 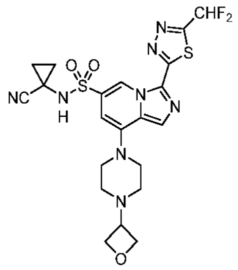 ,

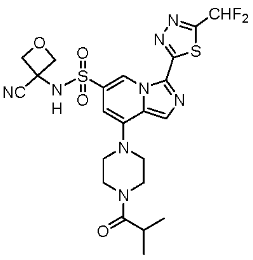 , 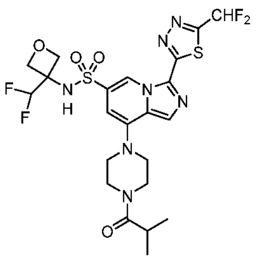 , 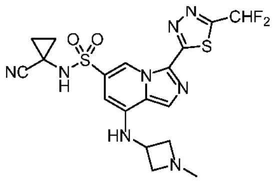 , 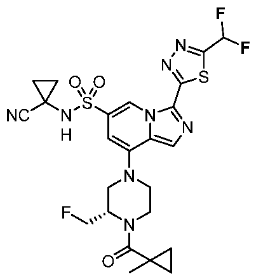 ,

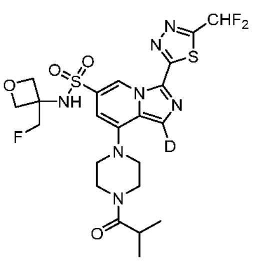 , 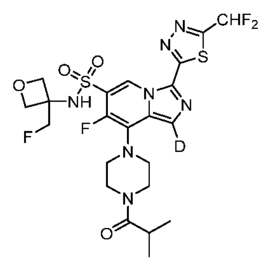 , 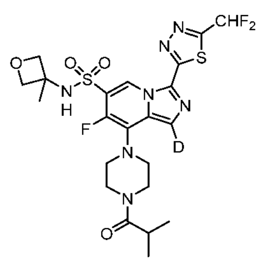 , 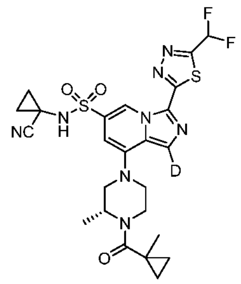 ,

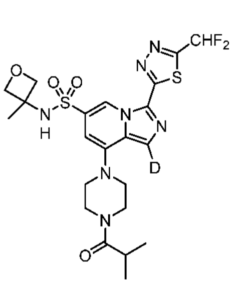 , 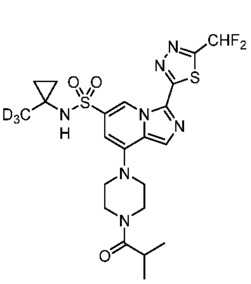 , 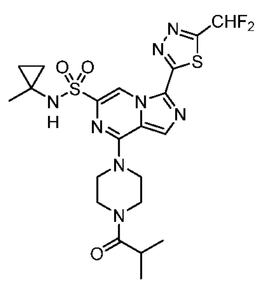 , 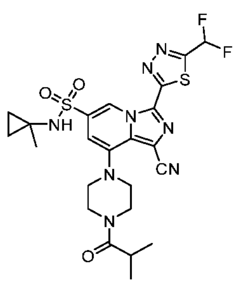 ,

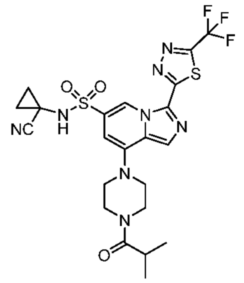,
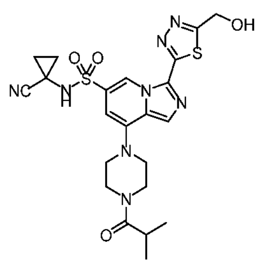,
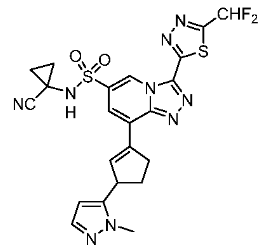,
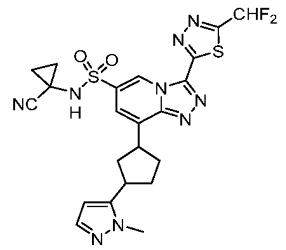,

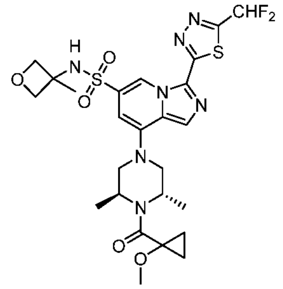,
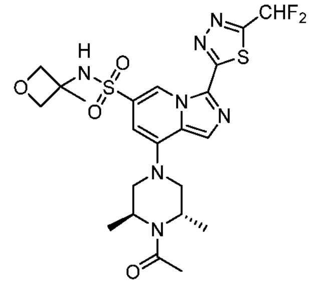,
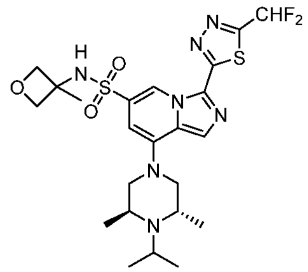,

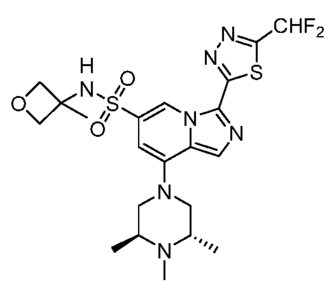,
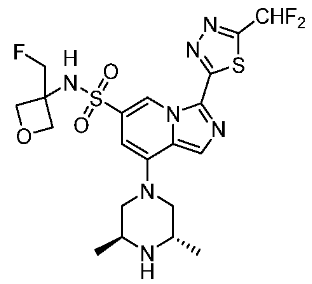,
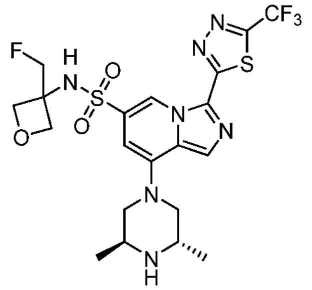,

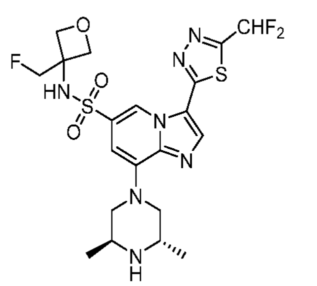,
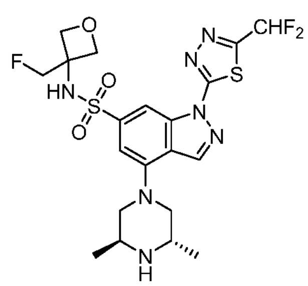,
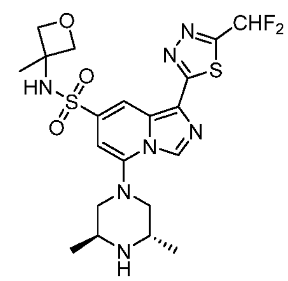,

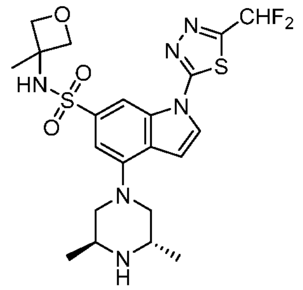,
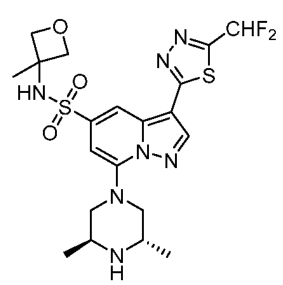,
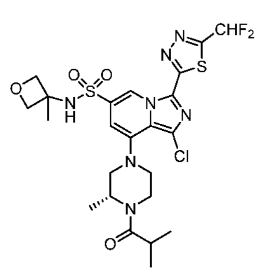,
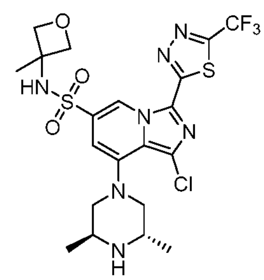,

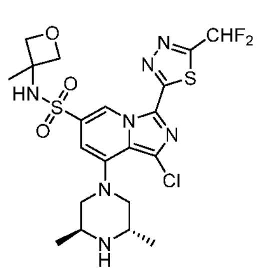,
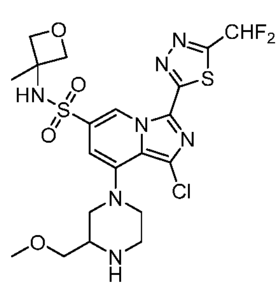,
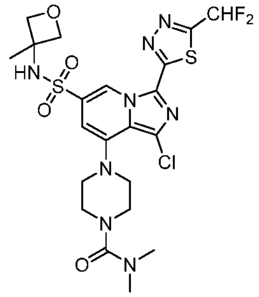,
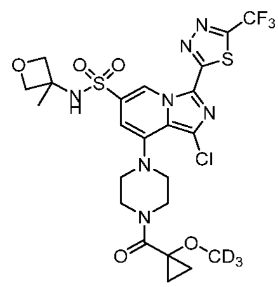,

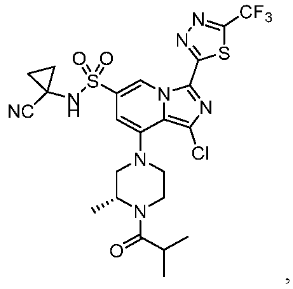,
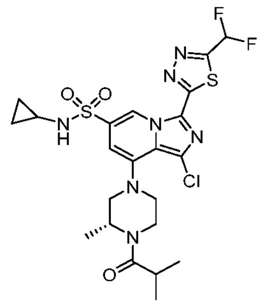,
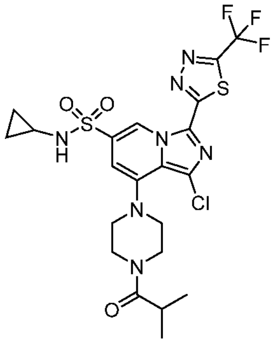,
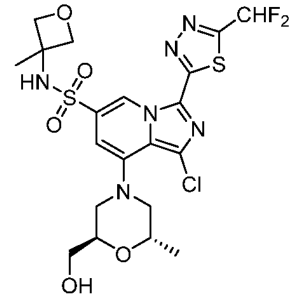,

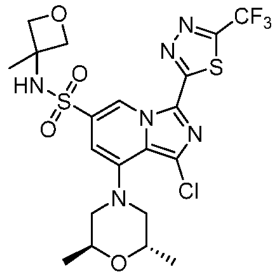,
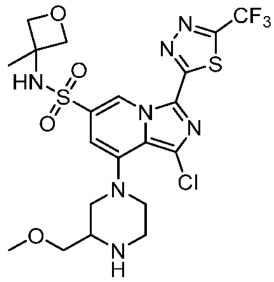,
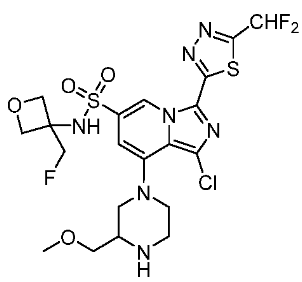,
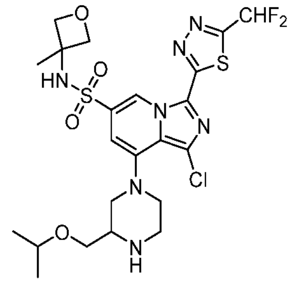,

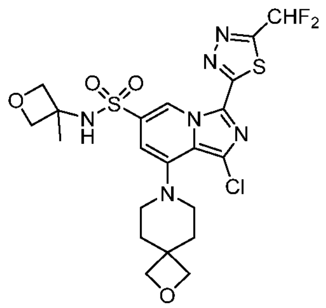,
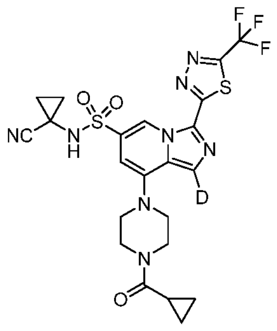,
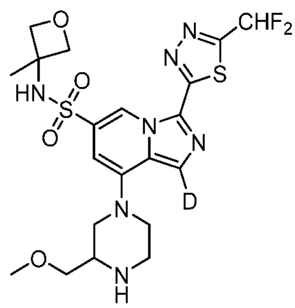,

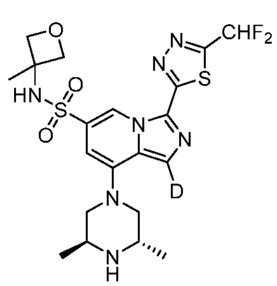,
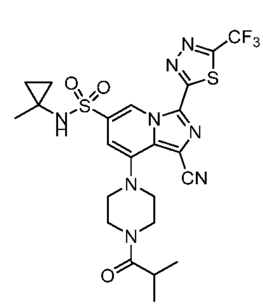,
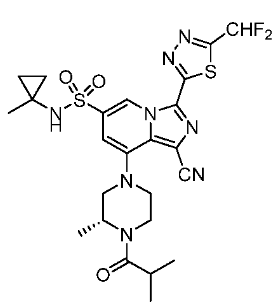,
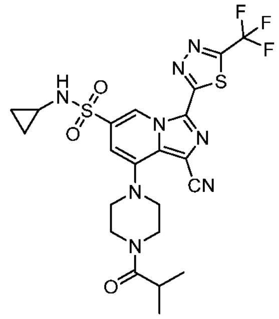,

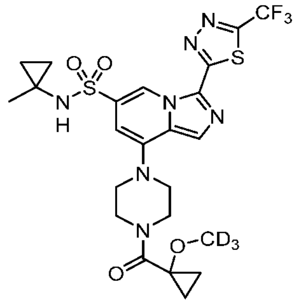 , 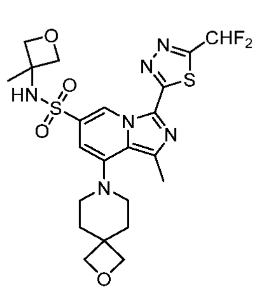 , 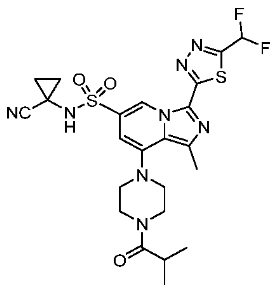 , 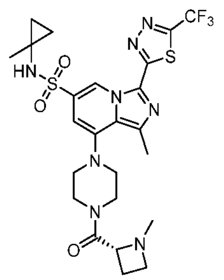 ,

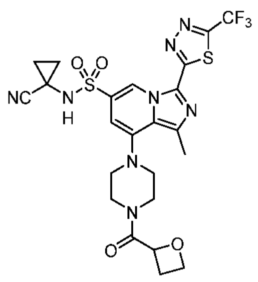 , 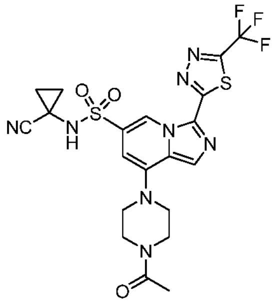 , 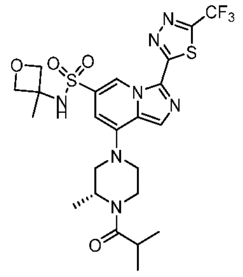 , 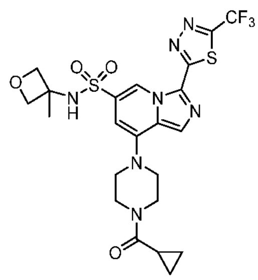 ,

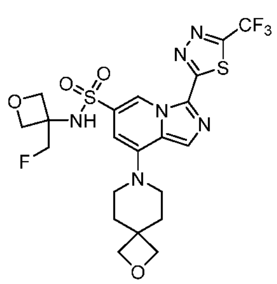 , 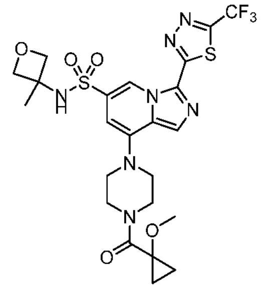 , 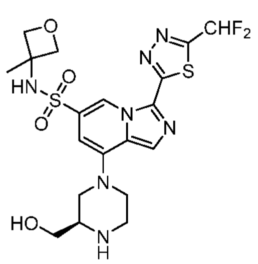 , 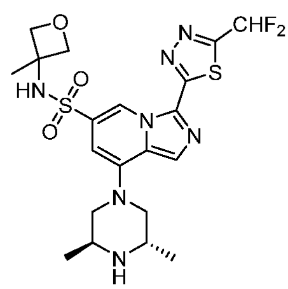 ,

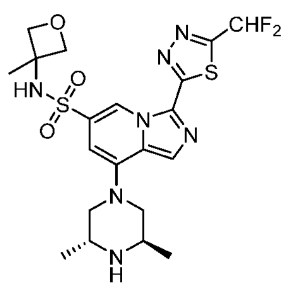 , 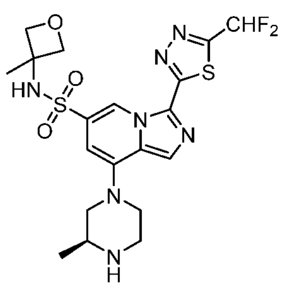 , 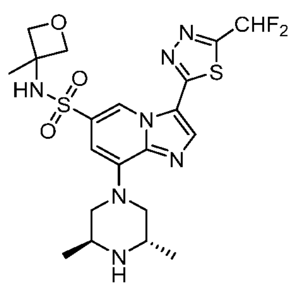 , 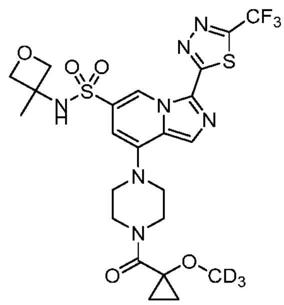 ,

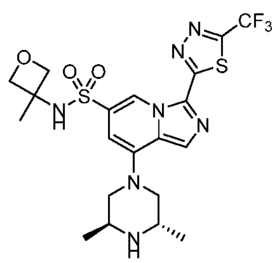 , 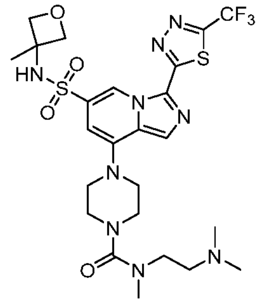 , 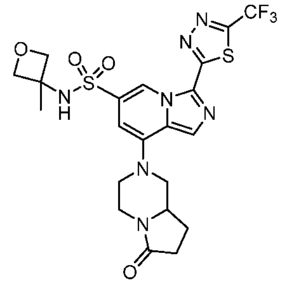 , 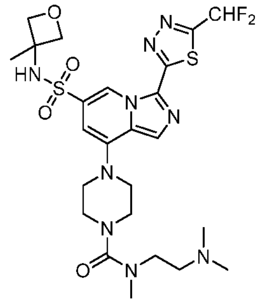 ,

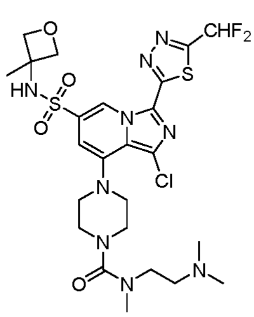 , 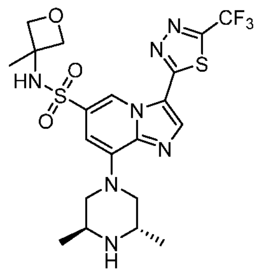 , 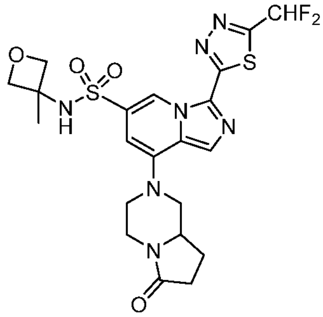 , 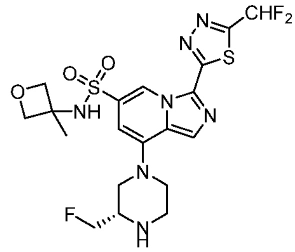 ,

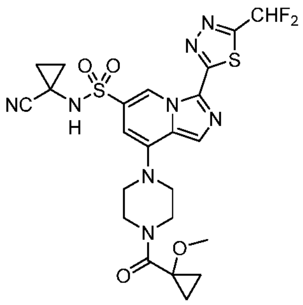 , 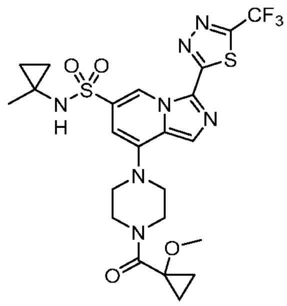 , 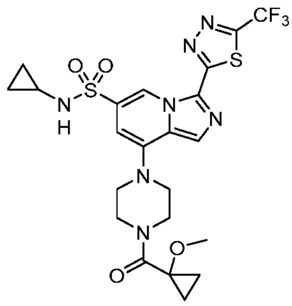 ,

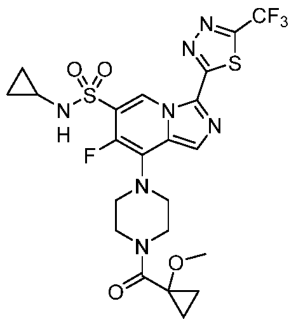 , 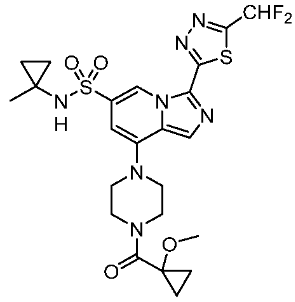 , 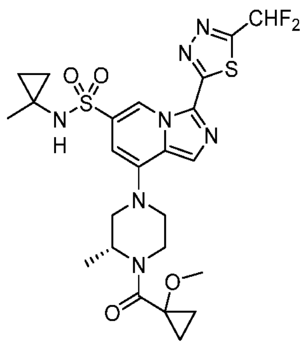 ,

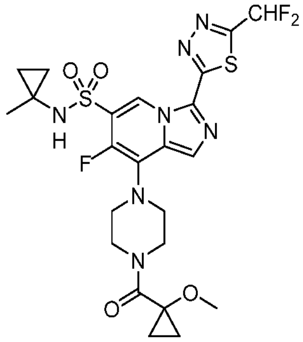 , 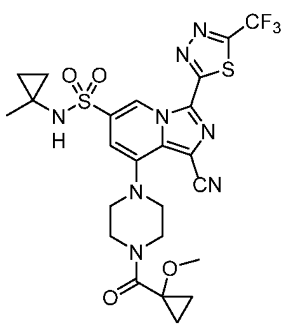 , 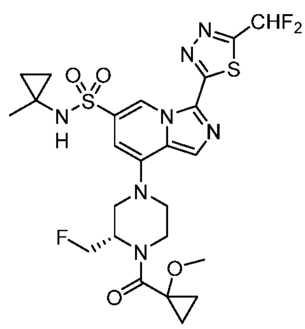 ,

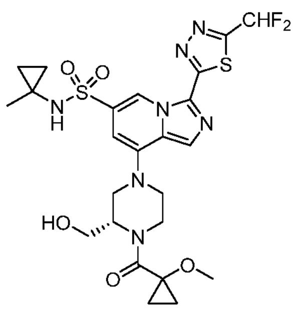,
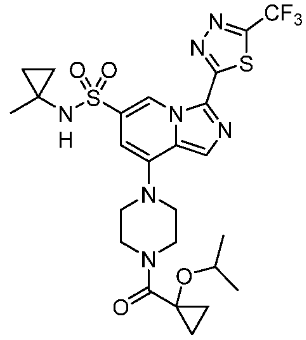,
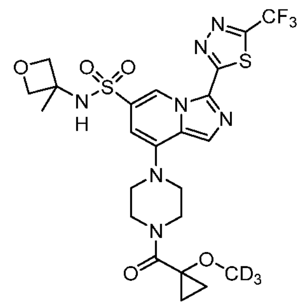,

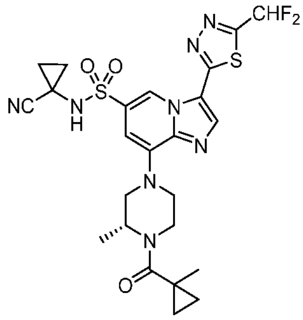,
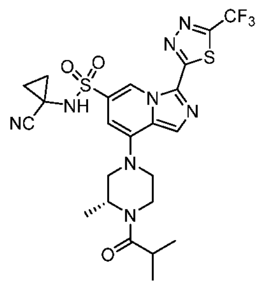,
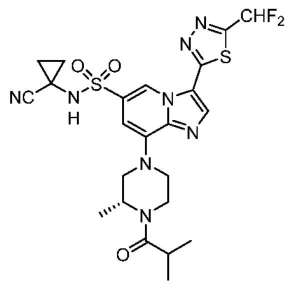,
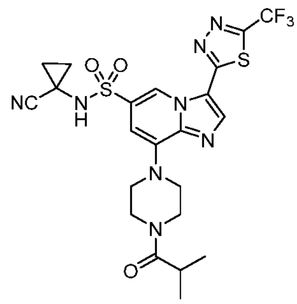,

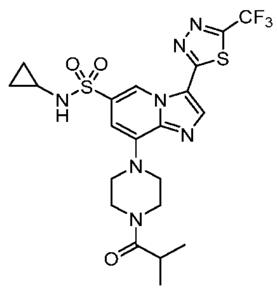,
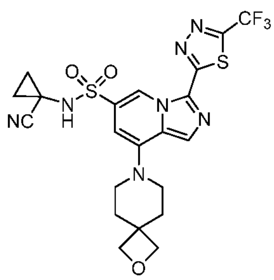,
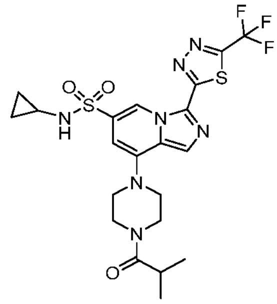,
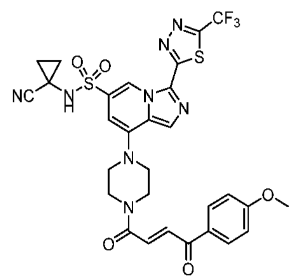,

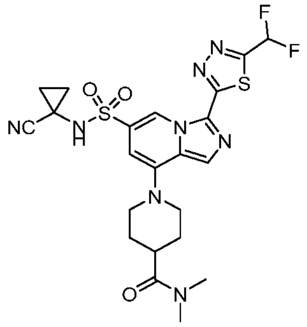,
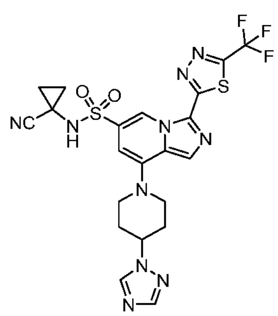,
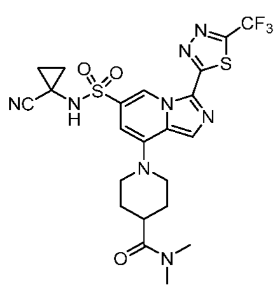,
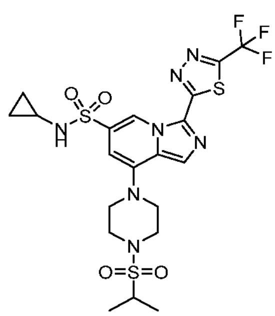,

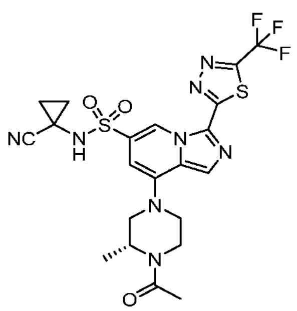,
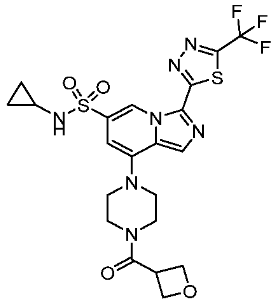,
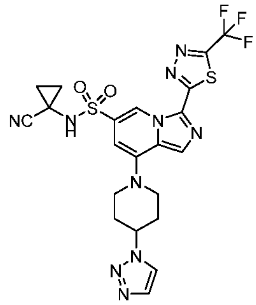,
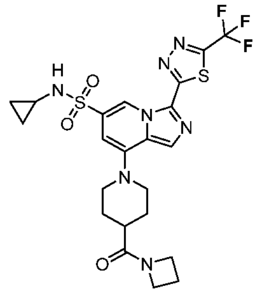,

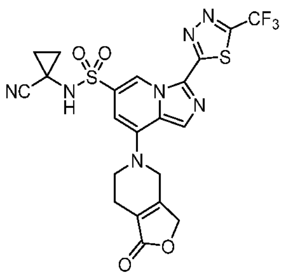,
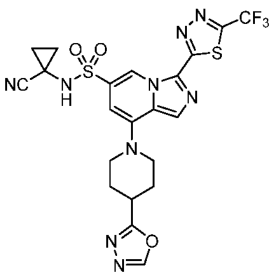,
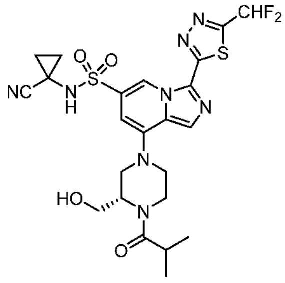,
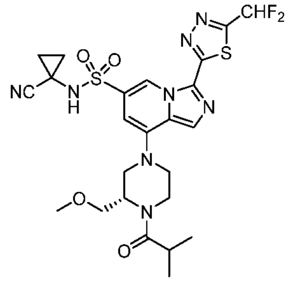,

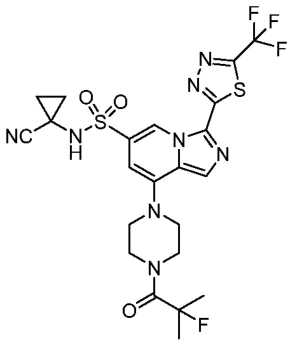,
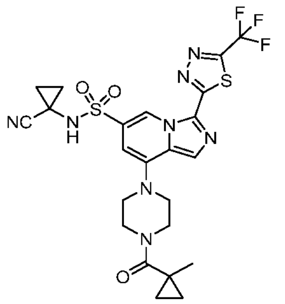,
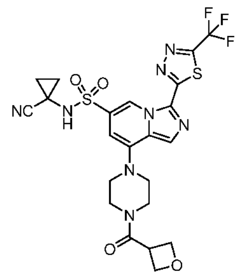,
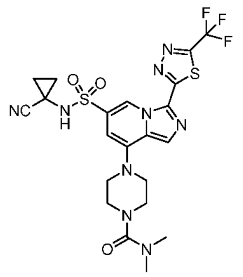,

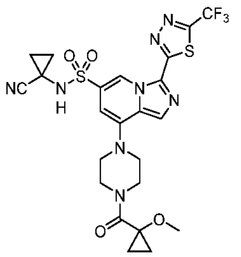,
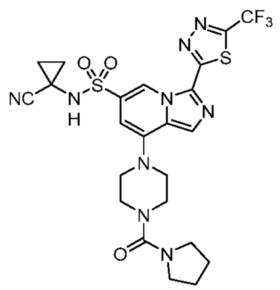,
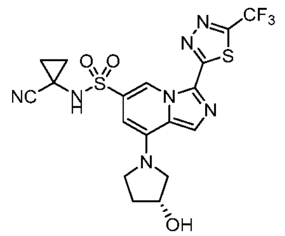,
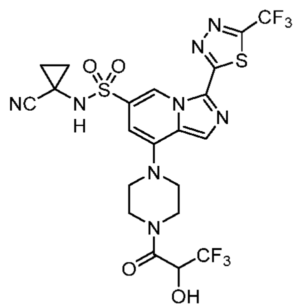,

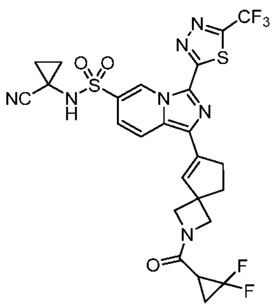,
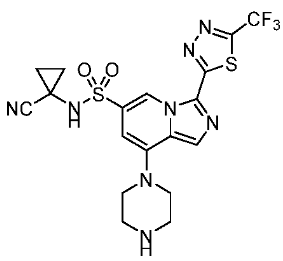,
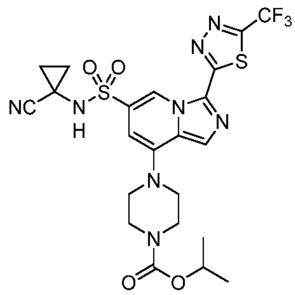,
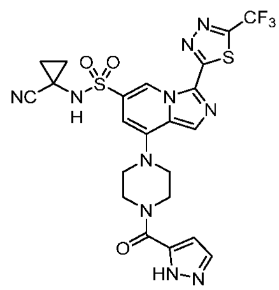,

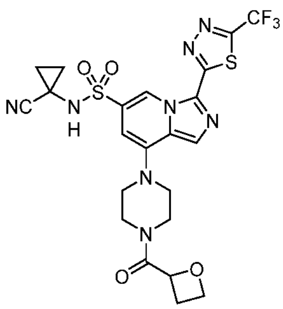,
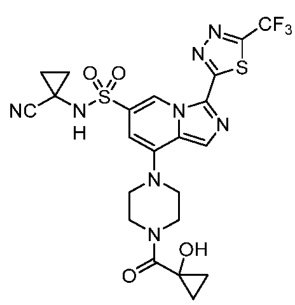,
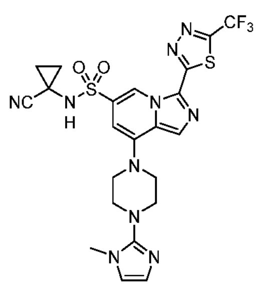,
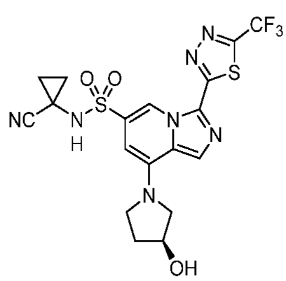,

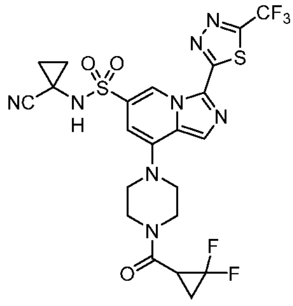 , 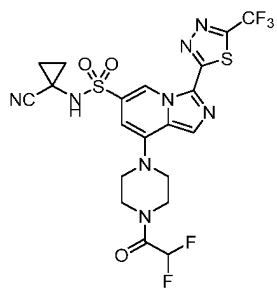 , 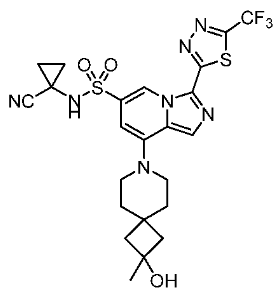 , 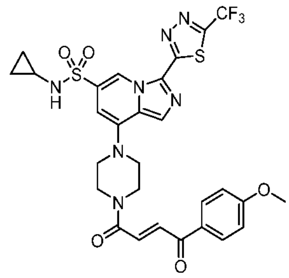 ,

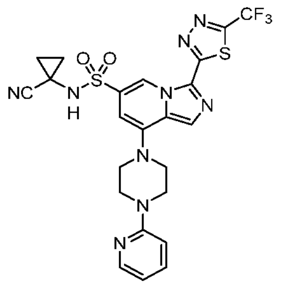 , 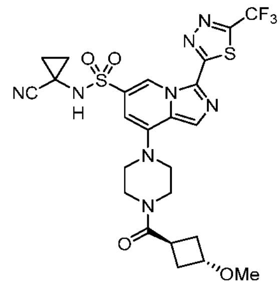 , 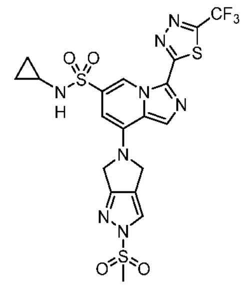 , 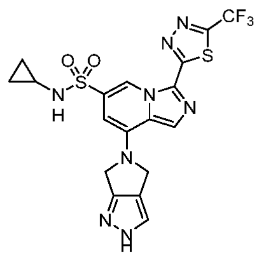 ,

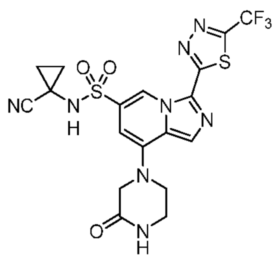 , 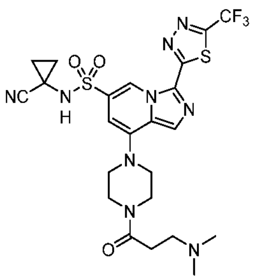 , 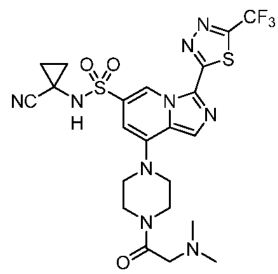 , 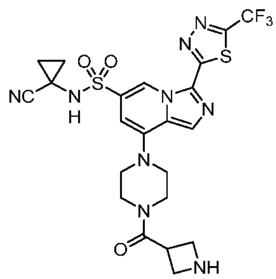 ,

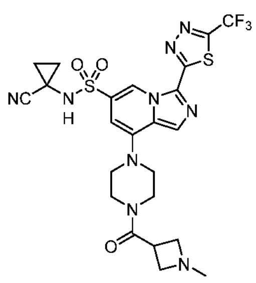 , 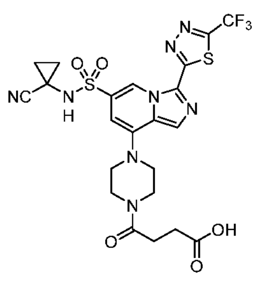 , 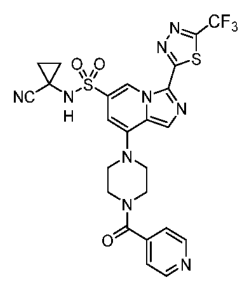 , 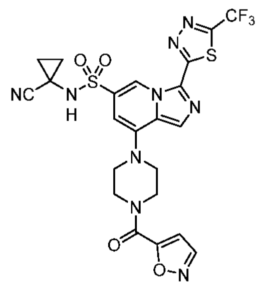 ,

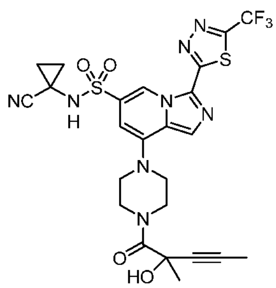 , 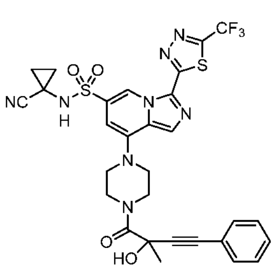 , 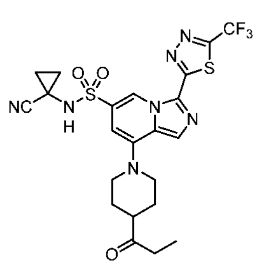 , 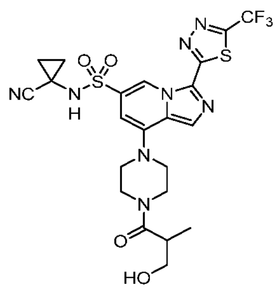 ,

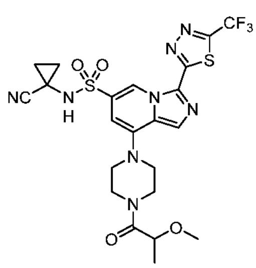 ,
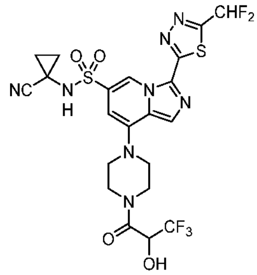 ,
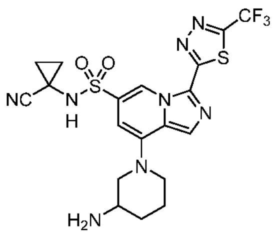 ,
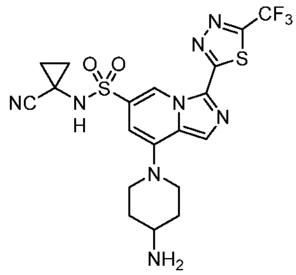 ,

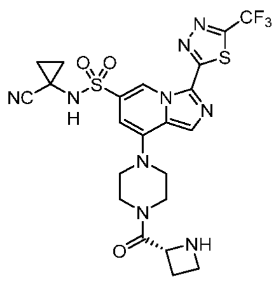 ,
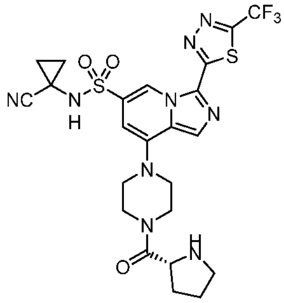 ,
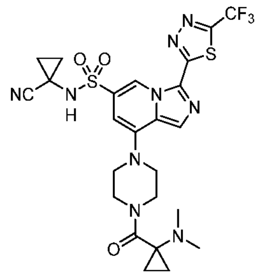 ,
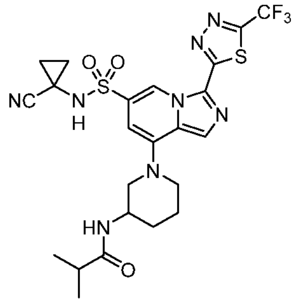 ,

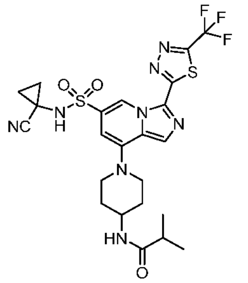 ,
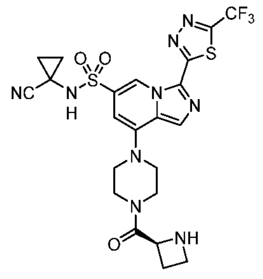 ,
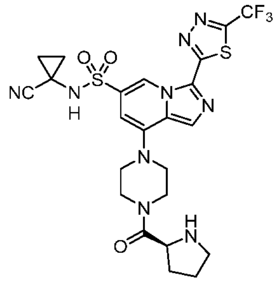 ,
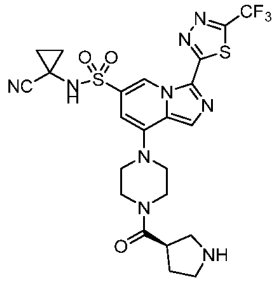 ,

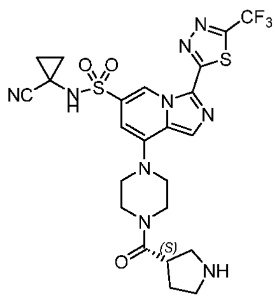 ,
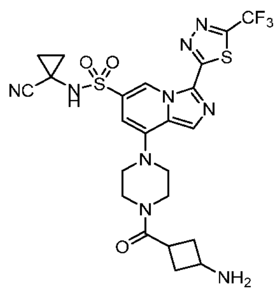 ,
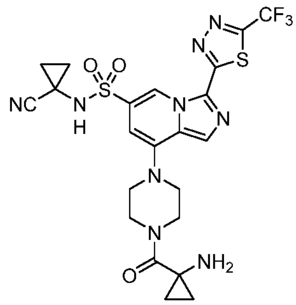 ,
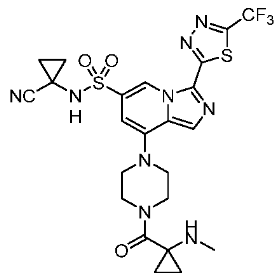 ,

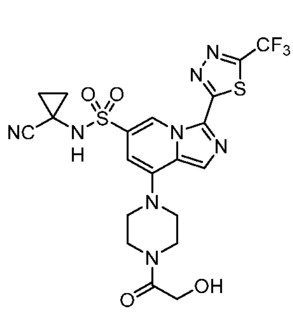 ,
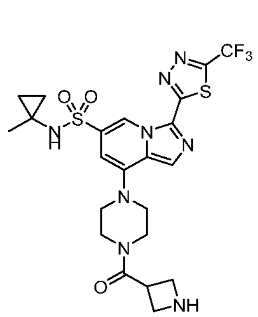 ,
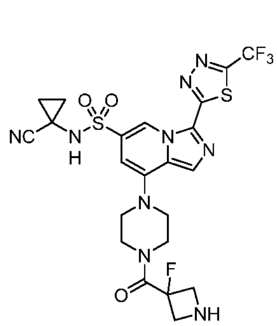 ,
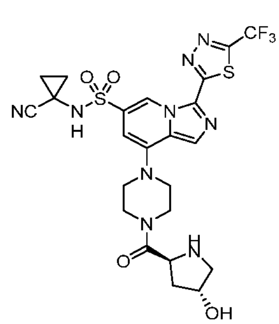 ,

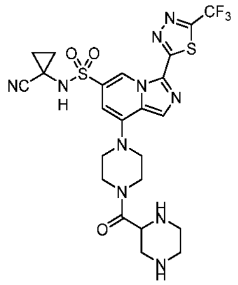 , 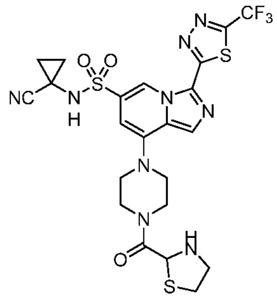 , 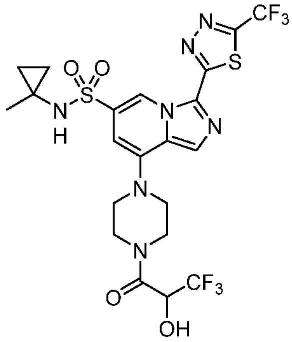 , 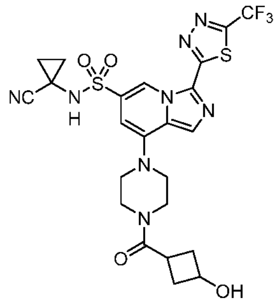 ,

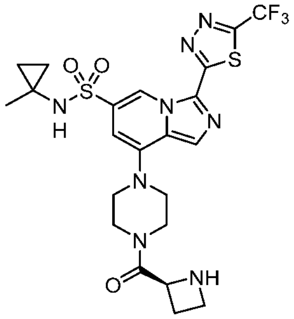 , 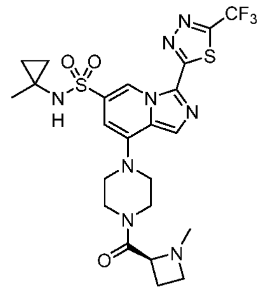 , 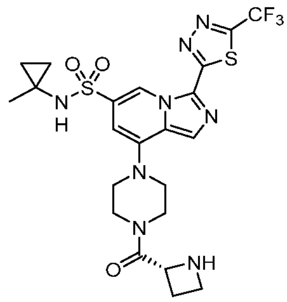 , 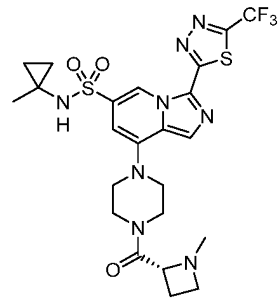 ,

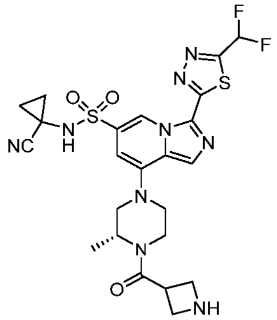 , 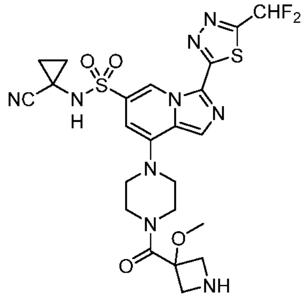 , 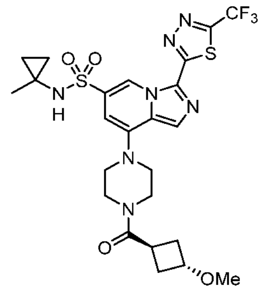 , 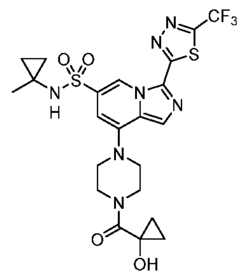 ,

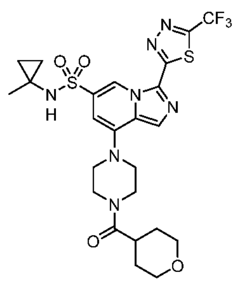 , 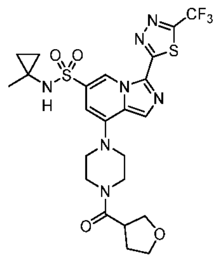 , 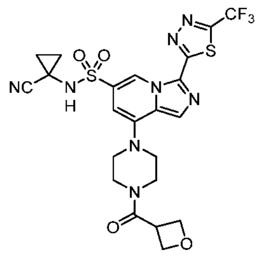 , 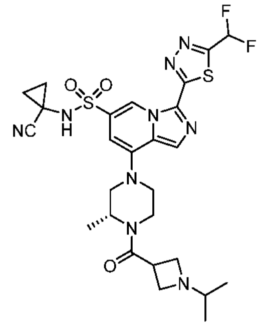 ,

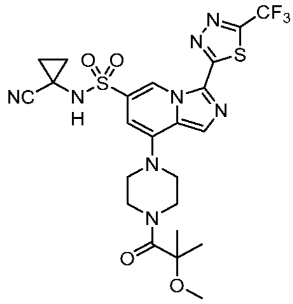 , 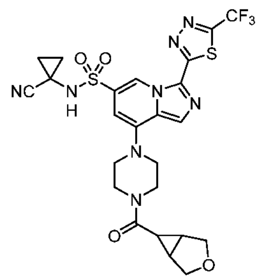 , 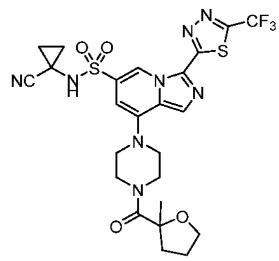 , 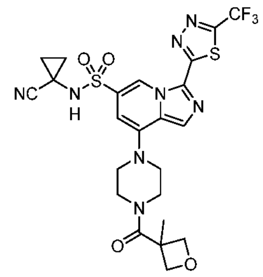 ,

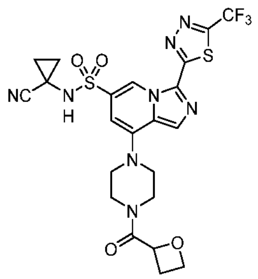 , 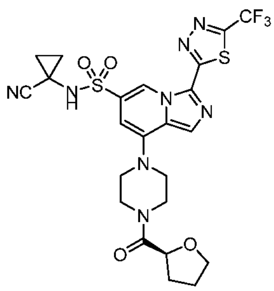 , 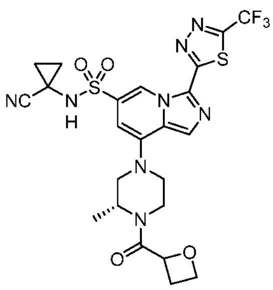 , 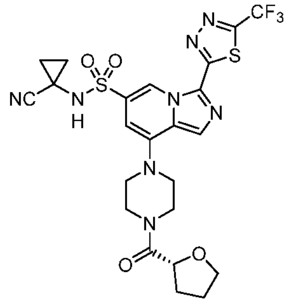 ,

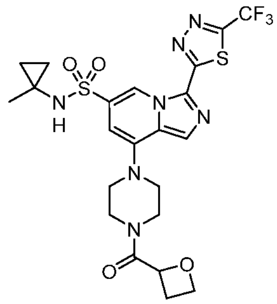 , 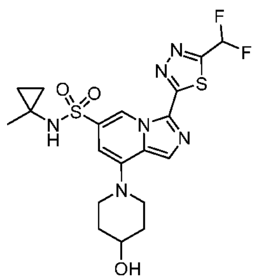 , 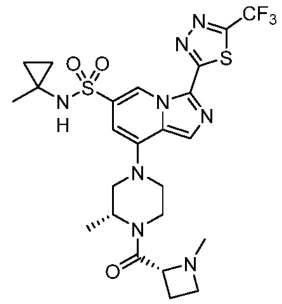 , 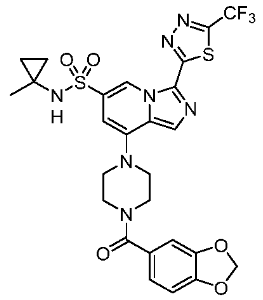 ,

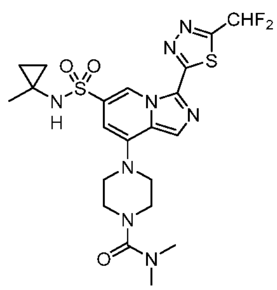 , 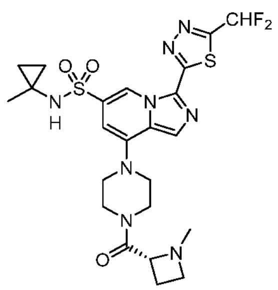 , 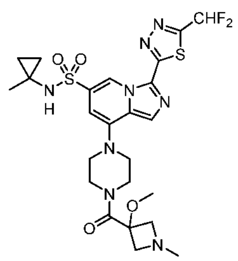 , 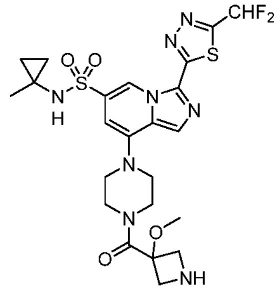 ,

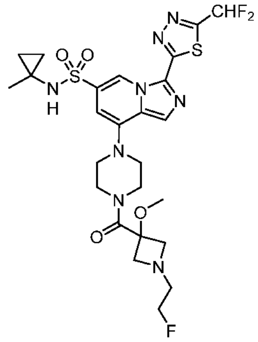 , 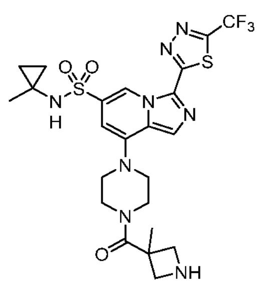 , 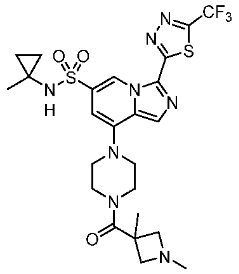 , 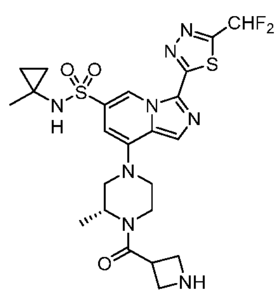 ,

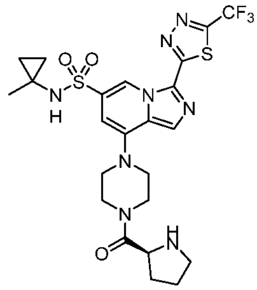 , 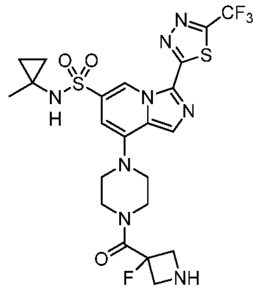 , 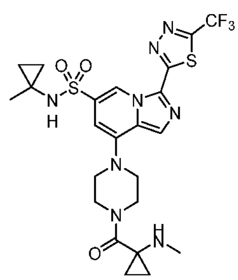 , 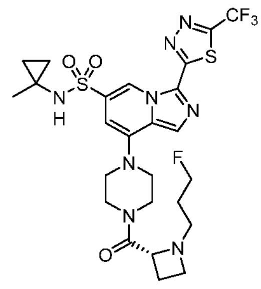 ,

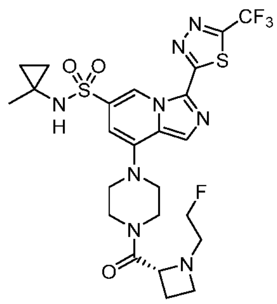 , 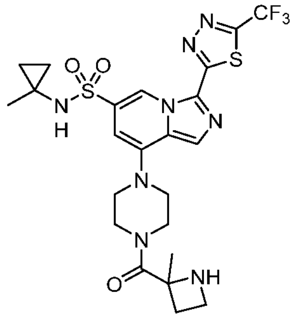 , 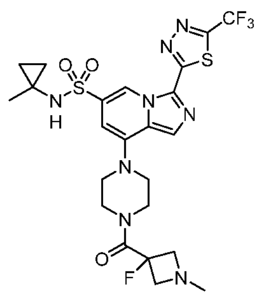 , 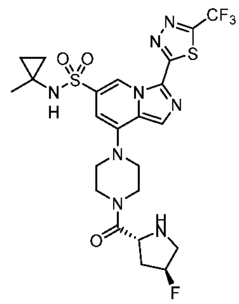 ,

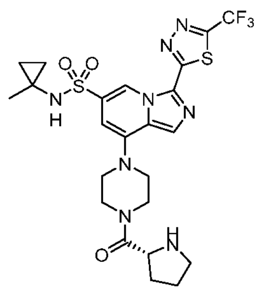 , 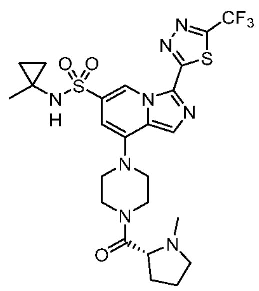 , 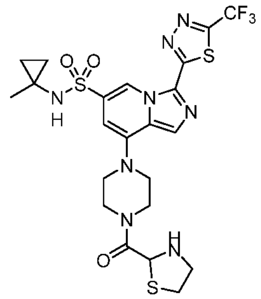 , 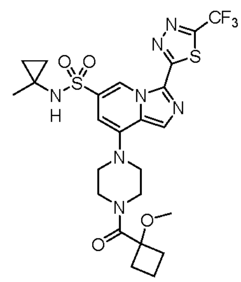 ,

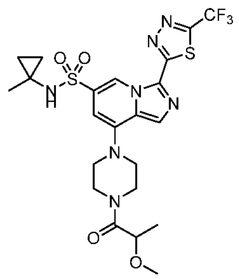 , 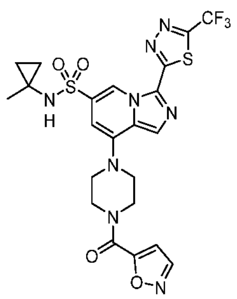 , 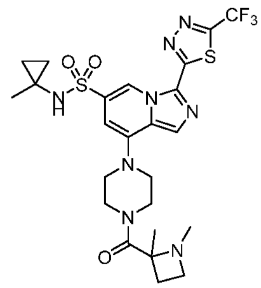 , 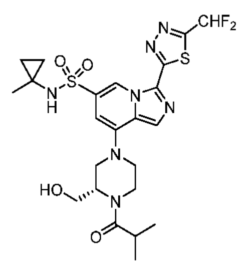 ,

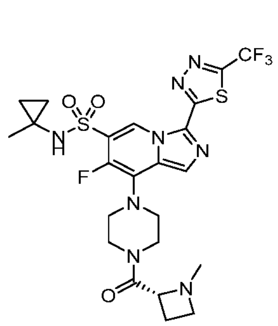 , 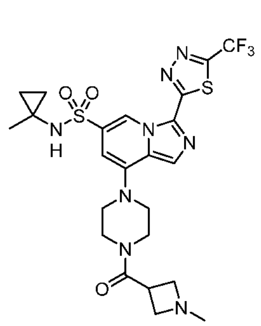 , 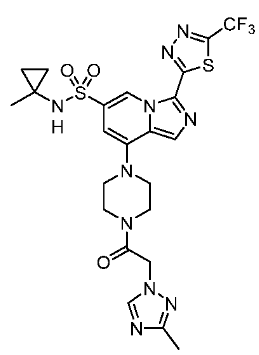 , 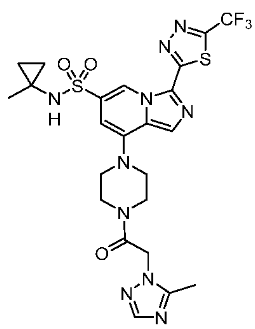 ,

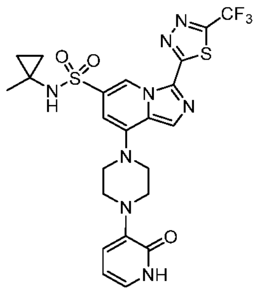 , 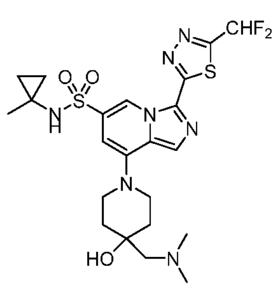 , 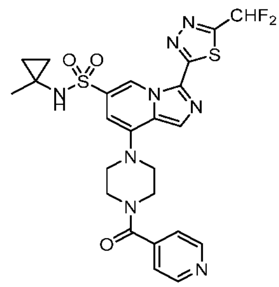 , 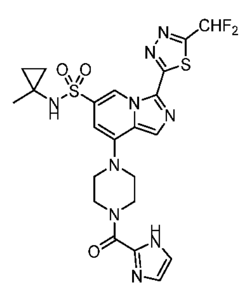 ,

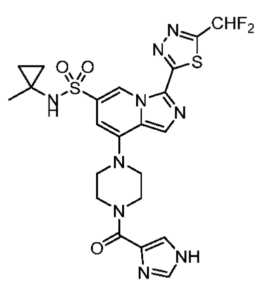 , 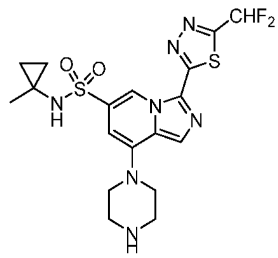 , 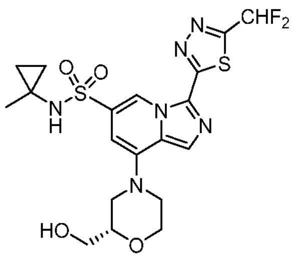 , 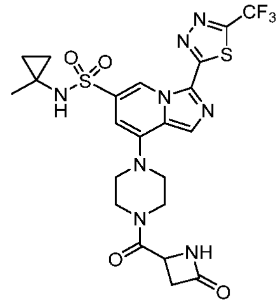 ,

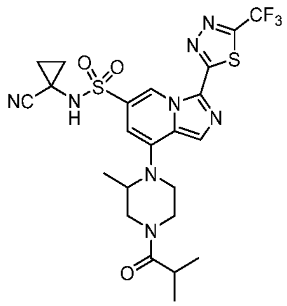 , 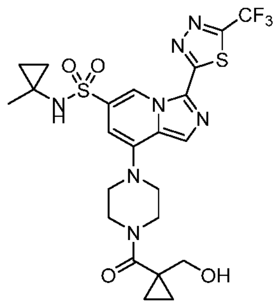 , 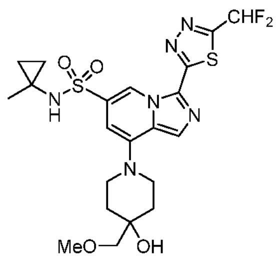 , 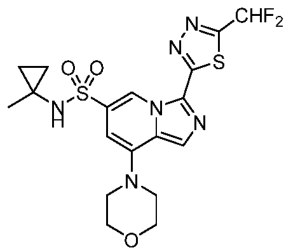 ,

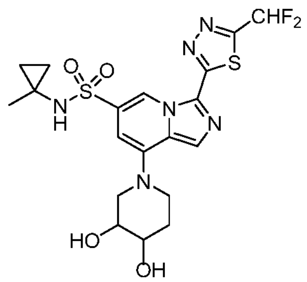 , 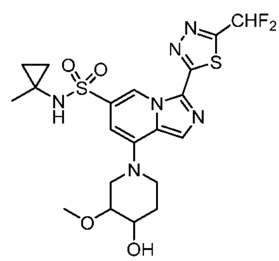 , 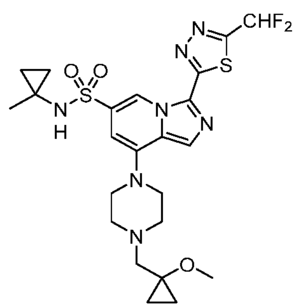 , 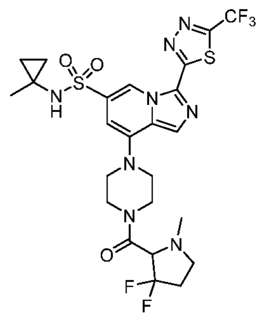 ,

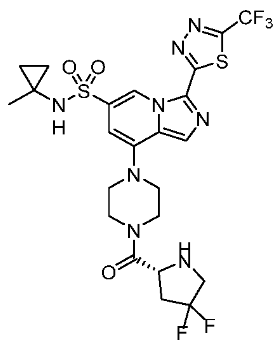 , 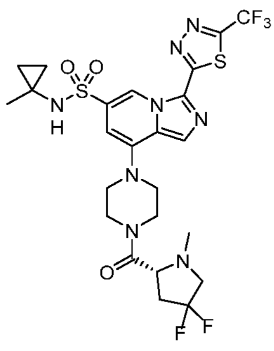 , 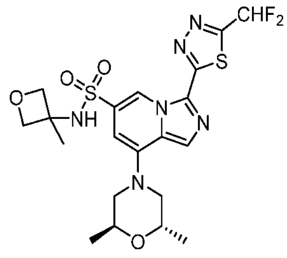 , 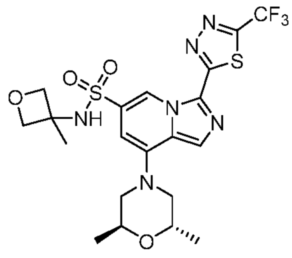 ,

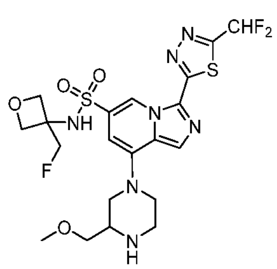 , 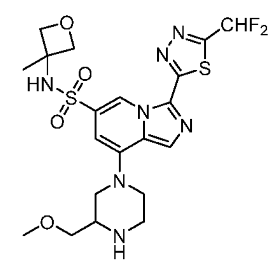 , 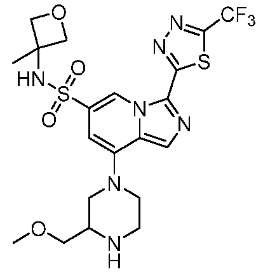 , 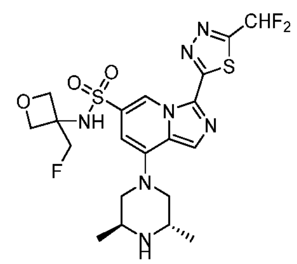 ,

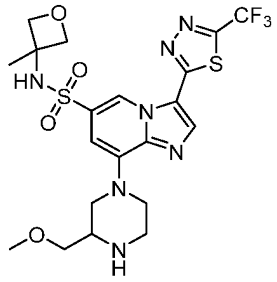 , 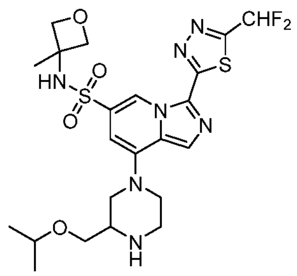 , 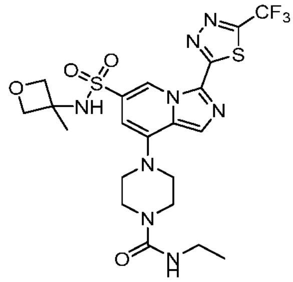 , 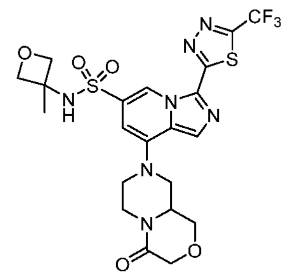 ,

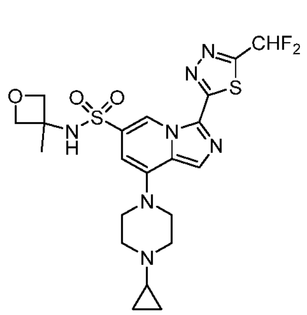 , 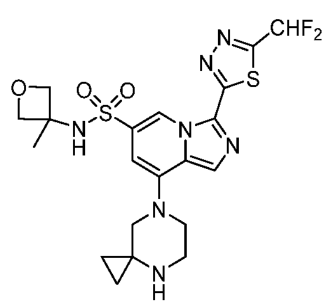 , 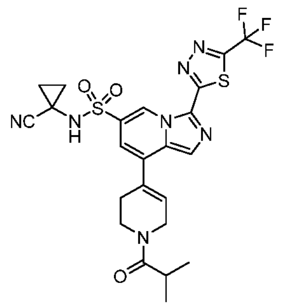 ,

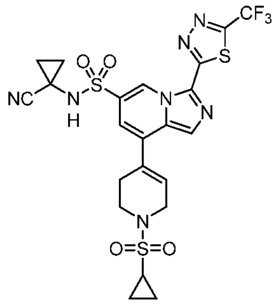 , 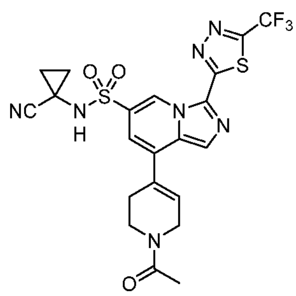 , 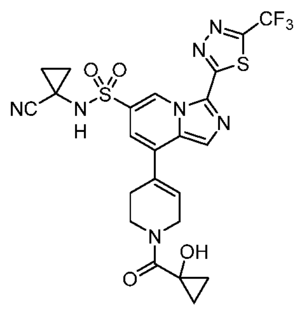 , 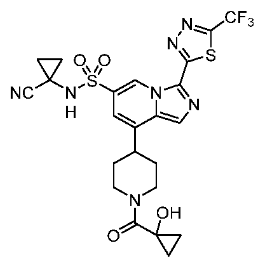 ,

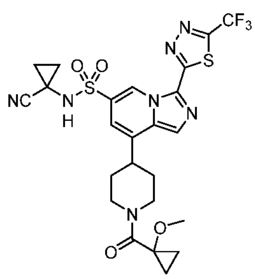 , 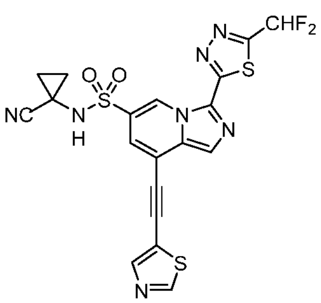 , 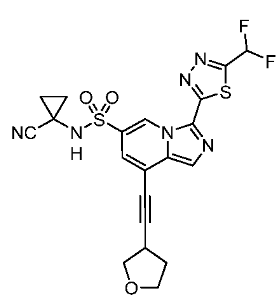 , 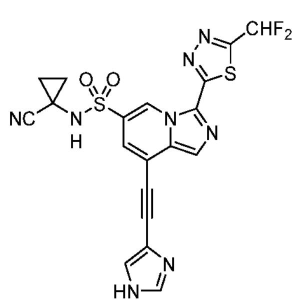 ,

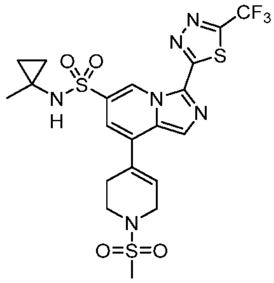 , 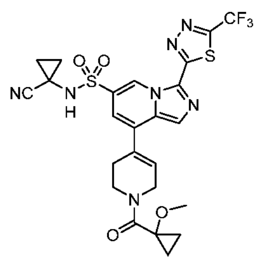 , 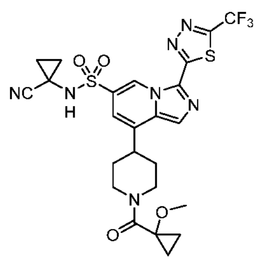 , 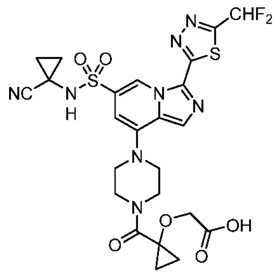 ,

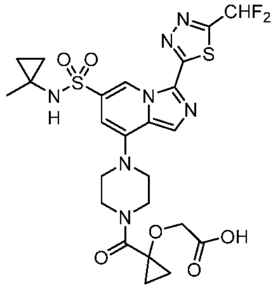 , 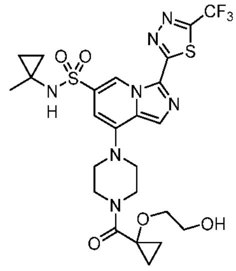 , 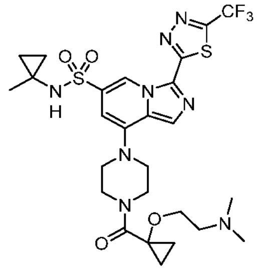 , 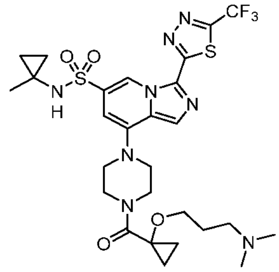 ,

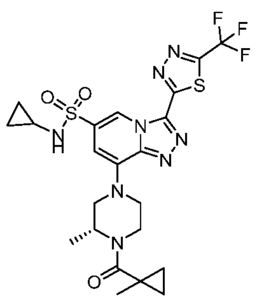 , 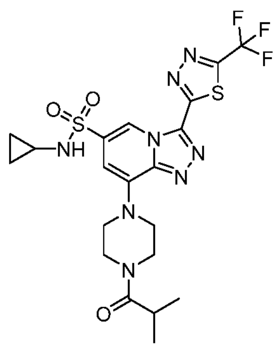 , 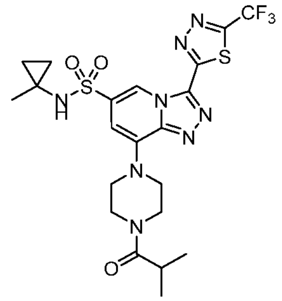 , 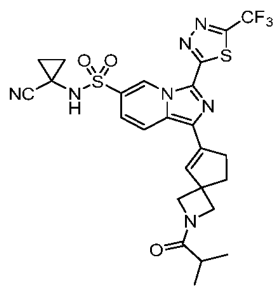 ,

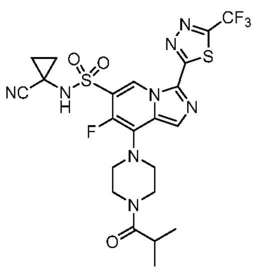 , 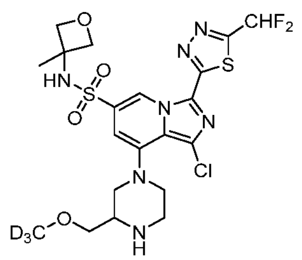 , 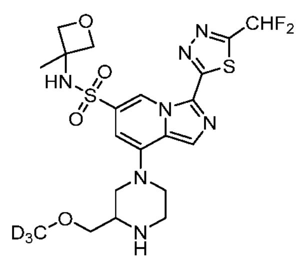 , 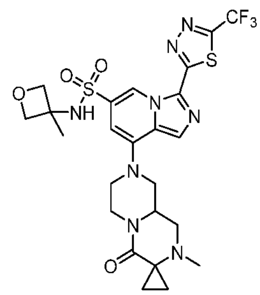 ,

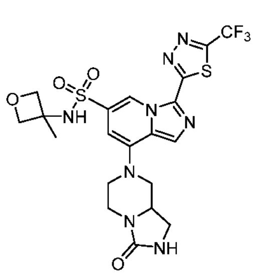 , 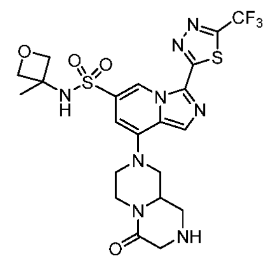 , 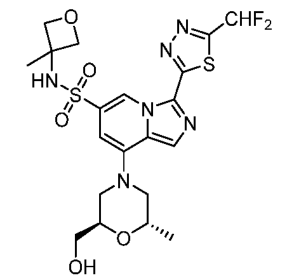 , 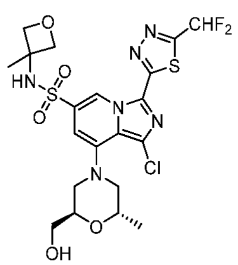 ,

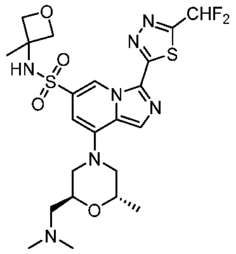 , 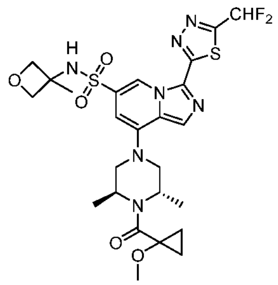 , 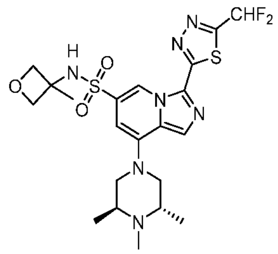 , 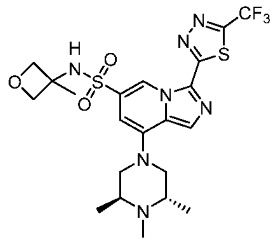 ,

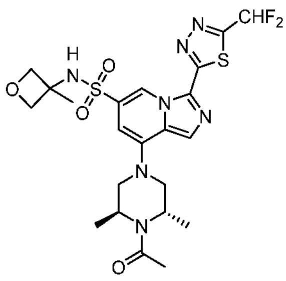 , 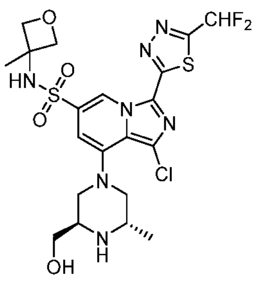 , 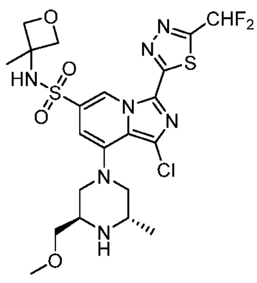 or 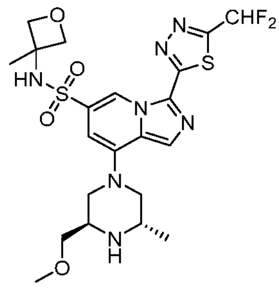

**21.** A pharmaceutical composition, comprising a substance Z and a pharmaceutic excipient, wherein said substance Z is the compound of formula I, the pharmaceutical composition thereof, or the isotopic compound thereof according to any one of claims 1-20.

**22.** Use of a substance Z in preparation of a PARG inhibitor and a medicament for treating and/or preventing PARG-related diseases, wherein said substance Z is a compound of formula I, a pharmaceutically acceptable salt thereof or an isotopic compound thereof, and the PARG-related diseases can be breast cancer, for example, triple negative breast cancer.

**23.** Use of a substance Z in preparation of a medicament for treating and/or preventing breast cancer, wherein said substance Z is the compound of formula I, the pharmaceutically acceptable salt thereof or the isotopic compound thereof according to any one of claims 1-20, and said breast cancer can be triple negative breast cancer.

**24.** The compound of formula II, III, IV or V or a salt thereof,

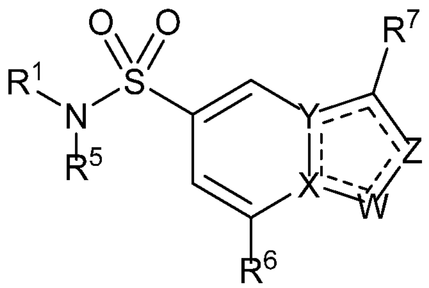

II ,

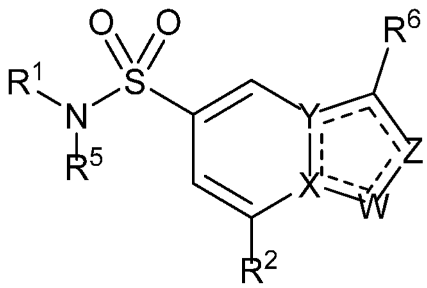

III ,

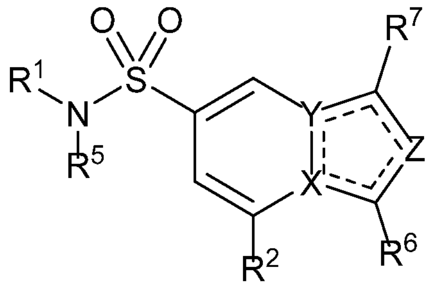

IV

, or

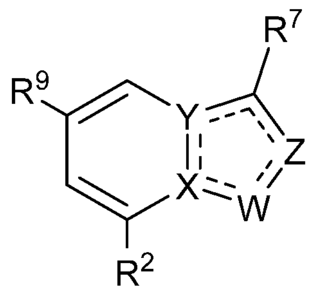

V

,

wherein, $R^5$ is hydrogen or PMB; $R^6$ is halogen, deuterium; $R^7$ is $R^3$, hydrogen or

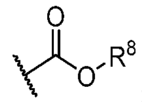

;

$R^8$ is hydrogen or $C_1$-$C_6$ alkyl; $R^9$ is

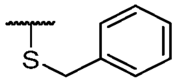

,

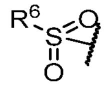

or halogen;

$R^1$, $R^2$, $R^3$, X, Y, Z and W are defined as in any one of claims 1-19;

preferably, in $R^6$, said halogen can be fluorine, chlorine, bromine or iodine, for example, chlorine or bromine;

and/or, in $R^8$, said $C_1$-$C_6$ alkyl can be $C_1$-$C_4$ alkyl, and can be methyl, ethyl, n-propyl, isopropyl, n-butyl, isobutyl, pr-butyl, sec-butyl or tert-butyl, or can be methyl or ethyl;

and/or, in $R^9$, said halogen can be fluorine, chlorine, bromine or iodine, for example, chlorine or bromine.

**25.** The compound of formula II, III, IV or V or the salt thereof according to claim 24, wherein the compound of formula II is of any one of the following structures:

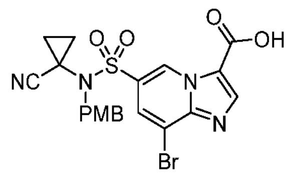

,

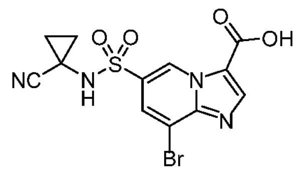

,

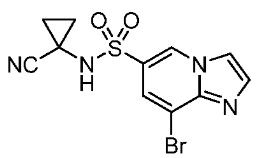

,

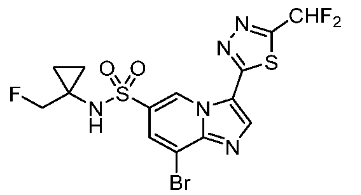

,

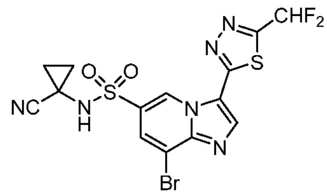

,

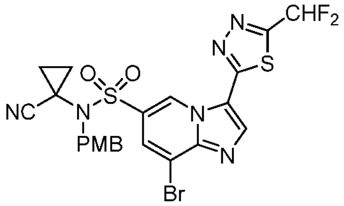, 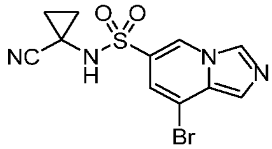, 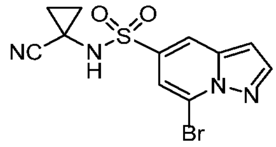,

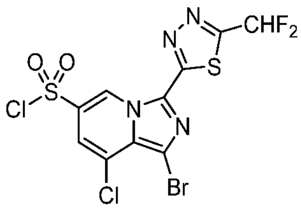, 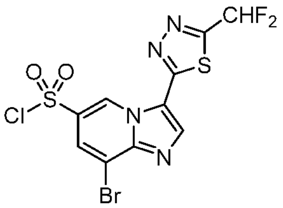, 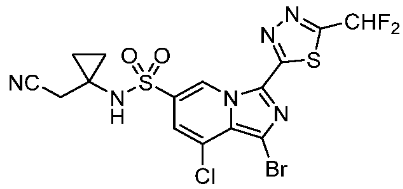,

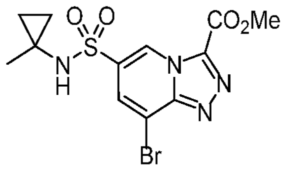, 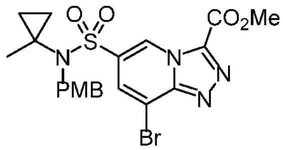, 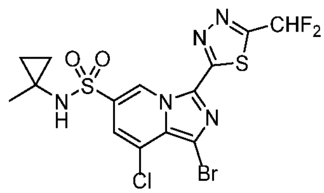,

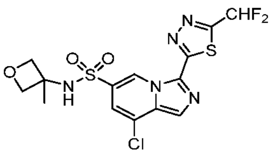, 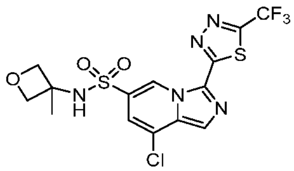, 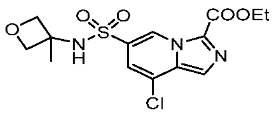, 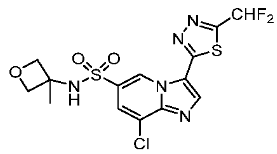,

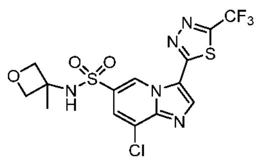, 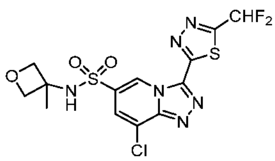, 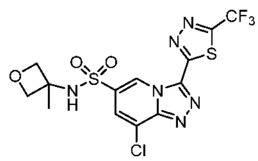, 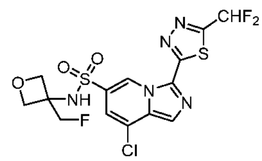

or

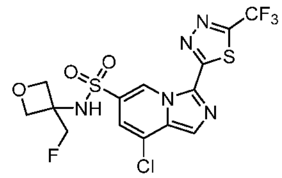;

and/or , said compound of formula III is of any one of the following structures:

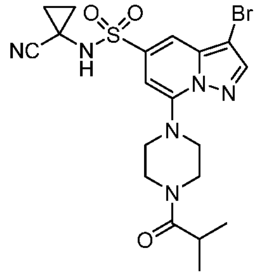,

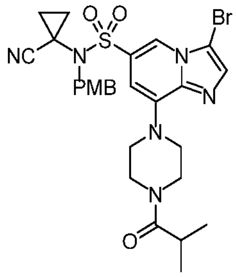 or 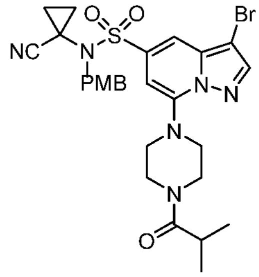 ;

and/or , said compound of formula IV is of any one of the following structures:

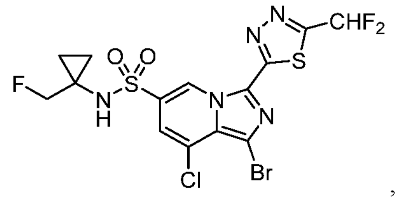 ,

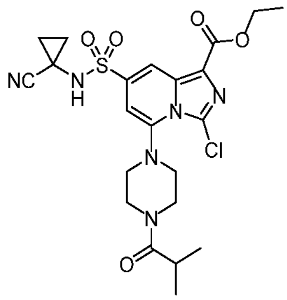 , 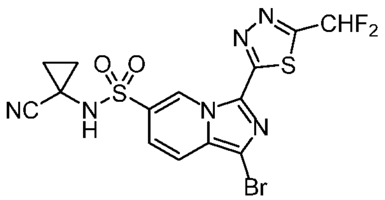 , 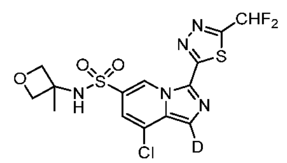 ,

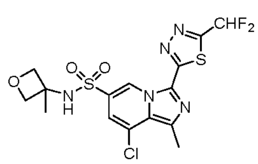 , 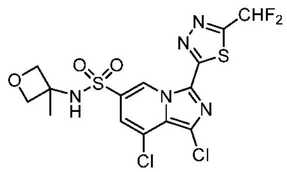 , 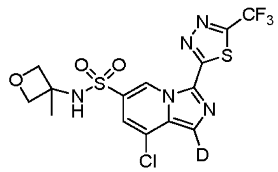 , 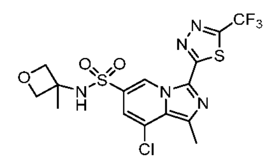 ,

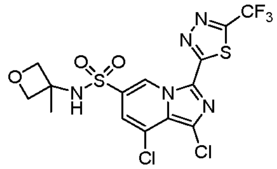 , 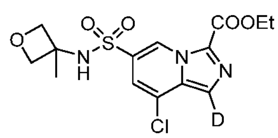 , 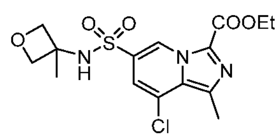 , 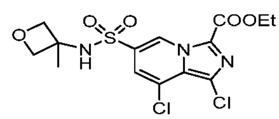 ,

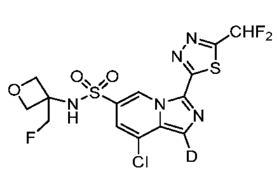 , 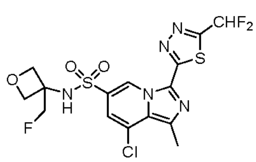 , 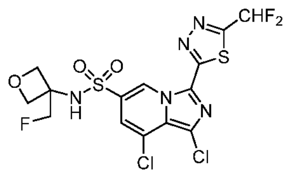 , 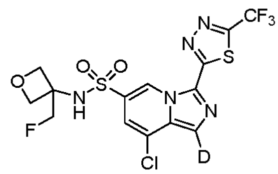 ,

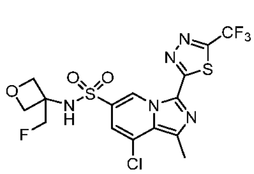 or 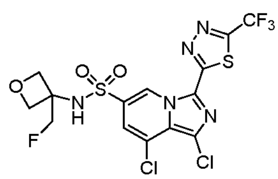 ;

and/or , said compound of formula V is of any one of the following structures:

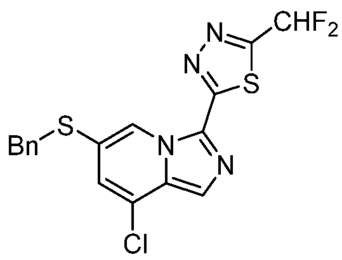

,

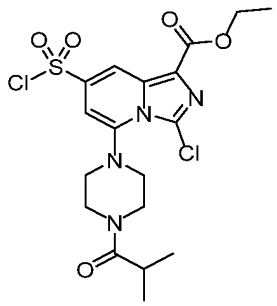

,

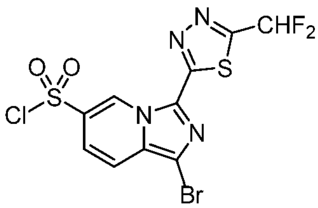

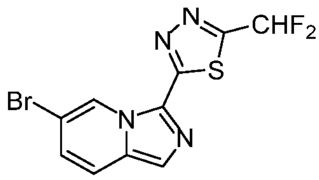

or

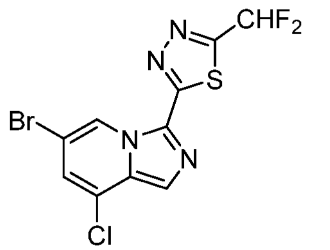

**INTERNATIONAL SEARCH REPORT**

International application No.
**PCT/CN2023/104130**

**A. CLASSIFICATION OF SUBJECT MATTER**

C07D471/04(2006.01)i； A61K31/437(2006.01)i； A61P35/00(2006.01)i

According to International Patent Classification (IPC) or to both national classification and IPC

**B. FIELDS SEARCHED**

Minimum documentation searched (classification system followed by classification symbols)

IPC: C07D, A61K, A61P

Documentation searched other than minimum documentation to the extent that such documents are included in the fields searched

Electronic data base consulted during the international search (name of data base and, where practicable, search terms used)

DWPI CNTXT ENTXTC STN: PARG抑制剂, 癌症, PARG, inhibitor?, cancer?, STN结构式检索, STN structural formula search

**C. DOCUMENTS CONSIDERED TO BE RELEVANT**

| Category* | Citation of document, with indication, where appropriate, of the relevant passages | Relevant to claim No. |
|---|---|---|
| PX | WO 2023057389 A1 (FORX THERAPEUTICS AG) 13 April 2023 (2023-04-13) claims 1-40 and embodiments 1-299 | 1, 7-9, 11, 13-25 |
| X | WO 2022123272 A1 (ADORX THERAPEUTICS LIMITED) 16 June 2022 (2022-06-16) claims 1-26, and embodiments 2, 2.1-2.3, 2.5-2.7, 2.9, and 3-5 | 1-8, 10, 11-16, 19, 21-23 |
| A | CN 111867581 A (MERCK PATENT GMBH et al.) 30 October 2020 (2020-10-30) claims 1-38 | 1-25 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **23 October 2023** | **26 October 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2023/104130**

| Patent document cited in search report | | | Publication date (day/month/year) | Patent family member(s) | | | Publication date (day/month/year) |
|---|---|---|---|---|---|---|---|
| WO | 2023057389 | A1 | 13 April 2023 | None | | | |
| WO | 2022123272 | A1 | 16 June 2022 | IL | 303492 | A | 01 August 2023 |
| | | | | GB | 202019622 | D0 | 27 January 2021 |
| | | | | AU | 2021397402 | A1 | 13 July 2023 |
| | | | | KR | 20230118162 | A | 10 August 2023 |
| | | | | CA | 3201252 | A1 | 16 June 2022 |
| CN | 111867581 | A | 30 October 2020 | CA | 3089769 | A1 | 01 August 2019 |
| | | | | RU | 2020128586 | A | 03 March 2022 |
| | | | | SG | 11202006832 | YA | 28 August 2020 |
| | | | | EP | 3746071 | A1 | 09 December 2020 |
| | | | | EP | 3746071 | A4 | 01 September 2021 |
| | | | | AR | 114236 | A1 | 05 August 2020 |
| | | | | AU | 2019212969 | A1 | 13 August 2020 |
| | | | | TW | 201940481 | A | 16 October 2019 |
| | | | | MX | 2020007797 | A | 18 September 2020 |
| | | | | US | 2021040083 | A1 | 11 February 2021 |
| | | | | IL | 276147 | A | 30 September 2020 |
| | | | | JP | 2021512078 | A | 13 May 2021 |
| | | | | WO | 2019148136 | A1 | 01 August 2019 |
| | | | | BR | 112020015396 | A2 | 08 December 2020 |
| | | | | KR | 20200115620 | A | 07 October 2020 |
| | | | | US | 2019233411 | A1 | 01 August 2019 |
| | | | | US | 10793563 | B2 | 06 October 2020 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2022107849339
- CN 2022115596106
- CN 2023107306040


### Non-patent literature cited in the description


- **MIRELLA et al.** Human poly (ADP-ribose) glycohydrolase is expressed in alternative splice variants yielding isoforms that localize to different cell compartments. *Experimental Cell Research*, 2004, vol. 297 (2), 521-532
- **MEYER B et al.** Clustered DNA damage induces pan-nuclear H2AX phosphorylation mediated by ATM and DNA-PK. *Nucleic Acids Res*, 2003, vol. 41, 6109-6118
- **O'SULLIVAN J. et al.** Emerging roles of eraser enzymes in the dynamic control of protein ADP-ribosylation.. *Nature Communication*, 2019, vol. 10, 1182
- **SLADE D et al.** The structure and catalytic mechanism of a poly (ADP-ribose) glycohydrolase.. *Nature*, 2011, vol. 477, 616-620
- **LAETITIA D et al.** Poly (ADP-ribose) glycohydrolase (PARG) and its therapeutic potential.. *Front Biosci*, 2009, vol. 14 (5), 1619-1626
- **RACK J D et al.** Macrodomain: structure, function, evolution, and catalytic activities. *Annu Rev Biochem.*, 2016, vol. 85, 431-454
- **MARQUES M et al.** Oncogenic activity of poly (ADP-ribose) glycohydrolase.. *Oncogene*, 2019, vol. 38, 2177-2191
- **MAUD M et al.** Oncogenic activity of poly (ADP-ribose) glycohydrolase.. *Oncogene*, 2019, vol. 38, 2177-2191
- **MINCHENG YET**. PARG inhibition limits HCC progression and potentiates the efficacy of immune checkpoint therapy. *Hepatic and Biliary Cancer*, 2022, vol. 00072-1 (22), S0168-8278
- **P. HEINRICH STAHL** ; **CAMILLE G. WERMUTH**. Handbook of Pharmaceutical Salts: Properties, Selection, and Use. 2011
- **GREENE et al.** Protective Groups in Organic Synthesis. Wiley and Sons, 1999